# EUROPÄISCHE PATENTANMELDUNG

(11) **EP 4 406 606 A2**
(43) Veröffentlichungstag der Anmeldung: **31.07.2024**
(21) Anmeldenummer: 24168990.0
(22) Anmeldetag: 20.06.2016
(51) Int. Cl.: A61P 35/00

(54) **BINDER-WIRKSTOFF-KONJUGATE (ADCS) UND BINDER-PRODRUG-KONJUGATE (APDCS) MIT ENZYMATISCH SPALTBAREN G**

(30) Priorität: 22.06.2015 EP 15173102; 16.03.2016 EP 16160738
(62) Teilanmeldung aus: 16730833.7
(71) Anmelder: Bayer Pharma Aktiengesellschaft, 13353 Berlin (DE)
(72) Erfinder: LERCHEN, Hans-Georg, 51375 Leverkusen (DE); REBSTOCK, Anne-Sophie, 69410 Champagne au Mont d'Or (FR); CANCHO GRANDE, Yolanda, 51373 Leverkusen (DE); MARX, Leo, 1920 Martigny (CH); STELTE-LUDWIG, Beatrix, 42489 Wülfrath (DE); TERJUNG, Carsten, 44799 Bochum (DE); MAHLERT, Christoph, 42111 Wuppertal (DE); GREVEN, Simone, 41541 Dormagen (DE); SOMMER, Anette, 10405 Berlin (DE); BERNDT, Sandra, 16540 Hohen Neuendorf (DE)
(74) Vertreter: HGF

(57) **Zusammenfassung**

Die vorliegende Anmeldung betrifft neuartige Binder-Prodrug-Konjugate (APDCs), bei denen Binder mit inaktiven Vorläuferverbindungen von Kinesin Spindel Protein-Inhibitoren konjugiert werden, sowie Antikörper-Wirkstoff-Konjugate ADCs und Verfahren zur Herstellung dieser APDCs bzw. ADCs.

## Beschreibung

### Einleitung und Stand der Technik

Die Erfindung betrifft neuartige Binder-Prodrug-Konjugate (APDCs), bei denen Binder mit inaktiven Vorläuferverbindungen von Kinesin Spindel Protein-Inhibitoren konjugiert werden, sowie Binder-Wirkstoff-Konjugate ADCs, aktive Metabolite dieser Binder-Prodrug-Konjugate und Binder-Wirkstoff-Konjugate, Verfahren zur Herstellung dieser APDCs bzw. ADCs, die Verwendung dieser Konjugate zur Behandlung und/oder Prävention von Krankheiten sowie die Verwendung dieser Konjugate zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen. Der Binder ist erfindungsgemäß vorzugsweise ein Antikörper.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Konjugate von Binderproteinen mit einem oder mehreren Wirkstoffmolekülen sind bekannt, insbesondere in Form sogannter "antibody drug conjugates" (ADCs), in welchen ein internalisierender, gegen ein Tumor-assoziiertes Antigen gerichteter Antikörper auf kovalente Weise über eine Verknüpfungseinheit ("linker") mit einem cytotoxisch wirkenden Agens verbunden ist. Nach Einschleusung des ADCs in die Tumorzelle und anschließender Spaltung des Konjugats wird dann innerhalb der Tumorzelle entweder das cytotoxische Agens selbst oder ein anderer daraus gebildeter cytotoxisch wirksamer Metabolit freigesetzt, und kann dort seine Wirkung direkt und selektiv entfalten. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5, 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)]. So beschreibt WO2012/171020 ADCs, bei denen mehrere Toxophormoleküle über einen polymeren Linker an einen Antikörper geknüpft ist. Als mögliche Toxophore werden in WO2012/171020 unter anderem die Substanzen SB 743921, SB 715992 (Ispinesib), MK-0371, AZD8477, AZ3146 und ARRY-520 erwähnt.

Die zuletzt genannten Substanzen sind sogenannte Kinesin Spindel Protein-Inhibitoren. Kinesin Spindel Protein (KSP, auch bekannt als Eg5, HsEg5, KNSL1 oder KIF11) ist ein Kinesin-ähnliches Motorprotein, welches für die Funktion der bipolaren mitotischen Spindel essentiell ist. Die Inhibierung von KSP führt zum mitotischen Stillstand und über einen längeren Zeitraum zur Apoptose (Tao et al., Cancer Cell 2005 Jul 8(1), 39-59). Nach dem Auffinden des ersten zellgängigen KSP-Inhibitors, Monastrol, haben sich KSP-Inhibitoren als eine Klasse neuer Chemotherapeutika etabliert (Mayer et al., Science 286: 971-974, 1999) und sind Gegenstand einer Reihe von Patentanmeldungen (z.B. WO2006/044825; WO2006/002236; WO2005/051922; WO2006/060737; WO03/060064; WO03/040979; und WO03/049527). Da KSP jedoch nur in einem kurzen Zeitraum der Mitosephase aktiv ist, müssen KSP-Inhibitoren in ausreichend hoher Konzentration während dieser Phase vorliegen. WO2014/151030 offenbart ADCs mit bestimmten KSP-Inhibitoren.

Legumain ist eine Tumor-assoziierte Asparaginyl-Endopeptidase (S. Ishii, Methods Enzymol. 1994, 244, 604; J. M. Chen et al. J. Biol. Chem. 1997, 272, 8090) und wurde zur Prozessierung von Prodrugs von kleinen cytotoxischen Molekülen wie zum Beispiel unter anderem von Doxorubicin und Etoposid Derivaten genutzt (W. Wu et al. Cancer Res. 2006, 66, 970; L. Stern et al. Bioconjugate Chem. 2009, 20, 500; K.M. Bajjuri et al. ChemMedChem 2011, 6, 54).

Andere lysosomale Enzyme sind beispielsweise Cathepsin oder Glycosidasen wie beispielsweise β-Glucuronidasen, die auch zur Freisetzung der aktiven Wirkstoffe durch enzymatische Spaltung von Prodrugs genutzt wurden. Enzymatisch *in vivo* spaltbaren Gruppen sind insbesondere 2-8-Oligopeptidgruppen oder Glycoside. Peptidspaltstellen sind in Bioconjugate Chem. 2002, 13, 855-869, sowie Bioorganic & Medicinal Chemistry Letters 8 (1998) 3341-3346 *sowie* Bioconjugate Chem. 1998, 9, 618-626 offenbart. Hierzu gehören z.B. Valin-Alanin, Valin-Lysin, Valin-Citrullin, Alanin-Lysin und Phenylalanin-Lysin (ggf. mit zusätzlicher Amidgruppe).

### Zusammenfassung der Erfindung

Um die Tumorselektivität von ADCs und ihrer Metabolite weiter zu verbessern, wurden Binder-Konjugate mit Peptidderivaten versehen, die durch Tumor-assoziierte Enzyme wie Legumain oder Cathepsin freigesetzt werden können. Die Tumorselektivität wird somit nicht nur durch die Wahl des Antikörpers, sondern zusätzlich durch die enzymatische Spaltung des Peptidderivates, z.B. durch das Tumor-assoziierte Enzym Legumain bestimmt.

Erfindungsgemäß kann das Peptidderivat in dem Linker, der den Binder mit dem KSP-Inhibitor verbindet, vorhanden sein. Dies sind die erfindungsgemäßen Binder-Wirkstoff-Konjugate (ADCs).

Die erfindungsgemäß verwendeten Kinesin Spindel Protein-Inhibitoren besitzen eine für die Wirkung essentielle Aminogruppe. Durch Modifizierung dieser Aminogruppe mit Peptidderivaten wird die Wirkung gegenüber dem Kinesin-Spindelprotein blockiert und damit auch die Entfaltung einer cytotoxischen Wirkung inhibiert. Kann dieser Peptidrest jedoch durch Tumor-assoziierte Enzyme wie Legumain freigesetzt werden, so lässt sich die Wirkung gezielt im Tumorgewebe wieder herstellen. Die Modifizierung der Aminogruppe ist in diesem Fall nicht Teil des Linkers. Daher betrifft die vorliegende Erfindung Binder-Konjugate mit inaktiven Vorläufermolekülen der Kinesin Spindel Protein-Inhibitoren, die erst im Tumor durch die Tumor-assoziierte lysosomale Endopeptidase Legumain zu den aktiven Metaboliten prozessiert werden, um somit gezielt im Tumor wieder ihre cytotoxische Aktivität entfalten zu können. Die Binder-Konjugate mit KSP-Inhibitoren, deren freie Aminogruppe entsprechend blockiert ist, werden erfindungsgemäß auch als APDCs bezeichnet. Die APDCs sind besonders bevorzugt.

Somit stellt die Erfindung bereit Konjugate eines Binders oder Derivats hiervon mit einem oder mehreren Wirkstoffmolekülen bzw. einem oder mehreren Prodrugs hiervon der folgenden Formel I: 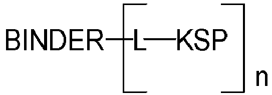
wobei BINDER für Binder bzw. ein Derivat hiervon (vorzugsweise für einen Antikörper), L für einen Linker, n für eine Zahl von 1 bis 50, vorzugsweise 1,2 bis 20 und besonders bevorzugt 2 bis 8 steht, und KSP für einen Kinesin Spindel Protein-Inhibitor bzw. Prodrug hiervon steht,
wobei L-KSP die folgende Formel (IIa) aufweist: 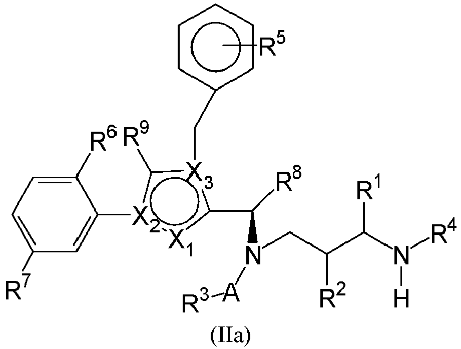 wobei
   X₁ N, X₂ N und X₃ C darstellt; oder
   X₁ N, X₂ C und X₃ N darstellt; oder
   X₁ CH oder CF, X₂ C und X₃ N darstellt; oder
   X₁ NH, X₂ C und X₃ C darstellt; oder
   X₁ CH, X₂ N und X₃ C darstellt;
   (wobei X₁ CH, X₂ C und X₃ N bevorzugt ist);
   R¹ H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
      wobei Y¹ und Y² unabhängig voneinander -H, -NH₂, -(CH₂CH₂O)₀₋₃-(CH₂)₀₋₃Z'(z.B. -(CH₂)₀₋₃Z'), oder -CH(CH₂W)Z' darstellen, und Y³ -H oder -(CH₂)₀₋₃Z' darstellt, wobei Z' -H, -NH₂, -SO₃H, -COOH, -NH-CO-CH₂-CH₂-CH(NH₂)COOH oder -(CO-NH-CHY⁴)₁₋₃COOH darstellt; wobei W -H oder -OH darstellt,
      wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
   R² -L-#1, H, -MOD, -CO-CHY⁴-NHY⁵ oder -(CH₂)₀₋₃Z darstellt,
      wobei Z -H, Halogen, -OY³, -SY³, NHY³, -CO-NY¹Y², oder -CO-OY³ darstellt,
      wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
      wobei Y⁴ lineares oder verzweigtes, gegebenenfalls mit-NHCONH₂ substituiertes C₁₋₆ Alkyl oder gegebenenfalls mit -NH₂ substituiertes Aryl oder Benzyl darstellt und Y^{5 -}H oder -CO-CHY⁶-NH₂ darstellt, wobei Y⁶ lineares oder verzweigtes C₁₋₆ Alkyl darstellt;
   R⁴ -L-#1, -H, -CO-CHY⁴-NHY⁵ oder -(CH₂)₀₋₃Z darstellt,
      wobei Z -H, Halogen, -OY³, -SY³, -NHY³, -CO-NY¹Y², oder -CO-OY³ darstellt, wobei Y¹ und Y² unabhängig voneinander -H, -NH₂, oder -(CH₂)₀₋₃Z' darstellen, und Y³ H oder -(CH₂)₀₋₃Z' darstellt, wobei Z' -H, -SO₃H, -NH₂ oder -COOH darstellt;
      wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y^{5 -}H oder -CO-CHY⁶-NH₂ darstellt, wobei Y⁶ lineares oder verzweigtes C₁₋₆ Alkyl darstellt;
   oder R⁴ eine Gruppe der Formel R²¹-(CO)₍₀₋₁₎-(P3)₍₀₋₂₎-P2-NH-CH(CH₂CONH₂)-CO- oder R²¹-(CO)₍₀₋₁₎-(P3)₍₀₋₂₎-P2-NH-CH(CH₂COOH)-CO- oder die durch Cathepsin spaltbare Gruppe der Formel R²¹-(CO)₍₀₋₁₎-(P3)₍₁₋₂₎-P2- darstellt, darstellt,
      wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - - NH₂, -NH-Alkyl, -N(Alkyl)₂, -NH-CO-Alkyl, -N(Alkyl)-COAlkyl, -SO₃H, -SO₂NH₂,--SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
      P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
      P3 eine Aminosäure ausgewählt wird aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist (wenn mehr als ein P3 vorliegt, kann P3 also unterschiedliche Bedeutungen aufweisen);
   oder R² und R⁴ gemeinsam (unter Bildung eines Pyrrolidinringes) -CH₂-CHR¹⁰- oder - CHR¹⁰-CH₂- darstellen, wobei R¹⁰ -H, -NH₂, -SO₃H, -COOH, -SH, Halogen (insbesondere F oder Cl), C₁₋₄ Alkyl, C₁₋₄ Haloalkyl, C₁₋₄ Alkoxy, Hydroxyl-substituiertes C₁₋₄ Alkyl, COO(C₁₋₄ Alkyl) -OH ist und wobei das Wasserstoffatom der sekundären Aminogruppe im Pyrrolidinring durch R²¹-CO-P3₍₀₋₂₎-P2-NH-CH(CH₂CONH₂)-CO-SIG- ersetzt sein kann, wobei SIG eine self-immolative Gruppe darstellt, die nach Spaltung der CO-SIG-Bindung das sekundäre Amin freisetzt;
   A -C(=O)-, -S(=O)-, -S(=O)z-, -S(=O)₂NH- oder -C(=N-NH₂)- darstellt;
   R³ -L-#1, -MOD, oder eine gegebenenfalls substituierte Alkyl-, Cycloalkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe,
      die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-C(=O)-Alkyl Gruppen, 1-3 -O-C(=O)-NH-Alkyl Gruppen, 1-3 - NH-C(=O)-Alkyl Gruppen, 1-3 -NH-C(=O)-NH-Alkyl Gruppen, 1-3 -S(=O)ₙ-Alkyl Gruppen, 1-3 -S(=O)z-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, n für 0, 1 oder 2 steht, Y¹ und Y² unabhängig voneinander -H, -NH₂, oder -(CH₂)₀₋₃Z' darstellen, und Y³ -H, -(CH₂)₀₋₃-CH(NHC(=O)CH₃)Z',-(CH₂)₀₋₃-CH(NH₂)Z', oder -(CH₂)₀₋₃Z' darstellt, wobei Z' -H, -SO₃H, -NH₂ oder -COOH darstellt,
      substituiert sein kann (wobei "Alkyl" bevorzugt C₁₋₁₀-Alkyl bedeutet);
   R⁵ -H, -NH₂, -NOz, Halogen (insbesondere -F, -Cl, -Br), -CN, -CF₃, -OCF₃, -CH₂F, -CH₂F, -SH oder -(CH₂)₀₋₃Z darstellt,
      wobei Z -H, -OY³, -SY³, Halogen, -NHY³, -CO-NY¹Y², oder -CO-OY³ darstellt,
      wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
      darstellt,
   R⁶ und R⁷ unabhängig voneinander -H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, -NO₂, -NH₂, -COOH oder Halogen (insbesondere -F, -Cl, -Br) darstellen,
   R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, -COzH, -NH₂ oder -L-#1 substituiert sein kann;
   R⁹ -H, -F, -CH₃, -CF₃, -CH₂F oder -CHF₂ darstellt;
   wobei einer der Substituenten R¹, R², R³, R⁴und R⁸ -L-91 darstellt bzw. (im Falle von R⁸) aufweist,
   -L- für den Linker und -#1 für die Bindung zum Binder bzw. Derivat hiervon steht,
   wobei -MOD -(NR¹⁰)ₙ-(G1)ₒ-G2-G3 darstellt, wobei
      R¹⁰ H, Halogen oder C₁-C₃-Alkyl darstellt;
      G1 -NHCO- , -CONH- oder 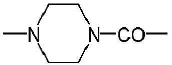 darstellt (wobei wenn G1 -NHCO- oder 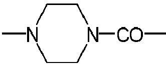 darstellt, R¹⁰ nicht NH₂ ist);
      n 0 oder 1 ist;
      o 0 oder 1 ist; und
      G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SOz, -NRY-, -NRYCO-, CONRY-, -NRYNRY-, -SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit -NHC(=O)NH₂, -COOH, -OH, -NH₂, -NH-(CH=N-NH₂), Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein kann, und G3 -H oder -COOH darstellt;
      wobei die Gruppe -MOD vorzugsweise zumindest eine Gruppe -COOH aufweist;
   wobei eine oder mehrere der folgenden Bedingungen (i) bis (iii) erfüllt ist:
      (i) -L-#1 eine Gruppe der Formel -(CO)₍₀₋₁₎-(P3)₍₀₋₂₎-P2-NH-CH(CH₂COX)-CO-umfasst,
         wobei X -NH2 oder -COOH, vorzugsweise -NH2 darstellt; P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist;
         P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
      (ii) R⁴ die Gruppe der Formel R²¹-(CO)₍₀₋₁₎(P3)₍₀₋₂₎-P2-NH-CH(CH₂CONH₂)-CO- oder die durch Cathepsin spaltbare Gruppe der Formel R²¹-(CO)₍₀₋₁₎-(P3)₍₀₋₂₎-P2-darstellt,
         wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist; P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His, oder eine der jeweiligen N-Alkyl-Aminosäuren, bevorzugt N-Methyl-Aminosäuren;
      (iii) R² und R⁴ gemeinsam (unter Bildung eines Pyrrolidinringes) -CH₂-CHR¹⁰- oder - CHR¹⁰-CH₂- darstellen, wobei das sekundäre Wasserstoffatom der sekundären Amingruppe des Pyrrolidinrings durch R²¹-CO-P3₍₀₋₂₎-P2-NH-CH(CH₂CONH₂)-CO-SIG- ersetzt ist, wobei SIG eine self-immolative Gruppe darstellt, die nach Spaltung der CO-SIG-Bindung das sekundäre Amin freisetzt;
   sowie ihre Salze, Solvate, Salze der Solvate und Epimere.

In den erfindungsgemäßen ADCs umfasst bzw. ist -L-#1 - die Gruppe der Formel -(CO)₍₀₋₁₎-(P3) ₍₀₋₂₎-P2-NH-CH(CH₂COX)-CO-. Besonders bevorzugt sind solche Gruppen der Formel -(CO)₍₀₋₁₎-(P3)₍₀₋₂₎-P2-NH-CH(CH₂CONH₂)-CO-, die sich in dem im Experimentalteil beschriebenen Legumain-Assay also spaltbar herausgestellt haben. Besonders bevorzugt stellt einer der Substituenten R¹, R³ oder R⁴-L-#1 dar. Wenn R⁴ -L-#1 darstellt, bindet die Carbonylgruppe des Asparagins bzw. der Asparaginsäure direkt an dem Stickstoffatom, das in obiger Formel an R⁴ bindet.

In den erfindungsgemäßen APDCs ist R⁴ R²¹-(CO)₍₀₋₁₎-(P3)₍₀₋₂₎-P2-NH-CH(CH₂COX)-CO- oder die durch Cathepsin spaltbare Gruppe der Formel R²¹-(CO)₍₀₋₁₎-(P3)₍₁₋₂₎-P2-, oder das Wasserstoffatom vom NH im Pyrrolidinring ist durch R²¹-CO-P3₍₀₋₂₎-P2-NH-CH(CH₂COX)-CO-SIG- ersetzt.

Die Gruppen R²¹-(CO)₍₀₋₁₎-(P3)₍₀₋₂₎-P2-NH-CH(CH₂COX)-CO- bzw. R²¹-CO-P3-P2-NH-CH(CH₂COX)-CO-SIG- werden *in vivo* wohl durch das Enzym Legumain gespalten. Diese Gruppen werden daher im Folgenden auch als "durch Legumain spaltbare Gruppen" bezeichnet. Die durch Legumain spaltbare Gruppe weist die Formel -(CO)₍₀₋₁₎-(P3)₍₀₋₂₎-P2-NH-CH(CH₂CONX)-CO- auf. In den erfindungsgemäßen APDCs weist die Gruppe vorzugsweise die Formel R21-(CO)₍₀₋₁₎-(P3)₍₀₋₂₎-P2-NH-CH(CH₂COX)-CO-# auf, d.h. die durch Legumain spaltbare Gruppe weist an einem Ende die Gruppe R²¹ auf, am anderen Ende (-#) bindet sie an die Aminogruppe entsprechend Position R4 in Formel IIa.

Hierbei liegt NH-CH(CH₂COX)-CO- (also Asparagin bzw. Asparaginsäure) in der natürlichen L-Konfiguration vor. Besonders bevorzugt sind solche Gruppen, die sich in dem im Experimentalteils beschriebenen Legumain-Assay also spaltbar herausgestellt haben. Die erfindungsgemäßen APDCs können zusätzlich zu der durch Legumain oder Cathepsin spaltbaren Gruppe R⁴ einen Linker -L-#1 mit einer durch Legumain oder Cathepsin spaltbaren Gruppe aufweisen.

-NH-CH(CH₂CONH₂)-CO- in der durch Legumain spaltbaren Gruppe ist Asparagin, - NH-CH(CH₂COOH)-CO- in der durch Legumain spaltbaren Gruppe ist Asparaginsäure. Asparagin und Asparaginsäure liegen hierbei als L(-)-Asparagin bzw. L-Asparaginsäure vor. Die durch Legumain spaltbare Gruppe weist neben Asparagin bzw. Asparaginsäure 1 bis 3 weitere Aminosäuren auf (im Falle von Asparagin also -P2-NH-CH(CH₂CONH₂)-CO-; -P3-P2-NH-CH(CH₂CONH₂)-CO; -(P3)₍₂₎-P2-NH-CH(CH₂CONH₂)-CO-), ist also ein Di-, Tri-, oder Tetrapeptid bzw. Derivat hiervon (Dipeptid: -P2-NH-CH(CH₂CONH₂)-CO-; Tripeptid: -P3-P2-NH-CH(CH₂CONH₂)-CO; Tetrapeptid: -(P3)₂-P2-NH-CH(CH₂CONH₂)-CO- (wobei die beiden Aminosären P3 unterschiedlich sein können).

P2 ist eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His, vorzugsweise ausgewählt aus Ala, Gly, Val, Leu, Ile, Pro, Ser, Thr, Citrullin und Asn. P2 liegt regelmäßig in der natürlichen L-Konfiguration vor. Besonders bevorzugt ist L-Ala.

P3 ist eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, Citrullin, oder eine der jeweiligen N-Alkyl-Aminsoäuren, bevorzugt N-Methyl-Aminsoäuren. P3 wird vorzugsweise ausgewählt aus His, Pro, Ala, Val, Leu, Ile, Gly, Ser, Phe, Citrullin und Gln. P3 liegt regelmäßig in der natürlichen L-Konfiguration vor. Besonders bevorzugt ist L-Ala. Wenn mehr als eine Aminosäure P3 vorliegt, können sich diese Aminosäuren im Rahmen der obigen Definition unterscheiden.

Besonders bevorzugt ist die durch Legumain spaltbare Gruppe -L-Ala-L-Ala-L-Asn- (im Falle der APDCs also R²¹-L-Ala-L-Ala-L-Asn-#).

R²¹ stellt vorzugsweise -H , eine C₁₋₅-Alkyl-, C₅₋₁₀-Aralkyl-, C₁₋₅-Alkoxy-, C₆₋₁₀-AryloxyGruppe, C₅₋₁₀-Heteroalkyl-, C₅₋₁₀-Heterocycloalkyl-, Heteroaryl-, Heteroaryl-alkyl-, C₅₋₁₀-Heteroalkoxy-, oder eine C₅₋₁₀-Heterocycloalkoxy-Gruppe dar, die jeweils mit - -COOH, COOAlkyl, COONH₂, NH₂ oder N(Alkyl)₂, substituiert sein kann, oder eine Gruppe -Oₓ-(CH₂CH₂O)_{y}-R²² dar, (wobei x 0 oder 1 und v eine Zahl von 1 bis 20 darstellt, und R²² - H, -Alkyl -CH2-COOH, -CH2-CH2-COOH, oder -CH2-CH2-NH2). "Alkyl" bezeichnet hierbei eine Alkylgruppe mit bis zu 20 Kohlenstoffatomen; vorzugsweise C₁₋₁₂-Alkyl.

Die durch Cathepsin spaltbare Gruppe weist die Formel -(CO)₍₀₋₁₎-(P3)₍₁₋₂₎-P2- auf. In den erfindungsgemäßen APDCs weist die Gruppe die Formel R²¹-(CO)₍₀₋₁₎-(P3)₍₁₋₂₎-P2-# auf, d.h. die durch Cathepsin spaltbare Gruppe weist an einem Ende die Gruppe R²¹ auf, am anderen Ende (-#) bindet sie an die Aminogruppe entsprechend Position R4 in Formel IIa. Hierbei haben R21, P2 und P3 die selbe Bedeutung wie bei der durch Legumain spaltbaren Gruppe. Ob die Gruppe der Formel R²¹-(CO)₍₀₋₁₎-(P3)₍₁₋₂₎-P2--# durch Cathepsin spaltbar ist, läßt sich anhand des im Experimentalteils beschriebenen Cathepsin-Assays bestimmen. Besonders bevorzugte durch Cathepsin spaltbare Gruppen sind solche, in denen P2 ausgewählt wird aus Alanin, Lysin und Citrullin, und P3 ausgewählt wird aus Valin, Alanin und Phenylalanin, insbesondere solche der Formel R²¹-(CO)₍₀₋₁₎-P3-P2-.

### Beschreibung der Figuren

- **Figur**: **1:** Alignment der TWEAKR-cysteinreichen Domäne (Aminosäure 34 bis 68) von verschiedenen Spezies. (Die Zahlen zeigen die Aminosäureposition in Vollängenkonstrukten inklusive der Signalsequenzen; SEQ ID NO: 169).
- **Figur**: **2:**
A - Schematisches Diagram der Struktur von TWEAKR (SEQ ID NO: 169). Das Diagramm zeigt die extrazelluläre Domäne (Aminosäuren 28-80) (SEQ ID NO: 168) einschließlich der cysteinreichen Domäne (36-67), der Transmembrandomäne - TM (81-101) und der intrazellulären Domäne (102-129). TPP-2202 - die vollständige Ektodomäne (28-80), an die Fc-Domäne von hIgG1 fusioniert. TPP-2203 - extrazelluläre Domäne mit N- und C-terminaler Verkürzung (34-68), fusioniert an die Fc-Domäne von hIgG1. Disulfidbrücken Cys36-Cys49, Cys52-Cys67 und Cys55-Cys64 sind durch schwarze Balken angezeigt. N-terminal erhält TPP-2203 zwei Aminosäuren und C-terminal eine Aminosäure mehr verglichen mit der reinen cysteinreichen Domäne, um eine anständige Faltung zu gewährleisten. TPP-1984 - extrazelluläre Domäne mit C-terminaler Verkürzung (28-68), an einen HIS6-Tag fusioniert. Alle drei Konstrukte zeigen eine vergleichbare Bindung an die erfindungsgemäßen Antikörper und PDL-192 (TPP-1104). P4A8 (TPP-1324) bindet nur an die Vollängen-extrazelluläre Domäne (TPP-2202).
B - Aminosäuresequenz der extrazellulären Domäne: Es ist veröffentlicht worden, dass die Aminosäure 64 essenziell für die TWEAK-Ligandenbindung ist; und die Aminosäure 47 ist essentiell für die Bindung der erfindungsgemäßen Antikörper, wie hier bestimmt wurde.
- **Figur**: **3:** Schematische Darstellung der Transglutaminase-katalysierten konjugationsstellenspezifischen Funktionalsisierung aglykosylierter Antikörper.
- **Figur**: **4:** Darstellung von nacheinander geschalteten enzymatischne Schritten zur Wirkstofffreisetzung durch Histone Deacetylase und Cathepsin L gemäß Nat. Commun., 2013, 4, 2735

### Detaillierte Beschreibung der Erfindung

Die Erfindung stellt Konjugate eines Binders oder Derivats hiervon mit einem oder mehreren Wirkstoffmolekülen bzw. Prodrugs hiervon bereit, wobei das Wirkstoffmolekül ein Kinesin Spindel Protein-Inhibitor (KSP-Inhibitor) ist.

Im Folgenden werden erfindungsgemäß verwendbare Binder, erfindungsgemäß verwendbare KSP-Inhibitoren bzw. Prodrugs hiervon sowie erfindungsgemäß verwendbare Linker beschrieben, die ohne Einschränkung in Kombination verwendet werden können. Insbesondere können die jeweils als bevorzugt bzw. besonders bevorzugt dargestellten Binder in Kombination mit den jeweils als bevorzugt bzw. besonders bevorzugt dargestellten KSP-Inhibitoren bzw. Prodrugs, ggf. in Kombination mit den jeweils als bevorzugt bzw. besonders bevorzugt dargestellten Linkern verwendet werden.

### KSP-Inhibitoren und deren Binderkonjugate

Erfindungsgemäß weist KSP-L in Formel I die folgende Formel (IIa) auf: 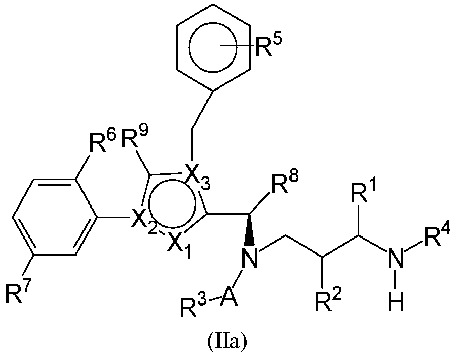 wobei
X₁ N, X₂ N und X₃ C darstellt; oder
X₁ N, X₂ C und X₃ N darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);

R¹ -H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
   wobei Y¹ und Y² unabhängig voneinander -H, -NH₂, -(CH₂CH₂O)₀₋₃-(CH₂)₀₋₃Z'(z.B. - (CH₂)₀₋₃Z'), oder -CH(CH₂W)Z' darstellen, und Y³ -H oder -(CH₂)₀₋₃Z' darstellt, wobei Z' -H, -NH₂, -SO₃H, -COOH, -NH-CO-CH₂-CH₂-CH(NH₂)COOH oder -(CO-NH-CHY⁴)₁₋₃COOH darstellt; wobei W -H oder -OH darstellt,
   wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R² -L-#1, -H, -MOD, -CO-CHY⁴-NHY⁵ oder -(CH₂)₀₋₃Z darstellt,
   wobei Z -H, Halogen, -OY³, -SY³, -NHY³, -CO-NY¹Y², oder -CO-OY³ darstellt,
   wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
   wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y^{5 -}H oder -CO-CHY⁶-NH₂ darstellt, wobei Y⁶ lineares oder verzweigtes C₁₋₆ Alkyl darstellt;
R⁴ -L-#1, H, -CO-CHY⁴-NHY⁵ oder -(CH₂)₀₋₃Z darstellt,
   wobei Z -H, Halogen, -OY³, -SY³, NHY³, -CO-NY¹Y², oder -CO-OY³ darstellt,
   wobei Y¹ und Y² unabhängig voneinander -H, -NH₂, oder -(CH₂)₀₋₃Z' darstellen, und Y³ H oder -(CH₂)₀₋₃Z' darstellt, wobei Z' -H, -SO₃H, -NH₂ oder -COOH darstellt;
   wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y^{5 -}H oder -CO-CHY⁶-NH₂ darstellt, wobei Y⁶ lineares oder verzweigtes C₁₋₆ Alkyl darstellt;
oder R⁴ eine Gruppe der Formel R²¹-(CO)₍₀₋₁₎-(P3)₍₀₋₂₎-P2-NH-CH(CH₂CONH₂)-CO- oder R²¹-(CO)₍₀₋₁₎-(P3)₍₀₋₂₎-P2-NH-CH(CH₂COOH)-CO- oder die durch Cathepsin spaltbare Gruppe der Formel R²¹-(CO)₍₀₋₁₎-(P3)₍₁₋₂₎-P2- darstellt,
   wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, NH-CO-Alkyl, N(Alkyl)-COAlkyl, -SO₃H, -SO₂NH₂, - SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
   P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
oder R² und R⁴ gemeinsam (unter Bildung eines Pyrrolidinringes) -CH₂-CHR¹⁰- oder - CHR¹⁰-CH₂- darstellen, wobei R¹⁰ H, NH₂, SO₃H, COOH, SH, Halogen (insbesondere F oder Cl), C₁₋₄ Alkyl, C₁₋₄ Haloalkyl, C₁₋₄ Alkoxy, Hydroxyl-substituiertes C₁₋₄ Alkyl, COO(C₁₋₄ Alkyl), OH oder R²¹-CO-P3-P2-NH-CH(CH₂CONH₂)-CO-SIG- darstellt, wobei SIG eine self-immolative Gruppe darstellt, die nach Spaltung der CO-SIG-Bindung das sekundäre Amin freisetzt;
A -C(=O)-, -S(=O)-, -S(=O)z-, -S(=O)₂NH- oder -C(=N-NH₂)- darstellt;
R³ -L-#1, -MOD, oder eine gegebenenfalls substituierte Alkyl-, Cycloalkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe,
   die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 - SOz-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, n für 0, 1 oder 2 steht, Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
   substituiert sein kann (wobei "Alkyl" bevorzugt C₁₋₁₀-Alkyl bedeutet);
R⁵ -H, -NH₂, -NOz, Halogen (insbesondere F, Cl, Br), -CN, -CF₃, -OCF₃, -CH₂F, -CH₂F, - SH oder -(CH₂)₀₋₃Z darstellt,
   wobei Z -H, -OY³, -SY³, Halogen, NHY³, -CO-NY¹Y², oder -CO-OY³ darstellt,
   wobei Y¹ und Y² unabhängig voneinander -H, -NH₂, oder -(CH₂)₀₋₃Z' darstellen, und Y³ H oder -(CH₂)₀₋₃Z' darstellt, wobei Z' -H, -SO₃H, -NH₂ oder -COOH darstellt;
   darstellt,
R⁶ und R⁷ unabhängig voneinander -H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, -NOz, -NH₂, -COOH oder Halogen (insbesondere -F, -Cl, -Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, -COzH, -NH₂ oder -L-#1 substituiert sein kann;
R⁹ -H, -F, -CH₃, -CF₃, -CH₂F oder -CHF₂ darstellt;
wobei einer der Substituenten R¹, R², R³, R⁴und R⁸ -L-91 darstellt bzw. (im Falle von R⁸) aufweist,
-L für einen Linker und -#1 für die Bindung zum Binder bzw. Derivat hiervon steht,
wobei -MOD -(NR¹⁰)ₙ-(G1)ₒ-G2-G3 darstellt, wobei
   R¹⁰ -H oder C₁-C₃-Alkyl darstellt;
   G1 -NHCO- , -CONH- oder 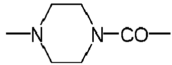 darstellt (wobei wenn G1 -NHCO- oder 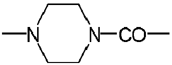 darstellt, R¹⁰ nicht -NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SOz, -NRY-, -NRYCO-, -CONRY-, -NRYNRY-, - SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit NHC(=O)NH₂, -COOH, -OH, -NH₂, -NH-CNNH₂, Sulfonamid, Sulfon, Sulfoxid, oder Sulfonsäure substituiert sein können), -C(=O)-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, -NH-C(=NNH₂), Sulfonamid, Sulfon, Sulfoxid, oder Sulfonsäure substituiert sein kann, G3 -H oder -COOH darstellt;
   wobei die Gruppe -MOD vorzugsweise zumindest eine Gruppe -COOH aufweist;
wobei eine oder mehrere der folgenden Bedingungen (i) bis (iii) erfüllt ist:
   (i) -L-#1 eine Gruppe der Formel -(CO)₍₀₋₁₎-(P3)₍₀₋₂₎-P2-NH-CH(CH₂COX)-CO- umfasst,
      wobei X -NH₂ oder -COOH, vorzugsweise -NH₂ darstellt,
      P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist;
      P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   (ii) R⁴ die Gruppe der Formel R²¹-(CO)₍₀₋₁₎(P3)₍₀₋₂₎-P2-NH-CH(CH₂CONH₂)-CO- oder die durch Cathepsin spaltbare Gruppe der Formel R²¹-(CO)₍₀₋₁₎-(P3)₍₁₋₂₎-P2- darstellt,
      wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist;
      P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   (iii) R² und R⁴ gemeinsam (unter Bildung eines Pyrrolidinringes) -CH₂-CHR¹⁰- oder -CHR¹⁰-CH₂- darstellen, wobei das sekundäre Wasserstoffatom der sekundären Amingruppe des Pyrrolidinrings durch R²¹-CO-P3-P2-NH-CH(CH₂CONH₂)-CO-SIG- ersetzt ist, wobei SIG eine self-immolative Gruppe darstellt, die nach Spaltung der CO-SIG-Bindung das sekundäre Amin freisetzt;;
sowie ihre Salze, Solvate, Salze der Solvate und Epimere.

### Definitionen

Der Begriff "substituiert" bedeutet, dass ein oder mehrere Wasserstoffe an dem bezeichneten Atom bzw. der bezeichneten Gruppe durch eine Auswahl aus der angegebenen Gruppe ersetzt ist/sind, mit der Maßgabe, dass die normale Wertigkeit des bezeichneten Atoms unter den vorliegenden Umständen nicht überschritten wird. Kombinationen von Substituenten und/oder Variablen sind zulässig.

Der Begriff "gegebenenfalls substituiert" bedeutet, dass die Anzahl an Substituenten gleich oder verschieden von Null sein kann. Wenn nicht anders angegeben, können gegebenenfalls substituierte Gruppen durch so viele fakultative Substituenten substituiert sein, wie sich durch Ersetzen eines Wasserstoffatoms durch einen Nicht-Wasserstoff-Substituenten an einem beliebigen verfügbaren Kohlenstoff oder Stickstoff- oder Schwefelatom unterbringen lassen. Normalerweise kann die Anzahl an fakultativen Substituenten (falls vorhanden) 1, 2, 3, 4 oder 5, insbesondere von 1, 2 oder 3 sein.

So, wie hier verwendet, bedeutet der Ausdruck "ein- oder mehrfach", zum Beispiel in der Definition der Substituenten der Verbindungen der allgemeinen Formeln der vorliegenden Erfindung, "1, 2, 3, 4 oder 5, bevorzugt 1, 2, 3 oder 4, besonders bevorzugt 1, 2 oder 3, ganz besonders bevorzugt 1 oder 2".

Sind Reste in den erfindungsgemäßen Verbindungen substituiert, so können die Reste, wenn nicht anders angegeben, ein- oder mehrfach substituiert sein. Im Schutzbereich der vorliegenden Erfindung sind die Bedeutungen aller Reste, die mehrfach auftreten, unabhängig voneinander. Eine Substitution durch einen, zwei oder drei gleiche oder verschiedene Substituenten ist bevorzugt. Die Substitution durch einen Substituenten ist besonders bevorzugt.

### Alkyl

Alkyl steht für einen linearen oder verzweigten, gesättigten, monovalenten Kohlenwasserstoffrest mit 1 bis 10 Kohlenstoffatomen (C₁-C₁₀-Alkyl), in der Regel 1 bis 6 (C₁-C₆-Alkyl), bevorzugt 1 bis 4 (C₁-C₄-Alkyl), und besonders bevorzugt 1 bis 3 Kohlenstoffatomen (C₁-C₃-Alkyl).

Beispielhaft und bevorzugt seien genannt:
Methyl-, Ethyl-, Propyl-, Butyl-, Pentyl-, Hexyl-, iso-Propyl-, iso-Butyl-, sec-Butyl, tert-Butyl-, iso-Pentyl-, 2-Methylbutyl-, 1-Methylbutyl-, 1-Ethylpropyl-, 1,2-Dimethylpropyl, neo-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- und 1,2-Dimethylbutyl-.
Besonders bevorzugt ist ein Methyl-, Ethyl-, Propyl-, Isopropyl- und tert-Butylrest.

### Heteroalkyl

Heteroalkyl steht für eine geradkettige und/oder verzweigte Kohlenwasserstoffkette mit 1 bis 10 Kohlenstoffatomen, die einfach oder mehrfach durch eine oder mehrere der Gruppen -O-, -S-, -C(=O)-, -S(=O)-, -S(=O)z-, -NRY-, -NRYC(=O)-, -C(=O)-NRY-, -NRYNRY-, -S(=O)z-NRYNRY-, -C(=O)-NRYNRY-, -CR^{x}=N-O- unterbrochen sein kann, und wobei die Kohlenwasserstoffkette einschließlich der Seitenketten, sofern vorhanden, mit -NH-C(=O)-NH₂, -C(=O)-OH, -OH, -NH₂, -NH-C(=NNH₂)-, Sulfonamid, Sulfon, Sulfoxid, oder Sulfonsäure substituiert sein kann,

Hierbei steht R^{y} jeweils für -H, Phenyl-, C₁-C₁₀-Alkyl-, C₂-C₁₀-Alkenyl- oder C₂-C₁₀-Alkinyl-, die wiederum jeweils mit -NH-C(=O)-NH₂, -C(=O)-OH, -OH, -NH₂, -NH-C(=NNH₂)-, Sulfonamid, Sulfon, Sulfoxid, oder Sulfonsäure substituiert sein können.

Hierbei steht R^{x} für -H, C₁-C₃-Alkyl- oder Phenyl-.

### Alkenyl

Alkenyl steht für eine geradkettige oder verzweigte einwertige Kohlenwasserstoffkette mit einer oder zwei Doppelbindungen und 2, 3, 4, 5, 6, 7, 8, 9 oder 10 Kohlenstoffatomen (C₂-C₁₀-Alkenyl), insbesondere 2 oder 3 Kohlenstoffatomen (C₂-C₃-Alkenyl), wobei es sich versteht, dass, wenn die Alkenylgruppe mehr als eine Doppelbindung enthält, die Doppelbindungen isoliert voneinander oder miteinander konjugiert sein können. Bei der Alkenylgruppe handelt es sich zum Beispiel um eine Ethenyl- (bzw. Vinyl-), Prop-2-en-1-yl- (bzw. "Allyl-"), Prop-1-en-1-yl-, But-3-enyl-, But-2-enyl-, But-1-enyl-, Pent-4-enyl-, Pent-3-enyl-, Pent-2-enyl-, Pent-1-enyl-, Hex-5-enyl-, Hex-4-enyl-, Hex-3-enyl-, Hex-2-enyl-, Hex-1-enyl-, Prop-1-en-2-yl- (bzw. "Isopropenyl-"), 2-Methylprop-2-enyl-, 1-Methylprop-2-enyl-, 2-Methylprop-1-enyl-, 1-Methylprop-1-enyl-, 3-Methylbut-3-enyl-, 2-Methylbut-3-enyl-, 1-Methylbut-3-enyl-, 3-Methylbut-2-enyl-, 2-Methylbut-2-enyl-, 1-Methylbut-2-enyl-, 3-Methylbut-1-enyl-, 2-Methylbut-1-enyl-, 1-Methylbut-1-enyl-, 1-,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, 3-Methylpent-3-enyl-, 2-Methylpent-3-enyl-, 1-Methylpent-3-enyl-, 4-Methylpent-2-enyl-, 3-Methylpent-2-enyl-, 2-Methylpent-2-enyl-, 1-Methylpent-2-enyl-, 4-Methylpent-1-enyl-, 3-Methylpent-1-enyl-, 2-Methylpent-1-enyl-, 1-Methylpent-1-enyl-, 3-Ethylbut-3-enyl-, 2-Ethylbut-3-enyl-, 1-Ethylbut-3-enyl-, 3-Ethylbut-2-enyl-, 2-Ethylbut-2-enyl-, 1-Ethylbut-2-enyl-, 3-Ethylbut-1-enyl-, 2-Ethylbut-1-enyl-, 1-Ethylbut-1-enyl-, 2-Propylprop-2-enyl-, 1-Propylprop-2-enyl-, 2-Isopropylprop-2-enyl-, 1-Isopropylprop-2-enyl-, 2-Propylprop-1-enyl-, 1-Propylprop-1-enyl-, 2-Isopropylprop-1-enyl-, 1-Isopropylprop-1-enyl-, 3,3-Dimethylprop-1-enyl-, 1-(1,1-Dimethylethyl)ethenyl-, Buta-1,3-dienyl-, Penta-1,4-dienyl- oder Hexa-1-5-dienylgruppe. Insbesondere handelt es sich bei der Gruppe um Vinyl oder Allyl.

### Alkinyl

Alkinyl steht für eine geradkettige oder verzweigte einwertige Kohlenwasserstoffkette mit einer Dreifachbindung und mit 2, 3, 4, 5, 6, 7, 8, 9 oder 10 Kohlenstoffatomen (C₂-C₁₀-Alkinyl), insbesondere 2 oder 3 Kohlenstoffatomen (C₂-C₃-Alkinyl). Bei der C₂-C₆-Alkinylgruppe handelt es sich zum Beispiel um eine Ethinyl-, Prop-1-inyl-, Prop-2-inyl- (bzw. Propargyl-), But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl-, 2-Methylpent-4-inyl-, 1-Methylpent-4-inyl-, 2-Methylpent-3-inyl-, 1-Methylpent-3-inyl-, 4-Methylpent-2-inyl-, 1-Methylpent-2-inyl-, 4-Methylpent-1-inyl-, 3-Methylpent-1-inyl-, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder 3,3-Dimethylbut-1-inylgruppe. Insbesondere handelt es sich bei der Alkinylgruppe um Ethinyl, Prop-1-inyl oder Prop-2-inyl.

### Cycloalkyl

Cycloalkyl steht für einen gesättigten einwertigen mono- oder bicyclischen Kohlenwasserstoffrest mit 3-12 Kohlenstoffatomen (C₃-C₁₂-Cycloalkyl).

Hierbei steht ein monocyclischer Kohlenwasserstoffrest für einen monovalenten Kohlenwasserstoffrest mit in der Regel 3 bis 10 (C₃-C₁₀-Cycloalkyl), bevorzugt 3 bis 8 (C₃-C₈-Cycloalkyl), und besonders bevorzugt 3 bis 7 (C₃-C₇-Cycloalkyl) Kohlenstoffatomen. Beispielhaft und bevorzugt für einen monocyclischen Kohlenwasserstoffrest seien genannt: Cyclopropyl-, Cyclobutyl-, Cyclopentyl-, Cyclohexyl-, Cycloheptyl- und Cyclooctyl-. Besonders bevorzugt ist ein Cyclopropyl-, Cyclobutyl-, Cylopentyl-, Cyclohexyl- und Cycloheptyl-.

Hierbei steht ein bicyclischer Kohlenwasserstoffrest für einen Kohlenwasserstoffrest mit in der Regel 3 bis 12 Kohlenstoffatomen (C₃-C₁₂-Cycloalkyl), wobei hierbei eine Fusion aus zwei gesättigten Ringsystemen zu verstehen ist, die sich gemeinsam zwei direkt benachbarte Atome teilen. Beispielhaft und bevorzugt für einen bicyclischen Kohlenwasserstoffrest seien genannt: Bicyclo[2.2.0]hexyl-, Bicyclo[3.3.0]octyl-, Bicyclo[4.4.0]decyl-, Bicyclo[5.4.0]undecyl-, Bicyclo[3.2.0]heptyl-, Bicyclo[4.2.0]octyl-, Bicyclo[5.2.0]nonyl-, Bicyclo[6.2.0]decyl-, Bicyclo[4.3.0]nonyl-, Bicyclo[5.3.0]decyl-, Bicyclo[6.3.0]undecyl- und Bicyclo[5.4.0]undecyl-,

### Heterocycloalkyl

Heterocycloalkyl steht für ein nicht aromatisches mono- oder bicyclisches Ringsystem mit ein, zwei, drei oder vier Heteroatomen, die gleich oder verschieden sein können. Als Heteroatome können Stickstoffatome, Sauerstoffatome oder Schwefelatome vorkommen.

Ein monocyclisches Ringsystem gemäß der vorliegenden Erfindung kann 3 bis 8, bevorzugt 4 bis 7, besonders bevorzugt 5 oder 6 Ringatome aufweisen.

Beispielhaft und bevorzugt für ein Heterocycloalkyl mit 3 Ringatomen seien genannt:
Aziridinyl-.

Beispielhaft und bevorzugt für ein Heterocycloalkyl mit 4 Ringatomen seien genannt:
Azetidinyl-, Oxetanyl-.

Beispielhaft und bevorzugt für ein Heterocycloalkyl mit 5 Ringatomen seien genannt: Pyrrolidinyl-, Imidazolidinyl-, Pyrazolidinyl-, Pyrrolinyl-, Dioxolanyl- und Tetrahydrofuranyl-.

Beispielhaft und bevorzugt für ein Heterocycloalkyl mit 6 Ringatomen seien genannt: Piperidinyl-, Piperazinyl-, Morpholinyl-, Dioxanyl-, Tetrahydropyranyl- und Thiomorpholinyl-.

Beispielhaft und bevorzugt für ein Heterocycloalkyl mit 7 Ringatomen seien genannt:
Azepanyl-, Oxepanyl-, 1,3-Diazepanyl-, 1,4-Diazepanyl-.

Beispielhaft und bevorzugt für ein Heterocycloalkyl mit 8 Ringatomen seien genannt:
Oxocanyl-, Azocanyl-.

Unter monocyclischem Heterocycloalkyl sind 4 bis 7-gliedrige, gesättigte Heterocyclylreste mit bis zu zwei Heteroatomen aus der Reihe O, N und S bevorzugt.

Besonders bevorzugt sind Morpholinyl-, Piperidinyl-, Pyrrolidinyl- und Tetrahydrofuranyl-.

Ein bicyclisches Ringsystem mit ein, zwei, drei oder vier Heteroatomen, die gleich oder verschieden sein können, können gemäß der vorliegenden Erfindung 6 bis 12, bevorzugt 6 bis 10 Ringatome aufweisen, wobei ein, zwei, drei oder vier Kohlenstoffatome gegen gleiche oder verschiedene Heteroatome aus der Reihe O, N und S ausgetauscht sein können.

Beispielhaft seien genannt: Azabicyclo[3.3.0]octyl-, Azabicyclo[4.3.0]nonyl-, Diazabicyclo[4.3.0]nonyl-, Oxazabicyclo[4.3.0]nonyl-, Thiazabicyclo[4.3.0]nonyl- oder Azabicyclo[4.4.0]decyl- sowie aus weiteren möglichen Kombinationen gemäß Definition abgeleitete Reste.

Besonders bevorzugt ist Perhydrocyclopenta[c]pyrrolyl-, Perhydrofuro[3,2-c]pyridinyl-, Perhydropyrrolo[1,2-a]pyrazinyl-, Perhydropyrrolo[3,4-c]pyrrolyl- und 3,4-Methylenedioxyphenyl.

### Aryl

Aryl bedeutet ein monovalentes, mono- oder bicyclisches, aus Kohlenstoffatomen bestehendes aromatisches Ringsystem. Beispiele sind Naphthyl- und Phenyl-; bevorzugt ist Phenylbeziehungsweise ein Phenylrest.

### C₆-C₁₀-Aralkyl

C₆₋₁₀-Aralkyl- steht im Rahmen der Erfindung für ein monocyclisches aromatisches Aryl, bespielhaft Phenyl, an das eine C₁-C₄-Alkylgruppe gebunden ist.

Eine beispielhafte C₆₋₁₀-Aralkyl-Gruppe ist Benzyl.

### Heteroaryl

Heteroaryl bedeutet ein einwertiges monocyclisches, bicyclisches oder tricyclisches aromatisches Ringsystem mit 5, 6, 8, 9, 10, 11, 12, 13 oder 14 Ringatomen (eine "5- bis 14- gliedrige Heteroaryl"gruppe), insbesondere 5, 6, 9 oder 10 Ringatomen, zu verstehen, das mindestens ein Ringheteroatom und gegebenenfalls ein, zwei oder drei weitere Ringheteroatome aus der Gruppe N, O und S enthält und das über ein Ringkohlenstoffatom oder gegebenenfalls (wenn es die Wertigkeit erlaubt) über ein Ringstickstoffatom gebunden ist.

Bei der Heteroarylgruppe kann es sich um eine 5-gliedrige Heteroarylgruppe wie zum Beispiel Thienyl, Furyl, Pyrrolyl, Oxazolyl, Thiazolyl, Imidazolyl, Pyrazolyl, Isoxazolyl, Isothiazolyl, Oxadiazolyl, Triazolyl, Thiadiazolyl oder Tetrazolyl; oder eine 6-gliedrige Heteroarylgrupoe wie zum Beispiel Pyridinyl, Pyridazinyl, Pyrimidinyl, Pyrazinyl oder Triazinyl; oder eine tricyclische Heteroarylgruppe wie zum Beispiel Carbazolyl, Acridinyl oder Phenazinyl; oder eine 9-gliedrige Heteroarylgruppe wie zum Beispiel Benzofuranyl, Benzothienyl, Benzoxazolyl, Benzisoxazolyl, Benzimidazolyl, Benzothiazolyl, Benzotriazolyl, Indazolyl, Indolyl, Isoindolyl, Indolizinyl oder Purinyl; oder eine 10-gliedrige Heteroarylgruppe wie zum Beispiel Chinolinyl, Chinazolinyl, Isochinolinyl, Cinnolinyl, Phthalazinyl, Chinoxalinyl oder Pteridinyl handeln.

Im Allgemeinen, und wenn nicht anders erwähnt, schließen die Heteroarylreste alle möglichen isomeren Formen davon ein, z. B. Tautomere und Positionsisomere in Bezug auf den Anbindungspunkt zum Rest des Moleküls. Somit schließt, als erläuterndes, nicht einschließendes Beispiel, der Begriff Pyridinyl Pyridin-2-yl, Pyridin-3-yl und Pyridin-4-yl ein; oder der Begriff Thienyl schließt Thien-2-yl und Thien-3-yl ein.

### C₅-C₁₀-Heteroaryl

C₅₋₁₀-Heteroaryl steht im Rahmen der Erfindung für ein mono-oder bicyclisches, aromatisches Ringsystem mit ein, zwei, drei oder vier Heteroatome, die gleich oder verschieden sein können. Als Heteroatome können vorkommen: N, O, S, S(=O) und/ oder S(=O)₂. Die Bindungsvalenz kann sich an einem beliebigen aromatischen Kohlenstoffatom oder an einem Stickstoffatom befinden.

Ein monocyclischer Heteroarylrest gemäß der vorliegenden Erfindung hat 5 oder 6 Ringatome. Bevorzugt sind solche Heteroarylreste mit ein oder zwei Heteroatomen. Besonders bevorzugt sind hierbei ein oder zwei Stickstoffatome.

Heteroarylreste mit 5 Ringatomen umfassen beispielsweise die Ringe:
Thienyl, Thiazolyl, Furyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

Heteroarylreste mit 6 Ringatomen umfassen beispielsweise die Ringe:
Pyridinyl, Pyridazinyl, Pyrimidinyl, Pyrazinyl und Triazinyl.

Ein bicyclischer Heteroarylrest gemäß der vorliegenden Erfindung hat 9 oder 10 Ringatome.

Heteroarylreste mit 9 Ringatomen umfassen beispielsweise die Ringe:
Phthalidyl, Thiophthalidyl, Indolyl, Isoindolyl, Indazolyl, Benzothiazolyl, Benzofuryl,

Benzothienyl, Benzimidazolyl, Benzoxazolyl, Azocinyl, Indolizinyl, Purinyl, Indolinyl.

Heteroarylreste mit 10 Ringatomen umfassen beispielsweise die Ringe:
Isochinolinyl, Chinolinyl, Chinolizinyl, Chinazolinyl, Chinoxalinyl, Cinnolinyl, Phthalazinyl, 1,7- und 1,8-Naphthyridinyl, Pteridinyl, Chromanyl.

### Heteroalkoxy

Heteroalkoxy steht für eine geradkettige und/oder verzweigte Kohlenwasserstoffkette mit 1 bis 10 Kohlenstoffatomen, die über -O- an den Rest des Moleküls gebunden ist und die weiterhin einfach oder mehrfach durch eine oder mehrere der Gruppen -O-, -S-, -C(=O)-, -S(=O)-, -S(=O)z-, -NRY-,
-NRYC(=O)-, -C(=O)-NRY-, -NRYNRY-, -S(=O)z-NRYNRY-, -C(=O)-NRYNRY-, -CR^{x}=N-O-unterbrochen sein kann, und wobei die Kohlenwasserstoffkette einschließlich der Seitenketten, sofern vorhanden, mit -NH-C(=O)-NH₂, -C(=O)-OH, -OH, -NH₂, -NH-C(=NNH₂)-, Sulfonamid, Sulfon, Sulfoxid, oder Sulfonsäure substituiert sein kann,

Hierbei steht R^{y} jeweils für -H, Phenyl-, C₁-C₁₀-Alkyl-, C₂-C₁₀-Alkenyl- oder C₂-C₁₀-Alkinyl-, die wiederum jeweils mit -NH-C(=O)-NH₂, -C(=O)-OH, -OH, -NH₂, -NH-C(=NNH₂)-, Sulfonamid, Sulfon, Sulfoxid, oder Sulfonsäure substituiert sein können.

Hierbei steht R^{x} für -H, C₁-C₃-Alkyl- oder Phenyl-.

Halogen beziehungsweise Halogenatom steht im Rahmen der Erfindung für Fluor (-F), Chlor (-Cl), Brom (-Br), oder Iod (-I).

Fluor-Alkyl, Fluor-Alkenyl und Fluor-Alkinyl bedeutet, dass das Alkyl, Alkenyl und Alkinyl ein oder mehrfach durch Fluor substituiert sein kann.

Die Kinesin Spindel Protein-Inhibitor- Prodrugs weisen vorzugsweise die folgende Formel (III) auf: 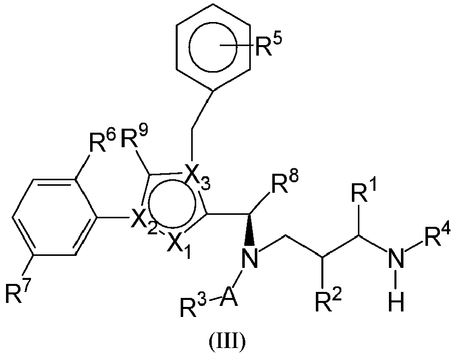 wobei
X₁ N, X₂ N und X₃ C darstellt; oder
X₁ N, X₂ C und X₃ N darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);
R¹ -H,-MOD oder -(CH₂)₀₋₃Z darstellt, wobei Z -H, -NHY³, -OY³, -SY³, Halogen, -CO-NY¹Y², oder -CO-OY³ darstellt,
   wobei Y¹ und Y² unabhängig voneinander -H, -NH₂, -(CH₂CH₂O)₀₋₃-(CH₂)₀₋₃Z'(z.B.-(CH₂)₀₋₃Z'), oder -CH(CH₂W)Z' darstellen, und Y³ -H oder -(CH₂)₀₋₃Z' darstellt, wobei Z' -H, -NH₂, -SO₃H, -COOH, -NH-CO-CH₂-CH₂-CH(NH₂)COOH oder -(CO-NH-CHY⁴)₁₋₃COOH darstellt; wobei W -H oder -OH darstellt,
   wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R² -H, -MOD, -CO-CHY⁴-NHY⁵ oder -(CH₂)₀₋₃Z darstellt,
   wobei Z -H, Halogen, -OY³, -SY³, NHY³, -CO-NY¹Y², oder -CO-OY³ darstellt,
   wobei Y¹ und Y² unabhängig voneinander -H, -NH₂, oder -(CH₂)₀₋₃Z' darstellen, und Y³ H oder -(CH₂)₀₋₃Z' darstellt, wobei Z' -H, -SO₃H, -NH₂ oder -COOH darstellt;
   wobei Y⁴ lineares oder verzweigtes, gegebenenfalls mit-NHC(=O)NHz substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ eine Gruppe der Formel R²¹-(CO)₍₀₋₁₎-(P3)₍₀₋₂₎-P2-NH-CH(CH₂CONH₂)-CO- oder R²¹-(CO)₍₀₋₁₎-(P3)₍₀₋₂₎-P2-NH-CH(CH₂COOH)-CO- oder die durch Cathepsin spaltbare Gruppe der Formel R²¹-(CO)₍₀₋₁₎-(P3)₍₁₋₂₎-P2- darstellt, ,
   wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, NH-CO-Alkyl, N(Alkyl)-COAlkyl, -SO₃H, -SO₂NH₂, - SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
   P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
A -C(=O)-, -S(=O)-, -S(=O)z-, -S(=O)₂NH- oder -C(=NNH₂)- darstellt;
R³ -MOD, oder eine gegebenenfalls substituierte Alkyl-, Cycloalkyl-, Aryl-, Heteroaryl-Heteroalkyl-, Heterocycloalkyl- Gruppe darstellt, eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 - SOz-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, n für 0, 1 oder 2 steht, Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' -H, -SO₃H, -NH₂ oder -COOH darstellt,
   substituiert sein kann (wobei "Alkyl" bevorzugt C₁₋₁₀-Alkyl bedeutet);
R⁵ -H, -NH₂, -NOz, Halogen (insbesondere F, Cl, Br), -CN, -CF₃, -OCF₃, -CH₂F, -CH₂F, - SH oder -(CH₂)₀₋₃Z darstellt,
   wobei Z -H, -OY³, -SY³, Halogen, -NHY³, -CO-NY¹Y², oder -CO-OY³ darstellt,
   wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;,
R⁶ und R⁷ unabhängig voneinander -H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, -NOz, -NH₂, -COOH oder Halogen (insbesondere -F, -Cl, -Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, -COzH oder -NH₂ substituiert sein kann;
R⁹ -H, -F, -CH₃, -CF₃, -CH₂F oder -CHF₂ darstellt;
wobei -MOD -(NR¹⁰)ₙ-(G1)ₒ-G2-G3 darstellt, wobei
   R¹⁰ -H oder C₁-C₃-Alkyl darstellt;
   G1 -NHC(=O)- , -C(=O)NH- oder 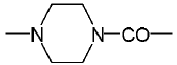 darstellt (wobei wenn G1 - NHCO- oder 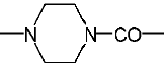 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SOz, -NRY-, -NRYCO-, -CONRY-, -NRYNRY-, - SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit -NHCONH₂, -COOH, -OH, - NH₂, -NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, und
   G3 H oder COOH darstellt;
   wobei die Gruppe -MOD vorzugsweise zumindest eine Gruppe -COOH aufweist;
sowie ihre Salze, Solvate und Salze der Solvate.

Durch Substitution eines H-Atoms an R¹, R², R³, R⁴, R⁵ oder R⁸ oder an dem durch R² und R⁴ gebildeten Pyrrolidinring (R¹⁰) kann in einer dem Durchschnittsfachmann bekannten Weise die Verbindung der Formel (III) mit einem Linker verbunden werden. Hierdurch werden Konjugate der Formel (IIa) erhalten, wobei einer der Substituenten R¹, R², R³, R⁴, R⁵, R⁸ oder R¹⁰ -L-#1 darstellt, L für den Linker steht und #1 für die Bindung zum Binder bzw. Derivat hiervon steht. Wenn der KSP-Inhibitor (bzw. KSP-L) nach Formel (IIa) mit einem Binder konjugiert ist, stellt einer der Substituenten R¹, R², R³, R⁴ , R⁵, R⁸ oder R¹⁰ also -L-#1 dar, wobei L für den Linker steht und #1 für die Bindung zum Binder bzw. Derivat hiervon steht. D.h. im Falle der Konjugate stellt einer der Substituenten R¹, R², R³, R⁴, R⁵ ,^{,}R⁸ und R¹⁰ -L-#1 dar, wobei -L-#1 an den Binder wie z.B. einen Antikörper darstellt. Besonders bevorzugt ist es, wenn einer der Substituenten R¹, R³ oder R⁴ -L-#1 darstellt. Der Binder ist vorzugsweise ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon oder ein anti-HER2-Antikörper. Besonders bevorzugt sind ein anti-TWEAKR-Antikörper, der spezifisch an die Aminosäure D in Position 47 (D47) von TWEAKR (SEQ ID NO: 169) bindet, insbesondere die anti-TWEAKR-Antikörper TPP-2090 und TPP-2658 oder die anti-EGFR-Antikörper Cetuximab oder Nimotuzumab oder der HER-2 Antikörper Trastuzumab

Anstelle von -L-#1 kann in der Formel IIa auch die Gruppe -L-#3 in der Verbindung vorhanden sein, wobei L für den Linker steht und #3 für die reaktive Gruppe zur Bindung an den Binder bzw. Derivat hiervon steht. Verbindungen mit -L-#3 sind reaktive Verbindungen, die mit dem Binder bzw. Derivat hiervon reagieren. #3 ist vorzugsweise eine Gruppe, die mit einer Amino- oder Thiolgruppe unter Bildung einer kovalenten Bindung reagiert, vorzugsweise mit dem Cysteinrest in einem Protein. Der Cysteinrest in einem Protein kann natürlich im Protein vorliegen, durch biochemische Methoden eingebracht werden oder vorzugsweise durch vorherige Reduktion von Disulfiden des Binders generiert werden kann.

Wenn R¹ nicht H darstellt, ist das Kohlenstoffatom, an das R¹ bindet, ein Stereozentrum, welches in der L- und/oder D-Konfiguration vorliegen kann, vorzugsweise in der L-Konfiguration.

Wenn R² nicht H darstellt, ist das Kohlenstoffatom, an das R² bindet, ein Stereozentrum, welches in der L- und/oder D-Konfiguration vorliegen kann.

Die Verbindungen der Formel (IIa), in der einer der Substituenten R¹, R³, und R⁴ -L-#1 darstellt, und in denen
X₁ N, X₂ N und X₃ C darstellt;
X₁ CH oder CF, X₂ C und X₃ N darstellt;
X₁ NH, X₂ C und X₃ C darstellt; oder
X₁ CH, X₂ N und X₃ C darstellt;
sind besonders bevorzugt,
insbesondere solche, in denen
X₁ N, X₂ N und X₃ C darstellt; oder X₁ CH, X₂ C und X₃ N darstellt. Insbesondere sind Verbindungen bevorzugt, in denen X₁ CH, X₂ C und X₃ N darstellt.

Als A ist CO (carbonyl) bevorzugt.

Als R¹ ist bevorzugt -L-#1, -MOD, -H, -COOH, -CONHNH₂, -(CH₂)₁₋₃NH₂, -CONZ"(CH₂)₁₋₃ NH₂ und -CONZ"CH₂COOH, wobei Z" -H oder -NH₂ darstellt. Wenn R⁴ -L-#1 darstellt, ist R¹ vorzugsweise -MOD (insbesondere, wenn R³ nicht -MOD darstellt).

Als R² ist bevorzugt -H.

Als R⁴ ist -H, -L-#1 oder die durch Legumain spaltbare Gruppe der Formel R²¹-(CO)₍₀₋₁₎-(P3)₍₀₋₂₎-P2-NH-CH(CH₂CONH₂)-CO- bevorzugt. Wie oben beschreiben weist hierbei -L-#1 die Gruppe der Formel -(CO)₍₀₋₁₎-(P3)₍₀₋₂₎-P2-NH-CH(CH₂COX)-CO*- auf, wobei die Carbonylgruppe des (L-)Asparagins bzw. der (L-)Asparaginsäure (mit * bezeichnet) direkt an dem Stickstoffatom bindet, das in obiger Formel an R⁴ bindet. Wenn R⁴ -L-#1 darstellt, ist R¹ oder R³ vorzugsweise -MOD.

Als R³ ist bevorzugt -L-#1. -MOD,oder eine C₁₋₁₀-Alkyl-, die ggf. mit -OH, -O-Alkyl, -SH, -S-Alkyl, -O-CO-Alkyl, -O-C(=O)-NH-Alkyl, NH-C(=O)-Alkyl, NH-C(=O)-NH-Alkyl, S(O)ₙ-Alkyl, SOz-NH-Alkyl, NH-Alkyl, N(Alkyl)₂, oder NH₂ substituiert sein kann, n für 0, 1 oder 2 steht, (wobei Alkyl vorzugsweise C₁₋₃ Alkyl bedeutet). Wenn R⁴ -L-#1 darstellt, ist R³ vorzugsweise - MOD (insbesondere, wenn R¹ nicht -MOD darstellt).

Als R⁵ ist bevorzugt -H oder -F.

Als R⁶ und R⁷ sind unabhängig voneinander bevorzugt -H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen.

Als R⁸ ist bevorzugt eine verzweigte C₁₋₅-Alkyl-Gruppe, insbesondere eine Gruppe der Formel - C(CH₃)₂-(CH₂)₀₋₂-R_{y}, wobei R_{y} -H, -OH, -CO₂H oder -NH₂. Besonders bevorzugt ist Gruppe der Formel -C(CH₃)₂-(CH₂)-R_{y}, wobei R_{y} -H ist.

Als R⁹ ist bevorzugt -H oder -F.

Als -MOD ist bevorzugt HOOC-(CHX)ₓ-AM-CH₂-CH₂-NH-CO-, wobei x eine Zahl von 2 bis 6 ist, X -H, -NH₂ oder -COOH darstellt, und AM -CO-NH- oder -NH-CO- darstellt, (besonders bevorzugt sind HOOC-CH₂-CH₂-CH(COOH)-NH-CO-CH₂-CH₂-NH-CO-; HOOC-CH(NH₂)-CH₂-CH₂-CO-NH-CH₂-CH₂-NH-CO-; HOOC-CH(NH₂)-(CH₂)₄-NH-CO-CH₂-CH₂-NH-CO-).

Insbesondere bevorzugt sind Verbindungen der Formel (IIa), in der einer der Substituenten R¹ und R³ -L-# 1 darstellt, und
in denen
X₁ N, X₂ N und X₃ C darstellt;
X₁ CH oder CF, X₂ C und X₃ N darstellt;
X₁ NH, X₂ C und X₃ C darstellt; oder
X₁ CH, X₂ N und X₃ C darstellt;
A -C=(O)- ist;
R¹ -H, -COOH, -CONHNH₂, -(CH₂)₁₋₃NH₂, -CONZ"(CH₂)₁₋₃ NH₂ und -CONZ"CH₂COOH ist, wobei Z" -H oder -NH₂;
R² -H darstellt;
R⁴ die durch Legumain spaltbare Gruppe der Formel R²¹-(CO)₍₀₋₁₎-(P3)₍₀₋₂₎-P2-NH-CH(CH₂CONH₂)-CO- darstellt;
R³ eine Phenylgruppe, die ein oder mehrfach mit Halogen (insbesondere -F) oder ggf. fluoriertes C₁₋₃ Alkyl substituiert sein kann, oder eine ggf. fluorierte C₁₋₁₀-Alkylgruppe darstellt, die ggf. mit -OY⁴, -SY⁴, -O-CO-Y⁴, -O-CO-NH-Y⁴, NH-CO-Y⁴, -NH-CO-NH-Y⁴, S(O)ₙ-Y⁴(wobei n 0, 1 oder 2 darstellt), -SO₂-NH-Y⁴, NH-Y⁴, oder N(Y⁴)₂, substituiert sein kann, wobei Y⁴ H, Phenyl (ggf. ein oder mehrfach mit Halogen (insbesondere F) oder ggf. fluoriertes C₁₋₃ Alkyl substituiert), oder Alkyl (wobei die Alkylgruppe mit -OH, -COOH, und/oder -NHCO-C₁₋₃-Alkyl substituiert sein kann, und wobei Alkyl vorzugsweise C₁₋₃ Alkyl bedeutet) darstellt;
wobei iinsbesondere bevorzugt R³mit -OH, -O-Alkyl, -SH, -S-Alkyl, -O-CO-Alkyl, -O-CO-NH-Alkyl, -NH-CO-Alkyl, -NH-CO-NH-Alkyl, -S(O)ₙ-Alkyl, -SO₂-NH-Alkyl, -NH-Alkyl, -N(Alkyl)₂, oder -NH₂ substituiert sein kann, n für 0, 1 oder 2 steht, (wobei Alkyl vorzugsweise C₁₋₃ Alkyl bedeutet)
R⁵ -H oder -F ist;
R⁶ und R⁷ unabhängig voneinander -H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ -H oder -F ist.
Zudem sind insbesondere bevorzugt Verbindungen der Formel (IIa), in der der Substituent R4-L-#1 darstellt, und
in denen
X₁ N, X₂ N und X₃ C darstellt;
X₁ CH oder CF, X₂ C und X₃ N darstellt;
X₁ NH, X₂ C und X₃ C darstellt; oder
X₁ CH, X₂ N und X₃ C darstellt;
A CO (carbonyl) ist;
R¹ -H, -COOH, -CONHNH₂, -(CH₂)₁₋₃NH₂, -CONZ"(CH₂)₁₋₃ NH₂, -CONZ"CH₂COOH, wobei Z" -H oder -NH₂ ist, oder HOOC-(CHX)ₓ-AM-CH₂-CH₂-NH-CO- darstellt, wobei x eine Zahl von 2 bis 6 ist, X -H, -NH₂ oder -COOH darstellt, und AM -CO-NH- oder -NH-CO- darstellt, (besonders bevorzugt sind HOOC-CH₂-CH₂-CH(COOH)-NH-CO-CH₂-CH₂-NH-CO-; HOOC-CH(NH₂)-CH₂-CH₂-CO-NH-CH₂-CH₂-NH-CO-; HOOC-CH(NH₂)-(CH₂)₄-NH-CO-CH₂-CH₂-NH-CO-).
R² -H darstellt;
R³ -(CH₂)OH, -CH(CH₃)OH, -CH₂SCH₂CH(COOH)NHCOCH₃, -CH(CH₃)OCH₃, eine Phenylgruppe, die mit 1-3 Halogenatomen, 1-3 Aminogruppen oder 1-3 Alkylgruppen (die ggf. halogeniert sein können), substituiert sein kann, oder HOOC-(CHX)ₓ-AM-CH₂-CH₂-NH-CO-darstellt, wobei x eine Zahl von 2 bis 6 ist, X H, NH₂ oder COOH darstellt, und AM -CO-NH- oder -NH-CO- darstellt, (besonders bevorzugt sind HOOC-CH₂-CH₂-CH(COOH)-NH-CO-CH₂-CH₂-NH-CO-; HOOC-CH(NH₂)-CH₂-CH₂-CO-NH-CH₂-CH₂-NH-CO-; HOOC-CH(NH₂)-(CH₂)₄-NH-CO-CH₂-CH₂-NH-CO-) oder -CH₂-Sₓ-(CH₂)₀₋₄-CHY⁵-COOH darstellt, wobei x 0 oder 1 ist, und Y⁵ -H oder -NHY⁶ darstellt, wobei Y⁶ -H oder -COCH₃ ist;
R⁵ -H oder -F ist;
R⁶ und R⁷ unabhängig voneinander -H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ -H oder -F ist.

Weiterhin sind bevorzugt (allein oder in Kombination), wenn
- R¹ -L-#1, -COOH, HOOC-CH₂-CH₂-CH(COOH)-NH-CO-CH₂-CH₂-NH-CO-; HOOC-CH(NH₂)-CH₂-CH₂-CO-NH-CH₂-CH₂-NH-CO-; HOOC-CH(NH₂)-(CH₂)₄-NH-CO-CH₂-CH₂-NH-CO- oder -H darstellt,
- R² -H darstellt,
- R⁴ die durch Legumain spaltbare Gruppe der Formel R²¹-(CO)₍₀₋₁₎-(P3)₍₀₋₂₎-P2-NH-CH(CH₂CONH₂)-CO- darstellt
- A -C(=O)- darstellt,
- R³ -(CH₂)OH, -CH(CH₃)OH, -CH₂SCH₂CH(COOH)NHCOCH₃, -CH(CH₃)OCH₃, eine Phenylgruppe, die mit 1-3 Halogenatomen, 1-3 Aminogruppen oder 1-3 Alkylgruppen (die ggf. halogeniert sein können, substituiert sein kann, HOOC-CH₂-CH₂-CH(COOH)-NH-CO-CH₂-CH₂-NH-CO-; HOOC-CH(NH₂)-CH₂-CH₂-CO-NH-CH₂-CH₂-NH-CO-; HOOC-CH(NH₂)-(CH₂)₄-NH-CO-CH₂-CH₂-NH-CO-, -CH₂-Sₓ-(CH₂)₀₋₄-CHY⁵-COOH auf, wobei x 0 oder 1 ist, und Y⁵ -H oder -NHY⁶ darstellt, wobei Y⁶ -H oder -COCH₃ darstellt, oder -L-# 1 darstellt,
- R⁵ -H darstellt,
- R⁶ und R⁷ unabhängig voneinander -H, C₁₋₃-Alkyl oder Halogen darstellen, insbesondere dass R⁶ und R⁷ -F darstellen;
- R⁸ C₁₋₄-Alkyl (vorzugsweise tert-butyl) darstellt; und/ oder
- R⁹ -H darstellt,
- wobei einer der Substituenten R¹ und R³ -L-# 1 darstellt.

Zudem ist es erfindungsgemäß bevorzugt, wenn
- R¹ -L-#1, COOH HOOC-CH₂-CH₂-CH(COOH)-NH-CO-CH₂-CH₂-NH-CO-; HOOC-CH(NH₂)-CH₂-CH₂-CO-NH-CH₂-CH₂-NH-CO-; HOOC-CH(NH₂)-(CH₂)₄-NH-CO-CH₂-CH₂-NH-CO- oder H darstellt,
- R² -H darstellt,
- R⁴ -H oder die durch Legumain spaltbare Gruppe der Formel R²¹-(CO)₍₀₋₁₎-(P3)_{( 0-2)}-P2-NH-CH(CH₂CONH₂)-CO- darstellt
- A -C(=O) darstellt,
- R³ -(CH₂)OH, -CH(CH₃)OH, -CH₂SCH₂CH(COOH)NHCOCH₃, -CH(CH₃)OCH₃, eine Phenylgruppe, die mit 1-3 Halogenatomen, 1-3 Aminogruppen oder 1-3 Alkylgruppen (die ggf. halogeniert sein können, substituiert sein kann, oder -L-# 1 darstellt,
- R⁵ -H darstellt,
- R⁶ und R⁷ unabhängig voneinander -H, C₁₋₃-Alkyl oder Halogen darstellen, insbesondere dass R⁶ und R⁷ -F darstellen;
- R⁸ C₁₋₄-Alkyl (vorzugsweise tert-butyl) darstellt; und
- R⁹ -H darstellt,
- wobei einer der Substituenten R¹ und R³,-L-#1 darstellt.

Weiterhin sind erfindungsgemäß bevorzugt folgende ADCs bzw. APDCs: 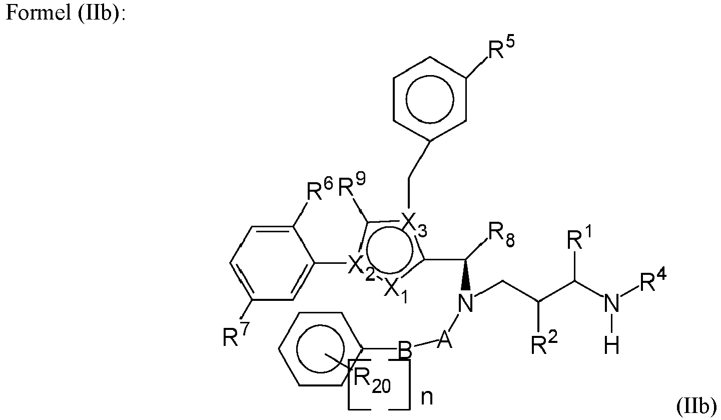 wobei X₁, X₂, X₃ die gleiche Bedeutung wie in Formel (IIa) aufweisen (vorzugsweise mit X₁ CH, X₂ C und X₃ N), R¹, R², R⁴, R⁵, R⁶, R⁷, R⁸ und R⁹ die gleiche Bedeutung wie in Formel (IIa) aufweisen, A -C(=O)- darstellt, B eine Einfachbindung, -O-CH₂- oder -CHz-O- darstellt, und R²⁰ - NH₂, -F, -CF3, oder -CH₃, und n 0, 1, oder 2 darstellt. 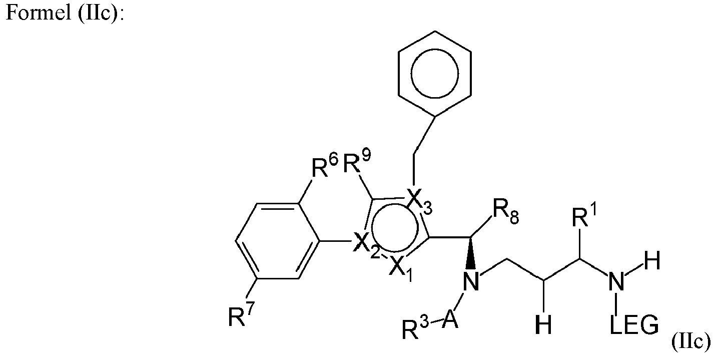 wobei X₁, X₂, X₃ die gleiche Bedeutung wie in Formel (IIIa) oder (III) aufweisen (vorzugsweise mit X₁ CH, X₂ C und X₃ N), A, R¹, R³, R⁶, R⁷, R⁸, und R⁹ die gleiche Bedeutung wie in Formel (IIa) aufweisen, wobei A vorzugsweise -C(=O)- und R³ -CHzOH, -CH₂OCH₃, -CH(CH3)OH oder - CH(CH₃)OCH₃ darstellt, und LEG die durch Legumain spaltbare Gruppe R²¹-(CO)₍₀₋₁₎-(P3)₍₀₋₂₎-P2-NH-CH(CH₂CONH₂)-CO- darstellt, wobei R²¹, P2 und P3 die gleiche Bedeutung wie in Formel (IIa) aufweisen. 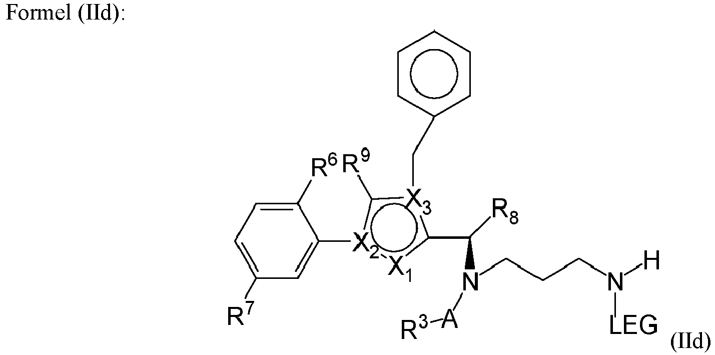 wobei X₁, X₂, X₃ die gleiche Bedeutung wie in Formel (IIa) aufweisen (vorzugsweise mit X₁ CH, Xz C und X₃ N), A, R³, R⁶, R⁷, R⁸ und R⁹ die gleiche Bedeutung wie in Formel (IIa) aufweisen, wobei A vorzugsweise -C(=O)- und R³ -CH₂-Sₓ-(CH₂)₀₋₄-CHY⁵-COOH ist, wobei x 0 oder 1 ist, und Y⁵ -H oder -NHY⁶ darstellt, wobei Y⁶ -H oder -COCH₃ darstellt, und LEG die durch Legumain spaltbare Gruppe R²¹-(CO)₍₀₋₁₎-(P3)_{( 0-2)}-P2-NH-CH(CH₂CONH₂)-CO- darstellt, wobei R²¹, P2 und P3 die gleiche Bedeutung wie in Formel (IIa) aufweisen. 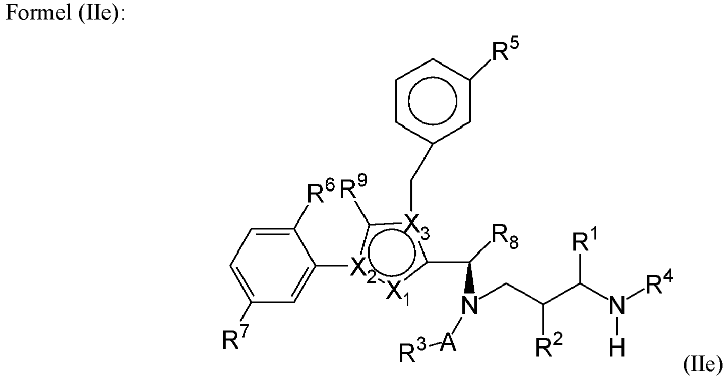 wobei X₁ CH, X₂ C und X₃ N, A, R³, R⁶, R⁷, R⁸ und R⁹ die gleiche Bedeutung wie in Formel (IIIa) oder (III) aufweisen, und R¹ -L-#, darstellt.

Weiterhin sind bevorzugt in den Verbindungen der Formeln (IIa), (IIb), (IIc), (IId) und (IIe) (allein oder in Kombination), wenn:
- Z -Cl oder -Br darstellt;
- R¹ -(CH₂)₀₋₃Z darstellt, wobei Z -CO-NY¹Y² darstellt, wobei Y² -(CH₂CH₂O)₀₋₃-(CH₂)₀₋₃Z' und Y¹ -H, -NH2, oder -(CH₂CH₂O)₀₋₃-(CH₂)₀₋₃Z'darstellt,
- Y¹ -H darstellt, Y² -(CH₂CH₂O)₃-CH₂CH₂Z' darstellt und Z' -COOH darstellt;
- Y¹ -H darstellt, Y² -CH₂CH₂Z' darstellt und Z` -(CONHCHY⁴)₂COOH stellt;
- Y¹ -H darstellt, Y² -CH₂CH₂Z' darstellt, Z' -(CONHCHY⁴)₂COOH stellt und eine Y⁴ *i-*propyl darstellt und der andere ein -(CH₂)₃-NHCONH₂ darstellt,
- Y¹ -H darstellt, Y² -CH₂CH₂Z' darstellt, Z' -(CONHCHY⁴)₂COOH stellt und eine Y⁴ -CH₃ darstellt und der andere ein -(CH₂)₃-NHCONH₂ darstellt,
- Y⁴ lineares oder verzweigtes, gegebenenfalls mit -NHCONH₂ substituiertes, C₁₋₆ Alkyl darstellt;
- mindestens ein Vertreter von Y⁴ ausgewählt wird aus i-propyl oder -CH₃.
- Y¹ -H darstellt, Y² -CH₂CH₂Z' darstellt, Z' -CONHCHY⁴COOH darstellt und Y⁴ gegebenenfalls mit -NH₂ substituiertes Aryl oder Benzyl darstellt;
- Y⁴ Aminobenzyl darstellt;
- R² -(CH₂)₀₋₃Z darstellt und Z -SY³ darstellt;
- R⁴ -CO-CHY⁴-NHY⁵ darstellt und Y⁵ -H darstellt;
- R⁴ -CO-CHY⁴-NHY⁵ darstellt und Y⁵-CO-CHY⁶-NH₂ darstellt;
- Y⁴ lineares oder verzweigtes, gegebenenfalls substituiertes mit -NHCONH₂ C₁₋₆ Alkyl darstellt.

Weiterhin ist es bevorzugt, wenn R¹, R² oder R³ in Formel (IIa) -MOD darstellt, insbesondere wenn R⁴ -L-#1 darstellt (insbesondere wenn -L ein spaltbarer Linker ist, der direkt an -N-R⁴ bzw. -N-L-#1 spaltet, so dass R⁴ bzw. L durch H ersetzt wird).

Besonders bevorzugt stellt R³ -MOD und R¹ oder R⁴-L-#1 bzw. -L-BINDER dar,
wobei -MOD -(NR¹⁰)ₙ-(G1)ₒ-G2-G3 darstellt, wobei
R¹⁰ -H oder C₁-C₃-Alkyl darstellt;
G1 -NHCO- , -CONH- oder 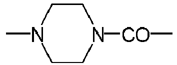 darstellt (wobei wenn G1 -NHCO- oder 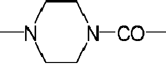 darstellt, R¹⁰ nicht -NH₂ ist);
n 0 oder 1 ist;
o 0 oder 1 ist; und
G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, -SOz, -NRY-, -NRYCO-, -CONRY-, -NRYNRY-, - SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit -NHCONH₂, -COOH, -OH, - NH₂, -NH-CNNH₂, Sulfonamid, Sulfon, Sulfoxid, oder Sulfonsäure substituiert sein können), -C(=O)-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein kann,
G3 -H oder -COOH darstellt, und
wobei die Gruppe -MOD vorzugsweise zumindest eine Gruppe -COOH aufweist.

Besonders bevorzugt weist die Gruppe -MOD eine (vorzugsweise terminale) -COOH-Gruppe auf, z.B. in einer Betaingruppe. Vorzugsweise weist die Gruppe -MOD die Formel -CH₂-Sₓ-(CH₂)₀₋₄-CHY⁵-COOH auf, wobei x 0 oder 1 ist, und Y⁵ -H oder -NHY⁶ darstellt, wobei Y6 -H oder - COCH₃ darstellt.

Weiterhin sind bevorzugt (allein oder in Kombination), wenn in Formel (IIa), (IIb), (IIc), (IId), oder (IIIe):
- Z -Cl oder -Br darstellt;
- R¹ -(CH₂)₀₋₃Z darstellt, wobei Z -CO-NY¹Y² darstellt, wobei Y² -(CH₂CH₂O)₀₋₃-(CH₂)₁₋₃Z' und Y¹ -H, -NH2, oder -(CH₂CH₂O)₀₋₃-(CH₂)₀₋₃Z'darstellt,
- Y¹ -H darstellt, Y² -(CH₂CH₂O)₃-CH₂CH₂Z' darstellt und Z` -COOH darstellt;
- Y¹ -H darstellt, Y² -CH₂CH₂Z' darstellt und Z' -(CONHCHY⁴)₂COOH stellt;
- Y¹ -H darstellt, Y² -CH₂CH₂Z' darstellt, Z' -(CONHCHY⁴)₂COOH stellt und ein Vertreter von Y⁴ i-propyl darstellt und der andere -(CH₂)₃-NHCONH₂ darstellt,
- Y¹ -H darstellt, Y² -CH₂CH₂Z' darstellt, Z' -(CONHCHY⁴)₂COOH stellt und ein Vertreter vonY⁴ -CH₃ darstellt und der andere -(CH₂)₃-NHCONH₂ darstellt,
- Y⁴ lineares oder verzweigtes, gegebenenfalls mit -NHCONH₂ substituiertes, C₁₋₆ Alkyl darstellt;
- mindestens ein Vertreter von Y⁴ ausgewählt wird aus i-propyl oder -CH₃.
- Y¹ -H darstellt, Y² -CH₂CH₂Z' darstellt, Z' -CONHCHY⁴COOH darstellt und Y⁴ gegebenenfalls mit -NH₂ substituiertes Aryl oder Benzyl darstellt;
- Y⁴ eine Aminobenzyl darstellt;
- R² -(CH₂)₀₋₃Z darstellt und Z -SY³ darstellt;
- R⁴ -CO-CHY⁴-NHY⁵ darstellt und Y⁵ -H darstellt;
- R⁴ -CO-CHY⁴-NHY⁵ darstellt und Y⁵-CO-CHY⁶-NH₂ darstellt;
- Y⁴ lineares oder verzweigtes gegebenenfalls substituiertes mit -NHCONH₂ C₁₋₆ Alkyl darstellt.

Weiterhin sind bevorzugt Verbindungen der Formel (IIa), (IIb), (IIc), (IId), oder (IIIe):,
wobei
X₁ N, X₂ N und X₃ C darstellt; oder
X₁ N, X₂ C und X₃ N darstellt; oder
X₁ CH oder CF, X₂ C und X₃ N darstellt; oder
X₁ NH, X₂ C und X₃ C darstellt; oder
X₁ CH oder CF, X₂ N und X₃ C darstellt;
(wobei X₁ CH, X₂ C und X₃ N bevorzugt ist);
R¹ -H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander -H, -NH₂, -(CH₂CH₂O)₀₋₃-(CH₂)₀₋₃Z'(z.B. - (CH₂)₀₋₃Z'), oder -CH(CH₂W)Z' darstellen, und Y³ H oder -(CH₂)₀₋₃Z' darstellt, wobei Z' - H, -NH₂, -SO₃H, -COOH, -NH-CO-CH₂-CH₂-CH(NH₂)COOH oder -(CO-NH-CHY⁴)₁₋₃COOH darstellt; wobei W -H oder -OH darstellt,
wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R² -H, -CO-CHY⁴-NHY⁵ oder -(CH₂)₀₋₃Z darstellt,
wobei Z -H, Halogen, -OY³, -SY³, -NHY³, -CO-NY¹Y², oder -CO-OY³ darstellt,
wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y^{5 -} H oder -CO-CHY⁶-NH₂ darstellt, wobei Y⁶ lineares oder verzweigtes C₁₋₆ Alkyl darstellt;
R⁴ -H oder die durch Legumain spaltbare Gruppe R²¹-(CO)₍₀₋₁₎-(P3)_{( 0-2)}-P2-NH-CH(CH₂CONH₂)-CO- darstellt;
A -C(=O)-, S(=O)-, S(=O)₂, S(=O)₂NH oder C(=NNH₂)- darstellt;
R³ -L-#1, -MOD oder eine gegebenenfalls substituierte Alkyl-, Cycloalkyl-, Aryl-, Heteroaryl-, Heteroalkyl-, Heterocycloalkyl- Gruppe darstellt, bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SOz-NH-Alkyl Gruppen, 1-3 -NH-Alkyl Gruppen, 1-3 - N(Alkyl)₂ Gruppen, 1-3 -NH((CH₂CH₂O)₁₋₂₀H)-Gruppen, 1-3 -NH₂ Gruppen oder 1-3 - (CH₂)₀₋₃Z Gruppen, wobei n für 0, 1 oder 2 steht, Z -H, Halogen, -OY³, -SY³, -NHY³, - CO-NY¹Y², oder -CO-OY³ darstellt, mit Y¹ und Y² unabhängig voneinander -H, -NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' -H, -SO₃H, -NH₂ oder -COOH darstellt, substituiert sein kann (wobei "Alkyl" bevorzugt C₁₋₁₀-Alkyl bedeutet);
R⁵ -H, -MOD, -NH₂, -NOz, Halogen (insbesondere -F, -Cl, -Br), -CN, -CF₃, -OCF₃, -CH₂F, -CH₂F, -SH oder -(CH₂)₀₋₃Z darstellt, wobei Z -H, -OY³, -SY³, Halogen, -NHY³, -CO-NY¹Y², oder -CO-OY³ darstellt,
wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
R⁶ und R⁷ unabhängig voneinander -H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, -NOz, -NH₂, -COOH oder Halogen (insbesondere -F, -Cl, -Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, oder (ggf. fluoriertes) C₄₋₁₀-Cycloalkyl;
wobei einer oder keiner der Substituenten R¹ und R³-L-#1 darstellt,
L für den Linker steht und #1 für die Bindung zum Binder bzw. Derivat hiervon steht,
R⁹ -H, -F, -CH₃, -CF₃, -CH₂F oder -CHF₂ darstellt;
wobei -MOD -(NR¹⁰)ₙ-(G1)ₒ-G2-G3 darstellt, wobei
   R¹⁰ -H oder C₁-C₃-Alkyl darstellt;
   G1 -NHCO- , -CONH- oder 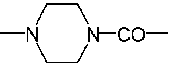 darstellt wobei wenn G1 -NHCO- oder 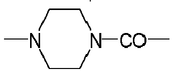 darstellt, R¹⁰ nicht -NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, -SOz-, -NRY-, -NRYCO-, -CONRY-, -NRYNRY-, - SO2NR^{y}NR^{y}-, -CONRYNRY-, (wobei R^{y} -H, Phenyl, C₁-C₁₀-Alkyl, C₂-C₁₀-Alkenyl, oder C₂-C₁₀-Alkinyl darstellt, die jeweils mit -NHCONH₂, -COOH, -OH,-NH₂, -NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein kann,
   G3 -H oder -COOH darstellt,
   wobei die Gruppe -MOD vorzugsweise zumindest eine Gruppe -COOH aufweist;
sowie ihre Salze, Solvate und Salze der Solvate.

Weiterhin sind bevorzugt Verbindungen der Formel (IIa), (IIb), (IIc), (IId), oder (IIIe), in denen
X₁ N, X₂ N und X₃ C darstellt; oder
X₁ N, X₂ C und X₃ N darstellt; oder
X₁ CH oder CF, X₂ C und X₃ N darstellt; oder
X₁ NH, X₂ C und X₃ C darstellt; oder
X₁ CH oder CF, X₂ N und X₃ C darstellt;
(wobei X₁ CH, X₂ C und X₃ N bevorzugt ist);
R¹ -H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander -H, -NH₂, -(CH₂CH₂O)₀₋₃-(CH₂)₀₋₃Z'(z.B. - (CH₂)₀₋₃Z'), oder -CH(CH₂W)Z' darstellen, und Y³ -H oder -(CH₂)₀₋₃Z' darstellt, wobei Z' - H, -NH₂, -SO₃H, -COOH, -NH-CO-CH₂-CH₂-CH(NH₂)COOH oder -(CO-NH-CHY⁴)₁₋₃COOH darstellt; wobei W -H oder -OH darstellt,
wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R² -H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt, wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
wobei Y⁴ lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y^{5 -} H oder -CO-CHY⁶-NH₂ darstellt, wobei Y⁶ lineares oder verzweigtes C₁₋₆ Alkyl darstellt;
R⁴ -H oder die durch Legumain spaltbare Gruppe R²¹-(CO)₍₀₋₁₎-(P3)_{( 0-2)}-P2-NH-CH(CH₂CONH₂)-CO- darstellt,
A -C(=O), S(=O), S(=O)₂, S(=O)₂NH oder C(=NNH₂)- darstellt;
R³ -L-#1, -MOD oder eine gegebenenfalls substituierte Alkyl-, Cycloalkyl-, Aryl-, Heteroaryl-, Heteroalkyl-, Heterocycloalkyl- Gruppe darstellt, bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 -O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SOz-NH-Alkyl Gruppen, 1-3 -NH-Alkyl Gruppen, 1-3 - N(Alkyl)₂ Gruppen, 1-3 -NH((CH₂CH₂O)₁₋₂₀H)-Gruppen, 1-3 -NH₂ Gruppen oder 1-3 - (CH₂)₀₋₃Z Gruppen, wobei n für 0, 1 oder 2 steht, Z -H, Halogen, -OY³, -SY³, -NHY³, -CO-NY¹Y², oder -CO-OY³ darstellt, mit Y¹ und Y² unabhängig voneinander H, -NH₂, oder - (CH₂)₀₋₃Z' darstellen, und Y³ H, -(CH₂)₀₋₃-CH(NHCOCH₃)Z',-(CH₂)₀₋₃-CH(NH₂)Z', oder - (CH₂)₀₋₃Z' darstellt, wobei Z' -H, -SO₃H, -NH₂ oder -COOH darstellt, substituiert sein kann (wobei "Alkyl" bevorzugt C₁₋₁₀-Alkyl bedeutet);
R⁵ -H, -MOD, -NH₂, -NOz, Halogen (insbesondere -F, -Cl, -Br), -CN, CF₃, -OCF₃, -CH₂F, - CH₂F, -SH oder -(CH₂)₀₋₃Z darstellt, wobei Z -H, -OY³, -SY³, Halogen, -NHY³, -CO-NY¹Y², oder -CO-OY³ darstellt,
wobei Y¹ und Y² unabhängig voneinander -H, -NH₂, oder -(CH₂)₀₋₃Z' darstellen, und Y³ -H oder -(CH₂)₀₋₃Z' darstellt, wobei Z' -H, -SO₃H, -NH₂ oder -COOH darstellt;
R⁶ und R⁷ unabhängig voneinander -H oder Halogen (insbesondere -F, -Cl, -Br) darstellen,
R⁸ (ggf. fluoriertes) C₁₋₁₀-Alkyl darstellt;
wobei einer oder keiner der Substituenten R¹ und R³-L-#1 darstellt,
L für den Linker steht und #1 für die Bindung zum Binder bzw. Derivat hiervon steht,
R⁹ -H, -F, -CH₃, -CF₃, -CH₂F oder -CHF₂ darstellt;
wobei -MOD -CH₂-Sₓ-(CH₂)₀₋₄-CHY⁵-COOH auf, wobei x 0 oder 1 ist, und Y⁵ -H oder - NHY⁶ darstellt, wobei Y6 -H oder -COCH₃ darstellt,
sowie ihre Salze, Solvate, Salze der Solvate und Epimere.

Erfindungsgemäß besonders bevorzugt sind die folgenden Verbindungen der Formeln V, VI oder VII, wobei R¹, R², R³, R⁴ und R⁵ die oben genannten Bedeutungen (wie z.B. für Formel (IIa) angegeben) haben:

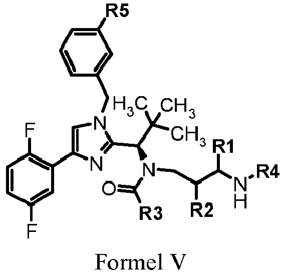

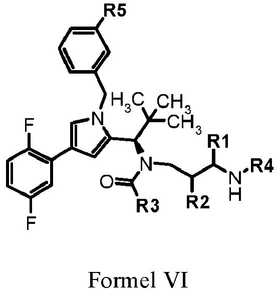

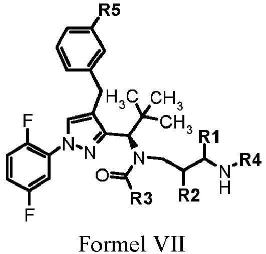

Besonders bevorzugt sind die Verbindungen der Formeln V, VI, VII, wobei R¹ und R⁵ H oder -L-#1 darstellen; R² H darstellt; R⁴ die Gruppe der Formel R²¹-(CO)₍₀₋₁₎-(P3)₍₀₋₂₎-P2-NH-CH(CH₂CONH₂)-CO- oder die durch Cathepsin spaltbare Gruppe der Formel R²¹-(CO)₍₀₋₁₎-(P3)₍₀₋₂₎-P2- darstellt, wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist; P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
wobei einer der Substituenten R¹ und R³-L-#1 darstellt. Insbesondere bevorzugt sind entsprechende Verbindungen der Formel VI.

Die erfindungsgemäßen Antikörper-Drug-Konjugate (ADCs) weisen vorzugsweise die folgende Formel VIII auf: 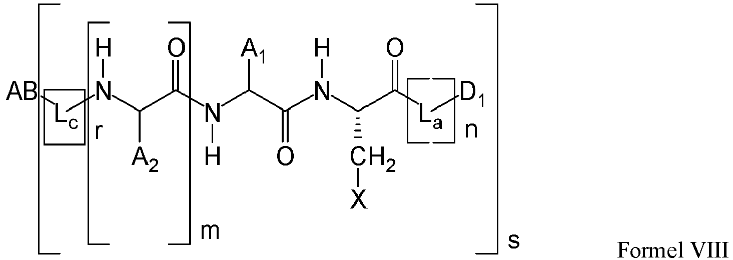 wobei
m eine Zahl von 0 bis 2 ist;
n 0 oder 1 ist;
X -CONH₂ oder -COOH ist;
Lₐ einen *self-immolative linker* darstellt;
L_{c} einen Linker darstellt;
A₁ ein Rest ist, der sich von einer der Aminosäuren Gly, Pro, Ala, Val, Nva, Leu, Ile, Met, Phe, Tyr, Trp, Ser, Thr, Cys, Asn, Gln, Asp, Glu, Lys, Arg, Citrullin und His ableitet;
A₂ ein Rest ist, der sich von einer der Aminosäuren Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ableitet (wenn mehr als ein P3 vorliegt, kann P3 also unterschiedliche Bedeutungen aufweisen)
D1 eine Verbindung der Formel III ist;
R Z₁-(CO)q- darstellt, wobei q 0 oder 1 ist und Z₁ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroarylalkyl-, C₅₋₁₀-Heteroaryl-alkoxy-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein- oder mehrfach mit - NH₂, -NH-Alkyl, -N(Alkyl)₂, -NH-CO-Alkyl, -N(Alkyl)-COAlkyl, -SO₃H, - SO₂NH₂, -SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R¹ darstellt, (wobei x 0 oder 1 und v eine Zahl von 1 bis 20 darstellt, und R¹ -H, -Alkyl (vorzugsweise C1-12-Alkyl), -CH2-COOH, -CH2-CH2-COOH, oder -CH2-CH2-NH2 darstellt), und AB einen Antikörper darstellt, und s eine Zahl von 1 bis 20, vorzugsgweise 2 bis 8, besonders bevorzugt 3 bis 5 wie z.B. 4 darstellt.

Die erfindungsgemäßen Antikörper-Prodrug-Konjugate (APDCs) weisen vorzugsweise die folgende Formel IX auf: 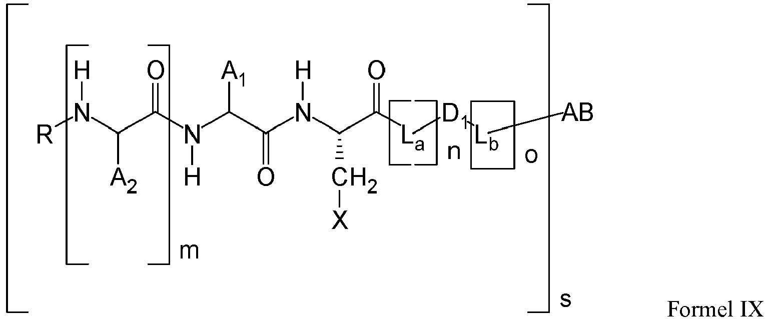 wobei
m eine Zahl von 0 bis 2 ist;
n 0 oder 1 ist;
o 0 oder 1 ist;
X -CONH₂ oder -COOH ist;
Lₐ einen *self-immolative linker* darstellt;
L_{b} einen Linker darstellt;
A₁ ein Rest ist, der sich von einer der Aminosäuren Gly, Pro, Ala, Val, Nva, Leu, Ile, Met, Phe, Tyr, Trp, Ser, Thr, Cys, Asn, Gln, Asp, Glu, Lys, Arg, Citrullin und His ableitet;
A₂ ein Rest ist, der sich von einer der Aminosäuren Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ableitet (wenn mehr als ein P3 vorliegt, kann P3 also unterschiedliche Bedeutungen aufweisen);
D1 eine Verbindung der Formel III ist;
R Z₁-(CO)q- darstellt, wobei q 0 oder 1 ist und Z₁ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroarylalkyl-, C₅₋₁₀-Heteroaryl-alkoxy-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein- oder mehrfach mit -NH₂, -NH-Alkyl, -N(Alkyl)₂, -NH-CO-Alkyl, -N(Alkyl)-COAlkyl, -SO₃H, - SO₂NH₂, -SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R¹ darstellt, (wobei x 0 oder 1 und v eine Zahl von 1 bis 20 darstellt, und R¹ -H, -Alkyl (vorzugsweise C1-12-Alkyl), -CH2-COOH, -CH2-CH2-COOH, oder -CH2-CH2-NH2 darstellt), und AB einen Antikörper darstellt, und s eine Zahl von 1 bis 20, vorzugsgweise 2 bis 8, besonders bevorzugt 3 bis 5 wie z.B. 4 darstellt.

### Linker

Aus der Literatur sind verschiedene Möglichkeiten der kovalenten Kopplung (Konjugation) von organischen Molekülen an Binder wie z.B. Antikörper bekannt (siehe z.B. K. Lang and J. W. Chin. Chem. Rev. 2014, 114, 4764-4806, M. Rashidian et al. Bioconjugate Chem. 2013, 24, 1277-1294). Erfindungsgemäß bevorzugt ist die Konjugation der KSP-Inhibitoren bzw. Prodrug an einen Antikörper über ein oder mehrere Schwefelatome von Cystein-Resten des Antikörpers, die entweder als freie Thiole bereits vorliegen oder durch Reduktion von Disulfidbrücken generiert werden, und/oder über eine oder mehrere NH-Gruppen von Lysin-Resten des Antikörpers. Es ist jedoch auch möglich, den KSP-Inhibitor bzw. Prodrug an den Antikörper über Tyrosinreste, über Glutaminreste, über Reste unnatürlicher Aminosäuren, über freie Carboxylgruppen oder über Zuckerreste des Antikörpers zu binden.

Es ist erfindungsgemäß auch möglich, die Wirkstoffmoleküle an spezifische Konjugationsstellen des Binders zu konjugieren, wodurch die Produkthomogenität verbessert wird. Die Literatur beschreibt verschiedene Methoden zur konjugationsstellenspezifischen Konjugation (Agarwal et al., Bioconjug. Chem. 26, 176-192 (2015); Cal et al., Angew. Chem. Int. Ed. Engl.53, 10585-10587 (2014); Behrens et al., MAbs 6, 46-53 (2014); Panowski et al., MAbs 6, 34-45 (2014)). Diese Methoden enthalten insbesondere auch enzymatische Konjugationsmethoden, die beispielsweise Transglutaminasen (TGases), Glykosyltransferasen oder das Formylglycin generierende Enzym ((Sochaj et al., Biotechnology Advances 33, 775-784, (2015)) verwenden.

Erfindungsgemäß können konjugationsstellenspezifische Binder-Konjugate des Kinesin-Spindel-Protein-Inhibitors, in welchen die Kinesin-Spindel-Protein-Inhibitoren an Glutamin-Seitenketten der Binder konjugiert sind, bereitgestellt werden.

Wenn der Binder ein Antikörper ist, beinhaltet er ein Akzeptor-Glutamin, bevorzugt in der konstanten Region. Solche Akzeptor-Glutamine können durch Mutation von geeigneten Positionen zu Glutamin eingeführt werden (zum Beispiel die Mutation N297Q der schweren Kette, Kabat EU Nummerierung) oder durch Generierung von deglycosylierten oder aglycosylierten Antikörpern (zum Beispiel durch enzymatische Deglykosylierung durch PNGase F oder durch Mutation N297X der schweren Kette, Kabat EU Nummerierung (X kann hier jede Aminosäure außer N sein)). Im letzteren Fall eines deglykosylierten oder aglykosylierten Antikörpers wird der Glutaminrest Q295 (Kabat EU Nummerierung) der schweren Kette ein Akzeptor-Glutamin. Besonders bevorzugt ist ein Antikörper, der die Mutation N297A oder N297Q (Kabat EU Nummerierung) enthält. Daher beinhalten alle in dieser Erfindung beschriebenen Antikörper ebenso aglykosylierte Varianten dieser Antikörper, die entweder durch Deglykosylierung durch PNGase F oder durch Mutation von N297 (Kabat EU Nummerierung) (Kabat numbering system of antibodies, see Kabat et al., Sequences of Proteins of Immulological Interest, 5th Ed. Public Health Service, National Institutes of Health, Bethesda, MD. (1991)) der schweren Kette zu jeder anderen Aminosäure außer N hergestellt werden. Desweiteren enthalten alle hier beschriebenen Antikörper ebenso Varianten der beschriebenen Antikörper, die durch ein Engineering ein oder mehrere Akzeptor-Glutamin-Reste für Transglutaminase-katalysierte Reaktionen enthalten.

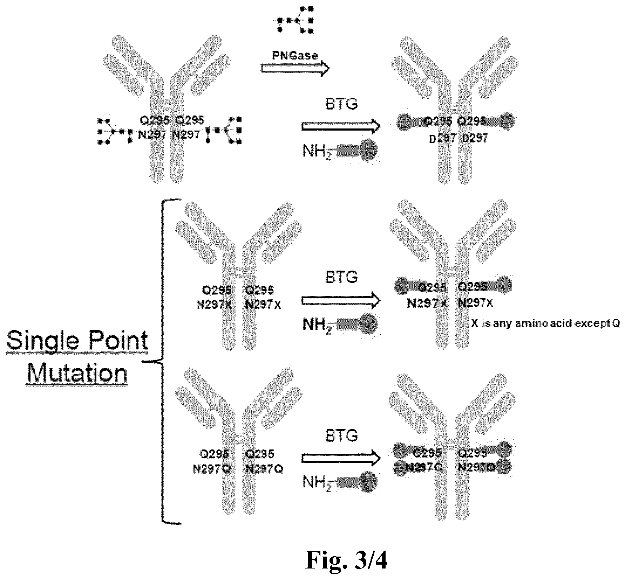

Ein Weg für solche konjugationsstellenspezifischen Konjugationen sind literaturbeschriebene Ansätze, die sich mit konjugationsstellenspezifischer Konjugation von Bindern mittels Transglutaminase befassen. Transglutaminasen (TGases), die auch die bakterielle Transglutaminase (BTG) (EC 2.3.2.13) beinhalten, sind eine Familie von Enzymen, die die Bildung einer kovalenten Bindung zwischen der γ-Carbonyl-Amid-Gruppe von Glutaminen und der primären Amin-Gruppe von Lysinen katalysieren. Da solche Transglutaminasen auch andere Substrate als Lysin als Amin-Donor akzeptieren, wurden sie verwendet, um Proteine einschließlich Antikörper an geeigneten Akzeptor-Glutaminen zu modifizieren (Jeger et al., Angewandte Chemie Int. Ed. Engl 49, 9995-9997 (2010); Josten et al., J. Immunol. Methods 240, 47-54 (2000); Mindt et al., Bioconjugate Chem. 19, 271-278 (2008); Dennler et al., in Antibody Drug Conjuagtes (Ducry, L., Ed.), pp 205-215, Humana Press. (2013)). Auf der einen Seite wurden Transglutaminasen für die Konjugation von Wirkstoffen an Antikörper verwendet, welche artifizielle Glutamin-Tags enthalten, welche Akzeptorglutamin-Reste sind, welche durch genetisches Engineering in den Antikörper eingefügt wurden (Strop et al., Chem. Biol. 20, 161-167 (2013)). Auf der anderen Seite wurde beschrieben, dass der konservierte Glutamin-Rest Q295 (Kabat EU Nummerierung) der konstanten Region der schweren Kette von Antikörpern der einzige γ-Carbonyl-Amid-Donor für die bakterielle Transglutaminase (EC 2.3.2.13) im Rückgrat von aglykosylierten IgG1-Molekülen ist, und somit ein Akzeptor-Glutamin darstellt, während kein Akzeptor-Glutamin im Rückgrat des IgG1 vorhanden ist, wenn der Antikörper an Position N297 (Kabat EU Nummerierung) der schweren Kette glykosyliert ist (Jeger et al., Angewandte Chemie Int. Ed. Engl 49, 9995-9997 (2010)). Zusammenfassend kann die bakterielle Transglutaminase für die Konjugation eines Amin-Donor-Substrats, zum Beispiel eines Wirkstoff-Linker-Konstrukts, an einen Akzeptor-Glutamin-Rest eines Antikörpers verwendet werden. Solche Akzeptor-Glutamine können durch Engineering des Antikörpers durch Mutationen oder durch die Generierung aglykosylierter Antikörper eingeführt werden. Solche aglykosylierten Antikörper können durch Deglykosylierung unter Verwendung von N-glycosidase F (PNGase F) oder durch Mutation von N297 der Glykosylierungsstelle der schweren Kette (Kabat EU Nummerierung) zu jeder anderen Aminosäure außer N eingeführt werden. Die enzymatische Konjugation solcher aglykosylierter Antikörper unter Verwendung von bakterieller Transglutaminase wurde für aglykosylierte Antikörpervarianten beschrieben, die die Mutationen N297D, N297Q (Jeger et al., Angewandte Chemie Int. Ed. Engl 49, 9995-9997 (2010)) oder N297S (siehe Patentanmeldungen WO2013092998A1 und WO2013092983A2) enthalten. Die enzymatische Konjugation solcher aglykosylierter Antikörperm mittels Transglutaminase liefert generell ADCs mit einer DAR von 2, in welchen beide schweren Ketten spezifisch an Position Q295 (Kabat EU Nummerierung) funktionalisiert sind. Nur die Mutation N297Q der schweren Kette liefert eine zusätzliche Konjugationsstelle pro schwerer Kette. Die Konjugation solcher Varianten führt zu ADCs mit einer DAR von 4, in welchen beide schweren Ketten spezifisch an den Positionen Q295 und Q297 funktionalisiert sind. Antikörpervarianten in welchen die schweren Ketten die Mutationen Q295N und N297Q tragen, weisen pro schwerer Kette nur einen Akzeptor-Glutamin-Rest an Position Q297 (Kabat Nummerierung) auf (Simone Jeger, Site specific conjugation of tumour targeting antibodies using transglutaminase, Dissertation at ETH Zurich (2009)). In der Literatur existieren mehrere Beispiele, die die konjugationsstellenspezifische Konjugation von aglykosylierten Antikörpern unter Verwendung von bakterieller Transglutaminase beschreiben (zum Beispiel Dennler et al., Bioconjugate Chemistry 19, 569-578 (2014); Lhospice et al., Molecular Pharmaceutics 12, 1863-1871 (2015)). Die Strategie der Transglutaminase-katalysierten konjugationsstellenspezifischen Funktionalsisierung aglykosylierter Antikörper ist Figur 3 zusammengefasst.

Zur Kopplung - sowohl konjugationsstellenspezifisch als auch konjugationsstellenunspezifisch - werden sogenannte Linker verwendet. Linker lassen sich in die Gruppe der *in vivo* spaltbaren Linker und in die Gruppe der *in vivo* stabilen Linker unterteilen (siehe L. Ducry und B. Stump, Bioconjugate Chem. 21, 5-13 (2010)). Die *in vivo* spaltbaren Linker weisen eine *in vivo* spaltbare Gruppe auf, wobei wiederum zwischen chemisch *in vivo* spaltbaren und enzymatisch *in vivo* spaltbaren Gruppen unterschieden werden kann. "Chemisch *in vivo* spaltbar" bzw. "enzymatisch *in vivo* spaltbar" bedeutet, dass die Linker bzw. Gruppen im Blutkreislauf stabil sind und erst an bzw. in der Zielzelle durch die dort veränderte chemische bzw. enzymatische Umgebung (niedrigerer pH-Wert; erhöhte Glutathion-Konzentration; Vorliegen lysosomaler Enzyme wie Legumain, Cathepsin oder Plasmin, oder Glyosidasen wie beispielsweise β-Glucuronidasen) sich spalten, um so den niedermolekularen KSP-Inhibitor oder ein Derivat davon freizusetzen. Die chemisch *in vivo* spaltbaren Gruppen sind insbesondere Disulfid, Hydrazon, Acetal und Aminal; die enzymatisch *in vivo* spaltbaren Gruppen sind insbesondere 2-8-Oligopeptidgruppe, insbesondere eine Dipeptidgruppe oder Glycoside. Peptidspaltstellen sind in Bioconjugate Chem. 2002, 13, 855-869, sowie Bioorganic & Medicinal Chemistry Letters 8 (1998) 3341-3346 *sowie* Bioconjugate Chem. 1998, 9, 618-626 offenbart. Hierzu gehören z.B. Alanin-Alanin-Asparagin, Valin-Alanin, Valin-Lysin, Valin-Citrullin, Alanin-Lysin und Phenylalanin-Lysin (ggf. mit zusätzlicher Amidgruppe).

Um eine effiziente Freisetzung des freien Wirkstoffs zu gewährleisten, können gegebenenfalls auch sogenannte self-immolative Linkerelemente (SIG z.B. in obiger Formel IIa bzw. La in den obigen Formeln VIII und IX) zwischen enzymatischer Spaltstelle und Wirkstoff eingebaut werden (Anticancer Agents in Medicinal Chemistry, 2008, 8, 618-637). Die Freisetzung des Wirkstoffs kann dabei nach verschiedenen Mechanismen erfolgen, beispielsweise nach initialer enzymatischer Freisetzung einer nukleophilen Gruppe durch anschließende Elimierung über eine elektronische Kaskade (Bioorg. Med. Chem., 1999, 7, 1597; J. Med. Chem., 2002, 45, 937; Bioorg. Med. Chem., 2002, 10, 71) oder durch Cyklisierung des entsprechenden Linkerelements (Bioorg. Med. Chem., 2003, 11, 2277; Bioorg. Med. Chem., 2007, 15, 4973; Bioorg. Med. Chem. Lett., 2007, 17, 2241) oder durch eine Kombination aus beidem (Angew. Chem. Inter. Ed., 2005, 44, 4378). Beispiele für solche Linkerelemente sind in der Abbildung dargestellt:

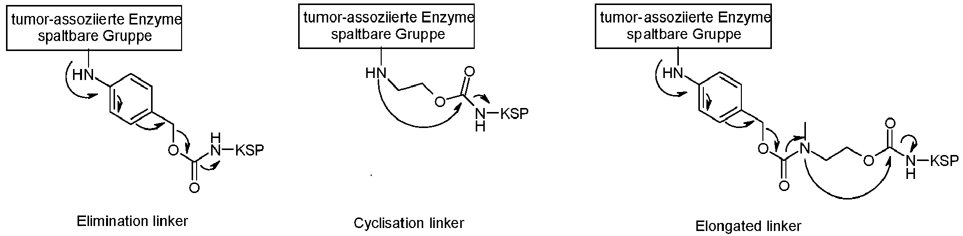

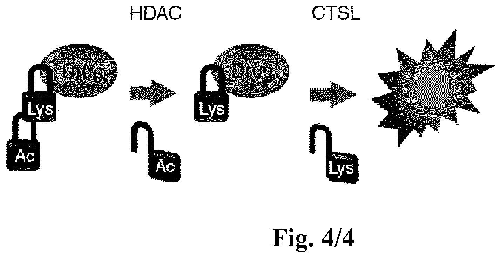

Beispiele für nacheinander geschaltete enzymatische Schritte zur Wirkstofffreisetzung z.B. durch Histone Deacetylase und Cathepsin L sind in Nat. Commun., 2013, 4, 2735 beschrieben (vgl. Figur 4).

Die *in vivo* stabilen Linker zeichnen sich durch eine hohe Stabilität aus (weniger als 5 % Metabolite nach 24 Stunden in Plasma) und weisen die oben genannten chemisch oder enzymatisch *in vivo* spaltbaren Gruppen nicht auf.

Der Linker -L- (wie auch Lc in Formel VIII und Lb in Formel IX) weist vorzugsweise eine der folgenden Grundstrukturen (i) bis (iv) auf:
(i) -(C=O)ₘ-SG1-L1-L2-
(ii) -(C=O)ₘ -L1-SG-L1-L2-
(iii) -(C=O)ₘ -L1-L2-
(iv) -(C=O)ₘ -L1-SG-L2
wobei m 0 oder 1 ist; SG eine (chemisch oder enzymatisch) *in vivo* spaltbare Gruppe (insbesondere Disulfid, Hydrazon, Acetal und Aminal; oder eine mit Legumain, Cathepsin oder Plasmin spaltbare 2-8-Oligopeptidgruppe) ist, SG1 eine Oligopeptidgruppe oder vorzugsweise eine Dipeptidgruppe ist, L1 für *in vivo* stabile organische Gruppen stehen, und L2 für eine Kopplungsgruppe an den Binder oder eine Einfachbindung steht. Die Kopplung erfolgt hierbei vorzugsweise an einen Cysteinrest oder einen Lysinrest des Antikörpers. Alternativ kann die Kopplung an einen Tyrosinrest, Glutaminrest oder an eine unnatürliche Aminosäure des Antikörpers erfolgen. Die unnatürlichen Aminosäuren können beispielsweise Aldehyd- oder Ketogruppen (wie z.B. Formylglycin) oder Azid- oder Alkingruppen enthalten (siehe hierzu Lan & Chin, Cellular Incorporation of Unnatural Amino Acids and Bioorthogonal Labeling of Proteins, Chem.Rev. 2014, 114, 4764-4806).

Erfindungsgemäß bevorzugt ist insbesondere die Linker-Grundstruktur (iii). Die Verabreichung eines erfindungsgemäßen Konjugats mit einer Linker-Grundstruktur (iii) und Kopplung des Linkers an einen Cystein- oder Lysinrest des Antikörpers führt durch Metabolisierung zu Cystein- bzw. Lysinderivaten der folgenden Formeln: 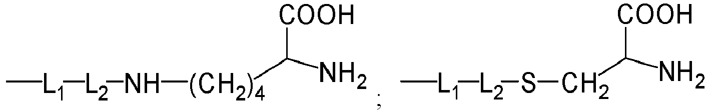 wobei L1 jeweils an den niedermolekularen KSP-Inhibitor gebunden ist, z.B. einer Verbindung der Formel (III) bzw. (IIa), (IIb), (IIc), (IId), (IIe), (IIf), oder (IV), wobei -L-#1 eine der beiden obigen Reste darstellt, die sich von Lysin bzw. Cystein ableiten.

Erfindungsgemäß bevorzugt sind auch die Linker-Grundstrukturen (ii) and (iv), insbesondere bei Anbindung an die Position R¹, insbesondere wenn die Gruppe L1 eine der folgenden Strukturen aufweist:
(a) -NH-(CH₂)₀₋₄₋(CHCH₃)₀₋₄-CHY⁵-CO-Y⁷, wobei Y⁵ -H oder -NHY⁶ darstellt, wobei Y⁶ -H oder -COCH₃ darstellt, und Y⁷ eine Einfachbindung oder -NH -(CH₂)₀₋₄ -CHNH₂-CO- darstellt, so dass nach der Spaltung die entsprechende Struktur -NH-(CH₂)₀₋₄₋(CHCH₃)₀₋₄-CHY⁵-COOH bzw. -NH-(CH₂)₀₋₄₋(CHCH₃)₀₋₄-CHY⁵-CO-NH -(CH₂)₀₋₄ -CHNH₂-COOH erhalten wird.
(b) -CH₂-Sₓ-(CH₂)₀₋₄-CHY⁵-CO- auf, wobei x 0 oder 1 ist, und Y⁵ -H oder -NHY⁶ darstellt, wobei Y6 -H oder -COCH₃ darstellt, so dass nach der Spaltung die entsprechende Struktur -CH₂-Sₓ-(CH₂)₀₋₄-CHY⁵-COOH erhalten wird.

Erfindungsgemäß bevorzugt ist auch die Linker-Grundstruktur (i) bei Anbindung an die Position R4, insbesondere wenn m=0.

Wenn der Linker an eine Cystein-Seitenkette bzw. einen Cysteinrest gebunden ist, leitet sich L2 vorzugsweise von einer mit der Sulfhydryl-Gruppe des Cysteins reaktiven Gruppe ab. Hierzu zählen Haloacetyle, Maleimide, Aziridine, Acryloyle, Arylierende Verbindungen, Vinylsulfone, Pyridyldisulfide, TNB-thiole and Disulfid-reduzierende Mittel. Diese Gruppen reagieren in der Regel elektrophil mit der Sulfhydryl-Bindung unter Bildung einer Sulfid (z.B. Thioether)- oder Disulfid-Brücke. Bevorzugt sind stabile Sulfidbrücken. L2 ist vorzugsweise 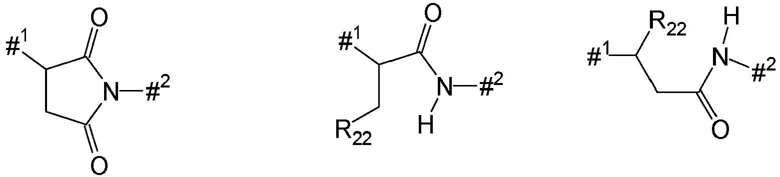 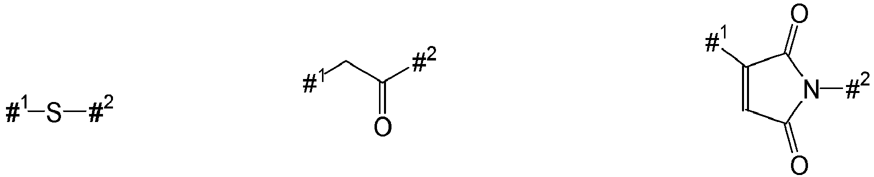 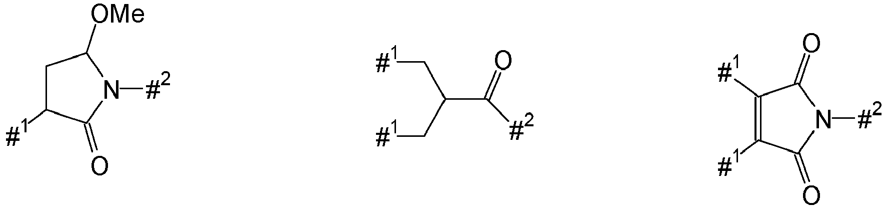 wobei
#¹ die Verknüpfungsstelle mit dem Schwefelatom des Antikörpers kennzeichnet,
#² die Verknüpfungsstelle mit der Gruppe L¹ kennzeichnet, und
R_{22 -}COOH, -COOR, -COR, -CONHR, -CONR₂ (mit R jeweils C1-3-Alkyl), -CONH2, vorzugsweise -COOH darstellt.

Besonders bevorzugt als L2 ist: 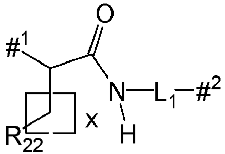 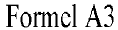 bzw. 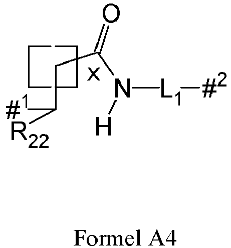 wobei #¹ die Verknüpfungsstelle mit dem Schwefelatom des Antikörpers kennzeichnet, #² die Verknüpfungsstelle mit der dem Wirkstoff kennzeichnet, x 1 oder 2 darstellt, und R²² -COOH, - COOR, -COR, -CONR₂, -CONHR (mit R jeweils C1-3-Alkyl), -CONH2, vorzugsweise -COOH darstellt. Bevorzugt ist es, wenn x=1 und R²² -COOH darstellt.

In einem erfindungsgemäßen Konjugat bzw. in einem Gemisch der erfindungsgemäßen Konjugate liegen die Bindungen an einen Cysteinrest des Antikörpers vorzugsweise zu über 80 %, besonders bevorzugt zu über 90 % (jeweils bezogen auf die Gesamtanzahl von Bindungen des Linkers an den Antikörper), besonders bevorzugt in einer der beiden Strukturen der Formel A3 oder A4 vor. Hierbei liegen die Strukturen der Formel A3 oder A4 in der Regel gemeinsam vor, vorzugsweise in einem Verhältnis von 60:40 bis 40:60 bezogen auf die Anzahl der Bindungen an den Antikörper. Die restlichen Bindungen liegen dann in der Struktur 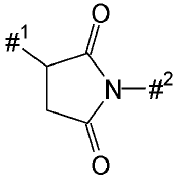 vor. Erfindungsgemäß wird L1 vorzugsweise durch die Formel

#¹-(NR¹⁰)ₙ-(G1)ₒ-G2-#²

dargestellt, wobei
R¹⁰ -H, -NH₂ oder C₁-C₃-Alkyl darstellt;
G1 -NHCO- , -CONH- oder 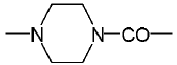 darstellt (R¹⁰ ist vorzugsweie nicht -NH₂, wenn
G1 -NHCO- oder 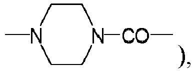
n 0 oder 1 ist;
o 0 oder 1 ist; und
G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -SO₂-, -NRY-, - NRYCO-, -C(NH)NRY-, -CONRY-, -NRYNRY-, -SO2NR^{y}NR^{y}-, -CONRYNRY-, (wobei R^{y} -H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, -NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) und/oder einen 3 bis 10-gliedriger, aromatischen oder nicht aromatischen Heterozyklus mit bis zu 4 Heteroatomen ausgewählt aus N, O und S, -SO- oder -SO2- unterbrochen sein kann (vorzugsweise 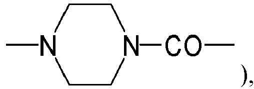 wobei die Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein können.
G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -SO2-, -NH-, - CO-, -NHCO-, -CONH-, -NMe-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, oder -SO- unterbrochen sein kann (vorzugsweise 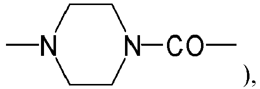 wobei die Seitenketten, sofern vorhanden, mit -NHCONH₂, -COOH, - OH, -NH₂, -NH-CNNH₂, Sulfonamid, Sulfon, Sulfoxid, oder Sulfonsäure substituiert sein können.
G2 ist vorzugsweise eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen - O-, -S-, -SO-, -SO2-, -NH-, -CO-, -NHCO-, -CONH-, -NMe-, -NHNH-, -SO2NHNH-, - CONHNH-, -CR^{x}=N-O- (wobei Rx H, C₁-C₃-Alkyl oder Phenyl darstellt) und einen 3 bis 10-gliedriger, z.B. 5 bis 10gliedriger aromatischen oder nicht aromatischen Heterozyklus mit bis zu 4 Heteroatomen ausgewählt aus N, O und S, -SO- oder -SO2- unterbrochen sein kann (vorzugsweise 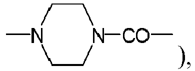 wobei Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein können.

Weitere unterbrechende Gruppen in G2 sind vorzugsweise 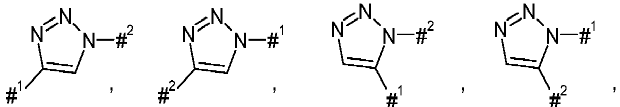 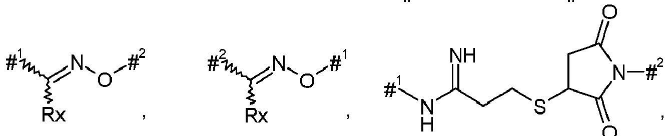 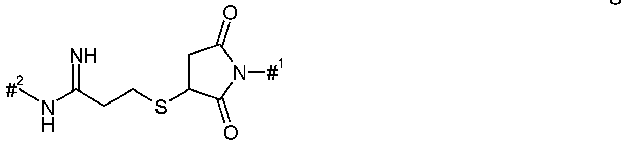 wobei Rx H, C₁-C₃-Alkyl oder Phenyl darstellt.

Hierbei ist #¹ die Bindung zum KSP-Inhibitor bzw. Prodrug und #² die Bindung zur Kopplungsgruppe zum Antikörper (z.B. L2).

Eine geradkettige oder verzweigte Kohlenwasserstoffkette aus Arylengruppen und/oder geradkettigen und/oder verzweigten und/oder cyclischen Alkylengruppen umfasst in der Regel einen α,ω-divalenten Alkylrest mit der jeweils angegebenen Zahl von Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen). Die Alkylengruppen in der Kohlenwasserstoffkette können jedoch auch verzweigt sein, d.h. ein oder mehrere H-Atome der oben genannten linearen Alkylengruppen können ggf. durch C1-10-Alkylgruppen substituiert sein und so Seitenketten bilden. Die Kohlenwasserstoffkette kann weiterhin cyclische Alkylengruppen (Cycloalkandiyl) enthalten, z.B. 1,4-Cyclohexandiyl oder 1,3-Cyclopentandiyl. Diese cyclischen Gruppen können ungesättigt sein. Insbesondere können in der Kohlenwasserstoffgruppe aromatische Gruppen (Arylengruppen) vorliegen, z.B. Phenylen. Auch in den cyclischen Alkylengruppen und den Arylengruppen können wiederum in oder mehrere H-Atome ggf. durch C1-10-Alkylgruppen substituiert sein. Es wird so eine Kohlenwasserstoffkette gebildet, die ggf. verzweigt ist. Diese Kohlenwasserstoffkette weist insgesamt 0 bis 100 Kohlenstoffatomen, vorzugsweise 1 bis 50, besonders bevorzugt 2 bis 25 Kohlenstoffatome auf.

Die Seitenketten können, sofern vorhanden, mit -NHCONH₂, -COOH, -OH, -NH₂, -NH-CNNH₂, Sulfonamid, Sulfon, Sulfoxid, oder Sulfonsäure ein- oder mehrfach, gleich oder verschieden substituiert sein.

Die Kohlenwasserstoffkette kann einfach oder mehrfach, gleich oder verschieden durch -O-, -S-, - SO-, -SO₂-, -NH-, -CO-, -NHCO-, -CONH-, -NMe-, -NHNH-, -SO₂NHNH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein (vorzugsweise

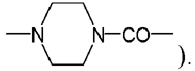

Weitere unterbrechende Gruppen in G2 sind vorzugsweise

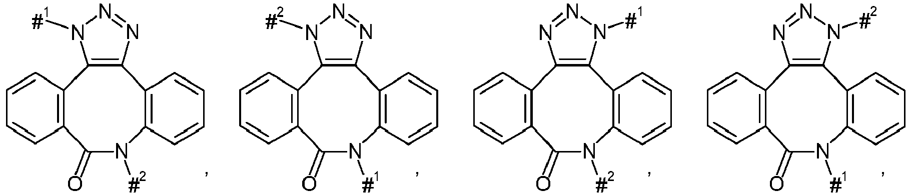

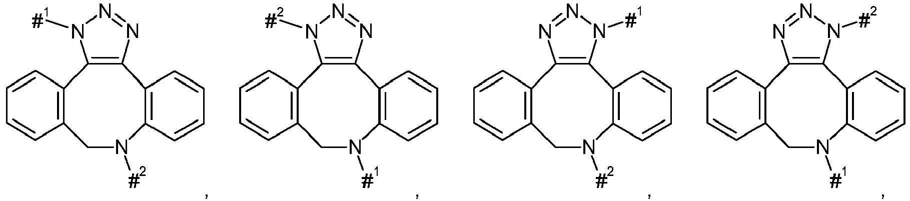

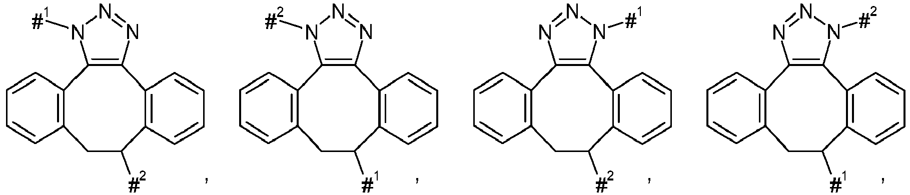

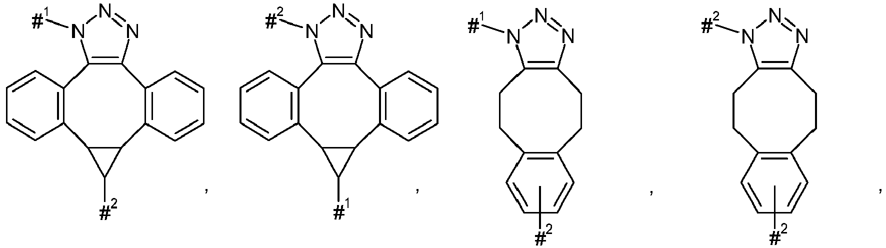

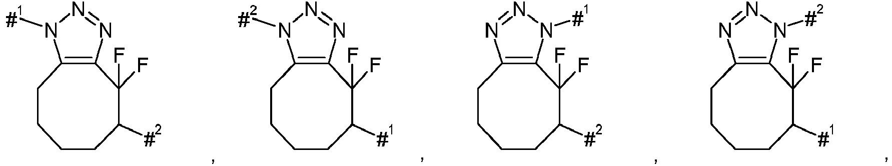

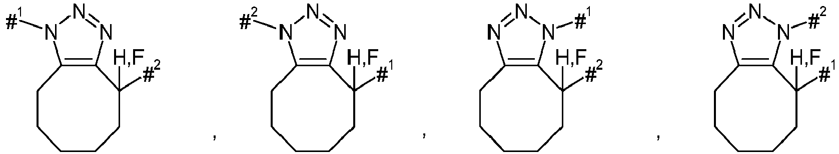

Vorzugsweise entspricht der Linker der folgenden Formel:

§-(CO)m-L1-L2-§§

wobei
m 0 oder 1 ist;
§ die Bindung an das Wirkstoffmolekül bzw. Prodrug darstellt und
§§ die Bindung an das Binderpeptid oder -protein darstellt, und
L1 und L2 die oben genannten Bedeutungen aufweisen.

Besonders bevorzugt weist L1 die Formel -NR¹¹B- auf, wobei
R¹¹ -H oder -NH₂ darstellt;
B -[(CH₂)ₓ-(X⁴)_{y}]_{W}-(CH₂)_{z}- darstellt,
w = 0 bis 20 ist;
x = 0 bis 5 ist;
x = 0 bis 5 ist;
y = 0 oder 1 ist;
z = 0 bis 5 ist; und
X⁴ -O-, -CONH-,-NHCO- oder 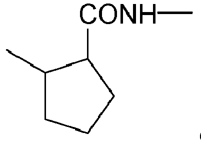 darstellt.

Erfindungsgemäß bevorzugte Linker L weisen die folgende Formel auf: 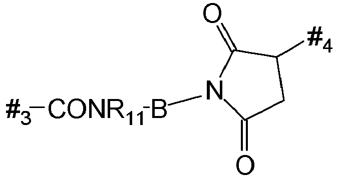 wobei
#3 die Bindung an das Wirkstoffmolekül bzw. Prodrug darstellt,
#4 die Bindung an das Binderpeptid oder -protein darstellt,
R¹¹ -H oder -NH2 darstellt;
B -[(CH₂)ₓ-(X⁴)_{y}]_{W}-(CH₂)_{z}- darstellt,
w = 0 bis 20 ist;
x = 0 bis 5 ist;
y = 0 oder 1 ist;
z = 1 bis 5 ist; und
X⁴ -O-, -CONH-, -NHCO- oder 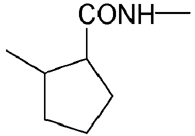 darstellt.

Weiterhin sind Linker bevorzugt, wobei wobei der Linker L₁ für eine der folgenden Gruppen steht:
§-NH-(CH₂)₂-§§;
§-NH-(CH₂)₆-§§;
§-NH-(CH₂)₂-O-(CH₂)₂-§§;
§-NH-CH(COOH)-(CH₂)₄-§§
§-NH-NH-C(=O)-(CH₂)₅-§§;
§-NH-(CH₂)₂-C(=O)-O-(CH₂)₂-§§;
§-NH-(CH₂)₂-C(=O)-NH-(CH₂)₂-§§;
§-NH-(CH₂)₂-NH-C(=O)-CH₂-§§;
§-NH-(CH₂)₃-NH-C(=O)-CH₂-§§;
§-NH-(CH₂)₂-NH-C(=O)-(CH₂)₂-§§;
§-NH-(CH₂)₂-NH-C(=O)-(CH₂)₅-§§;
§-NH-(CH₂)₂-NH-C(=O)-CH(CH₃)-§§;
§-NH-(CH₂)₂-O-(CH₂)₂-NH-C(=O)-CH₂-§§;
§-NH-CH(COOH)-CH₂-NH-C(=O)-CH₂-§§;
§-NH-CH(COOH)-(CH₂)₂-NH-C(=O)-CH₂-§§;
§-NH-CH(COOH)-(CH₂)₄-NH-C(=O)-CH₂-§§;
§-NH-CH(COOH)-CH₂-NH-C(=O)-(CH₂)₂-§§;
§-NH-(CH₂)₂-NH-C(=O)-CH(C₂H₄COOH)-§§;
§-NH-(CH₂)₂-NH-C(=O)-((CH₂)₂-O)₃-(CH₂)₂-§§;
§-NH-(CH₂)₂-S(=O)₂-(CH₂)₂-NH-C(=O)-CH₂-§§;
§-NH-(CH₂)₂-NH-C(=O)-CH₂-NH-C(=O)-CH₂-§§;
§-NH-(CH₂)₃-NH-C(=O)-CH₂-NH-C(=O)-CH₂-§§;
§-NH-CH(COOH)-CH₂-NH-C(=O)-CH(CH₂COOH)-§§;
§-NH-(CH₂)₂-NH-C(=O)-CH(C₂H₄COOH)-NH-C(=O)-CH₂-§§;
§-NH-CH(COOH)-CH₂-NH-C(=O)-(CH₂)₂-NH-C(=O)-CH₂-§§;
§-NH-(CH₂)₂-NH-C(=O)-(CH₂)₂-CH(COOH)-NH-C(=O)-CH₂-§§;
§-NH-CH(COOH)-CH₂-NH-C(=O)-CH(CH₂OH)-NH-C(=O)-CH₂-§§;
§-NH-CH[C(=O)-NH-(CH₂)₂-O)₄-(CH₂)₂COOH]-CH₂-NH-C(=O)-CH₂-§§;
§-NH-CH(COOH)-CH₂-NH-C(=O)-((CH₂)₂-O)₄-(CH₂)₂-NH-C(=O)-CH₂-§§;
§-NH-(CH₂)₄-CH(COOH)-NH-C(=O)-CH(CH₃)-NH-C(=O)-CH(isoC₃H₇)-§§;
§-NH-(CH₂)₄-CH(COOH)-NH-C(=O)-CH(CH₃)-NH-C(=O)-CH(isoC₃H₇)-NH-C(=O)-(CH₂)₅-§§;
§-NH-(CH₂)₂-C(=O)-NH-(CH₂)₄-CH(COOH)-NH-C(=O)-CH(CH₃)-NH-C(=O)-CH(isoC₃H₇)-NH-C(=O)-CH₂-§§;
§-NH-(CH₂)₂-C(=O)-NH-(CH₂)₄-CH(COOH)-NH-C(=O)-CH(CH₃)-NH-C(=O)-CH(isoC₃H₇)-NH-C(=O)-(CH₂)₅-§§;
§-NH-(CH₂)₄-CH(COOH)-NH-C(=O)-CH[(CH₂)₃-NH-C(=O)-NH₂]-NH-C(=O)-CH(isoC₃H₇)-NH-C(=O)-(CH₂)₅-§§;
§-NH-(CH₂)₂-NH-C(=O)-(CH₂)₂-CH(COOH)-NH-C(=O)-CH(CH₃)-NH-C(=O)-CH(isoC₃H₇)-NH-C(=O)-(CH₂)₅-§§;
§-NH-CH(CH₃)-C(=O)-NH-(CH₂)₄-CH(COOH)-NH-C(=O)-CH(CH₃)-NH-C(=O)-CH(isoC₃H₇)-NH-C(=O)-(CH₂)₅-§§;
§-NH-(CH₂)₂-C(=O)-NH-(CH₂)₄-CH(COOH)-NH-C(=O)-CH[(CH₂)₃-NH-C(=O)-NH₂]-NH-C(=O)-CH(isoC₃H₇)-NH-C(=O)-(CH₂)₅-§§; 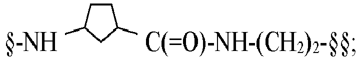 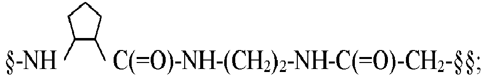 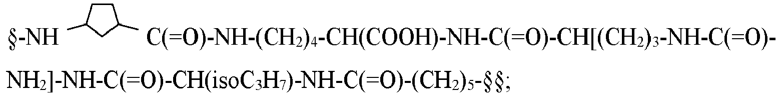 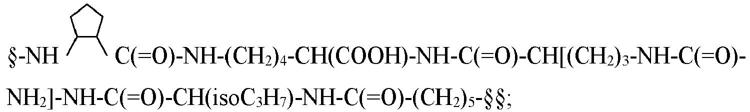 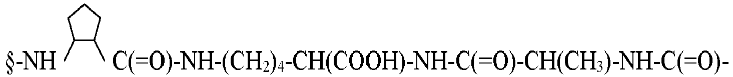
CH(isoC₃H₇)-NH-C(=O)-(CH₂)₅-§§;
§-NH-(CH₂)₂-C(=O)-NH-CH(isoC₃H₇)-C(=O)-NH-CH[(CH₂)₃-NH-C(=O)-NH₂]- 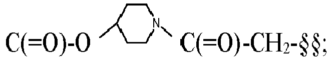 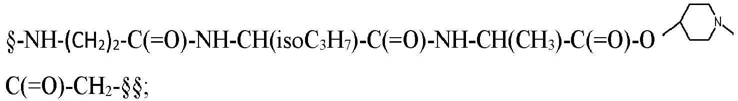 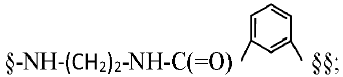 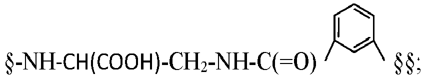 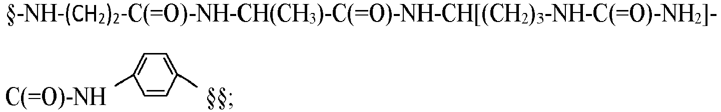 §-(CH₂)₂-C(=O)-NH-(CH₂)₂-§§;
§-(CH₂)₂-C(=O)-NH-(CH₂)₂-NH-C(=O)-CH₂-§§;
§-CH(CH₃)-NH-C(=O)-CH(isoC₃H₇)-§§;
§-CH(CH₃)-NH-C(=O)-CH(isoC₃H₇)-NH-C(=O)-CH₂-§§;
§-CH(CH₃)-NH-C(=O)-CH(isoC₃H₇)-NH-C(=O)-(CH₂)₅-§§;
§-(CH₂)₂-C(=O)-NH-((CH₂)₂-O)₄-(CH₂)₂-NH-C(=O)-CH₂-§§;
§-CH(CH3)-NH-C(=O)-CH(isoC3H7)-NH-C(=O)-((CH2)2-O)4-(CH2)2-NH-C(=O)-(CH₂)₂-§§; 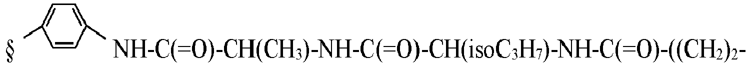
O)₄-(CH₂)₂-NH-C(=O)-(CH₂)₂-§§;
§-CH₂-S-(CH₂)₂-C(=O)-NH-(CH₂)₂-§§;
§-CH₂-S-(CH₂)₅-C(=O)-NH-(CH₂)₂-§§;
§-CH₂-S-CH₂CH(COOH)-NH-C(=O)-CH₂-§§;
§-CH₂-S-CH₂CH(COOH)-NH-C(=O)-(CH₂)₅-§§;
§-CH2-S-(CH2)2-C(=O)-NH-((CH2)2-O)2-(CH2)2-§§;
§-CH2-S-(CH2)2-C(=O)-NH-((CH2)2-O)2-(CH2)5-§§;
§-CH₂-S-(CH₂)₂-C(=O)-NH-(CH₂)₂-NH-C(=O)-CH₂-§§;
§-CH₂-S-(CH₂)₂-C(=O)-NH-(CH₂)₂-NH-C(=O)-CH₅-§§;
§-CH₂-S-(CH₂)₂-C(=O)-NH-CH(COOH)-CH₂-NH-C(=O)-CH₂-§§;
§-CH₂-S-CH₂CH(NH₂)-C(=O)-NH-(CH₂)₂-NH-C(=O)-(CH₂)₅-§§;
§-CH₂-S-(CH₂)₂-C(=O)-NH-((CH₂)₂-O)₂-(CH₂)₂-NH-C(=O)-CH₂-§§;
§-CH2-S-(CH2)2-C(=O)-NH-((CH2)2-O)4 -(CH2)2-NH-C(=O)-CH2-§§;
§-CH₂-S-(CH₂)₂-C(=O)-NH-((CH₂)₂-O)₂-(CH₂)₂-NH-C(=O)-(CH₂)₅-§§;
§-CH₂-S-(CH₂)₂-C(=O)-NH-((CH₂)₂-O)₄-(CH₂)₂-NH-C(=O)-(CH₂)₅-§§;
§-CH₂-S-CH₂CH(COOH)-NH-C(=O)-((CH₂)₂-O)₂-(CH₂)₂-NH-C(=O)-CH₂-§§;
§-CH₂-S-CH₂CH(COOH)-NH-C(=O)-((CH₂)₂-O)₄-(CH₂)₂-NH-C(=O)-CH₂-§§;
§-CH₂-S-CH₂CH(COOH)-NH-C(=O)-((CH₂)₂-O)₄-(CH₂)₂-NH-C(=O)-(CH₂)₂-§§;
§-CH₂-S-(CH₂)₂-CH(COOH)-NH-C(=O)-((CH₂)₂-O)₄-(CH₂)₂-NH-C(=O)-(CH₂)₂-§§;
§-CH₂-S-(CH₂)₂-C(=O)-NH-CH(C₂H₄COOH)-C(=O)-NH-(CH₂)₂-NH-C(=O)-CH₂-§§;
§-CH₂-S-CH₂CH[NH-C(=O)-(CH₂)₂-COOH]-C(=O)-NH-(CH₂)₂-NH-C(=O)-CH₂-§§;
§-CH₂-S-CH₂CH[NH-C(=O)-((CH₂)₂-O)₄-CH_{3]}-C(=O)-NH-(CH₂)₂-NH-C(=O)-CH₂-§§;
§-CH₂-S-CH₂CH(COOH)-NH-C(=O)-CH(CH₃)-NH-C(=O)-CH(isoC₃H₇)-NH-C(=O)-CH₂-§§;
§-CH₂-S-CH₂CH[NH-C(=O)-(CH₂)₂-COOH]-C(=O)-NH-(CH₂)₂-S(=O)2-(CH2)2-NH-C(=O)-CH₂-§§;
§-CH₂-S-CH₂CH[NH-C(=O)-(CH₂)₂-COOH]-C(=O)-NH-((CH₂)₂-O)₄-(CH₂)₂-NH-C(=O)-CH₂-§§;
§-CH₂-S-CH₂CH[C(=O)-NH-(CH₂)₂-COOH]-NH-C(=O)-((CH₂)₂-O)₄-(CH₂)₂-NH-C(=O)-CH₂-§§;
§-CH₂-S-CH₂CH[C(=O)-NH-(CH₂)₂-COOH]-NH-C(=O)-((CH₂)₂-O)₄-(CH₂)₂-NH-C(=O)-(CH₂)₂-§§;
§-CH₂-S-CH₂CH(COOH)-NH-C(=O)-(CH₂)₂CH(COOH)-NH-C(=O)-((CH₂)₂-O)₄-(CH₂)₂-NH-C(=O)-CH₂-§§
oder
§-CH₂-S-CH₂CH(COOH)-NH-C(=O)-CH[(CH₂)₂-COOH]-NH-C(=O)-((CH₂)₂-O)₄-(CH₂)₂-NH-C(=O)-(CH₂)₂-§§,
steht,
wobei
§ die Bindung an das Wirkstoffmolekül darstellt und
§§ die Bindung an den Antikörper darstellt und
isoC₃H₇ einen isoPropyl-Rest darstellt,

Die oben genannten Linker werden insbesondere bevorzugt in Konjugaten der Formel (IIa), in denen der Linker durch Substitution eines H-Atoms an R1 oder in Verbindung mit einem spaltbaren Linker SG1 an R4 ankoppelt, d.h. R1-L-#1 darstellt oder R4 -SG1-L-#1, wobei #1 die Bindung an den Antikörper darstellt.

Weiterhin sind die folgenden Linker erfindungsgemäß bevorzugt: In einem erfindungsgemäßen Konjugat bzw. in einem Gemisch der erfindungsgemäßen Konjugate liegen die Bindungen an einen Cysteinrest des Antikörpers vorzugsweise zu über 80 %, besonders bevorzugt zu über 90 %

(jeweils bezogen auf die Gesamtanzahl von Bindungen des Linkers an den Antikörper), besonders bevorzugt in einer der beiden Strukturen der Formel A5 oder A6 vor: 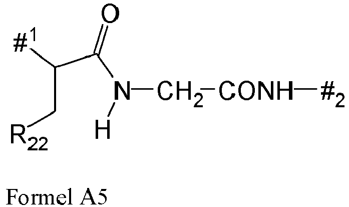 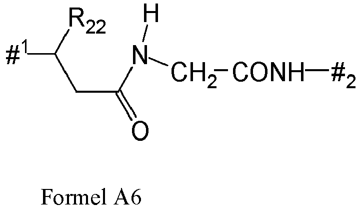 wobei
#¹ die Verknüpfungsstelle mit dem Schwefelatom des Antikörpers kennzeichnet,
#² die Verknüpfungsstelle mit der Gruppe L¹ kennzeichnet, und

R²² -COOH, -COOR, -COR, -CONR₂, -CONHR (mit R jeweils C1-3-Alkyl), -CONH₂, vorzugsweise -COOH darstellt.

Hierbei liegen die Strukturen der Formel A5 oder A6 in der Regel gemeinsam vor, vorzugsweise in einem Verhältnis von 60:40 bis 40:60 bezogen auf die Anzahl der Bindungen an den Antikörper. Die restlichen Bindungen liegen dann in der Struktur 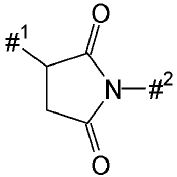 vor.

Andere Linker -L-, die an eine Cystein-Seitenkette bzw. Cysteinrest gebunden sind, weisen die folgende Formel auf: 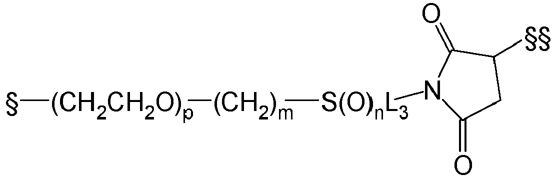 wobei
§ die Bindung an das Wirkstoffmolekül bzw. Prodrug darstellt und
§§ die Bindung an das Binderpeptid oder -protein darstellt,
m 0, 1, 2, oder 3 ist;
n 0, 1 oder 2 ist;
p 0 bis 20 beträgt; und
L3 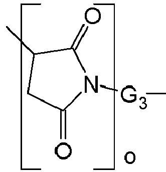 darstellt;
wobei
o 0 oder 1 ist;
   und
G₃ eine geradkettige oder verzweigte Kohlenwasserstoffkette mit 1 bis 100 Kohlenstoffatomen aus Arylengruppen und/oder geradkettigen und/oder cyclischen Alkylengruppen ist, die einfach oder mehrfach durch eine oder mehrere der Gruppen -O-, -S-, -SO-, SO2, -NH-, -CO-, -NHCO-, - CONH-, -NMe-, -NHNH-, -SO2NHNH-, -CONHNH- und einen 3 bis 10gliedriger (vorzugsweise 5 bis 10gliedriger), aromatischen oder nicht aromatischen Heterozyklus mit bis zu 4 Heteroatomen ausgewählt aus N, O und S, -SO- oder -SO2- unterbrochen sein kann (vorzugsweise 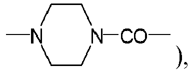 wobei die Seitenketten, sofern vorhanden, mit -NHCONH₂, -COOH, -OH, - NH₂, -NH-CNNH₂, Sulfonamid, Sulfon, Sulfoxid oder Sulfonsäure substituiert sein können.

Bevorzugt ist in der obigen Formel
m 1 ist;
p 0 ist;
n 0 ist;
und L3 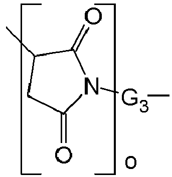 darstellt;
   wobei
   o 0 oder 1 ist; und
   G₃ -(CH₂CH₂O)ₛ(CH₂)ₜ(CONH)ᵤ CH₂CH₂O)_{y}(CH₂)_{w}- darstellt, wobei
   s, t, v und w jeweils unabhängig voneinander 0 bis 20 betragen, und u 0 oder 1 beträgt.

Bevorzugte Gruppen L1 in der obigen Formel §-(CO)m-L1-L2-§§ sind die folgenden, wobei r eine Zahl von 0 bis 20, vorzugsweise von 0 bis 15, besonders bevorzugt von 1 bis 20, insbesondere bevorzugt von 2 bis 10 aufweist:

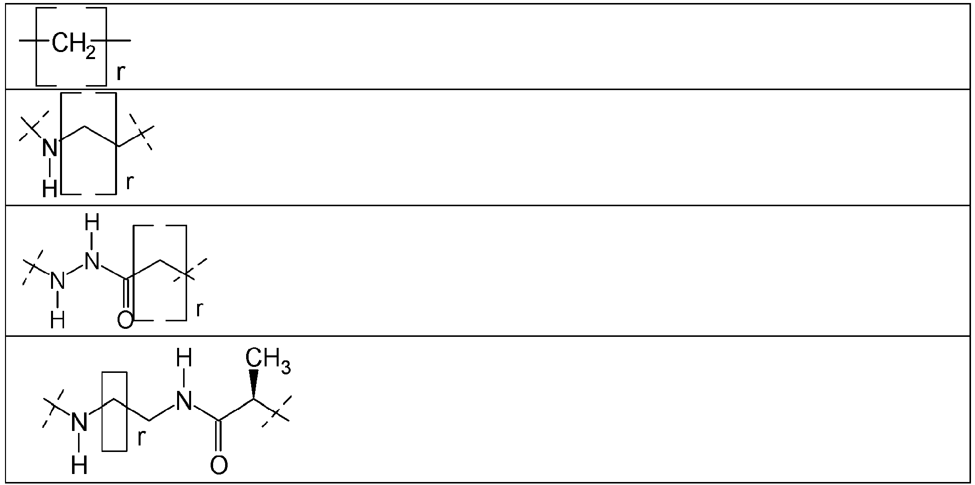

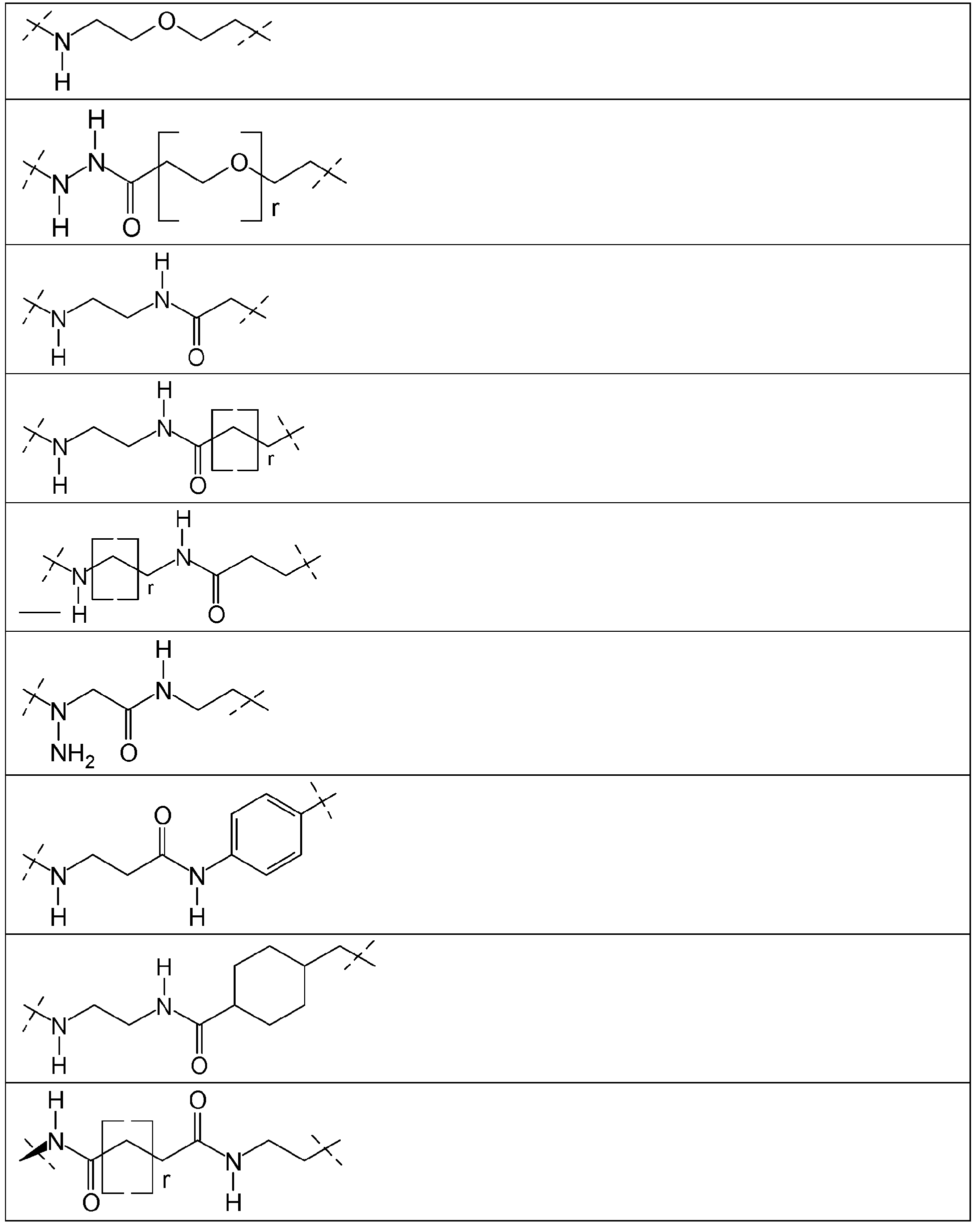

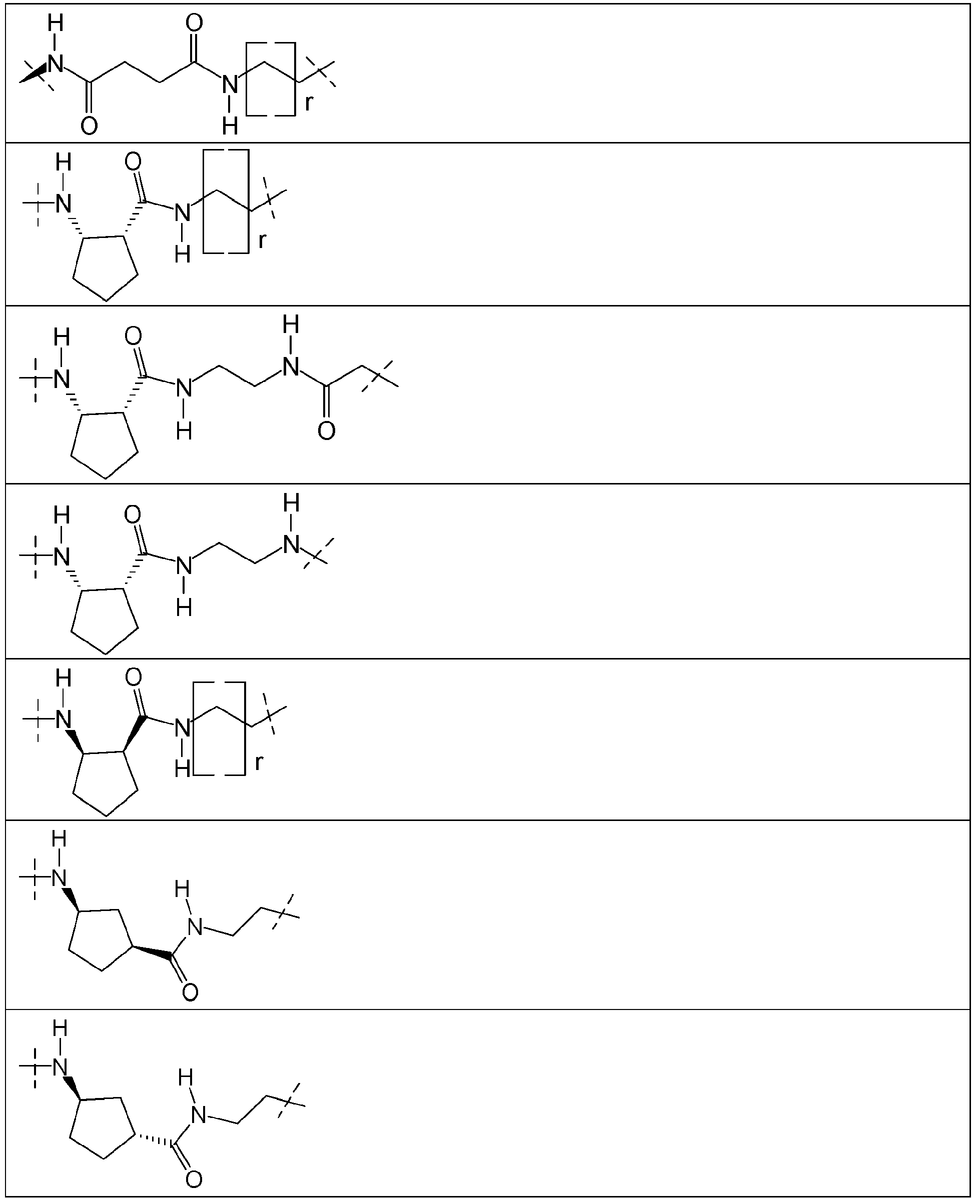

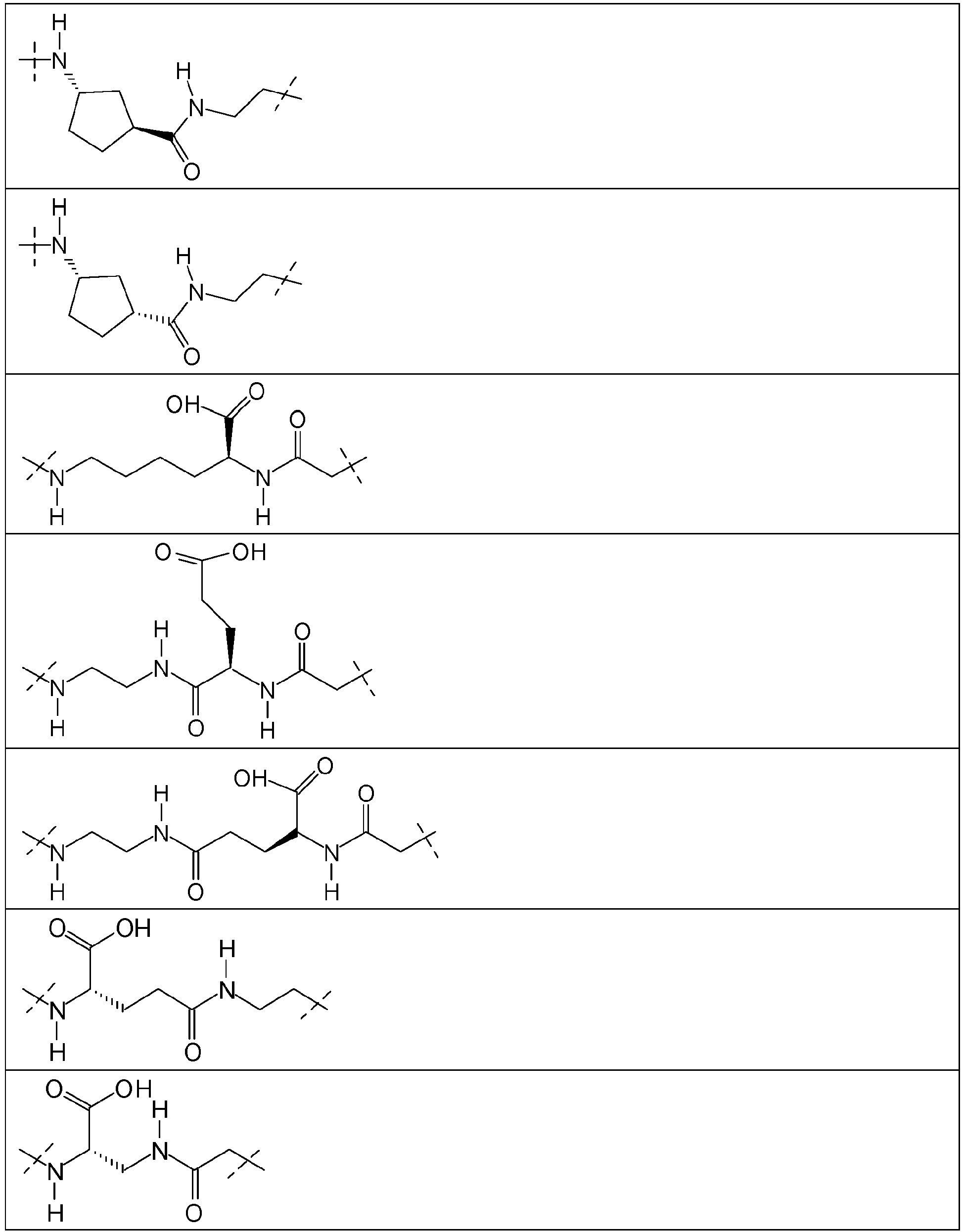

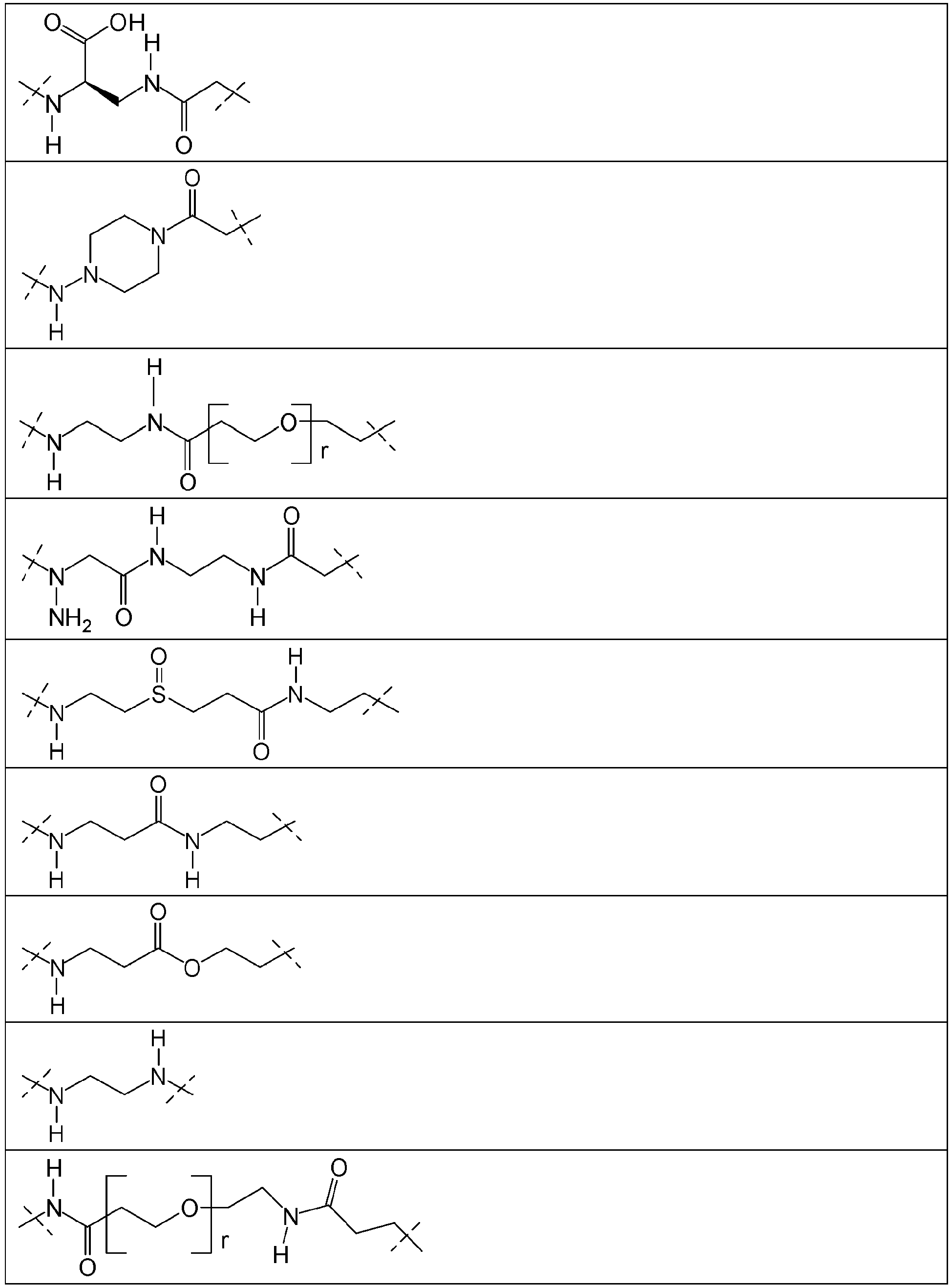

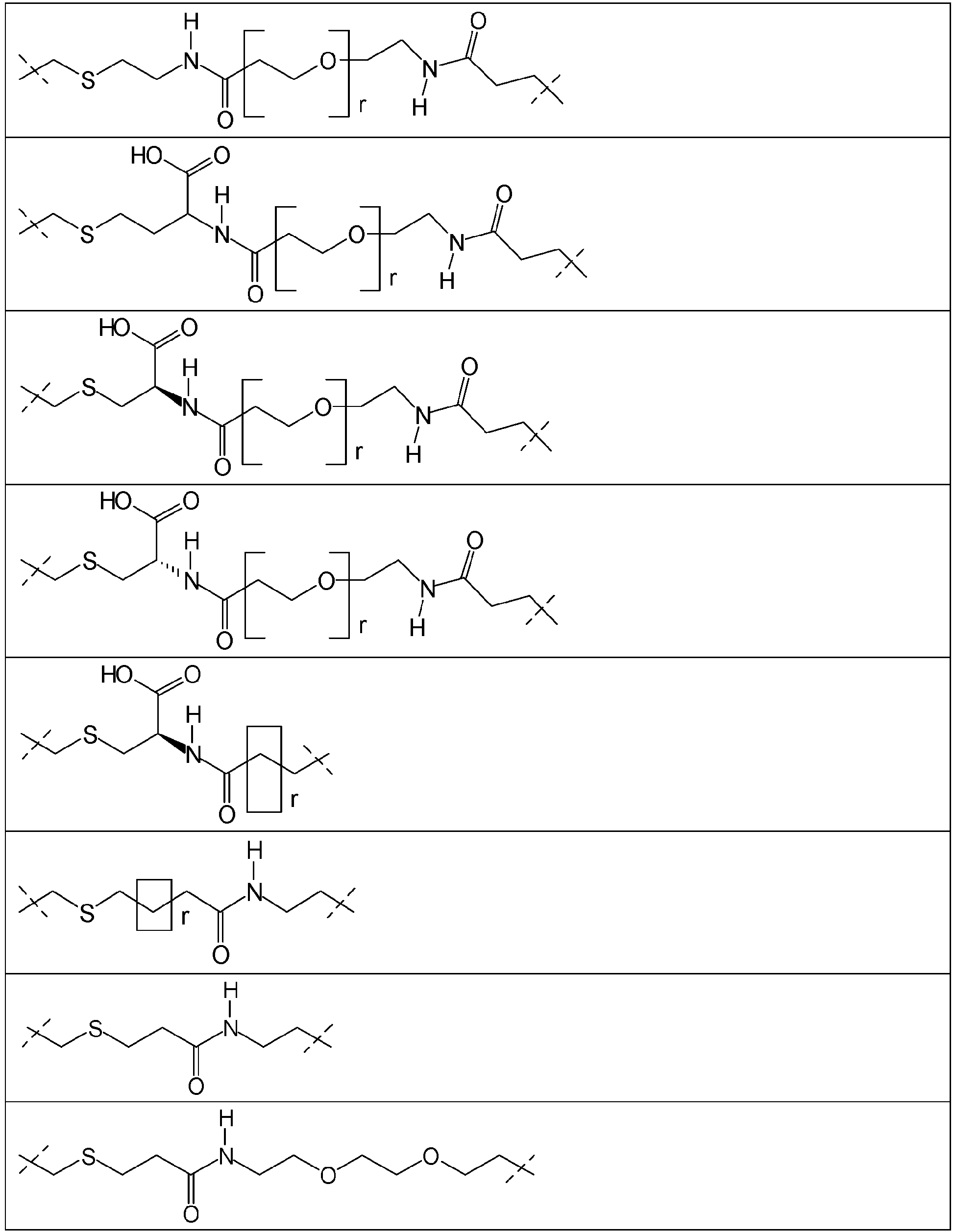

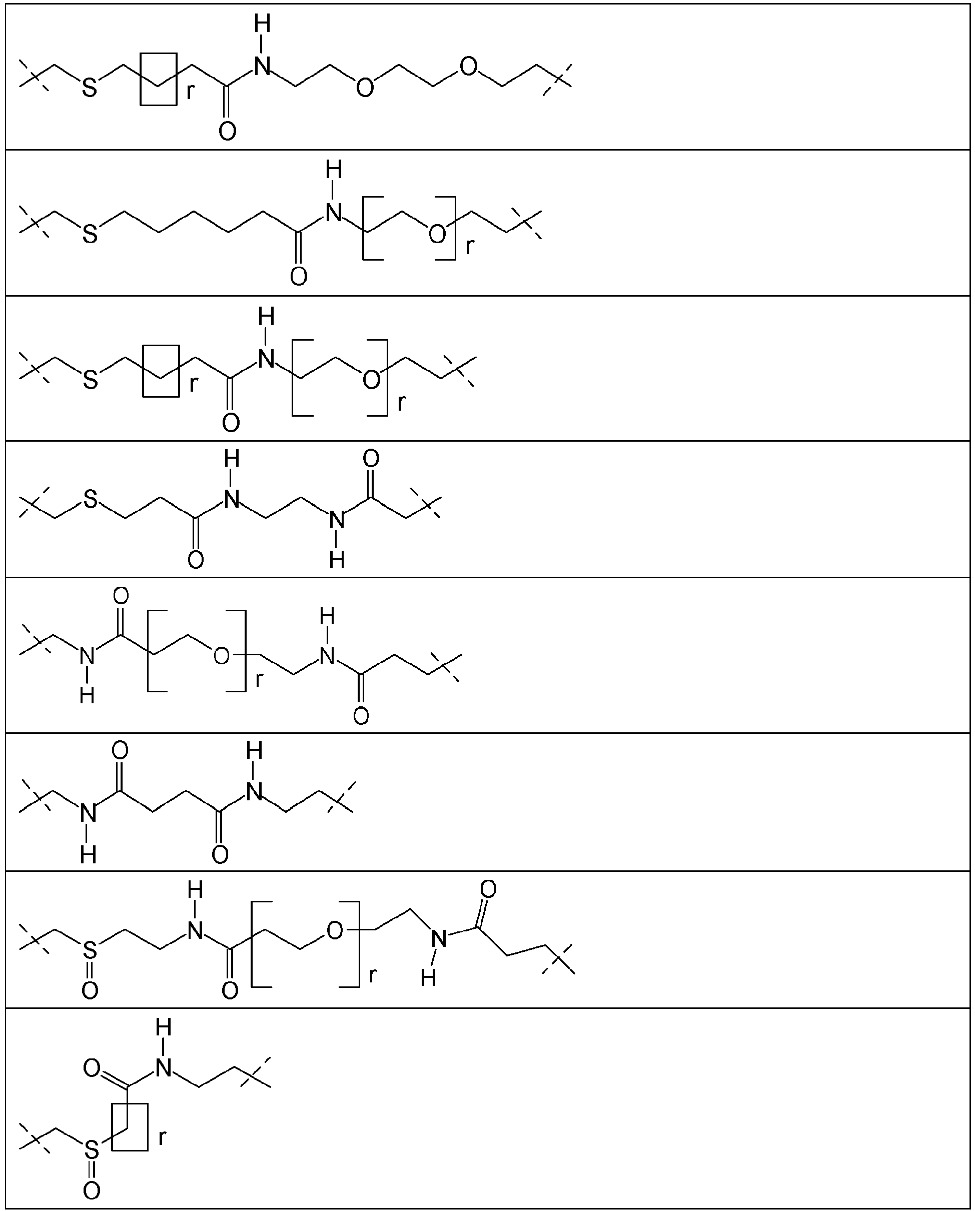

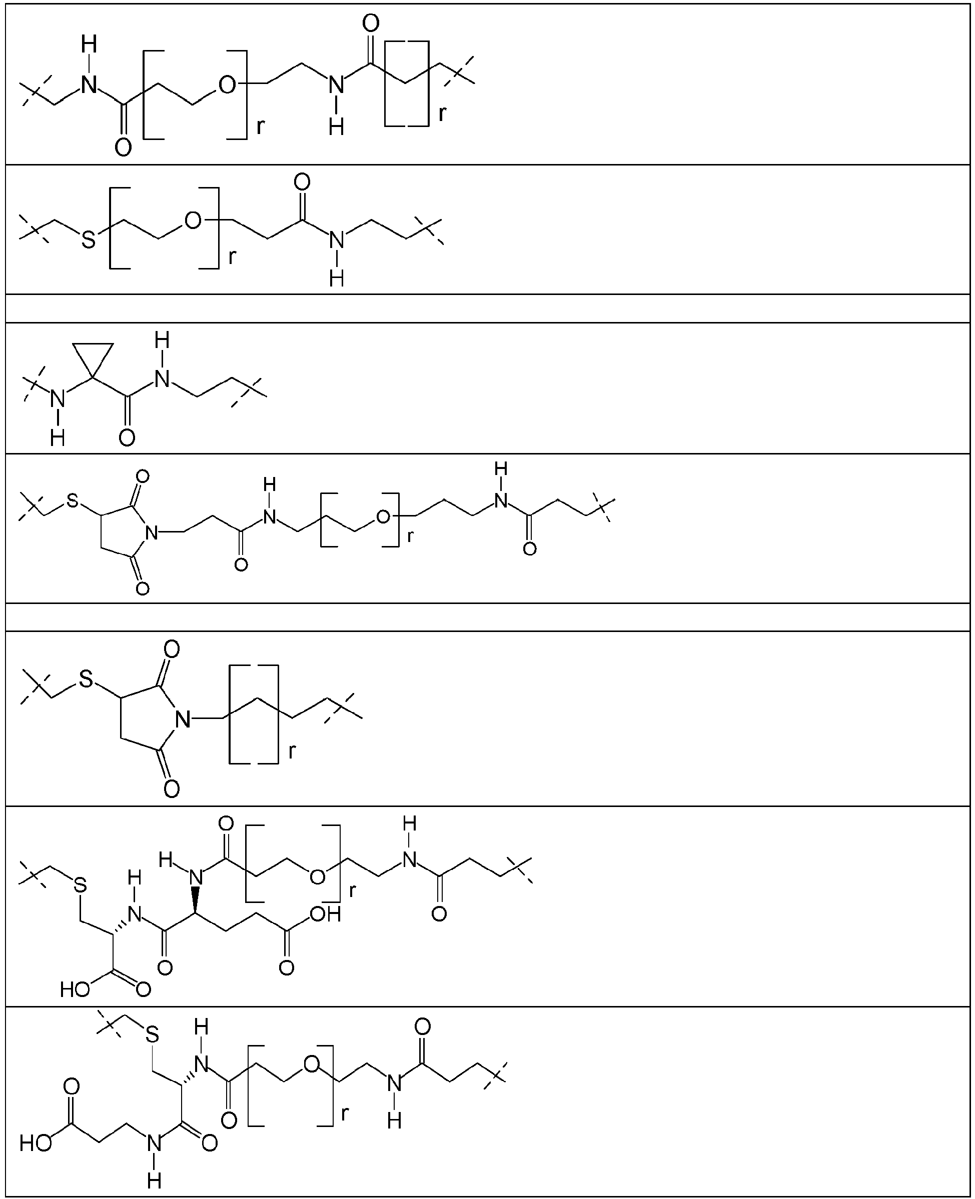

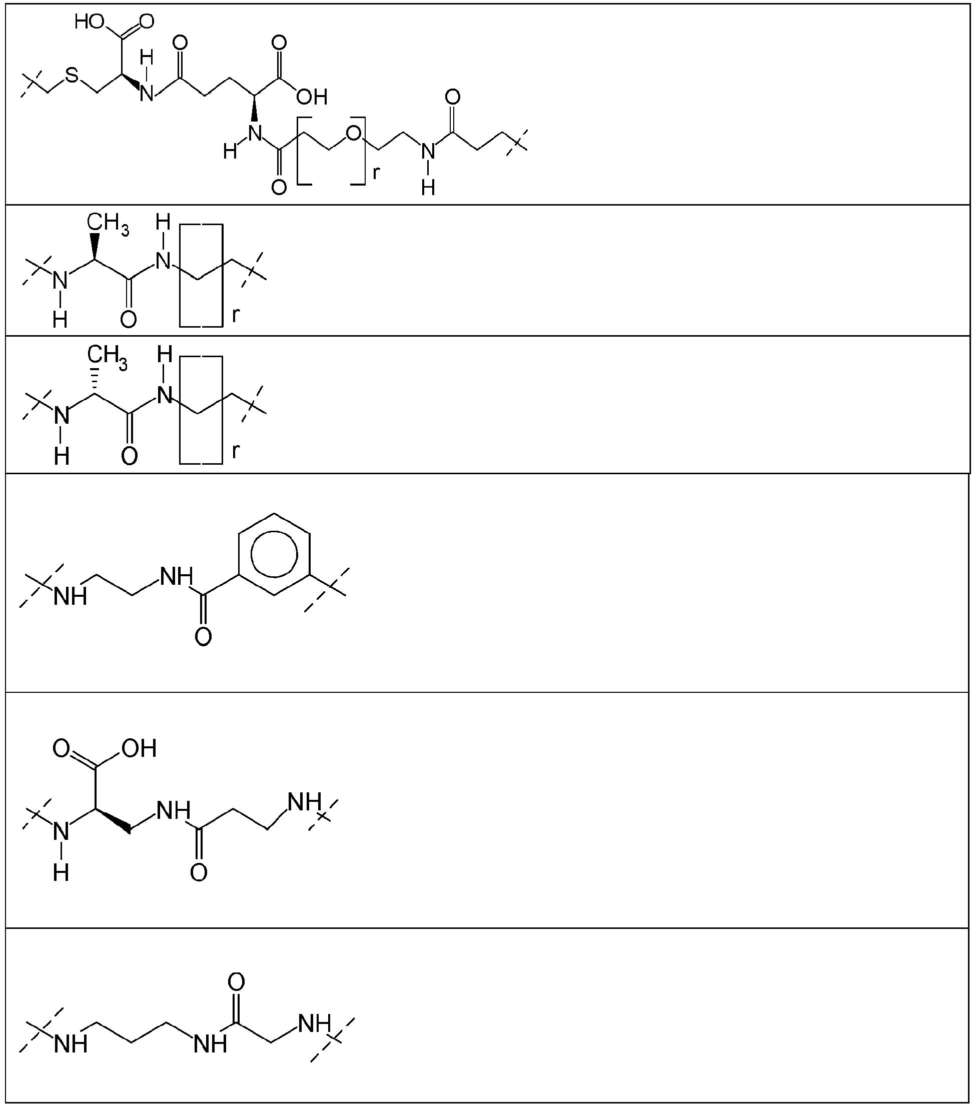

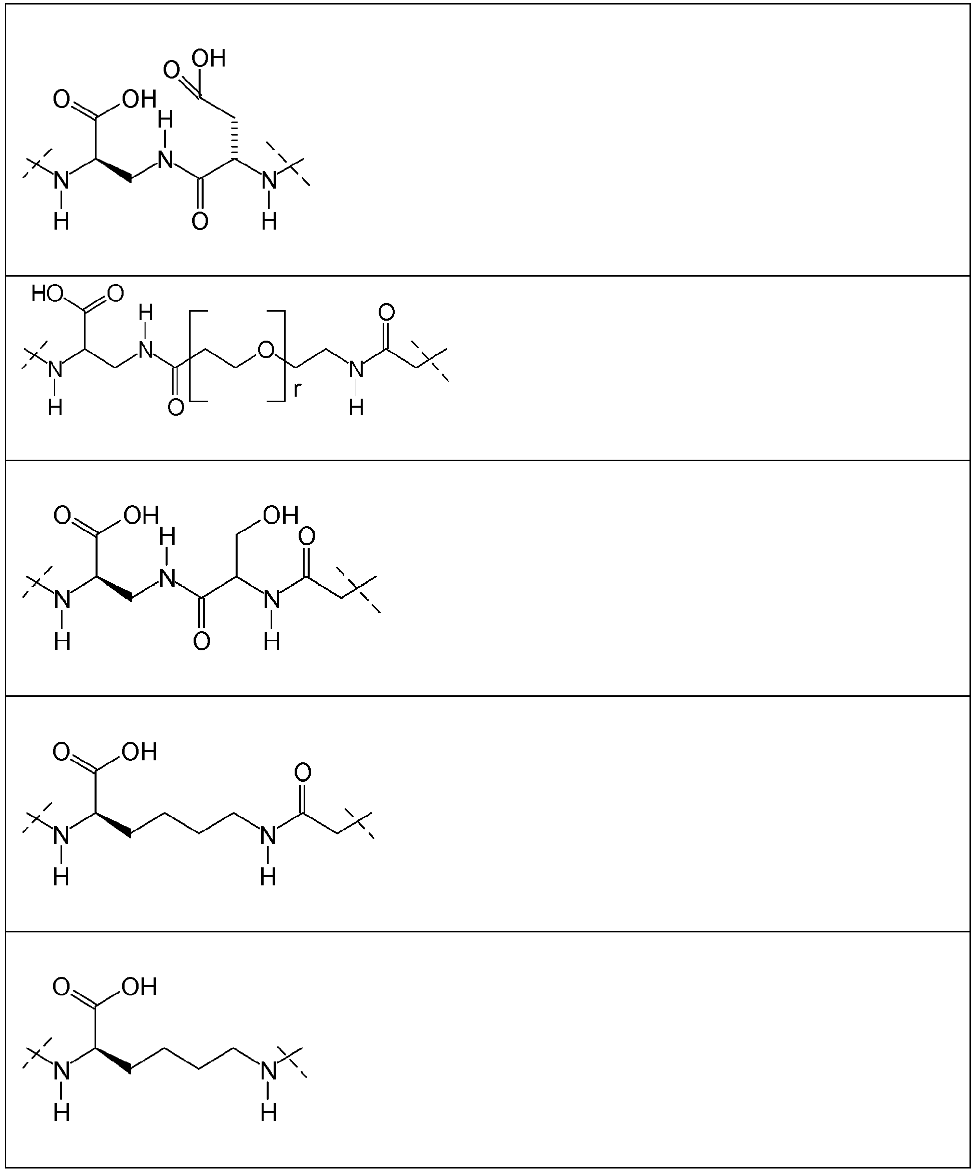

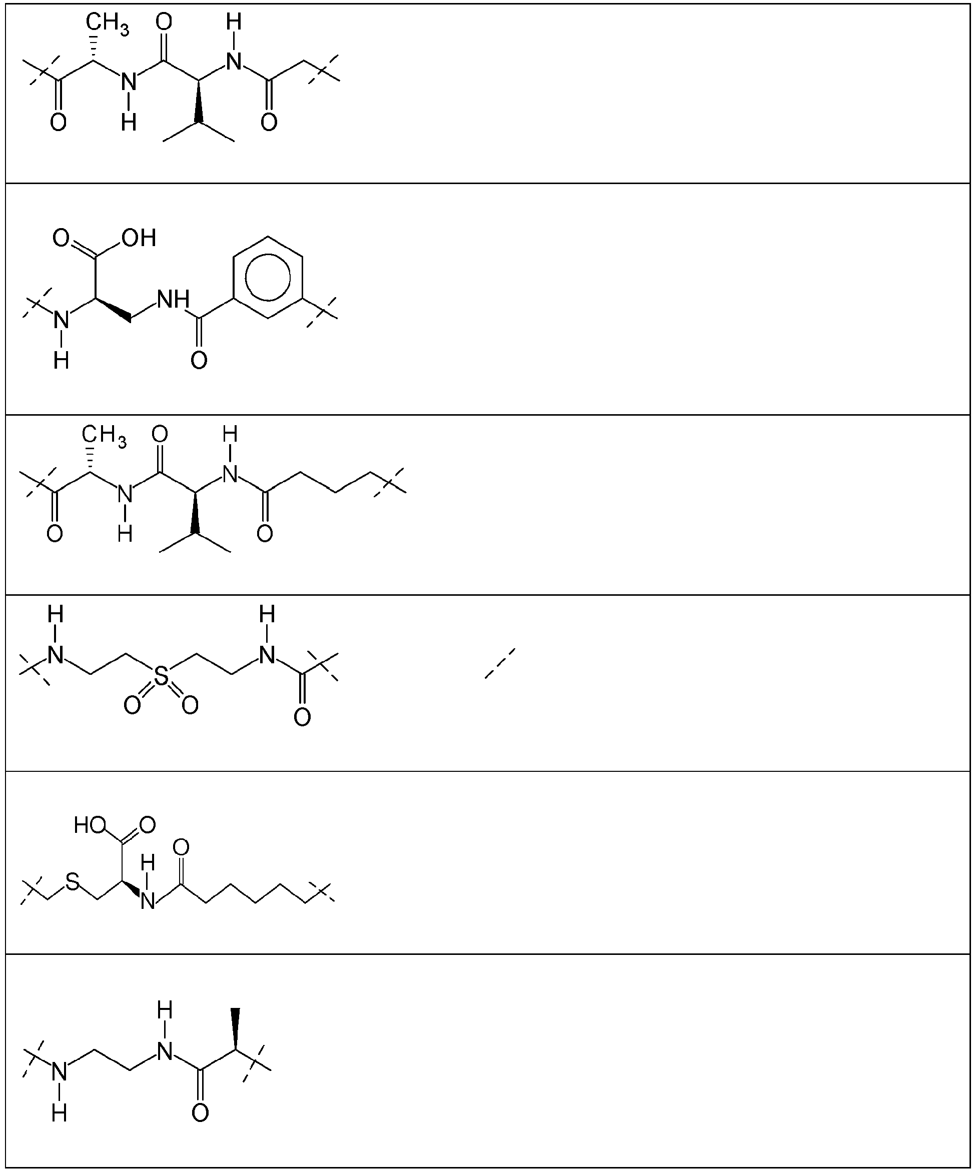

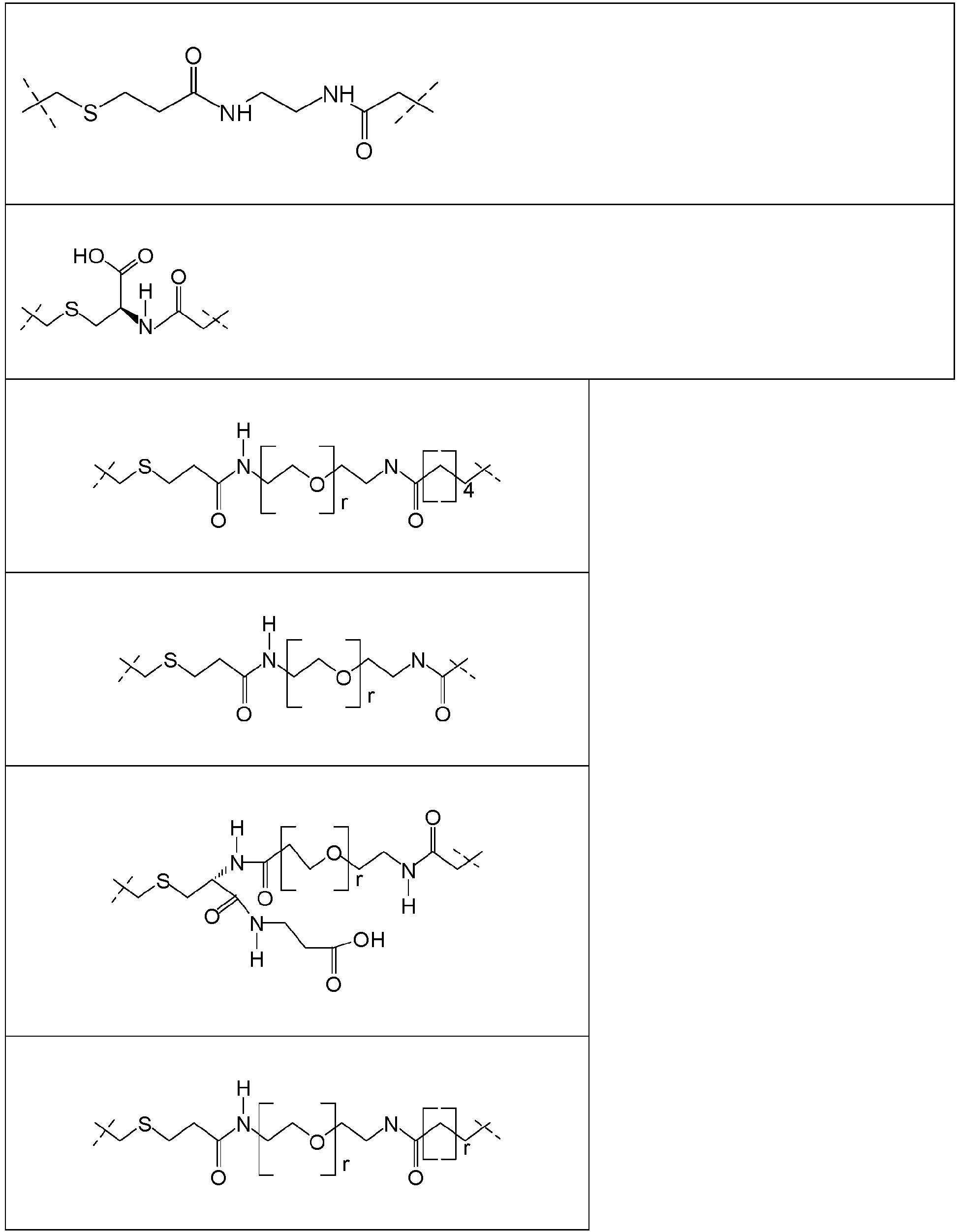

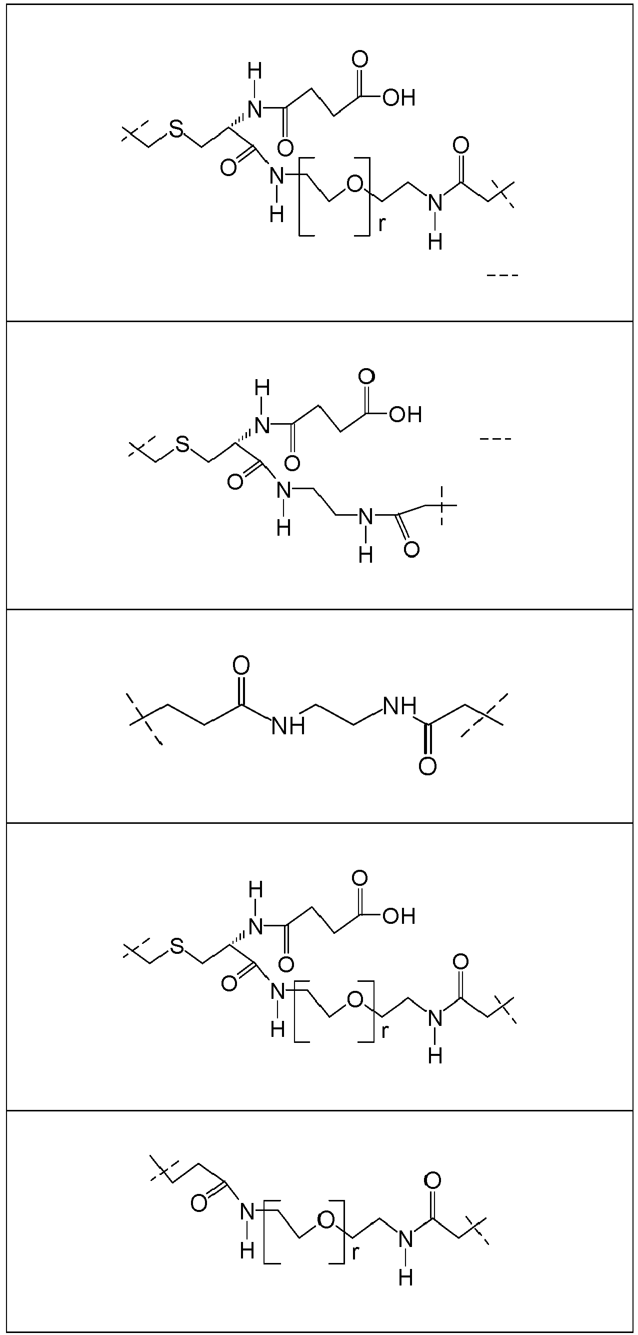

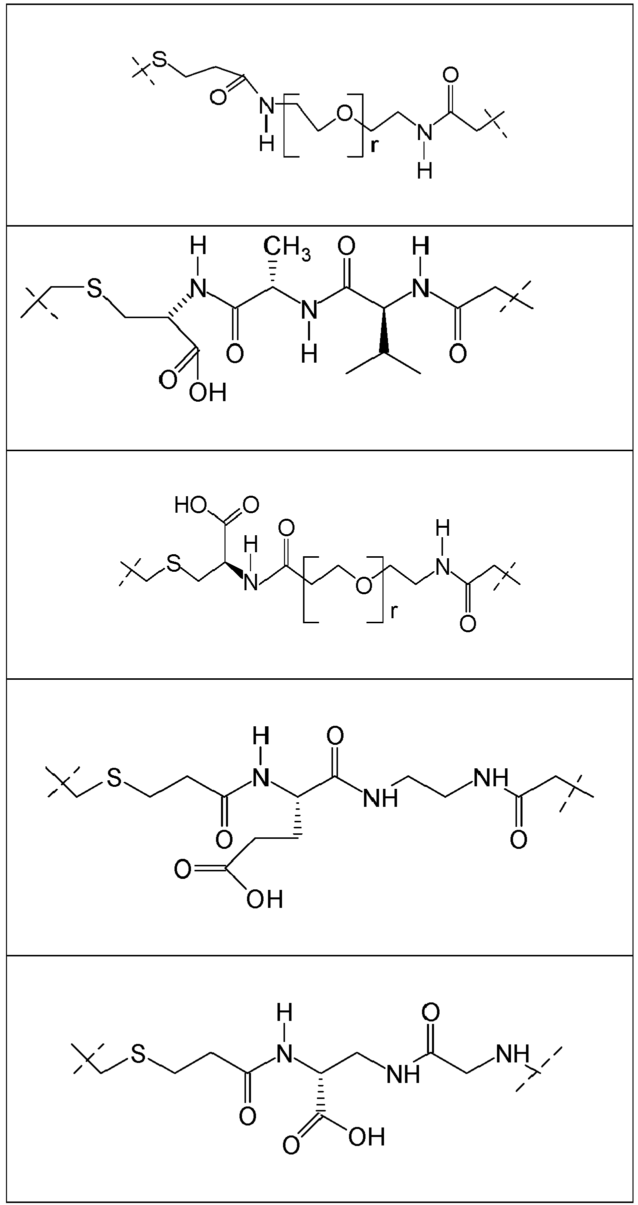

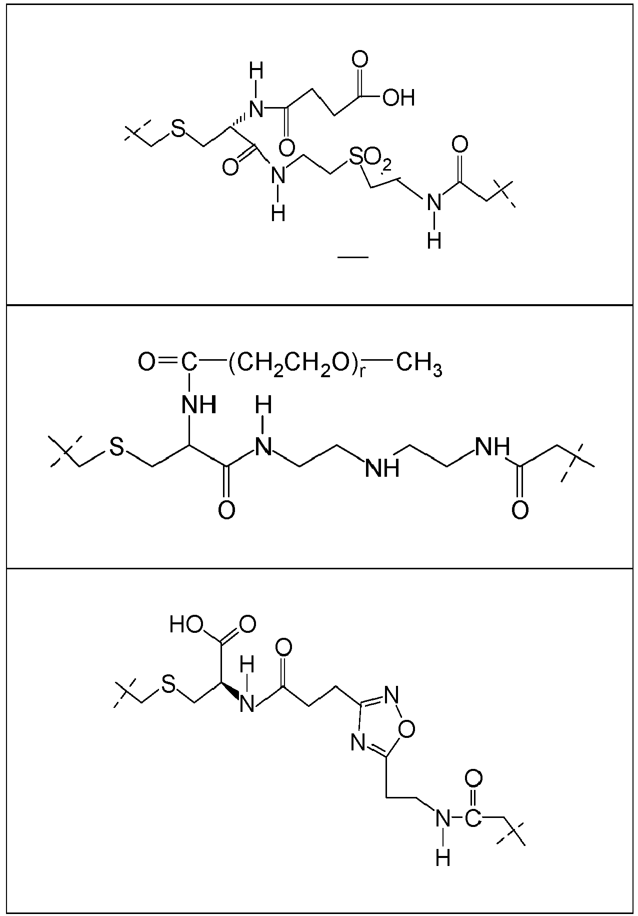

Weitere Beispiele für L1 sind in Tabelle C angegeben, in denen diese Gruppe in einem Kasten hervorgehoben ist.

In den folgenden Tabellen A und A` sind Beispiele für einen Linkerteil L1 angegeben. In der Tabelle ist weiterhin angegeben, mit welcher Gruppe L2 diese Beispiele für L1 vorzugsweise kombiniert werden, sowie die bevorzugte Ankopplungsstelle (R¹ oder R³ oder R⁴) sowie der bevorzugte Wert für m, also ob vor L1 eine Carbonylgruppe vorliegt oder nicht (vgl. §-(CO)m-L1-L2-§§). Diese Linker werden vorzugsweise an einen Cysteinrest gekoppelt. Wenn L2 ein Bernsteinsäureimid ist bzw. sich hiervon ableitet, kann dieses Imid ganz oder teilweise auch in Form des hydrolysierten offenkettigen Bernsteinsäureamids vorliegen, wie oben beschrieben. In Abhängigkeit von L1 kann diese Hydrolyse zu offenkettigen Bernsteinsäureamiden mehr oder weniger stark oder gar nicht ausgeprägt sein.

**Tabelle A**

| Subst. | m | L1 | L2 |
|---|---|---|---|
| R¹ | 1 | 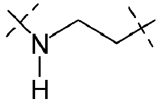 | 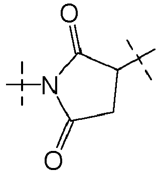 |
| R¹ | 1 | 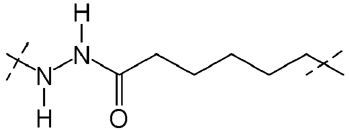 | 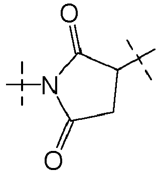 |
| R¹ | 1 | 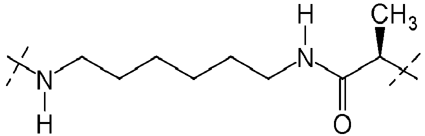 | 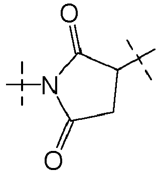 |
| R¹ | 1 | 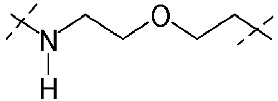 | 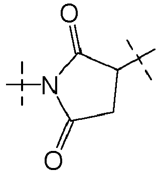 |
| R¹ | 1 | 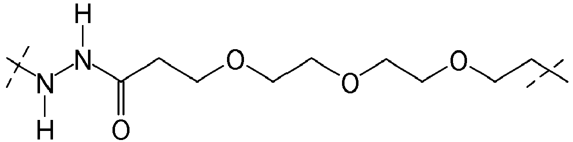 | 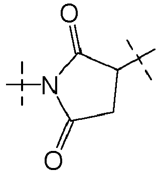 |
| R¹ | 1 | 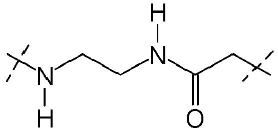 | 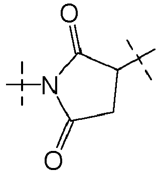 Siehe Anmerkung ** |
| R¹ | 1 | 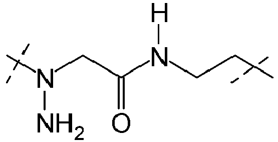 | 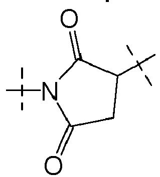 |
| R¹ | 1 | 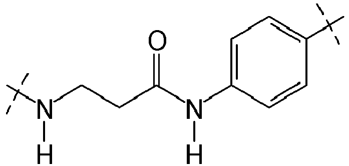 | 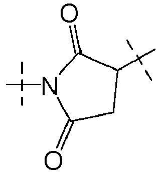 Siehe Anmerkung ** |
| R¹ | 1 | 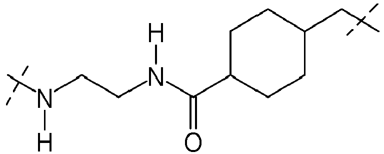 | 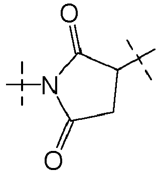 |
| R¹ | 1 | 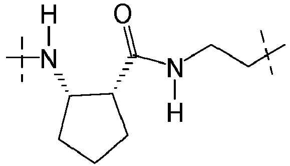 | 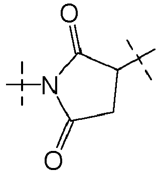 |
| R¹ | 1 | 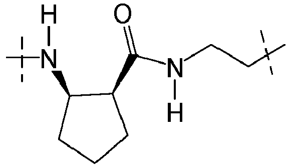 | 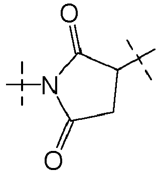 |
| R¹ | 1 | 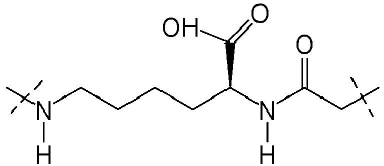 | 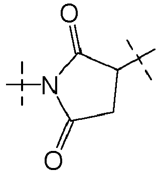 Siehe Anmerkung ** |
| R¹ | 1 | 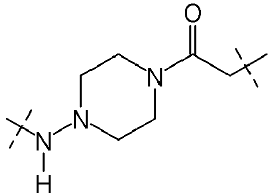 | 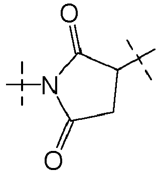 Siehe Anmerkung ** |
| R¹ | 1 | 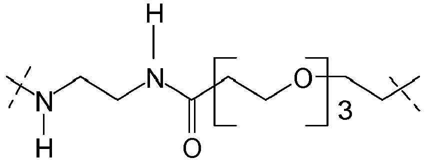 | 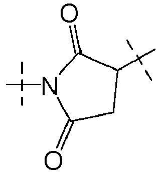 |
| R¹ | 1 | 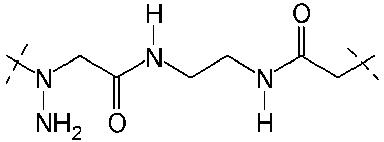 | 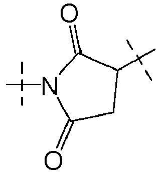 Siehe Anmerkung ** |
| R¹ | 1 | 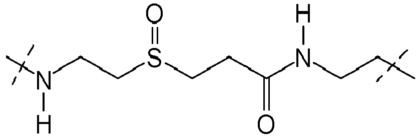 | 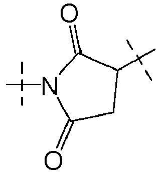 |
| R¹ | 1 | 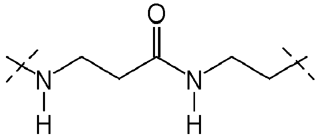 | 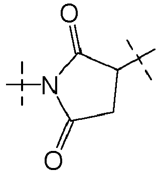 |
| R¹ | 1 | 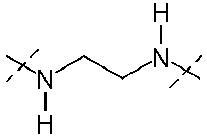 | 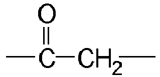 |
| R¹ | 1 | 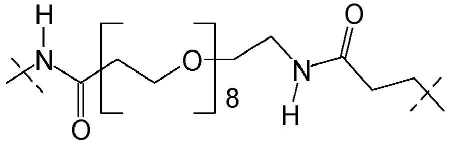 | 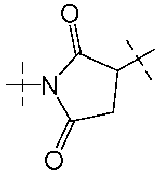 |
| R³ | 0 | 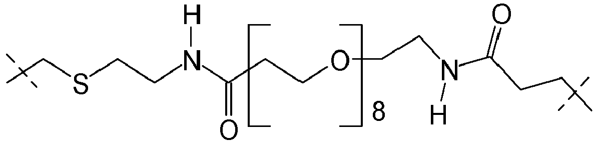 | 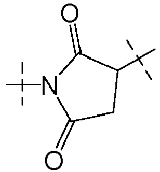 |
| R¹ | 1 | 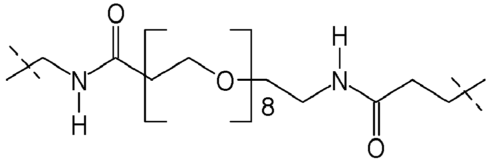 | 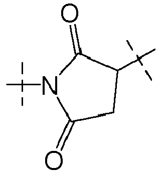 |
| R³ | 0 | 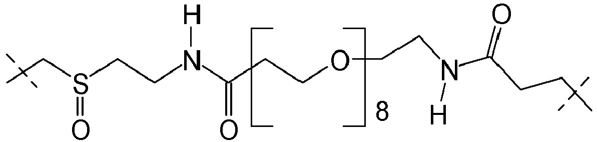 | 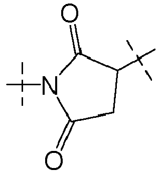 |
| R¹ | 1 | 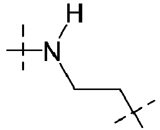 | 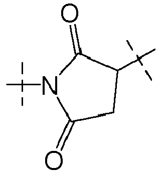 |
| R¹ | 0 | 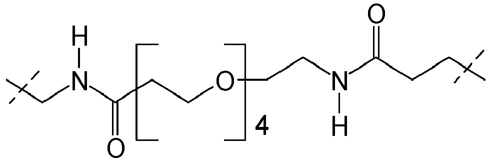 | 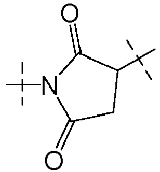 |
| R³ | 0 | 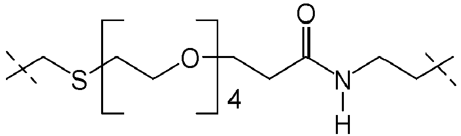 | 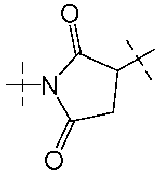 |
| R³ | 0 | 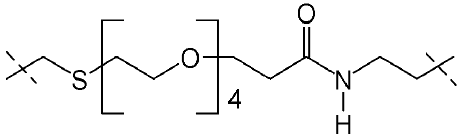 | 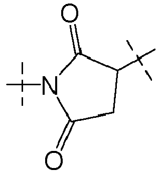 |
| R¹ | 1 | 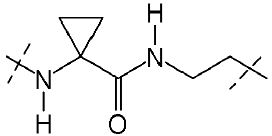 | 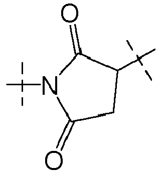 |
| R³ | 0 | 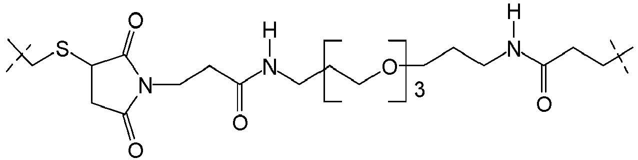 | 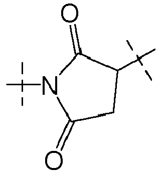 |
| R³ | 0 | 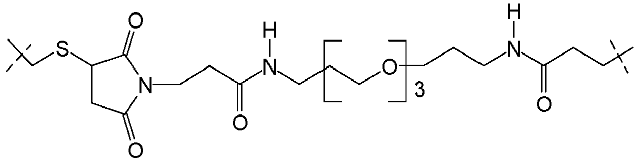 | 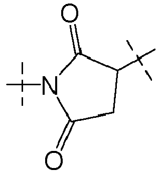 |
| R³ | 0 | 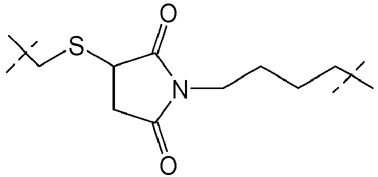 | 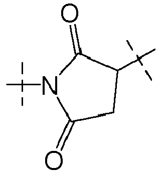 |
| R³ | 0 | 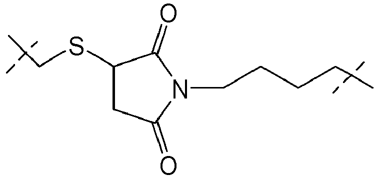 | 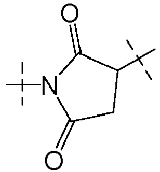 |
| R¹ | 1 | 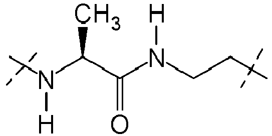 | 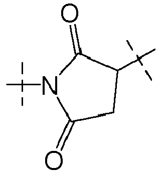 |
| R¹ | 1 | 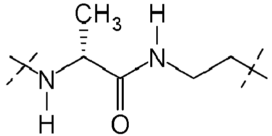 | 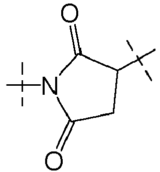 |

| | | | |
|---|---|---|---|
| **Besonders bevorzugt werden die in diesen Zeilen angegebenen Linker L1 mit einem Linker L2 verknüpft, der ausgewählt ist aus: | | | |

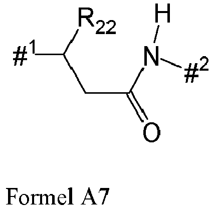

und/oder 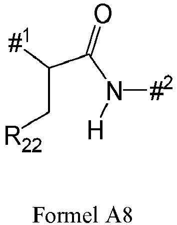 vor, wobei #¹ die Verknüpfungsstelle mit dem Schwefelatom des Binders kennzeichnet, #² die Verknüpfungsstelle mit der Gruppe L¹ kennzeichnet, R²² vorzugsweise COOH darstellt. In einem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 % (jeweils bezogen auf die Gesamtanzahl von Bindungen des Linkers an den Binder), besonders bevorzugt in einer der beiden Strukturen der Formel A7 oder A8 vor. Hierbei liegen die Strukturen der Formel A7 oder A8 in der Regel gemeinsam vor, vorzugsweise in einem Verhältnis von 60:40 bis 40:60 bezogen auf die Anzahl der Bindungen an den Binder. Die restlichen Bindungen liegen dann in der Struktur 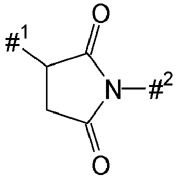 vor.

**Tabelle A`**

| Subst. | m | L1 | L2 |
|---|---|---|---|
| R¹ | 1 | 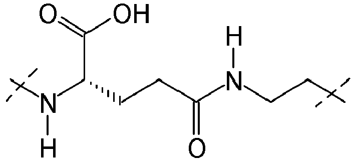 | 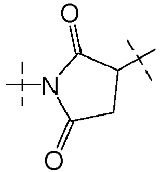 |
| R¹ | 1 | 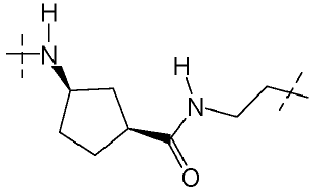 | 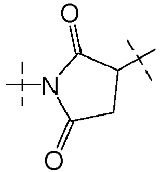 |
| R¹ | 1 | 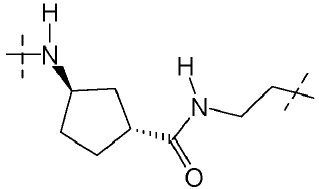 | 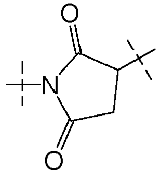 |
| R¹ | 1 | 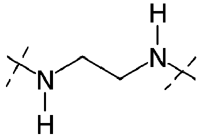 | 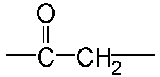 |
| R¹ | 1 | 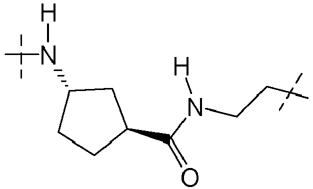 | 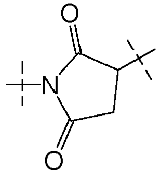 |
| R¹ | 1 | 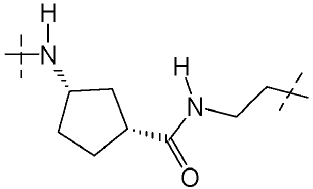 | 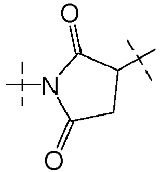 |
| R³ | 0 | 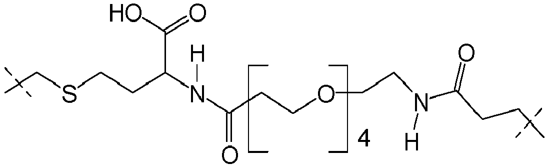 | 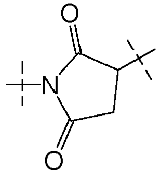 |
| R³ | 0 | 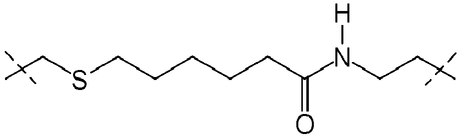 | 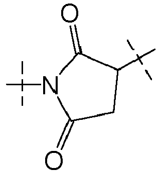 |
| R³ | 0 | 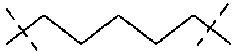 | 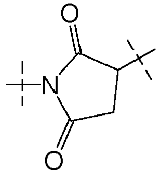 |
| R³ | 0 | 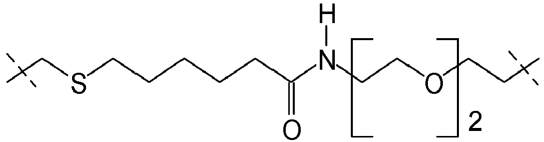 | 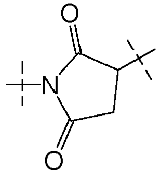 |
| R³ | 0 | 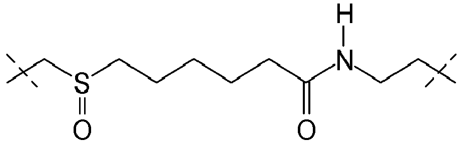 | 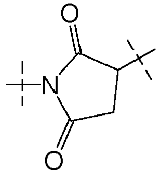 |
| R¹ | 1 | 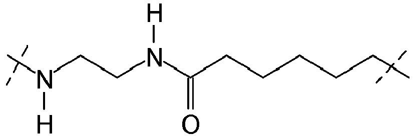 | 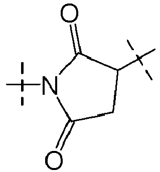 |
| R¹ | 1 | 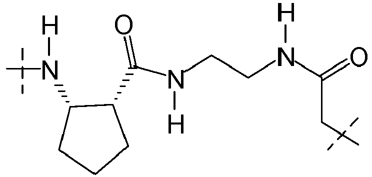 | 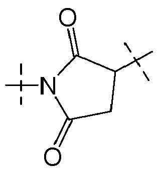 |
| | | | Siehe Anmerkung ** |
| R¹ | 1 | 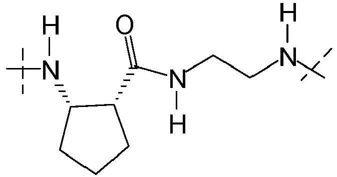 | 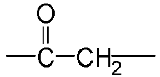 |
| R¹ | 1 | 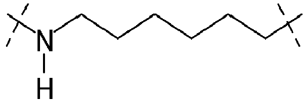 | 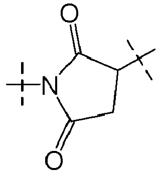 |
| R¹ | 1 | 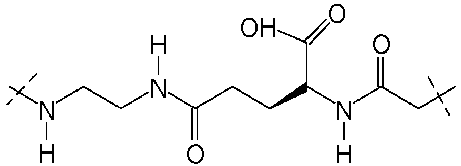 | 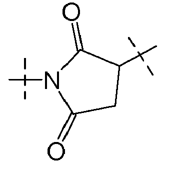 |
| | | | Siehe Anmerkung ** |
| R¹ | 1 | 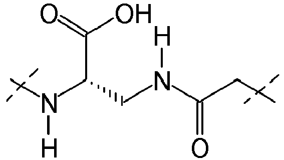 | 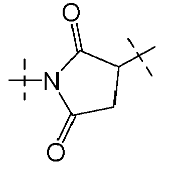 |
| | | | Siehe Anmerkung ** |
| R¹ | 1 | 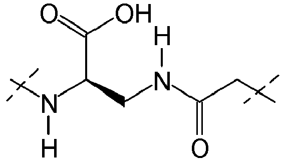 | 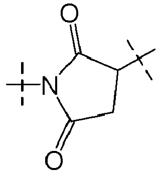 |
| | | | Siehe Anmerkung ** |
| R¹ | 0 | 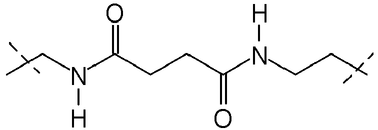 | 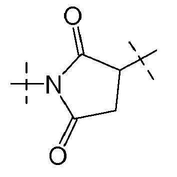 |
| R¹ | 1 | 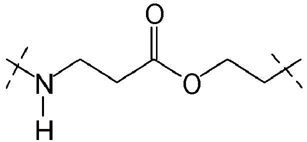 | 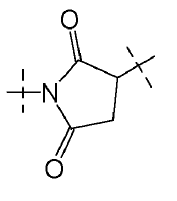 |
| R¹ | 1 | 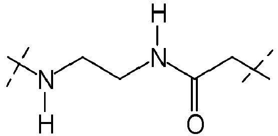 | 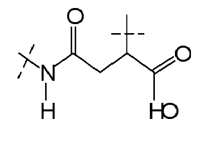 |
| | | | und |
| | | | 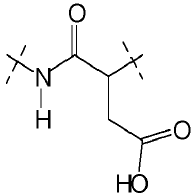 |
| | | | Siehe Anmerkung *** |
| R¹ | 1 | 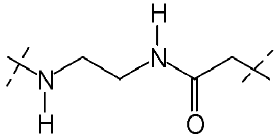 | 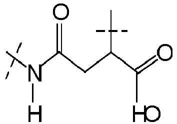 |
| R¹ | 1 | 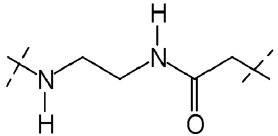 | 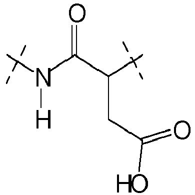 |
| R¹ | 1 | 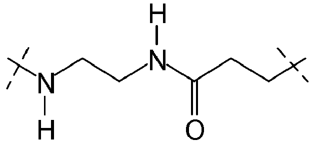 | 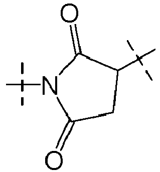 |
| R3 | 0 | 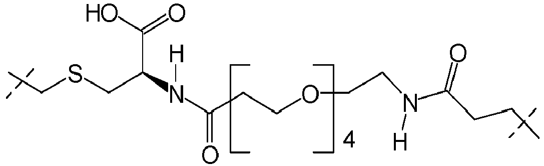 | 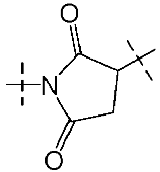 |
| R³ | 0 | 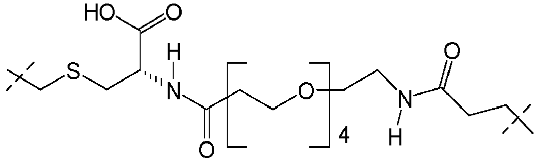 | 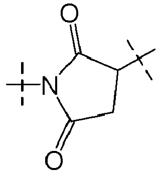 |
| R³ | 0 | 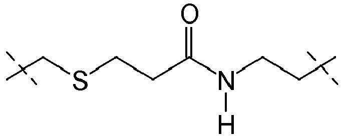 | 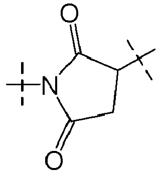 |
| R³ | 0 | 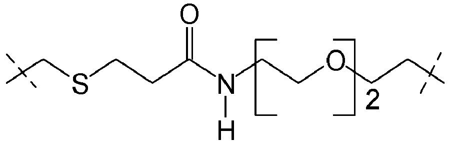 | 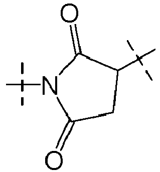 |
| R³ | 0 | 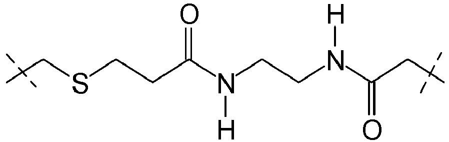 | 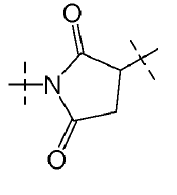 |
| | | | Siehe Anmerkung ** |
| R³ | 0 | 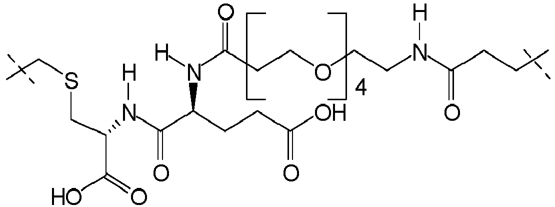 | 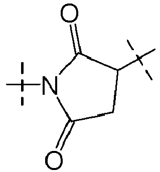 |
| R³ | 0 | 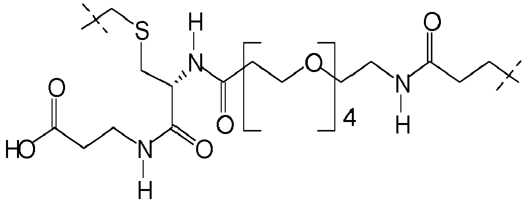 | 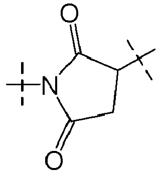 |
| R³ | 0 | 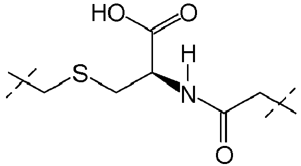 | 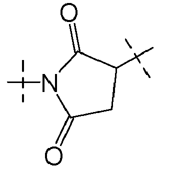 |
| | | | Siehe Anmerkung ** |
| R³ | 0 | 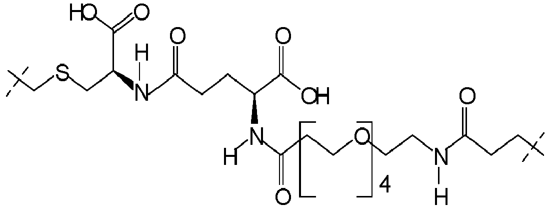 | 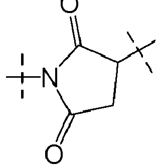 |
| R² | 0 | 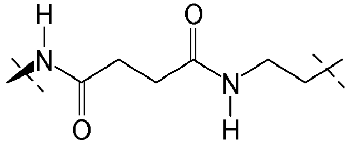 | 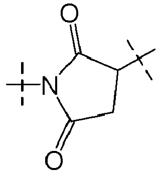 |
| R¹ | 1 | 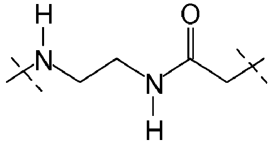 | 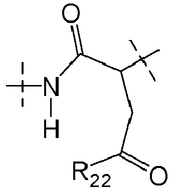 |
| | | | Mit R₂₂ = -OH oder _NH₂ |
| R¹ | 1 | 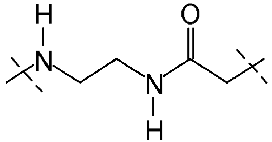 | 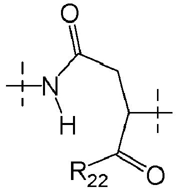 |
| | | | Mit R₂₂ = -OH oder _NH₂ |
| R¹ | 1 | 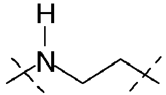 | 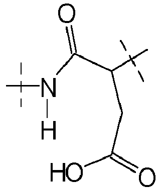 |
| | | | und |
| | | | 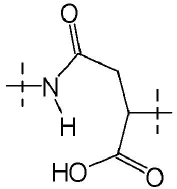 |
| | | | Siehe Anmerkung *** |
| R¹ | 1 | 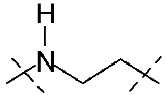 | 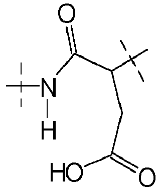 |
| R¹ | 1 | 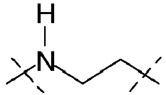 | 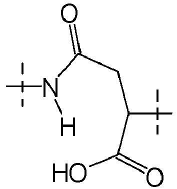 |
| R¹ | 1 | 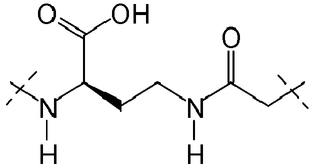 | 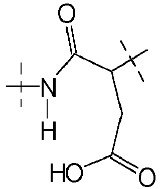 |
| | | | und |
| | | | 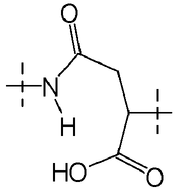 |
| | | | Siehe Anmerkung *** |
| R¹ | 1 | 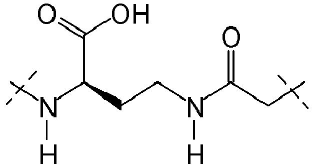 | 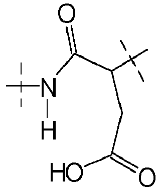 |
| R¹ | 1 | 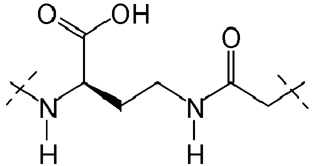 | 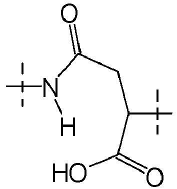 |
| R³ | 0 | 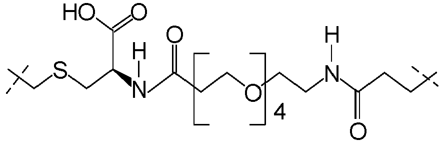 | 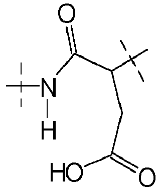 |
| | | | und |
| | | | 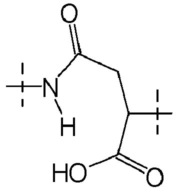 |
| | | | Siehe Anmerkung *** |
| R³ | 0 | 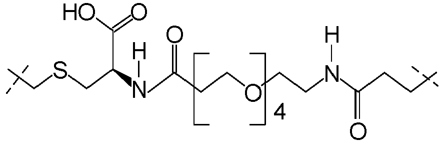 | 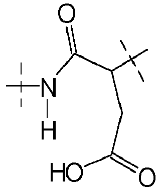 |
| R³ | 0 | 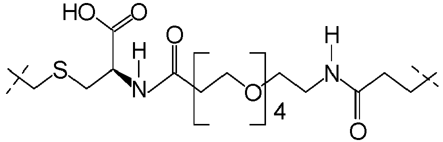 | 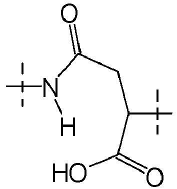 |
| R³ | 0 | 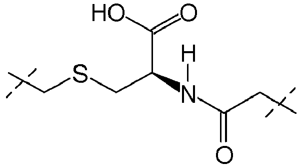 | 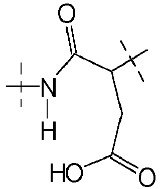 |
| | | | und |
| | | | 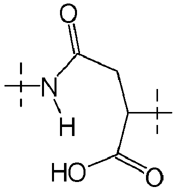 |
| | | | Siehe Anmerkung *** |
| R3 | 0 | 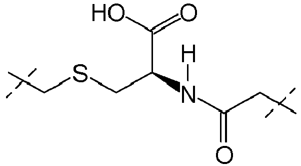 | 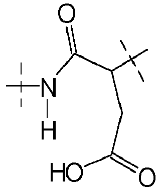 |
| R3 | 0 | 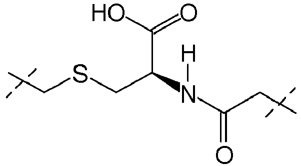 | 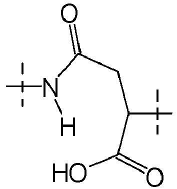 |
| R³ | 0 | 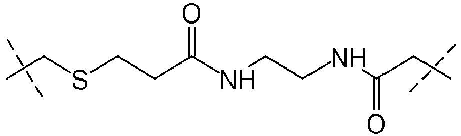 | 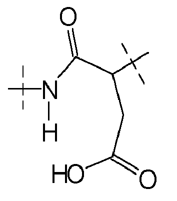 |
| | | | und |
| | | | 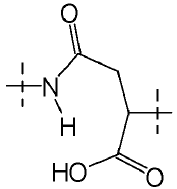 |
| | | | Siehe Anmerkung *** |
| R³ | 0 | 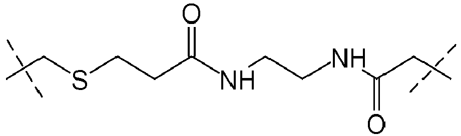 | 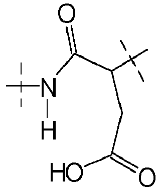 |
| R³ | 0 | 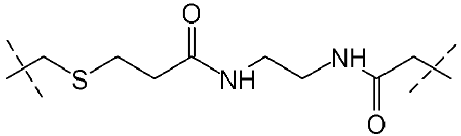 | 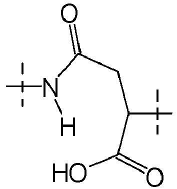 |
| R¹ | 1 | 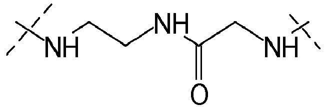 | 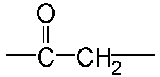 |
| R¹ | 1 | 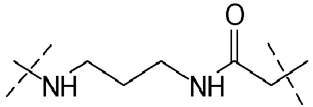 | 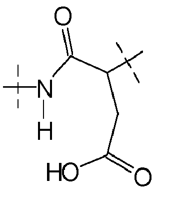 |
| | | | und |
| | | | 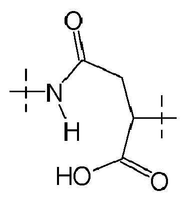 |
| | | | Siehe Anmerkung *** |
| R¹ | 1 | 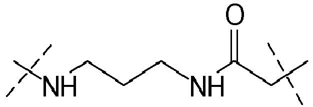 | 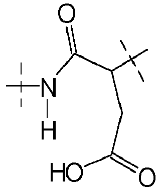 |
| R¹ | 1 | 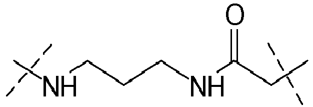 | 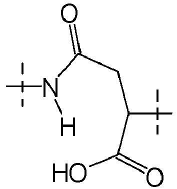 |
| R¹ | 1 | 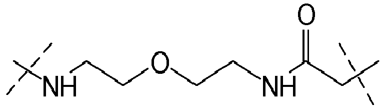 | 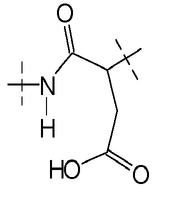 |
| | | | und |
| | | | 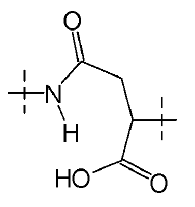 |
| | | | Siehe Anmerkung *** |
| R¹ | 1 | 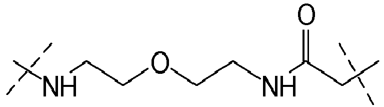 | 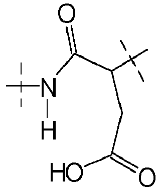 |
| R¹ | 1 | 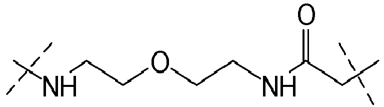 | 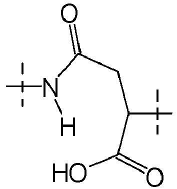 |
| R³ | 0 | 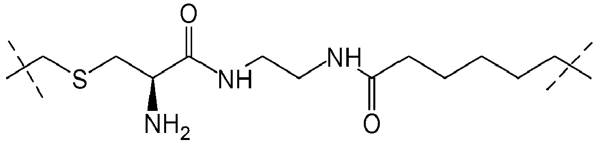 | 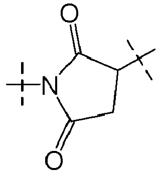 |
| R¹ | 0 | 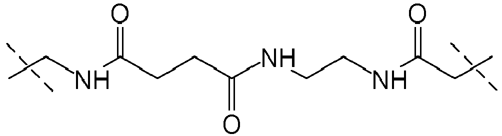 | 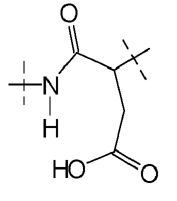 |
| | | | und |
| | | | 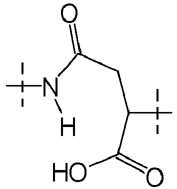 |
| | | | Siehe Anmerkung *** |
| R¹ | 0 | 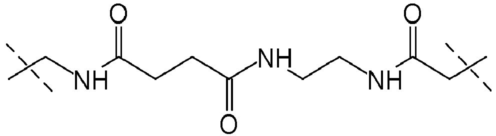 | 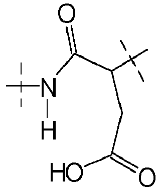 |
| R¹ | 0 | 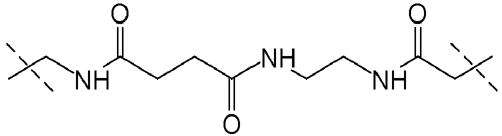 | 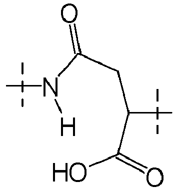 |
| R¹ | 1 | 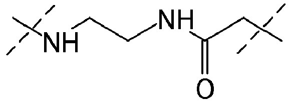 | 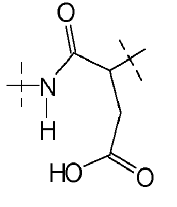 |
| | | | und |
| | | | 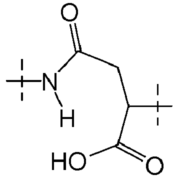 |
| R¹ | 1 | 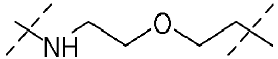 | 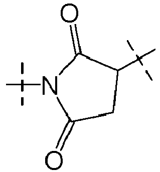 |
| R¹ | 1 | 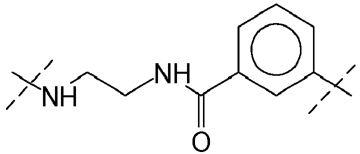 | 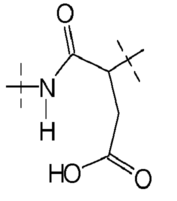 |
| | | | und |
| | | | 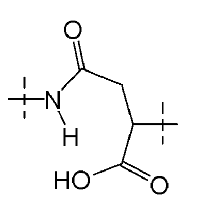 |
| R¹ | 1 | 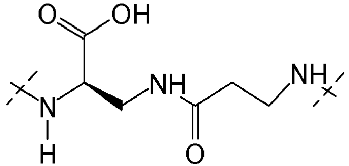 | 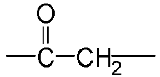 |
| R¹ | 1 | 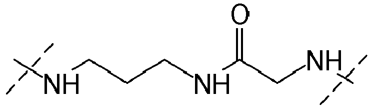 | 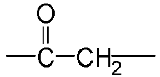 |
| R¹ | 1 | 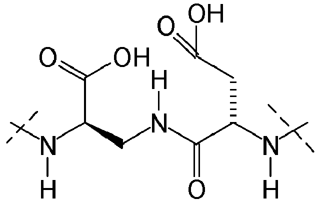 | 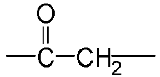 |
| R¹ | 1 | 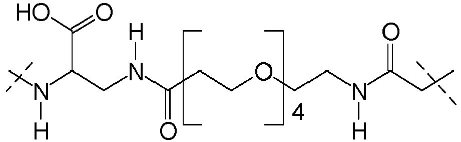 | 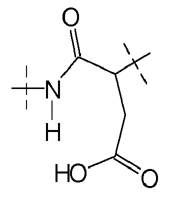 |
| | | | und |
| | | | 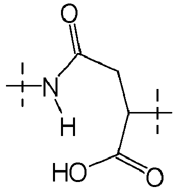 |
| | | | Siehe Anmerkung *** |
| R¹ | 1 | 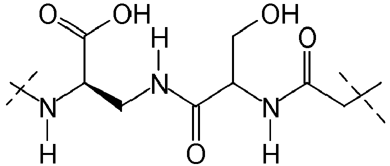 | 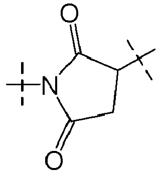 |
| | | | Siehe Anmerkung ** |
| R¹ | 1 | 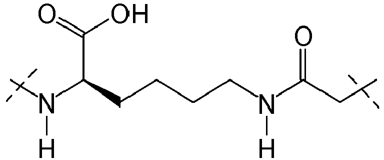 | 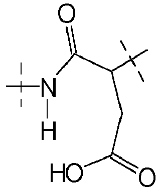 |
| | | | und |
| | | | 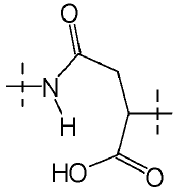 |
| | | | Siehe Anmerkung *** |
| R¹ | 1 | 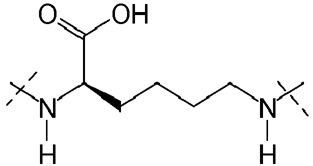 | 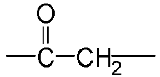 |
| R⁴ | 0 | 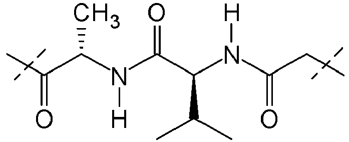 | 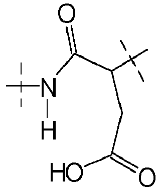 |
| | | | und |
| | | | 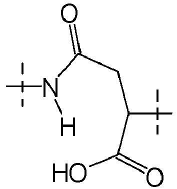 |
| | | | Siehe Anmerkung *** |
| R¹ | 1 | 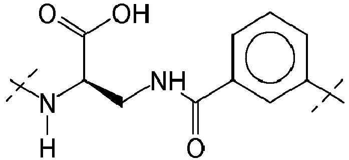 | 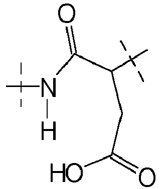 |
| | | | und |
| | | | 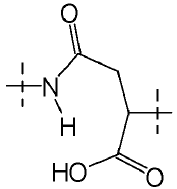 |
| | | | Siehe Anmerkung *** |
| R⁴ | 0 | 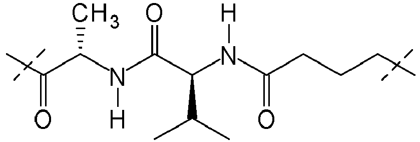 | 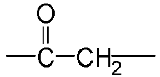 |
| R¹ | 1 | 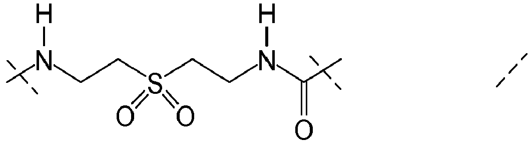 | 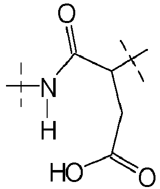 |
| | | | und |
| | | | 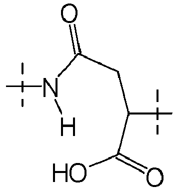 |
| | | | Siehe Anmerkung* * |
| R³ | 0 | 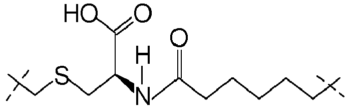 | 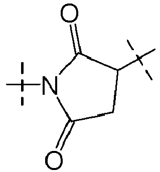 |
| | | | Siehe Anmerkung ** |
| R¹ | 1 | 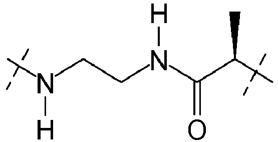 | 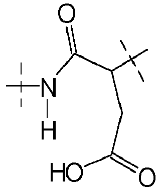 |
| | | | und |
| | | | 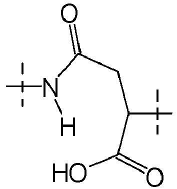 |
| | | | Siehe Anmerkung* * |
| R3 | 0 | 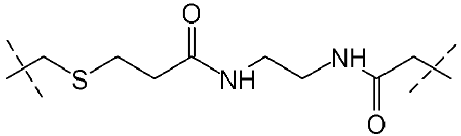 | 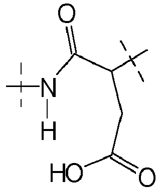 |
| | | | und |
| | | | 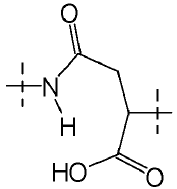 |
| | | | Siehe Anmerkung*** |
| R³ | 0 | 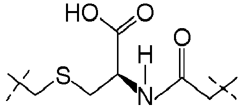 | 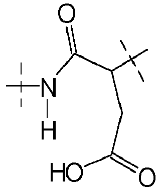 |
| | | | und |
| | | | 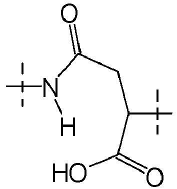 |
| | | | Siehe |
| | | | Anmerkung *** |
| R³ | 0 | 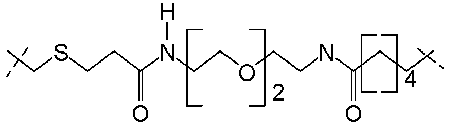 | 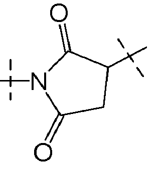 |
| R³ | 0 | 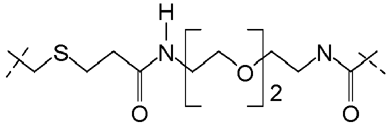 | 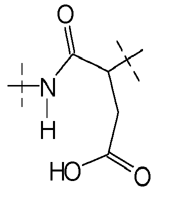 |
| | | | und |
| | | | 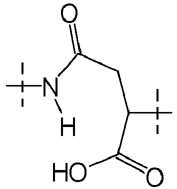 |
| | | | Siehe Anmerkung *** |
| R³ | 0 | 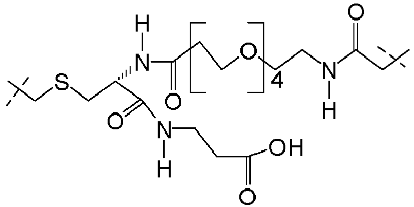 | 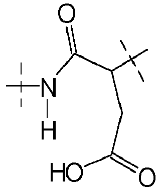 |
| | | | und |
| | | | 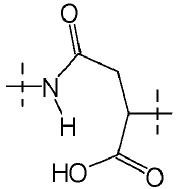 |
| | | | Siehe Anmerkung *** |
| R³ | 0 | 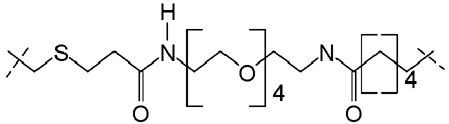 | 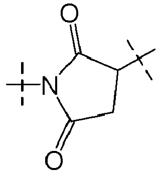 |
| | | | Siehe Anmerkung ** |
| R³ | 0 | 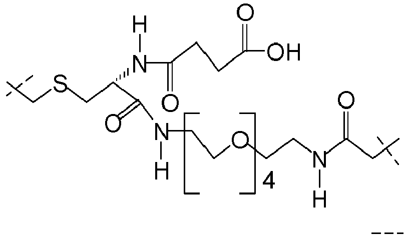 | 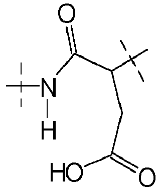 |
| | | | und |
| | | | 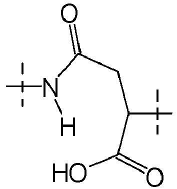 |
| | | | Siehe Anmerkung *** |
| R³ | 0 | 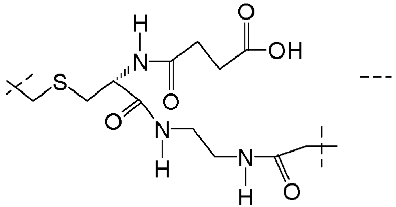 | 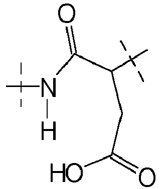 |
| | | | und |
| | | | 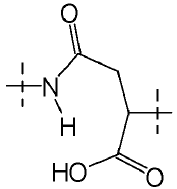 |
| | | | Siehe Anmerkung *** |
| R³ | 0 | 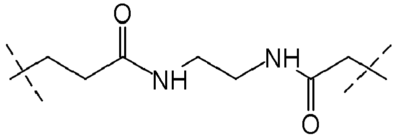 | 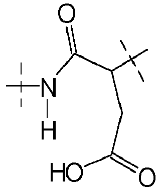 |
| | | | und |
| | | | 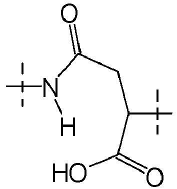 |
| | | | Siehe Anmerkung*** |
| R³ | 0 | 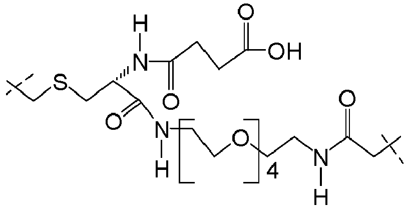 | 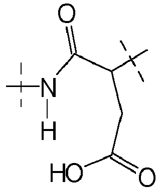 |
| | | | und |
| | | | 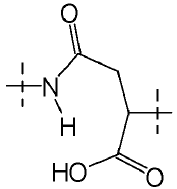 |
| | | | Siehe Anmerkung *** |
| R³ | 0 | 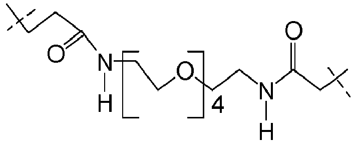 | 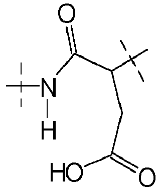 |
| | | | und |
| | | | 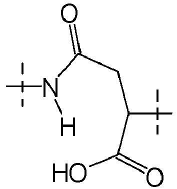 |
| | | | Siehe Anmerkung *** |
| R³ | 0 | 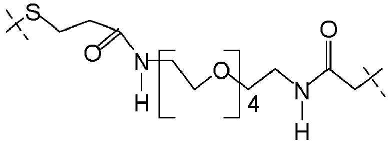 | 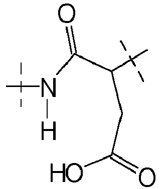 |
| | | | und |
| | | | 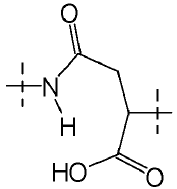 |
| | | | Siehe Anmerkung *** |
| R³ | 0 | 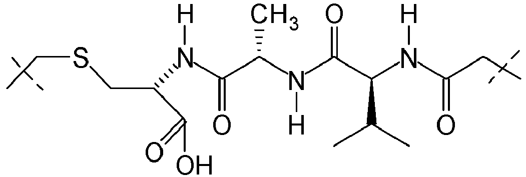 | 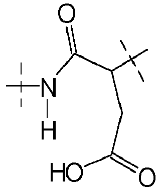 |
| | | | und |
| | | | 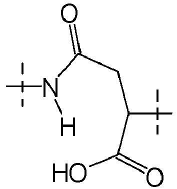 |
| | | | Siehe Anmerkung *** |
| R³ | 0 | 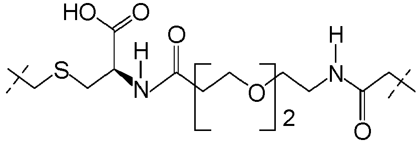 | 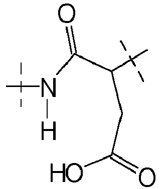 |
| | | | und |
| | | | 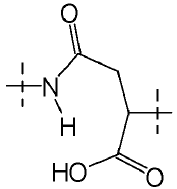 |
| | | | Siehe Anmerkung *** |
| R³ | 0 | 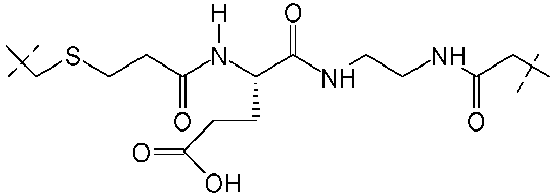 | 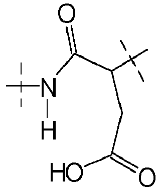 |
| | | | und |
| | | | 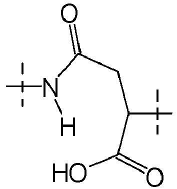 |
| | | | Siehe Anmerkung*** |
| R³ | 0 | 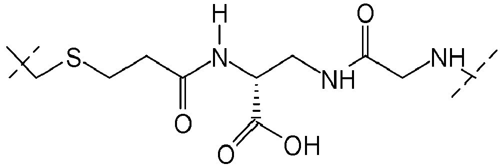 | 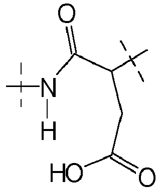 |
| | | | und |
| | | | 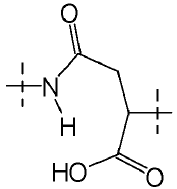 Siehe Anmerkung*** |
| R³ | 0 | 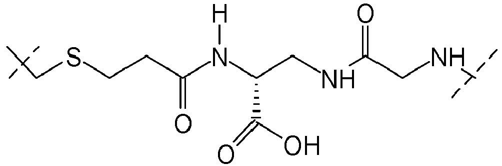 | 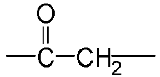 |

| | | | |
|---|---|---|---|
| **: Siehe Anmerkung ** zu Tabelle A. ***: Bei Vorhandensein dieser Struktur L2 kann zugleich eine Struktur L2 der folgenden Formel vorliegen: | | | |

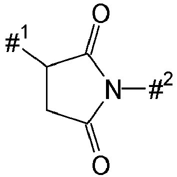

Beispiele für Konjugate mit entsprechenden Linkern weisen folgende Strukturen auf, wobei X1 CH, X2 C, und X3 N darstellt, und L1 die oben angegebenen Bedeutung aufweist, L2 und L3 dieselbe Bedeutung wie L1 hat, AK1 für einen -Antikörper steht, der über einen Cysteinrest gebunden ist, und n eine Zahl von 1 bis 10 ist. Besonders bevorzugt ist AK1 ein humaner, humanisierter oder chimärer monoklonaler Antikörper. Besonders bevorzugt ist ein aglycosylierter anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2658.

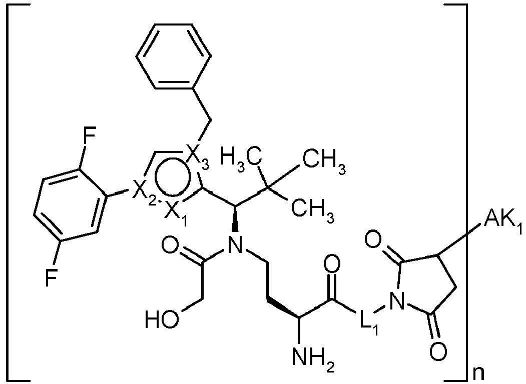

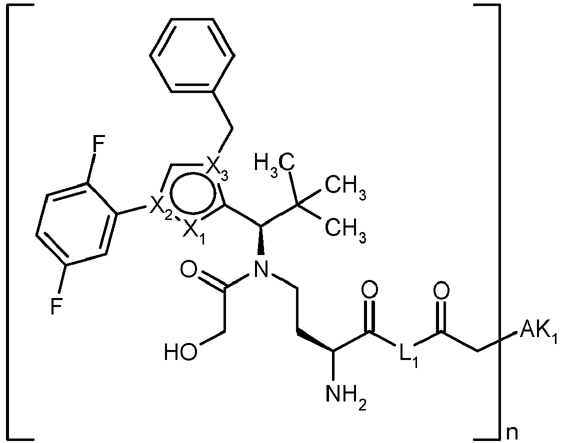

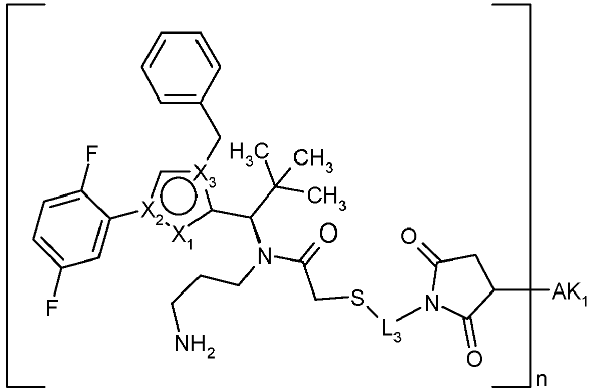

Wenn der Linker an eine Lysin-Seitenkette bzw. einen Lysinrest gebunden ist, können die selben Linker wie oben beschrieben für eine Ankopplung an eine Cystein-Seitenkette verwendet werden, wobei jedoch L2 vorzugsweise eine Carbonylgruppe ist (die Ankopplung erfolgt z.B. über eine entsprechende aktivierte Carbonsäure).

Beispiele für Konjugate mit der Grundstruktur (i) weisen eine der folgenden Strukturen auf, wobei X1 CH, X2 C, und X3 N darstellt, L4 dieselbe Bedeutung wie L1 hat, AK1 für einen aglycosylierten anti-TWEAKR-Antikörper steht, der über einen Cysteinrest gebunden ist, und n eine Zahl von 1 bis 10 ist, und das Wasserstoffatom in Position R⁴ gemäß Formel IIa (d.h. in der Gruppe -NH₂) durch eine durch Legumain spaltbare Gruppe der Formel R²¹-CO-P3-P2-NH-CH(CH₂CONH₂)-CO- ersetzt ist:. 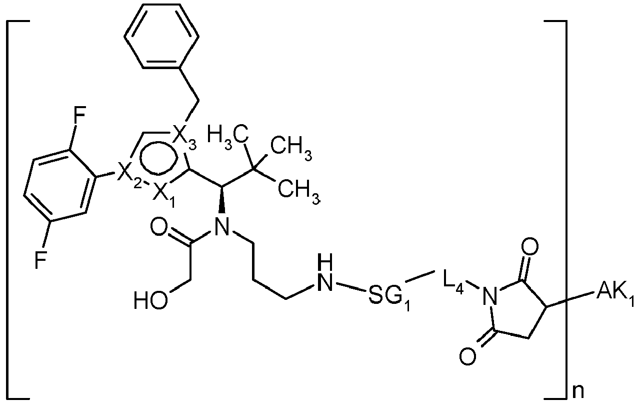 wobei R²¹ eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -SO₃H, -COOH, -SH, oder -OH substituiert sein kann: P2 eine Einfachbindung oder eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist;

P3 eine Einfachbindung oder eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist.

Besonders bevorzugt ist der anti-TWEAKR-Antikörper, der spezifisch an die Aminosäure D in Position 47 (D47) von TWEAKR (SEQ ID NO: 169) bindet, insbesondere der aglycosylierte anti-TWEAKR-Antikörper TPP-2658.

Im Falle der Transglutaminase-katalysierten Konjugation offenbart die Literatur verschiedene Möglichkeiten der kovalenten Kopplung (Konjugation) von organischen Molekülen an Binder wie z.B. Antikörper in einer konjugationsstellenspezifischen Art und Weise, (siehe zum Beispiel Sochaj et al., Biotechnology Advances, 33, 775-784, (2015), Panowski et al., MAbs 6, 34-45 (2014)). Erfindungsgemäß bevorzugt ist die Konjugation der KSP-Inhibitoren bzw. Prodrugs an einen Antikörper über Akzeptor-Glutaminreste des Antikörpers unter Verwendung von Transglutaminase. Solche Akzeptorglutaminreste können über Engineering des Antikörpers oder durch Mutationen generiert werden, die aglykosylierte Antikörper erzeugen. Die Anzahl dieser Akzeptorglutamine im Antikörper ist bevorzugt 2 oder 4. Für die Kopplung (Konjugation) werden geeignete Linker verwendet. Geeignete Linkerstrukturen sind solche, die über eine freie Amin-Donor-Funktionalität verfügen, die ein geeignetes Substrat für die Transglutaminase darstellt. Die Verknüpfung des Linkers an den Antikörper kann dabei auf vielfältige Weise erfolgen.

Vorzugsweise weist bei einer Transglutaminase-katalysierten Konjugation der Linker ein der obigen Grundstrukturen (i) bis (iv) auf, wobei L1, SG, SG1 und m die oben genannten Bedeutungen haben, L2 jedoch vorzugsweise eine der folgenden Gruppen darstellt: 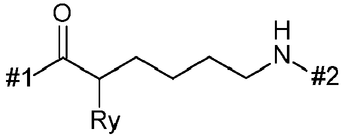
wobei Ry -H, NHCOalkyl, -NH₂ ist
   oder 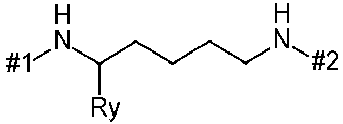
wobei Ry -CONHalkyl, -CONH₂,
   wobei
#¹ den Verknüpfungspunkt mit L¹darstellt,
#² den Verknüpfungspunkt mit dem Glutaminrest des Binders darstellt.

### Vorzugsweise ist Ry H oder -NHCOMe.

Beispiele entsprechender Konjugate weisen die folgenden Strukturen auf, wobei X1, X2, X3, Ry und L1 dieselbe Bedeutung wie oben aufweisen, AK einen Binder darstellt, vorzugsweise einen Antikörper, wobei n vorzugsweise 2 oder 4 ist.,

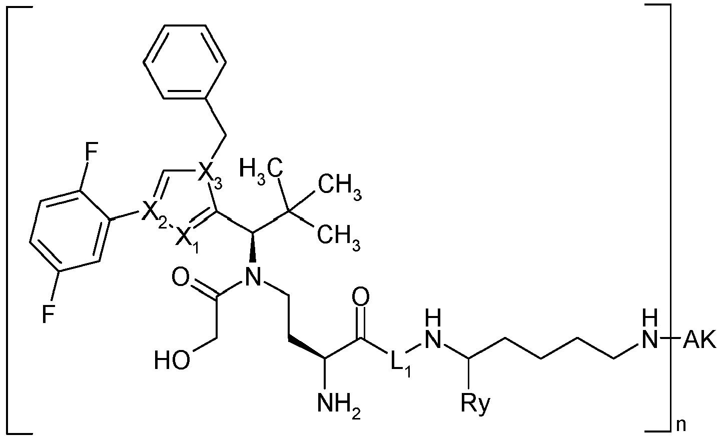

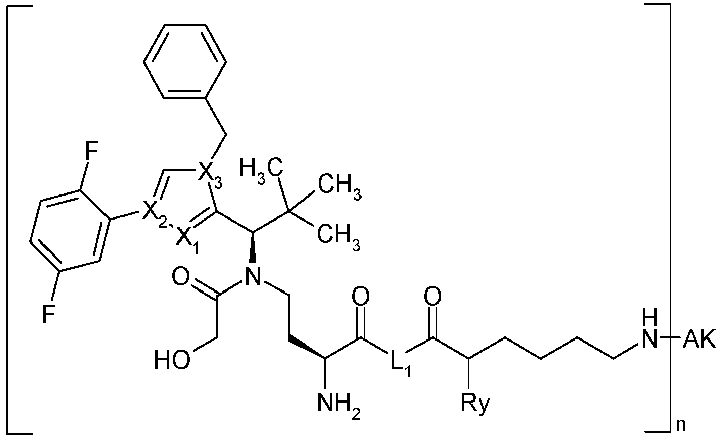

### Besonders bevorzugte KSP-Inhibitor-Konjugate

Besonders bevorzugt sind erfindungsgemäß die folgenden KSP-Inhibitor-Konjugate, wobei AK (AK₁; AK₂; AK₃) für Binder bzw. ein Derivat hiervon (vorzugsweise für einen Antikörper), und n für eine Zahl von 1 bis 50, vorzugsweise 1,2 bis 20 und besonders bevorzugt 2 bis 8 steht. AK₁ steht vorzugsweise für einen Antikörper, der über einen Cysteinrest an den KSP-Inhibitor gebunden ist; AK₂; steht vorzugsweise für einen Antikörper, der über einen Lysinrest an den KSP-Inhibitor gebunden ist AK₃ steht vorzugsweise für einen Antikörper, der über einen Glutaminrest an den KSP-Inhibitor gebunden ist. Als Binder oder Antikörper werden hierbei vorzugswiese die in der Beschreibung als bevorzugt beschriebenen Binder bzw. Antikörper verwendet.

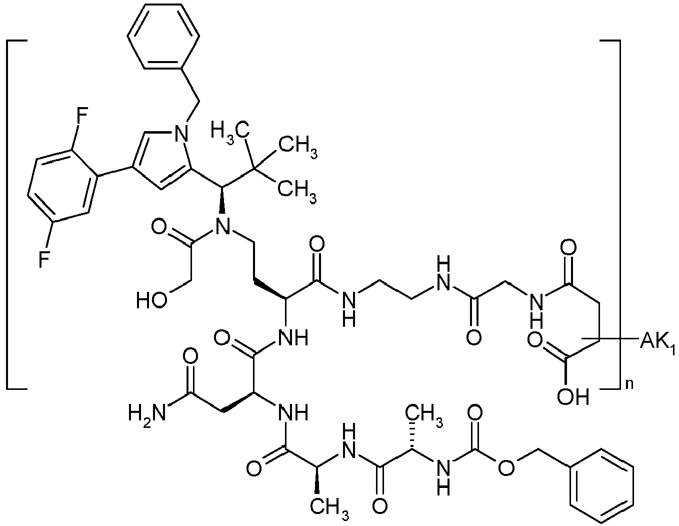

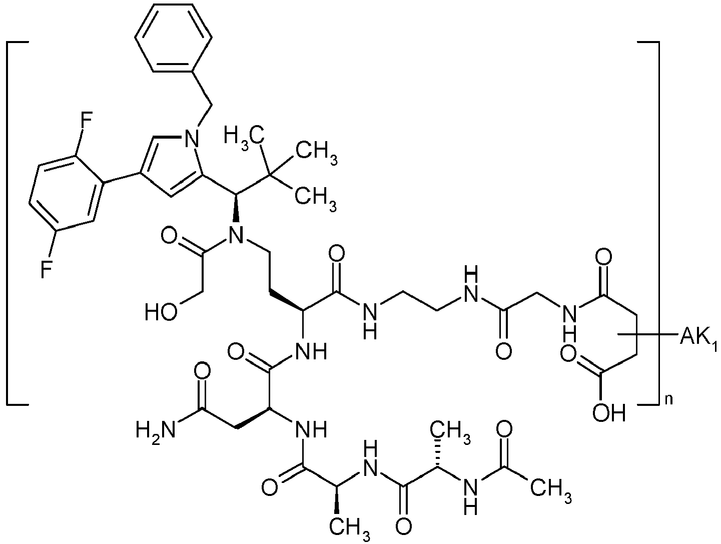

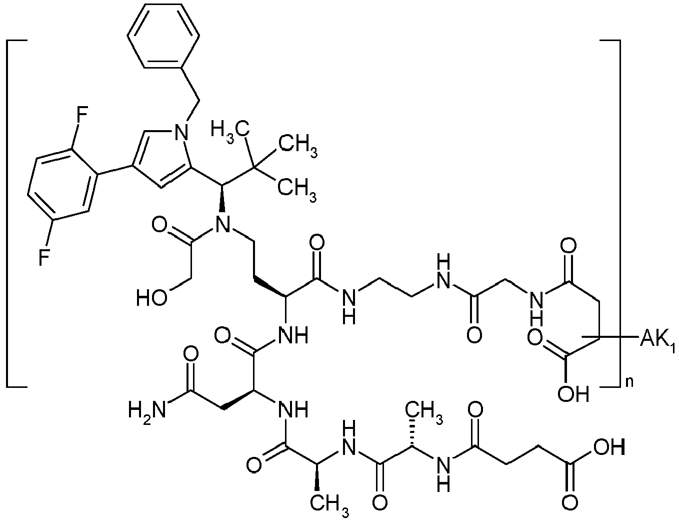

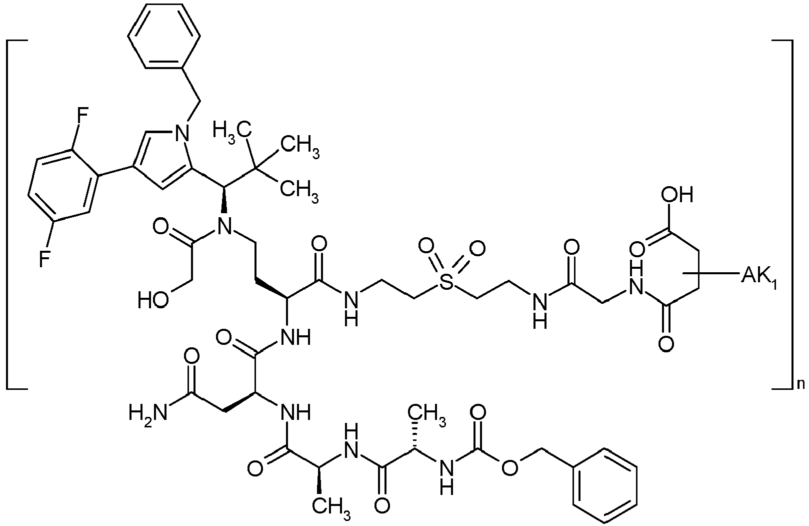

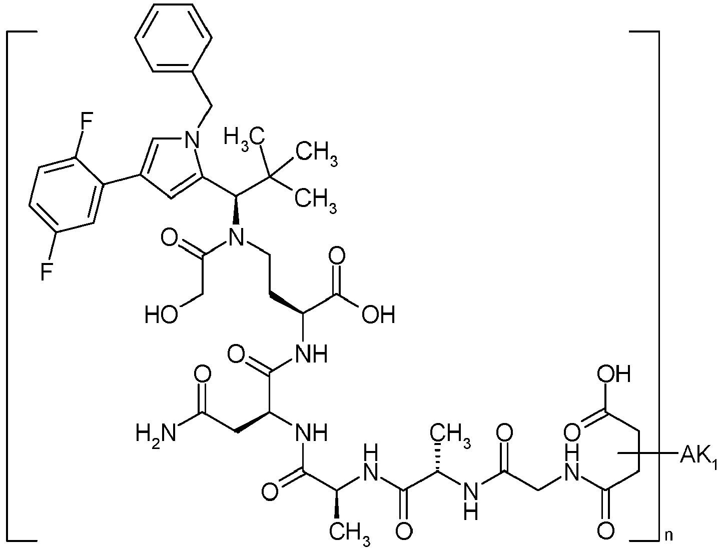

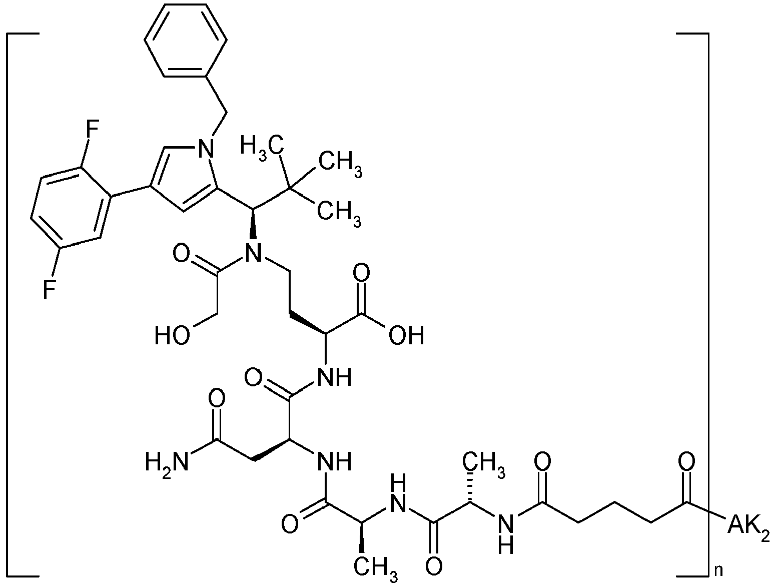

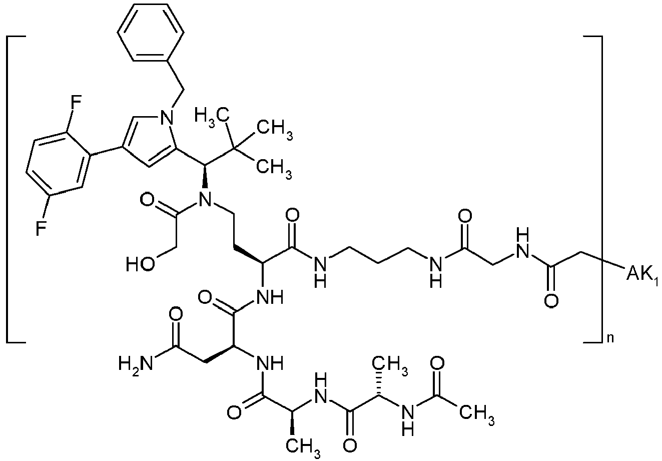

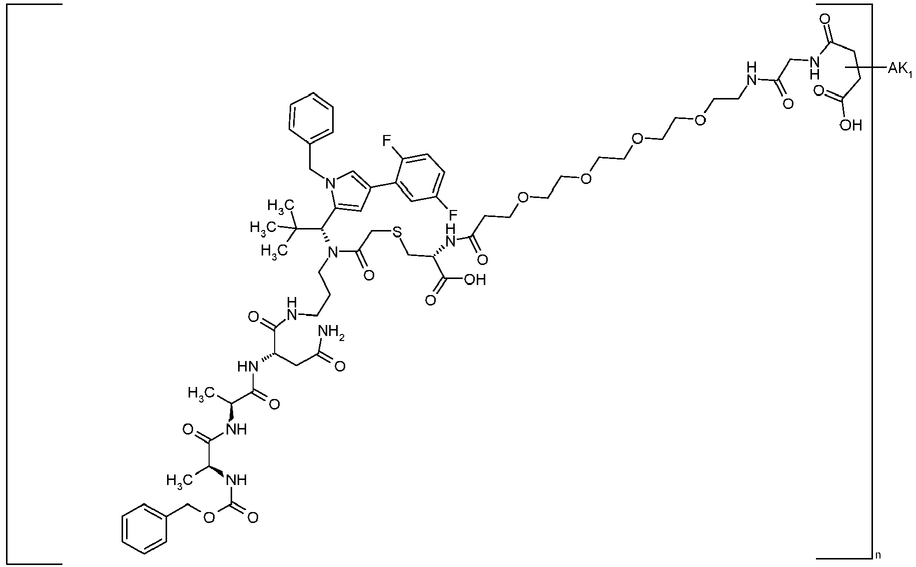

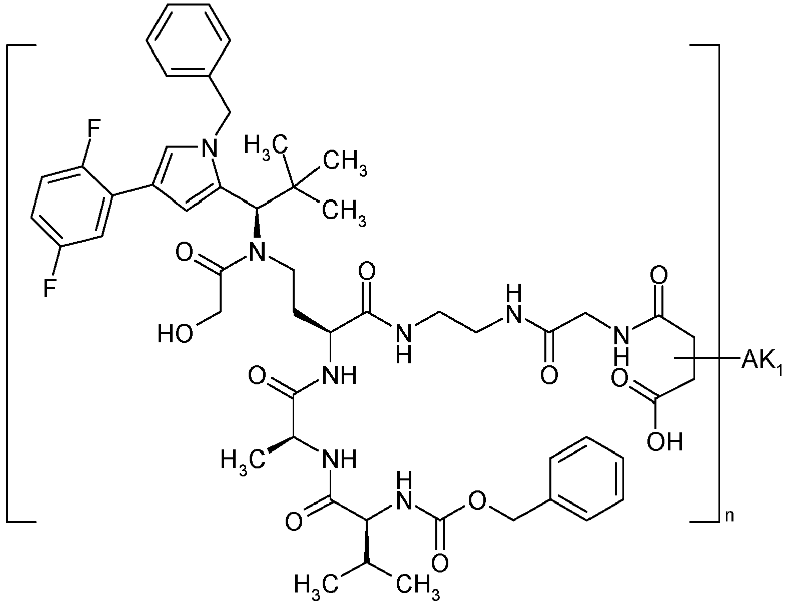

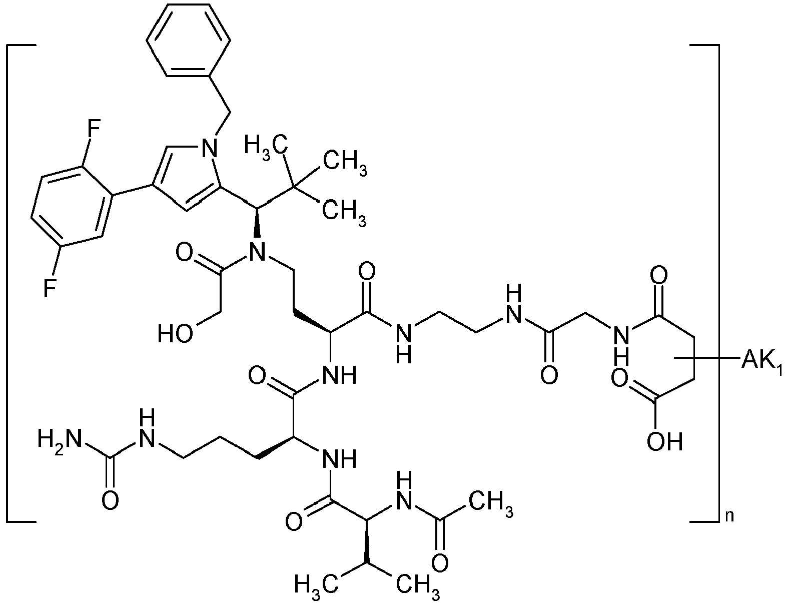

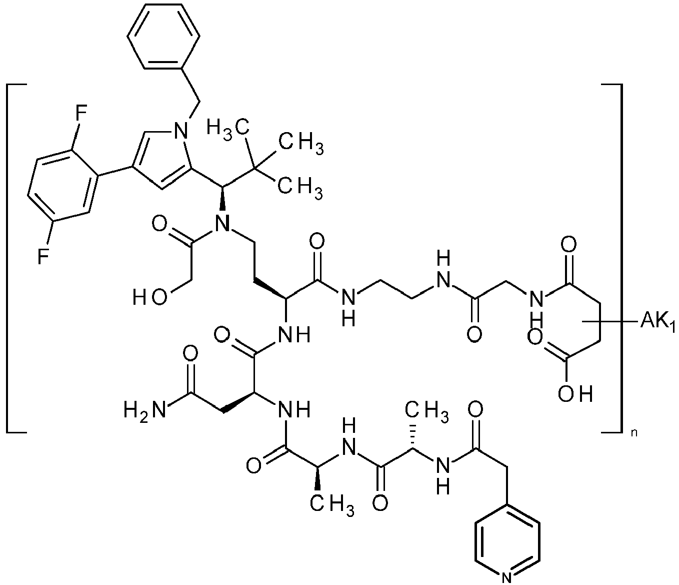

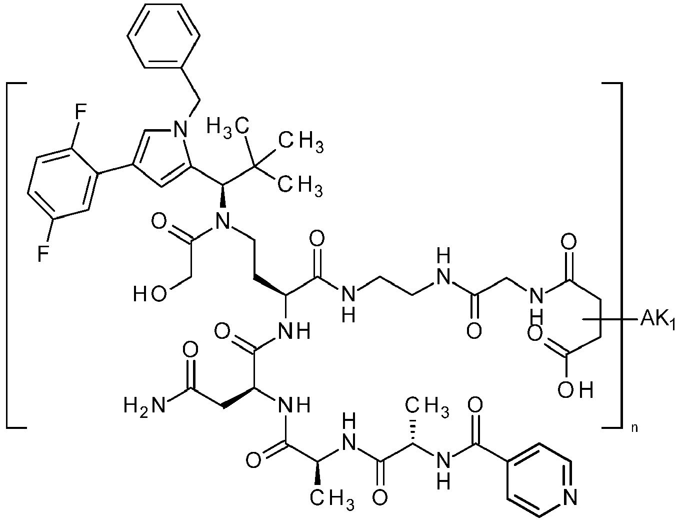

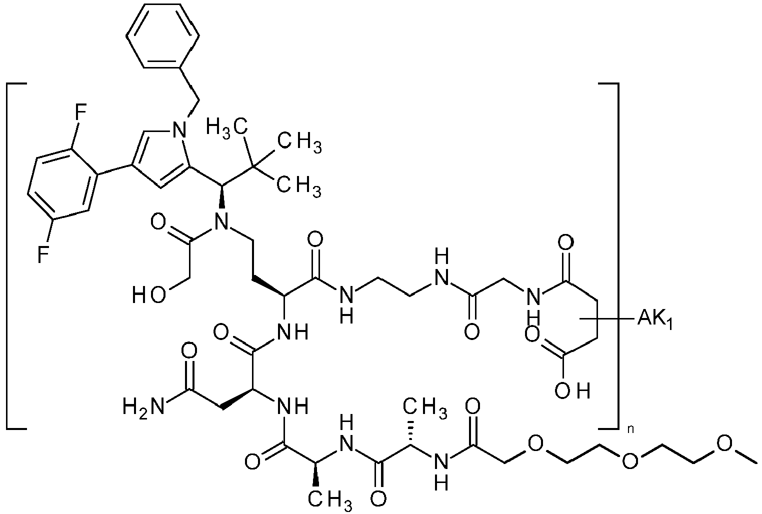

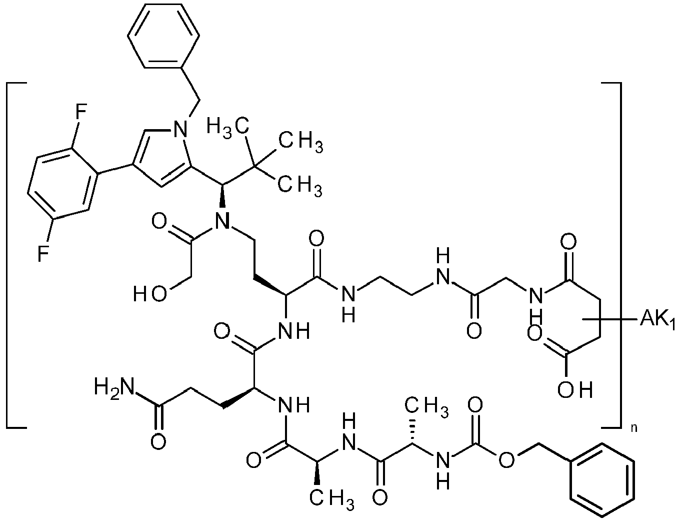

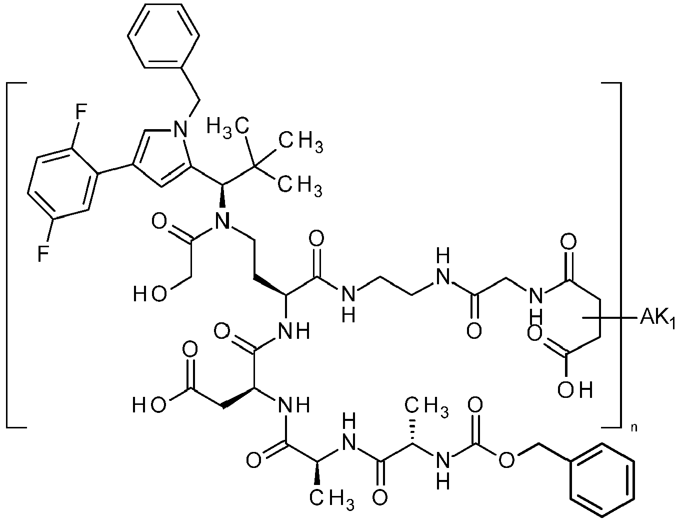

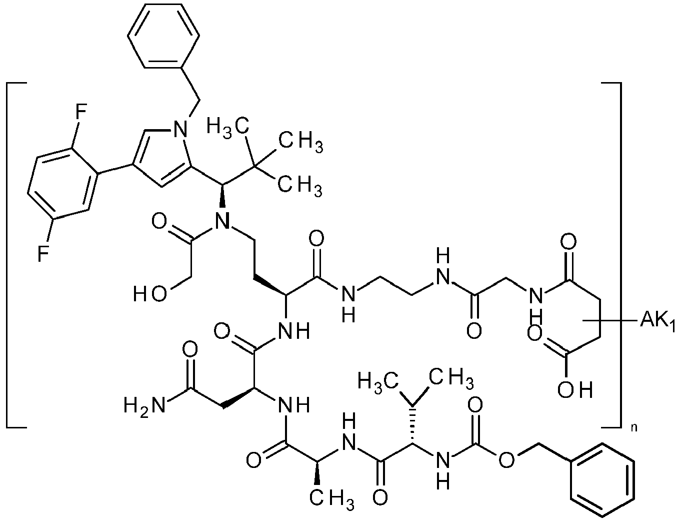

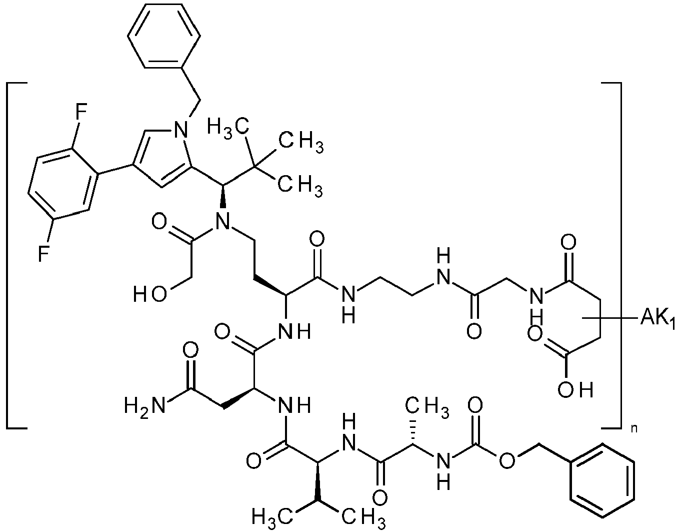

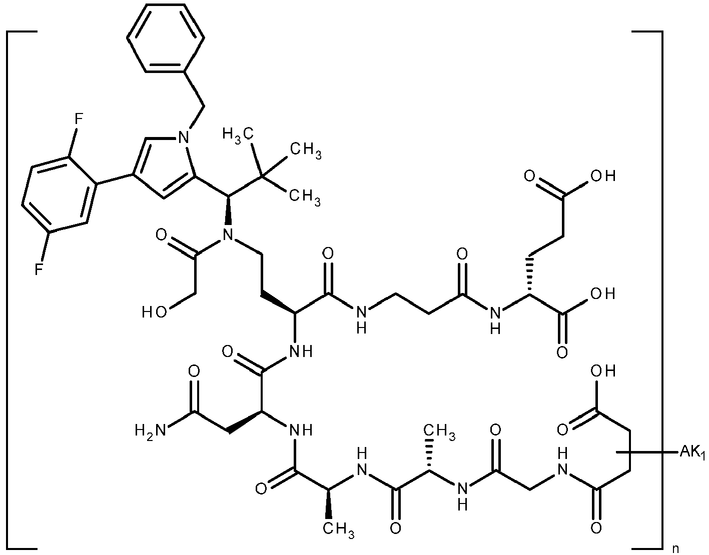

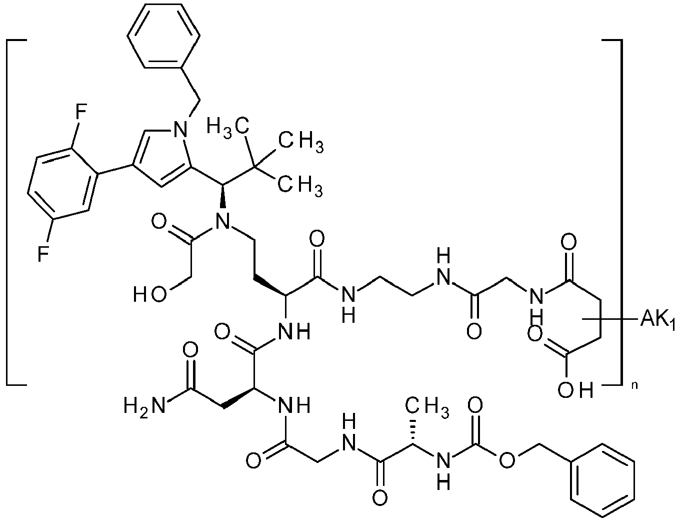

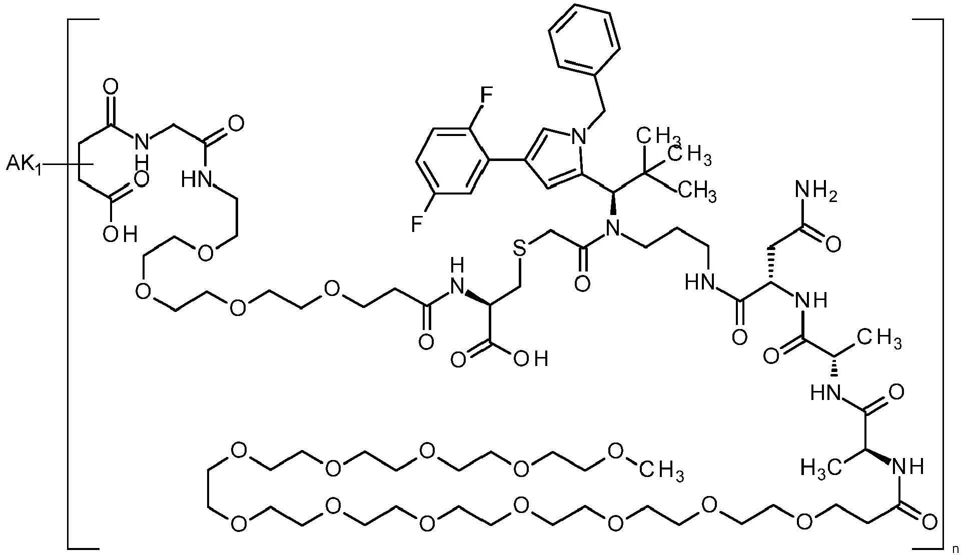

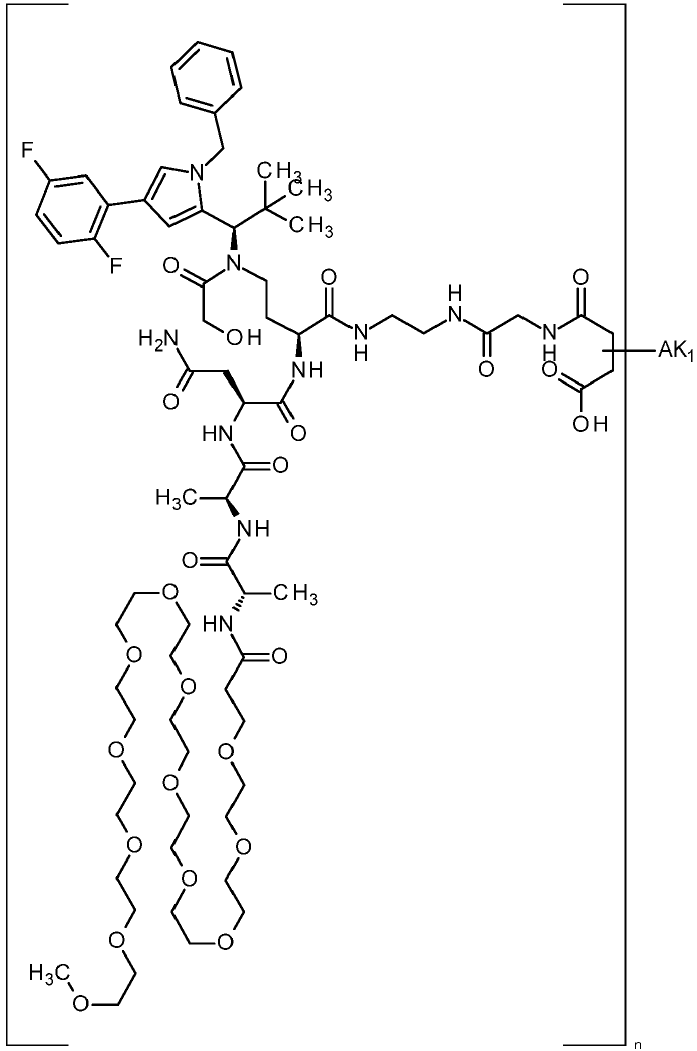

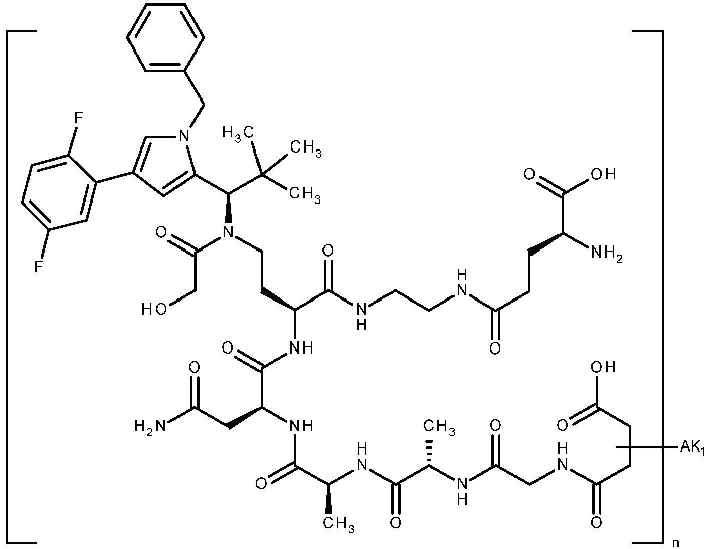

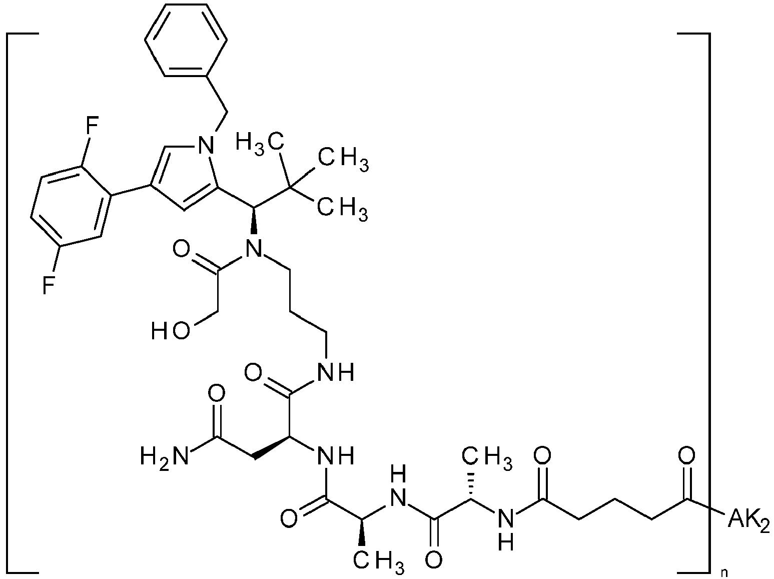

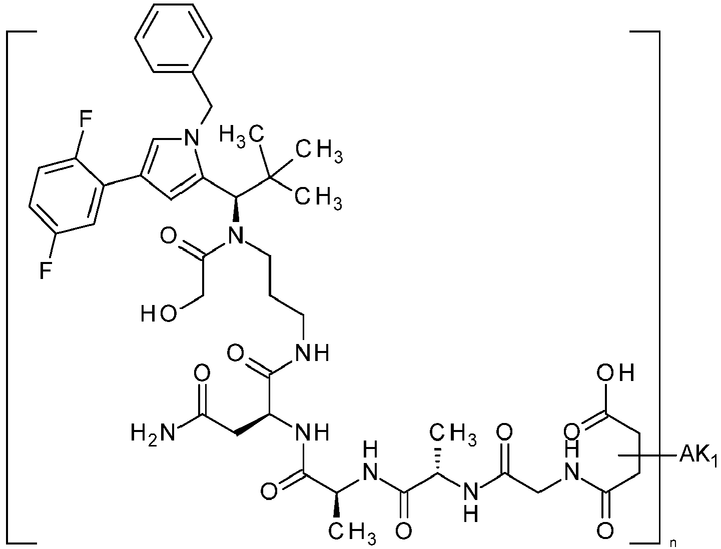

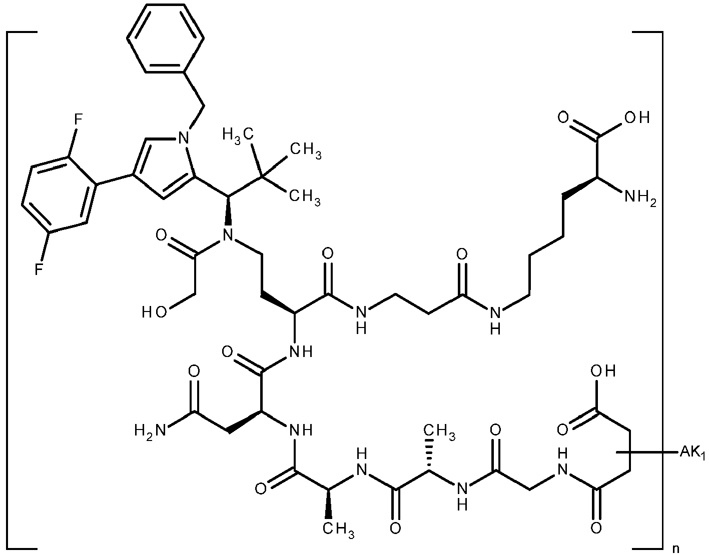

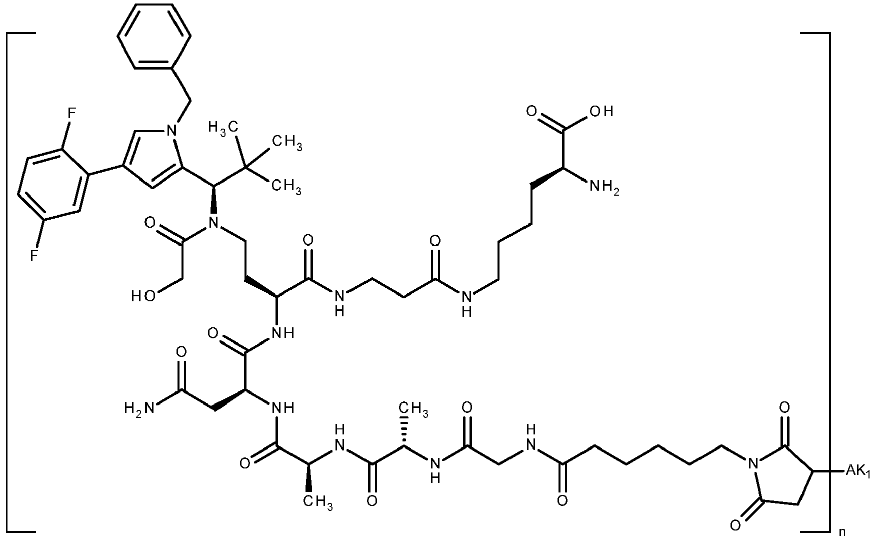

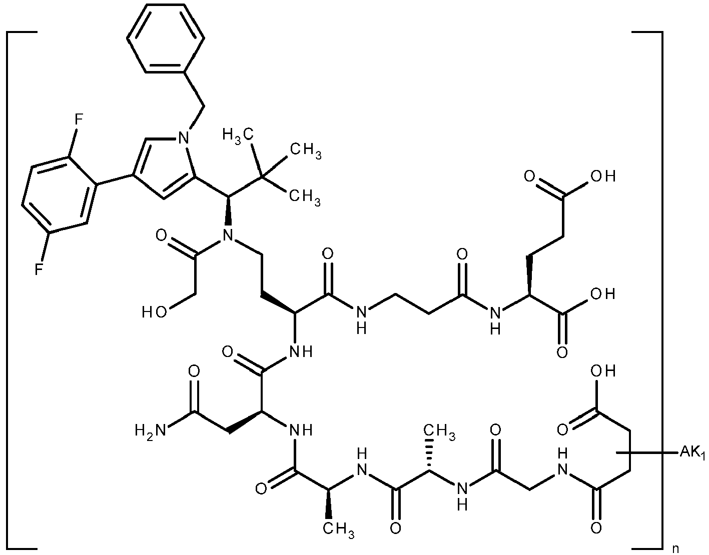

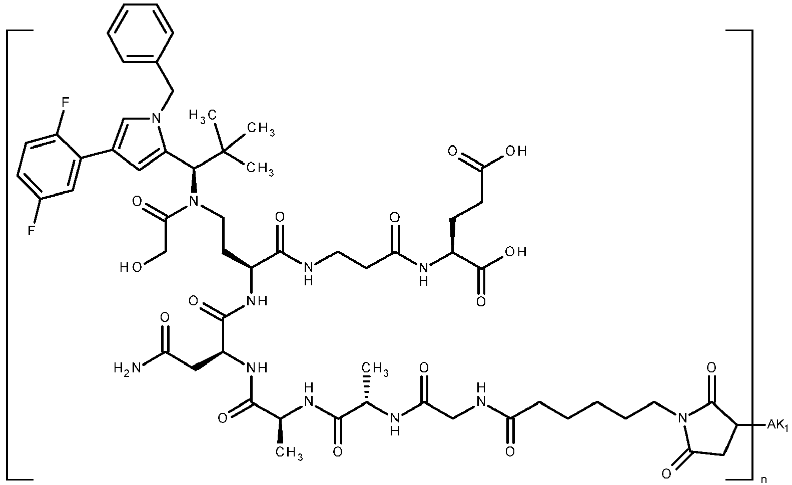

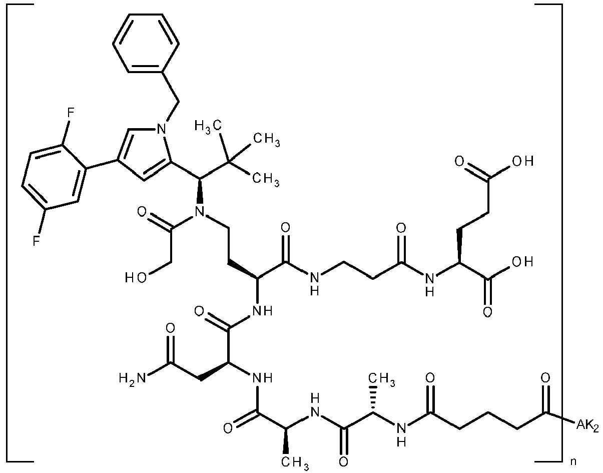

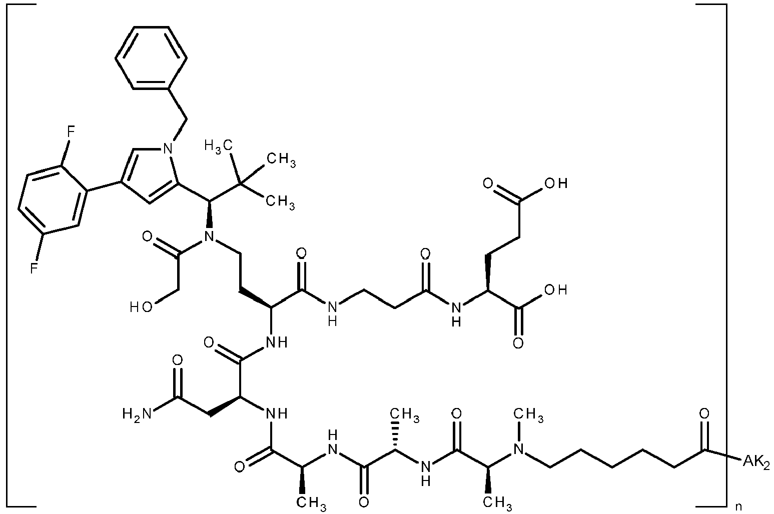

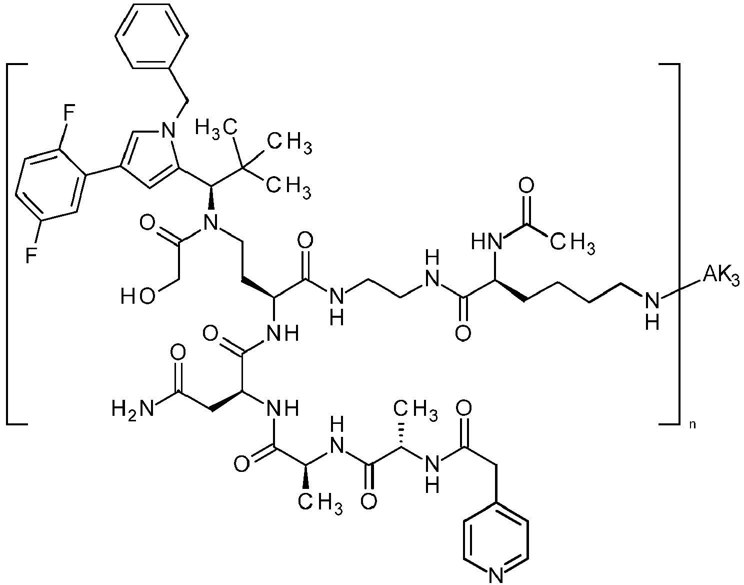

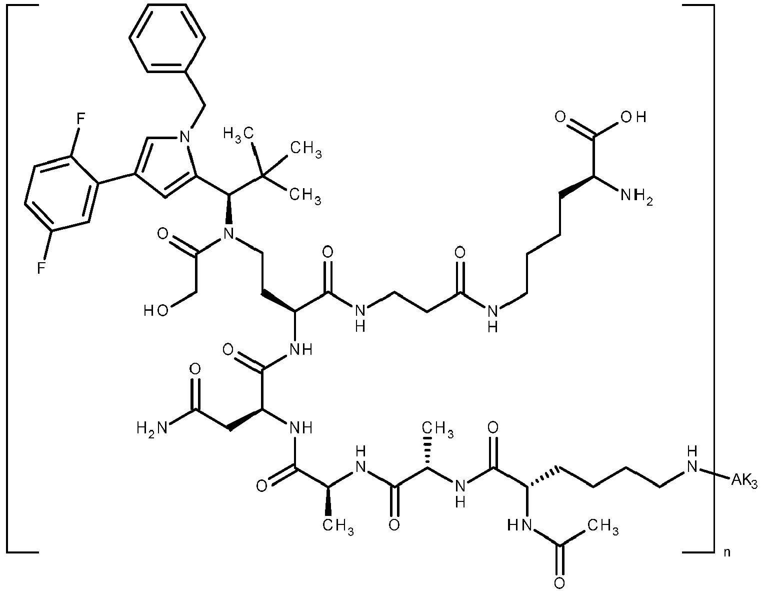

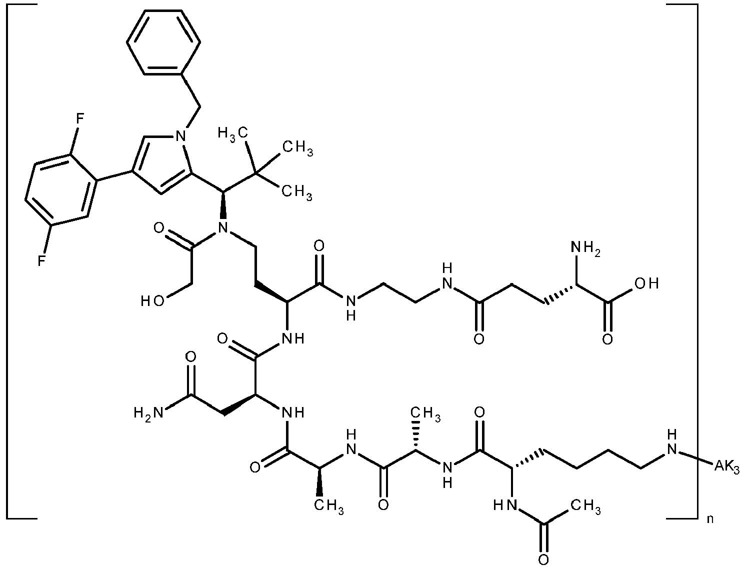

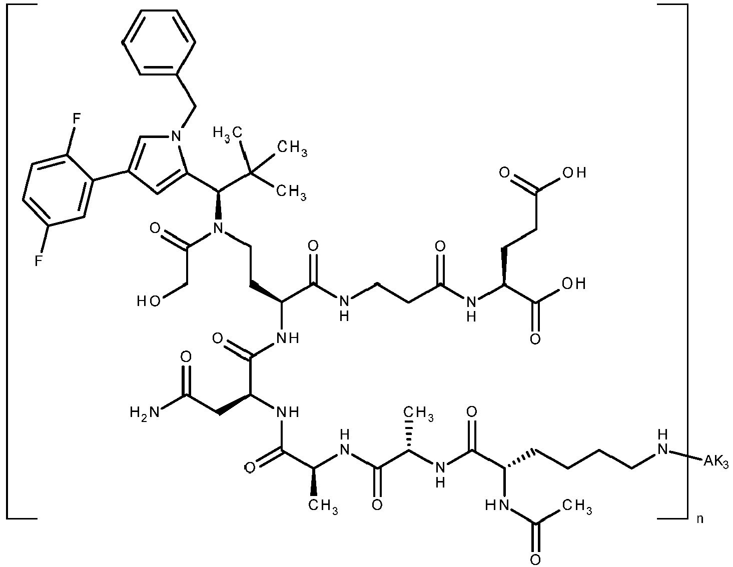

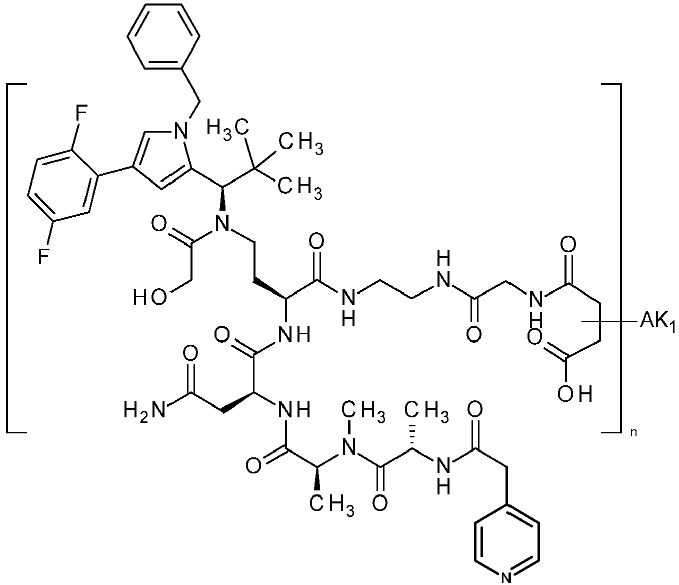

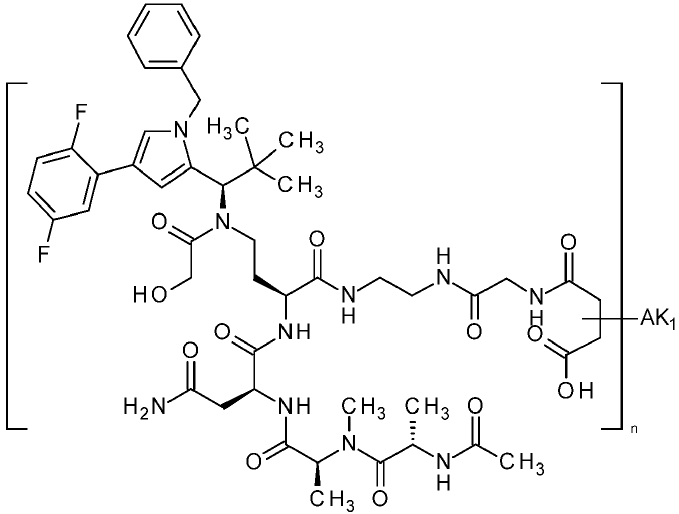

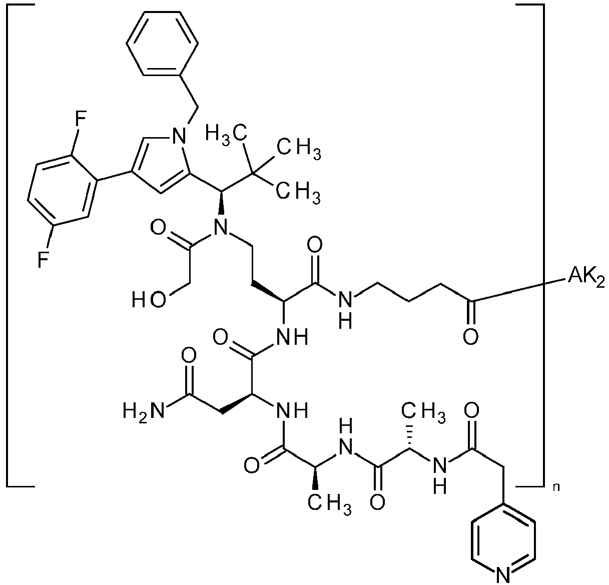

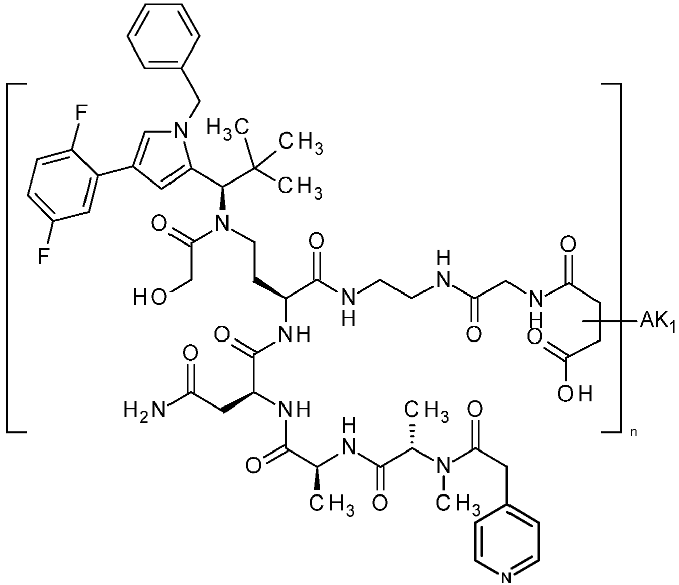

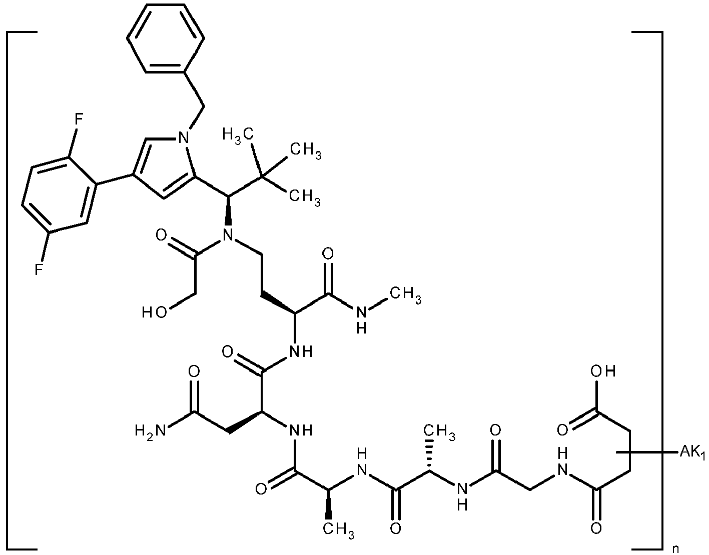

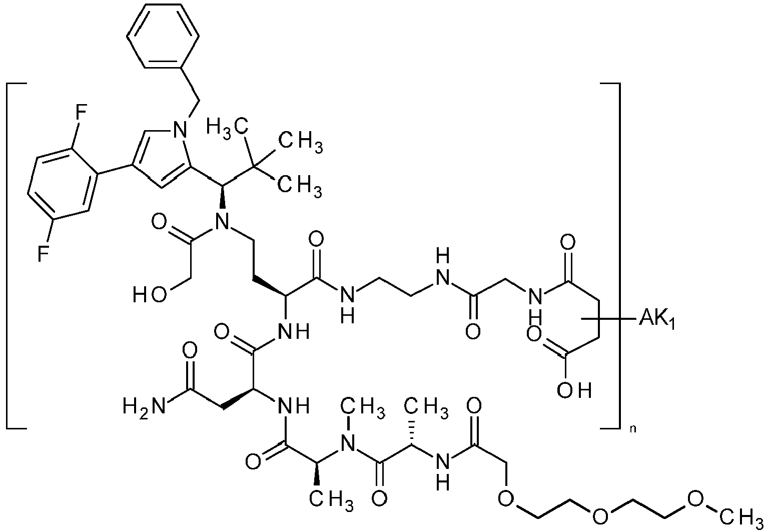

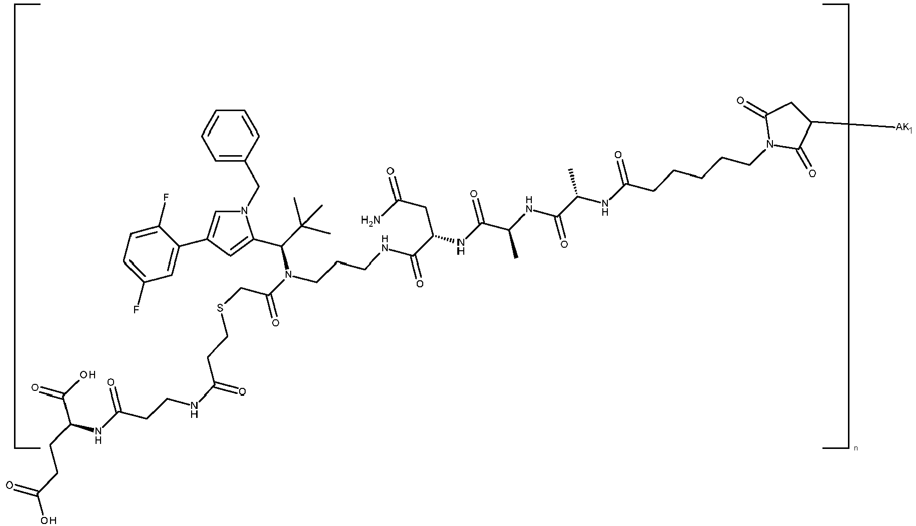

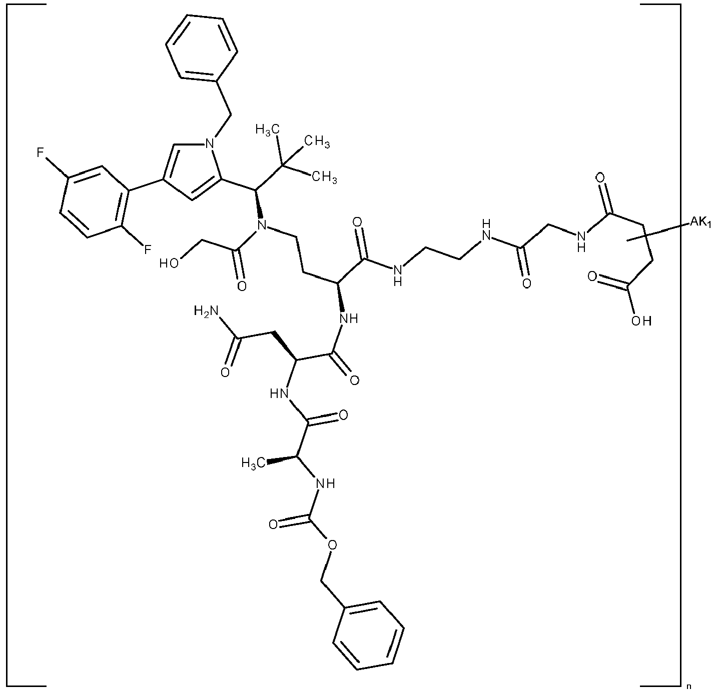

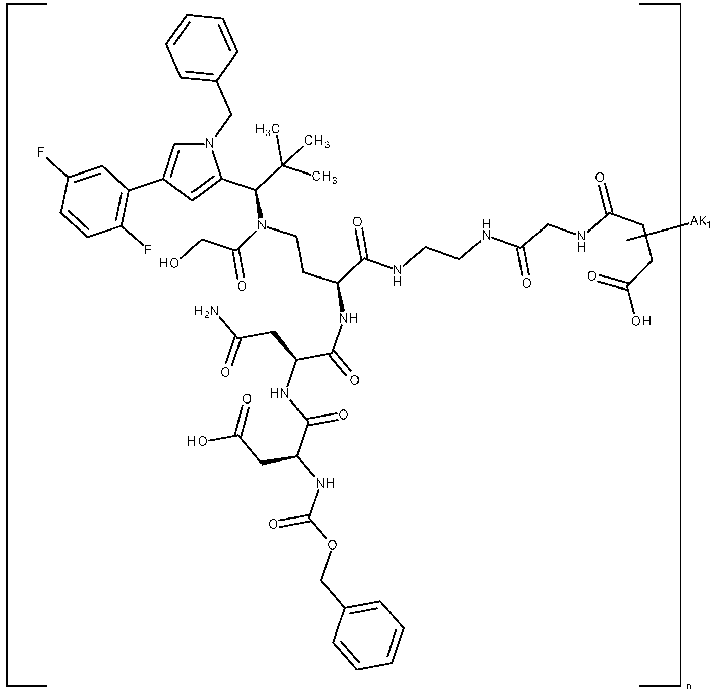

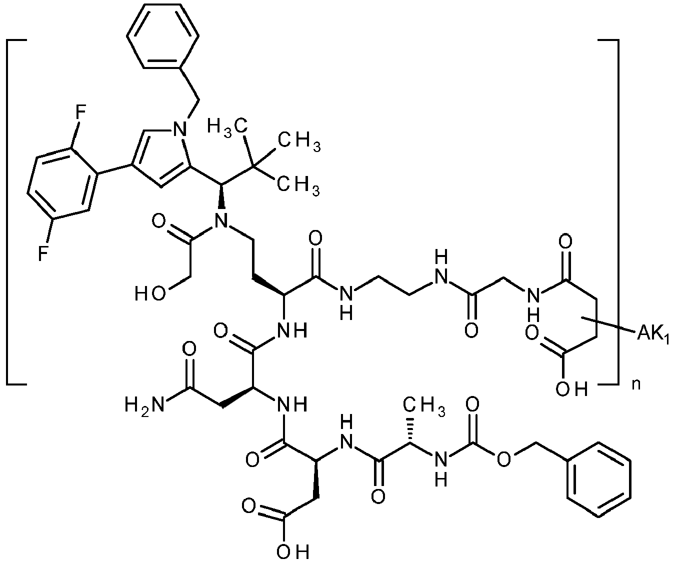

### KSP-Inhibitor - Linker-Intermediate bzw. Prodrug-Linker-Intermediate und Herstellung der Konjugate

Die erfindungsgemäßen Konjugate werden hergestellt, in dem zunächst der niedermolekulare KSP-Inhibitor bzw. Prodrug hiervon mit einem Linker versehen wird. Da so erhaltene Intermediat wird dann mit dem Binder (vorzugsweise Antikörper) umgesetzt.

Für die Kopplung an einen Cysteinrest wird vorzugsweise eine der folgenden Verbindungen mit dem Cystein-haltigen Binder wie z.B. einem Antikörper, der dazu ggf. partiell reduziert wurde, umgesetzt: 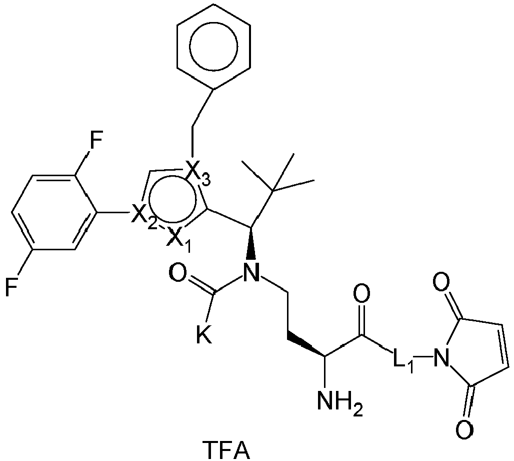 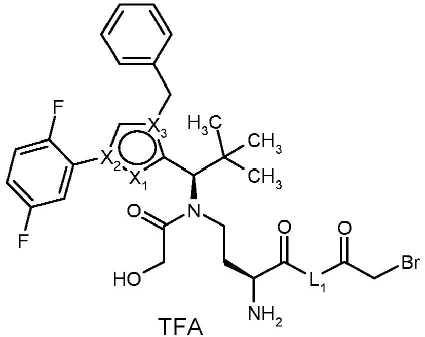 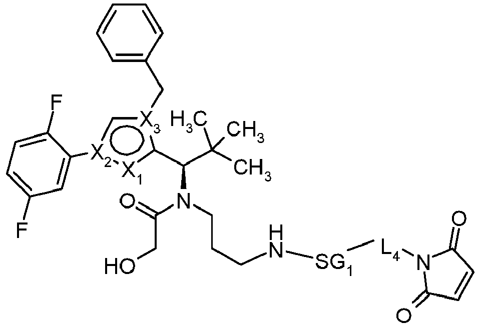 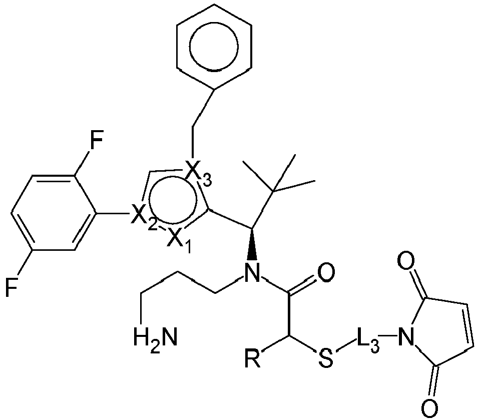 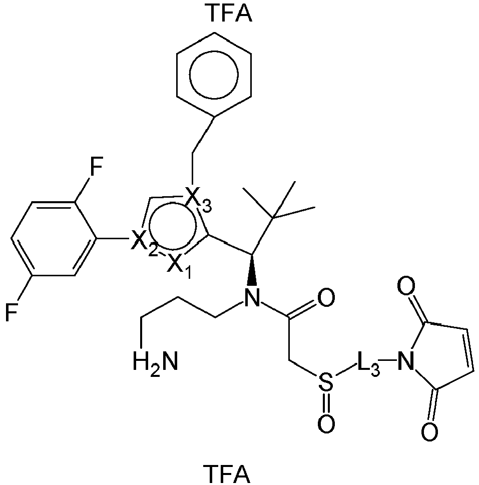 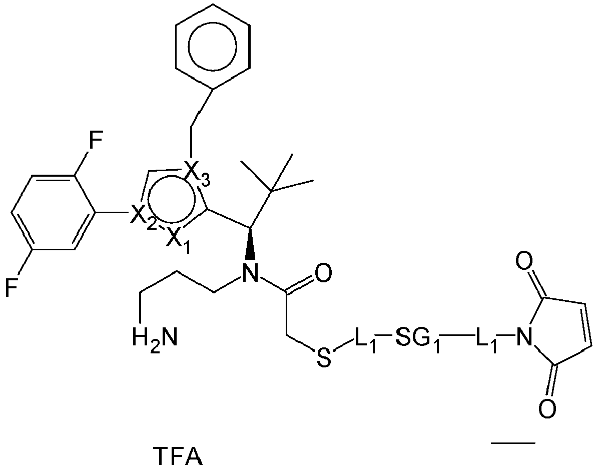 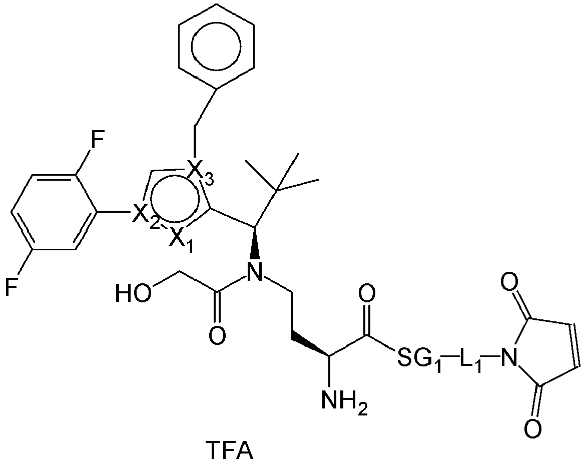 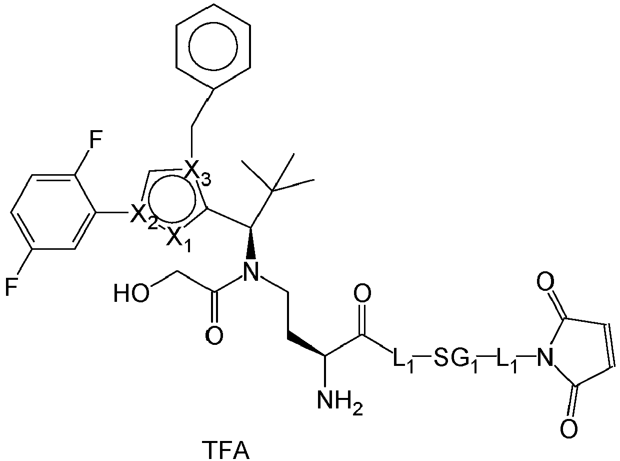 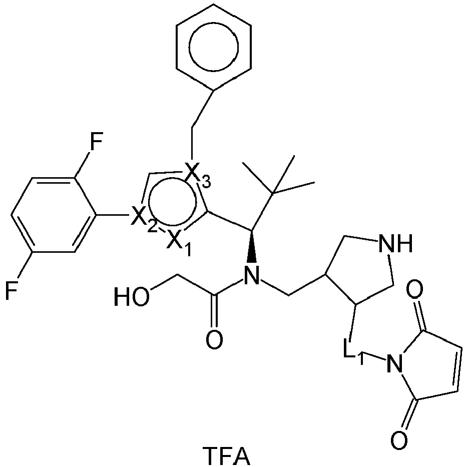 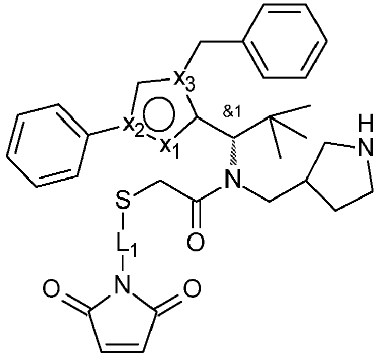 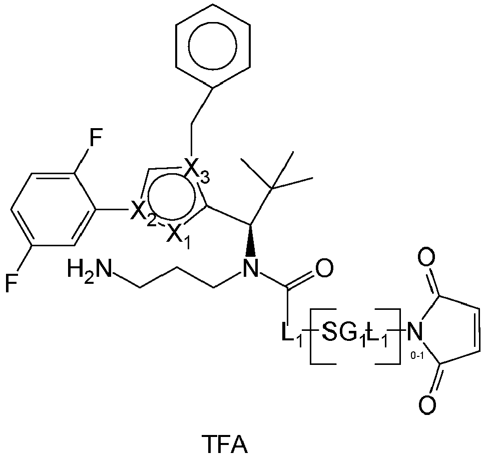 wobei R -H oder -COOH darstellt,
wobei K lineares oder verzweigtes, gegebenenfalls mit C₁-C₆ Alkoxy oder -OH substituiertes C₁-C₆ Alkyl darstellt, und
wobei X1 CH, X2 C, und X3 N darstellt, SG1, L1, L2, L3 und L4 die gleiche Bedeutung haben wie oben beschrieben.

In den zuvor beschriebenen Formeln wie auch in den folgenden Reaktionsschemata bzw. Strukturformeln kann das Wasserstoffatom in Position R⁴ gemäß Formel IIa (d.h. in der Gruppe - NH₂) durch die Gruppe der Formel R²¹-(CO)₍₀₋₁₎-(P3)₍₀₋₂₎-P2-NH-CH(CH₂CONH₂)-CO- oder die durch Cathepsin spaltbare Gruppe der Formel W¹-(CO)₍₀₋₁₎-(P3)₍₁₋₂₎-P2- ersetzt werden,
wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist;
P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist.
wobei R²¹ eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -SO₃H, -COOH, -SH, oder -OH substituiert sein kann.

In jeder der obigen Verbindungen wie auch den folgenden Verbindungen kann die tert.-butylGruppe durch Cyclohexyl ersetzt sein.

Die Verbindung kann z.B in Form ihres Trifluoressigsäuresalzes verwendet werden. Zur Reaktion mit dem Binder wie z.B. dem Antikörper wird die Verbindung vorzugsweise in 2 bis 12 fachem molaren Überschuss gegenüber dem Binder verwendet.

Für die Kopplung an einen Lysinrest wird vorzugsweise eine der folgenden Verbindungen mit dem Lysin-haltigen Binder wie z.B. einem Antikörper umgesetzt: 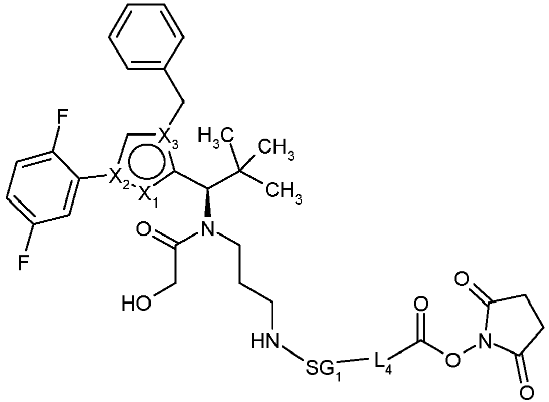 wobei X1 CH, X2 C, und X3 N darstellt,, und L4 die gleiche Bedeutung wie L1 hat, und L1 die gleiche Bedeutung hat wie oben beschrieben.

Für ein an einen Cysteinrest ankoppelndes Intermediat können die Reaktionen wie folgt veranschaulicht werden:

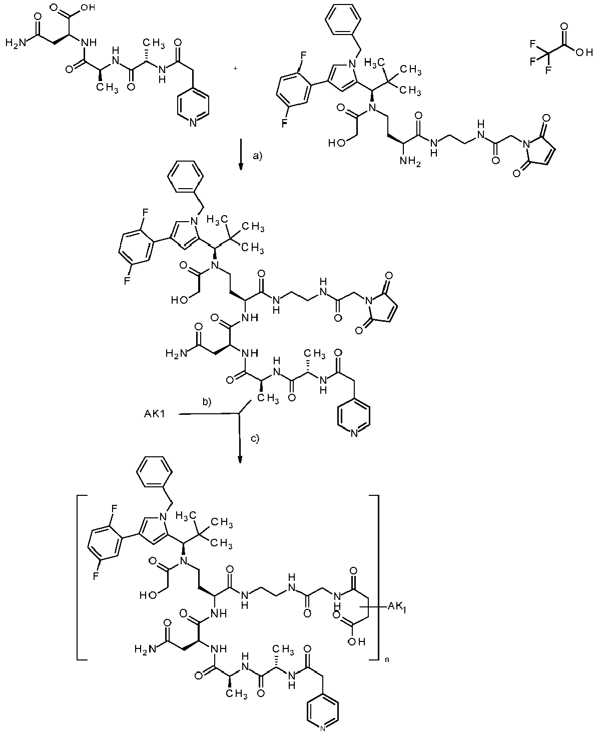

Die anderen Intermediate und andere Antikörper können entsprechend umgesetzt werden.

Für ein an einen Lysinrest ankoppelndes Intermediat kann die Reaktion wie folgt veranschaulicht werden:

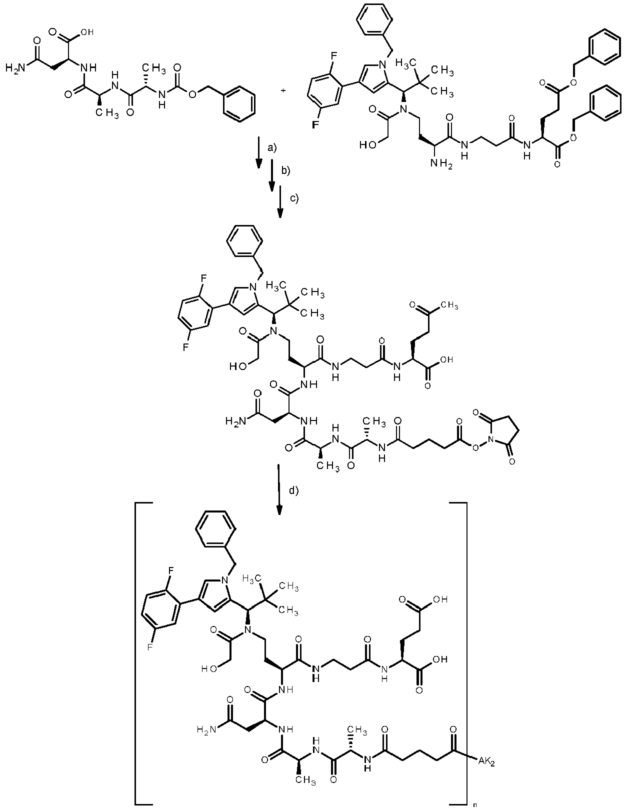

In Abhängigkeit vom Linker können Succinimid-verknüpfte ADCs nach der Konjugation in die offenkettigen Bernsteinsäureamide überführt werden, die ein vorteilhaftes Stabilitätsprofil aufweisen.

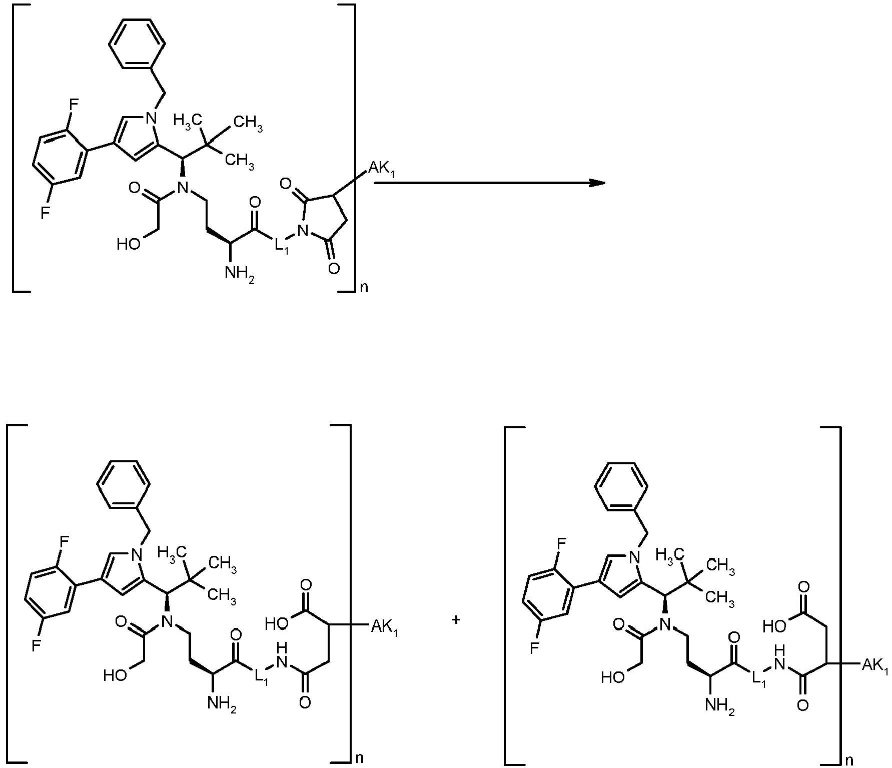

Diese Umsetzung (Ringöffnung) kann bei pH 7.5 bis 9, vorzugsweise bei pH 8, bei einer Temperatur von 25 ° C bis 37 °C erfolgen, z.B. durch Rühren. Die bevorzugte Rührzeit beträgt 8 bis 30 Stunden.

In den obigen Formeln stellen X1 CH, X2 C, und X3 N dar; haben SG1 und L1 die gleiche Bedeutung wie oben beschrieben, und L2, L3 und L4 die gleiche Bedeutung wie L1; und R und K haben die gleiche Bedeutung wie oben beschrieben. AK1 ist ein über einen Cysteinrest gekoppelter, aglycosylierter anti-TWEAKR-Antikörper, und AK2 ist ein über einen Lysinrest gekoppelter, aglycosylierter anti-TWEAKR-Antikörper. Besonders bevorzugt stellt AK1 und AK2 einen aglycosylierten anti-TWEAKR-Antikörper, der spezifisch an die Aminosäure D in Position 47 (D47) von TWEAKR (SEQ ID NO:169) bindet, insbesondere der aglycosylierte anti-TWEAKR-Antikörper TPP-2658.

### Weitere Definitionen

Der Ausdruck "Transglutaminase", austauschbar auch verwendet als "TGase" oder "TG", wird verstanden als ein Enzym, welches die Fähigkeit besitzt, Proteine durch eine Acyl-Transfer-Reaktion zwischen der γ-Carboxamid-Gruppe von peptidgebundenem Glutamin und der ε-Aminogruppe von Lysin oder einem strukturell verwandten primären Amin, so wie zum Beispiel einer Aminopentylgruppe oder zum Beispiel einem peptidgebundenen Lysin, zu verknüpfen, was in einer 8-(y-glutamyl)-Lysin-Isopeptidbindung resultiert. TGasen schließen unter anderem bakterielle Transglutaminase (BTG) wie zum Beispiel das Enzym mit der EC Referenznummer 2.3.2.13 (Protein-Glutamin-γ-Glutamyltransferase) ein.

Der Ausdruck Akzeptorglutamin meint, wenn er bezogen ist auf einen Aminosäurerest eines Antikörpers, einen Glutaminrest, der, unter geeigneten Bedingungen, durch eine Transglutaminase erkannt wird und transglutaminasekatalysiert durch eine Reaktion zwischen ebendiesem Glutamin und einem Lysin oder einem strukturell verwandten primären Amin, wie zum Beispiel einer Aminopentylgruppe, mit diesem verknüpft werden kann. Gegebenenfalls ist das Akzeptor-Glutamin ein oberflächen-exponiertes Glutamin.

Mit "Aminosäuremodifikation" oder "Mutation" ist hier eine Aminosäuresubstitution, -insertion, und/oder eine -deletion in einer Polypeptidsequenz gemeint. Die bevorzugte Aminosäuremodifikation ist hier eine Substitution. Mit "Aminosäuresubstitution" oder "Substitution" ist hier ein Austausch einer Aminosäure an einer gegebenen Position in einer Proteinsequenz durch eine andere Aminosäure gemeint. Zum Beispiel beschreibt die Substitution Y50W eine Variante eines parentalen Polypeptids, in welcher das Tyrosin an Position 50 durch ein Tryptophan ausgetauscht ist. Eine "Variante" von einem Polypeptid beschreibt ein Polypeptid, welches eine Aminosäuresequenz hat, die substanziell identisch zu einem Referenzpolypeptid ist, typischerweise einem nativen oder "parentalen" Polypeptid. Die Polypeptidvariante kann eine oder mehrere Aminosäureaustauche, -deletionen, und/oder -insertionen an bestimmten Positionen in der nativen Aminosäuresequenz aufweisen.

Der Ausdruck "konjugationsstellenspezifisches Konjugat" beschreibt ein Konjugat eines Binders, vorzugsweise eines Antikörpers, und einem Rest, vorzugsweise einem Linker-Wirkstoff-Rest, wobei der Binder an einer oder mehreren definierten Positionen, vorzugsweise Glutamin-Resten, funktionalisiert ist. Transglutaminasen (TGasen, TGases), beinhaltend bakterieller Transglutaminase (BTG) (EC 2.3.2.13), zeigen starke Spezifität in der Erkennung von Glutamin-Protein-Substraten und können eine "konjugationsstellenspezifische Konjugation" katalysieren.

Der Ausdruck "homogenes Konjugat" oder "homogenes ADC" beschreibt ein Gemisch von konjugationsstellenspezifischen Konjugaten wobei mindestens 60, 70, 80, oder 90 % der Binder dieselbe Anzahl von konjugierten Resten pro Binder haben. Im Fall eines Antikörpers sollte diese Anzahl eine gerade Zahl sein, vorzugsweise 2 oder 4.

### Binder

Der Begriff "Binder" wird im breitesten Sinne als ein Molekül verstanden, welches an ein Zielmolekül, das auf einer bestimmten, mit dem Binder-Wirkstoffkonjugat zu adressierenden Zielzellen-Population vorhanden ist, bindet. Der Begriff Binder ist in seiner breitesten Auslegung zu verstehen und umfasst z.B. auch Lektine, Proteine die bestimmte Zuckerketten binden können, oder Phospholipid-bindende Proteine. Solche Binder umfassen beispielsweise hochmolekulare Proteine (Bindeproteine), Polypeptide oder Peptide (Bindepeptide), nicht-peptidische (z.B. Aptamere (US5,270,163) Übersichtsartikel von Keefe AD., et al., Nat. Rev. Drug Discov. 2010; 9:537-550), oder Vitamine) und alle anderen zellbindenden Moleküle oder Substanzen. Bindeproteine sind z.B. Antikörper und Antikörperfragmente oder Antikörpermimetika wie z.B. Affibodies, Adnectins, Anticalins, DARPins, Avimers, Nanobodies (Übersichtsartikel von Gebauer M. et al., Curr. Opinion in Chem. Biol. 2009; 13:245-255; Nuttall S.D. et al., Curr. Opinion in Pharmacology 2008; 8:608-617). Bindepeptide sind z.B. Liganden eines Liganden-Rezeptorspaares, wie z.B. VEGF des Liganden-Rezeptorpaares VEGF/KDR, wie Transferrin des Liganden-Rezeptorpaares Transferrin/Transferrin-Rezeptor oder Cytokine/Cytokin-Rezeptor, wie TNFalpha des Liganden-Rezeptorpaares TNFalpha/TNFalpha Rezeptor.

Der "Binder" kann ein Akzeptor-Glutamin-Rest enthalten, der durch eine Transglutaminase (TGase) einschließlich der bakteriellen Transglutaminase (BTG) (EC 2.3.2.13) funktionalisiert werden kann. Dieses Akzeptor-Glutamin kann entweder natürlicherweise im Binder vorliegen oder es wird extra generiert. Ein Akzeptor-Glutamin kann über eine Insertion eines Glutaminrests an einer geeigneten Position genereiert werden (z.B. über einen Fusions-tag, der ein Akzeptor-Glutamin enthält, oder über eine Mutation einer geeigneten Position zu einem Glutaminrest), oder ein Akzeptor-Glutamin wird generiert durch eine Mutation irgendeiner Aminosäure, die dazu führt, dass ein bestimmter Glutaminrest, der zuvor nicht durch die Transglutaminase erkannt wurde, ein Akzeptorglutamin wird, oder ein Akzeptorglutamin wird genereiert durch eine Veränderung einer posttranslationalen Modifikation (z.B einer Glykosylierung), wobei diese Veränderung den Effekt hat, dass ein natürlich vorkommendes Glutamin, das zuvor nicht durch eine Transglutaminase erkannt wurde, zu einem Akzeptorglutamin wird. Wenn der Binder ein Antikörper ist, enthält er ein Akzeptorglutamin, vorzugsweise in der konstanten Region. Solche Akzeptor-Glutamine können durch Mutationen von geeigneten Positionen zu Glutamin (z.B. die Mutation N297Q, Kabat EU Nummerierung) oder durch die Generierung von deglykosylierten oder aglyklosylierten Antikörpern (z.B. durch Deglykosylierung durch PNGase F oder durch die Mutation N297X, Kabat EU Nummerierung) generiert werden. Im letzteren Fall eines deglykosylierten oder aglykosylierten Antikörpers wird der Glutaminrest Q295 (Kabat EU Nummerierung) der schweren Kette ein Akzeptor-Glutamin. Besonders bevorzugt ist ein Antikörper, der die Mutation N297A oder N297Q (Kabat EU Nummerierung) enthält.

Der Begriff "aglykosylierter Antikörper" oder "deglycosylierter Antikörper" wird hier gebraucht um einen Antikörper oder ein Antikörperderivat zu definieren, der eine FC-Region enthält, der die an die konservierte N-Glykosylierungsstelle in der CH2-Domäne geknüpften Glykane fehlen. Aglykosylierte Antikörper können beispielsweise durch Mutation der Glykosyliereungsstelle N297 (Kabat Eu Nummerierung) der schweren Kette oder durch Expression von Antikörpern in Expressionssystemen, denen die Fähigkeit zur Glykosylierung fehlt, hergestellt werden. Methoden zur Antikörper-Deglykosylierung sind allgemein bekannt (z.B. Winkelhake & Nicolson (1976), J Biol Chem. 251(4):1074-80)). Deglykosylierte Antikörper können zum Beispiel durch enzymatische Deglykosylierung durch PNGase F generiert werden. In einer Ausführungsform der Erfindung können aglykosylierte Antikörper durch Expression in prokaryotischen Wirten erhalten werden. Geeignete prokaryotische Wirte beinhalten aber sind nicht limitiert auf E. coli, Bacillus subtilis, Salmonella typhimurium und einige Spezies der Gattung Pseudomonas, Streptomyces, und Staphylococcus. In einer anderen Ausführungsform der Erfindung können aglykosylierte Antikörper durch die Verwendung von Säugerzell-Expressionssystemen zusammen mit dem Glykosylierungsinhibitor Tunicamycin (Nose & Wigzell (1983), Proc Natl Acad Sei USA, 80(21):6632-6) erhalten werden. Hier ist die Modifikation die Verhinderung der Glykosylierung an der konservierten N-Glykosylierungsstelle N297 (Kabat-Nummerierung) der schweren Kette in der CH₂-Domäne des Fc-Teils des Antikörpers.

Die Literatur offenbart auch verschiedene Optionen der konjugationsstellenspezifischen kovalenten Kopplung (Konjugation) von organischen Molekülen an Antikörper. Besonderes Augenmerk in Hinblick auf diese Erfindung wird dabei auf die Konjugation von Toxophoren an Antikörper über zwei oder vier Akzeptorglutaminreste des Antikörpers gelegt.

Aus der Literatur sind verschiedene Möglichkeiten der kovalenten Kopplung (Konjugation) von organischen Molekülen an Antikörper bekannt. Erfindungsgemäß bevorzugt ist die Konjugation der Toxophore an den Antikörper über ein oder mehrere Schwefelatome von Cystein-Resten des Antikörpers und/oder über ein oder mehrere NH-Gruppen von Lysin-Resten des Antikörpers. Es ist jedoch auch möglich, das Toxophor an den Antikörper über freie Carboxylgruppen oder über Zuckerreste des Antikörpers zu binden.

Ein "Zielmolekül" wird im breitesten Sinne als ein Molekül verstanden, welches in der Zielzellenpopulation vorhanden ist, und kann ein Protein (z.B. ein Rezeptor eines Wachstumsfaktors) oder ein nicht-peptidisches Molekül sein (z.B. ein Zucker oder Phospholipid). Bevorzugt ist es ein Rezeptor oder ein Antigen.

Der Begriff "extrazelluläres" Zielmolekül beschreibt ein an die Zelle gebundenes Zielmolekül, welches sich auf der Außenseite einer Zelle befindet oder den Teil eines Zielmoleküls, welcher sich auf der Außenseite einer Zelle befindet, d.h. ein Binder kann an einer intakten Zelle an sein extrazelluläres Zielmolekül binden. Ein extrazelluläres Zielmolekül kann in der Zellmembran verankert sein oder Bestandteil der Zellmembran sein. Der Fachmann kennt Verfahren, um extrazelluläre Zielmoleküle zu identifizieren. Für Proteine kann dies über eine Bestimmung der Transmembrandomäne(n) und die Orientierung des Proteins in der Membran geschehen. Diese Angaben sind in der Regel in Proteindatenbanken (z.B. SwissProt) hinterlegt.

Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Der Binder kann über eine Bindung mit dem Linker verknüpft werden. Die Verknüpfung des Binders kann mittels eines Heteroatoms des Binders erfolgen. Erfindungsgemäße Heteroatome des Binders, die zur Verknüpfung verwendet werden können, sind Schwefel (in einer Ausführungsform via einer Sulfhydrylgruppe des Binders), Sauerstoff (erfindungsgemäß mittels einer Carboxyl oder Hydroxylgruppe des Binders) und Stickstoff (in einer Ausführungsform via einer primären oder sekundären Amingruppe oder Amidgruppe des Binders). Diese Heteroatome können im natürlichen Binder vorhanden sein oder durch chemische oder molekularbiologische Methoden eingeführt werden. Erfindungsgemäß hat die Verknüpfung des Binders mit dem Toxophor nur einen geringen Einfluß auf die Bindeaktivität des Binders zum Zielmolekül. In einer bevorzugten Ausführungsform hat die Verknüpfung keinen Einfluß auf die Bindeaktivität des Binders zum Zielmolekül.

Der Begriff "Antikörper" wird gemäß der vorliegenden Erfindung in seinem breitesten Sinne verstanden und umfasst Immunglobulinmoleküle, beispielsweise intakte oder modifizierte monoklonale Antikörper, polyklonale Antikörper oder multispezifische Antikörper (z.B. bispezifische Antikörper). Ein Immunglobulinmolekül umfasst bevorzugt ein Molekül mit vier Polypeptidketten, zwei schwere Ketten (H Ketten) und zwei leichte Ketten (L Ketten), welche typischerweise durch Disulfidbrücken verknüpft sind. Jede schwere Kette umfasst eine variable Domäne der schweren Kette (abgekürzt VH) und konstante Domäne der schweren Kette. Die konstante Domäne der schweren Kette kann beispielsweise drei Domänen CH1, CH2 und CH3 umfassen. Jede leichte Kette umfasst eine variable Domäne (abgekürzt VL) und eine konstante Domäne. Die konstante Domäne der leichten Kette umfasst eine Domäne (abgekürzt CL). Die VH und VL Domänen können weiter unterteilt werden in Regionen mit Hypervariabilität, auch Komplementaritäts-bestimmende Regionen genannt ("complementarity determining region", abgekürzt CDR) und Regionen mit geringerer Sequenzvariabilität ("framework region", abgekürzt FR). Jede VH und VL Region setzt sich typischerweise aus drei CDRs und bis zu vier FRs zusammen. Beispielsweise vom Aminoterminus zum Carboxyterminus in der folgenden Reihenfolge FR1, CDR1, FR2, CDR2, FR3, CDR3, FR4. Ein Antikörper kann aus jeder dafür geeeigneten Spezies erhalten werden z.B. Kaninchen, Lama, Kamel, Maus, oder Ratte. In einer Ausführungsform ist der Antikörper humanen oder murinen Ursprungs. Ein Antikörper kann z.B. human, humanisiert oder chimär sein.

Der Begriff "monoklonaler" Antikörper bezeichnet Antikörper, die aus einer Population substantiell homogener Antikörper erhalten wurde, d.h. individuelle Antikörper der Population sind bis auf natürlich auftretende Mutationen, welche in geringfügiger Anzahl auftreten können, identisch. Monoklonale Antikörper erkennen mit hoher Spezifität eine einzige antigene Bindestelle. Der Begriff monoklonaler Antikörper bezieht sich nicht auf ein bestimmtes Herstellungsverfahren.

Der Begriff "intakter" Antikörper bezieht sich auf Antikörper, die sowohl eine Antigen-bindende Domäne als auch die konstante Domäne der leichten und schweren Kette umfassen. Die konstante Domäne kann eine natürlich vorkommende Domäne sein, oder eine Variante davon, bei der mehrere Aminosäurepositionen verändert wurden, und kann auch aglykosyliert sein.

Der Begriff "modifizierter intakter" Antikörper bezieht sich auf intakte Antikörper, die mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert wurden. Des Weiteren können Antikörper dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug;26(8):925-32).

Der Begriff "humaner" Antikörper bezeichnet Antikörper, die aus einem Menschen erhalten werden können oder synthetische humane Antikörper sind. Ein "synthetischer" humaner Antikörper ist ein Antikörper, der in Teilen oder schweren als ganzes von synthetischen Sequenzen in silico erhältlich ist, die auf der Analyse humaner Antikörpersequenzen basieren. Ein humaner Antikörper kann z.B. durch eine Nukleinsäure kodiert sein, die aus einer Bibliothek von Antikörpersequenzen, die humanen Ursprungs sind, isoliert wurde. Ein Beispiel solcher Antikörper ist in Söderlind et al., Nature Biotech. 2000, 18:853-856 zu finden. Solche "humanen" und "synthetischen" Antikörper beinhalten auch aglykosylierte Varianten, die entweder durch Deglykosylierung durch PNGaseF oder durch Mutation von N297 (kabat Nummerierung) der schweren Kette zu einer beliebigen anderen Aminosäure hergestellt wurden.

Der Begriff "humanisierter" oder "chimärer" Antikörper beschreibt Antikörper, die aus einem nicht-humanen und einem humanen Sequenzanteil bestehen. Bei diesen Antikörpern wird ein Teil der Sequenzen des humanen Immunoglobulins (Rezipient) durch Sequenzanteile eines nicht-humanen Immunoglobulins (Donor) ersetzt. Der Donor ist in vielen Fällen ein murines Immunoglobulin. Bei humanisierten Antikörpern werden Aminosäuren der CDR des Rezipienten durch Aminosäuren des Donors ersetzt. Mnachmal werden auch noch Aminosäuren des Frameworks durch korrespondierende Aminosäuren des Donors ersetzt. In machen Fällen enthält der humanisierte Antikörper Aminosäuren, die weder im Rezipient noch im Donor enthalten waren und die während der Optimierung des Antikörpers eingefügt wurden. Bei chimären Antikörpern werden die variablen Domänen des Donor-Immunoglobulins mit den konstanten Regionen eines humanen Antikörpers fusioniert. Solche "humanisierten" und "chimären" Antikörper beinhalten auch aglykosylierte Varianten, die entweder durch Deglykosylierung durch PNGaseF oder durch Mutation von N297 (kabat Nummerierung) der schweren Kette zu einer beliebigen anderen Aminosäure hergestellt wurden.

Der Begriff Komplementaritäts-bestimmende Region (CDR) wie er hier verwendet wird, bezieht sich auf diejenigen Aminosäuren einer variablen Antikörperdomäne, die für die Bindung an das Antigen notwendig sind. Jede variable Region hat typischerweise drei CDR Regionen, welche als CDR1, CDR2 und CDR3 bezeichnet werden. Jede CDR Region kann Aminosäuren nach der Definition von Kabat und/oder Aminosäuren eines Hypervariablen Loops definiert nach Chotia umfassen. Die Definition nach Kabat umfasst zum Beispiel die Region von ungefähr Aminosäureposition 24 - 34 (CDR1), 50 - 56 (CDR2) und 89 - 97 (CDR3) der variablen leichten Kette und 31 - 35 (CDR1), 50 - 65 (CDR2) und 95 - 102 (CDR3) der variablen schweren Kette (Kabat et al., Sequences of Proteins of Immunological Interest, 5th Ed. Public Health Service, National Institutes of Health, Bethesda, MD. (1991)). Die Definition nach Chotia umfasst zum Beispiel die Region von ungefähr Aminosäureposition 26 - 32 (CDR1), 50 - 52 (CDR2) und 91 - 96 (CDR3) der variablen leichetn Kette und 26 - 32 (CDR1), 53 - 55 (CDR2) und 96 - 101 (CDR3) der variablen schweren Kette Chothia and Lesk; J Mol Biol 196: 901-917 (1987)). In mnachen Fällen kann eine CDR Aminosäuren aus einer CDR Region definiert nach Kabat und Chotia umfassen.

Abhängig von der Aminosäure-Sequenz der konstanten Domäne der schweren Kette können Antikörper in verschiedene Klassen eingeteilt werden. Es gibt fünf Hauptklassen von intakten Antikörpern: IgA, IgD, IgE, IgG und IgM, wobei mehrere davon in weitere Unterklassen aufgegliedert werden können. (Isotypen), z.B. IgG1, IgG2, IgG3, IgG4, IgA1 und IgA2. Die konstante Domäne der schweren Kette, die zu den unterschiedlichen Klassen korrespondieren, werden als [alpha/α], [delta/δ], [epsilon/ε], [gamma/γ] und [my/µ] bezeichnet. Sowohl die dreidimensionale Struktur als auch die Untereinheitenstruktur von Antikörpern sind bekannt.

Der Begriff "funktionales Fragment" oder "antigen-bindendes Antikörperfragment" eines Antikörpers/Immunoglobulins ist definiert als ein Fragment eines Antikörpers/Immunoglobulins (z.B. die variable Domänen eines IgG), welches die Antigen-Bindedomänen des Antikörpers/Immunoglobulins noch umfasst. Die "Antigen-Bindedomäne" eines Antikörpers umfasst typischerweise eine oder mehrere Hypervariable Regionen eines Antikörpers, z.B. die CDR, CDR2 und/oder CDR3 Region. Allerdings kann auch die "Framework" oder die "Gerüst" Region eines Antikörpers zur Bindung des Antikörpers an das Antigen eine Rolle spielen. Die Framework Region bildet das Gerüst für die CDRs. Vorzugsweise umfasst die Antigen-Bindedomäne mindestens die Aminosäuren 4 bis 103 der variablen leichten Kette und Aminosäure 5 bis 109 der variablen schweren Kette, bevorzugter die Aminosäure 3 bis 107 der variablen leichten Kette und 4 bis 111 der variablen schweren Kette, besonders bevorzugt sind die kompletten variablen leichten und schweren Ketten , also Aminosäure 1 - 109 der VL und 1 bis 113 der VH (Nummerierung nach WO97/08320).

"Funktionale Fragmente" oder "antigen-bindende Antikörperfragmente" der Erfindung umfassen nicht abschliessend Fab, Fab', F(ab')₂ und Fv Fragmente, Diabodies, Single Domain Antibodies (DAbs), linerae Antikörper, Einzelketten Antikörper (single-chain Fv, abgekürzt scFv); und multispezifische, wie z.B. bi- und tri-spezifische, Antikörper, gebildet aus Antikörperfragmenten C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). Andere Antikörper als "multi-spezifische" oder "multi-funktionale" sind solche mit identischen Bindestellen. Multispezifische Antikörper können spezifisch für unterschiedliche Epitope eines Antigens sein oder spezifisch für Epitope von mehr als einem Antigen sein (siehe z.B. WO 93/17715; WO 92/08802; WO 91/00360; WO 92/05793; Tutt, et al., 1991, J. Immunol. 147:60 69; U. S. Pat. Nos. 4,474,893; 4,7 14,68 1 ; 4,925,648; 5,573,920; 5,601,8 19; oder Kostelny et al., 1992, J. Immunol. 148: 1547 1553). Ein F(ab')₂ oder Fab Molekül kann so konstruiert werden, dass die Zahl der intermolekularen Disulfidinteraktionen, die zwischen den Ch1 und den CL Domänen stattfindet, reduziert oder oder komplett verhindert werden kann.

"Epitope" bezeichnen Proteindeterminanten, die eine spezifische Bindung mit einem Immunglobulin oder T-Zell-Rezeptoren eingehen können. Epitopische Determinanten bestehen normalerweise aus chemisch aktiven Oberflächengruppen von Molekülen wie Aminosäuren oder Zuckerseitenketten, oder Kombinationen hiervon, und weisen normalerweise spezifische 3-dimensionale Struktureigenschaften wie auch spezifische Ladungseigenschaften auf.

"Funktionale Fragmente" oder "antigen-bindende Antikörperfragmente" können mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert werden. Des Weiteren können Antikörper und antigen-bindende Fragmente dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug; 26(8):925-32).

Polyklonale Antikörper können durch für den Durchschnittsfachmann bekannte Methoden hergestellt werden. Monoklonale Antiköper können durch für den Durchschnittsfachmann bekannte Methoden hergestellt werden (Köhler und Milstein, Nature, 256, 495-497, 1975). Humane bzw. humanisierte monoklonale Antiköper können durch für den Durchschnittsfachmann bekannte Methoden hergestellt werden (Olsson et al., Meth Enzymol. 92, 3-16 bzw. Cabilly et al US 4,816,567 oder Boss et al US 4,816,397).

Der Durchschnittsfachmann kennt vielfältige Methoden, um humane Antikörper und deren Fragmente herzustellen, wie beispielsweise mittels transgener Mäuse (N Lonberg und D Huszar, Int Rev Immunol. 1995; 13(1):65-93) oder Phage Display Technologien (Clackson et al., Nature. 1991 Aug 15;352(6336):624-8). Antikörper der Erfindung können aus rekombinanten Antikörperbibliotheken erhalten werden, die z.B. auf den Aminosäuresequenzen einer Vielzahl von Antikörpern besteht, die aus einer großen Anzahl gesunder Freiwilliger erstellt wurden. Antikörper können auch mittels bekannter rekombinanter DNS-Technologien hergestellt werden. Die Nukleinsäuresequenz eines Antikörpers kann durch Routinesequenzierung erhalten werden, oder ist aus öffentlich zugänglichen Datenbanken erhältlich.

Ein "isolierter" Antikörper oder Binder wurde von anderen Bestandteilen der Zelle gereinigt. Kontaminierende Bestandteile einer Zelle, welche mit einer diagnostischen oder therapeutischen Verwendung interferieren können, sind z.B. Enzyme, Hormone, oder andere peptidische- oder nicht-peptidische Bestandteile einer Zelle. Bevorzugt ist ein Antikörper oder Binder, der zu mehr als 95 Gew-% bezogen auf den Antikörper bzw. Binder aufgereinigt wurde (bestimmt durch z.B. Lowry Verfahren, UV-Vis Spektroskopie oder durch SDS-Kapillargelelektrophorese). Ausserdem ein Antikörper, der soweit aufgereinigt wurde, dass mindestens 15 Aminosäuren des Aminoterminus oder einer internen Aminosäuresequenz bestimmt werden können, oder zur Homogenität aufgereinigt wurde, wobei die Homogenität bestimmt wird durch SDS-PAGE unter reduzierenden oder nicht-reduzierenden Bedingungen (die Detektion kann mittels Coomassie Blau Anfärbung oder bevorzugt durch Silberfärbung bestimmt werden). Jedoch wird ein Antikörper normalerweise durch einen oder mehrere Reinigungsschritte hergestellt.

Der Begriff "spezifische Bindung" oder "bindet spezifisch" bezieht sich auf einen Antikörper oder Binder, der an ein vorbestimmtes Antigen/Zielmolekül bindet. Spezifische Bindung eines Antikörpers oder Binders beschreibt typischerweise einen Antikörper bzw. Binder mit einer Affinität von mindestens 10⁻⁷ M (als Kd-Wert; also vorzugsweise solche mit kleineren Kd-Werten als 10⁻⁷ M), wobei der Antikörper bzw. Binder eine mindestens zweifach höhere Affinität zum vorbestimmten Antigen/Zielmolekül als zu einem nicht-spezifischen Antigen/Zielmolekül hat (z.B. Rinder Serumalbumin, oder Casein), welches nicht das vorbestimmte Antigen/Zielmolekül oder ein eng verwandtes Antigen/Zielmolekül ist. Die Antikörper weisen vorzugsweise eine Affinität von mindestens 10⁻⁷ M (als Kd-Wert; also vorzugsweise solche mit kleineren Kd-Werten als 10⁻⁷ M), vorzugsweise von mindestens 10⁻⁸ M, besonders bevorzugt in dem Bereich von 10⁻⁹ M bis 10⁻¹¹ M auf. Die Kd-Werte können durch z.B. Oberflächenplasmonresonanzspektroskopie bestimmt werden.

Die erfindungsgemäßen Antikörper-Wirkstoff-Konjugate weisen ebenfalls Affinitäten in diesen Bereichen auf. Durch die Konjugation der Wirkstoffe wird die Affinität vorzugsweise nicht wesentlich beeinflusst (in der Regel wird die Affinität weniger als eine Größenordnung verringert, also z.B. maximal von 10⁻⁸ M auf 10⁻⁷ M).

Die erfindungsgemäßen verwendeten Antikörper zeichnen sich weiterhin vorzugsweise durch eine hohe Selektivität aus. Eine hohe Selektivität liegt vor, wenn der erfindungsgemäße Antikörper eine mindestens um den Faktor 2, bevorzugt Faktor 5 oder insbesondere bevorzugt Faktor 10 bessere Affinität am Zielprotein aufweisst als an einem unabhängigen anderen Antigen, z.B. humanem Serumalbumin (die Affinität kann z.B. durch Oberflächenplasmonenresonanzspektroskopie bestimmt werden).

Zudem sind die erfindungsgemäßen verwendeten Antikörper vorzugsweise kreuzreaktiv. Um präklinische Studien, z.B. toxikologische oder Wirksamkeitsstudien (z.B. in Xenograft-Mäusen), zu erleichtern und besser interpretieren zu können, ist es von Vorteil, wenn der erfindungsgemäß verwendete Antikörper nicht nur das humane Zielprotein bindet, sondern auch in der für die Studien verwendeten Spezies das Spezies-Zielprotein bindet. In einer Ausführungsform ist der erfindungsgemäß verwendete Antikörper zusätzlich zum humanen Zielprotein kreuzreaktiv zum Zielprotein mindestens einer weiteren Spezies. Für toxikologische und Wirksamkeitsstudien werden bevorzugt Spezien der Familien Nager, Hunde und nicht-humane Primaten, verwendet. Bevorzugte Nager Spezien sind Maus und Ratte. Bevorzugte nicht-humane Primaten sind Rhesusaffen, Schimpansen und Langschwanzmakaken.

In einer Ausführungsform ist der erfindungsgemäß verwendete Antikörper zusätzlich zum humanen Zielprotein kreuzreaktiv zum Zielprotein mindestens einer weiteren Spezies ausgewählt aus der Gruppe von Spezien bestehend aus Maus, Ratte und Langschwanzmakak (Macaca fascicularis). Insbesondere bevorzugt sind erfindungsgemäß verwendete Antikörper, die zusätzlich zum humanen Zielprotein mindestens kreuzreaktiv zum Maus-Zielprotein sind. Bevorzugt sind kreuzreaktive Antikörper, deren Affinität zum Zielprotein der weiteren nicht-humanen Spezies sich nicht um mehr als den Faktor 50, insbesondere nicht mehr als den Faktor zehn von der Affinität zum humanen Zielprotein unterscheidet.

### Gegen ein Krebs-Zielmolekül gerichtete Antikörper

Bevorzugt ist das Zielmolekül, gegen das der Binder, z.B. ein Antikörper oder ein Antigen bindendes Fragment davon, gerichtet ist, ein Krebs-Zielmolekül. Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Antikörper, welche spezifisch gegen ein Antigen, wie z.B. ein Krebszell-Antigen, gerichtet sind, können vom Durchschnittsfachmann mittels ihm bekannter Verfahren hergestellt werden (wie z.B. rekombinante Expression) oder kommerziell erworben werden (wie z.B. von Merck KGaA, Deustchland). Beispiele bekannter kommerziell erhältlicher Antikörper in der Krebstherapie sind Erbitux^{®} (Cetuximab, Merck KGaA), Avastin^{®} (Bevacizumab, Roche) und Herceptin^{®} (Trastuzumab, Genentech). Trastuzumab ist ein rekombinanter humanisierter monokloaler Antikörper vom IgG1kappa Typ, welcher mit hoher Affinität in einem Zell-basierten Assay (Kd = 5 nM) die extrazelluläre Domäne des humanen epidermalen Wachstumsrezeptors bindet. Der Antikörper wird rekombinant in CHO-Zellen hergestellt. Alle diese Antikörper können auch als aglykosylierte Varianten dieser Antikörper hergestellt werden, entweder durch Deglycosylierung durch PNGase F oder durch Mutation von N297 (Kabat Nummerierung) der schweren Kette zu einer beliebigen Aminosäure.

In einer bevorzugten Ausführungsform ist das Zielmolekül ein selektives Krebs-Zielmolekül.

In einer besonders bevorzugten Ausführungsform ist das Zielmolekül ein Protein.

In einer Ausführungsform ist das Zielmolekül ein extrazelluläres Zielmolekül. In einer bevorzugten Ausführungsform ist das extrazelluläre Zielmolekül ein Protein.

Krebs-Zielmoleküle sind dem Fachmann bekannt. Beispiele hierfür sind im Folgenden aufgeführt.

Beispiele für Krebs-Zielmoleküle sind:
(1) EGF-Rezeptor (NCBI-Referenzsequenz NP_005219.2), SEQ ID NO: 213(1210 Aminosäuren):
   >gi|29725609|ref|NP_005219.2| EGFR-Rezeptor-Vorläufer [Homo sapiens] Die extrazelluläre Domäne ist durch Unterstreichung gekennzeichnet.
(2) Mesothelin (SwissProt-Referenz Q13421-3), SEQ ID NO: 214 (622 Aminosäuren):
   >sp|Q13421-3|MSLN_HUMAN Isoform 2 of Mesothelin OS=Homo sapiens GN=MSLN 
MALPTARPLLGSCGTPALGSLLFLLFSLGWVQPSRTLAGETGQEAAPLDGVLANPPNISS
LSPRQLLGFPCAEVSGLSTERVRELAVALAQKNVKLSTEQLRCLAHRLSEPPEDLDALPL
DLLLFLNPDAFSGPQACTRFFSRITKANVDLLPRGAPERQRLLPAALACWGVRGSLLSEA
DVRALGGLACDLPGRFVAESAEVLLPRLVSCPGPLDQDQQEAARAALQGGGPPYGPPSTW
SVSTMDALRGLLPVLGQPIIRSIPQGIVAAWRQRSSRDPSWRQPERTILRPRFRREVEKT
ACPSGKKAREIDESLIFYKKWELEACVDAALLATQMDRVNAIPFTYEQLDVLKHKLDELY
PQGYPESVIQHLGYLFLKMSPEDIRKWNVTSLETLKALLEVNKGHEMSPQVATLIDRFVK
GRGQLDKDTLDTLTAFYPGYLCSLSPEELSSVPPSSIWAVRPQDLDTCDPRQLDVLYPKA
RLAFQNMNGSEYFVKIQSFLGGAPTEDLKALSQQNVSMDLATFMKLRTDAVLPLTVAEV
Q
KLLGPHVEGLKAEERHRPVRDWILRQRQDDLDTLGLGLQGGIPNGYLVLDLSMQEALSGT
PCLLGPGPVLTVLALLLASTLA
 Wobei Mesothelin von den Aminosäuren 296-598 kodiert wird. Aminosäuren 37-286 kodieren für "megakaryocyte-potentiating factor". Mesothelin ist durch einen GPI-Anker in der Zellmembran verankert und ist extrazellulär lokalisiert.
(3) Carboanhydrase IX (SwissProt-Referenz Q16790), SEQ ID NO: 215 (459 Aminosäuren):
   >sp|Q16790|CAH9_HUMAN Carbonic anhydrase 9 OS=Homo sapiens GN=CA9 PE=1 SV=2 
MAPLCPSPWLPLLIPAPAPGLTVQLLLSLLLLVPVHPQRLPRMQEDSPLGGGSSGEDDPL
GEEDLPSEEDSPREEDPPGEEDLPGEEDLPGEEDLPEVKPKSEEEGSLKLEDLPTVEAPG
DPQEPQNNAHRDKEGDDQSHWRYGGDPPWPRVSPACAGRFQSPVDIRPQLAAFCPALRPL
ELLGFQLPPLPELRLRNNGHSVQLTLPPGLEMALGPGREYRALQLHLHWGAAGRPGSEHT
VEGHRFPAEIHVVHLSTAFARVDEALGRPGGLAVLAAFLEEGPEENSAYEQLLSRLEEIA
EEGSETQVPGLDISALLPSDFSRYFQYEGSLTTPPCAQGVIWTVFNQTVMLSAKQLHTLS
DTLWGPGDSRLQLNFRATQPLNGRVIEASFPAGVDSSPRAAEPVQLNSCLAAGDILALVF
GLLFAVTSVAFLVQMRRQHRRGTKGGVSYRPAEVAETGA
 Die extrazelluläre Domäne ist durch Unterstreichung gekennzeichnet.
(4) C4.4a (NCBI -Referenzsequenz NP_055215.2; Synonym LYPD3), SEQ ID NO: 216 (346 Aminosäuren):
   >gi|93004088|ref|NP_055215.2| ly6/PLAUR domain-containing protein 3-Vorläufer [Homo sapiens] 
MDPARKAGAQAMIWTAGWLLLLLLRGGAQALECYSCVQKADDGCSPNKMKTVKCAPG
VDVCTEAVGAVETIHGQFSLAVRGCGSGLPGKNDRGLDLHGLLAFIQLQQCAQDRCNAK
LNLTSRALDPAGNESAYPPNGVECYSCVGLSREACQGTSPPVVSCYNASDHVYKGCFDGN
 
VTLTAANVTVSLPVRGCVQDEFCTRDGVTGPGFTLSGSCCQGSRCNSDLRNKTYFSPRIPP
LVRLPPPEPTTVASTTSVTTSTSAPVRPTSTTKPMAPTSQTPRQGVEHEASRDEEPRLTGG
AAGHQDRSNSGQYPAKGGPQQPHNKGCVAPTAGLAALLLAVAAGVLL
 Die maturierte, extrazelluläre Domäne ist durch Unterstreichung gekennzeichnet.
(5) CD52 (NCBI-Referenzsequenz NP_001794.2 ), SEQ ID NO: 217
   >gi|68342030|ref|NP_001794.2| CAMPATH-1 antigen-Vorläufer [Homo sapiens] MKRFLFLLLTISLLVMVQIQTGLSGQNDTSQTSSPSASSNISGGIFLFFVANAIIHLFCFS
(6) Her2 (NCBI-Referenzsequenz NP_004439.2), SEQ ID NO: 218
   >gi|54792096|ref|NP_004439.2| receptor tyrosine-protein kinase erbB-2 isoform a [Homo sapiens]
(7) CD20 (NCBI-Referenzsequenz NP_068769.2), SEQ ID NO: 219
   >gi|23110987|ref|NP_068769.2| B-lymphocyte antigen CD20 [Homo sapiens] 
MTTPRNSVNGTFPAEPMKGPIAMQSGPKPLFRRMSSLVGPTQSFFMRESKTLGAVQIMNG
LFHIALGGLLMIPAGIYAPICVTVWYPLWGGIMYIISGSLLAATEKNSRKCLVKGKMIMNS
LSLFAAISGMILSIMDILNIKISHFLKMESLNFIRAHTPYINIYNCEPANPSEKNSPSTQYCYSI
QSLFLGILSVMLIFAFFQELVIAGIVENEWKRTCSRPKSNIVLLSAEEKKEQTIEIKEEVVGLT
ETSSQPKNEEDIEIIPIQEEEEEETETNFPEPPQDQESSPIENDSSP
(8) das Lymphozyten aktivierungs Antigen CD30 (SwissProt ID P28908), SEQ ID NO: 220
   >gi|68348711|ref|NP_001234.2| tumor necrosis factor receptor superfamily member 8 isoform 1-Vorläufer [Homo sapiens] 
MRVLLAALGLLFLGALRAFPQDRPFEDTCHGNPSHYYDKAVRRCCYRCPMGLFPTQQCP
QRPTDCRKQCEPDYYLDEADRCTACVTCSRDDLVEKTPCAWNSSRVCECRPGMFCSTSA
VNSCARCFFHSVCPAGMIVKFPGTAQKNTVCEPASPGVSPACASPENCKEPSSGTIPQAKPT
PVSPATSSASTMPVRGGTRLAQEAASKLTRAPDSPSSVGRPSSDPGLSPTQPCPEGSGDCRK
QCEPDYYLDEAGRCTACVSCSRDDLVEKTPCAWNSSRTCECRPGMICATSATNSRARCVP
YPICAAETVTKPQDMAEKDTTFEAPPLGTQPDCNPTPENGEAPASTSPTQSLLVDSQASKT
LPIPTSAPVALSSTGKPVLDAGPVLFWVILVLVVVVGSSAFLLCHRRACRKRIRQKLHLCYP
VQTSQPKLELVDSRPRRSSTQLRSGASVTEPVAEERGLMSQPLMETCHSVGAAYLESLPLQ
DASPAGGPSSPRDLPEPRVSTEHTNNKIEKIYIMKADTVIVGTVKAELPEGRGLAGPAEPEL
EEELEADHTPHYPEQETEPPLGSCSDVMLSVEEEGKEDPLPTAASGK
(9) das Lymphozyten Adhesionsmolekül CD22 (SwissProt ID P20273), SEQ ID NO: 221
   >gi|157168355|ref|NP_001762.2| B-cell receptor CD22 isoform 1-Vorläufer [Homo sapiens] 
MHLLGPWLLLLVLEYLAFSDSSKWVFEHPETLYAWEGACVWIPCTYRALDGDLESFILFH
NPEYNKNTSKFDGTRLYESTKDGKVPSEQKRVQFLGDKNKNCTLSIHPVHLNDSGQLGLR
MESKTEKWMERIHLNVSERPFPPHIQLPPEIQESQEVTLTCLLNFSCYGYPIQLQWLLEGVP
MRQAAVTSTSLTIKSVFTRSELKFSPQWSHHGKIVTCQLQDADGKFLSNDTVQLNVKHTP
KLEIKVTPSDAIVREGDSVTMTCEVSSSNPEYTTVSWLKDGTSLKKQNTFTLNLREVTKDQ
SGKYCCQVSNDVGPGRSEEVFLQVQYAPEPSTVQILHSPAVEGSQVEFLCMSLANPLPTNY
TWYHNGKEMQGRTEEKVHIPKILPWHAGTYSCVAENILGTGQRGPGAELDVQYPPKKVT
TVIQNPMPIREGDTVTLSCNYNSSNPSVTRYEWKPHGAWEEPSLGVLKIQNVGWDNTTIA
CAACNSWCSWASPVALNVQYAPRDVRVRKIKPLSEIHSGNSVSLQCDFSSSHPKEVQFFW
EKNGRLLGKESQLNFDSISPEDAGSYSCWVNNSIGQTASKAWTLEVLYAPRRLRVSMSPG
DQVMEGKSATLTCESDANPPVSHYTWFDWNNQSLPYHSQKLRLEPVKVQHSGAYWCQG
TNSVGKGRSPLSTLTVYYSPETIGRRVAVGLGSCLAILILAICGLKLQRRWKRTQSQQGLQE
NSSGQSFFVRNKKVRRAPLSEGPHSLGCYNPMMEDGISYTTLRFPEMNIPRTGDAESSEMQ
RPPPDCDDTVTYSALHKRQVGDYENVIPDFPEDEGIHYSELIQFGVGERPQAQENVDYVIL
KH
(10) das Myloidzellen Oberflächenantigen CD33 (SwissProt ID P20138), SEQ ID NO: 222
   >gi|130979981|ref|NP_001763.3| myeloid cell surface antigen CD33 isoform 1-Vorläufer [Homo sapiens] 
MPLLLLLPLLWAGALAMDPNFWLQVQESVTVQEGLCVLVPCTFFHPIPYYDKNSPVHGY
WFREGAIISRDSPVATNKLDQEVQEETQGRFRLLGDPSRNNCSLSIVDARRRDNGSYFFRM
ERGSTKYSYKSPQLSVHVTDLTHRPKILIPGTLEPGHSKNLTCSVSWACEQGTPPIFSWLSA
APTSLGPRTTHSSVLIITPRPQDHGTNLTCQVKFAGAGVTTERTIQLNVTYVPQNPTTGIFPG
DGSGKQETRAGVVHGAIGGAGVTALLALCLCLIFFIVKTHRRKAARTAVGRNDTHPTTGS
ASPKHQKKSKLHGPTETSSCSGAAPTVEMDEELHYASLNFHGMNPSKDTSTEYSEVRTQ
(11) das Transmembran Glykoprotein NMB (SwissProt ID Q14956), SEQ ID NO: 223
   >gi|52694752|ref|NP_001005340.1| transmembrane glycoprotein NMB isoform α-Vorläufer [Homo sapiens] 
MECLYYFLGFLLLAARLPLDAAKRFHDVLGNERPSAYMREHNQLNGWSSDENDWNEKL
YPVWKRGDMRWKNSWKGGRVQAVLTSDSPALVGSNITFAVNLIFPRCQKEDANGNIVYE
KNCRNEAGLSADPYVYNWTAWSEDSDGENGTGQSHHNVFPDGKPFPHHPGWRRWNFIY
VFHTLGQYFQKLGRCSVRVSVNTANVTLGPQLMEVTVYRRHGRAYVPIAQVKDVYVVT
 
DQIPVFVTMFQKNDRNSSDETFLKDLPIMFDVLIHDPSHFLNYSTINYKWSFGDNTGLFVST
NHTVNHTYVLNGTFSLNLTVKAAAPGPCPPPPPPPRPSKPTPSLATTLKSYDSNTPGPAGDN
PLELSRIPDENCQINRYGHFQATITIVEGILEVNIIQMTDVLMPVPWPESSLIDFVVTCQGSIP
TEVCTIISDPTCEITQNTVCSPVDVDEMCLLTVRRTFNGSGTYCVNLTLGDDTSLALTSTLIS
VPDRDPASPLRMANSALISVGCLAIFVTVISLLVYKKHKEYNPIENSPGNVVRSKGLSVFLN
RAKAVFFPGNQEKDPLLKNQEFKGVS
(12) das Adhesionsmolekül CD56 (SwissProt ID P13591), SEQ ID NO: 224
   >gi|94420689|ref|NP_000606.3| neural cell adhesion molecule 1 isoform 1 [Homo sapiens] 
MLQTKDLIWTLFFLGTAVSLQVDIVPSQGEISVGESKFFLCQVAGDAKDKDISWFSPNGEK
LTPNQQRISVVWNDDSSSTLTIYNANIDDAGIYKCVVTGEDGSESEATVNVKIFQKLMFKN
APTPQEFREGEDAVIVCDVVSSLPPTIIWKHKGRDVILKKDVRFIVLSNNYLQIRGIKKTDE
GTYRCEGRILARGEINFKDIQVIVNVPPTIQARQNIVNATANLGQSVTLVCDAEGFPEPTMS
WTKDGEQIEQEEDDEKYIFSDDSSQLTIKKVDKNDEAEYICIAENKAGEQDATIHLKVFAK
PKITYVENQTAMELEEQVTLTCEASGDPIPSITWRTSTRNISSEEKTLDGHMVVRSHARVSS
LTLKSIQYTDAGEYICTASNTIGQDSQSMYLEVQYAPKLQGPVAVYTWEGN
QVNITCEVFAYPSATISWFRDGQLLPSSNYSNIKIYNTPSASYLEVTPDSENDFGNYNCTAV
NRIGQESLEFILVQADTPSSPSIDQVEPYSSTAQVQFDEPEATGGVPILKYKAEWRAVGEEV
WHSKWYDAKEASMEGIVTIVGLKPETTYAVRLAALNGKGLGEISAASEFKTQPVQGEPSA
PKLEGQMGEDGNSIKVNLIKQDDGGSPIRHYLVRYRALSSEWKPEIRLPSGSDHVMLKSLD
WNAEYEVYVVAENQQGKSKAAHFVFRTSAQPTAIPANGSPTSGLSTGAIVGILIVIFVLLLV
VVDITCYFLNKCGLFMCIAVNLCGKAGPGAKGKDMEEGKAAFSKDESKEPIVEVRTEEER
TPNHDGGKHTEPNETTPLTEPEKGPVEAKPECQETETKPAPAEVKTVPNDATQTKENESKA
(13) das Oberflächenmolekül CD70 (SwissProt ID P32970), SEQ ID NO: 225
   >gi|4507605|ref|NP_001243.1| CD70 antigen Homo sapiens] 
MPEEGSGCSVRRRPYGCVLRAALVPLVAGLVICLVVCIQRFAQAQQQLPLESLGWDVAEL
QLNHTGPQQDPRLYWQGGPALGRSFLHGPELDKGQLRIHRDGIYMVHIQVTLAICSSTTAS
RHHPTTLAVGICSPASRSISLLRLSFHQGCTIASQRLTPLARGDTLCTNLTGTLLPSRNTDET
FFGVQWVRP
(14) das Oberflächenmolekül CD74 (SwissProt ID P04233), SEQ ID NO: 226
   >gi|10835071|ref|NP_004346.1| HLA class II histocompatibility antigen gamma chain isoform b [Homo sapiens] 
MHRRRSRSCREDQKPVMDDQRDLISNNEQLPMLGRRPGAPESKCSRGALYTGFSILVTLLL
AGQATTAYFLYQQQGRLDKLTVTSQNLQLENLRMKLPKPPKPVSKMRMATPLLMQALP
MGALPQGPMQNATKYGNMTEDHVMHLLQNADPLKVYPPLKGSFPENLRHLKNTMETID
WKVFESWMHHWLLFEMSRHSLEQKPTDAPPKESLELEDPSSGLGVTKQDLGPVPM
(15) das B-Lymphozyten Antigen CD19 (SwissProt ID P15391), SEQ ID NO: 227
   >gi|296010921|ref|NP_001171569.1| B-lymphocyte antigen CD19 isoform 1-Vorläufer [Homo sapiens] 
MPPPRLLFFLLFLTPMEVRPEEPLVVKVEEGDNAVLQCLKGTSDGPTQQLTWSRESPLKPF
LKLSLGLPGLGIHMRPLAIWLFIFNVSQQMGGFYLCQPGPPSEKAWQPGWTVNVEGSGEL
 
FRWNVSDLGGLGCGLKNRSSEGPSSPSGKLMSPKLYVWAKDRPEIWEGEPPCLPPRDSLN
QSLSQDLTMAPGSTLWLSCGVPPDSVSRGPLSWTHVHPKGPKSLLSLELKDDRPARDMW
VMETGLLLPRATAQDAGKYYCHRGNLTMSFHLEITARPVLWHWLLRTGGWKVSAVTLA
YLIFCLCSLVGILHLQRALVLRRKRKRMTDPTRRFFKVTPPPGSGPQNQYGNVLSLPTPTSG
LGRAQRWAAGLGGTAPSYGNPSSDVQADGALGSRSPPGVGPEEEEGEGYEEPDSEEDSEF
YENDSNLGQDQLSQDGSGYENPEDEPLGPEDEDSFSNAESYENEDEELTQPVARTMDFLSP
HGSAWDPSREATSLAGSQSYEDMRGILYAAPQLRSIRGQPGPNHEEDADSYENMDNPDGP
DPAWGGGGRMGTWSTR
(16) das Oberflächenprotein Mucin-1 (SwissProt ID P15941), SEQ ID NO: 228
   >gi|65301117|ref|NP_002447.4| mucin-1 isoform 1-Vorläufer [Homo sapiens] 
MTPGTQSPFFLLLLLTVLTVVTGSGHASSTPGGEKETSATQRSSVPSSTEKNALSTGVSFFF
LSFHISNLQFNSSLEDPSTDYYQELQRDISEMFLQIYKQGGFLGLSNIKFRPGSVVVQLTLAF
REGTINVHDVETQFNQYKTEAASRYNLTISDVSVSDVPFPFSAQSGAGVPGWGIALLVLVC
VLVALAIVYLIALAVCQCRRKNYGQLDIFPARDTYHPMSEYPTYHTHGRYVPPSSTDRSPY
EKVSAGNGGSSLSYTNPAVAATSANL
(17) das Oberflächenprotein CD138 (SwissProt ID P18827), SEQ ID NO: 229
   >gi|29568086|ref|NP_002988.3| syndecan-1-Vorläufer [Homo sapiens] 
MRRAALWLWLCALALSLQPALPQIVATNLPPEDQDGSGDDSDNFSGSGAGALQDITLSQQ
TPSTWKDTQLLTAIPTSPEPTGLEATAASTSTLPAGEGPKEGEAVVLPEVEPGLTAREQEAT
PRPRETTQLPTTHQASTTTATTAQEPATSHPHRDMQPGHHETSTPAGPSQADLHTPHTEDG
GPSATERAAEDGASSQLPAAEGSGEQDFTFETSGENTAVVAVEPDRRNQSPVDQGATGAS
QGLLDRKEVLGGVIAGGLVGLIFAVCLVGFMLYRMKKKDEGSYSLEEPKQANGGAYQKP
TKQEEFYA
(18) das Integrin alphaV (Genbank Accession No.: NP_002201.1), SEQ ID NO: 230
   >gi|4504763|ref|NP_002201.1| integrin alpha-V isoform 1-Vorläufer [Homo sapiens] 
MAFPPRRRLRLGPRGLPLLLSGLLLPLCRAFNLDVDSPAEYSGPEGSYFGFAVDFFVPSASS
RMFLLVGAPKANTTQPGIVEGGQVLKCDWSSTRRCQPIEFDATGNRDYAKDDPLEFKSHQ
WFGASVRSKQDKILACAPLYHWRTEMKQEREPVGTCFLQDGTKTVEYAPCRSQDIDADG
QGFCQGGFSIDFTKADRVLLGGPGSFYWQGQLISDQVAEIVSKYDPNVYSIKYNNQLATRT
AQAIFDDSYLGYSVAVGDFNGDGIDDFVSGVPRAARTLGMVYIYDGKNMSSLYNFTGEQ
MAAYFGFSVAATDINGDDYADVFIGAPLFMDRGSDGKLQEVGQVSVSLQRASGDFQTTK
LNGFEVFARFGSAIAPLGDLDQDGFNDIAIAAPYGGEDKKGIVYIFNGRSTGLNAVPSQILE
GQWAARSMPPSFGYSMKGATDIDKNGYPDLIVGAFGVDRAILYRARPVITVNAGLEVYPS
ILNQDNKTCSLPGTALKVSCFNVRFCLKADGKGVLPRKLNFQVELLLDKLKQKGAIRRAL
FLYSRSPSHSKNMTISRGGLMQCEELIAYLRDESEFRDKLTPITIFMEYRLDYRTAADTTGL
QPILNQFTPANISRQAHILLDCGEDNVCKPKLEVSVDSDQKKIYIGDDNPLTLIVKAQNQGE
GAYEAELIVSIPLQADFIGVVRNNEALARLSCAFKTENQTRQVVCDLGNPMKAGTQLLAG
LRFSVHQQSEMDTSVKFDLQIQSSNLFDKVSPVVSHKVDLAVLAAVEIRGVSSPDHIFLPIP
NWEHKENPETEEDVGPVVQHIYELRNNGPSSFSKAMLHLQWPYKYNNNTLLYILHYDIDG
PMNCTSDMEINPLRIKISSLQTTEKNDTVAGQGERDHLITKRDLALSEGDIHTLGCGVAQC
LKIVCQVGRLDRGKSAILYVKSLLWTETFMNKENQNHSYSLKSSASFNVIEFPYKNLPIEDI
TNSTLVTTNVTWGIQPAPMPVPVWVIILAVLAGLLLLAVLVFVMYRMGFFKRVRPPQEEQ
EREQLQPHENGEGNSET
(19) das teratocarcinoma-derived growth factor 1 Protein TDGF1 (Genbank Accession No.: NP_003203.1), SEQ ID NO: 231
   >gi|4507425|ref|NP_003203.1| teratocarcinoma-derived growth factor 1 isoform 1-Vorläufer [Homo sapiens] 
MDCRKMARFSYSVIWIMAISKVFELGLVAGLGHQEFARPSRGYLAFRDDSIWPQEEPAIRP
RSSQRVPPMGIQHSKELNRTCCLNGGTCMLGSFCACPPSFYGRNCEHDVRKENCGSVPHD
TWLPKKCSLCKCWHGQLRCFPQAFLPGCDGLVMDEHLVASRTPELPPSARTTTFMLVGIC
LSIQSYY
(20) das Prostata spezifische Membranantigen PSMA (Swiss Prot ID: Q04609), SEQ ID NO: 232
   >gi|4758398|ref|NP_004467.1|glutamate carboxypeptidase 2 isoform 1 [Homo sapiens] 
MWNLLHETDSAVATARRPRWLCAGALVLAGGFFLLGFLFGWFIKSSNEATNITPKHNMK
AFLDELKAENIKKFLYNFTQIPHLAGTEQNFQLAKQIQSQWKEFGLDSVELAHYDVLLSYP
NKTHPNYISIINEDGNEIFNTSLFEPPPPGYENVSDIVPPFSAFSPQGMPEGDLVYVNYARTE
DFFKLERDMKINCSGKIVIARYGKVFRGNKVKNAQLAGAKGVILYSDPADYFAPGVKSYP
DGWNLPGGGVQRGNILNLNGAGDPLTPGYPANEYAYRRGIAEAVGLPSIPVHPIGYYDAQ
KLLEKMGGSAPPDSSWRGSLKVPYNVGPGFTGNFSTQKVKMHIHSTNEVTRIYNVIGTLR
GAVEPDRYVILGGHRDSWVFGGIDPQSGAAVVHEIVRSFGTLKKEGWRPRRTILFASWDA
EEFGLLGSTEWAEENSRLLQERGVAYINADSSIEGNYTLRVDCTPLMYSLVHNLTKELKSP
DEGFEGKSLYESWTKKSPSPEFSGMPRISKLGSGNDFEVFFQRLGIASGRARYTKNWETNK
FSGYPLYHSVYETYELVEKFYDPMFKYHLTVAQVRGGMVFELANSIVLPFDCRDYAVVLR
KYADKIYSISMKHPQEMKTYSVSFDSLFSAVKNFTEIASKFSERLQDFDKSNPIVLRMMND
QLMFLERAFIDPLGLPDRPFYRHVIYAPSSHNKYAGESFPGIYDALFDIESKVDPSKAWGEV
KRQIYVAAFTVQAAAETLSEVA
(21) die Tyrosin-Proteinkinase EPHA2 (Swiss Prot ID: P29317), SEQ ID NO: 233
   >gi|32967311|ref|NP_004422.2| ephrin type-A receptor 2-Vorläufer [Homo sapiens]
(22) das Oberflächenprotein SLC44A4 (Genbank Accession No: NP_001171515), SEQ ID NO: 234
   >gi|295849282|ref|NP_001171515.1| choline transporter-like protein 4 isoform 2 [Homo sapiens] 
MGGKQRDEDDEAYGKPVKYDPSFRGPIKNRSCTDVICCVLFLLFILGYIVVGIVAWLYGDP
RQVLYPRNSTGAYCGMGENKDKPYLLYFNIFSCILSSNIISVAENGLQCPTPQTVITSLQQEL
CPSFLLPSAPALGRCFPWTNVTPPALPGITNDTTIQQGISGLIDSLNARDISVKIFEDFAQSW
YWILVALGVALVLSLLFILLLRLVAGPLVLVLILGVLGVLAYGIYYCWEEYRVLRDKGASI
SQLGFTTNLSAYQSVQETWLAALIVLAVLEAILLLMLIFLRQRIRIAIALLKEASKAVGQMM
STMFYPLVTFVLLLICIAYWAMTALYLATSGQPQYVLWASNISSPGCEKVPINTSCNPTAH
LVNSSCPGLMCVFQGYSSKGLIQRSVFNLQIYGVLGLFWTLNWVLALGQCVLAGAFASFY
WAFHKPQDIPTFPLISAFIRTLRYHTGSLAFGALILTLVQIARVILEYIDHKLRGVQNPVARCI
MCCFKCCLWCLEKFIKFLNRNAYIMIAIYGKNFCVSAKNAFMLLMRNIVRVVVLDKVTDL
LLFFGKLLVVGGVGVLSFFFFSGRIPGLGKDFKSPHLNYYWLPIMTSILGAYVIASGFFSVF
GMCVDTLFLCFLEDLERNNGSLDRPYYMSKSLLKILGKKNEAPPDNKKRKK
(23) das Oberflächenprotein BMPR1B (SwissProt: O00238)
(24) das Transportprotein SLC7A5 (SwissProt: Q01650)
(25) das epitheliale Psostataantigen STEAP1 (SwissProt: Q9UHE8)
(26) das Ovarkarzinomantigen MUC16 (SwissProt: Q8WXI7)
(27) das Transportprotein SLC34A2 (SwissProt: O95436)
(28) das Oberflächenprotein SEMA5b (SwissProt: Q9P283)
(29) das Oberflächenprotein LYPD1 (SwissProt: Q8N2G4)
(30) der Endothelin Rezeptor Typ B EDNRB (SwissProt: P24530)
(31) das Ringfingerprotein RNF43 (SwissProt: Q68DV7)
(32) das Prostatakarzinom-assozierte Protein STEAP2 (SwissProt: Q8NFT2)
(33) der Kationenkanal TRPM4 (SwissProt: Q8TD43)
(34) der Komplementrezeptor CD21 (SwissProt: P20023)
(35) das B-Zell Antigen Rezeptorkomplex-assozierte Protein CD79b (SwissProt: P40259)
(36) das Zelladhäsionsantigen CEACAM6 (SwissProt: P40199)
(37) die Dipeptidase DPEP1 (SwissProt: P16444)
(38) der Interleukinrezeptor IL20Ralpha (SwissProt: Q9UHF4)
(39) das Proteoglykan BCAN (SwissProt: Q96GW7)
(40) der Ephrin Rezeptor EPHB2 (SwissProt: P29323)
(41) das Prostatastammzellen-assozierte Protein PSCA (Genbank Accession No: NP_005663.2 )
(42) das Oberflächenprotein LHFPL3 (SwissProt: Q86UP9)
(43) das Rezeptorprotein TNFRSF13C (SwissProt: Q96RJ3)
(44) das B-Zell Antigen Rezeptorkomplex-assozierte Protein CD79a (SwissProt: P11912)
(45) das Rezeptorprotein CXCR5 (SwissProt: P32302)
(46) der Ionenkanal P2X5 (SwissProt: Q93086)
(47) das Lymphozytenantigen CD180 (SwissProt: Q99467)
(48) das Rezeptorprotein FCRL1 (SwissProt: Q96LA6)
(49) das Rezeptorprotein FCRL5 (SwissProt: Q96RD9)
(50) das MHC Klasse II Molekül Ia Antigen HLA-DOB (Genbank Accession No: NP_002111.1)
(51) das T-Zell Protein VTCN1 (SwissProt: Q7Z7D3)
(52) TWEAKR (SEQ ID NO: 169 (Protein); SEQ ID NO: 170 (DNA).
(53) das Lymphozytenantigen CD37 (Swiss Prot: P11049)
(54) Der FGF Rezeptor 2; FGFR2 (Gene ID: 2263; Official Symbol: FGFR2). Der FGFR2 Rezeptor kommt in verschiedenen Spleißvarianten vor (alpha, beta, IIIb, IIIc). Alle Spleißevarianten können als Zielmolekül fungieren.
(55) das transmembrane Glycoprotein B7H3 (CD276; Gene ID: 80381)
(56) der B Zell Rezeptor BAFFR (CD268; Gene ID: 115650)
(57) das Rezeptorprotein ROR 1 (Gene ID: 4919)
(58) der Oberflächenrezeptor IL3RA (CD123; Gene ID: 3561)
(59) der CXC Chemokinrezeptor CXCR5 (CD185; Gene ID 643)
(60) das Rezeptorprotein Syncytin (Gene ID 30816)

In einem bevorzugten Gegenstand der Erfindung ist das Krebs-Zielmolekül ausgewählt aus der Gruppe bestehend aus den Krebs-Zielmolekülen (1) - (60), insbesondere (1), (6) und (52).

In einem weiteren besonders bevorzugten Gegenstand der Erfindung bindet der Binder an ein extrazelluläres Krebs-Zielmolekül, welches ausgewählt wird aus der Gruppe bestehend aus aus den Krebs-Zielmolekülen (1) - (60), insbesondere (1), (6) und (52).

In einem weiteren besonders bevorzugten Gegenstand der Erfindung bindet der Binder spezifisch an ein extrazelluläres Krebs-Zielmolekül, welches ausgewählt wird aus der Gruppe bestehend aus aus den Krebs-Zielmolekülen (1) - (60), insbesondere (1), (6) und (52).In einer bevorzugten Ausführungsform wird der Binder nach Bindung an sein extrazelluläres Zielmolekül auf der Zielzelle durch die Bindung von der Zielzelle internalisiert. Dies bewirkt, dass das Binder-Wirkstoffkonjugat, welches ein Immunokonjugat oder ein ADC sein kann, von der Zielzelle aufgenommen wird. Anschliessend wird der Binder vorzugsweise intrazellulär, bevorzugt lysosomal, prozessiert.

In einer Ausführungsform ist der Binder ein Bindeprotein. In einer bevorzugten Ausführungsform ist der Binder ein Antikörper, ein aglykosylierter Antikörper, ein antigen-bindendes Antikörperfragment, ein multispezifischer Antikörper oder ein Antikörpermimetikum.

Bevorzugte Antikörpermimetika sind Affibodies, Adnectins, Anticalins, DARPins, Avimers, oder Nanobodies. Bevorzugte multispezifischer Antikörper sind bispezifische und trispezifische Antikörper.

In einer bevorzugten Ausführungsform ist der Binder ein Antikörper oder ein antigen-bindendes Antikörperfragment, weiter bevorzugt ist ein isolierter Antikörper oder ein isoliertes antigen-bindendes Antikörperfragment.

Bevorzugte antigen-bindende Antikörperfragmente sind Fab, Fab', F(ab')₂ und Fv Fragmente, Diabodies, DAbs, lineare Antikörper und scFv. Besonders bevorzugt sind Fab, Diabodies und scFv.

In einer besonders bevorzugten Ausführungsform ist der Binder ein Antikörper. Besonders bevorzugt sind monoklonale Antikörper oder antigen-bindende Antikörperfragmente davon. Weiter besonders bevorzugt sind humane, humanisierte oder chimäre Antikörper oder antigen-bindende Antikörperfragmente davon.

Antikörper oder antigen-bindende Antikörperfragmente, die Krebs-Zielmoleküle binden, können vom Durchschnittsfachmann mit bekannten Verfahren hergestellt werden, wie z.B. chemische Synthese oder rekombinante Expression. Binder für Krebs-Zielmoleküle können kommerziell erworben werden oder können durch den Durchschnittsfachmann mit bekannten Verfahren hergestellt werden, wie z.B. chemische Synthese oder rekombinante Expression. Weitere Verfahren zur Herstellung von Antikörpern oder antigen-bindenden Antikörperfragmenten sind in WO 2007/070538 beschrieben (siehe Seite 22 "Antibodies"). Der Fachmann kennt Verfahren wie sogenannte Phage-Display Bibliotheken (z.B. Morphosys HuCAL Gold) erstellt und zur Auffindung von Antikörpern oder antigen-bindenden Antikörperfragmenten verwendet werden können (siehe WO 2007/070538, Seite 24 ff und AK-Beispiel 1 auf Seite 70, AK-Beispiel 2 auf Seite 72). Weitere Verfahren zur Herstellung von Antikörper, die DNA Bibliotheken aus B-Zellen verwenden, sind zum Beispiel auf Seite 26 (WO 2007/070538) beschrieben. Verfahren zur Humanisierung von Antikörpern sind auf Seite 30-32 von WO2007070538 und im Detail in Queen, et al.,.Pros. Natl. Acad. Sci. USA 86:10029-10033,1989 oder in WO 90/0786 beschrieben. Des Weiteren sind dem Fachmann Verfahren zur rekombinanten Expression von Proteinen im allgemeinen und im speziellen von Antikörpern bekannt (siehe z.B. in Berger and Kimmel (Guide to Molecular Cloning Techniques, Methods in Enzymology, Vol. 152, Academic Press, Inc.); Sambrook, et al., (Molecular Cloning: A Laboratory Manual, (Second Edition, Cold Spring Harbor Laboratory Press; Cold Spring Harbor, N.Y.; 1989) Vol. 1-3); Current Protocols in Molecular Biolony, (F. M. Ausabel et al. [Eds.], Current Protocols, Green Publishing Associates, Inc. / John Wiley & Sons, Inc.); Harlow et al., (MonocIonal Antibodies: A Laboratory Manual, Cold Spring Harbor Laboratory Press (19881, Paul [Ed.]); Fundamental Immunology, (Lippincott Williams & Wilkins (1998)); and Harlow, et al., (Using Antibodies: A Laboratory Manual, Cold Spring Harbor Laboratory Press (1998)). Der Fachmann kennt die entsprechenden Vektoren, Promotoren und Signalpeptide die zur Expression eines Proteins/Antikörpers notwendig sind. Gebräuchliche Verfahren sind auch in WO 2007/070538 auf den Seiten 41 - 45 beschrieben. Verfahren zur Herstellung eines IgG1-Antikörpers sind z.B. in WO 2007/070538 in Beispiel 6 auf Seite 74 ff beschrieben. Verfahren, mit denen die Internalisierung eines Antikörpers nach Bindung an sein Antigen bestimmt werden kann, sind dem Fachmann bekannt und sind z.B. in WO 2007/070538 auf Seite 80 beschrieben. Der Fachmann kann die in WO 2007/070538 beschriebenen Verfahren, die zur Herstellung von Carboanhydrase IX (Mn)-Antikörpern verwendet wurden, anlog zur Herstellung für Antikörper mit anderer Zielmolekülspezifität verwenden.

### anti-EGFR-Antikörper

Beispiele für Antikörper, die das Krebs-Zielmoleküle EGFR binden, sind Cetuximab (INN Nummer 7906), Panitumumab (INN Nummer 8499) und Nimotuzumab (INN Nummer 8545). Cetuximab (Drug Bank Accession Number DB00002) ist ein chimärer anti-EGFR1-Antikörper, der in SP2/0 Maus-Myelom-Zellen produziert und von ImClone Systems Inc/Merck KgaA/Bristol-Myers Squibb Co vertrieben wird. Cetuximab ist indiziert zur Behandlung des metastasierenden, EGFR exprimierenden Kolorektalkarzinoms mit Wildtyp-K-Ras Gen. Er hat eine Affinität von 10⁻¹⁰M.

### Sequenz:

Cetuximab Leichte Kette (kappa), SEQ ID NO: 235:

DILLTQSPVILSVSPGERVSFSCRASQSIGTNIHWYQQRTNGSPRLLIKYASESISGIPSRFSGS
GSGTDFTLSINSVESEDIADYYCQQNNNWPTTFGAGTKLELKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC

Cetuximab Schwere Kette, SEQ ID NO: 236:

QVQLKQSGPGLVQPSQSLSITCTVSGFSLTNYGVHWVRQSPGKGLEWLGVIWSGGNTDYN
TPFTSRLSINKDNSKSQVFFKMNSLQSNDTAIYYCARALTYYDYEFAYWGQGTLVTVSAA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQGN
VFSCSVMHEALHNHYTQKSLSLSPGK

Panitumumab (INN Nummer 8499) (Drug Bank Accession Number DB01269) ist ein rekombinanter monoklonaler humaner IgG2 Antikörper, der spezifisch an den humanen EGF-Rezeptor 1 bindet und von Abgenix / Amgen vertrieben wird. Panitumumab stammt aus der Immunisierung von transgenen Mäusen (XenoMouse). Diese Mäuse sind in der Lage humane Immunglobuline (leichte und schwere Ketten) zu produzieren. Es wurde ein spezieller B-Zell-Klon ausgewählt, der Antikörper gegen EGFR produziert, und dieser mit CHO-Zellen (Chinese hamster ovary cells) immortalisiert. Diese Zellen werden jetzt für die Produktion eines zu 100 % humanen Antikörpers verwendet. Panitumumab ist indiziert zur Behandlung des EGFR-exprimierenden, metastasierenden Kolorektalkarzinoms, das refraktär gegenüber einer chemotherapeutischen Behandlung mit Fluropyrimidin, Oxaliplatin und Irinotecan ist. Er hat eine Affinität von 10⁻¹¹M.

### Sequenz:

**Panitumumab** Leichte Kette (kappa), SEQ ID NO: 237:

DIQMTQSPSSLSASVGDRVTITCQASQDISNYLNWYQQKPGKAPKLLIYDASNLETGVPSR
FSGSGSGTDFTFTISSLQPEDIATYFCQHFDHLPLAFGGGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC

**Panitumumab** Schwere Kette, SEQ ID NO: 238:

QVQLQESGPGLVKPSETLSLTCTVSGGSVSSGDYYWTWIRQSPGKGLEWIGHIYYSGNTN
YNPSLKSRLTISIDTSKTQFSLKLSSVTAADTAIYYCVRDRVTGAFDIWGQGTMVTVSSAST
KGPSVFPLAPCSRSTSESTAALGCLVKDYFPEPVTVSWNSGALTSGVHTFPAVLQSSGLYS
LSSVVTVPSSNFGTQTYTCNVDHKPSNTKVDKTVERKCCVECPPCPAPPVAGPSVFLFPPK
PKDTLMISRTPEVTCVVVDVSHEDPEVQFNWYVDGVEVHNAKTKPREEQFNSTFRVVSVL
TVVHQDWLNGKEYKCKVSNKGLPAPIEKTISKTKGQPREPQVYTLPPSREEMTKNQVSLT
CLVKGFYPSDIAVEWESNGQPENNYKTTPPMLDSDGSFFLYSKLTVDKSRWQQGNVFSCS
VMHEALHNHYTQKSLSLSPG

Bei Nimotuzumab (INN Nummer 8545) (EP 00586002, EP 00712863) handelt es sich um einen humanisierten monoklonalen IgG1 Antikörper, der spezifisch an den humanen EGF-Rezeptor 1 bindet und von YM BioScienecs Inc. (Mississauga Canada) vertrieben wird. Er wird in nichtsekretierenden NSO-Zellen (Säugerzelllinie) produziert. Nimotuzumab ist zugelassen zur Behandlung von Kopf- und Halstumoren, hoch-malignem Astrocytoma und Glioblastoma multiforme (nicht in EU und US) und Pankreaskarzinom (Orphan drug, EMA). Er hat eine Affinität von 10⁻⁸ M.

Nimotuzumab Leichte Kette (SEQ ID NO: 239):

DIQMTQSPSSLSASVGDRVTITCRSSQNIVHSNGNTYLDWYQQTPGKAPKLLIYKVSNRFS
GVPSRFSGSGSGTDFTFTISSLQPEDIATYYCFQYSHVPWTFGQGTKLQITRTVAAPSVFIFP
PSDEQLKSGTASVVCLLNNFYPREAKVQWKVDNALQSGNSQESVTEQDSKDSTYSLSSTL
TLSKADYEKHKVYACEVTHQGLSSPVTKSFNRGEC

Nimotuzumab Schwere Kette (SEQ ID NO: 240):

QVQLQQSGAEVKKPGSSVKVSCKASGYTFTNYYIYWVRQAPGQGLEWIGGINPTSGGSNF
NEKFKTRVTITADESSTTAYMELSSLRSEDTAFYFCTRQGLWFDSDGRGFDFWGQGTTVT
VSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK

Weitere Ausführungsformen für EGFR-Antikörper sind:
- Zalutumumab / 2F8 / HuMax-EGFR, Firma Genmab A/S (WO 02/100348, WO 2004/056847, INN-Nummer 8605)
- Necitumumab / 11F8, ImClone / IMC-11F8, Firma ImClone Systems Inc [Eli Lilly & Co] (WO 2005/090407 (EP 01735348-A1, US 2007/0264253-A1, US 7,598,350, WO 2005/090407-A1), INN- Nummer 9083)
- Matuzumab / anti-EGFR MAb, Merck KGaA / anti-EGFR MAb, Takeda / EMD 72000 / EMD-6200 / EMD-72000 und EMD-55900 / MAb 425 / monoclonal antibody 425, Firma Merck KGaA / Takeda (WO 92/15683, INN-Nummer 8103 (Matuzumab))
- RG-7160 / GA-201 / GA201 / R-7160 / R7160 / RG7160 / RO-4858696 / RO-5083945 / RO4858696 / RO5083945, Firma Glycart Biotechnology AG (Roche Holding AG) (WO 2010/112413-A1, WO 2010/115554)
- GT-MAB 5.2-GEX / CetuGEX, Firma Glycotope GmbH (WO 2008/028686-A2 (EP 01900750-A1, EP 01911766-A1, EP 02073842-A2, US 2010/0028947-A1)
- ISU-101, Firma Isu Abxis Inc (ISU Chemical Co Ltd) / Scancell (WO 2008/004834-A1)
- ABT-806 / mAb-806 / ch-806 / anti-EGFR monoclonal antibody 806, Firma Ludwig Institute for Cancer Research / Abbott / Life Science Pharmaceuticals (WO 02/092771, WO 2005/081854 und WO 2009/023265)
- SYM-004 (consists of two chimeric IgG1 antibodies (992 and 1024)), Firma Symphogen A/S (WO 2010/022736-A2)
- MR1-1 /MR1-1KDEL, Firma IVAX Corp (Teva Pharmaceutical Industries Ltd) (Duke University), (Patent: WO2001/062931-A2)
- Antikörper gegen die Deletionsmutante, EGFRvIII, Firma Amgen/Abgenix (WO 2005/010151, US 7,628,986)
- SC-100, Firma Scancell Ltd (WO 01/088138-A1)
- MDX-447 / EMD 82633 / BAB-447 / H 447 / MAb, EGFR, Medarex/Merck KgaA, Firma Bristol-Myers Squibb (US) / Merck KGaA (DE) / Takeda (JP), (WO 91/05871, WO 92/15683)
- anti-EGFR-Mab, Firma Xencor (WO 2005/056606)
- DXL-1218 / anti-EGFR monoclonal antibody (cancer), InNexus, Firma InNexus Biotechnology Inc, Pharmaprojects PH048638

In einer bevorzugten Ausführungsform werden die anti-EGFR Antikörper ausgewählt aus der Gruppe bestehend aus Cetuximab, Panitumumab, Nimotuzumab, Zalutumumab, Necitumumab, Matuzumab, RG-716, GT-MAB 5.2-GEX, ISU-101, ABT-806, SYM-004, MR1-1, SC-100, MDX-447, und DXL-1218.

In einer besonders bevorzugten Ausführungsform werden die anti-EGFR Antikörper ausgewählt aus der Gruppe bestehend aus Cetuximab, Panitumumab, Nimotuzumab, Zalutumumab, Necitumumab und Matuzumab.

Der Fachmann kennt Verfahren, mit denen aus den CDR Regionen der obengenannten Antikörper durch Sequenzvariationen weitere Antikörper hergestellt werden können, die eine ähnliche oder besser Affinität und/oder Spezifität zum Zielmolekül haben.

In einer weiteren Ausführungsform werden die anti-EGFR Antikörper oder antigen-bindenden Antikörperfragmente ausgewählt aus der Gruppe bestehend aus

Antikörper oder antigenbindende Antikörperfragmente, die drei CDR Regionen der leichten Kette und die drei CDR Regionen der schweren Kette eines der folgenden Antikörper umfasst: Cetuximab, Panitumumab, Nimotuzumab, Zalutumumab, Necitumumab, Matuzumab, RG-716, GT-MAB 5.2-GEX, ISU-101, ABT-806, SYM-004, MR1-1, SC-100, MDX-447, und DXL-1218.

In einer weiteren Ausführungsform werden die anti-EGFR Antikörper oder antigen-bindenden Antikörperfragmente ausgewählt aus der Gruppe bestehend aus

Antikörper oder antigenbindende Antikörperfragmente, die drei CDR Regionen der leichten Kette und die drei CDR Regionen der schweren Kette eines der folgenden Antikörper umfasst: Cetuximab, Panitumumab, Nimotuzumab, Zalutumumab, Necitumumab, Matuzumab. Diese Antikörper und antigenbindende Fragmente davon sind hierin durch Verweis aufgenommen und können im Rahmen dieser Erfindung verwendet werden.

### anti-Carboanhydrase IXAntikörper

Beispiele für Antikörper, die das Krebs-Zielmoleküle Carboanhydrase IX binden, sind in WO 2007/070538-A2 (z.B. Anspüche 1 - 16) beschrieben.

In einer bevorzugten Ausführungsform werden die anti-Carboanhydrase IX Antikörper oder antigen-bindenden Antikörperfragmente ausgewählt aus der Gruppe bestehend aus anti-Carboanhydrase IX Antikörper oder antigen-bindenden Antikörperfragmente 3ee9 (Anspruch 4 (a) in WO 2007/070538-A2), 3ef2 (Anspruch 4 (b) in WO 2007/070538-A2), 1e4 (Anspruch 4 (c) in WO 2007/070538-A2), 3a4 (Anspruch 4 (d) in WO 2007/070538-A2), 3ab4 (Anspruch 4 (e) in WO 2007/070538-A2), 3ah10 (Anspruch 4 (f) in WO 2007/070538-A2), 3bb2 (Anspruch 4 (g) in WO 2007/070538-A2), 1aa1 (Anspruch 4 (h) in WO 2007/070538-A2), 5a6 (Anspruch 4 (i) in WO 2007/070538-A2) und 5aa3 (Anspruch 4 (j) in WO 2007/070538-A2).

### anti-C4.4a Antikörper:

Erfindungsgemäß können C4.4a Antikörper verwendet werden.
Beispiele für C4.4a Antikörper und antigen-bindende Fragmente sind in WO 2012/143499 A2 beschrieben. Alle Antikörper der WO 2012/143499 A2 sind hiermit in die Beschreibung der vorliegenden Erfindung durch Verweis aufgenommen und können in der vorliegenden Erfindung verwendet werden. Die Sequenzen der Antikörper sind in Tabelle 1 der WO 2012/143499 A2 angegeben, wobei jede Zeile die jeweiligen CDR Aminosäuresequenzen der variablen leichten Kette bzw. der variablen Schweren Kette des in Spalte 1 aufgeführten Antikörpers wiedergibt.

In einer Ausführungsform werden die anti-C4.4a Antikörper oder antigen-bindenden Antikörperfragmente davon nach Bindung an eine C4.4a exprimierende Zelle von der Zelle internalisiert.

In einer weiteren Ausführungsform umfassen die anti-C4.4a Antikörper oder antigen-bindenden Antikörperfragmente mindestens eine, zwei oder drei CDR Aminosäuresequenzen eines in Tabelle 1 von WO 2012/143499 A2 oder Tabelle 2 von WO 2012/143499 A2 angeführten Antikörpers. Bevorzugte Ausführungsformen solcher Antikörper sind ebenfalls in WO 2012/143499 A2 aufgeführt und hierin durch Verweis aufgenommen.

### anti-HER2-Antikörper:

Ein Beispiel für einen Antikörper, der das Krebs-Zielmoleküle Her2 bindet, ist Trastuzumab (Genentech). Trastuzumab ist ein humanisierter Antikörper, der zur unter anderem Behandlung von Brustkrebs eingesetzt wird.

Weitere Beispiele für Antikörper, die an HER2 binden, sind neben Trastuzumab (INN 7637, CAS NR: RN: 180288-69-1) und Pertuzumab (Cas NR: 380610-27-5), auch Antikörper, wie offenbart in WO 2009/123894-A2, WO 200/8140603-A2, oder in WO 2011/044368-A2. Beispiel für ein anti-HER2 Konjugat ist Trastuzumab-Emtansine (INN-Nr. 9295). Diese Antikörper und antigenbindende Fragmente davon sind hierin durch Verweis aufgenommen und können im Rahmen dieser Erfindung verwendet werden. Zusätzlich können aglycosylierte Varianten von Trastuzumab verwendet werden, die entweder durch Deglykosylierung durch PNGaseF oder durch Mutation von N297 (Kabat-Nummerierung) der schweren Kette zu einer beliebigen Aminosäure hergestellt werden. Weiterhin können auch Varianten der Antikörper verwendet werden, die engineert wurden, um ein oder mehrere Akzeptor-Glutamine für transglutaminasevermittelte Reaktionen zu enthalten.

### anti-CD20-Antikörper:

Ein Beispiel für einen Antikörper, der das Krebs-Zielmoleküle CD20 bindet, ist Rituximab (Genentech). Rituximab (CAS-Nummer: 174722-31-7) ist ein chimärer Antikörper, der zur Behandlung von Non-Hodgkin-Lymphom verwendet wird. Diese Antikörper und antigenbindende Fragmente davon sind hierin durch Verweis aufgenommen und können im Rahmen dieser Erfindung verwendet werden.

### anti-CD52-Antikörper:

Ein Beispiel für einen Antikörper, der das Krebs-Zielmoleküle CD52 bindet, ist Alemtuzumab (Genzyme). Alemtuzumab (CAS-Nummer: 216503-57-0) ist ein humanisierter Antikörper, der zur Behandlung von chronischer lymphatischer Leukämie eingesetzt wird. Diese Antikörper und antigenbindende Fragmente davon sind hierin durch Verweis aufgenommen und können im Rahmen dieser Erfindung verwendet werden.

### anti-Mesothelin-Antikörper:

Beispiele für anti-Mesothelin -Antikörper sind z.B. WO 2009/068204 beschrieben. Alle in WO 2009/068204 beschriebenen Antikörper sind in diese Beschreibung hiermit durch Verweis aufgenommen, so dass diese Antikörper im Rahmen der hierin offenbarten Erfindung verwendet werden können.

Die erfindungsgemäßen verwendeten anti-Mesothelin-Antikörper zeichnen sich weiterhin vorzugsweise durch eine invariante Bindung an Mesothelin aus. Invariante Bindung zeichnet sich zum Beispiel dadurch aus, dass der erfindungsgemäßen verwendeten Antikörper an ein Epitop von Mesothelin bindet, welches nicht durch ein weiteres extrazelluläres Protein maskiert werden kann. Solch ein weiteres extrazelluläres Protein ist z.B. das Protein Ovarian Cancer Antigen 125 (CA125). Vorzugsweise verwendete Antikörper zeichnen sich dadurch aus, dass deren Bindung an Mesothelin nicht durch CA125 blockiert wird.

### anti-CD30-Antikörper

Beispiele für Antikörper, die das Krebs-Zielmolekül CD30 binden und zur Behandlung von Krebs z.B. Hodgkin-Lymphoma verwendet werden können, sind Brentuximab, Iratumumab und Antikörper, wie in WO 2008/092117, WO 2008/036688 oder WO 2006/089232 offenbart. Beispiele für ein anti- CD30 Konjugat ist Brentuximab Vedotin (INN-Nr. 9144). Diese Antikörper und antigenbindende Fragmente davon sind hierin durch Verweis aufgenommen und können im Rahmen dieser Erfindung verwendet werden.

### anti-CD22-Antikörper

Beispiele für Antikörper, die das Krebs-Zielmolekül CD22 binden und zur Behandlung von Krebs z.B. Lymphoma verwendet werden können, sind Inotuzumab oder Epratuzumab. Beispiele für anti-CD22 Konjugate sind Inotuzumab Ozagamycin (INN-Nr. 8574), oder anti-CD22-MMAE und anti-CD22-MC-MMAE (CAS RN: 139504-50-0 bzw. 474645-27-7). Diese Antikörper und antigenbindende Fragmente davon sind hierin durch Verweis aufgenommen und können im Rahmen dieser Erfindung verwendet werden.

### anti-CD33-Antikörper

Beispiele für Antikörper, die das Krebs-Zielmolekül CD33 binden und zur Behandlung von Krebs z.B. Leukämie verwendet werden können, sind Gemtuzumab oder Lintuzumab (INN 7580). Ein Beispiel für ein anti-CD33 Konjugat ist Gemtuzumab-Ozagamycin. Diese Antikörper und antigenbindende Fragmente davon sind hierin durch Verweis aufgenommen und können im Rahmen dieser Erfindung verwendet werden.

### anti-NMB-Antikörper

Ein Beispiel für einen Antikörper, der das Krebs-Zielmolekül NMB bindet und zur Behandlung von Krebs z.B. Melanom oder Brustkrebs verwendet werden kann, ist Glembatumumab (INN 9199). Ein Beispiel für ein anti-NMB Konjugat ist Glembatumumab Vedotin (CAS RN: 474645-27-7). Diese Antikörper und antigenbindende Fragmente davon sind hierin durch Verweis aufgenommen und können im Rahmen dieser Erfindung verwendet werden.

### Anti-CD56-Antikörper

Ein Beispiel für einen Antikörper, der das Krebs-Zielmolekül CD56 bindet und zur Behandlung von Krebs z.B. Multiples Myelom, Kleinzelliges Lungenkarzinom, MCC oder Ovarialkarzinom verwendet werden kann, ist Lorvotuzumab. Ein Beispiel für ein anti-CD56 Konjugat ist Lorvotuzumab Mertansine (CAS RN: 139504-50-0). Diese Antikörper und antigenbindende Fragmente davon sind hierin durch Verweis aufgenommen und können im Rahmen dieser Erfindung verwendet werden.

### anti-CD70-Antikörper

Beispiele für Antikörper, die das Krebs-Zielmolekül CD70 binden und zur Behandlung von Krebs z.B. Non-Hodgkin-Lymphom oder Nierenzellkrebs verwendet werden können, sind in WO 2007/038637-A2 oder WO 2008/070593-A2 offenbart. Ein Beispiel für ein anti-CD70 Konjugat ist SGN-75 (CD70 MMAF). Diese Antikörper und antigenbindende Fragmente davon sind hierin durch Verweis aufgenommen und können im Rahmen dieser Erfindung verwendet werden.

### anti-CD74-Antikörper

Ein Beispiel für einen Antikörper, der das Krebs-Zielmolekül CD74 bindet und zur Behandlung von Krebs z.B. Multiples Myelom verwendet werden kann, ist Milatuzumab. Ein Beispiel für ein anti-CD74 Konjugat ist Milatuzumab-Doxorubicin (CAS RN: 23214-92-8). Diese Antikörper und antigenbindende Fragmente davon sind hierin durch Verweis aufgenommen und können im Rahmen dieser Erfindung verwendet werden.

### anti-CD19-Antikörper

Ein Beispiel für einen Antikörper, der das Krebs-Zielmolekül CD19 bindet und zur Behandlung von Krebs z.B. Non-Hodgkin-Lymphom verwendet werden kann, ist in WO 2008/031056-A2 offenbart. Weitere Antikörper und Beispiele für ein anti-CD19 Konjugat (SAR3419) sind in WO 2008/047242-A2 offenbart. Diese Antikörper und antigenbindende Fragmente davon sind hierin durch Verweis aufgenommen und können im Rahmen dieser Erfindung verwendet werden.

### anti-Mucin-Antikörper

Beispiele für Antikörper, die das Krebs-Zielmolekül Mucin-1 binden und zur Behandlung von Krebs z.B. Non-Hodkin-Lymphom verwendet werden können, sind Clivatuzumab oder die in WO 2003/106495-A2, WO 2008/028686-A2 offenbarten Antikörper. Beispiele für anti-Mucin Konjugate sind in WO 2005/009369-A2 offenbart. Diese Antikörper und antigenbindende Fragmente davon sind hierin durch Verweis aufgenommen und können im Rahmen dieser Erfindung verwendet werden.

### anti-CD138-Antikörper

Beispiele für Antikörper, die das Krebs-Zielmolekül CD138 binden und Konjugate davon, die zur Behandlung von Krebs z.B. Multiples Myelom verwendet werden können, sind WO 2009/080829-A1, WO 2009/080830-A1 offenbart. Diese Antikörper und antigenbindende Fragmente davon sind hierin durch Verweis aufgenommen und können im Rahmen dieser Erfindung verwendet werden.

### anti-Integrin-alphaV-Antikörper

Beispiele für Antikörper, die das Krebs-Zielmolekül Integrin alphaV binden und zur Behandlung von Krebs z.B. Melanoma, Sarcoma oder Carcinoma verwendet werden können, sind Intetumumab (Cas RN: 725735-28-4), Abciximab (Cas-RN: 143653-53-6), Etaracizumab (Cas-RN: 892553-42-3) oder die in US 7,465,449, EP 719859-A1, WO 2002/012501-A1 oder WO2006/062779-A2 offenbarten Antikörper. Beispiele für anti-Integrin AlphaV Konjugate sind Intetumumab-DM4 und weitere in WO 2007/024536-A2 offenbarte ADCs. Diese Antikörper und antigenbindende Fragmente davon sind hierin durch Verweis aufgenommen und können im Rahmen dieser Erfindung verwendet werden.

### anti-TDGF1-Antikörper

Beispiele für Antikörper, die das Krebs-Zielmolekül TDGF1 binden und zur Behandlung von Krebs verwendet werden können, sind die in WO 02/077033-A1, US 7,318,924, WO 2003/083041-A2 und WO 2002/088170-A2 offenbarten Antikörper. Beispiele für anti-TDGF1 Konjugate sind in WO 2002/088170-A2 offenbart. Diese Antikörper und antigenbindende Fragmente davon sind hierin durch Verweis aufgenommen und können im Rahmen dieser Erfindung verwendet werden.

### anti-PSMA-Antikörper

Beispiele für Antikörper, die das Krebs-Zielmolekül PSMA binden und zur Behandlung von Krebs z.B. Prostatakarzinom verwendet werden können, sind die in WO 97/35616-A1, WO 99/47554-A1, WO 01/009192-A1 und WO2003/034903 offenbarten Antikörper. Beispiele für anti-PSMA Konjugate sind in WO 2009/026274-A1 und WO 2007/002222 offenbart. Diese Antikörper und antigenbindende Fragmente davon sind hierin durch Verweis aufgenommen und können im Rahmen dieser Erfindung verwendet werden.

### anti-EPHA2-Antikörper

Beispiele für Antikörper, die das Krebs-Zielmolekül EPHA2 binden, zur Herstellung eines Konjugats und zur Behandlung von Krebs verwendet werden können, sind in WO 2004/091375-A2 offenbart. Diese Antikörper und antigenbindende Fragmente davon sind hierin durch Verweis aufgenommen und können im Rahmen dieser Erfindung verwendet werden.

### anti-SLC44A4-Antikörper

Beispiele für Antikörper, die das Krebs-Zielmolekül SLC44A4 binden, zur Herstellung eines Konjugats und zur Behandlung von Krebs, z.B. Pankreas- oder Prostatakarzinom verwendet werden können, sind in WO2009/033094-A2 und US2009/0175796-A1 offenbart. Diese Antikörper und antigenbindende Fragmente davon sind hierin durch Verweis aufgenommen und können im Rahmen dieser Erfindung verwendet werden.

### anti-HLA-DOB-Antikörper

Ein Beispiel für einen Antikörper, der das Krebs-Zielmolekül HLA-DOB bindet, ist der Antikörper Lym-1 (Cas-RN: 301344-99-0), der zur Behandlung von Krebs, z.B. Non-Hodgkin-Lymphom verwendet werden kann. Beispiele für anti-HLA-DOB Konjugate sind z.B. in WO 2005/081711-A2 offenbart. Diese Antikörper und antigenbindende Fragmente davon sind hierin durch Verweis aufgenommen und können im Rahmen dieser Erfindung verwendet werden.

### anti-VTCN1-Antikörper

Beispiele für Antikörper, die das Krebs-Zielmolekül VTCN1 binden, zur Herstellung eines Konjugats und zur Behandlung von Krebs, z.B. Ovarialkarzinom, Pankreas-, Lungen-, oder Brustkrebs verwendet werden können, sind in WO 2006/074418-A2 offenbart. Diese Antikörper und antigenbindende Fragmente davon sind hierin durch Verweis aufgenommen und können im Rahmen dieser Erfindung verwendet werden.

### anti--FGFR2-Antikörper

Erfindungsgemäß können anti-FGFR2 Antikörper verwendet werden.

Beispiele für anti-FGFR2 Antikörper und antigen-bindende Fragmente sind in WO2013076186 beschrieben. Alle Antikörper der WO2013076186 sind hiermit in die Beschreibung der vorliegenden Erfindung durch Verweis aufgenommen und können in der vorliegenden Erfindung verwendet werden. Die Sequenzen der Antikörper sind in Tabelle 9 und Tabelle 10 der WO2013076186 angegeben. Bevorzugt sind Antikörper, antigen-bindende Fragmente und Varianten der Antikörper, die sich von den als M048-D01 und M047-D08 bezeichneten Antikörpern ableiten. Bevorzugte anti-FGFR2 binden an die verschiedenen von FGFR2 bekannten Spleißvarianten.

In einer Ausführungsform werden die anti- FGFR2 Antikörper oder antigen-bindenden Antikörperfragmente davon nach Bindung an eine FGFR2 exprimierende Zelle von der Zelle internalisiert.

In einer weiteren Ausführungsform umfassen die anti- FGFR2Antikörper oder antigen-bindenden Antikörperfragmente mindestens eine, zwei oder drei CDR Aminosäuresequenzen eines in Tabelle 9 oder Tabelle 10 von WO2013076186 angeführten Antikörpers. Bevorzugte Ausführungsformen solcher Antikörper sind ebenfalls in WO2013076186 aufgeführt und hierin durch Verweis aufgenommen.

### Anti-TWEAKR-Antikörper

In einer bevorzugten Ausführungsform wird, wenn in den Verfahren gemäß der vorliegenden Erfindung ein anti-TWEAKR-Antikörper oder ein antigenbindendes Fragment davon verwendet wird, dieser/dieses unter den nachfolgend beschriebenen ausgewählt (ebenfalls veröffentlicht in WO2014/198817 (A1)). Darüber hinaus sind dem Fachmann Antikörper, die an TWEAKR binden bekannt, siehe z.B. WO2009/020933(A2) oder WO2009140177 (A2). Zusätzlich können aglycosylierte Varianten der beschriebenen anti-TweakR-Antikörper verwendet werden, die entweder durch Deglykosylierung durch PNGaseF oder durch Mutation von N297 (Kabat-Nummerierung) der schweren Kette zu einer beliebigen Aminosäure hergestellt werden. Weiterhin können auch Varianten der Antikörper verwendet werden, die engineert wurden ein oder mehrere Akzeptor-Glutamine für transglutaminasevermittelte Reaktionen zu enthalten.

Die Erfindung betrifft insbesondere Konjugate mit Antikörpern oder antigenbindende Antikörperfragmente davon oder Varianten davon, die zu einer starken Aktivierung des TWEAKR (SEQ ID NO:169 (Protein); SEQ ID NO:170 (DNA)) führen, was zu einer starken Induktion von Apoptose in verschiedenen Krebszellen, die eine Überexpression des TWEAKR zeigen, führt.

Die agonistische Aktivität von TWEAKR in Bezug auf die Induktion von Apoptose und Inhibition der Proliferation der früher beschriebenen anti-TWEAKR-Antikörper (z.B. PDL-192) ist beschränkt und erreicht nicht die Wirksamkeit des endogenen Liganden TWEAK. Dieser Mangel an agonistischer Aktivität beruht nicht auf einer verminderten Affinität, da diese Antikörper an den TWEAKR mit Affinitäten binden, die verglichen mit dem endogenen Liganden TWEAK in einem ähnlichen Bereich liegen (Michaelson JS et al, MAbs. 2011 Jul-Aug;3(4):362-75; Culp PA et al, Clin Cancer Res. 2010 Jan 15;16(2):497-508), und auch Antikörper mit höherer Bindeaffinität nicht notwendigerweise eine wirksamere Signalgebungsaktivität zeigen (Culp PA, et al, Clin Cancer Res. 2010 Jan 15;16(2):497-508). Zusätzlich wurde gezeigt, dass die Anti-Tumoraktivität der früher beschriebenen Antikörper abhängig von der Fc-Effektorfunktion ist, und es wurde gezeigt, dass ADCC eine bedeutsame Rolle für die in-vivo-Wirksamkeit in Mausmodellen spielt.

### Erzeugung der anti-TWEAKR-Antikörper

Eine vollständig humane Antikörper-Phagen-Bibliothek (Hoet RM et al, Nat Biotechnol 2005;23(3):344-8) wurde verwendet, um TWEAKR-spezifische, humane, monoklonale Antikörper der vorliegenden Erfindung durch Protein-Panning (Hoogenboom H.R., Nat Biotechnol 2005;23(3):1105-16) mit dimeren, Fc-fusionierten extrazellulären Domänen von humanem und Maus-TWEAKR als immobilisiertes Target zu isolieren. 11 unterschiedliche Fab-Phagen wurden identifiziert, und die entsprechenden Antikörper wurden in einen Säuger-EgG-Expressionsvektor umkloniert, der die im löslichen FAb fehlenden CH2-CH3 Domänen bereitstellt. Nach der Identifizierung bevorzugter Antikörper, wurden diese als Volllängen-IgGs exprimiert. Aglykosylierte Varianten der beschriebenen Antikörper wurden durch Einführung der Mutationen N297A oder N297Q der schweren kette der jeweiligen Antikörper hergestellt. Diese Konstrukte wurden zum Beispiel transient in Säugerzellen exprimiert, wie von Tom et al., Kapitel 12 in Methods Express: Expression Systems herausgegeben von Micheal R. Dyson and Yves Durocher, Scion Publishing Ltd, 2007 (siehe AK-Beispiel 1) beschrieben. Die Antikörper wurden durch Protein-A-Chomatographie gereinigt und weiter über ihre Bindungsaffinität an löslichen monomeren TWEAKR mittels ELISA- und BIAcore- Analyse charakterisiert, wie in AK-Beispiel 2 beschrieben. Um die Zellbindungscharakteristika der anti-TWEAKR Antikörper zu bestimmen, wurde die Bindung mittels Druchflusszytometrie an einer Reihe von Zelllinien (HT29, HS68, HS578) getestet. NFκB -Reporter-Gen-Assays wurden durchgeführt, um die agonistische Aktivität aller 11 identifizierten Antikörper zu untersuchen (humanes IgG1). Der Antikörper mit der stärksten in-vitro-Wirksamkeit (TPP-883) wurde für weitere Wirksamkeits- und Affinitätsreifung ausgewählt (siehe AK-Beispiel 1 für Details). Eine Einzelsubstitutionsvariante mit verbesserter agonistischer Aktivität wurde nachgewiesen: G102T von CDR-H3. Letztlich wurden 7 Varianten basierend auf der erhöhten Affinität, verglichen mit der besten Einzelsubstitutionsvariante G102T, ausgewählt. Die entsprechende DNA davon wurde in einen Säuger-IgG-Expressionsvektor umkloniert und auf funktionale Aktivität im zuvor erwähnten NF-kappaB-Reporter-Gen-Assay untersucht. Letztlich wurden die erhaltenen Sequenzen mit humanen Keimbahnsequenzen verglichen und Abweichungen ohne signifikanten Einfluss auf die Affinität und die Wirksamkeit wurden angepasst. Die folgenden Antikörper wurden durch Antikörper-Bibliotheksscreening und durch Affinitäts- und/oder Wirksamkeitsreifung erhalten: "TPP-2090", "TPP-2149", "TPP-2093", "TPP-2148", "TPP-2084", "TPP-2077", "TPP-1538", "TPP-883", "TPP-1854", "TPP-1853", "TPP-1857", und "TPP-1858".

Antikörper der Erfindung können weiter durch fachbekannte Verfahren wie Antikörper-Phagen-Display-Screening (z.B. siehe Hoet RM et al, Nat Biotechnol 2005;23(3):344-8), der gut etablierten Hybridomtechnologie (z.B. siehe Köhler and Milstein Nature. 1975 Aug 7;256(5517):495-7), oder Immunisierung von Mäusen, unter anderem Immunisierung von hMAb Mäusen (z.B. VelocImmune mouse^{®}) gewonnen werden.

### Besondere Ausführungsformen von anti-TWEAKR-Antikörpern

Eine Ausführungsform der Erfindung ist die Bereitstellung von Antikörpern oder antigenbindenden Antikörperfragmenten davon oder Varianten davon, die eine starke Induktion von Caspase 3/7 in einer oder mehreren TWEAKR-exprimierenden Zelllinien zeigen. In einer bevorzugten Ausführungsform ist die eine oder mehrere TWEAKR-exprimierende Zellline in der Gruppe bestehend aus WiDr, A253, NCI-H322, HT29 und 786-O enthalten. "Induktion von Caspase 3/7" kann durch übliche fachbekannte Verfahren gemessen werden, einschließlich der hierin beschriebenen. In einer Ausführungsform wird die "Induktion von Caspase 3/7" gemäß dieser Erfindung unter Verwendung der Aktivitätsbestimmung mit Caspase 3/7-Lösung (Promega, #G8093) und Auslesen der Lumineszenz auf einem VICTOR V (Perkin Elmer) bestimmt. Am Ende der Inkubationszeit wurde die Caspase 3/7-Aktivität bestimmt und der Faktor der Induktion von Caspase 3/7 wurde im Vergleich zu unbehundelten Zellen bestimmt. Es wird gesagt, dass ein Antikörper "starke Induktion" von Caspase 3/7 aufweist, wenn der Faktor der Induktion größer als 1,2, bevorzugt größer als 1,5, noch bevorzugter größer als 1,8, noch bevorzugter größer als 2,1, noch bevorzugter größer als 2,5 ist. Es werden anti-TWEAKR-Antikörper bereitgestellt, die zu einer stärkeren Induktion von Caspase 3/7 in HT29-Zellen führen, verglichen mit früher beschriebenen agonistischen Antikörpern [z.B. PDL-192(TPP-1104), P4A8(TPP-1324), 136.1(TPP-2194)] und auch verglichen mit 300 ng/ml rekombinantem humanen TWEAK. Diese starke Wirksamkeit, Caspase 3/7 in Krebszellen zu induzieren, wurde auch in WiDr-, A253-, NIC-H322- und 786-O-Zellen beobachtet, wo die untersuchten Antikörper der Erfindung in den meisten Experimenten höhere Faktoren der Veränderung verglichen mit den Referenzantikörpern [PDL-192(TPP-1104), P4A8(TPP-1324)] und mit 300 ng/ml TWEAK induzierten. Einige Antikörper der Erfindung binden an den TWEAKR mit lediglich moderater Affinität (>10nM), die klar niedriger ist, als die Affinität des endogenen Liganden TWEAK, als auch niedriger verglichen mit anderen bekannten agonitischen Antikörpern. Diese Eigenschaft bietet weitere mögliche Vorteile wie z.B. potentiell tieferes Eindringen in den Tumor.

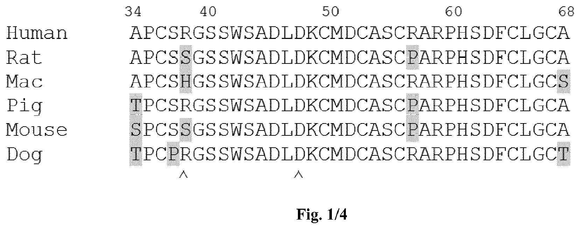

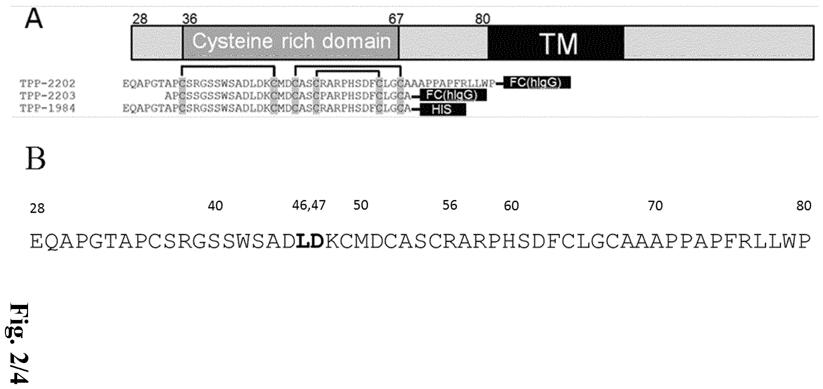

Diesbezüglich ist eine Ausführungsform der Erfindung die Bereitstellung von Antikörpern oder antigenbindenden Antikörperfragmenten davon, die spezifisch an einen TWEAKR an einem neuen Epitop binden, das durch das selektive Binden an Aspartat (D) an der Position 47 (D47) von TWEAKR (SEQ ID NO:169; siehe auch Figur 1) ausgezeichnet ist. Die identifizierten Abhängigkeiten von bestimmten TWEAKR-Aminosäuren für die Antikörperinteraktion korrelieren mit der agonistischen Aktivität, die für diese Antikörper bestimmt worden ist. Der native Ligand TWEAK zeigt eine wirksame Aktivierung des TWEAKR und bindet abhängig von Leucin 46 in der cysteinreichen Domäne von TWEAKR (Pellegrini et al, FEBS 280:1818-1829). P4A8 zeigt eine sehr niedrige agonistische Aktivität und interagiert mindestens teilweise mit Domänen außerhalb der cysteinreichen Domäne von TWEAKR. PDL-192 zeigt eine moderate agonistische Aktivität und bindet abhängig von R56 an die cysteinreiche Domäne, aber gegenüber der TWEAK-Ligandenstelle. Antikörper dieser Erfindung (z.B. TPP-2090) binden abhängig von D47, und TWEAK bindet abhängig von L46. Somit bindet TWEAK an eine ähnliche aber unterscheidbare Bindestelle (Figur 7). Deshalb binden die Antikörper dieser Erfindung, die eine starke agonistische Aktivität zeigen, an ein neues Epitop (D47-abhängig) für Antikörper, das mit sehr starker agonistischer Aktivität Zusammenhang steht.

Die Aminosäure an der Position 47 (D47) von TWEAKR (SEQ ID NO: 169) wird als kritisch für das Binden der erfindungsgemäßen Antikörper angesehen, was bedeutet, dass der Antikörper spezifisch an das D an der Position 47 (D47) von TWEAKR (SEQ ID NO: 169) bindet, wenn der Antikörper mehr als 20%, alternativ mehr als 30%, alternativ mehr als 40%, alternativ mehr als 50%, alternativ mehr als 60%, alternativ mehr als 70%, alternativ mehr als 80%, alternativ mehr als 90%, alternativ 100% seines ELISA-Signals durch Änderung dieses Rests in Alanin, wie in AK-Beispiel 2 und Figur 6 beschrieben, verliert. Alternativ bindet ein Antikörper spezifisch an das D an der Position 47 (D47) von TWEAKR (SEQ ID NO: 169), wenn der Antikörper mehr als 20%, alternativ mehr als 30%, alternativ mehr als 40%, alternativ mehr als 50%, alternativ mehr als 60%, alternativ mehr als 70%, alternativ mehr als 80%, alternativ mehr als 90%, alternativ 100% seines ELISA-Signals auf TPP-2614 verglichen mit TPP-2203 verliert. Bevorzugt bindet ein Antikörper spezifisch an das D an der Position 47 (D47) von TWEAKR (SEQ ID NO: 169), wenn der Antikörper mehr als 80% seines ELISA-Signals auf TPP-2614 verglichen mit TPP-2203 verliert.

In dieser Anmeldung wird auf die folgenden bevorzugten Antikörper der Erfindung Bezug genommen, wie in der nachstehenden Tabelle gezeigt: "TPP-2090", "TPP-2149", "TPP-2093", "TPP-2148", "TPP-2084", "TPP-2077", "TPP-1538", "TPP-883", "TPP-1854", "TPP-1853", "TPP-1857", "TPP-1858; "TPP-2658")".

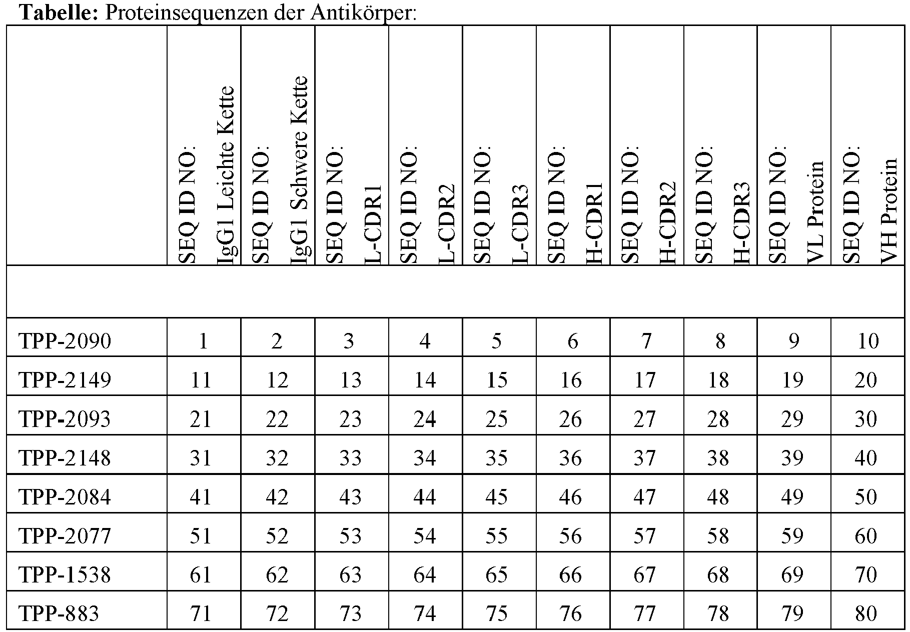

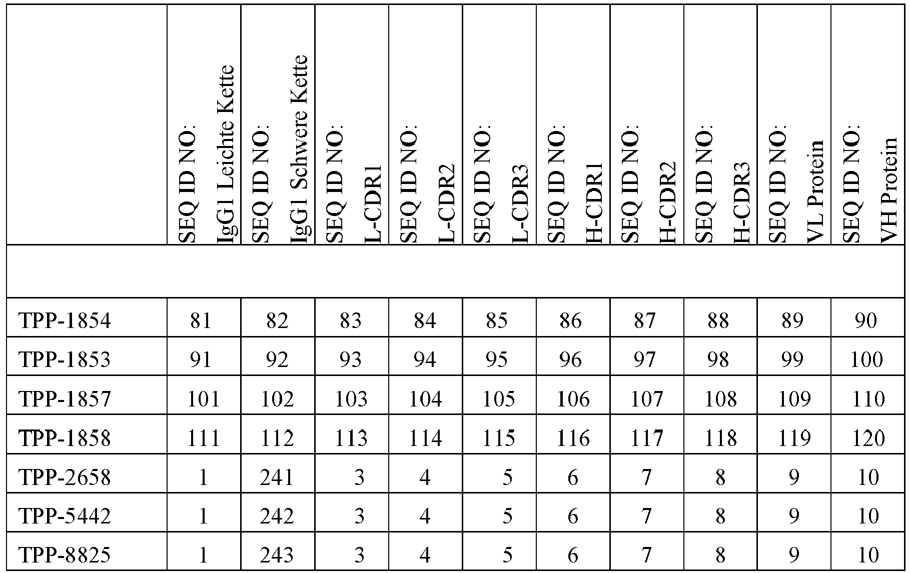

TPP-2090 ist: ein Antikörper umfassend eine Region der schweren Kette entsprechend SEQ ID NO: 2 sowie eine Region der leichten Kette entsprechend SEQ ID NO: 1. TPP-2658 ist: ein Antikörper umfassend eine Region der schweren Kette entsprechend SEQ ID NO: 241 sowie eine Region der leichten Kette entsprechend SEQ ID NO: 1. TPP-5442ist: ein Antikörper umfassend eine Region der schweren Kette entsprechend SEQ ID NO: 242 sowie eine Region der leichten Kette entsprechend SEQ ID NO: 1. TPP-8825ist: ein Antikörper umfassend eine Region der schweren Kette entsprechend SEQ ID NO: 243 sowie eine Region der leichten Kette entsprechend SEQ ID NO: 1. TPP-2149 ist: ein Antikörper umfassend eine Region der schweren Kette entsprechend SEQ ID NO: 12 sowie eine Region der leichten Kette entsprechend SEQ ID NO: 11. TPP-2093 ist: ein Antikörper umfassend eine Region der schweren Kette entsprechend SEQ ID NO: 22 sowie eine Region der leichten Kette entsprechend SEQ ID NO: 21. TPP-2148 ist: ein Antikörper umfassend eine Region der schweren Kette entsprechend SEQ ID NO: 32 sowie eine Region der leichten Kette entsprechend SEQ ID NO: 31. TPP-2084 ist: ein Antikörper umfassend eine Region der schweren Kette entsprechend SEQ ID NO: 42 sowie eine Region der leichten Kette entsprechend SEQ ID NO: 41. TPP-2077 ist: ein Antikörper umfassend eine Region der schweren Kette entsprechend SEQ ID NO: 52 sowie eine Region der leichten Kette entsprechend SEQ ID NO: 51. TPP-1538 ist: ein Antikörper umfassend eine Region der schweren Kette entsprechend SEQ ID NO: 62 sowie eine Region der leichten Kette entsprechend SEQ ID NO: 61. TPP-883 ist: ein Antikörper umfassend eine Region der schweren Kette entsprechend SEQ ID NO: 72 sowie eine Region der leichten Kette entsprechend SEQ ID NO: 71. TPP-1854 ist: ein Antikörper umfassend eine Region der schweren Kette entsprechend SEQ ID NO: 82 sowie eine Region der leichten Kette entsprechend SEQ ID NO: 81. TPP-1853 ist: ein Antikörper umfassend eine Region der schweren Kette entsprechend SEQ ID NO: 92 sowie eine Region der leichten Kette entsprechend SEQ ID NO: 91. TPP-1857 ist: ein Antikörper umfassend eine Region der schweren Kette entsprechend SEQ ID NO: 102 sowie eine Region der leichten Kette entsprechend SEQ ID NO: 101. TPP-1858 ist: ein Antikörper umfassend eine Region der schweren Kette entsprechend SEQ ID NO: 112 sowie eine Region der leichten Kette entsprechend SEQ ID NO: 111. TPP-2090 ist: ein Antikörper umfassend eine variable Region der schweren Kette entsprechend SEQ ID NO: 10 sowie eine variable Region der leichten Kette entsprechend SEQ ID NO: 9. TPP-2658 ist: ein Antikörper umfassend eine variable Region der schweren Kette entsprechend SEQ ID NO: 10 sowie eine variable Region der leichten Kette entsprechend SEQ ID NO: 9. TPP-5442 ist: ein Antikörper umfassend eine variable Region der schweren Kette entsprechend SEQ ID NO: 10 sowie eine variable Region der leichten Kette entsprechend SEQ ID NO: 9. TPP-8825 ist: ein Antikörper umfassend eine variable Region der schweren Kette entsprechend SEQ ID NO: 10 sowie eine variable Region der leichten Kette entsprechend SEQ ID NO: 9. TPP-2149 ist: ein Antikörper umfassend eine variable Region der schweren Kette entsprechend SEQ ID NO: 20 sowie eine variable Region der leichten Kette entsprechend SEQ ID NO: 19. TPP-2093 ist: ein Antikörper umfassend eine variable Region der schweren Kette entsprechend SEQ ID NO: 30 sowie eine variable Region der leichten Kette entsprechend SEQ ID NO: 29. TPP-2148 ist: ein Antikörper umfassend eine variable Region der schweren Kette entsprechend SEQ ID NO: 40 sowie eine variable Region der leichten Kette entsprechend SEQ ID NO: 39. TPP-2084 ist: ein Antikörper umfassend eine variable Region der schweren Kette entsprechend SEQ ID NO: 50 sowie eine variable Region der leichten Kette entsprechend SEQ ID NO: 49. TPP-2077 ist: ein Antikörper umfassend eine variable Region der schweren Kette entsprechend SEQ ID NO: 60 sowie eine variable Region der leichten Kette entsprechend SEQ ID NO: 59. TPP-1538 ist: ein Antikörper umfassend eine variable Region der schweren Kette entsprechend SEQ ID NO: 70 sowie eine variable Region der leichten Kette entsprechend SEQ ID NO: 69. TPP-883 ist: ein Antikörper umfassend eine variable Region der schweren Kette entsprechend SEQ ID NO: 80 sowie eine variable Region der leichten Kette entsprechend SEQ ID NO: 79. TPP-1854 ist: ein Antikörper umfassend eine variable Region der schweren Kette entsprechend SEQ ID NO: 90 sowie eine variable Region der leichten Kette entsprechend SEQ ID NO: 89. TPP-1853 ist: ein Antikörper umfassend a eine variable Region der schweren Kette entsprechend SEQ ID NO: 100 sowie eine variable Region der leichten Kette entsprechend SEQ ID NO: 99. TPP-1857 ist: ein Antikörper umfassend eine variable Region der schweren Kette entsprechend SEQ ID NO: 110 sowie eine variable Region der leichten Kette entsprechend SEQ ID NO: 109. TPP-1858 ist: ein Antikörper umfassend eine variable Region der schweren Kette entsprechend SEQ ID NO: 120 sowie eine variable Region der leichten Kette entsprechend SEQ ID NO: 119.

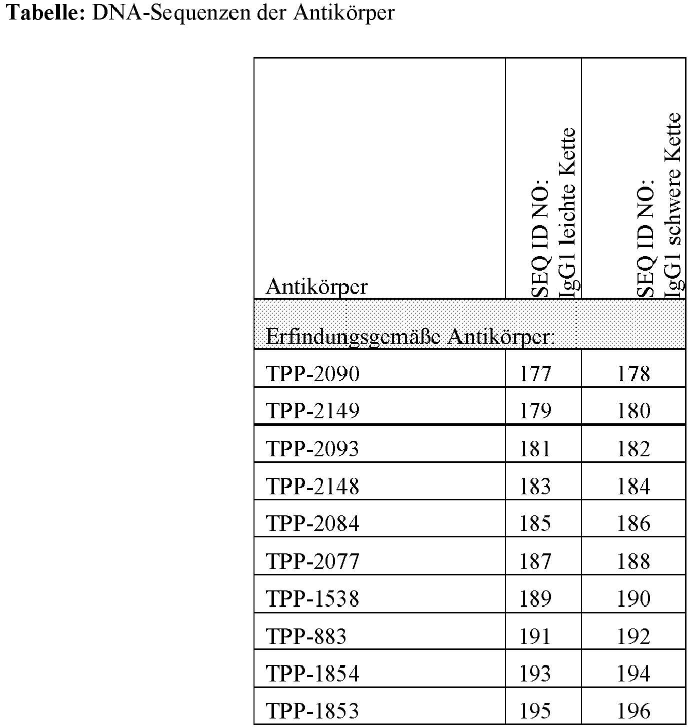

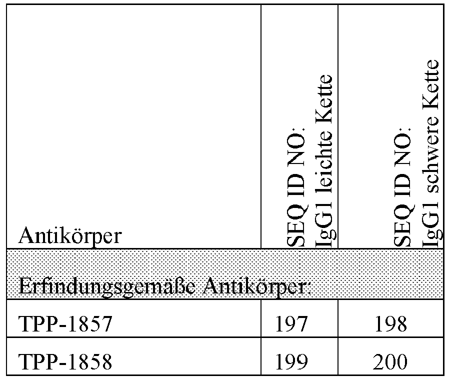

Bevorzugte Ausführungsformen des anti-TWEAKR-Antikörpers sind die folgenden:
Ein aglycoylierter anti-TWEAKR-Antikörper oder ein antigenbindendes Fragment davon, der/das spezifisch an das D an der Position 47 (D47) des TWEAKR (SEQ ID NO: 169) bindet.

Der Antikörper oder ein antigenbindendes Fragment davon gemäß Ausführungsform 1, wobei der Antikörper ein agonistischer Antikörper ist.

Der Antikörper oder ein antigenbindendes Fragment davon gemäß den Ausführungsformen 1 oder 2, der/das umfasst:
eine variable schwere Kette umfassend:
eine CDR1 der schweren Kette, die von einer Aminosäuresequenz umfassend die Formel PYPMX (SEQ ID NO: 171) kodiert wird, wobei X I oder Mist;
eine CDR2 der schweren Kette, die von einer Aminosäuresequenz umfassend die Formel YISPSGGXTHYADSVKG (SEQ ID NO: 172) kodiert wird, wobei X S oder K ist; und
eine CDR3 der schweren Kette, die von einer Aminosäuresequenz umfassend die Formel GGDTYFDYFDY (SEQ ID NO: 173) kodiert wird;
und eine variable leichte Kette umfassend:
   eine CDR1 der leichten Kette, die von einer Aminosäuresequenz umfassend die Formel RASQSISXYLN (SEQ ID NO: 174) kodiert wird, wobei X G oder S ist;
   eine CDR2 der leichten Kette, die von einer Aminosäuresequenz umfassend die Formel XASSLQS (SEQ ID NO: 175) kodiert wird, wobei X Q, A oder N ist; und
   eine CDR3 der leichten Kette, die von einer Aminosäuresequenz umfassend die Formel QQSYXXPXIT (SEQ ID NO: 176) kodiert wird, wobei X an der Position 5 T oder S ist, X an der Position 6 T oder S ist und X an der Position 8 G oder F ist.

Der Antikörper oder ein antigenbindendes Fragment davon gemäß einer der vorhergehenden Ausführungsformen umfassend:
eine variable schwere Kette umfassend die variable CDR1-Sequenz der schweren Kette, wie dargestellt durch SEQ ID NO: 6, die variable CDR2-Sequenz der schweren Kette, wie dargestellt durch SEQ ID NO: 7 und die variable CDR3-Sequenz der schweren Kette, wie dargestellt durch SEQ ID NO: 8, sowie
eine variable leichte Kette umfassend die variable CDR1-Sequenz der leichten Kette dargestellt durch SEQ ID NO: 3, die variable CDR2-Sequenz der leichten Kette dargestellt durch SEQ ID NO: 4 und die variable CDR3-Sequenz der leichten Kette dargestellt durch SEQ ID NO: 5 oder
eine variable schwere Kette umfassend die variable CDR1-Sequenz der schweren Kette, wie dargestellt durch SEQ ID NO: 16, die variable CDR2-Sequenz der schweren Kette, wie dargestellt durch SEQ ID NO: 17, die variable CDR3-Sequenz der schweren Kette, wie dargestellt durch SEQ ID NO: 18, sowie
eine variable leichte Kette umfassend die variable CDR1-Sequenz der leichten Kette dargestellt durch SEQ ID NO: 13, die variable CDR2-Sequenz der leichten Kette dargestellt durch SEQ ID NO: 14 und die variable CDR3-Sequenz der leichten Kette dargestellt durch SEQ ID NO: 15 oder
eine variable schwere Kette umfassend die variable CDR1-Sequenz der schweren Kette, wie dargestellt durch SEQ ID NO: 26, die variable CDR2-Sequenz der schweren Kette, wie dargestellt durch SEQ ID NO: 27, die variable CDR3-Sequenz der schweren Kette, wie dargestellt durch SEQ ID NO:28, sowie
eine variable leichte Kette umfassend die variable CDR1-Sequenz der leichten Kette dargestellt durch SEQ ID NO: 23, die variable CDR2-Sequenz der leichten Kette dargestellt durch SEQ ID NO: 24 und die variable CDR3-Sequenz der leichten Kette dargestellt durch SEQ ID NO:25 oder
eine variable schwere Kette umfassend die variable CDR1-Sequenz der schweren Kette, wie dargestellt durch SEQ ID NO: 36, die variable CDR2-Sequenz der schweren Kette, wie dargestellt durch SEQ ID NO: 37, die variable CDR3-Sequenz der schweren Kette, wie dargestellt durch SEQ ID NO:38, sowie
eine variable leichte Kette umfassend die variable CDR1-Sequenz der leichten Kette dargestellt durch SEQ ID NO: 33, die variable CDR2-Sequenz der leichten Kette dargestellt durch SEQ ID NO: 34 und die variable CDR3-Sequenz der leichten Kette dargestellt durch SEQ ID NO:35 oder
eine variable schwere Kette umfassend die variable CDR1-Sequenz der schweren Kette, wie dargestellt durch SEQ ID NO: 46, die variable CDR2-Sequenz der schweren Kette, wie dargestellt durch SEQ ID NO: 47, die variable CDR3-Sequenz der schweren Kette, wie dargestellt durch SEQ ID NO:48, sowie
eine variable leichte Kette umfassend die variable CDR1-Sequenz der leichten Kette dargestellt durch SEQ ID NO: 43, die variable CDR2-Sequenz der leichten Kette dargestellt durch SEQ ID NO: 44 und die variable CDR3-Sequenz der leichten Kette dargestellt durch SEQ ID NO:45 oder
eine variable schwere Kette umfassend die variable CDR1-Sequenz der schweren Kette, wie dargestellt durch SEQ ID NO: 56, die variable CDR2-Sequenz der schweren Kette, wie dargestellt durch SEQ ID NO: 57, die variable CDR3-Sequenz der schweren Kette, wie dargestellt durch SEQ ID NO:58, sowie
eine variable leichte Kette umfassend die variable CDR1-Sequenz der leichten Kette dargestellt durch SEQ ID NO: 53, die variable CDR2-Sequenz der leichten Kette dargestellt durch SEQ ID NO: 54 und die variable CDR3-Sequenz der leichten Kette dargestellt durch SEQ ID NO:55 oder
eine variable schwere Kette umfassend die variable CDR1-Sequenz der schweren Kette, wie dargestellt durch SEQ ID NO: 66, die variable CDR2-Sequenz der schweren Kette, wie dargestellt durch SEQ ID NO: 67, die variable CDR3-Sequenz der schweren Kette, wie dargestellt durch SEQ ID NO:68, sowie
eine variable leichte Kette umfassend die variable CDR1-Sequenz der leichten Kette dargestellt durch SEQ ID NO: 63, die variable CDR2-Sequenz der leichten Kette dargestellt durch SEQ ID NO: 64 und die variable CDR3-Sequenz der leichten Kette dargestellt durch SEQ ID NO:65oder
eine variable schwere Kette umfassend die variable CDR1-Sequenz der schweren Kette, wie dargestellt durch SEQ ID NO: 76, die variable CDR2-Sequenz der schweren Kette, wie dargestellt durch SEQ ID NO: 77, die variable CDR3-Sequenz der schweren Kette, wie dargestellt durch SEQ ID NO:78, sowie
eine variable leichte Kette umfassend die variable CDR1-Sequenz der leichten Kette dargestellt durch SEQ ID NO: 73, die variable CDR2-Sequenz der leichten Kette dargestellt durch SEQ ID NO: 74 und die variable CDR3-Sequenz der leichten Kette dargestellt durch SEQ ID NO:75 oder
eine variable schwere Kette umfassend die variable CDR1-Sequenz der schweren Kette, wie dargestellt durch SEQ ID NO: 86, die variable CDR2-Sequenz der schweren Kette, wie dargestellt durch SEQ ID NO: 87, die variable CDR3-Sequenz der schweren Kette, wie dargestellt durch SEQ ID NO: 88, sowie
eine variable leichte Kette umfassend die variable CDR1-Sequenz der leichten Kette dargestellt durch SEQ ID NO: 83, die variable CDR2-Sequenz der leichten Kette dargestellt durch SEQ ID NO: 84 und die variable CDR3-Sequenz der leichten Kette dargestellt durch SEQ ID NO:85 oder
eine variable schwere Kette umfassend die variable CDR1-Sequenz der schweren Kette, wie dargestellt durch SEQ ID NO: 96, die variable CDR2-Sequenz der schweren Kette, wie dargestellt durch SEQ ID NO: 97, die variable CDR3-Sequenz der schweren Kette, wie dargestellt durch SEQ ID NO:98, sowie
eine variable leichte Kette umfassend die variable CDR1-Sequenz der leichten Kette dargestellt durch SEQ ID NO: 93, die variable CDR2-Sequenz der leichten Kette dargestellt durch SEQ ID NO: 94 und die variable CDR3-Sequenz der leichten Kette dargestellt durch SEQ ID NO:95 oder
eine variable schwere Kette umfassend die variable CDR1-Sequenz der schweren Kette, wie dargestellt durch SEQ ID NO: 106, die variable CDR2-Sequenz der schweren Kette, wie dargestellt durch SEQ ID NO: 107, die variable CDR3-Sequenz der schweren Kette, wie dargestellt durch SEQ ID NO: 108, sowie
eine variable leichte Kette umfassend die variable CDR1-Sequenz der leichten Kette dargestellt durch SEQ ID NO: 103, die variable CDR2-Sequenz der leichten Kette dargestellt durch SEQ ID NO: 104 und die variable CDR3-Sequenz der leichten Kette dargestellt durch SEQ ID NO: 105 oder
eine variable schwere Kette umfassend die variable CDR1-Sequenz der schweren Kette, wie dargestellt durch SEQ ID NO: 116, die variable CDR2-Sequenz der schweren Kette, wie dargestellt durch SEQ ID NO: 117, die variable CDR3-Sequenz der schweren Kette, wie dargestellt durch SEQ ID NO:118, sowie
eine variable leichte Kette umfassend die variable CDR1-Sequenz der leichten Kette dargestellt durch SEQ ID NO: 113, die variable CDR2-Sequenz der leichten Kette dargestellt durch SEQ ID NO: 114 und die variable CDR3-Sequenz der leichten Kette dargestellt durch SEQ ID NO:115.

Der Antikörper oder das antigenbindende Fragment davon gemäß gemäß einer der vorhergehenden Ausführungsformen, umfassend:
eine variable Sequenz der schweren Kette, wie dargestellt durch SEQ ID NO:10 , sowie eine variable Sequenz der leichten Kette, wie dargestellt durch SEQ ID NO:9 oder
eine variable Sequenz der schweren Kette, wie dargestellt durch SEQ ID NO:20 , sowie eine variable Sequenz der leichten Kette, wie dargestellt durch SEQ ID NO:19 oder
eine variable Sequenz der schweren Kette, wie dargestellt durch SEQ ID NO:30 , sowie eine variable Sequenz der leichten Kette, wie dargestellt durch SEQ ID NO:29 oder
eine variable Sequenz der schweren Kette, wie dargestellt durch SEQ ID NO:40 , sowie eine variable Sequenz der leichten Kette, wie dargestellt durch SEQ ID NO:39 oder
eine variable Sequenz der schweren Kette, wie dargestellt durch SEQ ID NO:50 , sowie eine variable Sequenz der leichten Kette, wie dargestellt durch SEQ ID NO:49 oder
eine variable Sequenz der schweren Kette, wie dargestellt durch SEQ ID NO:60 , sowie eine variable Sequenz der leichten Kette, wie dargestellt durch SEQ ID NO:59 oder
eine variable Sequenz der schweren Kette, wie dargestellt durch SEQ ID NO:70 , sowie eine variable Sequenz der leichten Kette, wie dargestellt durch SEQ ID NO:69 oder
eine variable Sequenz der schweren Kette, wie dargestellt durch SEQ ID NO:80 , sowie eine variable Sequenz der leichten Kette, wie dargestellt durch SEQ ID NO:79 oder
eine variable Sequenz der schweren Kette, wie dargestellt durch SEQ ID NO:90 , sowie eine variable Sequenz der leichten Kette, wie dargestellt durch SEQ ID NO:89 oder
eine variable Sequenz der schweren Kette, wie dargestellt durch SEQ ID NO:100 , sowie eine variable Sequenz der leichten Kette, wie dargestellt durch SEQ ID NO:99 oder
eine variable Sequenz der schweren Kette, wie dargestellt durch SEQ ID NO:110 , sowie eine variable Sequenz der leichten Kette, wie dargestellt durch SEQ ID NO:109 oder
eine variable Sequenz der schweren Kette, wie dargestellt durch SEQ ID NO:120 , sowie eine variable Sequenz der leichten Kette, wie dargestellt durch SEQ ID NO:119.

Der Antikörper gemäß einer der vorhergehenden Ausführungsformen, der ein IgG Antikörper ist.

Der Antikörper gemäß einer der vorhergehenden Ausführungsformen, umfassend:
eine Sequenz der schweren Kette, wie dargestellt durch SEQ ID NO:2 , sowie eine Sequenz der leichten Kette, wie dargestellt durch SEQ ID NO:1 oder
eine Sequenz der schweren Kette, wie dargestellt durch SEQ ID NO:12, sowie eine Sequenz der leichten Kette, wie dargestellt durch SEQ ID NO:11 oder
eine Sequenz der schweren Kette, wie dargestellt durch SEQ ID NO:22, sowie eine Sequenz der leichten Kette, wie dargestellt durch SEQ ID NO:21 oder
eine Sequenz der schweren Kette, wie dargestellt durch SEQ ID NO:32, sowie eine Sequenz der leichten Kette, wie dargestellt durch SEQ ID NO:31 oder
eine Sequenz der schweren Kette, wie dargestellt durch SEQ ID NO:42, sowie eine Sequenz der leichten Kette, wie dargestellt durch SEQ ID NO:41 oder
eine Sequenz der schweren Kette, wie dargestellt durch SEQ ID NO:52, sowie eine Sequenz der leichten Kette, wie dargestellt durch SEQ ID NO:51 oder
eine Sequenz der schweren Kette, wie dargestellt durch SEQ ID NO:62, sowie eine Sequenz der leichten Kette, wie dargestellt durch SEQ ID NO:61 oder
eine Sequenz der schweren Kette, wie dargestellt durch SEQ ID NO:72, sowie eine Sequenz der leichten Kette, wie dargestellt durch SEQ ID NO:71 oder
eine Sequenz der schweren Kette, wie dargestellt durch SEQ ID NO:82, sowie eine Sequenz der leichten Kette, wie dargestellt durch SEQ ID NO:81 oder
eine Sequenz der schweren Kette, wie dargestellt durch SEQ ID NO:92, sowie eine Sequenz der leichten Kette, wie dargestellt durch SEQ ID NO:91 oder
eine Sequenz der schweren Kette, wie dargestellt durch SEQ ID NO:102, sowie eine Sequenz der leichten Kette, wie dargestellt durch SEQ ID NO:101 oder
eine Sequenz der schweren Kette, wie dargestellt durch SEQ ID NO:112, sowie eine Sequenz der leichten Kette, wie dargestellt durch SEQ ID NO:111 oder
eine Sequenz der schweren Kette, wie dargestellt durch SEQ ID NO:241 , sowie eine Sequenz der leichten Kette, wie dargestellt durch SEQ ID NO:1 oder
eine Sequenz der schweren Kette, wie dargestellt durch SEQ ID NO:242 , sowie eine Sequenz der leichten Kette, wie dargestellt durch SEQ ID NO:1 oder
eine Sequenz der schweren Kette, wie dargestellt durch SEQ ID NO:243 , sowie eine Sequenz der leichten Kette, wie dargestellt durch SEQ ID NO:1.

Das antigenbindende Fragment gemäß einer der vorhergehenden Ausführungsformen oder ein antigenbindendes Fragment eines Antikörpers gemäß einer der vorhergehenden Ausführungsformen, das ein scFv-, Fab-, Fab ' -Fragment oder ein F(ab')₂-Fragment ist.

Der Antikörper oder das antigenbindende Fragment gemäß gemäß einer der vorhergehenden Ausführungsformen, der ein monoklonaler Antikörper oder ein antigenbindendes Fragment davon ist.

Der Antikörper oder das antigenbindende Fragment gemäß einem der vorhergehenden Ansprüche, der ein humaner, humanisierter oder chimärer Antikörper oder ein antigenbindendes Fragment ist.

Besonders bevorzugt ist der anti-TWEAKR-Antikörper TPP-2658.

Es ist eine Ausführungsform der Erfindung Antikörper bereitzustellen, die geeignet für eine Transglutaminase-vermittelte Konjugation eines Kinesin Spindel Protein Inhibitors sind.

Wildtyp volle-Länge Antikörper des humanen Isotyps besitzen ein konserviertes Akzeptorglutamin an Position 295 (kabat EU Mummerierung) der schweren Kette, welches in Anwesenheit von Transglutaminase zugänglich und reaktiv ist, was dazu führt, dass ein Konjugat des Antikörpers und einer geeigneten Verbindung gebildet wird, wenn der Antikörper in nicht-glykosylierter Form vorliegt. Solchen aglykosylierten Antikörpern oder deglkosylierten Antikörpern fehlen die Glykane, die an die konservierte Glykosylierungsstelle N297 in derCH2 Domäne der Fc Region geknüpft sind. Aglykosylierte Antikörper können beispielsweise durch Mutation der Glykosylierungsstelle N297 (Kabat EU Nummerierung) der schweren Kette oder durch Expression von Antikörpern in Expressionssystemen, denen die Fähigkeit zur Glykosylierung fehlt, hergestellt werden. Methoden zur Antikörper-Deglykosylierung sind allgemein bekannt (z.B. e.g. Winkelhake & Nicolson (1976), J Biol Chem. 251(4): 1074-80)). Deglykosylierte Antikörper können zum Beispiel durch enzymatische Deglykosylierung durch PNGase F generiert werden. In einer Ausführungsform der Erfindung können aglykosylierte Antikörper durch Expression in prokaryotischen Wirten erfolgen. Geeignete prokaryotische Wirte beinhalten aber sind nicht limitiert auf E. coli, Bacillus subtilis, Salmonella typhimurium und einige Spezies der Gattung Pseudomonas, Streptomyces, und Staphylococcus. In einer anderen Ausführungsform der Erfindung können aglykosylierte Antikörper durch die Verwendung von Säugerzell-Expressionssystemen zusammen mit dem Glykosylierungsinhibitor Tunicamycin (Nose & Wigzell (1983), Proc Natl Acad Sei USA, 80(21):6632-6) erhalten werden. Hier ist die Modifikation die Verhinderung der Glykosylierung an der konservierten N-Glykosylierungsstelle N297 (Kabat-Nummerierung) der schweren Kette in der CH2-Doäne des Fc-Teils des Antikörpers.

In einer anderen Ausführungsform der Erfindung werden aglykosylierte Antikörper durch die Mutation der Glykosylierungsstelle N297 (kabat Nummerierung) der schweren Kette hergestellt. Die enzymatische Komjugation solcher engineerter aglykosylierter Antikörper wurde für Antikörpervarianten beschrieben, die die Mutationen N297D, N297Q (Jeger et al., Angewandte Chemie Int. Ed. Engl 49, 9995-9997 (2010)), oder N297S (siehe Patentanmeldungen WO2013092998A1 and WO2013092983A2) enthalten. Weiterhin zeigt diese Erfindung, dass Transglutaminase effizient die Konjugation an aglykosylierte Antikörpervarinaten katalysieren kann, die die Mutation N297A tragen (Kabat EU Nummerierung).

Zusätzliche oder alternative reaktive Reste in der Anwesenheit von Transglutaminase können durch Antikörperengineering erzeugt werden. Die Verbindungen der Erfindung schließen Glutaminengineerte Antikörper ein, in denen ein oder mehrere Aminosäuren eines wild-Typ oder parentalen Antikörpers durch Glutamine ersetzt sind, oder in denen ein Glutamin Rest, optional mit einer anderen Aminosäure (z.B. einem Tag, der das Akzeptor-Glutamin enthält), in das parentale oder wild-Typ Molekül eingeführt wird.

Die Glutaminreste eines Antikörpers, die in Anwesenheit der Transglutaminase reaktiv sind, liegen in der schweren Kette, typischerweise in der konstanten Domäne. In einer Ausführungsform ist ein Asparagin an Position N297 (Kabat Nummerierung) durch einen andren Rest als Glutamin ausgetauscht. Bevorzugt sind N297D, N297Q, N297S oder N297A, noch mehr bevorzugt N297A. Ein Antikörper mit einer N297X Substitution und einem Glutamin an Position 295 (kabat Nummerierung) hat daher ein Akzeptorglutamin pro schwerer Kette. Der komplette IgG hat daher zwei Konjugationsstellen pro Antikörper.

Die Glutaminreste eines Antikörpers die in Anwesenheit der Transglutaminase reaktiv sind liegen in der schweren Kette, typischerweise in der konstanten Domäne. In einer Ausführungsform ist ein Asparagin an Positzion N297 (Kabat Nummerierung) durch ein Glutamin ausgetauscht. Der Antikörper hat daher eine N297Q Substitution. Ein Antikörper mit einer N297Q Substizution und einem Glutamin an Position 295 (kabat Nummerierung) hat daher zwei Akzeptorglutamine und daher zwei Konjugationsstellen pro schwerer Kette. Der komplette IgG hat daher vier Konjugationsstellen pro Antikörper.

Die Glutaminreste eines Antikörpers, die in Anwesenheit der Transglutaminase reaktiv sind, liegen in der schweren Kette, typischerweise in der konstanten Domäne. In einer Ausführungsform ist ein Asparagin an Posittion N297 (Kabat Nummerierung) durch ein Glutamin ausgetauscht und an Position 295 ist das Glutamin ausgetauscht. Der Antikörper hat daher eine N297Q und eine Q295X Substitution. Bevorzugt ist eine Q295N Substitution. Ein Antikörper mit einer N297Q Substitution und keinem Glutamin an Position 295 (Kabat Nummerierung) hat daher ein Akzeptorglutamin und daher eine Konjugationsstelle pro schwerer Kette. Der komplette IgG hat daher zwei Konjugationsstellen pro Antikörper.

Bevorzugte Antikörper, die geeignet sind für eine Transglutaminase-vermittelte Konjugation sind, beinhalten also:
i. N297X Substitution, wobei X jede beliebige Aminosäure außer Asparagin ist; Mehr bevorzugt sind N297D, N297Q, N297S oder N297A, noch mehr bevorzugt sind N297A und N297Q.
ii. N297Q Substitution und eine Q295X Substitution wobei X jede Aminosäure außer Glutamin ist, bevorzugt ist Q295N.

### Isotope, Salze, Solvate, isotopische Varianten

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylpiperidin, N-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

### Besondere Ausführungsformen

Die folgenden Ausführungsformen sind besonders bevorzugt:

### Ausführungsform A:

Ein APDC der Formel 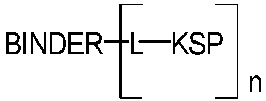 wobei KSP-L- eine Verbindung der Formel (IIa), (IIb), (IIc), (IId), (IIe) oder der folgenden Formel (IIf) ist, der Binder ein humaner, humanisierter oder chimärer monoklonaler Antikörper oder ein Antigen-bindendes Fragment hiervon ist (vorzugsweise ein anti-HER2-Antikörper, ein anti-EGFR-Antikörper oder ein anti-TWEAKR-Antikörper, besonders bevorzugt ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2658), und n eine Zahl von 1 bis 10 ist: 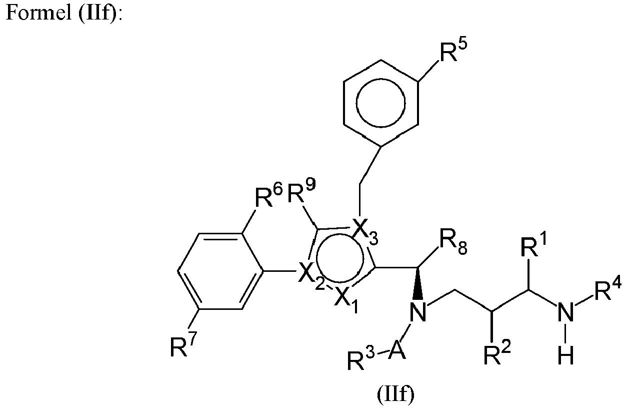 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A -C(=O)- (carbonyl) ist;
R¹ -L-#1, -H, -COOH, -CONHNH₂, -(CH₂)₁₋₃NH₂, -CONZ"(CH₂)₁₋₃ NH₂ und -CONZ"CH₂COOH ist, wobei Z" -H oder -NH₂;
R² -H ist;
R⁴ eine Gruppe der Formel R²¹-(CO)(₀₋₁₎-(P3)(₀₋₂₎-P2-NH-CH(CH₂CONH₂)-CO- oder die durch Cathepsin spaltbare Gruppe der Formel R²¹-(CO)₍₀₋₁₎-(P3)₍₀₋₂₎-P2- darstellt, ,
wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, NH-CO-Alkyl, N(Alkyl)-COAlkyl, -SO₃H, -SO₂NH₂, -SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
R³ -L-#1 oder eine C1-10-Alkyl-, die ggf. mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)₂, oder NH₂ substituiert sein kann, n für 0, 1 oder 2 steht, (wobei Alkyl vorzugsweise C₁₋₃ Alkyl bedeutet) ist;
R⁵ -H oder -F ist;
R⁶ und R⁷ unabhängig voneinander -H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist,; und
R⁹ -H oder -F ist,
wobei einer der Substituenten R¹, und R³-L-#1 darstellt, und
-L-den Linker und #1 die Bindung zum Antikörper darstellt,
sowie Salze, Solvate und Salze der Solvate des APDCs.

Der Linker ist vorzugsweise ein Linker

§-(CO)m-L1-L2-§§

wobei
m 0 oder 1 ist;
§ die Bindung an KSP darstellt und
§§ die Bindung an den Antikörper darstellt, und L2 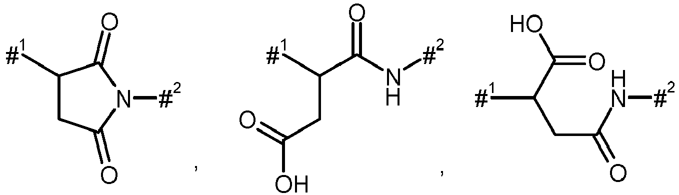 oder 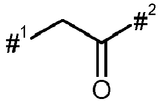 darstellt, wobei
   #¹ die Verknüpfungsstelle mit dem Schwefelatom des Antikörpers kennzeichnet,
   #² die Verknüpfungsstelle mit der Gruppe L¹ kennzeichnet,
   und L1 durch die Formel

      #¹-(NR¹⁰)ₙ-(G1)ₒ-G2-#²

      dargestellt wird,
      wobei
      R¹⁰ -H, -NH₂ oder C1-C3-Alkyl darstellt;
      G1 -NHCO- oder 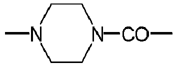 darstellt;
      n 0 oder 1 ist;
      o 0 oder 1 ist; und
      G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -SO₂-, -NH-, - CO-, -NHCO-, -CONH-, -NMe-, -NHNH-, -SO₂NHNH-, -CONHNH- und einen 3 bis 10gliedriger, aromatischen oder nicht aromatischen Heterozyklus mit bis zu 4 Heteroatomen ausgewählt aus N, O und S, oder -SO- unterbrochen sein kann (vorzugsweise 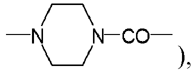
      wobei die Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein können.

Hierbei ist #¹ die Bindung zum KSP-Inhibitor und #² die Bindung zur Kopplungsgruppe zum Binder (z.B. L2).

### Ausführungsform B:

Ein APDC der Formel 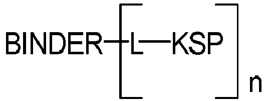 wobei KSP-L- eine Verbindung der Formel (IIa), (IIb), (IIc), (IId), (IIe), (IIf) oder der folgenden Formel (IIg)ist, der Binder ein Antikörper ist, und n eine Zahl von 1 bis 10 ist: Formel (IIg): 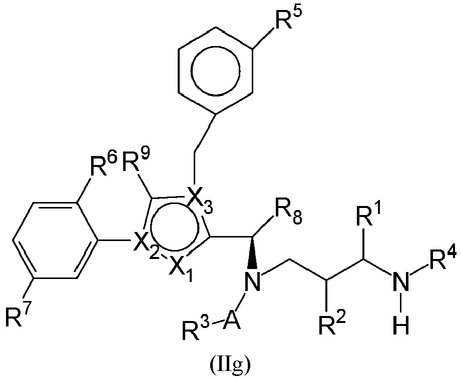 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A CO (carbonyl) ist;
R¹ -L-#1, -H, -COOH, -CONHNH₂, -(CH₂)₁₋₃NH₂, -CONZ"(CH₂)₁₋₃NH₂ und -CONZ"CH₂COOH ist, wobei Z" -H oder -NH₂;
R² -H ist;
R⁴ eine durch Legumain spaltbare Gruppe der Formel R²¹-(CO)(₀₋₁₎-(P3)(₀₋₎₂-P2-NH-CH(CH₂CONH₂)-CO- darstellt,
wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, NH-CO-Alkyl, N(Alkyl)-COAlkyl, -SO₃H, -SO₂NH₂, -SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
P2 eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist;
P3 eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His oder eine der jeweiligen N-Alkyl-Aminsoäuren, bevorzugt N-Methyl-Aminsoäuren ist;
R³ -L-#1 oder eine C₁₋₁₀-Alkyl-, die ggf. mit -OH, O-Alkyl, -SH, -S-Alkyl, -O-CO-Alkyl, -O-CO-NH-Alkyl, -NH-CO-Alkyl, -NH-CO-NH-Alkyl, -S(O)ₙ-Alkyl, -SO₂-NH-Alkyl, -NH-Alkyl, - N(Alkyl)₂, oder -NH₂ substituiert sein kann, n für 0, 1 oder 2 steht, (wobei Alkyl vorzugsweise C₁₋₃ Alkyl bedeutet) ist;
R⁵ -H oder -F ist;
R⁶ und R⁷ unabhängig voneinander -H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ -H oder -F ist,
wobei einer der Substituenten R¹ und R³-L-#1 darstellt, und -L-den Linker und # 1 die Bindung zum Antikörper darstellt.

Der -L- wird vorzugsweise dargestellt durch

§-(CO)m-L1-L2-§§

wobei
m 0 oder 1 ist;
§ die Bindung an KSP darstellt und
§§ die Bindung an den Antikörper darstellt, und
L2 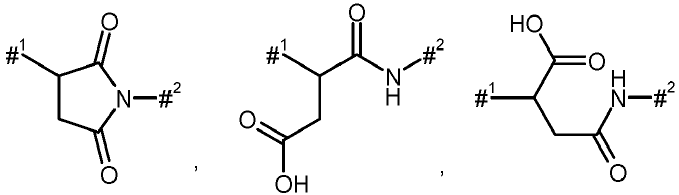 oder 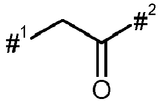 darstellt, wobei
   #¹ die Verknüpfungsstelle mit dem Schwefelatom des Antikörpers kennzeichnet,
   #² die Verknüpfungsstelle mit der Gruppe L¹ kennzeichnet,
   und L1 durch die Formel

      #¹-(NR¹⁰)ₙ-(G1)ₒ-G2-#²

      dargestellt wird,
      wobei
      R¹⁰ -H, -NH₂ oder C1-C3-Alkyl darstellt;
      G1 -NHCO- oder 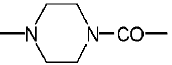 darstellt;
      n 0 oder 1 ist;
      o 0 oder 1 ist; und
      G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -SOz-, -NH-, - CO-, -NHCO-, -CONH-, -NMe-, -NHNH-, -SO₂NHNH-, -CONHNH- und einen 3 bis 10gliedriger, aromatischen oder nicht aromatischen Heterozyklus mit bis zu 4 Heteroatomen ausgewählt aus N, O und S, oder -SO- unterbrochen sein kann (vorzugsweise 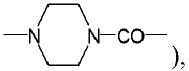
      wobei die Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein können,
      #¹ die Bindung zum KSP-Inhibitor und #² die Bindung zur Kopplungsgruppe zum Antikörper ist (z.B. L2),
      sowie Salze, Solvate und Salze der Solvate des APDCs.

Alternativ kann der Linker an eine Lysin-Seitenkette bzw. einen Lysinrest gebunden sein.

### Ausführungsform C:

Ein APDC der Formel 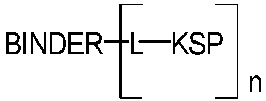 wobei KSP-L- eine Verbindung der folgenden Formel (IIa), (IIb), (IIc), (IId), (IIe), (IIf), (IIg) oder der folgenden Formel (IIh)ist, der Binder ein Antikörper, und n eine Zahl von 1 bis 10 ist: 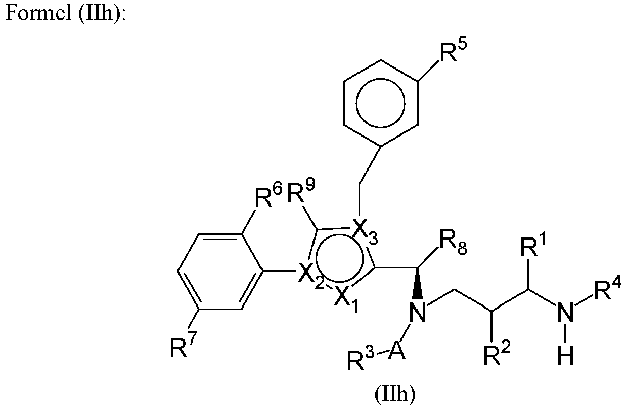 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A -C(=O)- (carbonyl) ist;
R¹ -L-#1 ist,;
R² -H ist;
R⁴ eine Gruppe der Formel R²¹-(CO)(₀₋₁₎-(P3)(₀₋₂₎-P2-NH-CH(CH₂CONH₂)-CO- oder die durch Cathepsin spaltbare Gruppe der Formel R²¹-(CO)(₀₋₁₎-(P3)₍₁₋₂₎-P2- darstellt, ,
   wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, - N(Alkyl)₂, -NH-CO-Alkyl, -N(Alkyl)-COAlkyl, -SO₃H, -SO₂NH₂, -SO₂-N(Alkyl)₂, -COOH, -CONH₂, -CON(Alkyl)₂, oder -OH substituiert sein kann, -H oder eine Gruppe -O_{X}-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
   P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
   R³ eine C₁₋₁₀-Alkyl-, die ggf. mit -OH, -O-Alkyl, -SH, -S-Alkyl, -O-CO-Alkyl, -O-CO-NH-Alkyl, -NH-CO-Alkyl, -NH-CO-NH-Alkyl, -S(O)ₙ-Alkyl, -SO₂-NH-Alkyl, -NH-Alkyl, -N(Alkyl)₂, oder -NH₂ substituiert sein kann, n für 0, 1 oder 2 steht, (wobei Alkyl vorzugsweise C₁₋₃ Alkyl bedeutet), oder -MOD ist;
   wobei -MOD -(NR¹⁰)ₙ-(G1)ₒ-G2-H darstellt, wobei
   R¹⁰ -H oder C₁-C₃-Alkyl darstellt;
   G1 -NHCO- , -CONH- oder 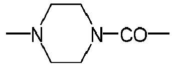 darstellt (wobei wenn G1 - NHCO- oder 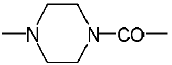 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, -SOz-, -NRY-, -NRYCO-, -CONRY-, -NRYNRY-, - SO2NRYNRY-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit -NHCONH₂, -COOH, - OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, - OH, -NH₂, -NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
R⁵ H oder Fist;
R⁶ und R⁷ unabhängig voneinander H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ H oder F ist,
wobei -L-den Linker und #1 die Bindung zum Antikörper darstellt,
wobei -L- dargestellt wird durch

   §-(CO)m-L1-L2-§§

   wobei
   m 0 oder 1 ist;
   § die Bindung an KSP darstellt und
   §§ die Bindung an den Antikörper darstellt, und
   L2 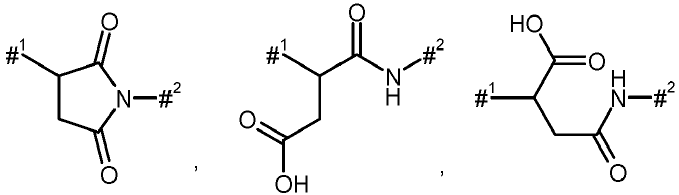 oder 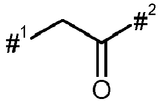 darstellt, wobei
      #¹ die Verknüpfungsstelle mit dem Schwefelatom des Antikörpers kennzeichnet,
      #² die Verknüpfungsstelle mit der Gruppe L¹ kennzeichnet,
      und L1 durch die Formel

         #¹-(NR¹⁰)ₙ-(G1)ₒ-G2-#²

         dargestellt wird,
         wobei
         R¹⁰ -H, -NH₂ oder C1-C3-Alkyl darstellt;
         G1 -NHCO- oder 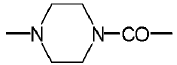 darstellt;
         n 0 oder 1 ist;
         o 0 oder 1 ist; und
         G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -SO2-, -NH-, - CO-, -NHCO-, -CONH-, -NMe-, -NHNH-, -SO2NHNH-, -CONHNH-, -CR^{x}=N-O- (wobei Rx H, C₁-C₃-Alkyl oder Phenyl darstellt) und einen 3 bis 10-gliedriger, aromatischen oder nicht aromatischen Heterozyklus mit bis zu 4 Heteroatomen ausgewählt aus N, O und S, -SO- oder - SO2- unterbrochen sein kann (vorzugsweise 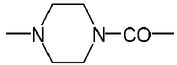 ), wobei Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein können,
         #¹ die Bindung zum KSP-Inhibitor und #² die Bindung zur Kopplungsgruppe zum Antikörper ist (z.B. L2),
         sowie Salze, Solvate und Salze der Solvate des APDCs.

### Ausführungsform D:

Ein Antikörper-Konjugat der Formel 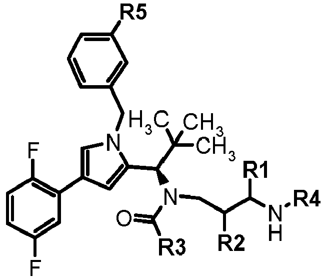 wobei
R2 und R5 -H darstellen;
R⁴ eine Gruppe der Formel R²¹-(CO)(₀₋₁₎-(P3)(₀₋₂₎-P2-NH-CH(CH₂CONH₂)-CO- oder die durch Cathepsin spaltbare Gruppe der Formel R²¹-(CO)₍₀₋₁₎-(P3)₍₀₋₂₎-P2- darstellt, ,
   wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit -NH₂, -NH-Alkyl, - N(Alkyl)₂, -NH-CO-Alkyl, -N(Alkyl)-COAlkyl, -SO₃H, -SO₂NH₂, -SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
   P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
R3 -CH2OH darstellt;
R1 -L1-L2-BINDER darstellt, wobei
L1 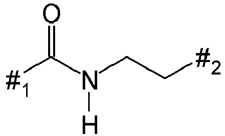 darstellt, wobei #2 die Verknüfung mit der L2 darstellt, und #1 die Verknüfung mit L1 darstellt;
und L2 eine oder beide der folgenden Strukturen der Formeln A5 und A6 darstellt: 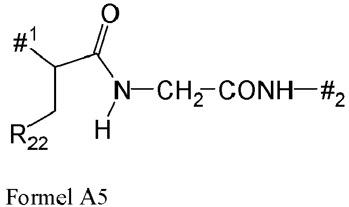 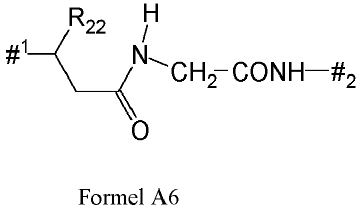 wobei
   #¹ die Verknüpfungsstelle mit dem Schwefelatom des Binders kennzeichnet,
   #² die Verknüpfungsstelle mit der Gruppe L¹ kennzeichnet, und
   R²² -COOH, -COOR, -COR, -CONHR (mit R jeweils C1-3-Alkyl), -CONH₂, vorzugsweise - COOH darstellt.

In einem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 % (jeweils bezogen auf die Gesamtanzahl von Bindungen des Linkers an den Binder), besonders bevorzugt in einer der beiden Strukturen der Formel A5 oder A6 vor:
Hierbei liegen die Strukturen der Formel A5 oder A6 in der Regel gemeinsam vor, vorzugsweise in einem Verhältnis von 60:40 bis 40:60 bezogen auf die Anzahl der Bindungen an den Binder. Die restlichen Bindungen liegen dann in der Struktur 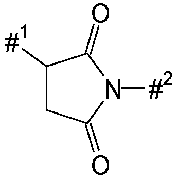 vor.

Der Binder ist vorzugsweise ein Binderprotein oder -peptid, besonders bevorzugt ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon. Besonders bevorzugt sind ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2658, oder die anti-EGFR-Antikörper Cetuximab oder Nimotuzumab. Alternativ zum Binder kann auch ein Cysteinrest vorliegen.

### Ausführungsform E:

Ein APDC der Formel 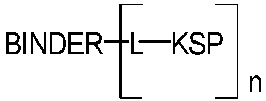 wobei KSP-L- eine Verbindung der folgenden Formel (IIa), (IIb), (IIc), (IId), (IIe), (IIf), (IIg) (IIh) oder der folgenden Formel (IIi)ist, der Binder ein Antikörper, und n eine Zahl von 1 bis 10 ist: 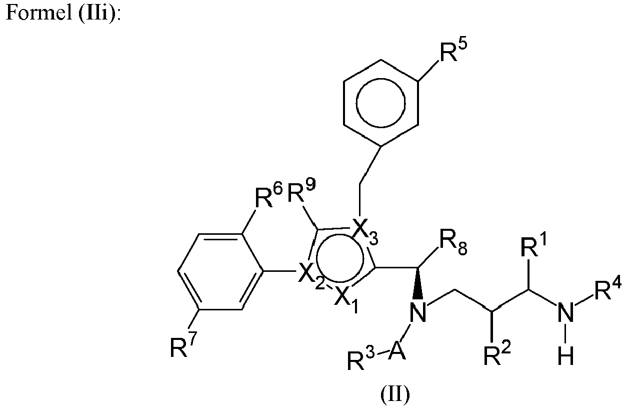 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A CO (carbonyl) ist;
R¹ -H oder -COOH ist,;
R² -H ist;
R⁴ eine Gruppe der Formel R²¹-(CO)(₀₋₁₎-(P3)(₀₋₂₎-P2-NH-CH(CH₂CONH₂)-CO- oder die durch Cathepsin spaltbare Gruppe der Formel R²¹-(CO)₍₀₋₁₎-(P3)₍₀₋₂₎-P2- darstellt,
   wobei R²¹ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit -NH₂, -NH-Alkyl, - N(Alkyl)₂, -NH-CO-Alkyl, -N(Alkyl)-COAlkyl, -SO₃H, -SO₂NH₂, -SO₂-N(Alkyl)₂, -COOH, -CONH₂, -CON(Alkyl)₂, oder -OH substituiert sein kann, -H oder eine Gruppe -Oₓ-(CH₂CH₂O)_{y}-R²² darstellt, (wobei x 0 oder 1 und v eine Zahl von 1 bis 20 darstellt, und R²² -H, -Alkyl (vorzugsweise C1-12-Alkyl), -CH2-COOH, -CH2-CH2-COOH, oder -CH2-CH2-NH2);
   P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
R³ --L-#1 ist;
R⁵ H oder F ist;
R⁶ und R⁷ unabhängig voneinander H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ -H oder -F ist,
wobei -L-den Linker und #1 die Bindung zum Antikörper darstellt,
wobei -L- dargestellt wird durch

   §-(C=O)m-L1-L2-§§

   wobei
   m 0 oder 1 ist;
   § die Bindung an KSP darstellt und
   §§ die Bindung an den Antikörper darstellt, und
   L2 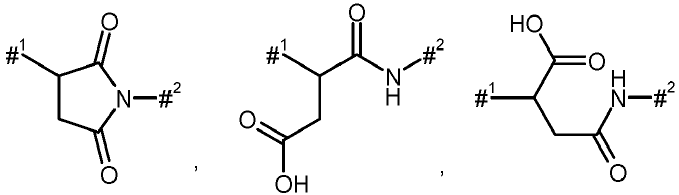 oder 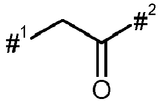 darstellt, wobei
      #¹ die Verknüpfungsstelle mit dem Schwefelatom oder Stickstoffatom des Antikörpers kennzeichnet,
      #² die Verknüpfungsstelle mit der Gruppe L¹ kennzeichnet,
      und L1 durch die Formel

         #¹-(NR¹⁰)ₙ-(G1)ₒ-G2-#²

         dargestellt wird,
         wobei
         R¹⁰ -H, -NH₂ oder C1-C3-Alkyl darstellt;
         G1 -NHCO- oder 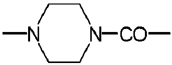 darstellt;
         n 0 oder 1 ist;
         o 0 oder 1 ist; und
         G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -SO2-, -NH-, - CO-, -NHCO-, -CONH-, -NMe-, -NHNH-, -SO2NHNH-, -CONHNH-, -CR^{x}=N-O- (wobei Rx H, C₁-C₃-Alkyl oder Phenyl darstellt) und einen 3 bis 10-gliedriger, aromatischen oder nicht aromatischen Heterozyklus mit bis zu 4 Heteroatomen ausgewählt aus N, O und S, -SO- oder - SO2- unterbrochen sein kann (vorzugsweise 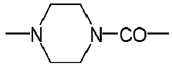 ), wobei Kohlenwasserstoffkette einschließlich der Seitenketten, sofern vorhanden, mit -NHCONH₂, -COOH, -OH, -NH₂, -NH-CNNH₂, Sulfonamid, Sulfon, Sulfoxid, oder Sulfonsäure substituiert sein können,
         #¹ die Bindung zum KSP-Inhibitor und #² die Bindung zur Kopplungsgruppe zum Antikörper ist (z.B. L2),
         sowie Salze, Solvate und Salze der Solvate des APDCs.

### Therapeutische Verwendung

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (Mammakarzinome einschließlich ductaler und lobulärer Formen, auch *in situ*)*,* Atemwegstumore (kleinzelliges und nicht-kleinzelliges Karzinom, Bronchialkarzinome), Hirntumore (z.B. des Himstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischthepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Hamtrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Die Behandlung der zuvor genannten Krebserkrankungen mittels der erfindungsgemäßen Verbindungen umfasst sowohl eine Behandlung der soliden Tumore als auch eine Behandlung metastasierter oder zirkulierender Formen hiervon.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Als geeignete Kombinationswirkstoffe seien beispielhaft genannt:
1311-chTNT, Abarelix, Abirateron, Aclarubicin, Ado-Trastuzumab Emtansin, Afatinib, Aflibercept, Aldesleukin, Alemtuzumab, Alendronsäure, Alitretinoin, Altretamin, Amifostine, Aminoglutethimid, Hexyl-5-Aminolevulinat, Amrubicin, Amsacrin, Anastrozol, Ancestim, Anethole dithiolethione, Angiotensin II, Antithrombin III, Aprepitant, Arcitumomab, Arglabin, Arsentrioxid, Asparaginase, Axitinib, Azacitidin, Belotecan, Bendamustin, Belinostat, Bevacizumab, Bexaroten, Bicalutamid, Bisantren, Bleomycin, Bortezomib, Buserelin, Bosutinib, Brentuximab Vedotin, Busulfan, Cabazitaxel, Cabozantinib, Calciumfolinat, Calciumlevofolinat, Capecitabin, Capromab, Carboplatin, Carfilzomib, Carmofur, Carmustin, Catumaxomab, Celecoxib, Celmoleukin, Ceritinib, Cetuximab, Chlorambucil, Chlormadinon, Chlormethin, Cidofovir, Cinacalcet, Cisplatin, Cladribin, Clodronsäure, Clofarabin, Copanlisib, Crisantaspase, Crizotinib, Cyclophosphamid, Cyproteron, Cytarabin, Dacarbazin, Dactinomycin, Dabrafenib, Dasatinib, Daunorubicin, Decitabin, Degarelix, Denileukin-Diftitox, Denosumab, Depreotid, Deslorelin, Dexrazoxane, Dibrospidiumchlorid, Dianhydrogalactitol, Diclofenac, Docetaxel, Dolasetron, Doxifluridin, Doxorubicin, Doxorubicin + Estron, Dronabinol, Edrecolomab, Elliptiniumacetat, Endostatin, Enocitabin, Enzalutamid, Epirubicin, Epitiostanol, Epoetin-alfa, Epoetin-beta, Epoetin-zeta, Eptaplatin, Eribulin, Erlotinib, Esomeprazol, Estramustin, Etoposid, Everolimus, Exemestan, Fadrozol, Fentanyl, Fluoxymesteron, Floxuridin, Fludarabin, Fluoruracil, Flutamid, Folinsäure, Formestan, Fosaprepitant, Fotemustin, Fulvestrant, Gadobutrol, Gadoteridol, Gadotersäure-Megluminsalz, Gadoversetamid, Gadoxetsäure Dinatriumsalz (Gd-EOB-DTPA Dinatriumsalz), Galliumnitrat, Ganirelix, Gefitinib, Gemcitabin, Gemtuzumab, Glucarpidase, Glutoxim, Goserelin, Granisetron, Granulocyten Kolonie stimulierender Factor (G-CSF), Granulocyten Macrophagen Kolonie stimulierender Factor (GM-CSF), Histamindihydrochlorid, Histrelin, Hydroxycarbamid,, I-125-Seeds, Ibandronsäure, Ibritumomab-Tiuxetan, Ibrutinib, Idarubicin, Ifosfamid, Imatinib, Imiquimod, Improsulfan, Indisetron, Incadronic acid, Ingenolmebutat, Interferon-alfa, Interferon-beta, Interferon-gamma, Iobitridol, Iobenguane (1231), Iomeprol, Ipilimumab, Irinotecan, Itraconazole, Ixabepilon, Lanreotid, Lansoprazole, Lapatinib, Lasocholine, Lenalidomid, Lentinan, Letrozol, Leuprorelin, Levamisol, Levonorgestrel, Levothyroxin-Natrium, Lipegfilgrastim, Lisurid, Lobaplatin, Lomustin, Lonidamin, Masoprocol, Medroxyprogesteron, Megestrol, Melarsoprol, Melphalan, Mepitiostan, Mercaptopurin, Mesna, Methadon, Methotrexat, Methoxsalen, Methylaminolevulinat, Methylprednisolon, Methyltestosteron, Metirosin, Mifamurtid, Miltefosin, Miriplatin, Mitobronitol, Mitoguazon, Mitolactol, Mitomycin, Mitotan, Mitoxantron, Mogamulizumab, Molgramostim, Mopidamol, Morphinhydrochlorid, Morphinsulfat, Nabilon, Nabiximols, Nafarelin, Naloxon + Pentazocin, Naltrexon, Nartograstim, Nedaplatin, Nelarabin, Neridronsäure, Nivolumabpentetreotid, Nilotinib, Nilutamid, Nimorazol, Nimotuzumab, Nimustin, Nitracrin, Nivolumab, Obinutuzumab, Octreotid, Ofatumumab, Omacetaxin-Mepesuccinat, Omeprazol, Ondansetron, Orgotein, Orilotimod, Oxaliplatin, Oxycodon, Oxymetholon, Ozogamicin, p53-Gentherapie, Paclitaxel, Palladium-103-Seed, Palonosetron, Pamidronsäure, Panitumumab, Pantoprazol, Pazopanib, Pegaspargase, Pembrolizumab, Peg-interferon-alfa-2b, Pemetrexed, Pentostatin, Peplomycin, Perflubutane, Perfosfamid, Pertuzumab, Picibanil, Pilocarpin, Pirarubicin, Pixantron, Plerixafor, Plicamycin, Poliglusam, Polyestradiolphosphat, Polyvinylpyrrolidone + Natriumhyaluronat, Polysaccharid-K, Pomalidomid, Ponatinib, Porfimer-Natrium, Pralatrexat, Prednimustin, Prednison, Procarbazin, Procodazole, Propranolol, Quinagolid, Rabeprazol, Racotumomab, Radium-223-chlorid, Radotinib, Raloxifen, Raltitrexed, Ramosetron, Ramucirumab, Ranimustin, Rasburicase, Razoxan, Refametinib, Regorafenib, Risedronsäure, Rhenium-186 Etidronat, Rituximab, Romidepsin, Romurtid, Roniciclib , Samarium (153Sm) lexidronam, Satumomab, Secretin, Sipuleucel-T, Sizofiran, Sobuzoxan, Natriumglycididazol, Sorafenib, Stanozolol, Streptozocin, Sunitinib, Talaporfin, Tamibaroten, Tamoxifen, Tapentadol, Tasonermin, Teceleukin, Technetium (99mTc) Nofetumomab Merpentan, 99mTc-HYNIC-[Tyr3]-octreotid, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Thyrotropin alfa, Tioguanin, Tocilizumab, Topotecan, Toremifen, Tositumomab, Trabectedin, Tramadol, Trastuzumab, Treosulfan, Tretinoin, Trifluridine + Tipiracil, Trametinib, Trilostan, Triptorelin, Trofosfamid, Thrombopoietin, Ubenimex, Valrubicin, Vandetanib, Vapreotid, Valatinib , Vemurafenib, Vinblastin, Vincristin, Vindesin, Vinflunin, Vinorelbin, Vismodegib, Vorinostat, Yttrium-90-Glasmikrokugeln, Zinostatin, Zinostatin-Stimalamer, Zoledronsäure, Zorubicin.

Zudem können die Antikörper ausgewählt sein aus der Klasse der MPS1-Inhibitoren oder Antikörper gegen die Targets OX-40, CD137 / 4-1BB, DR3, IDO1 / IDO2, LAG-3, und CD40.

Darüber hinaus können die erfindungsgemäßen Verbindungen auch in Kombination mit einer Strahlentherapie und/oder einer chirurgischen Intervention eingesetzt werden.

Generell können mit der Kombination von Verbindungen der vorliegenden Erfindung mit anderen, zytostatisch oder zytotoxisch wirksamen Agenzien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise, parenteral, möglicherweise inhalativ oder als Implantat bzw. Stent.

Für diese Applikationswege können die erfindungsgemäßen Verbindungen in geeigneten Applikationsformen verabreicht werden.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B.. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen oder Lyophilisaten. Bevorzugt ist die parenterale Applikation, insbesondere die intravenöse Applikation.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Beispiele

Die nachfolgenden Beispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Synthesewege:

Exemplarisch für die Ausführungsbeispiele sind in folgenden Schemata beispielhafte Synthesewege zu den Ausführungsbeispielen dargestellt. In diesen Schemata kann das Wasserstoffatom in Position R⁴ gemäß Formel IIa (d.h. in der Gruppe -NH₂) durch die Gruppe der Formel R²¹-(CO)₍₀₋₁₎-(P3)₍₀₋₂₎-P2-NH-CH(CH₂CONH₂)-CO- oder die durch Cathepsin spaltbare Gruppe der Formel R²¹-(CO)₍₋₁₎(P3)₍₀₋₂₎-P2- darstellt,
wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist;
P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist.
wobei R²¹ eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -SO₃H, -COOH, -SH, oder -OH substituiert sein kann. 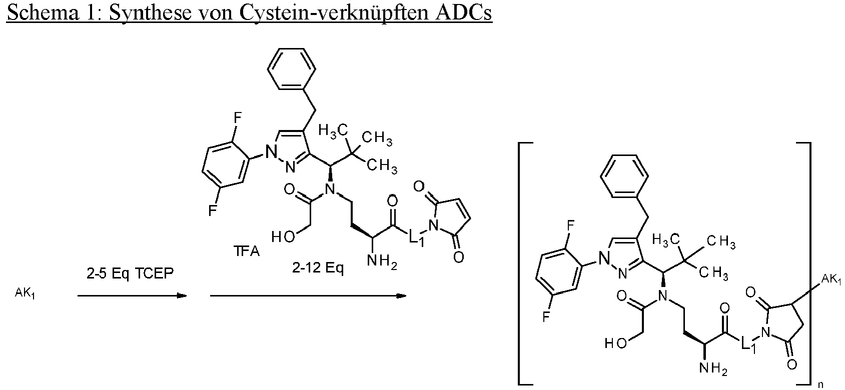 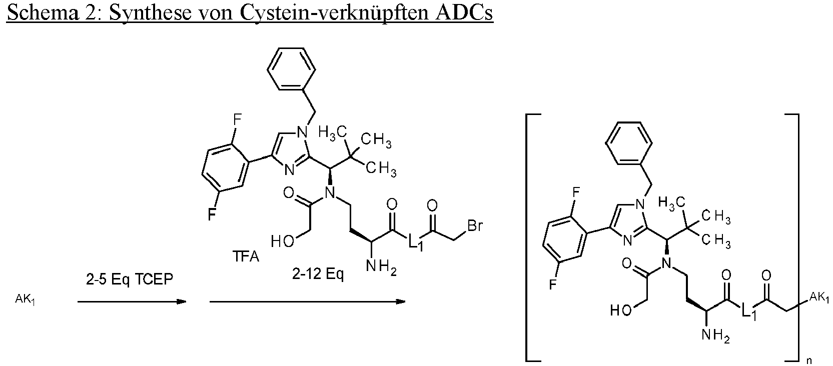 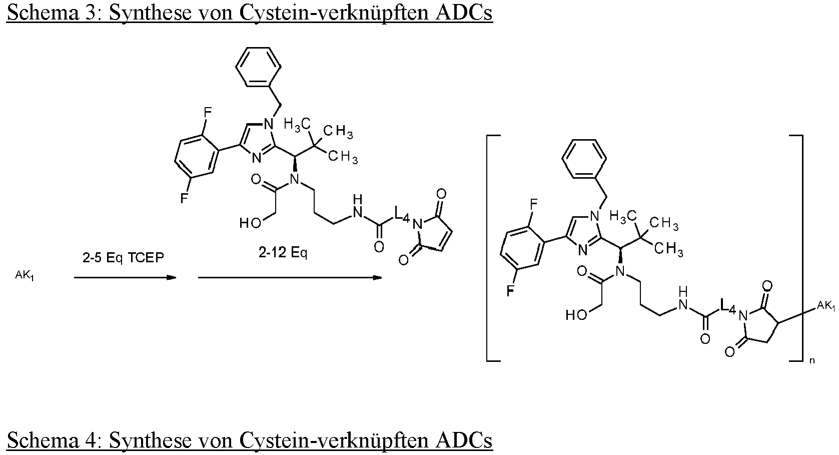 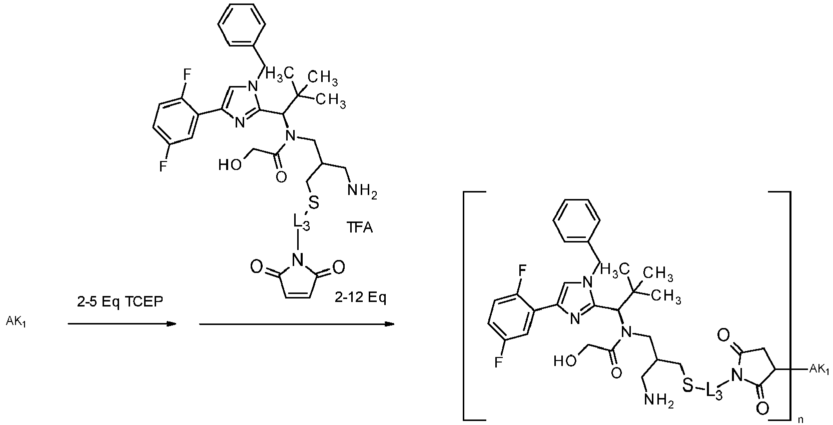 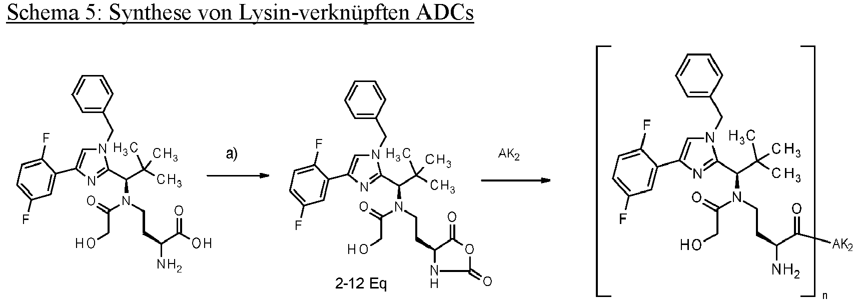 a) Triphosgen, THF, unter Argon 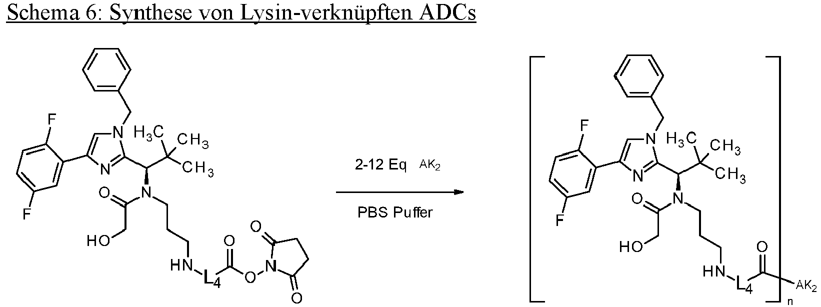 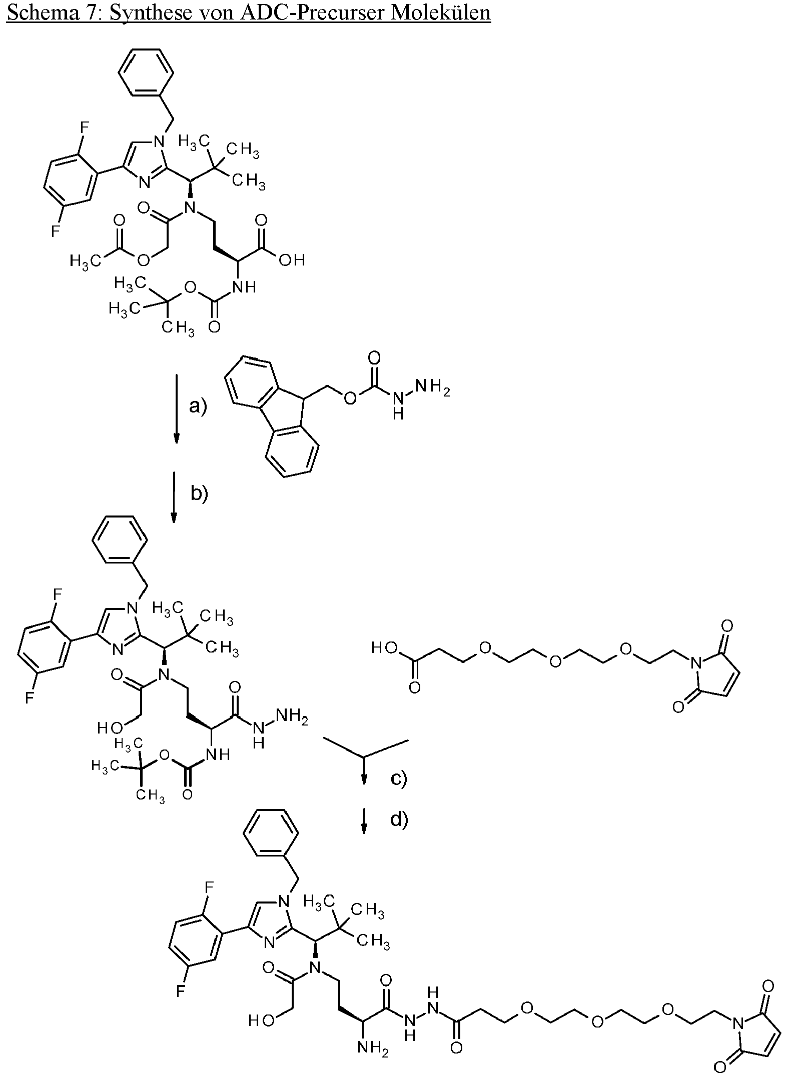 [a): EDCI, HOBT, Diisopropylethylamin, RT; b) Ethanol, Piperidin, Methylamin, Wasser, RT; c) HATU, Diisopropylethylamin, RT; d) TFA, DCM, RT] 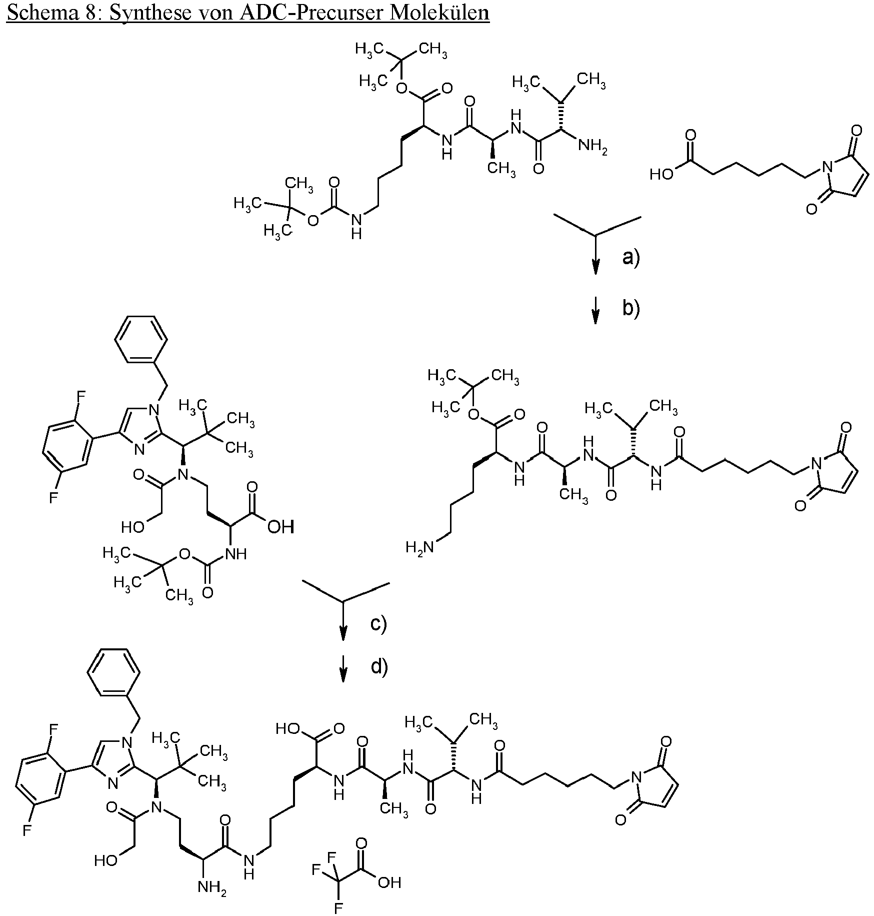 [a): beispielsweise EDCI, HOBT, Diisopropylethylamin, DMF, RT; b) beispielsweise DCM/TFA 20:1, RT; c) beispielsweise HATU, Diisopropylethylamin, DMF, RT; d) beispielsweise TFA, DCM, RT] 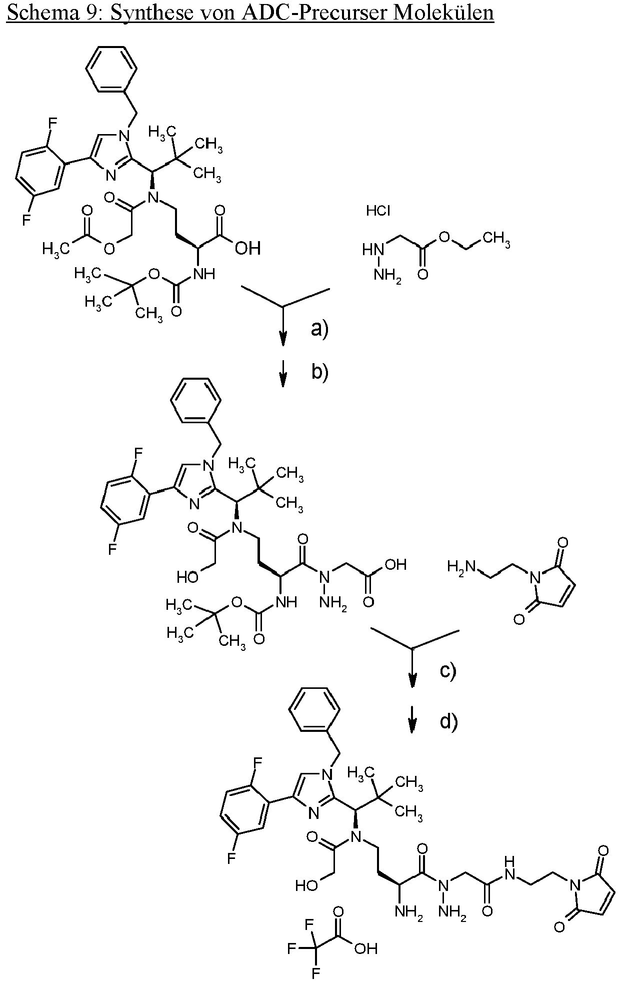 [a): beispielsweise 2-Bromo-1-ethylpyridinium tetrafluoroborate, Diisopropylethylamin, DCM, RT; b) beispielsweise 2M LiOH-Lösung, THF, Wasser, RT, HPLC-Trennung der Regiosisomeren; c) beispielsweise EDCI, HOBT, Diisopropylethylamin, DMF, RT; d) beispielsweise TFA, DCM, RT] 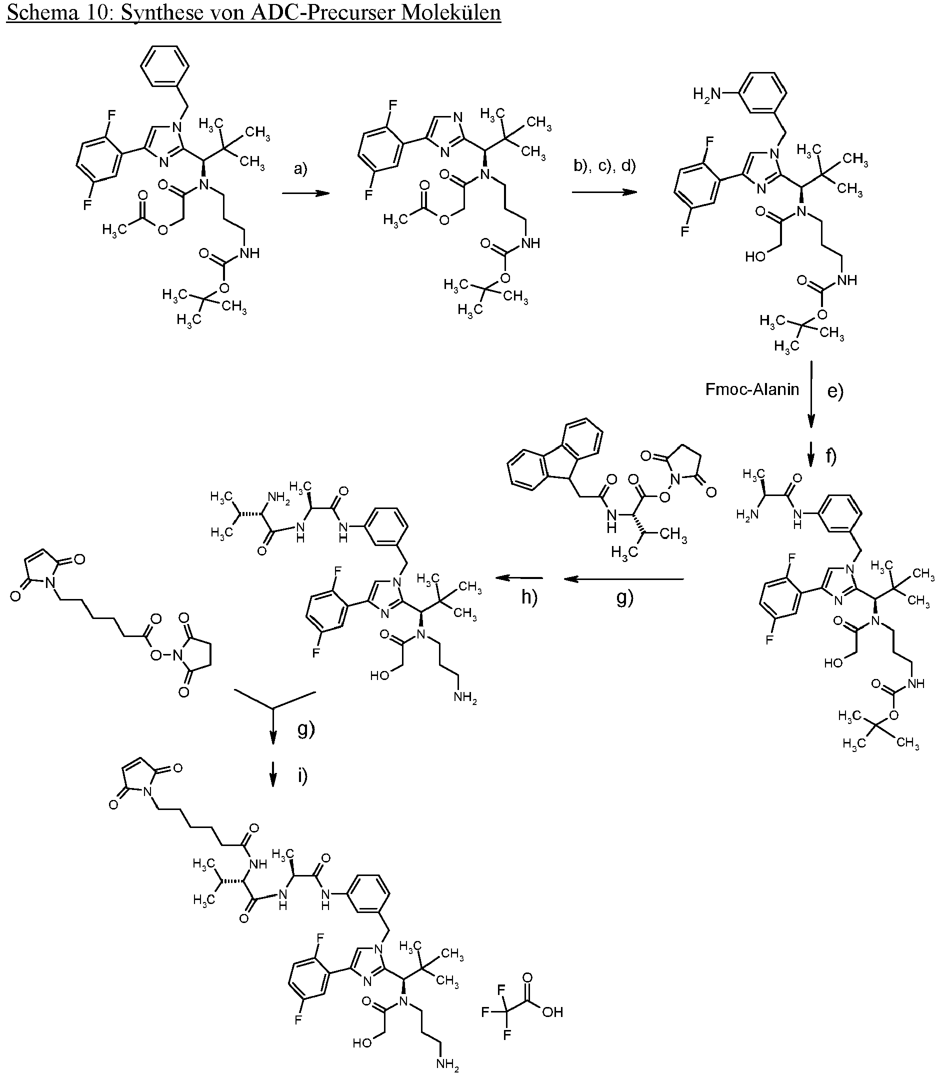 [a): beispielsweise H₂, Pd-C, EtOH, RT; b) beispielsweise p-Nitrobenzylbromid, K₂CO₃, DMF; c) beispielsweise Ethanol, 40%-ige Methylaminlösung in Wasser, 50 °C; d) beispielsweise Dinatriumdithionit, THF, Wasser, 50 °C; e) beispielsweise HATU, Diisopropylethylamin, DMF, RT; f) beispielsweise Piperidin, 40%-ige Methylaminlösung in Wasser, Ethanol, 50 °C; g) beispielsweise Diisopropylethylamin, DMF, RT; h) beispielsweise Piperidin, DMF, RT; i) TFA, DCM, RT] 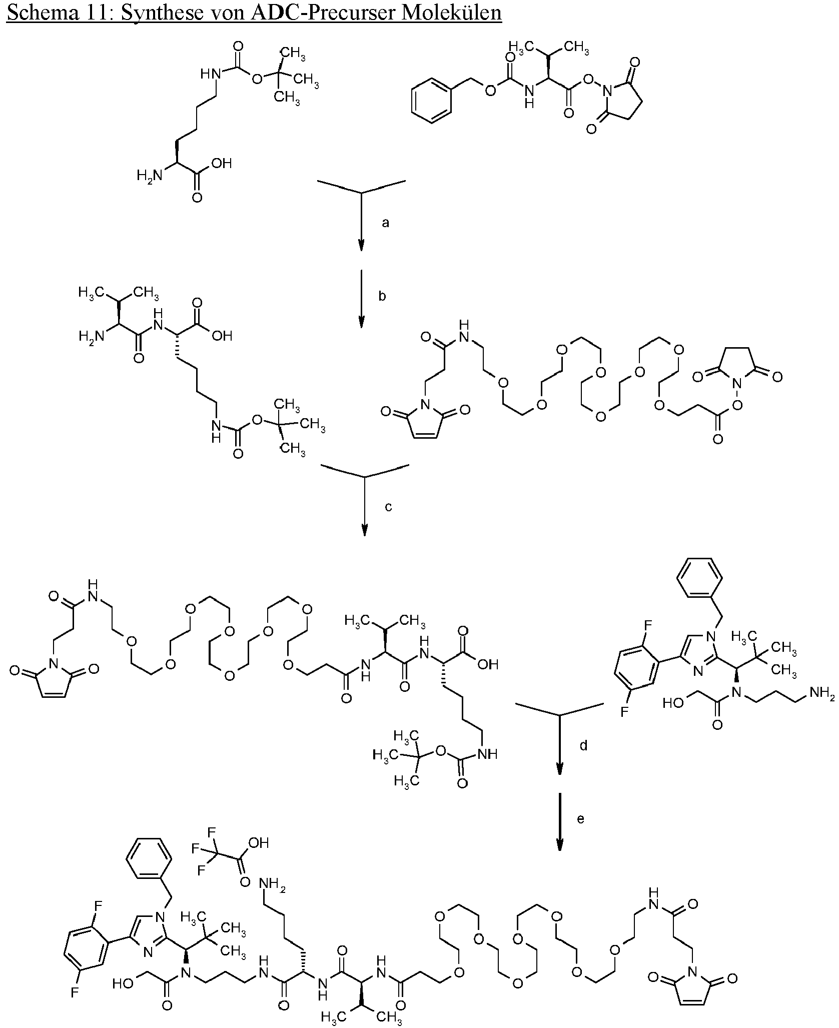 [a): beispielsweise Et₃N, DMF, RT; b) beispielsweise H₂, Pd-C, EtOH/Ethylacetat/THF (1:1:1), RT; c) beispielsweise 4-Methylmorpholin, DMF, RT; d) beispielsweise HATU, HOAt, Diisopropylethylamin, DMF, RT; e) beispielsweise TFA, RT] 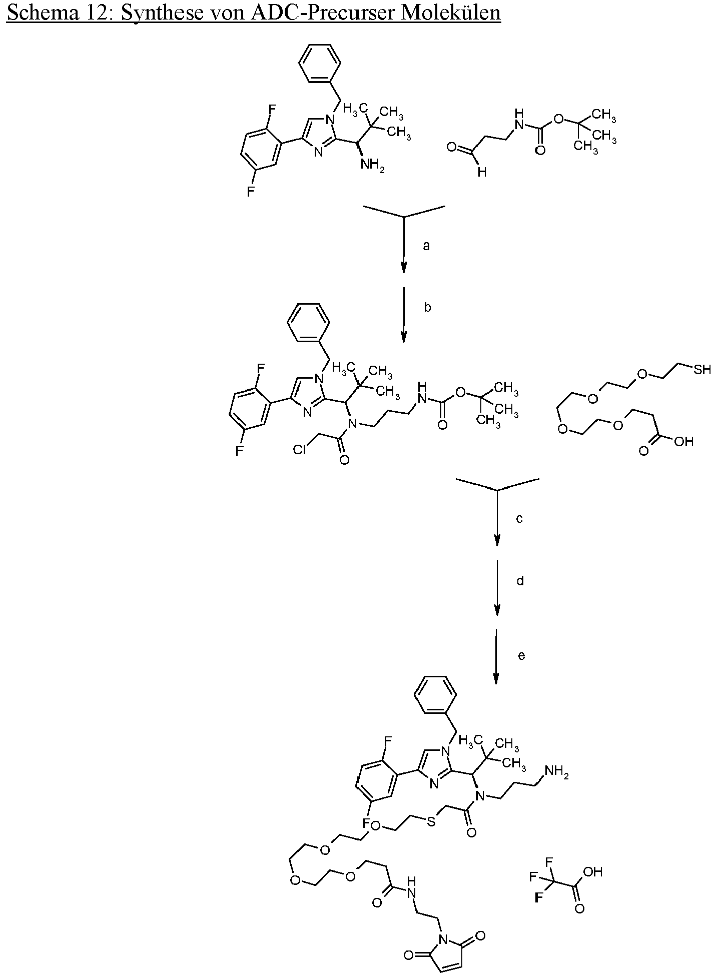 [a): beispielsweise NaBH(OAc)₃, HOAc, Dichlormethan, RT; b) beispielsweise Chloracetylchlorid, NEt₃, DCM, RT; c) beispielsweise Cs₂CO₃, DMF, 50 °C; d) beispielsweise 1-(2-Aminoethyl)-1H-pyrrol-2,5-dionhydrochlorid(1:1), T3P^{(R)}, Diisopropylethylamin, MeCN, RT; e) beispielsweise TFA, RT] 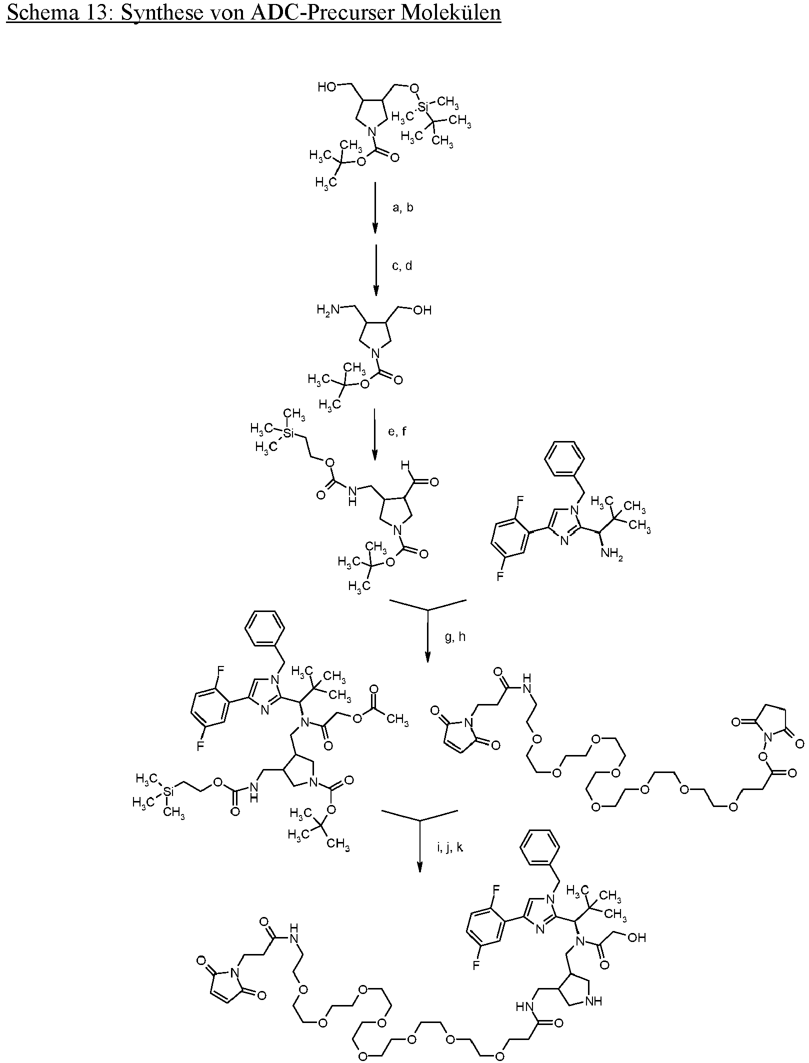 [a): beispielsweise Methansulfonsäurechlorid, NEt₃, Dichlormethan, 0 °C; b) beispielsweise NaN₃, DMF, 40 °C; c) beispielsweise H₂, Pd-C, EtOH/Ethylacetat (1:1), RT; d) beispielsweise TBAF, THF, RT; e) beispielsweise 1-({[2-(Trimethylsilyl)ethoxy]carbonyl}oxy)pyrrolidin-2,5-dion, NEts, CaCOs, 1,4-Dioxan, RT; f) beispielsweise N-Chlorsuccinimid, TEMPO, Tetra-n-butylammoniumchlorid, Chloroform, 0.05 N Kaliumcarbonat/0.05 N Natriumhydrogencarbonat-Lösung (1:1); g) beispielsweise NaBH(OAc)₃, HOAc, Dichlormethan, RT; h) beispielsweise Chloracetylchlorid, NEt₃, DCM, RT; i) beispielsweise TBAF, THF, Wasser, RT; j) beispielsweise 4-Methylmorpholin, DMF, RT; k) beispielsweise TFA, RT] 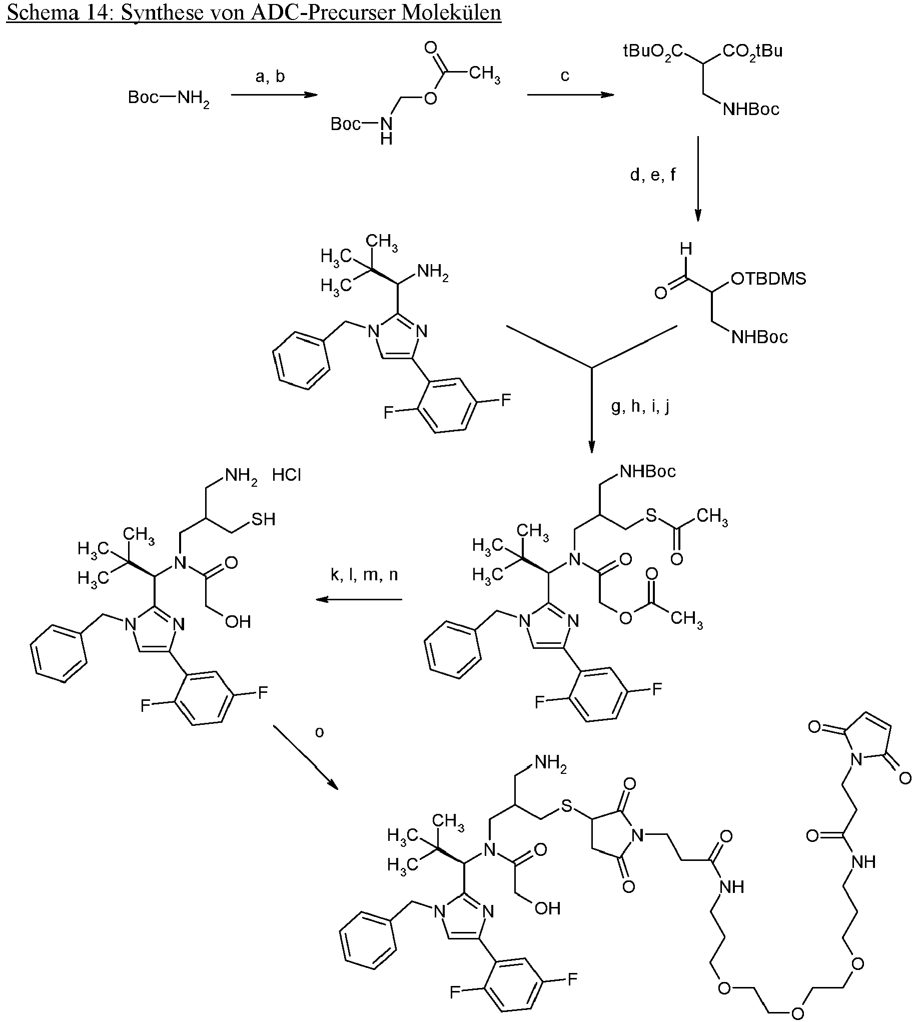 [a): beispielsweise Formaldehyd, Na₂CO₃, Wasser, RT; b) beispielsweise AczO, Pyridin, THF, RT; c) beispielsweise Di-tert-butylmalonat, KOtBu, THF, RT; d) beispielsweise LiBH₄, THF, RT; e) beispielsweise TBDMSCl, Imidazol, DCM, RT; f) beispielsweise Dess-Martin-Periodinan, DCM; g) beispielsweise Natriumtriacetoxyborhydrid, AcOH, DCM, RT; h) beispielsweise nBu₄NF, THF, RT; i) beispielsweise SOClz, THF, RT; j) beispielsweise AcSK, nBu₄NI, DMF, 90°C; k) beispielsweise NaOH, MeOH, THF, RT; l) beispielsweise TCEP, Dioxan, RT; m) beispielsweise Trennung der Epimere; n) beispielsweise 6N Salzsäure, THF, RT o) beispielsweise Mal-dPEG(3)-Mal, PBS-Puffer, ACN, RT] 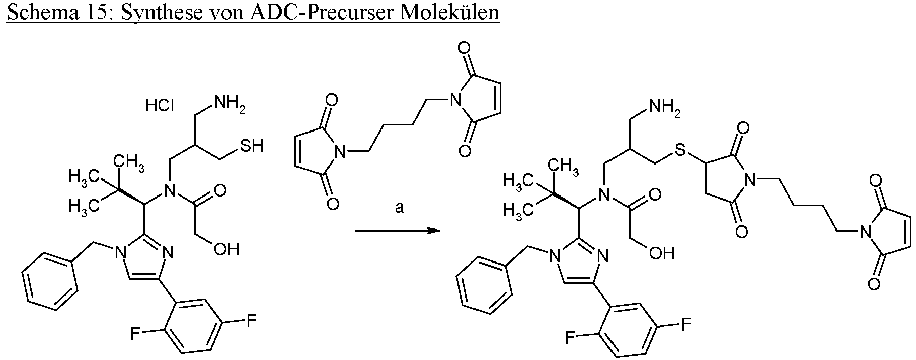 [a): beispielsweise Mal-dPEG(3)-Mal, PBS-Puffer, ACN, RT] 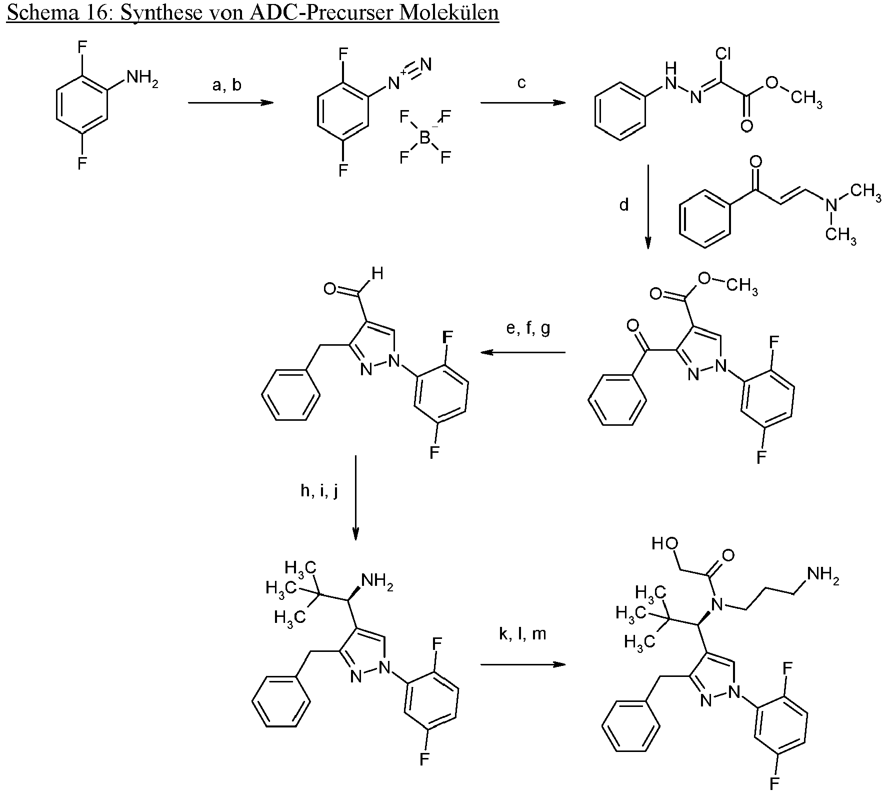 [a): beispielsweise BF₃OEt₂, THF, 0°C; b): beispielsweise Isoamylnitrit, -10°C, 0.5 h; c): beispielsweise Methyl-2-chlor-3-oxobutanoat, Pyridin, Wasser, -5°C; d): beispielsweise NEt₃, Toluol, RT; e): beispielsweise Et₃SiH, TFA, RT; f): beispielsweise LiBH₄, THF, 60°C; g): beispielsweise Dess-Martin-Periodinan, DCM, RT; h): beispielsweise (R)-(+)-Methyl-2-propansulfinsäureamid, Titan(IV)isopropylat, THF, RT; i): beispielsweise tert-BuLi, Pentan, THF, -78°C; j): beispielsweise HCl in Dioxan, THF, MeOH, RT; k): beispielsweise 3-(1,3-Dioxo-1,3-dihydro-2H-isoindol-2-yl)propanal, NaB(OAc)₃H, AcOH, DCM, RT; l): beispielsweise 2-Chlor-2-oxoethylacetat, NEt₃, DCM, RT; m): beispielsweise Methylamin, Wasser, EtOH, 50°C] 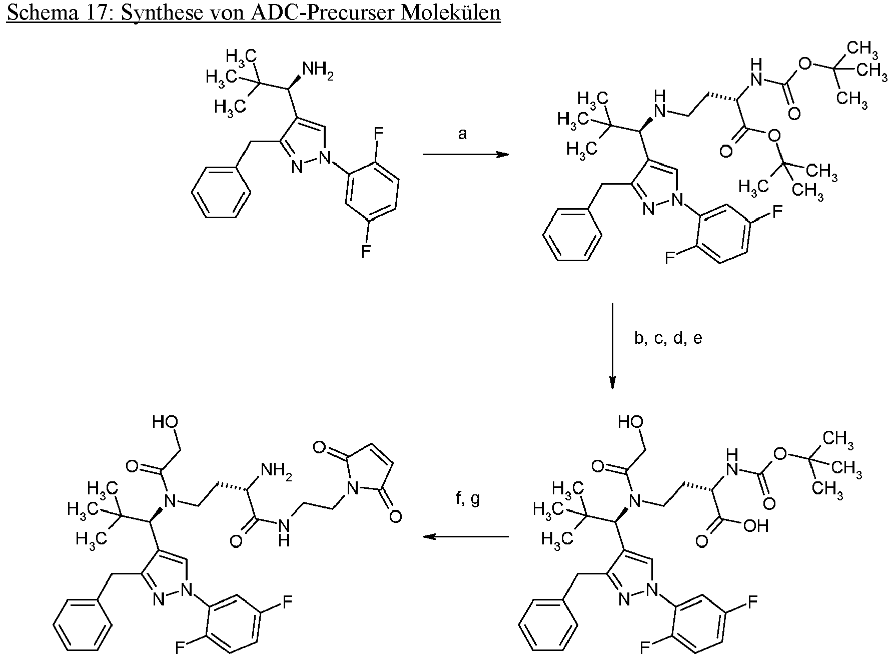 [a): beispielsweise tert-Butyl-N-(tert-butoxycarbonyl)-5-oxo-L-norvalinat, NaB(OAc)₃H, AcOH, DCM, RT; b): beispielsweise 2-Chlor-2-oxoethylacetat, NEt₃, DCM, RT; c): beispielsweise Methylamin, Wasser, EtOH, 60°C; d): beispielsweise THF, DCM, 50°C; e): beispielsweise BoczO, NEt₃, DCM, RT; f): beispielsweise Trifluoressigsäure-1-(2-aminoethyl)-1H-pyrrol-2,5-dion(1:1), HATU, Diisopropylethylamin, DMF, RT; f): beispielsweise TFA, DCM, RT] 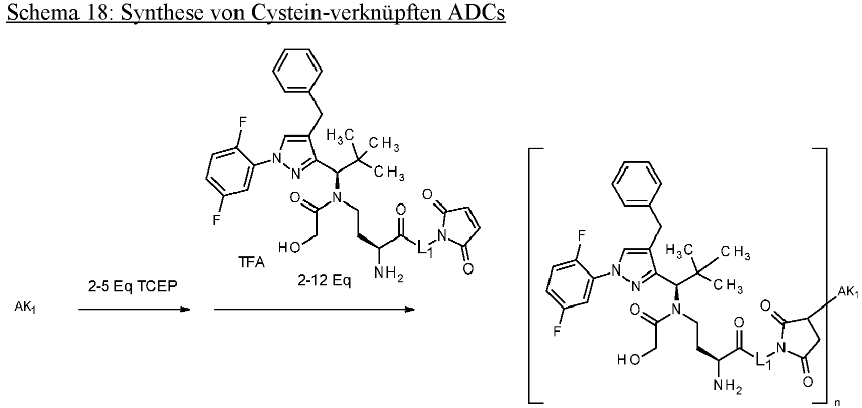 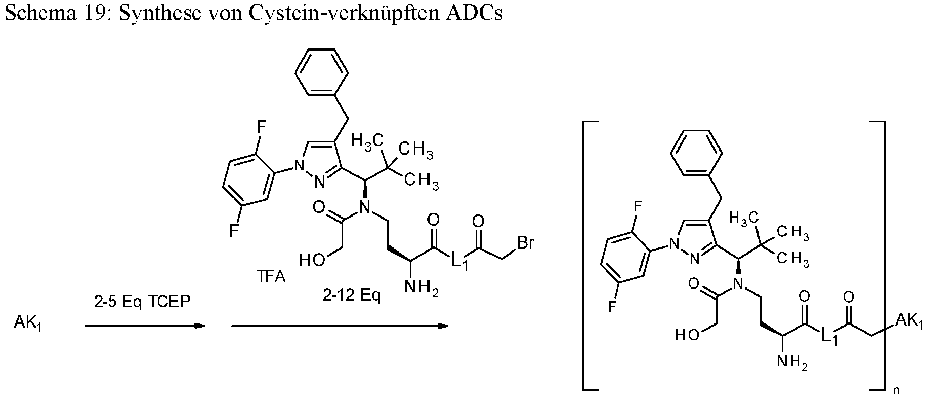 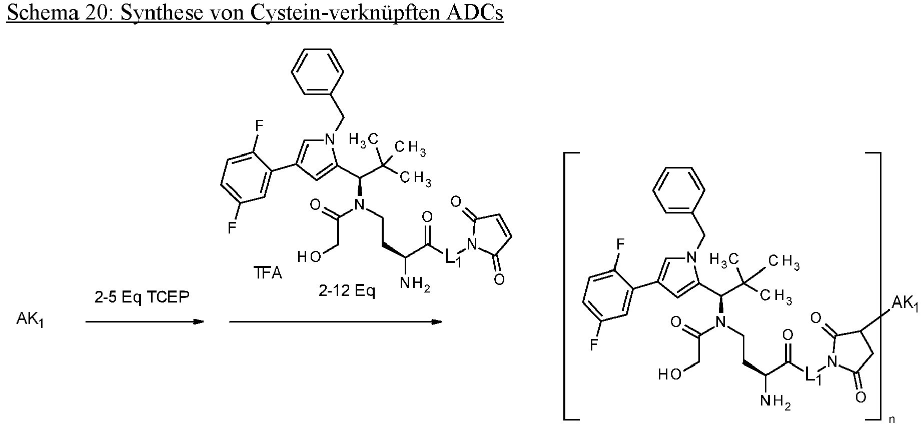 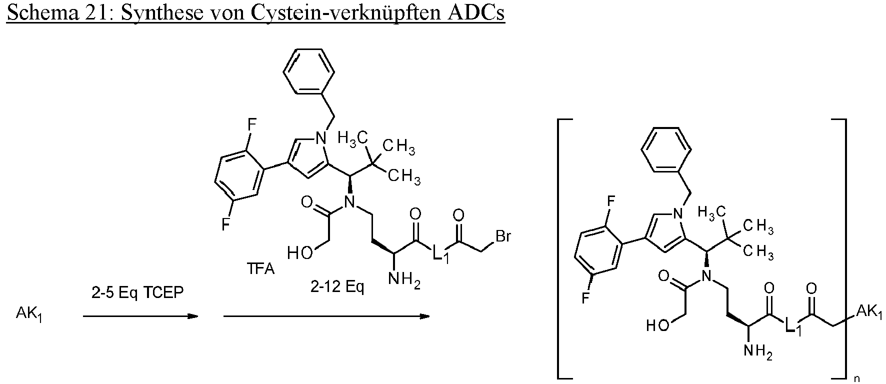 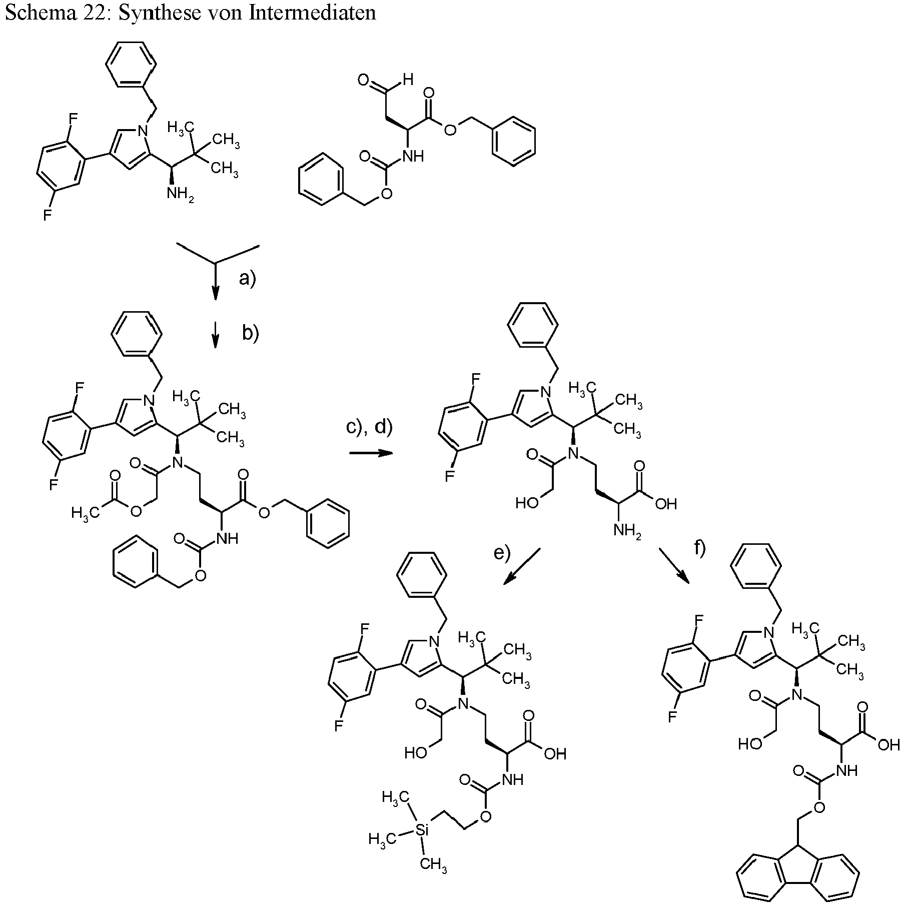 [a): beispielsweise Natriumtriacetoxyborhydrid, Essigsäure, DCM, RT; b) beispielsweise Acetoxyacetylchlorid, NEt3, DCM, RT; c) beispielsweise LiOH, THF/Wasser, RT; d) beispielsweise H₂, Pd-C, EtOH, RT; e) beispielsweise Teoc-OSu, NEt3, Dioxan, RT; f) beispielsweise Fmoc-Cl, Diisopropylethylamin, Dioxan/Wasser 2:1, RT] 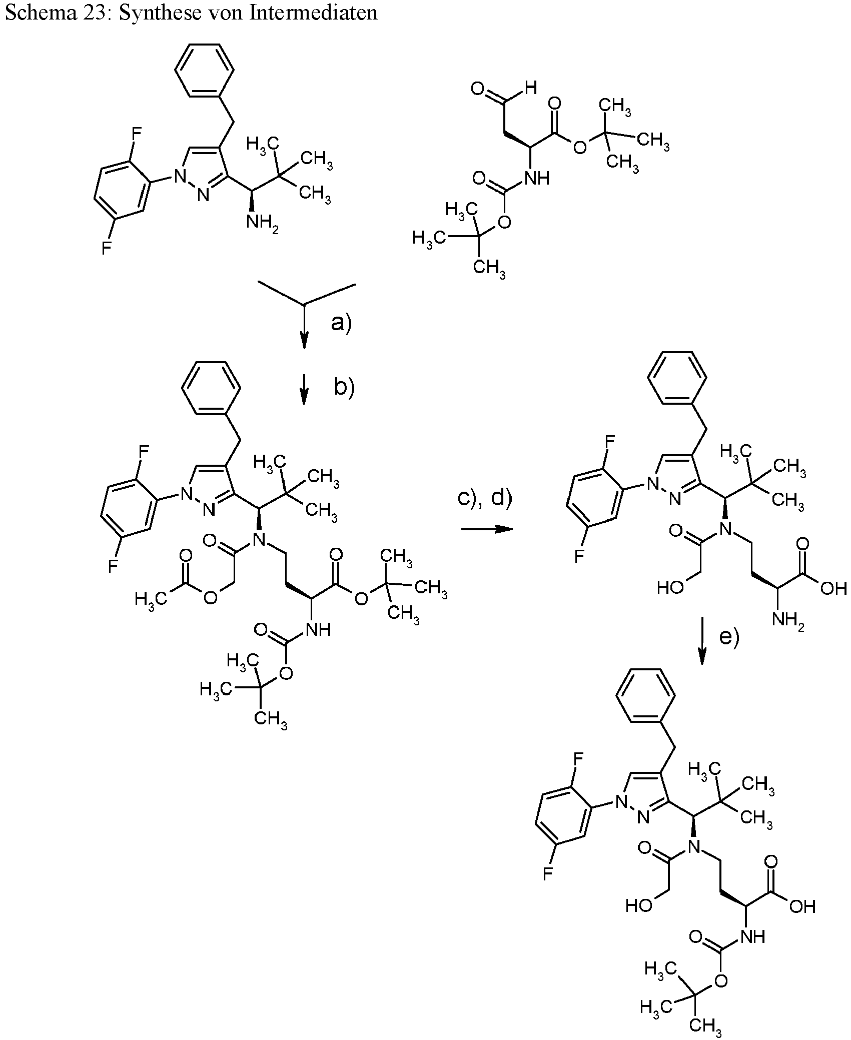 [a): beispielsweise Natriumtriacetoxyborhydrid, Essigsäure, DCM, RT; b) beispielsweise Acetoxyacetylchlorid, NEt3, DCM, RT;c) beispielsweise LiOH, Methanol, RT; d) beispielsweise TFA, DCM, RT; e) beispielsweise BoczO, Diisopropylethylamin, DCM, RT] 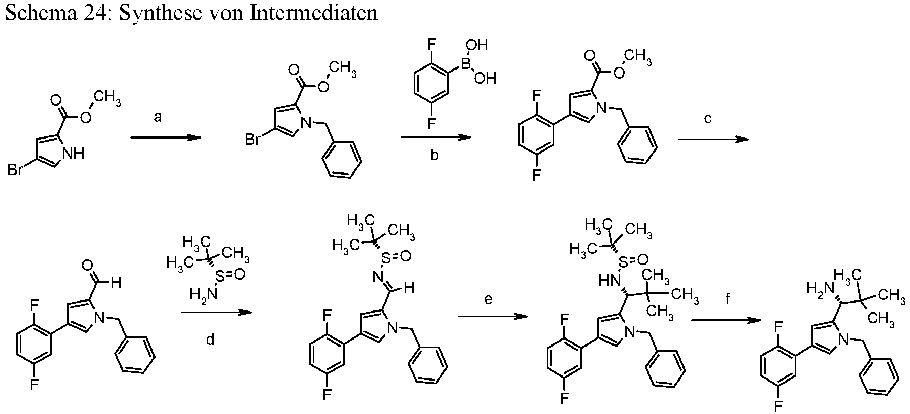 [a): beispielsweise Benzylbromid, Cs₂CO₃, DMF, RT; b) beispielsweise Pd(dppf)₂Cl₂, DMF, Na₂CO₃, 85 °C; c) beispielsweise LiAlH₄, THF, 0 °C; MnO₂, DCM, RT; d) beispielsweise Ti(iOPr)₄, THF, RT; e) beispielsweise tBuLi, THF, -78 °C; MeOH, NH₄Cl; f) beispielsweise HCl/1,4-Dioxan] 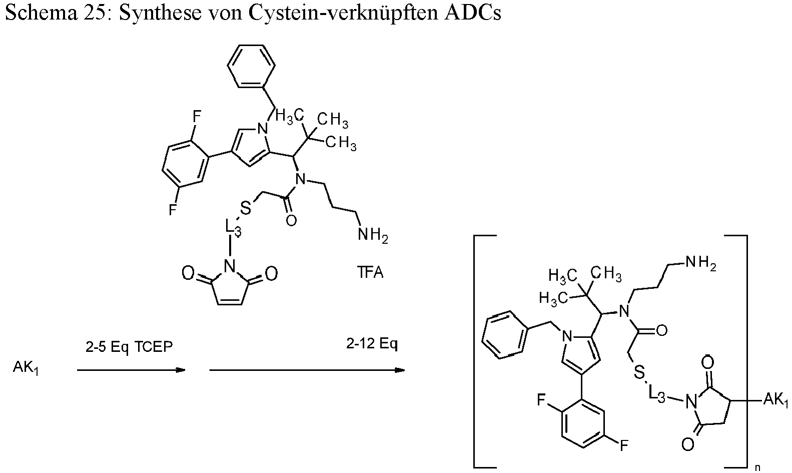 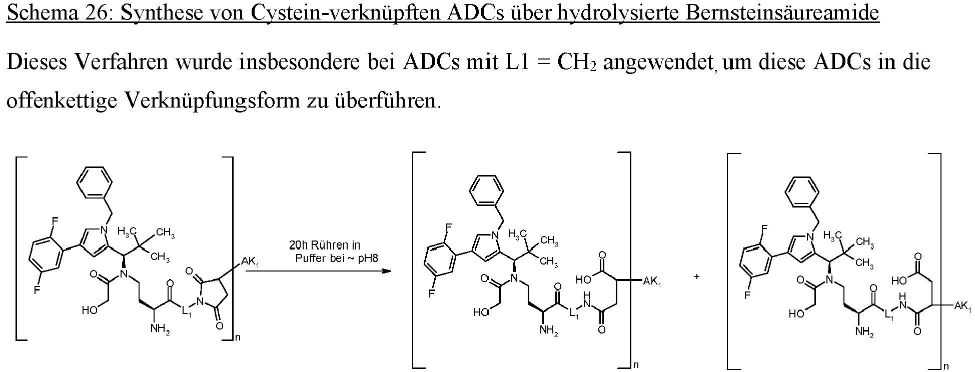 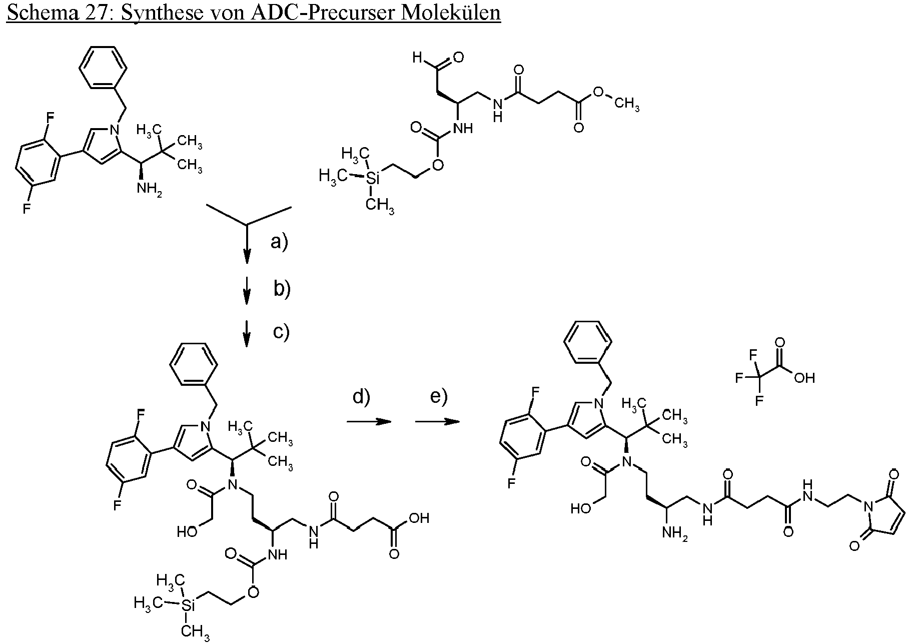 [a): Natriumtriacetoxyborhydrid, Essigsäure, DCM, RT; b) Acetoxyacetylchlorid, Diisopropylethylamin, DCM, RT; c) LiOH, MeOH, RT; d) Trifluoressigsäure--1-(2-aminoethyl)-1H-pyrrol-2,5-dion (1:1) HATU, DMF, Diisopropylethylamin, RT; e) Zinkchlorid, Trifluorethanol, 50°C, EDTA.] 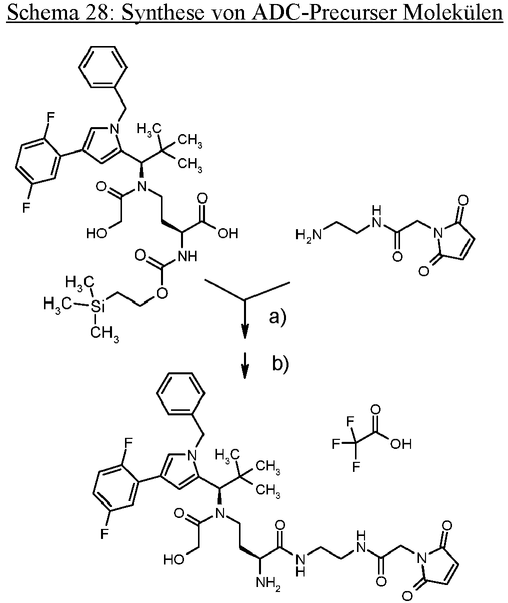 [a): HATU, DMF, Diisopropylethylamin, RT; b) Zinkchlorid, Trifluorethanol, 50°C, EDTA.] 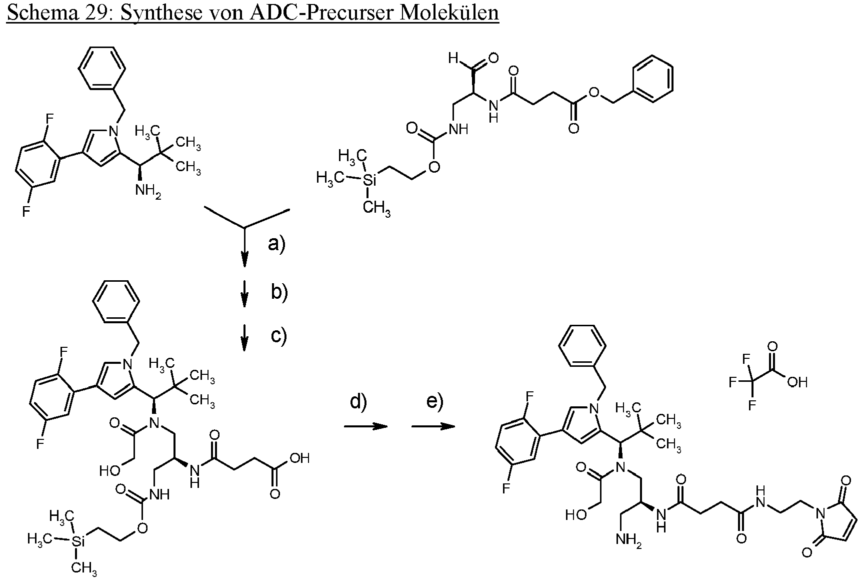 [a): Natriumtriacetoxyborhydrid, Essigsäure, DCM, RT; b) Acetoxyacetylchlorid, Triethylamin, DCM, RT; c) LiOH, MeOH, RT; d) Trifluoressigsäure--1-(2-aminoethyl)-1H-pyrrol-2,5-dion (1:1) HATU, DMF, Diisopropylethylamin, RT; e) Zinkchlorid, Trifluorethanol, 50°C, EDTA.] 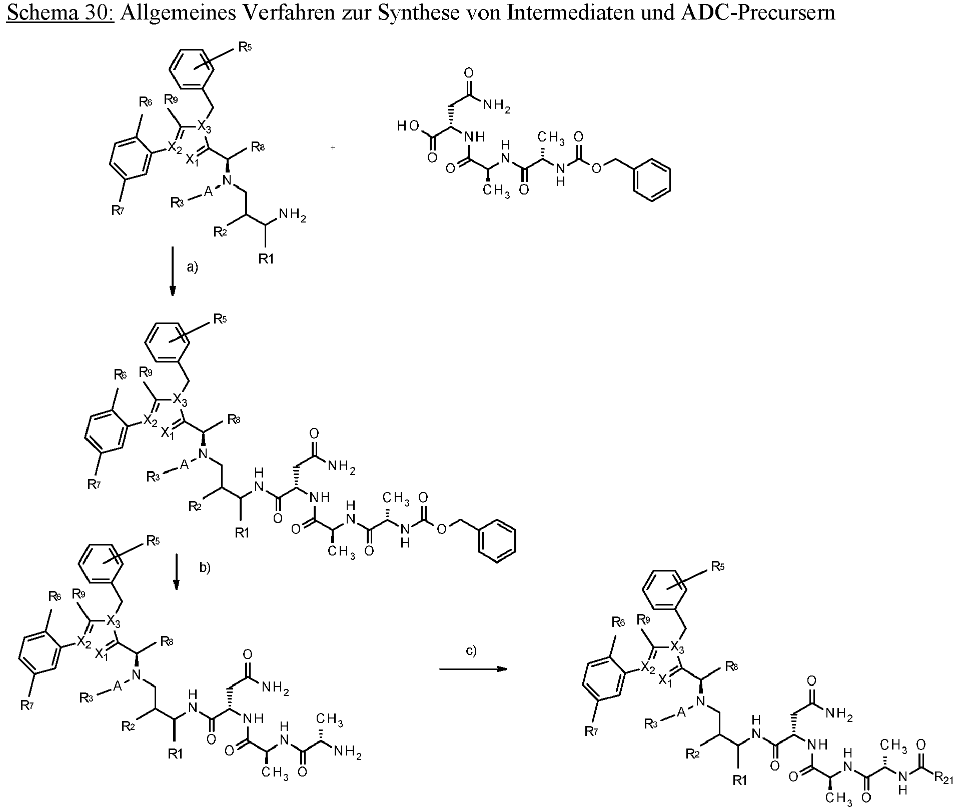 [a): HATU, DMF, N,N-Diisopropylethylamin, RT oder EDCI, HOBT, N,N-Diisopropylethylamin, DMF, RT b) H₂, 10% Pd-C, MeOH, RT; c) R²¹-COOH, EDCI, HOBT, N,N-Diisopropylethylamin, DMF, RT oder R²¹-COOH, HATU, N,N-Diisopropylethylamin, DMF, RT oder R²¹-COOSu, N,N-Diisopropylethylamin, DMF, RT] 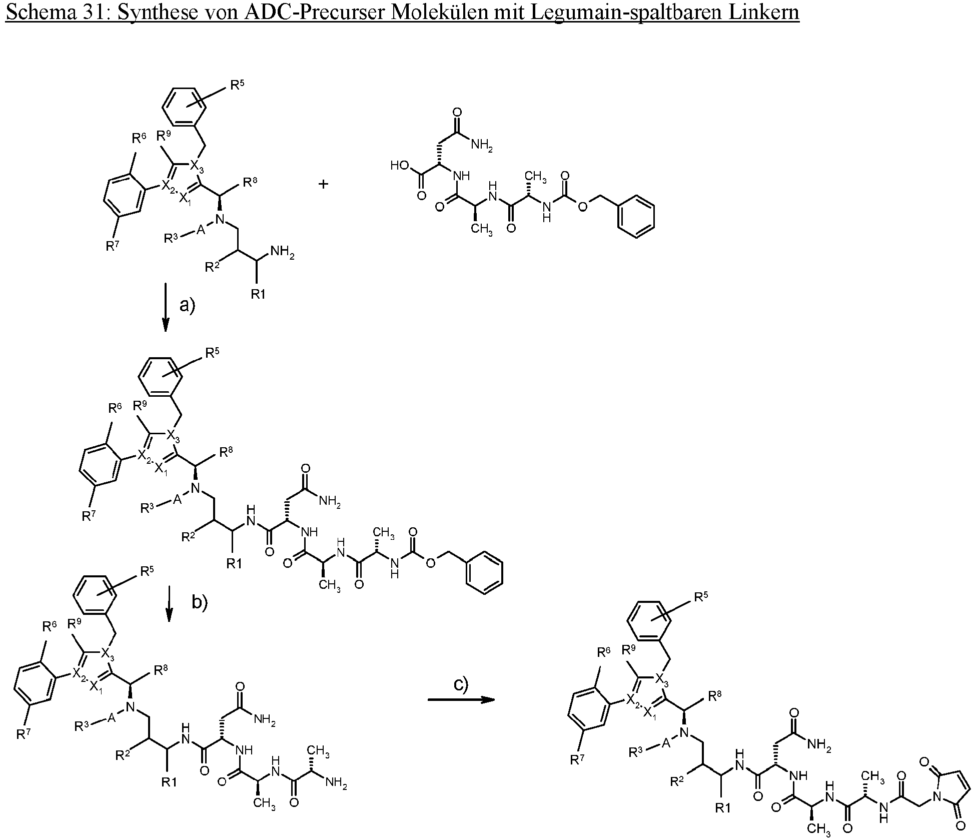 [a): HATU, DMF, N,N-Diisopropylethylamin, RT oder EDCI, HOBT, N,N-Diisopropylethylamin, DMF, RT b) H₂, 10% Pd-C, MeOH, RT; c) 1,1'-[(1,5-Dioxopentan-1,5-diyl)bis(oxy)]dipyrrolidin-2,5-dion, N,N-Diisopropylethylamin, DMF, RT] 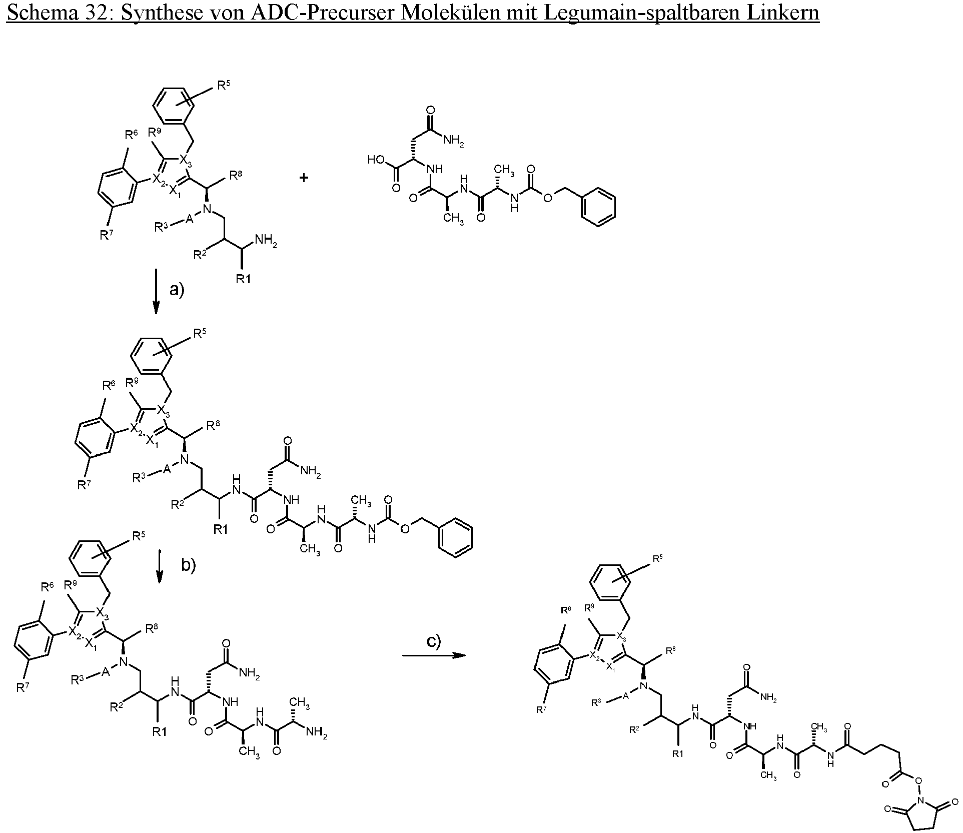 [a): HATU, DMF, N,N-Diisopropylethylamin, RT oder EDCI, HOBT, N,N-Diisopropylethylamin, DMF, RT b) H₂, 10% Pd-C, MeOH, RT; c) ) 1,1'-[(1,5-Dioxopentan-1,5-diyl)bis(oxy)]dipyrrolidin-2,5-dion, N,N-Diisopropylethylamin, DMF, RT]

Weiterhin können andere Intermediate gemäß Schema 32, 33 und 34 in Legumain-spaltbare ADC- und APDC Precursor überführt werden.

Alternativ zu der in Schemata 32-34 dargestellten Benzyloxycarbonyl-Gruppe können andere in der Peptidchemie etablierte Schutzgruppen eingesetzt werden und nach entsprechenden ebenso bekannten Methoden abgespalten werden. Die Auswahl der Schutzgruppenstrategie erfolgt nach entsprechenden dem Fachmann bekannten Anforderungen zur Kompatibilität mit anderen im Molekül vorkommenden Strukturelementen. Falls noch vorhanden können weitere Schutzgruppen im Molekül in einem letzten Schritt entfernt werden.

Die Synthesen können auch ggf. in ihrer Sequenz umgestellt werden. 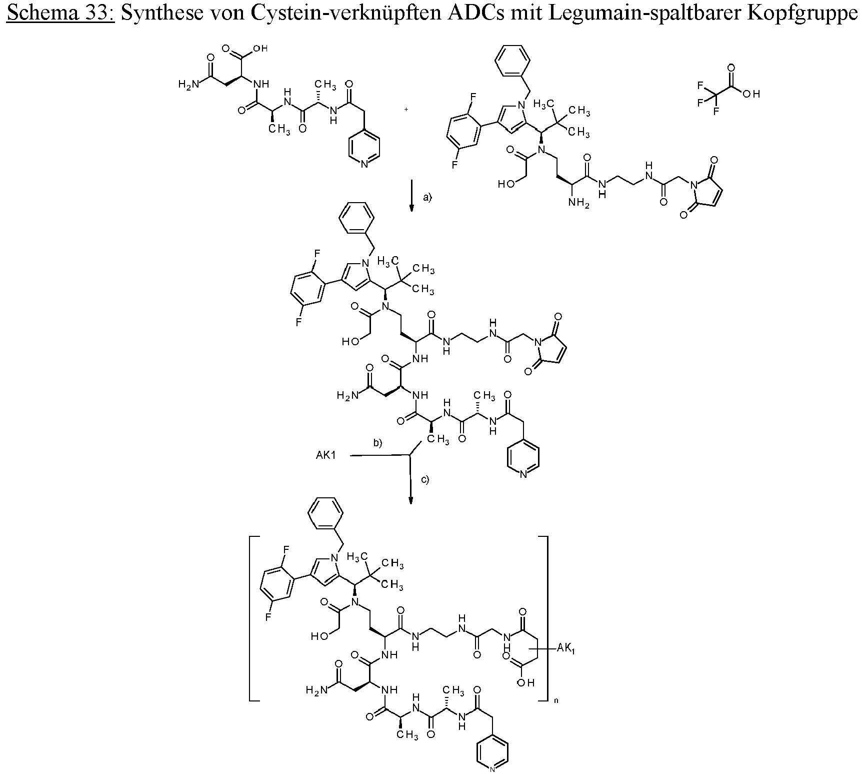 [a): HATU, DMF, N,N-Diisopropylethylamin, RT; b) 2-5 Eq TCEP, PBS pH7.2, 30 min Rühren bei RT; c) 90 min Rühren bei RT unter Argon, dann Umpuffern auf pH 8 mittels PD 10-Säulen (Sephadex^{®} G-25, GE Healthcare) und Rühren über Nacht unter Argon bei RT und anschließendes Aufkonzentrieren mittels Ultrazentrifugation und Einstellen der angestrebten Konzentration mit PBS bei pH 7.2)] 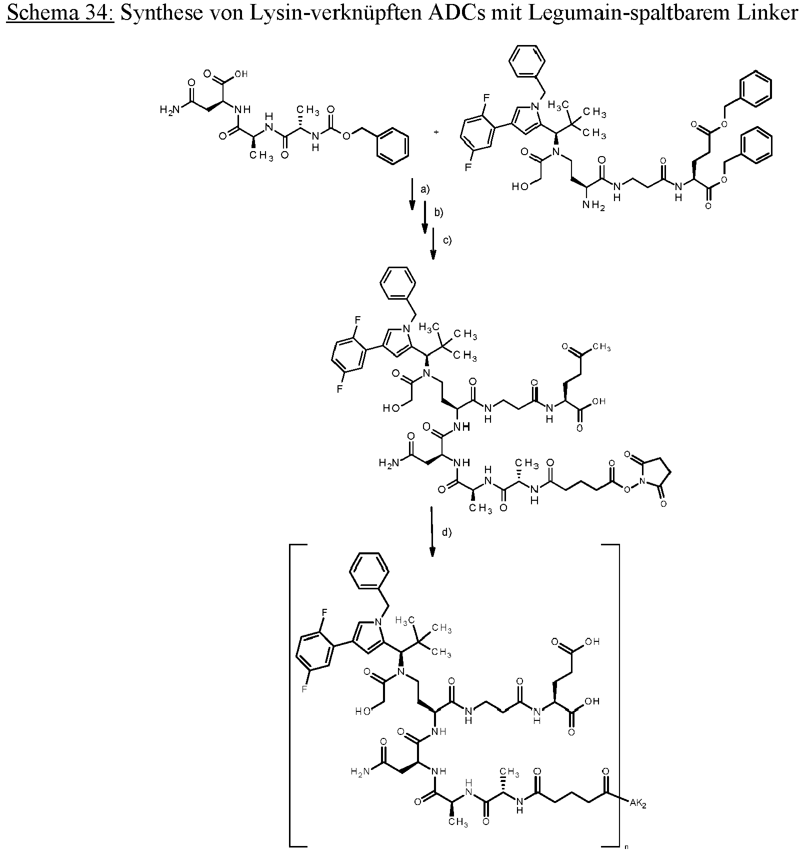 [a): HATU, DMF, N,N-Diisopropylethylamin, RT; b) H₂, 10% Pd-C, Methanol 1.5 h, RT; c) 1,1'-[(1,5-Dioxopentan-1,5-diyl)bis(oxy)]dipyrrolidin-2,5-dion, N,N-Diisopropylethylamin, DMF, Rühren über Nacht bei RT; d) AK2 in PBS, 5 Equiv. Aktivester gelöst in DMSO zugeben, 60 min Rühren bei RT unter Argon, erneut 5 Equiv. Aktivester gelöst in DMSO nachsetzen, 60 min Rühren bei RT unter Argon, dann Reinigung über mit PBS Puffer (pH 7.2) equillibrierte PD 10-Säulen (Sephadex^{®} G-25, GE Healthcare) und anschließendes Aufkonzentrieren mittels Ultrazentrifugation und Einstellen der angestrebten Konzentration mit PBS Puffer (pH 7.2)]. 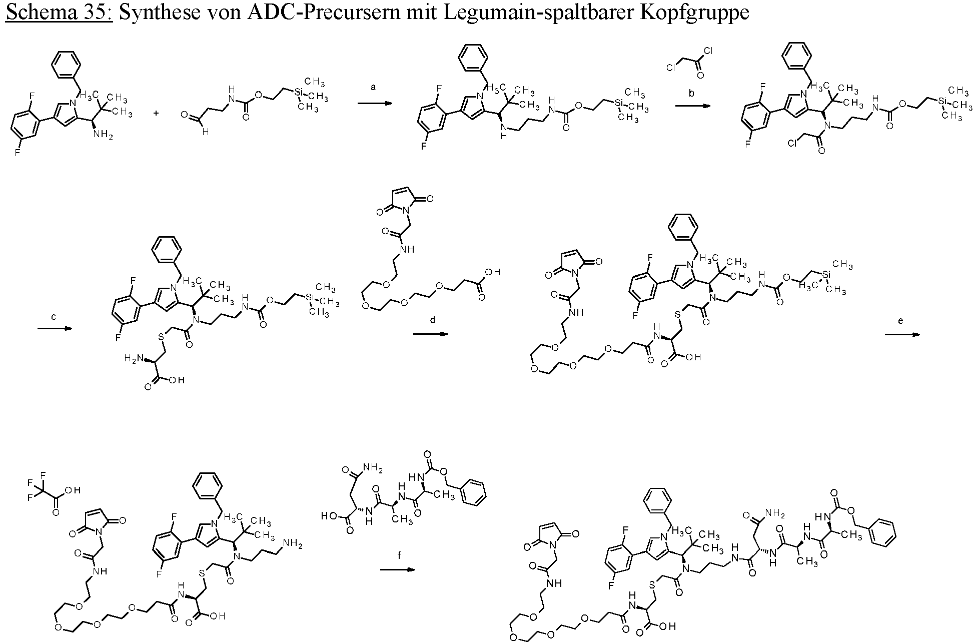 [a): NaBH(OAc)₃, HOAc, Dichlormethan, RT; b) Chloracetylchlorid, NEt₃, DCM, RT; c) L-Cystein, NaHCO₃, DBU, Isopropanol/Wasser, 50 °C; d) HATU, DMF, Diisopropylethylamin, RT; e) Zinkchlorid, Trifluorethanol, 50°C; f) d) HATU, DMF, Diisopropylethylamin, RT] 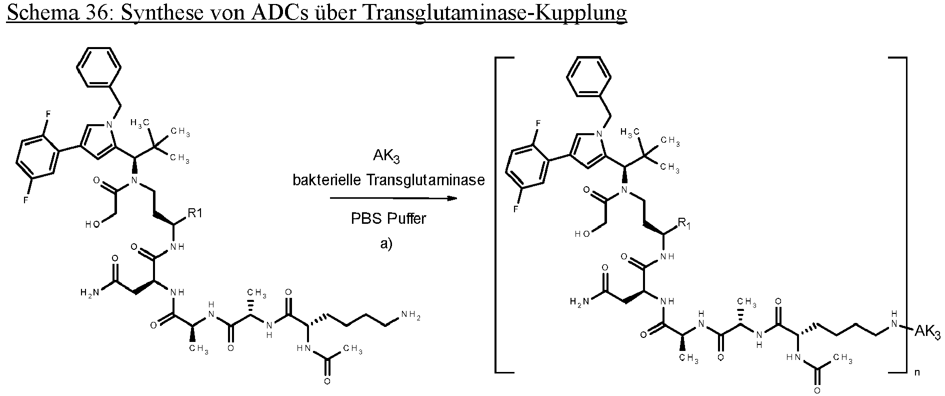 [a: 5 mg AK3 in DPBS pH 7.4 (c~10mg/mL), 6 Equivalente eines Toxophor-Linker-Precursors (e.g. Intermediat Q31-Q34), 50 µL einer Lösung aus 12.5 µL (1.25 U) rekombinanter bakterieller Transglutaminase Lösung in Wasser (100 U/mL) und 37.5 µL DPBS pH 7.4 hinzugegeben, 24h bei 37°C inkubieren]

### A. Beispiele

### Abkürzungen und Akronyme:

- A431NS: humane Tumorzelllinie
- A549: humane Tumorzelllinie
- ABCB1: ATP-binding cassette sub-family B member 1 (Synonym für P-gp und MDR1)
- abs.: Absolut
- Ac: Acetyl
- ACN: Acetonitril
- aq.: wässrig, wässrige Lösung
- ATP: Adenosintriphosphat
- BCRP: Brustkrebs-Resistenz-Protein, ein Efflux-Transporter
- BEP: 2-Brom-1-ethylpyridinium-Tetrafluoroborat
- Boc: *tert*.-Butoxycarbonyl
- br.: breit (bei NMR)
- Bsp.: Beispiel
- BxPC3: humane Tumorzelllinie
- *ca.*: *circa,* ungefähr
- CI: chemische Ionisation (bei MS)
- D: Dublett (bei NMR)
- D: Tag(e)
- DC: Dünnschichtchromatographie
- DCI: direkte chemische Ionisation (bei MS)
- DCM: Dichlormethan
- Dd: Dublett von Dublett (bei NMR)
- DMAP: 4-*N,N*-Dimethylaminopyridin
- DME: 1,2-Dimethoxyethan
- DMEM: Dulbecco's Modified Eagle Medium (standardisiertes Nährmedium für die Zellkultur)
- DMF: *N,N*-Dimethylformamid
- DMSO: Dimethylsulfoxid
- DPBS,: D-PBS, PBS Dulbecco's Phosphat-gepufferte Salz-Lösung
PBS = DPBS = D-PBS, pH7,4, Fa. Sigma, No D8537
Zusammensetzung:
0,2 g KCl
0,2 g KH₂PO₄ (anhyd)
8,0 g NaCl
1,15 g Na₂HPO₄ (anhyd)
ad 1 l mit H₂O auffüllen
- Dt: Dublett von Triplett (bei NMR)
- DTT: DL-Dithiothreitol
- d.: Th. der Theorie (bei chemischer Ausbeute)
- EDC: *N*'-(3-Dimethylaminopropyl)-*N*-ethylcarbodiimid-Hydrochlorid
- EGFR: Epidermal growth factor receptor = Epidermaler Wachstumsfaktor Rezeptor
- EI: Elektronenstoß-Ionisation (bei MS)
- ELISA: Enzyme-linked Immunosorbent Assay
- eq.: Äquivalent(e)
- ESI: Elektrospray-Ionisation (bei MS)
- ESI-MicroTofq: ESI- MicroTofq (Name des Massenspektrometer mit Tof = Time Of Flight und q = Quadrupol)
- FCS: fötales Kälberserum
- Fmoc: (9*H*-Fluoren-9-ylmethoxy)carbonyl
- ges.: gesättigt
- GTP: Guanosin-5'-triphosphat
- H: Stunde(n)
- HATU: *O*-(7-Azabenzotriazol-1-yl)-*N*,*N*,*N*',*N*'-tetramethyluronium-hexafluorophosphat
- HCT-116: humaneTumorzelllinie
- HEPES: 4-(2-Hydroxyethyl)piperazin-1-ethansulfonsäure
- HOAc: Essigsäure
- HOAt: 1-Hydroxy-7-azabenzotriazol
- HOBt: 1-Hydroxy-1*H*-benzotriazol-Hydrat
- HOSu: *N*-Hydroxysuccinimid
- HPLC: Hochdruck-, Hochleistungsflüssigchromatographie
- HT29: humane Tumorzelllinie
- IC₅₀: halbmaximale Inhibitionskonzentration
- i.m.: intramuskulär, Applikation in den Muskel
- i.v.: intravenös, Applikation in die Vene
- konz.: konzentriert
- KPL-4: humane Tumorzelllinien
- KU-19-19: humane Tumorzelllinie
- LC-MS: Flüssigchromatographie-gekoppelte Massenspektrometrie
- LLC-PK1-Zellen: Lewis lung carcinoma pork kidney cell line
- L-MDR: Human MDR1 transfizierte LLC-PK1 Zellen
- LoVo: humane Tumorzelllinie
- m: Multiplett (bei NMR)
- Me: Methyl
- MDR1: Multidrug resistence protein 1
- MeCN: Acetonitril
- min: Minute(n)
- MS: Massenspektrometrie
- MTT: 3-(4,5-Dimethylthiazol-2-yl)-2,5-diphenyl-2*H*-tetrazoliumbromid
- NCI-H292: humane Tumorzelllinie
- NCI-H520: humane Tumorzelllinie
- NMM: *N*-Methylmorpholin
- NMP: *N*-Methyl-2-pyrrolidinon
- NMR: Kernresonanzspektrometrie
- NMRI: Mausstamm, Herkunft aus dem Naval Medical Research Institute (NMRI)
- Nude: Mäuse Nacktmäuse(Versuchstiere)
- NSCLC: Non small cell lung cancer (Nicht-kleinzelliges Bronchialkarzinom)
- PBS: Phosphat-gepufferte Salz-Lösung
- Pd/C: Palladium auf Aktivkohle
- P-gp: P-Glycoprotein, ein Transporterprotein
- PNGaseF: Enzym zur Zuckerabspaltung
- quant.: quantitativ (bei Ausbeute)
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- s.c.: subcutan, Applikation unter die Haut
- SCC-4: humane Tumorzelllinie
- SCC-9: humane Tumorzelllinie
- SCID: Mäuse Versuchsmäuse mit einem schweren kombinierten Immundefekt (severe combined immunodeficiency)
- SK-HEP-1: humane Tumorzelllinie
- t: Triplett (bei NMR)
- TBAF: Tetra-n-butylammoniumfluorid
- TCEP: Tris(2-carboxyethyl)phosphin
- TEMPO: (2,2,6,6-Tetramethyl-piperidin-1-yl)oxyl
- *tert.*: tertiär
- TFA: Trifluoressigsäure
- THF: Tetrahydrofuran
- T3P^{®}: 2,4,6-Tripropyl-1,3,5,2,4,6-trioxatriphosphinan-2,4,6-trioxid
- UV: Ultraviolett-Spektrometrie
- v/v: Volumen zu Volumen-Verhältnis (einer Lösung)
- Z: Benzyloxycarbonyl
- 786-O: humane Tumorzelllinie

### Aminosäuren Abkürzungen

Ala = Alanin
Arg = Arginin
Asn = Asparagin
Asp = Asparaginsäure
Cys = Cystein
Glu = Glutaminsäure
Gln = Glutamin
Gly = Glycin
His = Histidin
Ile = Isoleucin
Leu = Leucin
Lys = Lysin
Met = Methionin
Nva = Norvalin
Phe = Phenylalanin
Pro = Prolin
Ser = Serin
Thr = Threonin
Trp = Tryptophan
Tyr = Tyrosin
Val = Valin

### HPLC- und LC-MS-Methoden:

### Methode 1 (LC-MS):

Instrument: Waters ACQUITY SQD UPLC System; Säule: Waters Acquity UPLC HSS T3 1.8 µ 50 x 1 mm; Eluent A: 1 l Wasser + 0.25 mL 99%ige Ameisensäure, Eluent B: 1 l Acetonitril + 0.25 mL 99%ige Ameisensäure; Gradient: 0.0 min 90% A → 1.2 min 5% A - 2.0 min 5% A Ofen: 50°C; Fluss: 0.40 mL/min; UV-Detektion: 208 - 400 nm.

### Methode 2 (LC-MS):

Gerätetyp MS: Waters Synapt G2S; Gerätetyp UPLC: Waters Acquity I-CLASS; Säule: Waters, BEH300, 2.1 × 150 mm, C18 1.7 µm; Eluent A: 1 l Wasser + 0.01% Ameisensäure; Eluent B: 1 l Acetonitril + 0.01% Ameisensäure; Gradient: 0.0 min 2% B → 1.5 min 2% B - 8.5 min 95% B - 10.0 min 95% B; Ofen: 50°C; Fluss: 0.50 mL/min; UV-Detektion: 220 nm

### Methode 3 (LC-MS):

Instrument MS: Waters (Micromass) QM; Instrument HPLC: Agilent 1100 Serie; Säule : Agient ZORBAX Extend-C18 3.0x50mm 3.5-Micron; Eluent A: 1 l Wasser + 0.01 mol Ammoniumcarbonat, Eluent B: 1 l Acetonitril; Gradient: 0.0 min 98% A - 0.2min 98% A - 3.0 min 5% A→ 4.5 min 5% A ; Ofen: 40°C; Fluss: 1.75 mL/min; UV-Detektion: 210 nm

### Methode 4 (LC-MS):

Gerätetyp MS: Waters Synapt G2S; Gerätetyp UPLC: Waters Acquity I-CLASS; Säule: Waters, HSST3, 2.1 × 50 mm, C18 1.8 µm; Eluent A: 1 l Wasser + 0.01% Ameisensäure; Eluent B: 1 l Acetonitril + 0.01% Ameisensäure; Gradient: 0.0 min 10% B - 0.3 min 10% B → 1.7 min 95% B → 2.5 min 95% B; Ofen: 50°C; Fluss: 1.20 mL/min; UV-Detektion: 210 nm

### Methode 5 (LC-MS):

Instrument: Waters ACQUITY SQD UPLC System; Säule: Waters Acquity UPLC HSS T3 1.8 µ 50 x 1 mm; Eluent A: 1 l Wasser + 0.25 mL 99%ige Ameisensäure , Eluent B: 1 l Acetonitril + 0.25 mL 99%ige Ameisensäure; Gradient: 0.0 min 95% A - 6.0 min 5% A - 7.5 min 5% A Ofen: 50°C; Fluss: 0.35 mL/min; UV-Detektion: 210 - 400 nm.

### Methode 6 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 mL 50%ige Ameisensäure, Eluent B: 1 l Acetonitril + 0.5 mL 50%ige Ameisensäure; Gradient: 0.0 min 97% A - 0.5 min 97% A - 3.2 min 5% A → 4.0 min 5% A Ofen: 50°C; Fluss: 0.3 mL/min; UV-Detektion: 210 nm.

### Methode 7 (LC-MS):

Instrument: Agilent MS Quad 6150;HPLC: Agilent 1290; Säule: Waters Acquity UPLC HSS T3 1.8 µ 50 x 2.1 mm; Eluent A: 1 l Wasser + 0.25 mL 99%ige Ameisensäure , Eluent B: 1 l Acetonitril + 0.25 mL 99%ige Ameisensäure; Gradient: 0.0 min 90% A - 0.3 min 90% A → 1.7 min 5% A - 3.0 min 5% A Ofen: 50°C; Fluss: 1,20 mL/min; UV-Detektion: 205 - 305 nm.

### Methode 8 (LC-MS):

Gerätetyp MS: Waters Synapt G2S; Gerätetyp UPLC: Waters Acquity I-CLASS; Säule: Waters, HSST3, 2.1 × 50 mm, C18 1.8 µm; Eluent A: 1 l Wasser + 0.01% Ameisensäure; Eluent B: 1 l Acetonitril + 0.01% Ameisensäure; Gradient: 0.0 min 2% B → 2.0 min 2% B - 13.0 min 90% B → 15.0 min 90% B; Ofen: 50°C; Fluss: 1.20 ml/min; UV-Detektion: 210 nm

Methode 9: LC-MS-Prep Reinigungsmethode für die Beispiele 181-191 (Methode LIND-LC-MS-Prep)

Instrument MS: Waters, Instrument HPLC: Waters (Säule Waters X-Bridge C18, 19 mm x 50 mm, 5 µm, Eluent A: Wasser + 0.05% Ammoniak, Eluent B: Acetonitril (ULC) mit Gradient; Fluss: 40 ml/min; UV-Detektion: DAD; 210 - 400 nm).
bzw.

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäure, Eluent B: Acetonitril (ULC) mit Gradient; Fluss: 40 ml/min; UV-Detektion: DAD; 210 - 400 nm).

### Methode 10: LC-MS-Analytik-Methode für die Beispiele 181-191 (LIND_SQD_SB_AQ)

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 11 (HPLC):

| | |
|---|---|
| Gerät: | HP1100 Serie |
| Säule: | Merck Chromolith SpeedROD RP-18e, 50-4,6mm, Best.-Nr.1.51450.0001, Vorsäule Chromolith Guard Cartridge Kit, RP-18e, 5-4,6mm, Best.-Nr. 1.51470.0001 |
| Gradient: | Flow 5mL/ Min |
| | Injection Volume 5µl |
| | Solvent A: HCLO4 (70%ig) in Wasser (4mL/ l) |
| | Solvent B: Acetonitril |
| | Start 20% B |
| | 0.50 Min 20% B |
| | 3.00 Min 90% B |
| | 3.50 Min 90% B |
| | 3.51 Min 20% B |
| | 4.00 Min 20% B |
| | Säulentemperatur: 40°C |
| Wellenlänge: | 210nm |

### Methode 12 (LC-MS):

Gerätetyp MS: Thermo Scientific FT-MS; Gerätetyp UHPLC+: Thermo Scientific UltiMate 3000; Säule: Waters, HSST3, 2.1 × 75 mm, C18 1.8 µm; Eluent A: 1 l Wasser + 0.01% Ameisensäure; Eluent B: 1 l Acetonitril + 0.01% Ameisensäure; Gradient: 0.0 min 10% B - 2.5 min 95% B - 3.5 min 95% B; Ofen: 50°C; Fluss: 0.90 ml/min; UV-Detektion: 210 nm/ Optimum Integration Path 210-300 nm

### Methode 13: (LC-MS):

Instrument MS: Waters (Micromass) Quattro Micro; Instrument Waters UPLC Acquity; Säule : Waters BEH C18 1.7 µ 50 x 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A→ 2.51 min 10% A → 3.0 min 10% A; Ofen: 40°C; Fluss: 0.5 ml/min; UV-Detektion: 210 nm

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Methode 14: (LC-MS) (MCW-LTQ-POROSHELL-TFA98-10min)

Gerätetyp MS: ThermoFisherScientific LTQ-Orbitrap-XL; Gerätetyp HPLC: Agilent 1200SL; Säule: Agilent, POROSHELL 120, 3 x 150 mm, SB - C18 2.7 µm; Eluent A: 1 l Wasser + 0.1% Trifluoressigsäure; Eluent B: 1 l Acetonitril + 0.1% Trifluoressigsäure; Gradient: 0.0 min 2% B - 0.3 min 2% B - 5.0 min 95% B - 10.0 min 95% B; Ofen: 40°C; Fluss: 0.75 ml/min; UV-Detektion: 210 nm

### Ausgangsverbindungen und Intermediate:

### Intermediat C1

### Trifluoressigsäure--(1R)-1-[1-benzyl-4-(2,5-difluorphenyl)-1H-imidazol-2-yl]-2,2-dimethylpropan-1-amin(1:1)

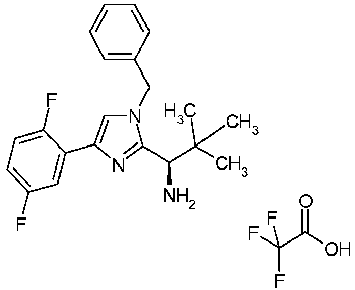

Die Titelverbindung wurde wie in WO2006/002326 beschrieben, hergestellt.

### Intermediat C2

### tert-Butyl-(2S)-4-({(1R)-1-[1-benzyl-4-(2,5-difluorphenyl)-1H-imidazol-2-yl]-2,2-dimethylpropyl}amino)-2-[(tert-butoxycarbonyl)amino]butanoat

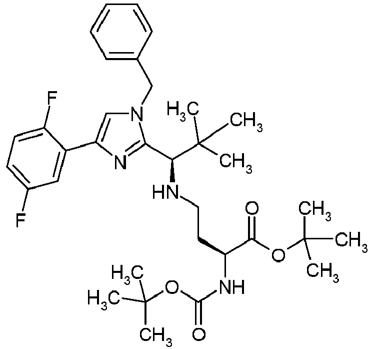

4.22 g (14.5 mmol) tert-Butyl-N-(tert-butoxycarbonyl)-L-homoserinat wurden in 180 mL Dichlormethan gelöst und dann mit 3.5 mL Pyridin sowie mit 9.2 g (21.7 mmol) 1,1,1-Triacetoxy-1lambda⁵,2-benziodoxol-3(1H)-on versetzt. Der Ansatz wurde 1 h bei RT gerührt, anschließend mit 500 mL Dichlormethan verdünnt und zweimal mit 10%-iger Natriumtiosulfatlösung und dann nacheinander zweimal mit 5%-iger Zitronensäure und zweimal mit 10%-iger Natriumhydrogencarbonatlösung ausgeschüttelt. Die organische Phase wurde abgetrennt, über Magnesiumsulfat getrocknet und anschließend im Vakuum eingeengt. Der Rückstand wurde in DCM aufgenommen und mit einer Mischung aus Diethylether und n-Pentan versetzt. Der Niederschlag wurde abfiltriert und das Filtrat anschließend einggengt und aus Acetonitril/Wasser lyophilisiert. Es wurden 3.7 g (93%) tert-Butyl-(2S)-2-[(tert-butoxycarbonyl)amino]-4-oxobutanoat erhalten, die ohne weitere Reinigung in die nächste Stufe eingesetzt wurden. (R_{f}-Wert: 0.5 (DCM/Methanol 95/5).

3.5 g (9.85 mmol) von Intermediat C1 wurden in 160 mL DCM gelöst und mit 3.13 g (14.77 mmol) Natriumtriacetoxyborhydrid sowie mit 0.7 mL Essigsäure versetzt. Nach 5 min Rühren bei RT wurden 3.23 g (11.85 mmol) tert-Butyl-(2S)-2-[(tert-butoxycarbonyl)amino]-4-oxobutanoat zugegeben und der Ansatz weitere 30 min bei RT gerührt. Dann wurde das Lösunsgmittel im Vakuum eingedampft und der Rückstand in Acetonitril/Wasser aufgenommen. Der ausgefallene Feststoff wurde abfiltriert und getrocknet und man erhielt 5.46 g (84%) der Titelverbindung. HPLC (Methode 11): Rₜ = 2.5 min;

LC-MS (Methode 1): Rₜ = 1.13 min; MS (ESIpos): m/z = 613 (M+H)⁺.

### Intermediat C3

### (2S)-4-[(Acetoxyacetyl){(1R)-1-[1-benzyl-4-(2,5-difluorphenyl)-1H-imidazol-2-yl] -2,2-dimethylpropyl} amino] -2-[(tert-butoxycarbonyl)amino]butansäure

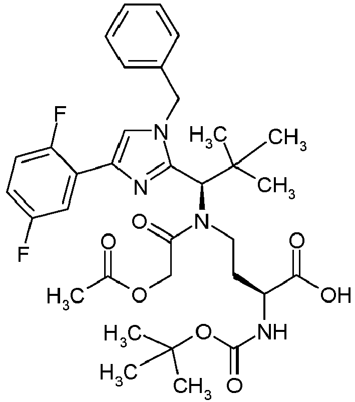

5.46 g (8.24 mmol) von Intermediat C2 wurden in 160 mL DCM gelöst und dann mit 4.8 mL Triethylamin sowie mit 2.2 mL (20.6 mmol) Acetoxyacetylchlorid versetzt. Der Ansatz wurde über Nacht bei RT gerührt und dann im Vakuum eingeengt. Der Rückstand wurde in Ethylacetat aufgenommen und dreimal mit gesättigter Natriumhydrogencarbonatlösung und dann mit gesättigter Natriumchloridlösung ausgeschüttelt. Die organische Phase wurde über Natriumsulfat getrocknet und anschließend eingeengt. Der Rückstand wurde durch Säulenchromatographie über Biotage/Isolera (SNAP 340g) mit Cyclohexan/Ethylacetat 2:1 als Eluent gereinigt. Es wurden 4.57 g (75%) der acylierten Zwischestufe erhalten.

LC-MS (Methode 1): Rₜ = 1.49 min; MS (ESIpos): m/z = 713 (M+H)⁺.

1g (1.36 mmol) von dieser Zwischenstufe wurden in 20 mL DCM gelöst und mit 20 mL TFA versetzt. Nach 5 h Rühren bei RT wurde eingeengt und der Rückstand zweimal mit n-Penan verrührt. Das n-Pentan wurde jeweils abdekantiert und der verbliebene Feststoff im Hochvakuum getrocknet. So wurden 1.1 g (2S)-4-[(Acetoxyacetyl){(1R)-1-[1-benzyl-4-(2,5-difluorphenyl)-1H-imidazol-2-yl]-2,2-dimethylpropyl}amino]-2-aminobutansäure-trifluoressigsäure(1:1) erhalten. LC-MS (Methode 1): Rₜ = 0.93 min; MS (ESIpos): m/z = 557 (M+H)⁺.

0.91 g (1.57 mmol) von dieser Zwischenstufe wurden in 70 mL DCM gelöst und mit 3.43 g (15.7 mmol) Di-tert-butyldicarbonat und 4.1 mL *N*,*N*-Diisopropylethylamin versetzt. Nach 30 min Rühren bei RT wurde der Ansatz mit DCM verdünnt und mit 5%-iger Zitronensäure ausgeschüttelt. Die organische Phase wurde über Natriumsulfat getrocknet und einggengt. Der Rückstand wurde zweimal mit n-Pentan verrührt und jeweils abdekantiert. Der verbliebene Feststoff wurde aus Acetonitril/Wasser 1: 1 lyophilisiert und so wurden 1.11 g der Titelverbindung erhalten.
HPLC (Methode 11): Rₜ = 2.55 min;
LC-MS (Methode 1): Rₜ = 1.3 min; MS (ESIpos): m/z = 657 (M+H)⁺.

### Intermediat C4

### (2S)-2-Amino-4-[{(1R)-1-[1-benzyl-4-(2,5-difluorphenyl)-1H-imidazol-2-yl]-2,2-dimethylpropyl}(glycoloyl)amino]butansäure-trifluoressigsäure(1:1)

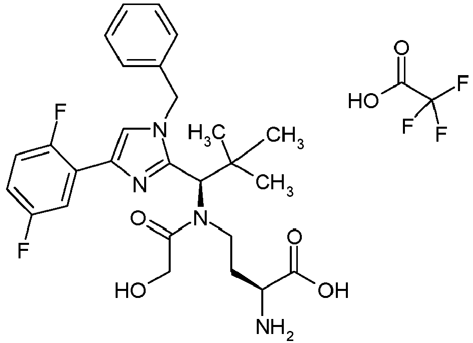

5.46 g (8.24 mmol) von Intermediat C2 wurden in 160 mL DCM gelöst und dann mit 4.8 mL Triethylamin sowie mit 2.2 mL (20.6 mmol) Acetoxyacetylchlorid versetzt. Der Ansatz wurde über Nacht bei RT gerührt und dann im Vakuum eingeengt. Der Rückstand wurde in Ethylacetat aufgenommen und dreimal mit gesättigter Natriumhydrogencarbonatlösung und dann mit gesättigter Natriumchloridlösung ausgeschüttelt. Die organische Phase wurde über Natriumsulfat getrocknet und anschließend eingeengt. Der Rückstand wurde durch Säulenchromatographie über Biotage/Isolera (SNAP 340g) mit Cyclohexan/Ethylacetat 2:1 als Eluent gereinigt. Es wurden 4.57 g (75%) der acylierten Zwischestufe erhalten.

LC-MS (Methode 1): Rₜ = 1.49 min; MS (ESIpos): m/z = 713 (M+H)⁺.

1.5 g (2.035 mmol) von dieser Zwischenstufe wurden in 50 mL Ethanol aufgenommen und mit 5.8 mL einer 40%-igen Methanaminlösung in Wasser versetzt. Der Ansatz wurde 4 h bei 50°C gerührt und anschließend eingeengt. Der Rückstand wurde in DCM aufgenommen und zweimal mit Wasser gewaschen. Die organische Phase wurde über Magnesiumsulfat getrocknet und anschließend eingeengt. Der Rückstand wurde im Hochvakuum getrocknet. Es wurden 1.235 mg dieser Zwischenstufe erhaltenen die ohne weitere Reinigung weiter umgesetzt wurden.

1.235 mg (1.5 mmol) von dieser Zwischenstufe wurden in 15 mL DCM gelöst und mit 15 mL TFA versetzt. Nach 4 h Rühren bei RT wurde eingeengt. Der Rückstand wurde durch präparative HPLC gereinigt. Die entsprechenden Fraktionen wurden eingeengt und der Rückstand aus Acetonitril lyophilisiert. Es wurden 1.04 g (quant) der Titelverbindung erhalten.
HPLC (Methode 11): Rₜ = 1.9 min;
LC-MS (Methode 1): Rₜ = 0.89 min; MS (ESIpos): m/z = 515 (M+H)⁺.

### Intermediat C5

### (2S)-4-[{(1R)-1-[1-Benzyl-4-(2,5-difluorphenyl)-1H-imidazol-2-yl]-2,2-dimethylpropyl}(glycoloyl)amino]-2-[(tert-butoxycarbonyl)amino]butansäure

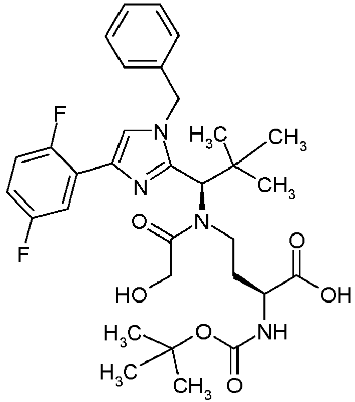

0.9 g (1.24 mmol) von Intermediat C4 wurden in 60 mL DCM gelöst und mit 2.7 g (12.5 mmol) Di-tert-butyldicarbonat sowie mit 3.3 mL *N,N-*Diisopropylethylamin versetzt. Nach 45 min Rühren bei RT wurde der Ansatz eingeengt, der Rückstand in Diethylether aufgenommen und bis zur einsetzenden Trübung mit n-Pentan versetzt. Der Ansatz wurde auf 0°C abgekühlt und anschließend dekantiert. Der Rückstand wurde erneut mit n-Pentan versetzt und abdekantiert. Der verbliebene Feststoff wurde aus Acetonitril/Wasser 1: 1 lyophilisiert und so wurden 0.95 g (quant) der Titelverbindung erhalten.
HPLC (Methode 11): Rₜ = 2.5 min;
LC-MS (Methode 1): Rₜ = 1.27 min; MS (ESIpos): m/z = 615 (M+H)⁺.

### Intermediat C6

### Trifluoressigsäure--tert-butyl-{(2S)-4-[{(1R)-1-[1-benzyl-4-(2,5-difluorphenyl)-1H-imidazol-2-yl]-2,2-dimethylpropyl}(glycoloyl)amino]-1-hydrazino-1-oxobutan-2-yl}carbamat(1:1)

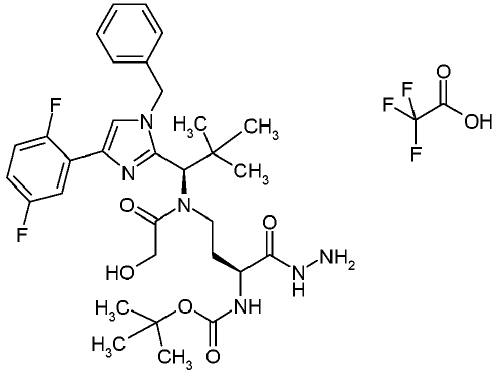

150 mg (0.16 mmol) von Intermediat C3 wurden in 21 mL DMF gelöst und dann mit 37.2 mg (0.19 mmol) N-(3-Dimethylaminopropyl)-*N*'-ethylcarbodiimid-Hydrochlorid (EDC), mit 37 mg (0.243 mmol) 1-Hydroxybenzotriazol, mit 85 µl *N,N*-Diisopropylethylamin und schließlich mit 45 mg (0.18mmol ) von kommerziell erhältlichem 9H-Fluoren-9-ylmethylhydrazincarboxylat versetzt. Der Ansatz wurde über Nacht bei RT gerührt und dann im Vakuum eingeengt. Der Rückstand wurde durch präparative HPLC gereinigt. Die entsprechenden Fraktionen wurden eingeengt und der Rückstand aus Acetonitril/Wasser lyophilisiert. Es wurden 60 mg (41% d. TH.) der geschützten Zwischenstufe erhalten.
HPLC (Methode 11): Rₜ = 2.9 min;
LC-MS (Methode 1): Rₜ = 1.47 min; MS (ESIpos): m/z = 893 (M+H)⁺.

60mg (0.067 mmol) dieser Zwischenstufe wurden in 19 mL Ethanol gelöst und mit 681 µl Piperidin sowie mit 386 µl einer 40%-igen Methanaminlösung in Wasser versetzt. Der Ansatz wurde 18 h bei 50°C gerührt und anschließend eingeengt. Der Rückstand wurde in Acetonitril/Wasser 2:1 aufgenommen und mit TFA auf pH 2 gebracht. Dann wurde erneut eingeengt und der Rückstand durch präparative HPLC gereinigt. Die entsprechenden Fraktionen wurden eingeengt und der Rückstand aus Acetonitril/Wasser lyophilisiert. Es wurden 25 mg (51% d.Th.) der Titelverbindung erhalten.
HPLC (Methode 11): Rₜ = 2.2 min;
LC-MS (Methode 1): Rₜ = 1.27 min; MS (ESIpos): m/z = 629 (M+H)⁺.

### Intermediat C7

### 1-{(2S)-4-[{(1R)-1-[1-Benzyl-4-(2,5-difluorphenyl)-1H-imidazol-2-yl]-2,2-dimethylpropyl}(glycoloyl)amino]-2-[(tert-butoxycarbonyl)amino]butanoyl}hydrazino)essigsäure - trifluoressigsäure (1:1)

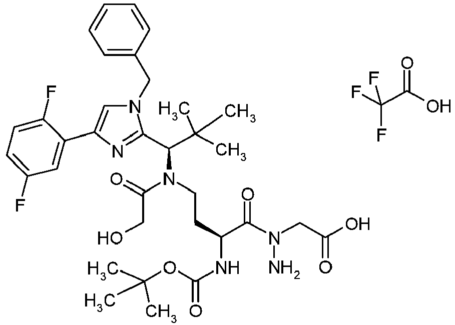

0.2 g (0.305 mmol) von Intermediat C3 wurden in 80 mL DCM gelöst und mit 0.125 g (0.46 mmol) 2-Brom-1-ethylpyridinium-Tetrafluoroborat (BEP), mit 94 mg (0.61 mmol) von kommerziell erhältlichem Hydrazinoessigsaeureethylester Hydrochlorid sowie mit 159 µl *N,N-*Diisopropylethylamin versetzt und die Mischung anschließend 1 h bei RT gerührt. Das Reaktionsgemisch wurde danach mit Ethylacetat und Wasser versetzt und die Phasen voneinander getrennt. Die organische Phase wurde mit gesättigter Natriumchloridlösung ausgeschüttelt und dann über Magnesiumsulfat getrocknet, filtriert und eingeengt. Der Rückstand wurde im Vakuum getrocknet und ohne Reinigung weiter umgesetzt. Dazu wurde er in 20 mL Tetrahydrofuran aufgenommen und mit 10 mL Wasser und 3.2 mL einer 2N-Lithiumhydroxidlösung versetzt. Der Ansatz wurde 1 h bei RT gerührt und anschließend mit TFA auf pH 7 gebracht. Danach wurde der Ansatz eingeengt und der Rückstand durch präparative HPLC gereinigt. So wurde die Titelverbindung von ihrem früher eluierenden Regioisomer abgetrennt. Nach Vereinigung der entsprechenden Fraktionen, Lyophilisation und Trocknung wurden 19.7 mg (8% d.Th. über 2 Stufen) der Titelverbindung als farbloser Schaum erhalten.
HPLC (Methode 11): Rₜ = 2.4 min;
LC-MS (Methode 1): Rₜ = 1.22 min; MS (ESIpos): m/z = 687 (M+H)⁺.

Die Strukturzuordnung der Regiosisomere erfolgte in einem separaten Experiment nach Auftrennung der Regioisomeren auf der geschützten Zwischenstufe durch NMR-Spektroskopie. Die geschützte Zwischenstufe Ethyl-(1-{(2S)-4-[(acetoxyacetyl) {(1R)-1-[1-benzyl-4-(2,5-difluorphenyl)-1H-imidazol-2-yl]-2,2-dimethylpropyl }amino]-2-[(tert-butoxycarbonyl) amino]butanoyl}hydrazino)acetat der Titelverbindung wies folgendes 1H-NMR-Spektrum auf:
¹H-NMR (500 MHz, DMSO-d₆): δ = 7.8 (m, 2H), 7.4-7.2 (m, 6H), 7.08 (m, 1H), 6.73 (d, 1H), 5.6 (s, 1H), 5.25 und 4,89 (2d, 2H), 4.89 und 4.77 (2d, 2H), 4.62 (t, 1H), 4.32 und 3.78 (2d, 2H), 4.1 (t, 2H), 3.62-3.47 (m), 2.13 (s, 3H), 1.41 und 0.72 (2m, 2H), 1.3 (s, 9H), 1.18 (t, 3H), 0.92 (s, 9H).

### Intermediat C8

### N-{(2S)-4-[{(1R)-1-[1-Benzyl-4-(2,5-difluorphenyl)-1H-imidazol-2-yl]-2,2-dimethylpropyl}(glycoloyl)amino]-2-[(tert-butoxycarbonyl)amino]butanoyl}-beta-alanin

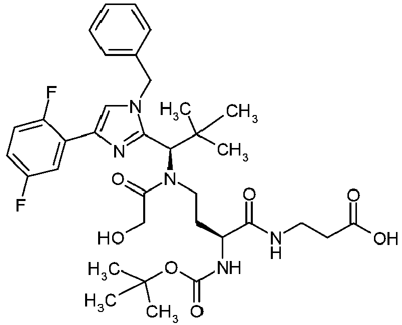

293 mg (0.41 mmol) von Intermediat C3 wurden in 25 mL DMF gelöst und dann mit 144 mg (0.75 mmol) N-(3-Dimethylaminopropyl)-*N*'-ethylcarbodiimid-Hydrochlorid (EDC), mit 128 mg (0.83 mmol) 1-Hydroxybenzotriazol, mit 218 µl *N,N*-Diisopropylethylamin und schließlich mit 70 mg (0.5mmol) von kommerziell erhältlichem 3-Methoxy-3-oxopropan-1-aminiumchlorid versetzt. Der Ansatz wurde 4 h bei RT gerührt und dann im Vakuum eingeengt. Der Rückstand wurde durch präparative HPLC gereinigt. Die entsprechenden Fraktionen wurden eingeengt und der Rückstand im Hochvakuum getrocknet. Es wurden 177 mg (53% d. TH.) der geschützten Zwischenstufe erhalten.
HPLC (Methode 11): Rₜ = 2.6 min;
LC-MS (Methode 1): Rₜ = 1.33 min; MS (ESIpos): m/z = 742 (M+H)⁺.

177 mg (0.22 mmol) dieser Zwischenstufe wurden in 20 mL Methanol aufgenommen und mit 2.8 mL einer 2N-Lithiumhydroxidlösung versetzt. Der Ansatz wurde 18 h bei RT gerührt. Anschließend wurde eingeengt, der Rückstand in Wasser aufgenommen und die Lösung mit 5%-iger Zitronensäure auf pH 5 gebracht. Danach wurde zweimal mit DCM extrahiert, die organische Phase über Magnesiumsulfat getrocknet und eingeengt. Der Rückstand wurde schließlich aus Acetonitrol/Wasser lyophilisiert und so wurden 133 mg (81% d. Th.) der Titelverbindung erhalten. HPLC (Methode 11): Rₜ = 2.3 min;

LC-MS (Methode 3): Rₜ = 7.4 min; MS (ESIpos): m/z = 686 (M+H)⁺.

### Intermediat C9

### (6S)-6-{2-[{(1R)-1-[1-Benzyl-4-(2,5-difluorphenyl)-1H-imidazol-2-yl]-2,2-dimethylpropyl} (glycoloyl)amino]ethyl}-2,2-dimethyl-4,7-dioxo-3,11,14,17-tetraoxa-5,8-diazaicosan-20-säure

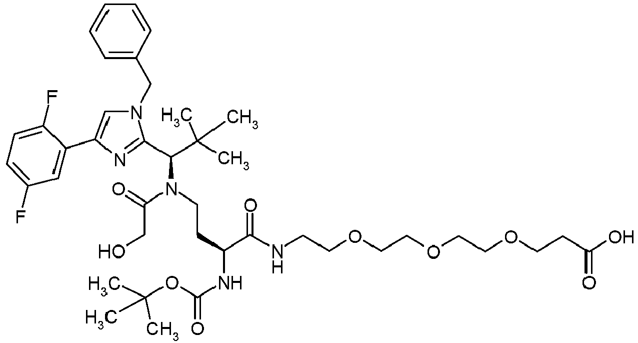

Im ersten Schritt wurden 70 mg (0.114 mmol) von Intermediat C5 mit 32 mg (0.114 mmol) tert-Butyl-3-{2-[2-(2-aminoethoxy)ethoxy]ethoxy}propanoat in 15 mL DMF in Gegenwart von 44 mg (0.228 mmol) 1-(3-Dimethylaminopropyl)-3-ethylcarbodiimid-Hydrochlorid, 35 mg (0.228 mmol) 1-Hydroxy-1*H*-benzotriazol-Hydrat und 60 µl *N,N*-Diisopropylethylamin gekuppelt.

Der Ansatz wurde über Nacht bei RT gerührt und das Produkt durch präparative HPLC gereinigt. Es wurden 33 mg (33% d.Th.) des geschützten Intermediats isoliert. Dieses wurde 1 h mit 1.1 mL Trifluoressigsäure in 11 mL Dichlormethan gerührt und nach Aufarbeitung wurden 26 mg (98%) der vollständig entschützten Verbindung erhalten.
Schließlich wurde das Intermediat in 2 mL DCM aufgenommen und die tert.-Butoxycarbonylschutzgruppe durch zweimalige Zugabe von jeweils 10 mg Di-tert-butyldicarbonat und 79 µl *N,N*-Diisopropylethylamin unter 3-tägigem Rühren bei RT eingeführt. Nach Reinigung des Produktes durch präparative HPLC wurden 16.4 mg (66% d.Th.) der Titelverbindung erhalten. HPLC (Methode 11): Rₜ = 2.3 min;
LC-MS (Methode 1): Rₜ = 1.22 min; MS (ESIpos): m/z = 818 (M+H)⁺.

### Intermediat C10

### tert-Butyl-{3-[{(1R)-1-[1-(3-aminobenzyl)-4-(2,5-difluorphenyl)-1H-imidazol-2-yl]-2,2-dimethylpropyl} (glycoloyl)amino]propyl} carbamat

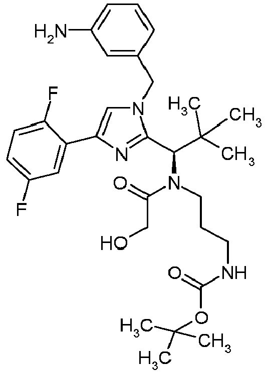

Die Titelverbindung wurde ausgehend von Intermediat C1 über 6 Stufen hergestellt: Im ersten Schritt wurden 1 g (2.77 mmol) von Intermediat C1 und 0.864 g (5 mmol) tert-Butyl-(3-oxopropyl)carbamat in 100 mL Methanol zusammengegeben und mit 400 mL Essigsäure sowie 1.288 g (13.9 mmol) Boran-Pyridin-Komplex versetzt. Der Ansatz wurde 3 Tage bei RT gerührt. Anschließend wurde im Vakuum eingeengt und der Rückstand mittels Flash-Chromatographie an Kieselgel (Eluent: Dichlormethan/Ethylacetat 9:1 -> Dichlormethan/Methanol 95:5) gereinigt. Nach Einengen der entsprechenden Fraktionen und Trocknung im Hochvakuum wurden 1255 g (80% d.Th.) des N-alkylierten Zwischenstufe erhalten.

LC-MS (Methode 1): Rₜ = 1.0 min; MS (ESIpos): m/z = 513 (M+H)⁺.

1.255 g (2.2 mmol) von dieser Zwischenstufe wurden in 50 mL DCM gelöst und dann mit 1.2 mL Triethylamin sowie mit 0.52 mL (4.85 mmol) Acetoxyacetylchlorid versetzt. Der Ansatz wurde über Nacht bei RT gerührt und dann im Vakuum eingeengt. Der Rückstand wurde in Ethylacetat aufgenommen und dreimal mit gesättigter Natriumhydrogencarbonatlösung und dann mit gesättigter Natriumchloridlösung ausgeschüttelt. Die organische Phase wurde über Natriumsulfat getrocknet und anschließend eingeengt. Der Rückstand wurde durch präparative HPLC gereinigt.
Es wurden 593 mg (41% d. Th.) der acylierten Zwischestufe erhalten.
LC-MS (Methode 1): Rₜ = 1.4 min; MS (ESIpos): m/z = 613 (M+H)⁺.

993 mg (0.91 mmol) dieser Zwischenstufe wurden in 100 mL Ethanol gelöst und nach Zugabe von 60 mg 10%-igem Palladium auf Aktivkohle 3 h lang bei RT unter Wasserstoff-Normaldruck hydriert. Der Katalysator wurde danach abfiltriert und das Lösungsmittel im Vakuum entfernt. Man erhielt 494 mg (91% d.Th.) des de-benzylierten Imidazol-Derivats als nahezu farbloses Öl. LC-MS (Methode 1): Rₜ = 1.17 min; MS (ESIpos): m/z = 523 (M+H)⁺.

150 mg (0.25 mmol) von dieser Zwischenstufe wurden in 15 mL DMF vorgelegt und mit 69.2 mg (0.5 mmol) Kaliumcarbonat versetzt. Nach 15min Rühren bei RT wurden 60 mg (0.28 mmol) p-Nitrobenzylbromid zugegeben und über Nacht gerührt. Das Lösungsmittel wurde anschließend im Vakuum entfernt, der Rückstand in Ethylacetat aufgenommen und mit gesättigter Natriumhydrogencarbonatlösung extrahiert. Die organische Phase wurde mit gesättigter Natriumchloridlösung gewaschen, einrotiert und mittels präparativer HPLC gereinigt. Die entsprechenden Fraktionen wurden einrotiert und der Rückstand aus 1,4-Dioxan lyophilisiert. Es wurden 169 mg (quant.) der Zwischenstufe erhalten.

LC-MS (Methode 1): Rₜ = 1.39 min; MS (ESIpos): m/z = 658 (M+H)⁺.

165 mg (0.251 mmol) dieser Zwischenstufe wurden in 30 mL Ethanol aufgenommen und mit 0.72 mL einer 40%-igen wässrigen Lösung von Methylamin versetzt. Der Ansatz wurde 5 h bei 50°C gerührt und dann wurde nochmals die gleiche Menge der Methylaminlösung zugegeben. Nach weiteren 10 h Rühren wurde der Ansatz im Vakuum eingeengt. Es wurde zweimal mit Diethylether nachdestilliert und der Rückstand anschließend aus Acetonitril/Wasser lyophilisiert. Es verblieben 148 mg (89%) dieser Zwischenstufe.

LC-MS (Methode 6): Rₜ = 2.97 min; MS (ESIpos): m/z = 616 (M+H)⁺.

98 mg (0.15 mmol) der Vorstufe wurden in 15 mL THF gelöst und dann wurde bei RT eine Lösung aus 569 mg (3.27 mmol) Dinatriumdithionit in 6 mL Wasser zugegeben. Nach 8 h Rühren bei 50°C wurde nochmal die gleiche Menge Dithionit - in 1mL H2O gelöst - zugeben. Nach weiterem 16 stündigem Rühren bei 50°C wurde der Ansatz auf RT abgekühlt und mit Ethylacetat extrahiert. Die organische Phase wurde eingeengt und der Rückstand durch präparative HPLC gereinigt. Nach Lyophilisation des Rückstandes aus 1,4-Dioxan wurden 44.5 mg (47% d. Th.) der Titelverbindung erhalten.

LC-MS (Methode 1): Rₜ = 1.24 min; MS (ESIpos): m/z = 586 (M+H)⁺.

### Intermediat C11

### R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-homocystein-trifluoressigsäure(1:1)

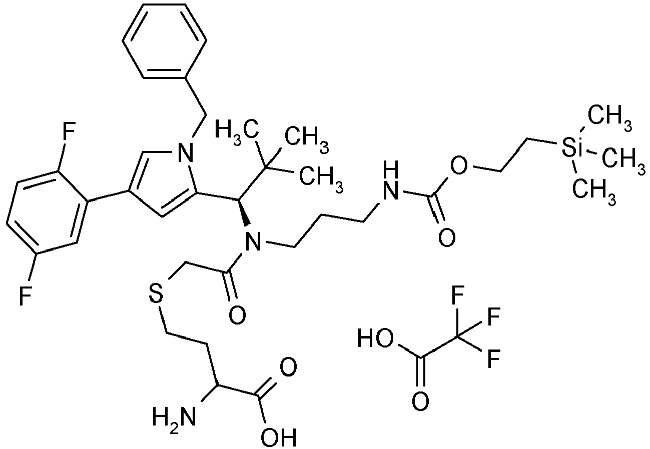

990.0 mg (2.79 mmol) (1R)-1-[1-Benzyl-4-(2,5-difluorphenyl)-1H-pyrrol-2-yl]-2,2-dimethylpropan-1-amin wurden in 15.0 mL Dichlormethan vorgelegt und mit 828.8 mg (3.91 mmol) Natriumtriacetoxyborhydrid und 129.9 mg (3.21 mmol) Essigsäure versetzt und 5 min bei RT grührt. Es wurden 698.1 mg (3.21 mmol) 2-(Trimethylsilyl)ethyl-(3-oxopropyl)carbamat (Intermediat L58) gelöst in 15.0 mL Dichlormethan zugegeben und die Reaktionsmischung über Nacht bei RT gerührt. Die Reaktionsmischung wurde mit Ethylacetat verdünnt und die organische Phase je zweimal mit ges. Natriumcarbonat-Lösung und ges. NaCl-Lösung gewaschen. Die organische Phase wurde über Magnesiumsulfat getrocknet und das Lösemittel im Vakuum verdampft. Der Rückstand wurde mittels Kieselgel (Laufmittel: Dichlormethan/Methanol 100:2) gereinigt. Die Lösemittel wurden im Vakuum verdampft und der Rückstand im Hochvakuum getrocknet. Man erhielt 1.25 g (73 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat.

LC-MS (Methode 1): Rₜ = 1.09 min; MS (ESIpos): m/z = 556 (M+H)⁺.

400.0 mg (0.65 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat wurden mit 151.4 mg (1.5 mmol) Triethylamin und mit 161.6 mg (1.43 mmol) Chloracetylchlorid versetzt. Die Reaktionsmischung wurde über Nacht bei RT gerührt. Die Reaktionsmischung wurde mit Ethylacetat versetzt und die organische Phase dreimal mit Wasser und einmal mit ges. NaCl-Lösung gewaschen. Es wurde über Magnesiumsulfat getrocknet und das Lösemittel im Vakuum verdampft. Der Rückstand wurde mittels Kieselgel (Laufmittel: Cyclohexan/Ethylacetat 3:1) gereinigt. Die Lösemittel wurden im Vakuum verdampft und der Rückstand im Hochvakuum getrocknet. Man erhielt 254.4 mg (57 % d. Th.) der Verbindung 2-(Trimethylsilyl)ethyl-{3-[{(1R)-1-[1-benzyl-4-(2,5-difluorphenyl)-1H-pyrrol-2-yl] -2,2-dimethylpropyl}(chloracetyl)amino]propyl} carbamat.

LC-MS (Methode 1): Rₜ = 1.49 min; MS (ESIneg): m/z = 676 (M+HCOO⁻)⁻.

117.4 mg (0.19 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat wurde in 10.0 mL Isopropanol gelöst und mit 928.4 µL 1M NaOH und 50.2 mg (0.37 mmol) DL-Homocystein versetzt. Die Reaktionsmischung wurde 4.5 h bei 50 °C gerührt. Die Reaktionsmischung wurde mit Ethylacetat versetzt und die organische Phase mit ges. Natriumhydrogencarbonat-Lösung und ges. NaCl-Lösung gewaschen. Die organische Phase wurde über Magnesiumsulfat getrocknet und das Lösemittel im Vakuum verdampft. Der Rückstand wurde mittles präp. RP-HPLC gereinigt (Säule: Reprosil 250x40; 10µ, Fluss: 50 mL/min, MeCN/Wasser, 0.1 % TFA). Die Lösemittel wurden im Vakuum verdampft und der Rückstand im Hochvakuum getrocknet. Man erhielt 75.3 mg (48 % d. Th.) der Titelverbindung.

LC-MS (Methode 1): Rₜ = 1.24 min; MS (ESIpos): m/z = 731 (M+H)⁺.

¹H-NMR (400 MHz, DMSO-d₆): δ [ppm] = 0.03 (s, 9H), 0.40 (m, 1H), 0.75-0.91 (m, 11H), 1.30 (m, 1H), 1.99-2.23 (m, 2H), 2.63-2.88 (m, 4H), 3.18-3.61 (m, 5H), 3.79-4.10 (m, 3H), 4.89 (d, 1H), 4.89 (d, 1H), 5.16 (d, 1H), 5.56 (s, 1H), 6.82 (m, 1H), 6.91 (s, 1H), 6.97 (m, 1H), 7.13-7.38 (m, 6H), 7.49 (s, 1H), 7.63 (m, 1H), 8.26 (s, 3H).

### Intermediat C12

### R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]homocystein

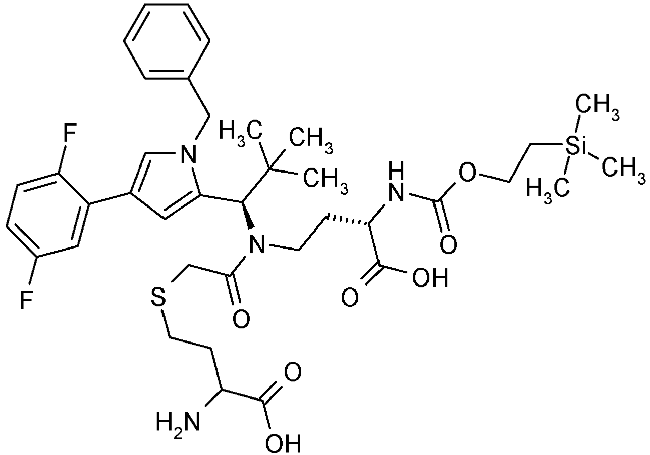

Die Synthese erfolgte in Analogie zur Synthese von Intermediat C11 mit Methyl-(2S)-4-oxo-2-({[2-(trimethylsilyl)ethoxy]carbonyl}amino)butanoat (Intermediat L57) und Intermediat C52 als Edukten.

LC-MS (Methode 1): Rₜ = 1.18 min; MS (ESIpos): m/z = 775 (M+H)⁺.

### Intermediat C13

### 9-{(1R)-1-[1-Benzyl-4-(2,5-difluorphenyl)-1H-imidazol-2-yl]-2,2-dimethylpropyl}-2,2-dimethyl-4,10-dioxo-3-oxa-12-thia-5,9-diazaoctadecan-18-säure

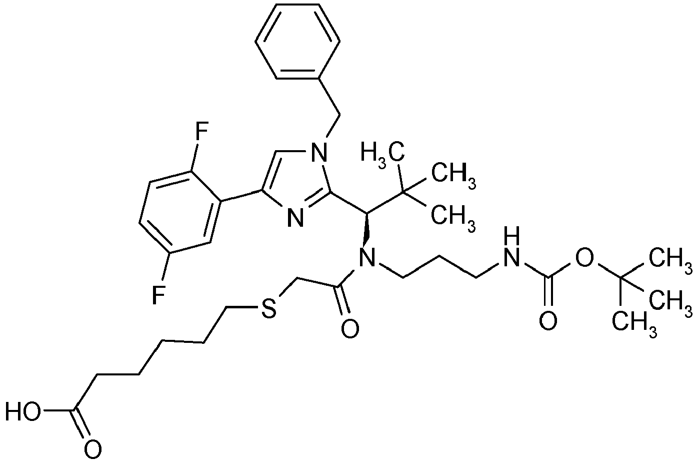

90.0 mg (0.15 mmol) Intermediat C16 und 43.6 mg (0.23 mmol) 6-(Acetylsulfanyl)hexansäure wurden in 9.0 mL Methanol gelöst und mit einem Tropfen Wasser und 73.9 mg (0.54 mmol) Kaliumcarbonat versetzt. Die Reaktionsmischung rührte 4 h bei 50 °C und wurde dann mit Ethylacetat verdünnt. Die organische Phase wurde mit Wasser/ges. NaCl-Lösung und ges. NaCl-Lösung gewaschen und anschließend über Magnesiumsulfat getrocknet. Das Lösemittel wurde im Vakuum verdampft und der Rückstand an Kieselgel chromatographiert (Laufmittel: Dichlormethan/Methanol = 100:2). Die Lösemittel wurden im Vakuum verdampft und der Rückstand im Hochvakuum getrocknet. Man erhielt die Titelverbindung in 83 % d. Th.

LC-MS (Methode 1): Rₜ = 1.44 min; MS (ESIpos): m/z = 701 (M+H)⁺.

### Intermediat C14

### R/S-[2-({(1R)-1-[1-Benzyl-4-(2,5-difluorphenyl)-1H-imidazol-2-yl]-2,2-dimethylpropyl}{3-[(tert-butoxycarbonyl)amino]propyl} amino)-2-oxoethyl]homocystein

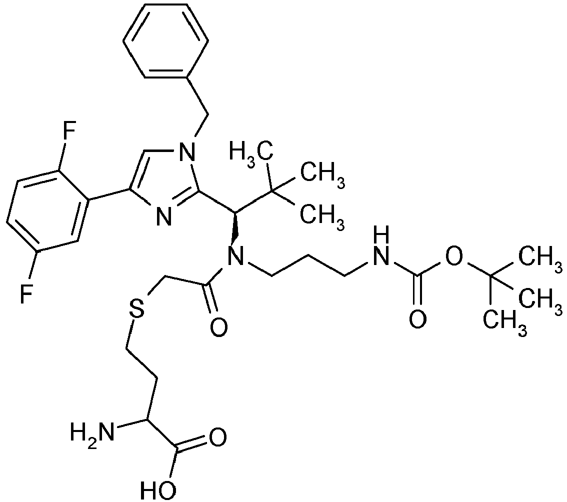

100.0 mg (0.17 mmol) tert-Butyl-{3-[{(1R)-1-[1-benzyl-4-(2,5-difluorphenyl)-1H-imidazol-2-yl]-2,2-dimethylpropyl}(chloracetyl)amino]propyl}carbamat (Intermediat C16) wurden in 4.0 mL isoPropanol vorgelegt und mit 276.5 mg (0.85 mmol) 1 M NaOH-Lösung und mit 45.9 mg (0.34 mmol) D/L-Homocystein versetzt. Die Reaktionsmischung wurde 1 h bei 50 °C gerührt. Die Reaktionsmischung wurde mit Ethylacetat verdünnt. Die organische Phase wurde mit ges. Natriumhydrogencarbonat-Lösung und ges. NaCl-Lösung gewaschen. Die Trocknung erfolgte über Magnesiumsulfat und das Lösemittel wurde im Vakuum verdampft. Der Rückstand wurde mittles präp. RP-HPLC gereinigt (Säule: Reprosil 250x40; 10µ, Fluss: 50 mL/min, MeCN/Wasser). Die Lösemittel wurden im Vakuum verdampft und der Rückstand im Hochvakuum getrocknet.

Man erhielt 92.6 mg (66 % d. Th.) der Titelverbindung.

LC-MS (Methode 1): Rₜ = 1.07 min; MS (ESIpos): m/z = 688 (M+H)⁺.

### Intermediat C15

### tert-Butyl-[3-({(1R)-1-[1-benzyl-4-(2,5-difluorphenyl)-1H-imidazol-2-yl]-2,2-dimethylpropyl} amino)propyl]carbamat

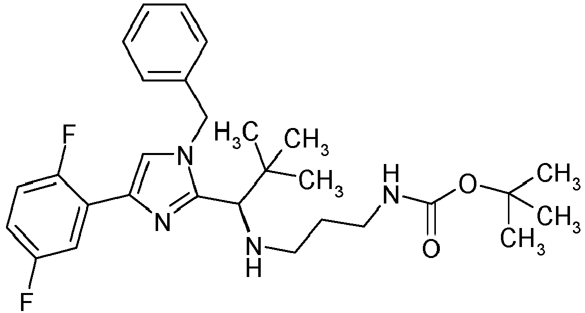

750.0 mg (2.11 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C1) wurden in 15.0 mL Dichlormethan gelöst und mit 626.0 mg (2.95 mmol) Natriumtriacetoxyborhydrid und 139 µL (2.43 mmol) HOAc versetzt und 5 min bei RT gerührt. Dann wurden 420.3 mg (2.43 mmol) tert-Butyl-(3-oxopropyl)carbamat (Die Synthese erfolgte nach Literaturvorschrift J.Med.Chem. 2003, 46, 3536.) zugegeben und bei RT über Nacht gerührt. Die Reaktionsmischung wurde mit Ethylacetat versetzt und zweimal mit ges. Natriumcarbonat-Lösung extrahiert. Die organische Phase wurde mit ges. NaCl-Lösung gewaschen und über Magnesiumsulfat getrocknet. Das Lösemittel wurde im Vakuum verdampft und der Rückstand wurde an Kieselgel chromatographiert. (Laufmittel: Cyclohexan/Ethylacetat = 4:1). Die Lösemittel wurden im Vakuum verdampft und der Rückstand im Hochvakuum getrocknet. Man erhielt 881.0 mg (82 % d. Th.) der Titelverbindung.

LC-MS (Methode 1): Rₜ = 1.07 min; MS (ESIpos): m/z = 513 [M+H]⁺.

### Intermediat C16

### tert-Butyl-{3-[{(1R)-1-[1-benzyl-4-(2,5-difluorphenyl)-1H-imidazol-2-yl]-2,2-dimethylpropyl}(chloracetyl)amino]propyl}carbamat

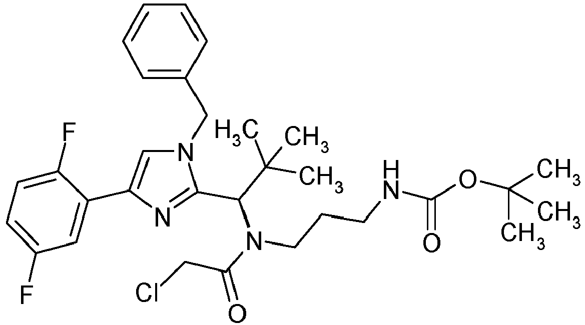

373.4 mg (0.73 mmol) tert-Butyl-[3-({(1R)-1-[1-benzyl-4-(2,5-difluorphenyl)-1H-imidazol-2-yl]-2,2-dimethylpropyl}amino)propyl]carbamat (Intermediat C15) wurden in 5.0 mL Dichlormethan vorgelegt und mit 169.5 mg (1.68 mmol) Triethylamin und 181.0 mg (1.60 mmol) Chloracetylchlorid versetzt. Die Reaktionsmischung wurde über Nacht bei RT gerührt und dann mit Ethylacetat versetzt und mehrmals mit Wasser extrahiert. Die organische Phase wurde mit ges. NaCl-Lösung gewaschen und über Magnesiumsulfat getrocknet. Das Lösemittel wurde im Vakuum verdampft und der Rückstand wurde an Kieselgel chromatographiert (Laufmittel: Dichlormethan/Methanol = 100:0.5). Die Lösemittel wurden im Vakuum verdampft und der Rückstand im Hochvakuum getrocknet. Man erhielt 336.0 mg (75 % d. Th.) der Titelverbindung.

LC-MS (Methode 1): Rₜ = 1.48 min; MS (ESIpos): m/z = 589 [M+H]⁺.

### Intermediat C17

### 9-{(1R)-1-[1-Benzyl-4-(2,5-difluorphenyl)-1H-imidazol-2-yl]-2,2-dimethylpropyl}-2,2-dimethyl-4,10-dioxo-3,15,18,21,24-pentaoxa-12-thia-5,9-diazaheptacosan-27-säure

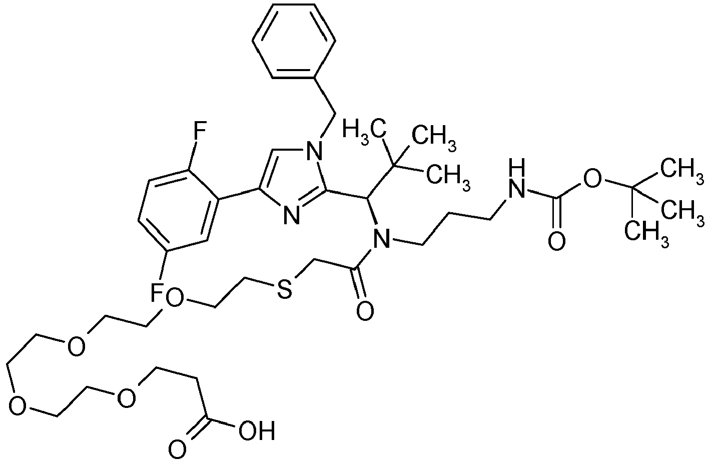

50.0 mg (0.09 mmol) tert-Butyl-{3-[{(1R)-1-[1-benzyl-4-(2,5-difluorphenyl)-1H-imidazol-2-yl]-2,2-dimethylpropyl}(chloracetyl)amino]propyl}carbamat (Intermediat C16) wurden in 2.0 mL DMF vorgelegt und mit 69.1 mg (0.21 mmol) Cäsiumcarbonat und 28.8 mg (0.10 mmol) 1-Sulfanyl-3,6,9,12-tetraoxapentadecan-15-säure versetzt. Es wurde über Nacht bei 50 °C gerührt. Die Reaktionsmischung wurde mit Wasser versetzt und direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 25.1 mg (35% d. Th.) der Titelverbindung.

LC-MS (Methode 1): Rₜ = 1.42 min; MS (ESIpos): m/z = 835 [M+H]⁺.

### Intermediat C18

### tert-Butyl-[22-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-4,21-dioxo-7,10,13,16-tetraoxa-19-thia-3,22-diazapentacosan-25-yl]carbamat

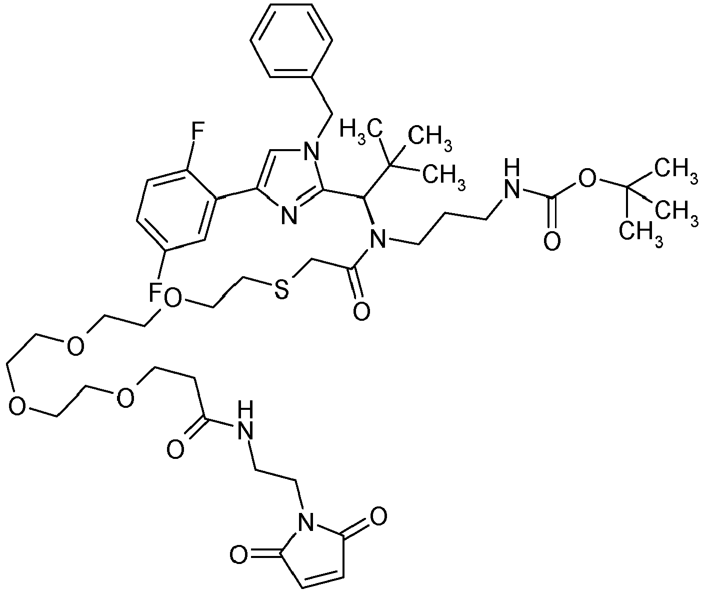

21.0 mg (0.03 mmol) 9-{(1R)-1-[1-Benzyl-4-(2,5-difluorphenyl)-1H-imidazol-2-yl]-2,2-dimethylpropyl}-2,2-dimethyl-4,10-dioxo-3,15,18,21,24-pentaoxa-12-thia-5,9-diazaheptacosan-27-säure (Intermediat C17) und 5.8 mg (0.0.3 mmol) 1-(2-Aminoethyl)-1H-pyrrol-2,5-dionhydrochlorid(1:1) wurden in 1.0 mL Acetonitril vorgelegt und mit 26.1 mg (0.20 mmol) N,N-Diisopropylethylamin und 20.9 mg (0.03 mmol) T3P (50 % in Ethylacetat) versetzt. Es wurde über Nacht bei RT gerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 19.7 mg (79 % d. Th.) der Titelverbindung.

LC-MS (Methode 1): Rₜ = 1.42 min; MS (ESIpos): m/z = 835 [M+H]⁺.

### Intermediat C19

### tert-Butyl-(13-{(1R)-1-[1-benzyl-4-(2,5-difluorphenyl)-1H-imidazol-2-yl]-2,2-dimethylpropyl}-2,2-dimethyl-6,12-dioxo-5-oxa-10-thia-7,13-diaza-2-silahexadecan-16-yl)carbamat

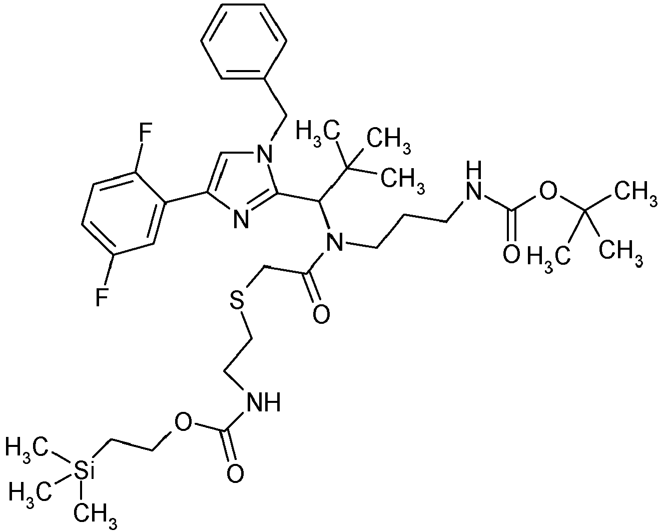

58.5 mg (0.10 mmol) tert-Butyl-{3-[{(1R)-1-[1-benzyl-4-(2,5-difluorphenyl)-1H-imidazol-2-yl]-2,2-dimethylpropyl}(chloracetyl)amino]propyl}carbamat (Intermediat C16) wurden in 2.0 mL DMF vorgelegt und mit 44.0 mg (0.20 mmol) 2-(Trimethylsilyl)ethyl-(2-sulfanylethyl)carbamat (Intermediat L39) und 64.7 mg (0.20 mmol) Cäsiumcarbonat versetzt. Es wurde über 4 h bei 50 °C gerührt. Der Ansatz wurde wiederholt mit 46.6 mg (0.079 mmol) Intermediat C16. Die beiden Reaktionsmischungen wurden vereinigt und direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 98.0 mg (71 % d. Th.) der Titelverbindung.

LC-MS (Methode 1): Rₜ = 1.62 min; MS (ESIpos): m/z = 774 [M+H]⁺.

### Intermediat C20

### Trifluoressigsäure--tert-Butyl-[3-({[(2-aminoethyl)sulfanyl]acetyl}{(1R)-1-[1-benzyl-4-(2,5-difluorphenyl)-1H-imidazol-2-yl]-2,2-dimethylpropyl }amino)propyl]carbamat

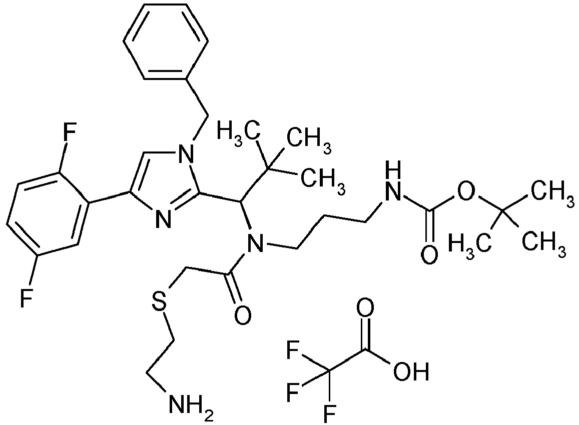

98.0 mg (0.13 mmol) tert-Butyl-(13-{(1R)-1-[1-benzyl-4-(2,5-difluorphenyl)-1H-imidazol-2-yl]-2,2-dimethylpropyl}-2,2-dimethyl-6,12-dioxo-5-oxa-10-thia-7,13-diaza-2-silahexadecan-16-yl)carbamat (Intermediat C19) wurden in 2.0 mL DMF/tert.-Butanol (9:1) vorgelegt und mit 96.2 mg (0.63 mmol) CsF versetzt. Es wurde 16 h bei 90 °C gerührt. Die Reaktionsmischung wurde direkt über die präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand lyophilisiert. Man erhielt 57.1 mg (61 % d. Th.) der Titelverbindung. Die Verbindung enthält auch das korrespondierende Sulfoxid.

LC-MS (Methode 1): Rₜ = 1.08 min; MS (ESIpos): m/z = 630 [M+H]⁺.

### Intermediat C21

### tert-Butyl-[38-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-3,31,37-trioxo-7,10,13,16,19,22,25,28-octaoxa-35-thia-4,32,38-triazahentetracontan-41-yl]carbamat

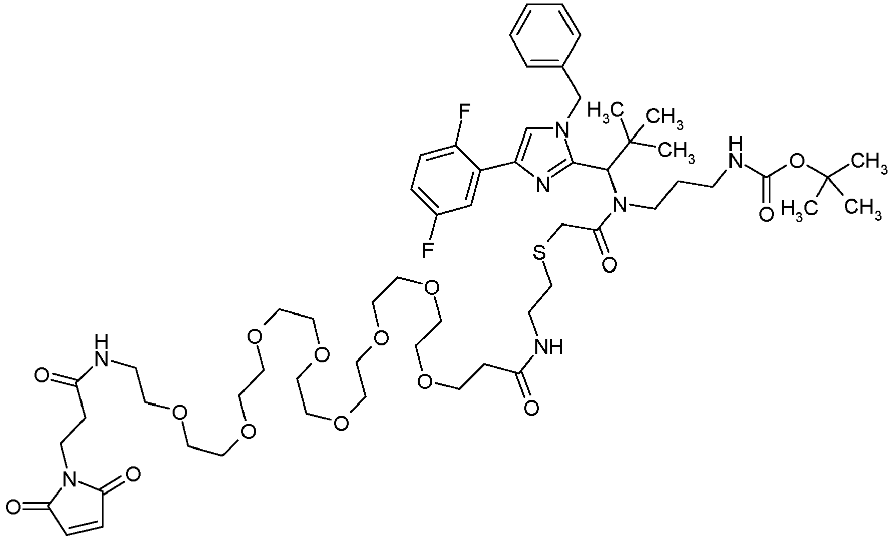

57.1 mg (0.08 mmol) Trifluoressigsäure--tert-Butyl-[3-({[(2-aminoethyl)sulfanyl]acetyl}{(1R)-1-[1-benzyl-4-(2,5-difluorphenyl)-1H-imidazol-2-yl]-2,2-dimethylpropyl}amino)propyl]carbamat (Intermediat C20) wurden in 3.0 mL DMF vorgelegt und mit 53.0 mg (0.08 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid und 15.5 mg (0.15 mmol) Triethylamin versetzt. Es wurde 16 h bei RT gerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand lyophilisiert. Man erhielt 49.7 mg (49 % d. Th.) der Titelverbindung. LC-MS (Methode 1): Rₜ = 1.34 min; MS (ESIpos): m/z = 1204 [M+H]⁺.

### Intermediat C22

### tert-Butyl-[38-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-35-oxido-3,31,37-trioxo-7,10,13,16,19,22,25,28-octaoxa-35lambda4-thia-4,32,38-triazahentetracontan-41-yl]carbamat

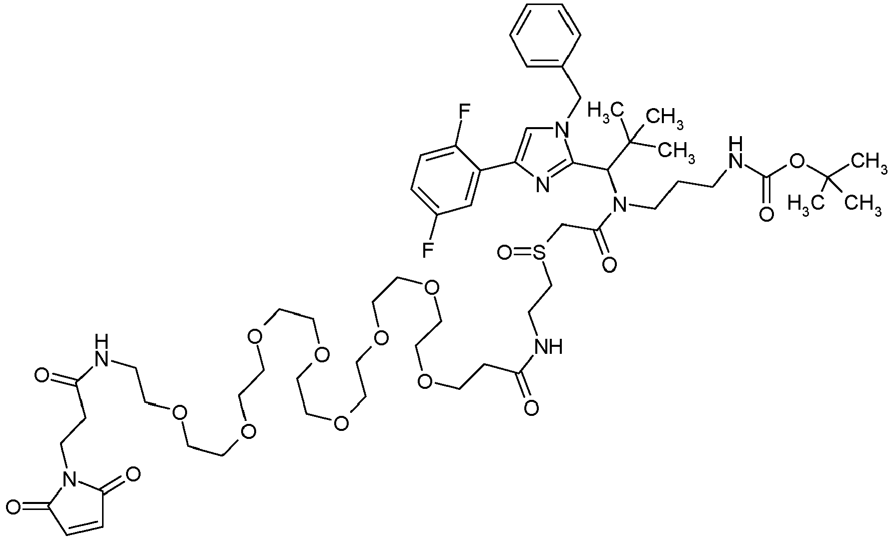

Die Titelverbindung entstand als Nebenprodukt bei der Synthese von Intermediat C21. Man erhielt 15.5 mg (15 % d. Th.) der Titelverbindung.

LC-MS (Methode 1): Rₜ = 1.25 min; MS (ESIpos): m/z = 1220 [M+H]⁺.

### Intermediat C23

### tert-Butyl-3-amino-4-{[{(1R)-1-[1-benzyl-4-(2,5-difluorphenyl)-1H-imidazol-2-yl]-2,2-dimethylpropyl}(glycoloyl)amino]methyl}pyrrolidin-1-carboxylat Mischung aus Stereoisomeren.

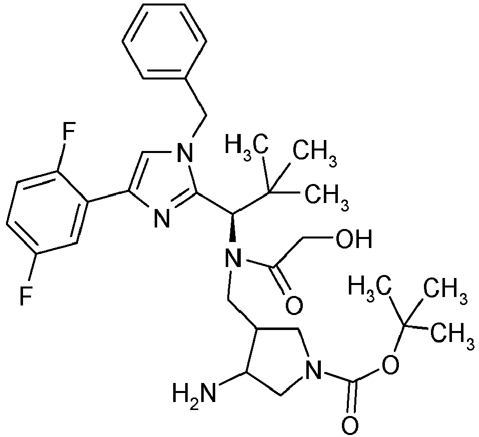

411.2 mg (1.15 mmol) tert-Butyl-3-formyl-4-({[2-(trimethylsilyl)ethoxy]carbonyl}amino)-pyrrolidin-1-carboxylat (Intermediat L28) und 339.7 mg (0.96 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C1) wurden in 6.0 mL Dichlormethan vorgelegt und mit 68.9 mg (1.15 mmol) HOAc versetzt und 1 h bei RT gerührt. Es wurden 405.2 mg (1.91 mmol) Natriumtriacetoxyborhydrid zugegeben und 2 h bei RT gerührt. Das Lösemittel wurde im Vakuum verdampft und der Rückstand mit Ethylacetat und Wasser versetzt. Die wässrige Phase wurde dreimal mit Ethylacetat extrahiert. Die vereinigten organischen Phasen wurden einmal mit ges. NaCl-Lösung gewaschen und über Magnesiumsulfat getrocknet. Das Lösemittel wurde im Vakuum verdampft und der Rückstand mittels Biotage Isolera gereinigt (Kieselgel, Säule 50 g SNAP, Fluss 40 mL/min, Petrolether/Ethylacetat). Die Lösemittel wurden im Vakuum verdampft und der Rückstand im Hochvakuum getrocknet. Man erhielt 541.5 mg (81 % d. Th.) der Verbindung tert-Butyl-3-[({(1R)-1-[1-benzyl-4-(2,5-difluorphenyl)-1H-imidazol-2-yl]-2,2-dimethylpropyl}amino)methyl]-4-({[2-(trimethylsilyl)ethoxy]carbonyl}amino)pyrrolidin-1-carboxylat.

LC-MS (Methode 1): Rₜ = 1.24 und 1.29 min; MS (ESIpos): m/z = 698 [M+H]⁺.

541.5 mg (0.78 mmol) tert-Butyl-3-[({(1R)-1-[1-benzyl-4-(2,5-difluorphenyl)-1H-imidazol-2-yl]-2,2-dimethylpropyl}amino)methyl]-4-({[2-(trimethylsilyl)ethoxy]carbonyl}amino)pyrrolidin-1-carboxylat wurden in 13.0 mL Dichlormethan gelöst und mit 180.6 mg (1.78 mmol) Triethylamin versetzt. Die Reaktionslösung wurde auf 0 °C abgekühlt und mit 233.1 mg (1.71 mmol) Acetoxyacetylchlorid versetzt und 16 h bei RT gerührt. Es wurden nochmals 180.6 mg (1.78 mmol) Triethylamin und 233.1 mg (1.71 mmol) Acetoxyacetylchlorid zugesetzt und bei RT nochmals 80 h gerührt. Das Lösemittel wurde im Vakuum verdampft und der Rückstand zwischen Wasser und Ethylacetat verteilt. Die wässrige Phase wurde dreimal mit Ethylacetat extrahiert. Die vereinigten organischen Phasen wurden einmal mit ges. NaCl-Lösung gewaschen und über Magnesiumsulfat getrocknet. Das Lösemittel wurde im Vakuum verdampft und der Rückstand mittels Biotage Isolera gereinigt (Kieselgel, Säule 50 g SNAP, Fluss 40 mL/min, Petrolether/Ethylacetat). Die Lösemittel wurden im Vakuum verdampft und der Rückstand im Hochvakuum getrocknet. Man erhielt 529.2 mg (86 % d. Th.) der Verbindung tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-({[2-(trimethylsilyl)ethoxy]carbonyl}amino)pyrrolidin-1-carboxylat.

LC-MS (Methode 1): Rₜ = 1.53 und 1.56 min; MS (ESIpos): m/z = 798 [M+H]⁺.

529.2 mg (0.66 mmol) tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-({[2-(trimethylsilyl)ethoxy]carbonyl}amino)pyrrolidin-1-carboxylat wurden in 10.0 mL DMF/tert.-Butanol (9: 1) vorgelegt und mit 503.7 mg (3.32 mmol) CsF versetzt. Dir Reaktionsmischung wurde 16 h bei 90 °C gerührt. Die Reaktionsmischung wurde zwischen Wasser und Ethylacetat verteilt. Die wässrige Phase wurde dreimal mit Ethylacetat extrahiert. Die vereinigten organischen Phasen wurden einmal mit ges. NaCl-Lösung gewaschen und über Magnesiumsulfat getrocknet. Das Lösemittel wurde im Vakuum verdampft und der Rückstand mittels Biotage Isolera gereinigt (Kieselgel, Säule 50 g SNAP, Fluss 25 mL/min, Dichlormethan/Methanol). Die Lösemittel wurden im Vakuum verdampft und der Rückstand im Hochvakuum getrocknet. Man erhielt 172.4 mg (40 % d. Th.) der Verbindung tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-aminopyrrolidin-1-carboxylat.

LC-MS (Methode 1): Rₜ = 1.05 und 1.35 min; MS (ESIpos): m/z = 654 [M+H]⁺.

172.4 mg (0.26 mmol) tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-aminopyrrolidin-1-carboxylat wurden in 4.5 mL Methanol/Wasser (2:1) vorgelegt und mit 80.2 mg (0.58 mmol) Kaliumcarbonat versetzt und 16 h bei RT gerührt. Die Reaktionsmischung wurde zwischen Wasser und Ethylacetat verteilt. Die wässrige Phase wurde dreimal mit Ethylacetat extrahiert. Die vereinigten organischen Phasen wurden einmal mit ges. NaCl-Lösung gewaschen und über Magnesiumsulfat getrocknet. Das Lösemittel wurde im Vakuum verdampft und der Rückstand wurde mittels präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 116.0 mg (72 % d. Th.) der Titelverbindung.

LC-MS (Methode 1): Rₜ = 1.01 min und 1.03 min; MS (ESIpos): m/z = 612 [M+H]⁺.

### Intermediat C24

### Trifluoressigsäure--tert-Butyl-3-(aminomethyl)-4-{[{(1R)-1-[1-benzyl-4-(2,5-difluorphenyl)-1H-imidazol-2-yl]-2,2-dimethylpropyl}(glycoloyl)amino]methyl}pyrrolidin-1-carboxylat(1:1)

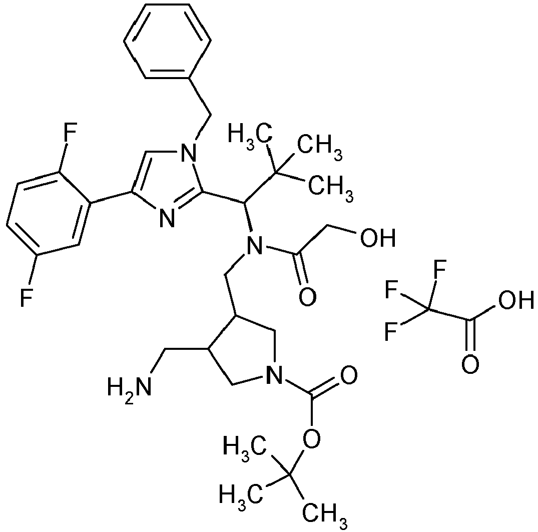

26.8 mg (N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C1) wurden in 3.0 mL Dichlormethan gelöst und mit 5.2 mg (0.09 mmol) HOAc und 22.4 mg (0.11 mmol) Natriumtriacetoxyborhydrid versetzt und 5 min bei RT gerührt. Es wurden 62.4 mg (0.09 mmol) tert-Butyl-3-formyl-4-[({[2-(trimethylsilyl)ethoxy]carbonyl}amino)methyl]pyrrolidin-1-carboxylat (Intermediat L29) zugegeben und über Nacht bei RT gerührt. Das Lösemittel wurde im Vakuum verdampft und der Rückstand wurde mittels präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 57.6 mg (91 % d. Th.) der Verbindung Trifluoressigsäure--tert-Butyl-3-[({(1R)-1-[1-benzyl-4-(2,5-difluorphenyl)-1H-imidazol-2-yl]-2,2-dimethylpropyl}amino)methyl]-4-[({[2-(trimethylsilyl)ethoxy]carbonyl}amino)methyl]pyrrolidin-1-carboxylat.

LC-MS (Methode 1): Rₜ = 1.25 und 1.27 min; MS (ESIpos): m/z = 712 [M+H]⁺.

77.0 mg (0.11 mmol) tert-Butyl-3-[({(1R)-1-[1-benzyl-4-(2,5-difluorphenyl)-1H-imidazol-2-yl]-2,2-dimethylpropyl}amino)methyl]-4-[({ [2-(trimethylsilyl)ethoxy]carbonyl}amino)methyl] pyrrolidin-1-carboxylat wurden in 1.5 mL Dichlormethan vorgelegt und mit 21.9 mg (0.22 mmol) Triethylamin versetzt. Dann wurden bei 0 °C 29.5 mg (0.22 mmol) Acetoxyessigsäurechlorid zugegeben und die Reaktionsmischung wurde über Nacht bei RT gerührt. Das Lösemittel wurde im Vakuum verdampft und der Rückstand in Ethylacetat aufgenommen. Die organische Phase wurde je einmal mit Wasser, ges. Natriumhydrogencarbonat-Lösung und ges. NaCl-Lösung gewaschen. Nach der Trocknung über Magnesiumsulfat wurde das Lösemittel im Vakuum verdampft. Wiederholung des Ansatzes mit 77.0 mg (0.11 mmol) tert-Butyl-3-[({(1R)-1-[1-benzyl-4-(2,5-difluorphenyl)-1H-imidazol-2-yl]-2,2-dimethylpropyl}amino)methyl]-4-[({[2-(trimethylsilyl)ethoxy]carbonyl}amino)methyl]. Die vereinigten Rückstände wurden mittels Kieselgel (Laufmittel: Cyclohexan/Ethylacetat = 2:1) gereinigt. Man erhielt 171.1 mg (85 % d. Th.) der Verbindung tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-[({[2-(trimethylsilyl)ethoxy]carbonyl} amino)methyl]pyrrolidin-1 -carboxylat

LC-MS (Methode 1): Rₜ = 1.56 und 1.57 min; MS (ESIpos): m/z = 812 [M+H]⁺.

30.0 mg (0.04 mmol) tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-[({[2-(trimethylsilyl)ethoxy]carbonyl}amino)methyl]pyrrolidin-1-carboxylat wurden in 0.5 mL TBAF-Lösung (1M in THF) gelöst. Es wurde über Nacht bei RT gerührt. Das Lösemittel wurde im Vakuum verdampft und der Rückstand mittels präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 25.0 mg (92 % d. Th.) der Titelverbindung.

LC-MS (Methode 1): Rₜ = 0.98 min; MS (ESIpos): m/z = 626 [M+H]⁺.

### Intermediat C25

### 4-{[{(1R)-1-[1-Benzyl-4-(2,5-difluorphenyl)-1H-imidazol-2-yl]-2,2-dimethylpropyl}(glycoloyl)amino]methyl}-1-(tert-butoxycarbonyl)pyrrolidin-3-carbonsäure

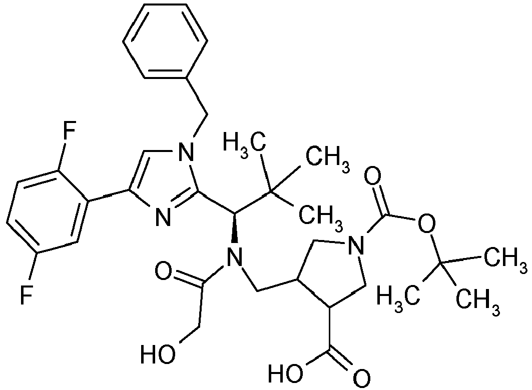

171.4 mg (0.48 mmol) (N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C1) wurden in 4.0 mL Dichlormethan vorgelegt und mit 248.5 mg (0.72 mmol) tert-Butyl-3-({[tert-butyl(dimethyl)silyl]oxy}methyl)-4-formylpyrrolidin-1-carboxylat (Intermediat L30) und 34.8 mg (0.58 mmol) HOAc versetzt. Die Reaktionsmischung wurde 1 h bei RT gerührt. Es wurde 204.4 mg (0.97 mmol) Natriumtriacetoxyborhydrid zugesetzt und 60 h bei RT gerührt. Das Lösemittel wurde im Vakuum entfernt und der Rückstand mittels Biotage Isolera gereinigt (Kieselgel, Säule 25 g SNAP, Fluss 25 mL/min, Petrolether/Ethylacetat). Die Lösemittel wurden im Vakuum verdampft und der Rückstand im Hochvakuum getrocknet. Man erhielt 267.0 mg (77 % d. Th.) der Verbindung tert-Butyl-3-[({(1R)-1-[1-benzyl-4-(2,5-difluorphenyl)-1H-imidazol-2-yl]-2,2-dimethylpropyl}amino)methyl]-4-({[tert-butyl(dimethyl)silyl]oxy}methyl)pyrrolidin-1-carboxylat. LC-MS (Methode 1): Rₜ = 1.49 min; MS (ESIpos): m/z = 683 [M+H]⁺.

267.0 mg (0.39 mmol) tert-Butyl-3-[({(1R)-1-[1-benzyl-4-(2,5-difluorphenyl)-1H-imidazol-2-yl]-2,2-dimethylpropyl}amino)methyl]-4-({[tert-butyl(dimethyl)silyl]oxy}methyl)pyrrolidin-1-carboxylat wurden in 5.0 mL Dichlormethan gelöst und mit 91.0 mg (0.90 mmol) Triethylamin versetzt und auf 0 °C abgekühlt. Es wurden 117.4 mg (0.86 mmol) Acetoxyacetylchlorid zugegeben und 16 h bei RT gerührt. Es wurden nochmals 593.4 mg (5.87 mmol) Triethylamin und 427.0 mg (3.13 mmol) Acetoxyacetylchlorid zugegeben und weitere 10 h bei RT gerührt. Das Lösemittel wurde im Vakuum verdampft und der Rückstand über die präp. RP-HPLC gereinigt (Säule: Reprosil 250x30; 10µ, Fluss: 50 mL/min, MeCN/Wasser). Die Lösemittel wurden dann im Vakuum verdampft und der Rückstand im Hochvakuum getrocknet. Man erhielt 216.3 mg (71 % d. Th.) der Verbindung tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-({[tert-butyl(dimethyl)silyl]oxy}methyl)pyrrolidin-l-carboxylat.

LC-MS (Methode 1): Rₜ = 1.70 und 1.72 min; MS (ESIpos): m/z = 783 [M+H]⁺.

216. 3 mg (0.28 mmol) tert-Butyl-3-{[(acetoxyacetyl){(1R)-1-[1-benzyl-4-(2,5-difluorphenyl)-1H-imidazol-2-yl]-2,2-dimethylpropyl}amino]methyl}-4-({[tert-butyl(dimethyl)silyl]oxy}methyl) pyrrolidin-1-carboxylat wurden in 4.0 mL THF vorgelegt und wurden mit 16.6 mg (0.28 mmol) HOAc und 361.1 mg (1.38 mmol) TBAF-Lösung (1M in THF) versetzt. Die Reaktionslösung wurde 4 h bei RT gerührt. Das Lösemittel wurde im Vakuum verdampft und der Rückstand über die präp. RP-HPLC gereinigt (Säule: Reprosil 250x30; 10µ, Fluss: 50 mL/min, MeCN/Wasser). Die Lösemittel wurden dann im Vakuum verdampft und der Rückstand im Hochvakuum getrocknet. Man erhielt 94.0 mg (51 % d. Th.) der Verbindung tert-Butyl-3-{[(acetoxyacetyl){(1R)-1-[1-benzyl-4-(2,5-difluorphenyl)-1H-imidazol-2-yl]-2,2-dimethylpropyl } amino]methyl} -4-(hydroxymethyl) pyrrolidin-1-carboxylat.

LC-MS (Methode 1): Rₜ = 1.34 min; MS (ESIpos): m/z = 669 [M+H]⁺.

52.0 mg (0.08 mmol) tert-Butyl-3-{[(acetoxyacetyl){(1R)-1-[1-benzyl-4-(2,5-difluorphenyl)-1H-imidazol-2-yl] -2,2-dimethylpropyl} amino]methyl} -4-(hydroxymethyl) pyrrolidin-1-carboxylat wurden in 4.0 mL PBS-Puffer/Acetonitril (1:1) vorgelegt und mit 1.2 mg (0.01 mmol) TEMPO versetzt. Es wurden dann gleichzeitig 14.1 mg (0.16 mmol) Natriumchlorit in 1.0 mL Wasser und 115.8 µL (0.16 mmol) 10%ige Natriumhypochlorit-Lösung zugegeben. Die Reaktionsmischung wurde 16 h bei RT gerührt. Die Reaktionsmischung wurde in eine 10%ige Natriumsulfit-Lösung gegossen und mit Ethylacetat versetzt. Die wässrige Phase wurde dreimal mit Ethylacetat extrahiert und die vereinigten organischen Phasen einmal mit ges. NaCl-Lösung gewaschen und über Magnesiumsulfat getrocknet. Das Lösemittel wurde im Vakuum verdampft und der Rückstand ohne weitere Reinigung in der nächsten Synthesestufe eingesetzt.

LC-MS (Methode 1): Rₜ = 1.34 min; MS (ESIpos): m/z = 683 [M+H]⁺.

103.0 mg (0.15 mmol) 4-{[(Acetoxyacetyl){(1R)-1-[1-benzyl-4-(2,5-difluorphenyl)-1H-imidazol-2-yl]-2,2-dimethylpropyl}amino]methyl}-1-(tert-butoxycarbonyl)pyrrolidin-3-carbonsäurewurden in 4.5 mL Methanol/Wasser (2:1) vorgelegt und mit 45.9 mg (0.33 mmol) Kaliumcarbonat versetzt und 3 h bei RT gerührt. Die Reaktionsmischung wurde zwischen Wasser und Ethylacetat verteilt. Die wässrige Phase wurde dreimal mit Ethylacetat extrahiert und die vereinigten organischen Phasen einmal mit ges. NaCl-Lösung gewaschen und über Magnesiumsulfat getrocknet. Das Lösemittel wurde im Vakuum verdampft und die Titelverbindung ohne weitere Reinigung in der nächsten Synthesestufe eingesetzt.

LC-MS (Methode 1): Rₜ = 1.35 min; MS (ESIpos): m/z = 641 [M+H]⁺.

### Intermediat C26

### tert-Butyl-[3-({(1R)-1-[1-benzyl-4-(2,5-difluorphenyl)-1H-imidazol-2-yl]-2,2-dimethylpropyl}amino)-2-({[tert-butyl(dimethyl)silyl]oxy}methyl)propyl]carbamat

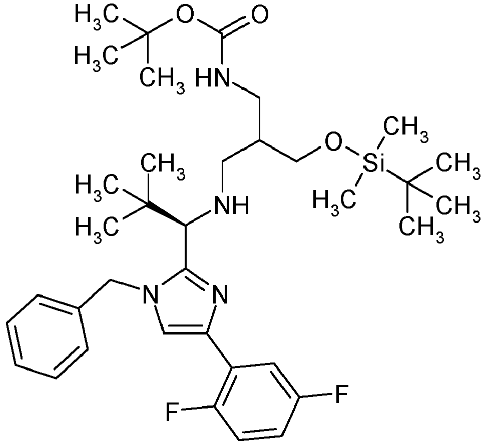

590 mg (1.69 mmol) Natriumtriacetoxyborhydrid sowie 155 µl (2.70 mmol, 162 mg) Essigsäure wurden in 30 mL Dichlormethan vorgelegt und des Gemisch 30 min. bei RT gerührt. Anschließend wurden 600 mg (1.687 mmol) (1R)-1-[1-benzyl-4-(2,5-difluorphenyl)-1H-imidazol-2-yl]-2,2-dimethylpropan-1-amin (welche aus Trifluoressigsäure--(1R)-1-[1-benzyl-4-(2,5-difluorphenyl)-1H-imidazol-2-yl]-2,2-dimethylpropan-1-amin(1:1) durch ausschütteln gegen 1N Natronlauge gewonnen wurde) und 750 mg (2.362 mmol) tert-Butyl-(3-{[tert-butyl(dimethyl)silyl]oxy}-2-formylpropyl)carbamat gelöst in 40 mL Dichlormethan zugetropft. Es wurde 2 h bei RT gerührt. Anschließend wurde Essigsäureethylester addiert, das Gemisch mit gesättigter Natriumcarbonat-Lösung gewaschen und die organische Phase eingeengt. Der Rückstand wurde mittels präparativer HPLC (Eluent: ACN/Wasser, Gradient) getrennt. Es wurden 510 mg (46% d. Th.) der Zielverbindung als Diastereomerengemisch erhalten.

### Isomer 1:

LC-MS (Methode 1): Rₜ = 1.36 min (51%); MS (EIpos): m/z = 657 [M+H]⁺.

### Isomer 2:

LC-MS (Methode 1): Rₜ = 1.41 min (49%); MS (EIpos): m/z = 657 [M+H]⁺.

### Intermediat C27

### 2-({(1R)-1-[1-Benzyl-4-(2,5-difluorphenyl)-1H-imidazol-2-yl]-2,2-dimethylpropyl}{3-[(tert-butoxycarbonyl)amino]-2-({[tert-butyl(dimethyl)silyl]oxy}methyl)propyl}amino)-2-oxoethylacetat

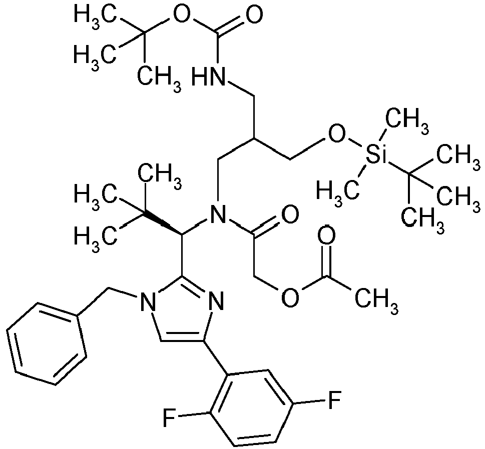

510 mg (0.776 mmol) tert-Butyl-[3-({(1R)-1-[1-benzyl-4-(2,5-difluorphenyl)-1H-imidazol-2-yl]-2,2-dimethylpropyl}amino)-2-({[tert-butyl(dimethyl)silyl]oxy}methyl)propyl]carbamat wurden in 30 mL Dichlormethan vorgelegt und bei RT mit 181 mg (249 µl, 1.786 mmol) Triethylamin sowie mit 219 mg (1.553 mmol) 2-Chlor-2-oxoethylacetat versetzt. Das Reaktionsgemisch wurde bei RT für 2 h gerührt und anschließend mit ges. Natriumhydrogencarbonat-Lösung gewaschen. Die organische Phase wurde über Natriumsulfat getrocknet und am Rotationsverdampfer eingeengt. Der Rückstand wurde mittels präparativer HPLC (Eluent: ACN/Wasser, Gradient) getrennt. Es wurden 290 mg (49% d. Th.) der Zielverbindung als Epimerengemisch erhalten.

### Isomer 1:

LC-MS (METHODE 1): Rₜ = 1.70 min; MS (EIpos): m/z = 757 [M+H]⁺.

### Isomer 2:

LC-MS (Methode 1): Rₜ = 1.72 min; MS (EIpos): m/z = 757 [M+H]⁺.

### Intermediat C28

### 2-({(1R)-1-[1-Benzyl-4-(2,5-difluorphenyl)-1H-imidazol-2-yl]-2,2-dimethylpropyl}{3-[(tert-butoxycarbonyl)amino]-2-(hydroxymethyl)propyl}amino)-2-oxoethylacetat

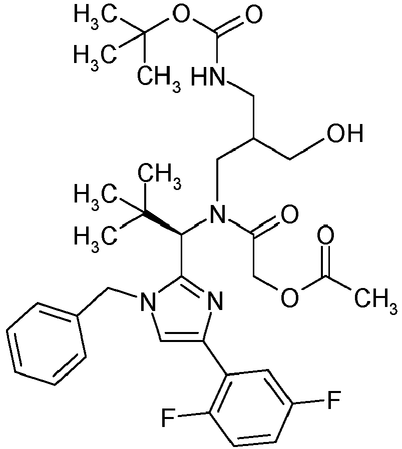

285 mg (0.376 mmol) 2-({(1R)-1-[1-Benzyl-4-(2,5-difluorphenyl)-1H-imidazol-2-yl]-2,2-dimethylpropyl}{3-[(tert-butoxycarbonyl)amino]-2-({[tert-butyl(dimethyl)silyl]oxy}methyl)propyl}amino)-2-oxoethylacetat wurden in 5 mL THF gelöst. Es wurden 452 µl (0.452 mmol) einer 1 M Lösung von Tetra-n-butylammoniumfluorid in THF addiert und das Reaktionsgemisch 3 h bei RT gerührt. Das Reaktionsgemisch wurde mittels präparativer HPLC (Eluent: ACN/Wasser, Gradient) getrennt und lyophilisiert. Es wurden 214 mg (81% d. Th., LC/MS-Reinheit = 92%) der Zielverbindung als Epimerengemisch erhalten.

### Isomer 1:

LC-MS (Methode 1): Rₜ = 1.37 min; MS (EIpos): m/z = 643 [M+H]⁺.

### Isomer 2:

LC-MS (METHODE 1): Rₜ = 1.40 min; MS (EIpos): m/z = 643 [M+H]⁺.

### Intermediat C29

### 2-([3-(Acetylsulfanyl)-2-{[(tert-butoxycarbonyl)amino]methyl}propyl]{(1R)-1-[1-benzyl-4-(2,5-difluorphenyl)-1H-imidazol-2-yl]-2,2-dimethylpropyl }amino)-2-oxoethylacetat

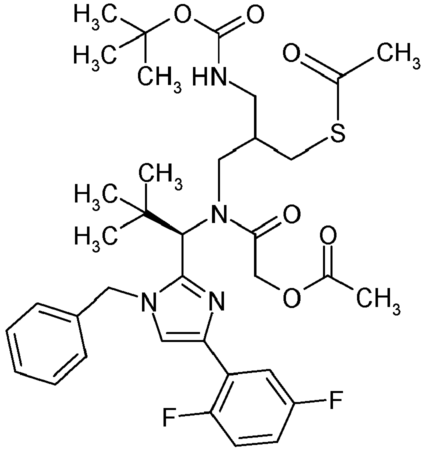

210 mg (0.301 mmol) 2-({(1R)-1-[1-Benzyl-4-(2,5-difluorphenyl)-1H-imidazol-2-yl]-2,2-dimethylpropyl} { 3 -[(tert-butoxycarbonyl)amino] -2-(hydroxymethyl)propyl} amino)-2-oxoethyl-acetat wurden in 8 mL absolutem THF vorgelegt, bei RT mit 178 mg (1.503 mmol, 109 µl) Thionylchlorid gelöst in 8 mL absolutem THF tropfenweise versetzt und 40 Min. Bei RT gerührt. Das Reaktionsgemisch wurde am Rotationsverdampfer eingeengt und im Hochvakuum getrocknet. Der Rückstand wurde in 16 mL absolutem DMF aufgenommen, mit 172 mg (1.503 mmol) Kaliumthioacetat sowei mit 133 mg (0.361 mmol) Tetra-n-butylammoniumiodid versetzt und 2 h bei 90°C gerührt. Nach dem Abkühlen wurde Wasser zugesetzt und das Gemisch mit Essigsäureethylester extrahiert. Die organische Phase wurde am Rotationsverdampfer eingeengt und der Rückstand mittels präparativer HPLC (Eluent: ACN/Wasser, Gradient) getrennt und lyophilisiert. Es wurden 155 mg (69% d. Th., LC/MS-Reinheit = 94%) der Zielverbindung als Epimerengemisch erhalten.

### Isomer 1:

LC-MS (METHODE 1): Rₜ = 1.50 min; MS (EIpos): m/z = 701 [M+H]⁺.

### Isomer 2:

LC-MS (METHODE 1): Rₜ = 1.51 min; MS (EIpos): m/z = 701 [M+H]⁺.

### Intermediat C30

### Di-tert-butyl-[disulfanediylbis(2-{[{(1R)-1-[1-benzyl-4-(2,5-difluorphenyl)-1H-imidazol-2-yl]-2,2-dimethylpropyl}(glycoloyl)amino]methyl}propan-3,1-diyl)]biscarbamat

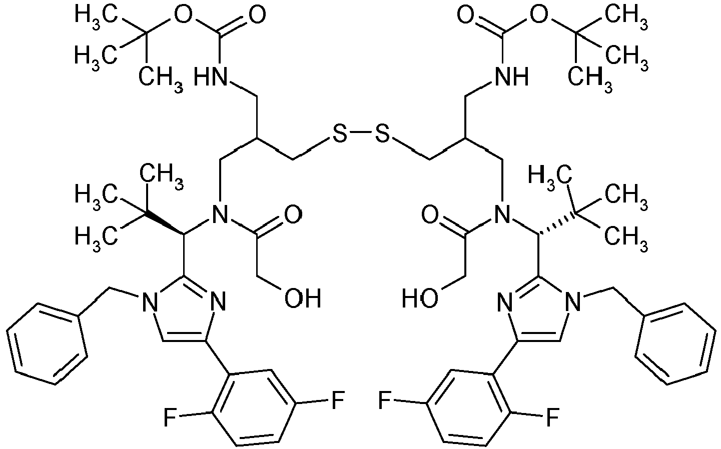

1.220 g (1.010 mmol, LC/MS Reinheit = 58%) 2-([3-(Acetylsulfanyl)-2-{[(tert-butoxycarbonyl)amino]methyl}propyl]{(1R)-1-[1-benzyl-4-(2,5-difluorphenyl)-1H-imidazol-2-yl]-2,2-dimethylpropyl}amino)-2-oxoethylacetat wurden in 30 mL THF und 30 mL Methanol vorgelegt, mit 10 mL einer 1 N Natronlauge versetzt und 2 h bei RT gerührt. Die Reaktionsmischung wurde mit Wasser versetzt und mit Dichlormethan extrahiert. Die organische Phase wurde über Natriumsulfat getrocknet und am Rotationsverdampfer eingeengt. Der Rückstand wurde mittels präparativer HPLC (Eluent: ACN/Wasser, Gradient) gereinigt. Es wurden 390 mg (54% d. Th., LC/MS-Reinheit = 86%) der Zielverbindung als Diastereomerengemisch erhalten.

### Isomere:

LC-MS (METHODE 1): Rₜ = 1.81 min; MS (EIpos): m/z = 1232 [M+H]⁺.

### Intermediat C31

### tert-Butyl-{3-[{(1R)-1-[1-benzyl-4-(2,5-difluorphenyl)-1H-imidazol-2-yl]-2,2-dimethylpropyl} (glycoloyl)amino] -2-(sulfanylmethyl)propyl }carbamat

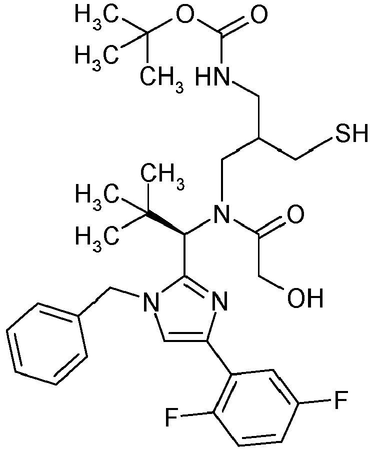

390 mg (0.272 mmol, LC/MS Reiheit = 86%) Di-tert-butyl-[disulfanediylbis(2-{[{(1R)-1-[1-benzyl-4-(2,5-difluorphenyl)-1H-imidazol-2-yl]-2,2-dimethylpropyl}(glycoloyl)amino]methyl}-propan-3,1-diyl)]biscarbamat wurden in 20 mL 1,4-Dioxansowie 10 mL PBS-Puffer aufgenommen und mit 234 mg (0.817 mmol) 3,3',3"-Phosphantriyltripropansäurehydrochlorid(1:1) versetzt. Es wurde 16 h bei RT gerührt. Anschließend wurde die Reaktionsmischung am Rotationsverdampfer eingeengt, mit Dichlormethan ausgerührt, das Filtrat eingeengt und im Hochvakuum getrocknet. Der Rückstand wurde in 8 mL iso-Propanol gelöst und an chiraler Chromatogrphie (Säule: 250x30 mm gefüllt mit Daicel Chiralpak AZ-H, Eluent: iso-Hexan/iso-Propanol = 90:10) gereinigt. Es wurden zwei Fraktionen der Zielverbindung erhalten erhalten. Fraktion 1 enthielt 181.2 mg (50% d.Th.) des Isomers 1 und Fraktion 2 ergab 90.2 mg (25% d. Th.) des Isomers 2.

### Isomer 1:

Chirale HPLC (Säule: 250x4.6 mm, gefüllt mit Diacel Chiralpak AZ-H, Eluent: iso-Hexan/ Ethanol 90:10): Rₜ = 6.98 min.

LC-MS (METHODE 1): Rₜ = 1.47 min; MS (EIpos): m/z = 617 [M+H]⁺.

### Isomer 2:

Chirale HPLC (Säule: 250x4.6 mm, gefüllt mit Diacel Chiralpak AZ-H, Eluent: iso-Hexan/ Ethanol 90:10): Rₜ = 9.39 min.

LC-MS (METHODE 1): Rₜ = 1.47 min; MS (EIpos): m/z = 617 [M+H]⁺.

### Intermediat C32

### N-[3-Amino-2-(sulfanylmethyl)propyl]-N-{(1R)-1-[1-benzyl-4-(2,5-difluorphenyl)-1H-imidazol-2-yl]-2,2-dimethylpropyl}-2-hydroxyacetamidhydrochlorid(1:1) (Isomer1)

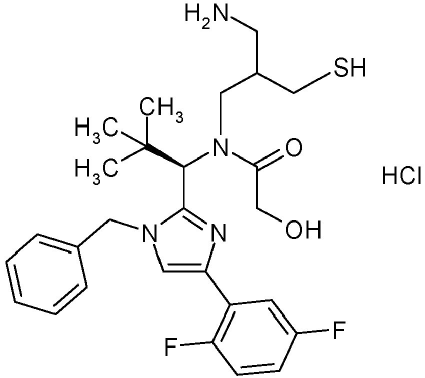

123 mg (199.42 µmol) tert-Butyl-{3-[{(1R)-1-[1-benzyl-4-(2,5-difluorphenyl)-1H-imidazol-2-yl]-2,2-dimethylpropyl}(glycoloyl)amino]-2-(sulfanylmethyl)propyl}carbamat (Isomer 1) wurden in 2 mL THF gelöst und mit 10 mL halbkonzentrierter Salzsäure 1 h lang bei RT gerührt. Die Reaktionslösung wurde unter Argon entgast und anschließend lyophilisiert. Es wurden 108 mg (98% d. Th.) der Zielverbindung enthalten.

### Isomer 1

LC-MS (METHODE 1): Rₜ = 0.95 min; MS (EIpos): m/z = 517 [M+H]⁺.

### Intermediat C33

### N-[3-Amino-2-(sulfanylmethyl)propyl]-N-{(1R)-1-[1-benzyl-4-(2,5-difluorphenyl)-1H-imidazol-2-yl]-2,2-dimethylpropyl}-2-hydroxyacetamidhydrochlorid(1:1) (Isomer2)

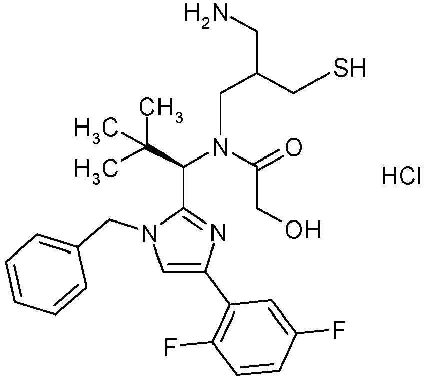

123 mg (199.42 µmol) tert-Butyl-{3-[{(1R)-1-[1-benzyl-4-(2,5-difluorphenyl)-1H-imidazol-2-yl]-2,2-dimethylpropyl}(glycoloyl)amino]-2-(sulfanylmethyl)propyl}carbamat (Isomer 2) wurden in 2 mL THF gelöst und mit 10 mL halbkonzentrierter Salzsäure 1 h lang bei RT gerührt. Die Reaktionslösung wurde unter Argon entgast und anschließend lyophilisiert. Es wurden 58 mg (63% d. Th., LC/MS-Reinheit = 91%) der Zielverbindung enthalten.

### Isomer 2

LC-MS (METHODE 1): Rₜ = 0.97 min; MS (EIpos): m/z = 517 [M+H]⁺.

### Intermediat C34

### tert-Butyl-[3-({(1R)-1-[1-benzyl-4-(2,5-difluorphenyl)-1H-imidazol-2-yl]-2,2-dimethylpropyl} amino)propyl]carbamat

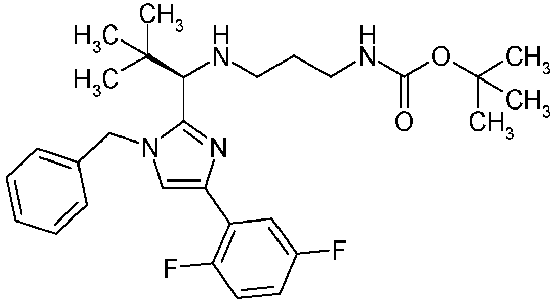

3.790 g (10.02 mmol) (1R)-1-[1-benzyl-4-(2,5-difluorphenyl)-1H-imidazol-2-yl]-2,2-dimethylpropan-1-amin (welche aus Trifluoressigsäure--(1R)-1-[1-benzyl-4-(2,5-difluorphenyl)-1H-imidazol-2-yl]-2,2-dimethylpropan-1-amin(1:1) durch ausschütteln gegen 1N Natronlauge gewonnen wurde) und 3.186 g (15.04 mmol) Natriumtriacetoxyborhydrid sowie 690 µl (12.03 mmol, 722 mg) wurden in 100 mL Dichlormethan vorgelegt. Es wurde 5 Min. bei RT gerührt. Anschließend wurden 4.687 g (27.06 mmol) tert-Butyl-(3-oxopropyl)carbamat zugegeben und für 16 h bei RT gerührt. Das Reaktionsgemisch wurde mit Dichlormethan verdünnt und mit gesättigter Natriumhydrogencarbonat-Lösung gewaschen. Die organische Phase wurde über Natriumsulfat getrocknet und am Rotationsverdampfer eingeengt. Der Rückstand wurde durch Chromatographie an Kieselgel (Eluent: Dichlormethan/Essigsäureethylester, Gradient = 4:1 → 1:1) gereinigt. Es wurden 2.57 g (48% d. Th., LC/MS-Reinheit = 96%) der Zeilverbindung erhalten.

LC-MS (Methode 1): Rₜ = 1.00 min; MS (EIpos): m/z = 513 [M+H]⁺.

### Intermediat C35

### tert-Butyl-{3-[{(1R)-1-[1-benzyl-4-(2,5-difluorphenyl)-1H-imidazol-2-yl]-2,2-dimethylpropyl}(4-nitrobenzoyl)amino]propyl}carbamat

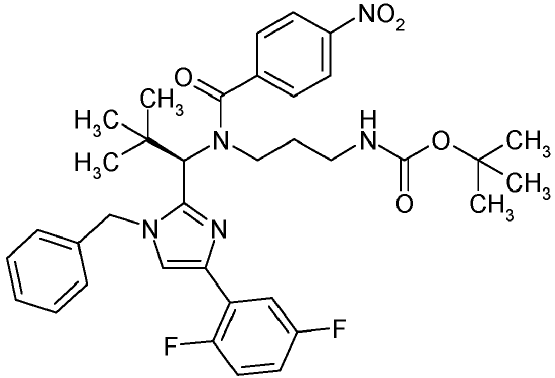

200 mg (0.38 mmol) tert-Butyl-[3-({(1R)-1-[1-benzyl-4-(2,5-difluorphenyl)-1H-imidazol-2-yl]-2,2-dimethylpropyl}amino)propyl]carbamat wurden in 9 mL absolutem Dichlormethan vorgelegt und bei RT mit 120 µl (0.86 mmol, 87 mg) Triethylamin versetzt. Bei RT wurden 83 mg (0.45 mmol) 4-Nitrobenzoylchlorid gelöst in 1 mL absolutem Dichlormethan tropfenweise addiert und 1 h bei RT gerührt. Es wurde Wasser addiert und das Gemisch am Rotationsverdampfer eingeengt. Der Rückstand wurde mittels präparativer HPLC (Eluent: ACN/Wasser + 0.1% TFA, Gradient) gereinigt und geterocknet. Es wurden 181 mg (73% d. Th.) der Zielverbindung erhalten.

LC-MS (Methode 1): Rₜ = 1.47 min; MS (EIpos): m/z = 662 [M+H]⁺.

### Intermediat C36

### tert-Butyl-{3-[(4-aminobenzoyl) {(1R)-1-[1-benzyl-4-(2,5-difluorphenyl)-1H-imidazol-2-yl]-2,2-dimethylpropyl}amino]propyl}carbamat

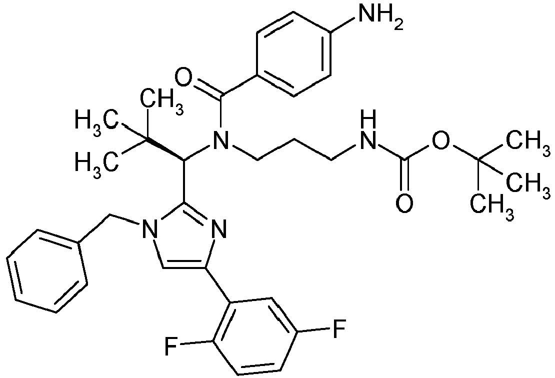

170 mg (0.26 mmol) tert-Butyl-{3-[{(1R)-1-[1-benzyl-4-(2,5-difluorphenyl)-1H-imidazol-2-yl]-2,2-dimethylpropyl}(4-nitrobenzoyl)amino]propyl}carbamat wurden in 10 mL Essigsäure vorgelegt. Es wurden 143 mg (2.57 mmol) Eisenpulver addiert und 16 h bei 50°C gerührt. Nach dem Abkühlen wurde Wasser addiert und das Gemisch mit Essigsäureethylester extrahiert. Die organische Phse wurde über Natriumsulfat getrocknet und am Rotationsverdampfer eingeengt. Der Rückstand wurde im HV getrocknet. Es wurden 154 mg (77% d. Th., LC/MS-Reinheit = 82 %) der Zielverbindung erhalten.

LC-MS (Methode 5): Rₜ = 4.73 min; MS (EIpos): m/z = 632 [M+H]⁺.

### Intermediat C37

### N-[19-(2,5-Dioxo-2,5-dihydro-1H-pyrrol-1-yl)-17-oxo-4,7,10,13-tetraoxa-16-azanonadecan-1-oyl]-L-valyl-N-[4-({(1R)-1-[1-benzyl-4-(2,5-difluorphenyl)-1H-imidazol-2-yl]-2,2-dimethylpropyl} { 3 -[(tert-butoxycarbonyl)amino]propyl} carbamoyl)phenyl] -L-alaninamid

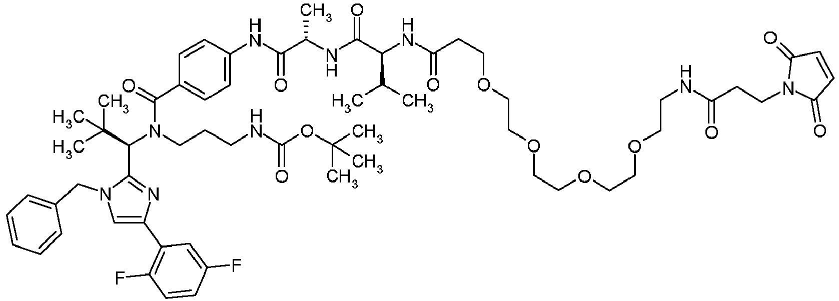

38.6 mg (0.05 mmol, LC/MS Reinheit = 82%) tert-Butyl-{3-[(4-aminobenzoyl){(1R)-1-[1-benzyl-4-(2,5-difluorphenyl)-1H-imidazol-2-yl]-2,2-dimethylpropyl}amino]propyl}carbamat wurden in absolutem DMF gelöst und mit 24.8 mg (0.06 mmol) HATU sowie mit 13.0 mg (0.10 mmol) N,N-Diisopropylethylamin versetzt. Das Gemisch wurde 5 Min. bei RT gerührt, anschließend mit 63 mg (0.06 mmol) N-[19-(2,5-Dioxo-2,5-dihydro-1H-pyrrol-1-yl)-17-oxo-4,7,10,13-tetraoxa-16-azanonadecan-1-oyl]-L-valyl-L-alanin versetzt und 3 h bei RT gerührt. Es wurden 7.5 mg (0.06 mmol) 3H-[1,2,3]triazolo[4,5-b]pyridin-3-ol (HOAt) addiert und 16 h gerührt. Es wurden 19.1 mg (0.05 mmol) HATU addiert und 2 h bei 50°C gerührt. Nach dem Abkühlen wurde das Reaktionsgemisch direkt mittels präparativer HPLC (Eluent: ACN/Wasser + 0.1% TFA, Gradient) gereinigt. Es wurden 6.5 mg (9% d. Th., LC/MS-Reinheit = 83%) der Zielverbindung erhalten.

LC-MS (Methode 2): Rₜ = 7.89 min; MS (EIpos): m/z = 1200.6 [M+H]⁺.

### Intermediat C38

### 2-[3-({(1R)-1-[1-Benzyl-4-(2,5-difluorphenyl)-1H-imidazol-2-yl]-2,2-dimethylpropyl}amino)propyl]-1H-isoindol-1,3(2H)-dion

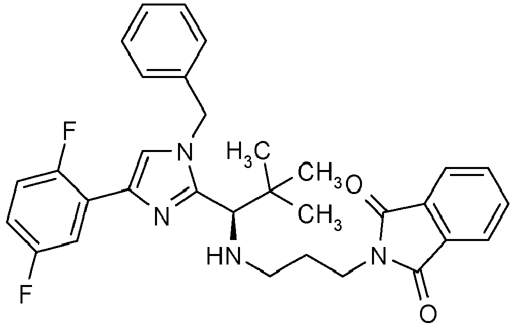

300.0 mg (0.84 mmol) 2-[3-({(1R)-1-[1-Benzyl-4-(2,5-difluorphenyl)-1H-imidazol-2-yl]-2,2-dimethylpropyl}amino)propyl]-1H-isoindol-1,3(2H)-dion (Intermediat C1) wurden in 4.0 mL Dichlormethan vorgelegt und mit 58.3 mg (0.97 mmol) HOAc und 250.4 mg (1.18 mmol) Natriumtriacetoxyborhydrid versetzt und 5 min bei RT gerührt. Es wurden 197.2 mg (0.97 mmol) 3-(1,3-Dioxo-1,3-dihydro-2H-isoindol-2-yl)propanal zugegeben. Die Reaktionsmischung wurde über Nacht bei RT gerührt. Die Reaktionsmischung wurde mit Ethylacetat verdünnt und die organische Phase zweimal mit ges. Natriumcarbonat-Lösung und einmal mit ges. NaCl-Lösung gewaschen. Nach der Trocknung über Magnesiumsulfat wurde das Lösemittel im Vakuum verdampft und der Rückstand mittels Kieselgel (Laufinittel: Ethylacetat/Cyclohexan 1:5) gereinigt. Die Lösemittel wurden im Vakuum verdampft und der Rückstand im Hochvakuum getrocknet. Man erhielt 333.3 mg (70 %) der Titelverbindung.

LC-MS (Methode 1): Rₜ = 1.05 min; MS (ESIpos): m/z = 543 [M+H]⁺.

### Intermediat C39

### 2-({(1R)-1-[1-Benzyl-4-(2,5-difluorphenyl)-1H-imidazol-2-yl]-2,2-dimethylpropyl}[3-(1,3-dioxo-1,3-dihydro-2H-isoindol-2-yl)propyl]amino)-2-oxoethylacetat

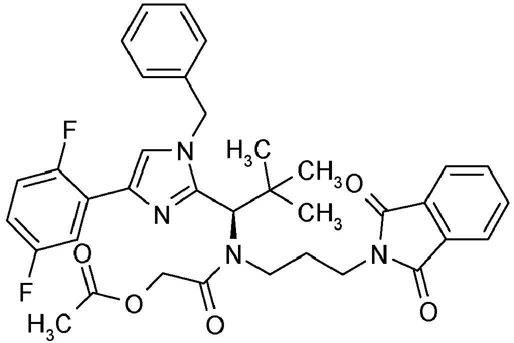

332.3 mg (0.61mmol) 2-[3-({(1R)-1-[1-Benzyl-4-(2,5-difluorphenyl)-1H-imidazol-2-yl]-2,2-dimethylpropyl}amino)propyl]-1H-isoindol-l,3(2H)-dion (Intermediat C38) wurden in 8.0 mL Dichlormethan vorgelegt und mit 142.5 mg (1.35 mmol) Triethylamin versetzt. Bei 0 °C wurden 184.0 mg (1.35 mmol) Acetoxyessigsäurechlorid zugegeben und die Reaktionsmischung wurde über Nacht bei RT gerührt. Die Reaktionsmischung wurde mit Ethylacetat verdünnt und die organische Phase zweimal mit ges. Natriumhydrogencarbonat-Lösung und einmal mit ges. NaCl-Lösung gewaschen. Nach der Trocknung über Magnesiumsulfat wurde das Lösemittel im Vakuum verdampft und der Rückstand wurde mittels Kieselgel (Laufinittel: Ethylacetat/Cyclohexan 1:3) gereinigt. Die Lösemittel wurden im Vakuum verdampft und der Rückstand im Hochvakuum getrocknet. Man erhielt 367.1 mg (63 %) der Titelverbindung.

LC-MS (Methode 1): Rₜ = 1.42 min; MS (ESIpos): m/z = 643 [M+H]⁺.

### Intermediat C40

### N-(3-Aminopropyl)-N-{(1R)-1-[1-benzyl-4-(2,5-difluorphenyl)-1H-imidazol-2-yl]-2,2-dimethylpropyl} -2-hydroxyacetamid

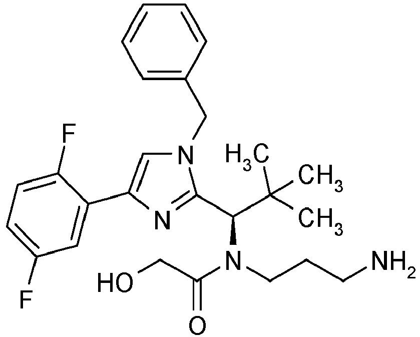

583.1 mg (0.91 mmol) 2-({(1R)-1-[1-Benzyl-4-(2,5-difluorphenyl)-1H-imidazol-2-yl]-2,2-dimethylpropyl}[3-(1,3-dioxo-1,3-dihydro-2H-isoindol-2-yl)propyl]amino)-2-oxoethylacetat (Intermediat C39) wurden in 15.0 mL Ethanol vorgelegt und mit 1.41 g (18.15 mmol) Methanamin (40 % in Wasser) versetzt. Die Reaktionsmischung wurde über Nacht bei 50 °C gerührt. Das Lösemittel wurde im Vakuum verdampft und der Rückstand dreimal mit Toluol kodestilliert. Der Rückstand wurde mittels Kieselgel (Laufinittel: Dichlormethan/Methanol = 100:5) gereinigt. Die Lösemittel wurden im Vakuum verdampft und der Rückstand im Hochvakuum getrocknet. Man erhielt 324.9 mg (73 %) der Titelverbindung.

LC-MS (Methode 1): Rₜ = 0.97 min; MS (ESIpos): m/z = 471 [M+H]⁺.

### Intermediat C41

### Trifluoressigsäure--L-valyl-N-{3-[{(1R)-1-[1-benzyl-4-(2,5-difluorphenyl)-1H-imidazol-2-yl]-2,2-dimethylpropyl}(glycoloyl)amino]propyl}-L-alaninamid(1:1)

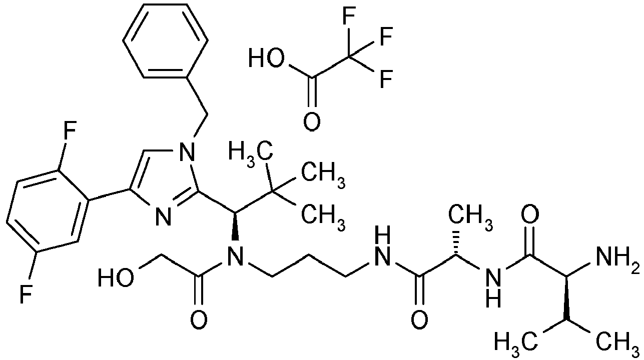

50.0 mg (0.11 mol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40) und 30.4 mg (0.11 mmol) 2,5-Dioxopyrrolidin-1-yl-N-(tert-butoxycarbonyl)-L-alaninat wurden in 2.0 mL DMF vorgelegt und mit 32.2 mg (0.32 mmol) 4-Methylmorpholin versetzt. Die Reaktionsmischung wurde über Nacht bei RT gerührt. 19.1 mg (0.32 mmol) HOAc wurden zugegeben und die Reaktionsmischung direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 38.0 mg (56 %) der Verbindung tert-Butyl-[(2S)-1-({3-[{(1R)-1-[1-benzyl-4-(2,5-difluorphenyl)-1H-imidazol-2-yl]-2,2-dimethylpropyl}(glycoloyl)amino]propyl}amino)-1-oxopropan-2-yl]carbamat.

LC-MS (Methode 1): Rₜ = 1.26 min; MS (ESIpos): m/z = 642 [M+H]⁺.

33.6 mg (0.05 mmol) tert-Butyl-[(2S)-1-({3-[{(1R)-1-[1-benzyl-4-(2,5-difluorphenyl)-1H-imidazol-2-yl] -2,2-dimethylpropyl}(glycoloyl)amino]propyl}amino)-1-oxopropan-2-yl]carbamat wurden in 3.0 mL Dichlormethan vorgelegt. Die Reaktionsmischung wurde mit 119.4 mg (1.05 mmol) TFA versetzt und über Nacht bei RT gerührt. Das Lösemittel wurde im Vakuum verdampft und der Rückstand direkt mittles präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 32.8 mg (96 %) der Verbindung Trifluoressigsäure--N-{3-[{(1R)-1-[1-benzyl-4-(2,5-difluorphenyl)-1H-imidazol-2-yl]-2,2-dimethylpropyl}(glycoloyl)amino]propyl}-L-alaninamid(1:1).

LC-MS (Methode 1): Rₜ = 0.93 min; MS (ESIpos): m/z = 542 [M+H]⁺.

29.5 mg (0.05 mmol) Trifluoressigsäure--N-{3-[{(1R)-1-[1-benzyl-4-(2,5-difluorphenyl)-1H-imidazol-2-yl]-2,2-dimethylpropyl}(glycoloyl)amino]propyl}-L-alaninamid(1:1) und 14.1 mg (0.05 mmol) 2,5-Dioxopyrrolidin-1-yl-N-(tert-butoxycarbonyl)-L-valinat wurden in 1.0 mL DMF vorgelegt und mit 18.2 mg (0.18 mmol) 4-Methylmorpholin versetzt. Die Reaktionsmischung rührte über Nacht bei RT und wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 23.1 mg (69 %) der Verbindung N-(tert-Butoxycarbonyl)-L-valyl-N-{3-[{(1R)-1-[1-benzyl-4-(2,5-difluorphenyl)-1H-imidazol-2-yl]-2,2-dimethylpropyl} (glycoloyl)amino]propyl} -L-alaninamid.

LC-MS (Methode 1): Rₜ = 1.30 min; MS (ESIpos): m/z = 741 [M+H]⁺.

19.4 mg (0.03 mmol) N-(tert-Butoxycarbonyl)-L-valyl-N-{3-[{(1R)-1-[1-benzyl-4-(2,5-difluorphenyl)-1H-imidazol-2-yl]-2,2-dimethylpropyl}(glycoloyl)amino]propyl}-L-alaninamid wurden in 1.5 mL Dichlormethan gelöst und mit 59.7 mg (0.52 mmol) TFA versetzt. Die Reaktionsmischung rührte über Nacht bei RT. Es wurden 119.4 mg (1.04 mmol) TFA zugegeben und nochmals über Nacht bei RT gerührt. Das Lösemittel wurde im Vakuum verdampft und der Rückstand direkt mittles präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 19.2 mg (97 %) der Titelverbindung.

LC-MS (Methode 1): Rₜ = 0.96 min; MS (ESIpos): m/z = 641 [M+H]⁺.

### Intermediat C42

### 2,5-Difluorbenzoldiazoniumtetrafluoroborat

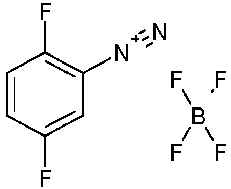

Es wurden 3.00 g (21.16 mmol, 2.68 ml) Bortrifluorid-Diethylether-Komplex vorgelegt und 1.37 g (10.58 mmol) 2,5-Difluoranilin gelöst in 27 ml absolutem THF bei 0°C langsam zu getropft. Bei - -10°C wurde eine Lösung von 1.61 g (13.75 mmol, 1.85 ml) Isoamylnitrit gelöst in 3 ml absolutem THF zu getropft und 30 Min. bei gleicher Temperatur weiter gerührt. Es wurden 15 ml Diethylether addiert, das ausgefallene Diazoniumsalz abfiltriert, mit etwas Diethylether gewaschen und im Hochvakuum getrocknet. Es wurden 2.27 g der Zielverbindung (94% d.Th.) erhalten.

LC-MS (Methode 6): Rₜ = 0.24 min; MS (ESIpos): m/z = 141 [M]⁺.

¹H-NMR (400 MHz, DMSO-d₆): δ [ppm] = 8.11-8.17 (m, 1H), 8.36-8.43 (m, 1H), 8.69-8.73 (m, 1H).

### Intermediat C43

### Methyl-chlor[2-(2,5-difluorphenyl)hydrazinyliden]acetat

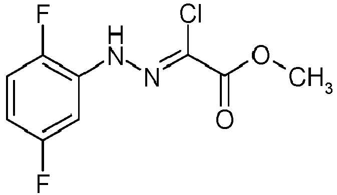

Unter Argonatmospähre wurden 3.63 g (24.13 mmol) Methyl-2-chlor-3-oxobutanoat in 100 ml Wasser vorgelegt und bei -5°C mit 48.90 g (618.19 mmol, 50.00 ml) Pyridin versetzt und 10 Min. bei dieser Temperatur gerührt. Anschließend wurden bei -5°C 5.00 g (21.94 mmol) 2,5-Difluorbenzoldiazoniumtetrafluoroborat zugegeben, wobei sich eine orangefarbene Suspension bildete. Es wurde 30 Min. bei dieser Temperatur gerührt, der Ansatz mit Wasser verdünnt und dreimal mit Dichlormethan extrahiert. Die vereinigten organischen Phasen wurden mit gesättigter Natriumchlorid-Lösung gewaschen, über Natriumsulfat getrocknet, am Rotationsverdampfer eingeengt und im Hochvakuum getrocknet. Es wurden 5.52 g der Zielverbindung (97% d. Th., LC/MS-Reinheit = 96%) erhalten.

LC-MS (Methode 1): Rₜ = 1.03 min; MS (ESIpos): m/z = 249 [M+H]⁺.

¹H-NMR (400 MHz, DMSO-d₆): δ [ppm] = 3.85 (s, 3H), 6.88-6.94 (m, 1H), 7.16-7.21 (m, 1H), 7.31-7.37 (m, 1H), 10.00 (s, 1H).

### Intermediat C44

### Methyl-4-benzoyl-1-(2,5-difluorphenyl)-1H-pyrazol-3-carboxylat

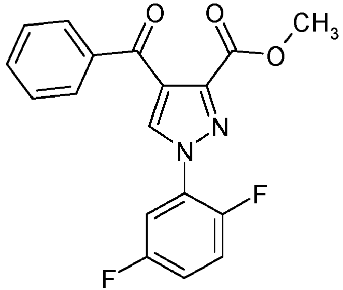

3.50 g (13.52 mmol) Methyl-chlor[2-(2,5-difluorphenyl)hydrazinyliden]acetat (LC/MS-Reinheit 96%) wurden in 9 ml absolutem Toluol gelöst, mit 2.61 g (14.87 mmol) (2E)-3-(Dimethylamino)-1-phenylprop-2-en-1-on sowie 3.01 g (29.73 mmol), 4.14 ml) Triethylamin versetzt und 16 h bei Raumtemperatur gerührt. Das Reaktionsgemisch wurde am Rotationsverdampfer eingeengt und der Rückstand mittels präparativer HPLC (Eluent: ACN/Wasser mit 0.1% Ameisensäure, Gradient) getrennt. Es wurden 1.79 g (39% d. Th.) der Zielverbindung erhalten.

LC-MS (Methode 1): Rₜ = 1.07 min; MS (ESIpos): m/z = 343 [M+H]⁺.

¹H-NMR (400 MHz, DMSO-d₆): δ [ppm] = 3.86 (s, 3H), 7.44-7.50 (m, 1H), 7.55-7.72 (m, 4H), 7.81-7.87 (m, 3H), 8.80 (d, 1H).

### Intermediat C45

### [4-Benzyl-1-(2,5-difluorphenyl)-1H-pyrazol-3-yl]methanol

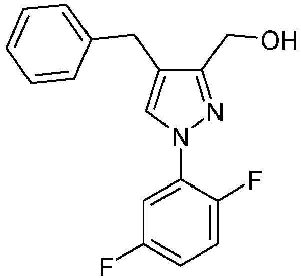

3.18 g (8.92 mmol) Methyl-4-benzoyl-1-(2,5-difluorphenyl)-1H-pyrazol-3-carboxylat (LC/MS-Reinheit = 96%) wurden in 50 ml Trifluoressigsäure vorgelegt, mit 8.74 g (75.13 mmol, 12 ml) Triethylsilan tropfenweise versetzt und 1 h bei Raumtemperatur gerührt. Das Reaktionsgemisch wurde am Rotationsverdampfer eingeengt und im Hochvakuum getrocknet. Der erhaltene Rückstand wurde in 120 ml absolutem THF aufgenommen und bei 0°C mit 2.89 g (33.63 mmol, 33.63 ml) Boran-Tetrahydrofuran-Komplex tropfenweise versetzt. Es wurde über Nacht gerührt. Aufgrund des geringen Umsatzes wurden noch 12.33 ml (12.33 mmol) einer 1M Lithiumborhydrid-Lösung in THF zugegeben. Es wurde 1 h bei Raumtemperatur 30 Min. bei 60°C und 2 h bei 80°C gerührt. Bei 0°C wurde vorsichtig mit 60 ml gesättigter Natriumhydrogencarbonat-Lösung gequencht. Das Gemisch wurde zweimal mit je 100 ml Essigsäureethylester extrahiert, die vereinigten organischen Phasen über Natriumsulfat getrocknet, am Rotationsverdampfer eingeengt und der Rückstand im Hochvakuum getrocknet. Es wurden 2.67 g (76% d. Th., Reinheit = 96%) der Zielverbindung erhalten.

LC-MS (Methode 3): Rₜ = 2.79 min; MS (ESIpos): m/z = 329 [M+H]⁺.

¹H-NMR (400 MHz, DMSO-d₆): δ [ppm] = 3.91 (s, 2H), 4.45 (d, 2H), 6.51 (s, 1H), 7.18-7.23 (m, 2H), 7.27-7.32 (m, 4H), 7.46-7.53 (m, 1H), 7.60-7.65 (m, 1H), 7.95 (d, 1H).

### Intermediat C46

### 4-Benzyl-1-(2,5-difluorphenyl)-1H-pyrazol-3-carbaldehyd

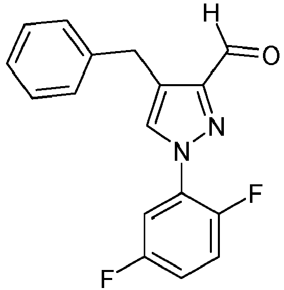

2.66 g (8.50 mmol) [4-Benzyl-1-(2,5-difluorphenyl)-1H-pyrazol-3-yl]methanol (Reinheit 96%) wurden in 150 ml Dichlormethan gelöst und mit 4.33 g (10.20 mmol) Dess-Martin-Periodinan portionsweise versetzt. Das Gemisch wurde 2 h bei Raumtemperatur gerührt und anschließend mit 100 ml einer halbkonzentrierten Natriumhydrogencarbonat-Lösung sowie mit 100 ml einer 10%-igen Natriumthiosulfat-Lösung versetzt, und für 20 Min. gerührt. Die organische Phase wurde abgetrennt, über Natriumsulfat getrocknet und im Hochvakuum eingeengt. Es wurden 2.35 g (88% d. Th., Reinheit = 95%) der Zielverbindung erhalten.

LC-MS (Methode 7): Rₜ = 1.49 min; MS (ESIpos): m/z = 299 [M+H]⁺.

¹H-NMR (400 MHz, DMSO-d₆): δ [ppm] = 4.12 (s, 2H), 7.17-7.21 (m, 1H), 7.27-7.31 (m, 4H), 7.37-7.42 (m, 1H), 7.57-7.62 (m, 1H), 7.75-7.78 (m, 1H), 8.22 (d, 1H), 10.06 (s, 1H).

### Intermediat C47

### (1R)-1-[4-benzyl-1-(2,5-difluorophenyl)-1H-pyrazol-3-yl]-2,2-dimethylpropan-1-amine

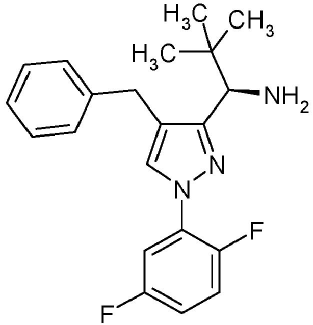

2.35 g (7.56 mmol) 4-Benzyl-1-(2,5-difluorphenyl)-1H-pyrazol-3-carbaldehyd wurden in 25 ml absolutem THF gelöst und mit 1.10 g (9.08 mmol) (R)-(+)-2-Methyl-2-propansulfinsäureamid sowie mit 4.73 g (16.64 mmol) Titan(IV)isopropylat versetzt. Das Reaktionsgemisch wurde bei Raumtemperatur für 16h gerührt, anschließend mit 20 ml einer gesättigten Natriumchlorid-Lösung und 30 ml Essigsäureethylester versetzt. Danach gab man ca. 3 g Kieselgur zu und kochte 1 h unter Rückfluss. Es wurde filtriert und vom Filtrat die organische Phase abgetrennt. Die wässrige Phase wurde mit Essigsäureethylester extrahiert, die vereinigten organischen Phasen mit gesättigter Natriumchlorid-Lösung gewaschen, über Natriumsulfat getrocknet, am Rotationsverdampfer eingeengt und im Hochvakuum getrocknet. Der Rückstand wurde ohne weitere Reinigung weiter eingesetzt.

Unter Argonatmospähre wurde der Rückstand in 60 ml absolutem THF gelöst und auf -78°C gekühlt und 14.5 ml (23.24 mmol) einer Lösung von tert.-Butyllithium in Pentan (c = 1.6 mol/l) zugetropft. Es wurde 3 h bei -78°C gerührt und anschließend mit 5 ml Methanol und 15 ml einer gesättigten Ammoniumchlorid-Lösung gequencht. Unter Rühren ließ man das Reaktionsgemisch auf Raumtemperatur kommen (ca. 30 Min.). Es wurde mit Essigsäureethylester extrahiert, die organische Phase mit gesättigter Natriumchlorid-Lösung extrahiert, am Rotationsverdampfer eingeengt und im Hochvakuum getrocknet. Der Rückstand wurde ohne weitere Reinigung weiter eingesetzt.

Der Rückstand wurde in 30 ml THF und 6 ml Methanol aufgenommen, mit 6 ml (24.00 mmol) einer 4N Chlorwasserstoff-Lösung in Dioxan versetzt und 1 h bei Raumtemperatur gerührt. Anschließend gab man 15 ml gesättigte Natriumcarbonat-Lösung zu und extrahierte mit Essigsäureethylester. Die organische Phase wurde abgetrennt, am Rotationsverdampfer eingeengt und im Hochvakuum getrocknet. Der Rückstand wurde mittels präparativer HPLC (Eluent: ACN/Wasser, Gradient) getrennt. Es wurden zwei Fraktionen der Zielverbindung erhalten. Die erste Fraktion ergab 1.31 g (72 % d. Th., LC/MS Reinheit = 97%) und die zweite 0.37 g (17% d. Th., LC/MS Reinheit = 83%) Produkt.

LC-MS (Methode 1): Rₜ = 0.88 min; MS (ESIpos): m/z = 356 [M+H]⁺.

¹H-NMR (400 MHz, DMSO-d₆): δ [ppm] = 0.91 (s, 9H), 1.71 (s, 2H), 3.59 (s, 1H), 3.87 (s, 2H), 7.17-7.32 (m, 6H), 7.45-7.51 (m, 1H), 7.61-7.65 (m, 1H), 7.84(s br, 1H).

### Intermediat C48

### Tert-butyl-(2S)-4-({(1R)-1-[4-benzyl-1-(2,5-difluorphenyl)-1H-pyrazol-3-yl]-2,2-dimethylpropyl}amino)-2-[(tert-butoxycarbonyl)amino]butanoat

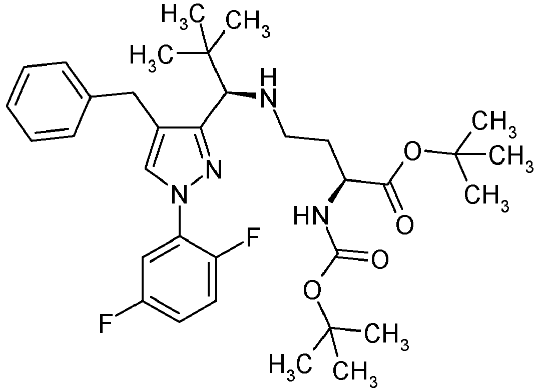

1.28 g (3.35 mmol, LC/MS Reinheit 93%) (1R)-1-[4-benzyl-1-(2,5-difluorophenyl)-1H-pyrazol-3-yl]-2,2-dimethylpropan-1-amine wurden in 100 ml absolutem Dichlormethan gelöst und bei Raumtemperatur mit 261 mg (4.35 mmol, 250 µl) Essigsäure sowie 1.14 g (4.34 mmol) Natriumtriacetoxyborhydrid versetzt und nach 5 Min rühren addierte man 1.19 g (4.35 mmol) tert-Butyl-(2S)-2-[(tert-butoxycarbonyl)amino]-4-oxobutanoat. Es wurde 15 Min. bei Raumtemperatur gerührt, am Rotationsverdampfer eingeengt in Acetonitril und Wasser aufgenommen und mittels präparativer HPLC (Eluent: ACN/Wasser + 0.1% TFA, Gradient) gereinigt. Es wurden 1.64 g (80% d. Th.) der Zielverbindung erhalten.

LC-MS (Methode 1): Rₜ = 1.10 min; MS (ESIpos): m/z = 613 [M+H]⁺.

¹H-NMR (400 MHz, DMSO-d₆): δ [ppm] = 1.01 (s, 9H), 1.32 (s, 9H), 1.35 (s, 9H), 1.80-1.89 (m, 1H), 2.01-2.11 (m, 1H), 2.54-2.71 (m, 2H), 3.75-3.81 (m, 1H), 3.90 (s, 2H), 4.18 (d, 1H), 7.13 (d, 1H), 7.20-7.24 (m, 1H), 7.28-7.34 (m, 5H), 7.52-7.58 (m, 1H), 7.76-7.80 (m, 1H), 8.10 (s br, 1H), 8.23 (s br, 1H).

### Intermediat C49

### (2S)-4-[{(1R)-1-[4-Benzyl-1-(2,5-difluorphenyl)-1H-pyrazol-3-yl]-2,2-dimethylpropyl } (glycoloyl)amino]-2-[(tert-butoxycarbonyl)amino]butansäure

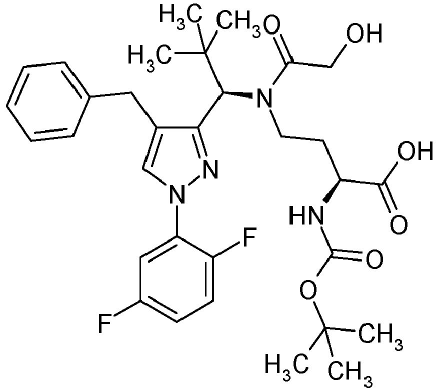

225 mg (0.37 mmol) Tert-butyl-(2S)-4-({(1R)-1-[4-benzyl-1-(2,5-difluorphenyl)-1H-pyrazol-3-yl]-2,2-dimethylpropyl}amino)-2-[(tert-butoxycarbonyl)amino]butanoat wurden in 10 ml absolutem Dichlormethan gelöst, mit 156 mg (1.54 mmol) Triethylamin versetzt. Bei 0°C wurden 125 mg (0.92 mmol) Acetoxyacetylchlorid zugegeben und bei RT für 16 h gerührt. Es wurden erneut 251 mg (1.84 mmol) Acetoxyacetylchlorid und 186 mg (1.84 mmol) Triethylamin addiert und 3 h bei RT gerührt. Es wurde etwas Dichlormethan zugegeben, mit gesättigter Natriumhydrogencarbonat-Lösung und gesättigter Natriumchlorid-Lösung gewaschen. Die organische Phase wurde über Natriumsulfat getrocknet, am Rotationsverdampfer eingeengt und im Hochvakuum getrocknet. Der Rückstand wurde in 10 ml Ethanol aufgenommen und mit einer 0.91 ml (12.67 mmol) einer 40%-igen wässrigen Methylamin-Lösung versetzt und bei 50°C 3 h gerührt. Das Gemisch wurde am Rotationsverdampfer eingeengt, der Rückstand in Dichlormethan aufgenommen und die organische Phase zweimal mit Wasser gewaschen. Die organische Phase wurde über Natriumsulfat getrocknet, am Rotationsverdampfer eingeengt und im Hochvakuum getrocknet. Der Rückstand wurde in 2 ml Dichlormethan aufgenommen mit 2 ml (25.96 mmol) Trifluoressigsäure versetzt und bei 50°C 4 h gerührt. Das Gemisch wurde am Rotationsverdampfer eingeengt und der Rückstand im Hochvakuum getrocknet. Der Rückstand wurde in 10 ml absolutem Dichlormethan aufgenommen, mit 298 mg (2.95 mmol) Triethylamin sowie 429 mg (1.97 mmol) Di-tert-butyldicarbonat versetzt und 1 h bei RT gerührt. Das Gemisch wurde am Rotationsverdampfer eingeengt und der Rückstand mittels präparativer HPLC (Eluent: ACN/Wasser, Gradient) gereinigt. Es wurden 62 mg (27% d. Th.) der Zielverbindung erhalten.

LC-MS (Methode 1): Rₜ = 1.32 min; MS (ESIpos): m/z = 615 [M+H]⁺.

¹H-NMR (400 MHz, DMSO-d₆): δ [ppm] = 0.91 (s, 9H), 1.32 (s, 9H), 2.64-2.72 (m, 4H), 3.50-3.58 (m, 1H), 3.72 (dd, 2H), 4.07-4.22 (m, 2H), 4.47-4.54 (m, 1H), 5.75 (s, 1H), 6.84-6.89 (m, 1H), 7.15-7.30 (m, 6H), 7.47-7.53 (m, 1H), 7.70-7.75 (m, 1H), 8.09-8.13 (m, 1H), 11.66 (s br, 1H).

### Intermediat C50

### tert-Butyl-[(2S)-4-[{(1R)-1-[4-benzyl-1-(2,5-difluorphenyl)-1H-pyrazol-3-yl]-2,2-dimethylpropyl}(glycoloyl)amino]-1-{[2-(2,5-dioxo-2,5-dihydro-1H-pyrrol-1-yl)ethyl]amino}-1-oxobutan-2-yl]carbamat

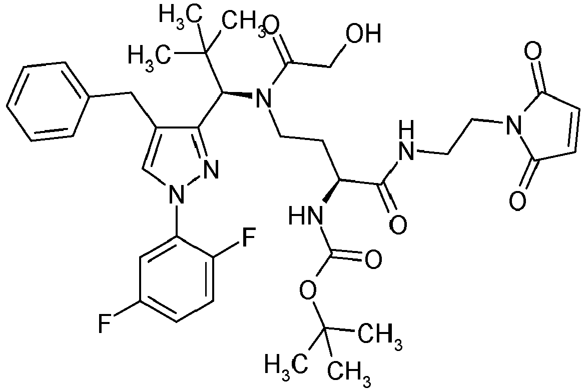

60 mg (0.1 mmol) (2S)-4-[{(1R)-1-[4-Benzyl-1-(2,5-difluorphenyl)-1H-pyrazol-3-yl]-2,2-dimethylpropyl}(glycoloyl)amino]-2-[(tert-butoxycarbonyl)amino]butansäure wurden in 10 ml absolutem DMF gelöst und mit 74 mg (0.20 mmol) HATU versetzt. 74 mg (0.29 mmol) Trifluoressigsäure-1-(2-aminoethyl)-1H-pyrrol-2,5-dion(1:1) wurden separat in 2 ml absolutem DMF gelöst und mit 38 mg (0.29 mmol) N,N-Diisopropylethylamin versetzt und tropfenweise zum Reaktionsgemisch gegeben. Es wurde 3 d bei RT gerührt. Das Gemisch wurde direkt mittels präparativer HPLC (Eluent: ACN/Wasser + 0.1% TFA, Gradient) gereinigt. Es wurden 9.3 mg (13% d. Th.) der Zielverbindung erhalten.

LC-MS (Methode 1): Rₜ = 1.34 min; MS (ESIpos): m/z = 737 [M+H]⁺.

### Intermediat C51

### N-{(2S)-4-[{(1R)-1-[4-Benzyl-1-(2,5-difluorphenyl)-1H-pyrazol-3-yl]-2,2-dimethylpropyl } (glycoloyl)amino]-2-[(tert-butoxycarbonyl)amino]butanoyl } -beta-alanin

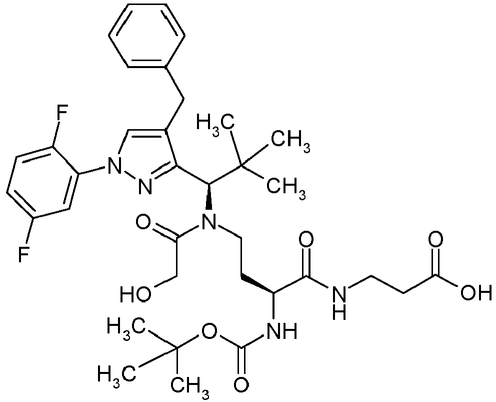

Zunächst wurde Intermediat C47 mit Benzyl-N-{(2S)-2-[(tert-butoxycarbonyl)amino]-4-oxobutanoyl}-beta-alaninat in Analogie zu Intermediat C2 reduktiv alkyliert. Anschließend wurde die sekundäre Aminogruppe mit 2-Chlor-2-oxoethylacetat wie in Intermediat C27 beschrieben acyliert und schließlich mit 2M Lithiumhydroxidlösung in Methanol die Hydrolyse der beiden Estergruppen durchgeführt. Es wurden 23 mg der Titelverbindung erhalten.

LC-MS (Methode 1): Rₜ = 1.24 min; MS (ESIpos): m/z = 686 (M+H)⁺.

### Intermediat C52

### (1R)-1-[1-Benzyl-4-(2,5-difluorphenyl)-1H-pyrrol-2-yl]-2,2-dimethylpropan-1-amin

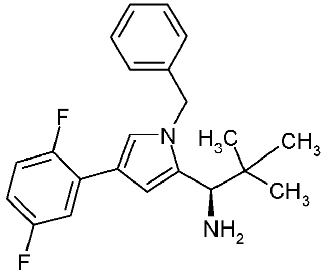

10.00 g (49.01 mmol) Methyl-4-brom-1H-pyrrol-2-carboxylat wurden in 100.0 mL DMF vorgelegt und mit 20.76 g (63.72 mmol) Cäsiumcarbonat und 9.22 g (53.91 mmol) Benzylbromid versetzt. Die Reaktionsmischung rührte über Nacht bei RT. Die Reaktionsmischung wurde zwischen Wasser und Ethylacetat verteilt und die wässrige Phase mit Ethylacetat extrahiert. Die vereinigten organischen Phasen wurden über Magnesiumsulfat getrocknet und das Lösemittel im Vakuum verdampft. Der Ansatz wurde mit 90.0 g Methyl-4-brom-1H-pyrrol-2-carboxylat wiederholt. Die Reinigung der vereinigten beiden Ansätze erfolgte mittles präp. RP-HPLC (Säule: Daiso 300x100; 10µ, Fluss: 250 mL/min, MeCN/Wasser). Die Lösemittel wurden im Vakuum verdampft und der Rückstand im Hochvakuum getrocknet. Man erhielt 125.15 g (87 % d. Th.) der Verbindung Methyl-1-benzyl-4-brom-1H-pyrrol-2-carboxylat.

LC-MS (Methode 1): Rₜ = 1.18 min; MS (ESIpos): m/z = 295 [M+H]⁺.

Unter Argon wurden 4.80 g (16.32 mmol) Methyl-1-benzyl-4-brom-1H-pyrrol-2-carboxylat in DMF vorgelegt und mit 3.61 g (22.85 mmol) (2,5-Difluorphenyl)boronsäure und 19.20 mL ges. Natriumcarbonat-Lösung und 1.33 g (1.63 mmol) [1,1'-Bis-(diphenylphosphino)-ferrocen]-dichlorpalladium(II):Dichlormethan versetzt. Die Reaktionsmischung wurde über Nacht bei 85 °C gerührt. Die Reaktionsmischung wurde über Celite filtriert und der Filterkuchen mit Ethylacetat gewaschen. Die organische Phase wurde mit Wasser extahiert und dann mit ges. NaCl-Lösung gewaschen. Die organische Phase wurde über Magnesiumsulfat getrocknet und das Lösemittel im Vakuum verdampft. Der Rückstand wurde mittels Kieselgel (Laufmittel: Cyclohexan/Ethylacetat 100:3) gereinigt. Die Lösemittel wurden im Vakuum verdampft und der Rückstand im Hochvakuum getrocknet. Man erhielt 3.60 g (67 % d. Th.) der Verbindung Methyl-1-benzyl-4-(2,5-difluorphenyl)-1H-pyrrol-2-carboxylat.

LC-MS (Methode 7): Rₜ = 1.59 min; MS (ESIpos): m/z = 328 [M+H]⁺.

3.60 g (11.00 mmol) Methyl-1-benzyl-4-(2,5-difluorphenyl)-1H-pyrrol-2-carboxylat wurden in 90.0 mL THF vorgelegt und bei 0 °C mit 1.04 g (27.50 mmol) Lithiumaluminiumhydrid (2.4 M in THF) versetzt. Die Reaktionsmischung wurde 30 Minuten bei 0 °C gerührt. Es wurde bei 0 °C ges. Kaliumnatriumtartrat-Lösung zugegeben und die Reaktionsmischung mit Ethylacetat versetzt. Die organische Phase wurde dreimal mit ges. Kaliumnatriumtartrat-Lösung extrahiert. Die organische Phase wurde einmal mit ges. NaCl-Lösung gewaschen und über Magnesiumsulfat getrocknet. Das Lösemittel wurde im Vakuum verdampft und der Rückstand in 30.0 mL Dichlormethan gelöst. Es wurden 3.38 g (32.99 mmol) Mangan(IV)oxid zugegeben und 48 h bei RT gerührt. Es wurden nochmals 2.20 g (21.47 mmol) Mangan(IV)oxid zugegeben und über Nacht bei RT gerührt. Die Reaktionsmischung wurde über Celite filtriert und der Filterkuchen mit Dichlormethan gewaschen.

Das Lösemittel wurde im Vakuum verdampft und der Rückstand 2.80 g (1-Benzyl-4-(2,5-difluorphenyl)-1H-pyrrol-2-carbaldehyd) wurde ohne weitere Reinigung in der nächsten Synthesestufe eingesetzt.

LC-MS (Methode 7): Rₜ = 1.48 min; MS (ESIpos): m/z = 298 [M+H]⁺.

28.21 g (94.88 mmol) 1-Benzyl-4-(2,5-difluorphenyl)-1H-pyrrol-2-carbaldehyd wurden zusammen mit 23.00 g (189.77 mmol) (R)-2-Methylpropan-2-sulfinamid in 403.0 mL absol. THF vorgelegt und mit 67.42 g (237.21 mmol) Titan(IV)isopropylat versetzt und über Nacht bei RT gerührt. Es wurden 500.0 mL ges. NaCl-Lösung und 1000.0 mL Ethylacetat zugegeben und 1 h bei RT gerührt. Es wurde über Kieselgur filtriert und das Filtrat zweimal mit ges. NaCl-Lösung gewaschen. Die org. Phase wurde über Magnesiumsulfat getrocknet und das Lösemittel wurde im Vakuum verdampft und der Rückstand wurde mittels Biotage Isolera gereinigt (Kieselgel, Säule 1500+340 g SNAP, Fluss 200 mL/min, Ethylacetat/Cyclohexan 1:10).

LC-MS (Methode 7): Rₜ = 1.63 min; MS (ESIpos): m/z = 401 [M+H]⁺.

25.00 g (62.42 mmol) (R)-N-{(E/Z)-[1-Benzyl-4-(2,5-difluorphenyl)-1H-pyrrol-2-yl]methylen}-2-methylpropan-2-sulfinamid wurden in absol. THF unter Argon vorgelegt und auf -78 °C abgekühlt. Dann wurden 12.00 g (187.27 mmol) tert.-Butyllithium (1.7 M Lösung in Pentan) bei -78 °C zugegeben und 3 h bei dieser Temperatur gerührt. Dann wurden bei -78 °C 71.4 mL Methanol und 214.3 mL ges. Ammoniumchlorid-Lösung nacheinander zugegeben und die Reaktionsmischung wurde auf RT kommen lassen und 1 h bei RT gerührt. Es wurde mit Ethylacetat verdünnt und mit Wasser gewaschen. Die organische Phase wurde über Magnesiumsulfat getrocknet und das Lösemittel wurde im Vakuum verdampft. Der Rückstand (R)-N-{(1R)-1-[1-Benzyl-4-(2,5-difluorphenyl)-1H-pyrrol-2-yl]-2,2-dimethylpropyl}-2-methylpropan-2-sulfinamid wurde ohne weitere Reinigung in der nächsten Synthesestufe eingesetzt.

LC-MS (Methode 6): Rₜ = 2.97 min; MS (ESIpos): m/z = 459 [M+H]⁺.

28.00 g (61.05 mmol) (R)-N-{(1R)-1-[1-Benzyl-4-(2,5-difluorphenyl)-1H-pyrrol-2-yl]-2,2-dimethylpropyl}-2-methylpropan-2-sulfinamid wurden in 186.7 mL 1,4-Dioxan vorgelegt und dann mit 45.8 mL HCl in 1,4-Dioxan-Lösung (4.0 M) versetzt. Die Reaktionsmischung wurde 2 h bei RT gerührt und das Lösemittel wurde im Vakuum verdampft. Der Rückstand wurde mittles präp. RP-HPLC gereinigt (Säule: Kinetix 100x30; Fluss: 60 mL/min, MeCN/Wasser). Das Acetonitril wurde im Vakuum verdampft und der wässrige Rückstand wurde mit Dichlormethan versetzt. Die organische Phase wurde mit Natriumhydrogencarbonat-Lösung gewaschen und über Magnesiumsulfat getrocknet. Das Lösemittel wurde im Vakuum verdampft und der Rückstand wurde im Hochvakuum getrocknet. Man erhielt 16.2 g (75 % d. Th.) der Titelverbindung.

LC-MS (Methode 6): Rₜ = 2.10 min; MS (ESIpos): m/z = 338 [M-NH₂]⁺, 709 [2M+H]⁺.

¹H-NMR (400 MHz, DMSO-d₆): δ [ppm] = 0.87 (s, 9H), 1.53 (s, 2H), 3.59 (s, 1H), 5.24 (d, 2H), 6.56 (s, 1H), 6.94 (m, 1H), 7.10 (d, 2H), 7.20 (m, 1H), 7.26 (m, 2H), 7.34 (m, 2H), 7.46 (m, 1H).

### Intermediat C53

### (2S)-4-[{(1R)-1-[1-Benzyl-4-(2,5-difluorphenyl)-1H-pyrrol-2-yl]-2,2-dimethylpropyl}(glycoloyl)amino]-2-{[(9H-fluoren-9-ylmethoxy)carbonyl]amino}butansäure

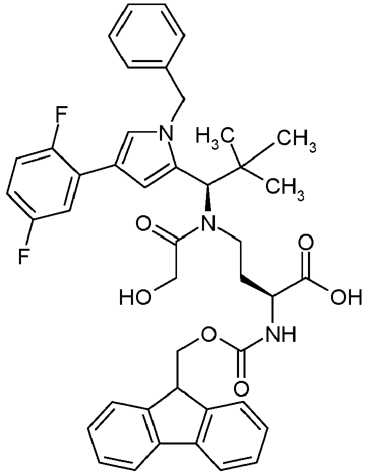

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat wie in Intermediat C27 beschrieben acyliert und schließlich mit 2M Lithiumhydroxidlösung in Methanol die Hydrolyse der beiden Estergruppen durchgeführt. Das so erhaltene Intermediat wurde in Ethanol gelöst, mit Palladium auf Kohle (10%ig) versetzt und bei RT mit Wasserstoff unter Normaldruck 1 h hydriert. Die entschützte Verbindung wurde in Dioxan/Wasser 2:1 aufgenommen und mit 9H-Fluoren-9-ylmethylchlorocarbonat in Gegenwart von N,N-Diisopropylethylamin wurde im letzten Schritt die Fmoc-Schutzgruppe eingeführt.

LC-MS (Methode 1): Rₜ = 1.37 min; MS (ESIpos): m/z = 734 (M-H)⁻.

### Intermediat C54

### N-[(2S)-4-[{(1R)-1-[1-Benzyl-4-(2,5-difluorphenyl)-1H-pyrrol-2-yl]-2,2-dimethylpropyl} (glycoloyl)amino]-2-{ [(9H-fluoren-9-ylmethoxy)carbonyl]amino}butanoyl]-beta-alanin

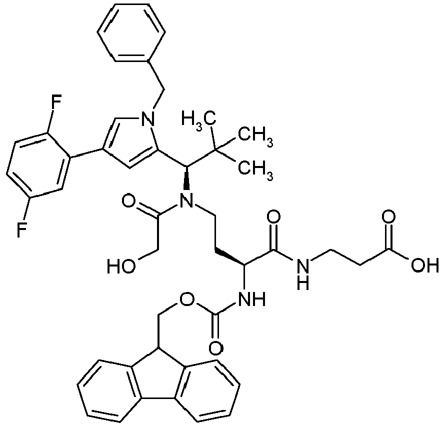

Zunächst wurde Intermediat C52 mit Benzyl-N-[(2S)-2-{[(benzyloxy)carbonyl]amino}-4-oxobutanoyl]-beta-alaninat in Analogie zu Intermediat C2 reduktiv alkyliert. Anschließend wurde die sekundäre Aminogruppe mit 2-Chlor-2-oxoethylacetat wie in Intermediat C27 beschrieben acyliert. Das so erhaltene Intermediat wurde in Methanol gelöst, mit Palladium auf Kohle (10%ig) versetzt und bei RT mit Wasserstoff unter Normaldruck 1 h hydriert. Anschließend wurde mit 2M Lithiumhydroxidlösung in Methanol die Hydrolyse der Estergruppe durchgeführt. Die entschützte Verbindung wurde in Dioxan/Wasser 2:1 aufgenommen und mit 9H-Fluoren-9-ylmethylchlorocarbonat in Gegenwart von N,N-Diisopropylethylamin wurde im letzten Schritt die Fmoc-Schutzgruppe eingeführt. Es wurden 48 mg der Titelverbindung erhalten.

LC-MS (Methode 1): Rₜ = 1.38 min; MS (ESIpos): m/z = 807 (M+H)⁺.

### Intermediat C55

### 2-[3-({(1R)-1-[4-Benzyl-1-(2,5-difluorphenyl)-1H-pyrazol-3-yl]-2,2-dimethylpropyl}amino)propyl]-1H-isoindol-1,3(2H)-dion

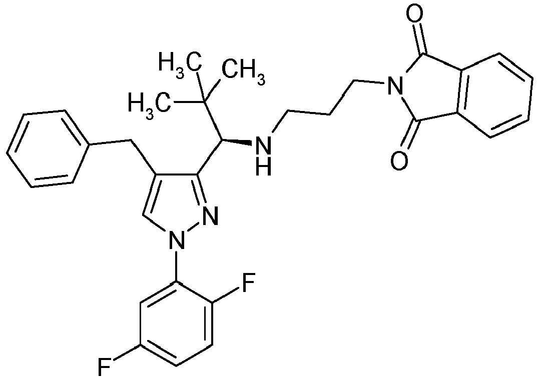

340 mg (0.96 mmol) (1R)-1-[4-benzyl-1-(2,5-difluorophenyl)-1H-pyrazol-3-yl]-2,2-dimethylpropan-1-amine wurden in 7 ml absolutem DCM gelöst und bei RT mit 69 mg (1.15 mmol, 60 µl) Essigsäure sowie 284 mg (1.34 mmol) Natriumtriacetoxyborhydrid versetzt. Es wurde 15 Min. gerührt und anschließend 233 mg (1.15 mmol) 3-(1,3-Dioxo-1,3-dihydro-2H-isoindol-2-yl)propanal addiert. Das Gemisch wurde 4.5 h bei RT gerührt. Es wurden erneut 233 mg (1.15 mmol) 3-(1,3-Dioxo-1,3-dihydro-2H-isoindol-2-yl)propanal, 69 mg (1.15 mmol, 60 µl) Essigsäure sowie 284 mg (1.34 mmol) Natriumtriacetoxyborhydrid zugegeben und 7 h bei RT gerührt. Zur Reaktionsmischung wurde Essigsäureethylester addiert und mit gesättigter Natriumcarbonat-Lösung gewaschen. Die organische Phase wurde eingeengt und der Rückstand zweimal mittels präparativer HPLC [1.) Eluent: ACN/Wasser + 0.1% TFA, Gradient; 2.) Eluent: ACN/Wasser + 1% TFA+1.0% NEt₃)] gereinigt. Es wurden 108 mg (21% d. Th.) der Zielverbindung erhalten.

LC-MS (Methode 1): Rₜ = 0.96 min; MS (ESIpos): m/z = 543 [M+H]⁺.

### Intermediat C56

### 2-({(1R)-1-[4-Benzyl-1-(2,5-difluorphenyl)-1H-pyrazol-3-y1]-2,2-dimethylpropyl}[3-(1,3-dioxo-1,3-dihydro-2H-isoindol-2-yl)propyl]amino)-2-oxoethylacetat

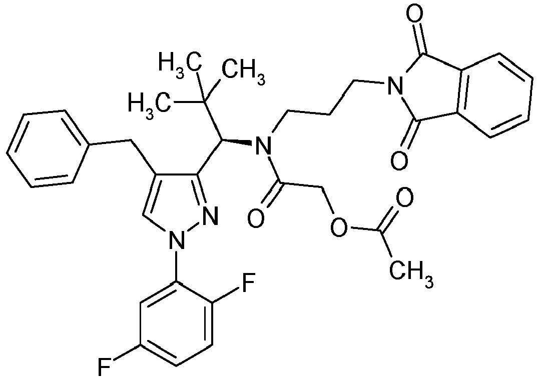

102 mg (0.19 mmol) 2-[3-({(1R)-1-[4-Benzyl-1-(2,5-difluorphenyl)-1H-pyrazol-3-yl]-2,2-dimethylpropyl}amino)propyl]-1H-isoindol-1,3(2H)-dion wurden in 2 ml absolutem DCM vorgelegt und bei RT mit 44 mg (0.43 mmol) Triethylamin versetzt. Bei 0°C wurden 31 mg (0.23 mmol) 2-Chlor-2-oxoethylacetat gelöst in 1 ml absolutem DCM addiert. Das Gemisch wurde 40 Min. bei RT gerührt. Es wurden erneut 26 mg ) 2-Chlor-2-oxoethylacetat gelöst in 0.5 ml absolutem DCM sowie 19 mg (0.19 mmol) Triethylamin zugesetzt und 60 Min. bei RT gerührt. Es wurde Wasser addiert, das Gemisch am Rotationsverdampfer eingeengt und der Rückstand mittels präparativer HPLC (Eluent: ACN/Wasser + 0.1% TFA, Gradient) gereinigt. Es wurden 106 mg (88% d. Th.) der Zielverbindung erhalten.

LC-MS (Methode 1): Rₜ = 1.37 min; MS (ESIpos): m/z = 643 [M+H]⁺.

### Intermediat C57

### Trifluoressigsäure--tert-butyl-{(2S)-1-[(2-aminoethyl)amino]-4-[{(1R)-1-[4-benzyl-1-(2,5-difluorphenyl)-1H-pyrazol-3-yl]-2,2-dimethylpropyl}(glycoloyl)amino]-1-oxobutan-2-yl}carbamat(1: 1)

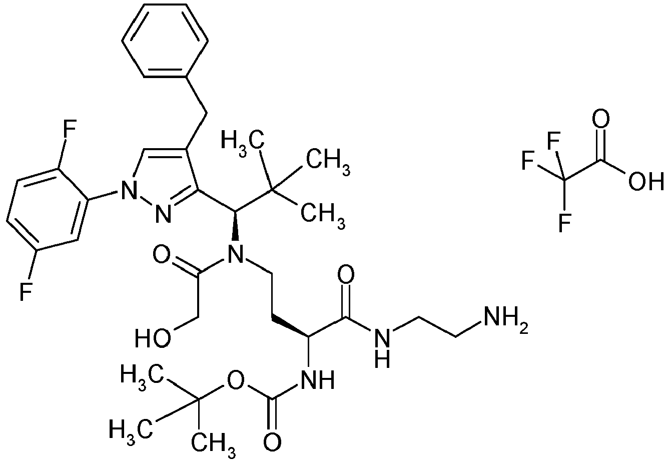

Die Titelverbindung wurde nach Standardmethoden durch Kupplung von Intermediat C49 mit 9H-Fluoren-9-ylmethyl-(2-aminoethyl)carbamat in Gegenwart von HATU und anschließender Abspaltung der Fmoc-Schutzgruppe mit Piperidin hergestellt. Es wurden 14 mg der Titelverbindung (40% d. Th. über 2 Stufen) erhalten.

LC-MS (Methode 1): Rₜ = 0.98 min; MS (ESIpos): m/z = 657 (M+H)⁺.

### Intermediat C58

### (2S)-4-[{(1R)-1-[1-Benzyl-4-(2,5-difluorphenyl)-1H-pyrrol-2-yl]-2,2-dimethylpropyl}(glycoloyl) amino]-2-({[2-(trimethylsilyl)ethoxy]carbonyl}amino)butansäure

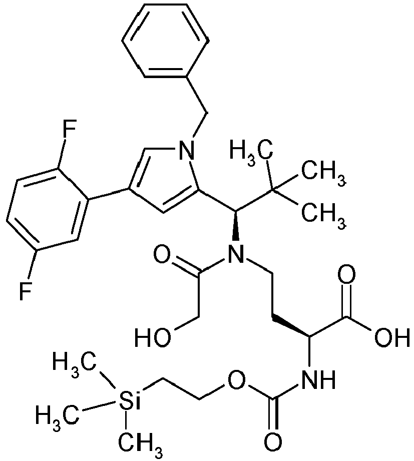

4.3 g (12.2 mmol) von Intermediat C52 wurden in 525 mL DCM gelöst und mit 3.63 g (17.12 mmol) Natriumtriacetoxyborhydrid sowie mit 8.4 mL Essigsäure versetzt. Nach 5 min Rühren bei RT wurden 8.99 g (24.5 mmol) von Intermediat L57 gelöst in 175 mL DCM hinzugegeben und der Ansatz weitere 45 min bei RT gerührt. Der Ansatz wurde dann mit 300 mL DCM verdünnt und zweimal mit 100 mL Natriumhydrogencarbonat-Lösung und einmal mit ges. NaCl-Lösung gewaschen. Die organische Phase wurde über Magnesiumsulfat getrocknet, das Lösemittel im Vakuum verdampft und der Rückstand im Hochvakuum getrocknet. Der Rückstand wurde anschließend mittels präparativer RP-HPLC (Säule: Chromatorex C18) gereinigt. Nach Vereinigung der entsprechenden Fraktionen wurde das Lösemittel im Vakuum verdampft und der Rückstand im Hochvakuum getrocknet. So wurden 4.6 g (61 % d. Th.) Methyl-(2S)-4-({(1R)-1-[1-benzyl-4-(2,5-difluorphenyl)-1H-pyrrol-2-yl]-2,2-dimethylpropyl}amino)-2-({[2-(trimethylsilyl)ethoxy]carbonyl}amino)butanoat erhalten.

LC-MS (Methode 12): Rₜ = 1.97 min; MS (ESIpos): m/z = 614 (M+H)⁺.

2.06 g (3.36 mmol) von diesem Intermediat wurden in 76 mL DCM vorgelegt und mit 0.81 mL (7.17 mmol) 2-Chlor-2-oxoethylacetat in Gegenwart von 2.1 ml Triethylamin acyliert. Nach 20 h Rühren bei RT wurden weitere 0.36 mL 2-Chlor-2-oxoethylacetat und 0.94 ml Triethylamin zugegeben und der Ansatz weitere 15 min bei RT gerührt. Anschließend wurde mit 500 mL Ethylacetat verdünnt und nacheinander zweimal mit 300 mL 5%-iger Zitronensäure, zweimal mit 300 mL gesättigter Natriumhydrogencarbonatlösung und einmal mit 100 mL gesättigter Natriumchloridlösung ausgeschüttelt, dann über Magnesiumsulfat getrocknet und eingeengt. Nach Trocknung im Hochvakuum wurden 2.17 g (79% d. Th.) des geschützten Intermediats erhalten.

LC-MS (Methode 1): Rₜ = 1.48 min; MS (ESIpos): m/z = 714 (M+H)⁺.

2.17 g (2.64 mmol) dieses Intermediats wurden in 54 mL THF und 27 mL Wasser gelöst und mit 26 mL einer 2 molaren Lithiumhydroxidlösung versetzt. Der Ansatz wurde 30 min bei RT gerührt und dann mit 1.4 mL TFA auf einen pH-Wert zwischen 3 und 4 eingestellt. Der Ansatz wurde im Vakuum aufkonzentriert. Nachdem THF weitgehend abdestilliert war wurde die wäßrige Lösung zweimal mit DCM extrahiert und anschließend im Vakuum bis zur Trockne eingeengt. Der Rückstand wurde durch präparative HPLC (Säule: Chromatorex C18) gereinigt. Nach Vereinigung der entsprechenden Fraktionen wurde das Lösemittel im Vakuum verdampft und der Rückstand und aus Acetonitril/Wasser lyophilisiert. So wurden 1.1 g (63% d. Th.) der Titelverbindung erhalten.

LC-MS (Methode 1): Rₜ = 1.34 min; MS (ESIpos): m/z = 656 (M-H)⁻.

¹H-NMR (400 MHz, DMSO-d₆): δ [ppm] = 0.03 (s, 9H), 0.58 (m, 1H), 0.74-0.92 (m, 11H), 1.40 (m, 1H), 3.3 (m, 2H), 3.7 (m, 1H), 3.8-4.0 (m, 2H), 4.15 (q, 2H), 4.9 und 5.2 (2d, 2H), 5.61 (s, 1H), 6.94 (m, 2H), 7.13-7.38 (m, 7H), 7.48 (s, 1H), 7.60 (m, 1H), 12.35 (s, 1H).

### Intermediat C59

### (2S)-4-({(1R)-1-[1-Benzyl-4-(2,5-difluorphenyl)-1H-pyrrol-2-yl]-2,2-dimethylpropyl}[(2S)-2-methoxypropanoyl]amino)-2-{[(9H-fluoren-9-ylmethoxy)carbonyl]amino}butansäure

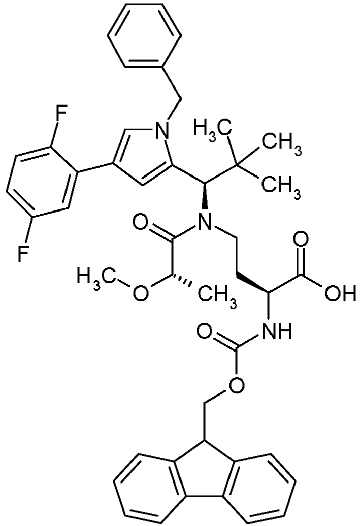

Zunächst wurde die sekundäre Aminogruppe von Benzyl-(2S)-4-({(1R)-1-[1-benzyl-4-(2,5-difluorphenyl)-1H-pyrrol-2-yl]-2,2-dimethylpropyl}amino)-2-{ [(benzyloxy)carbonyl]amino} butanoat mit (2S)-2-Methoxypropanoylchlorid (Zwischenstufe aus Intermediat C53) in Gegenwart von Triethylamin wie in Intermediat C53 beschrieben acyliert. Das erhaltene Intermediat wurde in Ethanol aufgenommen, mit Palladium auf Kohle (10%ig) versetzt und bei RT mit Wasserstoff unter Normaldruck 1 h hydriert. Die entschützte Verbindung wurde in Dioxan/Wasser 2:1 aufgenommen und mit 9H-Fluoren-9-ylmethylchlorocarbonat in Gegenwart von N,N-Diisopropylethylamin wurde im letzten Schritt die Fmoc-Schutzgruppe eingeführt.

LC-MS (Methode 1): Rₜ = 1.39 min; MS (ESIpos): m/z = 764 (M-H)⁻.

### Intermediat C60

### (2S)-4-({(1R)-1-[1-Benzyl-4-(2,5-difluorphenyl)-1H-pyrrol-2-yl]-2,2-dimethylpropyl}[(2S)-2-methoxypropanoyl]amino)-2-{[(9H-fluoren-9-ylmethoxy)carbonyl]amino}butansäure

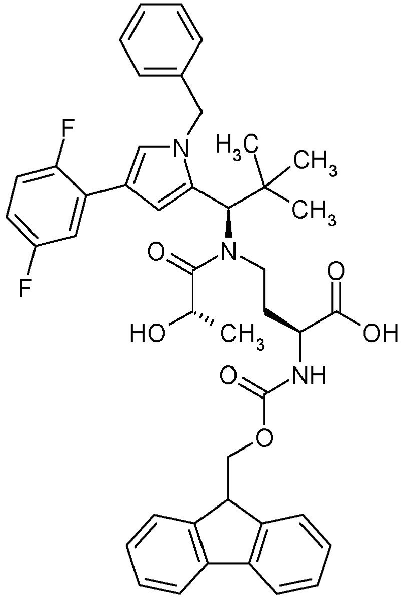

Die Synthese erfolgte in Analogie zu Intermediat C53.

LC-MS (Methode 1): Rₜ = 1.41 min; MS (ESIpos): m/z = 750 (M+H)⁺.

### Intermediat C61

### N-[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-beta-alanin

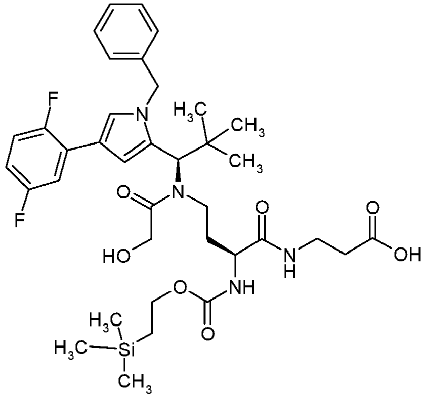

Die Titelverbindung wurde durch Kupplung von 60 mg (0.091 mmol) Intermediat C58 mit β-Alaninmethylester und anschließender Esterspaltung mit 2m Lithiumhydroxidlösung hergestellt. Es wurden 67mg (61% d.Th.) der Titelverbindung über 2 Stufen erhalten.

LC-MS (Methode 1): Rₜ = 1.29 min; MS (ESIpos): m/z = 729 (M+H)⁺.

### Intermediat C62

### N-[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-D-alanin

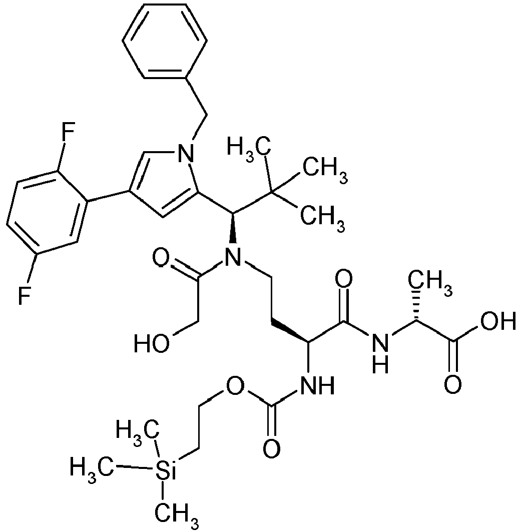

Die Titelverbindung wurde in Analogie zu Intermediat C61 aus Intermediat C58 und Methyl-D-alaninat hergestellt.

LC-MS (Methode 1): Rₜ = 1.32 min; MS (ESIpos): m/z = 729 (M+H)⁺.

### Intermediat C63

### Trifluoressigsäure--tert-butyl-{(2S)-1-[(2-aminoethyl)amino]-4-[{(1R)-1-[1-benzyl-4-(2,5-difluorphenyl)-1H-imidazol-2-yl]-2,2-dimethylpropyl}(glycoloyl)amino]-1-oxobutan-2-yl}carbamat (1:1)

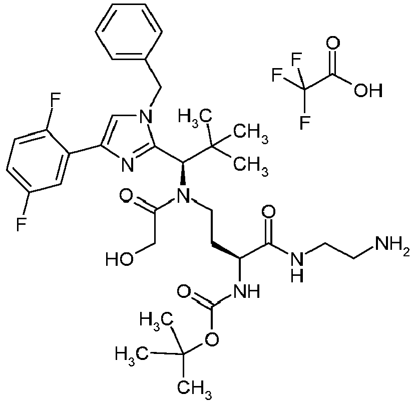

Die Synthese dieses Intermediats begann im ersten Schritt mit der Kupplung von 50 mg (0.075 mmol) Intermediat C3 mit 26.2 mg (0.082 mmol) 9H-Fluoren-9-ylmethyl-(2-aminoethyl)carbamathydrochlorid(1:1) in Gegenwart von 28.7 mg (0.15 mmol) 1-(3-Dimethylaminopropyl)-3-ethylcarbodiimid-Hydrochlorid, 22.9 mg (0.15 mmol) 1-Hydroxy-1*H-*benzotriazol-Hydrat und 39 µl *N,N-*Diisopropylethylamin. Nach 18 h Rühren bei RT wurde eingeengt und der Rückstand durch präparative HPLC gereinigt. 45 mg (65% d. Th.) dieser Zwischenstufe wurden erhalten.

LC-MS (Methode 1): Rₜ = 1.51 min; MS (ESIpos): m/z = 921 (M+H)⁺.

45 mg (0.049 mmol) von dieser Zwischenstufe wurden in 10 mL Ethanol aufgenommen und mit 176µ mL einer 40%-igen Methanaminlösung in Wasser versetzt. Der Ansatz wurde bei 50°C gerührt wobei nach 6h und nach 9h jeweils die gleiche Menge an Methanaminlösung zugegeben wurden. Nach weiteren 14 h Rühren bei 50°C wurden nochmals 700 µl der Mathenaminlösung zugegeben und nach weiteren 20h Rühren schließlich eingeengt. Der Rückstand wurde in DCM aufgenommen und mit Wasser gewaschen. Die organische Phase wurde eingeengt und der Rückstand durch präparative HPLC gereinigt. Nach Einengen der entsprechenden Fraktionen und Trocknung des Rückstandes im Hochvakuum wurden 32 mg (99% d.Th.) von tert-Butyl-{(2S)-1-[(2-aminoethyl)amino]-4-[{(1R)-1-[1-benzyl-4-(2,5-difluorphenyl)-1H-imidazol-2-yl]-2,2-dimethylpropyl } (glycoloyl)amino] -1 -oxobutan-2-yl} carbamat erhaltenen.

LC-MS (Methode 1): Rₜ = 0.95 min; MS (ESIpos): m/z = 657 (M+H)⁺.

### Intermediat C64

### Trifluoressigsäure --2-(trimethylsilyl)ethyl-{(2S)-1-[(2-aminoethyl)amino]-4-[{(1R)-1-[1-benzyl-4-(2,5-difluorphenyl)-1H-pyrrol-2-yl]-2,2-dimethylpropyl}(glycoloyl)amino]-1-oxobutan-2-yl}carbamat (1:1)

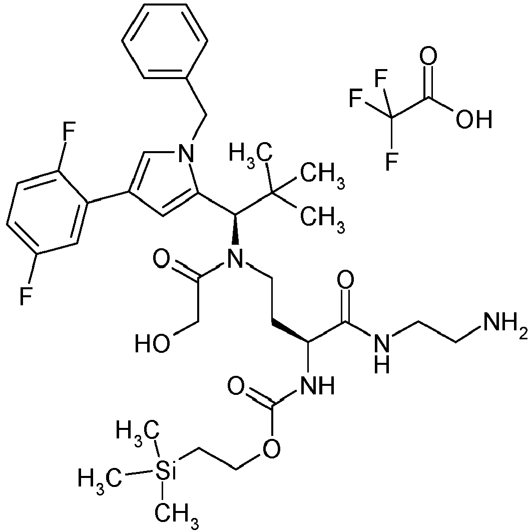

Die Titelverbindung wurde ausgehend von Intermediat C58 in Analogie zu Intermediat C63 hergestellt.
HPLC (Methode 11): Rₜ = 2.4 min;
LC-MS (Methode 1): Rₜ = 1.01 min; MS (ESIpos): m/z = 700 (M+H)⁺.

### Intermediat C65

### (8S)-8-{2-[{(1R)-1-[1-Benzyl-4-(2,5-difluorphenyl)-1H-pyrrol-2-yl]-2,2-dimethylpropyl} (glycoloyl)amino]ethyl}-2,2-dimethyl-6,11-dioxo-5-oxa-7,10-diaza-2-silatetradecan-14-säure

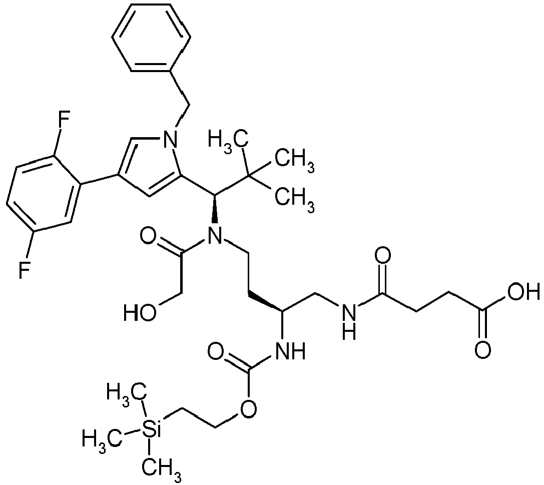

215 mg (0.59 mmol) von Intermediat L66 wurden in 25 mL Dichlormethan vorgelegt und mit 377 mg (0.89 mmol) Dess-Martin Periodinan und 144 µL (1.78 mmol) Pyridin versetzt. Man rührte 30 min bei RT. Anschließend wurde der Ansatz mit 300 ml Dichlormethan verdünnt und die organische Phase je zweimal mit 10%iger Na₂S₂O₃-Lösung, 10%iger Citronensäure-Lösung und ges. Natriumhydrogencarbonat-Lösung gewaschen. Die organische Phase wurde über Magnesiumsulfat getrocknet und das Lösemittel im Vakuum abgedampft. Man erhielt 305 mg des Aldehyds, der ohne weitere Reinigung umgesetzt wurde.

175 mg (0.49 mmol) von Intermediat C52 wurden in 50 ml Dichlormethan gelöst und mit 147mg (0.69 mmol) Natriumtriacetoxyborhydrid sowie 32.5 µL Essigsäure versetzt. Nach 5 min Rühren bei RT wurden 214 mg (0.593 mmol) vom oben beschriebenen Aldehyd hinzugegeben und der Ansatz über Nacht bei RT gerührt. Dabei ist anstelle erwarteten Produktes 2-(Trimethylsilyl)ethyl-[(2S)-4-({(1R)-1-[1-benzyl-4-(2,5-difluorphenyl)-1H-pyrrol-2-yl]-2,2-dimethylpropyl}amino)-1-(2,5-dioxopyrrolidin-1-yl)butan-2-yl]carbamat entstanden. Da auch dieses Imid sich weiter zur Titelverbindung umsetzen lassen sollte, wurde der Ansatz eingeengt und der Rückstand durch präparative HPLC gereinigt. Nach Vereinigung der entsprechenden Imid-enthaltenden Fraktionen wurde das Lösungsmittel im Vakuum abgedampft und der Rückstand im Hochvakuum getrocknet. So wurden 195 mg (58%) des oben benannten Imids erhalten.

LC-MS (Methode 5): Rₜ = 3.32 min; MS (ESIpos): m/z = 667 (M+H)⁺.

65 mg (97.5 µmol) dieses Imids wurden in 15 ml Dichlormethan aufgenommen und mit 367 µL (3.4 mmol) Acetoxyacetylchlorid sowie 595 µL *N,N-*Diisopropylethylamin versetzt. Nach 30 min Rühren bei RT wurde der Ansatz ohne Erwärmung im Vakuum eingeengt und der Rückstand durch präparative HPLC gereinigt. Die entsprechenden Fraktionen wurden vereinigt und nach Abdampfen der Lösungsmittel und Trocknung im Hochvakuum verblieben 28 mg (37% d. Th.) (8S)-11-{(1R)-1-[1-Benzyl-4-(2,5-difluorphenyl)-1H-pyrrol-2-yl]-2,2-dimethylpropyl}-8-[(2,5-dioxopyrrolidin-1-yl)methyl]-2,2-dimethyl-6,12-dioxo-5-oxa-7,11-diaza-2-silatridecan-13-yl-acetat.

LC-MS (Methode 1): Rₜ = 1.44 min; MS (ESIpos): m/z = 767 (M+H)⁺.

28 mg (37 µmol) von diesem Intermediat wurden in 3 mL Methanol gelöst und mit 548 µL einer 2M Lithiumhydroxidlösung versetzt. Nach 10 min Rühren bei RT wurde der Ansatz mit Trifluoressigsäure auf pH 4 gebracht und dann eingeengt. Der Rückstand wurde durch präparative HPLC gereinigt. Nach Vereinigung der entsprechenden Fraktionen, Abdampfen des Lösungsmittels und Trocknung des Rückstandes im Hochvakuum wurden 26 mg (96% d. Th.) der Titelverbindung als weißer Feststoff erhalten.

LC-MS (Methode 1): Rₜ = 1.33 min; MS (ESIpos): m/z = 743 (M+H)⁺.

### Intermediat C66

### 2-(Trimethylsilyl)ethyl-[(2S)-4-[{(1R)-1-[1-benzyl-4-(2,5-difluorphenyl)-1H-pyrrol-2-yl]-2,2-dimethylpropyl}(glycoloyl)amino]-1-{[2-(glycylamino)ethyl]amino}-1-oxobutan-2-yl]carbamat

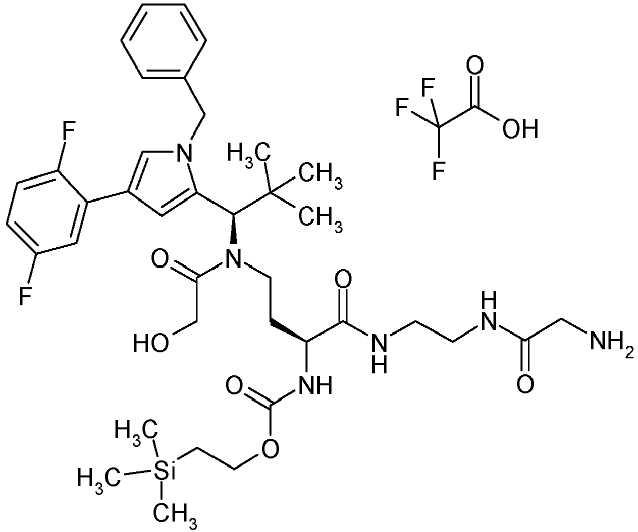

Zuächst wurde aus N-[(benzyloxy)carbonyl]glycin und tert-Butyl (2-aminoethyl)carbamat nach klassischen Methoden der Peptidchemie (HATU-Kupplung und Boc-Spaltung) Trifluoressigsäure - benzyl-{2-[(2-aminoethyl)amino]-2-oxoethyl}carbamat (1:1) hergestellt. 13 mg (0.036 mmol) von diesem Intermediat sowie 25 mg (0.033 mmol) von Intermediat C58 wurden in 3 mL DMF aufgenommen und mit 19 mg (0.05 mmol) HATU und 17 µl N,N-Diisopropylethylamin versetzt. Nach 10 min Rühren bei RT wurde eingeengt und der Rückstand durch präparative HPLC gereinigt. Es wurden 17.8 mg (60% d. Th.) der Zwischenstufe erhalten. LC-MS (Methode 1): Rₜ = 1.36 min; MS (ESIpos): m/z = 891 (M+H)⁺.

17 mg (0.019 mmol) dieser Zwischenstufe wurden in 10 ml Ethanol gelöst, mit Palladium auf Kohle (10%ig) versetzt und bei RT mit Wasserstoff unter Normaldruck 2 h hydriert. Der Katalysator wurde abfiltriert, die Lösemittel im Vakuum verdampft und der Rückstand im Hochvakuum getrocknet. Es wurden 9 mg (62% d. Th.) der Titelverbindung erhalten.

LC-MS (Methode 1): Rₜ = 1.03 min; MS (ESIpos): m/z = 757 (M+H)⁺.

### Intermediat C67

### 9H-Fluoren-9-ylmethyl-[3-({(1R)-1-[1-benzyl-4-(2,5-difluorphenyl)-1H-pyrrol-2-yl]-2,2-dimethylpropyl} amino)propyl]carbamat

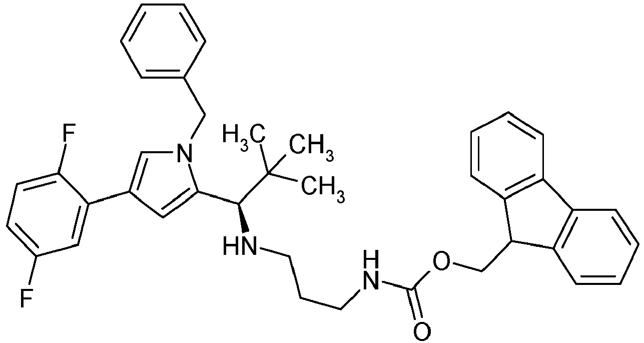

605.3 mg (1.71 mmol) (1R)-1-[1-Benzyl-4-(2,5-difluorphenyl)-1H-pyrrol-2-yl]-2,2-dimethylpropan-1-amin (Intermediat C52) wurden in 10.0 mL Dichlormethan vorgelegt und mit 506.7 mg (2.39 mmol) Natriumtriacetoxyborhydrid und 117.9 mg (1.96 mmol) Essigsäure versetzt und 5 min bei RT grührt. Es wurden 580.0 mg (1.96 mmol) 9H-Fluoren-9-ylmethyl-(3-oxopropyl)carbamat (Intermediat L70) gelöst in 10.0 mL Dichlormethan zugegeben und die Reaktionsmischung über Nacht bei RT gerührt. Die Reaktionsmischung wurde mit Ethylacetat verdünnt und die organische Phase je zweimal mit ges. Natriumcarbonat-Lösung und ges. NaCl-Lösung gewaschen. Die organische Phase wurde über Magnesiumsulfat getrocknet und das Lösemittel im Vakuum verdampft. Der Rückstand wurde mittels Kieselgel (Laufmittel: Cyclohexan/Ethylacetat 3:1) gereinigt. Die Lösemittel wurden im Vakuum verdampft und der Rückstand im Hochvakuum getrocknet. Man erhielt 514.7 mg (46 % d. Th.) der Titelverbindung.

LC-MS (Methode 1): Rₜ = 1.10 min; MS (ESIpos): m/z = 634 (M+H)⁺.

### Intermediat C68

### tert-Butyl-[3-({(1R)-1-[4-benzyl-1-(2,5-difluorphenyl)-1H-pyrazol-3-yl]-2,2-dimethylpropyl} amino)propyl]carbamat

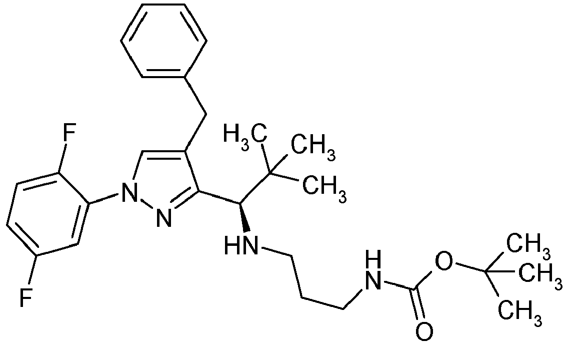

Die Synthese erfolgte in Analogie zur Synthese der Verbidnung Intermediat C67.
1000.0 mg (2.81 mmol) (1R)-1-[4-Benzyl-1-(2,5-difluorphenyl)-1H-pyrazol-3-yl]-2,2-dimethylpropan-1-amin (Intermediat C47)
835.0 mg (3.94 mmol) Natriumtriacetoxyborhydrid
194.0 mg (3.24 mmol) Essigsäure
560.0 mg (3.24 mmol) tert-Butyl-(3-oxopropyl)carbamat
Man erhielt 695.8 mg (48 % d. Th.) der Titelverbindung.
LC-MS (Methode 1): Rₜ = 1.02 min; MS (ESIpos): m/z = 513 (M+H)⁺.

### Intermediat C69

### 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure

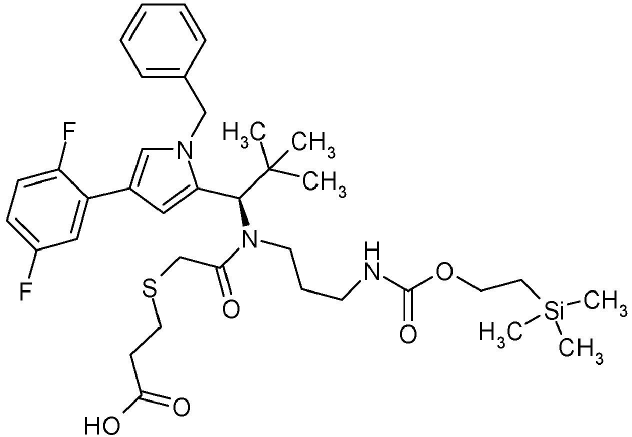

117.0 mg (0.19 mmol) (2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) und 21.6 mg (0.20 mmol) 3-Sulfanylpropansäure wurden in 3.0 mL Methanol vorgelegt und mit 89.5 mg (0.65 mmol) Kaliumcarbonat versetzt und 4 h bei 50 °C gerührt. Die Reaktionsmischung wurde mit Ethylacetat verdünnt und die organische Phase wurde mit Wasser und ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne weitere Reinigung in der nächsten Synthesestufe eingesetzt. Man erhielt 106.1 mg (73 % d. Th.) der Titelverbindung.

LC-MS (Methode 1): Rₜ = 1.42 min; MS (ESIneg): m/z = 700 (M-H)⁻.

### Intermediat C70

### (2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat

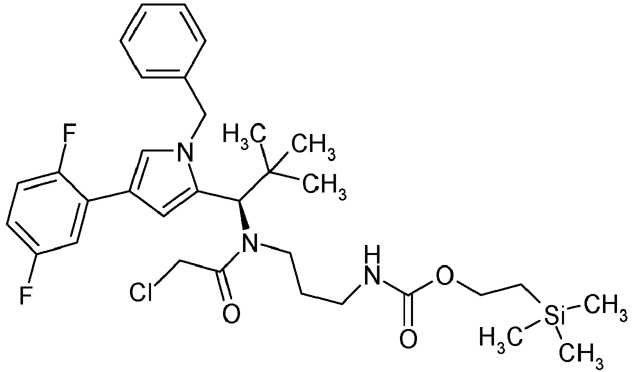

908.1 mg (1.63 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese Intermediat C11) und 545.6 mg (5.39 mmol) Triethylamin wurden in 10.0 mL Dichlormethan vorgelegt und auf 0 °C abgekühlt. Bei dieser Temperatur wurden 590.5 mg (5.23 mmol) Chloracetylchlorid zugegeben und über Nacht bei RT gerührt. Die Reaktionsmischung wurde mit Ethylacetat verdünnt und die organische Phase wurde je dreimal mit ges. Natriumhydrogencarbonat-Lösung und ges. Ammoniumchlorid-Lösung gewaschen. Die organische Phase wurde mit ges. NaCl-Lösung gewaschen und über Magnesiumsulfat getrocknet. Der Rückstand wurde mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 673.8 mg (65 % d. Th.) der Titelverbindung.

LC-MS (Methode 1): Rₜ = 1.53 min; MS (ESIneg): m/z = 676 (M+HCOO⁻)⁻.

### Intermediat C71

### S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1)

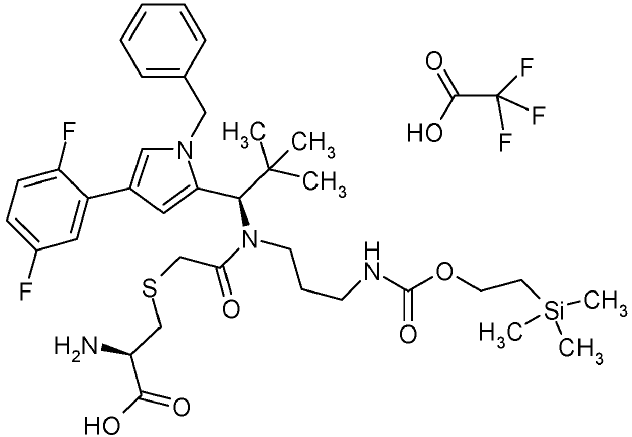

536.6 mg (4.43 mmol) L-Cystein wurden in 2.5 mL Wasser zusammen mit 531.5 mg (6.33 mmol) Natriumhydrogencarbonat suspendiert. Dazu wurden 400.0 mg (0.63 mmol) (2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) gelöst in 25.0 mL *iso-*Propanol und 1.16 g (7.59 mmol) 1,8-Diazabicyclo(5.4.0)undec-7-en gegeben. Die Reaktionsmischung wurde 1.5 h bei 50 °C gerührt. Die Reaktionsmischung wurde mit Ethylacetat versetzt und die org. Phase wurde mehrmals mit ges. Natriumhydrogencarbonat-Lösung und einmal mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 449.5 mg (86 % d. Th.) der Titelverbindung.

LC-MS (Methode 1): Rₜ = 1.20 min; MS (ESIpos): m/z = 717 (M+H)⁺.

### Intermediat C72

### (9S)-9-{[{(1R)-1-[1-Benzyl-4-(2,5-difluorphenyl)-1H-pyrrol-2-yl]-2,2-dimethylpropyl}(glycoloyl) amino]methyl}-2,2-dimethyl-6,11-dioxo-5-oxa-7,10-diaza-2-silatetradecan-14-säure

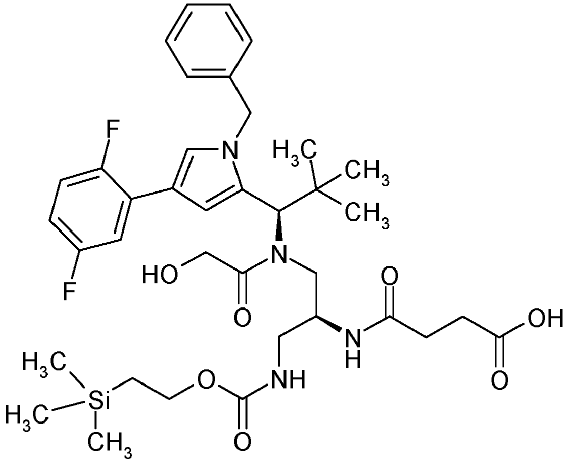

90 mg (0.212 mmol) von Intermediat L72 wurden in 6 mL Dichlormethan vorgelegt und mit 86 µL (1.06 mmol) Pyridin sowie 135 mg (0.318 mmol) Dess-Martin Periodinan versetzt. Man rührte 30 min bei RT. Anschließend wurde der Ansatz mit 30 ml Dichlormethan verdünnt und die organische Phase zweimal mit 10%iger Na₂S₂O₃-Lösung und einmal mit 5%iger Citronensäure-Lösung gewaschen. Die organische Phase wurde über Magnesiumsulfat getrocknet und das Lösemittel im Vakuum abgedampft. Der so erhaltene Aldehyd wurde ohne weitere Reinigung umgesetzt. 63 mg (0.177 mmol) von Intermediat C52 wurden in 15 ml Dichlormethan gelöst und mit 52.4 mg (0.247 mmol) Natriumtriacetoxyborhydrid sowie 20.2 µL Essigsäure versetzt. Nach 5 min Rühren bei RT wurden 89.6 mg (0.212 mmol) vom oben beschriebenen Aldehyd hinzugegeben und der Ansatz 20 min bei RT gerührt. Der Ansatz wurde im Vakuum eingeengt und der Rückstand durch präparative HPLC gereinigt. Nach Vereinigung der entsprechenden Fraktionen wurde das Lösungsmittel im Vakuum abgedampft und der Rückstand aus Acetonitril/Wasser lyophilisiert. So wurden 71 mg (53% d. Th. Über 2 Stufen) Benzyl-(9R)-9-[({(1R)-1-[1-benzyl-4-(2,5-difluorphenyl)-1H-pyrrol-2-yl]-2,2-dimethylpropyl}amino)methyl]-2,2-dimethyl-6,11-dioxo-5-oxa-7,10-diaza-2-silatetradecan-14-oat erhalten.

LC-MS (Methode 1): Rₜ = 1.21 min; MS (ESIpos): m/z = 761 (M+H)⁺.

70 mg (92 µmol) dieses Intermediats wurden in 15 ml Dichlormethan aufgenommen, auf 10°C abgekühlt und mit 54 µL Triethylamin sowie mit 25.5 µL (0.23 mmol) Acetoxyacetylchlorid versetzt. Nach 1 h Rühren bei RT wurden nochmals gleiche Mengen an Säurechlorid und Triethylamin zugegeben und nach einer weiteren Stunde Rühren bei RT erneut. Der Ansatz wurde anschließend für weitere 30 min bei RT gerührt und dann im Vakuum eingeengt und der Rückstand durch präparative HPLC gereinigt. Die entsprechenden Fraktionen wurden vereinigt und nach Abdampfen der Lösungsmittel und Lyophilisation des Rückstandes aus Acetonitril/Wasser verblieben 46.5 mg (59% d. Th.) des acylierten Intermediats.

LC-MS (Methode 1): Rₜ = 1.53 min; MS (ESIpos): m/z = 861 (M+H)⁺.

46 mg (53 µmol) von diesem Intermediat wurden in 5 mL Methanol gelöst und mit 2.7 mL einer 2M Lithiumhydroxidlösung versetzt. Nach 10 min Rühren bei RT wurde der Ansatz mit Essigsäure auf pH 3-4 gebracht und dann mit 15 ml Wasser verdünnt. Die wäßrige Phase wurde mit Ethylacetat extrahiert und die organische Phase über Magnesiumsulfat getrocknet und eingeengt. Der Rückstand wurde aus Acetronitril/Wasser lyophilisiert und nach Trocknung des Rückstandes im Hochvakuum wurden 37 mg (90% d. Th.) der Titelverbindung als weißer Feststoff erhalten.

LC-MS (Methode 1): Rₜ = 1.32 min; MS (ESIpos): m/z = 729 (M+H)⁺.

### Intermediat C73

### S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[3-(trimethylsilyl)propanoyl]-L-cystein

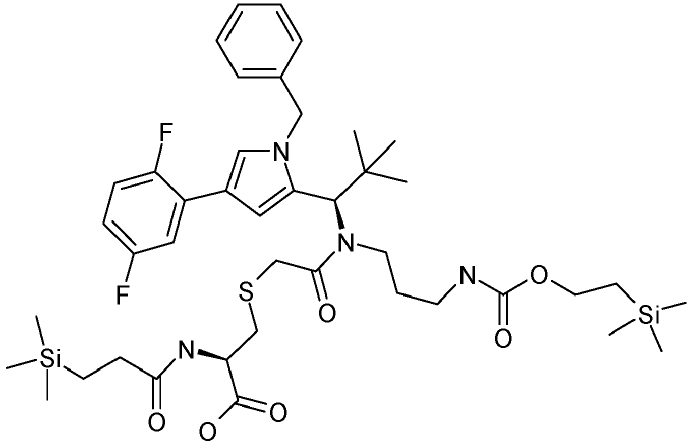

619 mg (0.86 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) wurden in 8.8 ml Dichlormethan vorgelegt und mit 87 mg (0.86 mmol) Triethylamin und 224 mg (0.86 mmol) N-[2-(Trimethylsilyl)-ethoxycarbonyloxy]-pyrrolidin-2,5-dion versetzt. Nach 1h wurden 45 mg (0.17 mmol) N-[2-(Trimethylsilyl)-ethoxycarbonyloxy]-pyrrolidin-2,5-dion zugegeben. Die Reaktionsmischung wurde 1 h bei RT gerührt. Das Gemisch wurde im Vakuum verdampft und der Rückstand in Dichlormethan aufgenommen und dann wurde die organische Phase zweimal mit Wasser und einer gesättigten Natriumhydrogenkarbonat-Lösung gewaschen. Die organische Phase wurde über Magnesiumsulfat getrocknet, am Rotationsverdampfer eingeengt und im Hochvakuum getrocknet. Der Rückstand wurde ohne weitere Reinigung weiter eingesetzt. Man erhielt 602 mg (71%, Reinheit 87%) der Titelverbindung.

LC-MS (Methode 1): Rₜ = 1.58 min; MS (ESIpos): m/z = 861 (M+H)⁺.

### Intermediat C74

### Trifluoressigsäure --2-(trimethylsilyl)ethyl-3-amino-N-[(2S)-4-[{(1R)-1-[1-benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-D-alaninat (1:1)

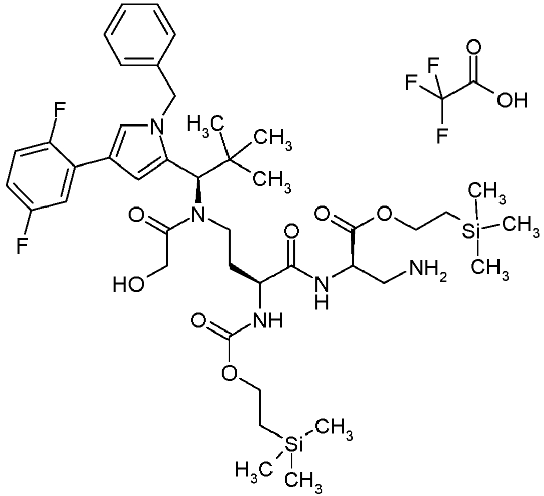

75mg (0.114 mmol) von Intermediat C58 wurden in 12.5 ml DMF aufgenommen und mit 78 mg (0.171 mmol) von Intermediat L75 in Gegenwart von 65 mg (0.11 mmol) HATU und 79 µL *N,N-*Diisopropylethylamin gekuppelt. Nach Reinigung durch präparative HPLC wurde das Intermediat in 20 ml Ethanol aufgenommen und über 10%-igem Palladium auf Aktivkohle 1 h bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1: 1 wurden 63 mg (64% d.Th. über 2 Stufen) der Titelverbindung erhalten.

LC-MS (Methode 1): Rₜ = 1.16 min; MS (EIpos): m/z = 844 [M+H]⁺.

### Intermediat C75

### Methyl-(2S)-4-[(acetoxyacetyl) {(1R)-1-[1-benzyl-4-(2,5-difluorphenyl)-1H-pyrrol-2-yl]-2,2-dimethylpropyl}amino]-2-({[2-(trimethylsilyl)ethoxy]carbonyl}amino)butanoat

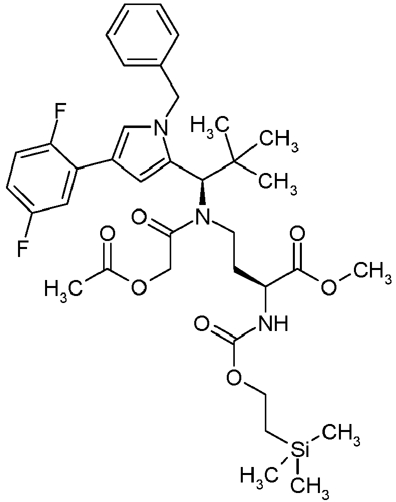

4.3 g (12.2 mmol) von Intermediat C52 wurden in 525 mL DCM gelöst und mit 3.63 g (17.12 mmol) Natriumtriacetoxyborhydrid sowie mit 8.4 mL Essigsäure versetzt. Nach 5 min Rühren bei RT wurden 3.23 g (11.85 mmol) Methyl-(2S)-4-oxo-2-({[2-(trimethylsilyl)ethoxy]carbonyl} amino)butanoat (hergestellt aus (3S)-3-Amino-4-methoxy-4-oxobutansäure nach klassischen Methoden) gelöst in 175 mL DCM zugegeben und der Ansatz weitere 45 min bei RT gerührt. Der Ansatz wurde dann mit DCM verdünnt und zweimal mit 100 mL gesättigter Natriumhydrogencarbonatlösung und dann mit gesättigter Natriumchloridlösung ausgeschüttelt. Die organische Phase wurde über Magnesiumsulfat getrocknet, filtriert und anschließend eingeengt. Der Rückstand wurde mittels präparativer HPLC aufgereinigt. Nach Vereinigung der entsprechenden Fraktionen, Einengen und Trocknen des Rückstandes im Hochvakuum erhielt man 4.6 g (61% d. Th.) des Intermediats.

LC-MS (Methode 12): Rₜ = 1.97 min; MS (ESIpos): m/z = 614.32 (M+H)⁺.

200 mg (0.33 mmol) dieses Intermediats wurden in 10 mL DCM gelöst und dann mit 105 µL Triethylamin sowie mit 77 µL (0.717 mmol) Acetoxyacetylchlorid versetzt. Der Ansatz wurde über Nacht bei RT gerührt und dann im Vakuum eingeengt. Der Rückstand wurde in Ethylacetat aufgenommen und zweimal mit gesättigter Natriumhydrogencarbonatlösung und dann mit gesättigter Natriumchloridlösung ausgeschüttelt. Die organische Phase wurde über Magnesiumsulfat getrocknet und anschließend eingeengt. Es wurden 213 mg (75%) der Titelverbindung als beiger Schaum erhalten.

LC-MS (Methode 1): Rₜ = 1.46 min; MS (ESIpos): m/z = 714 (M+H)⁺.

### Intermediat C76

### N-[(Benzyloxy)carbonyl]-L-valyl-N-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-alaninamid

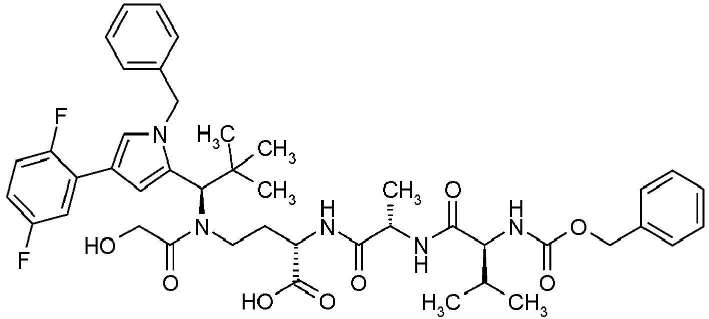

Die Titelverbindung wurde ausgehend von Intermediat C75 nach klassischen Methoden der Peptidchemie hergestellt (Spaltung der Teoc-Schutzgruppe mit Zinkchlorid, Acylierung mit N-[(Benzyloxy)carbonyl]-L-valyl-L-alanin in Gegenwart von HATU und Esterspaltung mit Lithiumhydroxid in THF/Wasser).

LC-MS (Methode 1): Rₜ = 1.23 min; MS (ESIpos): m/z = 818 (M+H)⁺.

### Intermediat C77

### S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,1 1-diaza-2-silatridecan-13-yl)-N-(4-tert-butoxy-4-oxobutanoyl)-L-cystein

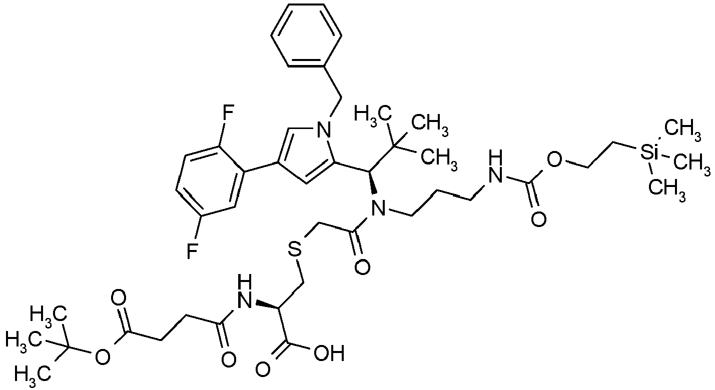

4-tert-Butoxy-4-oxobutansäure (8.39 mg, 48.1 µmol) wurde in 1.0 mL DMF vorgelegt, mit 7.37 mg (48.1 µmol) 1-Hydroxy-1H-benzotriazol Hydrat, 15.5 mg ((48.1 µmol) (Benzotriazol-1-yloxy)bisdimethylaminomethyliumfluoroborat und 8.60 µl (48.1 µmol) N,N-Diisopropylethylamin versetzt und 10 Minuten bei RT gerührt. 40.0 mg (0.048 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (Intermediat C71) wurden in 1.0 mL DMF vorgelegt, mit 25.4 µl (141.9 µmol) N,N-Diisopropylethylamin versetzt, zum Ansatz gegeben und 4 h bei RT gerührt. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 35.0 mg (83 % d. Th.) der Titelverbindung.

LC-MS (Methode 12): Rₜ = 2.76 min; MS (ESIpos): m/z = 873 [M+H]⁺

### Intermediat C78

### 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-säure

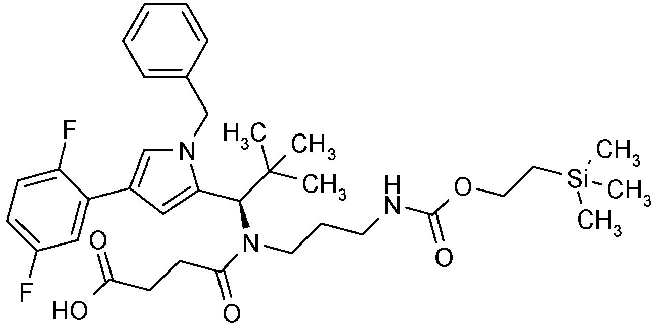

197 mg (0.354 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese Intermediat C11) wurden in 5.0 mL Dichlormethan vorgelegt und auf 40°C erhitzt. Bei dieser Temperatur wurden 240 µl (3.0 mmol) Pyridin und 220 µl (1.8 mmol) Methyl-4-chlor-4-oxobutanoat zugegeben und 1 h bei RT gerührt. Dann wurden 240 µl (3.0 mmol) Pyridin und 220 µl (1.8 mmol) Methyl-4-chlor-4-oxobutanoat zugegeben und 1 hbei RT gerührt. Dann wurden 240 µl (3.0 mmol) Pyridin und 220 µl (1.8 mmol) Methyl-4-chlor-4-oxobutanoat zugegeben und 1 h bei RT gerührt. Die Reaktionsmischung wurde mit Ethylacetat verdünnt und die organische Phase wurde je dreimal mit 5%iger KHSO₄-Lösung extrahiert. Die organische Phase wurde mit ges. NaCl-Lösung gewaschen und über Magnesiumsulfat getrocknet. Die Lösemittel wurden im Vakuum verdampft. Der Rückstand wurde mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 74.1 mg (31 % d. Th.) Methyl-11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-oat.
LC-MS (Methode 1): Rₜ = 1.49 min; MS (ESIpos): m/z = 670 [M+H]⁺

78.3 mg (117 µmol) Methyl-11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-oat wurden in 4.0 mL THF vorgelegt, mit 800 µl Methanol, 160 µl Wasser und 230 µl (230 µmol) wässriger LiOH-Lösung (1M) versetzt. Die Reaktionsmischung wurde 3 h bei RT gerührt, mit Essigsäure gequencht und direkt mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 64.8 mg (85 % d. Th.) der Titel Verbindung.
LC-MS (Methode 12): Rₜ = 2.61 min; MS (ESIneg): m/z = 654 [M-H]⁻

### Intermediat C79

### Trifluoressigsäure 2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1)

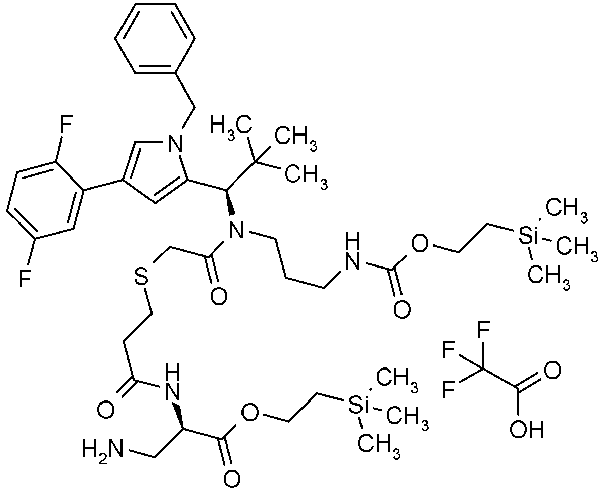

57.4 mg (81.8 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden in 5.7 mL DMF vorgelegt, mit 74.0 mg (164 µmol) Trifluoressigsäure 2-(trimethylsilyl)ethyl-3-{[(benzyloxy)carbonyl]amino}-D-alaninat (1:1) (Intermediat L75), 43 µl (250 µmol) N,N-Diisopropylethylamin und 62.2 mg (164 µmol) HATU versetzt und 1 h bei RT nachgerührt. Die Reaktionsmischung wurde 1 h bei RT gerührt, mit Essigsäure gequencht und direkt mittels präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 52.4 mg (63 % d. Th.) der Verbindung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{ [(benzyloxy)carbonyl]amino } -D-alaninat.
LC-MS (Methode 1): Rₜ = 1.64 min; MS (ESIpos): m/z = 1022 [M]⁺

Unter Argon wurden 6.23 mg (27.7 µmol) Palladium(II)acetat: in 3.0 ml Dichloromethan vorgelegt, mit 12 µL (83 µmol) Triethylamin und 89 µL (550 µmol) Triethylsilan versetzt und 5 Minuten. gerührt. Dann wurden 56.7 mg (55.5 µmol) 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{[(benzyloxy)carbonyl]amino}-D-alaninat in 3.0 mL Dichloromethan zugegeben und über Nacht bei RT gerührt. Der Ansatz wurde bis fast zur Trockene eingeengt, mit Acetonitril/Wasser versetzt, filtriert und mittels präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 37.4 mg (67 % d. Th.) der Titel Verbindung.
LC-MS (Methode 12): ): Rₜ = 2.15 min; MS (ESIpos): m/z = 888 [M+H]⁺

### Intermediat C80

### S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-(glycylamino)-4,7,10,13-tetraoxapentadecan-1-oyl]-L-cystein -trifluoressigsäure (1:1)

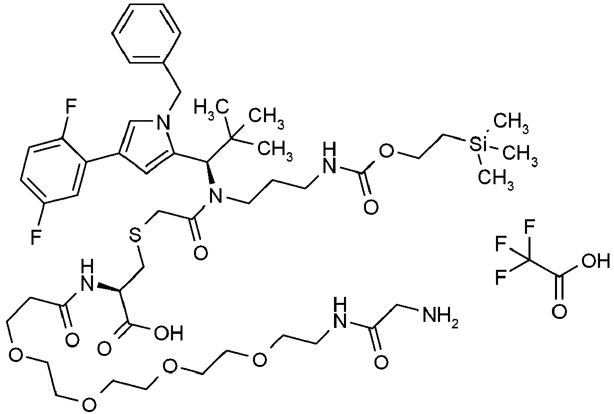

Unter Argon, wurden 43.4 mg (95.1 µmol) 1-({N-[(Benzyloxy)carbonyl]glycyl}amino)-3,6,9,12-tetraoxapentadecan-15-säure (Intermediat L90) in 2.5 ml DMF vorgelegt, mit 14.6 mg (95.1 µmol) 1-Hydroxy-1H-benzotriazol Hydrat, 30.5 mg (95.1 µmol) (Benzotriazol-1-yloxy)bisdimethylaminomethyliumfluoroborat und 16.5 µl (95.1 µmol) N,N-Diisopropylethylamin versetzt und 10 min gerührt. 79.0 mg (95.1 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (Intermediat C71) wurden in 2.5 ml DMF gelöst mit 49.5µl (285.3 µmol) N,N-Diisopropylethylamin versetzt und zum Ansatz gegeben. Die Reactionmischung wurde 2 h bei RT gerührt und direkt mittels präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 44.2 mg (40 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(benzyloxy)carbonyl]glycyl} amino)-4,7,10,13 -tetraoxapentadecan-1 -oyl] -L-cystein.
LC-MS (Methode 12): Rₜ = 2.57 min; MS (ESIpos): m/z = 1156 [M+H]⁺

60.2 mg (52.1 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(benzyloxy)carbonyl]glycyl}amino)-4,7,10,13-tetraoxapentadecan-1-oyl]-L-cystein wurden in 3.0 mL Ethanol suspendiert und mit 6.0 mg Palladium auf Aktivkohle (10%ig) versetzt und bei RT 1 h lang mit Wasserstoff unter Normaldruck hydriert. Zweimal wurden 6.0 mg Palladium auf Aktivkohle (10%ig) versetzt und bei RT 1 h lang mit Wasserstoff unter Normaldruck hydriert. Der Katalysator wurde abfiltriert und die Reaktionsmischung im Vakuum vom Lösemittel befreit und im Hochvakuum getrocknet. Der Rückstand wurde mittels präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 29.4 mg (50 % d. Th.) der Titel Verbindung.
LC-MS (Methode 5): Rₜ = 3.77 min; MS (ESIpos): m/z = 1021 [M+H]⁺

### Intermediat C81

### (R)-1-[1-Benzyl-4-(2,5-difluorphenyl)-1H-pyrrol-2-yl]-1-cyclohexylmethanamin

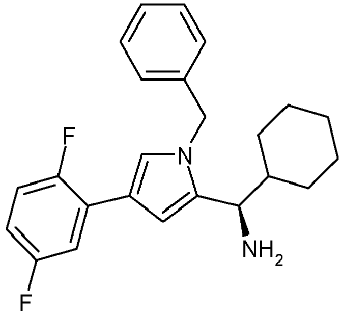

Eine Lösung von 3.12 ml (6.24 mmol) Dimethylzink in Toluol (2.0 M) unter Argon bei -78 °C wurde mit 18.7 ml (37.45 mmol) Cyclohexylmagnesiumchlorid in Diethylether (2M) versetzt und die Mischung wurde 30 Minuten bei -78 °C gerührt. Anschließend wurde eine Lösung von 5.0 g (12.48 mmol) (R)-N-{(E/Z)-[1-Benzyl-4-(2,5-difluorphenyl)-1H-pyrrol-2-yl]methylen}-2-methylpropan-2-sulfinamid in THF bei -78 °C zugegeben und das Reaktionsgemisch wurde 1 h bei dieser Temperatur gerührt und dann 4 h bei RT. Dann wurden bei -78 °C mL ges. Ammoniumchlorid-Lösung zugegeben und die Reaktionsmischung wurde auf RT kommen lassen. Es wurde mit Ethylacetat verdünnt und mit Wasser gewaschen. Die organische Phase wurde über Magnesiumsulfat getrocknet und das Lösemittel wurde im Vakuum verdampft. Der Rückstand wurde mittels Biotage Isolera gereinigt (Kieselgel, Ethylacetat/Cyclohexan 25:75). Man erhielt 1.59 g (26% d. Th.) der Zwischenstufe erhalten.
LC-MS (Methode 12): Rₜ = 2.76 min; MS (ESIneg): m/z = 483 [M-H]⁻

264.0 mg (0.54 mmol) dieser Zwischenstufe wurden in 0.5 mL 1,4-Dioxan unter Argon vorgelegt und dann mit 1.36 mL HCl in 1,4-Dioxan-Lösung (4.0 M) versetzt. Das Reaktionsgemisch wurde 1 h bei RT gerührt. Die Reaktionsmischung wurde mit Dichlormethan versetzt und mit einer wäs.1M Natriumhydroxid-Lösung gewaschen. Die organische Phase wurde mit Magnesiumsulfat getrocknet und das Lösemittel wurde im Vakuum verdampft. Der Rückstand wurde mittels Biotage Isolera gereinigt (Kieselgel, Methanol/Dichlornethan 98:2). Das Lösemittel wurde im Vakuum verdampft und der Rückstand wurde in Dichlormethan gelöst und mit einer Natriumhydrogencarbonat-Lösung gewaschen und über Natriumsulfat getrocknet. Das Lösemittel wurde im Vakuum verdampft und der Rückstand wurde im Hochvakuum getrocknet. Man erhielt 148 mg (72% d. Th.) der Titelverbindung.
LC-MS (Methode 13): Rₜ = 2.07 min; MS (ESIpos): m/z = 364 [M-NH₂]⁺

### Intermediat C82

### 2-(Trimethylsilyl)ethyl-(3-{ [(R)-[1-benzyl-4-(2,5-difluorphenyl)-1H-pyrrol-2-yl](cyclohexyl)methyl]amino}propyl)carbamat

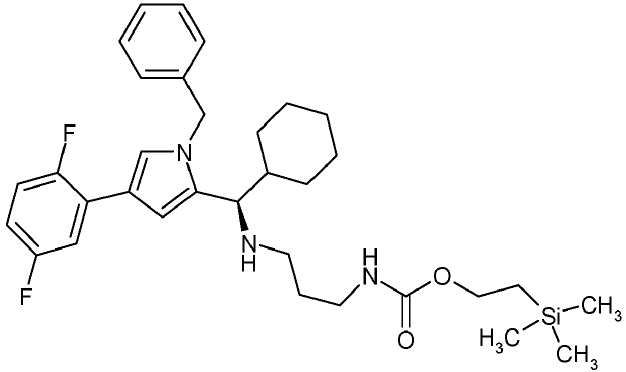

Eine Lösung von 503.0 mg (1.32 mmol) 1-[1-Benzyl-4-(2,5-difluorphenyl)-1H-pyrrol-2-yl]-1-cyclohexylmethanamin (Intermediat C81) in 1.4 ml Dichlormethan unter Argon wurde mit 392.2 mg (1.85 mmol) Natriumtriacetoxyborhydrid und 91.29 mg (1.52 mmol) Essigsaüre versetzt und das Reaktionsgemisch wurde 10 Minuten bei RT gerührt. Anschließend wurde eine Lösung von 574.6 (2.38 mmol) 2-(Trimethylsilyl)ethyl-(3-oxopropyl)carbamat in Dichlormethan hinzugegeben und das Gemisch wurde über Nacht bei RT gerührt. Nach Zugabe von 143 mg (0.66 mmol) 2-(Trimethylsilyl)ethyl-(3-oxopropyl)carbamat wurde die Mischung noch 2 h weiter gerührt. Das Reaktionsmgemisch wurde mit Dichlormethan verdünnt und die organische Phase wurde je zweimal mit ges. Natriumcarbonat-Lösung und mit ges. NaCl-Lösung gewaschen und über Natriumsulfat getrocknet und eingeengt. Der Rückstand wurde mittels präparative HPLC gereinigt. Die Lösemittel wurden im Vakuum verdampft und der Rückstand im Hochvakuum getrocknet. Man erhielt 488 g (63 % d. Th.) der Titelverbindung.

LC-MS (Methode 12): Rₜ = 1.89 min; MS (ESIpos): m/z = 582 (M+H)⁺.

### Intermediat C83

### 2-(Trimethylsilyl)ethyl-(3-{ [(R)-[1-benzyl-4-(2,5-difluorphenyl)-1H-pyrrol-2-yl](cyclohexyl)methyl](chloracetyl)amino}propyl)carbamat

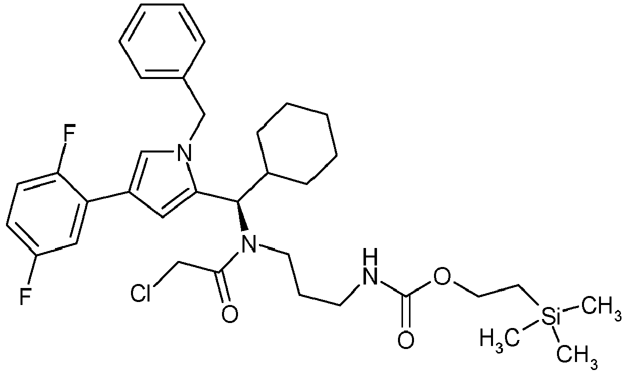

Zur eine Lösung von 487.9 mg (0.84 mmol) 2-(Trimethylsilyl)ethyl-(3-{[(R)-[1-benzyl-4-(2,5-difluorphenyl)-1H-pyrrol-2-yl](cyclohexyl)methyl]amino}propyl)carbamat (Intermediat C82) in 8.40 ml Dichlormethan mit Mol Sieb 4 Ä wurden 280.0 mg (2.77 mmol) Triethylamin und 397.8 mg (3.52 mmol) Chloracetylchlorid zugegeben und das Reakionsgemisch wurde 6 h bei RT gerührt. Die Reaktionsmischung wurde mit Dichlormethan verdünnt und die organische Phase wurde mit ges. Natriumhydrogencarbonat-Lösung und ges. Ammoniumchlorid-Lösung gewaschen. Die organische Phase wurde über Natriumsulfat getrocknet und eingeengt. Der Rückstand wurde ohne Reinigung weiter eingesetzt. Man erhielt 470 mg (85 % d. Th.) der Titelverbindung.

LC-MS (Methode 12): Rₜ = 2.88 min; MS (ESIpos): m/z = 680 (M+Na)⁺.

### Intermediat C84

### S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-L-cystein

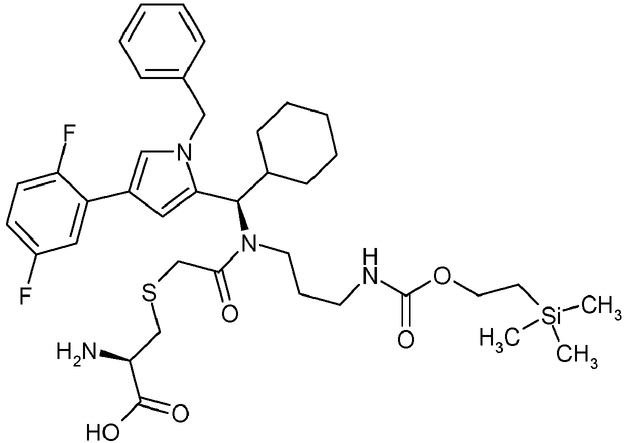

322.1 mg (2.66 mmol) L-Cystein wurden in 0.19 mL Wasser zusammen mit 319.0 mg (3.80 mmol) Natriumhydrogencarbonat suspendiert. Dazu wurden 250.0 mg (0.38 mmol) 2-(Trimethylsilyl)-ethyl-(3-{[(R)-[1-benzyl-4-(2,5-difluorphenyl)-1H-pyrrol-2-yl](cyclohexyl)methyl](chloracetyl)-amino}propyl)carbamat (Intermediat C83) gelöst in 1.90 mL iso-Propanol und 693.8 mg (4.56 mmol) 1,8-Diazabicyclo(5.4.0)undec-7-en gegeben. Die Reaktionsmischung wurde 3.5 h bei 50 °C gerührt. Die Reaktionsmischung wurde mit Ethylacetat versetzt und die org. Phase wurde mehrmals mit ges. Natriumhydrogencarbonat-Lösung und mit ges. NaCl-Lösung gewaschen. Die organische Phase wurde über Natriumsulfat getrocknet und das Lösemittel im Vakuum verdampft. Der Rückstand wurde ohne weitere Reinigung weiter eingesetzt. Man erhielt 276 mg (97 % d. Th.) der Titelverbindung.

LC-MS (Methode 12): Rₜ = 2.34 min; MS (ESIpos): m/z = 744 (M+H)⁺.

### Intermediat C85

### S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-N-[6-(2,5-dioxo-2,5-dihydro-1H-pyrrol-1-yl)hexanoyl] -L-cystein

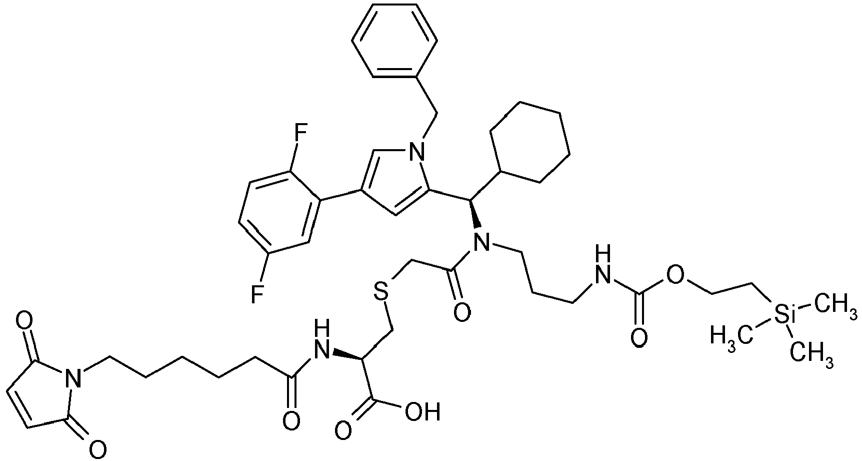

Zu einer Mischung von 100 mg (0.13 mmol) S-{11-[(R)-[1-Benzyl-4-(2,5-difluorphenyl)-1H-pyrrol-2-yl](cyclohexyl)methyl]-2,2-dimethyl-6,12-dioxo-5-oxa-7,11-diaza-2-silatridecan-13-yl}-L-cystein (1:1) (Intermediat C84) und 41.5 mg ( 0.13 mmol) 1-{6-[(2,5-Dioxopyrrolidin-1-yl)oxy]-6-oxohexyl}-1H-pyrrol-2,5-dion in 4.0 ml DMF wurden 34.8 mg ( 0.27 mmol) N,N-diisopropylethylamin hinzugegeben und das Reaktionsgemisch wurde 3 h bei RT gerührt. Die Mischung wurde ohne Aufarbeitung mittels präparative HPLC gereinigt. Man erhielt 88 mg (70% d. Th.) der Titelverbindung.

LC-MS (Methode 12): Rₜ = 2.71 min; MS (ESIpos): m/z = 936 (M+H)⁺.

### Intermediat C86

### 11-[(R)-[1-Benzyl-4-(2,5-difluorphenyl)-1H-pyrrol-2-yl](cyclohexyl)methyl]-2,2-dimethyl-6,12-dioxo-5-oxa-14-thia-7,11-diaza-2-silaheptadecan-17-säure

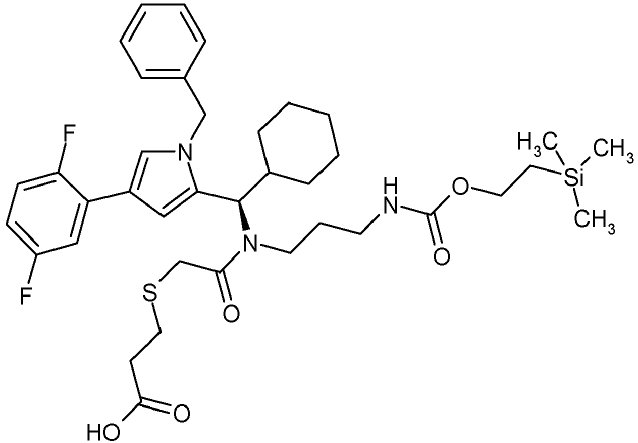

Eine Mischung von 220.0 mg (0.33 mmol) 2-(Trimethylsilyl)ethyl-(3-{[(R)-[1-benzyl-4-(2,5-difluorphenyl)-1H-pyrrol-2-yl](cyclohexyl)methyl](chloracetyl)amino}propyl)carbamat (Intermediat C83) und 39.02 mg (0.37 mmol) 3-Sulfanylpropansäure in 7.45 ml Methanol und einige Tropfen Wasser wurde mit 161.65 mg (1.17 mmol) Kaliumcarbonat versetzt. Das Reaktionsgemisch wurde 4 h bei 50 °C gerührt. Die Reaktionsmischung wurde mit Ethylacetat versetzt und die org. Phase wurde mehrmals mit Wasser und mit ges. NaCl-Lösung gewaschen. Die organische Phase wurde über Natriummsulfat getrocknet und das Lösemittel im Vakuum verdampft. Der Rückstand wurde ohne Aufarbeitung weiter eingesetzt. Man erhielt 201 mg (83 % d. Th.) der Titelverbindung.

LC-MS (Methode 12): Rₜ = 2.72 min; MS (ESIneg): m/z = 726 (M-H)⁻.

### Intermediat C87

### 2-(Trimethylsilyl)ethyl-{13-[(R)-[1-benzyl-4-(2,5-difluorphenyl)-1H-pyrrol-2-yl](cyclohexyl)methyl]-1-(2,5-dioxo-2,5-dihydro-1H-pyrrol-1-yl)-2,7,12-trioxo-10-thia-3,6,13-triazahexadecan-16-yl}carbamat

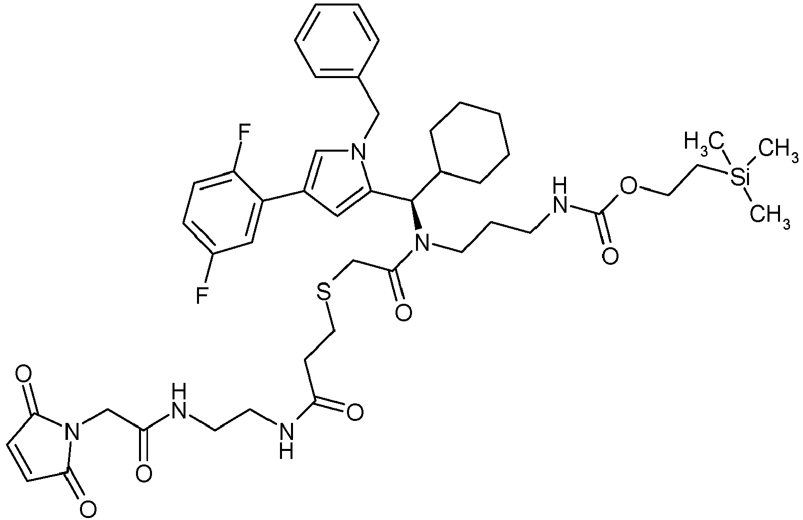

Zu einer Lösung von 100 mg (0.14 mmol) 11-[(R)-[1-Benzyl-4-(2,5-difluorphenyl)-1H-pyrrol-2-yl](cyclohexyl)methyl]-2,2-dimethyl-6,12-dioxo-5-oxa-14-thia-7,11-diaza-2-silaheptadecan-17-säure (Intermediat C86) in 1.37 ml DMF wurden 54.18 mg (0.28 mmol) N-(2-Aminoethyl)-2-(2,5-dioxo-2,5-dihydro-1H-pyrrol-1-yl)acetamid (Intermediat L1), 71.01 mg (0.50 mmol) N,N-diisopropylethylamin, 104.46 mg (0.27 mmol) HATU und 0.23 ml (0.14 mmol) 1-Hydoxy-7-Azabenzotriazole 0.5 M in DMF hinzugegeben. Das Reaktionsgemisch wurde 5 h bei RT gerührt. Die Mischung wurde ohne weitere Aufarbeitung mittels wurde mittels präparative HPLC gereinigt. Man erhielt 41 mg (33 % d. Th ) der Titelverbindung.

LC-MS (Methode 12): Rₜ = 2.61 min; MS (ESIpos): m/z = 907 (M+H)⁺.

### Intermediat C88

### tert-Butyl-3-[({(1R)-1-[1-benzyl-4-(2,5-difluorphenyl)-1H-pyrrol-2-yl]-2,2-dimethylpropyl} amino)methyl] pyrrolidin-1 -carboxylat-trifluoressigsäure (1:1)

### Mischung aus Stereoisomeren

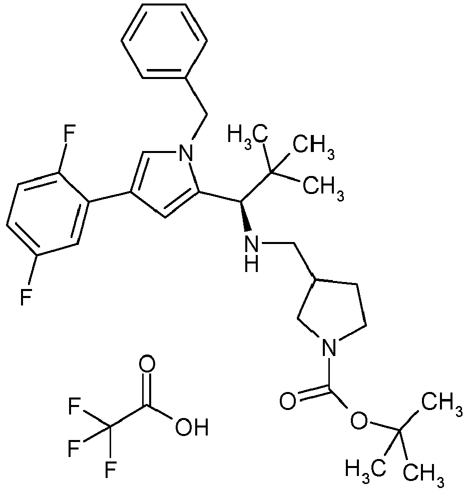

Eine Lösung von 2.04 g (5.75 mmol) (1R)-1-[1-Benzyl-4-(2,5-difluorphenyl)-1H-pyrrol-2-yl]-2,2-dimethylpropan-1-amin (Intermediat C52) in 51 ml Dichlormethan wurde mit 1.71 g (8.05 mmol) Natriumtriacetoxyborhydrid und 0.40 g (6.61 mmol) Essigsaüre versetzt und die Reaktionsgemisch wurde 5 Minuten bei RT gerührt. Anschließend wurde eine Lösung von 1.32 g (6.61 mmol) tert-Butyl-3-formylpyrrolidin-1-carboxylat in 20 ml Dichlormethan hinzugegeben und die Mischung wurde über Nacht bei RT gerührt. Die Reaktionsmischung wurde mit Ethylacetat verdünnt und die organische Phase wurde je zweimal mit ges. Natriumcarbonat-Lösung und mit ges. NaCl-Lösung gewaschen und über Magnesiumsulfat getrocknet und eingeengt. Der Rückstand wurde mittels präparative HPLC gereinigt. Die Lösemittel wurden im Vakuum verdampft und der Rückstand im Hochvakuum getrocknet. Man erhielt 1.86 g (50% d. Th.) der Titelverbindung.

LC-MS (Methode 1): Rₜ = 0.99 min; MS (ESIpos): m/z = 538 (M+H-CF₃CO₂H)⁺.

### Intermediat C89

### tert-Butyl-3-{[{(1R)-1-[1-benzyl-4-(2,5-difluorphenyl)-1H-pyrrol-2-yl]-2,2-dimethylpropyl}(chloracetyl)amino]methyl}pyrrolidin-1-carboxylat

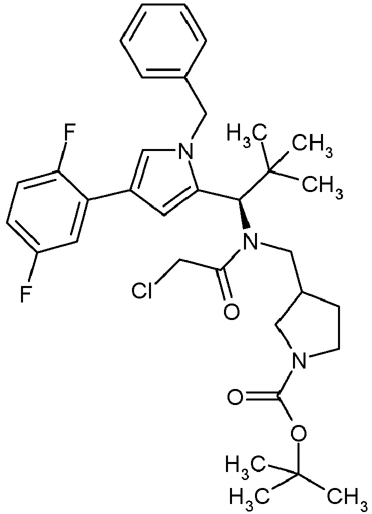

Eine Lösung von 2.89 g (4.19 mmol, 80 Reinheit) von tert-Butyl-3-[({(1R)-1-[1-benzyl-4-(2,5-difluorphenyl)-1H-pyrrol-2-yl]-2,2-dimethylpropyl}amino)methyl]pyrrolidin-1-carboxylat (Intermediat C88) in 42 ml Dichlormethan mit Molsieb 4 Ä wurde mit 1.36 g (13.42 mmol) Triethylamin und 2.13 g (18.87 mmol) Chloracetylchlorid versetzt. Das Reaktionsgemisch wurde bei RT 5 h gerührt. Die Mischung wurde einrotiert und der Rückstand wurde mittels präparative HPLC gereinigt. Man erhielt 449 mg (17 % d. Th.) von Isomere 1 und 442 mg (17 % d. Th) von Isomere 2 der Titelverbindung.

Isomer 1 LC-MS (Methode 1): Rₜ = 2.74 min; MS (ESIpos): m/z = 614 (M+H)⁺.

Isomer 2 LC-MS (Methode 1): Rₜ = 2.78 min; MS (ESIpos): m/z = 614 (M+H)⁺.

### Intermediat C90

### S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-L-cystein (Isomer 1)

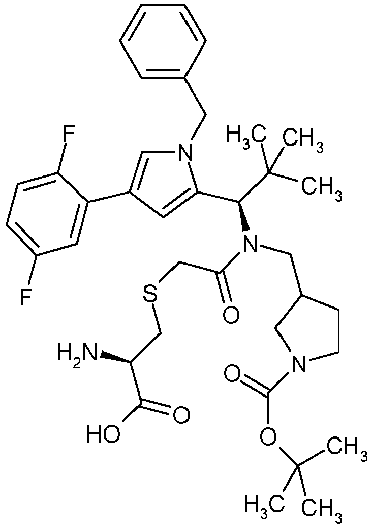

357.3 mg (0.58 mmol) L-Cystein wurden in 2.3 mL Wasser zusammen mit 488.7 mg (4.07 mmol) Natriumhydrogencarbonat suspendiert. Dazu wurden 357.0 mg (0.58 mmol tert-Butyl-3-{[{(1R)-1-[1-benzyl-4-(2,5-difluorphenyl)-1H-pyrrol-2-yl]-2,2-dimethylpropyl}(chloracetyl)amino]methyl}pyrrolidin-1-carboxylat (Isomer 1)

(Intermediat C89, Isomer 1) in 23.0 mL iso-Propanol gelöst und 1.06 g (6.98 mmol) 1,8-Diazabicyclo(5.4.0)undec-7-en gegeben. Die Reaktionsmischung wurde 3 h bei 50 °C gerührt. Die Reaktionsmischung wurde mit Ethylacetat versetzt und die org. Phase wurde mehrmals mit ges. Natriumhydrogencarbonat-Lösung und einmal mit ges. NaCl-Lösung gewaschen. Die organische Phase wurde über Magnesiumsulfat getrocknet und das Lösemittel im Vakuum verdampft. Der Rückstand wurde ohne Reinigung weiter eingesetzt. Man erhielt 255.0 mg (62 % d. Th.) der Titelverbindung.

LC-MS (Methode 1): Rₜ = 1.09 min; MS (ESIpos): m/z = 699 (M+H)⁺.

### Intermediat C91

### S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3 -yl]methyl} amino)-2-oxoethyl] -L-cystein (Isomer 2)

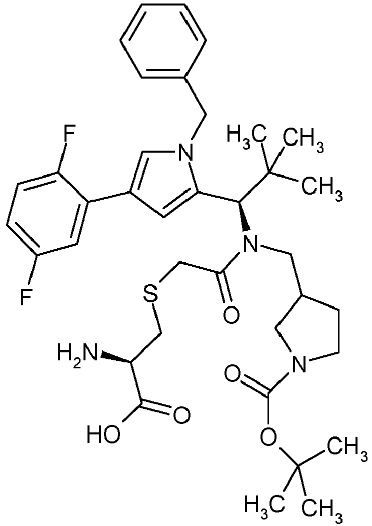

453.5 mg (3.74 mmol) L-Cystein wurden in 2.1 mL Wasser zusammen mit 449.2 mg (5.35 mmol) Natriumhydrogencarbonat suspendiert. Dazu wurden 3287.4 mg (0.54) mmol tert-Butyl-3-{[{(1R)-1-[1-benzyl-4-(2,5-difluorphenyl)-1H-pyrrol-2-yl]-2,2-dimethylpropyl}(chloracetyl)amino]methyl}pyrrolidin-1-carboxylat (Intermediat C89, Isomer 2) in 21.1 mL *iso*-Propanol gelöst und 0.98 g (6.42 mmol) 1,8-Diazabicyclo(5.4.0)undec-7-en gegeben. Die Reaktionsmischung wurde 3 h bei 50 °C gerührt. Die Reaktionsmischung wurde mit Ethylacetat versetzt und die org. Phase wurde mehrmals mit ges. Natriumhydrogencarbonat-Lösung und einmal mit ges. NaCl-Lösung gewaschen. Die organische Phase wurde über Magnesiumsulfat getrocknet und das Lösemittel im Vakuum verdampft. Der Rückstand wurde ohne Reinigung weiter eingesetzt. Man erhielt 221.0 mg (59 % d. Th.) der Titelverbindung.

LC-MS (Methode 1): Rₜ = 1.12 min; MS (ESIpos): m/z = 699 (M+H)⁺.

### Intermediat C92

### S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Isomer 1)

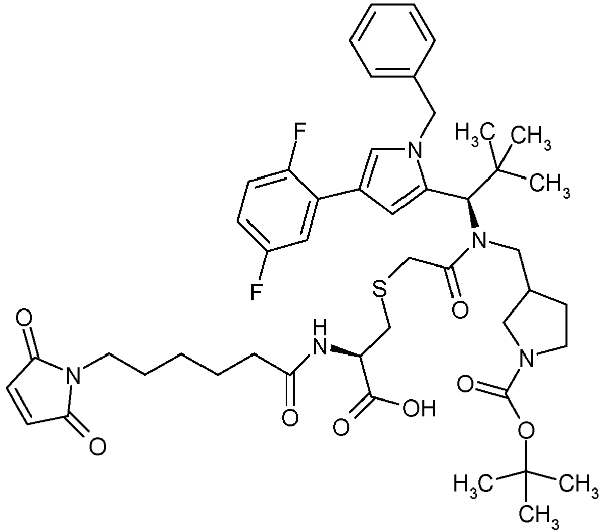

Eine Mischung von 50 mg ( 0.07 mmol) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-L-cystein (Intermediat C90) und 22.06 mg (0.07 mmol) 1-{6-[(2,5-Dioxopyrrolidin-1-yl)oxy]-6-oxohexyl}-1H-pyrrol-2,5-dion in 3.3 ml DMF wurde mit 18.49 mg (0.14 mmol) N,N-Diisopropylethylamin versetzt und das Reaktionsgemisch wurde bei RT 45 Minuten gerührt. Die Mischung wurde ohne Aufarbeitung mittels mittels präparative HPLC gereinigt. Man erhielt 65 mg (100 % d. Th, 71 % Reinheit) der Titelverbindung.

LC-MS (Methode 1): Rₜ = 1.31 min; MS (ESIpos): m/z = 892 (M+H)⁺.

### Intermediat C93

### S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Isomer 2)

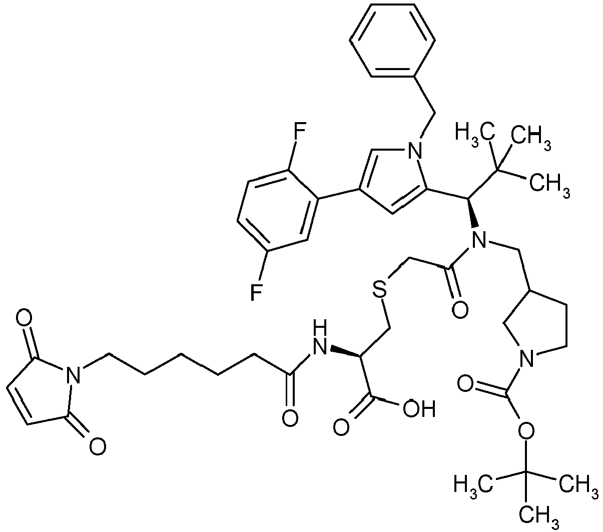

Eine Mischung von 50.0 mg ( 0.07 mmol) S-[2-({(1R)-1-[1-Benzyl-4-(2,5-difluorphenyl)-1H-pyrrol-2-yl]-2,2-dimethylpropyl} {[1-(tert-butoxycarbonyl)pyrrolidin-3-yl]methyl}amino)-2-oxoethyl]-L-cystein (Intermediat C91) und 22.06 mg (0.07 mmol) 1-{6-[(2,5-Dioxopyrrolidin-1-yl)oxy]-6-oxohexyl}-1H-pyrrol-2,5-dion in 3.0 ml DMF wurde mit 18.49 mg (0.14 mmol) N,N-Diisopropylethylamin versetzt und das Reaktionsgemisch wurde bei RT 90 Minuten gerührt. Die Mischung wurde ohne Aufarbeitung mittels präparative HPLC gereinigt. Man erhielt 63 mg (98 % d. Th, 73 % Reinheit)der Titelverbindung.

LC-MS (Methode 1): Rₜ = 1.34 min; MS (ESIpos): m/z = 892 (M+H)⁺.

### Intermediat C94

### S-[2-({(1R)-1-[1-Benzyl-4-(2,5-difluorphenyl)-1H-pyrrol-2-yl]-2,2-dimethylpropyl}{[(1-(tert-butoxycarbonyl)pyrrolidin-3-yl]methyl}amino)-2-oxoethyl]-N-[(2,5-dioxo-2,5-dihydro-1H-pyrrol-1-yl)acetyl]-L-cystein (Isomer 1)

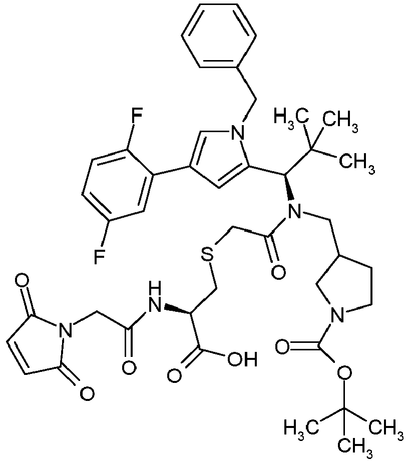

Eine Mischung von (50.0 mg, 0.07 mmol) S-[2-({(1R)-1-[1-Benzyl-4-(2,5-difluorphenyl)-1H-pyrrol-2-yl]-2,2-dimethylpropyl} {[(3R)-1-(tert-butoxycarbonyl)pyrrolidin-3 -yl]methyl} amino)-2-oxoethyl]-L-cystein (Intermediat C90) und 18.0 mg (0.07 mmol) -{2-[(2,5-Dioxopyrrolidin-1-yl)oxy]-2-oxoethyl}-1H-pyrrol-2,5-dion in 3.3 ml DMF wurde mit 18.5 mg (0.14 mmol) N,N-Diisopropylethylamin versetzt und das Reaktionsgemisch wurde 30 Minuten bei RT gerührt. Die Reaktionsmischung wurde mit Ethylacetat versetzt und die organische Phase wurde mehrmals mit ges. NH₄Cl-Lösung und mit ges. NaCl-Lösung gewaschen. Die organische Phase wurde über Magnesiumsulfat getrocknet und das Lösemittel im Vakuum verdampft. Der Rückstand wurde ohne weitere Reinigung eingesetzt. Man erhielt 57 mg (81 % d. Th, 85 % Reinheit) der Titelverbindung.

LC-MS (Methode 1): Rₜ = 0.96 min; MS (ESIpos): m/z = 836 (M+H)⁺.

### Intermediat C95

### 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3 -yl]methyl} amino)-2-oxoethyl] sulfanyl}propansäure (Isomer 1)

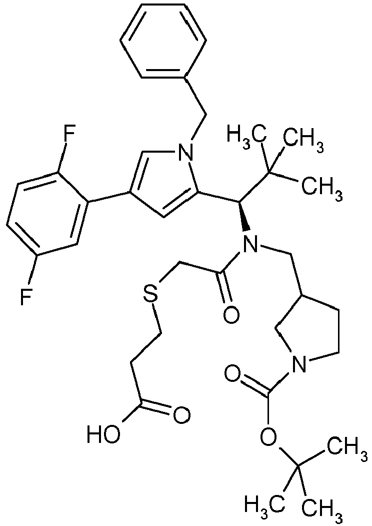

Eine Mischung von 384.0 mg (0.62 mmol) tert-Butyl-3-{[{(1R)-1-[1-benzyl-4-(2,5-difluorphenyl)-1H-pyrrol-2-yl]-2,2-dimethylpropyl } (chloracetyl)amino]methyl }pyrrolidin-1-carboxylat (Intermediat C89, Isomer 1) und 73.0 mg (0.69 mmol) 3-Sulfanylpropansäure in 14 ml Methanol und einige Tropfen Wasser wurde mit 302.5 mg (2.19 mmol) Kaliumcarbonat versetzt. Das Reaktionsgemisch wurde 2.5 h bei 50 °C gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne Aufarbeitung weiter eingesetzt. Man erhielt 358.0 mg (84 % d. Th.) der Titelverbindung.

LC-MS (Methode 1): Rₜ = 1.33 min; MS (ESIpos): m/z = 684 (M+H)⁺.

### Intermediat C96

### 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Isomer 2)

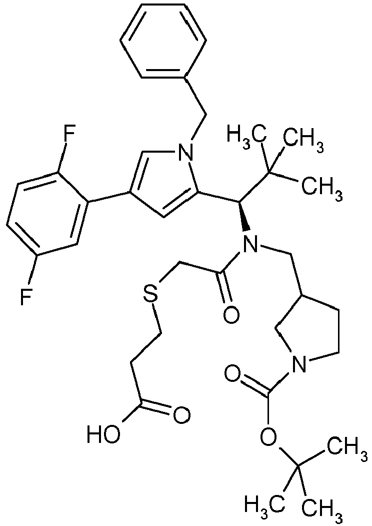

Eine Mischung von 287.0 mg (0.45 mmol) tert-Butyl-3-{[{(1R)-1-[1-benzyl-4-(2,5-difluorphenyl)-1H-pyrrol-2-yl]-2,2-dimethylpropyl } (chloracetyl)amino]methyl }pyrrolidin-1-carboxylat (Intermediat C89, Isomer 2) und 54.6 mg (0.51 mmol) 3-Sulfanylpropansäure in 14 ml Methanol und einige Tropfen Wasser wurde mit 226.0 mg (1.64 mmol) Kaliumcarbonat versetzt. Das Reaktionsgemisch wurde 2.5 h bei 50 °C gerührt. Die Reaktionsmischung wurde mit Ethylacetat versetzt und die org. Phase wurde mehrmals mit Wasser und mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne Aufarbeitung weiter eingesetzt. Man erhielt 318.7 mg (88 % d. Th., 88 % Reinheit) der Titelverbindung.

LC-MS (Methode 1): Rₜ = 1.36 min; MS (ESIpos): m/z = 684 (M+H)⁺.

### Intermediat C97

### tert-Butyl-3-[2-{(1R)-1-[1-benzyl-4-(2,5-difluorphenyl)-1H-pyrrol-2-yl]-2,2-dimethylpropyl}-14-(2,5-dioxo-2,5-dihydro-1H-pyrrol-1-yl)-3,8,13-trioxo-5-thia-2,9,12-triazatetradec-1-yl]pyrrolidin-1-carboxylat (Isomer 2)

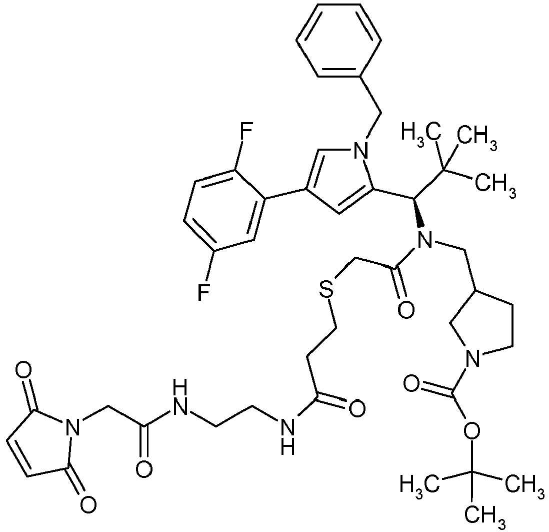

Zu einer Lösung von 25.0 mg (0.04 mmol) von 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Intermediat C96) in 2.81 ml DMF unter Argon wurden 14.17 mg (0.11 mmol) N,N-diisoprylethylamin und 27.80 mg (0.07 mmol) HATU hinzugegeben. Das Reaktionsgemisch wurde 10 Minuten bei RT gerührt. Anschließend wurde eine Lösung von 22.75 mg (0.07 mmol) N-(2-Aminoethyl)-2-(2,5-dioxo-2,5-dihydro-1H-pyrrol-1-yl)acetamid -ethan (1:1) Trifluoressigsäure (Intermediat L1) in 1.4 ml DMF und 5 mg (0.04 mmol) N,N-diisopropylethylamin hinzugegeben und das Gemisch wurde ü.N bei RT gerührt. Die Mischung wurde

LC-MS (Methode 5): Rₜ = 4.39 min; MS (ESIpos): m/z = 86 mit Wasser versetzt und mit Dichlormethan extrahiert. Die organische Phase wurde über Magnesiumsulfat getrocknet und das Lösemittel im Vakuum verdampft. Der Rückstand wurde ohne Aufarbeitung weiter eingesetzt. Man erhielt 26 mg (84 % d. Th.) der Titelverbindung.3 (M+H)⁺.

### Intermediat C98

### tert-Butyl-3-[2-{(1R)-1-[1-benzyl-4-(2,5-difluorphenyl)-1H-pyrrol-2-yl]-2,2-dimethylpropyl}-18-(2,5-dioxo-2,5-dihydro-1H-pyrrol-1-yl)-3,8,13-trioxo-5-thia-2,9,12-triazaoctadec-1-yl]pyrrolidin-1-carboxylat (Isomer 2)

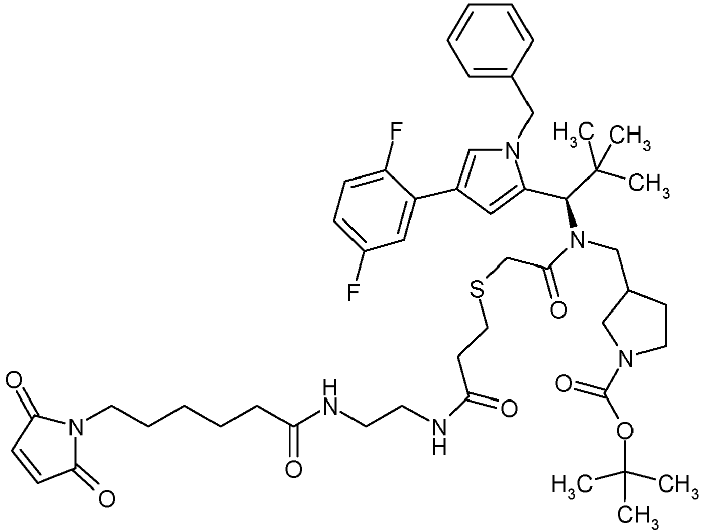

Zu einer Lösung von 25.0 mg (0.04 mmol) von 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Intermediat C96) in 2.81 ml DMF unter Argon wurden 14.17 mg (0.11 mmol) N,N-diisoprylethylamin und 27.80 mg (0.07 mmol) HATU hinzugegeben. Das Reaktionsgemisch wurde 10 Minuten bei RT gerührt. Anschließend wurde eine Lösung von 37.30 mg (0.07 mmol) N-(2-Aminoethyl)-6-(2,5-dioxo-2,5-dihydro-1H-pyrrol-1-yl)hexanamid -ethan (1:1) Trifluoressigsäure in 1.4 ml DMF und 5 mg (0.04 mmol) N,N-diisopropylethylamin hinzugegeben und das Gemisch wurde üN bei RT gerührt. Die Mischung wurde mit Wasser versetzt und mit Dichlormethan extrahiert. Die organische Phase wurde über Magnesiumsulfat getrocknet und das Lösemittel im Vakuum verdampft. Der Rückstand wurde ohne weitere Reinigung eingesetzt. Man erhielt 22 mg (63 % d. Th.) der Titelverbindung.

LC-MS (Methode 5): Rₜ = 4.54 min; MS (ESIpos): m/z = 919 (M+H)⁺.

### Intermediat C99

### tert-Butyl-3-[2-{(1R)-1-[1-benzyl-4-(2,5-difluorphenyl)-1H-pyrrol-2-yl]-2,2-dimethylpropyl}-24-(2,5-dioxo-2,5-dihydro-1H-pyrrol-1-yl)-3,8,19-trioxo-12,15-dioxa-5-thia-2,9,18-triazatetracos-1-yl]pyrrolidin-1-carboxylat (Isomer 2)

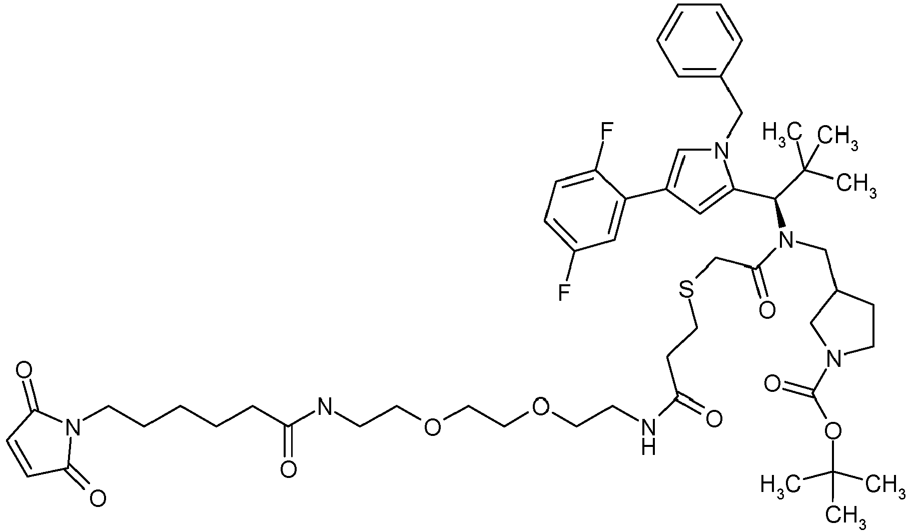

Zu einer Lösung von 25.0 mg (0.04 mmol) von 3-{[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]sulfanyl}propansäure (Intermediat C96) in 2.81 ml DMF unter Argon wurden 14.17 mg (0.11 mmol) N,N-diisoprylethylamin und 27.80 mg (0.07 mmol) HATU hinzugegeben. Das Reaktionsgemisch wurde 10 Minuten bei RT gerührt. Anschließend wurde eine Lösung von 35.05 mg (0.07 mmol) N-{2-[2-(2-Aminoethoxy)ethoxy]ethyl}-6-(2,5-dioxo-2,5-dihydro-1H-pyrrol-1-yl)hexanamid -ethan (1: 1)Trifluoressigsäure (Intermediat L82) in 1.4 ml DMF und 5 mg (0.04 mmol) N,N-diisopropylethylamin hinzugegeben und das Gemisch wurde üN bei RT gerührt. Die Mischung wurde mit Wasser versetzt und mit Dichlormethan extrahiert. Die organische Phase wurde über Magnesiumsulfat getrocknet und das Lösemittel im Vakuum verdampft und der Rückstand im Hochvakuum getrocknet. Der Rückstand wurdemittels präp. HPLC gereinigt Man erhielt 25 mg (60 % d. Th.) der Titelverbindung.

LC-MS (Methode 1): Rₜ = 4.52 min; MS (ESIpos): m/z = 1007 (M+H)⁺.

### Intermediat C100

### 2-(Trimethylsilyl)ethyl-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2R)-2-(2,5-dioxo-2,5-dihydro-1H-pyrrol-1-yl)propanoyl]amino}ethyl)amino}-1-oxobutan-2-yl}carbamat

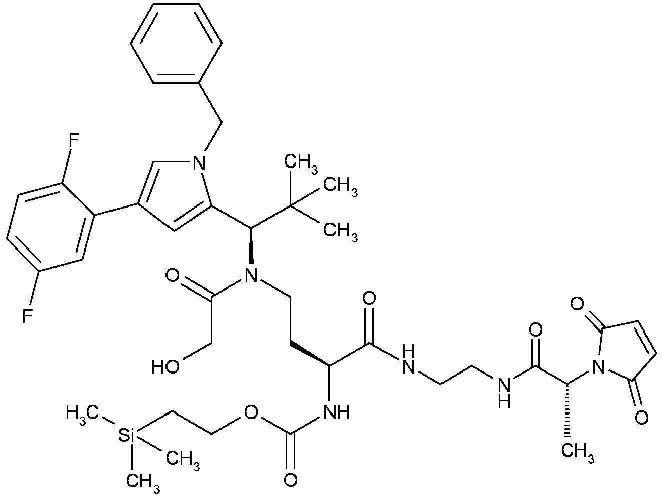

Zu einer Lösung von 45 mg (0.068 mmol) (2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butansäure (Intermediat C58) in 5.8 ml DMF wurden 22.2 mg (0.068 mmol) (2R)-N-(2-aminoethyl)-2-(2,5-dioxo-2,5-dihydro-1H-pyrrol-1-yl)propanamid (1:1) Trifluoressigsäure zugegeben. Nach 30 Minuten Rühren bei RT die Mischung wurde mit 39 mg (0.10 mmol) HATU und 36 mg (0.27 mmol) N,N-Diisopropylethylamin versetzt. Das Reaktionsgemisch wurde 1 h bei RT gerührt. Das Gemisch wurde ohne Aufarbeitung mittels präparative HPLC gereinigt. Man erhielt 7 mg (12 % d. Th) der Titelverbindung.

LC-MS (Methode 1): Rₜ = 1.41 min; MS (ESIpos): m/z 851 (M+H)⁺.

### Intermediat C101

### Trifluoressigsäure -methyl-(2S)-4-[(acetoxyacetyl){(1R)-1-[1-benzyl-4-(2,5-difluorphenyl)-1H-pyrrol-2-yl]-2,2-dimethylpropyl}amino]-2-aminobutanoat (1:1)

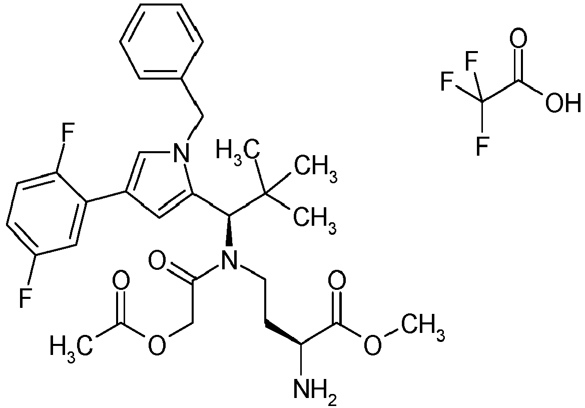

4.3 g (12.2 mmol) von Intermediat C52 wurden in 525 mL DCM gelöst und mit 3.63 g (17.12 mmol) Natriumtriacetoxyborhydrid sowie mit 8.4 mL Essigsäure versetzt. Nach 5 min Rühren bei RT wurden 3.23 g (11.85 mmol) Methyl-(2S)-4-oxo-2-({[2-(trimethylsilyl)ethoxy]carbonyl} amino)butanoat (hergestellt aus (3S)-3-Amino-4-methoxy-4-oxobutansäure nach klassischen Methoden) gelöst in 175 mL DCM zugegeben und der Ansatz weitere 45 min bei RT gerührt. Der Ansatz wurde dann mit DCM verdünnt und zweimal mit 100 mL gesättigter Natriumhydrogencarbonatlösung und dann mit gesättigter Natriumchloridlösung ausgeschüttelt. Die organische Phase wurde über Magnesiumsulfat getrocknet, filtriert und anschließend eingeengt. Der Rückstand wurde mittels präparativer HPLC aufgereinigt. Nach Vereinigung der entsprechenden Fraktionen, Einengen und Trocknen des Rückstandes im Hochvakuum erhielt man 4.6 g (61% d. Th.) des Intermediats.

LC-MS (Methode 12): Rₜ = 1.97 min; MS (ESIpos): m/z = 614.32 (M+H)⁺.

2.06 g (3.36 mmol) von diesem Intermediat wurden in 76 mL DCM vorgelegt und mit 0.81 mL (7.17 mmol) 2-Chlor-2-oxoethylacetat in Gegenwart von 2.1 ml Triethylamin acyliert. Nach 20 h Rühren bei RT wurden weitere 0.36 mL 2-Chlor-2-oxoethylacetat und 0.94 ml Triethylamin zugegeben und der Ansatz weitere 15 min bei RT gerührt. Anschließend wurde mit 500 mL Ethylacetat verdünnt und nacheinander zweimal mit 300 mL 5%-iger Zitronensäure, zweimal mit 300 mL gesättigter Natriumhydrogencarbonatlösung und einmal mit 100 mL gesättigter Natriumchloridlösung ausgeschüttelt, dann über Magnesiumsulfat getrocknet und eingeengt. Nach Trocknung im Hochvakuum wurden 2.17 g (79% d. Th.) des geschützten Intermediats erhalten.

LC-MS (Methode 1): Rₜ = 1.48 min; MS (ESIpos): m/z = 714 (M+H)⁺.

321 mg (0.342 mmol) von diesem Intermediat wurde in 7 mL 2,2,2-Trifluorethanol gelöst. Es wurden 279.5 mg (2.05 mmol) Zinkchlorid zugegeben und der Ansatz 2 h bei 50°C gerührt. Anschließend wurden 599 mg (2.05 mmol) Ethylendiamin-N,N,N',N'-tetraessigsäure und 2 mL einer 0.1%igen Trifluoressigsäurelösung in Wasser hinzugegeben und der Ansatz anschließend im Vakuum eingeengt. Der Rückstand wurde mittels präparativer HPLC gereinigt. Nach Einengen der entsprechenden Fraktionen und Lyophilisation des Rückstands aus Acetonitril/Wasser wurden 60 mg (26% d.Th.) der Titelverbindung erhalten, die zum Teil noch einen Teil der de-acetylierten Verbindung enthielt.

LC-MS (Methode 1): Rₜ = 0.91 min und 0.95 min; MS (ESIpos): m/z = 528 und 570 (M+H)⁺.

### Intermediat C102

### (2S)-4-[{(1R)-1-[1-Benzyl-4-(2,5-difluorphenyl)-1H-pyrrol-2-yl]-2,2-dimethylpropyl} (glycoloyl)amino] -2- { [(benzyloxy)carbonyl] amino }butansäure

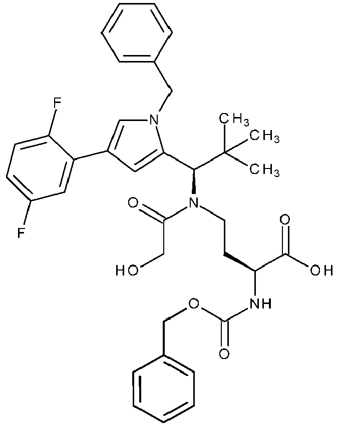

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat acyliert und schließlich mit 2M Lithiumhydroxidlösung in Methanol die Hydrolyse der beiden Estergruppen durchgeführt.

LC-MS (Methode 1): Rₜ = 1.31 min; MS (ESIpos): m/z = 646 (M-H)⁻.

### Intermediat C103

### 2-(Trimethylsilyl)ethyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-N2-{[2-(trimethylsilyl) ethoxy] carbonyl } -L-glutaminat

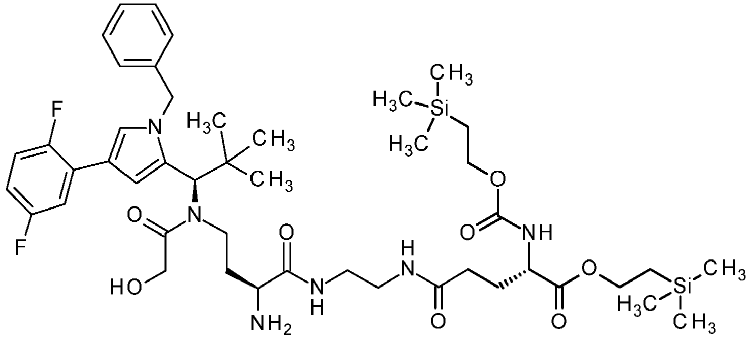

Die Titelverbindung wurde zunächst durch Kupplung von 151 mg (0.23 mmol) Intermediat C102 mit 128 mg (0.234 mmol) Intermediat L98 in DMF in Gegenwart von HATU und N,N-Diisopropylethylamin hergestellt. Anschließend wurde die Z-Schutzgruppe durch 30-minütige Hydrierung über 10%-igem Palladium auf Aktivkohle bei RT unter Wasserstoff-Normaldruck entfernt, wobei die Titelverbindung erhalten wurde.

Ausb.: 30% d. Th. über 2 Stufen

LC-MS (Methode 1): Rₜ = 1.14 min; MS (ESIpos): m/z = 929 (M+H)⁺.

### Intermediat C104

### 2-(Trimethylsilyl)ethyl-(3R,4R)-3-[({(1R)-1-[1-benzyl-4-(2,5-difluorphenyl)-1H-pyrrol-2-yl]-2,2-dimethylpropyl}amino)methyl]-4-fluorpyrrolidin-1-carboxylat

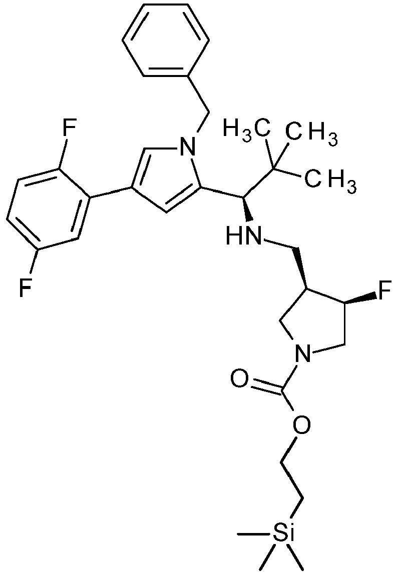

Eine Lösung von 2.24 g (6.31 mmol) (1R)-1-[1-Benzyl-4-(2,5-difluorphenyl)-1H-pyrrol-2-yl]-2,2-dimethylpropan-1-amin in 56.0 ml Dichlormethan mit Mol Sieb 4 Ä wurde mit 1.87 g (8.84 mmol) Natriumtriacetoxyborhydrid versetz, und das Gemisch wurde 15 Minuten bei Raumtemperatur gerührt. Anschließend wurden 2.20 g (7.58 mmol) 2-(Trimethylsilyl)ethyl-(3R,4S)-3-fluor-4-formylpyrrolidin-1-carboxylat (Ref: WO 2014/151030A1) zugegeben, und das Reaktionsgemisch wurde 3.5 h bei Raumtemperatur gerührt. Das Gemisch wurde mit Dichlormethan verdünnt und die organische Phase wurde mit ges. Natriumhydrogencarbonat-Lösung und Wasser gewaschen. Die organische Phase wurde über Natriumsulfat getrocknet und eingeengt. Der Rückstand wurdemittels präp. HPLC gereinigt. Man erhielt 1.39 g (24 % d. Th.) der Titelverbindung.

LC-MS (Methode 1): Rₜ = 1.15 min; MS (ESIpos): m/z = 600 (M+H)⁺.

### Intermediat C105

### 2-(Trimethylsilyl)ethyl-(3R,4R)-3-{[{(1R)-1-[1-benzyl-4-(2,5-difluorphenyl)-1H-pyrrol-2-yl]-2,2-dimethylpropyl}(chloracetyl)amino]methyl}-4-fluorpyrrolidin-1-carboxylat

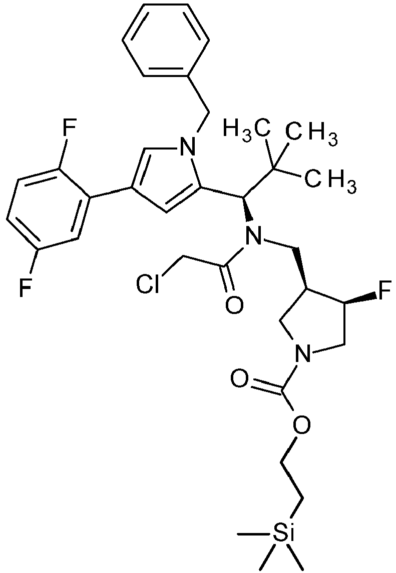

Zur eine Lösung von 692.8 mg (0.88 mmol) 2-(Trimethylsilyl)ethyl-(3R,4R)-3-[({(1R)-1-[1-benzyl-4-(2,5-difluorphenyl)-1H-pyrrol-2-yl]-2,2-dimethylpropyl}amino)methyl]-4-fluorpyrrolidin-1-carboxylat (Intermediat C104) in 8.7 ml Dichlormethan mit Mol Sieb 4 Ä wurden 295.0 mg (2.91 mmol) Triethylamin und 418.9 mg (3.71 mmol) Chloracetylchlorid zugegeben und das Reakionsgemisch wurde 2.5 h bei RT gerührt. Die Reaktionsmischung wurde mit Dichlormethan verdünnt und die organische Phase wurde mit ges. Natriumhydrogencarbonat-Lösung und ges. Ammoniumchlorid-Lösung gewaschen. Die organische Phase wurde über Natriumsulfat getrocknet und eingeengt. Der Rückstand wurde nochmal in 8.7 ml Dichlormethan mit Mol Sieb 4 Ä gelöst und 295.0 mg (2.91 mmol) Triethylamin und 418.9 mg (3.71 mmol) Chloracetylchlorid wurden zugegeben und das Reakionsgemisch wurde 3 h bei RT gerührt. Die Reaktionsmischung wurde mit Dichlormethan verdünnt und die organische Phase wurde mit ges. Natriumhydrogencarbonat-Lösung und ges. Ammoniumchlorid-Lösung gewaschen. Die organische Phase wurde über Natriumsulfat getrocknet und eingeengt. Die organische Phase wurde über Natriumsulfat getrocknet, eingeengt und ohne Reinigung weiter eingesetzt. Man erhielt 691 mg (74 % d. Th., 64% Reinheit) der Titelverbindung.

LC-MS (Methode 1): Rₜ = 1.78 min; MS (ESIpos): m/z = 676 (M+H)⁺.

### Intermediat C106

### 3-{[2-({(1R)-1-[1-Benzyl-4-(2,5-difluorphenyl)-1H-pyrrol-2-yl]-2,2-dimethylpropyl}{[(3R,4R)-4-fluor-1-{[2-(trimethylsilyl)ethoxy]carbonyl}pyrrolidin-3-yl]methyl}amino)-2-oxoethyl]sulfanyl}propansäure

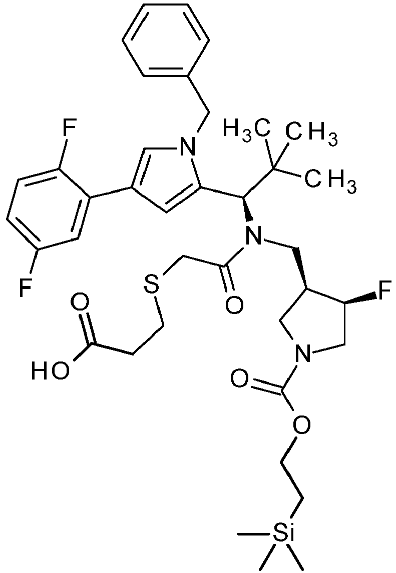

Eine Mischung von 691.0 mg (0.65 mmol) 2-(Trimethylsilyl)ethyl-(3R,4R)-3-{[{(1R)-1-[1-benzyl-4-(2,5-difluorphenyl)-1H-pyrrol-2-yl]-2,2-dimethylpropyl}(chloracetyl)amino]methyl}-4-fluorpyrrolidin-1-carboxylat (Intermediat C105) und 76.3 mg (0.72 mmol) 3-Sulfanylpropansäure in 15 ml Methanol und einige Tropfen Wasser wurde mit 316 mg (2.29 mmol) Kaliumcarbonat versetzt. Das Reaktionsgemisch wurde 1.5 h bei 50 °C gerührt. Die Reaktionsmischung wurde mit Ethylacetat versetzt und die org. Phase wurde mehrmals mit Wasser und mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne Aufarbeitung weiter eingesetzt. Man erhielt 502 mg (67 % d. Th., 65% Reinheit) der Titelverbindung.

LC-MS (Methode 1): Rₜ = 1.48 min; MS (ESIneg): m/z = 744 (M-H)⁻.

### Intermediat C107

### S-[2-({(1R)-1-[1-Benzyl-4-(2,5-difluorphenyl)-1H-pyrrol-2-yl]-2,2-dimethylpropyl}{[(3R,4R)-4-fluor-1 - { [2-(trimethylsilyl)ethoxy]carbonyl }pyrrolidin-3 -yl]methyl} amino)-2-oxoethyl] -L-cystein

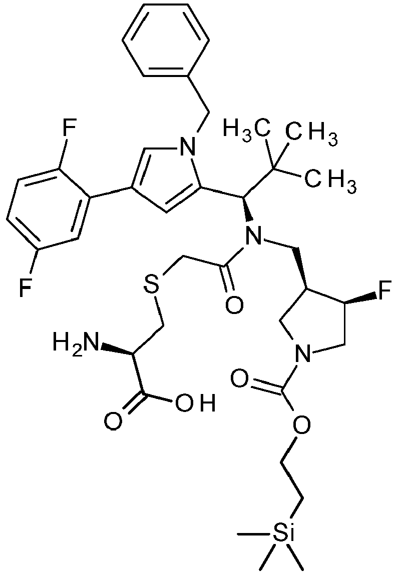

203.6 mg (1.68 mmol) L-Cystein wurden in 0.95 mL Wasser zusammen mit 201.7 mg (2.40 mmol) Natriumhydrogencarbonat suspendiert. Dazu wurden 170.0 mg (0.24 mmol) 2-(Trimethylsilyl)ethyl-(3R,4R)-3-{[{(1R)-1-[1-benzyl-4-(2,5-difluorphenyl)-1H-pyrrol-2-yl]-2,2-dimethylpropyl}(chloracetyl)amino]methyl}-4-fluorpyrrolidin-1-carboxylat (Intermediat 105) gelöst in 9.5 mL iso-Propanol und 438.5 mg (2.40 mmol) 1,8-Diazabicyclo(5.4.0)undec-7-en gegeben. Die Reaktionsmischung wurde 3 h bei 50 °C gerührt. Das Gemisch wurde mit Ethylacetat versetzt und die org. Phase wurde mehrmals mit ges. Natriumhydrogencarbonat-Lösung und mit ges. NaCl-Lösung gewaschen. Die organische Phase wurde über Natriumsulfat getrocknet und das Lösemittel im Vakuum verdampft. Der Rückstand wurde ohne weitere Reinigung weiter eingesetzt. Man erhielt 152 mg (83 % d. Th.) der Titelverbindung.

LC-MS (Methode 1): Rₜ = 1.26 min; MS (ESIpos): m/z = 762 (M+H)⁺.

### Intermediat C108

### 2-(Trimethylsilyl)ethyl N⁶-(N-{(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}-beta-alanyl)-N²-{[2-(trimethylsilyl)ethoxy] carbonyl} -L-ly sinat

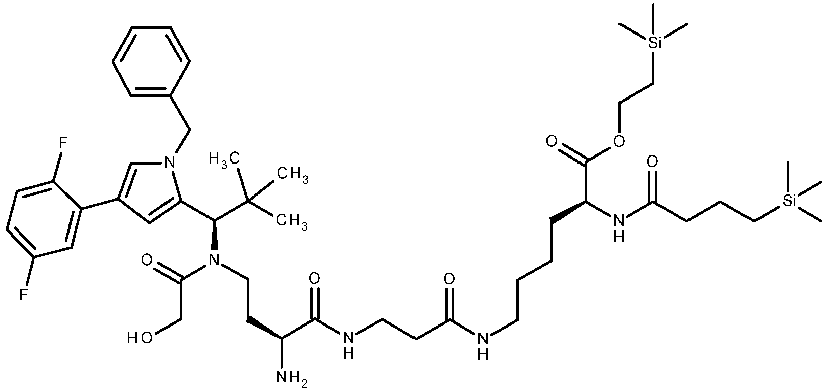

Die Titelverbindung wurde durch Kupplung von 103 mg (0.16 mmol) Intermediat C102 mit 110 mg (0.175 mmol) 2-(Trimethylsilyl)ethyl N⁶-beta-alanyl-N²-{[2-(trimethylsilyl)ethoxy]carbonyl}-L-lysinat in DMF in Gegenwart von EDCI, HOBT und N,N-Diisopropylethylamin hergestellt. Anschließend wurde die Z-Schutzgruppe durch 1-stündige Hydrierung über 10%-igem Palladium auf Aktivkohle in Dichlormethan/Methanol 1:1 bei RT unter Wasserstoff-Normaldruck entfernt, wobei die Titelverbindung in einer Ausbeute von 113 mg (75% d. Th. über 2 Stufen) erhalten wurde.

LC-MS (Methode 1): Rₜ = 1.17 min; MS (ESIpos): m/z = 957 (M+H)⁺.

Die hier eingesetzte Zwischenstufe wurde nach klassischen Methoden der Peptidchemie durch Kupplung von kommerziell erhältlichem N-(tert-Butoxycarbonyl)-beta-alanin und 2-(Trimethylsilyl)ethyl N²-[(benzyloxy)carbonyl]-L-lysinat in Gegenwart von HATU, hydrogenolytischer Abspaltung der Z-Schutzgruppe, Einführung der Trimethylsilyl-ethyloxy carbonyl(Teoc)-Schutzgruppe mit 1-({[2-(trimethylsilyl)ethoxy]carbonyl}oxy)pyrrolidine-2,5-dion und schließlich schonender Abspaltung der Boc-Schutzgruppe durch 45-minütiges Rühren in einer 7.5%-igen Trifluoressigsäurelösung in Dichlormethan hergestellt.

LC-MS (Methode 1): Rₜ = 0.83 min; MS (ESIpos): m/z = 462 (M+H)⁺.

### Intermediat C109

### Di-tert-butyl-N-{(2S)-2-amino-4-[{(1R)-1-[1-benzyl-4-(2,5-difluorphenyl)-1H-pyrrol-2-yl]-2,2-dimethylpropyl } (glycoloyl)amino]butanoyl } -beta-alanyl-L-glutamat

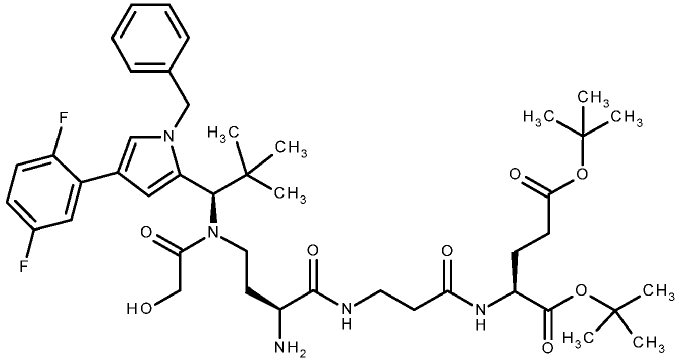

Zunächst wurde das Dipeptidderivat Di-tert-butyl-beta-alanyl-L-glutamat nach klassischen Methoden der Peptidchemie durch Kupplung von kommerziell erhältlichen N-[(Benzyloxy)carbonyl]-beta-alanin und Di-tert-butyl L-glutamat Hydrochlorid (1:1) in Gegenwart von HATU und anschließender hydrogenolytischer Abspaltung der Z-Schutzgruppe hergestellt. Die Titelverbindung wurde dann durch Kupplung dieses Intermediats mit Intermediat C102 in Gegenwart von HATU und N,N-Diisopropylethylamin und anschließender Abspaltung der Z-Schutzgruppe durch 45-minütige Hydrierung über 10%-igem Palladium auf Aktivkohle in Methanol bei RT unter Wasserstoff-Normaldruck hergestellt.

LC-MS (Methode 1): Rₜ = 0.99 min; MS (ESIpos): m/z = 826 [M+H]⁺.

### Intermediat C110

### Dibenzyl-N-{(2S)-2-amino-4-[{(1R)-1-[1-benzyl-4-(2,5-difluorphenyl)-1H-pyrrol-2-yl]-2,2-dimethylpropyl } (glycoloyl)amino]butanoyl } -beta-alanyl-L-glutamat

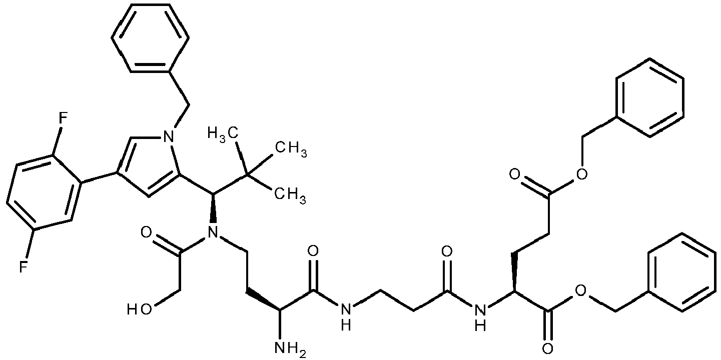

Die Titelverbindung wurde durch Kupplung von Dibenzyl-L-glutamat, das zuvor durch Verteilung zwischen Ethylacetat und 5%-iger Natriumhydrogencarbonatlösung aus seinem p-Toluolsulfon säure-Salz freigesetzt wurde mit Intermediat C61 in Gegenwart von HATU und N,N-Diisopropylethylamin und anschließender Abspaltung der Teoc-Schutzgruppe mittels Zinkchlorid in Trifluorethanol hergestellt.

LC-MS (Methode 1): Rₜ = 1.09 min; MS (ESIpos): m/z = 894 [M+H]⁺.

### Intermediat C111

### Di-tert-butyl-N-{(2S)-2-amino-4-[{(1R)-1-[1-benzyl-4-(2,5-difluorphenyl)-1H-pyrrol-2-yl]-2,2-dimethylpropyl } (glycoloyl)amino]butanoyl } -beta-alanyl-D-glutamat

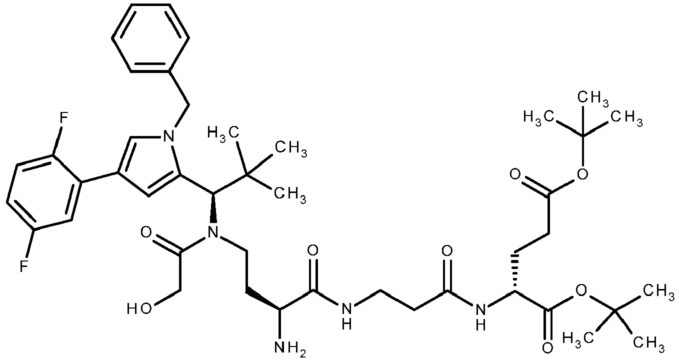

Die Synthese der Titelverbindung erfolfte in Analogie zu Intermediat C109.

LC-MS (Methode 1): Rₜ = 1.06 min; MS (ESIpos): m/z = 826 [M+H]⁺.

### Intermediat C112

### N²-Acetyl-N-[2-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)ethyl]-N⁶-(tert-butoxycarbonyl)-L-lysinamid

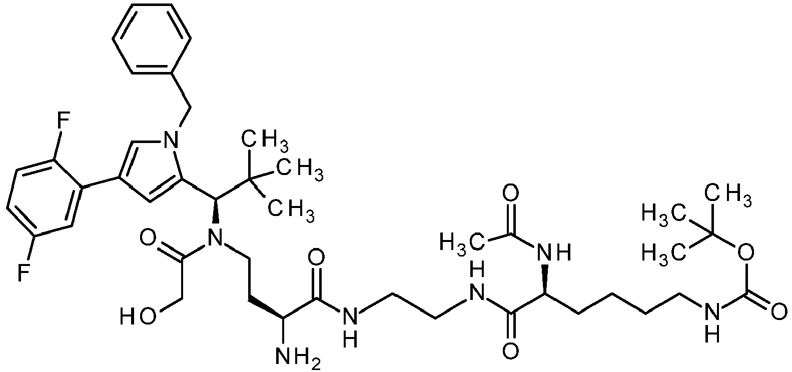

Die Titelverbindung wurde durch HATU-Kupplung von Intermediat C102 und Intermediat L108 in DMF in Gegenwart von N,N-Diisopropylethylamin und anschließender Abspaltung der Z-Schutzgruppe durch Hydrierung in DCM/Methanol 1: 1 über 10% Palladium auf Aktivkohle unter Normaldruck hergestellt.

LC-MS (Methode 1): Rₜ = 0.96 min; MS (ESIpos): m/z = 826 (M+H)⁺.

### Intermediat C113

### Trifluoressigsäure -benzyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-3-{[(benzyloxy)carbonyl]amino}-D-alaninat (1:1)

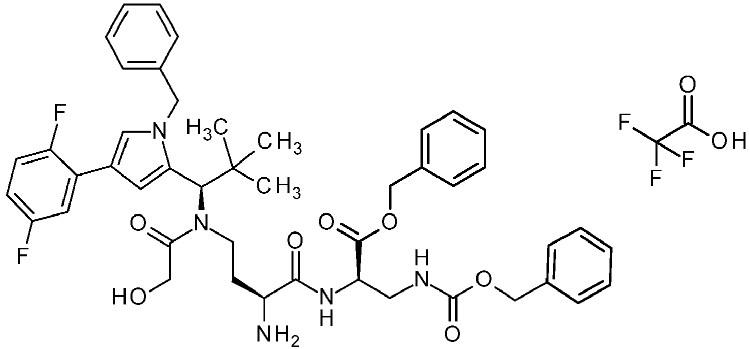

Zunächst wurde Trifluoressigsäure -benzyl-3-{[(benzyloxy)carbonyl]amino}-D-alaninat (1:1) ausgehend von kommerziell erhältlichem 3-{[(Benzyloxy)carbonyl]amino}-N-(tert-butoxycarbonyl)-D-alanin durch Veresterung mit Benzylalkohol in Gegenwart von EDC/DMAP und anschließender Abspaltung der Boc-Schutzgruppe mit Trifluoressigsäure hergestellt. Dieser Aminosäurebaustein wurde dann mit Intermediat C58 in Gegenwart von HATU und N,N-Diisopropylethylamin in DMF gekuppelt. Im letzten Schritt wurde durch 2-stündiges Rühren bei 50°C in Trifluorethanol mit 6 Equivalenten Zinkchlorid und Reinigung durch präparative HPLC die Titelverbindung erhalten.

LC-MS (Methode 1): Rₜ = 1.05 min; MS (ESIpos): m/z = 824 [M+H]⁺.

### Intermediat C114

### Trifluoressigsäure --tert-butyl-4-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)butanoat (1:1)

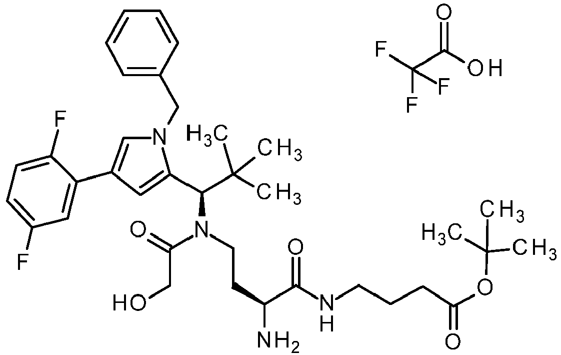

Zunächst wurde Intermediat C102 mit tert-Butyl-4-aminobutanoathydrochlorid (1:1) in Gegenwart von HATU und N,N-Diisopropylethylamin gekuppelt. Anschließend wurde durch 1-stündige Hydrierung über 10%-igem Palladium auf Aktivkohle in DCM-Methanol 1:1 bei RT unter Wasserstoff-Normaldruck die Titelverbindung erhalten.

LC-MS (Methode 1): Rₜ = 1.0 min; MS (ESIpos): m/z = 655 [M+H]⁺.

### Intermediat C115

### Trifluoressigsäure (2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1)

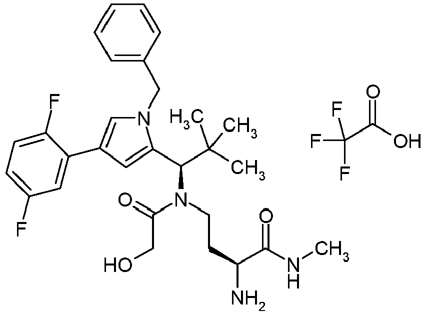

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat wie in Intermediat C27 beschrieben acyliert.

190 mg (0.244 mmol) von dieser Zwischenstufe wurden in 7.5 ml Ethanol aufgenommen und mit 0.35 mL einer 40%-igen Methanaminlösung in Wasser versetzt. Der Ansatz wurde 3 h bei 50°C gerührt und dann wurde nochmals die gleiche Menge Methanamin zugegeben. Nach weiteren 5 h Rühren bei 50°C wurde der Ansatz eingeengt und der Rückstand durch präparative HPLC gereinigt. Es wurden 78 mg (48% d.Th.) dieser Zwischenstufe erhaltenen.

LC-MS (Methode 1): Rₜ = 1.32 min; MS (EIpos): m/z = 661 [M+H]⁺.

78 mg (0.118 mmol) dieser Zwischenstufe wurden in 8 mL Ethanol gelöst und nach Zugabe von 15 mg 10%-igem Palladium auf Aktivkohle 3 h lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser wurden 33 mg (44% d.Th.) der Titelverbindung erhalten.

LC-MS (Methode 1): Rₜ = 0.88 min; MS (ESIpos): m/z = 527 (M+H)⁺.

¹H-NMR (500 MHz, DMSO-d₆): δ = 8.1 (m, 1H), 8.0 (m, 3H), 7.9 (m, 1H), 7.65 (m, 1H), 7.5 (s, 1H), 7.15-7.35 (m, 5H) 7.0 (m, 1H), 6.85 (m, 1H), 5.6 (s, 1H), 4.9 und 5.2 (2d, 2H), 4.02 und 4.22 (2d, 2H), 3.2-3.5 (m, 6H), 0.7 und 1.46 (2m, 2H), 0.8 (s, 9H).

### Intermediat C116

### Trifluoressigsäure N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid (1:1)

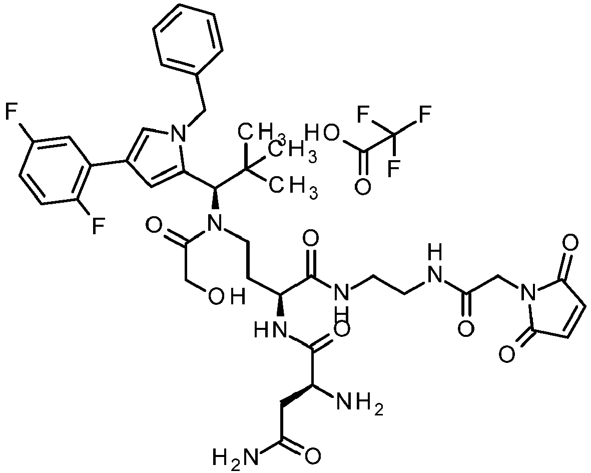

Trifluoressigsäure (2S)-2-amino-4-[{(1R)-1-[1-benzyl-4-(2,5-difluorphenyl)-1H-pyrrol-2-yl]-2,2-dimethylpropyl} (glycoloyl)amino] -N-(2- { [(2,5-dioxo-2,5-dihydro-1H-pyrrol-1-yl)acetyl]amino}ethyl)butanamid (1:1) (81.0 mg, 100 µmol) (Intermediat F104) und 2,5-Dioxopyrrolidin-1-yl-N²-(tert-butoxycarbonyl)-L-asparaginat (43.0 mg, 131 µmol) wurden in 5.0 ml DMF gelöst. Die Reaktionsmischung wurde mit N,N-Diisopropylethylamin (61 µl, 350 µmol), 1h bei RT nachgerührt, und dann direkt mittles präp. RP-HPLC (Säule: Chromatorex 125x30; 10µ, Fluss: 75 mL/min, MeCN/Wasser, 0.1 % TFA) gereinigt. Die Lösemittel wurden im Vakuum verdampft und der Rückstand lyophilisiert. Man erhielt 84 mg (88 % d. Th.) der Verbindung tert-Butyl-[(2S)-4-amino-1-({(2S)-4-[{(1R)-1-[1-benzyl-4-(2,5-difluorphenyl)-1H-pyrrol-2-yl]-2,2-dimethylpropyl} (glycoloyl)amino]-1-[(2-{ [(2,5-dioxo-2,5-dihydro-1H-pyrrol-1-yl)acetyl]amino}ethyl)amino]-1-oxobutan-2-yl}amino)-1,4-dioxobutan-2-yl]carbamat.

LC-MS (Methode 1): Rₜ = 1.09 min; MS (ESIpos): m/z = 907 [M+H]⁺

Tert-Butyl-[(2S)-4-amino-1-({(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}amino)-1,4-dioxobutan-2-yl]carbamat (83.0mg, 91.5 µmol) wurde in 5.0 ml Trifluorethanol gelöst. Die Reaktionsmischung wurde mit Zinkchlorid (74.8 mg, 549 µmol) versetzt und 15 min bei 50°C nachgerührt. Der Ansatz wurde mit Ethylendiamin-N,N,N',N'-tetraessigsaeure (160 mg, 549 µmol) versetzt, mit 5.0 ml Acetonitril/Wasser verdünnt, mit TFA (20 µl) versetzt und 10 min nachgerührt. Der Ansatz wurde durch einen Spritzenfilter filtriert und mittles präp. RP-HPLC (Säule: Chromatorex 125x30; 10µ, Fluss: 75 mL/min, MeCN/Wasser, 0.1% TFA) gereinigt. Die Lösemittel wurden im Vakuum verdampft und der Rückstand im Hochvakuum getrocknet. Man erhielt 50 mg (58 % d. Th.) der titel Verbindung.

LC-MS (Methode 1): Rₜ = 0.81 min; MS (ESIpos): m/z = 807 [M+H]⁺

### Intermediat L1

### Trifluoressigsäure--N-(2-aminoethyl)-2-(2,5-dioxo-2,5-dihydro-1H-pyrrol-1-yl)acetamid(1:1)

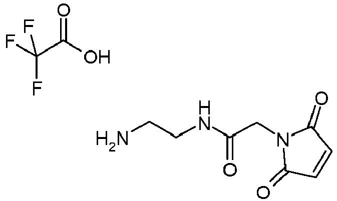

Die Titelverbindung wurde nach klassischen Methoden der Peptidchemie aus kommerziell erhältlicher (2,5-Dioxo-2,5-dihydro-1H-pyrrol-1-yl)essigsäure und tert-Butyl-(2-aminoethyl) carbamat hergestellt.
HPLC (Methode 11): Rₜ = 0.19 min;
LC-MS (Methode 1): Rₜ = 0.17 min; MS (ESIpos): m/z = 198 (M+H)⁺.

### Intermediat L2

### Trifluoressigsäure--rel-(1R,2S)-2-amino-N-[2-(2,5-dioxo-2,5-dihydro-1H-pyrrol-1-yl)ethyl]cyclopentancarboxamid(1:1)

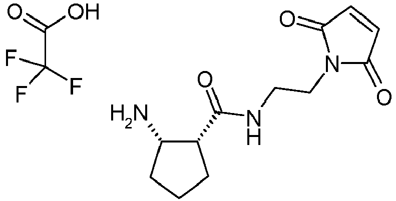

Die Titelverbindung wurde ausgehend von 50 mg (0.214 mmol) von kommerziell erhältlicher cis-2-[(tert-Butoxycarbonyl)amino]-1-cyclopentancarbonsäure mit 60 mg (0.235 mmol) von ebenso kommerziell erhältlichem Trifluoressigsäure--1-(2-aminoethyl)-1H-pyrrol-2,5-dion(1:1) durch Kupplung mit EDC/HOBT und anschließender Entschützung mit TFA hergestellt. Es wurden 36 mg (38% d.Th. über 2 Stufen) der Titelverbindung erhalten.
HPLC (Methode 11): Rₜ = 0.2 min;
LC-MS (Methode 1): Rₜ = 0.17 min; MS (ESIpos): m/z = 252 (M+H)⁺.

### Intermediat L3

### Trifluoressigsäure--(1S,2R)-2-amino-N-[2-(2,5-dioxo-2,5-dihydro-1H-pyrrol-1-yl)ethyl]cyclopentancarboxamid(1:1)

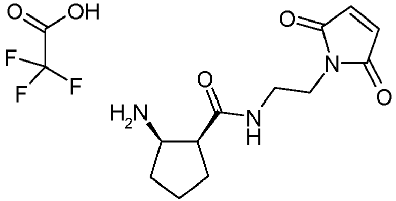

Die Titelverbindung wurde ausgehend von 50 mg (0.214 mmol) von kommerziell erhältlicher (1S,2R)-2-[(tert-Butoxycarbonyl)amino]cyclopentancarbonsäure mit 72 mg (0.283 mmol) von ebenso kommerziell erhältlichem Trifluoressigsäure--1-(2-aminoethyl)-1H-pyrrol-2,5-dion(1:1) durch Kupplung mit EDC/HOBT und anschließender Entschützung mit TFA hergestellt. Es wurden 13 mg (16% d.Th. über 2 Stufen) der Titelverbindung erhalten.
HPLC (Methode 11): Rₜ = 0.2 min;
LC-MS (Methode 1): Rₜ = 0.2 min; MS (ESIpos): m/z = 252 (M+H)⁺.

### Intermediat L4

### Trifluoressigsäure--N-(2-aminoethyl)-4-(2,5-dioxo-2,5-dihydro-1H-pyrrol-1-yl)cyclohexancarboxamid(1:1)

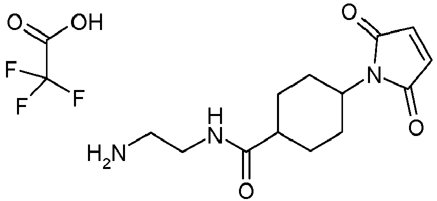

Die Titelverbindung wurde nach klassischen Methoden der Peptidchemie aus kommerziell erhältlichem 1-[(4-{[(2,5-Dioxopyrrolidin-1-yl)oxy]carbonyl}cyclohexyl)methyl]-1H-pyrrol-2,5-dion und tert-Butyl-(2-aminoethyl) carbamat hergestellt.
HPLC (Methode 11): Rₜ = 0.26 min;
LC-MS (Methode 1): Rₜ = 0.25 min; MS (ESIpos): m/z = 280 (M+H)⁺.

### Intermediat L5

### Trifluoressigsäure--N-[4-(2,5-dioxo-2,5-dihydro-1H-pyrrol-1-yl)phenyl]-beta-alaninamid(1:1)

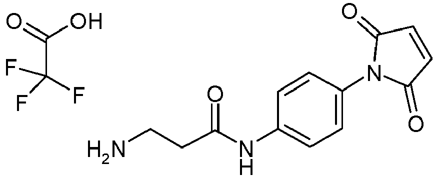

Die Titelverbindung wurde nach klassischen Methoden der Peptidchemie aus kommerziell erhältlichem 1-(4-Aminophenyl)-1H-pyrrol-2,5-dion und N-(tert-Butoxycarbonyl)-beta-alanin hergestellt.
HPLC (Methode 11): Rₜ = 0.22 min;
LC-MS (Methode 1): Rₜ = 0.22 min; MS (ESIpos): m/z = 260 (M+H)⁺.

### Intermediat L6

### Trifluoressigsäure--tert-butyl-N-[6-(2,5-dioxo-2,5-dihydro-1H-pyrrol-1-yl)hexanoyl]-L-valyl-L-alanyl-L-lysinat(1:1)

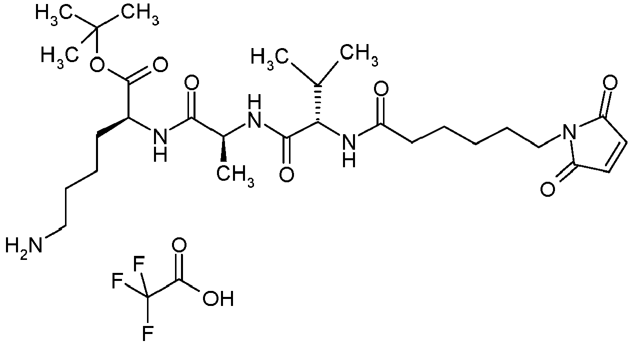

Die Titelverbindung wurde zunächst durch Kupplung von kommerziell erhältlicher 6-(2,5-Dioxo-2,5-dihydro-1H-pyrrol-1-yl)hexansäure mit dem nach klassischen Methoden der Peptidchemie hergestellten partiell geschützten Peptid tert-Butyl-L-valyl-L-alanyl-N⁶-(tert-butoxycarbonyl)-L-lysinat in Gegenwart von EDC/HOBT hergestellt. Anschließend erfolgte die Entschützung an der Aminogruppe unter schonenden Bedingungen durch Rühren in 5%iger Trifluoressigsäure in DCM bei RT, wobei in 37%iger Ausbeute die Titelverbindung erhalten wurde.
HPLC (Methode 11): Rₜ = 1.29 min;
LC-MS (Methode 1): Rₜ = 0.62 min; MS (ESIpos): m/z = 566 (M+H)⁺.

### Intermediat L7

### Trifluoressigsäure--beta-alanyl-L-valyl-N⁵-carbamoyl-N-[4-(2,5-dioxo-2,5-dihydro-1H-pyrrol-1-yl)phenyl] -L-ornithinamid(1: 1)

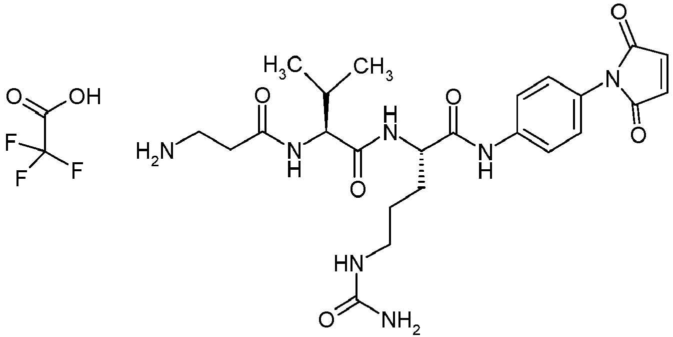

Die Titelverbindung wurde nach klassischen Methoden der Peptidchemie ausgehend von kommerziell erhältlichem 1-(4-Aminophenyl)-1H-pyrrol-2,5-dion durch sequentielle Kupplung mit N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-valinat, Entschützung mit TFA, Kupplung mit 2,5-Dioxopyrrolidin-1-yl-N-(tert-butoxycarbonyl)-beta-alaninat und erneute Entschützung mit TFA hergestellt. Es wurden 32 mg der Titelverbindung erhalten.
HPLC (Methode 11): Rₜ = 0.31 min;
LC-MS (Methode 1): Rₜ = 0.47 min; MS (ESIpos): m/z = 516 (M+H)⁺.

### Intermediat L8

### Trifluoressigsäure--L-alanyl-N⁵-carbamoyl-N-[4-(2,5-dioxo-2,5-dihydro-1H-pyrrol-1-yl)phenyl]-L-ornithinamid(1:1)

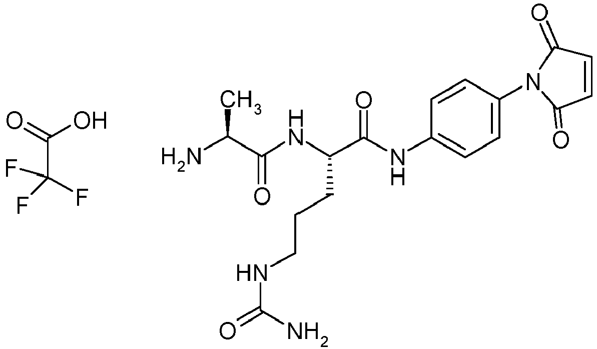

Die Titelverbindung wurde nach klassischen Methoden der Peptidchemie ausgehend von kommerziell erhältlichem 1-(4-Aminophenyl)-1H-pyrrol-2,5-dion durch sequentielle Kupplung mit N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-alaninat und erneute Entschützung mit TFA hergestellt. Es wurden 171 mg der Titelverbindung erhalten.
HPLC (Methode 11): Rₜ = 0.23 min;
LC-MS (Methode 7): Rₜ = 0.3 min; MS (ESIpos): m/z = 417 (M+H)⁺.

### Intermediat L9

### Trifluoressigsäure--beta-alanyl-L-valyl-N⁵-carbamoyl-N-[4-(2-methoxy-2-oxoethyl)phenyl]-L-ornithinamid(1:1)

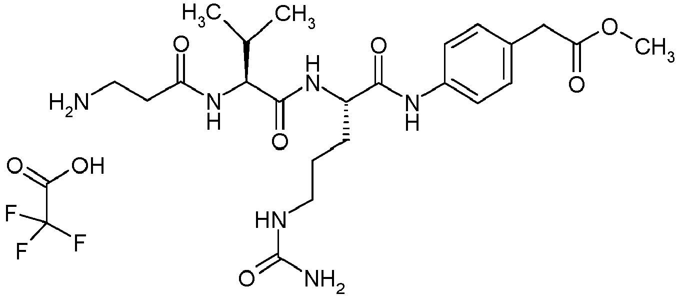

Die Titelverbindung in Analogie zu Intermediat L7 ausgehend von kommerziell erhältlichem Methyl-(4-aminophenyl)acetat hergestellt. Es wurden 320 mg der Titelverbindung erhalten. HPLC (Methode 11): Rₜ = 0.45 min;

LC-MS (Methode 1): Rₜ = 0.48 min; MS (ESIpos): m/z = 493 (M+H)⁺.

### Intermediat L10

### N-[6-(2,5-Dioxo-2,5-dihydro-1H-pyrrol-1-yl)hexanoyl]-L-valyl-L-alanyl-rel-N⁶-{[(1R,2S)-2-aminocyclopentyl]carbonyl}-L-lysin-trifluoressigsäure(1:2)

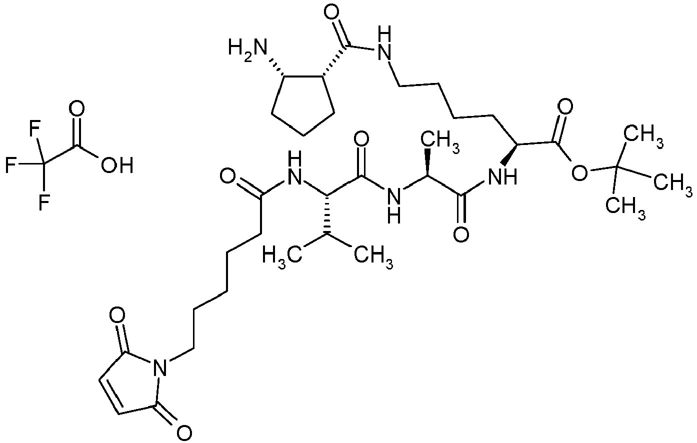

Die Titelverbindung wurde ausgehend von Intermediat L6 durch Kupplung mit cis-2-[(tert-Butoxycarbonyl)amino]-1-cyclopentancarbonsäure mit EDC/HOBT und anschließender Entschützung mit TFA hergestellt. Es wurden 12 mg (52% d.Th. über 2 Stufen) der Titelverbindung erhalten.
HPLC (Methode 11): Rₜ = 1.45 min;
LC-MS (Methode 1): Rₜ = 0.73 min; MS (ESIpos): m/z = 677 (M+H)⁺.

### Intermediat L11

### N-[6-(2,5-Dioxo-2,5-dihydro-1H-pyrrol-1-yl)hexanoyl]-L-valyl-L-alanyl-N⁶-{[(1S,2R)-2-aminocyclopentyl]carbonyl}-L-lysin-trifluoressigsäure(1:2)

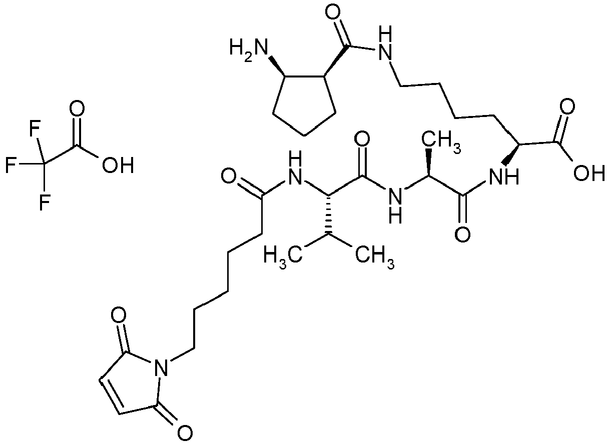

Die Titelverbindung wurde ausgehend von Intermediat L6 durch Kupplung mit (1S,2R)-2-[(tert-Butoxycarbonyl)amino]cyclopentancarbonsäure mit EDC/HOBT und anschließender Entschützung mit TFA hergestellt. Es wurden 11 mg (39% d.Th. über 2 Stufen) der Titelverbindung erhalten. HPLC (Methode 11): Rₜ = 1.45 min;

LC-MS (Methode 1): Rₜ = 0.74 min; MS (ESIpos): m/z = 677 (M+H)⁺.

### Intermediat L12

### Trifluoressigsäure--1-[2-(2-aminoethoxy)ethyl]-1H-pyrrol-2,5-dion(1:1)

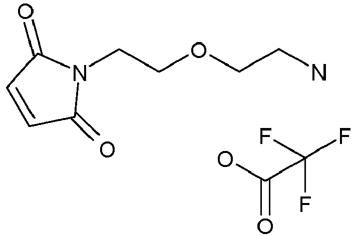

Zu 228 mg (1.12 mmol) tert-Butyl-[2-(2-aminoethoxy)ethyl]carbamat gelöst in 7 mL Dioxan/Wasser 1:1 gelöst wurden 381 mg (2.46 mmol) Methyl-2,5-dioxo-2,5-dihydro-1H-pyrrol-1-carboxylat gegeben. Dann wurden 1.2 mL einer gesättigten Natriumhydrogencarbonat Lösung zugesetzt und der Ansatz bei RT gerührt. Nach insgesamt 5-tägigem Rühren bei 2 weiteren Zugaben von gleichen Mengen der Natriumhydrogencarbonat Lösung wurde der Ansatz durch Ansäuern mit Trifluoressigsäure, Einrotieren und Reinigung des Rückstandes durch präparative HPLC aufgearbeitet. Die entsprechenden Fraktionen wurden vereinigt, das Lösungsmittel im Vakuum entfernt und der Rückstand aus Acetonitril/Wasser 1:1 lyophilisiert.

Der Rückstand wurde in 3 mL Dichlormethan aufgenommen und mit 1 mL Trifluoressigsäure versetzt. Nach 15 min Rühren bei RT wurde das Lösungsmittel im Vakuum entfernt und der Rückstand aus Acetonitril/Wasser 1:1 lyophilisiert. So wurden 70 mg (67% d.Th. über 2 Stufen) der Titelverbindung als harziger Rückstand erhalten.
HPLC (Methode 11): Rₜ = 0.2 min;
LC-MS (Methode 1): Rₜ = 0.18 min; MS (ESIpos): m/z = 185 (M+H)⁺.

### Intermediat L13

### Trifluoressigsäure--tert-butyl-N²-[(2,5-dioxo-2,5-dihydro-1H-pyrrol-1-yl)acetyl]-L-lysinat(1:1)

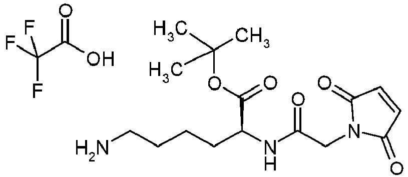

Die Titelverbindung wurde durch Kupplung von (2,5-Dioxo-2,5-dihydro-1H-pyrrol-1-yl)essigsäure mit tert-Butyl-N⁶-(tert-butoxycarbonyl)-L-lysinathydrochlorid(1:1) in Gegenwart von EDC/HOBT und anschließender schonender Abspaltung der tert-Butoxycarbonyl Schutzgruppe in Analogie zu Intermediat L6 hergestellt.
HPLC (Methode 11): Rₜ = 0.42 min;
LC-MS (Methode 1): Rₜ = 0.43 min; MS (ESIpos): m/z = 340 (M+H)⁺.

### Intermediat L14

### Trifluoressigsäure--1-[2-(4-aminopiperazin-1-yl)-2-oxoethyl]-1H-pyrrol-2,5-dion(1:1)

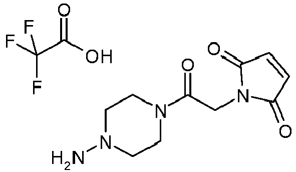

Die Titelverbindung wurde in Analogie zu Intermediat L2 über 2 Stufen aus tert-Butyl-piperazin-1-ylcarbamat und (2,5-dioxo-2,5-dihydro-1H-pyrrol-1-yl)essigsäure hergestellt.
HPLC (Methode 11): Rₜ = 0.2 min;
LC-MS (Methode 3): Rₜ = 0.25 min; MS (ESIpos): m/z = 239 (M+H)⁺.

### Intermediat L15

### Trifluoressigsäure--N-(2-aminoethyl)-3-(2-{2-[2-(2,5-dioxo-2,5-dihydro-1H-pyrrol-1-yl)ethoxy]ethoxy}ethoxy)propanamid(1:1)

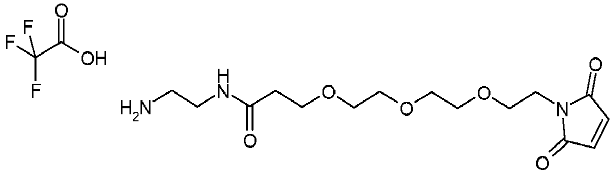

2.93 g (10.58 mmol) tert-Butyl-3-{2-[2-(2-aminoethoxy)ethoxy]ethoxy}propanoat wurden in 100 mL Dioxan/Wasser 1:1 gelöst und mit 3.28 g (21.15 mmol) Methyl-2,5-dioxo-2,5-dihydro-1H-pyrrol-1-carboxylat sowie einer gesättigten Natriumhydrogencarbonatlösung bis zum Erreichen eines pH-Wertes von 6-7 versetzt. Die Lösung wurde 30 min bei RT gerührt und anschließend wurde das 1,4-Dioxan im Vakuum abgedampft. Dann wurden 200 mL Wasser zugegeben und die Mischung dreimal mit jeweils 300 mL Ethylacetat ausgeschüttelt. Die organischen Extrakte wurden vereinigt, über Magnesiumsulfat getrocknet und filtriert. Nach Einengen wurde tert-Butyl-3-(2-{2-[2-(2,5-dioxo-2,5-dihydro-1H-pyrrol-1-yl)ethoxy]ethoxy}ethoxy)propanoat als braunes Öl erhalten, das anschließend im Hochvakuum getrocknet wurde.
HPLC (Methode 11): Rₜ = 1.5 min;
LC-MS (Methode 3): Rₜ = 0.88 min; MS (ESIpos): m/z = 375 (M+NH₄)⁺.

Dieses Intermediat wurde nach Standardverfahren (Entschützung mit TFA, Kupplung mit tert-Butyl-(2-aminoethyl)carbamat und erneute Entschützung mit TFA) in die Titelverbindung überführt.
HPLC (Methode 11): Rₜ = 0.2 min;
LC-MS (Methode 3): Rₜ = 0.25 min; MS (ESIpos): m/z = 344 (M+H)⁺.

### Intermediat L16

### N-[6-(2,5-Dioxo-2,5-dihydro-1H-pyrrol-1-yl)hexanoyl]-L-valyl-N⁵-carbamoyl-L-ornithin

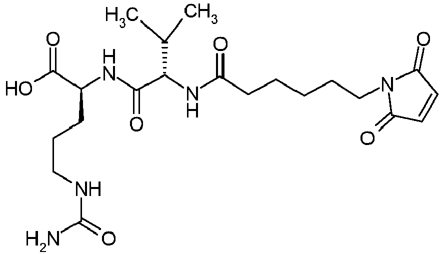

Zu einer Lösung von 266 mg (1.33 mmol) L-Valyl-N5-carbamoyl-L-ornithin in 24 mL DMF wurden 535 mg (1.73 mmol) von kommerziell erhältlichem 1-{6-[(2,5-Dioxopyrrolidin-1-yl)oxy]-6-oxohexyl}-1H-pyrrol-2,5-dion sowie 930 mL *N,N*-Diisopropylethylamin hinzugegeben. Der Ansatz wurde 24 h im Ultraschallbad behandelt und anschließend im Vakuum bis zur Trockene eingeengt. Der verbliebene Rückstand wurde mittels präparativer HPCL gereinigt und nach Einengen der entsprechenden Fraktionen und Trocknung des Rückstandes im Hochvakuum verblieben 337 mg (50% d.Th.) der Titelverbindung.
HPLC (Methode 11): Rₜ = 0.4 min;
LC-MS (Methode 3): Rₜ = 0.58 min; MS (ESIpos): m/z = 468 (M+H)⁺.

### Intermediat L17

### Trifluoressigsäure--tert-butyl-N-[6-(2,5-dioxo-2,5-dihydro-1H-pyrrol-1-yl)hexanoyl]-L-valyl-N⁵-carbamoyl-L-ornithyl-L-lysinat(1:1)

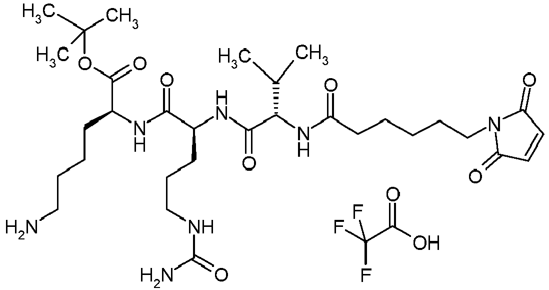

Die Titelverbindung wurde zunächst durch Kupplung von 172 mg (0.37 mmol) Intermediat L16 und 125 mg (0.37 mmol) tert-Butyl-N6-(tert-butoxycarbonyl)-L-lysinat hydrochlorid(1:1) in Gegenwart von EDC/HOBT und *N,N*-Diisopropylethylamin und anschließende Entschützung der Aminogruppe unter schonenden Bedingungen durch 2h Rühren in 10%iger Trifluoressigsäure in DCM bei RT hergestellt. Nach Gefriertrocknung aus Acetonitril/ Wasser wurden 194 mg (49% d.Th.) der Titelverbindung über 2 Stufen erhalten.
HPLC (Methode 11): Rₜ = 1.1 min;
LC-MS (Methode 1): Rₜ = 0.58 min; MS (ESIpos): m/z = 652 (M+H)⁺.

### Intermediat L18

### Trifluoressigsäure--beta-alanyl-L-alanyl-N⁵-carbamoyl-N-[4-(2-methoxy-2-oxoethyl)phenyl]-L-ornithinamid(1:1)

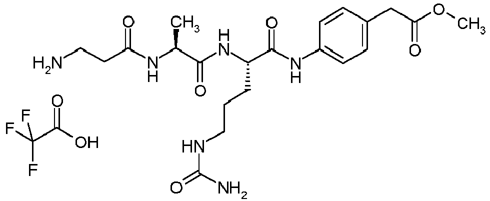

Die Titelverbindung wurde ausgehend von Methyl-(4-aminophenyl)acetat analog Intermediat L7 sequentiell nach klassischen Methoden der Peptidchemie durch Anknüpfung von N²-(tert-Butoxycarbonyl)-N⁴-carbamoyl-L-ornithin in Gegenwart von HATU, Entschützung mit TFA, Kupplung mit 2,5-Dioxopyrrolidin-1-yl-N-(tert-butoxycarbonyl)-L-alaninat, Entschützung mit TFA, Kupplung mit 2,5-Dioxopyrrolidin-1-yl-N-(tert-butoxycarbonyl)-beta-alaninat und erneute Entschützung mit TFA hergestellt. Es wurden 330 mg der Titelverbindung erhalten.
HPLC (Methode 11): Rₜ = 0.29 min;
LC-MS (Methode 1): Rₜ = 0.41 min; MS (ESIpos): m/z = 465 (M+H)⁺.

### Intermediat L19

### Trifluoressigsäure--L-alanyl-N5-carbamoyl-N-(4-{[(2,5-dioxo-2,5-dihydro-1H-pyrrol-1-yl)acetyl]amino}phenyl)-L-ornithinamid(1:1)

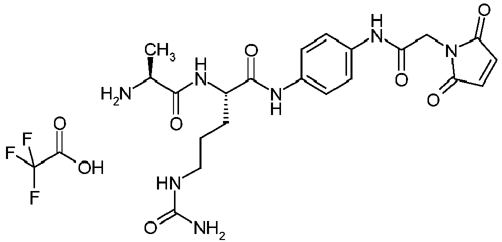

Die Titelverbindung wurde ausgehend von 1,4 Phenylendiamin sequentiell nach klassischen Methoden der Peptidchemie hergestellt. Im ersten Schritt wurden 942 mg (8.72 mmol) 1,4 Phenylendiamin mit 0.8 g (2.9 mmol) N²-(tert-Butoxycarbonyl)-N⁵-carbamoyl-L-ornithin in Gegenwart von HATU und *N*,*N-*Diisopropylethylamin monoacyliert. Im zweiten Schritt wurde in analoger Weise die zweite anilinische Aminogruppe mit (2,5-Dioxo-2,5-dihydro-1H-pyrrol-1-yl)essigsäure in Gegenwart von HATU und *N,N*-Diisopropylethylamin acyliert. Entschützung mit TFA, Kupplung mit 2,5-Dioxopyrrolidin-1-yl-N-(tert-butoxycarbonyl)-L-alaninat und erneute Entschützung mit TFA führten dann in 3 weiteren Syntheseschritten zur Titelverbindung, von der auf diesem Weg 148 mg erhalten wurden.
LC-MS (Methode 1): Rₜ = 0.21 min; MS (ESIpos): m/z = 474 (M+H)⁺.
LC-MS (Methode 4): Rₜ = 0.2 min; MS (ESIpos): m/z = 474 (M+H)⁺.

### Intermediat L20

### Trifluoressigsäure--L-valyl-N⁵-carbamoyl-N-[4-(2,5-dioxo-2,5-dihydro-1H-pyrrol-1-yl)phenyl]-L-ornithinamid(1: 1)

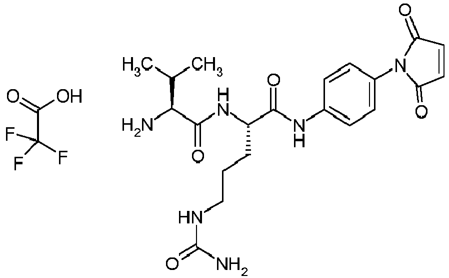

Die Titelverbindung wurde nach klassischen Methoden der Peptidchemie analog zu Intermediat L8 ausgehend von kommerziell erhältlichem 1-(4-Aminophenyl)-1H-pyrrol-2,5-dion durch sequentielle Kupplung mit N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-valinat und erneute Entschützung mit TFA hergestellt. Es wurden 171 mg der Titelverbindung erhalten.
HPLC (Methode 11): Rₜ = 0.28 min;
LC-MS (Methode 1): Rₜ = 0.39 min; MS (ESIpos): m/z = 445 (M+H)⁺.

### Intermediat L21

### L-Valyl-N⁶-(tert-butoxycarbonyl)-N-[4-(2-methoxy-2-oxoethyl)phenyl]-L-lysinamid

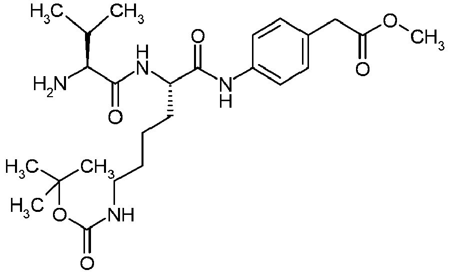

Die Titelverbindung wurde nach klassischen Methoden der Peptidchemie ausgehend von kommerziell erhältlichem 0.42 g (2.56 mmol) Methyl-(4-aminophenyl)acetat durch sequentielle Kupplung mit N6-(tert-Butoxycarbonyl)-N2-[(9H-fluoren-9-ylmethoxy)carbonyl]-L-lysin in Gegenwart von HATU, und *N,N-*Diisopropylethylamin, Entschützung mit Piperidin, Kupplung mit 2,5-Dioxopyrrolidin-1-yl-N-[(benzyloxy)carbonyl]-L-valinat in Gegenwart von *N,N*-Diisopropylethylamin und anschließende hydrogenolytische Abspaltung der Benzyloxycarbonylschutzgruppe über 10%-Palladium-Aktivkohle. Es wurden 360 mg (32% d.Th. über 4 Stufen) der Titelverbindung erhalten.
HPLC (Methode 11): Rₜ = 1.5 min;
LC-MS (Methode 1): Rₜ = 0.73 min; MS (ESIpos): m/z = 493 (M+H)⁺.

### Intermediat L22

### Trifluoressigsäure--N-[(9H-fluoren-9-ylmethoxy)carbonyl]-L-valyl-N-{4-[(2S)-2-amino-3-methoxy-3-oxopropyl]phenyl}-N⁵-carbamoyl-L-ornithinamid(1:1)

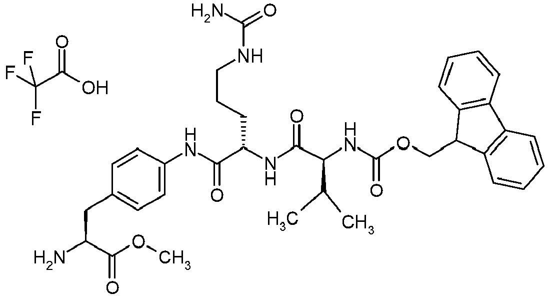

Die Titelverbindung wurde ausgehend von N-(tert-Butoxycarbonyl)-4-nitro-L-phenylalanin sequentiell nach klassischen Methoden der Peptidchemie hergestellt. 2.5 g (8.06 mmol) von diesem Edukt wurde im ersten Schritt zunächst in das Caesiumsalz und dann mit Iodmethan in DMF in den Methylester überführt.

Hydrogenolytisch wurde dann in Methanol über 10% Palladium-Aktivkohle die Nitrogruppe in eine Aminogruppe überführt.

Die so generierte Aminogruppe wurde dann mit N5-Carbamoyl-N2-[(9H-fluoren-9-ylmethoxy)carbonyl]-L-ornithin in DMF in Gegenwart von HATU und *N,N*-Diisopropylethylamin acyliert. Im nächsten Schritt wurde die Fmoc-Gruppe mit Piperidin in DMF abgespalten. Anschließend erfolgte in DMF die Kupplung mit N-[(9H-Fluoren-9-ylmethoxy)carbonyl]-L-valin in Gegenwart von 1-(3-Dimethylaminopropyl)-3-ethylcarbodiimid-Hydrochlorid, 1-Hydroxy-1*H-*benzotriazol-Hydrat und *N,N*-Diisopropylethylamin und schließlich die Abspaltung der tert-Butoxycarbonylgruppe mit Trifluoressigsäure.
HPLC (Methode 11): Rₜ = 1.6 min;
LC-MS (Methode 1): Rₜ = 0.77 min; MS (ESIpos): m/z = 673 (M+H)⁺.

### Intermediat L23

### Trifluoressigsäure--N-[2-(2,5-dioxo-2,5-dihydro-1H-pyrrol-1-yl)ethyl]-beta-alaninamid(1: 1)

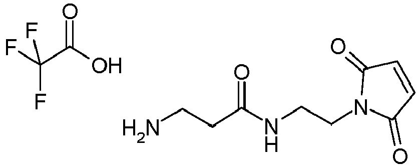

Die Titelverbindung wurde ausgehend von kommerziell erhältlichem Trifluoressigsäure--1-(2-aminoethyl)-1H-pyrrol-2,5-dion(1:1) durch Kupplung mit N-(tert-Butoxycarbonyl)-beta-alanin in Gegenwart von EDCI/HOBT und *N,N*-Diisopropylethylamin und anschließende Entschützung mit Trifluoressigsäure hergestellt.

HPLC (Methode 11): Rₜ = 0.19 min.

### Intermediat L24

### Trifluoressigsäure--1-amino-N-[2-(2,5-dioxo-2,5-dihydro-1H-pyrrol-1-yl)ethyl] cyclopropancarboxamid(1:1)

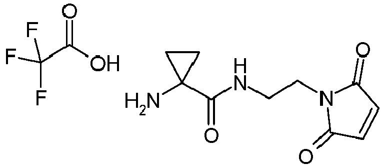

114 mg (0.67 mmol) von kommerziell erhältlicher 1-[(tert-Butoxycarbonyl)amino]cyclopropan carbonsäure wurden in 25 mL DCM gelöst und mit 110 mg (0.623 mmol) von kommerziell erhältlichem Trifluoressigsäure--1-(2-aminoethyl)-1H-pyrrol-2,5-dion(1:1) sowie mit 395 µl *N,N-*Diisopropylethylamin versetzt und auf -10°C abgekühlt. Dann wurden 217 mg (0.793 mmol) 2-Brom-1-ethylpyridiniumtetrafluoroborat zugegeben und der Ansatz 2 h bei RT gerührt. Anschließend wurde mit Ethylacetat verdünnt und nacheinander mit 10%-iger Zitronensäure, gesättigter Natriumhydrogencarbonatlösung un gesättigter Natriumchloridlösubng ausgeschüttelt, dann über Magnesiumsulfat getrocknet und eingeengt. Nach Trocknung im Hochvakuum wurden 152 mg des geschützten Intermediats erhalten.

Diese wurden anschließend in 10 mL DCM aufgenommen und mit 1 mL Trifluoressigsäure entschützt. Nach Lyophilisation aus Acetonitril/Wasser wurden 158 mg (71% d.Th. über 2 Stufen) der Titelverbindung erhalten.

HPLC (Methode 11): Rₜ = 0.19 min.

LC-MS (Methode 3): Rₜ = 0.98 min; MS (ESIpos): m/z = 224 (M+H)⁺.

### Intermediat L25

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-L-alanin

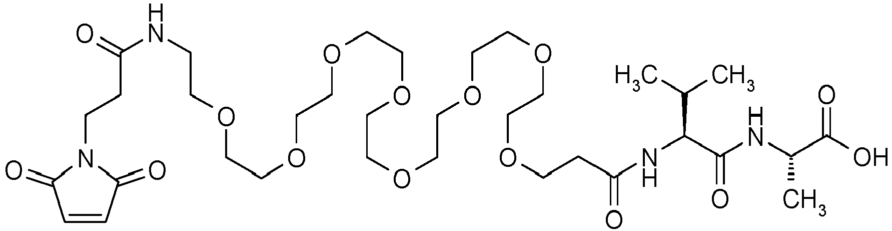

31.4 mg (0.17 mmol) Valyl-L-alanin wurden in 3.0 mL DMF gelöst und mit 115.0 mg (0.17 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid und mit 33.7 mg (0.0.33 mmol) Triethylamin versetzt. Es wurde über Nacht bei RT gerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 74.1 mg (58 % d. Th.) der Titelverbindung.

LC-MS (Methode 1): Rₜ = 0.61 min; MS (ESIpos): m/z = 763 [M+H]⁺.

### Intermediat L26

### L-Valyl-N6-(tert-butoxycarbonyl)-L-lysin

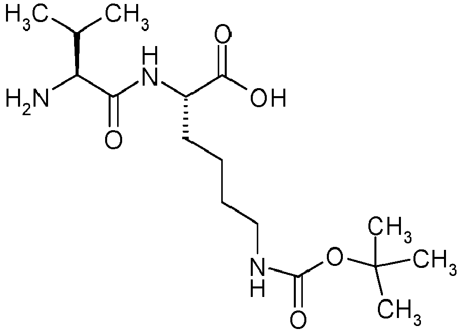

600.0 mg (1.58 mmol) N2-[(Benzyloxy)carbonyl]-N6-(tert-butoxycarbonyl)-L-lysin wurden in 25.0 mL Wasser/Ethanol/THF (1:1:0.5) suspendiert und mit Palladium auf Kohle (10%ig) versetzt und bei RT mit Wasserstoff unter Normaldruck 5 h hydriert. Der Katalysator wurde abfiltriert und die Lösemittel im Vakuum verdampft. Die erhaltene Verbindung wurde ohne weitere Reinigung in der nächsten Stufe eingesetzt.

LC-MS (Methode 1): Rₜ = 0.42 min; MS (ESIpos): m/z = 247 [M+H]⁺.

180 mg (0.73 mmol) N6-(tert-Butoxycarbonyl)-L-lysin wurden in 5.0 mL DMF gelöst und mit 74.0 mg (0.73 mmol) Triethylamin versetzt. Dann wurden 254.6 mg (0.73 mmol) 2,5-Dioxopyrrolidin-1-yl-N-[(benzyloxy)carbonyl]-L-valinat und 74.0 mg (0.73 mmol) Triethylamin zugegeben. Die Reaktionsmischung wurde 3.5 h bei RT gerührt. Die Reaktionslösung wurde direkt über die präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 294.1 mg (76 % d. Th.) N-[(Benzyloxy)carbonyl]-L-valyl-N6-(tert-butoxycarbonyl)-L-lysin.

LC-MS (Methode 1): Rₜ = 0.97 min; MS (ESIpos): m/z = 480 [M+H]⁺.

272.2 mg (0.57 mmol) N-[(Benzyloxy)carbonyl]-L-valyl-N6-(tert-butoxycarbonyl)-L-lysin wurden in 20.0 mL Ethylacetat/Ethanol/THF (1:1:1) vorgelegt und mit 27.2 mg Palladium auf Aktivkohle versetzt. Man hydrierte mit Wasserstoff bei RT unter Normaldruck 5 h. Es wurde mit Hilfe von Celite^{(R)} abfiltriert und der Filterkuchen mit Ethylacetat/Ethanol/THF (1:1:1) nachgewaschen. Die Lösemittel wurden im Vakuum verdampft und der Rückstand im Hochvakuum getrocknet. Die Titelverbindung (182 mg, 72 % d. Th.) wurde in der nächsten Reaktionsstufe ohne weitere Reinigung eingesetzt.

LC-MS (Methode 1): Rₜ = 0.53 min; MS (ESIpos): m/z = 346 [M+H]⁺.

### Intermediat L27

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N6-(tert-butoxycarbonyl)-L-lysin

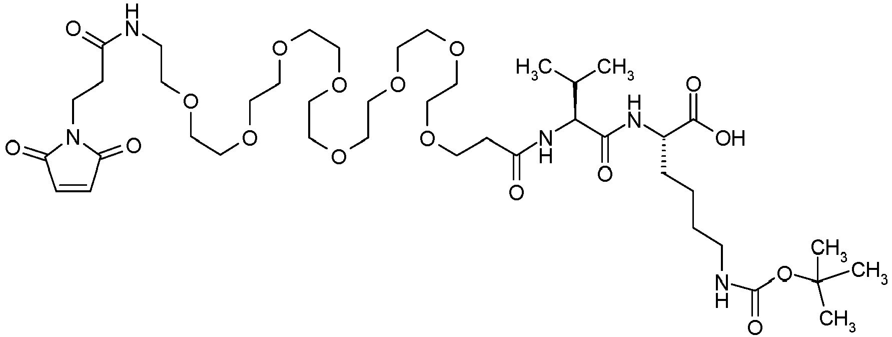

30 mg (0.07 mmol) L-Valyl-N6-(tert-butoxycarbonyl)-L-lysin (Intermediat L26) und 46.1 mg (0.07 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid wurden in 1.5 mL DMF vorgelegt und mit 6.8 mg (0.07 mmol) 4-Methylmorpholin versetzt. Die Reaktionslösung rührte bei RT über Nacht. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 55.6 mg (90 % d. Th.) der Titelverbindung.

LC-MS (Methode 1): Rₜ = 0.77 min; MS (ESIpos): m/z = 920 [M+H]⁺.

### Intermediat L28

### tert-Butyl-3-formyl-4-({[2-(trimethylsilyl)ethoxy]carbonyl}amino)pyrrolidin-1-carboxylat

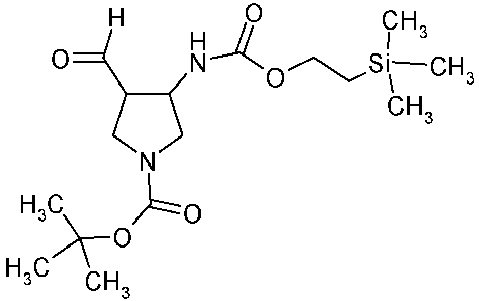

461.7 mg (1.15 mmol) 1-tert-Butyl-3-ethyl-4-({[2-(trimethylsilyl)ethoxy]carbonyl}amino)pyrrolidin-1,3-dicarboxylat (Diese Verbindung wurde gemäß Literaturvorschrift WO 2006/066896 hergestellt) wurden in 5.0 mL absol. Dichlormethan vorgelegt und auf -78 °C abgekühlt. Dann wurde langsam 326.2 mg (2.29 mmol) Diisobutylaluminiumhydrid-Lösung (1 M in THF) zugetropft und 2 h bei -78 °C gerührt (Dünnschichtchromatographische Kontrolle (Petrolether/Ethylacetat = 3: 1). Es wurden 1.3 g (4.59 mmol) Kaliumnatriumtartrat gelöst in 60 mL Wasser zugetropft und die Reaktionsmischung auf RT kommen gelassen. Die Reaktionsmischung wurde mit Ethylacetat versetzt und die wässrige Phase dreimal mit Ethylacetat extrahiert. Die vereinigten organischen Phasen wurden einmal mit ges. NaCl-Lösung gewaschen und über Magnesiumsulfat getrocknet. Das Lösemittel wurde im Vakuum verdampft und der Rückstand im Hochvakuum getrocknet. Man erhielt 629.0 mg der Titelverbindung als Rohprodukt, welches ohne weitere Reinigung sofort in der nächsten Reaktionsstufe eingesetzt wurde.

### Intermediat L29

### tert-Butyl-3-formyl-4-[({ [2-(trimethylsilyl)ethoxy]carbonyl}amino)methyl]pyrrolidin-1-carboxylat Mischung aus Diastereomeren.

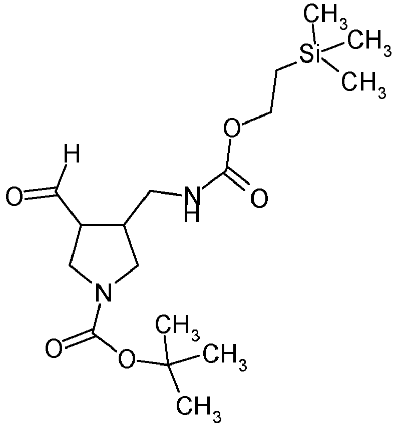

807.1 mg (2.34 mmol) tert-Butyl-3-({[tert-butyl(dimethyl)silyl]oxy}methyl)-4-(hydroxymethyl)pyrrolidin-1-carboxylat (Die Herstellung erfolgte gemäß der Literaturvorschrift WO 2006/100036) wurden in 8.0 mL Dichlormethan vorgelegt und 236.4 mg (2.34 mmol) Triethylamin zugegeben. Bei 0 °C wurden 267.6 mg (2.34 mmol) Methansulfonsäurechlorid zugetropft und die Reaktionsmischung über Nacht bei RT gerührt. Es erfolgte eine weitere Zugabe von 133.8 mg (1.17 mmol) Methansulfonsäurechlorid und 118.2 mg (1.17 mmol) Triethylamin. Die Reaktionsmischung wurde über Nacht bei RT gerührt. Es wurde mit Dichlormethan verdünnt und die organische Phase je einmal mit ges. Natriumhydrogencarbonat-Lösung, 5%iger Kaliumhydrogensulfat-Lösung und ges. NaCl-Lösung gewaschen. Nach der Trocknung über Magnesiumsulfat wurde das Lösemittel im Vakuum verdampft und der Rückstand wurde mittels Biotage Isolera gereinigt (Kieselgel, Säule 50 g SNAP, Fluss 66 mL/min, Cyclohexan/Ethylacetat). Die Lösemittel wurden im Vakuum verdampft und der Rückstand im Hochvakuum getrocknet. Man erhielt 402.0 mg (41 % d. Th.) der Verbindung tert-Butyl-3-({[tert-butyl(dimethyl)silyl]oxy}methyl)-4-{[(methylsulfonyl)oxy]methyl}pyrrolidin-1-carboxylat LC-MS (Methode 1): Rₜ = 1.38 min; MS (ESIpos): m/z = 424 [M+H]⁺.

400.0 mg (0.94 mmol) tert-Butyl-3-({[tert-butyl(dimethyl)silyl]oxy}methyl)-4-{ [(methylsulfonyl)oxy]methyl}pyrrolidin-1-carboxylat wurden in 5.0 mL DMF vorgelegt und mit 98.2 mg (1.51 mmol) Natriumazid versetzt. Die Reaktionsmischung wurde 10 h bei 40 °C gerührt.

Dann wurden nochmals 30.7 mg (0.47 mmol) Natriumazid zugegeben und weitere 10 h bei 40 °C gerührt. Es wurde Ethylacetat zugegeben und die organsiche Phase mehrmals mit Wasser gewaschen. Nach der Trocknung der organischen Phase über Magnesiumsulfat wurde das Lösemittel im Vakuum verdampft und der Rückstand im Hochvakuum getrocknet. Man erhielt 309.5 mg (89 % d. Th.) der Verbindung tert-Butyl-3-(azidomethyl)-4-({[tert-butyl(dimethyl)silyl]oxy}methyl)pyrrolidin-1-carboxylat. Die Verbindung wurde ohne weitere Reinigung in der nächsten Synthesestufe eingesetzt.

LC-MS (Methode 1): Rₜ = 1.50 min; MS (ESIpos): m/z = 371 [M+H]⁺.

250 mg (0.68 mmol) tert-Butyl-3-(azidomethyl)-4-({[tert-butyl(dimethyl)silyl]oxy}methyl)pyrrolidin-1-carboxylat wurden in 10.0 mL Ethylacetat/Ethanol (1:1) gelöst und mit 25.0 mg Palladium auf Aktivkohle (10%) versetzt. Man hydrierte mit Wasserstoff bei RT unter Normaldruck 8 h. Die Reaktion wurde über Celite^{(R)} filtiert und der Filterkuchen intensiv mit Ethylacetat gewaschen. Das Lösemittel wurde im Vakuum verdampft und der Rückstand im Hochvakuum getrocknet. Man erhielt 226.2 mg (82 % d. Th.) der Verbindung tert-Butyl-3-(aminomethyl)-4-({[tert-butyl(dimethyl)silyl]oxy}methyl)pyrrolidin-1-carboxylat. Die Verbindung wurde ohne weitere Reinigung in der nächsten Synthesestufe eingesetzt.

LC-MS (Methode 1): Rₜ = 0.89 min; MS (ESIpos): m/z = 345 [M+H]⁺.

715.0 mg (2.08 mmol) tert-Butyl-3-(aminomethyl)-4-({[tert-butyl(dimethyl)silyl]oxy}methyl)-pyrrolidin-1-carboxylat wurden in 15.0 mL THF gelöst und mit 2.28 mL (2.28 mmol) TBAF-Lösung (1M in THF) versetzt. Die Reaktionsmischung wurde bei RT über Nacht gerührt. Das Lösemittel wurde im Vakuum verdampft und der Rückstand (1.54 g) ohne weitere Reinigung in der nächsten Synthesestufe eingesetzt.

LC-MS (Methode 1): Rₜ = 0.41 min; MS (ESIpos): m/z = 231 [M+H]⁺.

1.54 g (4.88 mmol) tert-Butyl-3-(aminomethyl)-4-(hydroxymethyl)pyrrolidin-1-carboxylat wurden in 1,4-Dioxan vorgelegt mit 541.8 mg (4.88 mmol) Calciumchlorid (wasserfrei) und 488.6 mg (4.88 mmol) Calciumcarbonat versetzt und kräftig gerührt. Dann wurden 592.8 mg (5.86 mmol) Triethylamin und 1.52 g (5.86 mmol) 1-({[2-(Trimethylsilyl)ethoxy]carbonyl}oxy)pyrrolidin-2,5-dion zugegeben und die Reaktionsmischung über Nacht bei RT gerührt. Es wurden 644.9 mg (10.7 mmol) HOAc und Ethylacetat zugegeben. Die organische Phase wurde zweimal mit Wasser und einmal mit ges. NaCl-Lösung gewaschen. Nach der Trocknung über Magnesiumsulfat wurde das Lsöemittel im Vakuum verdampft und der Rückstand mittels Kieselgel (Laufmittel: Dichlormethan/Methanol = 100:1) gereinigt. Die Lösemittel wurden im Vakuum verdampft und der Rückstand im Hochvakuum getrocknet. Man erhielt 346.9 mg (19 % d. Th.) der Verbindung tert-Butyl-3-(hydroxymethyl)-4-[({[2-(trimethylsilyl)ethoxy]carbonyl}amino)methyl]pyrrolidin-1-carboxylat.

LC-MS (Methode 1): Rₜ = 1.08 min; MS (ESIpos): m/z = 375 [M+H]⁺.

804.0 mg (2.15 mmol) tert-Butyl-3-(hydroxymethyl)-4-[({[2-(trimethylsilyl)ethoxy]carbonyl}amino)methyl]pyrrolidin-1-carboxylat wurden in 20.0 mL Chloroform und 20.0 mL 0.05 N Kaliumcarbonat/0.05 N Natriumhydrogencarbonat-Lösung (1:1) vorgelegt. Dann wurden 59.7 mg (0.22 mmol) Tetra-n-butylammoniumchlorid, 429.9 mg (3.22 mmol) N-Chlorsuccinimid und 33.5 mg (0.22 mmol) TEMPO zugegeben und die Reaktionsmischung kräftig über Nacht bei RT gerührt. Die organische Phase wurde abgetrennt und im Vakuum vom Lösemittel befreit. Der Rückstand wurde mittels Kieselgel (Lausfmittel: Cyclohexan/Ethylacetat = 3:1) gereinigt. Man erhielt 517.0 mg (46 % d. Th.) der Titelverbindung. LC-MS (Methode 1): Rₜ = 1.13 min; MS (ESIpos): m/z = 373 [M+H]⁺.

### Intermediat L30

### tert-Butyl-3-({[tert-butyl(dimethyl)silyl]oxy}methyl)-4-formylpyrrolidin-1-carboxylat Mischung aus Stereoisomeren

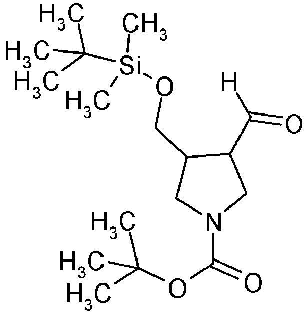

250.0 mg (0.72 mmol) tert-Butyl-3-({[tert-butyl(dimethyl)silyl]oxy}methyl)-4-(hydroxymethyl)pyrrolidin-1-carboxylat (Die Verbindung wurde gemäß der Literaturvorschrift WO2006/100036 hergestellt.) wurde in 12.5 mL Dichlormethan/DMSO (4:1) vorgelegt und mit 219.6 mg (2.17 mmol) Triethylamin versetzt. Bei 2 °C wurde 345.5 mg (2.17 mmol) Schwefeltrioxid-Pyridin-Komplex portionsweise zugegeben und 3 h bei 2 °C gerührt. Es wurden nochmals 345.5 mg (2.17 mmol) Schwefeltrioxid-Pyridin-Komplex portionsweise zugegeben und 17 h bei RT gerührt. Die Reaktionsmischung wurde zwischen Dichlormethan und Wasser verteilt. Die wässrige Phase wurde dreimal mit Dichlormethan extrahiert und die vereinigten organischen Phasen einmal mit Wasser gewaschen und über Magnesiumsulfat getrocknet. Das Lösemittel wurde im Vakuum verdampft und der Rückstand im Hochvakuum getrocknet. Die Rückstand wurde ohne weitere Reinigung in der nächsten Synthesestufe eingesetzt

(Dünnschichtchromatographie: Petrolether/Ethylacetat 7:3).

### Intermediat L31

### Di-tert-butyl-{[(tert-butoxycarbonyl)amino]methyl}malonat

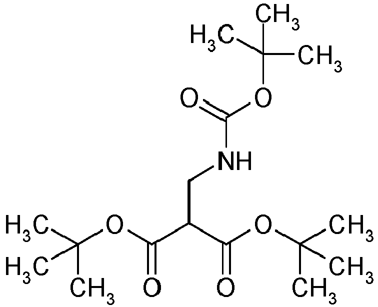

Zu 600 mL Wasser wurden 57.2 g (488.27 mmol) tert-Butylcarbamat, 51.2 mL (683.57 mmol) einer 37%-igen Lösung von Formaldehyd in Wasser und 25.9 g (244.13 mmol) Natriumcarbonat addiert. Das Gemisch wurde erwärmt, bis eine Lösung entstand und dann bei RT für 16 h gerührt. Die entstandene Suspension wurde mit 500 mL Dichlormethan extrahiert, die organische Phase abgetrennt, mit gesättigter Natriumchlorid-Lösung gewaschen und über Natriumsulfat getrocknet. Es wurde am Rotationsverdampfer eingeengt und der Rückstand im Hochvakuum getrocknet, wobei ein kristalliner Feststoff anfällt. Der Rückstand wurde in 1000 mL absoluten THF aufgenommen und bei RT ein Gemisch aus 322 mL (3.414 mol) Essigsäureanhydrid und 138 mL (1.707 mol) Pyridin zugetropft. Die Reaktionsmischung wurde bei RT für 16 h gerührt und anschließend am Rotationsverdampfer eingenegt, wobei das Wasserbad Raumtemperatur hatte. Der Rückstand wurde in Diethylether aufgenommen und drei mal mit einer gesättigten Natriumhydrogencarbonat-Lösung sowie ein mal mit einer gesättigten Natriumchlorid-Lösung gewaschen. Die organische Phase wurde über Natriumsulfat getrocknet, am Rotationsverdampfer eingeengt und der Rückstand 2 d im Hochvakuum getrocknet. Der Rückstand wurde in 2000 mL absolutem THF aufgenommen und unter Eiskühlung mit 456 mL (456.52 mmol) einer 1 M Lösung von Kalium-tert-butanolat in THF versetzt. Es wurde 20 Min. bei 0°C gerührt und anschließend 100.8 g (456.52 mmol) Di-tert-butylmalonat gelöst in 200 mL absolutem THF zugetropft. Es wurde 48 h bei RT gerührt und anschließend Wasser addiert. Das Reaktionsgemisch wurde am Rotationsverdampfer eingeengt und in 500 mL Essigsäureethylester aufgenommen. Das Gemisch wurde mit 500 mL Wasser und 100 mL einer gesättigten Natriumchlorid-Lösung gewaschen und die organische Phase über Natriumsulfat getrocknet. Die organische Phase wurde am Rotationsverdampfer eingeengt und der Rückstand im Hochvakuum getrocknet. Der Rückstand wurde mittels Filtration über Kieselgel (Eluent: Cylohexan/Essigsäureethylester, Gradient = 30:1 → 5:1) gereinigt. Es wurden 37.07 g (22% d. Th.) der Zielverbindung erhalten.

LC-MS (Methode 6): Rₜ = 2.87 min; MS (ESIpos): m/z = 346 [M+H]⁺.

### Intermediat L32

### tert-Butyl-[3-hydroxy-2-(hydroxymethyl)propyl]carbamat

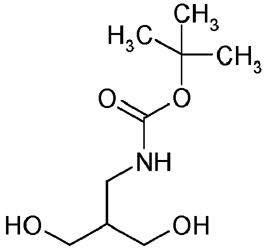

37.0 g (107.11 mmol) Di-tert-butyl-(acetoxymethyl)malonat wurden in 1000 mL absolutem THF gelöst und unter Eiskühlung mit 535.5 mL (1071.10 mmol) einer 2 M Lösung von Lithiumborhydrid in THF tropfenweise versetzt. Es wurden 19.3 mL (1071.10 mmol) Wasser zugetropft und 4.5 h bei RT gerührt. Das Reaktionsgemisch wurde am Rotationsverdampfer eingeengt und im Hochvakuum getrocknet. Der Rückstand wurde in 1500 mL Essigsäureethylester aufgenommen und mit 100 mL Wasser versetzt und 30 Min. unter Wasserkühlung gerührt (leicht exotherm). Die organische Phase wurde abgetrennt und die wässrige Phase zwei mal mit 500 mL Essigsäureethylester extrahiert. Die organische Phase wurde am Rotationsverdampfer eingeengt und der Rückstand im Hochvakuum getrocknet. Es wurden 20.7 g (94% d. Th.) der Zeilverbindung erhalten.

LC-MS (Methode 6): Rₜ = 1.49 min; MS (EIpos): m/z = 106 [M-C₅H₈O₂]⁺.

### Intermediat L33

### tert-Butyl- [3 - {[tert-butyl(dimethyl) silyl] oxy } -2-(hydroxymethyl)propyl] carbamat

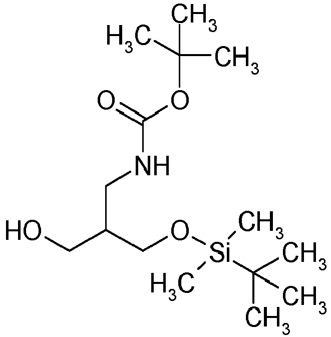

20.00 g (97.44 mmol) tert-Butyl-[3-hydroxy-2-(hydroxymethyl)propyl]carbamat wurden in 1000 mL absolutem Dichlormethan gelöst und bei RT mit 6.63 g (97.44 mmol) Imidazol sowie 16.16 g (107.18 mmol) tert-Butyl(chlor)dimethylsilan versetzt. Es wurde 16 h bei RT gerührt und das Reaktionsgemisch mit halbkonzentrierter Natriumchlorid-Lösung gewaschen. Die wässrige Phase wurde mit Essigsäureethylester extrahiert und die vereinigten oranischen Phasen über Natriumsulfat getrocknet, am Rotationsverdampfer eingeengt und im Hochvakuum getrocknet. Es wurden 28.50 g (92% d. Th.) der Zielverbindung erhalten.

¹H-NMR (400 MHz, DMSO-d₆): δ [ppm] = 0.02 (s, 6H), 0.86 (s, 9H), 1.37 (s, 9H), 1.58-1.73 (m, 1H), 2.91 (q, 2H), 3.33-3.36 [m, (2H, verdeckt)], 3.53-3.58 (m, 2H), 6.65-6.72 (m, 1H).

### Intermediat L34

### tert-Butyl-(3-{ [tert-butyl(dimethyl)silyl]oxy}-2-formylpropyl)carbamat

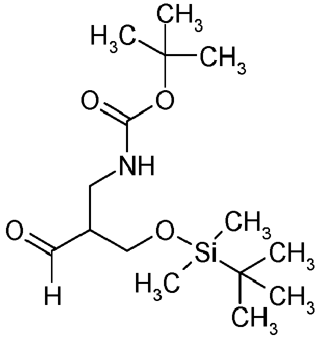

12.65 g ( 39.591 mmol) tert-Butyl-[3-{[tert-butyl(dimethyl)silyl]oxy}-2-(hydroxymethyl)propyl]carbamat wurden in 200 mL Dichlormethan gelöst und bei RT mit und mit 19.31 g (45.53 mmol) Dess-Martin-Periodinan gelöst in 150 mL Dichlormethan tropfenweise versetzt. Das Gemisch wurde 2 h bei Raumtemperatur gerührt und anschließend mit 250 mL einer halbkonzentrierten Natriumhydrogencarbonat-Lösung sowie mit 250 mL einer 10%-igen Natriumthiosulfat-Lösung versetzt, und für 20 Min. gerührt. Die organische Phase wurde abgetrennt und die wässrige Phase mit Essigsäureethylester extrahiert. Die vereinigten organischen Phasen wurden mit 300 mL Wasser gewaschen, über Natriumsulfat getrocknet, am Rotationsverdampfer eingeengt und im Hochvakuum getrocknet. Es wurden 11.35 g ( 90% d. Th.) der Zielverbindung erhalten.

¹H-NMR (400 MHz, DMSO-d₆): δ [ppm] = 0.02 (s, 6H), 0.84(s, 9H), 1.36 (s, 9H), 1.48-1.51 (m, 1H), 3.08-3.32 [m, (1H, verdeckt)], 3.50-3.58 (m, 2H), 3.81-3.91 (m, 1H), 6.71 (t, 1H), 9.60 (d, 1H).

### Intermediat L35

### tert-Butyl-(3-oxopropyl)carbamat

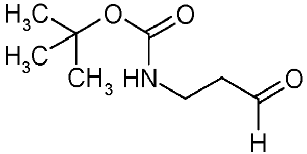

Die Titelverbindung wurde nach literaturbekannten Verfahren hergestellt (z.B. Jean Bastide et al. J. Med. Chem. 2003, 46(16), 3536-3545).

### Intermediat L36

### N-[(Benzyloxy)carbonyl]-L-valyl-N5-carbamoyl-L-omithin

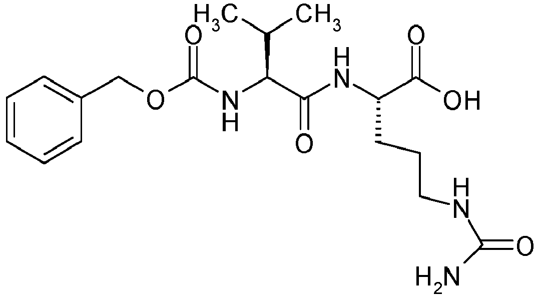

100 mg (0.57 mmol) N5-Carbamoyl-L-ornithin wurden in 4.0 mL DMF aufgenommen und mit 0.08 mL (0.57 mmol) Triethylamin versetzt. Dann wurden 199.0 mg (0.57 mmol) 2,5-Dioxopyrrolidin-1-yl-N-[(benzyloxy)carbonyl]-L-valin und 0.08 mL (0.57 mmol) Triethylamin zugesetzt. Es wurde 48 h bei RT gerührt. Die Reaktionsmischung wurde direkt über die präp. RP-HPLC gereinigt (Säule: Reprosil 250x30; 10µ, Fluss: 50 mL/min, MeCN/Wasser mit 0.1 % TFA). Die Lösemittel wurden im Vakuum verdampft und der Rückstand im Hochvakuum getrocknet. Man erhielt 75.7 mg (33 % d. Th.) der Titelverbindung.

LC-MS (Methode 1): Rₜ = 0.69 min; MS (ESIpos): m/z = 409 [M+H]⁺.

### Intermediat L37

### L- V alyl-N 5-carbamoyl-L-ornithin

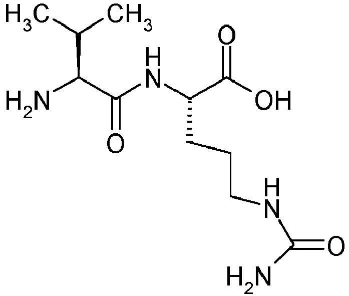

75.7 mg (0.19 mmol) von Intermediat L36 wurden in 25 mL Wasser/Ethanol/THF suspendiert und mit 7.5 mg Palladium auf Aktivkohle (10%ig) versetzt und bei RT 4.5 h lang mit Wasserstoff unter Normaldruck hydriert. Der Katalysator wurde abfiltriert und die Reaktionsmischung im Vakuum vom Lösemittel befreit und im Hochvakuum getrocknet. Der Rückstand wurde ohne weitere Reinigung in der nächsten Stufe eingesetzt. Man erhielt 64.9 mg (93 % d. Th.) der Titelverbindung. LC-MS (Methode 6): Rₜ = 0.25 min; MS (ESIpos): m/z = 275 [M+H]⁺.

### Intermediat L38

### N-[31-(2,5-Dioxo-2,5-dihydro-1H-pyrrol-1-yl)-29-oxo-4,7,10,13,16,19,22,25-octaoxa-28-azahentriacontan-1-oyl] -L-valyl-N5 -carbamoyl-L-ornithin

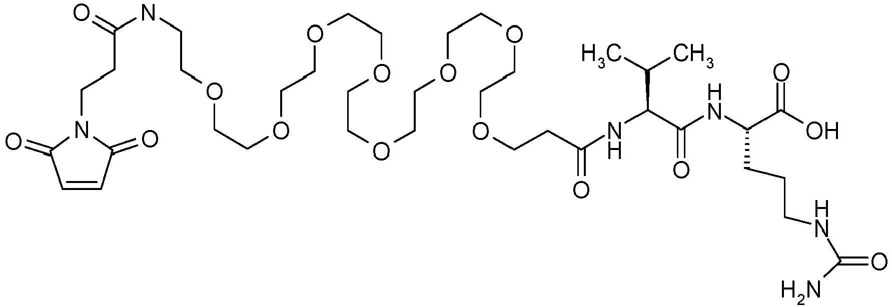

38.3 mg (0.14 mmol) von Intermediat L37 wurden in 3.0 mL DMF vorgelegt und mit 96.4 mg (0.14 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid und mit 39.0 µL (0.28 mmol) Triethylamin versetzt. Es wurde über Nacht bei RT gerührt. Die Reaktionsmischung wurde anschließend mit 16.0 µL (0.28 mmol) HOAc versetzt und direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 58.9 mg (45 % d. Th.) der Titelverbindung.

LC-MS (Methode 1): Rₜ = 0.61 min; MS (ESIpos): m/z = 849 [M+H]⁺.

### Intermediat L39

### 2-(Trimethylsilyl)ethyl-(2-sulfanylethyl)carbamat

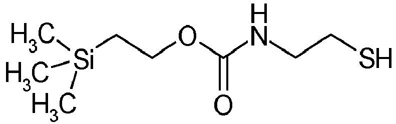

300 mg (2.64 mmol) 2-Aminoethanthiolhydrochlorid (1:1) wurden in 3.0 mL Dichlormethan vorgelegt und mit 668.0 mg (6.60 mmol)Triethylamin mit 719.1 mg (2.77 mmol) 1-({[2-(Trimethylsilyl)ethoxy]carbonyl}oxy)pyrrolidin-2,5-dion versetzt. Es wurde 2 Tage bei RT gerührt. (Dünnschichtchromatographische Kontrolle: Dichlormethan/Methanol = 100:1.5) Die Reaktionsmischung wurde mit Ethylacetat versetzt und dreimal mit Wasser gewaschen. Die organische Phase wurde zweimal mit ges. NaCl-Lösung gewaschen und über Magnesiumsulfat getrocknet. Das Lösemittel wurde im Vakuum verdampft und der Rückstand im Hochvakuum getrocknet. Einsatz der Verbindung ohne weitere Reinigung in der nächsten Synthesestufe.

### Intermediat L40

### N-[31-(2,5-Dioxo-2,5-dihydro-1H-pyrrol-1-yl)-29-oxo-4,7,10,13,16,19,22,25-octaoxa-28-azahentriacontan-1-oyl] -L-valyl-N6-(tert-butoxycarbonyl)-L-lysin

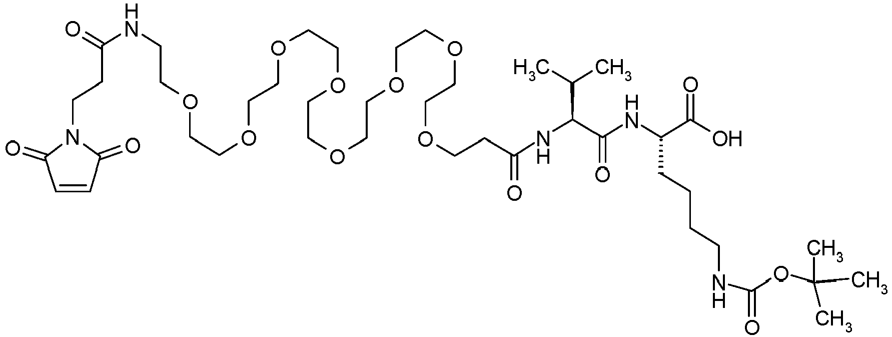

600 mg (1.58 mmol) N2-[(Benzyloxy)carbonyl]-N6-(tert-butoxycarbonyl)-L-lysin wurden in 25.0 mL Wasser/Ethanol/THF (1:1:0.5) mittels Palladium auf Kohle (10 %) bei RT unter Normaldruck mit Wasserstoff hydriert. Die Verbindung N6-(tert-Butoxycarbonyl)-L-lysin wird ohne weitere Reinigung in der nächsten Synthesestufe eingesetzt.

LC-MS (Methode 1): Rₜ = 0.99 min; MS (ESIpos): m/z = 247 [M+H]⁺.

180.0 (0.73 mmol) N6-(tert-Butoxycarbonyl)-L-lysin wurden in 5.0 mL DMF gelöst und mit 74.0 mg (0.73 mmol) Triethylamin versetzt. Es wurden 254.6 mg (0.73 mmol) 2,5-Dioxopyrrolidin-1-yl-N-[(benzyloxy)carbonyl]-L-valinat und 74.0 mg (0.73 mmol) Triethylamin zugegeben. Die Reaktionsmischung wurde 3.5 h bei RT gerührt. Die Reaktionsmischung wurde direkt mittles präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 294.1 mg (76 % d. Th.) der Verbindung N-[(Benzyloxy)carbonyl]-L-valyl-N6-(tert-butoxycarbonyl)-L-lysin.

LC-MS (Methode 1): Rₜ = 0.97 min; MS (ESIpos): m/z = 480 [M+H]⁺.

272.2 mg (0.57 mmol) N-[(Benzyloxy)carbonyl]-L-valyl-N6-(tert-butoxycarbonyl)-L-lysin wurden in 20 mL Ethylacetat/Ethanol/THF (1:1:1) gelöst und mit 27.2 mg Palladium auf Aktivkohle versetzt und unter Normaldruck und bei RT mit Wasserstoff hydriert. Es wurde über Celite^{(R)} filtriert und der Filterkuchen intensiv mit Ethylacetat/Ethanol/THF (1:1:1) gewaschen. Die Lösemittel wurden im Vakuum verdampft und der Rückstand im Hochvakuum getrocknet. Man erhielt 182.0 mg (72 % d. Th.) der Verbindung L-Valyl-N6-(tert-butoxycarbonyl)-L-lysin. LC-MS (Methode 1): Rₜ = 0.53 min; MS (ESIpos): m/z = 346 [M+H]⁺.

30.0 mg (0.07 mmol) L-Valyl-N6-(tert-butoxycarbonyl)-L-lysin und 46.1 mg (0.07 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid wurden in 1.5 mL DMF gelöst und mit 6.8 mg (0.07 mmol) 4-Methylmorpholin versetzt. Dir Reaktionsmischung wurde über Nacht bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 55.6 mg (90 % d. Th.) der Titelverbindung.

LC-MS (Methode 1): Rₜ = 0.77 min; MS (ESIpos): m/z = 920 [M+H]⁺.

### Intermediat L41

### N-[19-(2,5-Dioxo-2,5-dihydro-1H-pyrrol-1-yl)-17-oxo-4,7,10,13-tetraoxa-16-azanonadecan-1-oyl]-L-valyl-N6-(tert-butoxycarbonyl)-L-lysin

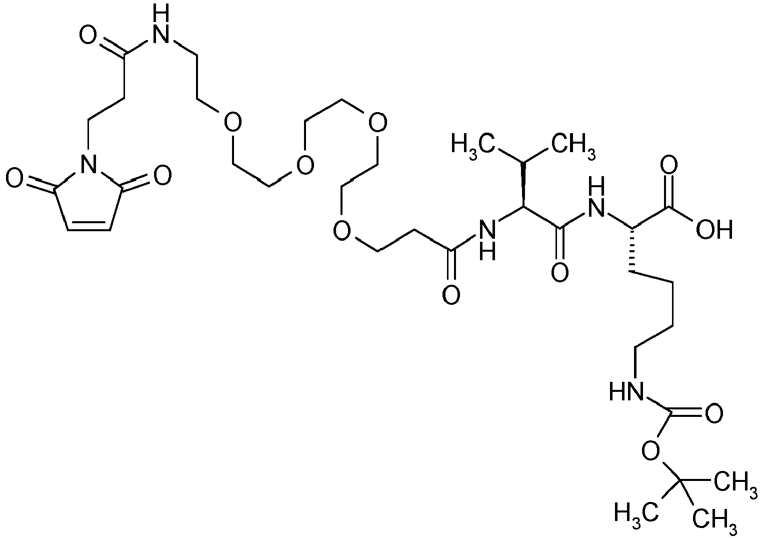

600 mg (1.58 mmol) N2-[(Benzyloxy)carbonyl]-N6-(tert-butoxycarbonyl)-L-lysin wurden in 25.0 mL Wasser/Ethanol/THF (1: 1:0.5) mittels Palladium auf Kohle (10 %) bei RT unter Normaldruck mit Wasserstoff hydriert. Die Verbindung N6-(tert-Butoxycarbonyl)-L-lysin wird ohne weitere Reinigung in der nächsten Synthesestufe eingesetzt.

LC-MS (Methode 1): Rₜ = 0.99 min; MS (ESIpos): m/z = 247 [M+H]⁺.

180.0 (0.73 mmol) N6-(tert-Butoxycarbonyl)-L-lysin wurden in 5.0 mL DMF gelöst und mit 74.0 mg (0.73 mmol) Triethylamin versetzt. Es wurden 254.6 mg (0.73 mmol) 2,5-Dioxopyrrolidin-1-yl-N-[(benzyloxy)carbonyl]-L-valinat und 74.0 mg (0.73 mmol) Triethylamin zugegeben. Die Reaktionsmischung wurde 3.5 h bei RT gerührt. Die Reaktionsmischung wurde direkt mittles präp. RP-HPLC gereinigt (Säule: Reprosil 250x30; 10µ, Fluss: 50 mL/min, MeCN/Wasser, 0.1 % TFA). Die Lösemittel wurden dann im Vakuum verdampft und der Rückstand im Hochvakuum getrocknet. Man erhielt 294.1 mg (76 % d. Th.) der Verbindung N-[(Benzyloxy)carbonyl]-L-valyl-N6-(tert-butoxycarbonyl)-L-lysin.

LC-MS (Methode 1): Rₜ = 0.97 min; MS (ESIpos): m/z = 480 [M+H]⁺.

272.2 mg (0.57 mmol) N-[(Benzyloxy)carbonyl]-L-valyl-N6-(tert-butoxycarbonyl)-L-lysin wurden in 20.0 mL Ethylacetat/Ethanol/THF (1:1:1) gelöst und mit 27.2 mg Palladium auf Aktivkohle versetzt und unter Normaldruck und bei RT mit Wasserstoff hydriert. Es wurde über Celite^{(R)} filtriert und der Filterkuchen intensiv mit Ethylacetat/Ethanol/THF (1:1:1) gewaschen. Die Lösemittel wurden im Vakuum verdampft und der Rückstand im Hochvakuum getrocknet. Man erhielt 182.0 mg (72 % d. Th.) der Verbindung L-Valyl-N6-(tert-butoxycarbonyl)-L-lysin.

LC-MS (Methode 1): Rₜ = 0.53 min; MS (ESIpos): m/z = 346 [M+H]⁺.

30.0 mg (0.07 mmol) L-Valyl-N6-(tert-butoxycarbonyl)-L-lysin und 34.3 mg (0.07 mmol) 3-(2,5-Dioxo-2,5-dihydro-1H-pyrrol-1-yl)-N-{ 15-[(2,5-dioxopyrrolidin-1-yl)oxy]-15-oxo-3,6,9,12-tetraoxapentadec-1-yl}propanamid wurden in 1.5 mL DMF gelöst und mit 6.8 mg (0.07 mmol) 4-Methylmorpholin versetzt. Dir Reaktionsmischung wurde über Nacht bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 40.6 mg (82 % d. Th.) der Titelverbindung.

LC-MS (Methode 1): Rₜ = 0.73 min; MS (ESIpos): m/z = 744 [M+H]⁺.

### Intermediat L42

### N-[19-(2,5-Dioxo-2,5-dihydro-1H-pyrrol-1-yl)-17-oxo-4,7,10,13-tetraoxa-16-azanonadecan-1-oyl]-L-valyl-N5-carbamoyl-L-ornithin

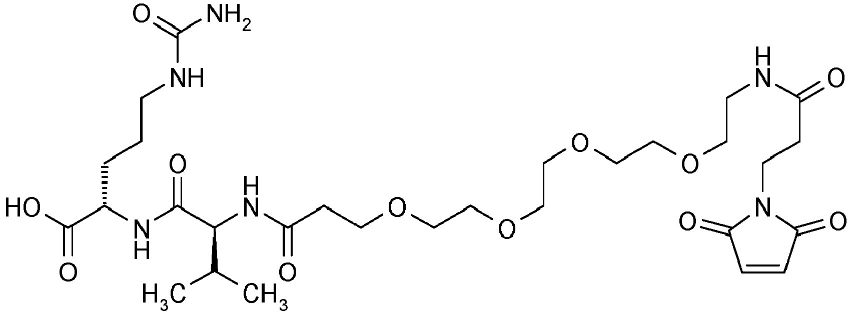

50.0 mg (0.18 mmol) L-Valyl-N5-carbamoyl-L-ornithin (Intermediat L37) wurden in DMF vorgelegt und mit 93.6 mg (0.18 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid und 36.9 mg (0.37 mmol) Triethylamin versetzt. Die Reaktionsmischung rührte bei RT über Nacht. Es wurden 21.9 mg (0.37 mmol) HOAc hinzugefügt und die Reaktionsmischung direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 20.6 mg (14 % d. Th.) der Titelverbindung.

LC-MS (Methode 1): Rₜ = 0.55 min; MS (ESIpos): m/z = 673 [M+H]⁺.

### Intermediat L43

### N-[67-(2,5-Dioxo-2,5-dihydro-1H-pyrrol-1-yl)-65-oxo-4,7,10,13,16,19,22,25,28,31,34,37,40,43,46,49,52,55,58,61-icosaoxa-64-azaheptahexacontan-1-oyl]-L-valyl-N5-carbamoyl-L-ornithin

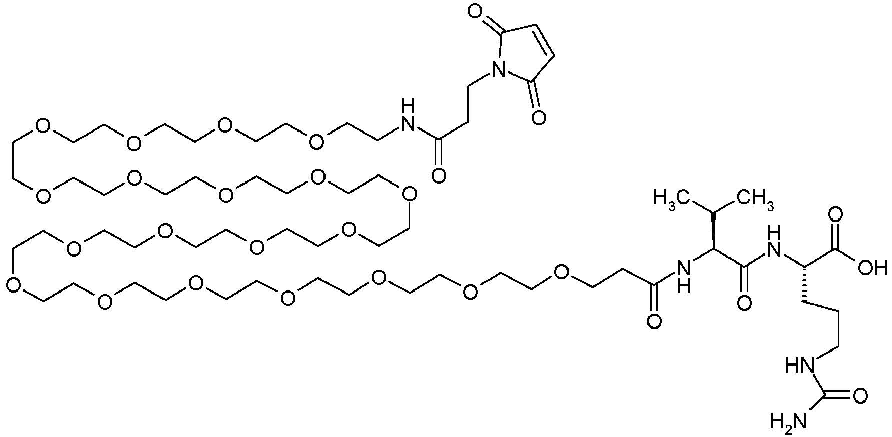

11.3 mg (0.04 mmol) L-Valyl-N5-carbamoyl-L-ornithin (Intermediat L37) wurden in DMF vorgelegt und mit 50.0 mg (0.04 mmol) 3-(2,5-Dioxo-2,5-dihydro-1H-pyrrol-1-yl)-N-{63-[(2,5-dioxopyrrolidin-1-yl)oxy]-63-oxo-3,6,9,12,15,18,21,24,27,30,33,36,39,42,45,48,51,54,57,60-icosaoxatrihexacont-1-yl}propanamid und 8.3 mg (0.08 mmol) Triethylamin versetzt. Die Reaktionsmischung rührte bei RT über Nacht. Es wurden 4.9 mg (0.08 mmol) HOAc hinzugefügt und die Reaktionsmischung direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 15.8 mg (20 % d. Th.) der Titelverbindung.

LC-MS (Methode 4): Rₜ = 0.94 min; MS (ESIpos): m/z = 1377 [M+H]⁺.

### Intermediat L44

### N-[19-(2,5-Dioxo-2,5-dihydro-1H-pyrrol-1-yl)-17-oxo-4,7,10,13-tetraoxa-16-azanonadecan-1-oyl]-L-valyl-L-alanin

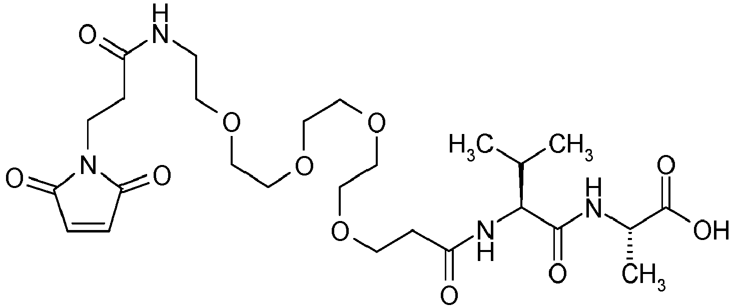

73.3 mg (0.39 mmol) L-Valyl-L-alanin wurden in 7.0 mL DMF gelöst und mit 200.0 mg (0.39 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid und 78.8 mg (0.78 mmol) Triethylamin versetzt. Die Reaktionsmischung wurde bei RT über Nacht gerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 103.3 mg (45 % d. Th.) der Titelverbindung.

LC-MS (Methode 1): Rₜ = 0.58 min; MS (ESIpos): m/z = 587 [M+H]⁺.

### Intermediat L45

### tert-Butyl-(2S)-2-[(tert-butoxycarbonyl)amino]-4-oxobutanoat

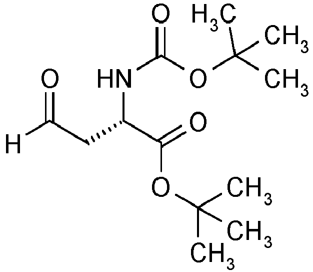

2.00 g (7.26 mmol) tert-Butyl-N-(tert-butoxycarbonyl)-L-homoserinat wurden in 90 ml Dichlormethan gelöst und dann mit 1.76 ml Pyridin sowie mit 4.62 g (10.90 mmol) 1,1,1-Triacetoxy-1lambda⁵,2-benziodoxol-3(1H)-on (Dess-Martin-Periodinan) versetzt. Der Ansatz wurde 2 h bei RT gerührt, anschließend mit 200 ml Dichlormethan verdünnt und zweimal mit 10%-iger Natriumtiosulfat-Lösung und dann nacheinander zweimal mit 5%-iger Zitronensäure und zweimal mit gesättigter Natriumhydrogencarbonat-Lösung ausgeschüttelt. Die organische Phase wurde abgetrennt, über Natriumsulfat getrocknet und anschließend im Vakuum eingeengt. Der Rückstand wurde mit 100 ml Diethylether und Cyclohecan (v/v=1:1) versetzt, etwas eingeengt, wobei ein weißer Niederschlag ausfiel. Dieser wurde abgesaugt. Das Filtrat wurde am Rotationsverdampfer eingeengt und im Hochvakuum getrocknet, wobei 1.74 g (88% d. Th.) der Zielverbindung als hellgelbes Öl erhalten wurden.

LC-MS (Methode 1): Rₜ = 0.85 min; MS (ESIpos): m/z = 274 [M+H]⁺.

¹H-NMR (400 MHz, DMSO-d₆): δ [ppm] = 1.38 (s, 18H), 2.64-2.81 (m, 2H), 4.31-4.36 (m, 1H), 7.23 (d, 1H), 9.59 (s, 1H).

### Intermediat L46

### Trifluoressigsäure--tert-butyl-N-[2-(2,5-dioxo-2,5-dihydro-1H-pyrrol-1-yl)ethyl]-L-glutaminat(1:1)

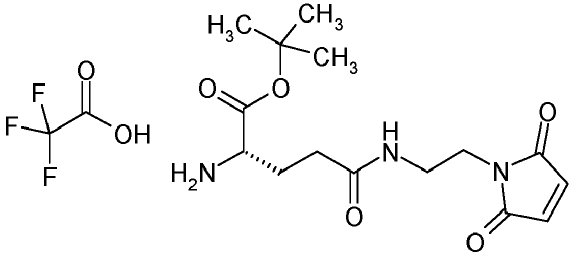

Die Titelverbindung wurde zunächst durch Kupplung von 200 mg (0.79 mmol) Trifluoressigsäure- -1-(2-aminoethyl)-1H-pyrrol-2,5-dion(1:1) mit 263 mg (0.87 mmol) (4S)-5-tert-Butoxy-4-[(tert-butoxycarbonyl)amino]-5-oxopentansäure-trifluoressigsäure(1:1) in Gegenwart von EDC/HOBT und *N,N*-Diisopropylethylamin und anschließende Entschützung der Aminogruppe unter schonenden Bedingungen durch 1h Rühren in 10%iger Trifluoressigsäure in DCM bei RT hergestellt. Nach Gefriertrocknung aus Acetonitril/ Wasser wurden 85 mg (20% d.Th.) der Titelverbindung über 2 Stufen erhalten.

LC-MS (Methode 1): Rₜ = 0.37 min; MS (ESIpos): m/z = 326 [M+H]⁺.

### Intermediat L47

### Trifluoressigsäure--beta-alanyl-L-alanyl-N⁵-carbamoyl-N-[4-(2,5-dioxo-2,5-dihydro-1H-pyrrol-1-yl)phenyl] -L-ornithinamid(1: 1)

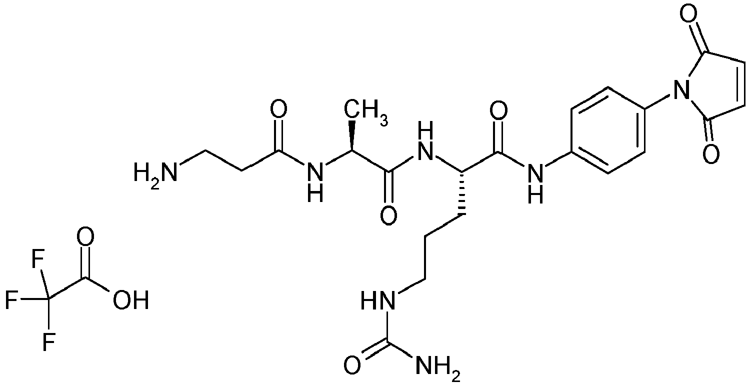

Die Titelverbindung wurde durch Kupplung von Intermediat L8 mit 2,5-Dioxopyrrolidin-1-yl-N-(tert-butoxycarbonyl)-beta-alaninat und anschließender Entschützung mit TFA hergestellt.

LC-MS (Methode 3): Rₜ = 1.36 min; MS (ESIpos): m/z = 488 (M+H)⁺.

### Intermediat L48

### Trifluoressigsäure--(1R,2S)-2-amino-N-[2-(2,5-dioxo-2,5-dihydro-1H-pyrrol-1-yl)ethyl]cyclopentancarboxamid(1:1)

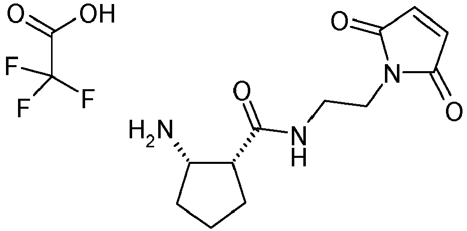

Die Titelverbindung wurde ausgehend von kommerziell erhältlicher (1R, 2S)-2-[(tert-Butoxycarbonyl)amino]cyclopentancarbonsäure in Analogie zu Intermediat L2 hergestellt.

LC-MS (Methode 3): Rₜ = 1.22 min; MS (ESIpos): m/z = 252 (M+H)⁺.

### Intermediat L49

### Trifluoressigsäure--tert-butyl-N-(bromacetyl)-L-valyl-L-alanyl-L-lysinat(1: 1)

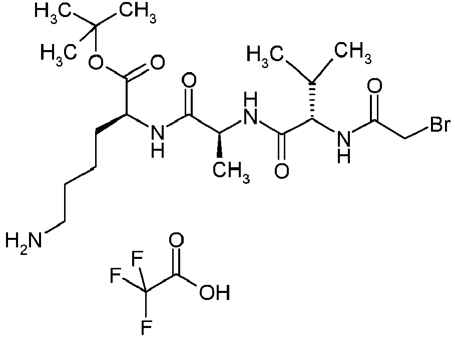

Die Titelverbindung wurde zunächst durch Kupplung von kommerziell erhältlichem Bromessigsäureanhydrid mit dem nach klassischen Methoden der Peptidchemie hergestellten partiell geschützten Peptid tert-Butyl-L-valyl-L-alanyl-N⁶-(tert-butoxycarbonyl)-L-lysinat in Gegenwart von *N,N-*Diisopropylethylamin in Dichlormethan hergestellt. Anschließend erfolgte die Entschützung an der Aminogruppe unter schonenden Bedingungen durch Rühren in 10%iger Trifluoressigsäure in DCM bei RT, wobei in 49%-iger Ausbeute über 2 Stufen die Titelverbindung erhalten wurde.

LC-MS (Methode 1): Rₜ = 1.09 min; MS (ESIpos): m/z = 593 und 595 (M+H)⁺.

### Intermediat L50

### Trifluoressigsäure--(1S,3R)-3-amino-N-[2-(2,5-dioxo-2,5-dihydro-1H-pyrrol-1-yl)ethyl]cyclopentancarboxamid(1:1)

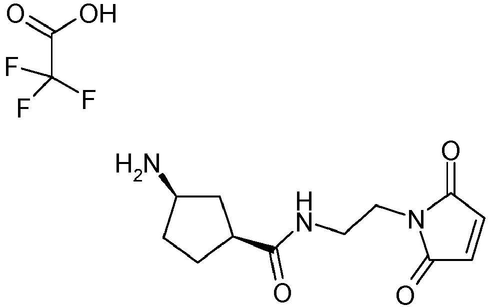

Die Titelverbindung wurde ausgehend von kommerziell erhältlicher (1S,3R)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von N,NDiisopropylethylamin und anschließender Entschützung mit TFA hergestellt.
HPLC (Methode 11): Rₜ = 0.2 min;
LC-MS (Methode 3): Rₜ = 0.88 min; MS (ESIpos): m/z = 252 (M+H)⁺.

### Intermediat L51

### Trifluoressigsäure--(1R,3R)-3-amino-N-[2-(2,5-dioxo-2,5-dihydro-1H-pyrrol-1-yl)ethyl]cyclopentancarboxamid(1:1)

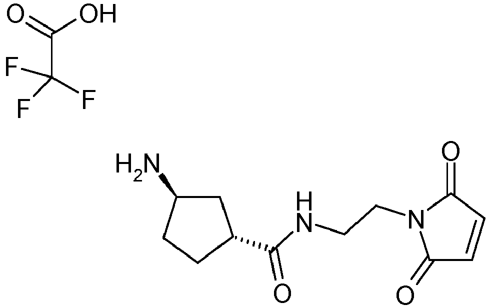

Die Titelverbindung wurde ausgehend von kommerziell erhältlicher (1R,3R)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von *N,N-*Diisopropylethylamin und anschließender Entschützung mit TFA hergestellt.

LC-MS (Methode 3): Rₜ = 0.98 min; MS (ESIpos): m/z = 250 (M-H)⁻.

### Intermediat L52

### Trifluoressigsäure --N-(2-aminoethyl)-2-bromacetamid (1:1)

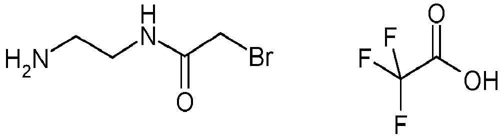

420 mg (2.62 mmol) tert-Butyl-(2-aminoethyl)carbamat wurden in 50 ml Dichlormethan aufgenommen und mit 817 mg (3.15 mmol) Bromessigsäureanhydrid sowie 913 µl (5.24 mmol) *N,N*-Diisopropylethylamin versetzt. Der Ansatz wurde 1 h bei RT gerührt und anschließend im Vakuum eingeengt. Der Rückstand wurde duch präparative HPLC gereinigt.

Es wurden 577 mg der geschützten Zwischenstufe erhalten, die anschließend in 50 ml Dichlormethan aufgenommen und mit 10 ml Trifluoressigsäure versetzt wurde. Nach 1 h Rühren bei RT wurde der Ansatz im Vakuum eingeengt und der Rückstand aus Acentonitril/Wasser lyophilisert. So wurden 705 mg (65% d. Th.) der Titelverbindung erhalten.

LC-MS (Methode 3): Rₜ = 0.34 min; MS (ESIpos): m/z = 181 und 183 (M+H)⁺.

### Intermediat L53

### Trifluoressigsäure--(1S,3S)-3-amino-N-[2-(2,5-dioxo-2,5-dihydro-1H-pyrrol-1-yl)ethyl]cyclopentancarboxamid(1:1)

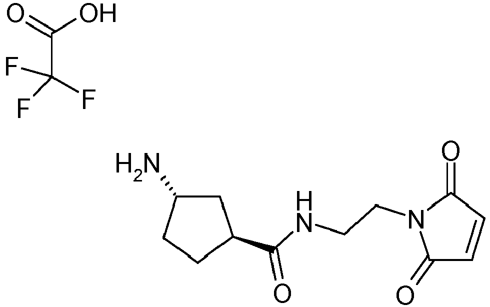

Die Titelverbindung wurde ausgehend von kommerziell erhältlicher (1S,3S)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von *N,N-*Diisopropylethylamin und anschließender Entschützung mit TFA hergestellt.
HPLC (Methode 11): Rₜ = 0.19 min;
LC-MS (Methode 3): Rₜ = 0.88 min; MS (ESIpos): m/z = 250 (M-H)⁻.

### Intermediat L54

### Trifluoressigsäure--(1R,3S)-3-amino-N-[2-(2,5-dioxo-2,5-dihydro-1H-pyrrol-1-yl)ethyl]cyclopentancarboxamid(1:1)

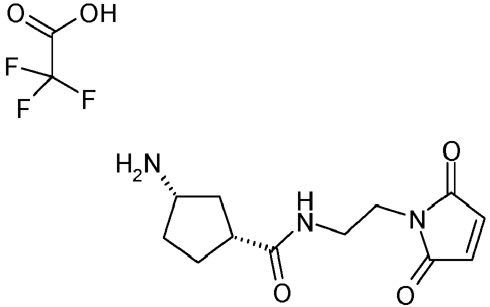

Die Titelverbindung wurde ausgehend von kommerziell erhältlicher (1R,3S)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von N,NDiisopropylethylamin und anschließender Entschützung mit TFA hergestellt.

LC-MS (Methode 3): Rₜ = 0.89 min; MS (ESIpos): m/z = 252 (M+H)⁺.

### Intermediat L55

### Trifluoressigsäure--tert-butyl-N⁶-D-alanyl-N²-{N-[6-(2,5-dioxo-2,5-dihydro-1H-pyrrol-1-yl)hexanoyl]-L-valyl-L-alanyl}-L-lysinat (1:1)

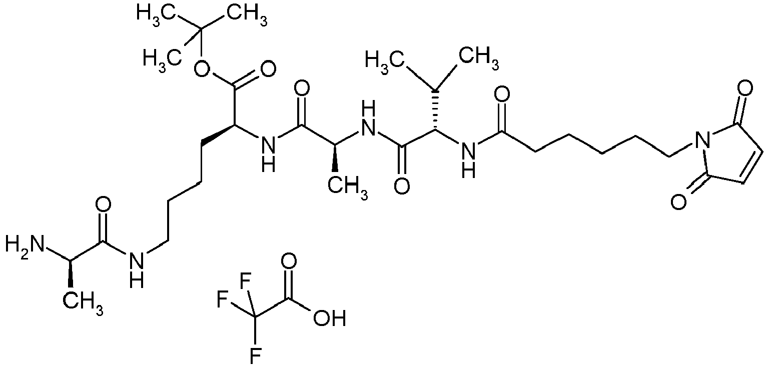

Die Titelverbindung wurde zunächst durch Kupplung von Intermediat L6 mit N-(tert-Butoxycarbonyl)-D-alanin in Gegenwart von HATU und anschließender Entschützung an der Aminogruppe unter schonenden Bedingungen durch 90 minütiges Rühren in 5%iger Trifluoressigsäure in DCM bei RT hergestellt.
HPLC (Methode 11): Rₜ = 1.35 min;
LC-MS (Methode 1): Rₜ = 0.67 min; MS (ESIpos): m/z = 637 (M+H)⁺.

### Intermediat L56

### Trifluoressigsäure --tert-butyl-N-[6-(2,5-dioxo-2,5-dihydro-1H-pyrrol-1-yl)hexanoyl]-L-valyl-L-alanyl-N⁶-{[(1R,3S)-3-aminocyclopentyl]carbonyl}-L-lysinat (1:1)

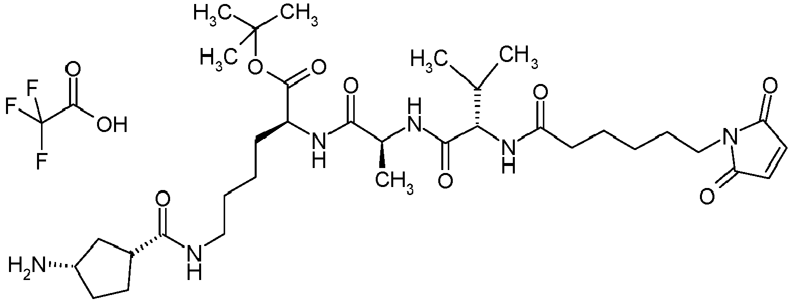

Die Titelverbindung wurde zunächst durch Kupplung von Intermediat L6 mit (1R,3S)-3-[(tert-Butoxycarbonyl)amino]cyclopentancarbonsäure in Gegenwart von HATU und anschließender Entschützung an der Aminogruppe unter schonenden Bedingungen durch 15 minütiges Rühren in 25%-iger Trifluoressigsäure in DCM bei RT hergestellt.
HPLC (Methode 11): Rₜ = 1.4 min;
LC-MS (Methode 1): Rₜ = 0.7 min; MS (ESIpos): m/z = 677 (M+H)⁺.

### Intermediat L57

### Methyl-(2S)-4-oxo-2-({[2-(trimethylsilyl)ethoxy]carbonyl}amino)butanoat

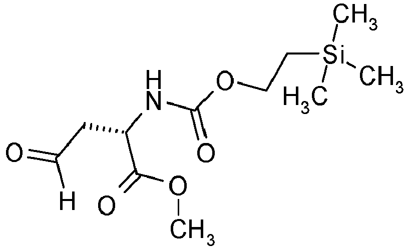

500.0 mg (2.72 mmol) L-Asparaginsäuremethylester Hydrochlorid und 706.3 mg (2.72 mmol) 2-(Trimethylsilyl)ethyl-2,5-dioxopyrrolidin-1-carboxylat wurden in 5.0 mL 1,4-Dioxan vorgelegt und mit 826.8 mg (8.17 mmol) Triethylamin versetzt. Die Reaktionsmischung wurde über Nacht bei RT gerührt. Die Reaktionsmischung wurde direkt mittles präp. RP-HPLC gereinigt (Säule: Reprosil 250x40; 10µ, Fluss: 50 mL/min, MeCN/Wasser, 0.1 % TFA). Die Lösemittel wurden dann im Vakuum verdampft und der Rückstand im Hochvakuum getrocknet. Man erhielt 583.9 mg (74 % d. Th.) der Verbindung (3S)-4-Methoxy-4-oxo-3-({[2-(trimethylsilyl)ethoxy]carbonyl}amino) butansäure.

LC-MS (Methode 1): Rₜ = 0.89 min; MS (ESIneg): m/z = 290 (M-H)⁻.

592.9 mg (3S)-4-Methoxy-4-oxo-3-({[2-(trimethylsilyl)ethoxy]carbonyl}amino)butansäure wurden in 10.0 mL 1,2-Dimethoxyethan vorgelegt und auf -15 °C abgekühlt und mit 205.8 mg (2.04 mmol) 4-Methylmorpholin und 277.9 mg (2.04 mmol) Isobutylchlorformiat versetzt. Der Niederschlag wird nach 15 min abgesaugt und zweimal mit je 10.0 mL 1,2-Dimethoxyethan gewaschen. Das Filtrat wird auf -10 °C abgekühlt und mit 115.5 mg (3.05 mmol) Natriumborhydrid gelöst in 10 mL Wasser unter starkem Rühren versetzt. Die Phasen werden getrennt und die organische Phase je einmal mit ges. Natriumhydrogencarbonat-Lösung und ges. NaCl-Lösung gewaschen. Die organische Phase wurde über Magnesiumsulfat getrocknet und das Lösemittel im Vakuum verdampft und der Rückstand im Hochvakuum getrocknet. Man erhielt 515.9 mg (91 % d. Th.) der Verbindung Methyl-N-{[2-(trimethylsilyl)ethoxy]carbonyl}-L-homoserinat.

LC-MS (Methode 1): Rₜ = 0.87 min; MS (ESIpos): m/z = 278 (M+H)⁺.

554.9 mg (2.00 mmol) Methyl-N-{[2-(trimethylsilyl)ethoxy]carbonyl}-L-homoserinat wurden in 30.0 mL Dichlormethan vorgelegt und mit 1.27 g (3.0 mmol) Dess-Martin periodinan und 474.7 mg (6.00 mmol) Pyridin versetzt. Man rührte über Nacht bei RT. Nach 4 h wurde der Ansatz mit Dichlormethan verdünnt und die organische Phase je dreimal mit 10%iger Na₂S₂O₃-Lösung, 10%iger Citronensäure-Lösung und ges. Natriumhydrogencarbonat-Lösung gewaschen. Die organische Phase wurde über Magnesiumsulfat getrocknet und das Lösemittel im Vakuum verdampft. Man erhielt 565.7 mg (97 % d. Th.) der Titelverbindung.

¹H-NMR (400 MHz, DMSO-d₆): δ [ppm] = 0.03 (s, 9H), 0.91 (m, 2H), 2.70-2.79 (m, 1H), 2.88 (dd, 1H), 3.63 (s, 3H), 4.04 (m, 2H), 4.55 (m, 1H), 7.54 (d, 1H), 9.60 (t, 1H).

### Intermediat L58

### 2-(Trimethylsilyl)ethyl-(3-oxopropyl)carbamat

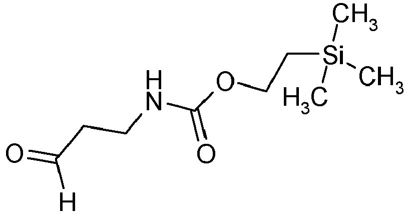

434.4 mg (5.78 mmol) 3-Amino-1-propanol und 1.50 g (5.78 mmol) 2-(Trimethylsilyl)ethyl-2,5-dioxopyrrolidin-1-carboxylat wurden in 10.0 mL Dichlormethan gelöst und mit 585.3 mg (5.78 mmol) Triethylamin versetzt und über Nacht bei RT gerührt. Die Reaktionsmischung wurde mit Dichlormethan verdünnt und die organische Phase mit Wasser und ges. Natriumhydrogencarbonat-Lösung gewaschen und anschließend über Magnesiumsulfat getrocknet. Das Lösemittel wurde im Vakuum verdampft. Der Rückstand 2-(Trimethylsilyl)ethyl-(3-hydroxypropyl)carbamat (996.4 mg, 79 % d. Th.) wurde im Hochvakuum getrocknet und ohne weitere Reinigung in der nächsten Synthesestufe eingesetzt.

807.0 mg (3.68 mmol) 2-(Trimethylsilyl)ethyl-(3-hydroxypropyl)carbamat wurden in 15.0 mL Chloroform und 15.0 mL 0.05 N Kaliumcarbonat/0.05 N Natriumhydrogencarbonat-Lösung (1:1) vorgelegt. Dann wurden 102.2 mg (0.37 mmol) Tetra-n-butylammoniumchlorid, 736.9 mg (5.52 mmol) N-Chlorsuccinimid und 57.5 mg (0.37 mmol) TEMPO zugegeben und die Reaktionsmischung kräftig über Nacht bei RT gerührt. Die Reaktionsmischung wurde mit Dichlormethan verdünnt und die organische Phase wurde mit Wasser und ges. NaCl-Lösung gewaschen. Die organische Phase wurde über Magnesiumsulfat getrocknet und das Lösemittel im Vakuum verdampft. Der Rückstand wurde im Hochvakuum getrocknet und ohne weitere Reinigung in der nächsten Synthesestufe eingesetzt (890.3 mg).

### Intermediat L59

### Trifluoressigsäure--1-{2-[2-(2-aminoethoxy)ethoxy]ethyl}-1H-pyrrol-2,5-dion(1:1)

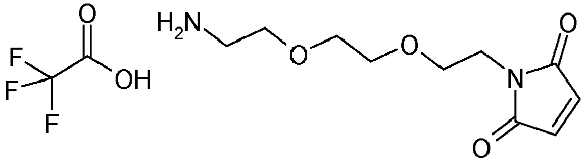

300.0 mg (0.91 mmol) tert-Butyl-(2-{2-[2-(2,5-dioxo-2,5-dihydro-1H-pyrrol-1-yl)ethoxy]ethoxy}ethyl)carbamat wurden in Dichlormethan vorgelegt und mit 4.2 g (36.54 mmol) TFA versetzt und 1 h bei RT gerührt (DC-Kontrolle: Dichlormethan/Methanol 10:1). Die flüchtigen Bestandteile wurden im Vakuum verdampft und der Rückstand viermal mit Dichlormethan kodestilliert. Der Rückstand wurde im Hochvakuum getrocknet und ohne weitere Reinigung in der nächsten Synthesestufe eingesetzt.

LC-MS (Methode 1): Rₜ = 0.19 min; MS (ESIpos): m/z = 229 (M+H)⁺.

### Intermediat L60

### 6-(2,5-Dioxo-2,5-dihydro-1H-pyrrol-1-yl)hexanoylchlorid

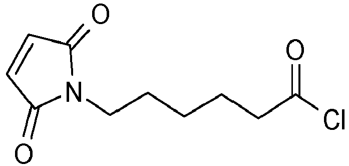

200.0 mg (0.95 mmol) 6-(2,5-Dioxo-2,5-dihydro-1H-pyrrol-1-yl)hexansäure wurden in 4.0 mL Dichlormethan gelöst und mit 338.0 mg (2.84 mmol) Thionylchlorid versetzt. Die Reaktionsmischung wurde 3 h bei RT gerührt und dann mit 1 Tropfen DMF vesetzt. Es wurde nochmal 1 h gerührt. Das Lösemittel wurde im Vakuum verdampft und es wurde dreimal mit Dichlormethan kodestilliert. Das Rohprodukt wurde ohne weitere Reinigung in der nächsten Synthesestufe eingesetzt.

### Intermediat L61

### Trifluoressigsäure --2-(trimethylsilyl)ethyl-N-[6-(2,5-dioxo-2,5-dihydro-1H-pyrrol-1-yl)hexanoyl]-L-valyl-L-alanyl-L-lysinat (1:1)

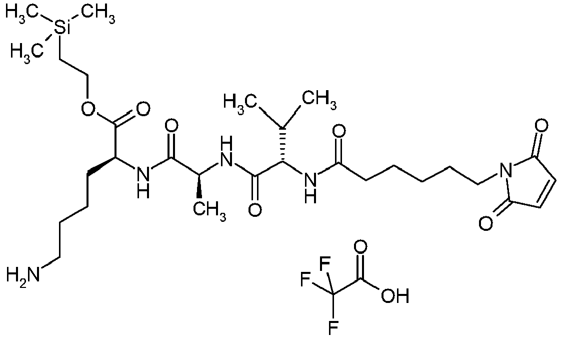

Zunächst wurde ausgehend von N2-[(Benzyloxy)carbonyl]-N6-(tert-butoxycarbonyl)-L-lysin nach klassischen Methoden der Peptidchemie (Veresterung mit 2-(Trimethylsilylethanol mittels EDCI/DMAP, Hydrogenolyse, Kupplung mit N-[(Benzyloxy)carbonyl]-L-valyl-L-alanin in Gegenwart von HATU und erneute Hydrogenolyse) das Tripeptidderivat 2-Trimethylsilyl)ethyl-L-valyl-L-alanyl-N6-(tert-butoxycarbonyl)-L-lysinat hergestellt. Die Titelverbindung wurde durch Kupplung dieses partiell geschützten Peptidderivats mit kommerziell erhältlicher 6-(2,5-Dioxo-2,5-dihydro-1H-pyrrol-1-yl)hexansäure in Gegenwart von HATU und *N,N*-Diisopropylethylamin hergestellt. Anschließend erfolgte die Entschützung an der Aminogruppe unter schonenden Bedingungen durch 2.5 stündiges Rühren in 5%iger Trifluoressigsäure in DCM bei RT unter Erhalt der Esterschutzgruppe. Nach Aufarbeitung und Reinigung durch präparative HPLC wurden 438 mg der Titelverbindung erhalten.
HPLC (Methode 11): Rₜ = 1.69 min;
LC-MS (Methode 1): Rₜ = 0.78 min; MS (ESIpos): m/z = 610 (M+H)⁺.

### Intermediat L62

### Trifluoressigsäure --2-(trimethylsilyl)ethyl-N-[6-(2,5-dioxo-2,5-dihydro-1H-pyrrol-1-yl)hexanoyl]-L-valyl-N⁵-carbamoyl-L-ornithyl-L-lysinat (1:1)

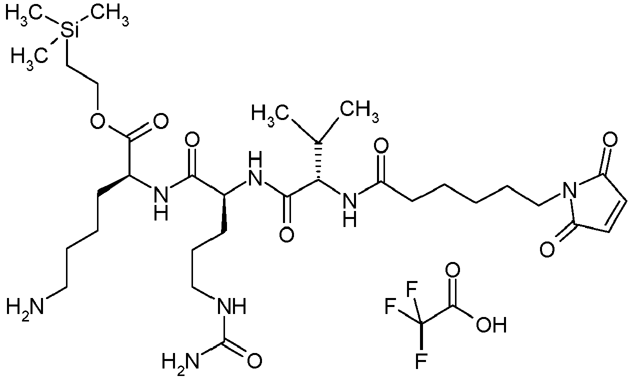

Zunächst wurde ausgehend von N2-[(Benzyloxy)carbonyl]-N6-(tert-butoxycarbonyl)-L-lysin nach klassischen Methoden der Peptidchemie 2-(Trimethylsilyl)ethyl-N6-(tert-butoxycarbonyl)-L-lysinat hergestellt. 148 mg (0.43 mmol) von diesem Intermediat wurden dann in Gegenwart von 195 mg (0.51 mmol) HATU und 149 µL *N,N*-Diisopropylethylamin mit 200 mg (0.43 mmol) von Intermediat L16 gekuppelt. Nach Einengen und Reinigung des Rückstandes mittels präparativer HPLC wurde das geschützte Intermediat in 20 mL DCM aufgenommen und durch Zugabe von 2 mL Trifluoressigsäure und 1 h Rühren bei RT die tert. Butoxycarbonyl-Schutzgruppe abgelöst. Nach Einengen und Lyophilisation des Rückstandes aus Acetonitril/Wasser wurden 254 mg (63% d. Th. über 2 Stufen) erhalten.
HPLC (Methode 11): Rₜ = 1.51 min;
LC-MS (Methode 1): Rₜ = 0.68 min; MS (ESIpos): m/z = 696 (M+H)⁺.

### Intermediat L63

### (4S)-4-{[(2S)-2-{[(2S)-2-{[6-(2,5-Dioxo-2,5-dihydro-1H-pyrrol-1-yl)hexanoyl]amino}-3-methylbutanoyl] amino }propanoyl] amino } -5 -oxo-5 -[2-(trimethylsilyl)ethoxy]pentansäure

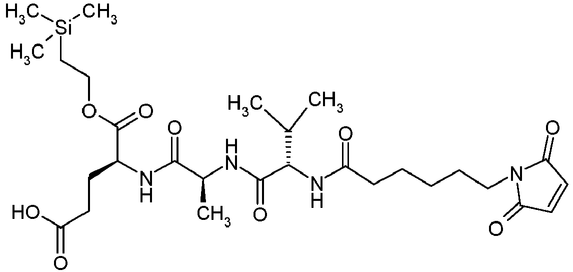

Zunächst wurde ausgehend von (2S)-5-(Benzyloxy)-2-[(tert-butoxycarbonyl)amino]-5-oxopentansäure nach klassischen Methoden der Peptidchemie (Veresterung mit 2-(Trimethylsilylethanol mittels EDCI/DMAP, Abspaltung der Boc-Schutzgruppe mit Trifluoressigsäure, Kupplung mit N-[(Benzyloxy)carbonyl]-L-valyl-L-alanin in Gegenwart von HATU und Hydrogenolyse in Methanol über 10%-igem Palladium auf Aktivkohle) das Tripeptidderivat (4S)-4-{[(2S)-2-{[(2S)-2-Amino-3-methylbutanoyl]amino}propanoyl]amino}-5-oxo-5-[2-(trimethylsilyl)ethoxy]pentansäure hergestellt. Die Titelverbindung wurde durch Kupplung dieses partiell geschützten Peptidderivats mit kommerziell erhältlichem 1-{6-[(2,5-Dioxopyrrolidin-1-yl)oxy]-6-oxohexyl}-1H-pyrrol-2,5-dion hergestellt. Nach Aufarbeitung und Reinigung durch präparative HPLC wurden 601 mg der Titelverbindung erhalten.

LC-MS (Methode 1): Rₜ = 0.96 min; MS (ESIpos): m/z = 611 (M+H)⁺.

### Intermediat L64

### (4S)-4-{[(2,5-Dioxo-2,5-dihydro-1H-pyrrol-1-yl)acetyl]amino}-5-oxo-5-[2-(trimethylsilyl)ethoxy] pentansäure

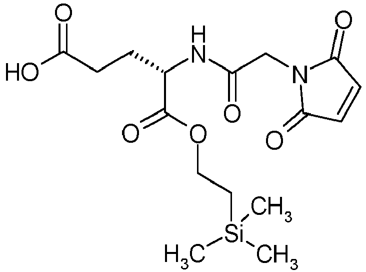

Die Titelverbindung wurde ausgehend von (2S)-5-(Benzyloxy)-2-[(tert-butoxycarbonyl)amino]-5-oxopentansäure nach klassischen Methoden der Peptidchemie (Veresterung mit 2-(Trimethylsilylethanol mittels EDCI/DMAP, Abspaltung der Boc-Schutzgruppe mit Trifluoressigsäure, hydrogenolytische Spaltung des Benzylesters in Methanol über 10%-igem Palladium auf Aktivkohle und Kupplung mit 1-{2-[(2,5-Dioxopyrrolidin-1-yl)oxy]-2-oxoethyl}-1H-pyrrol-2,5-dion in Gegenwart von *N,N*-Diisopropylethylamin) hergestellt.

LC-MS (Methode 1): Rₜ = 0.84 min; MS (ESIpos): m/z = 385 (M+H)⁺.

### Intermediat L65

### Trifluoressigsäure --2-(trimethylsilyl)ethyl-3-{[(benzyloxy)carbonyl]amino }-L-alaninat (1:1)

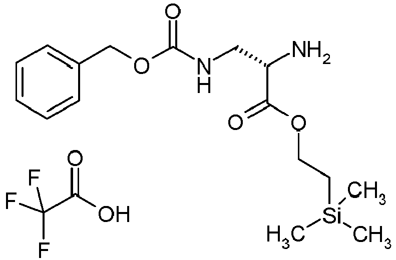

Die Titelverbindung wurde ausgehend von 3-{[(Benzyloxy)carbonyl]amino}-N-(tert-butoxycarbonyl)-L-alanin nach klassischen Methoden der Peptidchemie (Veresterung mit 2-(Trimethylsilylethanol mittels EDCI/DMAP und Abspaltung der Boc-Schutzgruppe mit Trifluoressigsäure hergestellt. Man erhielt 373 mg (79 % d. Th. über 2 Stufen) der Titelverbindung.

LC-MS (Methode 1): Rₜ = 0.72 min; MS (ESIpos): m/z = 339 (M+H)⁺.

### Intermediat L66

### Methyl-(8S)-8-(2-hydroxyethyl)-2,2-dimethyl-6,11-dioxo-5-oxa-7,10-diaza-2-silatetradecan-14-oat

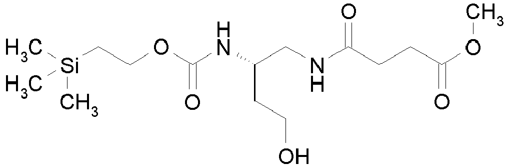

1000 mg (2.84 mmol) (3S)-3-{[(Benzyloxy)carbonyl]amino}-4-[(tert-butoxycarbonyl)amino] butansäure wurden in 10.0 mL 1,2-Dimethoxyethan vorgelegt und mit 344.4 mg (3.4 mmol) 4-Methylmorpholin und 504 mg (3.69 mmol) Isobutylchlorformiat versetzt. Nach 10min Rühren bei RT wurde der Ansatz auf 5°C abgekühlt und portionsweise mit 161 mg (4.26 mmol) Natriumborhydrid gelöst in 3 mL Wasser unter starkem Rühren versetzt. Nach 1 h erfolgte nochmals eine Zugabe der gleichen Menge an Natriumborhydrid und der Ansatz wurde anschließend langsam auf RT erwärmt. Es wurden 170 ml Wasser zugegeben und der Ansatz dann viermal mit jeweils 200 ml Ethylacetat extrahiert. Die Phasen wurden getrennt und die organische Phase einmal mit Citronensäure und anschließend mit gesättigter Natriumhydrogencarbonat-Lösung gewaschen. Die organische Phase wurde über Magnesiumsulfat getrocknet und das Lösemittel im Vakuum verdampft und der Rückstand im Hochvakuum getrocknet. Man erhielt 760 mg (78 % d. Th.) der Verbindung Benzyl-tert-butyl-[(2S)-4-hydroxybutan-1,2-diyl]biscarbamat.

LC-MS (Methode 1): Rₜ = 0.84 min; MS (ESIpos): m/z = 339 (M+H)⁺.

760 mg (2.16 mmol) von diesem Intermediat wurden in 13 ml Chlorwasserstoff/Dioxan gelöst 20 min bei RT gerührt. Anschließend wurde der Ansatz auf 5 ml aufkonzentriert und mit Diethylether versetzt. Der Niederschlag wurde abfiltriert und aus Acetonitril/Wasser 1: 1 lyophilisiert.

Das so erhaltene Produkt wurde in 132 ml DMF gelöst und mit 345.5 mg (2.35 mmol) 4-Methoxy-4-oxobutansäure, 970 mg (2.55 mmol) HATU und 1025 µl *N,N*-Diisopropylethylamin versetzt. Die Mischung wurde 5 min bei RT gerührt. Das Lösungsmittel wurde im Vakuum entfernt und der verbliebene Rückstand mittels präparativer HPLC gereinigt. Die entsprechenden Fraktionen wurden vereinigt und das Acetonitril im Vakuum abgedampft. Die verbleibende wässrige Phase wurde zweimal mit Ethylacetat extrahiert und die organische Phase anschließend einggengt und im Hochvakuum getrocknet.

Das so erhaltene Zwischenprodukt wurde in Methanol aufgenommen und über 10%-igem Palladium auf Aktivkohle 1 h lang bei RT unter Wasserstoff-Normaldruck hydriert. Der Katalysator wurde danach abfiltriert und das Lösungsmittel im Vakuum entfernt.

247 mg von dieser entschützten Verbindung wurden in 20 ml DMF aufgenommen und mit 352 mg (1.36 mmol) 1-({[2-(Trimethylsilyl)ethoxy]carbonyl}oxy)pyrrolidin-2,5-dion sowie 592 µL *N,N-*Diisopropylethylamin versetzt. Die Reaktionsmischung wurde 1h bei RT gerührt, anschließend eingeengt und der Rückstand mittles präparativer HPLC gereinigt. Die Lösemittel wurden dann im Vakuum verdampft und der Rückstand im Hochvakuum getrocknet. Man erhielt über diese 5 Reaktionsschritte in einer 21%-igen Gesamtausbeute 218 mg der Titelverbindung.

LC-MS (Methode 1): Rₜ = 0.74 min; MS (ESIpos): m/z = 363 (M+H)⁺.

### Intermediat L67

### Trifluoressigsäure --2-(2,5-dioxo-2,5-dihydro-1H-pyrrol-1-yl)ethyl-beta-alaninat (1:1)

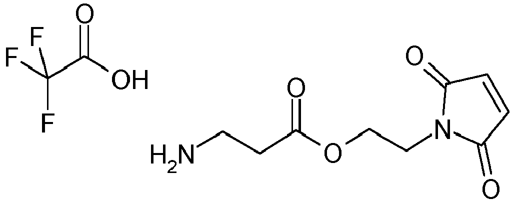

Die Titelverbindung wurde ausgehend von 50 mg (0.354 mmol) von kommerziell erhältlichem 1-(2-Hydroxyethyl)-1H-pyrrol-2,5-dion durch Kupplung mit 134 mg (0.71 mmol) N-(tert-Butoxycarbonyl)-beta-alanin in 10 ml Dichlormethan in Gegenwart von 1.5 Equivalenten EDCI und 0.1 Equivalent 4*-N, N*-Dimethylaminopyridin und anschließende Entschützung mit Trifluoressigsäure hergestellt.
Ausbeute: 56 mg (48% d. Th. über 2 Stufen)
LC-MS (Methode 3): Rₜ = 1.15 min; MS (ESIpos): m/z = 213 (M+H)⁺.

### Intermediat L68

### Trifluoressigsäure--N-(2-aminoethyl)-2-(2,5-dioxo-2,5-dihydro-1H-pyrrol-1-yl)propanamid(1:1)

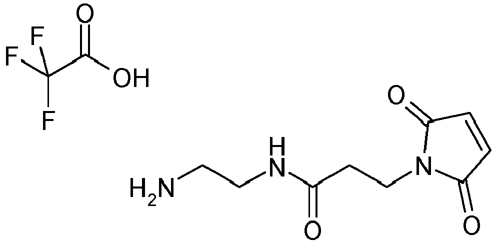

Die Titelverbindung wurde in Analogie zu Intermediat L1 nach klassischen Methoden der Peptidchemie aus kommerziell erhältlicher (2,5-Dioxo-2,5-dihydro-1H-pyrrol-1-yl)propansäure und tert-Butyl-(2-aminoethyl) carbamat hergestellt.

LC-MS (Methode 1): Rₜ = 0.17 min; MS (ESIpos): m/z = 212 (M+H)⁺.

### Intermediat L69

### Trifluoressigsäure--1-[(benzyloxy)carbonyl]piperidin-4-yl-L-valyl-N⁵-carbamoyl-L-ornithinat (1:1)

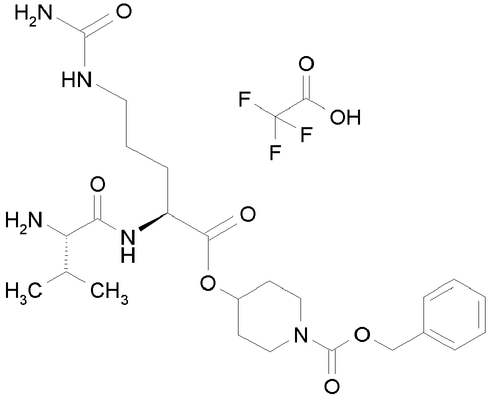

Die Titelverbindung wurde nach klassischen Methoden der Peptidchemie aus kommerziell erhältlichem Benzyl-4-hydroxypiperidin-1-carboxylat durch Veresterung mit N2-(tert-Butoxycarbonyl)-N5-carbamoyl-L-omithin mittels EDCI/DMAP, anschließende Boc-Spaltung mit TFA, dann folgende Kupplung mit N-[(tert.-Butoxy)carbonyl]-L-valin in Gegenwart von HATU und *N,N-*Diisopropylethylamin und schließlich erneute Boc-Abspaltung mit TFA hergestellt.

LC-MS (Methode 1): Rₜ = 0.62 min; MS (ESIpos): m/z = 492 (M+H)⁺.

### Intermediat L70

### 9H-Fluoren-9-ylmethyl-(3-oxopropyl)carbamat

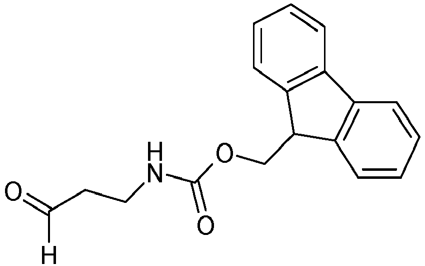

1000.0 mg (3.36 mmol) 9H-Fluoren-9-ylmethyl-(3-hydroxypropyl)carbamat wurden in 15.0 mL Chloroform und 15.0 mL 0.05 N Kaliumcarbonat/0.05 N Natriumhydrogencarbonat-Lösung (1:1) vorgelegt. Dann wurden 93.5 mg (0.34 mmol) Tetra-n-butylammoniumchlorid, 673.6 mg (5.04 mmol) N-Chlorsuccinimid und 52.5 mg (0.34 mmol) TEMPO zugegeben und die Reaktionsmischung kräftig über Nacht bei RT gerührt. Die Reaktionsmischung wurde mit Dichlormethan verdünnt und die organische Phase wurde mit Wasser und ges. NaCl-Lösung gewaschen. Die organische Phase wurde über Magnesiumsulfat getrocknet und das Lösemittel im Vakuum verdampft. Der Rückstand wurde im Hochvakuum getrocknet und mittels Kieselgel (Laufmittel: Cyclohexan/Ethylacetat 3:1-1:1) gereinigt. Die Lösemittel wurden im Vakuum verdampft und der Rückstand im Hochvakuum getrocknet. Man erhielt 589.4 mg (58 % d. Th.) der Titelverbindung.

LC-MS (Methode 6): Rₜ = 2.15 min; MS (ESIpos): m/z = 296 (M-H)⁺.

### Intermediat L71

### tert-Butyl-[4-(chlorcarbonyl)phenyl]carbamat

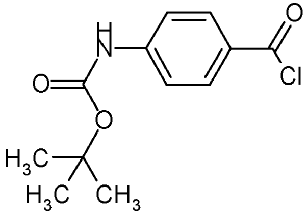

100.0 mg (0.42 mmol) 4-[(tert-Butoxycarbonyl)amino]benzoesäure wurden in 2.0 mL Dichlormethan vorgelegt und mit 64.2 mg (0.51 mmol) Oxalsäuredichlorid versetzt. Die Reaktionsmischung rührte bei RT 30 min (DC-Kontrolle: Dichlormethan/Methanol). Dann wurden nochmals 192.6 mg (1.53 mmol) Oxalsäuredichlorid und 1 Tropfen DMF zugegeben und 1 h bei RT gerührt. Das Lösemittel wurde im Vakuum verdampft und der Rückstand mehrmals mit Dichlormethan kodestilliert. Der Rückstand wurde ohne weitere Reinigung in der nächsten Synthesestufe eingesetzt.

### Intermediat L72

### Benzyl-(9S)-9-(hydroxymethyl)-2,2-dimethyl-6,11-dioxo-5-oxa-7,10-diaza-2-silatetradecan-14-oat

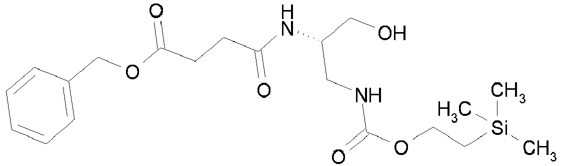

Die Titelverbindung wurde ausgehend von kommerziell erhältlichem Benzyl-tert-butyl-[(2S)-3-hydroxypropan-1,2-diyl]biscarbamat nach klassischen Methoden der Peptidchemie durch hydrogenolytische Abspaltung der Z-Schutzgruppe, anschließende Kupplung mit 4-(Benzyloxy)-4-oxobutansäure in Gegenwart von EDCI/HOBT, dann Abspaltung der Boc-Schutzgruppe mit TFA und schließlich durch Umsetzung mit 1-({[2-(Trimethylsilyl)ethoxy]carbonyl}oxy)pyrrolidin-2,5-dion in Gegenwart von Triethylamin hergestellt.

LC-MS (Methode 1): Rₜ = 0.94 min; MS (ESIpos): m/z = 425 [M+H]⁺.

### Intermediat L73

### N-(2-aminoethyl)-6-(2,5-dioxo-2,5-dihydro-1H-pyrrol-1-yl)hexanamide

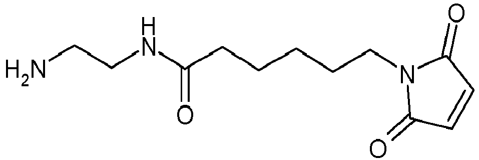

Zu einer Lösung von 300 mg (1.87 mmol) tert-butyl (2-aminoethyl)carbamate in 20 ml Dimethylformamid wurden 395.5 mg (1.87 mmol) 6-(2,5-Dioxo-2,5-dihydro-1H-pyrrol-1-yl)hexansäure, 1.21 g ( 9.36 mmol) N,N-Diisopropylethylamin und 854.3 mg (2.25 mmol) HATU zugegeben. Die Reaktionsmischung wurde 5 Minuten bei Rt gerührt. Nach Einengen der Mischung wurde der Rückstand in DCM aufgenommen und mit Wasser gewaschen. Die organische Phase wurde mit Brine gewaschen, über Magnesiumsulfat getrocknet, abfiltriert und eingeengt. Man erhielt 408 mg (33%, Reinheit 53%) der Titelverbindung, die ohne weitere Reinigung eingesetzt wurden.

LC-MS (Methode 1): Rₜ = 0.75 min; MS (ESIpos): m/z = 354 (M+H)⁺.

Zu einer Lösung von *tert*-butyl (2-{[6-(2,5-dioxo-2,5-dihydro-1H-pyrrol-1-yl)hexanoyl]amino}ethyl)carbamate (408 mg, 0.365 mmol) in 7 ml dichlormethan wurde 1 ml TFA zugegeben. Das Reaktionsgemisch wird 0.5 h bei RT gerührt. Die Reaktionsmischung wurde im Vakuum verdampft und der Rückstand wurde zweimal mit Dichlormethan kodestilliert. Der Rückstand wurde ohne weitere Reinigung weiter eingesetzt. Man erhielt 384 mg (94%, Reinheit 57%) der Titelverbindung.

LC-MS (Methode 1): Rₜ = 0.26 min; MS (ESIpos): m/z = 254 (M+H)⁺.

### Intermediat L74

### 3-[2-[2-[2-[2-[[2-(2,5-dioxopyrrol-1-yl)acetyl]amino]ethoxy]ethoxy]ethoxy]ethoxy]propansäure

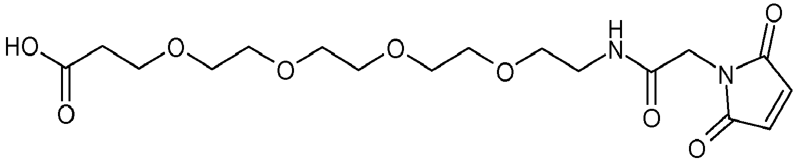

107 mg (0.335 mmol) *tert*-butyl 3-[2-[2-[2-(2-aminoethoxy)ethoxy]ethoxy]ethoxy]propanoate und 93 mg (0.369 mmol) (2,5-dioxopyrrolidin-1-yl) 2-(2,5-dioxopyrrol-1-yl)acetate wurden in 5 ml Dimethylformamid gelöst und mit 0.074 mL (0.671 mmol) N-Methylmorpholin versetzt. Die Reaktionsmischung wurde über Nacht bei RT gerührt. Es wurde 0.048 mL (0.838 mmol) Essigsäure zugegeben und die Reaktionsmischung wurde direkt mittles präp. RP-HPLC gereinigt (Säule: Reprosil 125x30; 10µ, Fluss: 50 mL/min, MeCN/Wasser/0.1%TFA). Die Lösemittel wurden im Vakuum verdampft und der Rückstand im Hochvakuum getrocknet. Man erhielt 133 mg (86%, Reinheit 100%) *tert-*butyl 3-[2-[2-[2-[2-[[2-(2,5-dioxopyrrol-1-yl)acetyl]amino]ethoxy]ethoxy]ethoxy]ethoxy]propanoate.

LC-MS (Methode 1): Rₜ = 0.82 min; MS (ESIpos): m/z = 459 (M+H)⁺.

Zu einer Lösung von *tert*-butyl 3-[2-[2-[2-[2-[[2-(2,5-dioxopyrrol-1-yl)acetyl]amino]ethoxy]ethoxy]ethoxy]ethoxy]propanoate (130 mg, 0.284 mmol) in 5 ml dichlormethan wurde 0.5 ml TFA zugegeben. Das Reaktionsgemisch wird über Nacht bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 102 mg (90%, Reinheit 100%) der Titelverbindung.

LC-MS (Methode 1): Rₜ = 0.52 min; MS (ESIpos): m/z = 402 (M+H)⁺.

### Intermediat L75

### Trifluoressigsäure --2-(trimethylsilyl)ethyl-3-{ [(benzyloxy)carbonyl]amino }-D-alaninat (1:1)

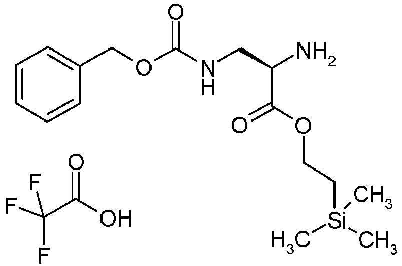

Die Titelverbindung wurde ausgehend von 3-{[(Benzyloxy)carbonyl]amino}-N-(tert-butoxycarbonyl)-D-alanin nach klassischen Methoden der Peptidchemie (Veresterung mit 2-(Trimethylsilylethanol mittels EDCI/DMAP und Abspaltung der Boc-Schutzgruppe mit Trifluoressigsäure hergestellt. Man erhielt 405 mg (58 % d. Th. über 2 Stufen) der Titelverbindung. LC-MS (Methode 1): Rₜ = 0.75 min; MS (ESIpos): m/z = 339 (M+H)⁺.

### Intermediat L76

### (2S)-2-Brom-4-oxo-4-[2-(trimethylsilyl)ethoxy]butansäure

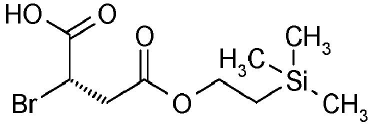

Zunächst wurde ein geeignet geschütztes Asparaginsäurederivat ausgehend von (3S)-4-(Benzyloxy)-3-{[(benzyloxy)carbonyl]amino}-4-oxobutansäure nach klassischen Methoden der Peptidchemie (Veresterung mit 2-(Trimethylsilylethanol mittels EDCI/DMAP und hydrogenolytische Abspaltung der Z-Schutzgruppe und des Benzylesters hergestellt.

470 mg (1.8 mmol) der so erhaltenen (2S)-2-Amino-4-oxo-4-[2-(trimethylsilyl)ethoxy]butansäure wurden in 10 ml Wasser suspendiert und mit 1.8 mL einer 1 molaren Salzsäure sowie 0.5 ml konzentrierter Schwefelsäure und anschließend mit 863 mg (7.25 mmol Kaliumbromid versetzt. Dann wurden bei 10°C über einen Zeitraum von 30 min eine Lösung aus 150 mg (2.175 mmol) Natriumnitrit in 1 ml Wasser zugetropft und der Ansatz 2 h bei 10-15°C gerührt. Anschließend wurde der Ansatz mit 50 mL Ethylacetat ausgeschüttelt. Die organische Phase wurde mit gesättigter Natriumchloridlösung gewaschen und über Magnesiumsulfat getrocknet. Nach Abdampfen des Lösungsmittels und Reinigung durch präparative HPLC wurden 260 mg (48% d.Th.) der Titelverbindung erhalten.

LC-MS (Methode 1): Rₜ = 1.03 min; MS (ESIneg): m/z = 295 und 297 (M-H)⁻.

¹H-NMR (400 MHz, CDCl₃): δ [ppm] = 0.03 (s, 9H), 0.95 (t, 2H), 2.94 und 3.2 (2dd, 2H), 4.18 (t, 2H), 4.57 (t, 1H).

### Intermediat L77

### Trifluoressigsäure --N-[2-(2-aminoethoxy)ethyl]-2-bromacetamid (1:1)

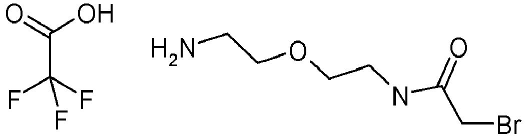

Zunächst wurden 418 mg (2.05 mmol) tert-Butyl-[2-(2-aminoethoxy)ethyl]carbamat mit 638 mg (2.46 mmol) Bromessigsäureanhydrid umgesetzt und anschließend die Boc-Schutzgruppe mit Trifluoressigsäure entfernt. Man erhielt 551 mg (63 % d. Th. über 2 Stufen) der Titelverbindung.

LC-MS (Methode ): Rₜ = 0.32 min; MS (ESIpos): m/z = 227 und 225 (M+H)⁺.

### Intermediat L78

### N-[(2,5-Dioxo-2,5-dihydro-1H-pyrrol-1-yl)acetyl]-beta-alanin

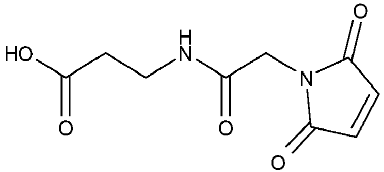

Die Titelverbindung wurde ausgehend von kommerziell erhältlichem (2,5-Dioxo-2,5-dihydro-1H-pyrrol-1-yl)essigsäure durch Kupplung mit *tert*-Butyl-beta-alaninathydrochlorid (1: 1) in Gegenwart von EDCI/HOBt und *N,N-*Diisopropylethylamin und anschließende Entschützung mit Trifluoressigsäure hergestellt.

LC-MS (Methode 1): Rₜ = 0.32 min; MS (ESIpos): m/z = 227 (M+H)⁺.

### Intermediat L79

### N-[6-(2,5-Dioxo-2,5-dihydro-1H-pyrrol-1-yl)hexanoyl]-beta-alanin

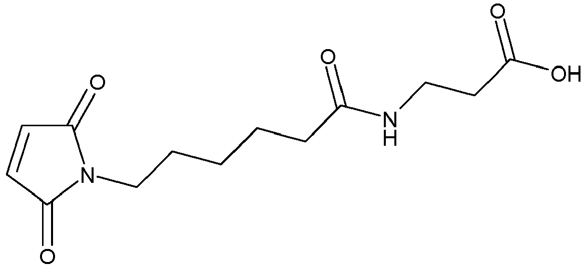

64.8 mg (0.357 mmol) *tert*-Butyl-beta-alaninathydrochlorid (1:1) und 100 mg (0.324 mmol) 1-{6-[(2,5-Dioxopyrrolidin-1-yl)oxy]-6-oxohexyl}-1H-pyrrol-2,5-dion wurden in 4 ml Dimethylformamid gelöst und mit 65.6 mg (0.649 mmol) N-Methylmorpholin versetzt. Die Reaktionsmischung wurde über Nacht bei RT gerührt. Es wurde 0.048 mL (0.838 mmol) Essigsäure zugegeben und die Reaktionsmischung wurde direkt mittles präp. RP-HPLC gereinigt (Säule: Reprosil 250x30; 10µ, Fluss: 50 mL/min, MeCN/Wasser/0.1%TFA). Die Lösemittel wurden im Vakuum verdampft und der Rückstand im Hochvakuum getrocknet. Man erhielt 84.5 mg (77%, Reinheit 100%) *tert*-Butyl-N-[6-(2,5-dioxo-2,5-dihydro-1H-pyrrol-1-yl)hexanoyl]-beta-alaninat.

LC-MS (Methode 1): Rₜ = 0.78 min; MS (ESIpos): m/z = 339 (M+H)⁺.

Zu einer Lösung von *tert*-Butyl-N-[6-(2,5-dioxo-2,5-dihydro-1H-pyrrol-1-yl)hexanoyl]-beta-alaninat (82.8 mg, 0.244 mmol) in 8 ml dichlormethan wurde 1.62 ml TFA zugegeben. Das Reaktionsgemisch wird 2 Stunden bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 62.7 mg (87%, Reinheit 95%) der Titelverbindung.

LC-MS (Methode 1): Rₜ = 0.75 min; MS (ESIpos): m/z = 283 (M+H)⁺.

### Intermediat L80

### 2-(Trimethylsilyl)ethyl-3-[(15-amino-4,7,10,13-tetraoxapentadecan-1-oyl)amino]-N-(tert-butoxycarbonyl)-D-alaninat

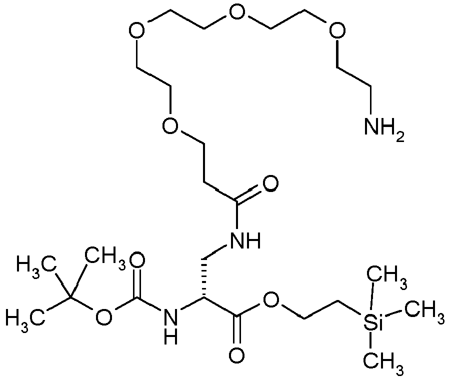

Die Titelverbindung wurde ausgehend von kommerziell erhältlichem 3-{[(Benzyloxy)carbonyl] amino}-N-(tert-butoxycarbonyl)-D-alanin --N-cyclohexylcyclohexanamin (1:1) nach klassischen Methoden der Peptidchemie (Freisetzung aus dem Salz und Veresterung mit 2-(Trimethylsilylethanol mittels EDCI/DMAP, hydrogenolytische Abspaltung der Z-Schutzgruppe, Kupplung mit kommerziell erhältlicher 3-Oxo-1-phenyl-2,7,10,13,16-pentaoxa-4-azanonadecan-19-säure in Gegenwart von HATU und N, N-Diisopropylethylamin und erneute hydrogenolytische Abspaltung der Z-Schutzgruppe) hergestellt.

LC-MS (Methode 1): Rₜ = 0.70 min; MS (ESIpos): m/z = 552 (M+H)⁺.

### Intermediat L81

### Trifluoressigsäure -benzyl-{2-[(2-aminoethyl)sulfonyl]ethyl}carbamat (1:1)

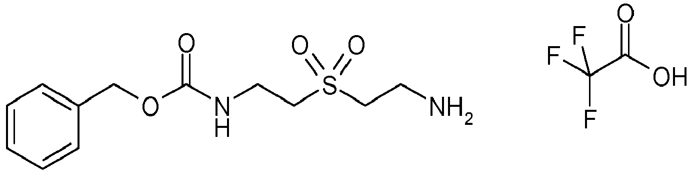

250 mg (1.11 mmol) 2,2'-Sulfonyldiethanamin wurden mit 92.3 mg (0.37 mmol) 1-{[(Benzyloxy)carbonyl] oxy}pyrrolidin-2,5-dion in Gegenwart von N, N-Diisopropylethylamin in DMF gekuppelt. Nach anschließender HPLC Reinigung wurden 70 mg (47% d. Th.) der Titelverbindung erhalten.

LC-MS (Methode 12): Rₜ = 0.64 min; MS (ESIpos): m/z = 257.11 (M+H)⁺.

### Intermediat L82

### Trifluoressigsäure N-{2-[2-(2-aminoethoxy)ethoxy]ethyl}-6-(2,5-dioxo-2,5-dihydro-1H-pyrrol-1-yl)hexanamid (1:1)

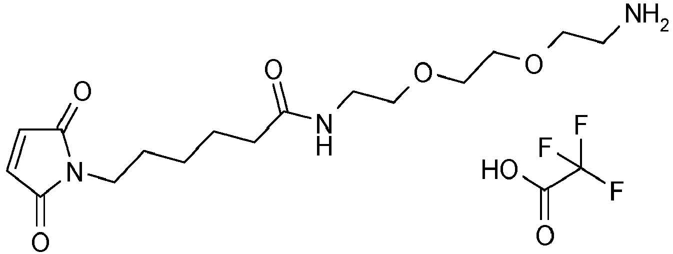

88.6 mg (0.357 mmol) N-Boc-2,2'-(ethylenedioxy)diethylamine und 100 mg (0.324 mmol) N-Succinimidyl 6-maleimidohexanoate wurden in 4.0 ml Dimethylformamid gelöst und mit 0.071 mL (0.650 mmol) N-Methylmorpholin versetzt. Die Reaktionsmischung wurde über Nacht bei RT gerührt. Es wurde 0.048 mL (0.838 mmol) Essigsäure zugegeben und die Reaktionsmischung wurde direkt mittles präp. RP-HPLC gereinigt (Säule: Reprosil 125x30; 10µ, Fluss: 75 mL/min, MeCN/Wasser/0.1%TFA). Die Lösemittel wurden im Vakuum verdampft und der Rückstand im Hochvakuum getrocknet. Man erhielt 127 mg (81% d. Th) tert-Butyl-{2-[2-(2-{[6-(2,5-dioxo-2,5-dihydro-1H-pyrrol-1-yl)hexanoyl]amino}ethoxy)ethoxy]ethyl}carbamat.

LC-MS (Methode 1): Rₜ = 0.78 min; MS (ESIpos): m/z = 442 (M+H)⁺.

Zu einer Lösung von 123 mg (225 µmol) tert-Butyl-{2-[2-(2-{[6-(2,5-dioxo-2,5-dihydro-1H-pyrrol-1-yl)hexanoyl]amino}ethoxy)ethoxy]ethyl}carbamat in 7.5 ml dichlormethan wurde 2.0 ml TFA zugegeben. Das Reaktionsgemisch wird 2 h bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 111 mg (100% d. Th) der Titelverbindung.

LC-MS (Methode 1): Rₜ = 0.31 min; MS (ESIpos): m/z = 342 (M+H)⁺.

¹H-NMR (400 MHz, DMSO-d₆): δ [ppm] = 1.17 (m, 2H), 1.47 (m, 4H), 2.04 (m, 2H), 2.98 (m, 2H), 3.19 (m, 2H), 3.39 (m, 4H), 3,56 (m, 6H), 7.01 (s, 2H), 7.72 (bs, 3H), 7.80 (m, 1H).

### Intermediat L83

### Trifluoressigsäure N-{2-[2-(2-aminoethoxy)ethoxy]ethyl}-2-(2,5-dioxo-2,5-dihydro-1H-pyrrol-1-yl)acetamid (1:1)

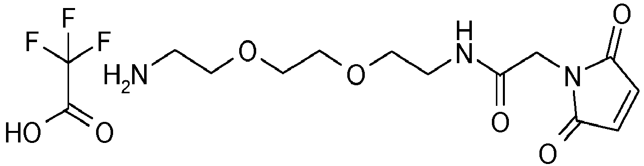

200 mg (0.805 mmol) tert-Butyl-{2-[2-(2-aminoethoxy)ethoxy]ethyl}carbamat, 150 mg (0.966 mmol) (2,5-Dioxo-2,5-dihydro-1H-pyrrol-1-yl)essigsäure und 560 µl (3.2 mmol) N,N-Diisopropylethylamin wurden in 10 ml Dimethylformamid gelöst und mit 459 mg (1,21 mmol) HATU versetzt. Die Reaktionsmischung wurde 30 Minuten bei RT gerührt. Die Lösemittel wurden im Vakuum verdampft und der Rückstand in Dichloromethan gelöst. Die organische Phase wurde zweimal mit 5%iger Citronensäure-Lösung gewaschen, über Magnesiumsulfat getrocknet und das Lösemittel im Vakuum verdampft. Der Rückstand wurde mittels Biotage Isolera gereinigt (Kieselgel, Säule 25 g SNAP, Dischloromethan:Methanol 98:2). Man erhielt 276 mg (89 % d. Th) tert-Butyl-{2-[2-(2-{[(2,5-dioxo-2,5-dihydro-1H-pyrrol-1-yl)acetyl]amino } ethoxy)ethoxy] ethyl} carbamat.

LC-MS (Methode 1): Rₜ = 0.67 min; MS (ESIpos): m/z = 386 (M+H)⁺.

Zu einer Lösung von tert-Butyl-{2-[2-(2-{[(2,5-dioxo-2,5-dihydro-1H-pyrrol-1-yl)acetyl]amino}ethoxy)ethoxy]ethyl}carbamat (275 mg, 714 µmol) in 15 ml dichlormethan wurde 4 ml TFA zugegeben. Das Reaktionsgemisch wird 30 Minuten bei RT gerührt. Die Reaktionsmischung wurde im Vakuum verdampft und der Rückstand wurde in Wasser aufgenommen und lyophilisiert. Man erhielt 281 mg (99% d. Th) der Titelverbindung.

LC-MS (Methode 1): Rₜ = 0.17 min; MS (ESIpos): m/z = 286 (M+H)⁺.

### Intermediat L84

### Trifluoressigsäure N-(14-amino-3,6,9,12-tetraoxatetradec-1-yl)-6-(2,5-dioxo-2,5-dihydro-1H-pyrrol-1-yl)hexanamid (1:1)

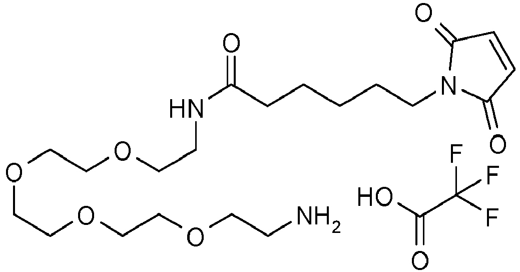

200 mg (0.594 mmol) tert-Butyl-(14-amino-3,6,9,12-tetraoxatetradec-1-yl)carbamat und 202 mg (0.654 mmol) 1-{6-[(2,5-Dioxopyrrolidin-1-yl)oxy]-6-oxohexyl}-1H-pyrrol-2,5-dion wurden in 4.0 ml Dimethylformamid gelöst und mit 0.130 mL (1.2 mmol) N-Methylmorpholin versetzt. Die Reaktionsmischung wurde über Nacht bei RT gerührt. Es wurde 0.085 ml (1.5 mmol) Essigsäure zugegeben und die Reaktionsmischung wurde direkt mittles präp. RP-HPLC gereinigt (Säule: Reprosil 125x30; 10µ, Fluss: 50 mL/min, MeCN/Wasser/0.1%TFA). Die Lösemittel wurden im Vakuum verdampft und der Rückstand im Hochvakuum getrocknet. Man erhielt 275 mg (73% d. Th) tert-Butyl-[21-(2,5-dioxo-2,5-dihydro-1H-pyrrol-1-yl)-16-oxo-3,6,9,12-tetraoxa-15-azahenicos-1-yl]carbamat.

LC-MS (Methode 1): Rₜ = 0.81 min; MS (ESIpos): m/z = 530 (M+H)⁺.

Zu einer Lösung von tert-Butyl-[21-(2,5-dioxo-2,5-dihydro-1H-pyrrol-1-yl)-16-oxo-3,6,9,12-tetraoxa-15-azahenicos-1-yl]carbamat (268 mg, 505 µmol) in 5.0 ml dichlormethan wurde 780 µl (10 mmol) TFA zugegeben. Das Reaktionsgemisch wurde über Nacht bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 266 mg (97% d. Th) der Titelverbindung.

LC-MS (Methode 1): Rₜ = 0.46 min; MS (ESIpos): m/z = 430 (M+H)⁺.

¹H-NMR (400 MHz, DMSO-d₆): δ [ppm] = 1.17 (m, 2H), 1.47 (m, 4H), 2.03 (m, 2H), 2.99 (m, 2H), 3.18 (m, 2H), 3.38 (m, 4H), 3,52 (m, 8H), 3,58 (m, 6H), 7.01 (s, 2H), 7.73 (bs, 3H), 7.80 (m, 1H).

### Intermediat L85

### Trifluoressigsäure N-(14-amino-3,6,9,12-tetraoxatetradec-1-yl)-2-(2,5-dioxo-2,5-dihydro-1H-pyrrol-1-yl)acetamid (1:1)

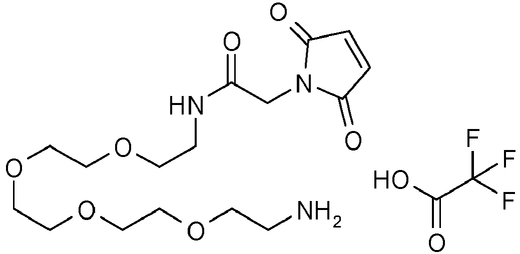

200 mg (0.594 mmol) tert-Butyl-(14-amino-3,6,9,12-tetraoxatetradec-1-yl)carbamat, 111 mg (0.713 mmol) (2,5-Dioxo-2,5-dihydro-1H-pyrrol-1-yl)essigsäure und 410 µl (2.4 mmol) N,N-Diisopropylethylamin wurden in 6 ml Dimethylformamid gelöst und mit 339 mg (0.892 mmol) HATU versetzt. Die Reaktionsmischung wurde 1 h bei RT gerührt und direkt mittles präp. RP-HPLC gereinigt (Säule: Reprosil 250x30; 10µ, Fluss: 50 mL/min, MeCN/Wasser/0.1%TFA). Die Lösemittel wurden im Vakuum verdampft und der Rückstand im Hochvakuum getrocknet. Man erhielt 130 mg (43% d: Th) tert-Butyl-[17-(2,5-dioxo-2,5-dihydro-1H-pyrrol-1-yl)-16-oxo-3,6,9,12-tetraoxa-15-azaheptadec-1-yl]carbamat.

LC-MS (Methode 1): Rₜ = 0.71 min; MS (ESIpos): m/z = 474 (M+H)⁺.

Zu einer Lösung tert-Butyl-[17-(2,5-dioxo-2,5-dihydro-1H-pyrrol-1-yl)-16-oxo-3,6,9,12-tetraoxa-15-azaheptadec-1-yl]carbamat (126 mg, 267 µmol) in 4.0 ml Dichlormethan wurde 410 µl (5.3 mmol) TFA zugegeben. Das Reaktionsgemisch wurde über Nacht bei RT gerührt. Die Reaktionsmischung wurde im Vakuum verdampft und der Rückstand am Hochvakuum getrocknet. Man erhielt 124 mg (95% d: Th) der Titelverbindung.

LC-MS (Methode 13): Rₜ = 0.74 min; MS (ESIpos): m/z = 374 (M+H)⁺.

¹H-NMR (400 MHz, DMSO-d₆): δ [ppm] = 2.99 (m, 2H), 3.22 (m, 2H), 3.41 (m, 2H), 3,53 (m, 8H), 3,58 (m, 6H), 4.02 (s, 2H), 7.09 (s, 2H), 7.73 (bs, 3H), 8.21 (m, 1H).

### Intermediat L86

### N-[(2,5-Dioxo-2,5-dihydro-1H-pyrrol-1-yl)acetyl]-L-valyl-L-alanin

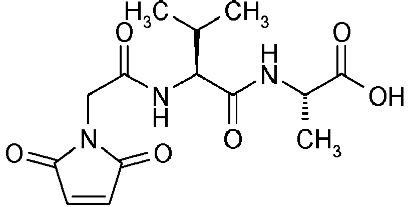

100 mg (0.531 mmol) L-Valyl-L-alanin und 134 mg (0.531 mmol) 1-{2-[(2,5-Dioxopyrrolidin-1-yl)oxy]-2-oxoethyl}-1H-pyrrol-2,5-dion wurden in 3 ml Dimethylformamid gelöst und mit 0.150 mL (1.1 mmol) Triethylamin versetzt. Die Reaktionsmischung wurde 8 h bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 71.5 mg (41 % d. Th.) der Titelverbindung.

LC-MS (Methode 1): Rₜ = 0.42 min; MS (ESIpos): m/z = 326 (M+H)⁺.

### Intermediat L87

### 3-[2-(2-{[(2,5-Dioxo-2,5-dihydro-1H-pyrrol-1-yl)acetyl]amino}ethoxy)ethoxy]propansäure

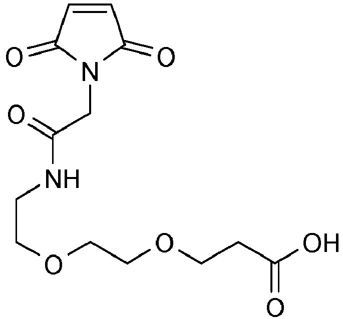

250 mg (1.07 mmol) tert-Butyl-3-[2-(2-aminoethoxy)ethoxy]propanoat, 151 mg (0.974 mmol) 2-(2,5-Dioxo-2,5-dihydro-1H-pyrrol-1-yl)acetic acid, 224 mg (1.46 mmol) 1-Hydroxy-1H-benzotriazol Hydrat und 224 mg (1.17 mmol) 1-(3-Dimethylaminopropyl)-3-ethylcarbodiimidhydrochlorid wurden in 5.0 ml Dimethylformamid gelöst. Die Reaktionsmischung wurde 1 h bei RT gerührt. Der Ansatz wurde mit Ethylacetat versetzt, zweimal mit 5%iger Citronensäure-Lösung und mit ges. Natriumhydrogencarbonat-Lösung extrartiert. Die organische Phase wurde zweimal mit ges. Natriumchlorid-Lösung gewaschen,. über Magnesiumsulfat getrocknet und das Lösemittel im Vakuum abgedampft. Der Rückstand wurde über die präp. RP-HPLC gereinigt (Säule: Reprosil 250x40; 10µ, Fluss: 50 mL/min, MeCN/Wasser/0.1%TFA). Die Lösemittel wurden im Vakuum verdampft und der Rückstand im Hochvakuum getrocknet. Man erhielt 267 mg (64% d: Th) tert-Butyl-3-[2-(2-{[(2,5-dioxo-2,5-dihydro-1H-pyrrol-1-yl)acetyl]amino}ethoxy)ethoxy]propanoat.

LC-MS (Methode 1): Rt = 0.73 min; MS (ESIpos): m/z = 371 (M+H)⁺.

Zu einer Lösung von tert-Butyl-3-[2-(2-{[(2,5-dioxo-2,5-dihydro-1H-pyrrol-1-yl)acetyl]amino}ethoxy)ethoxy]propanoat (263 mg, 710 µmol) in 10 ml dichlormethan wurde 1.1 ml (14 mmol) TFA zugegeben. Das Reaktionsgemisch wurde über Nacht bei RT gerührt. Die Reaktionsmischung wurde im Vakuum verdampft und der Rückstand am Hochvakuum getrocknet. Man erhielt 240 mg (94% d: Th) der Titelverbindung.

LC-MS (Methode 12): Rₜ = 0.57 min; MS (ESIpos): m/z = 315 (M+H)⁺.

### Intermediat L88

### 2,5-Dioxopyrrolidin-1-yl-N-[6-(2,5-dioxo-2,5-dihydro-1H-pyrrol-1-yl)hexanoyl]-L-valyl-L-alaninat

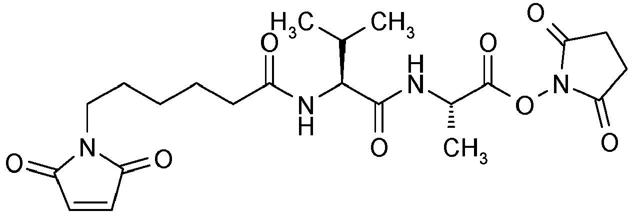

150 mg (0.797 mmol) L-Valyl-L-alanin und 246 mg (0.797 mmol) 1-{6-[(2,5-Dioxopyrrolidin-1-yl)oxy]-6-oxohexyl}-1H-pyrrol-2,5-dion wurden in 4.0 ml Dimethylformamid gelöst und mit 0.220 mL (1.6 mmol) Triethylamin versetzt. Die Reaktionsmischung wurde über Nacht bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 302 mg (97 % d. Th.) N-[6-(2,5-Dioxo-2,5-dihydro-1H-pyrrol-1-yl)hexanoyl]-L-valyl-L-alanin.

LC-MS (Methode 12): Rₜ = 1.02 min; MS (ESIpos): m/z = 382 (M+H)⁺.

¹H-NMR (400 MHz, DMSO-d₆): δ [ppm] = 0.82 (dd, 6H), 1.17 (m, 2H), 1.27 (d, 3H), 1.48 (m, 4H), 1.94 (m, 1H), 2.13 (m, 2H), 3.38 (t, 2H), 4.17 (m, 2H), 7.00 (s, 2H), 7.75 (d, 1H), 8.19 (d, 1H).

130 mg (0.531 mmol) N-[6-(2,5-Dioxo-2,5-dihydro-1H-pyrrol-1-yl)hexanoyl]-L-valyl-L-alanin wurden in 6.5 ml Dichloromethan gelöst und mit 58.8 mg (0.511 mmol) 1-Hydroxypyrrolidin-2,5-dion und 78.4 mg (0.409 mmol) 1-(3-Dimethylaminopropyl)-3-ethylcarbodiimidhydrochlorid versetzt. 58.8 mg (0.511 mmol) 1-Hydroxypyrrolidin-2,5-dion und 78.4 mg (0.409 mmol) 1-(3-Dimethylaminopropyl)-3-ethylcarbodiimidhydrochlorid wurden nocheinmal zugegeben. Der Ansatz wurde mit Dichloromethan versetzt une dreimal mit Wasser gewaschen. Die organische Phase wurde über Magnesiumsulfat getrocknet, das Lösemittel im Vakuum abgedampft und der Rückstand im Hochvakuum getrocknet. Man erhielt 172 mg (87 % d. Th.) der Titelverbindung.

LC-MS (Methode 12): Rₜ = 1.28 min; MS (ESIpos): m/z = 479 (M+H)⁺.

### Intermediat L89

### 1-Benzyl-5-[2-(trimethylsilyl)ethyl]-L-glutamathydrochlorid (1:1)

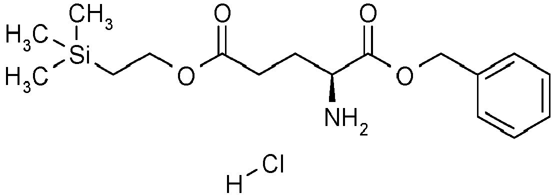

1.00 g (2.96 mmol) (4S)-5-(Benzyloxy)-4-[(tert-butoxycarbonyl)amino]-5-oxopentansäure wurden in 13.0 ml THF vorgelegt und mit 510 µl (3.6 mmol) 2-(Trimethylsilyl)ethanol und 109 mg (889 µmol)

4-Dimethylaminopyridin versetzt. Die Reactionmischung wurde auf 0°C gekühlt und mit 682 mg (3.56 mmol) N-Ethyl-N'-3-(dimethylaminopropyl)carbodiimid hydrochlorid versetzt. Die Reactionmischung wurde über Nacht bei RT gerührt. Die Lösemittel wurden im Vakuum verdampft und der Rückstand in Ethylacetat gelöst. Die organische Phase wurde zweimal mit 0.1N HCl-Lösung und ges. Natriumchlorid-Lösunggewaschen, über Magnesiumsulfat getrocknet und das Lösemittel im Vakuum verdampft. Der Rückstand wurde mittels Biotage Isolera gereinigt (Kieselgel, Säule 25 g SNAP, Cyclohexan:Ethylacetat 80:20). Man erhielt 649 mg (50 % d. Th) der Verbindung 1-Benzyl-5-[2-(trimethylsilyl)ethyl]-N-(tert-butoxycarbonyl)-L-glutamat.

LC-MS (Methode 1): Rₜ = 4.6 min; MS (ESIpos): m/z = 438 (M+H)⁺.

649 mg (1.48 mmol) 1-Benzyl-5-[2-(trimethylsilyl)ethyl]-N-(tert-butoxycarbonyl)-L-glutamat wurden in 7.0 ml Dioxan gelöst und unter Eisbadkühlung wurden 14 ml (59 mmol) 4N HCl in Dioxan dazugegeben. Die Reactionmischung wurde über Nacht bei RT gerührt. Die Reaktionsmischung wurde im Vakuum verdampft und der Rückstand am Hochvakuum getrocknet und mittels Biotage Isolera gereinigt (Kieselgel, Säule 25 g SNAP, Dischloromethan:Methanol 90:10). Man erhielt 320 mg (57 % d. Th) der Titel Verbindung.

LC-MS (Methode 1): Rₜ = 0.79 min; MS (ESIpos): m/z = 338 (M+H)⁺.

### Intermediat L90

### 1 -({N-[(Benzyloxy)carbonyl]glycyl} amino)-3,6,9,12-tetraoxapentadecan-15 -säure

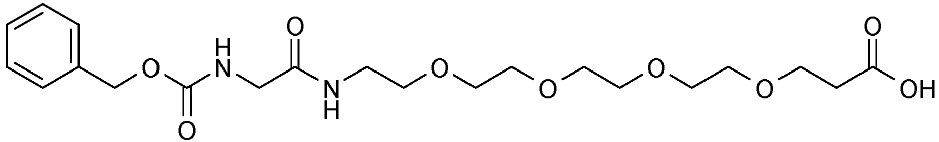

118 mg (566 µmol) N-[(Benzyloxy)carbonyl]glycin wurden in 5.0 ml DMF vorgelegt, mit 200 mg (622 µmol) tert-Butyl-1-amino-3,6,9,12-tetraoxapentadecan-15-oat, 130 mg (849 µmol) 1-Hydroxy-1H-benzotriazol Hydrat und 130 mg (679 µmol) 1-(3-Dimethylaminopropyl)-3-ethylcarbodiimidhydrochlorid versetzt und 1 h bei RT gerührt. Der Ansatz wurde mit Ethylacetat versetzt, zweimal mit 5%iger Citronensäure-Lösung und mit ges. Natriumhydrogencarbonat-Lösung extrartiert. Die organische Phase wurde zweimal mit ges. Natriumchlorid-Lösung gewaschen und. über Magnesiumsulfat getrocknet. Die Lösemittel wurden im Vakuum verdampft und der Rückstand im Hochvakuum getrocknet. Man erhielt 274 mg (95% d: Th) tert-Butyl-1-({N-[(benzyloxy)carbonyl]glycyl}amino)-3,6,9,12-tetraoxapentadecan-15-oat.

LC-MS (Methode 12): Rt = 1.69 min; MS (ESIpos): m/z = 513 (M+H)⁺.

Zu einer Lösung von 274 mg (535 µmol)tert-Butyl-1-({N-[(benzyloxy)carbonyl]glycyl}amino)-3,6,9,12-tetraoxapentadecan-15-oat in 5.0 ml dichlormethan wurde 820 µl (11 mmol) TFA zugegeben. Das Reaktionsgemisch wurde 3 h bei RT gerührt. Die Reaktionsmischung wurde im Vakuum verdampft und der Rückstand in Wasser aufgenommen und lyophilisiert.. Man erhielt 262 mg (100% d: Th) der Titelverbindung.

LC-MS (Methode 12): Rₜ = 1.12 min; MS (ESIpos): m/z = 457 (M+H)⁺.

### Intermediat L91

### Trifluoressigsäure --2-(trimethylsilyl)ethyl-1-{[3-amino-N-(tert-butoxycarbonyl)-D-alanyl]amino}-3,6,9,12-tetraoxapentadecan-15-oat (1:1)

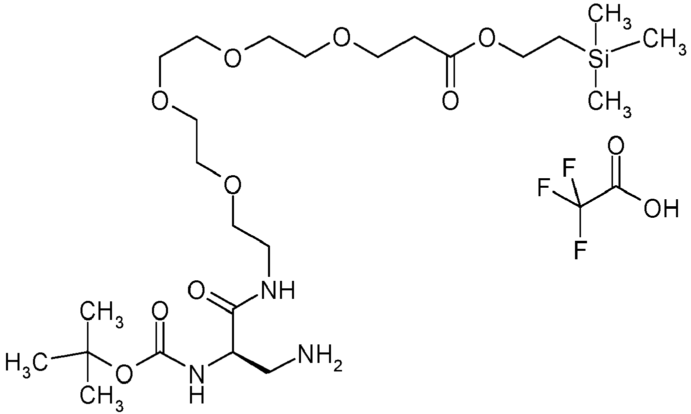

Die Titelverbindung wurde ausgehend von kommerziell erhältlicher 3-Oxo-1-phenyl-2,7,10,13,16-pentaoxa-4-azanonadecan-19-säure nach klassischen Methoden der Peptidchemie (Veresterung mit 2-Trimethylsilylethanol mittels EDCI/DMAP, hydrogenolytische Abspaltung der Z-Schutzgruppe, Kupplung mit kommerziell erhältlichem N-(tert-Butoxycarbonyl)-3-{[(9H-fluoren-9-ylmethoxy) carbonyl]amino}-D-alanin und Abspaltung der Fmoc-Schutzgruppe) hergestellt.

LC-MS (Methode 1): Rₜ = 0.74 min; MS (ESIpos): m/z = 552 (M+H)⁺.

### Intermediat L92

### N-[(Benzyloxy)carbonyl]-L-alanyl-L-alanyl-L-asparagin

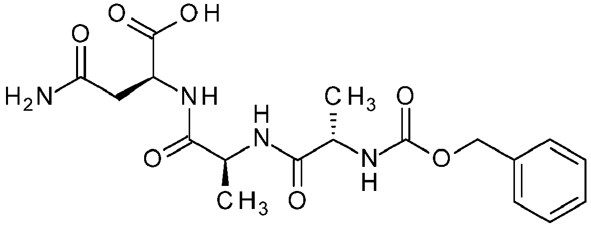

Die Titelverbindung wurde nach klassischen Methoden der Peptidchemie durch HATU-Kupplung in Gegenwart von N,N-Diisopropylethylamin von kommerziell erhältlichen N-[(Benzyloxy) carbonyl]-L-alanyl-L-alanin mit tert-Butyl-L-asparaginat und anschließender Entschützung der Carboxygruppe mit Trifluoressigsäure hergestellt.

LC-MS (Methode 1): Rₜ = 0.5 min; MS (ESIpos): m/z = 409 (M+H)⁺.

### Intermediat L93

### N-Acetyl-L-alanyl-L-alanyl-L-asparagin

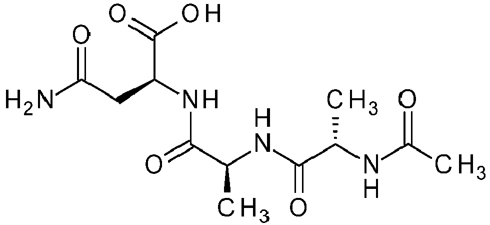

Die Titelverbindung wurde nach klassischen Methoden der Peptidchemie durch HATU-Kupplung in Gegenwart von N,N-Diisopropylethylamin von kommerziell erhältlichen N-[(Benzyloxy) carbonyl]-L-alanyl-L-alanin mit tert-Butyl-L-asparaginat, anschließender Abspaltung der Z-Schutzgruppe durch Hydrierung in DCM-Methanol über 10% Palladium auf Aktivkohle, dann folgender Acetylierung mit Essigsäure in DMF in Gegenwart von HATU und N,N-Diisopropylethylamin und schließlich Entschützung der Carboxygruppe mit Trifluoressigsäure hergestellt.

LC-MS (Methode 1): Rₜ = 0.16 min; MS (ESIpos): m/z = 317 (M+H)⁺.

¹H-NMR (400 MHz, DMSO-d₆): δ [ppm] = 1.19 (2d, 6H), 1.82 (s, 3H), 2.5 (m, 2H), 4.26 (m, 2H), 4.48 (q, 1H), 6.9 (s, 1H), 7.36 (s, 1H), 8.0 (m, 3H), 12.54 (s, 1H).

### Intermediat L94

### N-{4-Oxo-4-[2-(trimethylsilyl)ethoxy]butanoyl}-L-alanyl-L-alanyl-L-asparagin

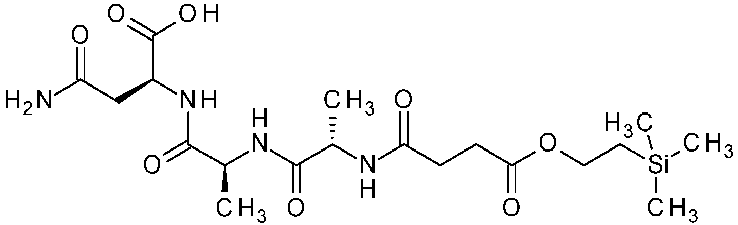

Zunächst wurde 4-Oxo-4-[2-(trimethylsilyl)ethoxy]butansäure durch Umsetzung von 4-(Benzyloxy)-4-oxobutansäure mit 2-(Trimethylsilyl)ethanol in Gegenwart von EDCI/DMAP in DCM und anschließender hydrogenolytischen Spaltung des Benzylesters hergestellt.

LC-MS (Methode 1): Rₜ = 0.89 min; MS (ESIpos): m/z = 217 (M-H)⁻.

Weiterhin wurde Trifluoressigsäure --4-nitrobenzyl-L-alanyl-L-alanyl-L-asparaginat (1:1) durch Kupplung von N-(tert-Butoxycarbonyl)-L-alanyl-L-alanin mit 4-Nitrobenzyl-L-asparaginat hydrobromid (1:1) in DMF in Gegenwart von HATU und N,N-Diisopropylethylamin und anschließender Entschützung der Aminogruppe mit Trifluoressigsäure in DCM hergestellt.

LC-MS (Methode 1): Rₜ = 0.43 min; MS (ESIpos): m/z = 410 (M+H)⁺.

Die Titelverbindung wurde dann durch Kupplung dieser beiden Intermediate in DMF in Gegenwart von HATU und N,N-Diisopropylethylamin und anschließender Abspaltung des p-Nitrobenzylesters durch Hydrierung in DCM-Methanol 1:9 über 10% Palladium auf Aktivkohle hergestellt.

LC-MS (Methode 1): Rₜ = 0.79 min; MS (ESIpos): m/z = 475 (M+H)⁺.

### Intermediat L95

### N-[(Benzyloxy)carbonyl]-L-valyl-L-alanin

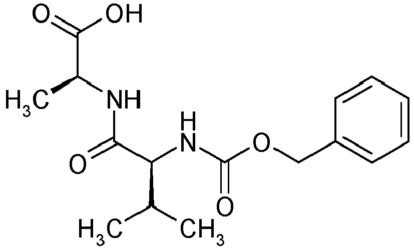

Dieses Intermediat wurde ausgehend von N-[(Benzyloxy)carbonyl]-L-valin und tert-Butyl-L-alaninathydrochlorid (1:1) mit klassischen Methoden der Peptidchemie hergestellt.

LC-MS (Methode 12): Rₜ = 1.34 min; MS (ESIpos): m/z = 323.16 (M+H)⁺.

### Intermediat L96

### N-Acetyl-L-valyl-N⁵-carbamoyl-L-ornithinamid

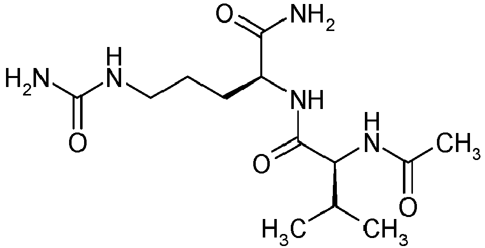

Dieses Intermediat wurde nach klassischen Methoden der Peptidchemie hergestellt beginnend mit der Kupplung von 2,5-Dioxopyrrolidin-1-yl-N-[(benzyloxy)carbonyl]-L-valinat mit N⁵-Carbamoyl-L-ornithin, dann folgender hydrogenolytischer Abspaltlung der Z-Schutzgruppe über 10% Palladium/Aktivkohle in Ethanol und schließlich durch Umsetzung des erhaltenen Dipeptids mit 1-Acetoxypyrrolidin-2,5-dion.

LC-MS (Methode 1): Rₜ = 0.25 min; MS (ESIpos): m/z = 317 (M+H)⁺.

### Intermediat L97

### 1-(2,5-Dioxo-2,5-dihydro-1H-pyrrol-1-yl)-2-oxo-6,9,12,15,18,21,24,27-octaoxa-3-azatriacontan-30-säure

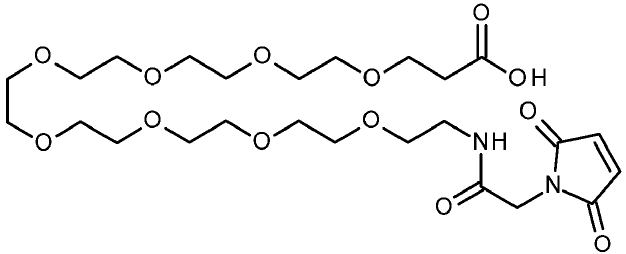

Tert-Butyl-1-amino-3,6,9,12,15,18,21,24-octaoxaheptacosan-27-oat (100 mg, 201 µmol) wurde in 1.0 ml DMF vorgelegt und mit (2,5-Dioxo-2,5-dihydro-1H-pyrrol-1-yl)essigsäure (46.8 mg, 301 µmol), 1-Hydroxy-1H-benzotriazol Hydrat (76.9 mg, 502 µmol) und 1-(3-Dimethylaminopropyl)-3-ethylcarbodiimidhydrochlorid (77.0 mg, 402 µmol) versetzt. Die Reaktionsmischung wurde über Nacht bei RT gerührt und dann mit Ethylacetat versetzt. Die organische Phase wurde zweimal mit 5%iger Citronensäure-Lösung, mit ges. Natriumhydrogencarbonat-Lösung und dann mit ges. Natriumchlorid-Lösung gewaschen. Die organische Phase wurde über Magnesiumsulfat getrocknet. Die Lösemittel wurden im Vakuum verdampft und der Rückstand mittles präp. RP-HPLC gereinigt (Säule: Reprosil 125x30; 10µ, Fluss: 50 mL/min, MeCN/Wasser/0.1%TFA). Die Lösemittel wurden im Vakuum verdampft und der Rückstand im Hochvakuum getrocknet. Man erhielt 19.1 mg (13% d. Th.) der Verbindung tert-Butyl-1-(2,5-dioxo-2,5-dihydro-1H-pyrrol-1-yl)-2-oxo-6,9,12,15,18,21,24,27-octaoxa-3-azatriacontan-30-oat.
LC-MS (Methode 1): Rₜ = 0.87 min; MS (ESIpos): m/z = 635 [M+H]⁺

Zu einer Lösung von tert-Butyl-1-(2,5-dioxo-2,5-dihydro-1H-pyrrol-1-yl)-2-oxo-6,9,12,15,18,21,24,27-octaoxa-3-azatriacontan-30-oat (19.1 mg, 30.1 µmol) in 1.0 ml DCM wurde TFA (62 µl, 600 µmol) zugegeben. Das Reaktionsgemisch wurde 3h bei RT gerührt. Die Reaktionsmischung wurde im Vakuum verdampft und der Rückstand wurde in Wasser aufgenommen und lyophilisiert. Der Rückstand wurde ohne weitere Reinigung weiter eingesetzt. Man erhielt 10.8 mg (46 % d. Th.) der Titelverbindung.
LC-MS (Methode 1): Rₜ = 0.55 min; MS (ESIneg): m/z = 577 [M-H]⁻.

### Intermediat L98

### 2,2-Dimethylpropansäure--2-(trimethylsilyl)ethyl-N-(2-aminoethyl)-N²-{[2-(trimethylsilyl) ethoxy] carbonyl} -L-glutaminat (1:1)

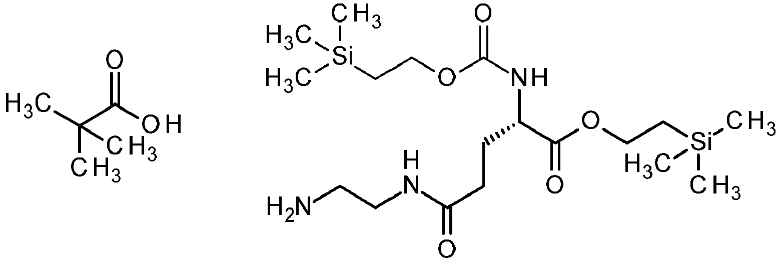

Zunächst wurde (4S)-5-tert-Butoxy-4-[(tert-butoxycarbonyl)amino]-5-oxopentansäure in Gegenwart von HATU und *N,N*-Diisopropylethylamin mit Benzyl-(2-aminoethyl)carbamat gekuppelt. Anschließend wurden mittels Trifluoressigsäure in DCM die Boc-Schutzgruppe sowie der tert.-Butylester abgespalten. Dann wurde zunächst die Aminogruppe durch Umsetzung mit 1-({[2-(Trimethylsilyl)ethoxy]carbonyl}oxy)pyrrolidin-2,5-dion in DMF/Wasser in Gegenwart von *N,N*-Diisopropylethylamin und anschließend die Carboxygruppe durch Umsetzung mit 2-(Trimethylsilyl)ethanol in DCM in Gegenwart von EDCI /DMAP erneut geschützt. Im letzten Schritt erfolgte die Entschützung der terminalen Aminogruppe mittels Hydrogenolyse über 10%-igem Palladium auf Aktivkohle in Ethanol unter Normaldruck. Nach Abfiltrieren des Katalysators, Einengen, Reinigung durch präparative HPLC und Gefriertrocknung des Rückstandes aus Acetonitril/Wasser wurde die Titelverbindung erhalten.

LC-MS (Methode 1): Rₜ = 0.82 min; MS (ESIpos): m/z = 434 (M+H)⁺.

### Intermediat L99

### Trifluoressigsäure --2-(trimethylsilyl)ethyl-N-[(2,5-dioxo-2,5-dihydro-1H-pyrrol-1-yl)acetyl]-L-valyl-L-alanyl-beta-alanyl-L-lysinat (1:1)

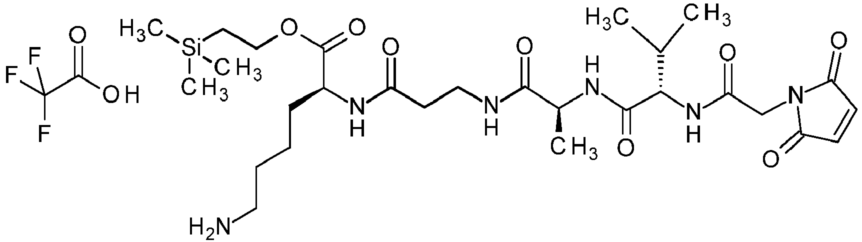

Zunächst wurde ausgehend von N2-[(Benzyloxy)carbonyl]-N6-(tert-butoxycarbonyl)-L-lysin nach klassischen Methoden der Peptidchemie 2-(Trimethylsilyl)ethyl-N6-(tert-butoxycarbonyl)-L-lysinat hergestellt. Dieses Intermediat wurde dann in Gegenwart von HATU und *N,N*-Diisopropylethylamin mit dem nach Standardmethoden hergestellten Tripeptidbaustein N-[(Benzyloxy) carbonyl]-L-valyl-L-alanyl-beta-alanin gekuppelt. Die Z-Schutzgruppe wurde anschließend durch Hydrogenolyse in Methanol entfernt und das erhaltene Intermediat mit (2,5-Dioxo-2,5-dihydro-1H-pyrrol-1-yl)essigsäure in Gegenwart von HATU und N, N Diisopropylethylamin gekuppelt. Im letzten Schritt erfolgte die Entschützung der Seitenkettenaminogruppe unter schonenden Bedingungen durch 1h Rühren in 10%iger Trifluoressigsäure in DCM bei RT. Nach Einengen und Gefriertrocknung aus Acetonitril/Wasser wurde die Titelverbindung erhalten.

LC-MS (Methode 1): Rₜ = 0.64 min; MS (ESIpos): m/z = 625 (M+H)⁺.

### Intermediat L100

### 3-[5-(2-{[(2,5-Dioxo-2,5-dihydro-1H-pyrrol-1-yl)acetyl]aminolethyl)-1,2,4-oxadiazol-3-yl]propansäure

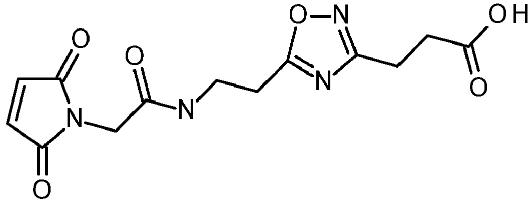

Zu einer Lösung von Methyl-3-cyanpropanoat (4 g, 35.4 mmol) in 120 ml Ethanol wurden 3.69 g (53 mmol) Hydroxylaminehydrochlorid und 15 ml (110 mmol) Triethylamin zugegeben. Das Reaktionsgemisch wurde bei 50 °C 3h gerührt. Das Gemischt wurde eingeengt und der Rückstand wurde in Ethylacetat gelöst und anschließend mit Wasser und Brine gewaschen. Die organische Phase wurde über Magnesiumsulfat getrocknet und eingeengt. Der Rückstand wurde ohne weitere Reinigung eingesetzt. Man erhielt 5 g (97% d. Th) Methyl-(4Z)-4-amino-4-(hydroxyimino)butanoat.

Zu einer Lösung von Methyl-(4Z)-4-amino-4-(hydroxyimino)butanoat (4.85 g, 33.19 mmol) in 120.0 ml Dioxan wurden 6.91 g (36.50 mmol) N-(tert-Butoxycarbonyl)-beta-alanin und 8.22 g (39.82 mmol) 1,3-Dicycloxexylcarbodiimid zugegeben. Das Reaktionsgemisch wurde bei Raumtemperatur 3h gerührt. Das Gemisch wurde eingeengt und der Rückstand wurde in Wasser gelöst und mit Ethylacetat extrahiert. Die organische Phase wurde über Natriumsulfat getrocknet und eingeengt. Der Rückstand wurde mittels Flash-Chromatographie. Man erhielt 6.0 g (57% d.Th) Methyl-(4E)-4-{[N-(tert-butoxycarbonyl)-beta-alanyl]amino}-4-(hydroxyimino)butanoat.

Eine Lösung von Methyl-(4E)-4-{[N-(tert-butoxycarbonyl)-beta-alanyl]amino}-4-(hydroxyimino)butanoat ( 6.0 g, 18.9 mmol) in 100 ml DMF wurde 5h bei 120 °C gerührt. Das Gemisch wurde mit Wasser versetzt und mit Ethylacetat extrahiert. Die organische Phase wurde über Natriumsulfat getrocknet und eingeengt. Der Rückstand wurde mittels präp. HPLC gereinigt. Man erhielt 4 g (71% d. Th.) Methyl-3-(5-{2-[(tert-butoxycarbonyl)amino]ethyl}-1,2,4-oxadiazol-3-yl)propanoat.

Zu einer Lösung von Methyl-(4E)-4-{[N-(tert-butoxycarbonyl)-beta-alanyl]amino}-4-(hydroxyimino)butanoat (4.00 g, 13.4 mmol) in 60 ml THF wurde eine Lösung von LiOH (1.60 g, 66.8 mmol) in 10 ml Wasser zugegeben. Das Reaktionsgemisch wurde über Nacht bei 60 °C gerührt. Das Gemisch wurde mit Wasser versetzt und mit Ethylacetat extrahiert. Die organische Phase wurde über Natriumsulfat getrocknet und eingeengt. Der Rückstand wurde ohne weitere Reinigung eingesetzt. Man erhielt 3.60 g (87% d. Th.) 3-(5-{2-[(tert-Butoxycarbonyl)amino]ethyl} -1,2,4-oxadiazol-3 -yl)propansäure.

Zu einer Lösung von 3-(5-{2-[(tert-Butoxycarbonyl)amino]ethyl}-1,2,4-oxadiazol-3-yl)propansäure (2.0 g, 7.01 mmol) in 30 ml Dichlormethan wurden 2.0 ml (26 mmol) Trifluoressigsäure zugegeben. Das Reaktionsgemisch wurde 1h bei Raumtemperatur gerührt. Das Gemisch wurde mit Wasser versetzt und extrahiert mit Dichlormethan. Die organische Phase wurde über Natriumsulfat getrocknet und eingeengt. Der Rückstand wurde ohne weitere reinigung eingesetzt. Man erhielt 1.50 g (72% d. Th.) 3-[5-(2-Aminoethyl)-1,2,4-oxadiazol-3-yl]propansäure -trifluoressigsäure (1:1).

Zu einer Lösung von 3-[5-(2-Aminoethyl)-1,2,4-oxadiazol-3-yl]propansäure ( 1.5 g, 5.01 mmol) in 25 ml DMF wurden 1.30 g (5.52 mmol) 1-[2-(2,5-Dioxopyrrolidin-1-yl)-2-oxoethyl]-1H-pyrrol-2,5-dion und 1.52 g (15.04 mmol) Triethylamin zugegeben. Das Reaktionsgemisch wurde 1h bei Raumtemperatur gerührt. Das Gemisch wurde mit Wasser versetzt und extrahiert mit Dichloromethan. Die organische Phase wurde über Natriumsulfat getrocknet und eingeengt. Der Rückstand wurde präp. HPLC gereinigt. Man erhielt 774 m g (47% d. Th.) der Titel Verbindung. ¹H-NMR (300 MHz, DMSO-d₆): δ [ppm] = 2.67 (t, 2H), 2.91 (t, 2H), 3.03 (t, 2H), 3.46 (q, 2H), 4.28 (s, 2H), 7.01 (s, 2H), 8.37 (t, 1H), 12.28 (bs, 1H).

### Intermediat L101

### tert-butyl-L-alanyl-L-alanyl-L-asparaginat

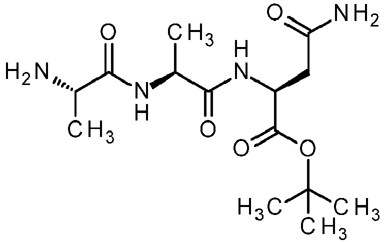

Die Titelverbindung wurde nach klassischen Methoden der Peptidchemie durch HATU-Kupplung in Gegenwart von N,N-Diisopropylethylamin von kommerziell erhältlichen N-[(Benzyloxy)carbonyl]-L-alanyl-L-alanin mit tert-Butyl-L-asparaginathydrochlorid, dann folgender hydrogenolytischer Abspaltlung der Z-Schutzgruppe über 10% Palladium/Aktivkohle in Methanol.

LC-MS (Methode 7): Rₜ = 0.23 min; MS (ESIneg): m/z = 329 (M-H)⁻.

### Intermediat L102

### N-(38-Oxo-2,5,8,11,14,17,20,23,26,29,32,35-dodecaoxaoctatriacontan-38-yl)-L-alanyl-L-alanyl-L-asparagin

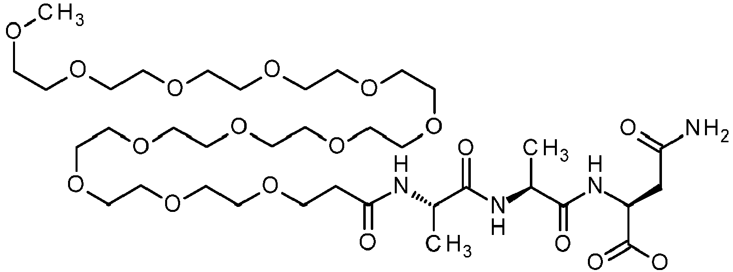

215 mg 2,5,8,11,14,17,20,23,26,29,32,35-Dodecaoxaoctatriacontan-38-säure (365 µmol) und 133 mg intermediat L101 (402 µmol) wurden in 1.4 ml DMF vorgelegt, mit 146 mg HATU (384 µmol) und 160 µl *N,N*-Diisopropylethylamin (910 µmol) versetzt und 3 h bei RT gerührt. Es wurde Wasser (1,5 mL) und ACN (0,5 mL) addiert. Die Reaktionslösung wurde mittels präparativer HPLC gereinigt (Eluent: ACN/Wasser + 0.1% TFA, Gradient = 1:9 → 3:2) und anschließender Abspaltung der Butoxycarbonylschutzgruppe mit 2 mL TFA in 2 mL DCM (3 h bei RT gerührt).

LC-MS (Methode 1): Rₜ = 0.56 min; MS (ESIneg): m/z = 844.5 (M+H)⁺.

### Intermediat L103

### N-(pyridin-4-ylacetyl)-L-alanyl-L-alanyl-L-asparagine trifluoroacetate (1:1)

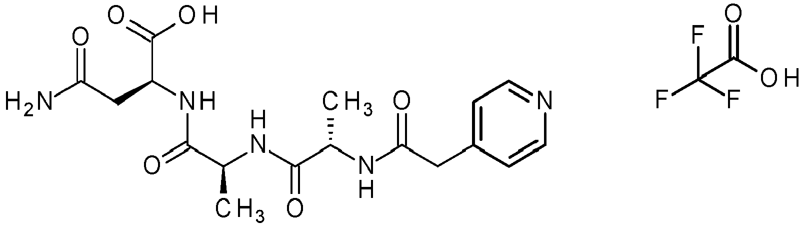

Die Titelverbindung wurde nach klassischen Methoden der Peptidchemie beginnend mit der Kupplung von 4-Pyridinessigsäure mit kommerziell erhältlichem tert-Butyl-L-alanyl-L-alaninat in Gegenwart von HATU und N,N-Diisopropylethylamin, dann folgender Entschützung mit Trifluoressigsäure, Kupplung mit tert-Butyl-L-asparaginat und anschließender Entschützung der Carboxygruppe mit Trifluoressigsäure hergestellt.

LC-MS (Methode 1): Rₜ = 0.15 min; MS (ESIpos): m/z = 394 (M+H)⁺.

### Intermediat L104

### N-Isonicotinoyl-L-alanyl-L-alanyl-L-asparagine trifluoroacetate (1:1)

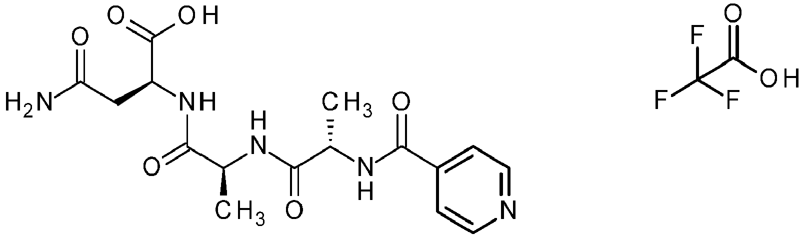

Die Titelverbindung wurde in Analogie zu Intermediat L103 beginnend mit der Kupplung von Isonicotinsäure mit kommerziell erhältlichem tert-Butyl-L-alanyl-L-alaninat hergestellt.

LC-MS (Methode 1): Rₜ = 0.17 min; MS (ESIpos): m/z = 380 (M+H)⁺.

### Intermediat L105

### tert-butyl N-{[2-(2-methoxyethoxy)ethoxy]acetyl}-L-alanyl-L-alanyl-L-asparaginate trifluoro acetate (1:1)

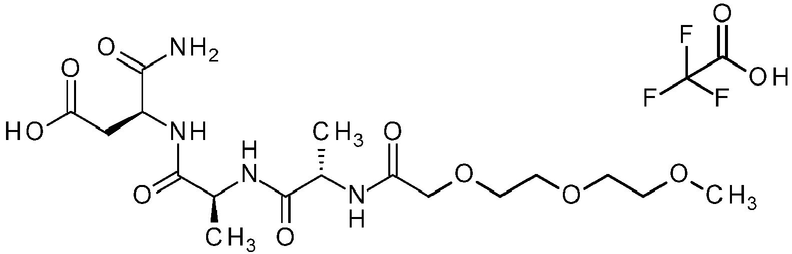

Die Titelverbindung wurde in Analogie zu Intermediat L103 beginnend mit der Kupplung von [2-(2-methoxyethoxy)ethoxy]essigsäure mit kommerziell erhältlichem tert-Butyl-L-alanyl-L-alaninat hergestellt.

LC-MS (Methode 1): Rₜ = 0.17 min; MS (ESIpos): m/z = 380 (M+H)⁺.

### Intermediat L106

### N-[(Benzyloxy)carbonyl]-L-alanyl-L-alanyl-L-asparagin

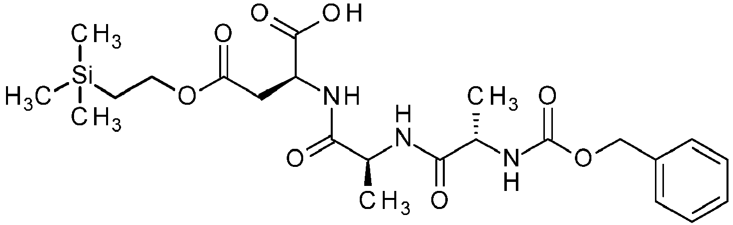

Die Titelverbindung wurde nach klassischen Methoden der Peptidchemie durch Kupplung von kommerziell erhältlichen N-[(Benzyloxy) carbonyl]-L-alanyl-L-alanin mit 1-tert-Butyl 4-[2-(trimethylsilyl)ethyl] L-aspartat in Gegenwart von HATU und N,N-Diisopropylethylamin hergestellt. Dieser Aminosäurebaustein wurde aus (3S)-4-tert-Butoxy-3-[(tert-butoxycarbonyl)amino]-4-oxobutanoic acid durch Veresterung mit 2-(Trimethylsilyl)ethanol in Gegenwart von EDCI und DMAP und anschließender schonender Entferung der tert.-Butoxycarbonylschutzgruppe mittels 5 %-iger Trifluoressigsäure in DCM hergestellt. Anschließend wurden 745 mg (1.317 mmol) des voll geschützten Intermediats in 43.5 ml DCM gelöst und durch Zugabe von 3.5 ml Trifluoressigsäure und 5-stündigem Rühren bei RT der tert-Butylester schonend gespalten. Aus dem entstandenen Produktgemisch wurden nach Reinigung durch präparative HPLC 168 mg (25 % d. Th.) der Titelverbindung isoliert.

LC-MS (Methode 1): Rₜ = 0.95 min; MS (ESIpos): m/z = 510 (M+H)⁺.

### Intermediat L107

### N-[(2,5-Dioxo-2,5-dihydro-1H-pyrrol-1-yl)acetyl]-L-alanyl-L-alanyl-L-asparagin

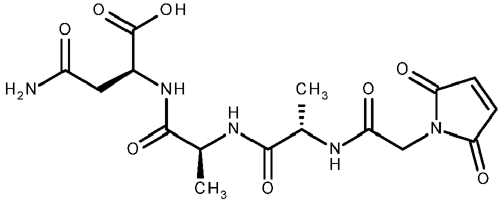

Die Titelverbindung wurde nach klassischen Methoden der Peptidchemie durch HATU-Kupplung von kommerziell erhältlichen N-[(Benzyloxy) carbonyl]-L-alanyl-L-alanin mit tert-Butyl-L-asparaginat in Gegenwart von N,N-Diisopropylethylamin, anschließender Abspaltung der Z-Schutzgruppe durch Hydrierung in Methanol über 10% Palladium auf Aktivkohle, dann folgender Acylierung mit 1-{2-[(2,5-dioxopyrrolidin-1-yl)oxy]-2-oxoethyl}-1H-pyrrole-2,5-dion in DMF in Gegenwart von N,N-Diisopropylethylamin und abschließender Entschützung der Carboxygruppe mittels Trifluoressigsäure hergestellt.

LC-MS (Methode 1): Rₜ = 0.35 min; MS (ESIpos): m/z = 412 (M+H)⁺.

### Intermediat L108

### N²-Acetyl-N-(2-aminoethyl)-N⁶-(tert-butoxycarbonyl)-L-lysinamid

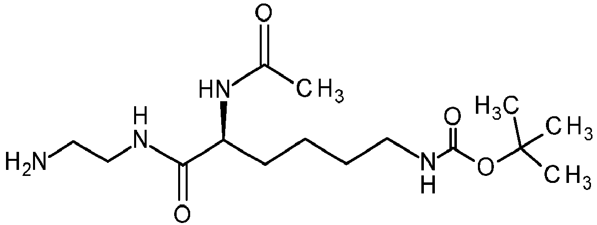

Die Titelverbindung wurde nach klassischen Methoden der Peptidchemie durch HATU-Kupplung von kommerziell erhältlichen N²-Acetyl-N⁶-(tert-butoxycarbonyl)-L-lysin mit Benzyl (2-aminoethyl)carbamat hydrochloride (1:1) in Gegenwart von N,N-Diisopropylethylamin und anschließender Abspaltung der Z-Schutzgruppe durch Hydrierung in DCM/Methanol 1: 1 über 10% Palladium auf Aktivkohle.

LC-MS (Methode 1): Rₜ = 0.43 min; MS (ESIpos): m/z = 331 (M+H)⁺.

### Intermediat L109

### N²-Acetyl-N⁶-{[2-(trimethylsilyl)ethoxy]carbonyl}-L-lysin

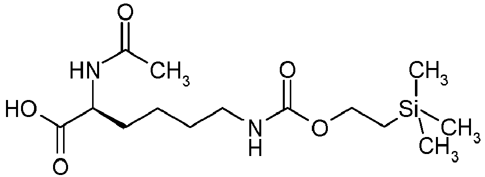

Dieses Intermediat wurde durch Umsetzung von kommerziell erhältlichem N²-Acetyl-L-lysin mit 1-({[2-(Trimethylsilyl)ethoxy]carbonyl}oxy) pyrrolidin-2,5-dion in DMF/Wasser 1:1 in Gegenwart von N,NDiisopropylethylamin erhalten.

LC-MS (Method 1): Rₜ = 0.87 min; MS (ESIpos): m/z = 333 (M+H)⁺.

### Intermediat L110

### N²-Acetyl-N⁶-{[2-(trimethylsilyl)ethoxy]carbonyl}-L-lysyl-L-alanyl-L-alanyl-L-asparagin

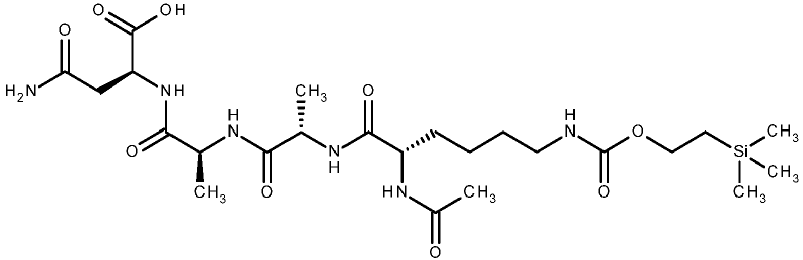

Die Synthese der Titelverbindung begann mit der Kupplung von N-[(Benzyloxy)carbonyl]-L-alanyl-L-alanin und tert-Butyl L-asparaginat in DMF in Gegenwart von HATU and N,N-Diisopropylethylamin and anschließender Abspaltung der Z-Schutzgruppe durch Hydrierung über 10% Palladium auf Aktivkohle in Methanol unter Normaldruck. Anschließend wurde das entschützte Intermediat mit Intermediat L109 in DMF in Gegenwart von HATU und N,N-Diisopropyl-ethylamin gekuppelt. Daraufhin erfolgte die vollständige Entschützung durch 1h Rühren in einer 7.5%-igen Lösung von Trifluoressigsäure in DCM. Im letzten Schritt wurde die Titelverbindung durch erneute Schützung der freien Aminogruppe durch Umsetzung mit 1-({[2-(Trimethylsilyl)ethoxy] carbonyl}oxy) pyrrolidin-2,5-dion in DMF/Wasser1:1 in Gegenwart von *N,N*-Diisopropylethylamin hergestellt.

LC-MS (Method 1): Rₜ = 0.71 min; MS (ESIpos): m/z = 589 (M+H)⁺.

### Intermediat L111

### N-(Pyridin-4-ylacetyl)-L-alanyl-N-methyl-L-alanyl-L-asparagin

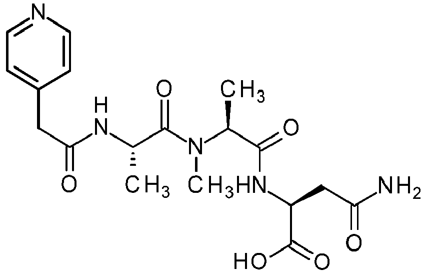

Die Synthese der Titelverbindung erfolgte nach klassischen Methoden der Peptidchemie beginnend mit der HATU-Kupplung von N-[(Benzyloxy)carbonyl]-L-alanin mit tert-Butyl-N-methyl-L-alaninat hydrochlorid (1:1) in Gegenwart von N,N-Diisopropylethylamin und anschließender Entschützung der Carboxygruppe mit Trifluoressigsäure in DCM. Anschließend erfolgte die Kupplung mit tert-Butyl-L-asparaginat in Gegenwart von HATU und N,N-Diisopropylethylamin und dann die hydrogenolytische Abspaltung der Z-Schutzgruppe in DCM/Methanol 1:1 über 10%-igem Palladium auf Aktivkohle bei RT unter Wasserstoff-Normaldruck. Schließlich wurde das erhaltene Intermediat durch Kupplung mit 4-Pyridinessigsäure in Gegenwart von HATU und N,N-Diisopropylethylamin und anschließender Entschützung der Carboxygruppe mit Trifluoressigsäure in DCM in die Titelverbindung überführt.

LC-MS (Methode 1): Rₜ = 0.16 min; MS (ESIpos): m/z = 408 (M+H)⁺.

### Intermediat L112

### N-(Pyridin-4-ylacetyl)-L-alanyl-N-methyl-L-alanyl-L-asparagin

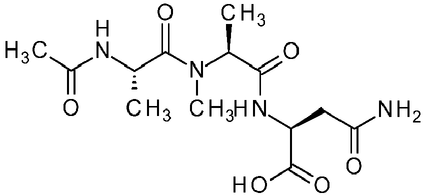

Die Synthese der Titelverbindung erfolgte nach klassischen Methoden der Peptidchemie beginnend mit der HATU-Kupplung von N-[(Benzyloxy)carbonyl]-L-alanin mit tert-Butyl-N-methyl-L-alaninat hydrochlorid (1:1) in Gegenwart von N,N-Diisopropylethylamin und anschließender Entschützung der Carboxygruppe mit Trifluoressigsäure in DCM. Anschließend erfolgte die Kupplung mit tert-Butyl-L-asparaginat in Gegenwart von HATU und N,N-Diisopropylethylamin und dann die hydrogenolytische Abspaltung der Z-Schutzgruppe in DCM/Methanol 1:1 über 10%-igem Palladium auf Aktivkohle bei RT unter Wasserstoff-Normaldruck. Schließlich wurde das erhaltene Intermediat durch Kupplung mit 1-Acetoxypyrrolidin-2,5-dion in Gegenwart von N,N-Diisopropylethylamin und anschließender Entschützung der Carboxygruppe mit Trifluoressigsäure in DCM in die Titelverbindung überführt.

LC-MS (Methode 1): Rₜ = 0.16 min; MS (ESIpos): m/z = 331 (M+H)⁺.

### Intermediat L113

### N-Methyl-N-(pyridin-4-ylacetyl)-L-alanyl-L-alanyl-L-asparagin -trifluoressigsäure (1:1)

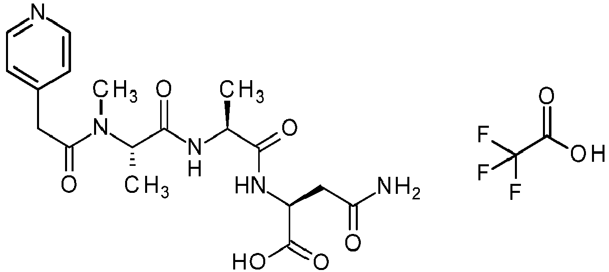

Die Synthese der Titelverbindung erfolgte nach klassischen Methoden der Peptidchemie beginnend mit der HATU-Kupplung von Pyridin-4-ylessigsäure mit tert-Butyl-N-methyl-L-alaninat hydrochlorid (1:1) in Gegenwart von N,N-Diisopropylethylamin und anschließender Entschützung der Carboxygruppe mit Trifluoressigsäure in DCM. Anschließend erfolgte die Kupplung mit tert-Butyl-L-alaninat in Gegenwart von HATU und N,N-Diisopropylethylamin und die erneute Entschützung der Carboxygruppe mit Trifluoressigsäure in DCM. Dann erfolgte die Kupplung mit tert-Butyl-L-asparaginat in Gegenwart von HATU und N,N-Diisopropylethylamin und schließlich die Entschützung der Carboxygruppe mit Trifluoressigsäure in DCM. Nach HPLC-Reinigung wurde die Titelverbindung erhalten.

LC-MS (Methode 1): Rₜ = 0.16 min; MS (ESIpos): m/z = 408 (M+H)⁺.

### Intermediat L114

### N- { [2-(2-Methoxyethoxy)ethoxy] acetyl } -L-alanyl-N-methyl-L-alanyl-L-asparagin

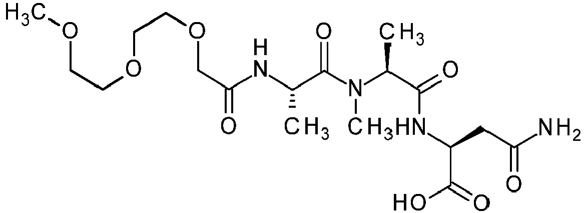

Die Synthese der Titelverbindung erfolgte nach klassischen Methoden der Peptidchemie beginnend mit der HATU-Kupplung von N-[(Benzyloxy)carbonyl]-L-alanin mit tert-Butyl-N-methyl-L-alaninat hydrochlorid (1:1) in Gegenwart von N,N-Diisopropylethylamin und anschließender Entschützung der Carboxygruppe mit Trifluoressigsäure in DCM. Anschließend erfolgte die Kupplung mit tert-Butyl-L-asparaginat in Gegenwart von HATU und N,N-Diisopropylethylamin und dann die hydrogenolytische Abspaltung der Z-Schutzgruppe in DCM/Methanol 1:1 über 10%-igem Palladium auf Aktivkohle bei RT unter Wasserstoff-Normaldruck. Schließlich wurde das erhaltene Intermediat durch Kupplung mit [2-(2-Methoxyethoxy)ethoxy]essigsäure in Gegenwart von HATU und N,N-Diisopropylethylamin und anschließender Entschützung der Carboxygruppe mit Trifluoressigsäure in DCM in die Titelverbindung überführt.

LC-MS (Methode 1): Rₜ = 0.36 min; MS (ESIpos): m/z = 449 (M+H)⁺.

### Intermediat L115

### Trifluoressigsäure dibenzyl-beta-alanyl-L-glutamat (1:1)

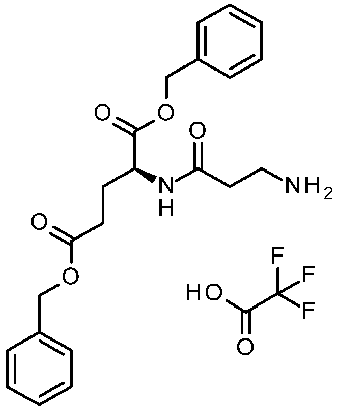

Die Titelverbindung wurde ausgehend von kommerziell erhältlichem 4-Methylbenzolsulfonsäure - dibenzyl-L-glutamat (1:1) nach klassischen Methoden der Peptidchemie durch Kupplung mit N-(tert-butoxycarbonyl)-beta-alanine in Gegenwart von HATU, und schließlich durch Abspaltung der Boc-Schutzgruppe mit TFA hergestellt.
LC-MS (Methode 1): Rₜ = 0.72 min; MS (ESIpos): m/z = 399 [M+H]⁺

### Intermediat F2

### Trifluoressigsäure (2S)-2-amino-4-[{(1R)-1-[1-benzyl-4-(2,5-difluorphenyl)-1H-imidazol-2-yl]-2,2-dimethylpropyl}(glycoloyl)amino]-N-[2-(2,5-dioxo-2,5-dihydro-1H-pyrrol-1-yl)ethyl]butanamid(1: 1)

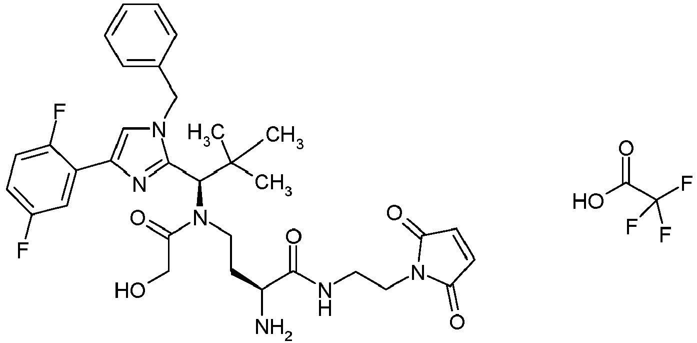

55 mg (0.089 mmol) (2S)-4-[{(1R)-1-[1-Benzyl-4-(2,5-difluorphenyl)-1H-imidazol-2-yl]-2,2-dimethylpropyl}(glycoloyl)amino]-2-[(tert-butoxycarbonyl)amino]butansäure (Intermediat C5) wurden in 12 mL DMF aufgenommen und nacheinander mit 68 mg (0.268 mmol) von kommerziell erhältlichem Trifluoressigsäure--1-(2-aminoethyl)-1H-pyrrol-2,5-dion(1:1), 34.3 mg (0.18 mmol) 1-(3-Dimethylaminopropyl)-3-ethylcarbodiimid-Hydrochlorid, 27.4 mg (0.18 mmol) 1-Hydroxy-1*H*-benzotriazol-Hydrat sowie 47 µl (0.27 mmol) *N,N*-Diisopropylethylamin versetzt. Die Mischung wurde über Nacht bei RT gerührt. Das Lösungsmittel wurde im Vakuum entfernt und der verbliebene Rückstand mittels präparativer HPLC gereinigt. Die entsprechenden Fraktionen wurden eingeengt und nach Lyophilisation aus 1,4-Dioxan wurden 20 mg (30% d.Th.) der Titelverbindung erhalten.
HPLC (Methode 11): Rₜ = 2.48 min;
LC-MS (Methode 1): Rₜ = 1.29 min; MS (ESIpos): m/z = 737 (M+H)⁺.
20 mg (0.027 mmol) dieses Intermediats wurden in 5 mL Dichlormethan aufgenommen, mit 1 mL Trifluoressigsäure versetzt und 1 h bei RT gerührt. Anschließend wurde das Reaktionsgemisch im Vakuum eingeengt und der verbliebene Rückstand aus Acetonitril/Wasser 1:1 lyophilisiert. Es wurden so 19 mg (95% d. Th.) der Titelverbindung erhalten.
HPLC (Methode 11): Rₜ = 2.0 min;
LC-MS (Methode 1): Rₜ = 0.9 min; MS (ESIpos): m/z = 637 (M+H)⁺.
¹H-NMR (500 MHz, DMSO-d₆): δ = 8.28 (t, 1H), 7.9-8.1 (m, 3H), 7.7-7.8 (m, 2H), 7.2-7.4 (m, 6H) 7.0-7.1 (m, 3H), 5.7 (s, 1H), 5.0 und 5.3 (2d, 2H), 4.08 und 4.25 (2d, 2H), 3.3-3.65 (m, 5H), 3.1-3.25 (m, 2H), 0.75 und 1.45 (2m, 2H), 0.9 (s, 9H).

### Intermediat F3

### Trifluoressigsäure--N-[(3S)-3-amino-4-{2-[6-(2,5-dioxo-2,5-dihydro-1H-pyrrol-1-yl)hexanoyl]hydrazino}-4-oxobutyl]-N-{(1R)-1-[1-benzyl-4-(2,5-difluorphenyl)-1H-imidazol-2-yl]-2,2-dimethylpropyl}-2-hydroxyacetamid(1:1)

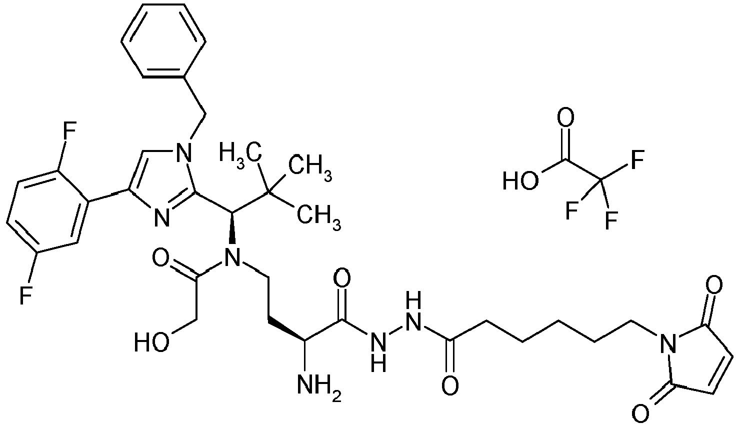

13 mg (0.021 mmol) (2S)-4-[{(1R)-1-[1-Benzyl-4-(2,5-difluorphenyl)-1H-imidazol-2-yl]-2,2-dimethylpropyl}(glycoloyl)amino]-2-[(tert-butoxycarbonyl)amino]butansäure (Intermediat C5) wurden in 5 mL DMF aufgenommen und anschließend mit 33 mg (86 µmol) *O*-(7-Azabenzotriazol-1-yl)-*N,N,N;N'*-tetramethyluronium-hexafluorophosphat, 15 µL *N,N-*Diisopropylethylamin sowie mit 22 mg (64 µmol) von kommerziell erhältlichem 6-(2,5-Dioxo-2,5-dihydro-1*H*-pyrrol-1-yl)hexanhydrazid versetzt. Das Reaktionsgemisch wurde 1 h bei RT gerührt. Anschließend wurde im Hochvakuum eingeengt und der verbliebene Rückstand mittels präparativer HPLC aufgereinigt. So wurden 9.5 mg (53 % d.Th.) des geschützten Intermediats als farbloser Schaum erhalten.
HPLC (Methode 11): Rₜ = 2.1 min;
LC-MS (Methode 1): Rₜ = 1.33 min; MS (ESIpos): m/z = 822 (M+H)⁺.
9.5 mg (0.011 mmol) dieses Intermediats wurden in 3 mL Dichlormethan aufgenommen, mit 1 mL Trifluoressigsäure versetzt und 2 h bei RT gerührt. Anschließend wurde das Reaktionsgemisch im Vakuum eingeengt und der verbliebene Rückstand aus Acetonitril/Wasser 1:1 lyophilisiert. Es wurden so 7 mg (70% d. Th.) der Titelverbindung erhalten.
HPLC (Methode 11): Rₜ = 1.75 min;
LC-MS (Methode 1): Rₜ = 0.91 min; MS (ESIpos): m/z = 722 (M+H)⁺.

### Intermediat F4

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-(6-{[(2R)-2-(2,5-dioxo-2,5-dihydro-1H-pyrrol-1-yl)propanoyl]amino}hexyl)butanamid(1:1)

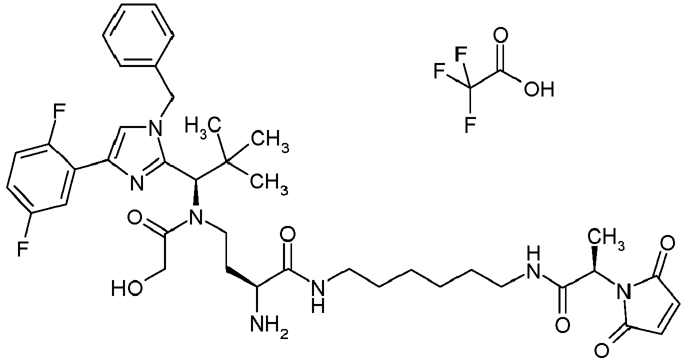

Zunächst wurden 30 mg (0.049 mmol) (2S)-4-[{(1R)-1-[1-Benzyl-4-(2,5-difluorphenyl)-1H-imidazol-2-yl]-2,2-dimethylpropyl}(glycoloyl)amino]-2-[(tert-butoxycarbonyl)amino]butansäure (Intermediat C5) in Analogie zu Intermediat F3 mit Trifluoressigsäure--9H-fluoren-9-ylmethyl-(6-aminohexyl) carbamat(1:1) in Gegenwart von HATU gekuppelt. Anschließend wurde mit Piperidin nach Standardmethoden die Fmoc-Schutzgruppe entfernt. Diese Aminkomponente wurde dann mit (2R)-2-(2,5-Dioxo-2,5-dihydro-1H-pyrrol-1-yl)propanoylchlorid, das aus der freien Säure mit Thionylchlorid hergestellt wurde, in Gegenwart von *N,N*-Diisopropylethylamin gekuppelt. Im letzten Schritt wurde die Boc-Schutzgruppe mit Trifluoressigsäure in DCM entfernt. Es wurden 1.1 mg (3% über 4 Stufen) der Titelverbindung erhalten.
HPLC (Methode 11): Rₜ = 1.83 min;
LC-MS (Methode 1): Rₜ = 0.96 min; MS (ESIpos): m/z = 764 (M+H)⁺.

### Intermediat F5

### Trifluoressigsäure--(2S)-2-amino-4-[{(1R)-1-[1-benzyl-4-(2,5-difluorphenyl)-1H-imidazol-2-yl]-2,2-dimethylpropyl}(propionyl)amino]-N-{2-[2-(2,5-dioxo-2,5-dihydro-1H-pyrrol-1-yl)ethoxy]ethyl}butanamid(1:1)

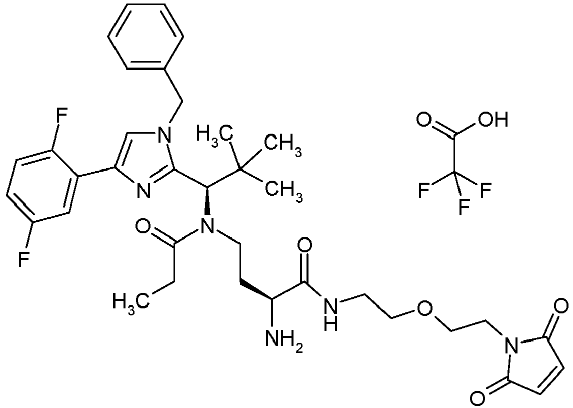

Die Titelverbindung wurde in Analogie zu Intermediat F2 aus 16 mg (0.026 mmol) Intermediat C5 und 8.5 mg (0.03 mmol) Intermediat L12 hergestellt. Es wurden 3 mg (13% d.Th. über 2 Stufen) der Titelverbindung erhalten.
HPLC (Methode 11): Rₜ = 2.0 min;
LC-MS (Methode 1): Rₜ = 0.96 min; MS (ESIpos): m/z = 681 (M+H)⁺.

### Intermediat F6

### Trifluoressigsäure--N-[(16S)-16-amino-1-(2,5-dioxo-2,5-dihydro-1H-pyrrol-1-yl)-12,15-dioxo-3,6,9-trioxa-13,14-diazaoctadecan-18-yl]-N-{(1R)-1-[1-benzyl-4-(2,5-difluorphenyl)-1H-imidazol-2-yl]-2,2-dimethylpropyl}-2-hydroxyacetamid(1:1)

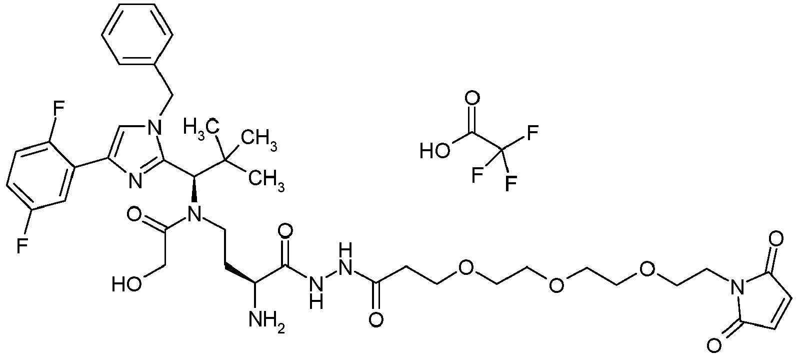

8 mg (12.7 µmol) Trifluoressigsäure--tert-butyl-{(2S)-4-[{(1R)-1-[1-benzyl-4-(2,5-difluorphenyl)-1H-imidazol-2-yl]-2,2-dimethylpropyl}(glycoloyl)amino]-1-hydrazino-1-oxobutan-2-yl}carbamat(1:1) (Intermediat C6) wurden in 8 mL DMF aufgenommen und mit 6 mg (19 µmol) von kommerziell erhältlicher 3-(2-{2-[2-(2,5-Dioxo-2,5-dihydro-1H-pyrrol-1-yl)ethoxy]ethoxy}ethoxy)propansäure, 5.8 mg (15 µmol) *O*-(7-Azabenzotriazol-1-yl)-*N,N,N',N'-*tetramethyluronium-hexafluorophosphat (HATU) sowie 7 µl (38 µmol) *N,N-*Diisopropylethylamin versetzt. Die Mischung wurde 15 min bei RT gerührt. Anschließend wurde das Lösungsmittel im Vakuum entfernt und der Rückstand in Acetonitril/Wasser 1:1 aufgenommen und mit Trifluoressigsäure auf pH 2 gebracht. Die Reinigung erfolgte durch präparative HPLC. Nach Vereinigung der entsprechenden Fraktionen, Einengen und Gefriertrocknung aus Acetonitril/Wasser 1: 1 wurden 5 mg (41% d.Th.) des Boc-geschützten Intermediats erhalten. Nach Abspaltung der Boc-Gruppe mit Trifluoressigsäure wurden 4 mg (32% d.Th. über 2 Stufen) der Titelverbindung erhalten.
HPLC (Methode 11): Rₜ = 1.89 min;
LC-MS (Methode 1): Rₜ = 0.89 min; MS (ESIpos): m/z = 812 (M+H)⁺.

### Intermediat F7

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-(2-{[(2,5-dioxo-2,5-dihydro-1H-pyrrol-1-yl)acetyl]amino}ethyl)butanamid(1:1)

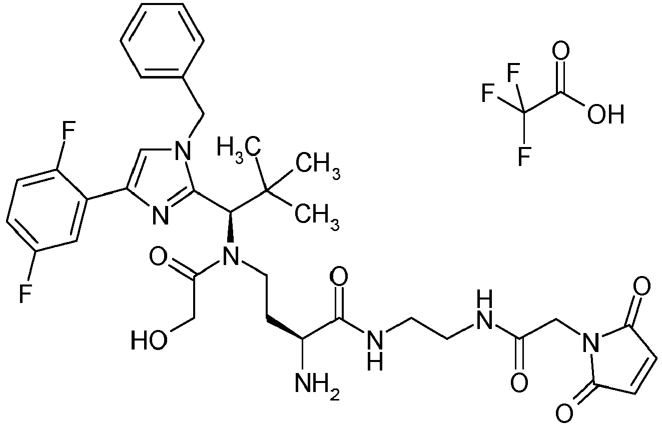

Die Titelverbindung wurde in Analogie zu Intermediat F2 aus 25 mg (0.037 mmol) Intermediat C5 und 35 mg (0.112 mmol) Intermediat L1 hergestellt. Es wurden 14.4 mg (29% d.Th. über 2 Stufen) der Titelverbindung erhalten.
HPLC (Methode 11): Rₜ = 2.0 min;
LC-MS (Methode 1): Rₜ = 0.9 min; MS (ESIpos): m/z = 694 (M+H)⁺.
¹H-NMR (500 MHz, DMSO-d₆): δ = 8.2 (m, 1H), 7.9-8.1 (m, 3H), 7.7-7.8 (m, 2H), 7.2-7.4 (m, 6H), 7.0-7.12 (m, 3H), 5.7 (s, 1H), 4.95 und 5.3 (2d, 2H), 4.1 und 4.25 (2d, 2H), 4.0 (s, 2H), 3.3-3.65 (m, 5H), 3.0-3.15 (m, 2H), 0.7 und 1.45 (2m, 2H), 0.88 (s, 9H).

### Intermediat F8

### N-[6-(2,5-Dioxo-2,5-dihydro-1H-pyrrol-1-yl)hexanoyl]-L-valyl-L-alanyl-N⁶-{(2S)-2-amino-4-[{(1R)-1-[1-benzyl-4-(2,5-difluorphenyl)-1H-imidazol-2-yl]-2,2-dimethylpropyl} (glycoloyl)amino]butanoyl}-L-lysin-trifluoressigsäure(1:1)

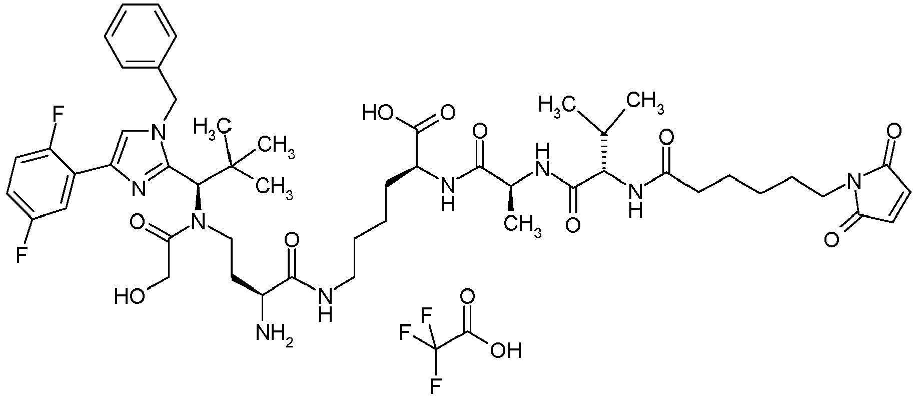

Die Titelverbindung wurde in Analogie zu Intermediat F2 aus 10 mg (0.016 mmol) Intermediat C5 und 13 mg (0.018 mmol) Intermediat L6 hergestellt. Es wurden 10 mg (49% d.Th. über 2 Stufen) der Titelverbindung erhalten.
HPLC (Methode 11): Rₜ = 1.97 min;
LC-MS (Methode 1): Rₜ = 0.93 min; MS (ESIpos): m/z = 1006 (M+H)⁺.

### Intermediat F9

### Trifluoressigsäure--N-{(3S)-3-amino-4-[1-(2-{[2-(2,5-dioxo-2,5-dihydro-1H-pyrrol-1-yl)ethyl]amino}-2-oxoethyl)hydrazino]-4-oxobutyl}-N-{(1R)-1-[1-benzyl-4-(2,5-difluorphenyl)-1H-imidazol-2-yl]-2,2-dimethylpropyl}-2-hydroxyacetamid(1:1)

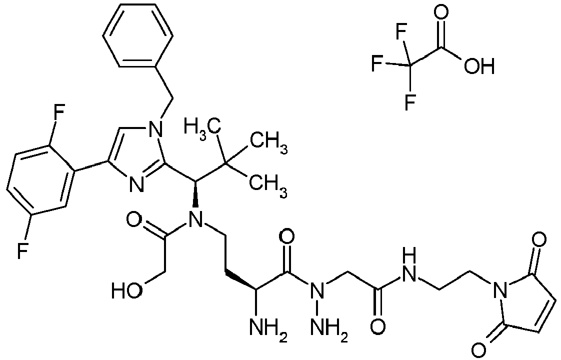

Die Titelverbindung wurde in Analogie zu Intermediat F2 aus 1.5 mg (0.002 mmol) Intermediat C7 und 0.95 mg (0.004 mmol) von kommerziell erhältlichem Trifluoressigsäure--1-(2-aminoethyl)-1H-pyrrol-2,5-dion(1:1) hergestellt. Es wurden 1.1 mg (52% d.Th. über 2 Stufen) der Titelverbindung erhalten.
HPLC (Methode 11): Rₜ = 1.9 min;
LC-MS (Methode 1): Rₜ = 0.89 min; MS (ESIpos): m/z = 709 (M+H)⁺.

### Intermediat F10

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-(3-{[4-(2,5-dioxo-2,5-dihydro-1H-pyrrol-1-yl)phenyl]amino}-3-oxopropyl)butanamid(1:1)

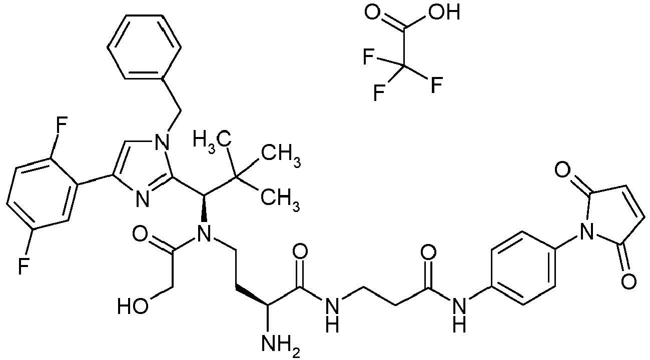

Die Titelverbindung wurde in Analogie zu Intermediat F2 aus 14 mg (0.022 mmol) Intermediat C5 und 10 mg (0.025 mmol) von Intermediat L5 hergestellt. Es wurden 4.5 mg (22% d.Th. über 2 Stufen) der Titelverbindung erhalten.
HPLC (Methode 11): Rₜ = 2.0 min;
LC-MS (Methode 1): Rₜ = 0.93 min; MS (ESIpos): m/z = 756 (M+H)⁺.

### Intermediat F11

### Trifluoressigsäure--N-[2-({(2S)-2-amino-4-[{(1R)-1-[1-benzyl-4-(2,5-difluorphenyl)-1H-imidazol-2-yl]-2,2-dimethylpropyl}(glycoloyl)amino]butanoyl}amino)ethyl]-4-(2,5-dioxo-2,5-dihydro-1H-pyrrol-1-yl)cyclohexancarboxamid(1:1)

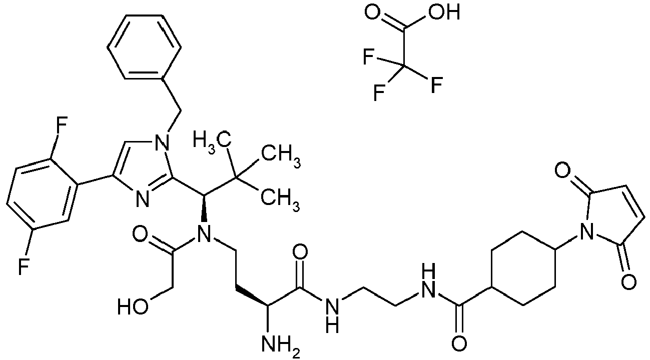

Die Titelverbindung wurde in Analogie zu Intermediat F2 aus 12 mg (0.019 mmol) Intermediat C5 und 10 mg (0.021 mmol) von Intermediat L4 hergestellt. Es wurden 7 mg (38% d.Th. über 2 Stufen) der Titelverbindung erhalten.
HPLC (Methode 11): Rₜ = 2.04 min;
LC-MS (Methode 1): Rₜ = 0.93 min; MS (ESIpos): m/z = 776 (M+H)⁺.

### Intermediat F12

### Trifluoressigsäure--(1R,2S)-2-({(2S)-2-amino-4-[{(1R)-1-[1-benzyl-4-(2,5-difluorphenyl)-1H-imidazol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

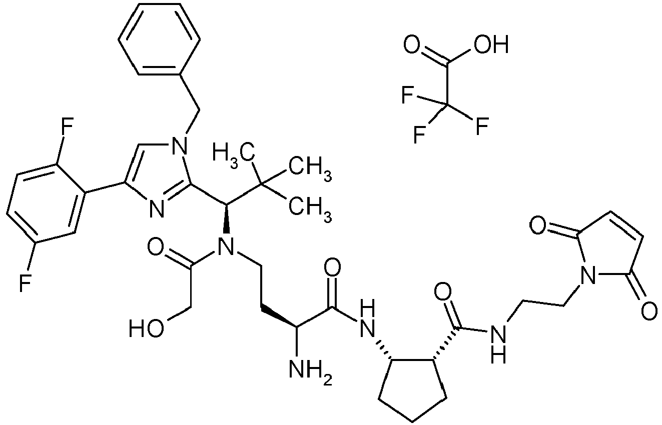

Die Titelverbindung wurde in Analogie zu Intermediat F2 aus 43 mg (0.071 mmol) Intermediat C5 und 30 mg (0.071 mmol) von Intermediat L2 hergestellt. Auf der Boc-geschützten Zwischenstufe wurden die enstandenen Diastereomere durch präparative HPLC (Chromatorex C18-10 / 125x30 / 12mL/min) voneinander getrennt. Durch Vergleiche mit dem aus kommerziell erhältlicher (1S, 2R)-2-[(tert-Butoxycarbonyl)amino]cyclopentancarbonsäure in analoger Weise hergestellten Einzeldiasteromer konnte die Stereochemie der aufgetrennten Diastereomere zugeordnet werden: Fraktion 1: 1S2R Diastereomer 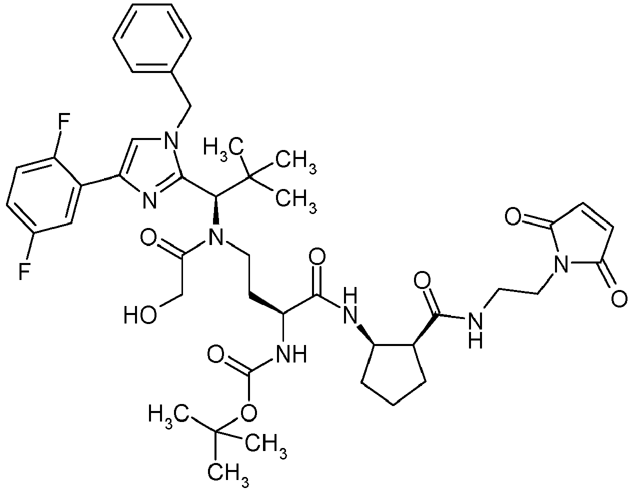
Ausbeute: 13mg (22%)
HPLC (Methode 11): Rₜ = 2.52 min;
LC-MS (Methode 1): Rₜ = 1.31 min; MS (ESIpos): m/z = 848 (M+H)⁺.
Fraktion 2: 1R2S Diastereomer 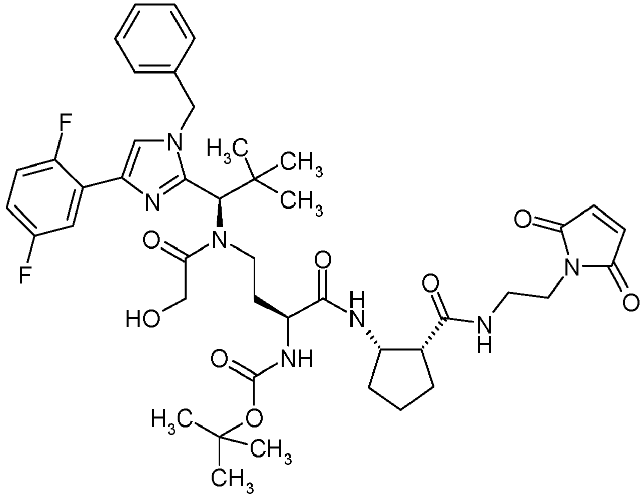
Ausbeute: 10 mg (17%)
HPLC (Methode 11): Rₜ = 2.56 min;

LC-MS (Methode 1): Rₜ = 1.33 min; MS (ESIpos): m/z = 848 (M+H)⁺.

Die Entschützung von 10 mg (0.011 mmol) vom 1R2S Diastereomer mit TFA führte dann zu 8 mg (75% d.Th.) der Titelverbindung.
HPLC (Methode 11): Rₜ = 2.04 min;
LC-MS (Methode 1): Rₜ = 0.92 min; MS (ESIpos): m/z = 748 (M+H)⁺.

### Intermediat F13

### Trifluoressigsäure--(1S,2R)-2-({(2S)-2-amino-4-[{(1R)-1-[1-benzyl-4-(2,5-difluorphenyl)-1H-imidazol-2-yl]-2,2-dimethylpropyl } (glycoloyl)amino]butanoyl } amino)-N-[2-(2,5-dioxo-2,5-dihydro-1H-pyrrol-1-yl)ethyl]cyclopentancarboxamid(1: 1)

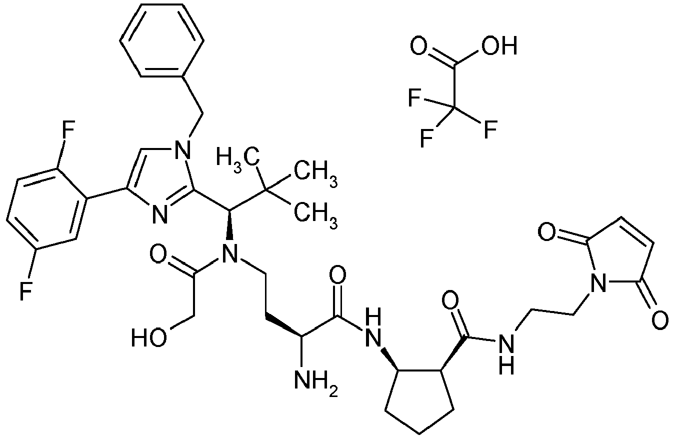

Die Synthese erfolgte analog Intermediat F13 und die Titelverbindung wurde durch Entschützung des 1S2R-Diastereomers erhalten.
HPLC (Methode 11): Rₜ = 2.1 min;
LC-MS (Methode 1): Rₜ = 0.94 min; MS (ESIpos): m/z = 748 (M+H)⁺.

### Intermediat F14

### Trifluoressigsäure--N-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-L-valyl-N⁵-carbamoyl-N-[4-(2,5-dioxo-2,5-dihydro-1H-pyrrol-1-yl)phenyl]-L-ornithinamid(1:1)

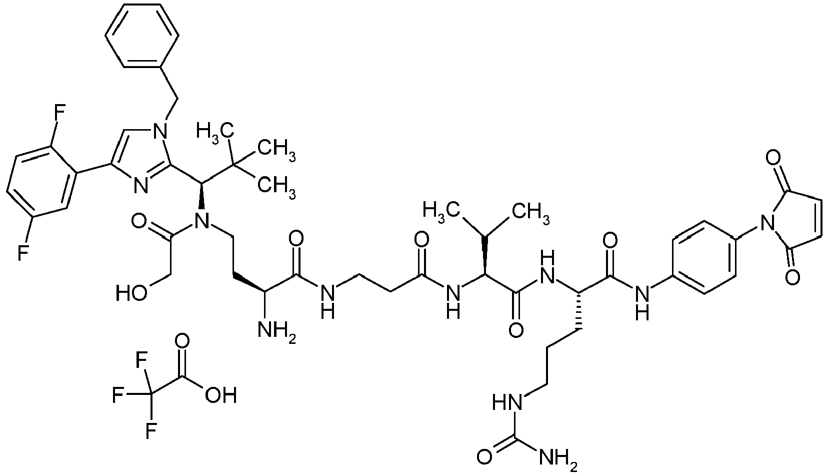

Die Titelverbindung wurde durch Kupplung von 20 mg (0.028 mmol) Intermediat C5 und 18 mg (0.028 mmol) Intermediat L7 in Gegenwart von HATU und anschließender Deblockierung mit TFA hergestellt. Es wurden 15 mg (49% d.Th. über 2 Stufen) der Titelverbindung erhalten. HPLC (Methode 11): Rₜ = 1.97 min;

LC-MS (Methode 1): Rₜ = 0.91 min; MS (ESIpos): m/z = 1012 (M+H)⁺.

### Intermediat F15

### Trifluoressigsäure--N-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-L-alanyl-N⁵-carbamoyl-N-[4-(2,5-dioxo-2,5-dihydro-1H-pyrrol-1-yl)phenyl]-L-ornithinamid(1:1)

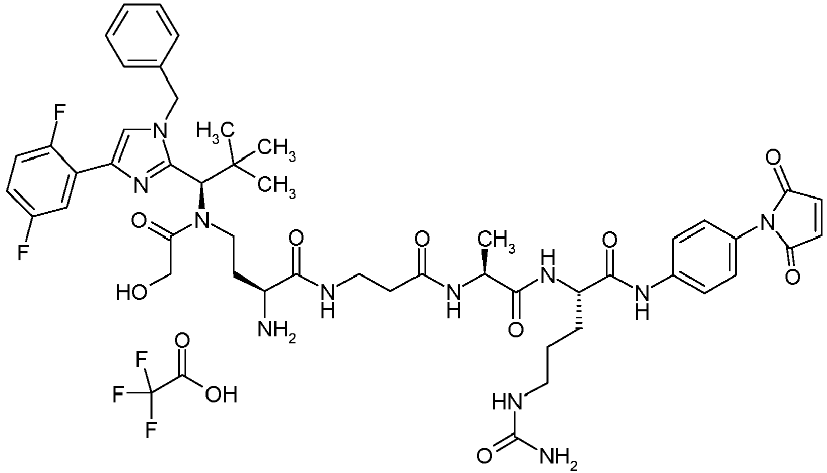

Dieses Intermediat wurde durch Kupplung von 15 mg (0.022 mmol) von Intermediat C8 und 14 mg (0.026 mmol) Intermediat L8 in Gegenwart von HATU und anschließender Deblockierung mit TFA hergestellt. Es wurden 7 mg (27% d.Th. über 2 Stufen) der Titelverbindung erhalten.
HPLC (Methode 11): Rₜ = 1.85 min;
LC-MS (Methode 1): Rₜ = 0.87 min; MS (ESIpos): m/z = 984 (M+H)⁺.

### Intermediat F16

### Trifluoressigsäure--N-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-L-valyl-N⁵-carbamoyl-N-[4-(2-{[2-(2,5-dioxo-2,5-dihydro-1H-pyrrol-1-yl)ethyl]amino}-2-oxoethyl)phenyl]-L-ornithinamid(1:1)

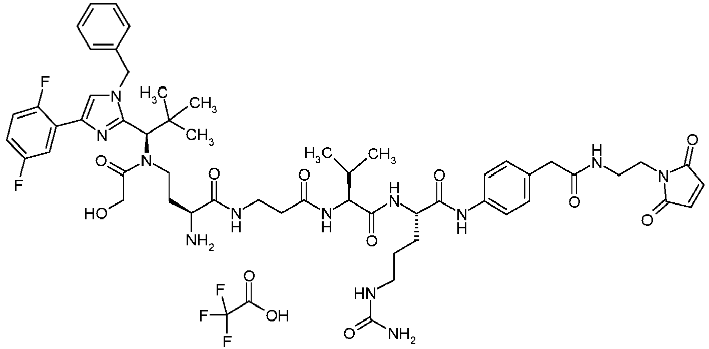

Zunächst wurden 20 mg (0.03 mmol) Intermediat C3 in Analogie zu Intermediat F3 mit Trifluoressigsäure--beta-alanyl-L-valyl-N5-carbamoyl-N-[4-(2-methoxy-2-oxoethyl)phenyl]-L-ornithinamid(1:1) (Intermediat L9) in Gegenwart von HATU gekuppelt (Ausbeute: 15 mg (44% d. Th.).

26 mg (0.023 mmol) von dieser Zwischenstufe N-{(2S)-4-[(Acetoxyacetyl){(1R)-1-[1-benzyl-4-(2,5-difluorphenyl)-1H-imidazol-2-yl]-2,2-dimethylpropyl} amino]-2-[(tert-butoxycarbonyl)amino] butanoyl}-beta-alanyl-L-valyl-N5-carbamoyl-N-[4-(2-methoxy-2-oxoethyl)phenyl]-L-omithinamid wurden in 5 mL Methanol gelöst, mit 1mL einer 2M Lithiumhydroxidlösung versetzt und der Ansatz 90 min bei RT gerührt. Anschließend wurde das Lösungsmittel im Vakuum entfernt, der Rückstand in Acetonitril/Wasse aufgenommen und der Ansatz dann mit TFA auf pH 2 gebracht. Dann wurde wiederum eingengt und nach Reinigung des Rückstands durch präparative HPLC wurden 20 mg (81%) der Carboxylverbindung erhalten.

Diese Zwischenstufe wurde dann in 5 mL DMF aufgenommen und mit 6 mg (0.022 mmol) von kommerziell erhältlichem Trifluoressigsäure--1-(2-aminoethyl)-1H-pyrrol-2,5-dion(1:1) in Gegenwart von 8,4 mg (0.022 mmol) HATU und 16µl *N*,*N*-Diisopropylethylamin gekuppelt. Nach Reinigung durch präparative HPLC wurden 17 mg (76% d.Th.) der geschützten Zwischenstufe erhalten. Diese wurden in 3 mL DCM aufgenommen und mit 1 mL TFA versetzt. Nach 45 min Rühren bei RT wurde eingeengt und der Rückstand mit Diethylether digeriert. Nach Absaugen und Trocknung des Rückstandes im Hochvakuum verblieben 15 mg (81%) der Titelverbindung HPLC (Methode 11): Rₜ = 1.9 min;

LC-MS (Methode 1): Rₜ = 0.9 min; MS (ESIpos): m/z = 1097 (M+H)⁺.

### Intermediat F17

### N-[6-(2,5-Dioxo-2,5-dihydro-1H-pyrrol-1-yl)hexanoyl]-L-valyl-L-alanyl-N⁶-{[(1R,2S)-2-({(2S)-2-amino-4-[{(1R)-1-[1-benzyl-4-(2,5-difluorphenyl)-1H-imidazol-2-yl]-2,2-dimethylpropyl} (glycoloyl)amino]butanoyl}amino)cyclopentyl]carbonyl}-L-lysin-trifluoressigsäure(1:1)

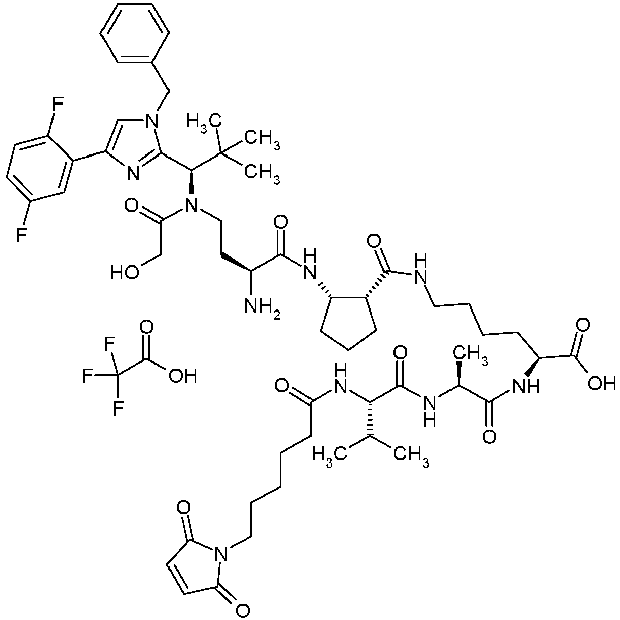

Die Titelverbindung wurde in Analogie zu Intermediat F12 aus 6 mg (0.01 mmol) Intermediat C5 und 8 mg (0.01 mmol) von Intermediat L10 hergestellt. Auf der Boc-geschützten Zwischenstufe wurden die enstandenen Diastereomere durch präparative HPLC (Chromatorex C18-10 / 125x30 / 12mL/min) voneinander getrennt. Durch Vergleiche mit dem aus kommerziell erhältlicher (1S, 2R)-2-[(tert-Butoxycarbonyl)amino]cyclopentancarbonsäure in analoger Weise hergestellten Einzeldiastereomer konnte die Stereochemie der aufgetrennten Diastereomere zugeordnet werden: Fraktion 1: 1S2R Diastereomer 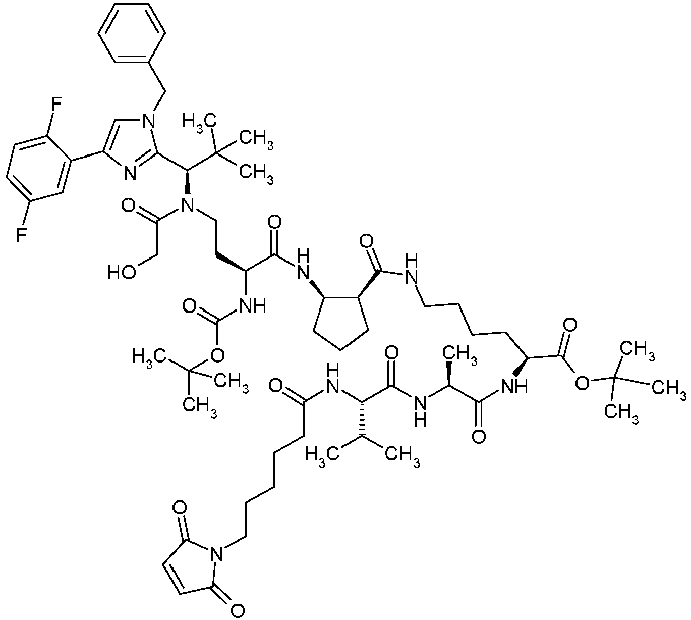
Ausbeute: 1mg
HPLC (Methode 11): Rₜ = 2.73 min;
LC-MS (Methode 1): Rₜ = 1.37 min; MS (ESIpos): m/z = 1274 (M+H)⁺.
Fraktion 2: 1R2S Diastereomer 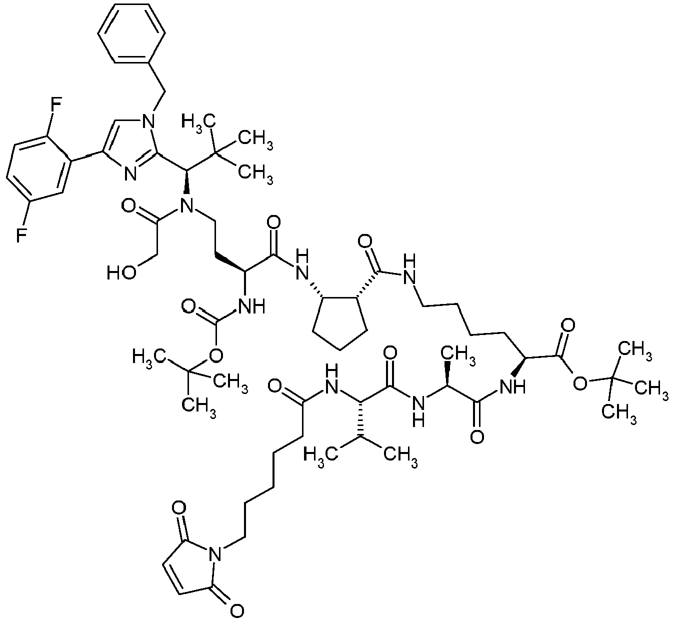
Ausbeute: 0.7 mg
HPLC (Methode 11): Rₜ = 2.81 min;

LC-MS (Methode 1): Rₜ = 1.41 min; MS (ESIpos): m/z = 1274 (M+H)⁺.

Die vollständige Entschützung von 0.7 mg (0.001 mmol) des 1R2S-Diastereomers wurde durch Lösen in 1 mL DCM, Zugabe von 1 mL TFA und 1h Rühren bei RT erreicht. Nach Einengen im Vakuum und Lyophilisation des Rückstandes aus Acetonitril/Wasser wurden 0.68 mg (94% d. Th.) der Titelverbindung erhalten.
HPLC (Methode 11): Rₜ = 2.1 min;
LC-MS (Methode 1): Rₜ = 0.97 min; MS (ESIpos): m/z = 1117 (M+H)⁺.

### Intermediat F18

### N-[6-(2,5-Dioxo-2,5-dihydro-1H-pyrrol-1-yl)hexanoyl]-L-valyl-L-alanyl-N⁶-{[(1S,2R)-2-({(2S)-2-amino-4-[{(1R)-1-[1-benzyl-4-(2,5-difluorphenyl)-1H-imidazol-2-yl]-2,2-dimethylpropyl} (glycoloyl)amino]butanoyl}amino)cyclopentyl]carbonyl}-L-lysin-trifluoressigsäure(1:1)

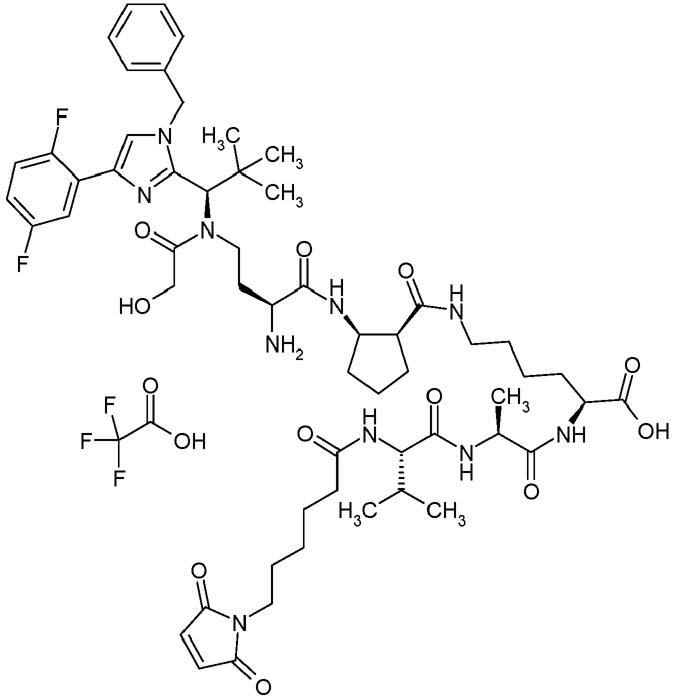

Die Titelverbindung wurde in Analogie zu Intermediat F17 aus 8.9 mg (0.014 mmol) Intermediat C5 und 13 mg (0.014 mmol) von Intermediat L11 hergestellt.
HPLC (Methode 11): Rₜ = 2.2 min;
LC-MS (Methode 1): Rₜ = 1.01 min; MS (ESIpos): m/z = 1117 (M+H)⁺.

### Intermediat F19

### N⁶-{(2S)-2-Amino-4-[{(1R)-1-[1-benzyl-4-(2,5-difluorphenyl)-1H-imidazol-2-yl]-2,2-dimethylpropyl}(glycoloyl)amino]butanoyl}-N²-[(2,5-dioxo-2,5-dihydro-1H-pyrrol-1-yl)acetyl]-L-lysin-trifluoressigsäure(1:1)

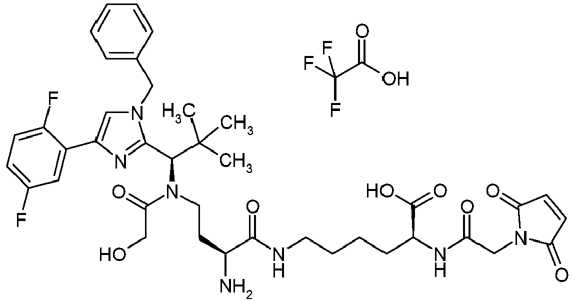

Die Titelverbindung wurde in Analogie zu Intermediat F2 durch Kupplung von 25 mg (0.041 mmol) Intermediat C5 mit 55 mg (0.122 mmol) von Intermediat L13 und anschließender Entschützung hergestellt.
HPLC (Methode 11): Rₜ = 1.84 min;
LC-MS (Methode 1): Rₜ = 0.88 min; MS (ESIpos): m/z = 780 (M+H)⁺.

### Intermediat F20

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-{4-[(2,5-dioxo-2,5-dihydro-1H-pyrrol-1-yl)acetyl]piperazin-1-yl}butanamid(1:1)

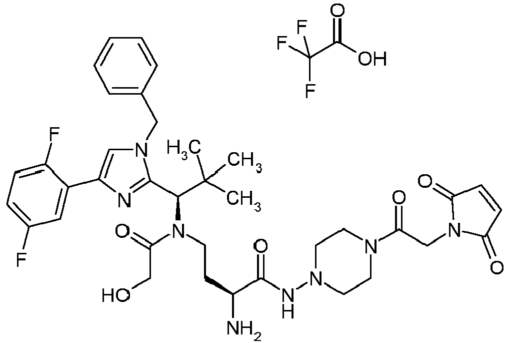

Die Titelverbindung wurde in Analogie zu Intermediat F2 durch Kupplung von 10 mg (0.015 mmol) Intermediat C5 mit 55 mg (0.122 mmol) von Intermediat L14 und anschließender Entschützung hergestellt.
HPLC (Methode 11): Rₜ = 1.9 min;
LC-MS (Methode 1): Rₜ = 0.87 min; MS (ESIpos): m/z = 735 (M+H)⁺.

### Intermediat F21

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-[1-(2,5-dioxo-2,5-dihydro-1H-pyrrol-1-yl)-12-oxo-3,6,9-trioxa-13-azapentadecan-15-yl]butanamid(1:1)

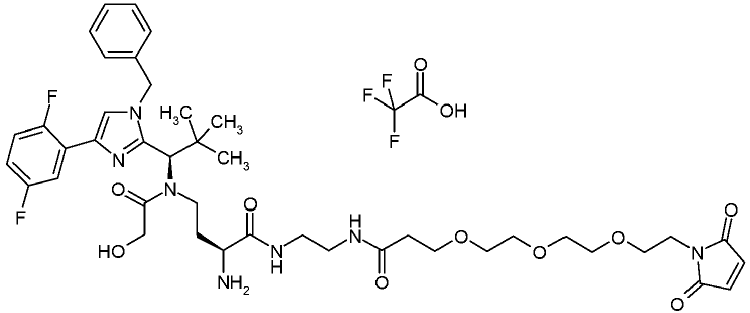

Die Titelverbindung wurde in Analogie zu Intermediat F2 durch Kupplung von 10 mg (0.015 mmol) Intermediat C5 mit 7 mg (0.015 mmol) von Intermediat L15 und anschließender Entschützung hergestellt.

LC-MS (Methode 1): Rₜ = 0.89 min; MS (ESIpos): m/z = 840 (M+H)⁺.

### Intermediat F22

### Trifluoressigsäure--N-[(3S)-3-amino-4-(1-{2-[(2-{[(2,5-dioxo-2,5-dihydro-1H-pyrrol-1-yl)acetyl]amino}ethyl)amino]-2-oxoethyl}hydrazino)-4-oxobutyl]-N-{(1R)-1-[1-benzyl-4-(2,5-difluorphenyl)-1H-imidazol-2-yl]-2,2-dimethylpropyl}-2-hydroxyacetamid(1:1)

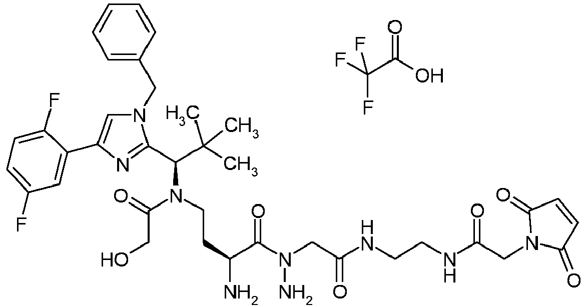

Die Titelverbindung wurde in Analogie zu Intermediat F9 durch Kupplung von 13.7 mg (0.017 mmol) Intermediat C7 mit 5.9 mg (0.017 mmol) von Intermediat L1 und anschließender Entschützung hergestellt.
HPLC (Methode 11): Rₜ = 2.3 min;
LC-MS (Methode 1): Rₜ = 1.2 min; MS (ESIpos): m/z = 866 (M+H)⁺.

### Intermediat F23

### N-[6-(2,5-Dioxo-2,5-dihydro-1H-pyrrol-1-yl)hexanoyl]-L-valyl-N⁵-carbamoyl-L-ornithyl-N⁶-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-L-lysin-trifluoressigsäure(1:1)

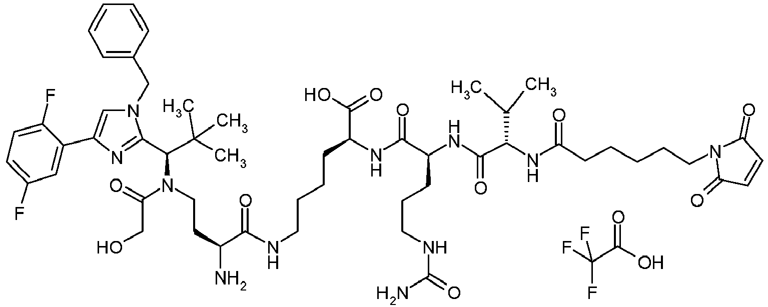

Die Titelverbindung wurde in Analogie zu Intermediat F2 durch Kupplung von 10 mg (0.016 mmol) Intermediat C5 mit 16.8 mg (0.016 mmol) von Intermediat L17 in Gegenwart von EDC/HOBT und *N*,*N*-Diisopropylethylamin und anschließender Entschützung hergestellt.
HPLC (Methode 11): Rₜ = 1.9 min;
LC-MS (Methode 1): Rₜ = 0.9 min; MS (ESIpos): m/z = 1092 (M+H)⁺.

### Intermediat F24

### Trifluoressigsäure--N-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-L-alanyl-N⁵-carbamoyl-N-[4-(2-{[2-(2,5-dioxo-2,5-dihydro-1H-pyrrol-1-yl)ethyl]amino}-2-oxoethyl)phenyl]-L-ornithinamid(1:1)

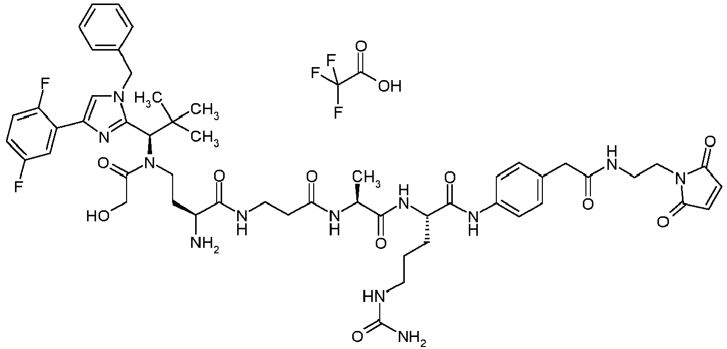

Die Herstellung der Titelverbindung erfolgte in Analogie zu Intermediat F16:
Zunächst wurden 30 mg (0.046 mmol) Intermediat C3 in Analogie zu Intermediat F3 mit Intermediat L18 in Gegenwart von HATU gekuppelt (Ausbeute: 25 mg (47% d. Th.). 27 mg (0.024 mmol) von dieser Zwischenstufe wurden in 5 mL Methanol gelöst, mit 1mL einer 2M Lithiumhydroxidlösung versetzt und der Ansatz 30 min bei RT gerührt, wobei der Methylester sowie die Acetylgruppe gespalten wurden. Anschließend wurde das Lösungsmittel im Vakuum entfernt, der Rückstand in Acetonitril/Wasse aufgenommen und der Ansatz dann mit TFA auf pH 2 gebracht. Dann wurde wiederum eingengt und nach Reinigung des Rückstands durch präparative HPLC wurden 15 mg (58%) der Carboxylverbindung erhalten.

Diese Zwischenstufe wurde dann in 3 mL DMF aufgenommen und mit 4.4 mg (0.017 mmol) von kommerziell erhältlichem Trifluoressigsäure--1-(2-aminoethyl)-1H-pyrrol-2,5-dion(1:1) in Gegenwart von 6.5 mg (0.017 mmol) HATU und 12µl *N*,*N*-Diisopropylethylamin gekuppelt. Nach Reinigung durch präparative HPLC wurden 12 mg (72% d.Th.) der geschützten Zwischenstufe erhalten. Diese wurden in 2 mL DCM aufgenommen und mit 1 mL TFA versetzt. Nach 30 min Rühren bei RT wurde eingeengt und aus Acetonitril/Wasser 1:1 lyophilisiert. Nach Trocknung des Rückstandes im Hochvakuum verblieben 11 mg (91%) der Titelverbindung.

LC-MS (Methode 1): Rₜ = 0.83 min; MS (ESIpos): m/z = 1069 (M+H)⁺.

### Intermediat F25

### Trifluoressigsäure--N-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-L-alanyl-N⁵-carbamoyl-N-(4-{[(2,5-dioxo-2,5-dihydro-1H-pyrrol-1-yl)acetyl]amino}phenyl)-L-ornithinamid(1:1)

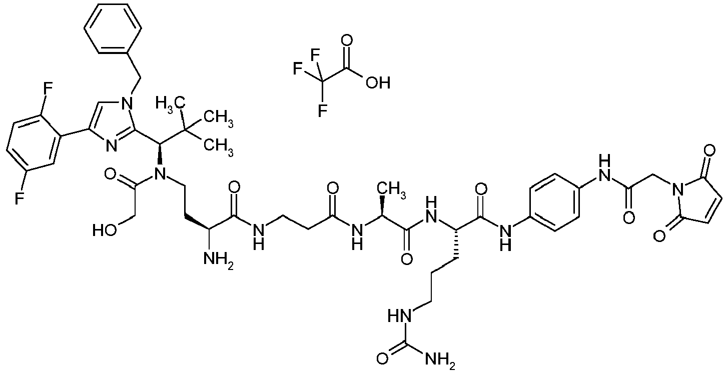

Dieses Intermediat wurde durch Kupplung von 9.6 mg (0.014 mmol) von Intermediat C8 mit10.8 mg (0.015 mmol) Intermediat L19 in Gegenwart von 6.4 mg (0.017 mmol) HATU und 72µl *N,N-*Diisopropylethylamin und anschließender Deblockierung mit TFA hergestellt. Es wurden 5 mg (31% d.Th. über 2 Stufen) der Titelverbindung erhalten.
HPLC (Methode 11): Rₜ = 1.8 min;
LC-MS (Methode 1): Rₜ = 0.85 min; MS (ESIpos): m/z = 1041 (M+H)⁺.

### Intermediat F26

### Trifluoressigsäure--N-{(15S,S,19R)-15-amino-19-[1-benzyl-4-(2,5-difluorphenyl)-1H-imidazol-2-yl]-18-glycoloyl-20,20-dimethyl-14-oxo-4,7,10-trioxa-13,18-diazahenicosan-1-oyl}-L-valyl-N⁵-carbamoyl-N-[4-(2,5-dioxo-2,5-dihydro-1H-pyrrol-1-yl)phenyl]-L-ornithinamid( 1: 1)

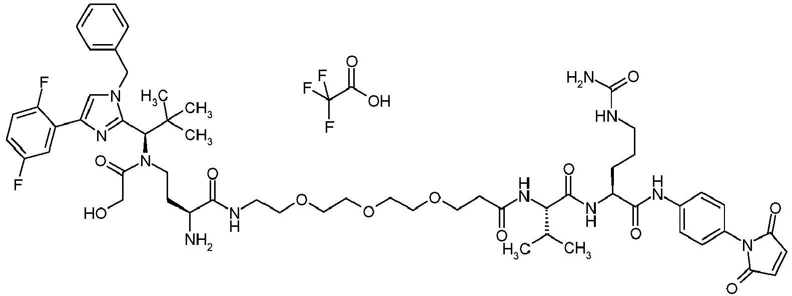

Dieses Intermediat wurde durch Kupplung von 16.4 mg (0.02 mmol) Intermediat C9 mit 11.2 mg (0.02 mmol) Intermediat L20 in Gegenwart von 8 mg (0.04 mmol) 1-(3-Dimethylaminopropyl)-3-ethylcarbodiimid-Hydrochlorid, 6 mg (0.04 mmol) 1-Hydroxy-1H-benzotriazol-Hydrat und 11 µl (0.06 mmol) *N*,*N*-Diisopropylethylamin und anschließender Deblockierung mit TFA hergestellt. Es wurden 10 mg (37% d.Th. über 2 Stufen) der Titelverbindung erhalten.
HPLC (Methode 11): Rₜ = 2.0 min;
LC-MS (Methode 1): Rₜ = 0.91 min; MS (ESIpos): m/z = 1144 (M+H)⁺.

### Intermediat F27

### Trifluoressigsäure--N-{(2S)-2-amino-4-[{(1R)-1-[1-benzyl-4-(2,5-difluorphenyl)-1H-imidazol-2-yl]-2,2-dimethylpropyl } (glycoloyl)amino]butanoyl }-beta-alanyl-L-valyl-N-[4-(2-{ [2-(2,5-dioxo-2,5-dihydro-1H-pyrrol-1-yl)ethyl]amino}-2-oxoethyl)phenyl]-L-lysinamid(2:1)

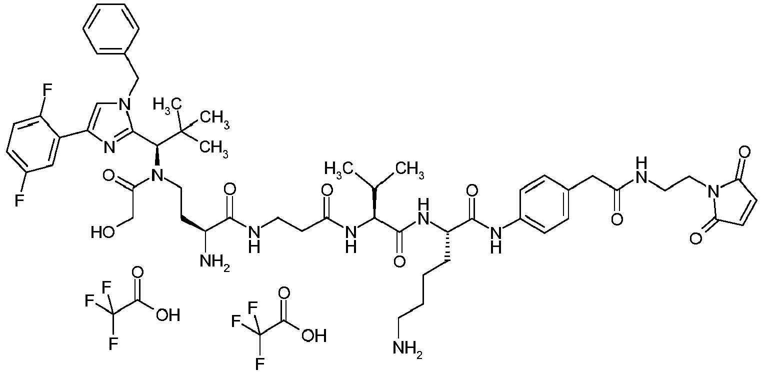

Dieses Intermediat wurde über 4 Stufen hergestellt:
Im ersten Schritt wurden 20 mg (0.028 mmol) von Intermediat C8 mit16.7 mg (0.031 mmol) Intermediat L21 in Gegenwart von 11 mg (0.057 mmol) 1-(3-Dimethylaminopropyl)-3-ethyl-carbodiimid-Hydrochlorid, 8.7 mg (0.057 mmol) 1-Hydroxy-1*H*-benzotriazol-Hydrat und 15 µl (0.085 mmol) *N*,*N*-Diisopropylethylamin in 5 mL DMF gekuppelt. Nach 4 Tagen Rühren bei RT wurde der Ansatz eingengt und das Produkt durch präparative HPLC gereinigt. Ausbeute: 18 mg (54.5% d.Th.).

18 mg (0.016 mmol) von dieser Zwischenstufe wurden in 4 mL Methanol gelöst, mit 194 µl einer 2M Lithiumhydroxidlösung versetzt und der Ansatz über Nacht bei RT gerührt. Dann wurden nochmals 116 µl Lithiumhydroxidlösung nachgesetzt und der Ansatz weitere 4 h bei RT gerührt. Anschließend wurde das Lösungsmittel im Vakuum entfernt, der Rückstand in Wasser aufgenommen und der Ansatz dann mit 5%-iger Citronensäure auf pH 5 gebracht. Es wurde zweimal mit Dichlormethan ausgeschüttelt und die organische Phase über Natriumsulfat getrocknet. Sie wurde dann filtriert, eingengt und der Rückstand im Hochvakuum getrocknet. Es verblieben 10.5 mg (58%) der Carboxylverbindung erhalten.

10.5 mg (0.009 mmol) von dieser Zwischenstufe wurde dann in 4 mL DMF aufgenommen und mit 3 mg (0.012 mmol) von kommerziell erhältlichem Trifluoressigsäure--1-(2-aminoethyl)-1H-pyrrol-2,5-dion(1:1) in Gegenwart von 3.5 mg (0.018 mmol) 1-(3-Dimethylaminopropyl)-3-ethyl-carbodiimid-Hydrochlorid, 2.8 mg (0.018 mmol) 1-Hydroxy-1*H*-benzotriazol-Hydrat und 6 µl *N,N-*Diisopropylethylamin gekuppelt. Nach Rühren über Nacht wurden nochmals die gleichen Mengen an Kupplungsreagenzien nachgesetzt und der Ansatz weitere 3 Tage bei RT gerührt. Anschließend wurde eingengt und das Produkt durch präparative HPLC gereinigt. Ausbeute: 6 mg (52% d. Th.). 6 mg (0.005 mmol) dieses Intermediats wurden dann in 3 mL DCM mit 1 mL Trifluoressigsäure entschützt. Nach Lyophilisation aus Acetonitril/Wasser wurden 6 mg (83% d.Th.) der Titelverbindung erhalten.
HPLC (Methode 11): Rₜ = 1.84 min;
LC-MS (Methode 4): Rₜ = 0.93 min; MS (ESIpos): m/z = 1068 (M+H)⁺.

### Intermediat F28

### N-{(2S)-2-Amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-4-({N-[6-(2,5-dioxo-2,5-dihydro-1H-pyrrol-1-yl)hexanoyl]-L-valyl-N⁵-carbamoyl-L-ornithyl}amino)-L-phenylalanin-trifluoressigsäure(1: 1)

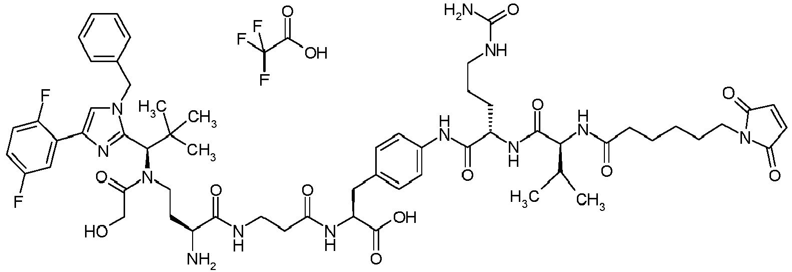

Dieses Intermediat wurde über 5 Stufen hergestellt:
Im ersten Schritt wurden 40 mg (0.058 mmol) von Intermediat C8 mit 46 mg (0.058 mmol) Intermediat L22 in Gegenwart von 44.3 mg (0.117 mmol) HATU und 30 µl *N,N-*Diisopropylethylamin gekuppelt. Nach 1 h Rühren bei RT wurde der Ansatz eingengt und das Produkt durch präparative HPLC gereinigt. Ausbeute: 53 mg (62.5% d.Th.).

Im nächsten Schritt wurde die Fmoc-Gruppe mit 0.6 mL Piperidin in 3 mL DMF abgespalten. Nach 1 h Rühren bei RT wurde der Ansatz eingengt und das Produkt durch präparative HPLC gereinigt. Ausbeute: 42 mg (82% d.Th.).

Zur Spaltung des Methylesters wurden 42 mg (0.033 mmol) von dieser Zwischenstufe in 2 mL THF und 1 mL Wasser gelöst, mit 330 µl einer 2M Lithiumhydroxidlösung versetzt und der Ansatz 1 h bei RT gerührt. Der Ansatz wurde dann mit TFA neutral gestellt, eingeengt und der Rückstand durch präparative HPLC gereinigt. Nach Trocknung im Hochvakuum verblieben 32 mg (78%) der Carboxylverbindung. 32 mg (0.026 mmol) dieses Intermediats wurden anschließend in 2.3 mL DMF mit 14.6 mg (0.047 mmol) von kommerziell erhältlichem 1-{6-[(2,5-Dioxopyrrolidin-1-yl)oxy]-6-oxohexyl}-1H-pyrrol-2,5-dion in Gegenwart von 18 µl *N*,*N*-Diisopropylethylamin gekuppelt. Nach 4-stündiger Behandlung im Ultraschallbad wurde der Ansatz eingengt und das Produkt durch präparative HPLC gereinigt. Ausbeute: 20.4 mg (60% d.Th.).

Im letzten Schritt wurden 20.4 mg (0.016 mmol) dieses Intermediats in DCM mit Trifluoressigsäure entschützt. Nach Lyophilisation aus Acetonitril/Wasser wurden 20 mg (85% d.Th.) der Titelverbindung erhalten.
HPLC (Methode 11): Rₜ = 1.9 min;
LC-MS (Methode 5): Rₜ = 2.84 min; MS (ESIpos): m/z = 1197 (M+H)⁺.

### Intermediat F29

### N-{(2S)-2-Amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl-4-({N-[6-(2,5-dioxo-2,5-dihydro-1H-pyrrol-1-yl)hexanoyl]-L-valyl-L-alanyl}amino)-L-phenylalanin-trifluoressigsäure(1:1)

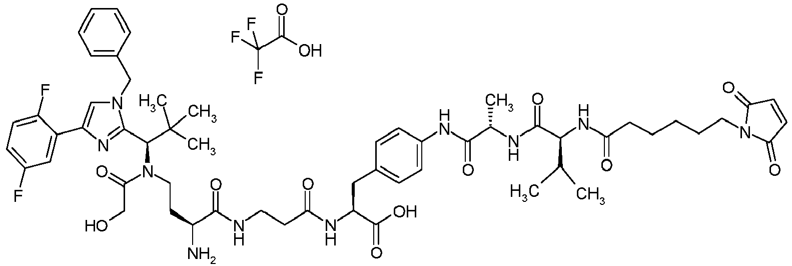

Die Herstellung der Titelverbindung erfolgte in Analogie zu Intermediat F28.
HPLC (Methode 11): Rₜ = 2.0 min;
LC-MS (Methode 1): Rₜ = 0.92 min; MS (ESIpos): m/z = 1111 (M+H)⁺.

### Intermediat F30

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-{2-[(3-{[2-(2,5-dioxo-2,5-dihydro-1H-pyrrol-1-yl)ethyl]amino}-3-oxopropyl)sulfinyl]ethyl}butanamid(1:1)

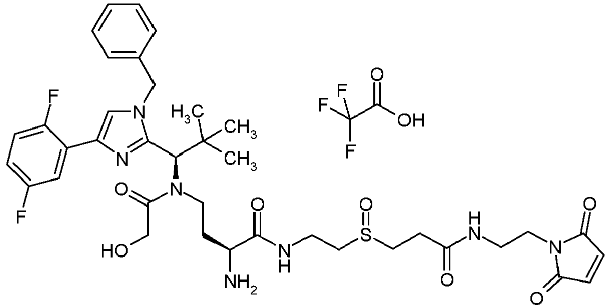

Dieses Intermediat wurde über 4 Stufen hergestellt:
Im ersten Schritt wurden 37.5 mg (0.055 mmol) von Intermediat C3 mit 15 mg (0.066 mmol) von kommerziell erhältlichem Methyl-3-[(2-aminoethyl)sulfanyl]propanoathydrochlorid (1:1) in DMF in Gegenwart von 25 mg (0.066 mmol) HATU und 29 µl *N*,*N*-Diisopropylethylamin gekuppelt. Nach 15 min Rühren bei RT wurden nochmals die Kupplungsreagenzien nachgesetzt. Der Ansatz wurde nochmals 15 min bei RT gerührt, anschließend eingengt und das Produkt durch präparative HPLC gereinigt. Ausbeute: 21 mg (48% d.Th.).

LC-MS (Methode 1): Rₜ = 1.41 min; MS (ESIpos): m/z = 802 (M+H)⁺.

Zur Spaltung des Methylesters wurden 21 mg (0.026 mmol) von dieser Zwischenstufe in 5 mL Methanol gelöst, mit 655 µl einer 2M Lithiumhydroxidlösung versetzt und der Ansatz über Nacht bei RT gerührt. Dabei ist teilweise Oxidation am Schwefel eingetreten. Der Ansatz wurde eingeengt und der Rückstand in Wasser aufgenommen und dann mit Essigsäure auf pH 3 gebracht. Es wurde zweimal mit 50 mL Ethylacetat extrahiert und anschließend die organische Phase über Magnesiumsulfat getrocknet, filtriert und eingeengt. Das nach Trocknung des Rückstandes im Hochvakuum erhaltene Gemisch wurde ohne weitere Reinigung in die nächste Stufe zur Kupplung mit 8.4 mg (0.033 mmol) von kommerziell erhältlichem Trifluoressigsäure--1-(2-aminoethyl)-1H-pyrrol-2,5-dion(1:1) in Gegenwart von 11.6 mg (0.031 mmol) HATU und 22 µl *N,N-*Diisopropylethylamin eingesetzt. Der Ansatz wurde 5 min bei RT gerührt und anschließend eingengt. Der Rückstand wurde in Ethylacetat aufgenommen und die Lösung mit 5%-iger Citronensäure und dann mit Wasser ausgeschüttelt. Anschließend wurde die organische Phase über Magnesiumsulfat getrocknet, filtriert und eingeengt. Das nach Trocknung des Rückstandes im Hochvakuum erhaltene Gemisch wurde ohne weitere Reinigung in die nächste Stufe eingesetzt. 22 mg von diesem Rohmaterial wurden dann in 2 mL DCM gelöst und mit 0.5 mL Trifluoressigsäure entschützt. Nach 10 min Rühren bei RT wurde der Ansatz eingeengt und der Rückstand durch präparative HPLC gereinigt. Nach Trocknung im Hochvakuum wurden 2.1 mg der Titelverbindung erhalten.
HPLC (Methode 11): Rₜ = 1.8 min;
LC-MS (Methode 1): Rₜ = 0.83 min; MS (ESIpos): m/z = 784 (M+H)⁺.

### Intermediat F31

### Trifluoressigsäure--N-[6-(2,5-dioxo-2,5-dihydro-1H-pyrrol-1-yl)hexanoyl]-L-valyl-N-(3-{[2-{(1R)-1-[(3-aminopropyl)(glycoloyl)amino]-2,2-dimethylpropyl}-4-(2,5-difluorphenyl)-1H-imidazol-1-yllmethyltphenyl)-L-alaninamid(1:1)

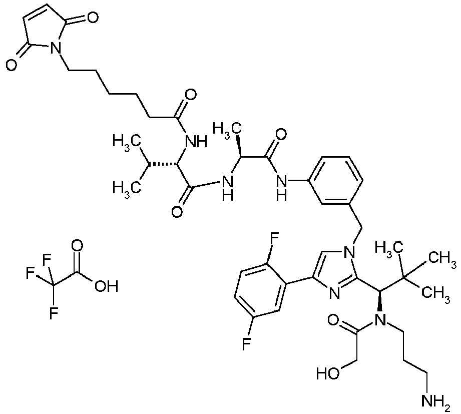

Dieses Intermediat wurde ausgehend von Intermediat C10 über 6 Stufen mit klassischen Methoden der Peptidchemie aufgebaut.

Im ersten Schritt wurden 42 mg (0.066 mmol) von Intermediat C10 mit 20.7 mg (0.066 mmol) von N-[(9H-Fluoren-9-ylmethoxy)carbonyl]-L-alanin in 5 mL DMF in Gegenwart von 100 mg (0.266 mmol) HATU und 46 µl *N,N*-Diisopropylethylamin gekuppelt. Der Ansatz wurde über Nacht bei RT gerührt und das Produkt durch präparative HPLC gereinigt. Es wurden 16 mg (27% d.Th.) der N-acylierten Verbindung sowie 9 mg (12% d.Th.) der N-, O- bis-acylierten Verbindung erhalten. Die Entschützung der N-acylierten Verbindung wurde in DMF mit Piperidin durchgeführt. Die bisacylierte Verbindung wurde in Ethanol sowohl mit Piperidin als auch mit einer wässrigen Lösung von Methylamin behandelt. In beiden Fällen entstand tert-Butyl-(3-{[(1R)-1-{1-[3-(L-alanylamino)benzyl]-4-(2,5-difluorphenyl)-1H-imidazol-2-yl}-2,2-dimethylpropyl](glycoloyl) amino}propyl)carbamat und nach Reinigung durch präparative HPLC wurden 13 mg in 95%iger Reinheit isoliert.

LC-MS (Methode 1): Rₜ = 0.95 min; MS (ESIpos): m/z = 657 (M+H)⁺.

13 mg (0.019 mmol) dieses Intermediats wurden in 2 mL DMF mit 9.1 mg (0.021 mmol) 2,5-Dioxopyrrolidin-1-yl-N-[(9H-fluoren-9-ylmethoxy)carbonyl]-L-valinat in Gegenwart von 7 µl *N*,*N*-Diisopropylethylamin gekuppelt. Nach 20h Rühren bei RT wurde eingeengt und der Rückstand durch präparative HPLC gereinigt. Nach Lyophilisation aus 1,4-1,4-Dioxan/Wasser verbleiben 10 mg (54% d.Th.).

Bei der dann folgenden Abspaltung der Fmoc-Schutzgruppe mit Piperidin in DMF wurden 9 mg (quant.) des partiell entschützten Intermediats erhalten.

9 mg (0.01 mmol) diese Intermediats wurden anschließend in 2 mL DMF mit 3.2 mg (0.01 mmol) von kommerziell erhältlichem 1-{6-[(2,5-Dioxopyrrolidin-1-yl)oxy]-6-oxohexyl}-1H-pyrrol-2,5-dion in Gegenwart von 5 µl *N*,*N*-Diisopropylethylamin gekuppelt. Nach Rühren über Nacht bei RT wurde der Ansatz eingengt und das Produkt durch präparative HPLC gereinigt. Nach Lyophilisation aus Acetonitril/Wasser und einigen Tropfen 1,4-Dioxanverblieben 3 mg (32% d.Th.), die im letzten Schritt in 2 mL DCM mit 0.5 mL Trifluoressigsäure entschützt wurden. Nach Lyophilisation aus Acetonitril/Wasser wurden 3.8 mg (quant.) der Titelverbindung erhalten.

HPLC (Methode 11): Rₜ = 1.9 min;

LC-MS (Methode 1): Rₜ = 0.89 min; MS (ESIpos): m/z = 849 (M+H)⁺.

### Intermediat F32

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

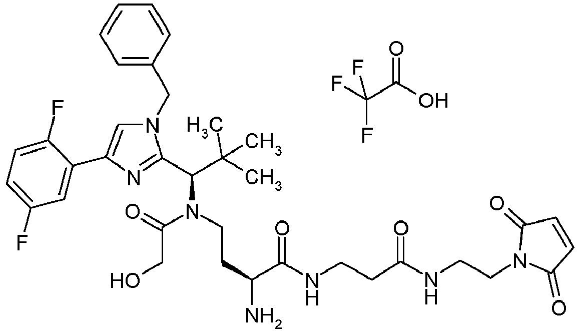

Dieses Intermediat wurde durch Kupplung von 15 mg (0.041 mmol) Intermediat C5 mit 16.8 mg (0.027 mmol) Intermediat L23 in Gegenwart von 10.5 mg (0.055 mmol) 1-(3-Dimethylaminopropyl)-3-ethylcarbodiimid-Hydrochlorid, 8.4 mg (0.055 mmol) 1-Hydroxy-1H-benzotriazol-Hydrat und 14 µl (0.08 mmol) *N*,*N*-Diisopropylethylamin und anschließender Deblockierung mit TFA hergestellt. Es wurden 3.4 mg (15% d.Th. über 2 Stufen) der Titelverbindung erhalten.
HPLC (Methode 11): Rₜ = 1.9 min;
LC-MS (Methode 1): Rₜ = 0.85 min; MS (ESIpos): m/z = 708 (M+H)⁺.

### Intermediat F33

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-{2-[(bromacetyl)amino]ethyl}butanamid(1:1)

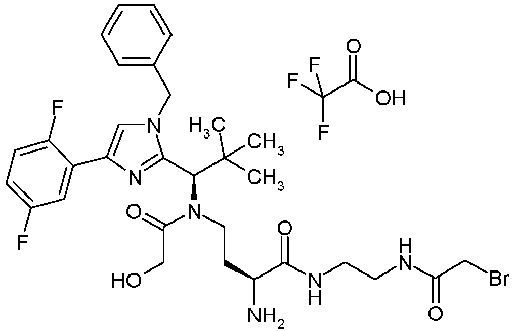

Die Synthese dieses Intermediats begann im ersten Schritt mit der Kupplung von 50 mg (0.075 mmol) Intermediat C3 mit 26.2 mg (0.082 mmol) 9H-Fluoren-9-ylmethyl-(2-aminoethyl)carbamathydrochlorid(1:1) in Gegenwart von 28.7 mg (0.15 mmol) 1-(3-Dimethylaminopropyl)-3-ethylcarbodiimid-Hydrochlorid, 22.9 mg (0.15 mmol) 1-Hydroxy-1H-benzotriazol-Hydrat und 39 µl *N*,*N*-Diisopropylethylamin. Nach 18 h Rühren bei RT wurde eingeengt und der Rückstand durch präparative HPLC gereinigt. 45 mg (65% d. Th.) dieser Zwischenstufe wurden erhalten.

LC-MS (Methode 1): Rₜ = 1.51 min; MS (ESIpos): m/z = 921 (M+H)⁺.

45 mg (0.049 mmol) von dieser Zwischenstufe wurden in 10 mL Ethanol aufgenommen und mit 176µ mL einer 40%-igen Methanaminlösung in Wasser versetzt. Der Ansatz wurde bei 50°C gerührt wobei nach 6h und nach 9h jeweils die gleiche Menge an Methanaminlösung zugegeben wurden. Nach weiteren 14 h Rühren bei 50°C wurden nochmals 700 µl der Mathenaminlösung zugegeben und nach weiteren 20h Rühren schließlich eingeengt. Der Rückstand wurde in DCM aufgenommen und mit Wasser gewaschen. Die organische Phase wurde eingeengt und der Rückstand durch präparative HPLC gereinigt. Nach Einengen der entsprechenden Fraktionen und Trocknung des Rückstandes im Hochvakuum wurden 32 mg (99% d.Th.) von tert-Butyl-{(2S)-1-[(2-aminoethyl)amino]-4-[{(1R)-1-[1-benzyl-4-(2,5-difluorphenyl)-1H-imidazol-2-yl]-2,2-dimethylpropyl } (glycoloyl)amino] -1 -oxobutan-2-yl} carbamat erhaltenen.

LC-MS (Methode 1): Rₜ = 0.95 min; MS (ESIpos): m/z = 657 (M+H)⁺.

8.3 mg (0.013 mmol) dieser Zwischenstufe wurden in 4 mL Dichlormethan gelöst und mit 3.3 mg (0.013 mmol) Bromessigsäureanhydrid sowie 2 µl *N,N*-Diisopropylethylamin versetzt. Nach 1 h Rühren bei RT wurde eingeengt und der Rückstand durch präparative HPLC gereinigt. Die entsprechenden Fraktionen wurden eingeengt und der Rückstand aus Acetonitril/Wasser lyophilisiert. Er wurde in 1 mL Dichlormethan aufgenommen und mit 0.5 mL Trifluoressigsäure entschützt. Nach Einengen und Lyophilisation aus Acetonitril/Wasser wurden 1.1 mg (9% d.Th. über 2 Stufen) der Titelverbindung erhalten.
HPLC (Methode 11): Rₜ = 1.9 min;
LC-MS (Methode 1): Rₜ = 0.88 min; MS (ESIpos): m/z = 677/679 (M+H)⁺.

### Intermediat F34

### Trifluoressigsäure--N-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-L-alanyl-N⁵-carbamoyl-N-[4-(2,5-dioxo-2,5-dihydro-1H-pyrrol-1-yl)phenyl]-L-omithinamid(1:1)

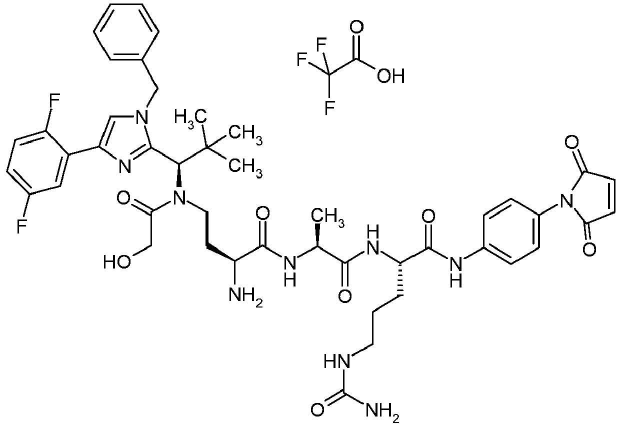

Dieses Intermediat wurde durch Kupplung von 14 mg (0.022 mmol) Intermediat C5 mit 12.7 mg (0.024 mmol) Intermediat L8 in Gegenwart von 9.9 mg (0.026 mmol) HATU und 19 µl *N,N-*Diisopropylethylamin gekuppelt. Der Ansatz wurde 30 min bei RT gerührt und das Produkt durch präparative HPLC gereinigt und anschließend aus Acetonitri/Wasser lyophilisiert.

Das erhaltene Intermediat wurde in 3 mL Dichlormethan aufgenommen und mit 1 mL Trifluoressigsäure deblockiert. Nach 30 min Rühren bei RT wurde der Ansatz eingeengt und der Rückstand aus Acetonitril/Wasser lyophilisiert. Es wurden 8.2 mg (36% d.Th. über 2 Stufen) der Titelverbindung erhalten.
HPLC (Methode 11): Rₜ = 1.8 min;
LC-MS (Methode 1): Rₜ = 0.87 min; MS (ESIpos): m/z = 913 (M+H)⁺.

### Intermediat F35

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-imidazol-2-yl]-2,2-dimethylpropyl}(glycoloyl)amino]propyl}-N5-carbamoyl-L-ornithinamid

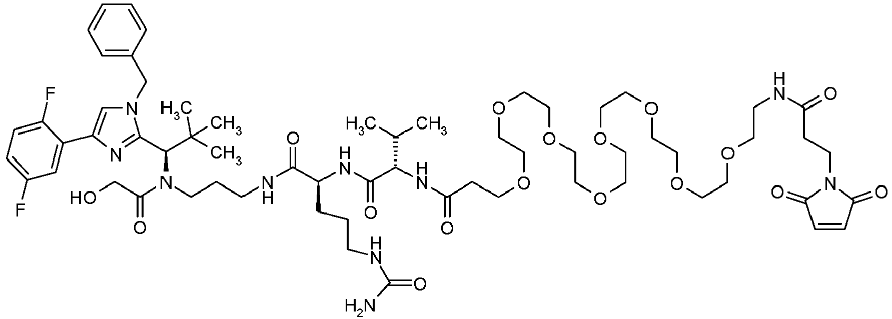

Unter Argon wurden bei 0 °C 31.8 mg (0.07 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40) in 4.0 mL DMF mit 57.3 mg (0.07 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N5-carbamoyl-L-omithin (Intermediat L38), 9.2 mg (0.07 mmol) HOAt und 32 mg (0.08 mmol) HATU versetzt. Dann wurden 23.5 µL (0.14 mmol) N,N-Diisopropylethylamin zugegeben und über Nacht bei RT nachgerührt. Die Reaktionsmischung wurde mit 7.7 µL HOAc versetzt und direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 33.4 mg (38 % d. Th.) der Titelverbindung.

LC-MS (Methode 1): Rₜ = 1.12 min; MS (ESIpos): m/z = 651 [M+2H]²⁺.

### Intermediat F36

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-imidazol-2-yl] -2,2-dimethylpropyl} (glycoloyl)amino]propyl} -L-alaninamid

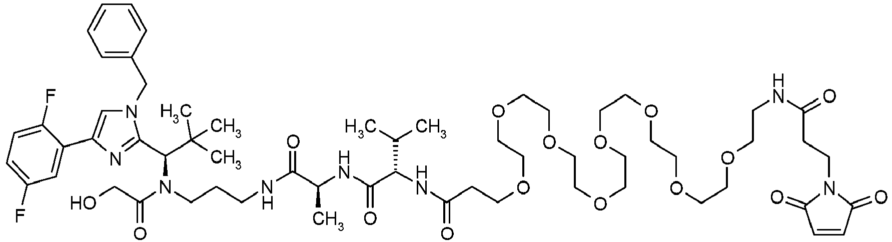

Die Synthese der Titelverbindung erfolgte in Analogie zur Herstellung von Intermediat F35. 15.4 mg (0.03 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40). 25.0 mg (0.03 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-L-alanin (Intermediat L25). Man erhielt 10.7 mg (27 % d. Th.) der Titelverbindung.

LC-MS (Methode 1): Rₜ = 1.13 min; MS (ESIpos): m/z = 1215 [M+H]⁺.

### Intermediat F37

### Trifluoressigsäure--N-(4-{[{(1R)-1-[1-Benzyl-4-(2,5-difluorphenyl)-1H-imidazol-2-yl]-2,2-dimethylpropyl}(glycoloyl)amino]methyl}pyrrolidin-3-yl)-31-(2,5-dioxo-2,5-dihydro-1H-pyrrol-1-yl)-29-oxo-4,7,10,13,16,19,22,25-octaoxa-28-azahentriacontan-1-amid(1:1) Mischung aus Diastereomeren.

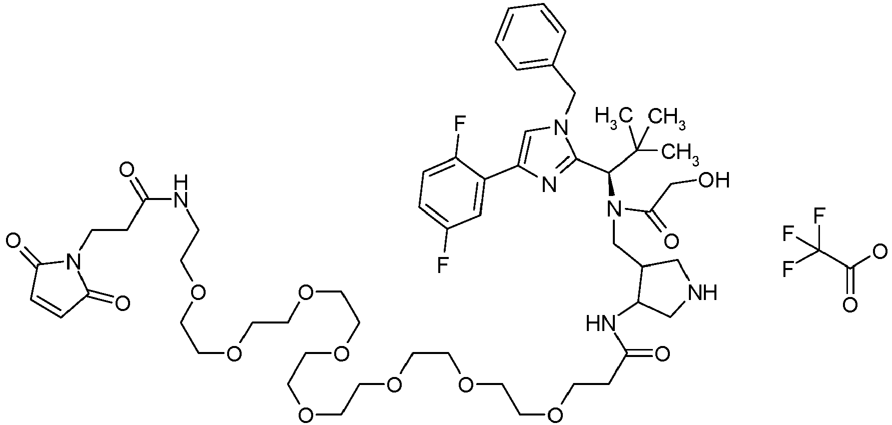

Die Verbindung tert-Butyl-3-{[{(1R)-1-[1-benzyl-4-(2,5-difluorphenyl)-1H-imidazol-2-yl]-2,2-dimethylpropyl} (glycoloyl)amino]methyl}-4-{ [31-(2,5-dioxo-2,5-dihydro-1H-pyrrol-1-yl)-29-oxo-4,7,10,13,16,19,22,25-octaoxa-28-azahentriacontan-1-oyl]amino}pyrrolidin-1-carboxylat wurde in Analogie zur Synthese von Intermediat C21 hergestellt.

8.0 (0.01 mmol) bzw. 13.0 mg (0.02 mmol) tert-Butyl-3-amino-4-{[{(1R)-1-[1-benzyl-4-(2,5-difluorphenyl)-1H-imidazol-2-yl]-2,2-dimethylpropyl}(glycoloyl)amino]methyl}pyrrolidin-1-carboxylat (Intermediat C23).

9.0 mg (0.01 mmol) bzw. 14.7 mg (0.02 mmol) 3-(2,5-Dioxo-2,5-dihydro-1H-pyrrol-1-yl)-N-{27-[(2,5 -dioxopyrrolidin-1 -yl)oxy] -27-oxo-3,6,9,12,15,18,21,24-octaoxaheptacos-1 -yl} propanamid.

Ausbeute (beide Ansätze vereinigt):
10.5 mg (42 %) Diastereomer 1
11.6 mg (46 %) Diastereomer 2

Die Titelverbindung wurde in Analogie zur Synthese von Intermediat F38 hergestellt.

10.5 mg (0.01 mmol) tert-Butyl-3-{[{(1R)-1-[1-benzyl-4-(2,5-difluorphenyl)-1H-imidazol-2-yl]-2,2-dimethylpropyl}(glycoloyl)amino]methyl}-4-{[31-(2,5-dioxo-2,5-dihydro-1H-pyrrol-1-yl)-29-oxo-4,7,10,13,16,19,22,25-octaoxa-28-azahentriacontan-1-oyl]amino}pyrrolidin-1-carboxylat (Diastereomer 1)
60.6 mg (0.54 mmol) TFA.
Man erhielt 7.4 mg (70 % d. Th.) der Titelverbindung.
LC-MS (Methode 1): Rₜ = 1.09 min; MS (ESIpos): m/z = 1086 [M+H]⁺.

### Intermediat F38

### Trifluoressigsäure--N-(3-Aminopropyl)-N-{(1R)-1-[1-benzyl-4-(2,5-difluorphenyl)-1H-imidazol-2-yl]-2,2-dimethylpropyl}-37-(2,5-dioxo-2,5-dihydro-1H-pyrrol-1-yl)-7,35-dioxo-10,13,16,19,22,25,28,31-octaoxa-3-thia-6,34-diazaheptatriacontan-1-amid(1:1)

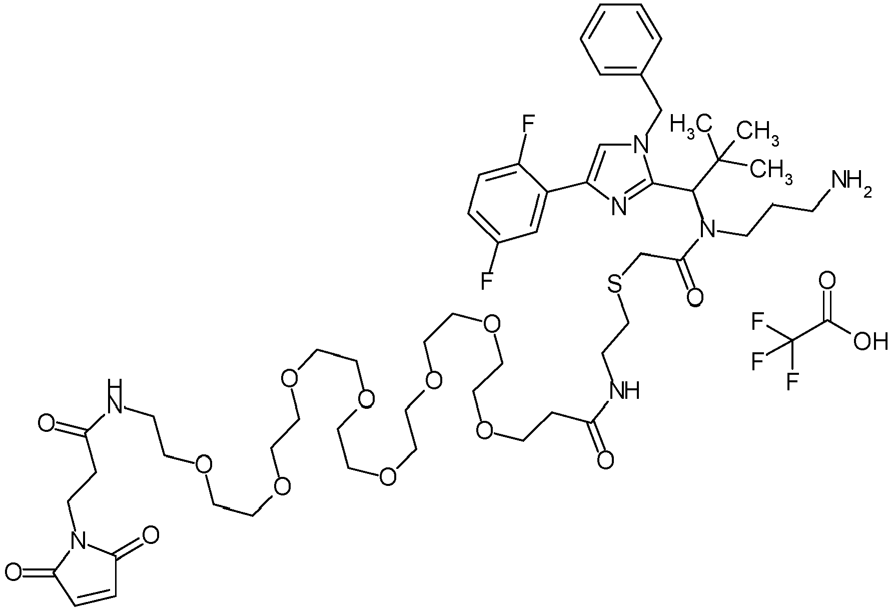

24.8 mg (0.02 mmol) tert-Butyl-[38-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-3,31,37-trioxo-7,10,13,16,19,22,25,28-octaoxa-35-thia-4,32,38-triazahentetracontan-41-yl]carbamat (Intermediat C21) wurden in 1.0 mL Dichlormethan vorgelegt und mit 85.8 mg (0.75 mmol) TFA versetzt. Es wurde 16 h bei RT gerührt. Das Lösemittel wurde im Vakuum verdampft und der Rückstand wurde direkt über die präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 23.0 mg (95 % d. Th.) der Titelverbindung.

LC-MS (Methode 1): Rₜ = 0.96 min; MS (ESIpos): m/z = 1104 [M+H]⁺.

### Intermediat F39

### N-[19-(2,5-Dioxo-2,5-dihydro-1H-pyrrol-1-yl)-17-oxo-4,7,10,13-tetraoxa-16-azanonadecan-1-oyl]-L-valyl-N-{3-[{(1R)-1-[1-benzyl-4-(2,5-difluorphenyl)-1H-imidazol-2-yl]-2,2-dimethylpropyl} (glycoloyl)amino]propyl} -L-alaninamid

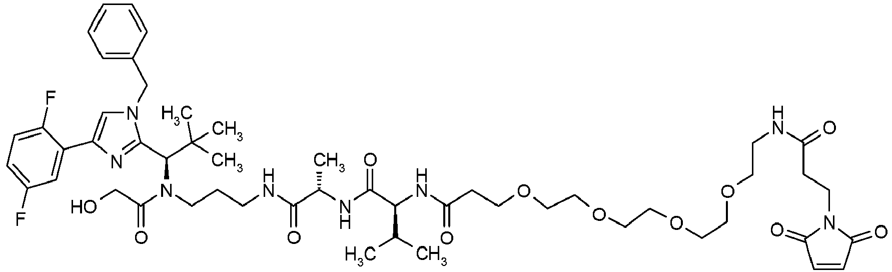

Die Titelverbindung wurde in Analogie zur Synthese von Intermediat F35 hergestellt.
56.1 mg (0.10 mmol) N-[19-(2,5-Dioxo-2,5-dihydro-1H-pyrrol-1-yl)-17-oxo-4,7,10,13-tetraoxa-16-azanonadecan-1-oyl]-L-valyl-L-alanin (Intermediat L44).
45.0 mg (0.10 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40).
Man erhielt 20.9 mg (21 % d. Th.) der Titelverbindung.

LC-MS (Methode 1): Rₜ = 1.16 min; MS (ESIpos): m/z = 1040 [M+H]⁺.

### Intermediat F40

### Trifluoressigsäure--N-[(4-{[{(1R)-1-[1-Benzyl-4-(2,5-difluorphenyl)-1H-imidazol-2-yl]-2,2-dimethylpropyl}(glycoloyl)amino]methyl}pyrrolidin-3-yl)methyl]-31-(2,5-dioxo-2,5-dihydro-1H-pyrrol-1-yl)-29-oxo-4,7,10,13,16,19,22,25-octaoxa-28-azahentriacontan-1-amid(1:1)

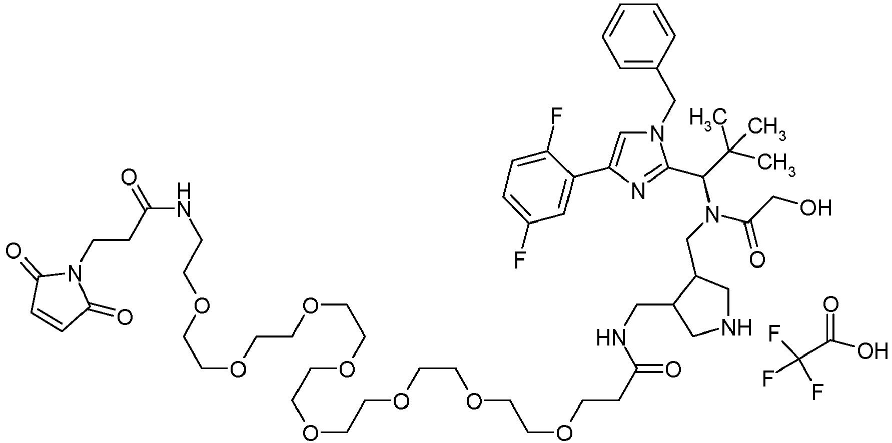

tert-Butyl-3-{[{(1R)-1-[1-benzyl-4-(2,5-difluorphenyl)-1H-imidazol-2-yl]-2,2-dimethylpropyl(glycoloyl)amino]methyl}-4-[33-(2,5-dioxo-2,5-dihydro-1H-pyrrol-1-yl)-3,31-dioxo-6,9,12,15,18,21,24,27-octaoxa-2,30-diazatritriacont-1-yl]pyrrolidin-1-carboxylat wurde in Analogie zur Synthese von Intermediat C21 hergestellt. 25.0 mg (0.04 mmol) Trifluoressigsäure--tert-Butyl-3-(aminomethyl)-4-{[{(1R)-1-[1-benzyl-4-(2,5 -difluorphenyl)- 1H-imidazol-2-yl] -2,2-dimethylpropyl} (glycoloyl)amino]methyl }pyrrolidin-1 - carboxylat(1:1)(Intermediat C24). 27.6 mg (0.04 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid. Ausbeute: 20.6 mg (39 % d. Th.) LC-MS (Methode 1): Rₜ = 1.23 min; MS (ESIpos): m/z = 1200 [M+H]⁺.

Die Titelverbindung wurde in Analogie zur Synthese von Intermediat F37 hergestellt. 26.1 mg (0.02 mmol) (tert-Butyl-3-{[{(1R)-1-[1-benzyl-4-(2,5-difluorphenyl)-1H-imidazol-2-yl]-2,2-dimethylpropyl}(glycoloyl)amino]methyl}-4-[33-(2,5-dioxo-2,5-dihydro-1H-pyrrol-1-yl)-3,31-dioxo-6,9,12,15,18,21,24,27-octaoxa-2,30-diazatritriacont-1-yl]pyrrolidin-1-carboxylat. 90.6 mg (0.80 mmol) TFA.

Man erhielt von der Titelverbindung 22.9 mg (95 % d. Th.).

LC-MS (Methode 1): Rₜ = 0.91 und 0.92 min; MS (ESIpos): m/z = 1100 [M+H]⁺.

### Intermediat F41

### Trifluoressigsäure--N-(3-Aminopropyl)-N-{(1R)-1-[1-benzyl-4-(2,5-difluorphenyl)-1H-imidazol-2-yl]-2,2-dimethylpropyl}-37-(2,5-dioxo-2,5-dihydro-1H-pyrrol-1-yl)-7,35-dioxo-10,13,16,19,22,25,28,31-octaoxa-3-thia-6,34-diazaheptatriacontan-1-amid-3-oxid(1:1)

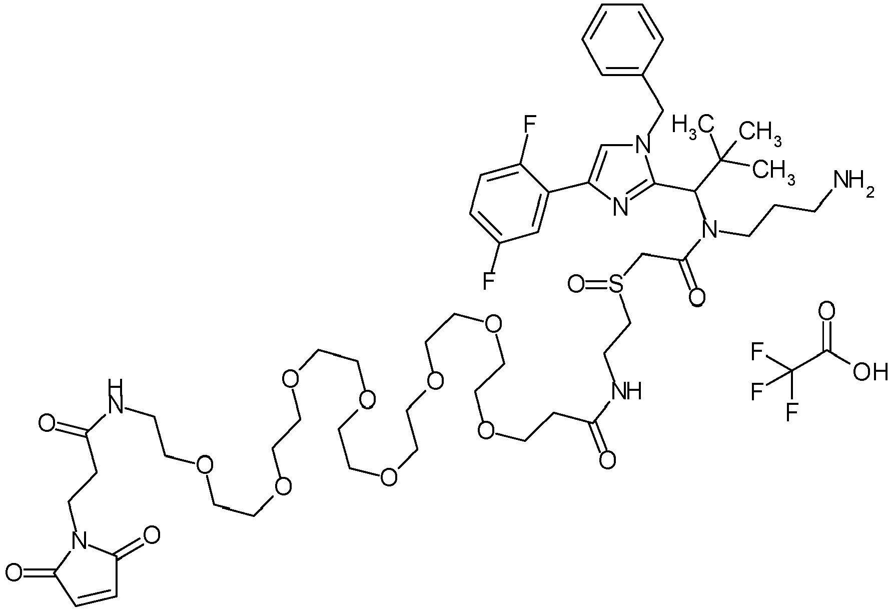

Die Titelverbindung wurde in Analogie zu Intermediat F38 aus 15.5 mg (0.01 mmol) Intermediat C22 hergestellt. Man erhielt 4.0 mg (27 % d. Th.) der Titelverbindung.

LC-MS (Methode 1): Rₜ = 0.90 min; MS (ESIpos): m/z = 1120 [M+H]⁺.

### Intermediat F42

### Trifluoressigsäure--4-{[{(1R)-1-[1-Benzyl-4-(2,5-difluorphenyl)-1H-imidazol-2-yl]-2,2-dimethylpropyl } (glycoloyl)amino]methyl }-N-[2-(2,5-dioxo-2,5-dihydro-1H-pyrrol-1-yl)ethyl]pyrrolidin-3-carboxamid(1: 1)

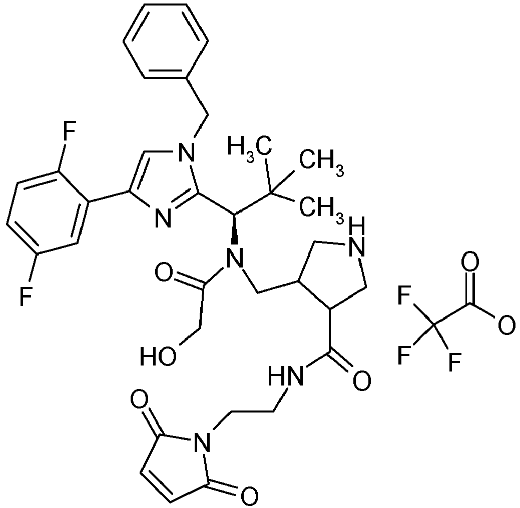

40.5 mg (0.06 mmol) 4-{[{(1R)-1-[1-Benzyl-4-(2,5-difluorphenyl)-1H-imidazol-2-yl]-2,2-dimethylpropyl} (glycoloyl)amino]methyl}-1-(tert-butoxycarbonyl)pyrrolidin-3 -carbonsäure (Intermediat C25) und 14.5 mg (0.08 mmol) 1-(2-Aminoethyl)-1H-pyrrol-2,5-dionhydro-chlorid(1:1) wurden in 1.0 mL Acetonitril vorgelegt und mit 64.4 mg (0.51 mmol) N,N-Diisopropylethylamin und 50.0 mg (0.08 mmol) T3P versetzt und 16 h bei RT gerührt. Es wurden nochmals die gleichen Mengen an 1-(2-Aminoethyl)-1H-pyrrol-2,5-dionhydrochlorid(1:1), N,N-Diisopropylethylamin und T3P zugegeben und weitere 4 h bei RT gerührt. Die Reaktionsmischung wurde direkt über die präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 7.2 mg (15 % d. Th.) der Verbindung tert-Butyl-3-{[{(1R)-1-[1-benzyl-4-(2,5-difluorphenyl)-1H-imidazol-2-yl]-2,2-dimethylpropyl}(glycoloyl)amino]methyl}-4-{[2-(2,5-dioxo-2,5-dihydro-1H-pyrrol-1-yl)ethyl]carbamoyl}pyrrolidin-1-carboxylat.

LC-MS (Methode 1): Rₜ = 1.30 min; MS (ESIpos): m/z = 763 [M+H]⁺.

7.2 mg (0.01 mmol) tert-Butyl-3-{[{(1R)-1-[1-benzyl-4-(2,5-difluorphenyl)-1H-imidazol-2-yl]-2,2-dimethylpropyl} (glycoloyl)amino]methyl}-4-{[2-(2,5-dioxo-2,5-dihydro-1H-pyrrol-1-yl)ethyl]carbamoyl}pyrrolidin-1-carboxylat wurden in 1.0 mL Dichlormethan vorgelegt und mit 43.0 mg (0.38 mmol) TFA versetzt. Die Reaktionsmischung wurde 16 h bei RT gerührt. Das Lösemittel wurde im Vakuum verdampft und der Rückstand über die präp. RP-HPLC gereinigt (Säule: Reprosil 125x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 4.5 mg (50 % d. Th.) der Titelverbindung.

LC-MS (Methode 1): Rₜ = 0.92 min; MS (ESIpos): m/z = 663 [M+H]⁺.

### Intermediat F43

### N-[19-(2,5-Dioxo-2,5-dihydro-1H-pyrrol-1-yl)-17-oxo-4,7,10,13-tetraoxa-16-azanonadecan-1-oyl]-L-valyl-N-{3-[{(1R)-1-[1-benzyl-4-(2,5-difluorphBeiDirenyl)-1H-imidazol-2-yl]-2,2-dimethylpropyl} (glycoloyl)amino]propyl} -N5 -carbamoyl-L-ornithinamid

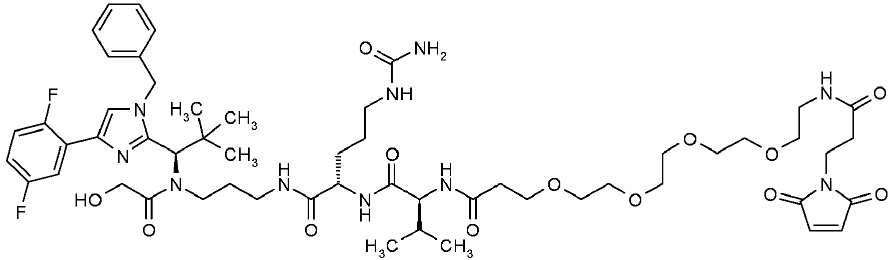

22.4 mg (0.03 mmol) N-[19-(2,5-Dioxo-2,5-dihydro-1H-pyrrol-1-yl)-17-oxo-4,7,10,13-tetraoxa-16-azanonadecan-1-oyl]-L-valyl-NS-carbamoyl-L-ornithin (Intermediat L42) wurden in 2.0 mL DMF gelöst und mit 4.5 mg (0.03 mmol) HOAt, 15.8 mg (0.04 mmol) HATU und 15.7 mg (0.03 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40) versetzt. 8.6 mg (0.07 mmol) N,N-Diisopropylethylamin wurden hinzugefügt und die Reaktionsmischung rührte über Nacht bei RT. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 16.7 mg (45 % d. Th.) der Titelverbindung.

LC-MS (Methode 4): Rₜ = 1.34 min; MS (ESIpos): m/z = 1125 [M+H]⁺.

### Intermediat F44

### Trifluoressigsäure--N-[(4-{ [{(1R)-1-[1-benzyl-4-(2,5-difluorphenyl)-1H-imidazol-2-yl]-2,2-dimethylpropyl} (glycoloyl)amino]methyl }pyrrolidin-3 -yl)methyl] -19-(2,5 -dioxo-2,5 -dihydro- 1H-pyrrol-1-yl)-17-oxo-4,7,10,13-tetraoxa-16-azanonadecan-1-amid(1:1)

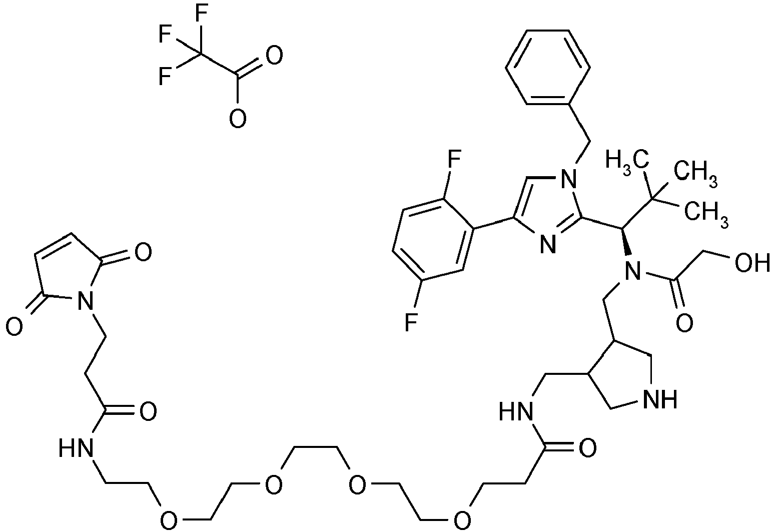

Die Titelverbindung wurde in Analogie zur Synthese von Intermediat F40 hergestellt. 25.0 mg (0.04 mmol) Trifluoressigsäure--tert-Butyl-3-(aminomethyl)-4-{[{(1R)-1-[1-benzyl-4-(2,5 -difluorphenyl)- 1H-imidazol-2-yl] -2,2-dimethylpropyl} (glycoloyl)amino]methyl }pyrrolidin-1 - carboxylat(1: 1) (Intermediat C24).

20.5 mg (0.04 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid.

Man erhielt 21.8 mg (48 % d. Th.) der Verbindung tert-Butyl-3-{[{(1R)-1-[1-benzyl-4-(2,5-difluorphenyl)-1H-imidazol-2-yl]-2,2-dimethylpropyl}(glycoloyl)amino]methyl}-4-[21-(2,5-dioxo-2,5-dihydro-1H-pyrrol-1-yl)-3,19-dioxo-6,9,12,15-tetraoxa-2,18-diazahenicos-1-yl]pyrrolidin-1-carboxylat.

LC-MS (Methode 1): Rₜ = 1.22 min; MS (ESIpos): m/z = 1025 [M+H]⁺.

21.0 mg (0.02 mmol) tert-Butyl-3-{[{(1R)-1-[1-benzyl-4-(2,5-difluorphenyl)-1H-imidazol-2-yl]-2,2-dimethylpropyl}(glycoloyl)amino]methyl}-4-[21-(2,5-dioxo-2,5-dihydro-1H-pyrrol-1-yl)-3,19-dioxo-6,9,12,15-tetraoxa-2,18-diazahenicos-1-yl]pyrrolidin-1-carboxylat.
168.3 mg (1.48 mmol) TFA.
Man erhielt 17.3 mg (90 % d. Th.) der Titelverbindung.
LC-MS (Methode 1): Rₜ = 0.92 und 0.94 min; MS (ESIpos): m/z = 924 [M+H]⁺.

### Intermediat F45

### Trifluoressigsäure--N-[19-(2,5-Dioxo-2,5-dihydro-1H-pyrrol-1-yl)-17-oxo-4,7,10,13-tetraoxa-16-azanonadecan-1-oyl]-L-valyl-N-{3-[{(1R)-1-[1-benzyl-4-(2,5-difluorphenyl)-1H-imidazol-2-yl]-2,2-dimethylpropyl}(glycoloyl)amino]propyl}-L-lysinamid (1:1)

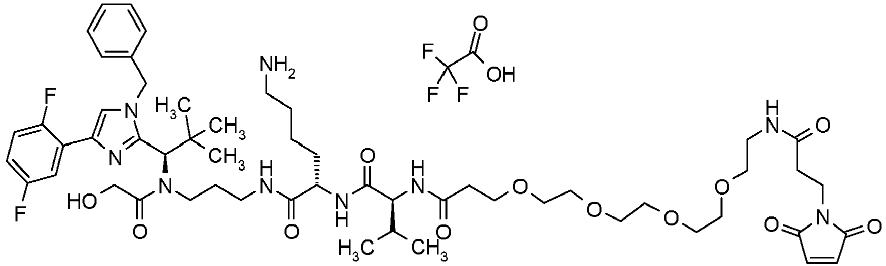

Die Synthese erfolgte in Analogie zur Synthese von Intermediat F46.

22.9 mg (0.05 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40).

36.2 mg (0.05 mmol) N-[19-(2,5-Dioxo-2,5-dihydro-1H-pyrrol-1-yl)-17-oxo-4,7,10,13-tetraoxa-16-azanonadecan-1-oyl]-L-valyl-N6-(tert-butoxycarbonyl)-L-lysin (Intermediat L41).

Man erhielt 19.8 mg (34 %) der Verbindung N-[19-(2,5-Dioxo-2,5-dihydro-1H-pyrrol-1-yl)-17-oxo-4,7,10,13-tetraoxa-16-azanonadecan-1-oyl]-L-valyl-N-{3-[{(1R)-1-[1-benzyl-4-(2,5-difluorphenyl)-1H-imidazol-2-yl]-2,2-dimethylpropyl}(glycoloyl)amino]propyl}-N6-(tert-butoxycarbonyl)- L-lysinamid.

LC-MS (Methode 1): Rₜ = 1.20 min; MS (ESIpos): m/z = 1196 [M+H]⁺.

17.0 mg (0.01 mmol) N-[19-(2,5-Dioxo-2,5-dihydro-1H-pyrrol-1-yl)-17-oxo-4,7,10,13-tetraoxa-16-azanonadecan-1-oyl]-L-valyl-N-{3-[{(1R)-1-[1-benzyl-4-(2,5-difluorphenyl)-1H-imidazol-2-yl]-2,2-dimethylpropyl } (glycoloyl)amino]propyl }-N6-(tert-butoxycarbonyl)-L-lysinamid.

Man erhielt 13.6 mg (79% d. Th.) der Titelverbindung.

LC-MS (Methode 1): Rₜ = 0.94 min; MS (ESIpos): m/z = 1096 [M+H]⁺.

### Intermediat F46

### Trifluoressigsäure--N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-imidazol-2-yl]-2,2-dimethylpropyl}(glycoloyl)amino]propyl}-L-lysinamid (1:1)

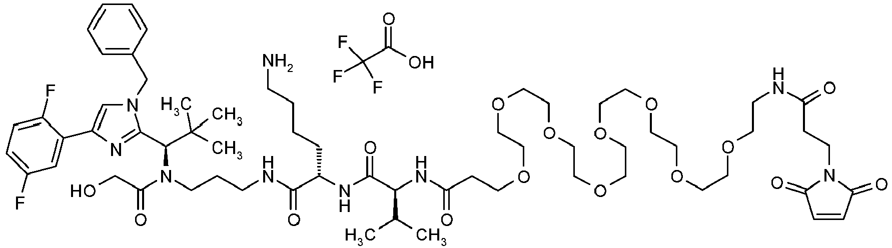

Unter Argon wurden bei 0 °C 25.1 mg (0.05 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40) in 2.0 mL DMF mit 49.0 mg (0.05 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N6-(tert-butoxycarbonyl)-L-lysin (Intermediat L40), 7.3 mg (0.05 mmol) HOAt und 25.3 mg (0.07 mmol) HATU versetzt. Dann wurden 18.6 µL (0.11 mmol) N,N-Diisopropylethylamin zugegeben und über Nacht bei RT nachgerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 29.2 mg (37 % d. Th.) der Verbindung N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-imidazol-2-yl]-2,2-dimethylpropyl}(glycoloyl)amino]propyl}-N6-(tert-butoxycarbonyl)-L-lysinamid.

LC-MS (Methode 4): Rₜ = 1.51 min; MS (ESIpos): m/z = 1372 [M+H]⁺.

25.2 mg (0.02 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-imidazol-2-yl]-2,2-dimethylpropyl}(glycoloyl)amino]propyl}-N6-(tert-butoxycarbonyl)-L-lysinamid wurden in 3.0 mL Dichlormethan vorgelegt und mit 83.7 mg (0.73 mmol) TFA versetzt. Die Reaktionslösung wurde 48 h bei RT gerührt. Das Lösemittel wurde im Vakuum verdampft und über die präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 24.5 mg (96 % d. Th.) der Titelverbindung. LC-MS (Methode 1): Rₜ = 0.95 min; MS (ESIpos): m/z = 1272 [M+H]⁺.

### Intermediat F47__

### N-[67-(2,5-Dioxo-2,5-dihydro-1H-pyrrol-1-yl)-65-oxo-4,7,10,13,16,19,22,25,28,31,34,37,40,43,46,49,52,55,58,61-icosaoxa-64-azaheptahexacontan-1-oyl]-L-valyl-N-{3-[{(1R)-1-[1-benzyl-4-(2,5-difluorphenyl)-1H-imidazol-2-yl]-2,2-dimethylpropyl} (glycoloyl)amino]propyl} -N5 -carbamoyl-L-ornithinamid

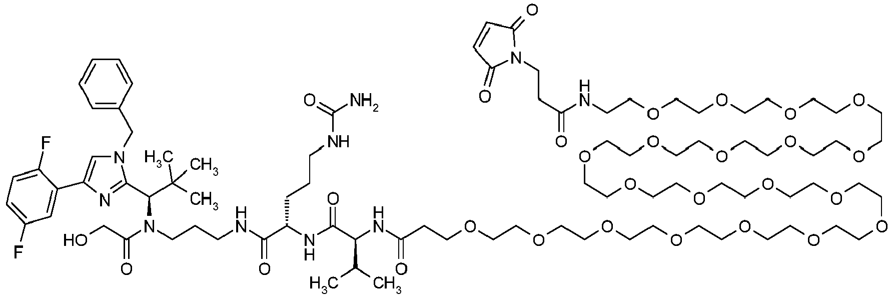

15.2 mg (0.01 mmol) N-[67-(2,5-Dioxo-2,5-dihydro-1H-pyrrol-1-yl)-65-oxo-4,7,10,13,16,19,22,25,28,31,34,37,40,43,46,49,52,55,58,61-icosaoxa-64-azaheptahexacontan-1-oyl]-L-valyl-N5-carbamoyl-L-omithin (Intermediat L43) wurden in 1.0 mL DMF gelöst und mit 1.5 mg (0.01 mmol) HOAt, 5.2 mg (0.01 mmol) HATU und 5.2 mg (0.01 mmol) N-(3-Aminopropyl)-N-{(1R)-1-[1-benzyl-4-(2,5-difluorphenyl)-1H-imidazol-2-yl]-2,2-dimethylpropyl}-2-hydroxyacetamid (Intermediat C40) versetzt. 2.9 mg (0.02 mmol) N,N-Diisopropylethylamin wurden hinzugefügt und die Reaktionsmischung rührte über Nacht bei RT. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 6.8 mg (33 % d. Th.) der Titelverbindung.

LC-MS (Methode 4): Rₜ = 1.37 min; MS (ESIpos): m/z = 1831 [M+H]⁺.

### Intermediat F48

### Trifluoressigsäure--N1-(3-Aminopropyl)-N1-{(1R)-1-[1-benzyl-4-(2,5-difluorphenyl)-1H-imidazol-2-yl]-2,2-dimethylpropyl}-N18-[2-(2,5-dioxo-2,5-dihydro-1H-pyrrol-1-yl)ethyl]-6,9,12,15-tetraoxa-3-thiaoctadecan-1,18-diamid(1:1)

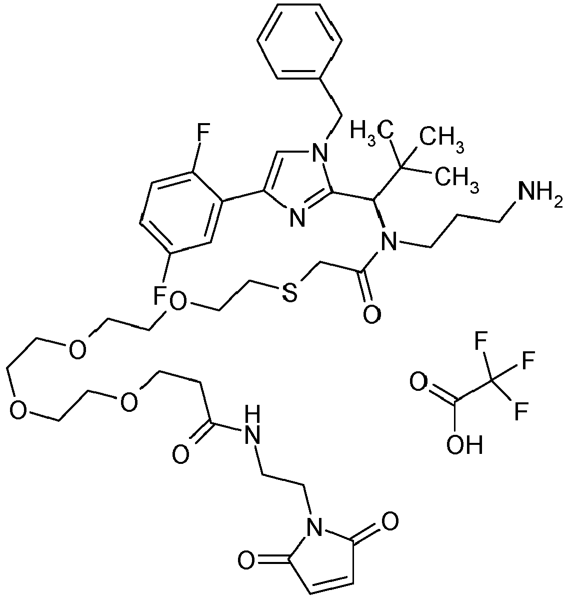

16.1 mg (0.02 mmol) tert-Butyl-[22-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-4,21-dioxo-7,10,13,16-tetraoxa-19-thia-3,22-diazapentacosan-25-yl]carbamat (Intermediat C18) wurden in 1.5 mL Dichlormethan vorgelegt und mit 26 µL (0.34 mmol) TFA versetzt. Es wurde über Nacht bei RT gerührt und dann nochmals 26 µL (0.34 mmol) TFA hinzugefügt. Es wurde nochmals über Nacht bei RT gerührt. Das Lösemittel wurde im Vakuum verdampft und der Rückstand mittels präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand in Wasser aufgenommen und lyophilisiert. Man erhielt 10.8 mg (66 % d. Th.) der Titelverbindung.

LC-MS (Methode 1): Rₜ = 0.98 min; MS (ESIpos): m/z = 857 [M+H]⁺.

### Intermediat F49

### (25S)-25-Amino-22-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-4,21-dioxo-7,10,13,16-tetraoxa-19-thia-3,22-diazahexacosan-26-säure-trifluoressigsäure( 1:1)

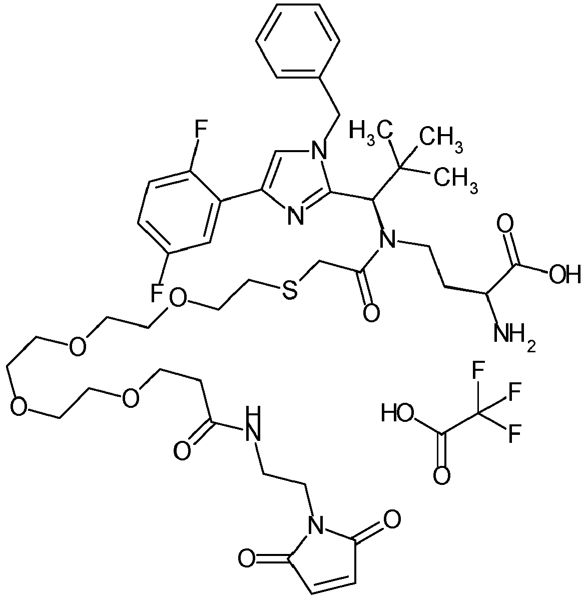

Die Synthese der Verbindung tert-Butyl-(2S)-4-[{(1R)-1-[1-benzyl-4-(2,5-difluorphenyl)-1H-imidazol-2-yl]-2,2-dimethylpropyl}(chloracetyl)amino]-2-[(tert-butoxycarbonyl)amino]butanoat erfolgte in Analogie zur Synthese von Intermediat C16.
50.0 mg (0.08 mmol) Intermediat C2
20.3 mg (0.18 mmol) Chloracetylchlorid
19.0 mg (0.19 mmol) Triethylamin

Man erhielt 43.1 mg (77 % d. Th.) der Verbindung tert-Butyl-(2S)-4-[{(1R)-1-[1-benzyl-4-(2,5-difluorphenyl)-1H-imidazol-2-yl]-2,2-dimethylpropyl}(chloracetyl)amino]-2-[(tert-butoxycarbonyl)amino]butanoat.

LC-MS (Methode 1): Rₜ = 1.55 min; MS (ESIpos): m/z = 689 [M+H]⁺.

Die Synthese der Verbindung (6S)-9-{(1R)-1-[1-Benzyl-4-(2,5-difluorphenyl)-1H-imidazol-2-yl]-2,2-dimethylpropyl}-6-(tert-butoxycarbonyl)-2,2-dimethyl-4,10-dioxo-3,15,18,21,24-pentaoxa-12-thia-5,9-diazaheptacosan-27-säure erfolgte in Analogie zur Synthese von Intermediat C17.

38.8 mg (0.06 mmol) tert-Butyl-(2S)-4-[{(1R)-1-[1-benzyl-4-(2,5-difluorphenyl)-1H-imidazol-2-yl]-2,2-dimethylpropyl}(chloracetyl)amino]-2-[(tert-butoxycarbonyl)amino]butanoat.

19.1 mg (0.07 mmol) 1-Sulfanyl-3,6,9,12-tetraoxapentadecan-15-säure

45.9 mg (0.14 mmol) Cäsiumcarbonat

Man erhielt 40.7 mg (77 % d. Th.) der Verbindung (6S)-9-{(1R)-1-[1-Benzyl-4-(2,5-difluorphenyl)-1H-imidazol-2-yl]-2,2-dimethylpropyl }-6-(tert-butoxycarbonyl)-2,2-dimethyl-4,10-dioxo-3,15,18,21,24-pentaoxa-12-thia-5,9-diazaheptacosan-27-säure.

LC-MS (Methode 1): Rₜ = 1.45 min; MS (ESIpos): m/z = 935 [M+H]⁺.

Die Synthese der Verbindung tert-Butyl-(25S)-22-{(1R)-1-[1-benzyl-4-(2,5-difluorphenyl)-1H-imidazol-2-yl]-2,2-dimethylpropyl}-25-[(tert-butoxycarbonyl)amino]-1-(2,5-dioxo-2,5-dihydro-1H-pyrrol-1-yl)-4,21-dioxo-7,10,13,16-tetraoxa-19-thia-3,22-diazahexacosan-26-oat erfolgte in Analogie zur Synthese von Intermediat C18.

37.4 mg (0.04 mmol) (6S)-9-{(1R)-1-[1-Benzyl-4-(2,5-difluorphenyl)-1H-imidazol-2-yl]-2,2-dimethylpropyl}-6-(tert-butoxycarbonyl)-2,2-dimethyl-4,10-dioxo-3,15,18,21,24-pentaoxa-12-thia-5,9-diazaheptacosan-27-säure.

9.2 mg (0.05 mmol) 1-(2-Aminoethyl)-1H-pyrrol-2,5-dionhydrochlorid(1:1).

Man erhielt 23.4 mg (49 % d. Th.) der Verbindung tert-Butyl-(25S)-22-{(1R)-1-[1-benzyl-4-(2,5-difluorphenyl)-1H-imidazol-2-yl]-2,2-dimethylpropyl}-25-[(tert-butoxycarbonyl)amino]-1-(2,5-dioxo-2,5-dihydro-1H-pyrrol-1-yl)-4,21-dioxo-7,10,13,16-tetraoxa-19-thia-3,22-diazahexacosan-26-oat.

LC-MS (Methode 1): Rₜ = 1.47 min; MS (ESIpos): m/z = 1057 [M+H]⁺.

Die Synthese der Titelverbindung erfolgte in Analogie zur Synthese von Intermediat F38.
20.8 mg (0.02 mmol) tert-Butyl-(25S)-22-{(1R)-1-[1-benzyl-4-(2,5-difluorphenyl)-1H-imidazol-2-yl]-2,2-dimethylpropyl}-25-[(tert-butoxycarbonyl)amino]-1-(2,5-dioxo-2,5-dihydro-1H-pyrrol-1-yl)-4,21-dioxo-7,10,13,16-tetraoxa-19-thia-3,22-diazahexacosan-26-oat.
157.0 mg (1.37 mmol) TFA.
Man erhielt 13.0 mg (65 %) der Titelverbindung.

LC-MS (Methode 1): Rₜ = 0.93 min; MS (ESIpos): m/z = 901 [M+H]⁺.

### Intermediat F50

### Trifluoressigsäure--1-({(2S)-2-amino-4-[{(1R)-1-[1-benzyl-4-(2,5-difluorphenyl)-1H-imidazol-2-yl]-2,2-dimethylpropyl}(glycoloyl)amino]butanoyl}amino)-N-[2-(2,5-dioxo-2,5-dihydro-1H-pyrrol-1-yl)ethyl]cyclopropancarboxamid(1:1)

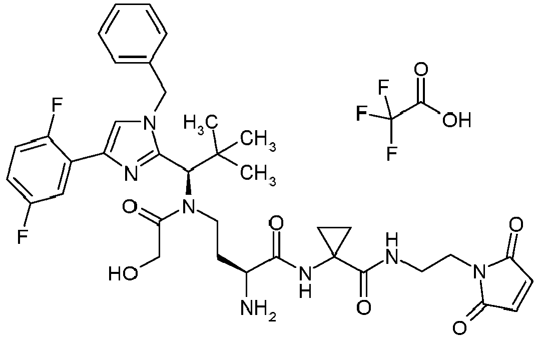

15 mg (0.024 mmol) von Intermediat C5 wurden in 6.5 mL DCM gelöst und mit 19 mg (0.049 mmol) von Intermediat L24 sowie mit 13 µl *N*,*N*-Diisopropylethylamin und 10 mg (0.037 mmol) 2-Brom-1-ethylpyridiniumtetrafluoroborat versetzt. Der Ansatz wurde 3 h bei RT gerührt und anschließend im Vakuum eingeengt. Der Rückstand wurde durch präparative HPLC gereinigt und es wurden 2.4 mg der geschützten Zwischestufe erhalten.

Diese wurden anschließend in 1 mL DCM aufgenommen und mit 0.1 mL Trifluoressigsäure entschützt. Nach Lyophilisation aus Acetonitril/Wasser wurden 2.6 mg (11 % d.Th. über 2 Stufen) der Titelverbindung erhalten.

HPLC (Methode 11): Rₜ = 2.4 min.

LC-MS (Methode 1): Rₜ = 1.25 min; MS (ESIpos): m/z = 819 [M+H]⁺.

### Intermediat F51

### 3-{3-[(3-Amino-2-{[{(1R)-1-[1-benzyl-4-(2,5-difluorphenyl)-1H-imidazol-2-yl]-2,2-dimethylpropyl}(glycoloyl)amino]methyl}propyl)sulfanyl]-2,5-dioxopyrrolidin-1-yl}-N-[17-(2,5-dioxo-2,5-dihydro-1H-pyrrol-1-yl)-15-oxo-4,7,10-trioxa-14-azaheptadec-1-yl]propanamid (Isomer 1)

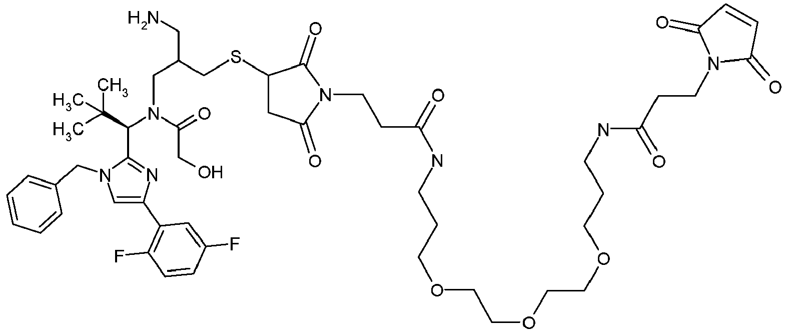

10.0 mg (18.079 µmοl) N-[3-Amino-2-(sulfanylmethyl)propyl]-N-{(1R)-1-[1-benzyl-4-(2,5-difluorphenyl)-1H-imidazol-2-yl]-2,2-dimethylpropyl}-2-hydroxyacetamidhydrochlorid(1:1) (Isomer1) wurden in 100 µl PBS-Puffer (Sigma D8537) und 200 µl ACN vorgelegt. Es wurden 17.1 mg (32.543 µmοl) 3-(2,5-Dioxo-2,5-dihydro-1H-pyrrol-1-yl)-N-[17-(2,5-dioxo-2,5-dihydro-1H-pyrrol-1-yl)-15-oxo-4,7,10-trioxa-14-azaheptadec-1-yl]propanamid zugegeben und 16 h bei RT gerührt. Die Reaktionslösung wurde mittels präparativer HPLC (Eluent: ACN/Wasser + 0.1% TFA, Gradient) gereinigt und lyophilisiert. Es wurden 14.0 mg (75 % d. Th.) der Zielverbindung erhalten.

### Isomer 1

LC-MS (METHODE 1): Rₜ = 0.94 min; MS (ESIpos): m/z = 1039 [M+H]⁺.

### Intermediat F52

### 3-{3-[(3-Amino-2-{[{(1R)-1-[1-benzyl-4-(2,5-difluorphenyl)-1H-imidazol-2-yl]-2,2-dimethylpropyl}(glycoloyl)amino]methyl}propyl)sulfanyl]-2,5-dioxopyrrolidin-1-yl}-N-[17-(2,5-dioxo-2,5-dihydro-1H-pyrrol-1-yl)-15-oxo-4,7,10-trioxa-14-azaheptadec-1-yl]propanamid (Isomer 2)

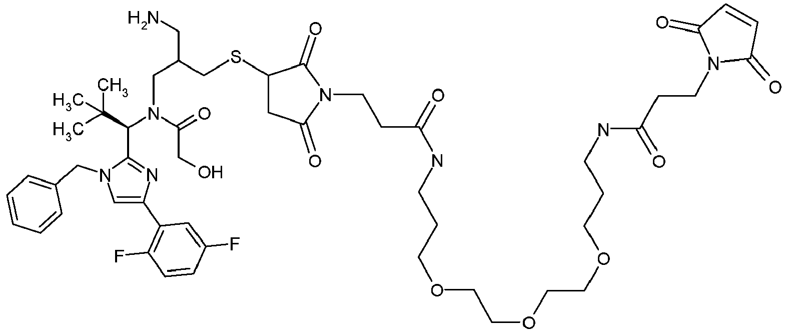

6.5 mg (10.694 µmol, LC/MS Reinheit = 91%) N-[3-Amino-2-(sulfanylmethyl)propyl]-N-{(1R)-1-[1-benzyl-4-(2,5-difluorphenyl)-1H-imidazol-2-yl]-2,2-dimethylpropyl}-2-hydroxyacetamidhydro-chlorid(1:1) (Isomer2) wurden in 100 µl PBS-Puffer (Sigma D8537) und 200 µl ACN vorgelegt. Es wurden 10.1 mg (19.249 µmοl) 3-(2,5-Dioxo-2,5-dihydro-1H-pyrrol-1-yl)-N-[17-(2,5-dioxo-2,5-dihydro-1H-pyrrol-1-yl)-15-oxo-4,7,10-trioxa-14-azaheptadec-1-yl]propanamid zugegeben und 16 h bei RT gerührt. Die Reaktionslösung wurde mittels präparativer HPLC (Eluent: ACN/Wasser + 0.1% TFA, Gradient) gereinigt und lyophilisiert. Es wurden 5.0 mg (45% d. Th. Der Zielverbindung erhalten.

### Isomer 2

LC-MS (Methode 5): Rₜ = 2.87 min; MS (ESIpos): m/z = 1039 [M+H]⁺.

### Intermediat F53

### N-{3-Amino-2-[({ 1-[4-(2,5-dioxo-2,5-dihydro-1H-pyrrol-1-yl)butyl]-2,5-dioxopyrrolidin-3-yl}sulfanyl)methyl]propyl}-N-{(1R)-1-[1-benzyl-4-(2,5-difluorphenyl)-1H-imidazol-2-yl]-2,2-dimethylpropyl}-2-hydroxyacetamid (Isomer 1)

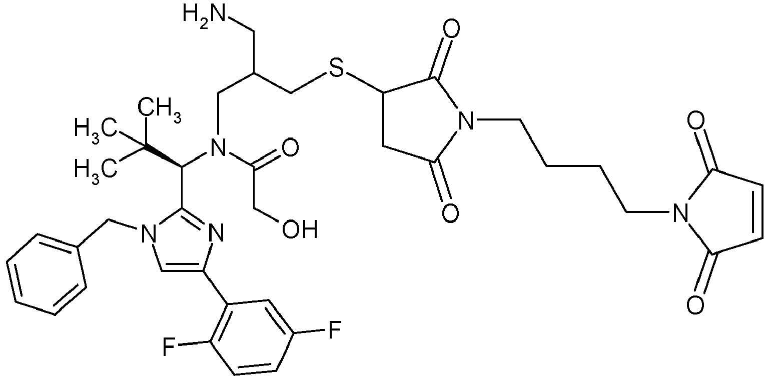

10.0 mg (18.079 µmοl) N-[3-Amino-2-(sulfanylmethyl)propyl]-N-{(1R)-1-[1-benzyl-4-(2,5-difluorphenyl)-1H-imidazol-2-yl]-2,2-dimethylpropyl}-2-hydroxyacetamidhydrochlorid(1:1) (Isomer!) wurden in 200 µl PBS-Puffer (Sigma D8537) und 400 µl ACN corgelegt. Es wurden 8.1 mg (32.543 µmol) 1,1'-Butan-1,4-diylbis(1H-pyrrol-2,5-dion) zugegeben und 1 h bei RT gerührt. Anschließend wurden 300µl DMF addiert und weitere 1.5 h gerührt. Die Reaktionslösung wurde mittels präparativer HPLC (Eluent: ACN/Wasser + 0.1% TFA, Gradient) gereinigt und lyophilisiert. Es wurden 4.0 mg (29 % d. Th.) der Zielverbindung erhalten.

### Isomer 1

LC-MS (METHODE 1): Rₜ = 0.97 min; MS (ESIpos): m/z = 765 [M+H]⁺.

### Intermediat F54

### N-{3-Amino-2-[({ 1-[4-(2,5-dioxo-2,5-dihydro-1H-pyrrol-1-yl)butyl]-2,5-dioxopyrrolidin-3-yl}sulfanyl)methyl]propyl}-N-{(1R)-1-[1-benzyl-4-(2,5-difluorphenyl)-1H-imidazol-2-yl]-2,2-dimethylpropyl}-2-hydroxyacetamid (Isomer 2)

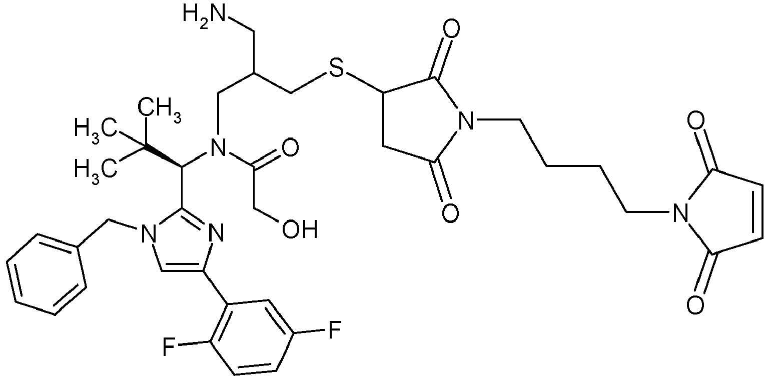

5.0 mg (9.040 µmοl) N-[3-Amino-2-(sulfanylmethyl)propyl]-N-{(1R)-1-[1-benzyl-4-(2,5-difluorphenyl)-1H-imidazol-2-yl]-2,2-dimethylpropyl}-2-hydroxyacetamidhydrochlorid(1:1) (Isomer2) wurden in 500 µl DMF corgelegt. Es wurden 4.0 mg (16.271 µmοl) 1,1'-Butan-1,4-diylbis(1H-pyrrol-2,5-dion) zugegeben und 16 h bei RT gerührt. Die Reaktionslösung wurde mittels präparativer HPLC (Eluent: ACN/Wasser + 0.1% TFA, Gradient) gereinigt und lyophilisiert. Es wurden1.1 mg (16 % d. Th.) der Zielverbindung erhalten.

### Isomer 2

LC-MS (Methode 6): Rₜ = 2.41 min; MS (ESIpos): m/z = 765 [M+H]⁺.

### Intermediat F55

### N-[19-(2,5-dioxo-2,5-dihydro-1H-pyrrol-1-yl)-17-oxo-4,7,10,13-tetraoxa-16-azanonadecan-1-oyl]-L-valyl-N-{4-[(3-aminopropyl) {(1R)-1-[1-benzyl-4-(2,5-difluorophenyl)-1H-imidazol-2-yl]-2,2-dimethylpropyl} carbamoyl]phenyl } -L-alaninamide

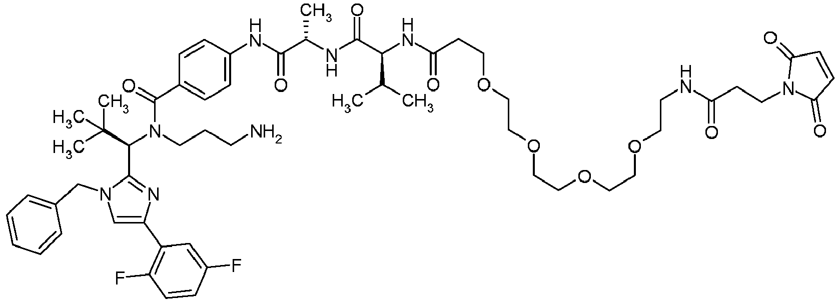

6.5 mg (4.5 µmοl) N-[19-(2,5-Dioxo-2,5-dihydro-1H-pyrrol-1-yl)-17-oxo-4,7,10,13-tetraoxa-16-azanonadecan-1-oyl]-L-valyl-N-[4-({(1R)-1-[1-benzyl-4-(2,5-difluorphenyl)-1H-imidazol-2-yl]-2,2-dimethylpropyl} {3-[(tert-butoxycarbonyl)amino]propyl}carbamoyl)phenyl]-L-alaninamid wurden in 441 µl Dichlormethan gelöst und mit 44 µl (573.1 µmol) Trifluoressigsäure versetzt. Der Ansatz wurde bei RT am Rotationsverdampfer eingeengt, in Wasser sowie ACN aufgenommen und lyophilisiert. Es wurden 5.6 mg (94% d. Th., LC/MS-Reinheit = 92%) der Zielverbindung erhalten.

LC-MS (Methode 1): Rₜ = 1.01 min; MS (ESIpos): m/z = 1100.6 [M+H]⁺.

### Intermediat F56

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-[(2S)-1-{ [2-(2,5-dioxo-2,5-dihydro-1H-pyrrol-1-yl)ethyl]amino}-1-oxopropan-2-yl]butanamid(1:1)

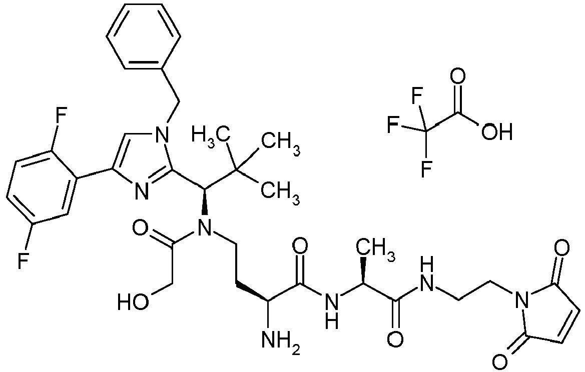

Die Titelverbindung wurde in Analogie zu Intermediat F50 hergestellt.

LC-MS (Methode 1): Rₜ = 0.9 min; MS (EIpos): m/z = 708 [M+H]⁺.

### Intermediat F57

### Trifluoressigsäure--(2S)-2-amino-4-[{(1R)-1-[1-benzyl-4-(2,5-difluorphenyl)-1H-imidazol-2-yl]-2,2-dimethylpropyl}(glycoloyl)amino]-N-[(2R)-1-{[2-(2,5-dioxo-2,5-dihydro-1H-pyrrol-1-yl)ethyl]amino}-1-oxopropan-2-yl]butanamid(1:1)

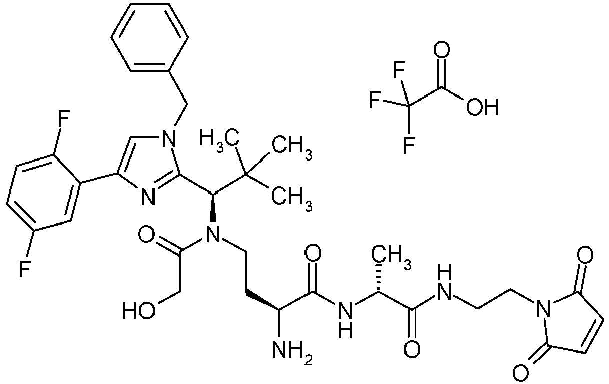

Die Titelverbindung wurde in Analogie zu Intermediat F56 hergestellt.

LC-MS (Methode 1): Rₜ = 0.91 min; MS (EIpos): m/z = 708 [M+H]⁺.

### Intermediat F58

### N-{5-[(2,5-Dioxopyrrolidin-1-yl)oxy]-5-oxopentanoyl}-L-valyl-N-{3-[{(1R)-1-[1-benzyl-4-(2,5-difluorphenyl)-1H-imidazol-2-yl]-2,2-dimethylpropyl}(glycoloyl)amino]propyl}-L-alaninamid

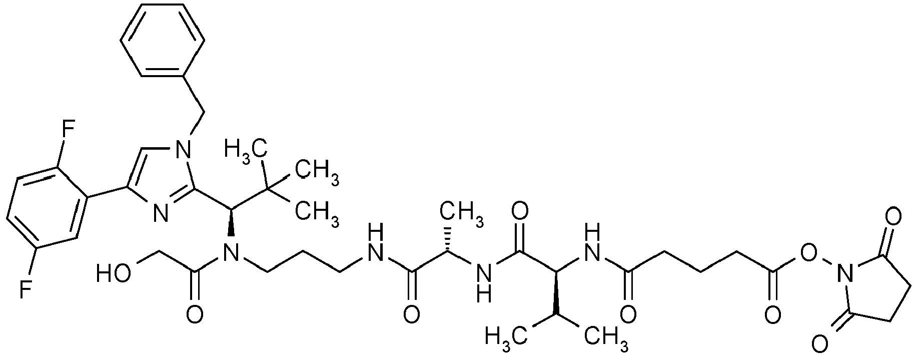

16.2 mg (0.02 mmol) Trifluoressigsäure--L-valyl-N-{3-[{(1R)-1-[1-benzyl-4-(2,5-difluorphenyl)-1H-imidazol-2-yl]-2,2-dimethylpropyl}(glycoloyl)amino]propyl}-L-alaninamid(1:1) (Intermediat C41) wurden in 1.0 mL DMF vorgelegt und mit 8.3 mg (0.06 mmol) N,N-Diisopropylethylamin und 12.6 mg (0.04 mmol) 1,1'-[(1,5-Dioxopentan-1,5-diyl)bis(oxy)]dipyrrolidin-2,5-dion versetzt. Die Reaktionsmischung rührte über Nacht bei RT und wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 11.0 mg (60 % d. Th.) der Titelverbindung.

LC-MS (Methode 1): Rₜ = 1.18 min; MS (ESIpos): m/z = 852 [M+H]⁺.

### Intermediat F82

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{1-[1-benzyl-4-(2,5-difluorphenyl)-1H-pyrrol-2-yl]-2,2-dimethylpropyl}(glycoloyl)amino]propyl}-L-alaninamid

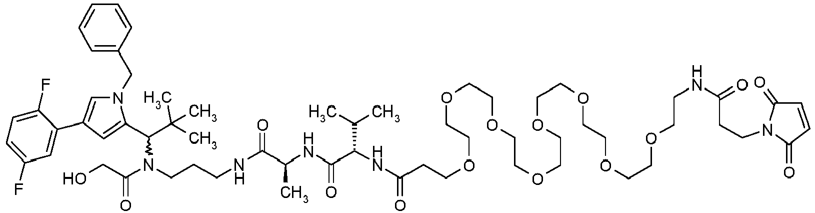

Die Synthese der Titelverbindung erfolgte in Analogie zur Synthese von Intermediat F83. Die dabei verwendeten racemischen Intermediate wurden in Analogie zu den entsprechenden R-Isomer Intermediaten erhalten.

LC-MS (Methode 2): Rₜ = 7.07 min; MS (EIpos): m/z = 1236 [M+Na]⁺.

### Intermediat F83

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid

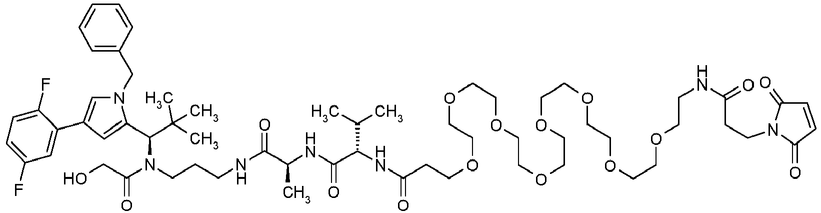

30.0 mg (0.06 mmol) N-(3-Aminopropyl)-N-{(1R)-1-[1-benzyl-4-(2,5-difluorphenyl)-1H-pyrrol-2-yl]-2,2-dimethylpropyl}-2-hydroxyacetamid (Beispiel 98) und 26.1 mg (0.06 mmol) 2,5-Dioxopyrrolidin-1-yl-N-[(9H-fluoren-9-ylmethoxy)carbonyl]-L-alaninat wurden in 2.0 mL DMF vorgelegt und mit 19.4 mg (0.19 mmol) 4-Methylmorpholin versetzt. Die Reaktionsmischung wurde über Nacht bei RT gerührt und mit 11.5 mg (0.19 mmol) HOAc versetzt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 41.9 mg (79 % d. Th.) der Verbindung 9H-Fluoren-9-ylmethyl-[(2S)-1-({3-[{(1R)-1-[1-benzyl-4-(2,5-difluorphenyl)-1H-pyrrol-2-yl]-2,2-dimethylpropyl}(glycoloyl)amino]propyl}amino)-1-oxopropan-2-yl]carbamat.

LC-MS (Methode 1): Rₜ = 1.44 min; MS (ESIpos): m/z = 763 [M+H]⁺.

37.2 mg (0.05 mmol) 9H-Fluoren-9-ylmethyl-[(2S)-1-({3-[{(1R)-1-[1-benzyl-4-(2,5-difluorphenyl)-1H-pyrrol-2-yl]-2,2-dimethylpropyl } (glycoloyl)amino]propyl } amino)-1-oxopropan-2-yl]carbamat wurden in 1.5 mL DMF gelöst und mit 124.6 mg (1.46 mmol) 2-Aminoethanol versetzt. Die Reaktionsmischung wurde über Nacht bei RT gerührt. Die Reaktionsmischung wurde zwischen Ethylacetat und Wasser verteilt und die organische Phase zweimal mit Wasser und einmal mit ges. NaCl-Lösung gewaschen. Nach der Trocknung über Magnesiumsulfat wurde das Lösemittel im Vakuum verdampft und der Rückstand mittels Kieselgel (Laufmittel: Dichlormethan/Methanol 10:1) gereinigt. Die Lösemittel wurden im Vakuum verdampft und der Rückstand im Hochvakuum getrocknet. Man erhielt 14.2 mg (50 % d. Th.) der Verbindung N-{3-[{(1R)-1-[1-Benzyl-4-(2,5-difluorphenyl)-1H-pyrrol-2-yl]-2,2-dimethylpropyl} (glycoloyl)amino]propyl} -L-alaninamid.

LC-MS (Methode 1): Rₜ = 0.92 min; MS (ESIpos): m/z = 541 [M+H]⁺.

14.1 mg (0.03 mmol) N-{3-[{(1R)-1-[1-Benzyl-4-(2,5-difluorphenyl)-1H-pyrrol-2-yl]-2,2-dimethylpropyl}(glycoloyl)amino]propyl} -L-alaninamid und 11.4 (0.03 mmol) 2,5-Dioxopyrrolidin-1-yl-N-[(9H-fluoren-9-ylmethoxy)carbonyl]-L-valinat wurden in 1.5 mL DMF gelöst und mit 7.9 mg (0.08 mmol) 4-Methylmorpholin versetzt. Die Reaktionsmischung wurde über Nacht bei RT gerührt und mit 4.7 mg (0.08 mmol) HOAc versetzt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 15.9 mg (71 % d. Th.) der Verbindung N-[(9H-Fluoren-9-ylmethoxy)carbonyl]-L-valyl-N-{3-[{(1R)-1-[1-benzyl-4-(2,5-difluorphenyl)-1H-pyrrol-2-yl]-2,2-dimethylpropyl} (glycoloyl)amino]propyl} -L-alaninamid.

LC-MS (Methode 1): Rₜ = 1.46 min; MS (ESIpos): m/z = 862 (M+H)⁺.

14.9 mg (0.02 mmol) N-[(9H-Fluoren-9-ylmethoxy)carbonyl]-L-valyl-N-{3-[{(1R)-1-[1-benzyl-4-(2,5-difluorphenyl)-1H-pyrrol-2-yl]-2,2-dimethylpropyl} (glycoloyl)amino]propyl}-L-alaninamid wurden in 1.5 mL DMF gelöst und mit 44.2 mg (0.52 mmol) 2-Aminoethanol versetzt. Die Reaktionsmischung wurde über Nacht bei RT gerührt. Die Reaktionsmischung wurde zwischen Ethylacetat und Wasser verteilt und die organische Phase zweimal mit Wasser und einmal mit ges. NaCl-Lösung gewaschen. Nach der Trocknung über Magnesiumsulfat wurde das Lösemittel im Vakuum verdampft und der Rückstand mittels Kieselgel (Laufmittel: Dichlormethan/Methanol 10:1) gereinigt. Die Lösemittel wurden im Vakuum verdampft und der Rückstand im Hochvakuum getrocknet. Man erhielt 5.7 mg (52 % d. Th.) der Verbindung L-Valyl-N-{3-[{(1R)-1-[1-benzyl-4-(2,5-difluorphenyl)-1H-pyrrol-2-yl]-2,2-dimethylpropyl} (glycoloyl)amino]propyl}-L-alaninamid.

LC-MS (Methode 1): Rₜ = 0.92 min; MS (ESIpos): m/z = 640 (M+H)⁺.

5.5 mg (8.6 µmοl) L-Valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid und 6.5 mg (6.5 µmοl) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid wurden in 1.0 mL DMF gelöst und mit 0.9 mg (8.6 mmol) 4-Methylmorpholin versetzt. Die Reaktionsmischung wurde über Nacht bei RT gerührt und mit 0.8 mg (0.01 mmol) HOAc versetzt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 7.7 mg (74 % d. Th.) der Titelverbindung.

LC-MS (Methode 1): Rₜ = 1.10 min; MS (ESIpos): m/z = 1214 (M+H)⁺.

### Intermediat F84

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

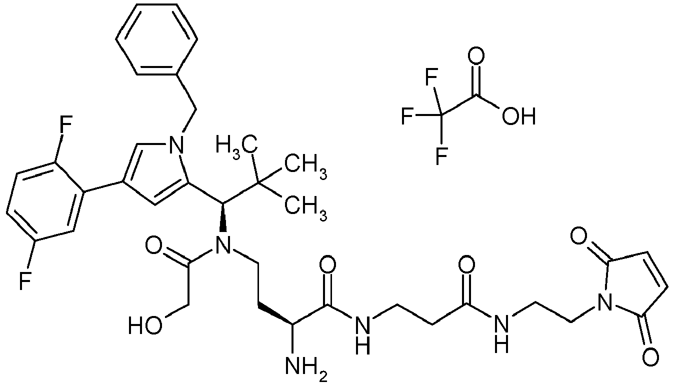

Zunächst wurden 16.5 mg (0.02 mmol) Intermediat C54 in 5 ml DMF aufgenommen und mit 10.4 mg (0.041 mmol) Trifluoressigsäure--1-(2-aminoethyl)-1H-pyrrol-2,5-dion(1:1) in Gegenwart von 11.7 mg ( 0.03 mmol) HATU sowie 18 µl N,N-Diisopropylethylamin umgesetzt. Nach 5 min Rühren bei RT wurde eingeengt und der Rückstand in Acetonitril/Wasser 1: 1 aufgenommen. Mit Trifluoressigsäure wurde ein pH-Wert von 2 eingestellt und der Ansatz erneut eingeengt. Der verbliebene Rückstand durch präparative HPLC gereinigt. Es wurden 8 mg (42% d. Th.) des geschützten Intermediats erhalten.

LC-MS (Methode 1): Rₜ = 1.38 min; MS (EIpos): m/z = 929 [M+H]⁺.

7.6 mg (0.008 mmol) dieses Intermediats wurden in 3 ml DMF aufgenommen und mit 92 mg (0.82 mmol) 1,4-Diazabicyclo(2.2.2)octan versetzt. Der Ansatz wurde 1 h im Ultraschallbad behandelt. Anschließend wurden 31 µl Essigsäure zugegeben und der Ansatz im Hochvakuum eingeengt. Der Rückstand wurde durch präparative HPLC gereinigt. Es wurden 3 mg (45% d. Th.) der Titelverbindung erhalten.

LC-MS (Methode 1): Rₜ = 0.86 min; MS (EIpos): m/z = 707 [M+H]⁺.

¹H-NMR (500 MHz, DMSO-d₆): δ = 8.15 (t, 1H), 7.9-8.1 (m, 4H), 7.6 (m, 1H), 7.5 (s, 1H), 7.15-7.35 (m, 6H), 6.9-7.0 (m, 3H), 6.85 (s, 1H), 5.6 (s, 1H), 4.9 und 5.2 (2d, 2H), 4.05 und 4.2 (2d, 2H), 3.1-3.2 (m, 4H), 2.15 (m, 2H), 0.7 und 1.45 (2m, 2H), 0.8 (s, 9H).

### Intermediat F85

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-[2-(2,5-dioxo-2,5-dihydro-1H-pyrrol-1-yl)ethyl]butanamid(1:1)

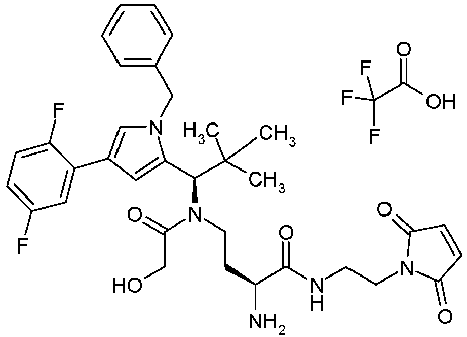

Zunächst wurden 10 mg (0.014 mmol) Intermediat C53 in 3.4 ml DMF aufgenommen und mit 7 mg (0.027 mmol) Trifluoressigsäure--1-(2-aminoethyl)-1H-pyrrol-2,5-dion(1:1) in Gegenwart von 7.8 mg ( 0.02 mmol) HATU sowie 12 µl N,N-Diisopropylethylamin umgesetzt. Nach 15 min Rühren bei RT wurde eingeengt und der Rückstand durch präparative HPLC gereinigt. Es wurden 6.6 mg (57% d. Th.) des geschützten Intermediats erhalten.

LC-MS (Methode 1): Rₜ = 1.4 min; MS (EIpos): m/z = 858 [M+H]⁺.

6.6 mg (0.008 mmol) dieses Intermediats wurden in 2 ml DMF aufgenommen und mit 86 mg (0.77 mmol) 1,4-Diazabicyclo(2.2.2)octan versetzt. Der Ansatz wurde 2 h im Ultraschallbad behandelt. Anschließend wurden 44 µl Essigsäure zugegeben und der Ansatz im Hochvakuum eingeengt. Der Rückstand wurde durch präparative HPLC gereinigt. Es wurden 3.3 mg (53% d. Th.) der Titelverbindung erhalten.

LC-MS (Methode 1): Rₜ = 0.88 min; MS (EIpos): m/z = 636 [M+H]⁺.

### Intermediat F86

### Trifluoressigsäure--(2S)-2-amino-4-[{(1R)-1-[4-benzyl-1-(2,5-difluorphenyl)-1H-pyrazol-3-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

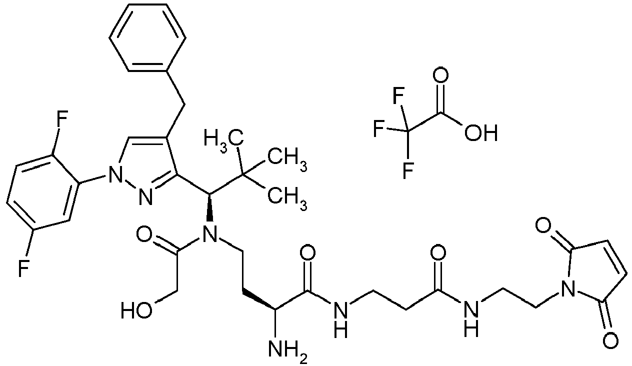

Die Titelverbindung wurde ausgehend von 8 mg (0.012 mmol) Intermediat C51 durch Umsetzung mit 4.5 mg (0.017 mmol) Trifluoressigsäure--1-(2-aminoethyl)-1H-pyrrol-2,5-dion(1:1) in Gegenwart von 5.8 mg ( 0.015 mmol) HATU sowie 10 µl N,N-Diisopropylethylamin und anschließender Entschützung mit Trifluoressigsäure hergestellt. Es wurden 7 mg (78% d. Th. über 2 Stufen) erhalten.

LC-MS (Methode 1): Rₜ = 0.83 min; MS (EIpos): m/z = 708 [M+H]⁺.

### Intermediat F87

### Trifluoressigsäure--(2S)-2-amino-4-[{(1R)-1-[4-benzyl-1-(2,5-difluorphenyl)-1H-pyrazol-3-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

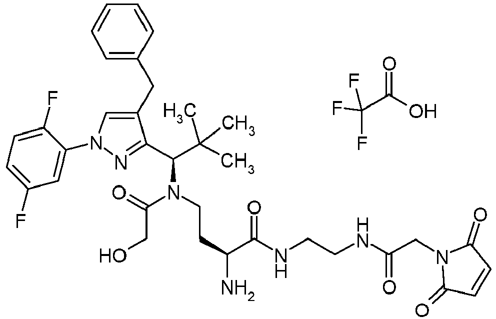

Die Titelverbindung wurde in Analogie zu Intermediat F2 ausgehend von 16 mg (0.025 mmol) Intermediat C49 durch Umsetzung mit 24 mg (0.076 mmol) von Intermediat L1 in Gegenwart von EDCI/HOBT und *N*,*N*-Diisopropylethylamin und anschließender Entschützung mit Trifluoressigsäure hergestellt. Es wurden 3 mg der Titelverbindung (14% d. Th. über 2 Stufen) erhalten.

LC-MS (Methode 1): Rₜ = 0.88 min; MS (EIpos): m/z = 694 [M+H]⁺.

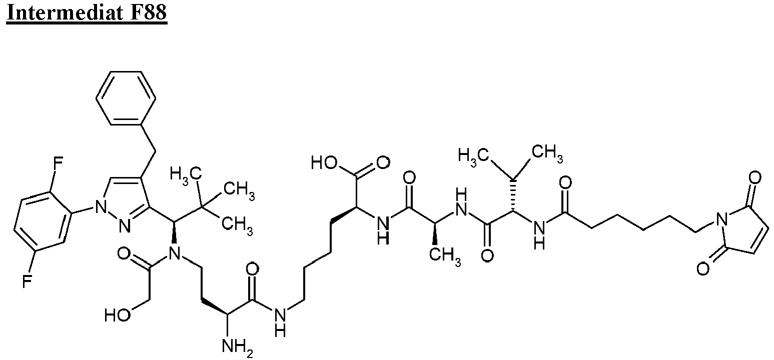

Die Verbindung wurde in Analogie zu Intermediat F8 hergestellt.

LC-MS (Methode 5): Rₜ = 2.97 min; MS (EIpos): m/z = 1006 [M+H]⁺.

### Intermediat F89

### Trifluoressigsäure--N-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N⁵-carbamoyl-N-[4-(2,5-dioxo-2,5-dihydro-1H-pyrrol-1-yl)phenyl]-L-ornithinamid(1:1)

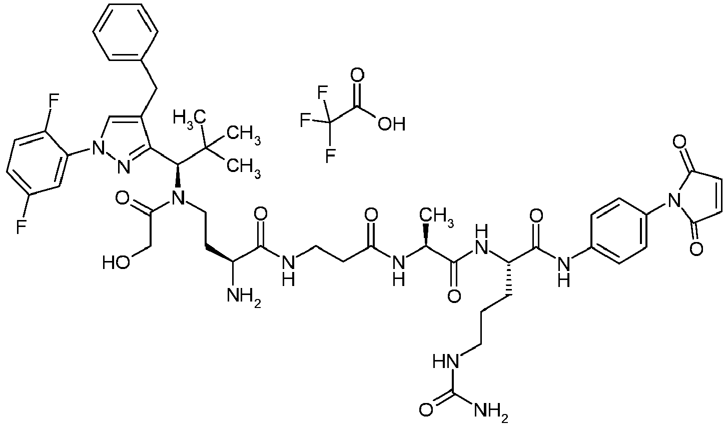

Die Titelverbindung wurde ausgehend von 8 mg (0.012 mmol) Intermediat C51 durch Umsetzung mit 7.4 mg (0.014 mmol) Intermediat L8 in Gegenwart von 5.8 mg ( 0.015 mmol) HATU sowie 10 µl N,N-Diisopropylethylamin und anschließender Entschützung mit Trifluoressigsäure hergestellt. Es wurden 10 mg (78% d. Th. über 2 Stufen) erhalten.

LC-MS (Methode 1): Rₜ = 0.87 min; MS (EIpos): m/z = 984 [M+H]⁺.

### Intermediat F90

### N-[6-(2,5-Dioxo-2,5-dihydro-1H-pyrrol-1-yl)hexanoyl]-L-valyl-N⁵-carbamoyl-L-ornithyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin-trifluoressigsäure(1:1)

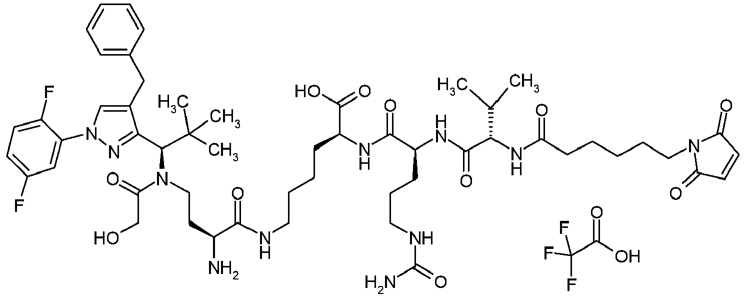

Die Titelverbindung wurde ausgehend von 11 mg (0.018 mmol) Intermediat C49 durch Umsetzung mit 13.7 mg (0.018 mmol) Intermediat L17 in Gegenwart von 34 mg ( 0.089 mmol) HATU sowie 19 µl N,N-Diisopropylethylamin und anschließender Entschützung mit Trifluoressigsäure hergestellt. Es wurden 7.5 mg (35% d. Th. über 2 Stufen) erhalten.

LC-MS (Methode 8): Rₜ = 6.78 min; MS (EIpos): m/z = 1092 [M+H]⁺.

### Intermediat F91

### Trifluoressigsäure--(2S)-2-amino-4-[{(1R)-1-[4-benzyl-1-(2,5-difluorphenyl)-1H-pyrazol-3-yl]-2,2-dimethylpropyl}(glycoloyl)amino]-N-[2-(2,5-dioxo-2,5-dihydro-1H-pyrrol-1-yl)ethyl]butanamid(1:1)

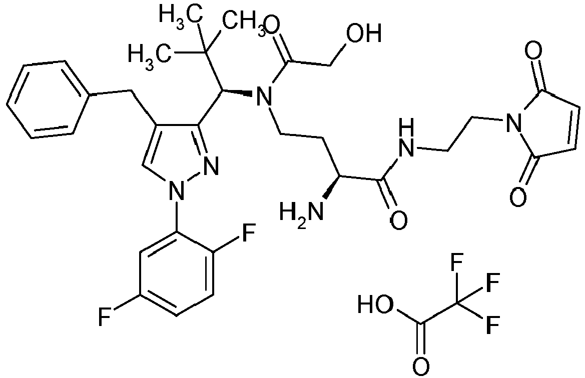

9.3 mg (0.01 mmol) tert-Butyl-[(2S)-4-[{(1R)-1-[4-benzyl-1-(2,5-difluorphenyl)-1H-pyrazol-3-yl]-2,2-dimethylpropyl}(glycoloyl)amino]-1-{[2-(2,5-dioxo-2,5-dihydro-1H-pyrrol-1-yl)ethyl]amino}-1-oxobutan-2-yl]carbamat wurden in 2 ml Dichlormethan gelöst und mit 740 mg (6.49 mmol, 0.50 ml) Trifluoressigsäure versetzt und 1.5 h bei RT gerührt. Anschließend wurde das Reaktionsgemisch eingeengt und der Rückstand in Acetonitril und Wasser aufgenommen und lyophilisiert. Es wurden 9.2 mg (96% d. Th.) der Zielverbindung erhalten.

LC-MS (Methode 1): Rₜ = 0.88 min; MS (EIpos): m/z = 637 [M+H]⁺.

### Intermediat F103

### N-[19-(2,5-Dioxo-2,5-dihydro-1H-pyrrol-1-yl)-17-oxo-4,7,10,13-tetraoxa-16-azanonadecan-1-oyl]-L-valyl-N-(3-{[(1R)-1-(3-benzyl-7-chlor-4-oxo-3,4-dihydrochinazolin-2-yl)-2-methylpropyl] (4-methylbenzoyl)amino }propyl)-L-alaninamid

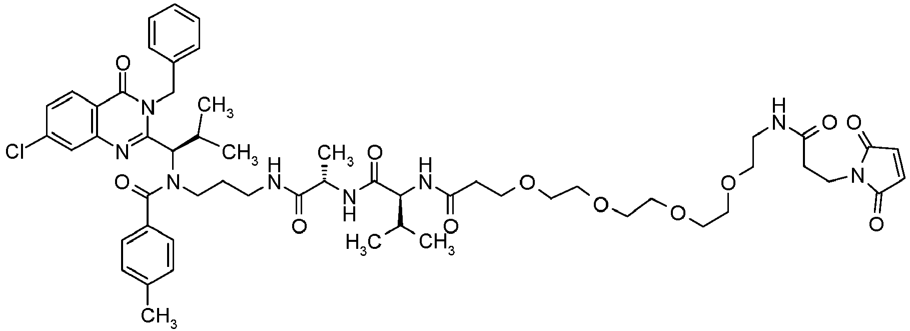

Die Titelverbindung wurde ausgehend von 10 mg (0.019 mmol) N-(3-Aminopropyl)-N-[(1R)-1-(3-benzyl-7-chlor-4-oxo-3,4-dihydrochinazolin-2-yl)-2-methylpropyl]-4-methylbenzamid durch Umsetzung mit 11.3 mg (0.019 mmol) Intermediat L44 in Gegenwart von 8.8 mg ( 0.023 mmol) HATU sowie 10 µl N,N-Diisopropylethylamin erhalten. Die Reinigung erfolgte durch präparative HPLC.

Ausbeute: 8.5 mg (35% d. Th.)

LC-MS (Methode 5): Rₜ = 3.82 min; MS (EIpos): m/z = 1085 [M+H]⁺.

### Intermediat F104

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{[(2,5-dioxo-2,5-dihydro-1H-pyrrol-1-yl)acetyl]amino}ethyl)butanamid(1:1)

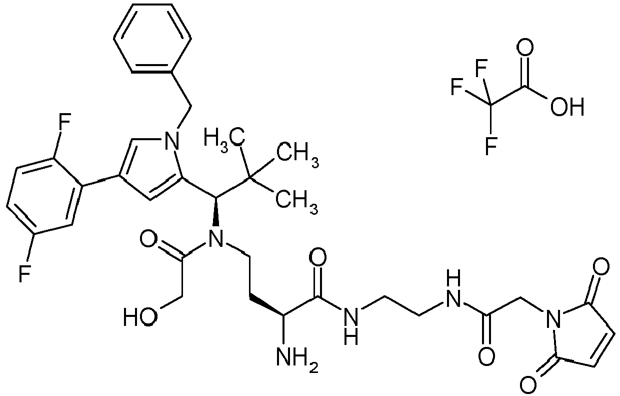

15 mg (0.023 mmol) von Intermediat C58 wurden zunächst mit 11 mg (0.036 mmol) Intermediat L1 in Gegenwart von 13 mg ( 0.034 mmol) HATU sowie 10 µl N,N-Diisopropylethylamin umgesetzt. Nach 60 min Rühren bei RT wurde eingeengt und der Rückstand durch präparative HPLC gereinigt. Es wurden 12.3 mg (63% d. Th.) des geschützten Intermediats erhalten.

LC-MS (Methode 1): Rₜ = 1.3 min; MS (EIpos): m/z = 837 [M+H]⁺.

Im zweiten Schritt wurde dieses Intermediat in 3 mL 2,2,2-Trifluorethanol gelöst. Es wurden 12 mg (0.088 mmol) Zinkchlorid zugegeben und der Ansatz 2 h bei 50°C gerührt. Anschließend wurden 26 mg (0.088 mmol) Ethylendiamin-N,N,N',N'-tetraessigsäure sowie 2 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 8.1 mg (68% d.Th.) der Titelverbindung erhalten.

LC-MS (Methode 1): Rₜ = 0.89 min; MS (ESIpos): m/z = 693 (M+H)⁺.

### Intermediat F105

### N²-{(2S)-2-Amino-4-[{(1R)-1-[1-benzyl-4-(2,5-difluorphenyl)-1H-imidazol-2-yl]-2,2-dimethylpropyl}(glycoloyl)amino]butanoyl}-N-[2-(2,5-dioxo-2,5-dihydro-1H-pyrrol-1-yl)ethyl]-L-glutamin-trifluoressigsäure(1:1)

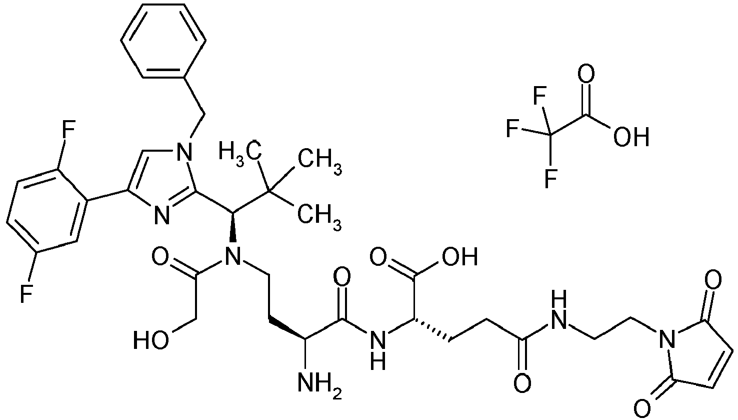

Die Titelverbindung wurde in Analogie zu Intermediat F32 ausgehend von Intermediat C5 und Intermediat L46 hergestellt.

LC-MS (Methode 1): Rₜ = 0.82 min; MS (EIpos): m/z = 766 [M+H]⁺.

### Intermediat F106

### Trifluoressigsäure--N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-L-alanyl-N⁵-carbamoyl-N-[4-(2,5-dioxo-2,5-dihydro-1H-pyrrol-1-yl)phenyl]-L-ornithinamid(1:1)

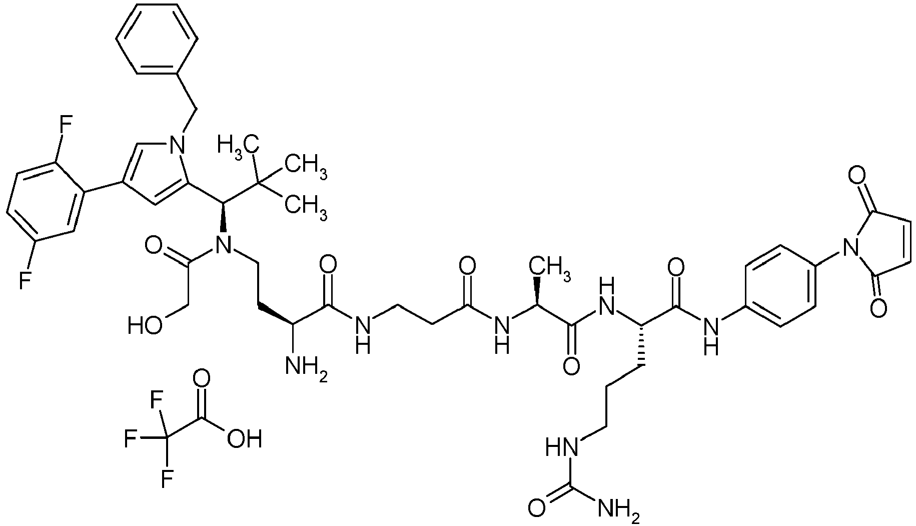

Die Titelverbindung wurde in Analogie zu Intermediat F104 ausgehend von Intermediat C53 und Intermediat L47 hergestellt.
HPLC (Methode 11): Rₜ = 1.85 min;
LC-MS (Methode 1): Rₜ = 0.86 min; MS (ESIpos): m/z = 983 (M+H)⁺.

### Intermediat F107

### Trifluoressigsäure--(1R,2S)-2-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

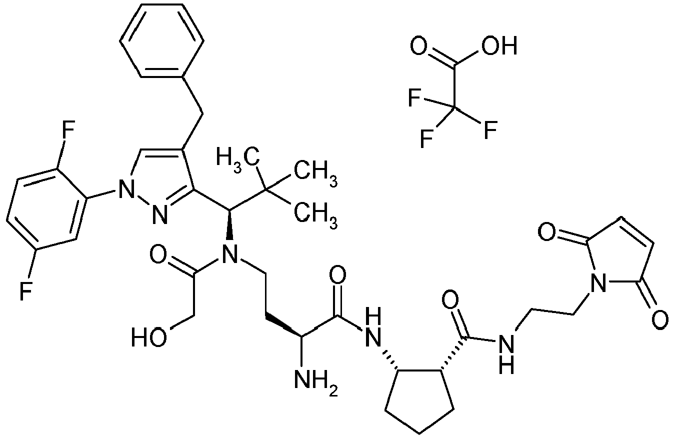

Die Titelverbindung wurde ausgehend von 15 mg (0.024 mmol) Intermediat C49 durch Umsetzung mit 22.3 mg (0.049 mmol) Intermediat L48 in Gegenwart von 14 mg ( 0.037 mmol) HATU sowie 21 µl N,N-Diisopropylethylamin und anschließender Entschützung mit Trifluoressigsäure hergestellt. Es wurden 13 mg (60% d. Th. über 2 Stufen) erhalten.

LC-MS (Methode 1): Rₜ = 0.9 min; MS (EIpos): m/z = 748 [M+H]⁺.

### Intermediat F108

### Trifluoressigsäure--(1R,2S)-2-({(2S)-2-amino-4-[{(1R)-1-[1-benzyl-4-(2,5-difluorphenyl)-1H-pyrrol-2-yl]-2,2-dimethylpropyl } (glycoloyl)amino]butanoyl }amino)-N-[2-(2,5-dioxo-2,5-dihydro-1H-pyrrol-1-yl)ethyl]cyclopentancarboxamid(1:1)

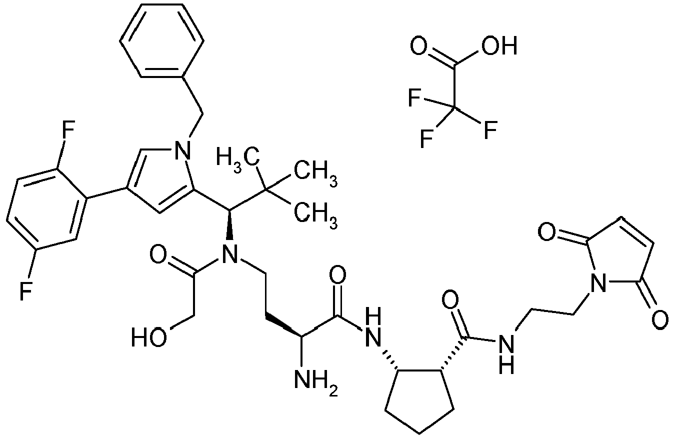

Die Titelverbindung wurde in Analogie zu Intermediat F104 aus 20 mg (0.027 mmol) Intermediat C53 und 24 mg (0.054 mmol) von Intermediat L48 hergestellt. Es wurden 3 mg (14% d. Th. über 2 Stufen) erhalten.

LC-MS (Methode 1): Rₜ = 0.93 min; MS (EIpos): m/z = 747 [M+H]⁺.

### Intermediat F109

### Trifluoressigsäure--(2S)-2-amino-4-[{(1R)-1-[4-benzyl-1-(2,5-difluorphenyl)-1H-pyrazol-3-yl]-2,2-dimethylpropyl}(glycoloyl)amino]-N-{2-[(bromacetyl)amino]ethyl}butanamid(1:1)

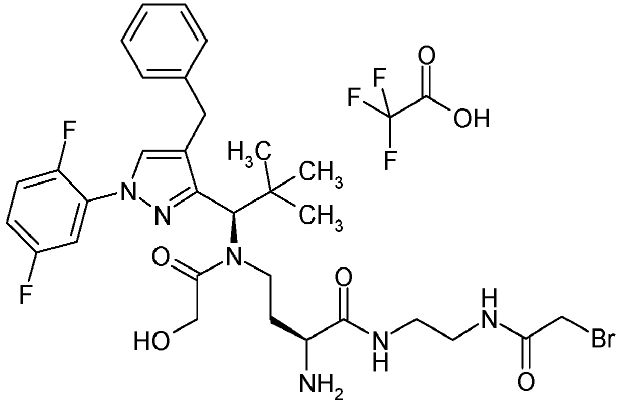

17 mg (0.026 mmol) von Intermediat C57 wurden in 3 mL DMF aufgenommen und mit 7 mg (0.027 mmol) von kommerziell erhältlichem 1-(2-Bromacetoxy)pyrrolidin-2,5-dion in Gegenwart von 14 µl *N,N-*Diisopropylethylamin umgesetzt. Nach 15min Rühren bei RT wurde eingeengt und der Rückstand durch präparative HPLC gereinigt. Es wurden 7 mg (33% d. Th.) dieser Zwischenstufe erhalten.

LC-MS (Methode 1): Rₜ = 1.29 min; MS (ESIpos): m/z = 777 und 779 (M+H)⁺.

Dieses Intermediat wurde in 1 mL Dichlormethan aufgenommen und mit 1 mL Trifluoressigsäure entschützt. Nach Einengen und Lyophilisation aus Acetonitril/Wasser wurden 6 mg (88% d.Th.) der Titelverbindung erhalten.

LC-MS (Methode 1): Rₜ = 0.86 min; MS (ESIpos): m/z = 677/679 (M+H)⁺.

### Intermediat F110

### N-(Bromacetyl)-L-valyl-L-alanyl-N⁶-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-L-lysin-trifluoressigsäure(1:1)

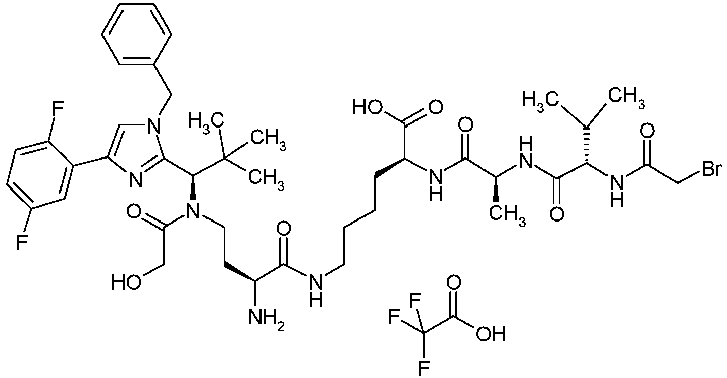

Die Titelverbindung wurde in Analogie zu Intermediat F109 aus 16 mg (0.023 mmol) Intermediat C5 und 17 mg (0.025 mmol) Intermediat L49 hergestellt. Es wurden 6 mg (24% d.Th. über 2 Stufen) der Titelverbindung erhalten.
HPLC (Methode 11): Rₜ = 1.93 min;
LC-MS (Methode 1): Rₜ = 0.88 min; MS (ESIpos): m/z = 933 und 935 (M+H)⁺.

### Intermediat F111

### Trifluoressigsäure--(1S,3R)-3-({(2S)-2-amino-4-[{(1R)-1-[1-benzyl-4-(2,5-difluorphenyl)-1H-imidazol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

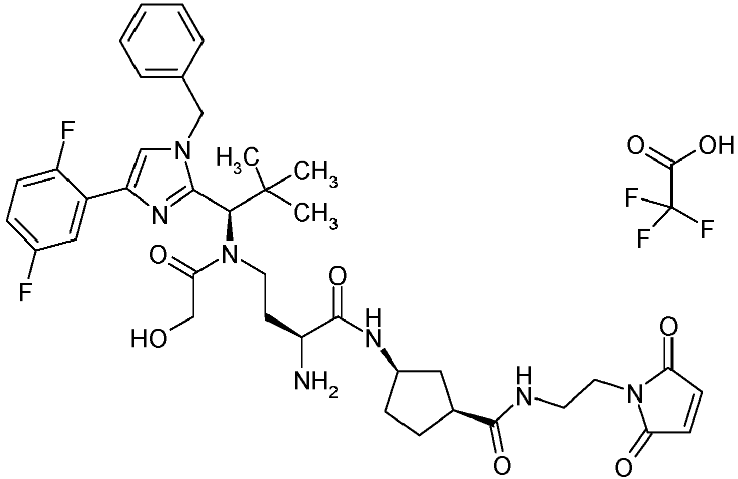

Die Titelverbindung wurde ausgehend von 15 mg (0.022 mmol) Intermediat C5 durch Umsetzung mit 16 mg (0.044 mmol) Intermediat L50 in Gegenwart von 12.5 mg ( 0.032 mmol) HATU sowie 19 µl N,N-Diisopropylethylamin und anschließender Entschützung mit Trifluoressigsäure hergestellt. Es wurden 13 mg (67% d. Th. über 2 Stufen) erhalten.

LC-MS (Methode 1): Rₜ = 0.89 min; MS (EIpos): m/z = 748 [M+H]⁺.

### Intermediat F112

### Trifluoressigsäure--(1S,3R)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

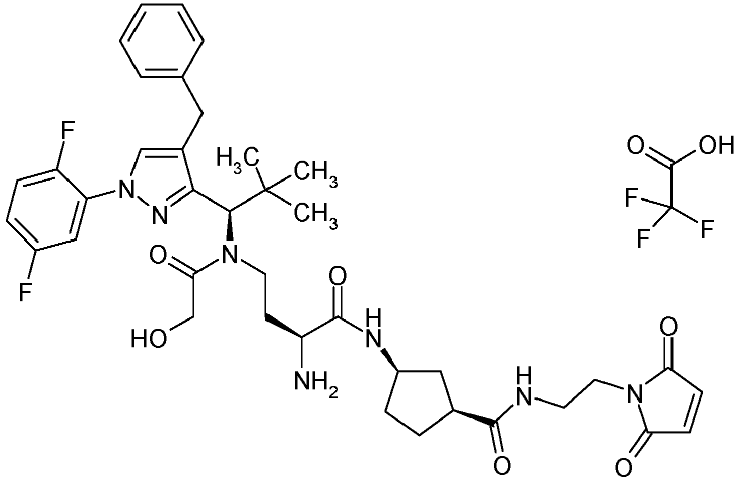

Die Titelverbindung wurde ausgehend von 15 mg (0.024 mmol) Intermediat C49 durch Umsetzung mit 18 mg (0.049 mmol) Intermediat L50 in Gegenwart von 14 mg ( 0.037 mmol) HATU sowie 21 µl N,N-Diisopropylethylamin und anschließender Entschützung mit Trifluoressigsäure hergestellt. Es wurden 12 mg (51% d. Th. über 2 Stufen) erhalten.

LC-MS (Methode 1): Rₜ = 0.89 min; MS (EIpos): m/z = 748 [M+H]⁺.

### Intermediat F113

### Trifluoressigsäure--(1S,3R)-3-({(2S)-2-amino-4-[{(1R)-1-[1-benzyl-4-(2,5-difluorphenyl)-1H-pyrrol-2-yl]-2,2-dimethylpropyl } (glycoloyl)amino]butanoyl }amino)-N-[2-(2,5-dioxo-2,5-dihydro-1H-pyrrol-1-yl)ethyl]cyclopentancarboxamid(1:1)

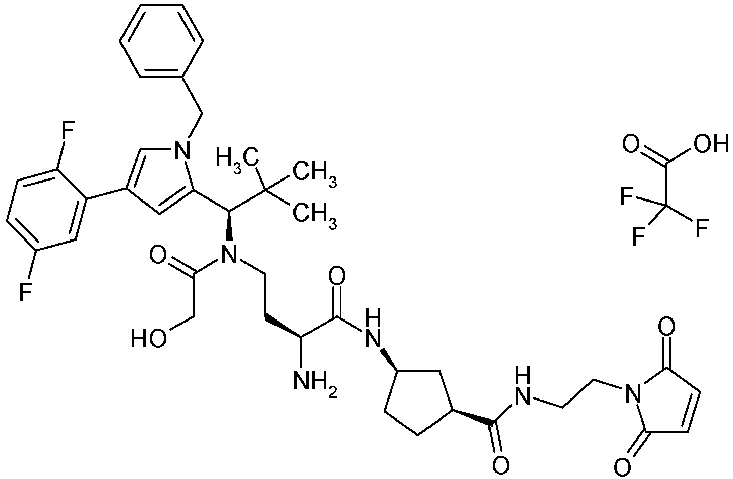

Die Titelverbindung wurde ausgehend von 15 mg (0.019 mmol) Intermediat C53 durch Umsetzung mit 14 mg (0.038 mmol) Intermediat L50 in Gegenwart von 11 mg ( 0.029 mmol) HATU sowie 17 µl N,N-Diisopropylethylamin und anschließender Entschützung mit 133 mg DABCO in 2 ml DMF hergestellt. Nach HPLC-Reinigung wurden 4 mg (24% d. Th. über 2 Stufen) erhalten.

LC-MS (Methode 5): Rₜ = 2.77 min; MS (EIpos): m/z = 747 [M+H]⁺.

### Intermediat F114

### Trifluoressigsäure--(1R,3R)-3-({(2S)-2-amino-4-[{(1R)-1-[1-benzyl-4-(2,5-difluorphenyl)-1H-imidazol-2-yl]-2,2-dimethylpropyl } (glycoloyl)amino]butanoyl } amino)-N-[2-(2,5-dioxo-2,5-dihydro-1H-pyrrol-1-yl)ethyl]cyclopentancarboxamid(1: 1)

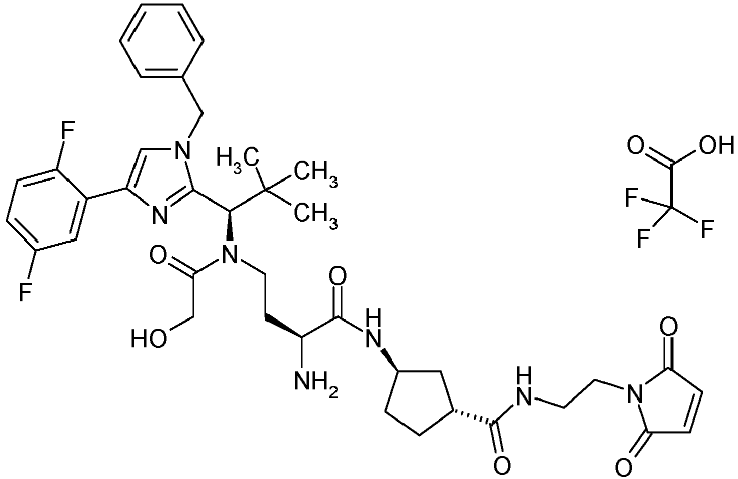

Die Titelverbindung wurde ausgehend von 15 mg (0.022 mmol) Intermediat C5 durch Umsetzung mit 16 mg (0.044 mmol) Intermediat L51 in Gegenwart von 12.6 mg ( 0.032 mmol) HATU sowie 19 µl N,N-Diisopropylethylamin und anschließender Entschützung mit Trifluoressigsäure hergestellt. Es wurden 11 mg (53% d. Th. über 2 Stufen) erhalten.

LC-MS (Methode 1): Rₜ = 0.89 min; MS (EIpos): m/z = 748 [M+H]⁺.

### Intermediat F115

### Trifluoressigsäure--(1R,3R)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5 - dihydro-1H-pyrrol-1-yl)ethyl]cyclopentancarboxamid(1: 1)

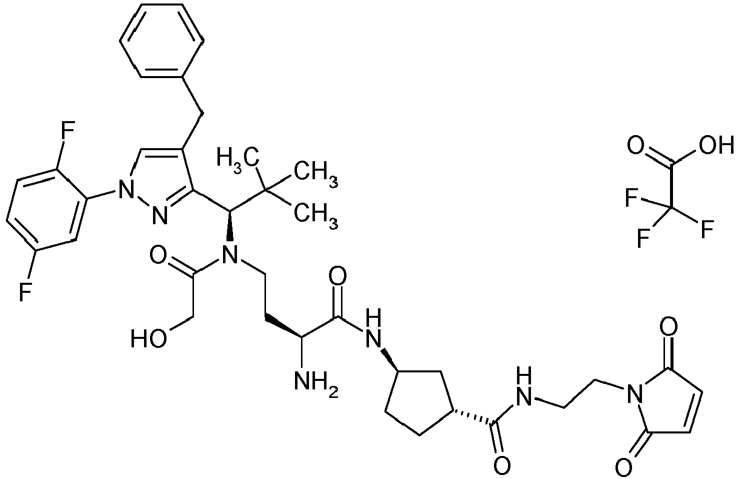

Die Titelverbindung wurde ausgehend von 15 mg (0.024 mmol) Intermediat C49 durch Umsetzung mit 18 mg (0.047 mmol) Intermediat L51 in Gegenwart von 13 mg ( 0.035 mmol) HATU sowie 21 µl N,N-Diisopropylethylamin und anschließender Entschützung mit Trifluoressigsäure hergestellt. Es wurden 12 mg (51% d. Th. über 2 Stufen) erhalten.

LC-MS (Methode 1): Rₜ = 0.87 min; MS (EIpos): m/z = 748 [M+H]⁺.

### Intermediat F116

### Trifluoressigsäure--(1R,3R)-3-({(2S)-2-amino-4-[{(1R)-1-[1-benzyl-4-(2,5-difluorphenyl)-1H-pyrrol-2-yl]-2,2-dimethylpropyl } (glycoloyl)amino]butanoyl }amino)-N-[2-(2,5-dioxo-2,5-dihydro-1H-pyrrol-1-yl)ethyl]cyclopentancarboxamid(1:1)

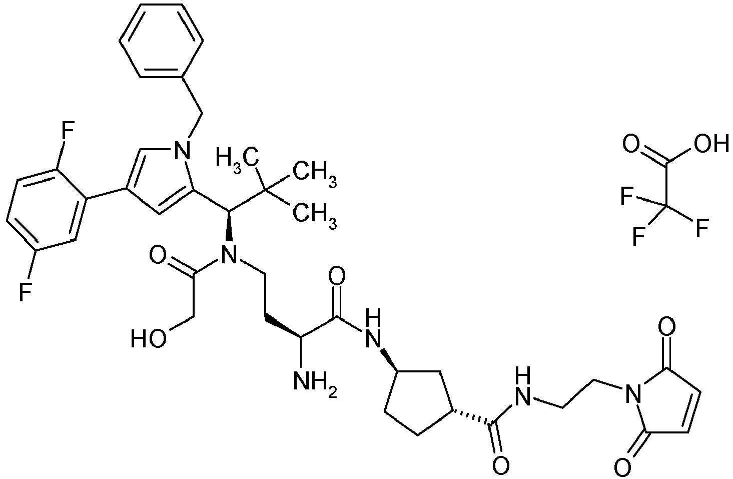

Die Titelverbindung wurde ausgehend von 11 mg (0.014 mmol) Intermediat C53 durch Umsetzung mit 11 mg (0.028 mmol) Intermediat L51 in Gegenwart von 8 mg ( 0.021 mmol) HATU sowie 12 µl N,N-Diisopropylethylamin und anschließender Entschützung mit 87 mg DABCO in 2 ml DMF hergestellt. Nach HPLC-Reinigung wurden 3.3 mg (28% d. Th. über 2 Stufen) erhalten.

LC-MS (Methode 1): Rₜ = 0.92 min; MS (EIpos): m/z = 747 [M+H]⁺.

### Intermediat F117

### Trifluoressigsäure--N-[(3S)-3-amino-4-{2-[6-(2,5-dioxo-2,5-dihydro-1H-pyrrol-1-yl)hexanoyl]hydrazino}-4-oxobutyl]-N-{(1R)-1-[4-benzyl-1-(2,5-difluorphenyl)-1H-pyrazol-3-yl]-2,2-dimethylpropyl} -2-hydroxyacetamid (1:1)

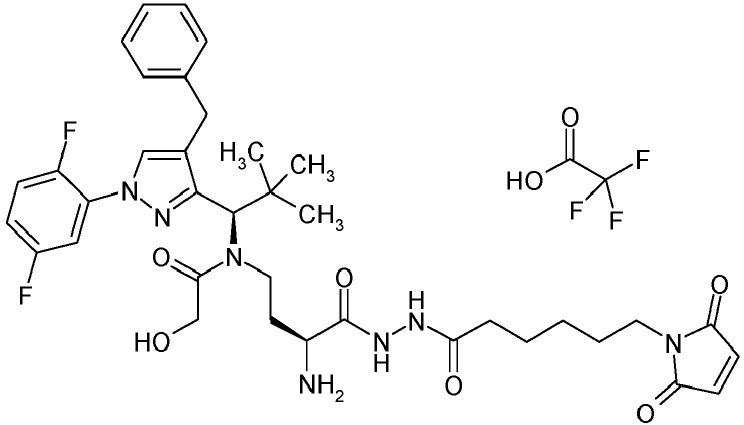

Die Titelverbindung wurde nach klassischen Methoden der Peptidchemie ausgehend von Intermediat C49 hergestellt. C49 wurde zunächst mit 9H-Fluoren-9-ylmethyl-hydrazincarboxylat in Gegenwart von HATU gekuppelt. Anschließend wurde die Fmoc-Schutzgruppe mit Piperidin in DMF entfernt und das erhaltene Hydrazid mit 6-(2,5-Dioxo-2,5-dihydro-1H-pyrrol-1-yl)hexansäure in Gegenwart von HATU gekuppelt. Im letzten Schritt wurde die Boc-Schutzgruppe mit TFA in Dichlormethan entfernt.

LC-MS (Methode 1): Rₜ = 0.93 min; MS (EIpos): m/z = 722 [M+H]⁺.

### Intermediat F118

### Trifluoressigsäure--N-[(3S)-3-amino-4-{2-[6-(2,5-dioxo-2,5-dihydro-1H-pyrrol-1-yl)hexanoyl]hydrazino}-4-oxobutyl]-N-{(1R)-1-[1-benzyl-4-(2,5-difluorphenyl)-1H-pyrrol-2-yl]-2,2-dimethylpropyl}-2-hydroxyacetamid (1:1)

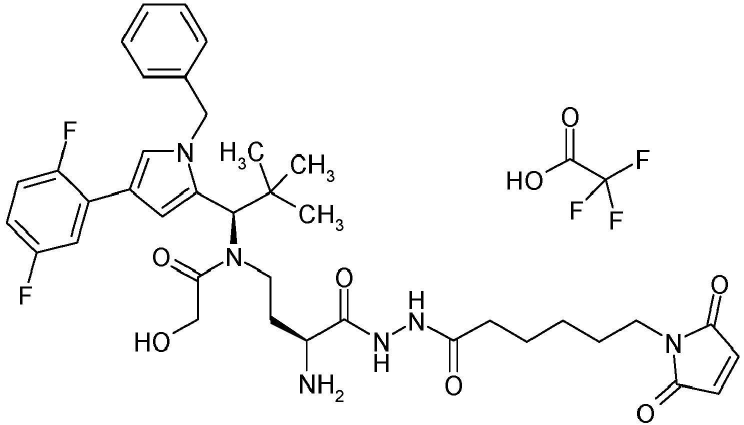

Die Titelverbindung wurde im ersten Schritt analog zu Intermediat F3 ausgehend von 15 mg (0.019 mmol) Intermediat C53 durch Kupplung mit kommerziell erhältlichem 6-(2,5-Dioxo-2,5-dihydro-1H-pyrrol-1-yl)hexanhydrazid in Gegenwart von HATU hergestellt. Anschließend erfolgte die Abspaltung der Fmoc-Schutzgrruppe mit 142 mg DABCO in DMF. Nach HPLC Reinigung wurden 3 mg (19 % d. Th.) der Titelverbindung erhalten.

LC-MS (Methode 1): Rₜ = 0.90 min; MS (EIpos): m/z = 721 [M+H]⁺.

### Intermediat F119

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-{2-[(bromacetyl)amino]ethyl}butanamid (1:1)

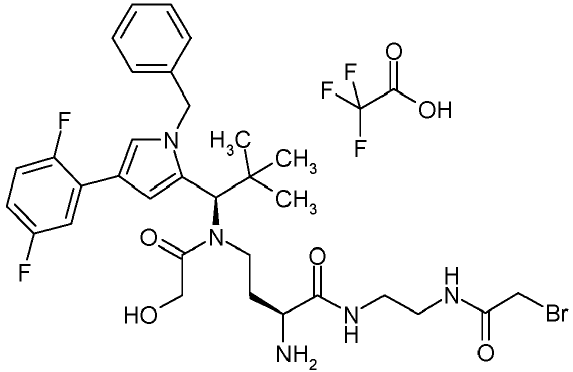

29 mg (0.044 mmol) von Intermediat C58 wurden in 3.4 mL DMF aufgenommen und mit 36 mg (0.087 mmol) von Intermediat L52 sowie mit 25 mg ( 0.065 mmol) HATU und 19 µl N,N-Diisopropylethylamin versetzt. Nach 60 min Rühren bei RT wurde eingeengt und der Rückstand durch präparative HPLC gereinigt. Es wurden 26.4 mg (73% d. Th.) der Zwischenstufe erhalten.

LC-MS (Methode 1): Rₜ = 1.34 min; MS (ESIpos): m/z = 820 und 822 (M+H)⁺.

Dieses Intermediat wurde in 3 mL 2,2,2-Trifluorethanol gelöst. Es wurden 6.5 mg (0.048 mmol) Zinkchlorid zugegeben und der Ansatz 4 h bei 50°C gerührt. Dann wurden 13.9 mg (0.048 mmol) Ethylendiamin-N,N,N',N'-tetraessigsäure sowie 2 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 14.4 mg (58% d.Th.) der Titelverbindung erhalten.

LC-MS (Methode 1): Rₜ = 0.88 min; MS (ESIpos): m/z = 676 und 678 (M+H)⁺.

### Intermediat F120

### Trifluoressigsäure--(1S,3S)-3-({(2S)-2-amino-4-[{(1R)-1-[1-benzyl-4-(2,5-difluorphenyl)-1H-imidazol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

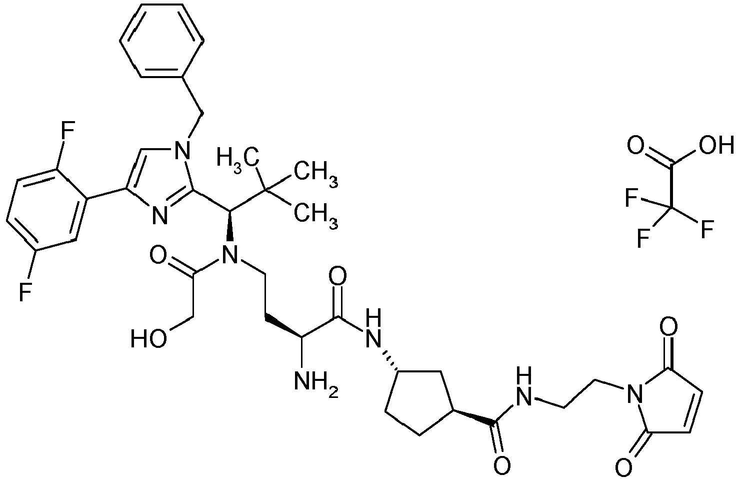

Die Titelverbindung wurde ausgehend von 10 mg (0.015 mmol) Intermediat C5 durch Umsetzung mit 11 mg (0.03 mmol) Intermediat L53 in Gegenwart von 8.4 mg ( 0.022 mmol) HATU sowie 13 µl N,N-Diisopropylethylamin und anschließender Entschützung mit Trifluoressigsäure hergestellt. Es wurden 7.5 mg (59% d. Th. über 2 Stufen) erhalten.

LC-MS (Methode 1): Rₜ = 0.85 min; MS (EIpos): m/z = 748 [M+H]⁺.

### Intermediat F121

### Trifluoressigsäure--(1S,3S)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

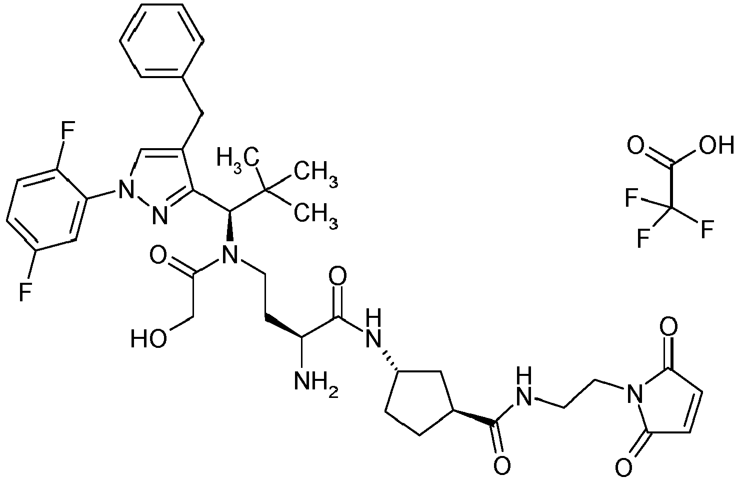

Die Titelverbindung wurde ausgehend von 10 mg (0.016 mmol) Intermediat C49 durch Umsetzung mit 11.5 mg (0.031 mmol) Intermediat L53 in Gegenwart von 9 mg ( 0.024 mmol) HATU sowie 14 µl N,N-Diisopropylethylamin und anschließender Entschützung mit Trifluoressigsäure hergestellt. Es wurden 9 mg (61% d. Th. über 2 Stufen) erhalten.

LC-MS (Methode 1): Rₜ = 0.84 min; MS (EIpos): m/z = 748 [M+H]⁺.

### Intermediat F122

### Trifluoressigsäure--(1S,3S)-3-({(2S)-2-amino-4-[{(1R)-1-[1-benzyl-4-(2,5-difluorphenyl)-1H-pyrrol-2-yl]-2,2-dimethylpropyl } (glycoloyl)amino]butanoyl }amino)-N-[2-(2,5-dioxo-2,5-dihydro-1H-pyrrol-1-yl)ethyl]cyclopentancarboxamid(1:1)

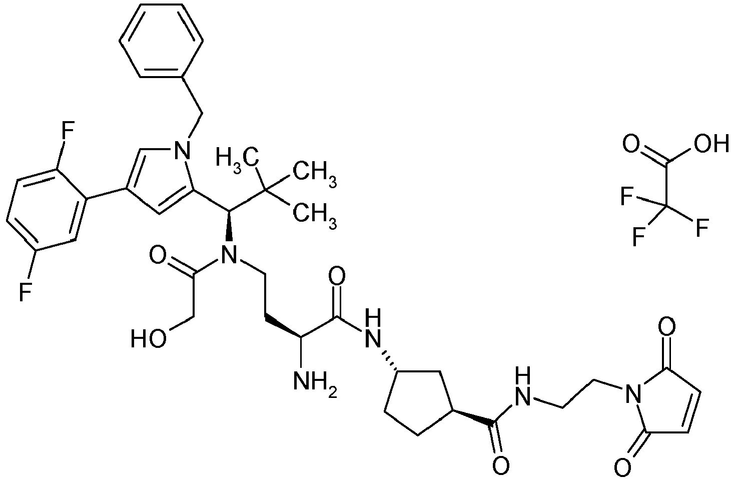

Die Titelverbindung wurde ausgehend von 15 mg (0.019 mmol) Intermediat C53 durch Umsetzung mit 14 mg (0.038 mmol) Intermediat L53 in Gegenwart von 11 mg ( 0.029 mmol) HATU sowie 17 µl N,N-Diisopropylethylamin und anschließender Entschützung mit 202 mg DABCO in 3 ml DMF hergestellt. Nach HPLC-Reinigung wurden 4 mg (24% d. Th. über 2 Stufen) erhalten.

LC-MS (Methode 1): Rₜ = 0.87 min; MS (EIpos): m/z = 747 [M+H]⁺.

### Intermediat F123

### Trifluoressigsäure--(1R,3S)-3-({(2S)-2-amino-4-[{(1R)-1-[1-benzyl-4-(2,5-difluorphenyl)-1H-imidazol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1: 1)

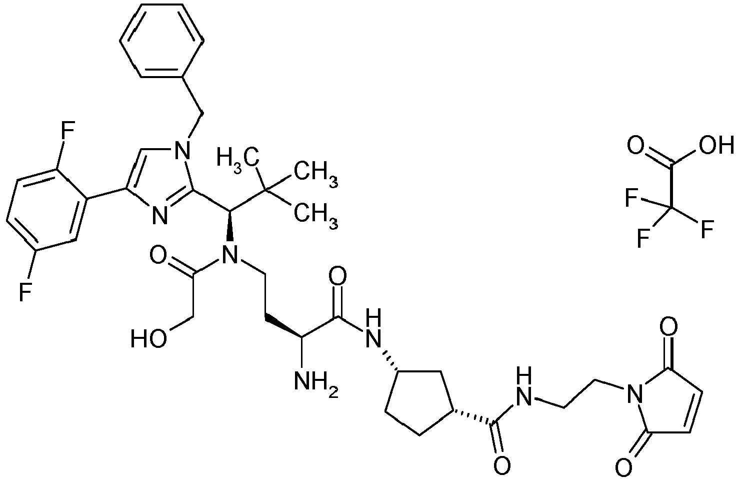

Die Titelverbindung wurde ausgehend von 10 mg (0.015 mmol) Intermediat C5 durch Umsetzung mit 11 mg (0.030 mmol) Intermediat L54 in Gegenwart von 8.4 mg ( 0.022 mmol) HATU sowie 13 µl N,N-Diisopropylethylamin und anschließender Entschützung mit Trifluoressigsäure hergestellt. Es wurden 4 mg (31% d. Th. über 2 Stufen) erhalten.

LC-MS (Methode 1): Rₜ = 0.86 min; MS (EIpos): m/z = 748 [M+H]⁺.

### Intermediat F124

### Trifluoressigsäure--(1R,3S)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5 - dihydro-1H-pyrrol-1-yl)ethyl]cyclopentancarboxamid(1: 1)

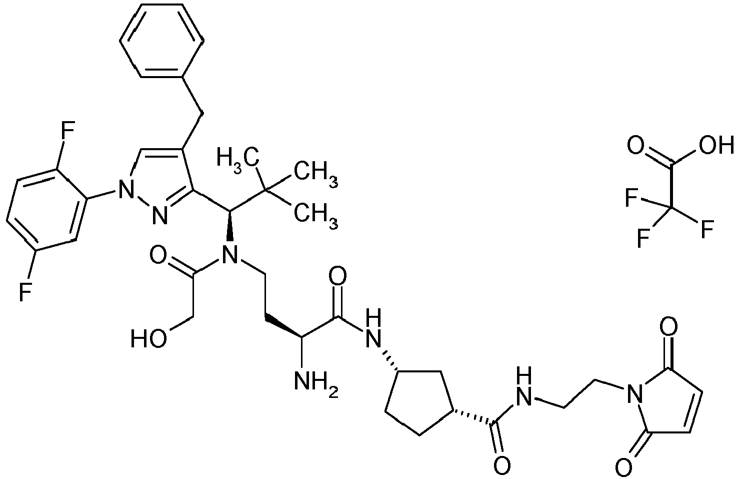

Die Titelverbindung wurde ausgehend von 10 mg (0.016 mmol) Intermediat C49 durch Umsetzung mit 11.5 mg (0.031 mmol) Intermediat L54 in Gegenwart von 9 mg ( 0.024 mmol) HATU sowie 14 µl N,N-Diisopropylethylamin und anschließender Entschützung mit Trifluoressigsäure hergestellt. Es wurden 9 mg (66% d. Th. über 2 Stufen) erhalten.

LC-MS (Methode 1): Rₜ = 0.84 min; MS (EIpos): m/z = 748 [M+H]⁺.

### Intermediat F125

### Trifluoressigsäure--(1R,3S)-3-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

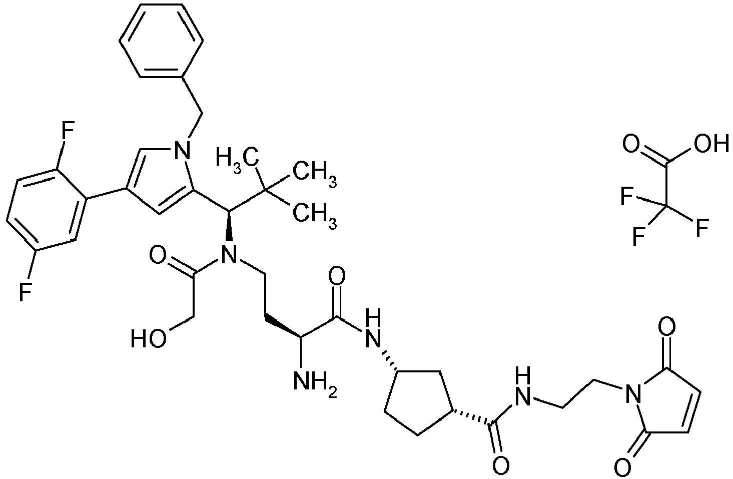

Die Titelverbindung wurde ausgehend von 15 mg (0.019 mmol) Intermediat C53 durch Umsetzung mit 14 mg (0.038 mmol) Intermediat L54 in Gegenwart von 11 mg ( 0.029 mmol) HATU sowie 17 µl N,N-Diisopropylethylamin und anschließender Entschützung mit 127 mg DABCO in 3 ml DMF hergestellt. Nach HPLC-Reinigung wurden 3 mg (17% d. Th. über 2 Stufen) erhalten.

LC-MS (Methode 4): Rₜ = 1.08 min; MS (EIpos): m/z = 769 [M+Na]⁺.

### Intermediat F126

### N-(Bromacetyl)-L-valyl-L-alanyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin -trifluoressigsäure (1:1)

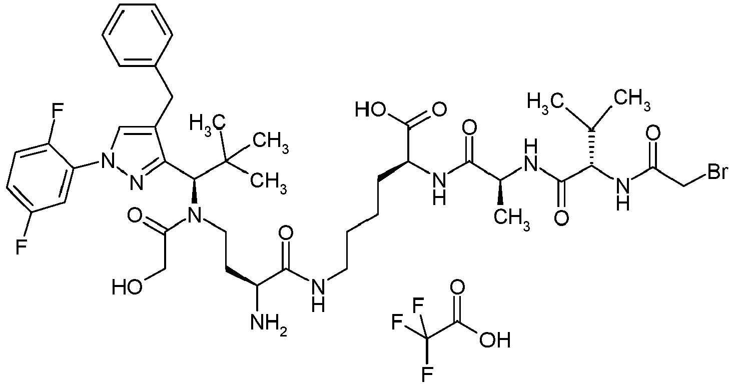

Die Titelverbindung wurde in Analogie zu Intermediat F110 aus 18 mg (0.027 mmol) von Intermediat C49 und 21 mg (0.027 mmol) von Intermediat L49 hergestellt. Es wurden 8.7 mg (30% d. Th. über 2 Stufen) der Titelverbindung erhalten.
HPLC (Methode 11): Rₜ = 1.94 min;
LC-MS (Methode 1): Rₜ = 0.86 min; MS (ESIpos): m/z = 933 und 935 (M+H)⁺.

### Intermediat F127

### Trifluoressigsäure--(2S)-2-amino-4-({(1R)-1-[1-benzyl-4-(2,5-difluorphenyl)-1H-pyrrol-2-yl]-2,2-dimethylpropyl}[(2S)-2-methoxypropanoyl]amino)-N-(2-{[(2,5-dioxo-2,5-dihydro-1H-pyrrol-1-yl)acetyl]amino}ethyl)butanamid (1:1)

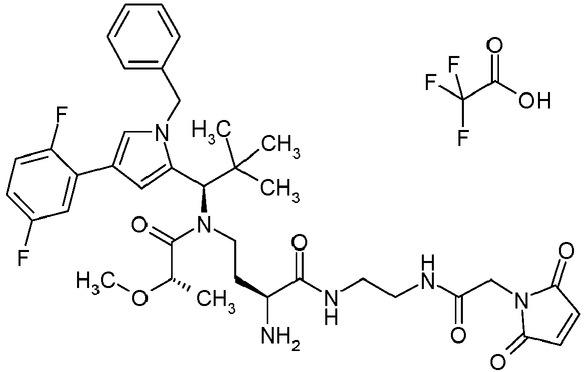

12 mg (0.015 mmol) von Intermediat C59 wurden in 2.4 mL DMF gelöst und mit 14.6 mg (0.046 mmol) von Intermediat L1 sowie mit 6 mg (0.031 mmol) 1-(3-Dimethylaminopropyl)-3-ethyl-carbodiimid-Hydrochlorid, 5.9 mg (0.039 mmol) 1-Hydroxy-1*H*-benzotriazol-Hydrat und 8 µl *N,N-*Diisopropylethylamin versetzt. Nach 1 h Rühren bei RT wurde eingeengt und der Rückstand durch präparative HPLC gereinigt. 11 mg (70% d. Th.) dieser Zwischenstufe wurden erhalten.

LC-MS (Methode 1): Rₜ = 1.34 min; MS (ESIpos): m/z = 942 (M+H)⁺.

11 mg (0.011 mmol) dieses Intermediats wurden in 2 ml DMF aufgenommen und mit 123 mg (1.1 mmol) 1,4-Diazabicyclo(2.2.2)octan versetzt. Der Ansatz wurde 2 h im Ultraschallbad behandelt. Anschließend wurden 63 µl Essigsäure zugegeben und der Ansatz im Hochvakuum eingeengt. Der Rückstand wurde durch präparative HPLC gereinigt. Es wurden 2 mg (22% d. Th.) der Titelverbindung erhalten.

LC-MS (Methode 1): Rₜ = 0.89 min; MS (EIpos): m/z = 721 [M+H]⁺.

HPLC (Methode 11): Rₜ = 1.95 min.

### Intermediat F128

### N⁶-(N-{(2S)-2-Amino-4-[{(1R)-1-[1-benzyl-4-(2,5-difluorphenyl)-1H-imidazol-2-yl]-2,2-dimethylpropyl}(glycoloyl)amino]butanoyl}-D-alanyl)-N²-{N-[6-(2,5-dioxo-2,5-dihydro-1H-pyrrol-1-yl)hexanoyl]-L-valyl-L-alanyl}-L-lysin-trifluoressigsäure (1:1)

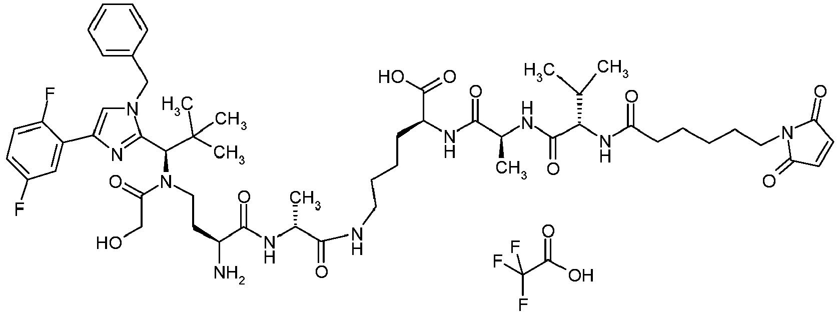

Die Titelverbindung wurde ausgehend von 3 mg (0.005 mmol) Intermediat C5 durch Umsetzung mit 2.5 mg (0.003 mmol) Intermediat L55 in Gegenwart von 2.5 mg ( 0.007 mmol) HATU sowie 3 µl N,N-Diisopropylethylamin und anschließender Entschützung mit Trifluoressigsäure hergestellt. Es wurden 1.4 mg (32% d. Th. über 2 Stufen) erhalten.

LC-MS (Methode 1): Rₜ = 0.93 min; MS (EIpos): m/z = 1077 [M+H]⁺.

### Intermediat F129

### N-[6-(2,5-Dioxo-2,5-dihydro-1H-pyrrol-1-yl)hexanoyl]-L-valyl-L-alanyl-N⁶-{[(1R,3S)-3-({(2S)-2-amino-4-[{(1R)-1-[4-benzyl-1-(2,5-difluorphenyl)-1H-pyrazol-3-yl]-2,2-dimethylpropyl} (glycoloyl)amino]butanoyl}amino)cyclopentyl]carbonyl}-L-lysin-trifluoressigsäure (1:1)

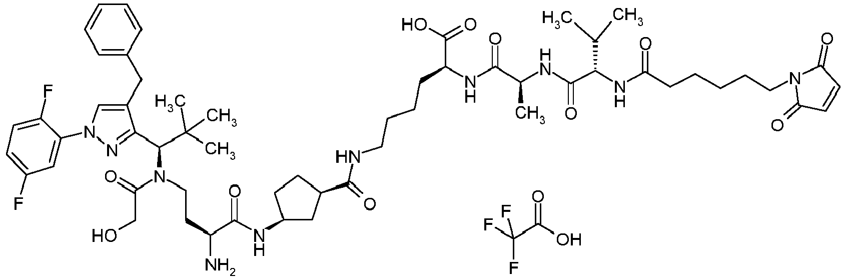

Die Titelverbindung wurde in Analogie zu Intermediat F128 ausgehend von 10 mg (0.016 mmol) Intermediat C49 durch Umsetzung mit 19 mg (0.024 mmol) Intermediat L56 in Gegenwart von 12 mg ( 0.031 mmol) HATU sowie 14 µl N,N-Diisopropylethylamin und anschließender Entschützung mit Trifluoressigsäure hergestellt. Es wurden 13.5 mg (70% d. Th. über 2 Stufen) erhalten.

LC-MS (Methode 1): Rₜ = 0.9 min; MS (EIpos): m/z = 1117 [M+H]⁺.

### Intermediat F142

### R/S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-homocystein-trifluoressigsäure(1:1)

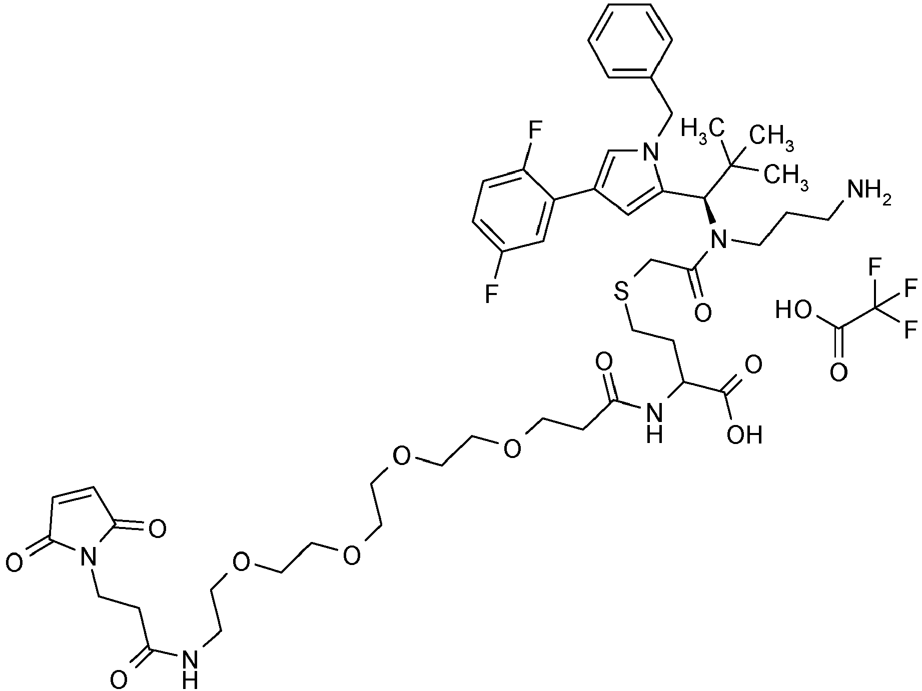

20.0 mg (23.7 µmol) R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-homocystein-trifluoressigsäure(1:1) und 13.4 mg (26.04 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 1.0 mL DMF gelöst und mit 4.8 mg (47.34 µmol) 4-Methylmorpholin versetzt. Die Reaktionsmischung wurde über Nacht bei RT gerührt. Es wurden 3.6 mg (0.06 mmol) Essigsäure zugegeben und die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 12.4 mg (44 % d. Th.) der Verbindung R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-homocystein.

LC-MS (Methode 1): Rₜ = 1.30 min; MS (ESIpos): m/z = 1129 (M+H)⁺.

10.0 mg (8.85 µmol) R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-homocystein wurden in Trifluorethanol gelöst und mit 3.1 mg (22.71 µmol) Zinkdichlorid versetzt. Die Reaktionsmischung rührte über Nacht bei 50 °C. Die Reaktionsmischung wurde mit 3.9 mg (0.01 mmol) Ethylendiamin-N,N,N',N'-tetraessigsäure versetzt, kurz gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand mit etwas Wasser lyophilisiert. Man erhielt 7.6 mg (78 % d. Th.) der Titelverbindung.

LC-MS (Methode 1): Rₜ = 0.94 min; MS (ESIpos): m/z = 983 (M+H)⁺.

¹H-NMR (400 MHz, DMSO-d₆): δ [ppm] = 0.50 (m, 1H), 0.81 (s, 9H), 1.49 (m, 1H), 1.89 (m, 1H), 2.05 (m, 1H), 2.29-2.43 (m, 4H), 2.45-2.55 (m, 2H), 2.58-2.74 (m, 2H), 3.10-3.20 (m, 2H), 3.21-3.40 (m, 2H), 3.42-3.54 (m, 16H), 3.55-3.65 (m, 4H), 4.28 (m, 1H), 4.91 (dd, 1H), 5.18 (dd, 1H), 5.60 (s, 1H), 6.95 (m, 1H), 7.00 (s, 2H), 7.15-7.38 (m, 7H), 7.53 (s, 1H), 7.68 (m, 1H), 8.00 (m, 2H).

### Intermediat F143

### Trifluoressigsäure--6-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-[2-(2,5-dioxo-2,5-dihydro-1H-pyrrol-1-yl)ethyl]hexanamid(1:1)

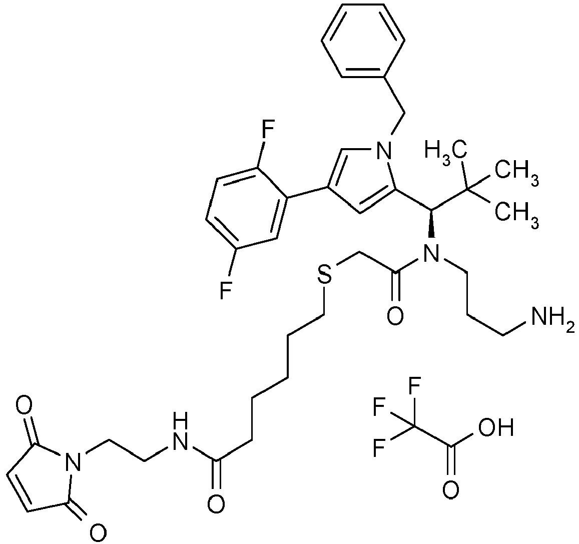

30.0 mg (0.05 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat und 13.5 mg (0.07 mmol) 6-(Acetylsulfanyl)hexansäure wurden in 2.0 mL Methanol mit einem Tropfen Wasser vorgelegt. 23.0 mg (0.17 mmol) Kaliumcarbonat wurden hinzugefügt. Die Reaktionsmischung wurde 4 h bei 50 °C gerührt. Die Reaktionsmischung wurde mit Ethylacetat versetzt. Die organische Phase wurde mit ges. NaCl-Lösung gewaschen und über Magnesiumsulfat getrocknet. Das Lösemittel wurde im Vakuum verdampft. Der Rückstand wurde mittles präp. RP-HPLC gereinigt (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 54.2 mg (90 % d. Th.) der Verbindung 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaicosan-20-säure.

LC-MS (Methode 1): Rₜ = 1.49 min; MS (ESIpos): m/z = 1106 (M+H)⁺.

54.0 mg (0.07 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaicosan-20-säure und 16.7 mg (0.09 mmol) 1-(2-Aminoethyl)-1H-pyrrol-2,5-dionhydrochlorid(1:1) wurden in 3.0 mL Acetonitril vorgelegt und mit 75.0 mg (0.58 mmol) N,N-Diisopropylethylamin versetzt. Es wurden 60.0 mg (0.09 mmol) T3P (50% in Acetonitril) zugegeben und über Nacht bei RT gerührt. Es wurde mit Wasser gequencht und die Reaktionsmischung direkt mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 42.8 mg (68 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl}{[(6-{[2-(2,5-dioxo-2,5-dihydro-1H-pyrrol-1-yl)ethyl]amino}-6-oxohexyl)sulfanyl]acetyl} amino)propyl] carbamat.

LC-MS (Methode 1): Rₜ = 1.48 min; MS (ESIpos): m/z = 866 (M+H)⁺.

20.0 mg (0.02 mmol) 2-(Trimethylsilyl)ethyl-[3-({(1R)-1-[1-benzyl-4-(2,5-difluorphenyl)-1H-pyrrol-2-yl]-2,2-dimethylpropyl}{[(6-{[2-(2,5-dioxo-2,5-dihydro-1H-pyrrol-1-yl)ethyl]amino}-6-oxohexyl)sulfanyl]acetyl}amino)propyl]carbamat wurden in 2.0 ml Trifluorethanol gelöst und mit 4.7 mg (0.04 mmol) Zinkdichlorid versetzt. Die Reaktionsmischung wurde über Nacht bei 50 °C gerührt und danach mit 10.1 mg (0.04 mmol) Ethylendiamin-N,N,N',N'-tetraessigsäure versetzt und 10 min gerührt. Wasser (0.1% TFA) wurde zugesetzt und die Reaktionsmischung mittles präp. RP-HPLC gereinigt (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 9.2 mg (48 % d. Th.) der Titelverbindung.

LC-MS (Methode 1): Rₜ = 0.98 min; MS (ESIpos): m/z = 722 (M+H)⁺.

### Intermediat F144

### Trifluoressigsäure--N-(3-aminopropyl)-N-{(1R)-1-[1-benzyl-4-(2,5-difluorphenyl)-1H-imidazol-2-yl]-2,2-dimethylpropyl}-6-(2,5-dioxo-2,5-dihydro-1H-pyrrol-1-yl)hexanamid(1:1)

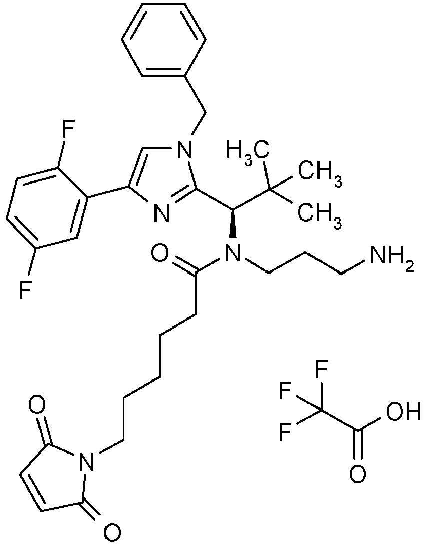

50.0 mg (0.1 mmol) tert-Butyl-[3-({(1R)-1-[1-benzyl-4-(2,5-difluorphenyl)-1H-imidazol-2-yl]-2,2-dimethylpropyl}amino)propyl]carbamat (Intermediat C15) wurden in 2.0 mL Dichlormethan vorgelegt und mit 22.7 mg (0.22 mmol) Triethylamin und 49.3 mg (0.22 mmol) 6-(2,5-Dioxo-2,5-dihydro-1H-pyrrol-1-yl)hexanoylchlorid (Intermediat L60) WISV1648-1-1 versetzt.

Die Reaktionsmischung wurde über Nacht bei RT gerührt. Es wurde dann alle 2 h dreimal 1 Äquivalent Intermediat L60 und 1.2 Äquivalente Triethylamin zugesetzt und anschließend über Nacht bei RT gerührt. Dieser Vorgang wurde noch zweimal wiederholt. Das Lösemittel wurde im Vakuum entfernt und der Rückstand mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 30.9 mg (43 % d. Th.) der Verbindung tert-Butyl-[3-({(1R)-1-[1-benzyl-4-(2,5-difluorphenyl)-1H-imidazol-2-yl]-2,2-dimethylpropyl}[6-(2,5-dioxo-2,5-dihydro-1H-pyrrol-1-yl)hexanoyl]amino)propyl]carbamat

LC-MS (Methode 1): Rₜ = 1.51 min; MS (ESIpos): m/z = 706 (M+H)⁺.

24.6 mg (0.04 mmol) tert-Butyl-[3-({(1R)-1-[1-benzyl-4-(2,5-difluorphenyl)-1H-imidazol-2-yl]-2,2-dimethylpropyl} [6-(2,5-dioxo-2,5-dihydro-1H-pyrrol-1-yl)hexanoyl]amino)propyl]carbamat wurden in 3.0 mL Dichlormethan gelöst und mit 79.5 mg (0.7 mmol) TFA versetzt und 6 h bei RT gerührt. Es wurden nochmals 79.5 mg (0.7 mmol) TFA zugesetzt und über Nacht bei RT gerührt. Das Lösemittel wurde im Vakuum entfernt und der Rückstand dreimal mit Dichlormethan kodestilliert. Der Rückstand wurde mittles präp. RP-HPLC gereinigt (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 24.2 mg (97 % d. Th.) der Titelverbindung. LC-MS (Methode 1): Rₜ = 1.02 min; MS (ESIpos): m/z = 606 (M+H)⁺.

### Intermediat F145

### Trifluoressigsäure--6-({2-[(3-Aminopropyl){(1R)-1-[1-benzyl-4-(2,5-difluorphenyl)-1H-imidazol-2-yl] -2,2-dimethylpropyl} amino] -2-oxoethyl} sulfanyl)-N-[2-(2,5 -dioxo-2,5 -dihydro- 1H-pyrrol-1-yl)ethyl]hexanamid

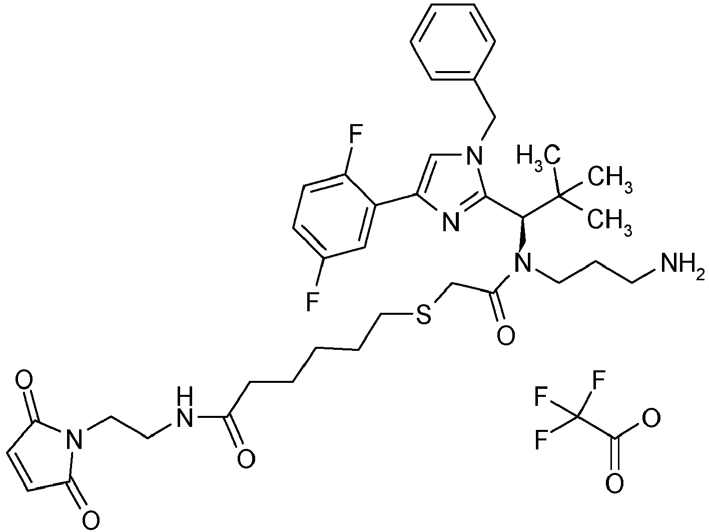

90.0 mg (0.15 mmol) tert-Butyl-{3-[{(1R)-1-[1-benzyl-4-(2,5-difluorphenyl)-1H-imidazol-2-yl]-2,2-dimethylpropyl}(chloracetyl)amino]propyl}carbamat Intermediat C16 und 43.6 mg (0.23 mmol) 6-(Acetylsulfanyl)hexansäure wurden in 9.0 mL Methanol mit einem Tropfen Wasser vorgelegt und mit 73.9 mg (0.54 mmol) Kaliumcarbonat versetzt. Die Reaktionsmischung wurde 4 h bei RT gerührt und anschließend mit Ethylacetat versetzt. Die organische Phase wurde mit Wasser/ges. NaCl-Lösung und mit ges. NaCl-Lösung gewaschen. Die organische Phase wurde über Magnesiumsulfat getrocknet und die Lösemittel im Vakuum verdampft. Der Rückstand wurde mittels Kieselgel (Laufmittel: Dichlormethan/Methanol 100:2) gereinigt. Die Lösemittel wurden im Vakuum verdampft und der Rückstand im Hochvakuum getrocknet. Man erhielt 98.7 mg (83 % d. Th.) der Verbindung 9-{(1R)-1-[1-Benzyl-4-(2,5-difluorphenyl)-1H-imidazol-2-yl]-2,2-dimethylpropyl}-2,2-dimethyl-4,10-dioxo-3-oxa-12-thia-5,9-diazaoctadecan-18-säure.

LC-MS (Methode 1): Rₜ = 1.44 min; MS (ESIpos): m/z = 701 (M+H)⁺.

20.0 mg (0.03 mmol) 9-{(1R)-1-[1-Benzyl-4-(2,5-difluorphenyl)-1H-imidazol-2-yl]-2,2-dimethylpropyl}-2,2-dimethyl-4,10-dioxo-3-oxa-12-thia-5,9-diazaoctadecan-18-säure und 6.5 (0.04 mmol) 1-(2-Aminoethyl)-1H-pyrrol-2,5-dionhydrochlorid(1:1) wurden in 1.5 mL Acetonitril vorgelegt und mit 23.6 mg (0.04 mmol) T3P sowie mit 29.5 mg (0.23 mmol) N,N-Diisopropylethylamin versetzt. Die Reaktionsmischung wurde über Nacht bei RT gerührt und anschließend mit Wasser versetzt. Die Reaktionsmischung wurde mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 16.7 mg (99 % d. Th.) der Verbindung tert-Butyl-[3-({(1R)-1-[1-benzyl-4-(2,5-difluorphenyl)-1H-imidazol-2-yl]-2,2-dimethylpropyl}{[(6-{[2-(2,5-dioxo-2,5-dihydro-1H-pyrrol-1-yl)ethyl]amino}-6-oxohexyl)sulfanyl]acetyl} amino)propyl] carbamat.

LC-MS (Methode 1): Rₜ = 1.40 min; MS (ESIpos): m/z = 823 (M+H)⁺.

14.8 mg (0.02 mmol) tert-Butyl-[3-({(1R)-1-[1-benzyl-4-(2,5-difluorphenyl)-1H-imidazol-2-yl]-2,2-dimethylpropyl}{[(6-{[2-(2,5-dioxo-2,5-dihydro-1H-pyrrol-1-yl)ethyl]amino}-6-oxohexyl)sulfanyl]acetyl}amino)propyl]carbamat wurden in 1.5 mL Dichlormethan gelöst und mit 41.0 mg (0.36 mmol) TFA versetzt. Die Reaktionsmischung wurde über Nacht bei RT gerührt. Dann wurden noch jeweils zweimal 41.0 mg (0.36 mmol) TFA hinzugefügt und über Nacht bei RT gerührt. Das Lösemittel wurde im Vakuum verdampft und der Rückstand mittles präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand wurde in 1,4-Dioxan und Wasser aufgenommen und lyophilisiert. Man erhielt 2.9 mg (19 % d. Th.) der Titelverbindung.

LC-MS (Methode 1): Rₜ = 0.93 min; MS (ESIpos): m/z = 723 (M+H)⁺.

### Intermediat F146

### R/S-[2-([(3S)-3-Amino-3-carboxypropyl]{(1R)-1-[1-benzyl-4-(2,5-difluorphenyl)-1H-pyrrol-2-yl]-2,2-dimethylpropyl}amino)-2-oxoethyl]-N-[19-(2,5-dioxo-2,5-dihydro-1H-pyrrol-1-yl)-17-oxo-4,7,10,13-tetraoxa-16-azanonadecan-1-oyl]homocystein-trifluoressigsäure(1:1)

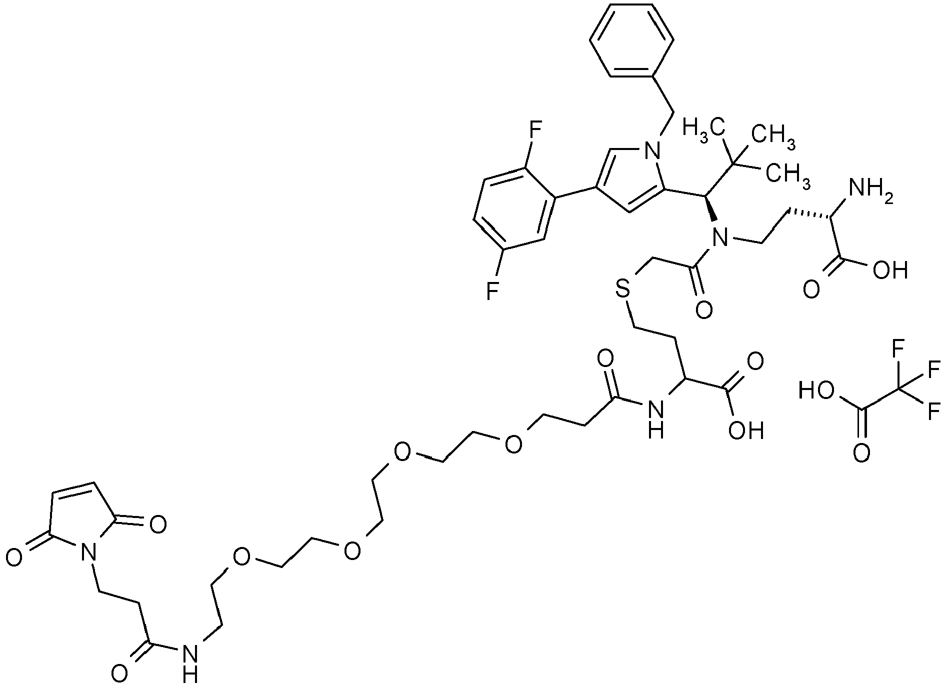

25.0 mg (28.12 µmol) R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]homocystein (Intermediat C12) und 15.9 mg (30.93 µmol) 3-(2,5-Dioxo-2,5-dihydro-1H-pyrrol-1-yl)-N-{15-[(2,5-dioxopyrrolidin-1-yl)oxyl-15-oxo-3,6,9,12-tetraoxapentadec-1-yl}propanamid wurden in 2.0 mL DMF gelöst und mit 11.4 mg (112.48 µmol) 4-Methylmorpholin versetzt. Die Reaktionsmischung wurde über Nacht bei RT gerührt. Es wurden 7.6 mg (0.13 mmol) Essigsäure zugegeben und die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 23.9 mg (59 % d. Th.) der Verbindung R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]-N-[19-(2,5-dioxo-2,5-dihydro-1H-pyrrol-1-yl)-17-oxo-4,7,10,13-tetraoxa-16-azanonadecan-1-oyl]homocystein.

LC-MS (Methode 1): Rₜ = 1.26 min; MS (ESIpos): m/z = 1173 (M+H)⁺.

11.8 mg (8.23 µmol) R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]-N-[19-(2,5-dioxo-2,5-dihydro-1H-pyrrol-1-yl)-17-oxo-4,7,10,13-tetraoxa-16-azanonadecan-1-oyl]homocystein wurden in Trifluorethanol gelöst und mit 1.7 mg (12.35 µmol) Zinkdichlorid versetzt. Die Reaktionsmischung rührte über Nacht bei 50 °C. Die Reaktionsmischung wurde mit 3.6 mg (0.01 mmol) Ethylendiamin-N,N,N',N'-tetraessigsäure versetzt, kurz gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.8 mg (62 % d. Th.) der Titelverbindung.

LC-MS (Methode 4): Rₜ = 1.20 min; MS (ESIpos): m/z = 1029 (M+H)⁺.

### Intermediat F147

### Trifluoressigsäure--N-(3-Aminopropyl)-N-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-10-oxo-3,6-dioxa-16-thia-9-azaoctadecan-18-amid(1:1)

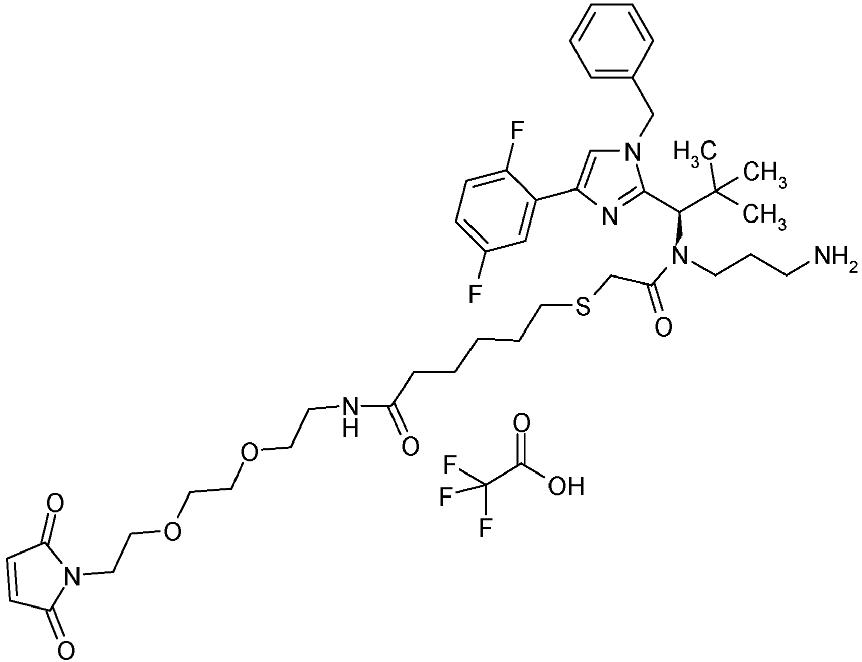

15.0 mg (0.03 mmol) 9-{(1R)-1-[1-Benzyl-4-(2,5-difluorphenyl)-1H-imidazol-2-yl]-2,2-dimethylpropyl}-2,2-dimethyl-4,10-dioxo-3-oxa-12-thia-5,9-diazaoctadecan-18-säure (Intermediat C13) wurden in 1.5 mL Acetonitril vorgelegt und mit 22.1 mg (0.17 mmol) N,N-Diisopropylethylamin und dann mit 17.7 mg (0.03 mmol) T3P versetzt. Es wurde 5 min bei RT gerührt und dann 9.5 mg (0.03 mmol) Trifluoressigsäure--1-{2-[2-(2-aminoethoxy)ethoxy]ethyl}-1H-pyrrol-2,5-dion(1:1) (Intermediat L59) zugegeben. Die Reaktionsmischung rührte über Nacht bei RT und wurde mit Wasser gequencht. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 14.8 mg (57 % d. Th.) der Verbindung tert-Butyl-[19-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-10,18-dioxo-3,6-dioxa-16-thia-9,19-diazadocosan-22-yl]carbamat.

LC-MS (Methode 1): Rₜ = 1.40 min; MS (ESIpos): m/z = 911 (M+H)⁺.

14.2 mg (0.02 mmol) tert-Butyl-[19-{(1R)-1-[1-benzyl-4-(2,5-difluorphenyl)-1H-imidazol-2-yl]-2,2-dimethylpropyl}-1-(2,5-dioxo-2,5-dihydro-1H-pyrrol-1-yl)-10,18-dioxo-3,6-dioxa-16-thia-9,19-diazadocosan-22-yl]carbamat wurden in 1.5 mL Dichlormethan gelöst und mit 35.5 mg (0.31 mmol) TFA versetzt und bei RT über Nacht gerührt. Es wurden nochmals 71.0 mg (0.62 mmol) TFA zugesetzt und über Nacht bei RT gerührt. Die Lösemittel wurden im Vakuum verdampft und der Rückstand mittles präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand in wenig Wasser aufgenommen und lyophilisiert. Man erhielt 14.0 mg (97 % d. Th.) der Titelverbindung.

LC-MS (Methode 1): Rₜ = 1.01 min; MS (ESIpos): m/z = 811 (M+H)⁺.

### Intermediat F148

### Trifluoressigsäure--6-({2-[(3-aminopropyl){(1R)-1-[1-benzyl-4-(2,5-difluorphenyl)-1H-imidazol-2-yl] -2,2-dimethylpropyl} amino] -2-oxoethyl} sulfinyl)-N-[2-(2,5 -dioxo-2,5 -dihydro- 1H-pyrrol-1-yl)ethyl]hexanamid(1:1)

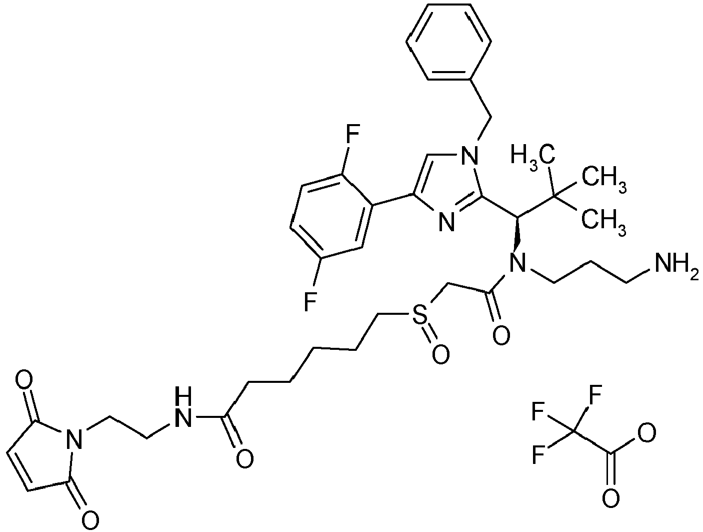

Die Titelverbindung entstand als Nebenprodukt bei der Synthese von Intermediat F145. Man erhielt 8.1 mg (53 % d. Th.) der Titelverbindung.

LC-MS (Methode 1): Rₜ = 1.25 min; MS (ESIpos): m/z = 739 [M+H]⁺.

### Intermediat F149

### Trifluoressigsäure--R/S-{2-[(3-aminopropyl){(1R)-1-[1-benzyl-4-(2,5-difluorphenyl)-1H-imidazol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]homocystein(1:1)

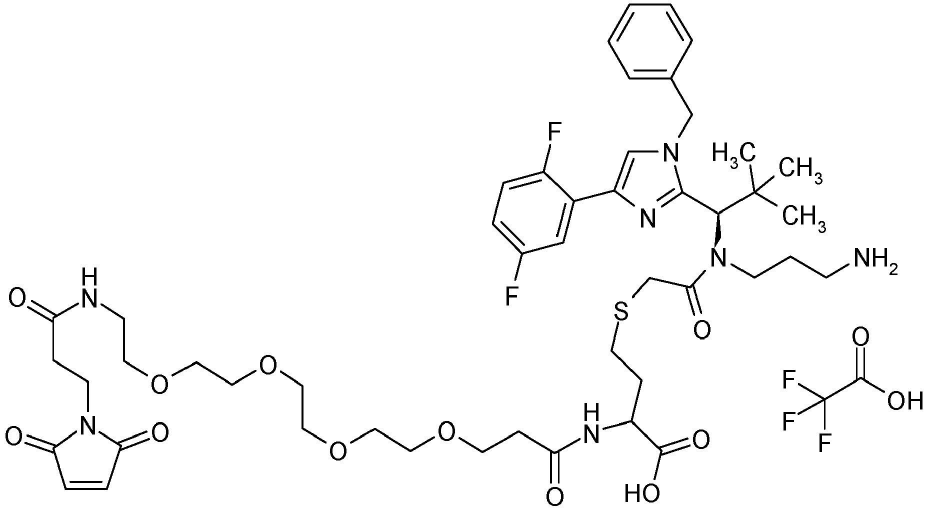

20.0 mg (24.94 µmol) R/S-[2-({(1R)-1-[1-Benzyl-4-(2,5-difluorphenyl)-1H-imidazol-2-yl]-2,2-dimethylpropyl} { 3 - [(tert-butoxycarbonyl)amino]propyl} amino) -2-oxoethyl] homocy stein (Intermediat C14) und 14.1 mg (27.44 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 1.0 mL DMF vorgelegt und mit 5.1 mg (49.88 µmol) 4-Mehylmorpholin versetzt. Die Reaktionsmischung rührte über Nacht. Es wurden 3.7 mg (0.06 mmol) Essigsäure zugegeben und die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 18.2 mg (67 % d. Th.) der Verbindung R/S-[2-({(1R)-1-[1-Benzyl-4-(2,5-difluorphenyl)-1H-imidazol-2-yl]-2,2-dimethylpropyl}{3-[(tert-butoxycarbonyl)amino]propyl}amino)-2-oxoethyl]-N-[19-(2,5-dioxo-2,5-dihydro-1H-pyrrol-1-yl)-17-oxo-4,7,10,13-tetraoxa-16-azanonadecan-1-oyl]homocystein.

LC-MS (Methode 1): Rₜ = 1.23 min; MS (ESIpos): m/z = 1086 (M+H)⁺.

17.6 mg (0.02 mmol) R/S-[2-({(1R)-1-[1-Benzyl-4-(2,5-difluorphenyl)-1H-imidazol-2-yl]-2,2-dimethylpropyl}{3-[(tert-butoxycarbonyl)amino]propyl}amino)-2-oxoethyl]-N-[19-(2,5-dioxo-2,5-dihydro-1H-pyrrol-1-yl)-17-oxo-4,7,10,13-tetraoxa-16-azanonadecan-1-oyl]homocystein wurden in 1.5 mL Dichlormethan gelöst und mit 37.0 mg (0.32 mmol) TFA versetzt und bei RT über Nacht gerührt. Es wurden nochmals 74.0 mg (0.64 mmol) TFA zugesetzt und über Nacht bei RT gerührt. Die Lösemittel wurden im Vakuum verdampft und der Rückstand mittles präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand in wenig Wasser aufgenommen und lyophilisiert. Man erhielt 16.0 mg (90 % d. Th.) der Titelverbindung.

LC-MS (Methode 1): Rₜ = 0.92 min; MS (ESIpos): m/z = 986 (M+H)⁺.

### Intermediat F150

### Trifluoressigsäure--N-[31-(2,5-dioxo-2,5-dihydro-1H-pyrrol-1-yl)-29-oxo-4,7,10,13,16,19,22,25-octaoxa-28-azahentriacontan-1-oyl]-L-valyl-N-[2-({2-[(3-aminopropyl){(1R)-1-[1-benzyl-4-(2,5-difluorphenyl)-1H-imidazol-2-yl]-2,2-dimethylpropyl}amino]-2-oxoethyl}sulfanyl)ethyl]-L-alaninamid (1:1)

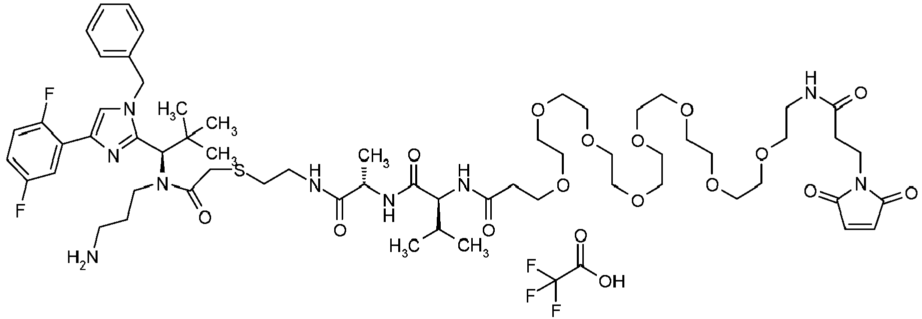

Unter Argon wurden bei 0 °C 10.0 mg (0.02 mmol) Trifluoressigsäure--tert-Butyl-[3-({[(2-aminoethyl)sulfanyl]acetyl}{(1R)-1-[1-benzyl-4-(2,5-difluorphenyl)-1H-imidazol-2-yl]-2,2-dimethylpropyl}amino)propyl]carbamat (Intermediat C20) in 1.0 mL DMF mit 12.1 mg (0.02 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-L-alanin (Intermediat L25), 2.2 mg (0.02 mmol) HOAt und 7.6 mg (0.02 mmol) HATU versetzt. Dann wurden 5.5 µL (0.03 mmol) N,N-Diisopropylethylamin zugegeben und über Nacht bei RT nachgerührt. Die Reaktionsmischung wurde mit 1.8 µL HOAc versetzt und direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 10.4 mg (48 % d. Th.) der Verbindung N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-(9-{(1R)-1-[1-benzyl-4-(2,5-difluorphenyl)-1H-imidazol-2-yl]-2,2-dimethylpropyl}-2,2-dimethyl-4,10-dioxo-3-oxa-12-thia-5,9-diazatetradecan-14-yl)-L-alaninamid.

LC-MS (Methode 4): Rₜ = 1.60 min; MS (ESIpos): m/z = 687.5 [M+2H]²⁺.

9.5 mg (0.01 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-(9-{(1R)-1-[1-benzyl-4-(2,5-difluorphenyl)-1H-imidazol-2-yl]-2,2-dimethylpropyl}-2,2-dimethyl-4,10-dioxo-3-oxa-12-thia-5,9-diazatetradecan-14-yl)-L-alaninamid wurden in 1.0 mL Dichlormethan vorgelegt und mit 15.8 mg (0.14 mmol) TFA versetzt und über Nacht gerührt. Es wurden nochmals 31.6 mg (0.28 mmol) TFA zugegeben und über Nacht gerührt. Das Lösemittel wurde im Vakuum verdampft und der Rückstand wurde mittels präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand in wenig Wasser aufgenommen und lyophilisiert. Man erhielt 10.2 mg (98 % d. Th.) der Titelverbindung.

LC-MS (Methode 4): Rₜ = 1.13 min; MS (ESIpos): m/z = 637.5 [M+2H]²⁺.

### Intermediat F151

### N-[19-(2,5 -Dioxo-2,5-dihydro-1H-pyrrol-1-yl)-17-oxo-4,7,10,13-tetraoxa-16-azanonadecan-1-oyl]-L-valyl-N-(3-{(2S)-5-(2,5-difluorphenyl)-3-[methoxy(methyl)carbamoyl]-2-phenyl-2,3-dihydro-1,3,4-thiadiazol-2-yl}propyl)-L-alaninamid

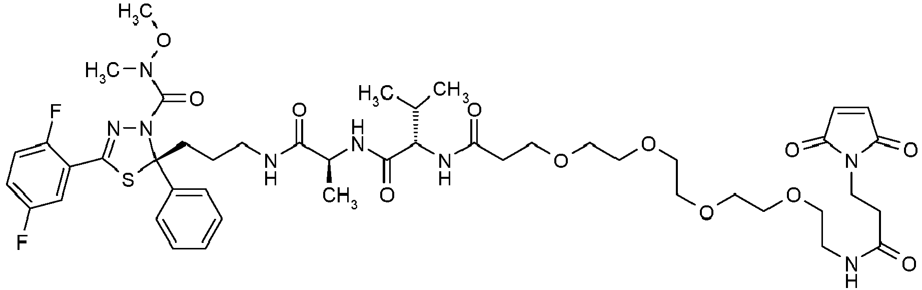

5.0 mg (0.01 mmol) (2S)-2-(3-Aminopropyl)-5-(2,5-difluorphenyl)-N-methoxy-N-methyl-2-phenyl-1,3,4-thiadiazol-3(2H)-carboxamid wurden in 1.0 mL Acetonitril vorgelegt und mit 7.7 mg (0.06 mmol) N,N-Diisopropylethylamin sowie 9.8 (0.02 mmol) T3P versetzt. Es wurde 5 min bei RT gerührt und dann wurden 9.1 mg (0.02 mmol) N-[19-(2,5-Dioxo-2,5-dihydro-1H-pyrrol-1-yl)-17-oxo-4,7,10,13-tetraoxa-16-azanonadecan-1-oyl]-L-valyl-L-alanin (Intermediat L44) zugegeben. Die Reaktionsmischung wurde über Nacht bei RT gerührt. Es wurde Wasser zugegeben und die Reaktionsmischung direkt mittels präp. RP-HPLC gereinigt (Säule: Reprosil 250x40; 10µ, Fluss: 50 mL/min, MeCN/Wasser, 0.1% TFA). Die Lösemittel wurden im Vakuum verdampft und der Rückstand im Hochvakuum getrocknet. Man erhielt 4.3 mg (35 % d. Th.) der Titelverbindung.

LC-MS (Methode 1): Rₜ = 1.02 min; MS (ESIpos): m/z = 989 [M+H]⁺.

### Intermediat F152

### N-[31-(2,5-Dioxo-2,5-dihydro-1H-pyrrol-1-yl)-29-oxo-4,7,10,13,16,19,22,25-octaoxa-28-azahentriacontan-1 -oyl] -L-valyl-N-(3 - {(2 S) -5 -(2,5 -difluorphenyl)-3 - [methoxy (methyl)carbamoyl] - 2-phenyl-2,3-dihydro-1,3,4-thiadiazol-2-yl}propyl)-L-alaninamid

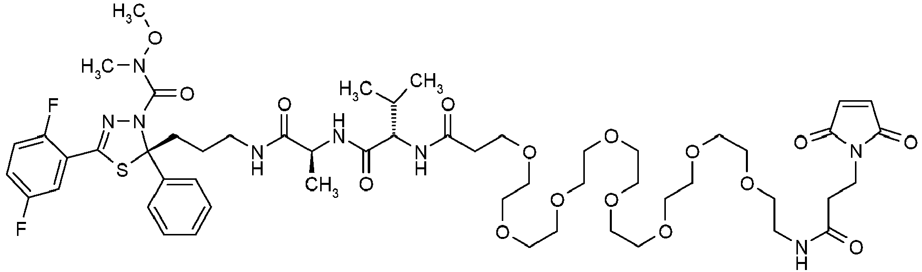

5.0 mg (0.01 mmol) (2S)-2-(3-Aminopropyl)-5-(2,5-difluorphenyl)-N-methoxy-N-methyl-2-phenyl-1,3,4-thiadiazol-3(2H)-carboxamid wurden in 1.0 mL Acetonitril vorgelegt und mit 7.7 mg (0.06 mmol) N,N-Diisopropylethylamin sowie 9.8 (0.02 mmol) T3P versetzt. Es wurde 5 min bei RT gerührt und dann wurden 11.8 mg (0.02 mmol) N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-L-alanin (Intermediat L25) zugegeben. Die Reaktionsmischung wurde über Nacht bei RT gerührt. Es wurde Wasser zugegeben und die Reaktionsmischung direkt mittels präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 4.7 mg (34 % d. Th.) der Titelverbindung.

LC-MS (Methode 4): Rₜ = 1.34 min; MS (ESIpos): m/z = 1165 [M+H]⁺.

### Intermediat F153

### Trifluoressigsäure --(2S)-2-amino-4-({(1R)-1-[1-benzyl-4-(2,5-difluorphenyl)-1H-pyrrol-2-yl]-2,2-dimethylpropyl}[(2S)-2-hydroxypropanoyl]amino)-N-(2-{[(2,5-dioxo-2,5-dihydro-1H-pyrrol-1-yl)acetyl]amino }ethyl)butanamid (1:1)

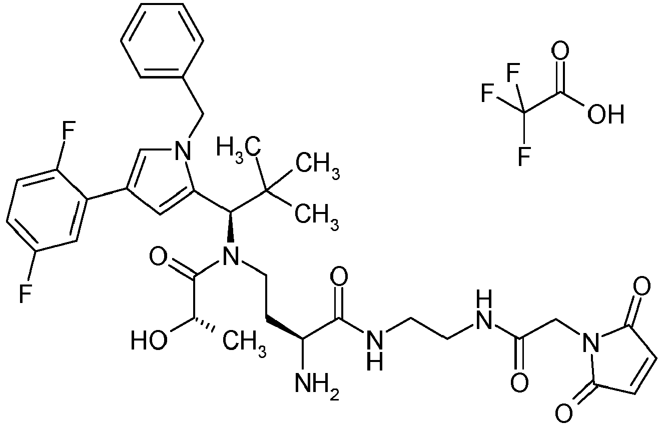

Die Synthese erfolgte in Analogie zu Intermediat F104 ausgehend von Intermediat C60.

LC-MS (Methode 1): Rₜ = 1.1 min; MS (ESIpos): m/z = 707 (M+H)⁺.

### Intermediat F154

### N⁶-(N-{(2S)-2-Amino-4-[{(1R)-1-[1-benzyl-4-(2,5-difluorphenyl)-1H-imidazol-2-yl]-2,2-dimethylpropyl}(glycoloyl)amino]butanoyl}-beta-alanyl)-N²-{N-[6-(2,5-dioxo-2,5-dihydro-1H-pyrrol-1-yl)hexanoyl]-L-valyl-L-alanyl}-L-lysin -trifluoressigsäure (1:1)

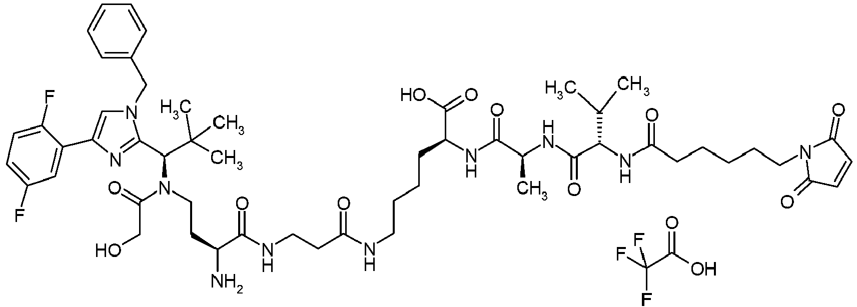

Die Titelverbindung wurde in Analogie zu Intermediat F2 aus 10 mg (0.015 mmol) Intermediat C8 und 15 mg (0.022 mmol) Intermediat L6 hergestellt.
HPLC (Methode 11): Rₜ = 1.91 min;
LC-MS (Methode 1): Rₜ = 0.89 min; MS (ESIpos): m/z = 1077 (M+H)⁺.

### Intermediat F155

### N⁶-(N-{(2S)-2-Amino-4-[{(-1R)-1-[1-benzyl-4-(2,5-difluorphenyl)-1H-pyrrol-2-yl]-2,2 dimethylpropyl}(glycoloyl)amino]butanoyl}-beta-alanyl)-N²-{N-[6-(2,5-dioxo-2,5-dihydro-1H-pyrrol-1-yl)hexanoyl]-L-valyl-L-alanyl}-L-lysin -trifluoressigsäure (1:1)

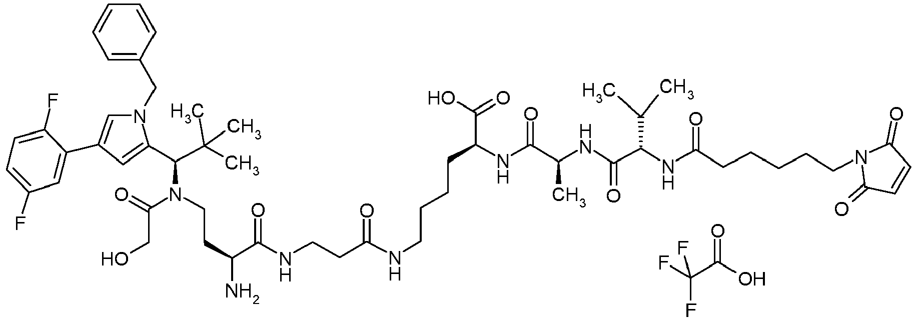

Die Titelverbindung wurde durch Kupplung von 14 mg (0.019 mmol) des Intermediats C61 mit 15 mg (0.021 mmol) von Intermediat L61 in Gegenwart von 8.7 mg (0.023 mmol) HATU sowie 17 µl N,N-Diisopropylethylamin und anschließender Entschützung mit Zinkchlorid in Trifluorethanol wie in Intermediat F119 beschrieben hergestellt. Nach Reinigung durch präparative HPLC wurden 13 mg (59% d. Th. über 2 Stufen) der Titelverbindung erhalten.

LC-MS (Methode 1): Rₜ = 0.86 min; MS (ESIpos): m/z = 1076 (M+H)⁺.

### Intermediat F156

### N-(Bromacetyl)-L-valyl-L-alanyl-N⁶-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-L-lysin -trifluoressigsäure (1:1)

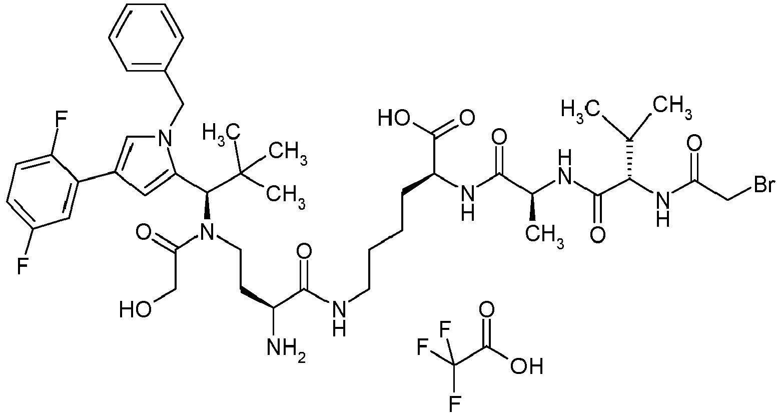

Zunächst wurde ausgehend von N2-[(Benzyloxy)carbonyl]-N6-(tert-butoxycarbonyl)-L-lysin nach klassischen Methoden der Peptidchemie (Veresterung mit 2-(Trimethylsilylethanol mittels EDCI/DMAP, Hydrogenolyse, Kupplung mit N-[(Benzyloxy)carbonyl]-L-valyl-L-alanin in Gegenwart von HATU und erneute Hydrogenolyse) das Tripeptidderivat 2-Trimethylsilyl)ethyl-L-valyl-L-alanyl-N6-(tert-butoxycarbonyl)-L-lysinat hergestellt.

84mg (0.163 mmol) von diesem Intermediat wurden in 2.5 ml DMF aufgenommen und mit 58 mg (0.244 mmol) 1-(2-Bromacetoxy)pyrrolidin-2,5-dion versetzt. Nach 10min Rühren bei RT wurde eingeengt, der Rückstand in Acetonitril/Wasser 1: 1 aufgenommen und mit Trifluoressigsäure auf pH 2 gebracht und durch präparative HPLC gereinigt. Nach Einengen der entsprechenden Fraktionen wurde der Rückstand in 15 ml einer 5%-igen Trifluoressigsäurelösung in DCM aufgenommen und 2 h bei RT gerührt. Anschließend wurde bei leichter Kühlung eingeengt und der Rückstand aus Acetonitril/Wasser 1:1 lyophilisiert. Von diesem Intermediat wurden 53mg (50% d. Th.) über 2 Stufen erhalten.

LC-MS (Methode 1): Rₜ = 0.72 min; MS (ESIpos): m/z = 537 und 539 (M+H)⁺.

Zur Synthese der Titelverbindung wurden 18 mg (0.027 mmol) von diesem Intermediat in 4 ml DMF aufgenommen, mit 16 mg (0.025 mmol) Intermediat C61 sowie mit 19 mg HATU und 9 µL N,N-Diisopropylethylamin versetzt. Nach 5 min Rühren bei RT wurden einige Tropfen Trifluoressigsäure zugegeben und der Ansatz mittels präparativer HPLCC gereinigt. Nach Einengen der entsprechenden Fraktionen und Lyophilsation aus Acetonitril/Wasser 1: 1 wurde das erhaltene Intermediat in 3 mL 2,2,2-Trifluorethanol gelöst. Nach Zugabe von 4.8 mg (0.035 mmol) Zinkchlorid wurde der Ansatz 2.5 h bei 50°C gerührt. Dann wurden 10 mg (0.035 mmol) Ethylendiamin-N,N,N',N'-tetraessigsäure hinzugegeben und der Ansatz mit Acetonitril/Wasser verdünnt und filtriert. Die Reinigung erfolgte mittels präparativer HPLC. Nach Einengen der entsprechenden Fraktionen und Lyophilisation des Rückstands aus Acetonitril/Wasser wurden 3.2 mg (13% d.Th.) der Titelverbindung über 2 Stufen erhalten.
HPLC (Methode 11): Rₜ = 1.94 min;
LC-MS (Methode 5): Rₜ = 2.79 min; MS (ESIpos): m/z = 932 und 934 (M+H)⁺.

### Intermediat F163

### N-[6-(2,5-Dioxo-2,5-dihydro-1H-pyrrol-1-yl)hexanoyl]-L-valyl-L-alanyl-N⁶-{(2S)-2-amino-4-[{(1R)-1-[1-benzyl-4-(2,5-difluorphenyl)-1H-pyrrol-2-yl]-2,2-dimethylpropyl}(glycoloyl)amino] butanoyl}-L-lysin -trifluoressigsäure (1:1)

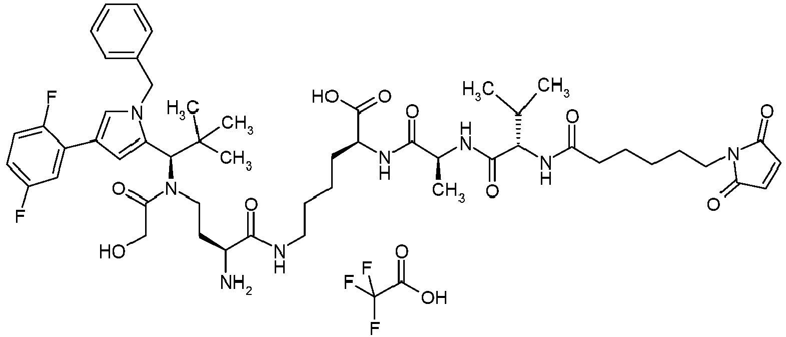

Die Titelverbindung wurde durch Kupplung von 37 mg (0.056 mmol) Intermediat C58 und 41 mg (0.056 mmol) Intermediat L61 in Gegenwart von HATU und anschließende Deblockierung mit Zinkchlorid wie in Intermediat F119 beschrieben, hergestellt. Es wurden 12 mg (19% d.Th. über 2 Stufen) der Titelverbindung erhalten.
HPLC (Methode 11): Rₜ = 1.49 min;
LC-MS (Methode 1): Rₜ = 0.89 min; MS (ESIpos): m/z = 1005 (M+H)⁺.

### Intermediat F164

### N-[6-(2,5-Dioxo-2,5-dihydro-1H-pyrrol-1-yl)hexanoyl]-L-valyl-N⁵-carbamoyl-L-ornithyl-N⁶-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-L-lysin-trifluoressigsäure (1:1)

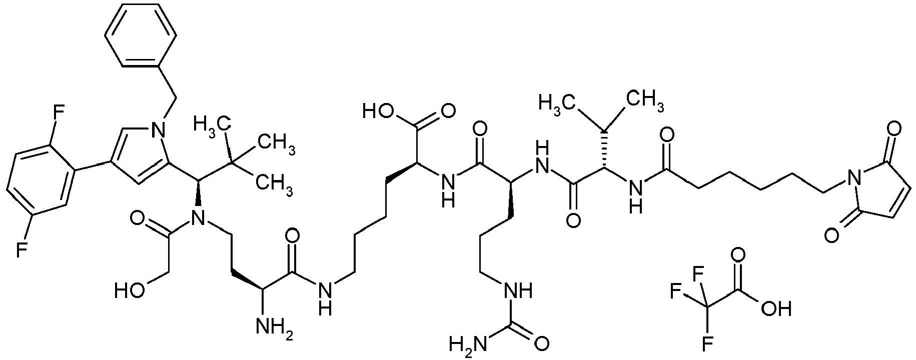

Die Titelverbindung wurde in Analogie zu Intermediat F155 durch Kupplung von 20 mg (0.030 mmol) Intermediat C58 mit 27 mg (0.033 mmol) von Intermediat L62 in Gegenwart von HATU und *N,N-*Diisopropylethylamin und anschließender Entschützung mittels Zinkchlorid in Trifluorethanol hergestellt.
HPLC (Methode 11): Rₜ = 1.92 min;
LC-MS (Methode 1): Rₜ = 0.87 min; MS (ESIpos): m/z = 1091 (M+H)⁺.

### Intermediat F165

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[6-(2,5-dioxo-2,5-dihydro-1H-pyrrol-1-yl)hexanoyl]-L-valyl-N⁵-carbamoyl-L-ornithyl}-L-lysin-trifluoressigsäure (1:1)

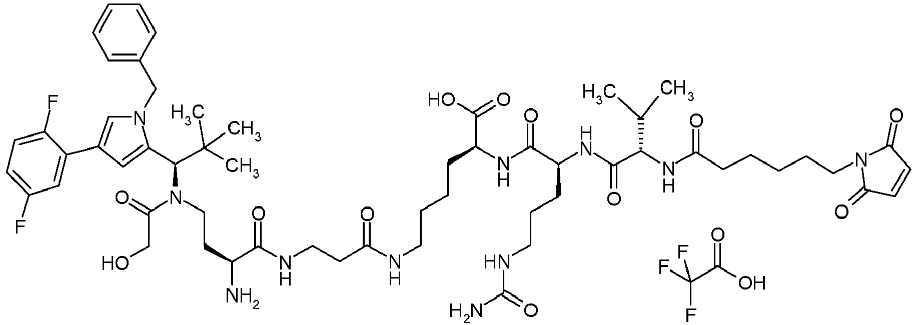

Die Titelverbindung wurde in Analogie zu Intermediat F155 durch Kupplung von 15 mg (0.021 mmol) Intermediat C61 mit 18 mg (0.023 mmol) Intermediat L62 in Gegenwart von HATU und anschließender Entschützung mit Zinkchlorid in Trifluorethanol hergestellt.

LC-MS (Methode 1): Rₜ = 0.88 min; MS (ESIpos): m/z = 1162 (M+H)⁺.

### Intermediat F166

### N-[6-(2,5-Dioxo-2,5-dihydro-1H-pyrrol-1-yl)hexanoyl]-L-valyl-N⁵-carbamoyl-L-ornithyl-N⁶-{[(1R,3S)-3-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)cyclopentyl]carbonyl}-L-lysin-trifluoressigsäure (1:1)

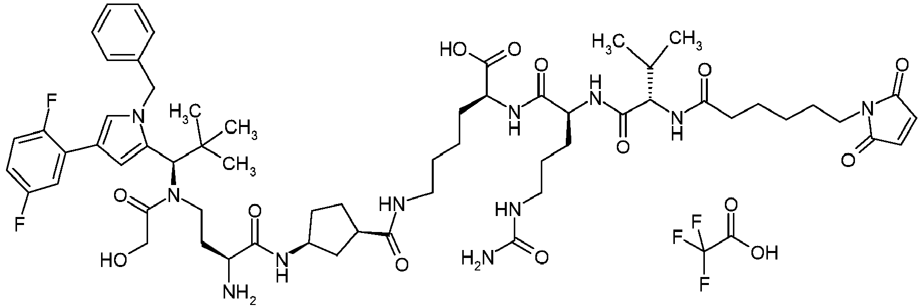

Zunächst wurde ausgehend von kommerziell erhältlicher (1R, 3S)-3-[(tert-Butoxycarbonyl) amino]cyclopentancarbonsäure nach klassischen Methoden der Peptidchemie durch Veresterung mit Benzylalkohol mittels EDCI/DMAP und anschließender Abspaltung der tert Butoxycarbonylschutzgruppe mit TFA in DCM Trifluoressigsäure -benzyl-(1R, 3S)-3-aminocyclo-pentancarboxylat (1:1) hergestellt.

51mg (0.076 mmol) von diesem Intermediat wurden in 6 ml DMF aufgenommen und mit 50 mg (0.076 mmol) von Intermediat C58 in Gegenwart von HATU und *N,N-*Diisopropylethylamin gekuppelt. Nach Reinigung durch präparative HPLC wurde das Intermediat in Methanol aufgenommen und über 10%-igem Palladium auf Aktivkohle 2 h lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Dioxan wurden 21 mg (34% d.Th. über 2 Stufen) von (1R,3S)-3-{[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl) ethoxy] carbonyl} amino)butanoyl] amino } cyclopentancarbonsäure erhalten.

Die Titelverbindung wurde dann in Analogie zu Intermediat F155 durch Kupplung von 10.5 mg (0.013 mmol) dieses Intermediats mit 11.4 mg (0.014 mmol) von Intermediat L62 in Gegenwart von HATU und anschließender Entschützung mit Zinkchlorid in Trifluorethanol hergestellt. Nach Reinigung durch präparative HPLC wurden 8.6 mg (48% d. Th. über 2 Stufen) der Titelverbindung erhalten.

LC-MS (Methode 1): Rₜ = 0.88 min; MS (ESIpos): m/z = 1203 (M+H)⁺.

### Intermediat F167

### N-[6-(2,5-Dioxo-2,5-dihydro-1H-pyrrol-1-yl)hexanoyl]-L-valyl-L-alanyl-N⁶-{[(1R,3S)-3-({(2S)-2-amino-4-[{(1R)-1-[1-benzyl-4-(2,5-difluorphenyl)-1H-imidazol-2-yl]-2,2-dimethylpropyl} (glycoloyl)amino]butanoyl}amino)cyclopentyl]carbonyl}-L-lysin -trifluoressigsäure (1:1)

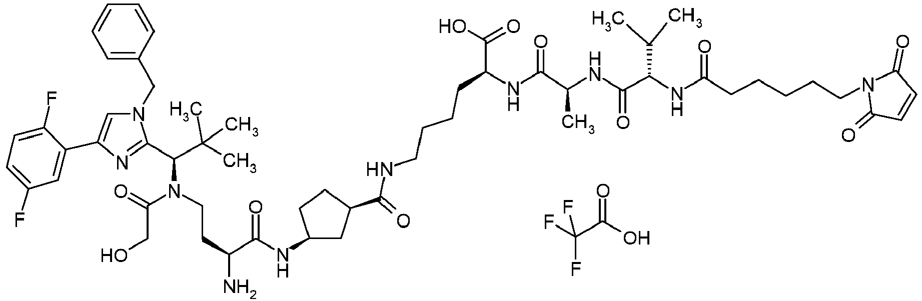

Die Titelverbindung wurde in Analogie zu Intermediat F129 ausgehend von 11 mg (0.016 mmol) Intermediat C5 durch Umsetzung mit 20 mg (0.024 mmol) Intermediat L56 in Gegenwart von 12 mg ( 0.032 mmol) HATU sowie 14 µl N,N-Diisopropylethylamin und anschließender Entschützung mit Trifluoressigsäure hergestellt. Es wurden 11 mg (46% d. Th. über 2 Stufen) erhalten.

LC-MS (Methode 4): Rₜ = 1.13 min; MS (EIpos): m/z = 1117 [M+H]⁺.

### Intermediat F168

### N-[6-(2,5-Dioxo-2,5-dihydro-1H-pyrrol-1-yl)hexanoyl]-L-valyl-L-alanyl-N⁶-{[(1R,2S)-2-({(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}amino)cyclopentyl]carbonyl}-L-lysin -trifluoressigsäure (1:1)

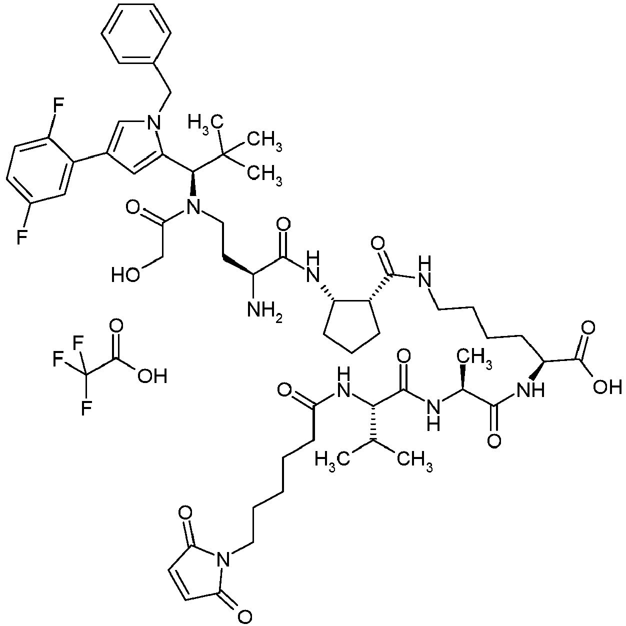

Zunächst wurde ausgehend von kommerziell erhältlicher (1R,2S)-2-[(tert-Butoxycarbonyl) amino]cyclopentancarbonsäure nach klassischen Methoden der Peptidchemie durch Veresterung mit Benzylalkohol mittels EDCI/DMAP und anschließender Abspaltung der tert Butoxycarbonylschutzgruppe mit TFA in DCM Trifluoressigsäure -benzyl-(1R,2S)-2-aminocyclopentancarboxylat (1:1) hergestellt.

102mg (0.305 mmol) von diesem Intermediat wurden in 12 ml DMF aufgenommen und mit 100 mg (0.152 mmol) von Intermediat C58 in Gegenwart von HATU und *N*,*N*-Diisopropylethylamin gekuppelt. Nach Reinigung durch präparative HPLC wurde das Intermediat in Methanol aufgenommen und über 10%-igem Palladium auf Aktivkohle 2 h lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1: 1 wurden 70 mg (59% d.Th. über 2 Stufen) von (1R,2S)-2-{[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl} amino)butanoyl]amino } cyclopentancarbonsäure erhalten.

Die Titelverbindung wurde dann durch Kupplung von 20 mg (0.013 mmol) dieses Intermediats mit 16.6 mg (0.023 mmol) von Intermediat L61 in Gegenwart von 9.5 mg (0.025 mmol) HATU sowie 18 µl N,N-Diisopropylethylamin und anschließender Entschützung mit Zinkchlorid in Trifluorethanol wie in Intermediat F119 beschrieben hergestellt. Nach Reinigung durch präparative HPLC wurden 9.3 mg (30% d. Th. über 2 Stufen) der Titelverbindung erhalten.

LC-MS (Methode 1): Rₜ = 0.98 min; MS (ESIpos): m/z = 1116 (M+H)⁺.

### Intermediat F169

### N-[6-(2,5-Dioxo-2,5-dihydro-1H-pyrrol-1-yl)hexanoyl]-L-valyl-N⁵-carbamoyl-L-ornithyl-N⁶-{[(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)cyclopentyl]carbonyl}-L-lysin-trifluoressigsäure (1:1)

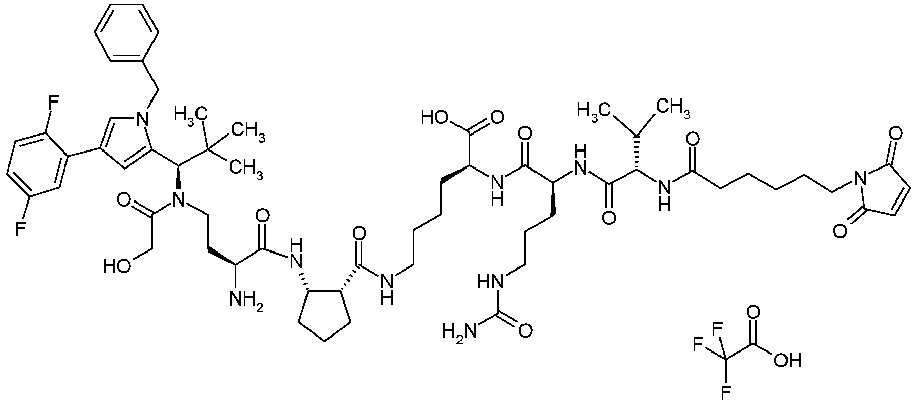

Die Synthese der Titelverbindung erfolgte in Analogie zu Intermediat F168 ausgehend von den Intermediaten C58 und L62.

LC-MS (Methode 1): Rₜ = 0.91 min; MS (ESIpos): m/z = 1202 (M+H)⁺.

### Intermediat F170

### N-[6-(2,5-Dioxo-2,5-dihydro-1H-pyrrol-1-yl)hexanoyl]-L-valyl-N⁵-carbamoyl-L-ornithyl-N⁶-{(2S)-2-amino-4-[{(1R)-1-[1-benzyl-4-(2,5-difluorphenyl)-1H-imidazol-2-yl]-2,2-dimethylpropyl}(glycoloyl)amino]butanoyl}-D-lysin-trifluoressigsäure(1:1)

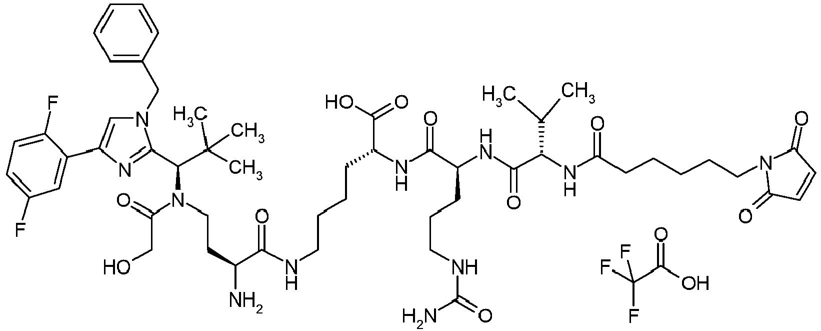

Die Titelverbindung wurde in Analogie zu seinem Diastereomer Intermediat F23 hergestellt. HPLC (Methode 11): Rₜ = 1.9 min;

LC-MS (Methode 1): Rₜ = 0.83 min; MS (ESIpos): m/z = 1092 (M+H)⁺.

### Intermediat F171

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-D-alanyl)-N²-{N-[6-(2,5-dioxo-2,5-dihydro-1H-pyrrol-1-yl)hexanoyl]-L-valyl-L-alanyl}-L-lysin -trifluoressigsäure (1:1)

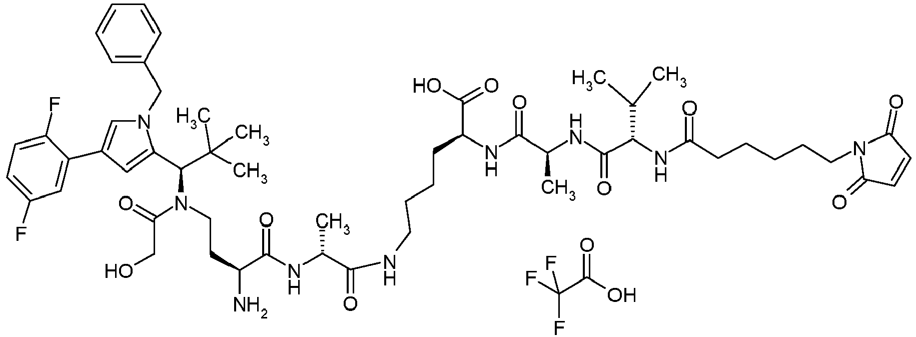

Die Synthese der Titelverbindung erfolgte in Analogie zu Intermediat F155 ausgehend von den Intermediaten C62 und L61.

LC-MS (Methode 1): Rₜ = 0.93 min; MS (ESIpos): m/z = 1076 (M+H)⁺.

### Intermediat F172

### N-[6-(2,5-Dioxo-2,5-dihydro-1H-pyrrol-1-yl)hexanoyl]-L-valyl-L-alanyl-N-[2-({(2S)-2-amino-4-[{(1R)-1-[1-benzyl-4-(2,5-difluorphenyl)-1H-imidazol-2-yl]-2,2-dimethylpropyl}(glycoloyl) amino]butanoyl}amino)ethyl]-L-glutamin -trifluoressigsäure (1:1)

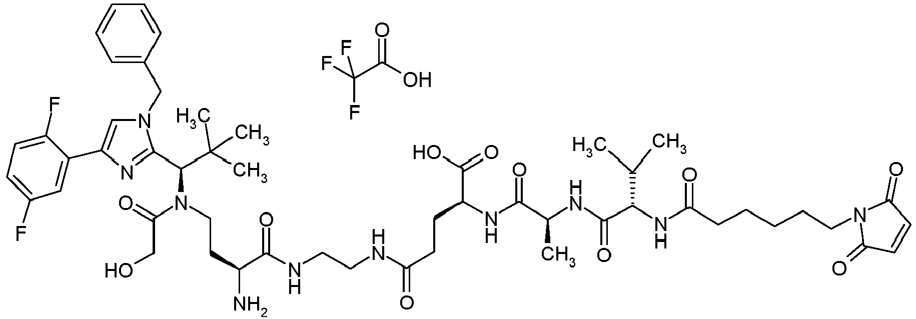

Die Titelverbindung wurde ausgehend von 10 mg (0.013 mmol) Intermediat C63 durch Kupplung mit 9 mg (0.014 mmol) von Intermediat L63 in Gegenwart von 5.5 mg ( 0.014 mmol) HATU sowie 11 µl N,N-Diisopropylethylamin und anschließender Entschützung durch 2.5 stündiges Rühren in einer Lösung aus Trifluoressigsäure/Dichlormethan 1:1 hergestellt. Es wurden 11 mg (72% d. Th. über 2 Stufen) erhalten.

LC-MS (Methode 1): Rₜ = 0.9 min; MS (EIpos): m/z = 1049 [M+H]⁺.

### Intermediat F173

### N-[6-(2,5-Dioxo-2,5-dihydro-1H-pyrrol-1-yl)hexanoyl]-L-valyl-L-alanyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino] butanoyl}amino)ethyl]-L-glutamin -trifluoressigsäure (1:1)

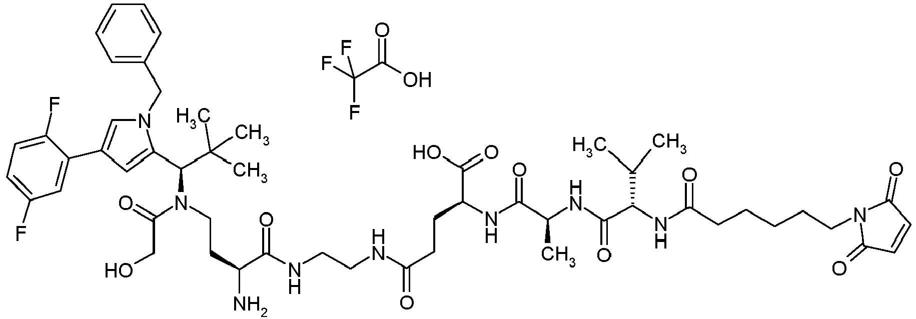

Die Titelverbindung wurde ausgehend von 15 mg (0.018 mmol) Intermediat C64 durch Kupplung mit 12 mg (0.02 mmol) von Intermediat L63 in Gegenwart von 7.7 mg ( 0.02 mmol) HATU sowie 16 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 12 mg (58% d. Th. über 2 Stufen) der Titelverbindung erhalten.

LC-MS (Methode 1): Rₜ = 0.91 min; MS (EIpos): m/z = 1048 [M+H]⁺.

### Intermediat F174

### N-[6-(2,5-Dioxo-2,5-dihydro-1H-pyrrol-1-yl)hexanoyl]-L-valyl-L-alanyl-N-[2-({(2S)-2-amino-4-[{(1R)-1-[4-benzyl-1-(2,5-difluorphenyl)-1H-pyrazol-3-yl]-2,2-dimethylpropyl}(glycoloyl)amino] butanoyl}amino)ethyl]-L-glutamin -trifluoressigsäure (1:1)

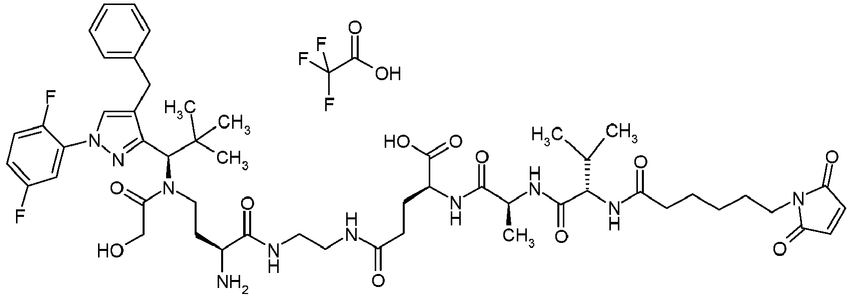

Die Titelverbindung wurde analog zu Intermediat F172 ausgehend Intermediat C57 und L63 hergestellt.

LC-MS (Methode 1): Rₜ = 0.9 min; MS (EIpos): m/z = 1049 [M+H]⁺.

### Intermediat F175

### Trifluoressigsäure --N-[2-({(2S)-2-amino-4-[{(1R)-1-[1-benzyl-4-(2,5-difluorphenyl)-1H-pyrrol-2-yl] -2,2-dimethylpropyl} (glycoloyl)amino]butanoyl} amino)ethyl] -6-(2,5 -dioxo-2,5 -dihydro- 1H-pyrrol-1-yl)hexanamid (1:1)

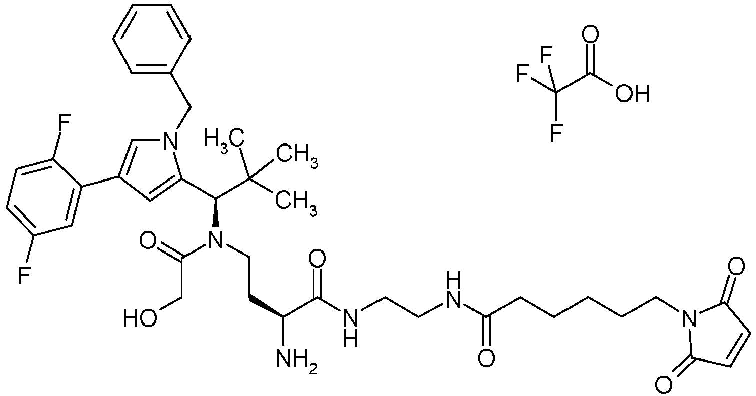

Die Titelverbindung wurde durch Kupplung von 11 mg (0.013 mmol) Intermediat C64 mit 3.4 mg (0.016 mmol) von 6-(2,5-Dioxo-2,5-dihydro-1H-pyrrol-1-yl)hexansäure in Gegenwart von 6.7 mg ( 0.018 mmol) HATU sowie 9 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 8 mg (69% d. Th. über 2 Stufen) der Titelverbindung erhalten.

LC-MS (Methode 1): Rₜ = 1.35 min; MS (EIpos): m/z = 893 [M+H]⁺.

### Intermediat F176

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-[1-(2,5-dioxo-2,5-dihydro-1H-pyrrol-1-yl)-12-oxo-3,6,9-trioxa-13-azapentadecan-15-yl]butanamid (1:1)

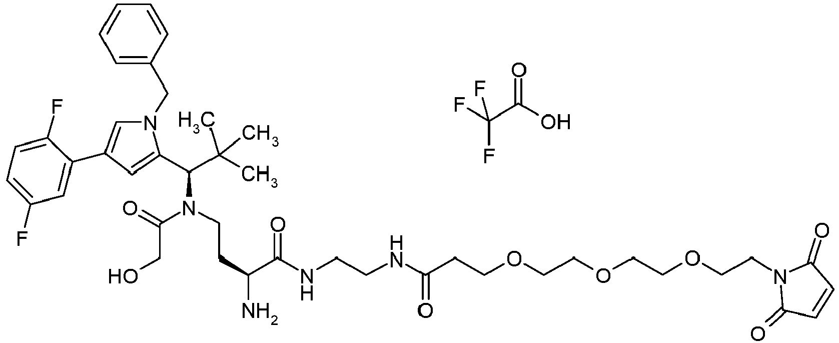

Die Titelverbindung wurde durch Kupplung von 5 mg (0.006 mmol) Intermediat C64 mit 2 mg (0.007 mmol) von 3-(2-{2-[2-(2,5-Dioxo-2,5-dihydro-1H-pyrrol-1-yl)ethoxy]ethoxy}ethoxy) propansäure, deren Herstellung unter Intermediat L15 beschrieben ist, in Gegenwart von 3.5 mg ( 0.009 mmol) HATU sowie 4 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 2 mg (35% d. Th. über 2 Stufen) der Titelverbindung erhalten.

LC-MS (Methode 1): Rₜ = 0.86 min; MS (EIpos): m/z = 839 [M+H]⁺.

### Intermediat F177

### Trifluoressigsäure--(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-(2-{[(2,5-dioxo-2,5-dihydro-1H-pyrrol-1-yl)acetyl]amino}ethyl)cyclopentancarboxamid (1:1)

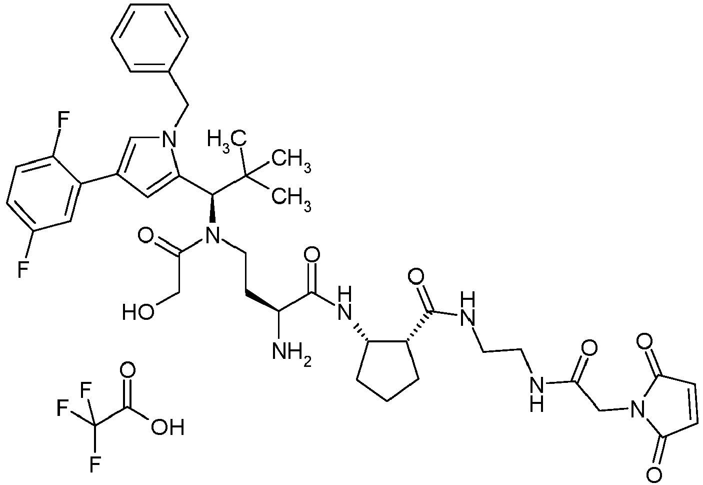

Die Titelverbindung wurde in Anlogie zu Intermediat F168 hergestellt, wobei an Stelle von Intermediat L61 das Intermediat L1 eingesetzt wurde.

LC-MS (Methode 1): Rₜ = 0.86 min; MS (EIpos): m/z = 804 [M+H]⁺.

### Intermediat F178

### Trifluoressigsäure--(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-{2-[(bromacetyl)amino] ethyl}cyclopentancarboxamid (1:1)

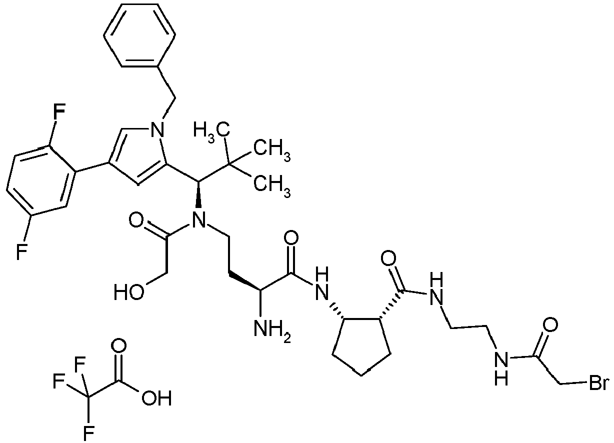

Die Titelverbindung wurde in Anlogie zu Intermediat F177 hergestellt, wobei an Stelle von Intermediat L1 das Intermediat L52 eingesetzt wurde.

LC-MS (Methode 1): Rₜ = 0.89 min; MS (EIpos): m/z = 787 und 789 [M+H]⁺.

### Intermediat F179

### Trifluoressigsäure --(2S)-2-amino-4-[{(1R)-1-[1-benzyl-4-(2,5-difluorphenyl)-1H-pyrrol-2-yl]-2,2-dimethylpropyl } (glycoloyl)amino]-N-[6-(2,5-dioxo-2,5-dihydro-1H-pyrrol-1-yl)hexyl]butanamid (1:1)

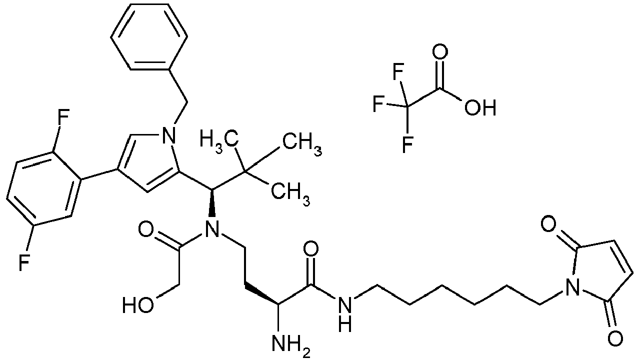

Die Titelverbindung wurde durch Kupplung von 15 mg (0.023 mmol) Intermediat C58 mit 6 mg (0.025 mmol) 1-(6-Aminohexyl)-1H-pyrrol-2,5-dion in Gegenwart von 13 mg ( 0.034 mmol) HATU sowie 16 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 8.5 mg (46% d. Th. über 2 Stufen) der Titelverbindung erhalten.

LC-MS (Methode 6): Rₜ = 2.22 min; MS (EIpos): m/z = 692 [M+H]⁺.

### Intermediat F180

### N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-N²-[(2,5-dioxo-2,5-dihydro-1H-pyrrol-1-yl)acetyl]-L-glutamin-trifluoressigsäure (1:1)

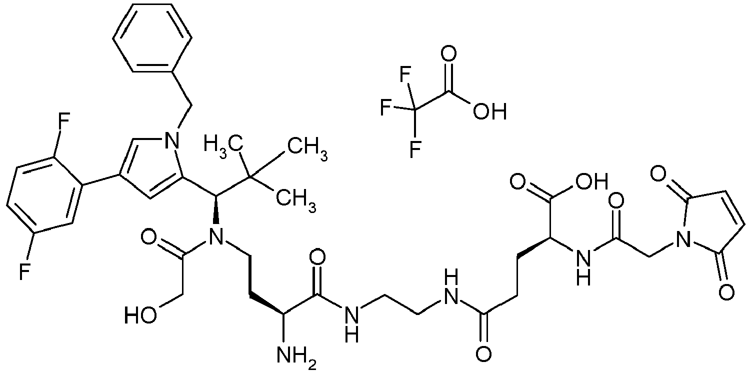

Die Titelverbindung wurde durch Kupplung von 9.6 mg (0.012 mmol) Intermediat C64 mit 5 mg (0.013 mmol) von Intermediat L64 in Gegenwart von 7 mg ( 0.018 mmol) HATU sowie 6 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 3.1 mg (28% d. Th. über 2 Stufen) der Titelverbindung erhalten.

LC-MS (Methode 1): Rₜ = 0.85 min; MS (EIpos): m/z = 822 [M+H]⁺.

### Intermediat F192

### N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[(2,5-dioxo-2,5-dihydro-1H-pyrrol-1-yl)acetyl]amino}-L-alanin-trifluoressigsäure (1:1)

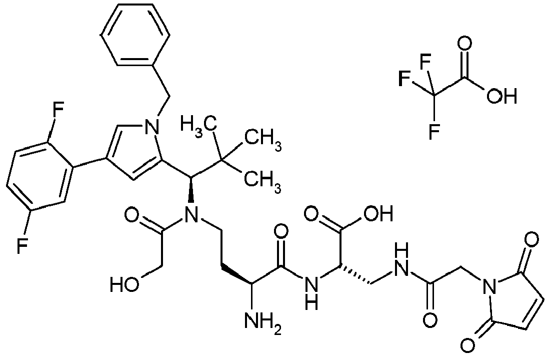

60mg (0.091 mmol) von Intermediat C58 wurden in 8 ml DMF aufgenommen und mit 45 mg (0.100 mmol) von Intermediat L65 in Gegenwart von 42 mg (0.11 mmol) HATU und 64 µL *N,N-*Diisopropylethylamin gekuppelt. Nach Reinigung durch präparative HPLC wurde das Intermediat in 10 ml Ethanol aufgenommen und über 10%-igem Palladium auf Aktivkohle 45 min lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1: 1 wurden 24.5 mg (31% d.Th. über 2 Stufen) von 2-(Trimethylsilyl)ethyl-3-amino-N-[(2S)-4-[{(1R)-1-[1-benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino) butanoyl]-L-alaninat erhalten.

LC-MS (Methode 1): Rₜ = 1.17 min; MS (EIpos): m/z = 844 [M+H]⁺.

Die Titelverbindung wurde dann durch Kupplung von 10 mg (0.012 mmol) dieses Intermediats mit 2 mg (0.013 mmol) von kommerziell erhältlicher (2,5-Dioxo-2,5-dihydro-1H-pyrrol-1-yl)essigsäure Intermediat in Gegenwart von 5.4 mg ( 0.014 mmol) HATU sowie 8 µl N,N-Diisopropylethylamin und anschließender Entschützung mit Zinkchlorid in Trifluorethanol wie in Intermediat F119 beschrieben hergestellt. Nach Reinigung durch präparative HPLC wurden 3.5 mg (33% d. Th. über 2 Stufen) der Titelverbindung erhalten.

LC-MS (Methode 1): Rₜ = 0.81 min; MS (ESIpos): m/z = 737 (M+H)⁺.

### Intermediat F193

### N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[(2,5-dioxo-2,5-dihydro-1H-pyrrol-1-yl)acetyl]amino}-D-alanin-trifluoressigsäure (1:1)

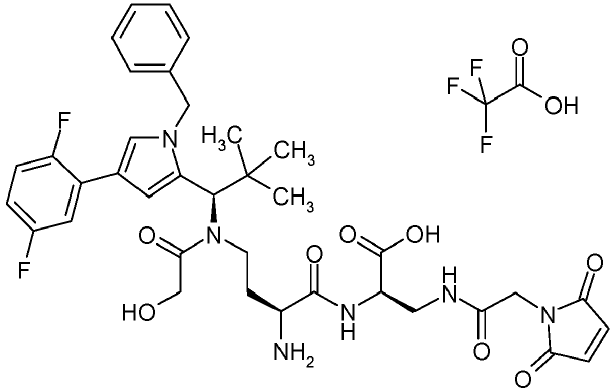

Die Synthese der Titelverbindung erfolgte in Analogie zu Intermediat F192 ausgehend von 3-{[(Benzyloxy)carbonyl]amino}-N-(tert-butoxycarbonyl)-D-alanin - N-cyclohexylcyclohexanamine (1:1).

LC-MS (Methode 1): Rₜ = 0.87 min; MS (ESIpos): m/z = 737 (M+H)⁺.

### Intermediat F194

### N-{5-[(2,5-Dioxopyrrolidin-1-yl)oxy]-5-oxopentanoyl}-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid

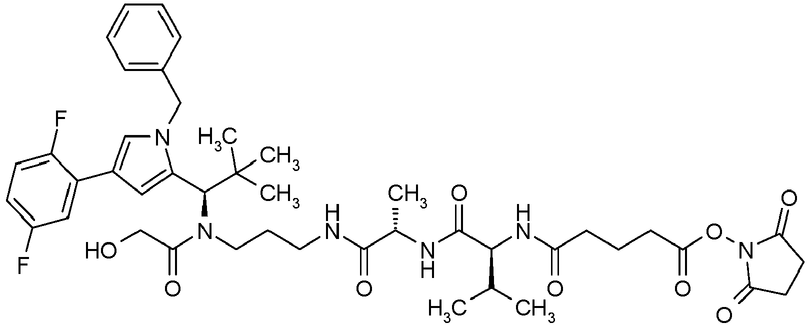

Die Titelverbindung wurde ausgehend von Beispiel 98 zunächst durch Kupplung mit N-[(Benzyloxy)carbonyl]-L-valyl-L-alanin in Gegenwart von HATU und N,N-Diisopropylethylamin hergestellt. Im nächsten Schritt wurde die Z-Schutzgruppe durch 1-stündige Hydrierung über 10%-igem Palladium auf Aktivkohle bei RT unter Wasserstoff-Normaldruck entfernt und das entschützte Intermediat anschließend wie für Intermediat F58 beschrieben durch Umsetzung mit 1,1'-[(1,5-Dioxopentan-1,5-diyl)bis(oxy)] dipyrrolidin-2,5-dion in die Titelverbindung überführt.

LC-MS (Methode 1): Rₜ = 1.19 min; MS (ESIpos): m/z = 851 [M+H]⁺.

### Intermediat F195

### Trifluoressigsäure --N-{(2S)-2-amino-4-[{(1R)-1-[1-benzyl-4-(2,5-difluorphenyl)-1H-pyrrol-2-yl]-2,2-dimethylpropyl}(glycoloyl)amino]butyl}-N'-[2-(2,5-dioxo-2,5-dihydro-1H-pyrrol-1-yl)ethyl]succinamid (1:1)

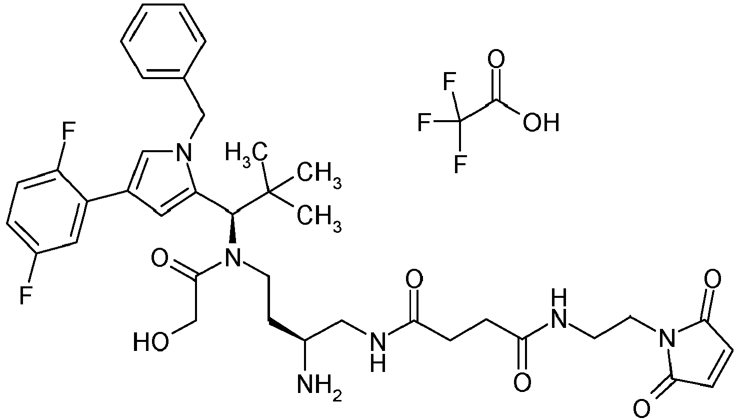

Die Titelverbindung wurde durch Kupplung von 26 mg (0.035 mmol) von Intermediat C65 mit 18 mg (0.07 mmol) von kommerziell erhältlichem Trifluoressigsäure --1-(2-aminoethyl)-1H-pyrrol-2,5-dion (1:1) in 8 ml DMF in Gegenwart von 40 mg ( 0.1054 mmol) HATU sowie 61 µl N,N-Diisopropylethylamin und anschließender Entschützung mit Zinkchlorid in Trifluorethanol wie in Intermediat F119 beschrieben hergestellt. Nach Reinigung durch präparative HPLC wurden 16 mg (43% d. Th. über 2 Stufen) der Titelverbindung erhalten.

LC-MS (Methode 1): Rₜ = 0.85 min; MS (ESIpos): m/z = 721 (M+H)⁺.

¹H-NMR (500 MHz, DMSO-d₆): δ = 7.99 (t, 1H), 7.95 (t, 1H), 7.6-7.75 (m, 4H), 7.5 (s, 1H) 7.2-7.4 (m, 6H), 6.8-7.0 (m, 4H), 5.63 (s, 1H), 4.9 und 5.2 (2d, 2H), 4.26 und 4.0 (2d, 2H), 3.3-3.6 (m, 4H), 3.15-3.25 (m, 3H), 2.85-3.0 (m, 2H), 2.2-2.3 (m, 4H), 0.64 und 1,49 (2m, 2H), 0.81 (s, 9H).

### Intermediat F196

### Trifluoressigsäure --2-(2,5-dioxo-2,5-dihydro-1H-pyrrol-1-yl)ethyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alaninat (1:1)

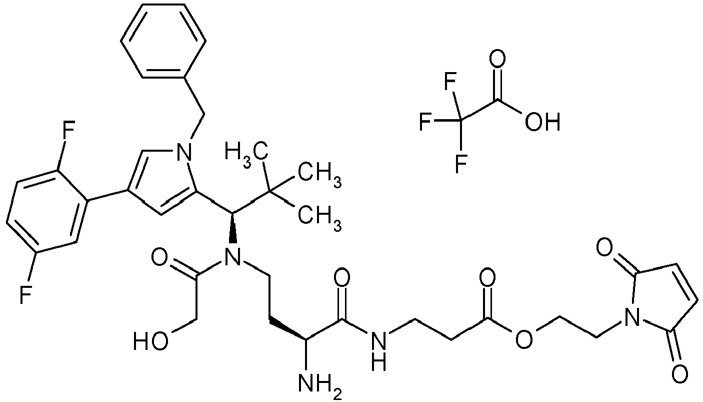

Zunächst wurden 15 mg (0.023 mmol) Intermediat C58 in 4 ml DMF aufgenommen und mit 8.2 mg (0.025 mmol) Intermediat L67 in Gegenwart von 13.0 mg ( 0.034 mmol) HATU sowie 16 µl N,N-Diisopropylethylamin umgesetzt. Nach 30 min Rühren bei RT wurde eingeengt und der Rückstand durch präparative HPLC gereinigt. Nach Vereinigung der entsprechenden Fraktionen und Einengen des Lösungsmittels wurde der Rückstand aus Acetonitril/Wasser 1:1 lyophilisiert. Es wurden 4.3 mg (20% d. Th.) des geschützten Intermediats erhalten.

LC-MS (Methode 1): Rₜ = 1.35 min; MS (EIpos): m/z = 852 [M+H]⁺.

4.3 mg (4.5 µmol) der Zwischenstufe wurden in 1 ml Trifluorethanol gelöst und wie für Intermediat F119 beschrieben mit 3.65 mg (27 µmol) Zinkchlorid entschützt. Nach Reinigung durch präparative HPLC wurde 1 mg (25% d. Th. über 2 Stufen) der Titelverbindung erhalten.

LC-MS (Methode 1): Rₜ = 0.88 min; MS (ESIpos): m/z = 708 (M+H)⁺

### Intermediat F204

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{[3-(2,5-dioxo-2,5-dihydro-1H-pyrrol-1-yl)propanoyl]amino}ethyl)butanamid (1:1)

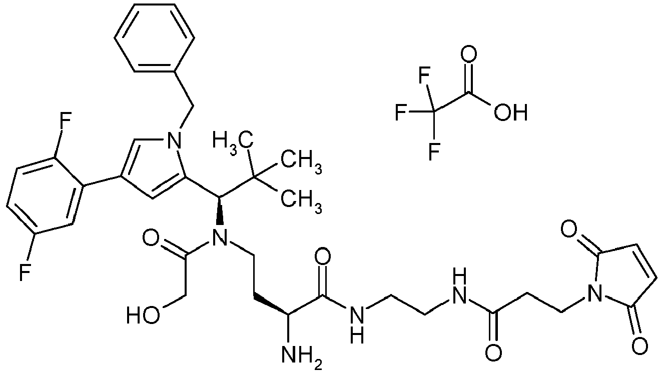

25 mg (0.038 mmol) von Intermediat C58 wurden zunächst mit 16.5 mg (75%-ig) (0.038 mmol) Intermediat L68 in Gegenwart von 17 mg (0.046 mmol) HATU sowie 20 µl N,N-Diisopropylethylamin umgesetzt. Nach 60 min Rühren bei RT wurde eingeengt und der Rückstand durch präparative HPLC gereinigt. Es wurden 18.3 mg (56% d. Th.) des geschützten Intermediats erhalten.

LC-MS (Methode 1): Rₜ = 1.32 min; MS (EIpos): m/z = 851 [M+H]⁺.

Im zweiten Schritt wurde dieses Intermediat in 3 mL 2,2,2-Trifluorethanol gelöst. Es wurden 12 mg (0.086 mmol) Zinkchlorid zugegeben und der Ansatz 2 h bei 50°C gerührt. Anschließend wurden 25 mg (0.086 mmol) Ethylendiamin-N,N,N',N'-tetraessigsäure sowie 2 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 11 mg (62% d.Th.) der Titelverbindung erhalten.

LC-MS (Methode 1): Rₜ = 0.85 min; MS (ESIpos): m/z = 707 (M+H)⁺.

### Intermediat F205

### Trifluoressigsäure--1-[(2,5-dioxo-2,5-dihydro-1H-pyrrol-1-yl)acetyl]piperidin-4-yl-N-{(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}-beta-alanyl-L-valyl-N⁵-carbamoyl-L-ornithinat (1:1)

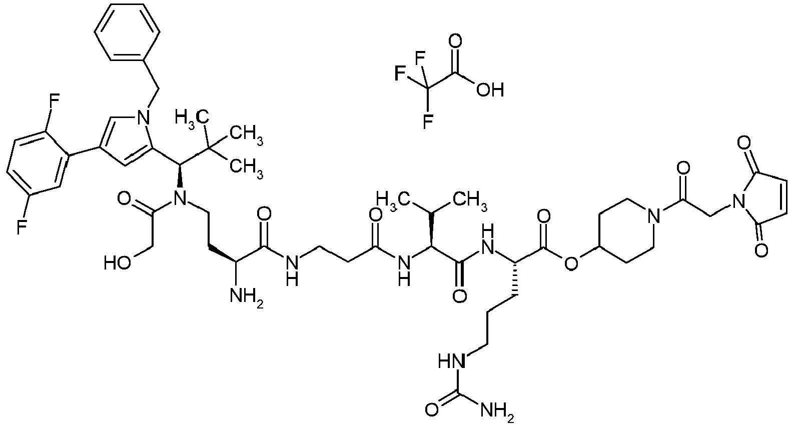

Die Synthese erfolgte durch Kupplung von 25 mg (0.034 mmol) Intermediat C61 und 29 mg (0.041 mmol) Intermediat L69 in Gegenwart von HATU und *N, N*-Diisopropylethylamin, anschließende Hydrierung mit Palladium auf Aktivkohle (10%ig) unter Normaldruck, dann folgende Kupplung mit (2,5-Dioxo-2,5-dihydro-1H-pyrrol-1-yl)essigsäure in Gegenwart von HATU und *N, N-*Diisopropylethylamin und abschließende Abspaltung der 2-(Trimethylsilyl)ethoxycarbonyl-Schutzgruppe mit Zinkchlorid. Nach HPLC Reinigung wurden 11mg (26 % d. Th. über 4 Stufen) erhalten.

LC-MS (Methode 1): Rₜ = 0.86 min; MS (ESIpos): m/z = 1061 (M+H)⁺.

### Intermediat F206

### Trifluoressigsäure--1-[(2,5-dioxo-2,5-dihydro-1H-pyrrol-1-yl)acetyl]piperidin-4-yl-N-{(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}-beta-alanyl-L-valyl-L-alaninat (1:1)

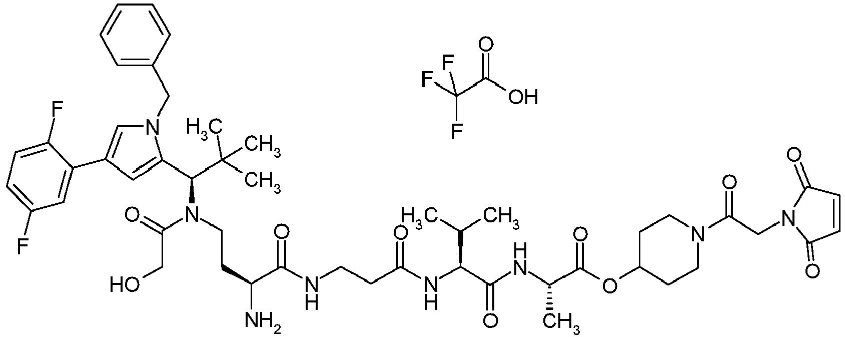

Die Synthese erfolgte in Analogie zu Intermediat F205.

LC-MS (Methode 1): Rₜ = 0.86 min; MS (ESIpos): m/z = 975 (M+H)⁺.

### Intermediat F207

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[(2,5-dioxo-2,5-dihydro-1H-pyrrol-1-yl)acetyl]-L-valyl-L-alanyl}-L-lysin -trifluoressigsäure (1:1)

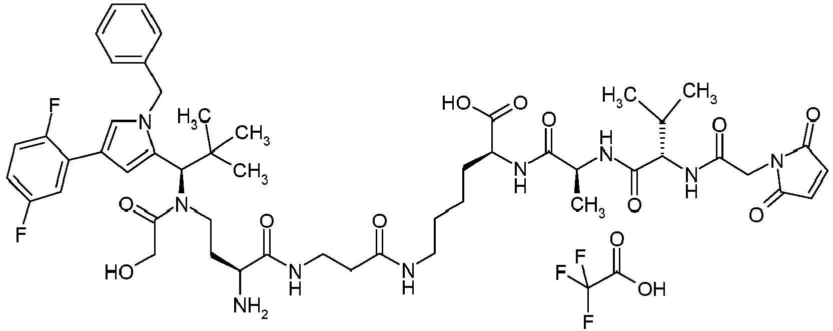

Die Titelverbindung wurde in Analogie zu Intermediat F155 hergestellt.

LC-MS (Methode 1): Rₜ = 0.81 min; MS (ESIpos): m/z = 1020 (M+H)⁺.

### Intermediat F209

### R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cystein-trifluoressigsäure (1:1)

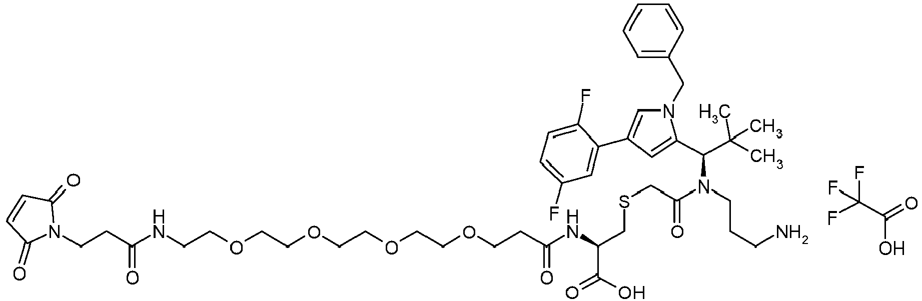

93.9 mg (0.78 mmol) L-Cystein wurden in einer Lösung aus 93.0 mg (1.11 mmol) Natriumhydrogencarbonat und 0.9 mL Wasser suspendiert. Es wurden 70.0 mg (0.11 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) gelöst in 6.0 mL *iso-*Propanol und 202.3 mg (1.33 mmol) 1,8-Diazabicyclo(5.4.0)undec-7-en zugegeben. Die Reaktionsmischung wurde 90 min bei 50 °C gerührt. Der Ansatz wurde mit Wasser (0.1% TFA) versetzt und mittles präp. RP-HPLC gereinigt (Säule: Reprosil 125x30; 10µ, Fluss: 50 mL/min, MeCN/Wasser; 0.1% TFA). Die Lösemittel wurden im Vakuum verdampft und der Rückstand im Hochvakuum getrocknet. Man erhielt 53.9 mg (59 % d. Th.) der Verbindung R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1).

LC-MS (Methode 1): Rₜ = 1.24 min; MS (ESIpos): m/z = 717 (M+H)⁺.

86.0 mg (0.1 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) und 58.5 mg (0.11 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 4.0 mL DMF gelöst und mit 20.9 mg (0.21 mmol) 4-Methylmorpholin versetzt. Die Reaktionsmischung wurde über Nacht bei RT gerührt. Es wurden 15.5 mg (0.26 mmol) HOAc zugegeben und die Reaktionsmischung wurde direkt mittels präp. RP-HPLC gereinigt (Säule: Reprosil 125x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 68.6 mg (59 % d. Th.) der Verbindung R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-cystein. LC-MS (Methode 6): Rₜ = 2.88 min; MS (ESIpos): m/z = 1115 (M+H)⁺.

46.4 mg (0.04 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-cystein wurden in 2.0 mL Trifluorethanol gelöst und mit 17.0 mg (0.13 mmol) Zinkdichlorid versetzt. Die Reaktionsmischung rührte über Nacht bei 50 °C. Es wurden nochmals 8.5 mg (0.07 mmol) Zinkdichlorid zugegeben und über Nacht bei 50 °C gerührt. Die Reaktionsmischung wurde mit 36.5 mg (0.13 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 19.4 mg (43 % d. Th.) der Titelverbindung.

LC-MS (Methode 1): Rₜ = 0.94 min; MS (ESIpos): m/z = 971 (M+H)⁺.

### Intermediat F210

### S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-D-Cystein-trifluoressigsäure (1:1)

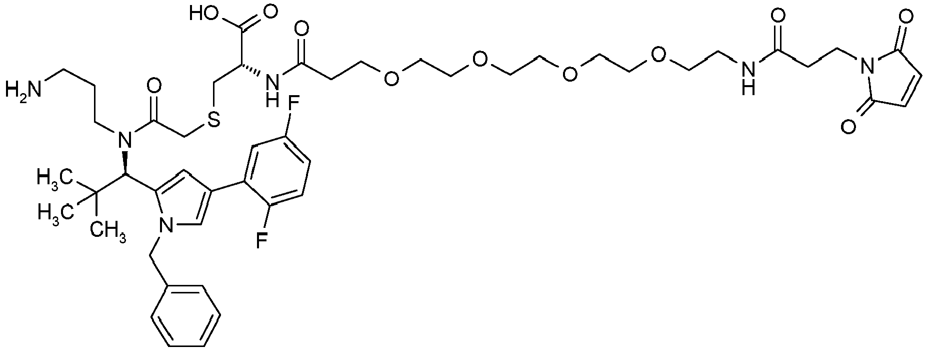

Die Titelverbindung wurde in Analogie zur Synthese des Intermediats F209 unter Verwendung von D-Cystein hergestellt.

LC-MS (Methode 1): Rₜ = 0.91 min; MS (ESIpos): m/z = 971 (M+H)⁺.

### Intermediat F211

### Trifluoressigsäure--3-({2-[(3-aminopropyl){(1R)-1-[1-benzyl-4-(2,5-difluorphenyl)-1H-pyrrol-2-yl]-2,2-dimethylpropyl } amino]-2-oxoethyl } sulfanyl)-N-[2-(2,5-dioxo-2,5-dihydro-1H-pyrrol-1-yl)ethyl]propanamid (1:1)

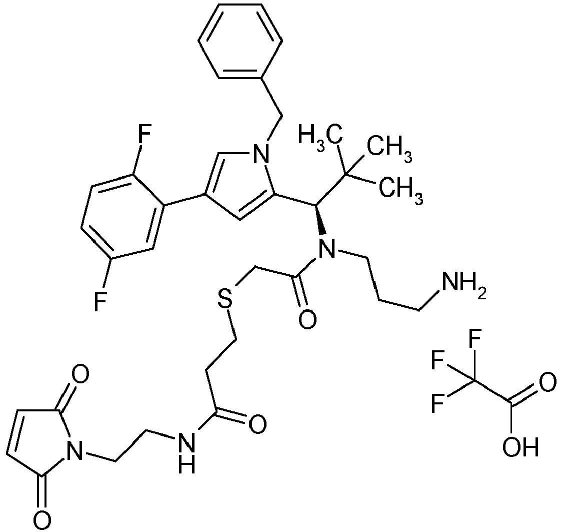

30.0 mg (0.05 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) wurden zusammen mit 5.5 mg (0.05 mmol) 3-Sulfanylpropansäure in 0.5 mL Methanol mit einem Tropfen Wasser vorgelegt. Dann wurden 23.0 mg (0.17 mmol) Kaliumcarbonat zugegeben und die Reaktionsmischung wurde 4 h bei 50 °C gerührt. Es wurde Ethylacetat zugesetzt und die org. Phase einmal mit Wasser und einmal mit ges. NaCl-Lösung gewaschen. Die org. Phase wurde über Magnesiumsulfat getrocknet und das Lösemittel im Vakuum verdampft. Der Rückstand wurde ohne weitere Aufreinigung in der nächsten Synthesestufe eingesetzt. Man erhielt 30.3 mg (86 % d. Th.) der Verbindung 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure

LC-MS (Methode 1): Rₜ = 1.39 min; MS (ESIpos): m/z = 702 (M+H)⁺.

30.0 mg (0.04 mol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure und 9.8 mg (0.06 mmol) 1-(2-Aminoethyl)-1H-pyrrol-2,5-dionhydrochlorid (1:1) wurden in 2.0 mL Acetonitril vorgelegt und mit 44.2 mg (0.34 mmol) N,N-Diisopropylethylamin versetzt. Es wurden 35.4 mg (0.06 mmol) T3P (50 % in Ethylacetat) zugegeben und die Reaktionsmischung wurde über Nacht bei RT gerührt. Es wurde Wasser zugegeben und die Reinigung erfolgte direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 22.0 mg (63 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl} { [(3-{ [2-(2,5-dioxo-2,5-dihydro-1H-pyrrol-1-yl)ethyl]amino}-3-oxopropyl)sulfanyl]acetyl}amino)propyl]carbamat.

LC-MS (Methode 1): Rₜ = 1.41 min; MS (ESIpos): m/z = 824 (M+H)⁺.

22.0 mg (0.03 mol) 2-(Trimethylsilyl)ethyl-[3-({(1R)-1-[1-benzyl-4-(2,5-difluorphenyl)-1H-pyrrol-2-yl]-2,2-dimethylpropyl}{[(3-{[2-(2,5-dioxo-2,5-dihydro-1H-pyrrol-1-yl)ethyl]amino}-3-oxopropyl)sulfanyl]acetyl}amino)propyl]carbamat wurden in 1.0 mL Trifluorethanol gelöst und mit 9.1 mg (0.07 mmol) Zinkdichlorid versetzt. Die Reaktionsmischung rührte 5 h bei 50 °C. Die Reaktionsmischung wurde mit 19.5 mg (0.07 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 15.0 mg (71 % d. Th.) der Titelverbindung.

LC-MS (Methode 1): Rₜ = 0.88 min; MS (ESIpos): m/z = 680 (M+H)⁺.

### Intermediat F212

### Trifluoressigsäure--N-(3-aminopropyl)-N-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-10-oxo-3,6-dioxa-13-thia-9-azapentadecan-15 -arnid (1:1)

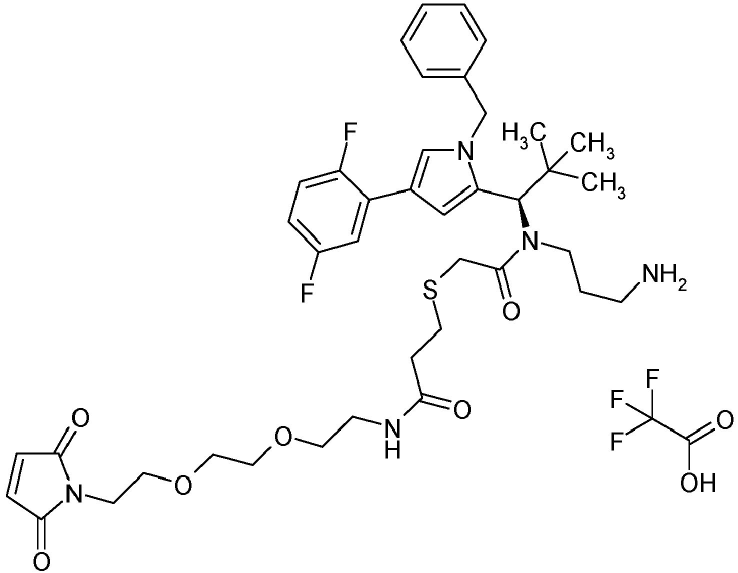

28.8 mg (0.04 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure (Intermediat C69) wurden zusammen mit 18.3 mg (0.05 mmol) Trifluoressigsäure--1-{2-[2-(2-aminoethoxy)ethoxy]ethyl}-1H-pyrrol-2,5-dion (1:1) (Intermediat L59) in 1.9 mL Acetonitril vorgelegt. Dann wurden 42.4 mg (0.33 mmol) N,N-Diisopropylethylamin zugegeben und 33.9 mg (0.05 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt 10.7 mg (26 % d. Th.) der Verbindung 2-(Trimethylsilyl)ethyl-[16-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-10,15-dioxo-3,6-dioxa-13-thia-9,16-diazanonadecan-19-yl] carbamat.

LC-MS (Methode 1): Rₜ = 1.44 min; MS (ESIpos): m/z = 812 (M+H)⁺.

10.7 mg (0.01 mol) 2-(Trimethylsilyl)ethyl-[16-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-10,15-dioxo-3,6-dioxa-13-thia-9,16-diazanonadecan-19-yl]carbamat wurden in 0.8 mL Trifluorethanol gelöst und mit 8.0 mg (0.06 mmol) Zinkdichlorid versetzt. Die Reaktionsmischung rührte 5 h bei 50 °C. Die Reaktionsmischung wurde mit 17.1 mg (0.06 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet.

LC-MS (Methode 1): Rₜ = 1.03 min; MS (ESIpos): m/z = 768 (M+H)⁺.

### Intermediat F213

### Trifluoressigsäure--3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-(2-{[(2,5-dioxo-2,5-dihydro-1H-pyrrol-1-yl)acetyl]amino}ethyl)propanamid (1:1)

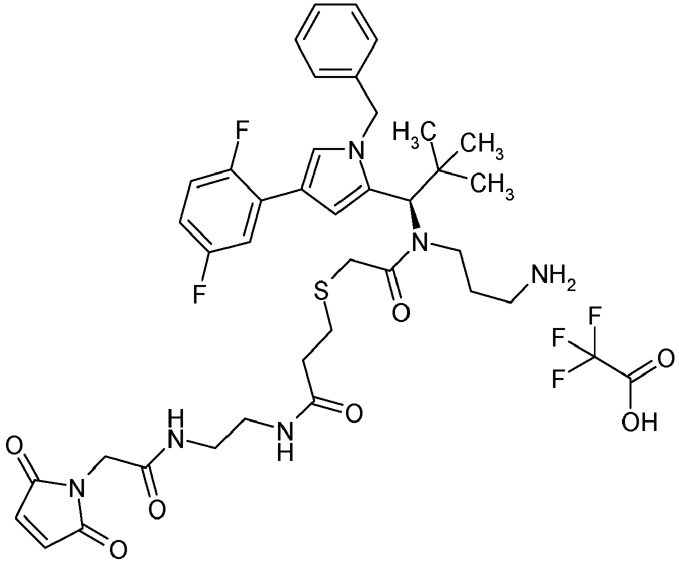

27.5 mg (0.04 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure (Intermediat C69) wurden zusammen mit 15.9 mg (0.05 mmol) Trifluoressigsäure --N-(2-aminoethyl)-2-(2,5-dioxo-2,5-dihydro-1H-pyrrol-1-yl)acetamid (1:1) (Intermediat L1) in 1.8 mL Acetonitril vorgelegt. Dann wurden 32.4 mg (0.31 mmol) N,N-Diisopropylethylamin zugegeben und 32.4 mg (0.05 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 11.9 mg (35 % d. Th.) der Verbindung 2-(Trimethylsilyl)ethyl-[13-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]carbamat.

LC-MS (Methode 1): Rₜ = 1.39 min; MS (ESIpos): m/z = 881 (M+H)⁺.

11.9 mg (0.01 mol) 2-(Trimethylsilyl)ethyl-[13-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]carbamat wurden in 1.0 mL Trifluorethanol gelöst und mit 5.5 mg (0.04 mmol) Zinkdichlorid versetzt. Die Reaktionsmischung rührte über Nacht bei 50 °C. Die Reaktionsmischung wurde mit 11.8 mg (0.04 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.4 mg (60 % d. Th.) der Titelverbindung.

LC-MS (Methode 5): Rₜ = 2.75 min; MS (ESIpos): m/z = 737 (M+H)⁺.

### Intermediat F214

### N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

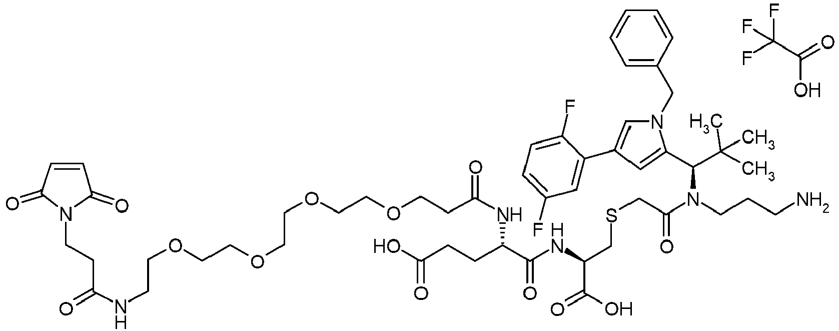

111.7 mg (0.30 mmol) (2S)-5-(Benzyloxy)-2-{[(benzyloxy)carbonyl]amino}-5-oxopentansäure wurden in 3.0 mL DMF vorgelegt und mit 46.1 (0.30 mmol) HOBt, 96.6 mg (0.30 mmol) TBTU und 38.9 mg (0.30 mmol) N,N-Diisopropylethylamin versetzt. Die Reaktionsmischung wurde 10 min bei RT gerührt. 250.0 mg (0.30 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) gelöst in 116.3 mg (0.9 mmol) N,N-Diisopropylethylamin und 3.0 mL DMF wurden dann zugegeben. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 257.0 mg (80 % d. Th.) der Verbindung (16R)-11-{(1R)-1-[1-Benzyl-4-(2,5-difluorphenyl)-1H-pyrrol-2-yl]-2,2-dimethylpropyl} -16- { [(2S)-5 -(benzyloxy)-2- {[(benzyloxy)carbonyl]amino } -5 - oxopentanoyl]amino}-2,2-dimethyl-6,12-dioxo-5-oxa-14-thia-7,11-diaza-2-silaheptadecan-17-säure.

LC-MS (Methode 1): Rₜ = 1.55 min; MS (ESIpos): m/z = 1071 (M+H)⁺.

Unter Argon wurden 24.6 mg (0.11 mmol) Palladium(II)acetat in 5.0 mL Dichlormethan vorgelegt und mit 33.2 mg (0.33 mmol) Triethylamin und 254.3 mg (2.19 mmol) Triethylsilan versetzt. Die Reaktionsmischung wurde 5 min bei RT gerührt und mit 234.1 mg (0.22 mmol) (16R)-11-{(1R)-1-[1-Benzyl-4-(2,5-difluorphenyl)-1H-pyrrol-2-yl] -2,2-dimethylpropyl}-16-{[(2S)-5-(benzyloxy)-2-{ [(benzyloxy)carbonyl]amino } -5 -oxopentanoyl] amino }-2,2-dimethyl-6,12-dioxo-5-oxa-14-thia-7,11-diaza-2-silaheptadecan-17-säure gelöst in 5.0 mL Dichlormethan versetzt. Die Reaktionsmischung wurde über Nacht bei RT gerührt. Die Reaktionsmischung wurde über einen Pappfilter filtriert und der Filterkuchen mit Dichlormethan nachgewaschen. Das Lösemittel wurde im Vakuum ohne Erwärmen verdampft. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 177.5 mg (85 % d. Th.) der Verbindung L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1).

LC-MS (Methode 1): Rₜ = 1.07 min; MS (ESIpos): m/z = 846 (M+H)⁺.

20.0 mg (20.83 µmol) L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) wurden zusammen mit 11.8 mg (22.91 µmol) 3-(2,5-Dioxo-2,5-dihydro-1H-pyrrol-1-yl)-N-{ 15-[(2,5-dioxopyrrolidin-1-yl)oxy]-15-oxo-3,6,9,12-tetraoxapentadec-1-yl}propanamid in 1.5 mL DMF vorgelegt und mit 6.3 mg (62.49 µmol) 4-Methylmorpholin versetzt. Die Reaktionsmischung wurde über Nacht bei RT gerührt und dann mit 4.4 mg (0.07 mmol) Essigsäure versetzt. Die Reaktionsmischung wurde direkt mittles präp. RP-HPLC (Säule: Reprosil 250x30; 10µ, Fluss: 50 mL/min, MeCN/Wasser) gereinigt. Die Lösemittel wurden im Vakuum verdampft und der Rückstand im Hochvakuum getrocknet. Man erhielt 19.1 mg (74 % d. Th.) der Verbindung N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2- yl] -2,2-dimethylpropyl} -2,2-dimethyl-6,12-dioxo-5 -oxa-7,11 -diaza-2-silatridecan-13 -yl)-L-cystein.

LC-MS (Methode 1): Rₜ = 1.24 min; MS (ESIpos): m/z = 1244 (M+H)⁺.

17.5 mg (14.06 µmol) N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 1.5 mL Trifluorethanol gelöst und mit 11.5 mg (84.37 µmol) Zinkdichlorid versetzt. Die Reaktionsmischung rührte 4 h bei 50 °C. Die Reaktionsmischung wurde mit 24.7 mg (0.08 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 10.8 mg (63 % d. Th.) der Titelverbindung

LC-MS (Methode 1): Rₜ = 0.89 min; MS (ESIpos): m/z = 1100 (M+H)⁺.

### Intermediat F215

### N-[19-(2,5-Dioxo-2,5-dihydro-1H-pyrrol-1-yl)-17-oxo-4,7,10,13-tetraoxa-16-azanonadecan-1-oyl]-L-valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid

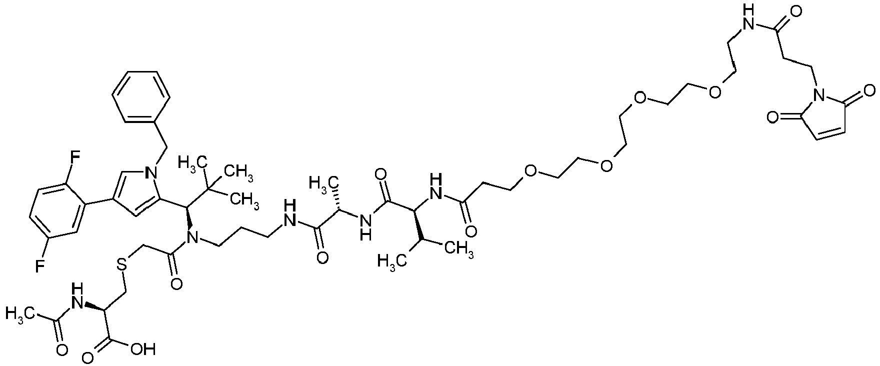

14.9 mg (0.02 mmol) N-Acetyl-S-[2-([3-(L-alanylamino)propyl]{(1R)-1-[1-benzyl-4-(2,5-difluorphenyl)-1H-pyrrol-2-yl]-2,2-dimethylpropyl}amino)-2-oxoethyl]-L-cystein - trifluoressigsäure (1:1) (Beispiel 229) und 7.1 mg (0.02 mmol) 2,5-Dioxopyrrolidin-1-yl-N-[(benzyloxy)carbonyl]-L-valinat wurden in 1.0 mL DMF vorgelegt und mit 5.7 mg (0.06 mmol) 4-Methylmorpholin versetzt. Die Reaktionsmischung wurde über Nacht bei RT gerührt und dann mit 4.5 mg (0.08 mmol) Essigsäure versetzt. Die Reaktionsmischung wurde direkt mittles präp. RP-HPLC (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 13.3 mg (78 % d. Th.) der Verbindung N-[(Benzyloxy)carbonyl]-L-valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2 dimethylpropyl} amino]propyl} -L-alaninamid.

LC-MS (Methode 1): Rₜ = 1.24 min; MS (ESIpos): m/z = 919 (M+H)⁺.

11.1 mg (0.01 mmol) N-[(Benzyloxy)carbonyl]-L-valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid wurden in 5.0 mL Ethanol gelöst, mit 1.0 mg Palladium auf Aktivkohle (10 %) versetzt und bei RT und Normaldruck über Nacht hydriert. Die Reaktionsmischung wurde über Celite filtriert und der Filterkuchen wurde mit einem Ethanol/THF/Wasser Gemisch nachgewaschen. Die Lösemittel wurden im Vakuum verdampft. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand wurde lyophilisiert. Man erhielt 7.5 mg (69 % d. Th.) der Verbindung L-Valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid-trifluoressigsäure (1:1).

LC-MS (Methode 1): Rₜ = 0.86 min; MS (ESIpos): m/z = 785 (M+H)⁺.

7.3 mg (8.12 µmol) L-Valyl-N-{3-[({[(2R)-2-acetamido-2-carboxyethyl]sulfanyl}acetyl){(1R)-1-[1-benzyl-4-(2,5 -difluorphenyl)- 1H-pyrrol-2-yl] -2,2-dimethylpropyl} amino]propyl} -L-alaninamid -trifluoressigsäure (1:1) wurden zusammen mit 4.6 mg (8.93 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 0.5 mL DMF vorgelegt und mit 2.5 mg (24.36 µmol) 4-Methylmorpholin versetzt. Die Reaktionsmischung wurde über Nacht bei RT gerührt und dann mit 4.4 mg (0.03 mmol) Essigsäure versetzt. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 4.9 mg (50 % d. Th.) der Titelverbindung.

LC-MS (Methode 1): Rₜ = 1.07 min; MS (ESIpos): m/z = 1183 (M+H)⁺.

### Intermediat F216

### S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cysteinyl-beta-alanin -trifluoressigsäure (1:1)

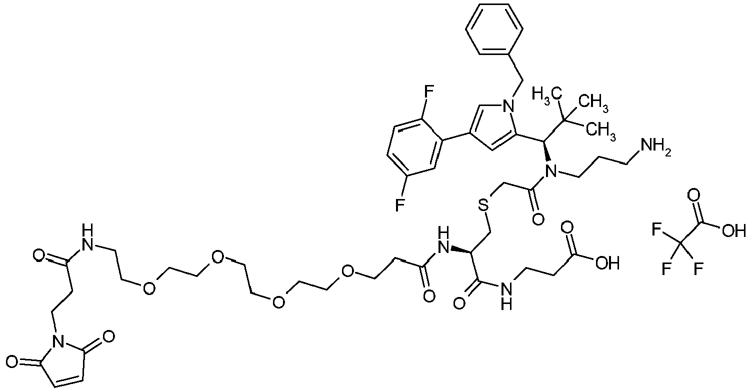

Unter Argon wurden 30.2 mg (0.06 mmol) N,N'-Bis[(benzyloxy)carbonyl]-L-cystin in 2.0 mL Wasser und 2.0 mL iso-Propanol vorgelegt und mit 56.7 mg (0.20 mmol) TCEP versetzt. Die Reaktionsmischung wurde 30 min bei RT gerührt. Es wurden dann 50.0 mg (0.08 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) gelöst in 2.0 mL *iso-*Propanol und 122.2 mg (0.48 mmol) 1,8-Diazabicyclo(5.4.0)undec-7-en zugegeben und die Reaktionsmischung wurde 7 h bei 50 °C gerührt. Dann wurden nochmals 122.2 mg (0.48 mmol) 1,8-Diazabicyclo(5.4.0)undec-7-en zugegeben und die Reaktionsmischung wurde 1 h bei 50 °C gerührt. Es wurde mit Ethylacetat verdünnt und die organische Phase wurde mit Wasser und ges. Natriumhydrogencarbonat-Lsg. extrahiert und mit ges. NaCl-Lsg. gewaschen. Die organische Phase wurde über Magnesiumsulfat getrocknet und das Lösemittel im Vakuum verdampft. Der Rückstand wurde mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 43.1 mg (64 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl]-L-cystein.

LC-MS (Methode 1): Rₜ = 1.46 min; MS (ESIpos): m/z = 851 (M+H)⁺.

16.5 mg (0.05 mmol) 4-Methylbenzolsulfonsäure-benzyl-beta-alaninat (1:1) wurden zusammen mit 14.0 mg (0.11 mmol) N,N-Diisopropylethylamin in 1.5 mL Acetonitril vorgelegt. Die Reaktionsmischung wurde 3 min bei RT gerührt und dann wurden 30.8 mg (0.04 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl]-L-cystein gelöst in 1.5 mL Acetonitril, 23.4 mg (0.18 mmol) N,N-Diisopropylethylamin und 29.9 mg (0.05 mmol) T3P (50 % in Ethylacetat) zugegeben. Die Reaktionsmischung wurde über Nacht bei RT gerührt. Es wurde Wasser zugegeben und die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Benzyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl]-L-cysteinyl-beta-alaninat wurde als Verbindung erhalten.

LC-MS (Methode 1): Rₜ = 1.59 min; MS (ESIpos): m/z = 1012 (M+H)⁺.

43.8 mg (43.3 µmol) Benzyl-S-(11-{(1R)-1-[1-benzyl-4-(2,5-difluorphenyl)-1H-pyrrol-2-yl]-2,2-dimethylpropyl} -2,2-dimethyl-6,12-dioxo-5 -oxa-7,11 -diaza-2-silatridecan-13 -yl)-N-[(benzyloxy)carbonyl]-L-cysteinyl-beta-alaninat wurden in 8.0 mL Ethanol gelöst, mit 4.4 mg Palladium auf Aktivkohle (10%) versetzt und bei RT und Normaldruck über Nacht hydriert. Die Reaktionsmischung wurde über einen Pappfilter filtriert und der Filterkuchen wurde mit einem Ethanol nachgewaschen. Das Lösemittel wurden im Vakuum verdampft. Der Rückstand wurde noch zweimal wie soeben beschrieben behandelt. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 14.5 mg (37 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin -trifluoressigsäure (1:1).

LC-MS (Methode 1): Rₜ = 1.08 min; MS (ESIpos): m/z = 788 (M+H)⁺.

14.5 mg (16.1 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin-trifluoressigsäure (1:1) wurden zusammen mit 9.1 mg (17.7 µmol) 3-(2,5-Dioxo-2,5-dihydro-1H-pyrrol-1-yl)-N-{ 15-[(2,5-dioxopyrrolidin-1-yl)oxy]-15-oxo-3,6,9,12-tetraoxapentadec-1-yl}propanamid in 1.0 mL DMF vorgelegt und mit 4.9 mg (48.2 µmol) 4-Methylmorpholin versetzt. Die Reaktionsmischung wurde über Nacht bei RT gerührt und dann mit 3.4 mg (0.06 mmol) Essigsäure versetzt. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 4.9 mg (50 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin-trifluoressigsäure (1:1).

LC-MS (Methode 1): Rₜ = 1.28 min; MS (ESIpos): m/z = 1186 (M+H)⁺.

14.1 mg (11.9 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-cysteinyl-beta-alanin-trifluoressigsäure (1:1) wurden in 1.5 mL Trifluorethanol gelöst und mit 9.7 mg (71.3 µmol) Zinkdichlorid versetzt. Die Reaktionsmischung rührte 3 h bei 50 °C. Es wurden nochmals 9.7 mg (71.3 µmol) Zinkdichlorid zugegeben und die Reaktionsmischung rührte 3 h bei 50 °C. Es wurden nochmals 9.7 mg (71.3 µmol) Zinkdichlorid zugegeben und die Reaktionsmischung rührte 4 h bei 70 °C. Die Reaktionsmischung wurde mit 20.8 mg (0.07 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand wurde lyophilisiert. Man erhielt 6.2 mg (44 % d. Th.) der Titelverbindung.

LC-MS (Methode 1): Rₜ = 0.82 min; MS (ESIpos): m/z = 1042 (M+H)⁺.

### Intermediat F217

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-[(2,5-dioxo-2,5-dihydro-1H-pyrrol-1-yl)acetyl]-L-cystein - trifluoressigsäure (1:1)

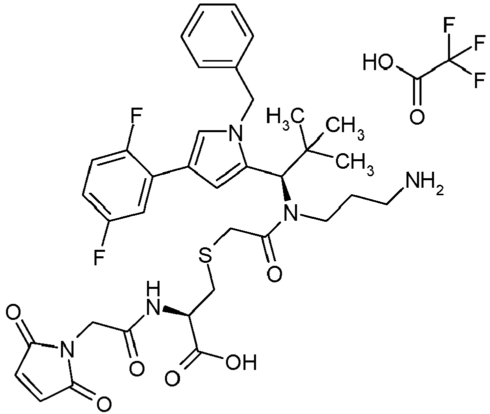

Unter Argon wurden 7.5 mg (0.05 mmol) (2,5-Dioxo-2,5-dihydro-1H-pyrrol-1-yl)essigsäure in 1.5 mL DMF vorgelegt und mit 7.5 mg (0.05 mmol) HOBt, 15.5 mg (0.05 mmol) TBTU und 6.2 mg (0.05 mmol) N,N-Diisopropylethylamin versetzt. Die Reaktionsmischung wurde 10 min bei RT gerührt. Es wurden dann 40.0 mg (0.05 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) gelöst in 1.5 mL DMF und 18.7 mg (0.14 mmol) N,N-Diisopropylethylamin zugegeben und die Reaktionsmischung wurde über Nacht bei RT gerührt. Die Reaktionsmischung wurde direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 11.2 mg (25 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein.

LC-MS (Methode 1): Rₜ = 1.37 min; MS (ESIpos): m/z = 854 (M+H)⁺.

10.9 mg (12.8 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein wurden in 2.0 mL Trifluorethanol gelöst und mit 10.4 mg (76.6 µmol) Zinkdichlorid versetzt. Die Reaktionsmischung rührte 4 h bei 50 °C. Die Reaktionsmischung wurde mit 22.4 mg (0.08 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand wurde lyophilisiert. Man erhielt 7.5 mg (65 % d. Th.) der Titelverbindung.

LC-MS (Methode 1): Rₜ = 0.92 min; MS (ESIpos): m/z = 710 (M+H)⁺.

### Intermediat F218

### N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

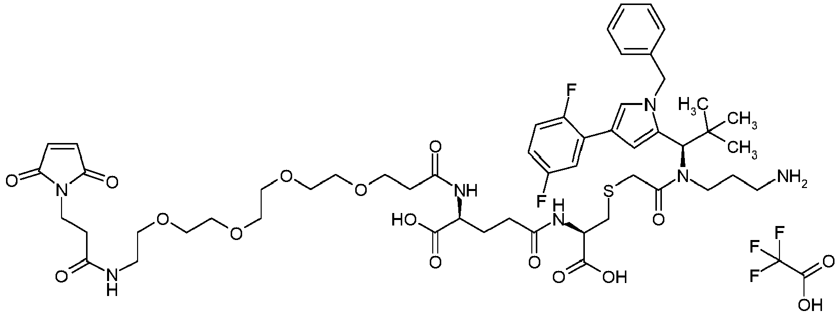

Unter Argon wurden 22.9 mg (0.06 mmol) (4S)-5-(Benzyloxy)-4-{[(benzyloxy)carbonyl]amino}-5-oxopentansäure in 2.0 mL DMF vorgelegt und mit 9.4 mg (0.05 mmol) HOBt, 19.8 mg (0.06 mmol) TBTU und 8.0 mg (0.06 mmol) N,N-Diisopropylethylamin versetzt. Die Reaktionsmischung wurde 10 min bei RT gerührt. Es wurden dann 51.2 mg (0.06 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein (Intermediat C71) gelöst in 1.0 mL DMF und 23.9 mg (0.19 mmol) N,N-Diisopropylethylamin zugegeben und die Reaktionsmischung wurde über Nacht bei RT gerührt Die Reaktionsmischung wurde direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 11.2 mg (25 % d. Th.) der Verbindung (16R)-11-{(1R)-1-[1-Benzyl-4-(2,5-difluorphenyl)-1H-pyrrol-2-yl]-2,2-dimethylpropyl}-16-{[(4S)-5-(benzyloxy)-4-{[(benzyloxy)carbonyl]amino}-5-oxopentanoyl]amino}-2,2-dimethyl-6,12-dioxo-5-oxa-14-thia-7,11-diaza-2-silaheptadecan-17-säure.

LC-MS (Methode 1): Rₜ = 1.52 min; MS (ESIpos): m/z = 1070 (M+H)⁺.

Unter Argon wurden 3.9 mg (0.02 mmol) Palladium(II)acetat in 1.0 mL Dichlormethan vorgelegt und mit 5.3 mg (0.05 mmol) Triethylamin und 254.3 mg (2.19 mmol) Triethylsilan versetzt. Die Reaktionsmischung wurde 5 min bei RT gerührt und mit 18.6 mg (0.02 mmol) (16R)-11-{(1R)-1-[1-Benzyl-4-(2,5-difluorphenyl)-1H-pyrrol-2-yl]-2,2-dimethylpropyl}-16-{[(4S)-5-(benzyloxy)-4-{[(benzyloxy)carbonyl]amino}-5-oxopentanoyl]amino}-2,2-dimethyl-6,12-dioxo-5-oxa-14-thia-7,11-diaza-2-silaheptadecan-17-säure gelöst in 1.0 mL Dichlormethan versetzt. Das Lösemittel wurde im Vakuum ohne Erwärmen verdampft. Der Rückstand wurde in Aceonitril aufgenommen, durch einen Spritzenfilter filtriert und mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet Man erhielt 11.0 mg (66 % d. Th.) der Verbindung L-gamma-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1).

LC-MS (Methode 1): Rₜ = 1.14 min; MS (ESIpos): m/z = 846 (M+H)⁺.

15.0 mg (15.6 µmol) L-gamma-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) wurden zusammen mit 8.8 mg (17.2 µmol) 3-(2,5-Dioxo-2,5-dihydro-1H-pyrrol-1-yl)-N-{ 15-[(2,5-dioxopyrrolidin-1-yl)oxy]-15-oxo-3,6,9,12-tetraoxapentadec-1-yl}propanamid in 1.0 mL DMF vorgelegt und mit 4.7 mg (46.9 µmol) 4-Methylmorpholin versetzt. Die Reaktionsmischung wurde über Nacht bei RT gerührt und dann mit 3.3 mg (0.06 mmol) Essigsäure versetzt. Die Reaktionsmischung wurde direkt mittles präp. RP-HPLC (Säule: Reprosil 250x30; 10µ, Fluss: 50 mL/min, MeCN/Wasser) gereinigt. Die Lösemittel wurden im Vakuum verdampft und der Rückstand im Hochvakuum getrocknet. Man erhielt 14.2 mg (70 % d. Th.) der Verbindung N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein.

LC-MS (Methode 4) Rₜ = 1.24 min; MS (ESIpos): m/z = 1244 (M+H)⁺.

13.8 mg (11.1 µmol) N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,1 1-diaza-2-silatridecan-13-yl)-L-cystein wurden in 2.0 mL Trifluorethanol gelöst und mit 9.1 mg (66.5 µmol) Zinkdichlorid versetzt. Die Reaktionsmischung rührte 4 h bei 50 °C. Die Reaktionsmischung wurde mit 19.4 mg (0.07 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 6.9 mg (50 % d. Th.) der Titelverbindung.

LC-MS (Methode 1): Rₜ = 0.89 min; MS (ESIpos): m/z = 1100 (M+H)⁺.

### Intermediat F235

### Trifluoressigsäure--N-[19-(2,5-dioxo-2,5-dihydro-1H-pyrrol-1-yl)-17-oxo-4,7,10,13-tetraoxa-16-azanonadecan-1-oyl]-L-valyl-N-{4-[(3-aminopropyl){(1R)-1-[1-benzyl-4-(2,5-difluorphenyl)-1H-pyrrol-2-yl] -2,2-dimethylpropyl} carbamoyl]phenyl} -L-alaninamid (1:1)

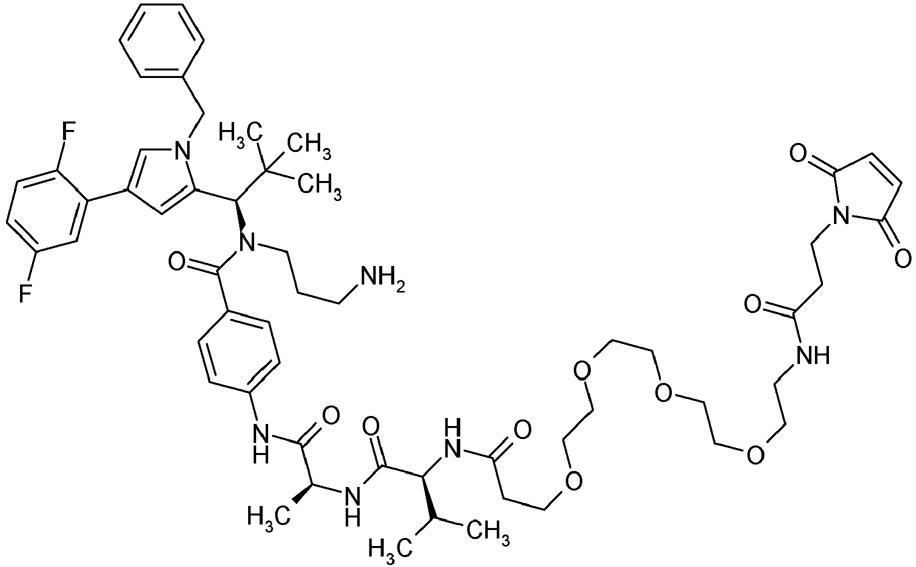

120.0 mg (0.22 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese Intermediat C11) und 52.1 mg (0.28 mmol) 4-Nitrobenzoylchlorid wurden in 8.0 mL Dichlormethan gelöst und mit 28.4 mg (0.28 mmol) Triethlyamin versetzt. Die Reaktionsmischung rührte über Nacht bei RT. Das Lösemittel wurde im Vakuum verdampft und der Rückstand wurde mittles präp. RP-HPLC (Säule: Reprosil 125x30; 10µ, Fluss: 50 mL/min, MeCN/Wasser, 0.1% TFA) gereinigt. Man erhielt 97.7 mg (64 % d. Th.) der Verbindung 2-(Trimethylsilyl)ethyl-{3-[{(1R)-1-[1-benzyl-4-(2,5-difluorphenyl)-1H-pyrrol-2-yl]-2,2-dimethylpropyl } (4-nitrobenzoyl)amino]propyl} carbamat.

LC-MS (Methode 1): Rₜ = 1.54 min; MS (ESIpos): m/z = 705 (M+H)⁺.

97.0 mg (0.14 mmol) 2-(Trimethylsilyl)ethyl-{3-[{(1R)-1-[1-benzyl-4-(2,5-difluorphenyl)-1H-pyrrol-2-yl]-2,2-dimethylpropyl}(4-nitrobenzoyl)amino]propyl}carbamat wurden in 5.0 mL Ethanol gelöst, mit 9.7 mg Palladium auf Aktivkohle (10 %) versetzt und 5 h bei Normaldruck hydriert. Die Reaktionsmischung wurde über einen Papierfilter filtriert und der Filterkuchen wurde mit einem Ethanol nachgewaschen. Das Lösemittel wurde im Vakuum verdampft. Der Rückstand wurde ohne weitere Reinigung in der nächsten Synthesestufe eingesetzt. Man erhielt 87.4 mg (88 % d. Th.) der Verbindung 2-(Trimethylsilyl)ethyl-{3-[(4-aminobenzoyl){(1R)-1-[1-benzyl-4-(2,5-difluorphenyl)-1H-pyrrol-2-yl]-2,2-dimethylpropyl}amino]propyl}carbamat.

LC-MS (Methode 1): Rₜ = 1.47 min; MS (ESIpos): m/z = 675 (M+H)⁺.

59.3 mg (0.09 mmol) 2-(Trimethylsilyl)ethyl-{3-[(4-aminobenzoyl){(1R)-1-[1-benzyl-4-(2,5-difluorphenyl)-1H-pyrrol-2-yl]-2,2-dimethylpropyl}amino]propyl}carbamat und 25.5 mg (0.11 mmol) N-[(Benzyloxy)carbonyl]-L-alanin wurden zusammen mit 68.1 mg (0.53 mmol) N,N-Diisopropylethylamin in 5.0 mL Acetonitril vorgelegt. Es wurde 72.7 mg (0.11 mmol) T3P (50 % in Ethylacetat) langsam zugegeben. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 52.2 mg (68 % d. Th.) der Verbindung Benzyl-[(2S)-1-{[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]amino}-1-oxopropan-2-yl]carbamat.

LC-MS (Methode 1): Rₜ = 1.48 min; MS (ESIpos): m/z = 880 (M+H)⁺.

23.9 mg (0.03 mmol) Benzyl-[(2S)-1-{[4-({(1R)-1-[1-benzyl-4-(2,5-difluorphenyl)-1H-pyrrol-2-yl]-2,2-dimethylpropyl} [3-({ [2-(trimethylsilyl)ethoxy]carbonyl}amino)propyl]carbamoyl)phenyl] amino}-1-oxopropan-2-yl]carbamat wurden in 3.0 mL Ethylacetat gelöst, mit 2.4 mg Palladium auf Aktivkohle (10 %) versetzt und 2 h bei Normaldruck hydriert. Die Reaktionsmischung wurde über einen Papierfilter filtriert und der Filterkuchen wurde mit Ethylacetat nachgewaschen. Das Lösemittel wurde im Vakuum verdampft. Der Rückstand wurde ohne weitere Reinigung in der nächsten Synthesestufe eingesetzt. Man erhielt 20.1 mg (90 % d. Th.) der Verbindung 2-(Trimethylsilyl)ethyl-[3-([4-(L-alanylamino)benzoyl]{(1R)-1-[1-benzyl-4-(2,5-difluorphenyl)-1H-pyrrol-2-yl]-2,2-dimethylpropyl}amino)propyl]carbamat.

LC-MS (Methode 1): Rₜ = 1.13 min; MS (ESIpos): m/z = 746 (M+H)⁺.

20.0 mg (0.03 mmol) 2-(Trimethylsilyl)ethyl-[3-([4-(L-alanylamino)benzoyl]{(1R)-1-[1-benzyl-4-(2,5-difluorphenyl)-1H-pyrrol-2-yl]-2,2-dimethylpropyl}amino)propyl]carbamat wurden zusammen mit 14.9 mg (0.04 mmol) 2,5-Dioxopyrrolidin-1-yl-N-[(benzyloxy)carbonyl]-L-valinat in 2.0 mL DMF vorgelegt und mit 5.4 mg (0.05 mmol) 4-Methylmorpholin versetzt. Die Reaktionsmischung wurde direkt mittles präp. RP-HPLC (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt die Verbindung N-[(Benzyloxy)carbonyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy] carbonyl} amino)propyl] carbamoyl)phenyl] -L-alaninamid.

LC-MS (Methode 1): Rₜ = 1.49 min; MS (ESIpos): m/z = 979 (M+H)⁺.

17.0 mg (17.4 µmol) N-[(Benzyloxy)carbonyl]-L-valyl-N-[4-({(1R)-1-[1-benzyl-4-(2,5-difluorphenyl)-1H-pyrrol-2-yl]-2,2-dimethylpropyl} [3-({[2-(trimethylsilyl)ethoxy]carbonyl}amino)propyl]carbamoyl)phenyl]-L-alaninamid wurden in 2.5 mL Ethylacetat gelöst, mit 1.7 mg Palladium auf Aktivkohle (10 %) versetzt und über Nacht bei Normaldruck hydriert. Die Reaktionsmischung wurde über einen Papierfilter filtriert und der Filterkuchen wurde mit einem Ethylacetat nachgewaschen. Das Lösemittel wurde im Vakuum verdampft und der Rückstand im Hochvakuum getrocknet. Man erhielt 15.3 mg (60 % d. Th.) der Verbindung L-Valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid.

LC-MS (Methode 1): Rₜ = 1.15 min; MS (ESIpos): m/z = 845 (M+H)⁺.

15.3 mg (0.01 mmol) L-Valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid wurden zusammen mit 7.9 mg (0.02 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 2.4 mL DMF vorgelegt und mit 1.9 mg (0.02 mmol) 4-Methylmorpholin versetzt. Die Reaktionsmischung wurde über Nacht bei RT gerührt und dann mit 1.4 mg (0.02 mmol) Essigsäure versetzt. Die Reaktionsmischung wurde direkt mittles präp. RP-HPLC (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt 11.7 mg (70 % d. Th.) der Verbindung N-[19-(2,5-Dioxo-2,5-dihydro-1H-pyrrol-1-yl)-17-oxo-4,7,10,13-tetraoxa-16-azanonadecan-1-oyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid.

11.7 mg (0.01 mmol) N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-(11-{(1R)-1-[1-N-[19-(2,5-Dioxo-2,5-dihydro-1H-pyrrol-1-yl)-17-oxo-4,7,10,13-tetraoxa-16-azanonadecan-1-oyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid wurden in 2.0 mL Trifluorethanol gelöst und mit 3.9 mg (0.03 mmol) Zinkdichlorid versetzt. Die Reaktionsmischung rührte über Nacht bei 50 °C. Die Reaktionsmischung wurde mit 8.3 mg (0.03 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.4 mg (47 % d. Th.) der Titelverbindung. LC-MS (Methode 1): Rₜ = 0.94 min; MS (ESIpos): m/z = 1100 (M+H)⁺.

### Intermediat F236

### (2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-4-{[(2,5-dioxo-2,5-dihydro-1H-pyrrol-1- yl)acetyl]amino}butansäure -trifluoressigsäure (1:1)

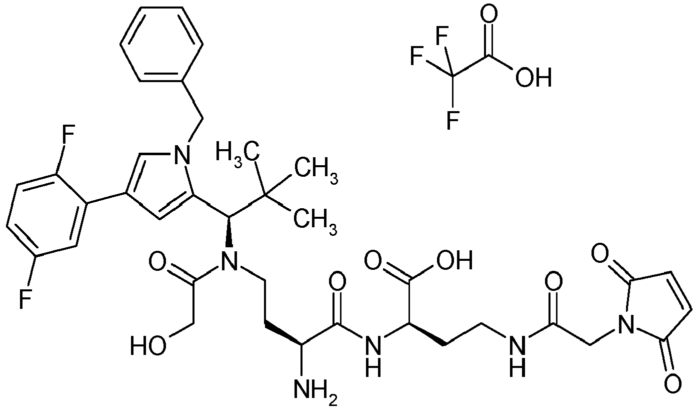

Die Synthese der Titelverbindung erfolgte in Analogie zu Intermediat F192 ausgehend von (2R)-4-{[(Benzyloxy)carbonyl]amino}-2-[(tert-butoxycarbonyl)amino]butansäure --N-cyclohexyl cyclohexanamin (1:1).

LC-MS (Methode 4): Rₜ = 1.1 min; MS (ESIpos): m/z = 751 (M+H)⁺.

### Intermediat F238

### Trifluoressigsäure --N-{(2S)-1-amino-3-[{(1R)-1-[1-benzyl-4-(2,5-difluorphenyl)-1H-pyrrol-2-yl]-2,2-dimethylpropyl}(glycoloyl)amino]propan-2-yl}-N'-[2-(2,5-dioxo-2,5-dihydro-1H-pyrrol-1-yl)ethyl]succinamid (1:1)

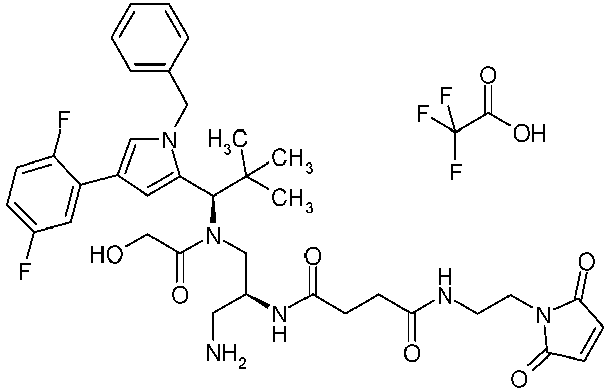

18 mg (0.025 mmol) von Intermediat C72 wurden in 6 ml DMF aufgenommen und mit 7.5 mg (0.03 mmol) von Trifluoressigsäure --1-(2-aminoethyl)-1H-pyrrol-2,5-dion (1:1) in Gegenwart von 11.3 mg (0.03 mmol) HATU und 22 µL N,NDiisopropylethylamin gekuppelt. Nach 1 h Rühren bei RT wurde der Ansatz eingeengt und der Rückstand durch präparative HPLC gereinigt. Die entsprechenden Farktionen wurden eingeengt und der Rückstand aus Acetonitril/Wasser 1:1 lyophilisiert. Es wurden 15 mg (67% d. Th.) des Intermediats erhalten.

LC-MS (Methode 4): Rₜ = 1.71 min; MS (EIpos): m/z = 873 [M+Na]⁺.

Die Titelverbindung wurde anschließend aus diesem Intermediat durch Entschützung mit Zinkchlorid in 4 ml Trifluorethanol wie in Intermediat F119 beschrieben hergestellt. Nach Reinigung durch präparative HPLC wurden 8.5 mg (63% d. Th.) der Titelverbindung erhalten.

LC-MS (Methode 1): Rₜ = 0.86 min; MS (ESIpos): m/z = 707 (M+Na)⁺.

### Intermediat F239

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-[(2,5-dioxo-2,5-dihydro-1H-pyrrol-1-yl)acetyl]-L-cystein - trifluoressigsäure (1:1)

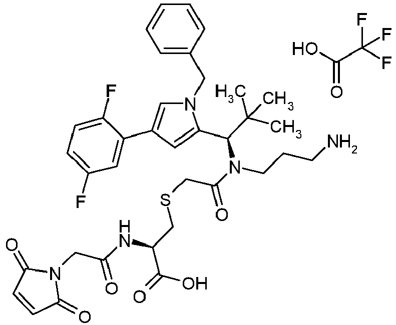

Unter Argon wurden 7.5 mg (0.05 mmol) (2,5-Dioxo-2,5-dihydro-1H-pyrrol-1-yl)essigsäure in 1.5 mL DMF vorgelegt und mit 7.5 mg (0.05 mmol) HOBt, 15.5 mg (0.05 mmol) TBTU und 6.2 mg (0.05 mmol) N,N-Diisopropylethylamin versetzt. Die Reaktionsmischung wurde 10 min bei RT gerührt. Es wurden dann 40.0 mg (0.05 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) gelöst in 1.5 mL DMF und 18.7 mg (0.14 mmol) N,N-Diisopropylethylamin zugegeben und die Reaktionsmischung wurde über Nacht bei RT gerührt. Die Reaktionsmischung wurde direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 11.2 mg (25 % d. Th.) der Verbindung S-(1 1-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein.

LC-MS (Methode 1): Rₜ = 1.37 min; MS (ESIpos): m/z = 854 (M+H)⁺.

10.9 mg (12.8 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein wurden in 2.0 mL Trifluorethanol gelöst und mit 10.4 mg (76.6 µmol) Zinkdichlorid versetzt. Die Reaktionsmischung rührte 4 h bei 50 °C. Die Reaktionsmischung wurde mit 22.4 mg (0.08 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand wurde lyophilisiert. Man erhielt 7.5 mg (65 % d. Th.) der Titelverbindung.

LC-MS (Methode 1): Rₜ = 0.92 min; MS (ESIpos): m/z = 710 (M+H)⁺.

### Intermediat F240

### Trifluoressigsäure--3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-(2-{[(2,5-dioxo-2,5-dihydro-1H-pyrrol-1-yl)acetyl]amino}ethyl)propanamid (1:1)

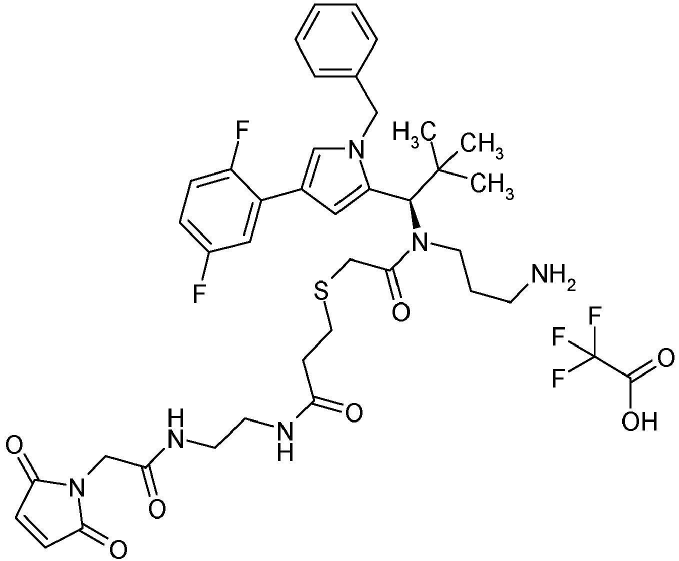

27.5 mg (0.04 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure (Intermediat C69) wurden zusammen mit 15.9 mg (0.05 mmol) Trifluoressigsäure --N-(2-aminoethyl)-2-(2,5-dioxo-2,5-dihydro-1H-pyrrol-1-yl)acetamid (1:1) (Intermediat L1) in 1.8 mL Acetonitril vorgelegt. Dann wurden 32.4 mg (0.31 mmol) N,N-Diisopropylethylamin zugegeben und 32.4 mg (0.05 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 11.9 mg (35 % d. Th.) der Verbindung 2-(Trimethylsilyl)ethyl-[13-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]carbamat.

LC-MS (Methode 1): Rₜ = 1.39 min; MS (ESIpos): m/z = 881 (M+H)⁺.

11.9 mg (0.01 mol) 2-(Trimethylsilyl)ethyl-[13-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]carbamat wurden in 1.0 mL Trifluorethanol gelöst und mit 5.5 mg (0.04 mmol) Zinkdichlorid versetzt. Die Reaktionsmischung rührte über Nacht bei 50 °C. Die Reaktionsmischung wurde mit 11.8 mg (0.04 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.4 mg (60 % d. Th.) der Titelverbindung.

LC-MS (Methode 5): Rₜ = 2.75 min; MS (ESIpos): m/z = 737 (M+H)⁺.

### Intermediat F241

### Trifluoressigsäure --(2S)-2-amino-4-[{(1R)-1-[1-benzyl-4-(2,5-difluorphenyl)-1H-pyrrol-2-yl]-2,2-dimethylpropyl} (glycoloyl)amino] -N-(2- { [N-(bromacetyl)glycyl]amino } ethyl)butanamid (1:1)

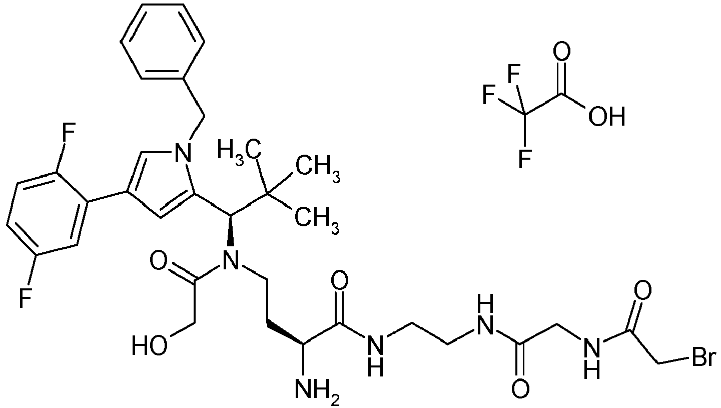

Die Titelverbindung wurde in Analogie aus Intermediat C66 durch Kupplung mit kommerziell erhältlichem 1-(2-Bromacetoxy)pyrrolidin-2,5-dion und anschließender Deblockierung mit Zinkclorid hergestellt.

LC-MS (Methode 1): Rₜ = 0.84 min; MS (EIpos): m/z = 733 und 735 [M+H]⁺.

### Intermediat F242

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-(3-{[(2,5-dioxo-2,5-dihydro-1H-pyrrol-1-yl)acetyl]amino}propyl)butanamid (1:1)

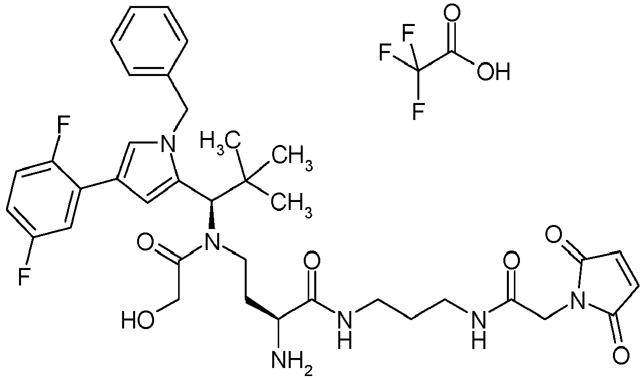

Die Synthese der Titelverbindung erfolgte in Analogie zu Intermediat F104.

LC-MS (Methode 1): Rₜ = 0.84 min; MS (ESIpos): m/z = 707 (M+H)⁺.

### Intermediat F243

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-[2-(2-{[(2,5-dioxo-2,5-dihydro-1H-pyrrol-1-yl)acetyl]amino}ethoxy)ethyl]butanamid (1:1)

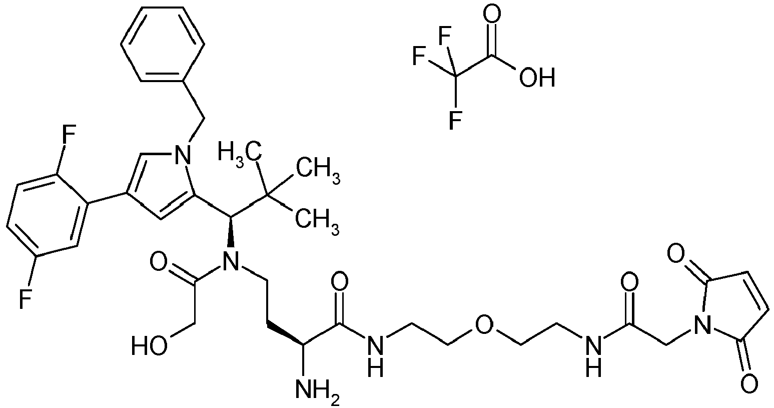

Die Synthese der Titelverbindung erfolgte in Analogie zu Intermediat F242.

LC-MS (Methode 1): Rₜ = 0.81 min; MS (ESIpos): m/z = 737 (M+H)⁺.

### Intermediat F244

### N-{2-[(S-{2-[(3-Aminopropyl) {(1R)-1-[1-benzyl-4-(2,5-difluorphenyl)-1H-pyrrol-2-yl]-2,2-dimethylpropyl} amino] -2-oxoethyl} -L-cysteinyl)amino] ethyl} -6-(2,5 -dioxo-2,5 -dihydro- 1H-pyrrol-1-yl)hexanamid

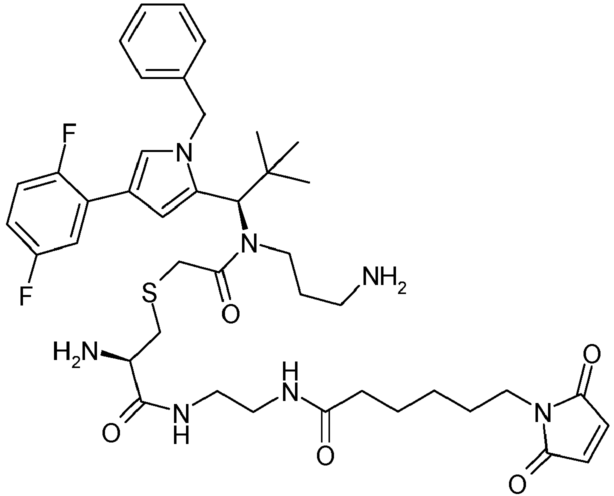

100 mg (ca. 0.101 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[3-(trimethylsilyl)propanoyl]-L-cystein ( Intermediat C 73) wurden in 88 ml Dimethylformamid vorgelegt und mit 107 mg (ca.0.15 mmol) N-(2-aminoethyl)-6-(2,5-dioxo-2,5-dihydro-1H-pyrrol-1-yl)hexanamide (Intermediat L73 ), 46 mg (0.12 mmol) HATU und 88 µl (0.50 mmol) versetzt. Die Reaktionsgemisch wurde 15 minuten bei RT gerührt. Das Gemisch wurde mit Wasser/Dichlormethan versetzt und dann die organische Phase wurde mit Wasser und Brine gewaschen, über Magnesiumsulfat getrocknet, am Rotationsverdampfer eingeengt und im Hochvakuum getrocknet. Der Rückstand wurde ohne weitere Reinigung weiter eingesetzt. Man erhielt 92 mg (59%, Reinheit 72%) der Titelverbindung.

LC-MS (Methode 1): Rₜ = 1.59 min; MS (ESIpos): m/z = 1096 (M+H)⁺.

Zu einer Lösung von 91 mg (ca. 0.06 mmol) 2-(Trimethylsilyl)ethyl-[(9R)-4-{(1R)-1-[1-benzyl-4-(2,5-difluorphenyl)-1H-pyrrol-2-yl]-2,2-dimethylpropyl}-20-(2,5-dioxo-2,5-dihydro-1H-pyrrol-1-yl)-5,10,15 -trioxo-9-{[3-(trimethylsilyl)propanoyl] amino } -7-thia-4,11,14-triazaicos-1 -yl]carbamat in 1.45 ml Trifluorethanol unter Argon wurden 40 mg (0.30 mmol) Zinkchlorid zugegeben. Das Reaktionsgemisch wurde 2h bei 50 °C gerührt. Danach wurden 30 mg (0.22 mmol) Zinkchlorid zugegeben und das Gemisch wurde 1 h bei RT weiter gerührt. Das Gemisch wurde mit 52 mg (0.18 mmol) EDTA versetzt und nach 10 Minuten Rühren bei RT wurde mit Wasser/Acetonitril etwas vedünnt und mittels präparativer HPLC ( Eluent: ACN/Wasser +0.1 %TFA, Gradient) gereinigt. Man erhielt 17 mg (31%) der Titelverbindung.

LC-MS (Methode 1): Rₜ = 0.80 min; MS (ESIpos): m/z = 808 (M+H)⁺.

### Intermediat F245

### Trifluoressigsäure --N-{(2S)-2-amino-4-[{(1R)-1-[1-benzyl-4-(2,5-difluorphenyl)-1H-pyrrol-2-yl]-2,2-dimethylpropyl}(glycoloyl)amino]butyl}-N'-(2-{[(2,5-dioxo-2,5-dihydro-1H-pyrrol-1-yl)acetyl]amino}ethyl)succinamid (1:1)

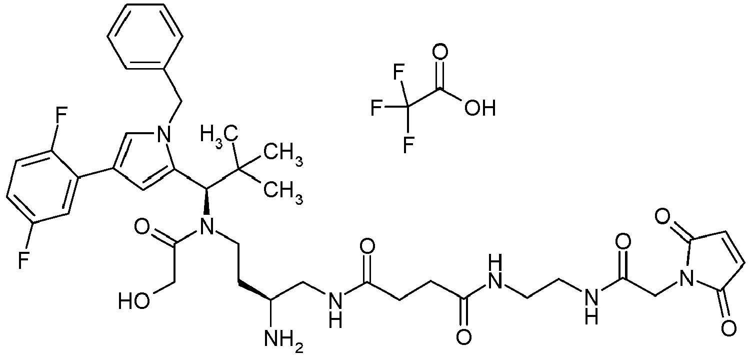

Die Titelverbindung wurde durch Kupplung von 10 mg (0.0135 mmol) von Intermediat C65 mit 8 mg (0.027 mmol) von Intermediat L1 in 8 ml DMF in Gegenwart von 15 mg ( 0.04 mmol) HATU sowie 9 µl N,N-Diisopropylethylamin und anschließender Entschützung mit Zinkchlorid in Trifluorethanol wie in Intermediat F119 beschrieben hergestellt. Nach Reinigung durch präparative HPLC wurden 8.8 mg (58% d. Th. über 2 Stufen) der Titelverbindung erhalten.

LC-MS (Methode 1): Rₜ = 0.84 min; MS (ESIpos): m/z = 778 (M+H)⁺.

### Intermediat F247

### Trifluoressigsäure -methyl-4-[(2-{[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-brom-4-oxobutanoat (1:1)

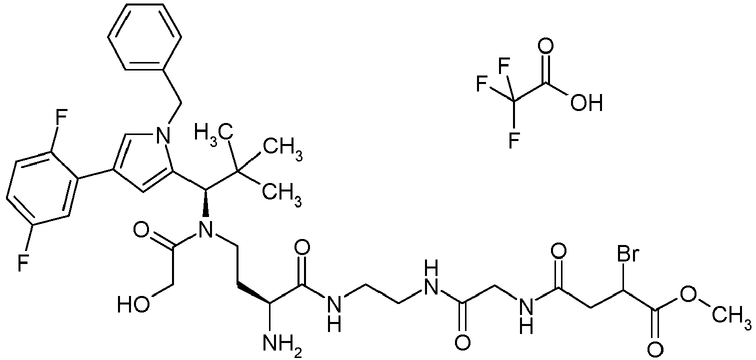

14 mg (0.018 mmol) von Intermediat C66 wurden in 14 mL DCM gelöst und mit 10.1 mg (0.037 mmol) 2-Brom-1-ethylpyridinium-Tetrafluoroborat (BEP) sowie portionsweise mit insgesamt 250 µl Pyridin versetzt, wobei der pH-Wert zwischen 5 und 6 gehalten wurde. Dann wurde mit Essigsäure ein pH-Wert von 4 eingestellt, der Ansatz eingeengt und der Rückstand durch präparative HPLC gereinigt. Nach Vereinigung der entsprechenden Fraktionen, Lyophilisation und Trocknung wurden 4 mg (21% d.Th.) der geschützten Zwischenstufe erhalten, die anschließend mit Zinkchlorid an der Aminofunktion entschützt wurde. Nach HPLC-Reinigung und Lyophilisation wurden 3 mg (72% d.Th.) der Titelverbindung als farbloser Schaum erhalten.

LC-MS (Methode 1): Rₜ = 0.88 min; MS (ESIpos): m/z = 805 und 807(M+H)⁺.

### Intermediat F248

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-{2-[2-(2,5-dioxo-2,5-dihydro-1H-pyrrol-1-yl)ethoxy]ethyl}butanamid (1:1)

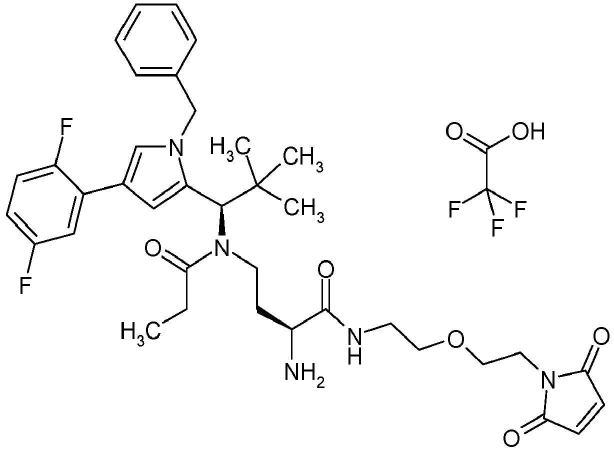

Die Titelverbindung wurde durch Kupplung von 10 mg (0.015 mmol) Intermediat C58 mit 5 mg (0.017 mmol) Intermediat L12 in Gegenwart von HATU und anschließende Entschützung mit Zinkchlorid hergestellt. Es wurden 6.5 mg (52% d.Th. über 2 Stufen) der Titelverbindung erhalten. LC-MS (Methode 1): Rₜ = 0.91 min; MS (ESIpos): m/z = 680 (M+H)⁺.

### Intermediat F254

### Trifluoressigsäure -methyl-(3S)-4-[(2-{[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl] amino}-2-oxoethyl)amino]-3-brom-4-oxobutanoat (1:1)

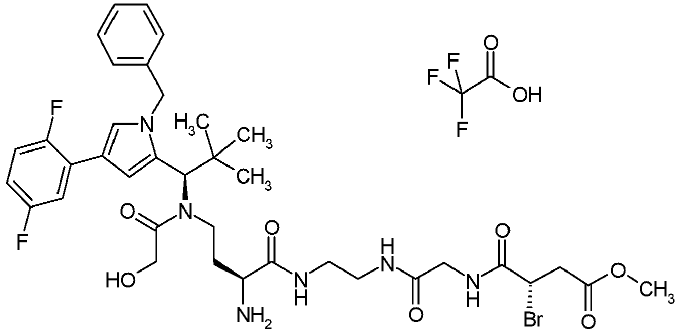

Die Titelverbindung wurde in Analogie zu Intermediat 247 durch Kupplung von 15 mg (0.02 mmol) Intermediat C66 mit 21 mg (0.099 mmol) (2S)-2-Brom-4-methoxy-4-oxobutansäure, die wie in (J.Org.Chem. 200, 65, 517-522) beschrieben aus (2S)-2-Amino-4-methoxy-4-oxobutansäurehydrochlorid (1:1) synthetisiert wurde, hergestellt.

LC-MS (Methode 1): Rₜ = 0.89 min; MS (ESIpos): m/z = 805 und 807(M+H)⁺.

### Intermediat F255

### R/S-(N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl})- homocystein-trifluoressigsäure (1:1)

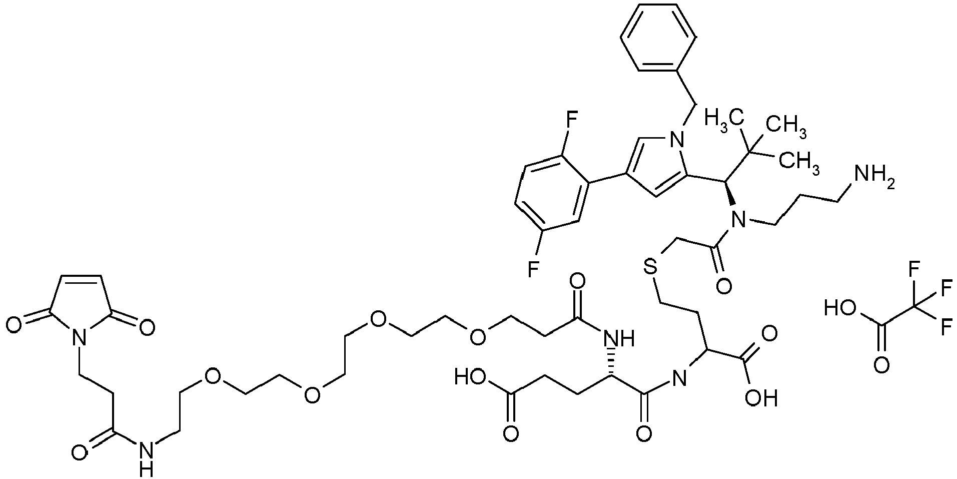

13.1 mg (0.04 mmol) (2S)-5-(Benzyloxy)-2-{[(benzyloxy)carbonyl]amino}-5-oxopentansäure wurden in 1.0 mL DMF vorgelegt und mit 5.4 mg (0.04 mmol) HOBt, 11.4 mg (0.04 mmol) TBTU und 4.6 mg (0.04 mmol) N,N-Diisopropylethylamin versetzt. Die Reaktionsmischung wurde 10 min bei RT gerührt. 30.0 mg (0.04 mmol) R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-homocystein-trifluoressigsäure(1:1) (Intermediat C11) gelöst in 12.9 mg (0.1 mmol) N,N-Diisopropylethylamin und 1 mL DMF wurden dann zugegeben. Die Reaktionsmischung wurde über Nacht bei RT gerührt. Die Reaktionsmischung wurde direkt mittles präp. RP-HPLC (Säule: Reprosil 250x30; 10µ, Fluss: 50 mL/min, MeCN/Wasser/0.1%TFA). Die Lösemittel wurden im Vakuum verdampft und der Rückstand im Hochvakuum getrocknet. Man erhielt 32 mg (73 %) der Verbindung 4-[2-[[(1R)-1-[1-benzyl-4-(2,5-difluorophenyl)pyrrol-2-yl]-2,2-dimethyl-propyl]-[3-(2-trimethylsilylethoxycarbonylamino)propyl]amino]-2-oxo-ethyl]sulfanyl-2-[[(2S)-5-benzyloxy-2-(benzyloxycarbonylamino)-5 -oxo-pentanoyl] amino]butansäure.

LC-MS (Methode 1): Rₜ = 1.53 min; MS (ESIpos): m/z = 1084 (M+H)⁺.

41.4 mg (0.038 mmol) 4-[2-[[(1R)-1-[1-benzyl-4-(2,5-difluorophenyl)pyrrol-2-yl]-2,2-dimethylpropyl]-[3-(2-trimethylsilylethoxycarbonylamino)propyl]amino]-2-oxo-ethyl]sulfanyl-2-[[(2S)-5-benzyloxy-2-(benzyloxycarbonylamino)-5-oxo-pentanoyl]amino]butansäure wird in 10 mL Ethanol, mit 4.2 mg Pd/C versetzt und mit Normaldruck hydriert. Die Reaktionsmischung wurde über einen Pappfilter filtriert und der Filterkuchen mit Ethanol nachgewaschen. Das Lösemittel wurde im Vakuum ohne Erwärmen verdampft. Der Rückstand wurde mittles präp. RP-HPLC (Säule: Reprosil 250x40; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 21.1 mg (56 %) der Verbindung R/S-(L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein -trifluoressigsäure (1:1).

LC-MS (Methode 1): Rₜ = 1.11 min; MS (ESIpos): m/z = 860 (M+H)⁺.

20.4 mg (20.94 µmol) R/S-(L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein -trifluoressigsäure (1:1) wurden zusammen mit 11.8 mg (23.04 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 1.0 mL DMF vorgelegt und mit 4.2 mg (41.88 µmol) 4-Methylmorpholin versetzt. Die Reaktionsmischung wurde über Nacht bei RT gerührt und dann mit 3.1 mg (0.05 mmol) Essigsäure versetzt. Die Reaktionsmischung wurde direkt mittles präp. RP-HPLC (Säule: Reprosil 250x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 9.5 mg (36 %) der Verbindung R/S-(N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein.

LC-MS (Methode 1): Rₜ = 1.66 min; MS (ESIpos): m/z = 1259 (M+H)⁺.

9.4 mg (7.47 µmol) R/S-(N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl))-homocystein wurden in 1.5 mL Trifluorethanol gelöst und mit 6.1 mg (44.81 µmol) Zinkdichlorid versetzt. Die Reaktionsmischung rührte 3 h bei 50 °C. Die Reaktionsmischung wurde mit 13.1 mg (0.05 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 6.9 mg (75 %) der Titelverbindung

LC-MS (Methode 1): Rₜ = 0.87 min; MS (ESIpos): m/z = 1114 (M+H)⁺.

### Intermediat F256

### Trifluoressigsäure --N-{(2S)-2-amino-4-[{(1R)-1-[1-benzyl-4-(2,5-difluorphenyl)-1H-pyrrol-2-yl]-2,2-dimethylpropyl}(glycoloyl)amino]butyl}-N'-[2-(2-{[(2,5-dioxo-2,5-dihydro-1H-pyrrol-1-yl)acetyl]amino}ethoxy)ethyl]succinamid (1:1)

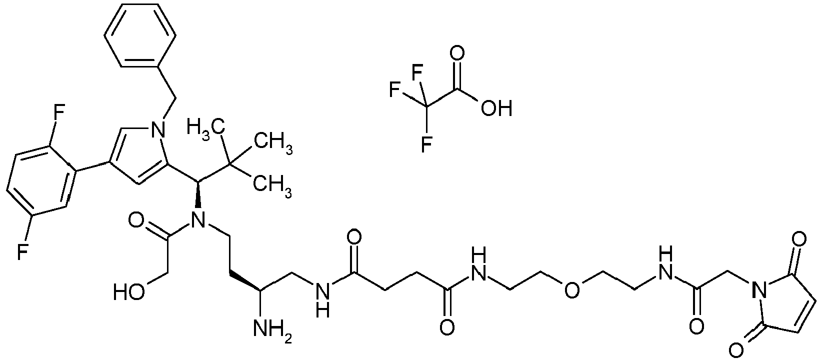

Die Titelverbindung wurde durch Kupplung von 10 mg (0.014 mmol) von Intermediat C65 und 9.6 mg (0.027 mmol) Trifluoressigsäure --N-[2-(2-aminoethoxy)ethyl]-2-(2,5-dioxo-2,5-dihydro-1H-pyrrol-1-yl)acet amid (1:1) in Gegenwart von HATU und N,N-Diisopropylethylamin und anschließender Entschützung mit Zinkchlorid in Trifluorethanol wie in Intermediat F119 beschrieben hergestellt. Nach Reinigung durch präparative HPLC wurden 8 mg (64% d. Th. über 2 Stufen) der Titelverbindung erhalten.

LC-MS (Methode 1): Rₜ = 0.84 min; MS (ESIpos): m/z = 822 (M+H)⁺.

### Intermediat F257

### R-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-[18-(2,5-dioxo-2,5-dihydro-1H-pyrrol-1-yl)-17-oxo-4,7,10,13-tetraoxa-16-azaoctadecan-1-oyl]-L-cystein-trifluoressigsäure (1:1)

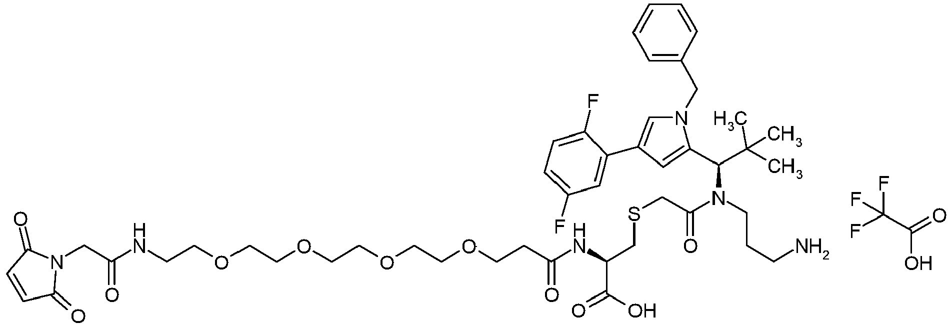

50.0 mg (0.06 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) und 29 mg (0.07 mmol) 3-[2-[2-[2-[2-[[2-(2,5-dioxopyrrol-1-yl)acetyl]amino]ethoxy]ethoxy]ethoxy]ethoxy]propansäure (Intermediat L74) wurden in 3.0 mL DMF gelöst und mit 27.3 mg (0.07 mmol) HATU und 23.3 mg (0.18 mmol) N,N-Diisopropylethylamin versetzt. Die Reaktionsmischung wurde 2 Stunde bei RT gerührt. Die Reaktionsmischung wurde direkt mittels präp. RP-HPLC gereinigt (Säule: Reprosil 125x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 17.4 mg (26 %) der Verbindung R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[18-(2,5-dioxo-2,5-dihydro-1H-pyrrol-1-yl)-17-oxo-4,7,10,13-tetraoxa-16-azaoctadecan-1-oyl]-L-cystein.

LC-MS (Methode 6): Rₜ = 1.34 min; MS (ESIpos): m/z = 1101 (M+H)⁺.

17 mg (0.02 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[18-(2,5-dioxo-2,5-dihydro-1H-pyrrol-1-yl)-17-oxo-4,7,10,13-tetraoxa-16-azaoctadecan-1-oyl]-L-cystein wurden in 1.0 mL Trifluorethanol gelöst und mit 6.3 mg (0.05 mmol) Zinkdichlorid versetzt. Die Reaktionsmischung rührte über Nacht bei 50 °C. Die Reaktionsmischung wurde mit 13.5 mg (0.05 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.6 mg (46 %) der Titelverbindung.

LC-MS (Methode 1): Rₜ = 0.91 min; MS (ESIpos): m/z = 957 (M+H)⁺.

### Intermediat F258

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-[3-({2-[(bromacetyl)amino]ethyl}amino)-3- oxopropyl]butanamid (1:1)

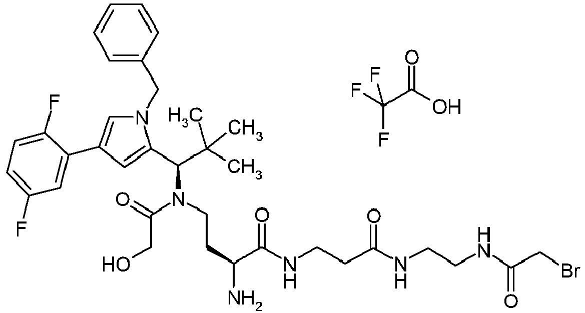

Die Titelverbindung wurde durch Kupplung von Intermediat C58 mit Trifluoressigsäure -benzyl-[2-(beta-alanylamino)ethyl]carbamat (1:1) mittels HATU, anschließender Hydrogenolyse, dann folgender Kupplung mit 1-(2-Bromacetoxy)pyrrolidin-2,5-dion und schließlich durch Entschützung mit Zinkchlorid hergestellt.

LC-MS (Methode 1): Rₜ = 0.86 min; MS (ESIpos): m/z = 747 und 749(M+H)⁺.

### Intermediat F259

### N-{(2S)-2-Amino-4-[{(1R)-1-[1-benzyl-4-(2,5-difluorphenyl)-1H-pyrrol-2-yl]-2,2-dimethyl propyl}(glycoloyl)amino]butanoyl}-3-{[N-(bromacetyl)glycyl]amino}-D-alanin -trifluoressigsäure (1:1)

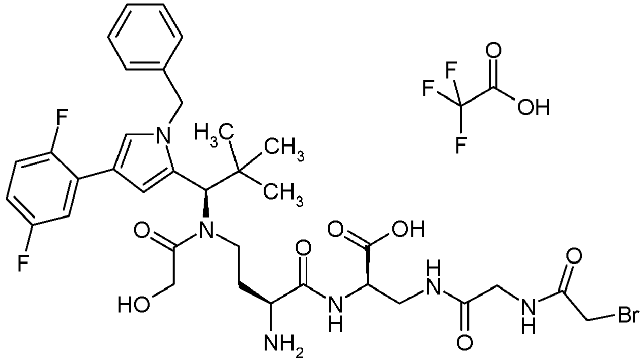

75mg (0.114 mmol) von Intermediat C58 wurden in 12.5 ml DMF aufgenommen und mit 78 mg (0.171 mmol) von Intermediat L75 in Gegenwart von 65 mg (0.11 mmol) HATU und 79 µL *N,N-*Diisopropylethylamin gekuppelt. Nach Reinigung durch präparative HPLC wurde das Intermediat in 20 ml Ethanol aufgenommen und über 10%-igem Palladium auf Aktivkohle 1 h bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1:1 wurden 63 mg (64% d.Th. über 2 Stufen) von 2-(Trimethylsilyl)ethyl-3-amino-N-[(2S)-4-[{(1R)-1-[1-benzyl-4-(2,5-difluorphenyl)-1H-pyrrol-2-yl]-2,2-dimethylpropyl} (glycoloyl)amino]-2-({[2-(trimethylsilyl)ethoxy] carbonyl} amino) butanoyl] -D-alaninat erhalten.

LC-MS (Methode 1): Rₜ = 1.16 min; MS (EIpos): m/z = 844 [M+H]⁺.

40 mg (0.047 mmol) von diesem Intermediat wurden dann wie oben beschrieben mit N-[(Benzyloxy)carbonyl]glycin in Gegenwart von HATU gekuppelt und anschließend erneut hydrogenolytisch entschützt.

Die Titelverbindung wurde dann durch Kupplung von 10 mg (0.012 mmol) dieses Intermediats mit 7.7 mg (0.032 mmol) von kommerziell erhältlichem 1-(2-Bromacetoxy)pyrrolidin-2,5-dion in Gegenwart von 4 µl N,N-Diisopropylethylamin und anschließender Entschützung mit Zinkchlorid in Trifluorethanol wie in Intermediat F119 beschrieben hergestellt. Nach Reinigung durch präparative HPLC wurden 1.3 mg der Titelverbindung erhalten.

LC-MS (Methode 1): Rₜ = 0.83 min; MS (ESIpos): m/z = 777 und 779 (M+H)⁺.

### Intermediat F260

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[(2,5-dioxo-2,5-dihydro-1H-pyrrol-1-yl)acetyl]-L-valyl-L-alanyl}-L-lysin -trifluoressigsäure (1:1)

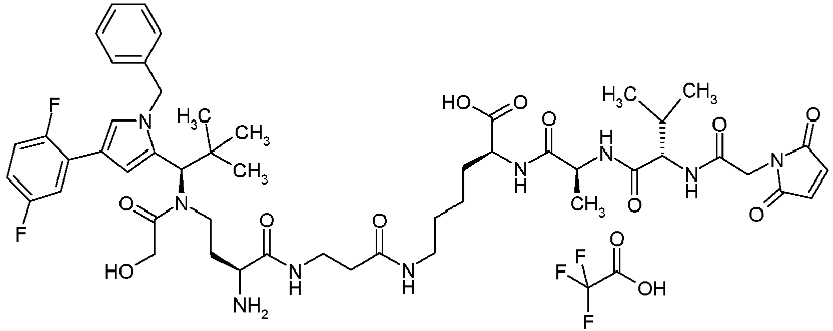

Die Titelverbindung wurde in Analogie zu Intermediat F155 hergestellt.

LC-MS (Methode 1): Rₜ = 0.81 min; MS (ESIpos): m/z = 1020 (M+H)⁺.

### Intermediat F261

### Trifluoressigsäure --(2S)-2-amino-4-[{(1R)-1-[1-benzyl-4-(2,5-difluorphenyl)-1H-pyrrol-2-yl]-2,2-dimethylpropyl} (glycoloyl)amino]-N-(2-{2-[(bromacetyl)amino]ethoxy}ethyl)butanamid (1:1)

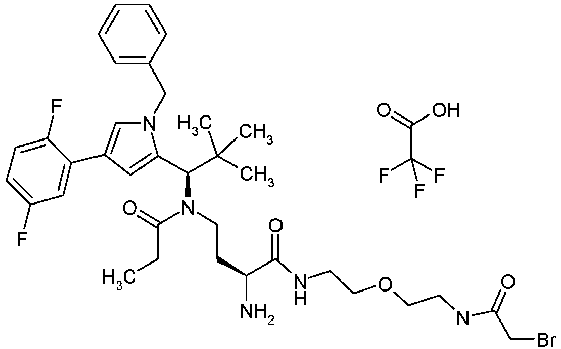

Die Titelverbindung wurde durch Kupplung von 20 mg (0.03 mmol) Intermediat C58 mit 25.8 mg (0.061 mmol) Intermediat L77 in Gegenwart von HATU und anschließende Entschützung mit Zinkchlorid hergestellt. Es wurden 11.9 mg (47% d.Th. über 2 Stufen) der Titelverbindung erhalten.

LC-MS (Methode 1): Rₜ = 0.84 min; MS (ESIpos): m/z = 722 und 720 (M+H)⁺.

### Intermediat F262

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-{3-[2-(2-{[3-(2,5-dioxo-2,5-dihydro-1H-pyrrol-1-yl)propanoyl]amino}ethoxy)ethoxy]propanoyl}-L-cystein -trifluoressigsäure (1:1)

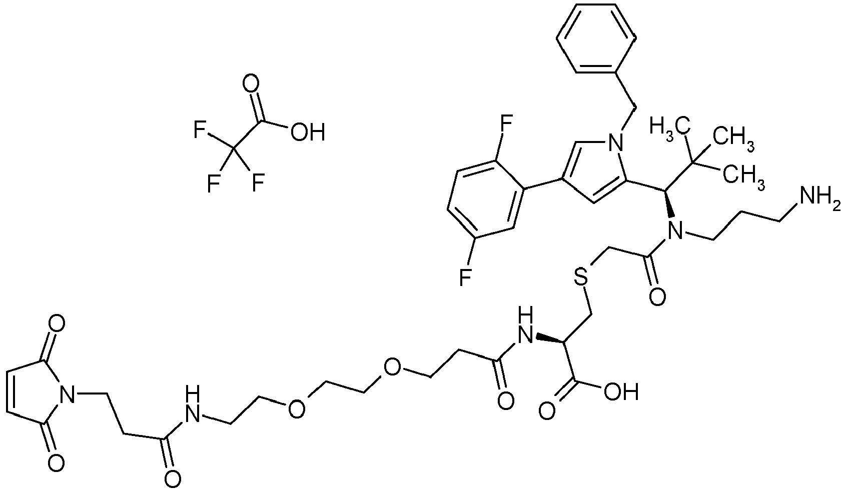

30 mg (36 µmol) S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1) (Intermediat C71) wurden zusammen mit 16,9 mg (40 µmol) 3-(2,5-Dioxo-2,5-dihydro-1H-pyrrol-1-yl)-N-[2-(2-{3-[(2,5-dioxopyrrolidin-1-yl)oxy]-3-oxopropoxy}ethoxy)ethyl]propanamid in 1.5 mL DMF vorgelegt und mit 10,9 mg (108 µmol) 4-Methylmorpholin versetzt. Die Reaktionsmischung wurde über Nacht bei RT gerührt und dann mit 7.58 mg (0.13 mmol) Essigsäure versetzt. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 33,4 mg (80 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{ [3-(2,5-dioxo-2,5-dihydro-1H-pyrrol-1-yl)propanoyl]amino}ethoxy)ethoxy]propanoyl}-L-cystein.

LC-MS (Methode 1): Rₜ = 1.34 min; MS (ESIpos): m/z = 1027 (M+H)⁺.

32.8 mg (32 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{[3-(2,5-dioxo-2,5-dihydro-1H-pyrrol-1-yl)propanoyl]amino}ethoxy)ethoxy]propanoyl}-L-cystein wurden in 3.0 mL Trifluorethanol gelöst und mit 26.1 mg (192 µmol) Zinkdichlorid versetzt. Die Reaktionsmischung rührte 2 h bei 50 °C. Die Reaktionsmischung wurde mit 56.0 mg (0.192 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand wurde lyophilisiert. Man erhielt 22.9 mg (71 % d. Th.) der Titelverbindung.

LC-MS (Methode 1): Rₜ = 0.88 min; MS (ESIpos): m/z = 883 (M+H)⁺.

### Intermediat F263

### N-[(2,5-Dioxo-2,5-dihydro-1H-pyrrol-1-yl)acetyl]-beta-alanyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein - trifluoressigsäure (1:1)

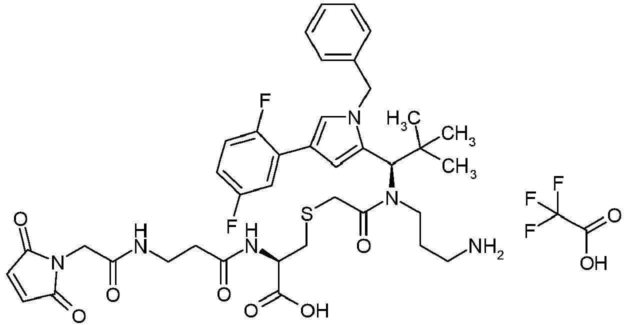

30.0 mg (0.036 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) und 9.8 mg (0.04 mmol) N-[(2,5-Dioxo-2,5-dihydro-1H-pyrrol-1-yl)acetyl]-beta-alanin (Intermediat L78) wurden in 1.0 mL DMF gelöst und mit 16.4 mg (0.04 mmol) HATU und 14.0 mg (0.11 mmol) N,N-Diisopropylethylamin versetzt. Die Reaktionsmischung wurde 2 Stunde bei RT gerührt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 4.2 mg (13 %) der Verbindung N-[(2,5-Dioxo-2,5-dihydro-1H-pyrrol-1-yl)acetyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11 -diaza-2-silatridecan-13 -yl)-L-cystein. LC-MS (Methode 6): Rₜ = 1.31 min; MS (ESIpos): m/z = 925 (M+H)⁺.

11.3 mg (0.011 mmol) N-[(2,5-Dioxo-2,5-dihydro-1H-pyrrol-1-yl)acetyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 5.0 mg (0.04 mmol) Zinkdichlorid versetzt. Die Reaktionsmischung rührte 2 Stunde bei 50 °C. Die Reaktionsmischung wurde mit 10.7 mg (0.04 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 4.4 mg (40 %) der Titelverbindung.

LC-MS (Methode 1): Rₜ = 0.91 min; MS (ESIpos): m/z = 781 (M+H)⁺.

### Intermediat F264

### N-[6-(2,5-Dioxo-2,5-dihydro-1H-pyrrol-1-yl)hexanoyl]-beta-alanyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

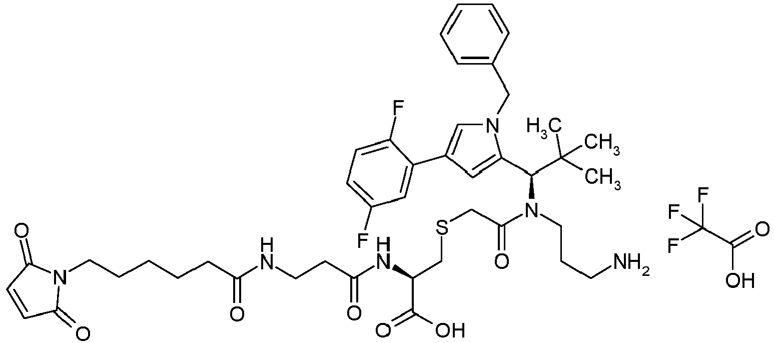

30.0 mg (0.036 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) und 12.2 mg (0.04 mmol) N-[6-(2,5-Dioxo-2,5-dihydro-1H-pyrrol-1-yl)hexanoyl]-beta-alanin (Intermediat L79) wurden in 1.0 mL DMF gelöst und mit 16.4 mg (0.04 mmol) HATU und 14.0 mg (0.11 mmol) N,N-Diisopropylethylamin versetzt. Die Reaktionsmischung wurde 2 Stunde bei RT gerührt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 8.9 mg (24 %) der Verbindung N-[6-(2,5-Dioxo-2,5-dihydro-1H-pyrrol-1-yl)hexanoyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein. LC-MS (Methode 6): Rₜ = 1.38 min; MS (ESIpos): m/z = 981 (M+H)⁺.

15.3 mg (0.015 mmol) N-[6-(2,5-Dioxo-2,5-dihydro-1H-pyrrol-1-yl)hexanoyl]-beta-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 6.3 mg (0.045 mmol) Zinkdichlorid versetzt. Die Reaktionsmischung rührte 2 Stunde bei 50 °C. Die Reaktionsmischung wurde mit 13.5 mg (0.045 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 9.1 mg (62 %) der Titelverbindung.

LC-MS (Methode 1): Rₜ = 0.92 min; MS (ESIpos): m/z = 837 (M+H)⁺.

### Intermediat F265

### Trifluoressigsäure N-(3-aminopropyl)-N-{(1R)-1-[1-benzyl-4-(2,5-difluorphenyl)-1H-pyrrol-2-yl]-2,2-dimethylpropyl}-22-(2,5-dioxo-2,5-dihydro-1H-pyrrol-1-yl)-6,17-dioxo-10,13-dioxa-3-thia-7,16-diazadocosan-1-amid (1:1)

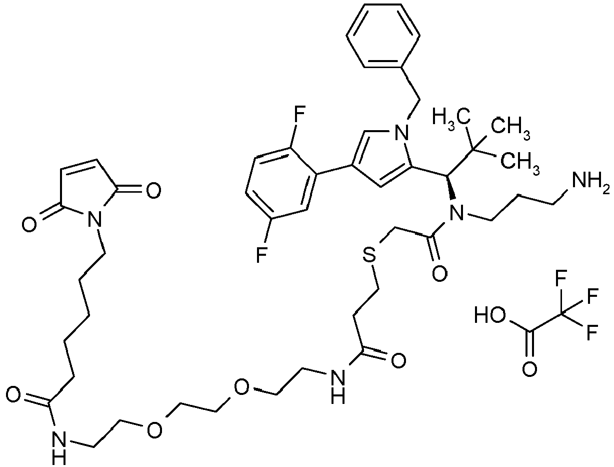

30.0 mg (42.7 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure (Intermediat C69) und 25.3 mg (55.6 µmol) Trifluoressigsäure N-{2-[2-(2-aminoethoxy)-ethoxy]ethyl}-6-(2,5-dioxo-2,5-dihydro-1H-pyrrol-1-yl)hexanamid (1:1) (Intermediat L82) wurden in 1.9 mL Acetonitril vorgelegt und mit 60 µl (340 µmol) N,N-Diisopropylethylamin und 33 µl (56 µmol) 2,4,6-Tripropyl-1,3,5,2,4,6-trioxatriphosphinan-2,4,6-trioxid 50 % in Ethylacetat versetzt. Die Reaktionsmischung wurde über Nacht bei RT nachgerührt. Es wurde Wasser (2.0 mL) zugegeben und die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 26.7 mg (60 % d. Th.) der Verbindung 2-(Trimethylsilyl)ethyl-[4-{(1R)-1-[1-benzyl-4-(2,5-difluorphenyl)-1H-pyrrol-2-yl]-2,2-dimethylpropyl}-26-(2,5-dioxo-2,5-dihydro-1H-pyrrol-1-yl)-5,10,21-trioxo-14,17-dioxa-7-thia-4,11,20-triazahexacos-1-yl]carbamat.

LC-MS (Methode 1): Rₜ = 1.40 min; MS (ESIpos): m/z = 1025 (M+H)⁺.

25.3 mg (24.7 µmol) 2-(Trimethylsilyl)ethyl-[4-{(1R)-1-[1-benzyl-4-(2,5-difluorphenyl)-1H-pyrrol-2-yl]-2,2-dimethylpropyl}-26-(2,5-dioxo-2,5-dihydro-1H-pyrrol-1-yl)-5,10,21-trioxo-14,17-dioxa-7-thia-4,11,20-triazahexacos-1-yl]carbamat wurden in 2.0 mL Trifluorethanol gelöst und mit 20.2 mg (148 µmol) Zinkdichlorid versetzt. Die Reaktionsmischung rührte 1 h bei 50 °C. Die Reaktionsmischung wurde mit 43.3 mg (148 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 23.4 mg (95 % d. Th.) der Titel Verbindung.

LC-MS (Methode 1): Rₜ = 0.89 min; MS (ESIpos): m/z = 881 (M+H)⁺.

### Intermediat F266

### Trifluoressigsäure N-(3-aminopropyl)-N-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,13-dioxo-6,9-dioxa-16-thia-3,12-diazaoctadecan-18-amid (1:1)

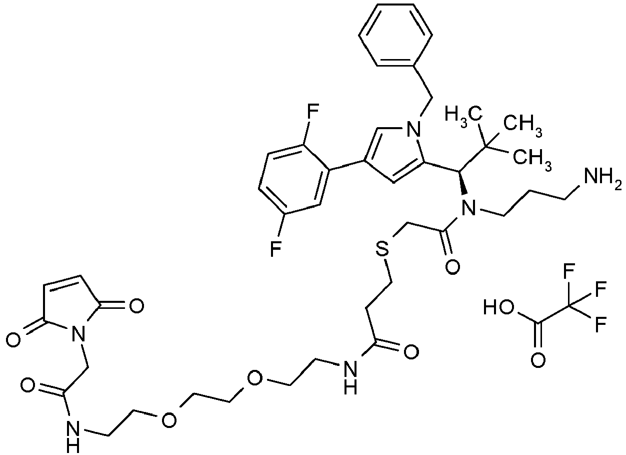

30.0 mg (0.043 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden zusammen mit 22.2 mg (0.056 mmol) Trifluoressigsäure N-{2-[2-(2-aminoethoxy)ethoxy]ethyl}-2-(2,5-dioxo-2,5-dihydro-1H-pyrrol-1-yl)acetamid (1:1) (Intermediat L83) in 1.9 mL Acetonitril vorgelegt. Dann wurden 60 µl (0.34 mmol) N,N-Diisopropylethylamin zugegeben und 33 µl (0.056 mmol) T3P (50 % in Ethylacetat) zugetropft. Die Reaktionsmischung wurde über Nacht bei RT gerührt. Es wurde Wasser (2.0 mL) zugegeben. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 20.5 mg (49 % d. Th.) der Verbindung 2-(Trimethylsilyl)ethyl-[19-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,13,18-trioxo-6,9-dioxa-16-thia-3,12,19-triazadocosan-22-yl] carbamat.

LC-MS (Methode 1): Rₜ = 1.38 min; MS (ESIpos): m/z = 969 (M+H)⁺.

19.1 mg (19.7 µmol) 2-(Trimethylsilyl)ethyl-[19-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,13,18-trioxo-6,9-dioxa-16-thia-3,12,19-triazadocosan-22-yl]carbamat wurden in 2.0 mL Trifluorethanol gelöst und mit 16.1 mg (118 µmol) Zinkdichlorid versetzt. Die Reaktionsmischung rührte 1 h bei 50 °C. Die Reaktionsmischung wurde mit 34.6 mg (118 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 13.9 mg (75 % d. Th.) der Titel Verbindung.

LC-MS (Methode 1): Rₜ = 0.86 min; MS (ESIpos): m/z = 825 (M+H)⁺.

### Intermediat F267

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-[1-(2,5-dioxo-2,5-dihydro-1H-pyrrol-1-yl)-2,18-dioxo-6,9,12,15-tetraoxa-3-azaoctadecan-18-yl]-L-cysteinyl-beta-alanin -trifluoressigsäure (1:1)

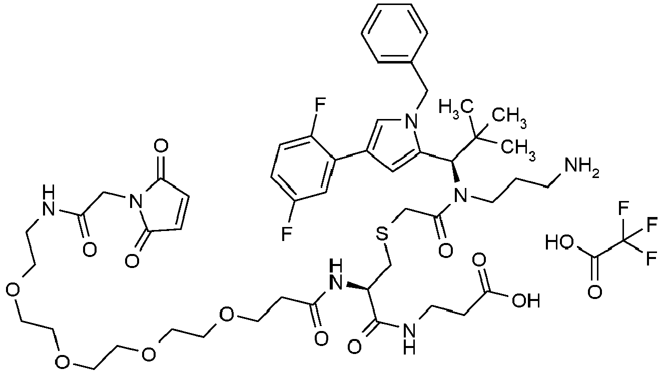

Unter Argon wurden 13.4 mg (33.3 µmol) 1-(2,5-Dioxo-2,5-dihydro-1H-pyrrol-1-yl)-2-oxo-6,9,12,15-tetraoxa-3-azaoctadecan-18-säure (Intermediat L74) in 1.0 ml DMF vorgelegt, mit 9.3 µl (54.4 µmol) N,N-Diisopropylethylamin und 12.6 mg (33.3 µmol) HATU versetzt. Die Reaktionsmischung wurde 10 min bei RT gerührt. 25.0 mg (27.7 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin -trifluoressigsäure (1:1) (siehe Synthese Intermediat F216) gelöst in 4,7 µl (27.7 µmol) N,N-Diisopropylethylamin und 1.0 mL DMF wurden dann zugegeben. Die Reaktionsmischung wurde 90 Minuten bei RT gerührt. Die Reaktionsmischung wurde direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 6.90 mg (19 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[1-(2,5-dioxo-2,5-dihydro-1H-pyrrol-1-yl)-2,18-dioxo-6,9,12,15-tetraoxa-3-azaoctadecan-18-yl]-L-cysteinyl-beta-alanin.

LC-MS (Methode 5): Rₜ = 4.44 min; MS (ESIpos): m/z = 1172 (M+H)⁺.

6.70 mg (5.71 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[1-(2,5-dioxo-2,5-dihydro-1H-pyrrol-1-yl)-2,18-dioxo-6,9,12,15-tetraoxa-3-azaoctadecan-18-yl]-L-cysteinyl-beta-alanin wurden in 1.0 mL Trifluorethanol gelöst und mit 4.67 mg (34.3 µmol) Zinkdichlorid versetzt. Die Reaktionsmischung rührte 1 h bei 50 °C. Die Reaktionsmischung wurde mit 10 mg (34.3 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 4.4 mg (67 % d. Th.) der Titel Verbindung.

LC-MS (Methode 1): Rₜ = 0.85 min; MS (ESIpos): m/z = 1028 (M+H)⁺.

### Intermediat F268

### Trifluoressigsäure N-(3-aminopropyl)-N-{(1R)-1-[1-benzyl-4-(2,5-difluorphenyl)-1H-pyrrol-2-yl]-2,2-dimethylpropyl}-28-(2,5-dioxo-2,5-dihydro-1H-pyrrol-1-yl)-6,23-dioxo-10,13,16,19-tetraoxa-3-thia-7,22-diazaoctacosan-1-amid (1:1)

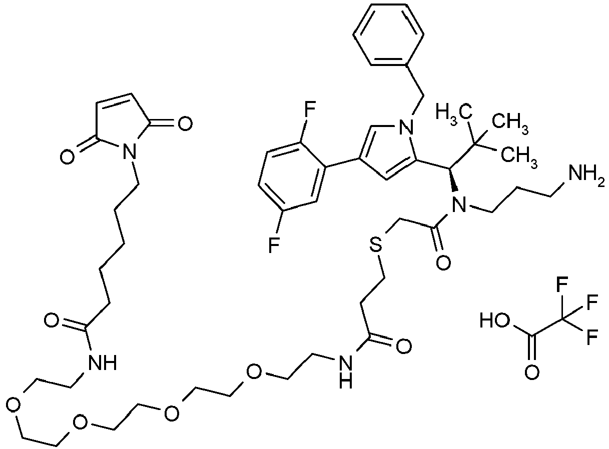

30.0 mg (0.043 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden zusammen mit 30.2 mg (0.056 mmol) Trifluoressigsäure N-(14-amino-3,6,9,12-tetraoxatetradec-1-yl)-6-(2,5-dioxo-2,5-dihydro-1H-pyrrol-1-yl)hexanamid (1:1) (Intermediat L84) in 2.0 mL Acetonitril vorgelegt. Dann wurden 60 µl (0.34 mmol) N,N-Diisopropylethylamin zugegeben und 33 µl (0.056 mmol) T3P (50 % in Ethylacetat) zugetropft. Die Reaktionsmischung wurde über Nacht bei RT gerührt. Es wurde Wasser (2.0 mL) zugegeben. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 27.9 mg (59 % d. Th.) der Verbindung 2-(Trimethylsilyl)ethyl-[4-{(1R)-1-[1-benzyl-4-(2,5-difluorphenyl)-1H-pyrrol-2-yl]-2,2-dimethylpropyl}-32-(2,5-dioxo-2,5-dihydro-1H-pyrrol-1-yl)-5,10,27-trioxo-14,17,20,23-tetraoxa-7-thia-4,11,26-triazadotriacont-1-yl]carbamat.

LC-MS (Methode 1): Rₜ = 1.41 min; MS (ESIpos): m/z = 1114 (M+H)⁺.

25.6 mg (23.0 µmol) 2-(Trimethylsilyl)ethyl-[4-{(1R)-1-[1-benzyl-4-(2,5-difluorphenyl)-1H-pyrrol-2-yl]-2,2-dimethylpropyl}-32-(2,5-dioxo-2,5-dihydro-1H-pyrrol-1-yl)-5,10,27-trioxo-14,17,20,23-tetraoxa-7-thia-4,11,26-triazadotriacont-1-yl]carbamat wurden in 2.5 mL Trifluorethanol gelöst und mit 18.8 mg (138 µmol) Zinkdichlorid versetzt. Die Reaktionsmischung rührte 1 h bei 50 °C. Die Reaktionsmischung wurde mit 40.3 mg (138 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 22.2 mg (88 % d. Th.) der Titel Verbindung.

LC-MS (Methode 1): Rₜ = 0.94 min; MS (ESIpos): m/z = 969 (M+H)⁺.

### Intermediat F269

### 4-{[(8R,14R)-13-(3-Aminopropyl)-14-[1-benzyl-4-(2,5-difluorphenyl)-1H-pyrrol-2-yl]-1-(2,5-dioxo-2,5-dihydro-1H-pyrrol-1-yl)-15,15-dimethyl-2,7,12-trioxo-10-thia-3,6,13-triazahexadecan-8-yl]amino}-4-oxobutansäure -trifluoressigsäure (1:1)

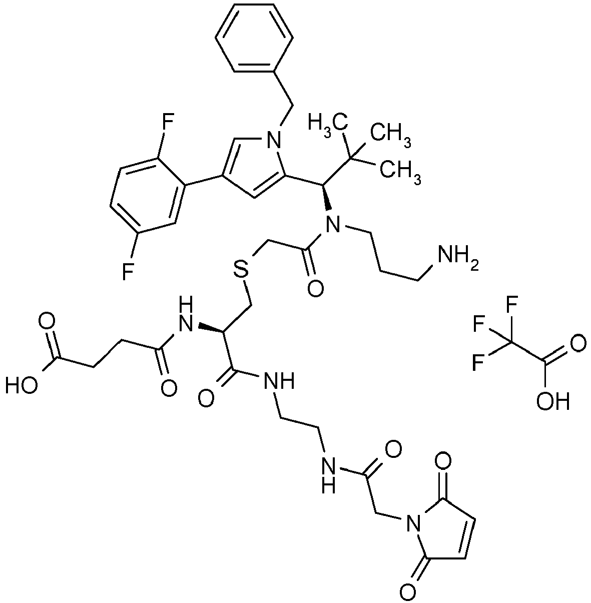

17.0 mg (0.0195 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) wurden zusammen mit 4.99 mg (0.0253 mmol) N-(2-Aminoethyl)-2-(2,5-dioxo-2,5-dihydro-1H-pyrrol-1-yl)acetamid (Intermediat L1) in 1.0 mL Acetonitril vorgelegt. Dann wurden 27 µl (0.16 mmol) N,N-Diisopropylethylamin zugegeben und 15 µl (0.025 mmol) T3P (50 % in Ethylacetat) zugetropft. Die Reaktionsmischung wurde über Nacht bei RT gerührt. Es wurde Wasser (2.0 mL) zugegeben. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 9.5 mg (46 % d. Th.) der Verbindung tert-Butyl-4-{[(16R)-11-{(1R)-1-[1-benzyl-4-(2,5-difluorphenyl)-1H-pyrrol-2-yl]-2,2-dimethylpropyl}-23-(2,5-dioxo-2,5-dihydro-1H-pyrrol-1-yl)-2,2-dimethyl-6,12,17,22-tetraoxo-5-oxa-14-thia-7,11,18,21-tetraaza-2-silatricosan-16-yl]amino } -4-oxobutanoat.

LC-MS (Methode 1): Rₜ = 1.47 min; MS (ESIpos): m/z = 1052 (M+H)⁺.

8.3 mg (7.89 µmol) tert-Butyl-4-{[(16R)-11-{(1R)-1-[1-benzyl-4-(2,5-difluorphenyl)-1H-pyrrol-2-yl]-2,2-dimethylpropyl}-23-(2,5-dioxo-2,5-dihydro-1H-pyrrol-1-yl)-2,2-dimethyl-6,12,17,22-tetraoxo-5-oxa-14-thia-7,11,18,21-tetraaza-2-silatricosan-16-yl]amino}-4-oxobutanoat wurden in 1.0 mL Trifluorethanol gelöst und mit 6.45 mg (47.3 µmol) Zinkdichlorid versetzt. Die Reaktionsmischung rührte 6 h bei 50 °C. 6.45 mg (47.3 µmol) Zinkdichlorid wurden zugegeben und die Reaktionsmischung rührte über Nacht bei 50 °C Die Reaktionsmischung wurde mit 27.7 mg (94.6 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 1.10 mg (14 % d. Th.) der Titel Verbindung.

LC-MS (Methode 1): Rₜ = 0.89 min; MS (ESIpos): m/z = 852 (M+H)⁺.

### Intermediat F270

### Trifluoressigsäure --N-(3-aminopropyl)-N-{(1R)-1-[1-benzyl-4-(2,5-difluorphenyl)-1H-pyrrol-2-yl]-2,2-dimethylpropyl}-N'-(2-{[(2,5-dioxo-2,5-dihydro-1H-pyrrol-1-yl)acetyl]amino}ethyl)succinamid (1:1)

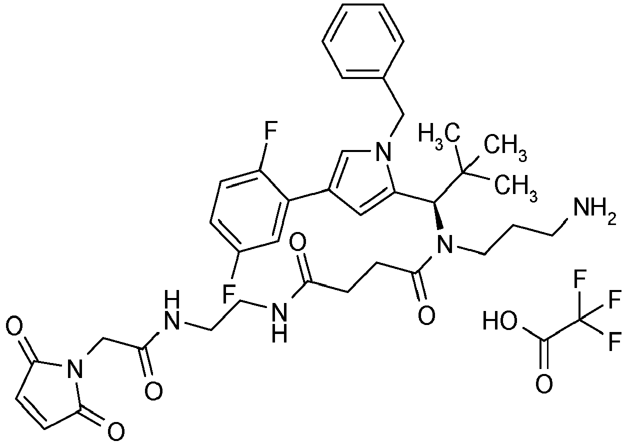

Unter Argon wurden 15.0 mg (22.9 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-säure (Intermediat C78) in 1.0 ml DMF vorgelegt, mit 8.0 µl (45.8 µmol) N,N-Diisopropylethylamin und 10.4 mg (27.4 µmol) HATU versetzt. Die Reaktionsmischung wurde 10 min bei RT gerührt. 8.54 mg (27.4 µmol) Trifluoressigsäure N-(2-aminoethyl)-2-(2,5-dioxo-2,5-dihydro-1H-pyrrol-1-yl)acetamid (1:1) (Intermediat L1) gelöst in 4.0 µl (22.9 µmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 14.7 mg (77 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl} {4-[(2-{ [(2,5-dioxo-2,5-dihydro-1H-pyrrol-1-yl)acetyl] amino } ethyl)amino]-4-oxobutanoyl} amino)propyl] carbamat.

LC-MS (Methode 5): Rₜ = 1.33 min; MS (ESIpos): m/z = 835 (M+H)⁺.

13.2 mg (15.8 µmol) 2-(Trimethylsilyl)ethyl-[3-({(1R)-1-[1-benzyl-4-(2,5-difluorphenyl)-1H-pyrrol-2-yl]-2,2-dimethylpropyl}{4-[(2-{[(2,5-dioxo-2,5-dihydro-1H-pyrrol-1-yl)acetyl]amino}ethyl)amino]-4-oxobutanoyl}amino)propyl]carbamat wurden in 2.0 mL Trifluorethanol gelöst und mit 12.9 mg (94.8 µmol) Zinkdichlorid versetzt. Die Reaktionsmischung rührte 1 h bei 50 °C. Die Reaktionsmischung wurde mit 27.7 mg (94.6 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 10.9 mg (83 % d. Th.) der Titel Verbindung.

LC-MS (Methode 1): Rₜ = 0.83 min; MS (ESIpos): m/z = 691 (M+H)⁺.

### Intermediat F271

### 4-{[(20R,26R)-25-(3-Aminopropyl)-26-[1-benzyl-4-(2,5-difluorphenyl)-1H-pyrrol-2-yl]-1-(2,5-dioxo-2,5-dihydro-1H-pyrrol-1-yl)-27,27-dimethyl-2,19,24-trioxo-6,9,12,15-tetraoxa-22-thia-3,18,25-triazaoctacosan-20-yl]amino}-4-oxobutansäure -trifluoressigsäure (1:1)

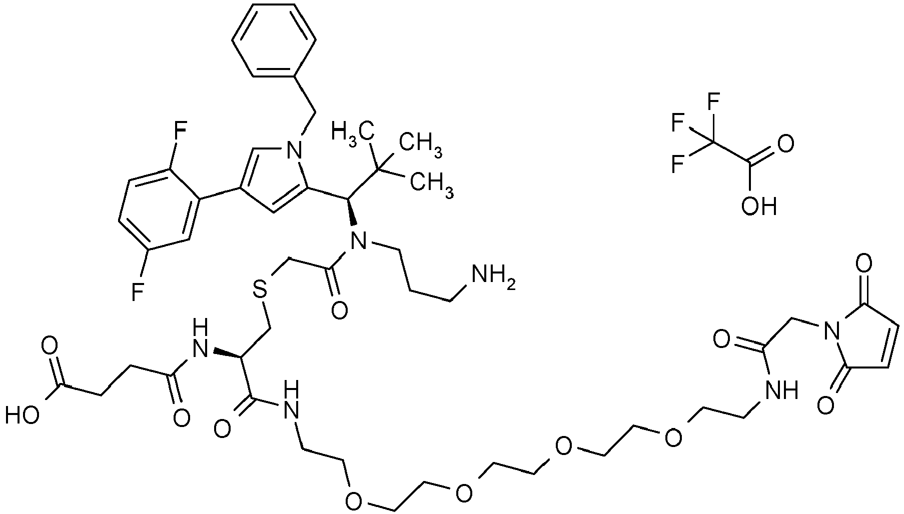

Unter Argon wurden 19.4 mg (22.2 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) in 2.0 ml DMF vorgelegt und mit 21.7 mg (44.4 µmol) Trifluoressigsäure --N-(14-amino-3,6,9,12-tetraoxatetradec-1-yl)-2-(2,5-dioxo-2,5-dihydro-1H-pyrrol-1-yl)acetamid (1:1) (Intermediat L74), 12 µl (67 µmol) N,N-Diisopropylethylamin und 16.9 mg (44.4 µmol) HATU versetzt. Die Reaktionsmischung wurde 1 h bei RT gerührt. Die Reaktionsmischung wurde direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 18.1 mg (66 % d. Th.) der Verbindung tert-Butyl-4-{[(16R)-11-{(1R)-1-[1-benzyl-4-(2,5-difluorphenyl)-1H-pyrrol-2-yl]-2,2-dimethylpropyl}-35-(2,5-dioxo-2,5-dihydro-1H-pyrrol-1-yl)-2,2-dimethyl-6,12,17,34-tetraoxo-5,21,24,27,30-pentaoxa-14-thia-7,11,18,33-tetraaza-2-silapentatriacontan-16-yl]amino}-4-oxobutanoat.

LC-MS (Methode 4): Rₜ = 1.79 min; MS (ESIpos): m/z = 1250 (M+Na)⁺.

18.1 mg (14.7 µmol) tert-Butyl-4-{[(16R)-11-{(1R)-1-[1-benzyl-4-(2,5-difluorphenyl)-1H-pyrrol-2-yl]-2,2-dimethylpropyl}-35-(2,5-dioxo-2,5-dihydro-1H-pyrrol-1-yl)-2,2-dimethyl-6,12,17,34-tetraoxo-5,21,24,27,30-pentaoxa-14-thia-7,11,18,33-tetraaza-2-silapentatriacontan-16-yl]amino}-4-oxobutanoat wurden in 2.0 mL Trifluorethanol gelöst und mit 12.0 mg (88.4 µmol) Zinkdichlorid versetzt. Die Reaktionsmischung rührte 4 h bei 50 °C. Die Reaktionsmischung wurde mit 25.8 mg (88.4 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 12.3 mg (73 % d. Th.) der Titel Verbindung.

LC-MS (Methode 1): Rₜ = 0.87 min; MS (ESIpos): m/z = 1028 (M+H)⁺.

### Intermediat F272

### Trifluoressigsäure --N-(3-aminopropyl)-N-{(1R)-1-[1-benzyl-4-(2,5-difluorphenyl)-1H-pyrrol-2-yl]-2,2-dimethylpropyl}-N'-[17-(2,5-dioxo-2,5-dihydro-1H-pyrrol-1-yl)-16-oxo-3,6,9,12-tetraoxa-15-azaheptadec-1-yl]succinamid (1:1)

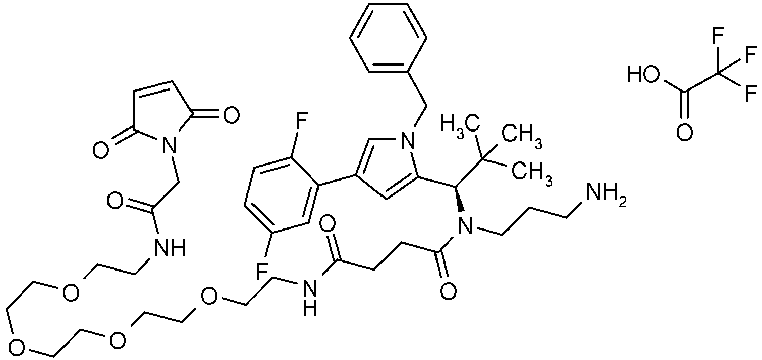

Unter Argon wurden 15.0 mg (22.9 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-säure (Intermediat C78) in 1.0 ml DMF vorgelegt, mit 8.0 µl (45.8 µmol) N,N-Diisopropylethylamin und 10.4 mg (27.4 µmol) HATU versetzt. Die Reaktionsmischung wurde 10 min bei RT gerührt. 13.4 mg (27.4 µmol) Trifluoressigsäure N-(14-amino-3,6,9,12-tetraoxatetradec-1-yl)-2-(2,5-dioxo-2,5-dihydro-1H-pyrrol-1-yl)acetamid (1:1) (Intermediat L85) gelöst in 4.0 µl (22.9 µmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 15.8 mg (68 % d. Th.) der Verbindung 2-(Trimethylsilyl)ethyl-[23-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,22-trioxo-6,9,12,15-tetraoxa-3,18,23-triazahexacosan-26-yl]carbamat.

LC-MS (Methode 1): Rₜ = 1.35 min; MS (ESIpos): m/z = 1011 (M+H)⁺.

15.1 mg (14.9 µmol) 2-(Trimethylsilyl)ethyl-[23-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,22-trioxo-6,9,12,15-tetraoxa-3,18,23-triazahexacosan-26-yl]carbamat wurden in 2.0 mL Trifluorethanol gelöst und mit 12.2 mg (89.6 µmol) Zinkdichlorid versetzt. Die Reaktionsmischung rührte 1 h bei 50 °C. Die Reaktionsmischung wurde mit 26.2 mg (89.6 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 10.3 mg (70 % d. Th.) der Titel Verbindung.

LC-MS (Methode 1): Rₜ = 0.88 min; MS (ESIpos): m/z = 867 (M+H)⁺.

### Intermediat F273

### Trifluoressigsäure N-(3-aminopropyl)-N-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19-dioxo-6,9,12,15-tetraoxa-22-thia-3,18-diazatetracosan-24-amid (1:1)

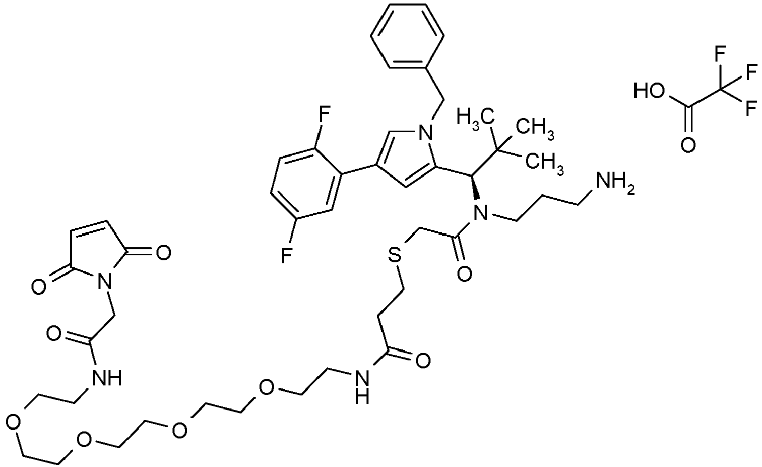

Unter Argon wurden 20.0 mg (28.5 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) in 1.0 ml DMF vorgelegt, mit 10.0 µl (57.0 µmol) N,N-Diisopropylethylamin und 13.0 mg (34.2 µmol) HATU versetzt. Die Reaktionsmischung wurde 10 min bei RT gerührt. 16.7 mg (34.2 µmol) Trifluoressigsäure N-(14-amino-3,6,9,12-tetraoxatetradec-1-yl)-2-(2,5-dioxo-2,5-dihydro-1H-pyrrol-1-yl)acetamid (1:1) (Intermediat L85) gelöst in 5.0 µl (28.5 µmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 18.6 mg (62 % d. Th.) der Verbindung 2-(Trimethylsilyl)ethyl-[25-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,24-trioxo-6,9,12,15-tetraoxa-22-thia-3,18,25-triazaoctacosan-28-yl]carbamat.

LC-MS (Methode 1): Rₜ = 1.37 min; MS (ESIpos): m/z = 1057 (M+H)⁺.

17.1 mg (16.2 µmol) 2-(Trimethylsilyl)ethyl-[25-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,19,24-trioxo-6,9,12,15-tetraoxa-22-thia-3,18,25-triazaoctacosan-28-yl]carbamat wurden in 2.0 mL Trifluorethanol gelöst und mit 13.2 mg (97.0 µmol) Zinkdichlorid versetzt. Die Reaktionsmischung rührte 1 h bei 50 °C. Die Reaktionsmischung wurde mit 28.4 mg (97.0 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 9.80 mg (59 % d. Th.) der Titel Verbindung.

LC-MS (Methode 1): Rₜ = 0.88 min; MS (ESIpos): m/z = 913 (M+H)⁺.

### Intermediat F274

### N-[(2,5-Dioxo-2,5-dihydro-1H-pyrrol-1-yl)acetyl]-L-valyl-L-alanyl-S-{2-[(3-aminopropyl) {(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:1)

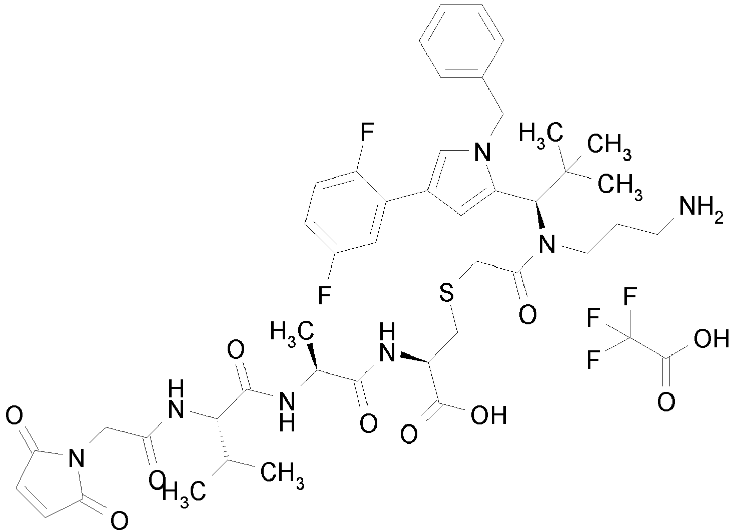

13.9 mg (0.0167 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein - trifluoressigsäure (1:1) (Intermediat C71) wurden zusammen mit 7.07 mg (0.0217 mmol) N-[(2,5-Dioxo-2,5-dihydro-1H-pyrrol-1-yl)acetyl]-L-valyl-L-alanin (Intermediat L86) in 2.0 mL Acetonitril vorgelegt. Dann wurden 23 µl (0.13 mmol) N,N-Diisopropylethylamin zugegeben und 13 µl (0.022 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 3.70 mg (19 % d. Th.) der Verbindung N-[(2,5-Dioxo-2,5-dihydro-1H-pyrrol-1-yl)acetyl]-L-valyl-L-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein.

LC-MS (Methode 1): Rₜ = 1.34 min; MS (ESIpos): m/z = 1024 (M+H)⁺.

10.6 mg (10.3 µmol) N-[(2,5-Dioxo-2,5-dihydro-1H-pyrrol-1-yl)acetyl]-L-valyl-L-alanyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 8.46 mg (62.1 µmol) Zinkdichlorid versetzt. Die Reaktionsmischung rührte 1 h bei 50 °C. Die Reaktionsmischung wurde mit 18.1 mg (62.1 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.60 mg (54 % d. Th.) der Titel Verbindung.

LC-MS (Methode 12): Rₜ = 1.69 min; MS (ESIpos): m/z = 880 (M+H)⁺.

### Intermediat F275

### N-[3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-N-(2-{[(2,5-dioxo-2,5-dihydro-1H-pyrrol-1-yl)acetyl]amino}ethyl)-L-alpha-glutamin -trifluoressigsäure (1:1)

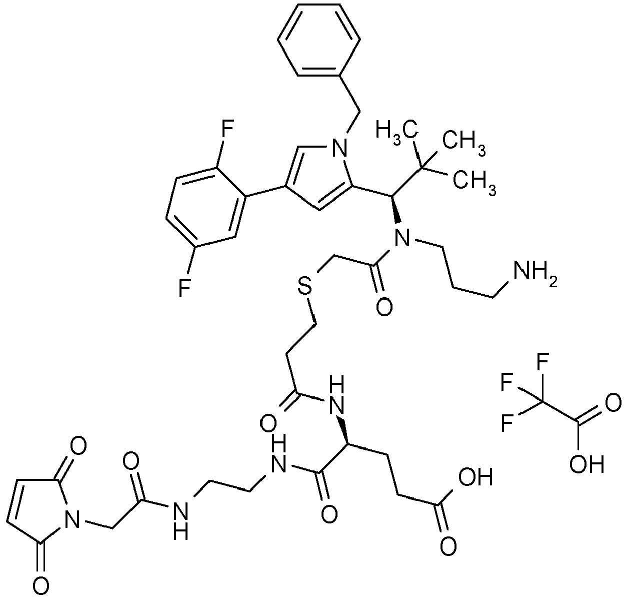

39.0 mg (55.6 µmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure (Intermediat C69) wurden in 4.0 mL DMF vorgelegt, mit 41.6 mg (111 µmol) 1-Benzyl-5-[2-(trimethylsilyl)ethyl]-L-glutamathydrochlorid (1:1) (Intermediat L89), 29 µl (170 µmol) N,N-Diisopropylethylamin und 42.3 mg (111 µmol) HATU versetzt und 1 Stunde bei RT nachgerührt. Die Reaktionsmischung wurde 1 Stunde bei RT gerührt, mit Essigsäure gequencht und direkt mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 53.1 mg (93 % d. Th.) der Verbindung 1-Benzyl-5-[2-(trimethylsilyl)ethyl]-N-(11-{(1R)-1-[1-benzyl-4-(2,5-difluorphenyl)-1H-pyrrol-2-yl]-2,2-dimethylpropyl} -2,2-dimethyl-6,12,17 -trioxo-5 -oxa-14-thia-7,11 -diaza-2-silaheptadecan-17 -yl)-L-glutamat.

LC-MS (Methode 1): Rₜ = 1.71 min; MS (ESIpos): m/z = 1021 [M+H]⁺

Unter Argon wurden 7.60 mg (33,9 µmol) Palladium(II)acetat in 3.0 ml Dichloromethan vorgelegt und mit 14 µl (100 µmol) Triethylamin und 110 µl (680 µmol) Triethylsilan versetzt. Die Reaktionsmischung wurde 5 min bei RT gerührt und mit 69.2 mg (67.7 µmol) 1-Benzyl-5-[2-(trimethylsilyl)ethyl]-N-(11-{(1R)-1-[1-benzyl-4-(2,5-difluorphenyl)-1H-pyrrol-2-yl]-2,2-dimethylpropyl} -2,2-dimethyl-6,12,17 -trioxo-5 -oxa-14-thia-7,11 -diaza-2-silaheptadecan-17 -yl)-L-glutamat gelöst in 3.0 mL Dichlormethan versetzt. Die Reaktionsmischung wurde über Nacht bei RT gerührt. Die Reaktionsmischung wurde über einen Pappfilter filtriert und der Filterkuchen mit Dichlormethan nachgewaschen. Das Lösemittel wurde im Vakuum verdampft. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 38.4 mg (61 % d. Th.) der Verbindung 19S)-11-{(1R)-1-[1-Benzyl-4-(2,5-difluorphenyl)-1H-pyrrol-2-yl]-2,2-dimethylpropyl}-2,2-dimethyl-6,12,17-trioxo-19-{3-oxo-3-[2-(trimethylsilyl)ethoxy]propyl}-5-oxa-14-thia-7,11,18-triaza-2-silaicosan-20-säure. LC-MS (Methode 1): Rₜ = 1.53 min; MS (ESIpos): m/z = 931 (M+H)⁺.

10.0 mg (10.7 µmol) (19S)-11-{(1R)-1-[1-Benzyl-4-(2,5-difluorphenyl)-1H-pyrrol-2-yl]-2,2-dimethylpropyl}-2,2-dimethyl-6,12,17-trioxo-19-{3-oxo-3-[2-(trimethylsilyl)ethoxy]propyl}-5-oxa-14-thia-7,11,18-triaza-2-silaicosan-20-säure wurden in 1.0 mL DMF vorgelegt, mit 6.73 mg (21.5 µmol) N-(2-Aminoethyl)-2-(2,5-dioxo-2,5-dihydro-1H-pyrrol-1-yl)acetamid 2,2,2-trifluorethan-1,1-diol (1:1) (Intermediat L1), 5.6 µl (32 µmol) N,N-Diisopropylethylamin und 8.17 mg (21.5 µmol) HATU versetzt und 1 Stunde bei RT nachgerührt. Die Reaktionsmischung wurde 3 Stunde bei RT gerührt, mit Essigsäure gequencht und direkt mittels präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 6.90 mg (58 % d. Th.) der Verbindung 2-(Trimethylsilyl)ethyl-N2-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-N-(2-{[(2,5-dioxo-2,5-dihydro-1H-pyrrol-1-yl)acetyl] amino } ethyl) -L-alpha-glutaminat.

LC-MS (Methode 1): Rₜ = 1.57 min; MS (ESIpos): m/z = 1110 [M+H]⁺

6.90 mg (6.21 µmol) 2-(Trimethylsilyl)ethyl-N²-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-N-(2-{[(2,5-dioxo-2,5-dihydro-1H-pyrrol-1-yl)acetyl]amino}ethyl)-L-alpha-glutaminat wurden in 2.0 mL Trifluorethanol gelöst und mit 5.1 mg (37.2 µmol) Zinkdichlorid versetzt. Die Reaktionsmischung rührte 3 h bei 50 °C. 5.1 mg (37.2 µmol) Zinkdichlorid wurden zugegeben und die Reaktionsmischung rührte 3 h bei 50 °C. 5.1 mg (37.2 µmol) Zinkdichlorid wurden zugegeben und die Reaktionsmischung rührte 3 h bei 50 °C. 10.1 mg (74.4 µmol) Zinkdichlorid wurden zugegeben und die Reaktionsmischung rührte über Nacht bei 50 °C und 72 h bei RT. Die Reaktionsmischung wurde mit 54.5 mg (186 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 2.4 mg (39 % d. Th.) der Titel Verbindung.

LC-MS (Methode 1): Rₜ = 0.86 min; MS (ESIpos): m/z = 866 (M+H)⁺.

### Intermediat F276

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-{3-[2-(2-{[(2,5-dioxo-2,5-dihydro-1H-pyrrol-1-yl)acetyl]amino}ethoxy)ethoxy]propanoyl}-L-cystein -trifluoressigsäure (1:1)

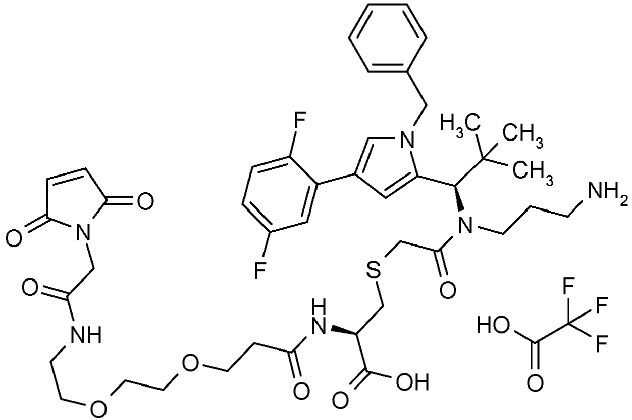

Unter Argon wurden 9.08 mg (28.9 µmol) 3-[2-(2-{[(2,5-Dioxo-2,5-dihydro-1H-pyrrol-1-yl)acetyl]amino}ethoxy)ethoxy]propansäure (Intermediat L87) in 1.0 ml DMF vorgelegt, mit 8.33 µl (48.2 µmol) N,N-Diisopropylethylamin und 11.0 mg (28.9 µmol) HATU versetzt. Die Reaktionsmischung wurde 10 min bei RT gerührt. 20.0 mg (27.7 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (Intermediat C71) gelöst in 4,67 µl (24.1 µmol) N,N-Diisopropylethylamin und 1.0 mL DMF wurden dann zugegeben. Die Reaktionsmischung wurde 1 h bei RT gerührt. Die Reaktionsmischung wurde direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 4.70 mg (19 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{ [(2,5-dioxo-2,5-dihydro-1H-pyrrol-1-yl)acetyl]amino}ethoxy)ethoxy]propanoyl}-L-cystein.

LC-MS (Methode 12): Rₜ = 2.47 min; MS (ESIpos): m/z = 1013 (M+H)⁺.

13.9 mg (13.7 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[2-(2-{[(2,5-dioxo-2,5-dihydro-1H-pyrrol-1-yl)acetyl]amino}ethoxy)ethoxy]propanoyl}-L-cystein wurden in 2.0 mL Trifluorethanol gelöst und mit 5.6 mg (41.2 µmol) Zinkdichlorid versetzt. Die Reaktionsmischung rührte 1 h bei 50 °C. 5.6 mg (41.2 µmol) Zinkdichlorid wurden zugegeben und die Reaktionsmischung rührte 30 Minuten bei 50 °C. Die Reaktionsmischung wurde mit 24.1 mg (82.4 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 10.8 mg (80 % d. Th.) der Titel Verbindung.

LC-MS (Methode 12): Rₜ = 1.58 min; MS (ESIpos): m/z = 869 (M+H)⁺.

### Intermediat F277

### N-[3-({2-[(3-Aminopropyl){(1R)-1-[1-benzyl-4-(2,5-difluorphenyl)-1H-pyrrol-2-yl]-2,2-dimethylpropyl} amino] -2-oxoethyl} sulfanyl)propanoyl] -3-[(bromacetyl)amino] -D-alanin-trifluoressigsäure (1:1)

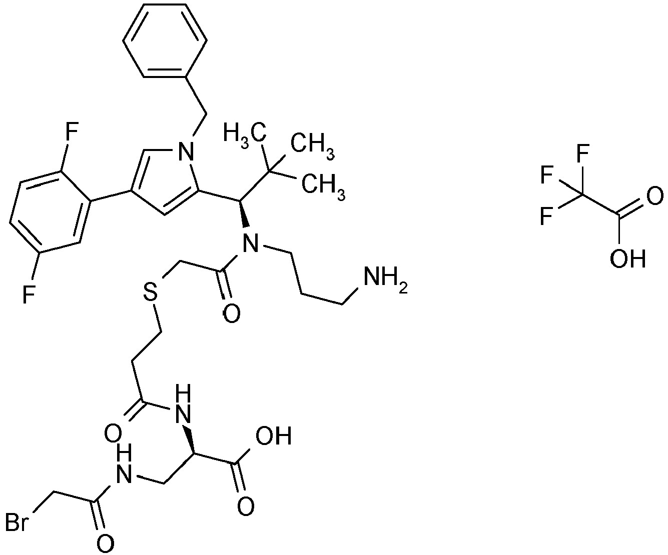

8.90 mg (8.88 µmol) Trifluoressigsäure 2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1) (Intermediat C80) und 2.31 mg (9.77 µmol) 1-(2-Bromacetoxy)pyrrolidin-2,5-dion wurden in 1 ml Dimethylformamid gelöst und mit 2.9 µl (27 µmol) N-Methylmorpholin versetzt. Die Reaktionsmischung wurde 1 h bei RT gerührt. Die Reaktionsmischung wurde direkt mittles präp. RP-HPLC gereinigt (Säule: Reprosil 125x30; 10µ, Fluss: 50 mL/min, MeCN/Wasser/0.1%TFA). Die Lösemittel wurden im Vakuum verdampft und der Rückstand im Hochvakuum getrocknet. Man erhielt 5.80 mg (65% d. Th.) der Verbinfung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-[(bromacetyl)amino] -D-alaninat.

LC-MS (Methode 1): Rₜ = 1.57 min; MS (ESIpos): m/z = 1008 (M+H)⁺.

5.80 mg (5.75 µmol) 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-[(bromacetyl)amino]-D-alaninat wurden in 2.0 mL Trifluorethanol gelöst und mit 4.70 mg (34.5 µmol) Zinkdichlorid versetzt. Die Reaktionsmischung rührte 3 h bei 50 °C. 4.70 mg (34.5 µmol) Zinkdichlorid wurden zugegeben und die Reaktionsmischung rührte 5 h bei 50 °C. Die Reaktionsmischung wurde mit 20.2 mg (69.0 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 1.70 mg (34 % d. Th.) der Titel Verbindung.

LC-MS (Methode 1): Rₜ = 0.90 min; MS (ESIpos): m/z = 764 (M+H)⁺.

### Intermediat F278

### N-[3-({2-[(3-Aminopropyl){(1R)-1-[1-benzyl-4-(2,5-difluorphenyl)-1H-pyrrol-2-yl]-2,2-dimethylpropyl} amino] -2-oxoethyl}sulfanyl)propanoyl]-3-{[(2,5-dioxo-2,5-dihydro-1H-pyrrol-1-yl)acetyl]amino}-D-alanin -trifluoressigsäure (1:1)

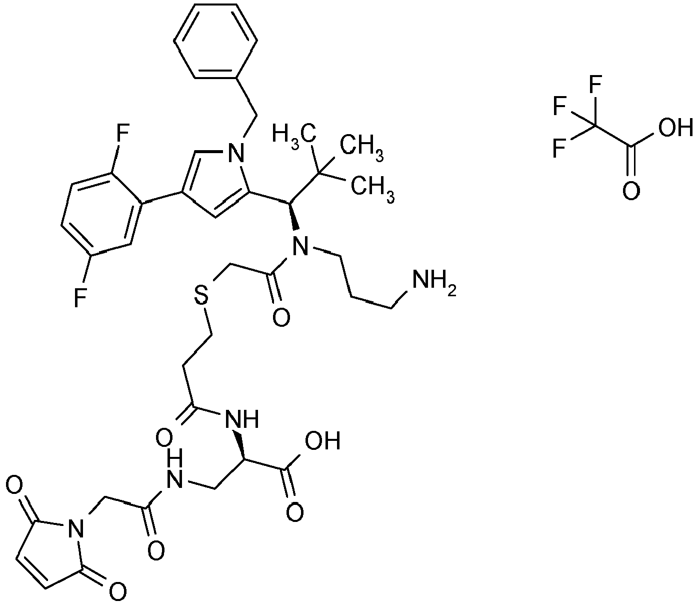

10.0 mg (9.98 µmol) Trifluoressigsäure 2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1) (Intermediat C80) und 2.77 mg (11.0 µmol) 1-{2-[(2,5-Dioxopyrrolidin-1-yl)oxy]-2-oxoethyl}-1H-pyrrol-2,5-dion wurden in 1 ml Dimethylformamid gelöst und mit 3.3 µl (30 µmol) N-Methylmorpholin versetzt. Die Reaktionsmischung wurde über Nacht bei RT gerührt. Die Reaktionsmischung wurde mit 2.0 µl (35 µmol) Essigsaeure versetzt und direkt mittles präp. RP-HPLC gereinigt (Säule: Reprosil 125x30; 10µ, Fluss: 50 mL/min, MeCN/Wasser/0.1%TFA). Die Lösemittel wurden im Vakuum verdampft und der Rückstand im Hochvakuum getrocknet. Man erhielt 5.50 mg (54% d. Th.) der Verbinfung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{[(2,5-dioxo-2,5-dihydro-1H-pyrrol-1-yl)acetyl]amino}-D-alaninat.

LC-MS (Methode 1): Rₜ = 1.51 min; MS (ESIpos): m/z = 1024 (M+H)⁺.

5.50 mg (5.36 µmol) 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-{ [(2,5-dioxo-2,5-dihydro-1H-pyrrol-1-yl)acetyl] amino}-D-alaninat wurden in 1.0 mL Trifluorethanol gelöst und mit 4.39 mg (32.2 µmol) Zinkdichlorid versetzt. Die Reaktionsmischung rührte 1 h bei 50 °C. 4.39 mg (32.2 µmol) Zinkdichlorid wurden zugegeben und die Reaktionsmischung rührte 1 h bei 50 °C. . 4.39 mg (32.2 µmol) Zinkdichlorid wurden zugegeben und die Reaktionsmischung rührte 4 h bei 50 °C. Die Reaktionsmischung wurde mit 28.2 mg (96.5 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 2.70 mg (56 % d. Th.) der Titel Verbindung.

LC-MS (Methode 1): Rₜ = 0.89 min; MS (ESIpos): m/z = 781 (M+H)⁺.

### Intermediat F279

### N-[6-(2,5-Dioxo-2,5-dihydro-1H-pyrrol-1-yl)hexanoyl]-L-valyl-N-[3-({(1R)-1-[1-benzyl-4-(2,5-difluorphenyl)-1H-pyrrol-2-yl]-2,2-dimethylpropyl}[({(2R)-2-carboxy-2-[(3-carboxypropanoyl)amino]ethyl}sulfanyl)acetyl]amino)propyl]-L-alaninamid

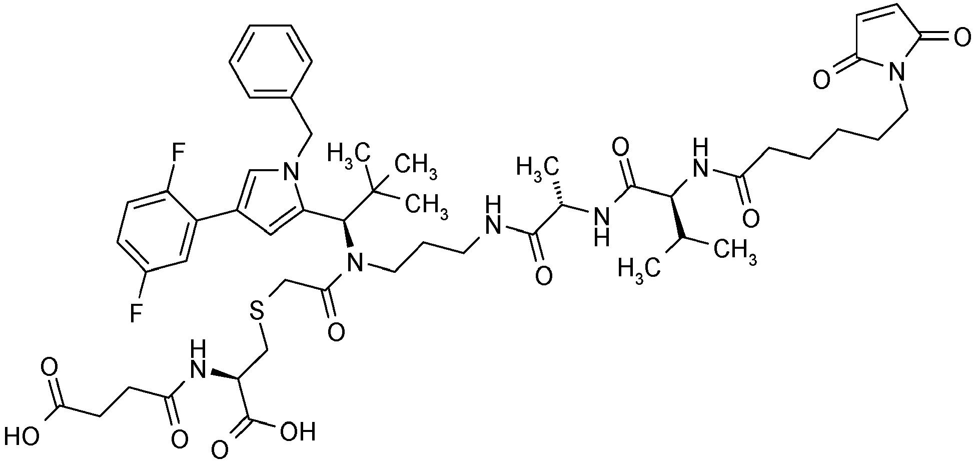

12.2 mg (14 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) wurden in 2.0 mL Trifluorethanol gelöst und mit 11.4 mg (83.8 µmol) Zinkdichlorid versetzt. Die Reaktionsmischung rührte 3 h bei 50 °C. Die Reaktionsmischung wurde mit 24.5 mg (83.8 µmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 4.60 mg (42 % d. Th.) der Verbindung 4-{[(1R)-2-({2-[(3-Aminopropyl){(1R)-1-[1-benzyl-4-(2,5-difluorphenyl)-1H-pyrrol-2-yl]-2,2-dimethylpropyl} amino]-2-oxoethyl}sulfanyl)-1-carboxyethyl]amino } -4-oxobutansäure-trifluoressigsäure (1:1).

LC-MS (Methode 1): Rₜ = 0.88 min; MS (ESIpos): m/z = 673 (M+H)⁺.

10.0 mg (12.7 µmol) 4-{[(1R)-2-({2-[(3-Aminopropyl){(1R)-1-[1-benzyl-4-(2,5-difluorphenyl)-1H-pyrrol-2-yl]-2,2-dimethylpropyl} amino] -2-oxoethyl} sulfanyl)-1-carboxyethyl]amino}-4-oxobutansäure -trifluoressigsäure (1:1) und 7.41 mg (12.7 µmol) 2,5-Dioxopyrrolidin-1-yl-N-[6-(2,5-dioxo-2,5-dihydro-1H-pyrrol-1-yl)hexanoyl]-L-valyl-L-alaninat (Intermediat L88) wurden in 1.5 ml Dimethylformamid gelöst und mit 4.4 µl (25 µmol) N,N-Diisopropylethylamin versetzt. Die Reaktionsmischung wurde 2 h bei RT gerührt. Die Reaktionsmischung wurde mit 2.0 µl (35 µmol) Essigsaeure versetzt und direkt mittles präp. RP-HPLC gereinigt (Säule: Reprosil 250x30; 10µ, Fluss: 50 mL/min, MeCN/Wasser/0.1%TFA). Die Lösemittel wurden im Vakuum verdampft und der Rückstand im Hochvakuum getrocknet. Man erhielt 5.20 mg (39% d. Th.) der Titel Verbinfung.

LC-MS (Methode 1): Rₜ = 1.11 min; MS (ESIpos): m/z = 1036 (M+H)⁺.

### Intermediat F280

### Trifluoressigsäure --N-[2-({(2S)-2-amino-4-[{(1R)-1-[1-benzyl-4-(2,5-difluorphenyl)-1H-pyrrol-2-yl] -2,2-dimethylpropyl} (glycoloyl)amino]butanoyl} amino)ethyl] -3 -(2,5 -dioxo-2,5 -dihydro- 1H-pyrrol-1-yl)benzamid (1:1)

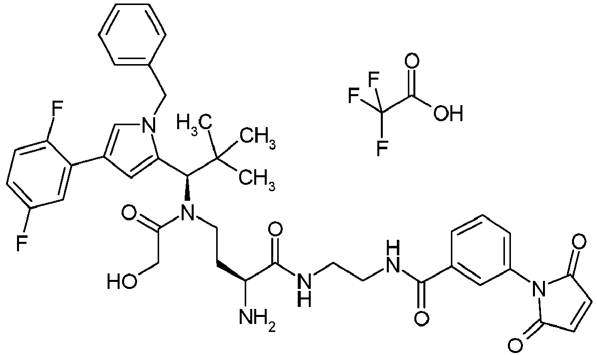

Die Titelverbindung wurde ausgehend von Intermediat C64 durch Kupplung mit kommerziell erhältlichem 1-(3-{[(2,5-Dioxopyrrolidin-1-yl)oxy]carbonyl}phenyl)-1H-pyrrol-2,5-dion und anschließender Entschützung mit Zinkchlorid hergestellt.

LC-MS (Methode 1): Rₜ = 0.88 min; MS (ESIpos): m/z = 755 (M+H)⁺.

### Intermediat F281

### N-{(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} -3-{ [N-(bromacetyl)-beta-alanyl]amino } -D-alanintrifluoressigsäure (1:1)

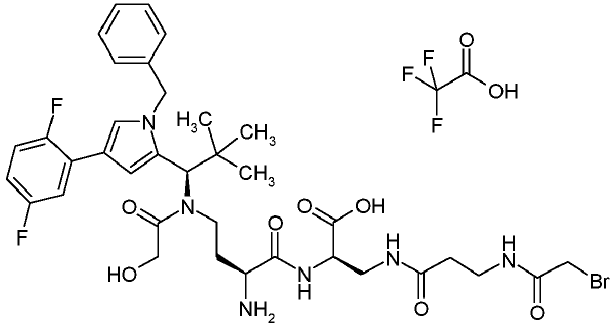

Zunächst wurden die modifizierten Aminosäurebausteine N-(Bromacetyl)-beta-alanin sowie 2-(Trimethylsilyl)ethyl-3-amino-N-(tert-butoxycarbonyl)-D-alaninat nach klassischen Methoden der Peptidchemie hergestellt. Anschließend wurden diese in Gegenwart von HATU und Morpholin miteinander gekuppelt. Dann wurde mittels 10%-iger Trifluoressigsäure in Dichlormethan die tert. Butoxycarbonyl-Schutzgruppe abgelöst und so das Intermediat 2-(Trimethylsilyl) ethyl-3-{[N-(bromacetyl)-beta-alanyl]amino}-D-alaninat erhalten.

Schließlich wurde die Titelverbindung durch Kupplung dieses Intermediats mit Intermediat C58 in Gegenwart von HATU und 4-Methylmorpholin und anschließender Entschützung mit Zinkchlorid hergestellt.

LC-MS (Methode 1): Rₜ = 0.87 min; MS (ESIpos): m/z = 791 und 793 (M+H)⁺.

### Intermediat F282

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-(3-{[N-(bromacetyl)glycyl]amino}propyl)butanamid (1:1)

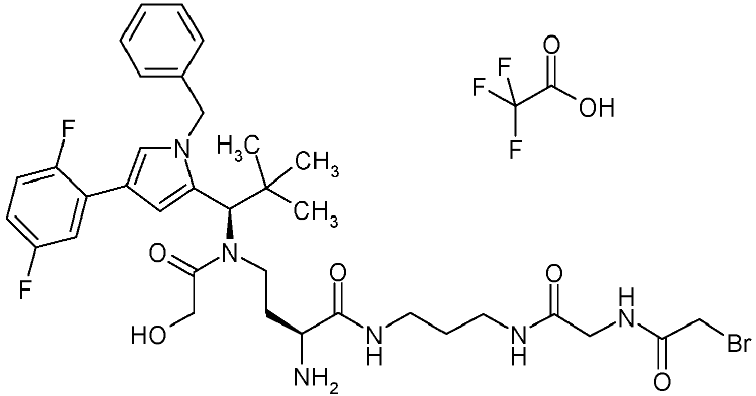

Zunächst wurde nach klassischen Methoden der Peptidchemie ausgehend von tert-Butylglycinat und Bromessigsäureanhydrid das Intermediat Trifluoressigsäure --N-(3-aminopropyl)-N2-(bromacetyl)glycinamid (1:1) hergestellt.

Schließlich wurde die Titelverbindung durch Kupplung dieses Intermediats mit Intermediat C58 in Gegenwart von HATU und 4-Methylmorpholin und anschließender Entschützung mit Zinkchlorid hergestellt.

LC-MS (Methode 1): Rₜ = 0.83 min; MS (ESIpos): m/z = 747 und 749 (M+H)⁺.

### Intermediat F283

### N-[(2R)-2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}amino)-2-carboxyethyl]-N²-(bromacetyl)-L-alpha-asparagin -trifluoressigsäure (1:1)

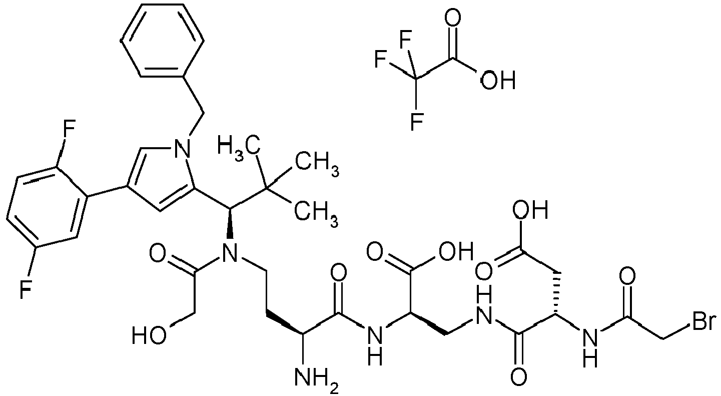

Zunächst wurde ausgehend von (2S)-2-Amino-4-oxo-4-[2-(trimethylsilyl)ethoxy]butansäure und Bromessigsäureanhydrid der modifizierten Aminosäurebaustein (2S)-2-[(Bromacetyl)amino]-4-oxo-4-[2-(trimethylsilyl)ethoxy]butansäure sowie ausgehend von kommerziell erhältlichem 3-{[(Benzyloxy)carbonyl]amino}-N-(tert-butoxycarbonyl)-D-alanin --N-cyclohexylcyclohexanamin (1:1) der Aminosäurebaustein 2-(Trimethylsilyl)ethyl-3-amino-N-(tert-butoxycarbonyl)-D-alaninat hergestellt. Beide Bausteine wurden in Gegenwart von HATU und Morpholin miteinander gekuppelt und anschließend wurde mittels 5%-iger Trifluoressigsäure in Dichlormethan die tert. Butoxycarbonyl-Schutzgruppe unter Erhalt der Silylethylester-Schutzgruppen abgelöst und so das Intermediat Trifluoressigsäure --2-(trimethylsilyl)ethyl-N-{(2R)-2-amino-3-oxo-3-[2-(trimethylsilyl)ethoxy] propyl}-N2-(bromacetyl)-L-alpha-asparaginat (1:1) erhalten.

Schließlich wurde die Titelverbindung durch Kupplung dieses Intermediats mit Intermediat C58 in Gegenwart von HATU und 4-Methylmorpholin und anschließender Entschützung mit Zinkchlorid hergestellt.

LC-MS (Methode 1): Rₜ = 0.84 min; MS (ESIpos): m/z = 835 und 837 (M+H)⁺.

### Intermediat F284

### N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[1-(2,5-dioxo-2,5-dihydro-1H-pyrrol-1-yl)-2,18-dioxo-6,9,12,15-tetraoxa-3-azaoctadecan-18-yl]amino}-D-alanin -trifluoressigsäure (1:1)

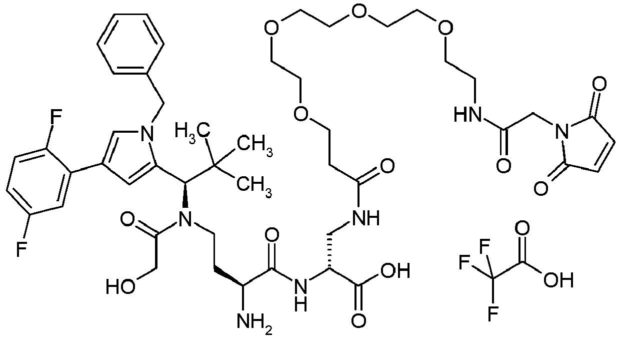

Zunächst wurde Intermediat L80 mit kommerziell erhältlicher (2,5-Dioxo-2,5-dihydro-1H-pyrrol-1-yl)essigsäure in Gegenwart von HATU und N,N-Diisopropylethylamin gekuppelt und anschließend wurde mittels 16%-iger Trifluoressigsäure in Dichlormethan die tert. Butoxycarbonyl-Schutzgruppe unter Erhalt der Silylethylester-Schutzgruppe abgelöst.

Schließlich wurde die Titelverbindung durch Kupplung dieses Intermediats mit Intermediat C58 in Gegenwart von HATU und N, N-Diisopropylethylamin und anschließender Entschützung mit Zinkchlorid hergestellt.

LC-MS (Methode 12): Rₜ = 1.46 min; MS (ESIpos): m/z = 984.45 (M+H)⁺.

### Intermediat F285

### N-{(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}-3-[(18-brom-17-oxo-4,7,10, 13-tetraoxa-16-azaoctadecan-1-oyl)amino]-D-alanin -trifluoressigsäure (1:1)

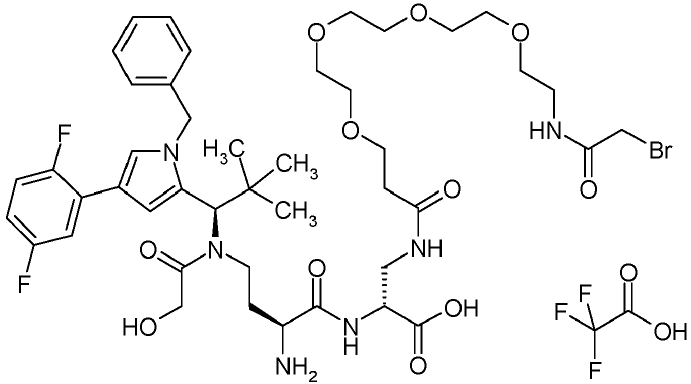

Zunächst wurde Intermediat L80 mit kommerziell erhältlichem Bromessigsäureanhydrid acyliert und anschließend wurde mittels 20%-iger Trifluoressigsäure in Dichlormethan die tert. Butoxycarbonyl-Schutzgruppe unter Erhalt der Silylethylester-Schutzgruppe abgelöst.

Schließlich wurde die Titelverbindung durch Kupplung dieses Intermediats mit Intermediat C58 in Gegenwart von HATU und N, N-Diisopropylethylamin und anschließender Entschützung mit Zinkchlorid hergestellt.

LC-MS (Methode 1): Rₜ = 0.85 min; MS (ESIpos): m/z = 967 und 969 (M+H)⁺.

### Intermediat F286

### 1-[(N-{(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} -3 - {[(2,5 -dioxo-2,5 -dihydro- 1H-pyrrol-1-yl) acetyl]amino}-D-alanyl)amino]-3,6,9,12-tetraoxapentadecan-15-säure -trifluoressigsäure (1:1)

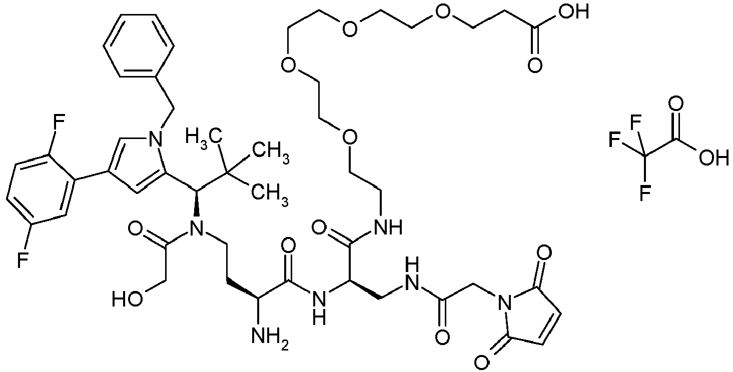

Zunächst wurde Intermediat L91 mit (2,5-Dioxo-2,5-dihydro-1H-pyrrol-1-yl)essigsäure in Gegenwart von HATU und N, N Diisopropylethylamin gekuppelt und anschließend wurde mit 12.5%-iger TFA in DCM die Boc-Schutzgruppe abgelöst. Das so erhaltene Intermediat wurde mit Intermediat C58 in Gegenwart von HATU und N, N Diisopropylethylamin gekuppelt und anschließend durch Entschützung mit Zinkchlorid in die Titelverbindung überführt.

LC-MS (Methode 1): Rₜ = 0.84 min; MS (ESIpos): m/z = 984 (M+H)⁺.

### Intermediat F288

### N-{(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}-3-({N-[(2,5-dioxo-2,5-dihydro-1H-pyrrol-1-yl)acetyl]-L-seryl}amino)-D-alanin -trifluoressigsäure (1:1)

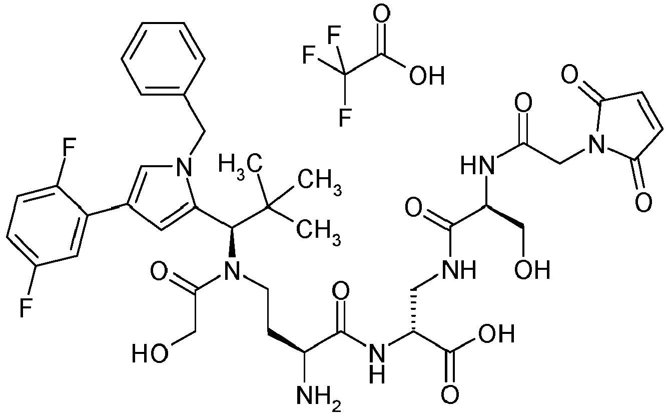

35 mg (39 µmol) von Intermediat C74 wurden in Gegenwart von HATU und N, N-Diisopropyethylamin mit N-[(2,5-Dioxo-2,5-dihydro-1H-pyrrol-1-yl)acetyl]-L-serin gekuppelt, das zuvor ausgehend von tert-Butyl-O-tert-butyl-L-serinat und (2,5-Dioxo-2,5-dihydro-1H-pyrrol-1-yl)essigsäure hergestellt wurde. Nach Entschützung mit Zinkchlorid und HPLC Reinigung wurden 14 mg (38% d. Th.) der Titelverbindung erhalten.

LC-MS (Methode 12): Rₜ = 1.43 min; MS (ESIpos): m/z = 824.34 (M+H)⁺.

### Intermediat F289

### N²-{(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}-N⁶-[(2,5-dioxo-2,5-dihydro-1H-pyrrol-1-yl)acetyl]-D-lysin -trifluoressigsäure (1:1)

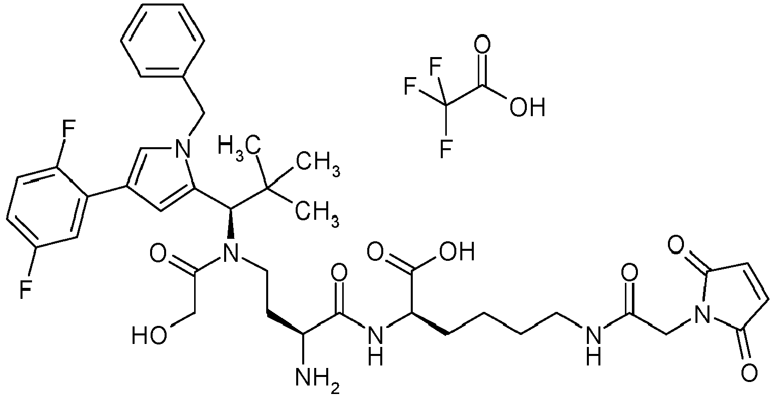

Zunächst wurde Trifluoressigsäure --2-(trimethylsilyl)ethyl-N⁶-[(2,5-dioxo-2,5-dihydro-1H-pyrrol-1-yl)acetyl]-D-lysinat (1:1) nach klassischen Methoden der Peptidchemie ausgehend von N⁶-[(Benzyloxy)carbonyl]-N²-(tert-butoxycarbonyl)-D-lysin hergestellt.

12.5 mg (25 µmol) von diesem Intermediat wurden in Gegenwart von HATU und 4-Methylmorpholin mit 15 mg (23 µmol) von Intermediat C58 gekuppelt. Nach Entschützung mit Zinkchlorid und HPLC Reinigung wurden 14 mg (53% d. Th.) der Titelverbindung erhalten.

LC-MS (Methode 1): Rₜ = 0.83 min; MS (ESIpos): m/z = 779 (M+H)⁺.

### Intermediat F290

### N²-{(2S)-2-Amino-4-[-{(1R)-1-[1-benzyl-4-(2,5-difluorphenyl)-1H-pyrrol-2-yl]-2,2 dimethylpropyl}(glycoloyl)amino]butanoyl}-N⁶-(bromacetyl)-D-lysin -trifluoressigsäure (1:1)

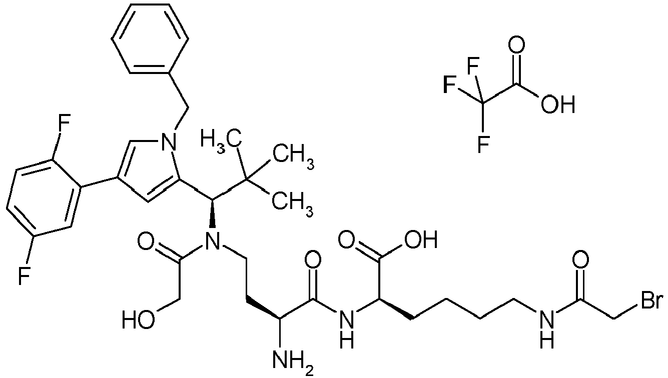

Zunächst wurde Trifluoressigsäure --2-(trimethylsilyl)ethyl-N6-(bromacetyl)-D-lysinat (1:1) nach klassischen Methoden der Peptidchemie ausgehend von N⁶-[(Benzyloxy)carbonyl]-N²-(tert-butoxycarbonyl)-D-lysin hergestellt.

12 mg (25 µmol) von diesem Intermediat wurden in Gegenwart von HATU und 4-Methylmorpholin mit 15 mg (23 µmol) von Intermediat C58 gekuppelt. Nach Entschützung mit Zinkchlorid und HPLC Reinigung wurden 7 mg (36% d. Th.) der Titelverbindung erhalten.

LC-MS (Methode 1): Rₜ = 0.86 min; MS (ESIpos): m/z = 762 und 764 (M+H)⁺.

### Intermediat F291

### N-[(2,5-Dioxo-2,5-dihydro-1H-pyrrol-1-yl)acetyl]-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid

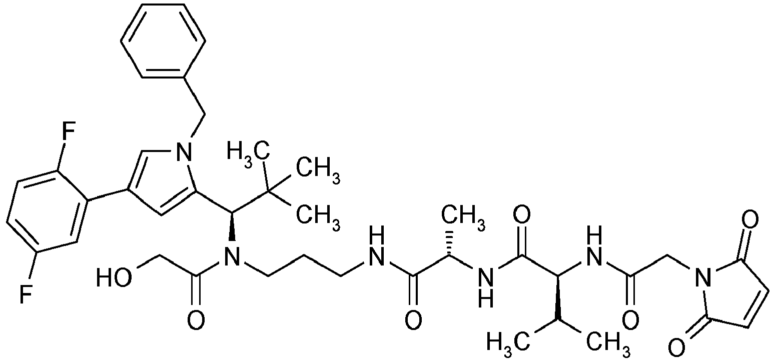

Die Titelverbindung wurde ausgehend von Beispiel M9 zunächst durch Kupplung mit N-[(Benzyloxy)carbonyl]-L-valyl-L-alanin in Gegenwart von HATU und N,N-Diisopropylethylamin hergestellt. Im nächsten Schritt wurde die Z-Schutzgruppe durch 1-stündige Hydrierung über 10%-igem Palladium auf Aktivkohle bei RT unter Wasserstoff-Normaldruck entfernt und das entschützte Intermediat anschließend durch Kupplung mit (2,5-Dioxo-2,5-dihydro-1H-pyrrol-1-yl)essigsäure in Gegenwart von HATU und N, N Diisopropylethylamin in die Titelverbindung überführt.

LC-MS (Methode 1): Rₜ = 1.21 min; MS (ESIpos): m/z = 777 (M+H)⁺.

### Intermediat F293

### N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[3-(2,5-dioxo-2,5-dihydro-1H-pyrrol-1-yl)benzoyl]amino}-D-alanin -trifluoressigsäure (1:1)

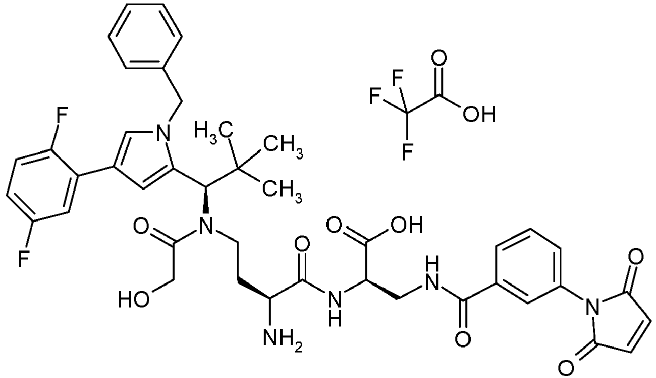

35 mg (39 µmol) von Intermediat C74 wurden in 4 ml DMF gelöst und in Gegenwart von N, N-Diisopropylethylamin mit 13.5 mg (43 µmol) kommerziell erhältlichem 1-(3-{[(2,5-Dioxopyrrolidin-1-yl)oxy] carbonyl}phenyl)-1H-pyrrol-2,5-dion gekuppelt. Nach Entschützung mit Zinkchlorid und HPLC Reinigung wurden 12 mg (34% d. Th.) der Titelverbindung erhalten.

LC-MS (Methode 12): Rₜ = 0.93 min; MS (ESIpos): m/z = 799 (M+H)⁺.

### Intermediat F294

### N-{5-[(2,5-Dioxopyrrolidin-1-yl)oxy]-5-oxopentanoyl}-L-valyl-N-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-alaninamid

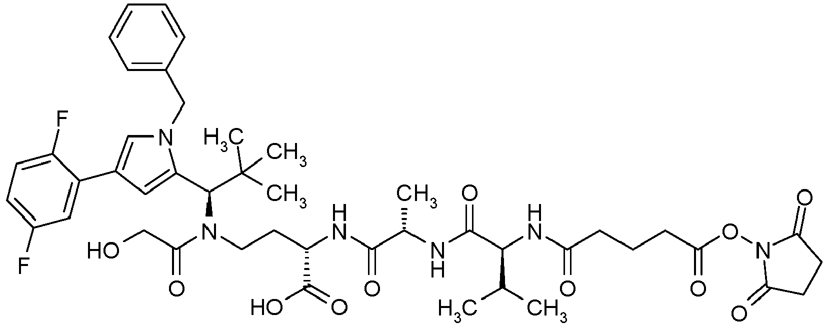

41 mg (0.05 mmol) von Intermediat C76 gelöst in 12 mL Methanol wurden über 10 mg 10%-igem Palladium auf Aktivkohle 1 h lang bei RT unter Wasserstoff-Normaldruck hydriert. Der Katalysator wurde danach abfiltriert und das Lösungsmittel im Vakuum entfernt. Man erhielt 32 mg (92% d.Th.) des entschützten Intermediats.

15 mg (0.022 mmol) von diesem Intermediat wurden in DMF gelöst und mit 13 mg (0.039 mmol) 1,1'-[(1,5-Dioxopentan-1,5-diyl)bis(oxy)]dipyrrolidin-2,5-dion und 7 µL N, N-Diisopropylethyl amin versetzt. Nach 1 h Rühren bei RT wurde der Ansatz eingeengt und der Rückstand mittels HPLC gereinigt. So wurden 9 mg (45% d. Th.) der Titelverbindung erhalten.

LC-MS (Methode 1): Rₜ = 1.08 min; MS (ESIpos): m/z = 895 (M+H)⁺.

### Intermediat F295

### N-[(2,5-Dioxo-2,5-dihydro-1H-pyrrol-1-yl)acetyl]-L-valyl-N-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-alaninamid

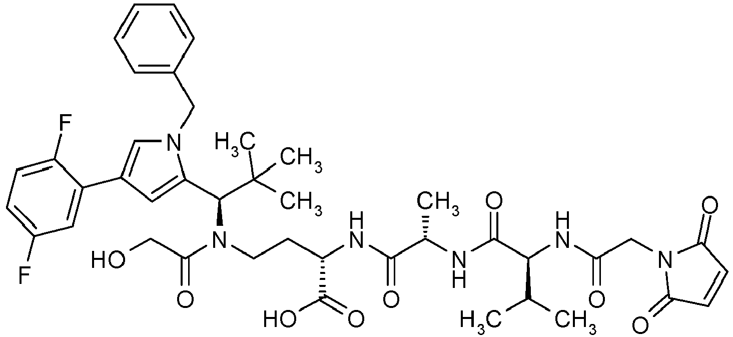

41 mg (0.05 mmol) von Intermediat C76 gelöst in 12 mL Methanol wurden über 10 mg 10%-igem Palladium auf Aktivkohle 1 h lang bei RT unter Wasserstoff-Normaldruck hydriert. Der Katalysator wurde danach abfiltriert und das Lösungsmittel im Vakuum entfernt. Man erhielt 32 mg (92% d.Th.) des entschützten Intermediats.

15 mg (0.022 mmol) von diesem Intermediat wurden in 4 mL DMF gelöst und mit 10 mg (0.039 mmol) 1-{2-[(2,5-Dioxopyrrolidin-1-yl)oxy]-2-oxoethyl}-1H-pyrrol-2,5-dion und 7 µL N, N-Diisopropylethyl amin versetzt. Nach 2 h Rühren bei RT wurde der Ansatz eingeengt und der Rückstand mittels HPLC gereinigt. So wurden 10 mg (56% d. Th.) der Titelverbindung erhalten.

LC-MS (Methode 1): Rₜ = 1.08 min; MS (ESIpos): m/z = 821 (M+H)⁺.

### Intermediat F296

### Trifluoressigsäure --(2S)-2-amino-4-[{(1R)-1-[1-benzyl-4-(2,5-difluorphenyl)-1H-pyrrol-2-yl]-2,2-dimethylpropyl } (glycoloyl)amino]-N-{2-[(2-{ [(2,5-dioxo-2,5-dihydro-1H-pyrrol-1-yl)acetyl]amino}ethyl)sulfonyl]ethyl}butanamid (1:1)

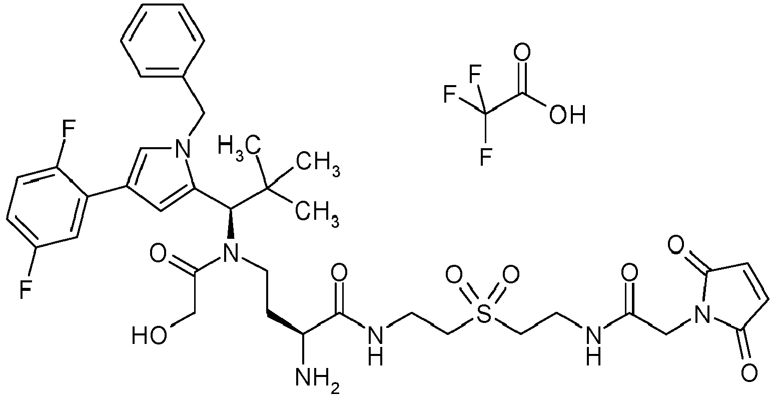

Die Titelverbindung wurde ausgehend von Intermediat L81 durch Kupplung mit Intermediat C58 in Gegenwart von HATU und N,N-Diisopropylethylamin hergestellt. Im nächsten Schritt wurde die Z-Schutzgruppe durch 30-minütige Hydrierung über 10%-igem Palladium auf Aktivkohle in DCM/Methanol 1:1 bei RT unter Wasserstoff-Normaldruck entfernt. Das entschützte Intermediat wurde anschließend durch Kupplung mit (2,5-Dioxo-2,5-dihydro-1H-pyrrol-1-yl)essigsäure in Gegenwart von HATU und N, N Diisopropylethylamin und schließlich durch Entschützung mit Zinkchlorid in die Titelverbindung überführt.

LC-MS (Methode 1): Rₜ = 0.83 min; MS (ESIpos): m/z = 785 (M+H)⁺.

### Intermediat F297

### S-{2-[{(1R)-1-[1-Benzyl-4-(2,5-difluorphenyl)-1H-pyrrol-2-yl]-2,2-dimethylpropyl}(pyrrolidin-3-ylmethyl)amino]-2-oxoethyl}-N-[6-(2,5-dioxo-2,5-dihydro-1H-pyrrol-1-yl)hexanoyl] -L-cysteintrifluoressigsäure (1:1) (Isomer 1)

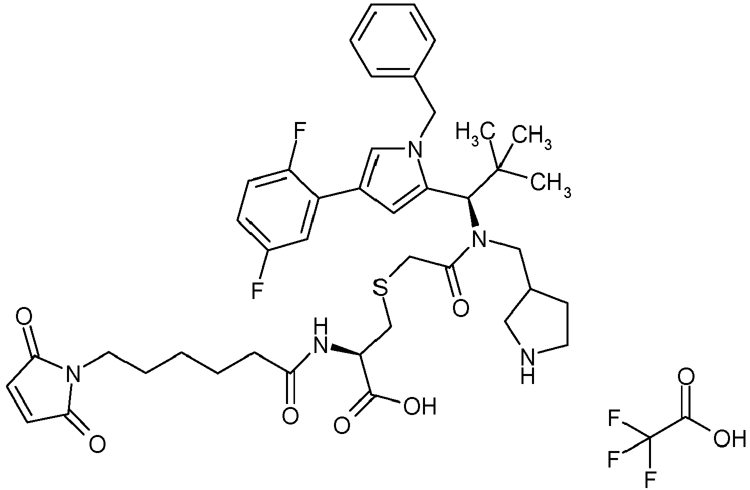

Eine Lösung von 36 mg (0.03 mmol, 68% Reinheit) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Intermediat C92) in 0.74 ml 2,2,2-Trifluoroethanol unter Argon wurde mit 15 mg (0.11 mmol) Zinkchlorid versetzt und das Reaktionsgemisch wurde 7 h bei 50 °C gerührt. Anschließend wurden 32 mg (0.11 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 6,4 mg (25 % d. Th) der Titelverbindung.

LC-MS (Methode 1): Rₜ = 0.95 min; MS (ESIpos): m/z = 792 (M+H-CF₃CO₂H)⁺.

### Intermediat F298

### S-{2-[{(1R)-1-[1-Benzyl-4-(2,5-difluorphenyl)-1H-pyrrol-2-yl]-2,2-dimethylpropyl}(pyrrolidin-3-ylmethyl)amino]-2-oxoethyl}-N-[6-(2,5-dioxo-2,5-dihydro-1H-pyrrol-1-yl)hexanoyl]-L-cystein-trifluoressigsäure (1:1) (Isomer 2)

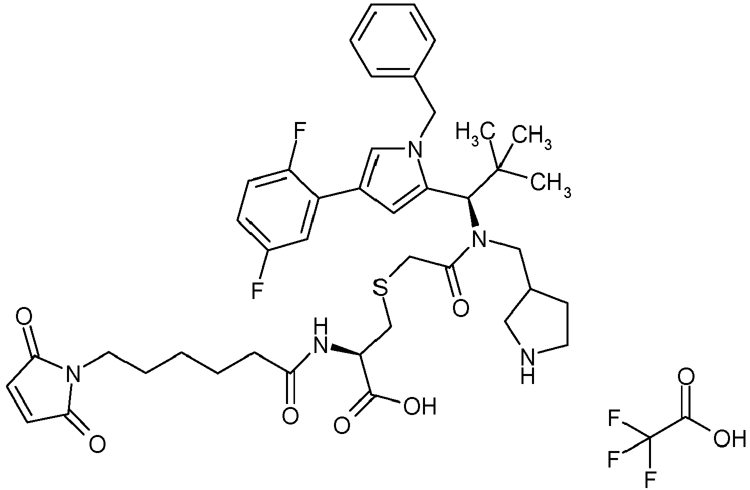

Eine Lösung von 45 mg (0.04 mmol, 71% Reinheit) S-[2-({(1R)-1-[1-Benzyl-4-(2,5-difluorphenyl)-1H-pyrrol-2-yl]-2,2-dimethylpropyl}{[1-(tert-butoxycarbonyl)pyrrolidin-3-yl]methyl}amino)-2-oxoethyl]-N-[6-(2,5-dioxo-2,5-dihydro-1H-pyrrol-1-yl)hexanoyl]-L-cystein (Intermediat C91) in 0.94 ml 2,2,2-Trifluoroethanol unter Argon wurde mit 19 mg (0.14 mmol) Zinkchlorid versetzt und das Reaktionsgemisch wurde 3 h bei 50 °C gerührt. Anschließend wurden 42 mg (0.14 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 5,7 mg (18 % d. Th) der Titelverbindung.

LC-MS (Methode 1): Rₜ = 0.96 min; MS (ESIpos): m/z = 791 (M+H-CF₃CO₂H)⁺.

### Intermediat F299

### S-(2-{(3-Aminopropyl)[(R)-[1-benzyl-4-(2,5-difluorphenyl)-1H-pyrrol-2-yl](cyclohexyl)methyl]amino}-2-oxoethyl)-N-[6-(2,5-dioxo-2,5-dihydro-1H-pyrrol-1-yl)hexanoyl]-L-cystein -trifluoressigsäure (1:1)

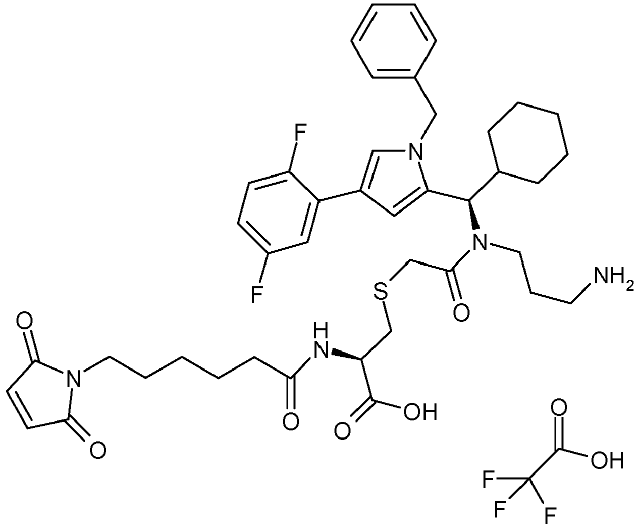

Eine Lösung von 88.0 mg (0.09 mmol) S-{11-[(R)-[1-Benzyl-4-(2,5-difluorphenyl)-1H-pyrrol-2-yl] (cyclohexyl)methyl] -2,2-dimethyl-6,12-dioxo-5 -oxa-7,11 -diaza-2-silatridecan-13 -yl} -N-[6-(2,5 - dioxo-2,5-dihydro-1H-pyrrol-1-yl)hexanoyl]-L-cystein (Intermediat C84) in 1.88 ml 2,2,2-Trifluoroethanol wurde mit 76.8 mg (0.57 mmol) Zinkchlorid versetzt und das Reaktionsgemisch wurde 3 h bei 50 °C gerührt. Anschließend wurden 164.6 mg (0.57 mmol) EDTA hinzugegeben und die Mischung wurde 15 Minuten gerührt. Die Reaktionsmischung wurde mit Ethylacetat versetzt und die org. Phase wurde mehrmals mit Wasser und mit ges. NaCl-Lösung gewaschen. Die organische Phase wurde über Natriumsulfat getrocknet und das Lösemittel im Vakuum verdampft. Der Rückstand wurde mittels präparative HPLC gereinigt. Man erhielt 31 mg (35 % d. Th ) der Titelverbindung.

LC-MS (Methode 12): Rₜ = 1.82 min; MS (ESIpos): m/z = 792 (M+H)⁺.

### Intermediat F300

### (2S)-2-Amino-4-[{(1R)-1-[1-benzyl-4-(2,5-difluorphenyl)-1H-pyrrol-2-yl]-2,2-dimethylpropyl}(glycoloyl)amino]-N-(2-{[(2R)-2-(2,5-dioxo-2,5-dihydro-1H-pyrrol-1-yl)propanoyl]amino}ethyl)butanamid

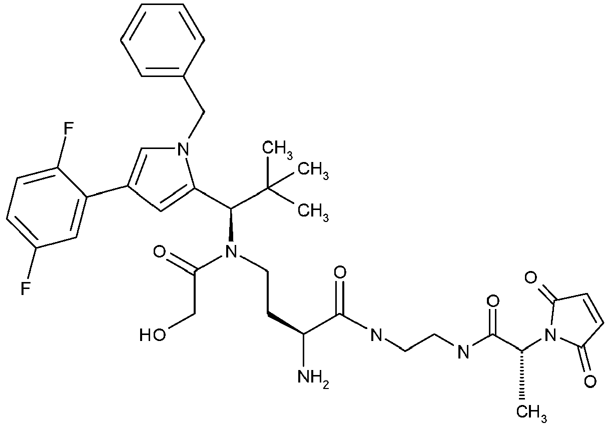

Eine Lösung von 7 mg (0.08 mmol)) 2-(Trimethylsilyl)ethyl-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2R)-2-(2,5-dioxo-2,5-dihydro-1H-pyrrol-1-yl)propanoyl]amino}ethyl)amino]-1-oxobutan-2-yl}carbamat (Intermediat C100) in 0.2 ml 2,2,2-Trifluoroethanol unter Argon wurde mit 11 mg (0.08 mmol) Zinkchlorid versetzt und das Reaktionsgemisch wurde 8 h bei 50 °C gerührt. Anschließend wurden 14 mg (0.05 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 1,6 mg (27 % d. Th) der Titelverbindung.

LC-MS (Methode 1): Rₜ = 0.88 min; MS (ESIpos): m/z = 707 (M+H-CF₃CO₂H)⁺.

### Intermediat F302

### S-{2-[{(1R)-1-[1-benzyl-4-(2,5-difluorophenyl)-1H-pyrrol-2-yl]-2,2-dimethylpropyl}(pyrrolidin-3-ylmethyl)amino]-2-oxoethyl}-N-[(2,5-dioxo-2,5-dihydro-1H-pyrrol-1-yl)acetyl]-L-cysteintrifluoressigsäure (1:1) (Isomer 1)

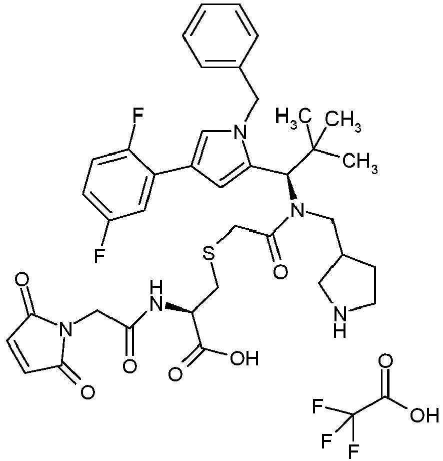

Eine Mischung von 56.9 mg (58.2 mmol, 85 % Reinheit) S-[2-({(1R)-1-[1-Benzyl-4-(2,5-difluorphenyl)-1H-pyrrol-2-yl]-2,2-dimethylpropyl}{[(1-(tert-butoxycarbonyl)pyrrolidin-3-yl]methyl}amino)-2-oxoethyl]-N-[(2,5-dioxo-2,5-dihydro-1H-pyrrol-1-yl)acetyl]-L-cystein (Intermediat C94) in 1.4 ml 2,2,2-Trifluoroethanol unter Argon wurde mit 31.7 mg (0.23 mmol) Zinkchlorid versetzt und das Reaktionsgemisch wurde 3 h bei 50 °C gerührt. Anschließend wurden 68.0 mg (0.23 mmol) EDTA hinzugegeben und die Mischung wurde 15 Minuten gerührt. Die Reaktionsmischung wurde mit Ethylacetat versetzt und die organische Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 7 mg (13 % d. Th) der Titelverbindung.

LC-MS (Methode 1): Rₜ = 0.91 min; MS (ESIpos): m/z = 736 (M+H-CF₃CO₂H)⁺.

### Intermediat F305

### N-{(1R)-1-[1-Benzyl-4-(2,5-difluorphenyl)-1H-pyrrol-2-yl]-2,2-dimethylpropyl}-22-(2,5-dioxo-2,5-dihydro-1H-pyrrol-1-yl)-6,17-dioxo-N-(pyrrolidin-3-ylmethyl)-10,13-dioxa-3-thia-7,16-diazadocosan-1-amid trifluoressigsäure (1:1) (Isomer 2)

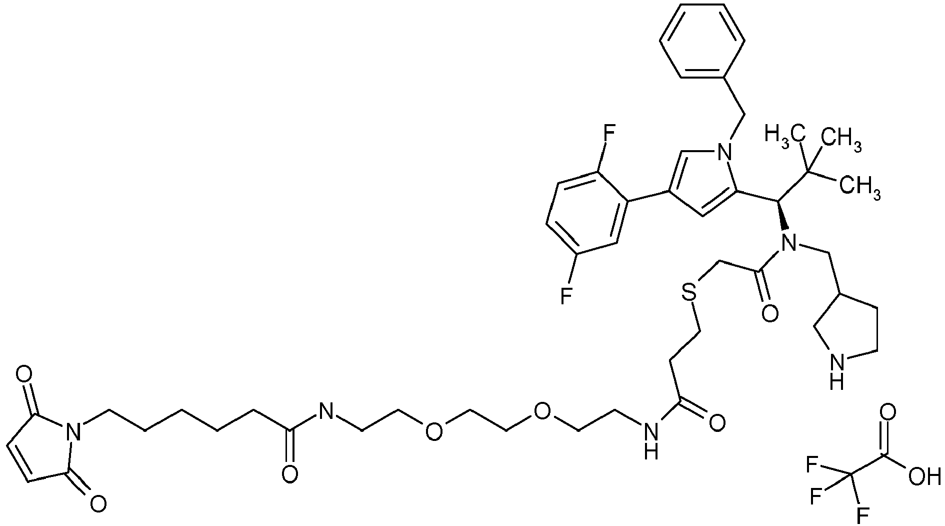

Eine Lösung von 24.80 mg (0.02 mmol) tert-Butyl-3-[2-{(1R)-1-[1-benzyl-4-(2,5-difluorphenyl)-1H-pyrrol-2-yl]-2,2-dimethylpropyl}-24-(2,5-dioxo-2,5-dihydro-1H-pyrrol-1-yl)-3,8,19-trioxo-12,15-dioxa-5-thia-2,9,18-triazatetracos-1-yl]pyrrolidin-1-carboxylat (Intermediat C99) in 0.65 ml 2,2,2-Trifluoroethanol wurde mit 13.42 mg (0.10 mmol) Zinkchlorid versetzt und das Reaktionsgemisch wurde 8 h bei 50 °C gerührt. Anschließend wurden 28.78 mg (0.10 mmol) EDTA hinzugegeben und die Mischung wurde 15 Minuten gerührt. Die Reaktionsmischung wurde mit Ethylacetat versetzt und die org. Phase wurde mehrmals mit Wasser und mit ges. NaCl-Lösung gewaschen. Die organische Phase wurde über Magnesiumsulfat getrocknet und das Lösemittel im Vakuum verdampft. Der Rückstand wurde mittels präparative HPLC gereinigt. Man erhielt 10 mg (44 % d. Th) der Titelverbindung.

LC-MS (Methode 5): Rₜ = 3.11 min; MS (ESIpos): m/z = 907 (M+H-CF₃CO₂H)⁺.

### Intermediat F306

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[(2,5-dioxo-2,5-dihydro-1H-pyrrol-1-yl)acetyl]-L-valyl-L-alanyl-beta-alanyl}-L-lysin -trifluoressigsäure (1:1)

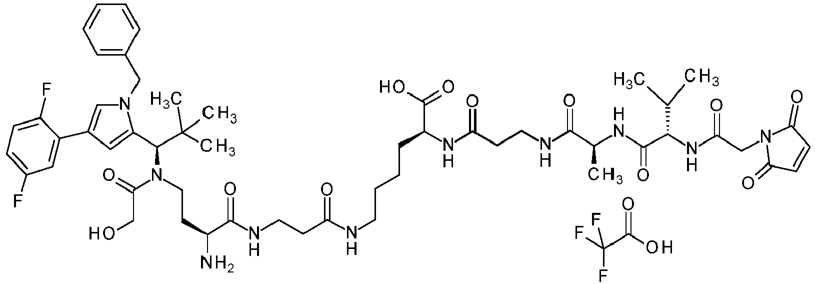

Die Titelverbindung wurde durch Kupplung von 24 mg (0.029 mmol) des Intermediats C61 mit 30 mg (0.035 mmol) von Intermediat L99 in Gegenwart von 16.7 mg (0.044 mmol) HATU sowie 15 µl N,N-Diisopropylethylamin und anschließender Entschützung mit Zinkchlorid in Trifluorethanol wie in Intermediat F119 beschrieben hergestellt. Nach Reinigung durch präparative HPLC wurden 19 mg (52% d. Th. über 2 Stufen) der Titelverbindung erhalten.

LC-MS (Methode 1): Rₜ = 0.84 min; MS (ESIpos): m/z = 1091 (M+H)⁺.

### Intermediat F307

### N-[6-(2,5-Dioxo-2,5-dihydro-1H-pyrrol-1-yl)hexanoyl]-L-valyl-L-alanyl-S-{(5R,14R)-13-(3-aminopropyl)-14-[1-benzyl-4-(2,5-difluorphenyl)-1H-pyrrol-2-yl]-5-carboxy-15,15-dimethyl-2,7,12-trioxo-10-thia-3,6,13-triazahexadec-1-yl}-L-cystein -trifluoressigsäure (1:1)

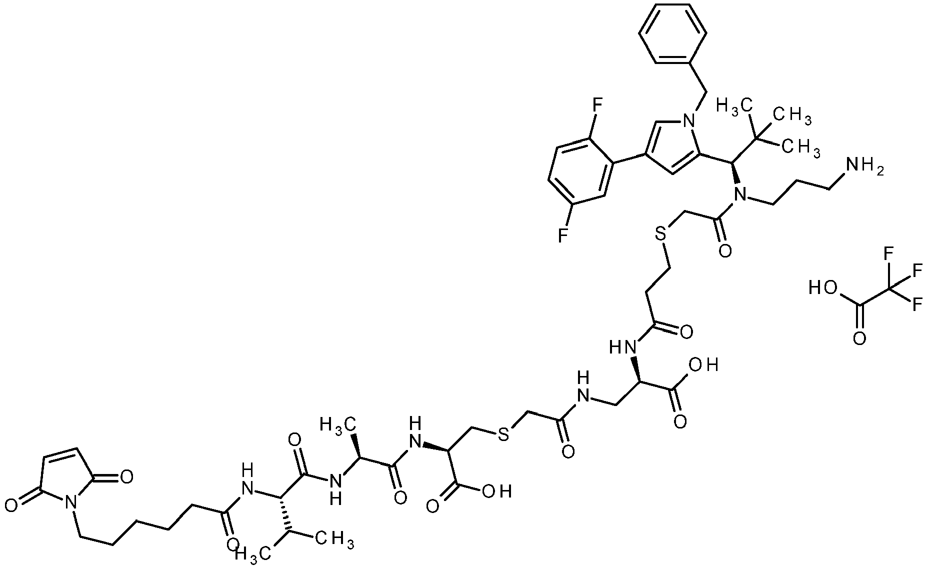

8.90 mg (8.88 µmol) Trifluoressigsäure 2-(trimethylsilyl)ethyl-3-amino-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-D-alaninat (1:1) (Intermediat C80) und 2.31 mg (9.77 µmol) 1-(2-Bromacetoxy)pyrrolidin-2,5-dion wurden in 1 ml Dimethylformamid gelöst und mit 2.9 µl (27 µmol) N-Methylmorpholin versetzt. Die Reaktionsmischung wurde 1 h bei RT gerührt. Die Reaktionsmischung wurde direkt mittles präp. RP-HPLC gereinigt (Säule: Reprosil 125x30; 10µ, Fluss: 50 mL/min, MeCN/Wasser/0.1%TFA). Die Lösemittel wurden im Vakuum verdampft und der Rückstand im Hochvakuum getrocknet. Man erhielt 5.80 mg (65% d. Th.) der Verbinfung 2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-[(bromacetyl)amino] -D-alaninat.

LC-MS (Methode 1): Rₜ = 1.57 min; MS (ESIpos): m/z = 1008 (M+H)⁺.

2-(Trimethylsilyl)ethyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-3-[(bromacetyl)amino]-D-alaninat (31.9 mg, 31.6 µmol) und L-Cystein (7.66 mg, 63.2 µmol) wurden in 3.0 ml DMF gelöst und über Nacht bei RT nachgerührt. Die Reaktionsmischung wurde direkt mittles präp. RP-HPLC gereinigt (Säule: Reprosil 250x30; 10µ, Fluss: 50 mL/min, MeCN/Wasser/0.1%TFA). Die Lösemittel wurden im Vakuum verdampft und der Rückstand im Hochvakuum getrocknet. Man erhielt 28.1 mg (76% d. Th.) der Verbindung S-[(19R)-11-{(1R)-1-[1-Benzyl-4-(2,5-difluorphenyl)-1H-pyrrol-2-yl]-2,2-dimethylpropyl}-2,2-dimethyl-6,12,17,22-tetraoxo-19-{[2-(trimethylsilyl)ethoxy]carbonyl}-5-oxa-14-thia-7,11,18,21-tetraaza-2-silatricosan-23-yl]-L-cystein -trifluoressigsäure (1:1).

LC-MS (Methode 12): Rₜ = 2.52 min; MS (ESIpos): m/z = 1049 [M+H]⁺

S-[(19R)-11-{(1R)-1-[1-Benzyl-4-(2,5-difluorphenyl)-1H-pyrrol-2-yl]-2,2-dimethylpropyl}-2,2-dimethyl-6,12,17,22-tetraoxo-19-{[2-(trimethylsilyl)ethoxy]carbonyl}-5-oxa-14-thia-7,11,18,21-tetraaza-2-silatricosan-23-yl]-L-cystein -trifluoressigsäure (1:1) (13.5 mg, 11.6 µmol) wurde in 1.0 ml DMF gelöst, mit 2,5-Dioxopyrrolidin-1-yl-N-[6-(2,5-dioxo-2,5-dihydro-1H-pyrrol-1-yl)hexanoyl]-L-valyl-L-alaninat (6.76 mg, 11.6 µmol) (Intermediat L88) und N,N-Diisopropylethylamin (4.0 µl, 23 µmol) versetzt und 1h bei RT nachgerührt. Die Reaktionsmischung wurde direkt mittles präp. RP-HPLC gereinigt (Säule: Reprosil 250x30; 10µ, Fluss: 50 mL/min, MeCN/Wasser/0.1%TFA). Die Lösemittel wurden im Vakuum verdampft und der Rückstand im Hochvakuum getrocknet. Man erhielt 11.1 mg (68% d. Th.) der Verbinfung N-[6-(2,5-Dioxo-2,5-dihydro-1H-pyrrol-1-yl)hexanoyl]-L-valyl-L-alanyl-S-[(19R)-11-{(1R)-1-[1-benzyl-4-(2,5-difluorphenyl)-1H-pyrrol-2-yl]-2,2-dimethylpropyl}-2,2-dimethyl-6,12,17,22-tetraoxo-19-{[2-(trimethylsilyl)ethoxy]carbonyl}-5-oxa-14-thia-7,11,18,21-tetraaza-2-silatricosan-23-yl]-L-cystein.

LC-MS (Methode 14): Rₜ = 7.38 min; MS (ESIpos): m/z = 1412 [M+H]⁺

N-[6-(2,5-Dioxo-2,5-dihydro-1H-pyrrol-1-yl)hexanoyl]-L-valyl-L-alanyl-S-[(19R)-11-{(1R)-1-[1-benzyl-4-(2,5-difluorphenyl)-1H-pyrrol-2-yl]-2,2-dimethylpropyl}-2,2-dimethyl-6,12,17,22-tetraoxo-19-{[2-(trimethylsilyl)ethoxy]carbonyl}-5-oxa-14-thia-7,11,18,21-tetraaza-2-silatricosan-23-yl]-L-cystein (9.40 mg, 6.65 µmol) wurde in 2.0 ml Trifluorethanol gelöst, mit Zinkdichlorid (5.44 mg, 39.9 µmol) versetzt. Die Reaktionsmischung rührte 1 h bei 50 °C. Zinkdichlorid (5.44 mg, 39.9 µmol) wurden zugegeben und die Reaktionsmischung rührte 1 h bei 50 °C. Die Reaktionsmischung wurde mit Ethylendiamin-N,N,N',N'-tetraessigsäure (23.4 mg, 79.8 µmol) versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.60 mg (66 % d. Th.) der Titel Verbindung.

LC-MS (Methode 1): Rₜ = 0.93 min; MS (ESIpos): m/z = 1168 (M+H)⁺.

### Intermediat F308

### N-[6-(2,5-Dioxo-2,5-dihydro-1H-pyrrol-1-yl)hexanoyl]-L-valyl-N-[(12R,19R)-19-amino-4-{(1R)-1-[1-benzyl-4-(2,5-difluorphenyl)-1H-pyrrol-2-yl]-2,2-dimethylpropyl}-12,19-dicarboxy-5,10,15-trioxo-7,17-dithia-4,11,14-triazanonadec-1-yl]-L-alaninamid -trifluoressigsäure (1:1)

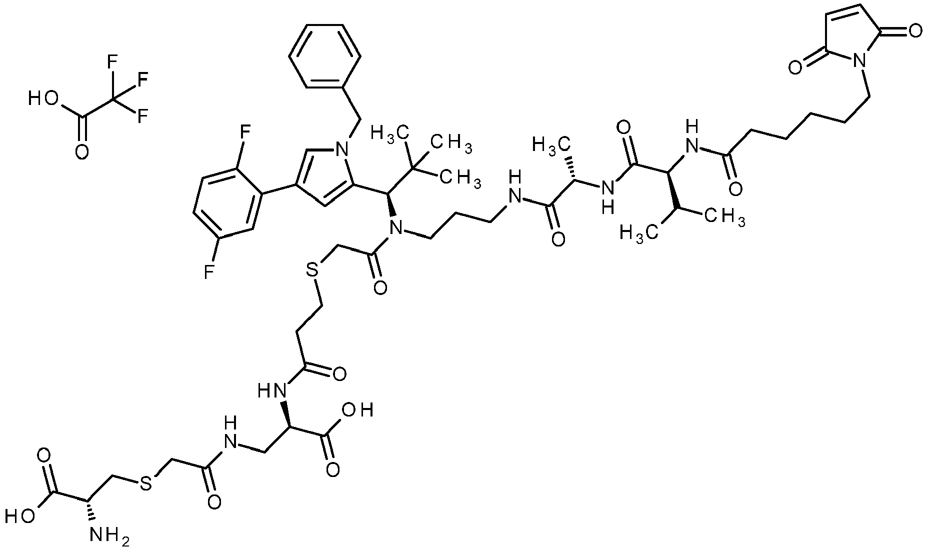

N-[3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-3-[(bromacetyl)amino]-D-alanin-trifluoressigsäure (1:1) (12.7 mg, 14.5 µmol) und N-{[2-(Trimethylsilyl)ethoxy]carbonyl}-L-cystein (3.84 mg, 14.5 µmol) wurden in 1.5 ml DMF gelöst und über Nacht bei RT nachgerührt. Dann wurde N,N-Diisopropylethylamin (2.5 µl, 14 µmol)zugegeben. Die Reaktionsmischung rührte 3 h bei RT und wurde dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.40 mg (48 % d. Th.) der Verbindung S-{(5R,14R)-13-(3-Aminopropyl)-14-[1-benzyl-4-(2,5-difluorphenyl)-1H-pyrrol-2-yl]-5-carboxy-15,15-dimethyl-2,7,12-trioxo-10-thia-3,6,13-triazahexadec-1-yl}-N-{[2-(trimethylsilyl)ethoxy]carbonyl}-L-cystein-trifluoressigsäure (1:1).

LC-MS (Methode 1): Rₜ = 1.03 min; MS (ESIpos): m/z = 949 [M+H]⁺

S-{(5R,14R)-13-(3-Aminopropyl)-14-[1-benzyl-4-(2,5-difluorphenyl)-1H-pyrrol-2-yl]-5-carboxy-15 J 5-dimethyl-2,7,12-trioxo-10-thia-3,6,13-triazahexadec-1-yl}-N-{[2-(trimethylsilyl)-ethoxy]carbonyl}-L-cystein -trifluoressigsäure (1:1) (7.50 mg, 7.05 µmol) wurde in 1.0 ml DMF gelöst und mit 2,5-Dioxopyrrolidin-1-yl-N-[6-(2,5-dioxo-2,5-dihydro-1H-pyrrol-1-yl)hexanoyl]-L-valyl-L-alaninat (4.11 mg, 82 % Reinheit, 7.05 µmol) (Intermediat L88) und N,N-Diisopropylethylamin (2.5 µl, 14 µmol) versetzt. Die Reaktionsmischung wurde 1h bei RT gerührt und dann direkt mittles präp. RP-HPLC (Säule: Reprosil 250x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 4.30 mg (46 %) der Verbindung N-[6-(2,5-Dioxo-2,5-dihydro-1H-pyrrol-1-yl)hexanoyl]-L-valyl-N-[(8R,15R)-23-{(1R)-1-[1-benzyl-4-(2,5-difluorphenyl)-1H-pyrrol-2-yl]-2,2-dimethylpropyl}-8,15-dicarboxy-2,2-dimethyl-6,12,17,22-tetraoxo-5-oxa-10,20-dithia-7,13,16,23-tetraaza-2-silahexacosan-26-yl]-L-alaninamid.

LC-MS (Methode 14): Rₜ = 6.47 min; MS (ESIpos): m/z = 1312 [M+H]⁺

N-[6-(2,5-Dioxo-2,5-dihydro-1H-pyrrol-1-yl)hexanoyl]-L-valyl-N-[(8R,15R)-23-{(1R)-1-[1-benzyl-4-(2,5-difluorphenyl)-1H-pyrrol-2-yl]-2,2-dimethylpropyl}-8,15-dicarboxy-2,2-dimethyl-6,12,17,22-tetraoxo-5-oxa-10,20-dithia-7,13,16,23-tetraaza-2-silahexacosan-26-yl]-L-alaninamid (4.00 mg, 3.05 µmol) wurde in 1.0 ml Trifluorethanol gelöst und mit Zinkdichlorid (2.49 mg, 18.3 µmol) versetzt. Die Reaktionsmischung rührte 1 h bei 50 °C und wurde dann mit Ethylendiamin-N,N,N',N'-tetraessigsäure (5.34 mg, 18.3 µmol) versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 2.50 mg (64 % d. Th.) der Titel Verbindung.

LC-MS (Methode 1): Rₜ = 1.00 min; MS (ESIpos): m/z = 1168 [M+H]⁺

### Intermediat F309

### 4-{[(11R,17R)-16-(3-Aminopropyl)-17-[1-benzyl-4-(2,5-difluorphenyl)-1H-pyrrol-2-yl]-1-(2,5-dioxo-2,5-dihydro-1H-pyrrol-1-yl)-18,18-dimethyl-6,6-dioxido-2,10,15-trioxo-6lambda⁶,13-dithia-3,9,16-triazanonadecan-1 1-yl]amino}-4-oxobutansäure -trifluoressigsäure (1:1)

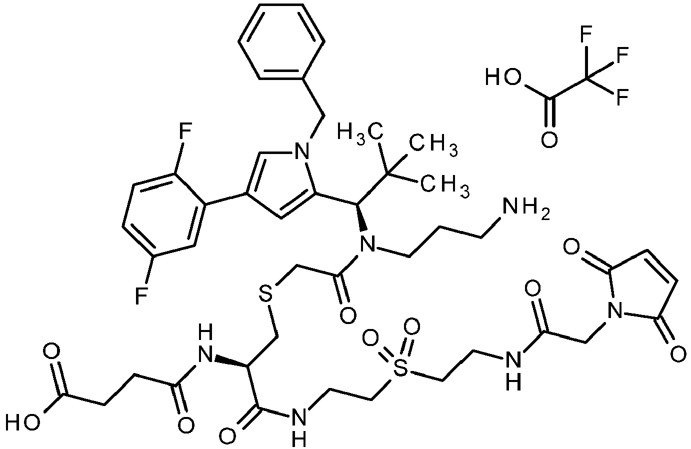

S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (50.0 mg, 57.3 µmol) (Intermediat C77) und Trifluoressigsäure -benzyl-{2-[(2-aminoethyl)sulfonyl]ethyl}carbamat (1:1) (27.5 mg, 68.7 µmol) (Intermediat L81) wurden in 4.0 ml DMF vorgelegt, mit HATU (26.1 mg, 68.7 µmol) und N,N-Diisopropylethylamin: (30 µl, 170 µmol) versetzt. Die Reaktionsmischung wurde 10 min bei RT gerührt und dann direkt mittles präp. RP-HPLC (Säule: Reprosil 250x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 53.9 mg (81 %) der Verbindung tert-Butyl-4-{[(12R)-17-{(1R)-1-[1-benzyl-4-(2,5-difluorphenyl)-1H-pyrrol-2-yl]-2,2-dimethylpropyl}-26,26-dimethyl-7,7-dioxido-3,11,16,22-tetraoxo-1-phenyl-2,23-dioxa-7lambda6,14-dithia-4,10,17,21-tetraaza-26-silaheptacosan-12-yl] amino } -4-oxobutanoat.

LC-MS (Methode 1): Rₜ = 1.54 min; MS (ESIpos): m/z = 1141 [M+H]⁺

Unter Argon, wurde Palladium(II)acetat (5.12 mg, 22.8 µmol) in 3.0 ml DCM vorgelegt, mit Triethylamin (9.5 µl, 68 µmol) und Triethylsilan (73 µl, 460 µmol) versetzt und 5 min. gerührt. Dann wurde tert-Butyl-4-{[(12R)-17-{(1R)-1-[1-benzyl-4-(2,5-difluorphenyl)-1H-pyrrol-2-yl]-2,2-dimethylpropyl}-26,26-dimethyl-7,7-dioxido-3,11,16,22-tetraoxo-1-phenyl-2,23-dioxa-7lambda⁶,14-dithia-4,10,17,21-tetraaza-26-silaheptacosan-12-yl]amino}-4-oxobutanoat (52.1 mg, 45.6 µmol) in 2.0 ml DCM zugegeben. Die Reaktionsmischung wurde über Nacht bei RT nachgerührt und mit 2.0 ml Wasser versetzt. Die Lösemittel wurden im Vakuum verdampft. Der Rückstand wurde mit Acetonitril versetzt, filtriert und mittles präp. RP-HPLC (Säule: Reprosil 125x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 43.4 mg (85 %) der Verbindung trifluoressigsäure-tert-butyl-4-{[(16R)-23-amino-11-{(1R)-1-[1-benzyl-4-(2,5-difluorphenyl)-1H-pyrrol-2-yl]-2,2-dimethylpropyl}-2,2-dimethyl-21,21-dioxido-6,12,17-trioxo-5-oxa-14,21lambda6-dithia-7,11,18-triaza-2-silatricosan-16-yl]amino}-4-oxobutanoat (1:1).

LC-MS (Method 1): Rₜ = 1.21 min; MS (ESIpos): m/z = 1007 [M+H]⁺

Trifluoressigsäure-tert-butyl-4-{[(16R)-23-amino-11-{(1R)-1-[1-benzyl-4-(2,5-difluorphenyl)-1H-pyrrol-2-yl]-2,2-dimethylpropyl}-2,2-dimethyl-21,21-dioxido-6,12,17-trioxo-5-oxa-14,21lambda⁶-dithia-7,11,18-triaza-2-silatricosan-16-yl]amino}-4-oxobutanoat (1:1) (20.0 mg, 17.8 µmol) und (2,5-dioxo-2,5-dihydro-1H-pyrrol-1-yl)essigsäure (3.32 mg, 21.4 µmol) wurden in 2.0 ml DMF vorgelegt, und mit HATU (8.14 mg, 21.4 µmol) und N,N-Diisopropylethylamin (9.3 µl, 54 µmol) versetzt.

Die Reaktionsmischung wurde 10 min bei RT nachgerührt. Die Reaktionsmischung wurde direkt mittles präp. RP-HPLC (Säule: Reprosil 250x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 17.4 mg (85 %) der Verbindung tert-Butyl-4-{[(16R)-11-{(1R)-1-[1-benzyl-4-(2,5-difluorphenyl)-1H-pyrrol-2-yl]-2,2-dimethylpropyl}-26-(2,5-dioxo-2,5-dihydro-1H-pyrrol-1-yl)-2,2-dimethyl-21,21-dioxido-6,12,17,25-tetraoxo-5-oxa-14,21lambda6-dithia-7,11,18,24-tetraaza-2-silahexacosan-16-yl]amino}-4-oxobutanoat.

LC-MS (Methode 1): Rₜ = 1.46 min; MS (ESIpos): m/z = 1144 [M+H]⁺

Tert-Butyl-4-{[(16R)-11-{(1R)-1-[1-benzyl-4-(2,5-difluorphenyl)-1H-pyrrol-2-yl]-2,2-dimethylpropyl}-26-(2,5-dioxo-2,5-dihydro-1H-pyrrol-1-yl)-2,2-dimethyl-21,21-dioxido-6,12,17,25-tetraoxo-5-oxa-14,21lambda⁶-dithia-7,11,18,24-tetraaza-2-silahexacosan-16-yl]amino}-4-oxobutanoat (15.9 mg, 13.9 µmol) wurde in 2.0 ml Trifluorethanol gelöst, mit Zinkdichlorid (11.4 mg, 83.4 µmol) versetzt. Die Reaktionsmischung rührte 1 h bei 50 °C. Zinkdichlorid (11.4 mg, 83.4 µmol) wurden zugegeben und die Reaktionsmischung rührte 1 h bei 50 °C. Zinkdichlorid (11.4 mg, 83.4 µmol) wurden zugegeben und die Reaktionsmischung rührte 1 h bei 50 °C. Die Reaktionsmischung wurde mit Ethylendiamin-N,N,N',N'-tetraessigsäure (73.2 mg, 250 µmol) versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 10 mg (68 % d. Th.) der Titel Verbindung.

LC-MS (Methode 12): Rₜ = 1.45 min; MS (ESIpos): m/z = 944 [M+H]⁺

### Intermediat F310

### Trifluoressigsäure-N-[(8R,14R)-13-(3-aminopropyl)-14-[1-benzyl-4-(2,5-difluorphenyl)-1H-pyrrol-2-yl]-1-(2,5-dioxo-2,5-dihydro-1H-pyrrol-1-yl)-15,15-dimethyl-2,7,12-trioxo-10-thia-3,6,13-triazahexadecan-8-yl]-2,5,8,11-tetraoxatetradecan-14-amid (1:1)

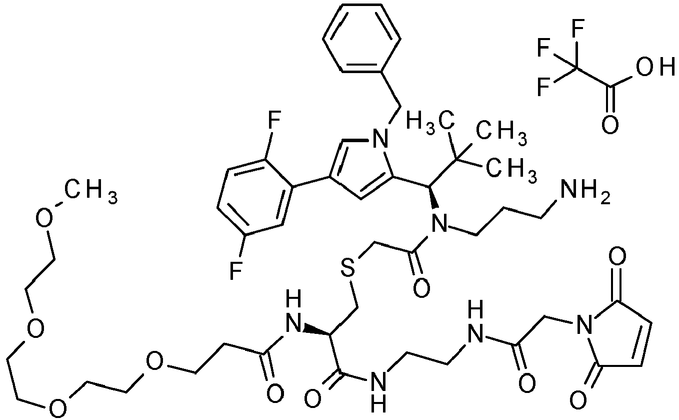

S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein -trifluoressigsäure (1:1) (100 mg, 120 µmol) (Intermediat C70) und 1-[(14-Oxo-2,5,8,11-tetraoxatetradecan-14-yl)oxy]pyrrolidin-2,5-dion (44.1 mg, 132 µmol) wurden in 3.0 ml DMF vorgelegt und mit 4-Methylmorpholin (40 µl, 360 µmol) versetzt. Die Reaktionsmischung wurde über Nacht bei RT gerührt, mit Essigsäure (420 µmol) gequencht und direkt mittles präp. RP-HPLC (Säule: Reprosil 125x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 69.4 mg (62 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(14-oxo-2,5, 8,11-tetraoxatetradecan-14-yl)-L-cystein.

LC-MS (Methode 12): Rₜ = 2.61 min; MS (ESIneg): m/z = 933 [M-H]⁻

S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(14-oxo-2,5,8,11-tetraoxatetradecan-14-yl)-L-cystein (27.0 mg, 28.9 µmol) wurde in 2.0 ml DMF vorgelegt und mit N-(2-Aminoethyl)-2-(2,5-dioxo-2,5-dihydro-1H-pyrrol-1-yl)acetamid (11.4 mg, 57.7 µmol) (Intermediat L1), N,N-Diisopropylethylamin (15 µl, 87 µmol) und HATU (22.0 mg, 57.7 µmol) versetzt. Die Reaktionsmischung wurde 3h bei RT gerührt und direkt mittles präp. RP-HPLC (Säule: Reprosil 250x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 13.7 mg (43 % d. Th.) der Verbindung 2-(Trimethylsilyl)ethyl-{(16R)-21-{(1R)-1-[1-benzyl-4-(2,5-difluorphenyl)-1H-pyrrol-2-yl]-2,2-dimethylpropyl}-16-[(2-{ [(2,5-dioxo-2,5-dihydro-1H-pyrrol-1-yl)acetyl]amino}ethyl)carbamoyl]-14,20-dioxo-2,5,8,11-tetraoxa-18-thia-15,21-diazatetracosan-24-yl}carbamat.

LC-MS (Methode 12): Rₜ = 2.54 min; MS (ESIpos): m/z = 1114 [M+H]⁺

2-(Trimethylsilyl)ethyl-{(16R)-21-{(1R)-1-[1-benzyl-4-(2,5-difluorphenyl)-1H-pyrrol-2-yl]-2,2-dimethylpropyl}-16-[(2-{ [(2,5-dioxo-2,5-dihydro-1H-pyrrol-1-yl)acetyl]amino}ethyl)carbamoyl]-14,20-dioxo-2,5,8,11-tetraoxa-18-thia-15,21-diazatetracosan-24-yl}carbamat (13.7 mg, 12.3 µmol) wurde in 2.0 ml Trifluorethanol gelöst, mit Zinkdichlorid (10.1 mg, 73.8 µmol) versetzt. Die Reaktionsmischung rührte 4 h bei 50 °C. Die Reaktionsmischung wurde mit Ethylendiamin-N,N,N',N'-tetraessigsäure (21.6 mg, 73.8 µmol) versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.30 mg (47 % d. Th.) der Titel Verbindung.

LC-MS (Methode 1): Rₜ = 1.01 min; MS (ESIpos): m/z = 970 [M+H]⁺

### Intermediat F311

### S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[1-(2,5-dioxo-2,5-dihydro-1H-pyrrol-1-yl)-2,30-dioxo-6,9,12,15,18,21,24,27-octaoxa-3-azatriacontan-30-yl]-L-cystein-trifluoressigsäure (1:1)

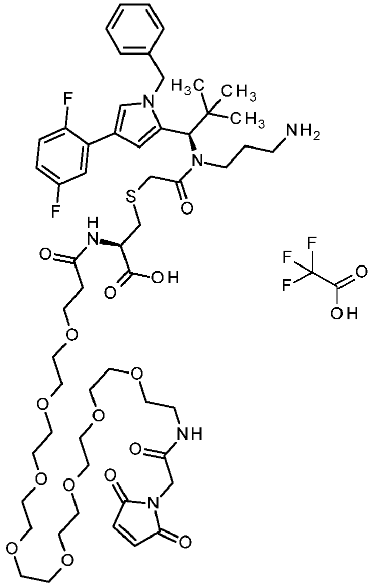

1-(2,5-Dioxo-2,5-dihydro-1H-pyrrol-1-yl)-2-oxo-6,9,12,15,18,21,24,27-octaoxa-3-azatriacontan-30-säure (10.8 mg, 18.7 µmol) (Intermediat L97) wurde in 1.0 ml DMF vorgelegt, mit N,N-Diisopropylethylamin (5.4 µl, 31.2 µmol) und HATU (7.10 mg, 18.7 µmol) versetzt und 10min. nachgerührt. Dann wurde S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein - trifluoressigsäure (1:1) (12.9 mg, 15.6 µmol) (Intermediat C71) in 1.0 ml DMF und N,N-Diisopropylethylamin (2.7 µl, 15.6 µmol) gelöst, zugegeben. Die Reaktionsmischung wurde 2 h bei RT gerührt und dann direkt mittles präp. RP-HPLC (Säule: Reprosil 125x30; 10µ, Fluss: 50 mL/min, MeCN/Wasser/0.1% TFA) gereinigt. Die Lösemittel wurden im Vakuum verdampft und der Rückstand im Hochvakuum getrocknet. Man erhielt 3.5 mg (18 %) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[1-(2,5-dioxo-2,5-dihydro-1H-pyrrol-1-yl)-2,30-dioxo-6,9,12,15,18,21,24,27-octaoxa-3-azatriacontan-30-yl]-L-cystein.

LC-MS (Methode 1): Rₜ = 1.30 min; MS (ESIneg): m/z = 1276 [M-H]⁻

S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[1-(2,5-dioxo-2,5-dihydro-1H-pyrrol-1-yl)-2,30-dioxo-6,9,12,15,18,21,24,27-octaoxa-3-azatriacontan-30-yl]-L-cystein (3.50 mg, 2.74 µmol) wurde in 1.0 ml Trifluorethanol gelöst, mit Zinkdichlorid (6.25 mg, 16.4 µmol) versetzt. Die Reaktionsmischung rührte 4 h bei 50 °C. Die Reaktionsmischung wurde mit Ethylendiamin-N,N,N',N'-tetraessigsäure (47 µl, 16 µmol) versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 2.0 mg (59 % d. Th.) der Titel Verbindung. LC-MS (Methode 1): Rₜ = 0.94 min; MS (ESIpos): m/z = 1133 (M+H)⁺.

### Intermediat F312

### N-[(2,5-Dioxo-2,5-dihydro-1H-pyrrol-1-yl)acetyl]-L-valyl-N-[(2S)-4-[{(1R)-1-[1-benzyl-4-(2,5-difluorphenyl)-1H-pyrrol-2-yl]-2,2-dimethylpropyl}(glycoloyl)amino]-1-{[2-(L-gamma-glutamylamino)ethyl]amino}-1-oxobutan-2-yl]-L-alaninamid -trifluoressigsäure (1:1)

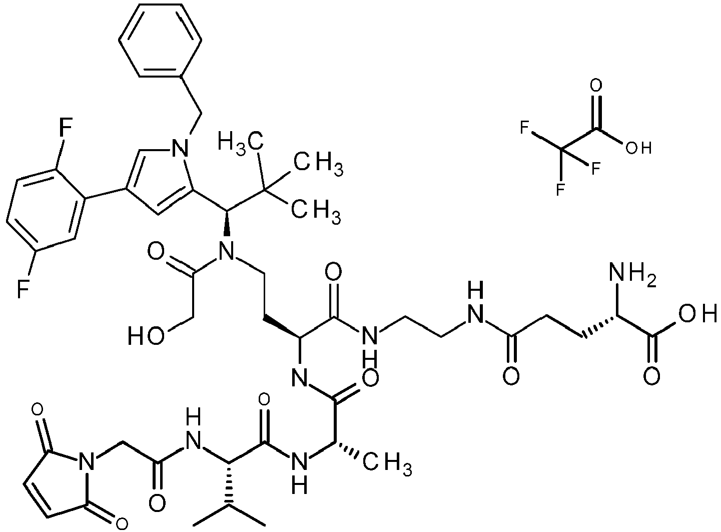

Die Titelverbindung wurde ausgehend von Intermediat C103 durch Kupplung mit N-[(Benzyloxy)carbonyl]-L-valyl-L-alanin in Gegenwart von HATU und N,N-Diisopropylethylamin hergestellt. Im nächsten Schritt wurde die Z-Schutzgruppe durch 1-stündige Hydrierung über 10%-igem Palladium auf Aktivkohle in DCM/Methanol 1:1 bei RT unter Wasserstoff-Normaldruck entfernt. Das entschützte Intermediat wurde anschließend durch Kupplung mit (2,5-Dioxo-2,5-dihydro-1H-pyrrol-1-yl)essigsäure in Gegenwart von HATU und N, N Diisopropylethylamin und schließlich durch Entschützung mit Zinkchlorid und Reinigung durch präparative HPLC in die Titelverbindung überführt.

LC-MS (Methode 1): Rₜ = 0.9 min; MS (ESIpos): m/z = 992 (M+H)⁺.

### Intermediat F313

### S-[2-({(1R)-1-[1-Benzyl-4-(2,5-difluorphenyl)-1H-pyrrol-2-yl]-2,2-dimethylpropyl}{[(3R,4R)-4-fluorpyrrolidin-3-yl]methyl}amino)-2-oxoethyl]-N-[1-(2,5-dioxo-2,5-dihydro-1H-pyrrol-1-yl)-2,18-dioxo-6,9,12,15-tetraoxa-3-azaoctadecan-18-yl]-L-cystein-trifluoressigsäure (1:1)

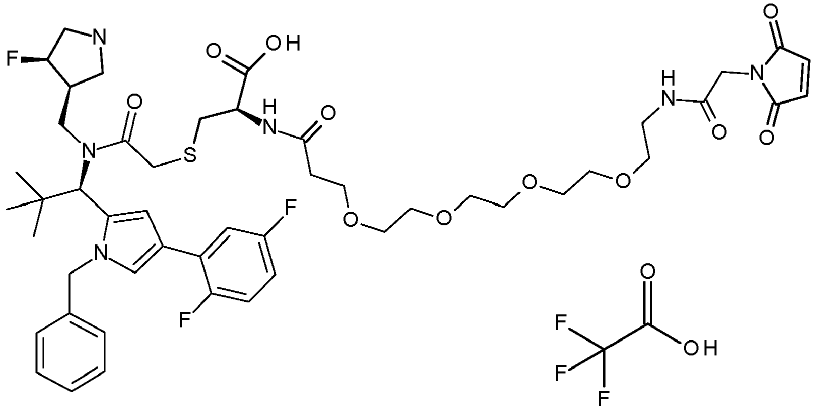

Zu einer Lösung von 55.0 mg (0.14 mmol) von 1-(2,5-Dioxo-2,5-dihydro-1H-pyrrol-1-yl)-2-oxo-6,9,12,15-tetraoxa-3-azaoctadecan-18-säure in 2.60 ml DMF unter Argon wurden 16.9 mg (0.13 mmol) N,N-diisopropylethylamin und 50.0 mg (0.13 mmol) HATU hinzugegeben. Das Reaktionsgemisch wurde 10 Minuten bei RT gerührt. Anschließend wurde eine Lösung von 40.0 mg (0.05 mmol) S-[2-({(1R)-1-[1-Benzyl-4-(2,5-difluorphenyl)-1H-pyrrol-2-yl]-2,2-dimethylpropyl} { [(3R,4R)-4-fluor-1-{ [2-(trimethylsilyl)ethoxy]carbonyl}pyrrolidin-3-yl]methyl}amino)-2-oxoethyl]-L-cystein (Intermediat C107) hinzugegeben und das Gemisch über Nacht bei RT gerührt. Die Mischung wurde mit Wasser versetzt und mit Dichlormethan extrahiert. Die organische Phase wurde über Magnesiumsulfat getrocknet und das Lösemittel im Vakuum verdampft und der Rückstand im Hochvakuum getrocknet. Der Rückstand wurdemittels präp. HPLC gereinigt Man erhielt 10 mg (13 % d. Th., Reinheit 82%) der Titelverbindung.

LC-MS (Methode 1): Rₜ = 1.36 min; MS (ESIpos): m/z = 1145 (M+H)⁺.

Eine Lösung von 10.9 mg (7.8 mmol, 82% Reinheit) S-[2-({(1R)-1-[1-Benzyl-4-(2,5-difluorphenyl)-1H-pyrrol-2-yl]-2,2-dimethylpropyl}{[(3R,4R)-4-fluor-1-{[2-(trimethylsilyl)ethoxy]carbonyl}pyrrolidin-3-yl]methyl}amino)-2-oxoethyl]-N-[1-(2,5-dioxo-2,5-dihydro-1H-pyrrol-1-yl)-2,18-dioxo-6,9,12,15-tetraoxa-3-azaoctadecan-18-yl]-L-cystein in 0.85 ml 2,2,2-Trifluoroethanol wurde mit 4.3 mg (0.03 mmol) Zinkchlorid versetzt und das Reaktionsgemisch wurde 2.5 h bei 50 °C gerührt. Anschließend wurden 9.1 mg (0.03 mmol) EDTA hinzugegeben und die Mischung wurde 15 Minuten gerührt. Die Reaktionsmischung mittels präp. HPLC gereinigt. Man erhielt 2.3 mg ( 26 % d. Th ) der Titelverbindung.

LC-MS (Methode 1): Rₜ 0.89 min; MS (ESIpos): m/z = 781 (M+H-CF₃CO₂H)⁺.

### Intermediat F314

### Trifluoressigsäure-3-{[2-({(1R)-1-[1-Benzyl-4-(2,5-difluorphenyl)-1H-pyrrol-2-yl]-2,2-dimethylpropyl}{[(3S,4R)-4-fluorpyrrolidin-3-yl]methyl}amino)-2-oxoethyl]sulfanyl}-N-(2-{[(2,5-dioxo-2,5-dihydro-1H-pyrrol-1-yl)acetyl]amino}ethyl)propanamid

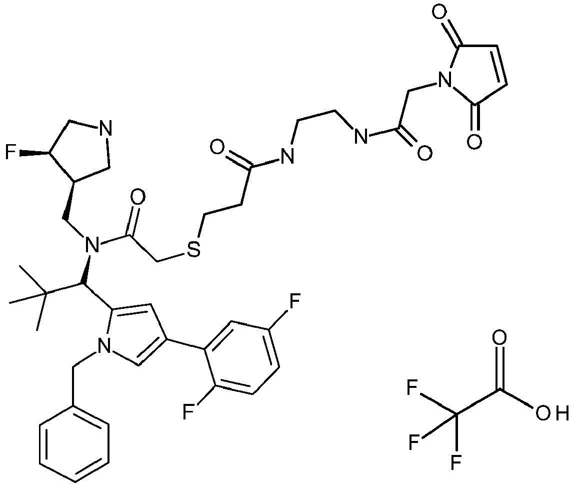

Zu einer Lösung von 50.0 mg (0.04 mmol, ) von 3-{[2-({(1R)-1-[1-Benzyl-4-(2,5-difluorphenyl)-1H-pyrrol-2-yl]-2,2-dimethylpropyl}{[(3R, 4R)-4-fluor-1-{[2-(trimethylsilyl)ethoxy]carbonyl} pyrrolidin-3-yl]methyl}amino)-2-oxoethyl]sulfanyl}propansäure (Intermediat 106) in 3.14 ml DMF unter Argon wurden 16.89 mg (0.13 mmol) N,N-diisopropylethylamin und 33.13 mg (0.087 mmol) HATU hinzugegeben. Das Reaktionsgemisch wurde 10 Minuten bei RT gerührt.

Anschließend wurde eine Lösung von 27.29 mg (0.09 mmol) N-(2-Aminoethyl)-2-(2,5-dioxo-2,5-dihydro-1H-pyrrol-1-yl)acetamidtrifluoressigsäure (1:1) (Intermediat L1) hinzugegeben und das Gemisch 15 Minuten bei RT gerührt. Die Mischung wurde mit Wasser versetzt und mit Dichlormethan extrahiert. Die organische Phase wurde über Magnesiumsulfat getrocknet und das Lösemittel im Vakuum verdampft und der Rückstand im Hochvakuum getrocknet. Der Rückstand wurdemittels präp. HPLC gereinigt Man erhielt 41 mg (68 % d. Th, Reinheit 66%.) der Titelverbindung.

LC-MS (Methode 12): Rₜ = 2.55 min; MS (ESIneg): m/z = 959 (M-H+Na)⁻.

Eine Lösung von 41.1 mg (0.03 mmol, Reinheit 66%) 2-(Trimethylsilyl)ethyl-(3R,4R)-3-[2-{(1R)-1-[1-benzyl-4-(2,5-difluorphenyl)-1H-pyrrol-2-yl]-2,2-dimethylpropyl}-14-(2,5-dioxo-2,5-dihydro-1H-pyrrol-1-yl)-3,8,13-trioxo-5-thia-2,9,12-triazatetradec-1-yl]-4-fluorpyrrolidin-1-carboxylat in 2.54 ml 2,2,2-Trifluoroethanol wurde mit 24.7 mg (0.18 mmol) Zinkchlorid versetzt und das Reaktionsgemisch wurde 2.5 h bei 50 °C gerührt. Anschließend wurden 53.0 mg (0.18 mmol) EDTA hinzugegeben und die Mischung wurde 15 Minuten gerührt. Die Reaktionsmischung mittels präp. HPLC gereinigt. Man erhielt 10 mg ( 36 % d. Th ) der Titelverbindung.

LC-MS (Methode 1): Rₜ 0.89 min; MS (ESIpos): m/z = 781 (M+H-CF₃CO₂H)⁺.

### Intermediat F315

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-N-{3-[5-(2-{ [(2,5-dioxo-2,5-dihydro-1H-pyrrol-1-yl)acetyl]amino}ethyl)-1,2,4-oxadiazol-3-yl]propanoyl}-L-cystein

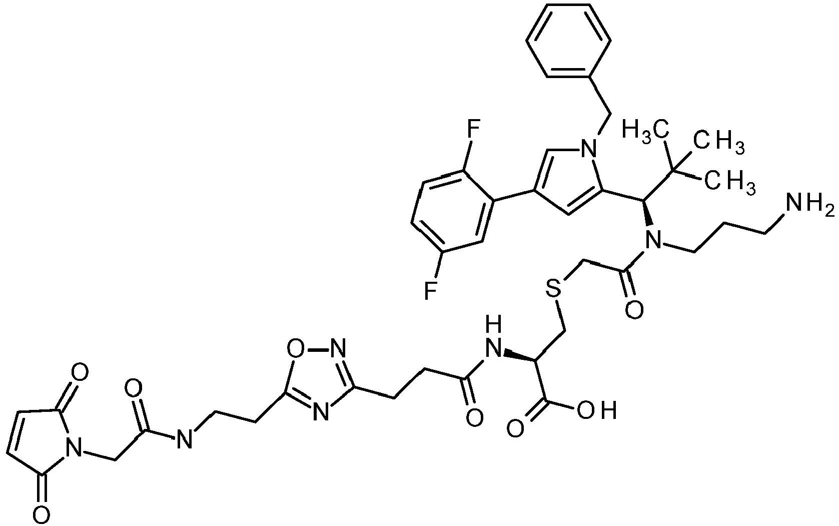

Zu einer Lösung von 50.0 mg (0.07 mmol) von 3-[5-(2-{[(2,5-Dioxo-2,5-dihydro-1H-pyrrol-1-yl)acetyl]amino}ethyl)-1,2,4-oxadiazol-3-yl]propansäure (Intermediat L100) in 3.5 ml DMF unter Argon wurden 18.02 mg (0.14 mmol) N,N-diisopropylethylamin und 31.82 mg (0.09 mmol) HATU hinzugegeben. Das Reaktionsgemisch wurde 10 Minuten bei RT gerührt. Anschließend wurde eine Lösung von 50.0 mg (0.07 mmol) N-(2-Aminoethyl)-6-(2,5-dioxo-2,5-dihydro-1H-pyrrol-1-yl)hexanamidacetat (1:1) (Intermediat C107) hinzugegeben und das Gemisch 2h bei RT gerührt. Die Mischung wurde mit Wasser versetzt und mit Dichlormethan extrahiert. Die organische Phase wurde über Magnesiumsulfat getrocknet und das Lösemittel im Vakuum verdampft und der Rückstand im Hochvakuum getrocknet. Der Rückstand wurde ohne Reinigung weiter eingesetzt. Man erhielt 49 mg (21 % d. Th, Reinheit 31%.) der Titelverbindung.

LC-MS (Methode 1): Rₜ = 1.30 min; MS (ESIpos): m/z = 1022 (M+H)⁺.

Eine Lösung von 49.0 mg (0.015 mmol, 31% Reinheit) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-{3-[5-(2-{[(2,5-dioxo-2,5-dihydro-1H-pyrrol-1-yl)acetyl]amino}ethyl)-1,2,4-oxadiazol-3-yl]propanoyl}-L-cystein in 0.5 ml 2,2,2-Trifluoroethanol wurde mit 8.0 mg (0.06 mmol) Zinkchlorid versetzt und das Reaktionsgemisch wurde 2h bei 50 °C gerührt. Anschließend wurden 17.2 mg (0.06 mmol) EDTA hinzugegeben und die Mischung wurde 15 Minuten gerührt. Die Reaktionsmischung mittels präp. HPLC gereinigt. Man erhielt 3 mg (21 % d. Th.) der Titelverbindung.

LC-MS (Methode 1): Rₜ = 0.91 min; MS (ESIpos): m/z = 877 (M+H-CF₃CO₂H)⁺.

### Intermediat F316

### Trifluoressigsäure-N-{2-[(3-{[2-({(1R)-1-[1-benzyl-4-(2,5-difluorphenyl)-1H-pyrrol-2-yl]-2,2-dimethylpropyl}{[(3S,4R)-4-fluorpyrrolidin-3-yl]methyl}amino)-2-oxoethyl]sulfanyl}propanoyl)amino]ethyl}-6-(2,5-dioxo-2,5-dihydro-1H-pyrrol-1-yl)hexanamid (1:1)

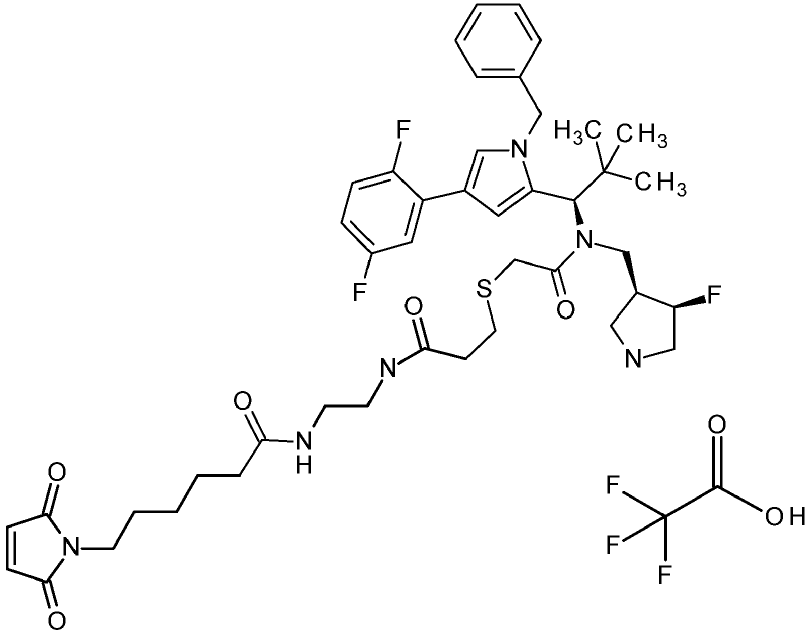

Zu einer Lösung von 50.0 mg (0.04 mmol, 65% Reinheit) von 3-{[2-({(1R)-1-[1-Benzyl-4-(2,5-difluorphenyl)-1H-pyrrol-2-yl]-2,2-dimethylpropyl}{[(3R, 4R)-4-fluor-1-{[2-(trimethylsilyl) ethoxy]carbonyl}pyrrolidin-3-yl]methyl}amino)-2-oxoethyl]sulfanyl}propansäure (Intermediat 106) in 3.0 ml DMF unter Argon wurden 16.89 mg (0.13 mmol) N,N-diisoprylethylamin und 33.13 mg (0.087 mmol) HATU hinzugegeben. Das Reaktionsgemisch wurde 10 Minuten bei RT gerührt. Anschließend wurde eine Lösung von 37.2 mg (0.09 mmol, Reinheiete 70%) N-(2-Aminoethyl)-6-(2,5-dioxo-2,5-dihydro-1H-pyrrol-1-yl)hexanamidacetat (1:1) (Intermediat L73) hinzugegeben und das Gemisch 7 Minuten bei RT gerührt. Die Mischung wurde mit Wasser versetzt und mit Dichlormethan extrahiert. Die organische Phase wurde über Magnesiumsulfat getrocknet und das Lösemittel im Vakuum verdampft und der Rückstand im Hochvakuum getrocknet. Der Rückstand wurde ohne Reinigung weiter eingesetzt. Man erhielt 57 mg (77 % d. Th, Reinheit 59%.) der Titelverbindung.

LC-MS (Methode 12): Rₜ = 2.60 min; MS (ESIpos): m/z = 981 (M+H)⁺.

Eine Lösung von 56.0 mg (0.03 mmol, 59% Reinheit) 2-(Trimethylsilyl)ethyl-(3R,4R)-3-[2-{(1R)-1-[1-benzyl-4-(2,5-difluorphenyl)-1H-pyrrol-2-yl]-2,2-dimethylpropyl}-18-(2,5-dioxo-2,5-dihydro-1H-pyrrol-1-yl)-3,8,13-trioxo-5-thia-2,9,12-triazaoctadec-1-yl]-4-fluorpyrrolidin-1-carboxylat in 2.8 ml 2,2,2-Trifluoroethanol wurde mit 36.0 mg (0.27 mmol) Zinkchlorid versetzt und das Reaktionsgemisch wurde 2h bei 50 °C gerührt. Anschließend wurden 78.3 mg (0.27 mmol) EDTA hinzugegeben und die Mischung wurde 15 Minuten gerührt. Die Reaktionsmischung mittels präp. HPLC gereinigt. Man erhielt 16 mg ( 44 % d. Th., 85% Reinheit) der Titelverbindung.

LC-MS (Methode 1): Rₜ = 0.89 min; MS (ESIpos): m/z = 837 (M+H-AcOH)⁺.

### Allgemeine Vorschrift zur Synthese der APDC oder ADC Precursor (Intermediatserie O)

Die zuvor beschriebenen Intermediate der F-Serie (F1-F305) können gemäß dem Schema 1 in die APDC-Precurser Q überführt werden. Im Falle einer Freisetzung der N-terminalen Aminogruppe des Legumain-spaltbaren Tripeptids im APDC-Precursormolekül kann diese zur Verbesserung des Eigenschaftsprofils im letzten Schritt mit strukturdiversen substituierten Acylresten oder Alkylresten modifiziert werden.

Eine examplarische Vorschrift ist hier dargestellt:
0.037 mmol eines geeigneten Intermediats F1-F305 außer F194 und F294 oder einer geeignet geschützten Vorstufe davon werden in 1-20 mL bevorzugt 5-10 mL eines geeigneten Lösungsmittels wie beispielsweise DMF, DMSO, DCM, Chloroform, Toluol, THF, Methanol oder einer Mischung derselben aufgenommen und mit 0.039 mmol eines N-terminal modifizierten Tripeptid-Derivats wie z.B. Intermediat L92 sowie mit 0.041 mmol eines gängigen Kupplungsreagenzes wie z. B. HATU, EDCI/HOBT, BEP etc. und 0.11 mmol einer gängigen Base wie z.B. N,N-Diisopropylethylamin, Triethylamin, 4-Methylmorpholin etc. versetzt. Nach 5 min Rühren bei RT wird der Ansatz mit 2 Tropfen Trifluoressigsäure angesäuert und eingeengt. Der Rückstand wird durch präparative HPLC gereinigt. Die entsprechenden Fraktionen werden im Vakuum eingeengt und der Rückstand aus Acetonitril/Wasser lyophilisiert.

Wenn es sich bei der besagten N-terminalen Modifizierung des angeknüpften Tripeptid-Derivats um eine Schutzgruppe handelt, kann diese anschließend ebenso wie ggf. noch vorhandene Schutzgruppen im Precursor-Molekül nach bekannten Methoden abgespalten werden, beispielsweise eine Z-Schutzgruppe bevorzugt mittels Hydrogenolyse, eine Boc-Schutzgruppe mittels Säurespaltung, eine Fmoc-Schutzgruppe durch basische Spaltung oder eine Teoc-Gruppe mittels Fluoriden oder mit Zinkchorid.

Schließlich kann die so freigesetzte Aminogruppe zur Verbesserung des Eigenschaftsprofils acyliert oder alkyliert werden, beispielsweise mit aminreaktiven Gruppen wie Aktivestern, Säurechloriden, Isocyanaten etc. oder durch Kupplung mit Carbonsäurederivaten in

Gegenwart eines gängigen Kupplungsreagenzes wie z. B. HATU, EDCI/HOBT, BEP etc. sowie einer gängigen Base wie z.B. N,N-Diisopropylethylamin, Triethylamin, 4-Methylmorpholin etc. Falls noch vorhanden können weitere Schutzgruppen im Molekül in einem letzten Schritt entfernt werden. 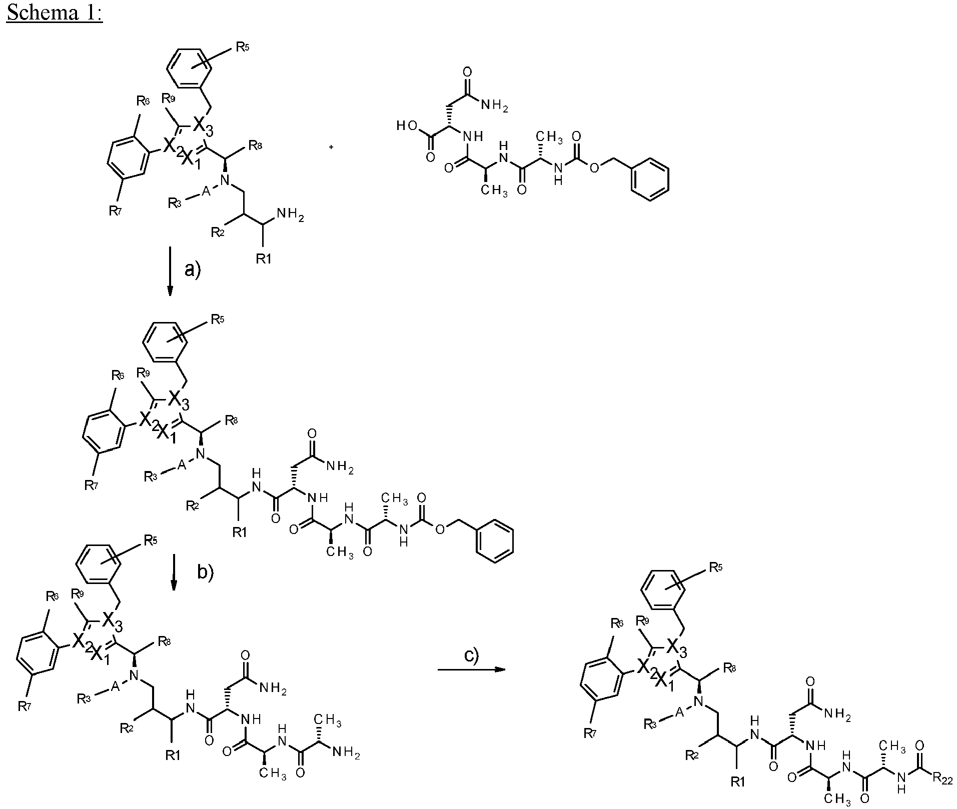 [a): HATU, DMF, N,N-Diisopropylethylamin, RT oder EDCI, HOBT, N,N-Diisopropylethylamin, DMF, RT b) H₂, 10% Pd-C, MeOH, RT; c) R22-COOH, EDCI, HOBT, N,N-Diisopropylethylamin, DMF, RT oder R²²-COOH, HATU, N,N-Diisopropylethylamin, DMF, RT oder R²²-COOSu, N,N-Diisopropylethylamin, DMF, RT]

Weiterhin können andere Intermediate gemäß Schema 2 und 3 in Legumain-spaltbare ADC-Precursor überführt werden: 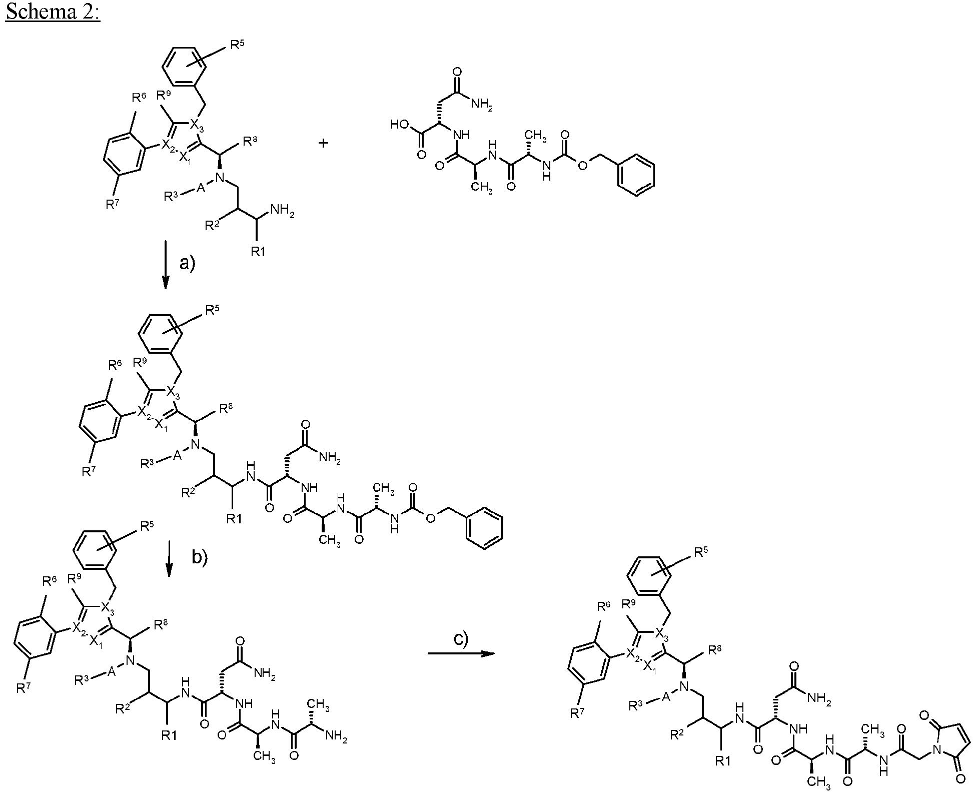 [a): HATU, DMF, N,N-Diisopropylethylamin, RT oder EDCI, HOBT, N,N-Diisopropylethylamin, DMF, RT b) H₂, 10% Pd-C, MeOH, RT; c) 1,1'-[(1,5-Dioxopentan-1,5-diyl)bis(oxy)]dipyrrolidin-2,5-dion, N,N-Diisopropylethylamin, DMF, RT] 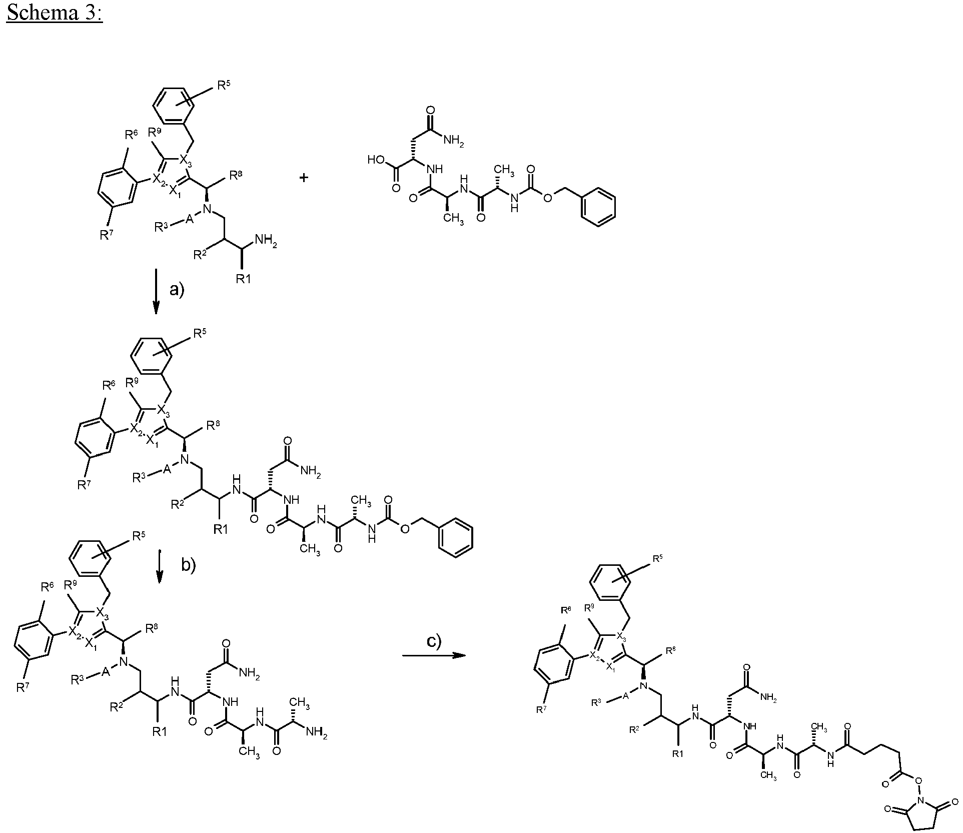 [a): HATU, DMF, N,N-Diisopropylethylamin, RT oder EDCI, HOBT, N,N-Diisopropylethylamin, DMF, RT b) H₂, 10% Pd-C, MeOH, RT; c) ) 1,1'-[(1,5-Dioxopentan-1,5-diyl)bis(oxy)]dipyrrolidin-2,5-dion, N,N-Diisopropylethylamin, DMF, RT]

Alternativ zu der in Schemata 1-3 dargestellten Benzyloxycarbonyl-Gruppe können andere in der Peptidchemie etablierte Schutzgruppen eingesetzt werden und nach entsprechenden ebenso bekannten Methoden abgespalten werden. Die Auswahl der Schutzgruppenstrategie erfolgt nach entsprechenden dem Fachmann bekannten Anforderungen zur Kompatibilität mit anderen im Molekül vorkommenden Strukturelementen. Falls noch vorhanden können weitere Schutzgruppen im Molekül in einem letzten Schritt entfernt werden.

Die Synthesen können auch ggf. in ihrer Sequenz umgestellt werden.

Weiterhin kann die proteinreaktive Gruppe im Sinne des Linkerstrukturen L1-L2 im Rahmen der Ansprüche variiert werden.

### Intermediat Q1

### N-[(Benzyloxy)carbonyl]-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

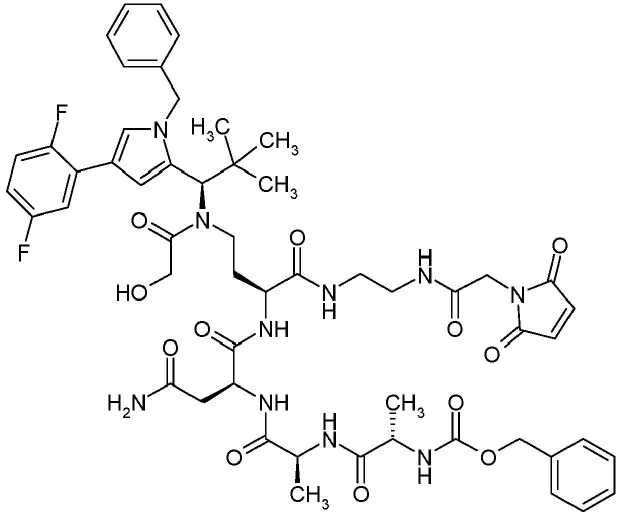

30 mg (0.037 mmol) von Intermediat F104 wurden in 6 mL DMF aufgenommen und mit 16 mg (0.039 mmol) von Intermediat L92 sowie mit 15.5 mg (0.041 mmol) HATU und 0.11 mmol N,N-Diisopropylethylamin versetzt. Nach 5 min Rühren bei RT wurde der Ansatz mit 2 Tropfen Trifluoressigsäure angesäuert und eingeengt. Der Rückstand wurde durch präparative HPLC gereinigt. Die entsprechenden Fraktionen wurden eingeengt und der Rückstand aus Acetonitril/Wasser lyophilisiert. Es wurden 21.5 mg (53% d. Th.) der Titelverbindung erhalten.

LC-MS (Methode 1): Rₜ = 1.08 min; MS (ESIpos): m/z = 1083 (M+H)⁺.

### Intermediat Q2

### N-Acetyl-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

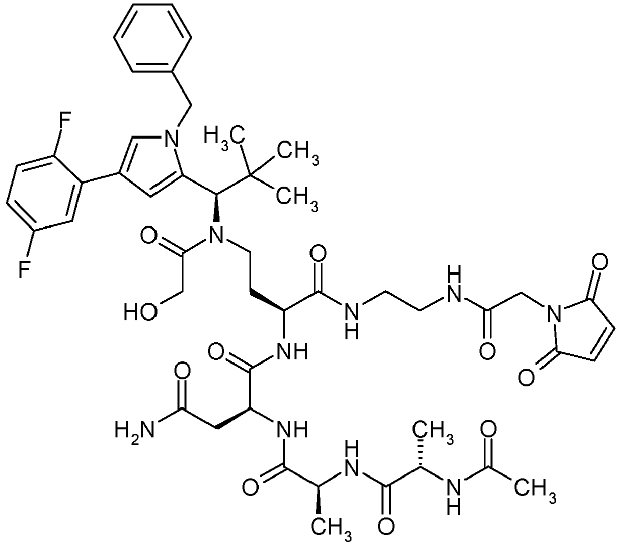

30 mg (0.037 mmol) von Intermediat F104 wurden in 6 mL DMF aufgenommen und mit 14 mg (0.045 mmol) von Intermediat L93 sowie mit 15.5 mg (0.041 mmol) HATU und 0.112 mmol N,N-Diisopropylethylamin versetzt. Nach 5 min Rühren bei RT wurden nochmals 1.5 mg HATU und 0.01 mmol N,N-Diisopropylethylamin hinzugegeben und weitere 10 min bei RT gerührt. Dann wurde der Ansatz mit 2 Tropfen Trifluoressigsäure angesäuert und im Vakuum eingeengt. Der Rückstand wurde durch präparative HPLC gereinigt. Die entsprechenden Fraktionen wurden eingeengt und der Rückstand aus Acetonitril/Wasser lyophilisiert. Es wurden 12.2 mg (33% d. Th.) der Titelverbindung erhalten.

LC-MS (Methode 1): Rₜ = 0.94 min; MS (ESIpos): m/z = 991 (M+H)⁺.

### Intermediat Q3

### N-(3-Carboxypropanoyl)-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

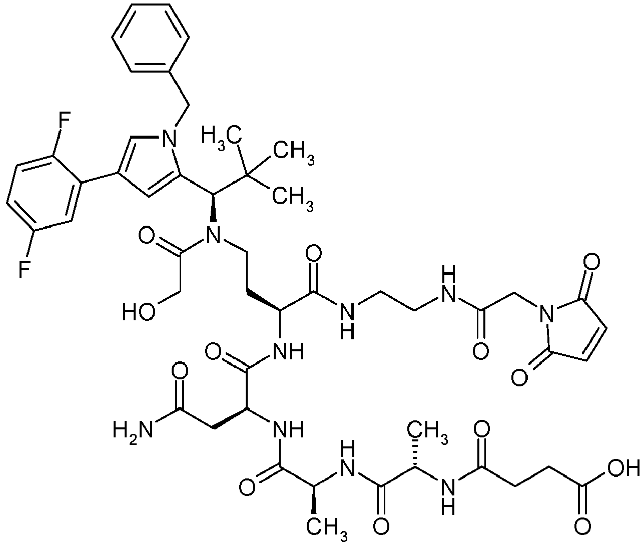

15 mg (0.019 mmol) von Intermediat F104 wurden in 5 mL DMF aufgenommen und mit 9.7 mg (0.02 mmol) von Intermediat L94 sowie mit 8.5 mg (0.022 mmol) HATU und 0.056 mol N,N-Diisopropylethylamin versetzt und 5 min bei RT gerührt. Dann wurde der im Vakuum eingeengt und der Rückstand durch präparative HPLC gereinigt. Die entsprechenden Fraktionen wurden eingeengt und der Rückstand aus Acetonitril/Wasser lyophilisiert. Es wurden 5.7 mg (27% d. Th.) der Titelverbindung erhalten.

LC-MS (Methode 1): Rₜ = 1.17 min; MS (ESIpos): m/z = 1149 (M+H)⁺.

Dieses Intermediat wurde in 2 mL 2,2,2-Trifluorethanol gelöst. Es wurden 2.6 mg (0.019 mmol) Zinkchlorid zugegeben und der Ansatz 45 min bei 50°C gerührt. Anschließend wurden 5.6 mg (0.019 mmol) Ethylendiamin-N,N,N',N'-tetraessigsäure zugesetzt und der Ansatz mit 3 mL Acetonitril/Wasser 9:1 verdünnt. Dann wurden 10 µL Trifluoressigsäure zugesetzt, 10 min bei RT gerührt und der Ansatz anschließend im Vakuum eingeengt. Der Rückstand wurde mittels präparativer HPLC gereinigt. Nach Einengen der entsprechenden Fraktionen und Lyophilisation des Rückstands aus Acetonitril/Wasser wurden 3.1 mg (62% d.Th.) der Titelverbindung erhalten.

LC-MS (Methode 1): Rₜ = 0.93 min; MS (ESIpos): m/z = 1049 (M+H)⁺.

### Intermediat Q4

### Trifluoressigsäure --N-[(benzyloxy)carbonyl]-L-alanyl-L-alanyl-N¹-[(11S,15R)-15-[1-benzyl-4-(2,5-difluorphenyl)-1H-pyrrol-2-yl]-1-(2,5-dioxo-2,5-dihydro-1H-pyrrol-1-yl)-14-glycoloyl-16,16-dimethyl-6,6-dioxido-2,10-dioxo-6lambda⁶-thia-3,9,14-triazaheptadecan-11-yl]-L-aspartamid (1:1)

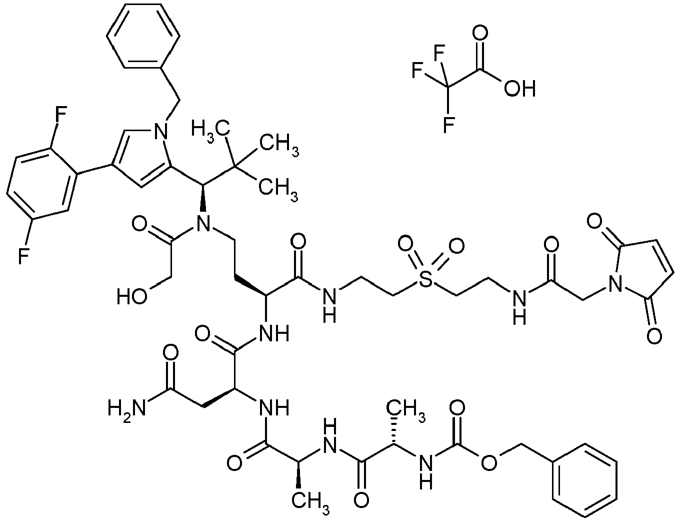

5 mg (0.006 mmol) von Intermediat F296 wurden in 3 mL DMF aufgenommen und mit 2.8 mg (0.007 mmol) von Intermediat L92 sowie mit 2.3 mg (0.006 mmol) HATU und 0.017mmol N,N-Diisopropylethylamin versetzt. Nach 15 min Rühren bei RT wurde der Ansatz mit 2 Tropfen Trifluoressigsäure angesäuert und eingeengt. Der Rückstand wurde durch präparative HPLC gereinigt. Die entsprechenden Fraktionen wurden eingeengt und der Rückstand aus Acetonitril/Wasser lyophilisiert. Es wurden 0.93 mg (13% d. Th.) der Titelverbindung erhalten.

LC-MS (Methode 1): Rₜ = 1.07 min; MS (ESIpos): m/z = 1175 (M+H)⁺.

### Intermediat Q5

### N-[(2,5-Dioxo-2,5-dihydro-1H-pyrrol-1-yl)acetyl]-L-alanyl-L-alanyl-N¹-{(1S)-3-[{(1R)-1-[1-benzyl-4-(2,5-difluorphenyl)-1H-pyrrol-2-yl]-2,2-dimethylpropyl} (glycoloyl)amino] -1-carboxypropyl } -L-aspartamid

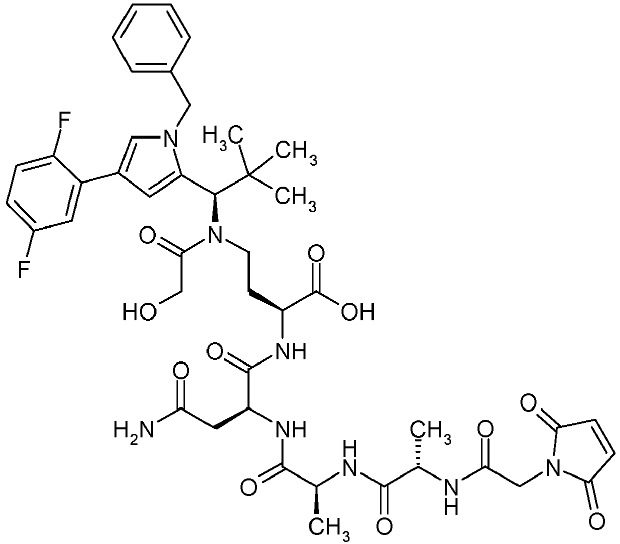

55 mg des Gemisches in Intermediat C101 wurden in 33 mL DMF aufgenommen und mit 27.5 mg (0.067 mmol) von Intermediat L92 sowie mit 28 mg (0.073 mmol) HATU und 32 µL N,N-Diisopropylethylamin versetzt. Nach 10 min Rühren bei RT wurde der Ansatz eingeengt und der Rückstand durch präparative HPLC gereinigt.

Das so erhaltene Rohprodukt wurde in 4 mL THF vorgelegt und mit 2 mL Wasser sowie 0.375 mL einer 2M Lithiumhydroxidlösung versetzt. Der Ansatz wurde 1 h bei RT gerührt und anschließend mit 1N Salzsäure neutral gestellt und im Vakuum eingeengt. Der Rückstand wurde durch präparative HPLC gereinigt. Die entsprechenden Fraktionen wurden eingeengt und der Rückstand aus Acetonitril/Wasser lyophilisiert. Es wurden 10.2 mg von N-[(Benzyloxy)carbonyl]-L-alanyl-L-alanyl-N1-{(1S)-3-[{(1R)-1-[1-benzyl-4-(2,5-difluorphenyl)-1H-pyrrol-2-yl]-2,2-dimethylpropyl} (glycoloyl)amino]-1-carboxypropyl}-L-aspartamiderhalten.

LC-MS (Methode 1): Rₜ = 1.09 min; MS (ESIpos): m/z = 904 (M+H)⁺.

Im nächsten Schritt wurde die Z-Schutzgruppe durch 1-stündige Hydrierung über 10%-igem Palladium auf Aktivkohle in Methanol bei RT unter Wasserstoff-Normaldruck entfernt und so 7.5 mg (87% d. Th.) von L-Alanyl-L-alanyl-N1-{(1S)-3-[{(1R)-1-[1-benzyl-4-(2,5-difluorphenyl)-1H-pyrrol-2-yl] -2,2-dimethylpropyl} (glycoloyl)amino] -1 -carboxypropyl} -L-aspartamid erhalten.

LC-MS (Methode 1): Rₜ = 0.80 min; MS (ESIpos): m/z = 770 (M+H)⁺.

3.75 mg (0.005 mmol) von L-Alanyl-L-alanyl-N¹-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-aspartamid wurden in 4 mL DMF aufgenommen und mit 1.72 mg (0.007 mmol) 1-{2-[(2,5-Dioxopyrrolidin-1-yl)oxy]-2-oxoethyl}-1H-pyrrol-2,5-dion sowie mit 1.2 µL (0.007 mmol) N,N-Diisopropylethylamin versetzt. Die Reaktionsmischung wurde 1 h bei RT gerührt, dann im Vakuum eingeengt und der Rückstand mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 1.3 mg (29 % d. Th.) der Titelverbindung.

LC-MS (Methode 1): Rₜ = 1.03 min; MS (ESIpos): m/z = 907 (M+H)⁺.

### Intermediat Q6

### N-{5-[(2,5-Dioxopyrrolidin-1-yl)oxy]-5-oxopentanoyl}-L-alanyl-L-alanyl-N-{(1S)-3-[{(1R)-1-[1-benzyl-4-(2,5-difluorphenyl)-1H-pyrrol-2-yl]-2,2-dimethylpropyl}(glycoloyl)amino]-1-carboxypropyl}-L-aspartamid

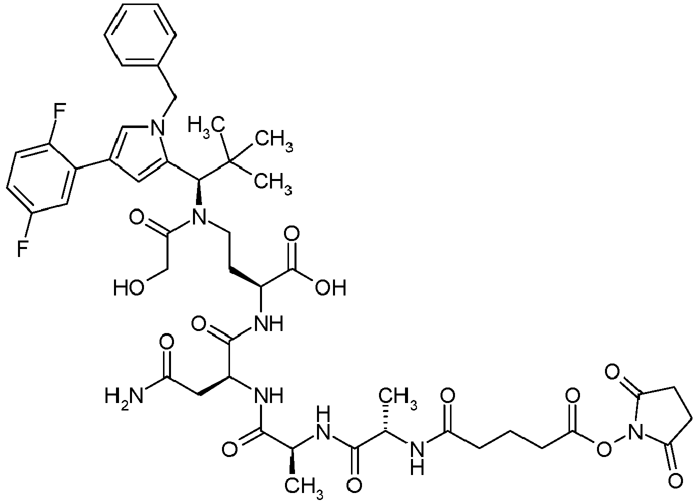

3.75 mg (0.005 mmol) von des unter F5 beschriebenen Intermediats L-Alanyl-L-alanyl-N¹-{(1S)-3-[{(1R)-1-[1-benzyl-4-(2,5 -difluorphenyl)- 1H-pyrrol-2-yl] -2,2-dimethylpropyl} (glycoloyl)amino] - 1-carboxypropyl}-L-aspartamid wurden in 4 mL DMF aufgenommen und mit 2.2 mg (0.007 mmol) 1,1'-[(1,5-Dioxopentan-1,5-diyl)bis(oxy)]dipyrrolidin-2,5-dion sowie mit 1.2 µL (0.007 mmol) N,N-Diisopropylethylamin versetzt. Die Reaktionsmischung wurde 1 h bei RT gerührt, dann im Vakuum eingeengt und der Rückstand mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 1.9 mg (27 % d. Th.) der Titelverbindung.

LC-MS (Methode 1): Rₜ = 1.04 min; MS (ESIpos): m/z = 981 (M+H)⁺.

### Intermediat Q7

### N-Acetyl-L-alanyl-L-alanyl-N¹-[(2S)-4-[{(1R)-1-[1-benzyl-4-(2,5-difluorphenyl)-1H-pyrrol-2-yl]-2,2-dimethylpropyl}(glycoloyl)amino]-1-({3-[(bromacetyl)amino]propyl}amino)-1-oxobutan-2-yl]-L-aspartamid

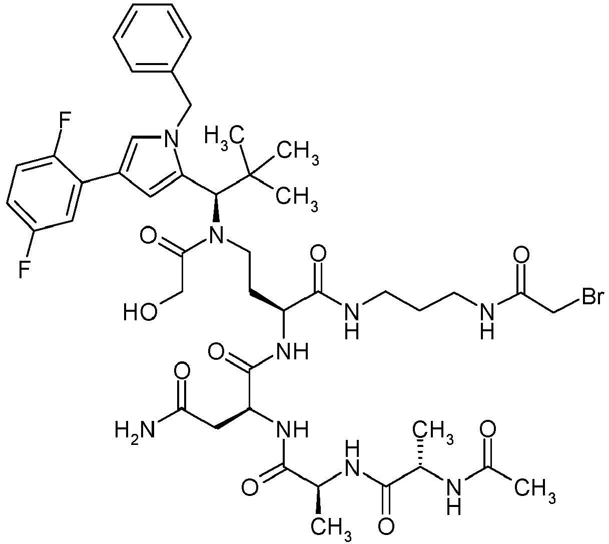

4.1 mg (0.005 mmol) von Intermediat F282 wurden in 1 mL DMF aufgenommen und mit 1.7 mg (0.005 mmol) von Intermediat L93 sowie mit 2.2 mg (0.006 mmol) HATU und 0.6 µl 4-Methylmorpholin versetzt. Nach 60 min Rühren bei RT wurde der Ansatz eingeengt und der Rückstand durch präparative HPLC gereinigt. Die entsprechenden Fraktionen wurden eingeengt und der Rückstand aus Acetonitril/Wasser lyophilisiert. Es wurden 1.2 mg (24% d. Th.) der Titelverbindung erhalten.

LC-MS (Methode 1): Rₜ = 0.98 min; MS (ESIpos): m/z = 1045 und 1047 (M+H)⁺.

### Intermediat Q8

### N-[(Benzyloxy)carbonyl]-L-alanyl-L-alanyl-N¹-[(20R)-25-{(1R)-1-[1-benzyl-4-(2,5-difluorphenyl)-1H-pyrrol-2-yl]-2,2-dimethylpropyl}-20-carboxy-1-(2,5-dioxo-2,5-dihydro-1H-pyrrol-1-yl)-2,18,24-trioxo-6,9,12,15-tetraoxa-22-thia-3,19,25-triazaoctacosan-28-yl]-L-aspartamid

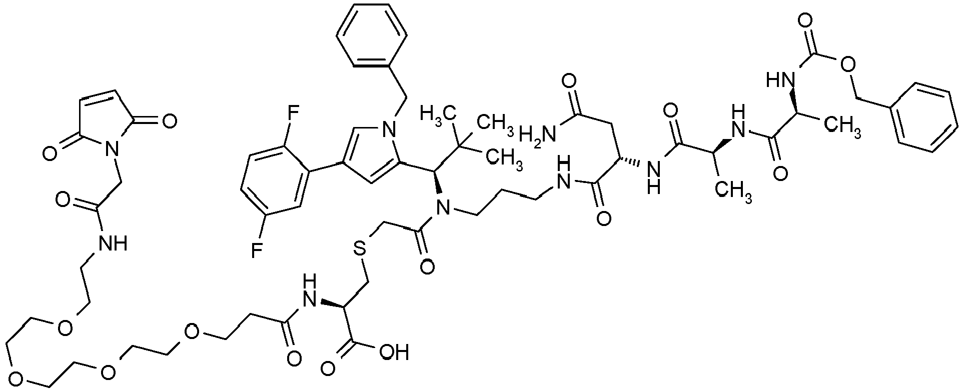

6.80 mg (6.35 µmol) von S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[1-(2,5-dioxo-2,5-dihydro-1H-pyrrol-1-yl)-2,18-dioxo-6,9,12,15-tetraoxa-3-azaoctadecan-18-yl]-L-cystein -trifluoressigsäure (1:1) (Intermediat F257) und 2.71 mg (6.63 µmol) von N-[(Benzyloxy)carbonyl]-L-alanyl-L-alanyl-L-asparagin (Intemediat L92) wurden in 1.0 mL DMF gelöst und mit 2.66 mg (6.98 µmol) HATU und 3.3 µl (19 µmol)N,N-Diisopropylethylamin versetzt. Nach 45 min Rühren bei RT wurde der Ansatz mit 4 µl Trifluoressigsäure angesäuert und durch präparative HPLC gereinigt. Die entsprechenden Fraktionen wurden eingeengt und der Rückstand aus Acetonitril/Wasser lyophilisiert. Es wurden 1.20 mg (14% d. Th.) der Titelverbindung erhalten.

LC-MS (Methode 1): Rₜ = 1.08 min; MS (ESIpos): m/z = 1346 (M+H)⁺.

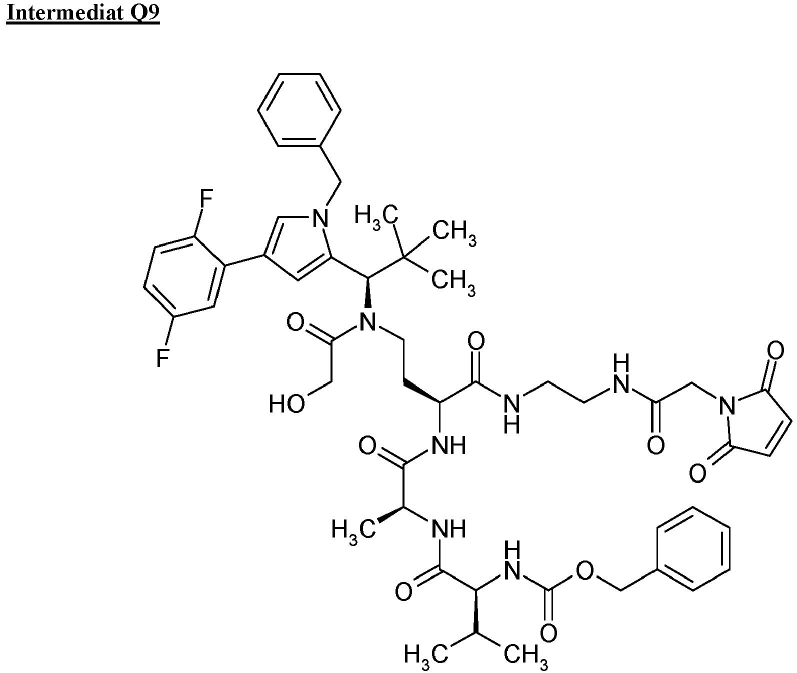

5 mg (0.0062 mmol) von Intermediat F104 wurden in 2 mL DMF aufgenommen und mit 2.2 mg (0.0068 mmol) von Intermediat L95 sowie mit 4.7 mg (0.012 mmol) HATU und 5 Equivalenten N,N-Diisopropylethylamin versetzt. Nach 1 h Rühren bei RT wurde der Ansatz im Vakuum eingeengt und der Rückstand durch präparative HPLC gereinigt. Die entsprechenden Fraktionen wurden eingeengt und der Rückstand aus Acetonitril/Wasser lyophilisiert. Es wurden 2 mg (32% d. Th.) der Titelverbindung erhalten.

LC-MS (Methode 1): Rₜ = 1.28 min; MS (ESIpos): m/z = 997 (M+H)⁺.

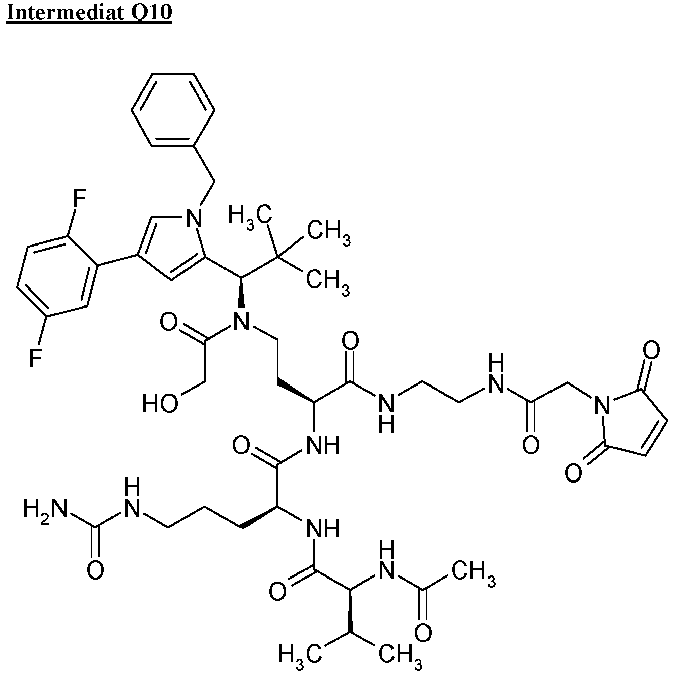

5 mg (0.0062 mmol) von Intermediat F104 wurden in 2 mL DMF aufgenommen und mit 2.2 mg (0.0068 mmol) von Intermediat L96 sowie mit 5.9 mg (0.016 mmol) HATU und 4 Equivalenten 4-Methylmorpholin versetzt. Nach 2 h Rühren bei RT wurde der Ansatz im Vakuum eingeengt und der Rückstand durch präparative HPLC gereinigt. Die entsprechenden Fraktionen wurden eingeengt und der Rückstand aus Acetonitril/Wasser lyophilisiert. Es wurden 2.5 mg (41% d. Th.) der Titelverbindung erhalten.

LC-MS (Methode 1): Rₜ = 0.99 min; MS (ESIpos): m/z = 991 (M+H)⁺.

### Intermediat Q11

### N-(pyridin-4-ylacetyl)-L-alanyl-L-alanyl-N1-{(2S)-4-[{(1R)-1-[1-benzyl-4-(2,5-difluorophenyl)-1H-pyrrol-2-yl]-2,2-dimethylpropyl } (glycoloyl)amino]-1-[(2-{ [(2,5-dioxo-2,5-dihydro-1H-pyrrol-1-yl)acetyl]amino}ethyl)amino]-1-oxobutan-2-yl}-L-aspartamide trifluoroacetate (1:1)

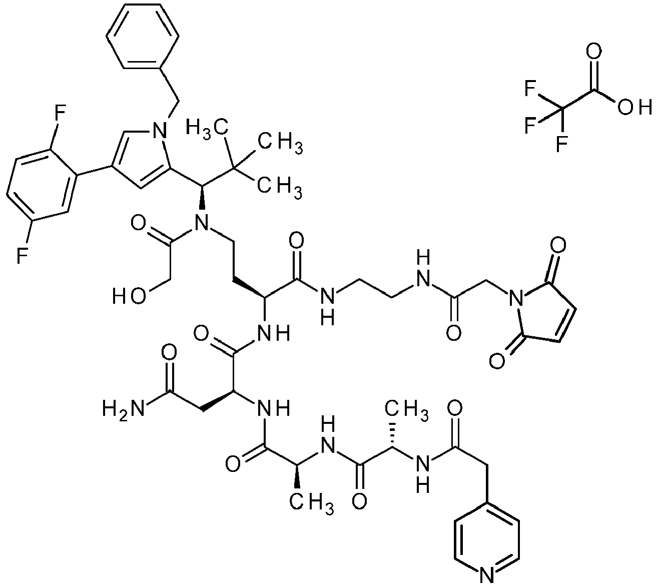

7 mg (0.0087 mmol) von Intermediat F104 wurden in Analogie zu Intermediat Q9 mit Intermediat L103 gekuppelt.. Es wurden 3.5 mg (34% d. Th.) der Titelverbindung erhalten.

LC-MS (Methode 1): Rₜ = 0.83 min; MS (ESIpos): m/z = 1068 (M+H)⁺.

### Intermediat Q12

### N-isonicotinoyl-L-alanyl-L-alanyl-N1-{(2S)-4-[{(1R)-1-[1-benzyl-4-(2,5-difluorophenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamide trifluoroacetate (1:1)

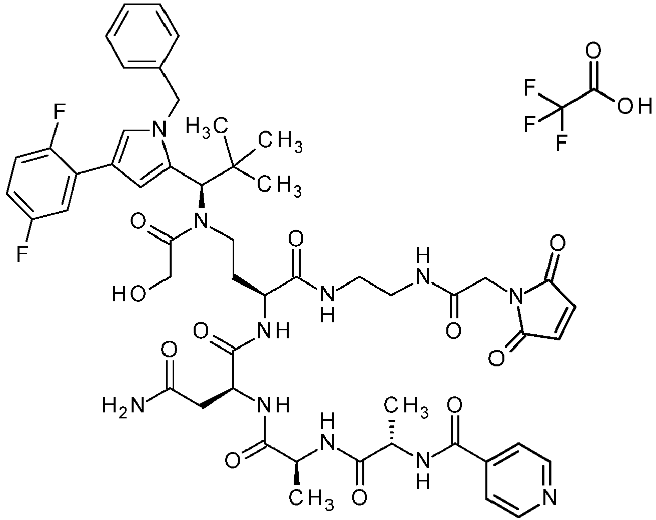

8 mg (0.0092 mmol) von Intermediat F104 wurden in Analogie zu Intermediat Q9 mit Intermediat L104 gekuppelt. Es wurden 6 mg (46% d. Th.) der Titelverbindung erhalten.

LC-MS (Methode 12): Rₜ = 1.71 min; MS (ESIpos): m/z = 1054 (M+H)⁺.

### Intermediat Q13

### N-{[2-(2-methoxyethoxy)ethoxy]acetyl}-L-alanyl-L-alanyl-N<sup>1</sup>-{(2S)-4-[{(1R)-1-[1-benzyl-4-(2,5-difluorophenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamide

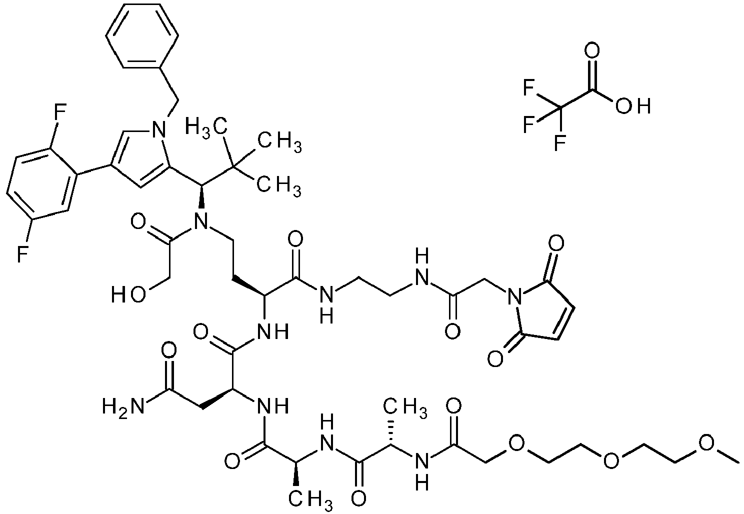

7 mg (0.0087 mmol) von Intermediat F104 wurden in Analogie zu Intermediat Q9 mit Intermediat L105 gekuppelt.. Es wurden 4.6 mg (47% d. Th.) der Titelverbindung erhalten.

LC-MS (Methode 1): Rₜ = 0.97 min; MS (ESIpos): m/z = 1109 (M+H)⁺.

### Intermediat Q14

### N-[(Benzyloxy)carbonyl]-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-glutamid

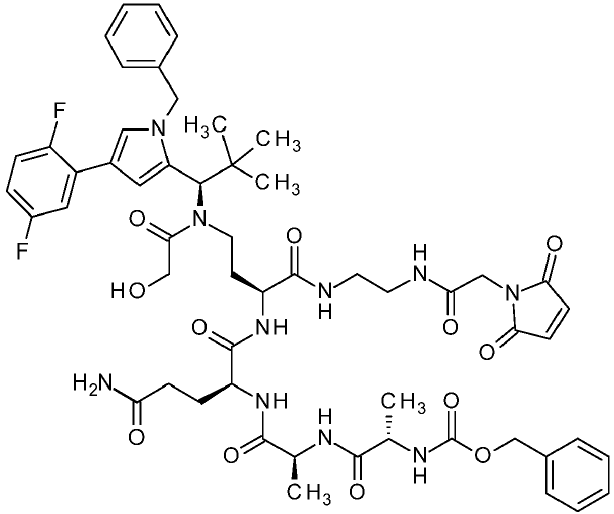

LC-MS (Methode 1): Rₜ = 1.15 min; MS (ESIpos): m/z = 1097 (M+H)⁺.

Dieses Intermediat wurde in Analogie zu Intermediat Q1 hergestellt.

### Intermediat Q15

### N-[(Benzyloxy)carbonyl]-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-alpha-asparagin

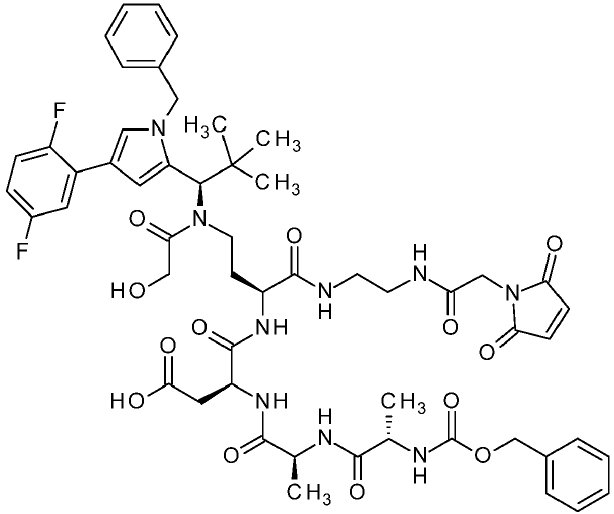

10 mg (0.012 mmol) von Intermediat F104 wurden in Analogie zu Intermediat Q1 mit Intermediat L106 gekuppelt. Anschließend wurde durch Spaltung des Trimethylsilylethylesters mit Zinkchlorid die Titelverbindung hergestellt.

LC-MS (Methode 1): Rₜ = 1.1 min; MS (ESIpos): m/z = 1085 (M+H)⁺.

In Analogie zu Intermediat Q1 wurden mit entsprechenden Intermediat-Vorstufen die folgenden Intermediate Q16-Q22 hergestellt:

### Intermediat Q16

### N-[(Benzyloxy)carbonyl]-L-valyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

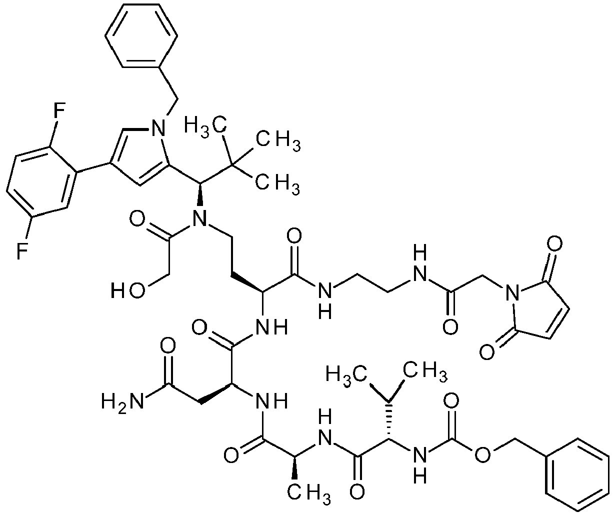

LC-MS (Methode 1): Rₜ = 1.13 min; MS (ESIpos): m/z = 1111 (M+H)⁺.

### Intermediat Q17

### N-[(Benzyloxy)carbonyl]-L-alanyl-L-valyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

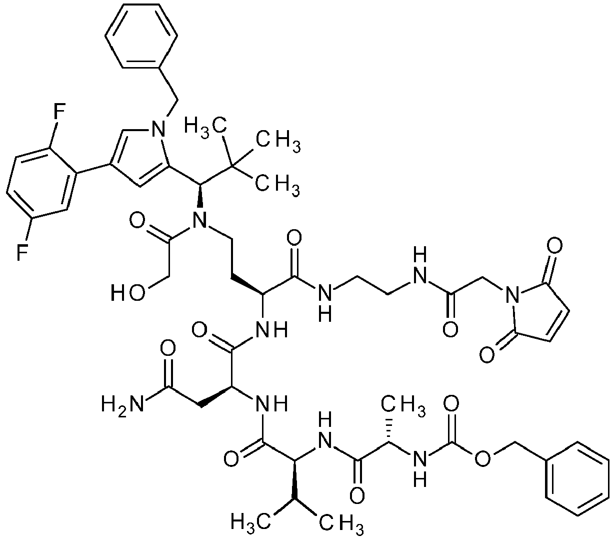

LC-MS (Methode 1): Rₜ = 1.13 min; MS (ESIpos): m/z = 1111 (M+H)⁺.

### Intermediat Q18

### N-[(2,5-Dioxo-2,5-dihydro-1H-pyrrol-1-yl)acetyl]-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid

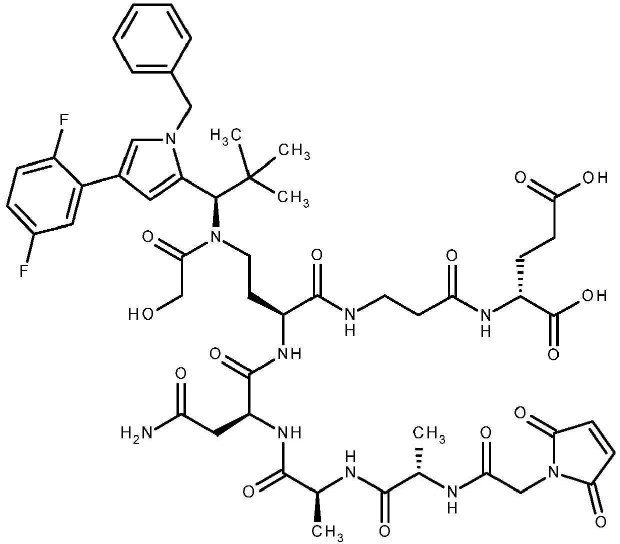

Die Titelverbindung wurde ausgehend von Verbindung C111 zunächst durch Kupplung mit Intermediat L107 in Gegenwart von HATU und N,N-Diisopropylethylamin und anschließende Entschützung mittels Zinkchlorid in Trifluorethanol hergestellt.

LC-MS (Methode 1): Rₜ = 0.89 min; MS (ESIpos): m/z = 1107 [M+H]⁺.

### Intermediat Q19

### N-[(Benzyloxy)carbonyl]-L-alanyl-glycyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

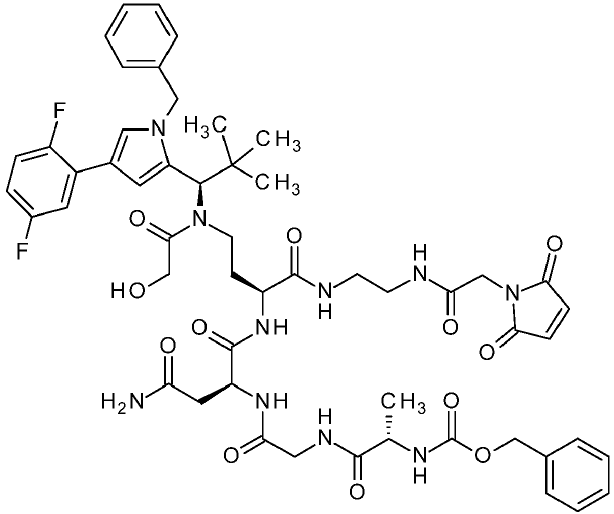

LC-MS (Methode 1): Rₜ = 1.06 min; MS (ESIpos): m/z = 1069 (M+H)⁺.

### Intermediat Q20

### N-(38-Oxo-2,5,8,11,14,17,20,23,26,29,32,35-dodecaoxaoctatriacontan-38-yl)-L-alanyl-L-alanyl-N1-[(20R)-25-{(1R)-1-[1-benzyl-4-(2,5-difluorphenyl)-1H-pyrrol-2-yl]-2,2-dimethylpropyl}-20-carboxy-1-(2,5-dioxo-2,5-dihydro-1H-pyrrol-1-yl)-2,18,24-trioxo-6,9,12,15-tetraoxa-22-thia-3,19,25-triazaoctacosan-28-yl]-L-aspartamid

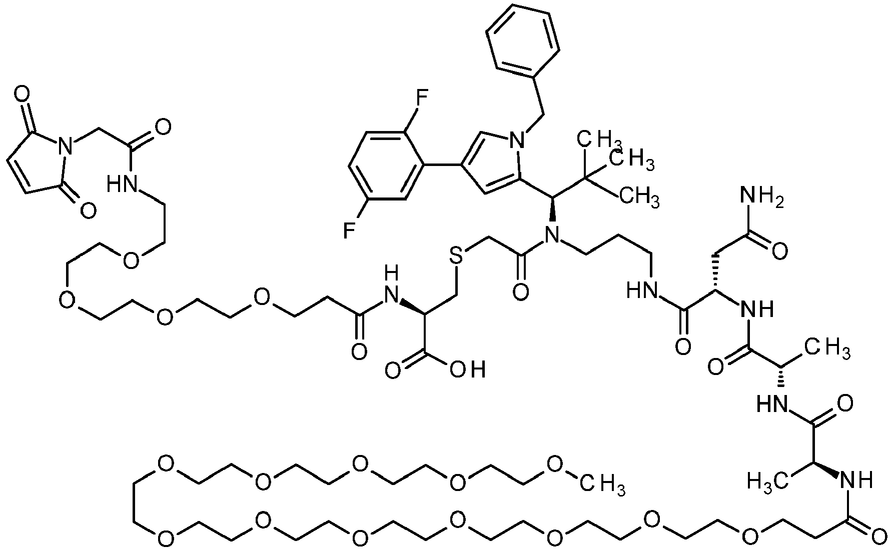

Bei 0°C wurden 47,2 mg (0.0559 mmol) von Intermediat L102 in 0,50 mL DMF aufgenommen und mit 7,2 mg (0.053 mmol) HOAt sowie mit 20,2 mg (0,0532 mmol) HATU und 74,1 µl (0.425 mmol) N,N-Diisopropylethylamin versetzt. Nach 5 min Rühren bei 0°C wurde 57,0 mg (0.0532 mmol) Intermediat F257 in DMF (0,5 mL) addiert. Nach 2 h Rühren bei 0°C wurde Wasser (1,0 mL) und ACN (1,5 mL) addiert. Der Ansatzt wurde zweimal durch präparative HPLC gereinigt (Eluent: ACN/Wasser + 0.1% TFA, Gradient = 1:2 → 2: 1). Die entsprechenden Fraktionen wurden eingeengt und der Rückstand aus Acetonitril/Wasser lyophilisiert. Es wurden 9.0 mg (9.5% d. Th.) der Titelverbindung erhalten.

LC-MS (Methode 14): Rₜ = 5,43 min; MS (ESIpos): m/z = 1783.8643 (M+H)⁺.

### Intermediat Q21

### N-(38-Oxo-2,5,8,11,14,17,20,23,26,29,32,35-dodecaoxaoctatriacontan-38-yl)-L-alanyl-L-alanyl-N1-{(2S)-4-[{(1R)-1-[1-benzyl-4-(2,5-difluorphenyl)-1H-pyrrol-2-yl]-2,2-dimethylpropyl} (glycoloyl)amino]-1-[(2-{ [(2,5-dioxo-2,5-dihydro-1H-pyrrol-1-yl)acetyl]amino } ethyl)amino] -1-oxobutan-2-yl} -L-aspartamid

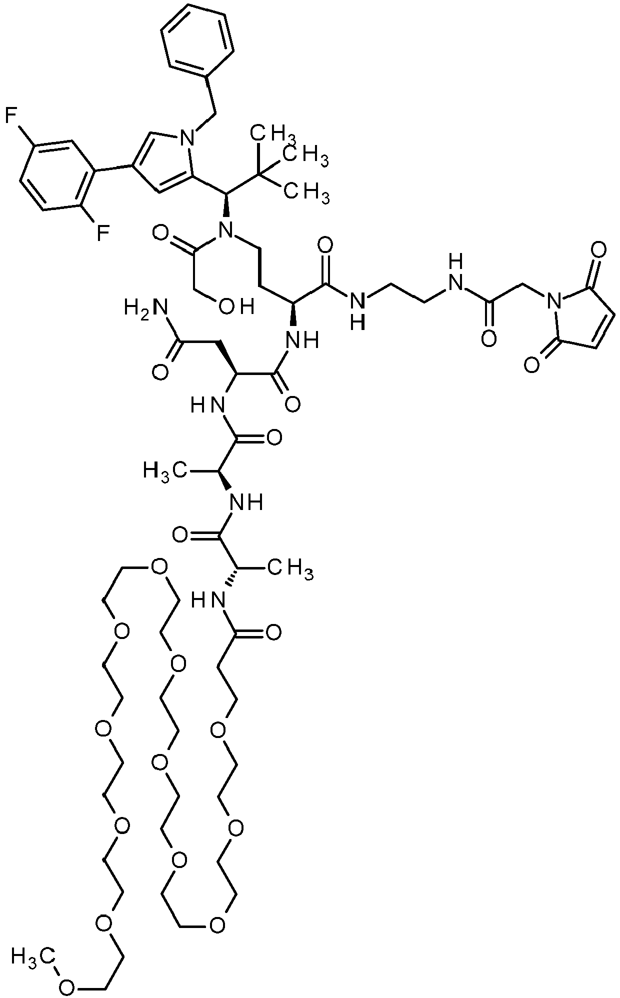

Bei 0°C wurden 57,5 mg (0.0713 mmol) von Intermediat F104 in 0,80 mL DMF aufgenommen und mit 61,2 mg (0,0727 mmol) von Intermediat L102 sowie mit 27,6 mg (0,0727 mmol) HATU und 74,5 µl (0.428 mmol) N,N-Diisopropylethylamin versetzt. Nach 30 min Rühren bei 0°C wurden Wasser (1,0 mL) und ACN (1,5 mL) addiert. Der Ansatzt wurde durch präparative HPLC gereinigt (Eluent: ACN/Wasser + 0.1% TFA, Gradient = 45% → 85%). Die entsprechenden Fraktionen wurden eingeengt und der Rückstand aus Acetonitril/Wasser lyophilisiert. Es wurden 39 mg (36% d. Th.) der Titelverbindung erhalten.

LC-MS (Methode 14): Rₜ = 5,44 min; MS (ESIpos): m/z = 1519.7598 (M+H)⁺.

### Intermediat Q22

### N-[(2,5-Dioxo-2,5-dihydro-1H-pyrrol-1-yl)acetyl]-L-alanyl-L-alanyl-N¹-[(2S)-4-[{(1R)-1-[1-benzyl-4-(2,5 -difluorphenyl)- 1H-pyrrol-2-yl] -2,2-dimethylpropyl} (glycoloyl)amino] -1-{ [2-(L-gamma-glutamylamino)ethyl] amino } -1-oxobutan-2-yl] -L-aspartamid

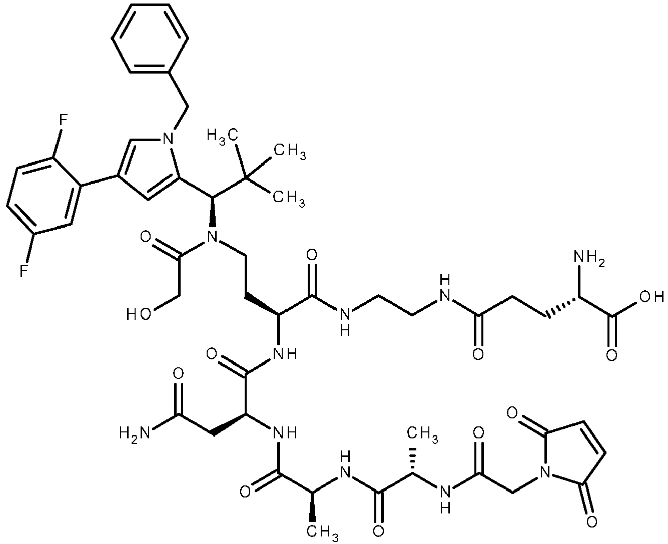

Die Titelverbindung wurde ausgehend von Verbindung C103 zunächst durch Kupplung mit Intermediat L92 in Gegenwart von HATU und N,N-Diisopropylethylamin hergestellt. Im nächsten Schritt wurde die Z-Schutzgruppe durch 30-minütige Hydrierung über 10%-igem Palladium auf Aktivkohle in Ethanol unter Wasserstoff-Normaldruck bei RT entfernt und das entschützte Intermediat anschließend mit 1-{6-[(2,5-Dioxopyrrolidin-1-yl)oxy]-6-oxohexyl}-1H-pyrrole-2,5-dion in Gegenwart von N,N-Diisopropylethylamin in DMF umgesetzt. Im letzten Schritt erfolgte die Entschützung mittels Zinkchlorid.

LC-MS (Methode 1): Rₜ = 0.84 min; MS (ESIpos): m/z = 1078 [M+H]⁺.

### Intermediat Q23

### N-{5-[(2,5-dioxopyrrolidin-1-yl)oxy]-5-oxopentanoyl}-L-alanyl-L-alanyl-N¹-{3-[{(1R)-1-[1-benzyl-4-(2,5-difluorophenyl)-1H-pyrrol-2-yl]-2,2-dimethylpropyl } (glycoloyl)amino]propyl }-L-aspartamide

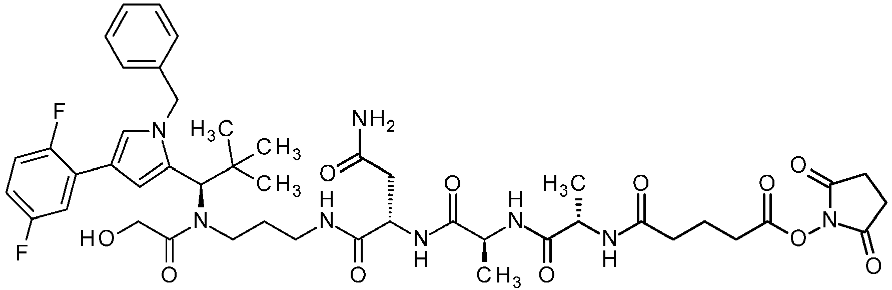

Die Titelverbindung wurde ausgehend von Verbindung M9 zunächst durch Kupplung mit Intermediat L92 in Gegenwart von HATU und N,N-Diisopropylethylamin hergestellt. Im nächsten Schritt wurde die Z-Schutzgruppe durch 30-minütige Hydrierung über 10%-igem Palladium auf Aktivkohle in Ethanol bei RT unter Wasserstoff-Normaldruck entfernt und das entschützte Intermediat anschließend wie in Intermediat Q6 beschrieben durch Umsetzung mit 1,1'-[(1,5-Dioxopentan-1,5-diyl)bis(oxy)] dipyrrolidin-2,5-dion in die Titelverbindung überführt.

LC-MS (Methode 12): Rₜ = 1.89 min; MS (ESIpos): m/z = 938 [M+H]⁺.

### Intermediat Q24

### N-[(2,5-dioxo-2,5-dihydro-1H-pyrrol-1-yl)acetyl]-L-alanyl-L-alanyl-N<sup>1</sup>-{3-[{(1R)-1-[1-benzyl-4-(2,5-difluorophenyl)-1H-pyrrol-2-yl]-2,2-dimethylpropyl}(glycoloyl)amino]propyl}-L-aspartamide trifluoroacetate (1:1)

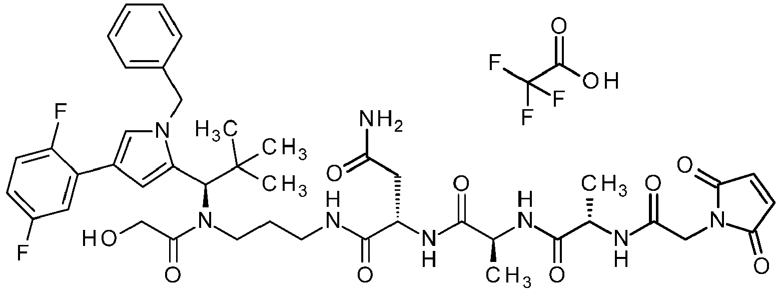

Die Titelverbindung wurde ausgehend von Verbindung M9 zunächst durch Kupplung mit Intermediat L92 in Gegenwart von HATU und N,N-Diisopropylethylamin hergestellt. Im nächsten Schritt wurde die Z-Schutzgruppe durch 30-minütige Hydrierung über 10%-igem Palladium auf Aktivkohle in Ethanol bei RT unter Wasserstoff-Normaldruck entfernt und das entschützte Intermediat anschließend durch Umsetzung mit 1-{2-[(2,5-dioxopyrrolidin-1-yl)oxy]-2-oxoethyl}-1H-pyrrole-2,5-dion in Gegenwart von N,N-Diisopropylethylamin in die Titelverbindung überführt.

LC-MS (Methode 1): Rₜ = 1.01 min; MS (ESIpos): m/z = 863 [M+H]⁺.

### Intermediat Q25

### N-[(2,5-dioxo-2,5-dihydro-1H-pyrrol-1-yl)acetyl]-L-alanyl-L-alanyl-N¹-{(2S)-1-[(3-{[(5S)-5-amino-5-carboxypentyl]amino}-3-oxopropyl)amino]-4-[{(1R)-1-[1-benzyl-4-(2,5-difluorophenyl)-1H-pyrrol-2-yl]-2,2-dimethylpropyl}(glycoloyl)amino]-1-oxobutan-2-yl}-L-aspartamide trifluoroacetate (1:1)

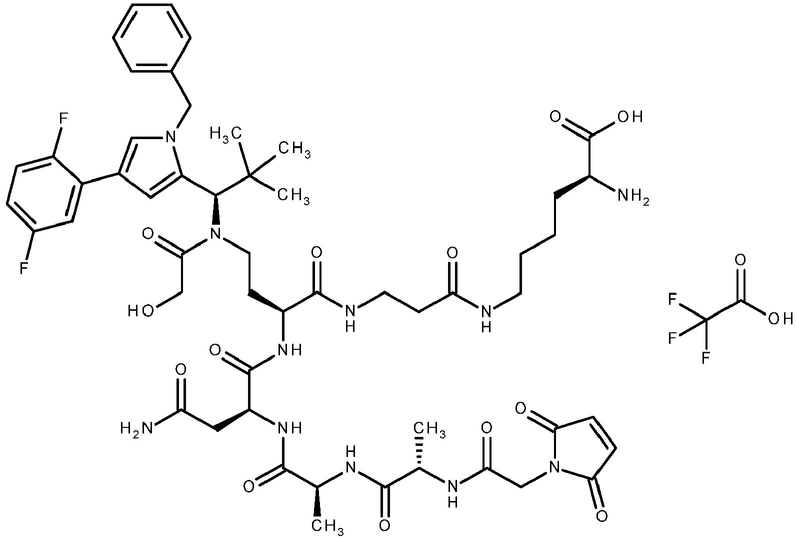

Die Titelverbindung wurde ausgehend von Verbindung C108 zunächst durch Kupplung mit Intermediat L92 in Gegenwart von HATU und N,N-Diisopropylethylamin hergestellt. Im nächsten Schritt wurde die Z-Schutzgruppe durch 1-stündige Hydrierung über 10%-igem Palladium auf Aktivkohle in DCM-Methanol 1:1 unter Wasserstoff-Normaldruck bei RT entfernt und das entschützte Intermediat anschließend durch Umsetzung mit 1-{2-[(2,5-dioxopyrrolidin-1-yl)oxy]-2-oxoethyl}-1H-pyrrole-2,5-dion in Gegenwart von N,N-Diisopropylethylamin und schließlich durch Entschützung mittels Zinkchlorid in die Titelverbindung überführt.

LC-MS (Methode 12): Rₜ = 1.46 min; MS (ESIpos): m/z = 1106 [M+H]⁺.

### Intermediat Q26

### N-[6-(2,5-dioxo-2,5-dihydro-1H-pyrrol-1-yl)hexanoyl]-L-alanyl-L-alanyl-N¹-{(2S)-1-[(3-{[(5S)-5-amino-5-carboxypentyl]amino}-3-oxopropyl)amino]-4-[{(1R)-1-[1-benzyl-4-(2,5-difluorophenyl)-1H-pyrrol-2-yl]-2,2-dimethylpropyl } (glycoloyl)amino]-1-oxobutan-2-yl }-L-aspartamide trifluoroacetate (1:1)

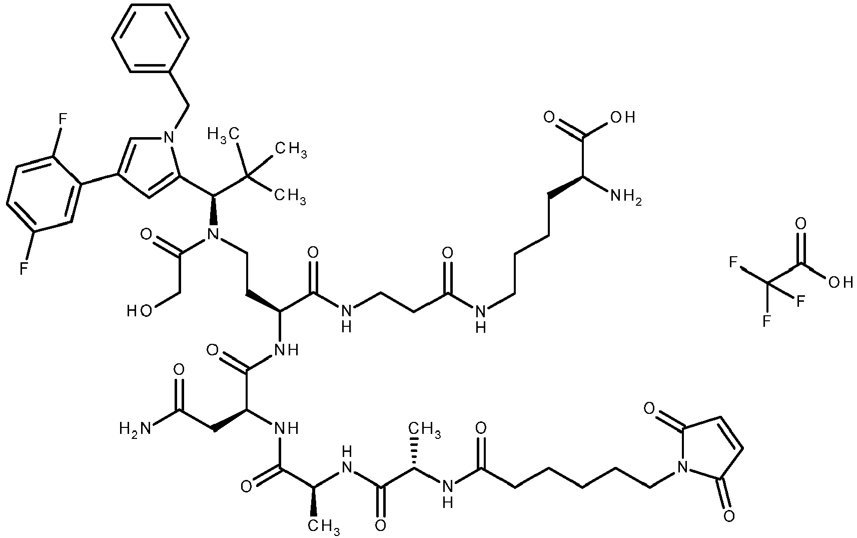

Die Titelverbindung wurde in Analogie zu Intermediat Q25 hergestellt.

LC-MS (Methode 12): Rₜ = 1.55 min; MS (ESIpos): m/z = 1162 [M+H]⁺.

### Intermediat 027

### N-[(2,5-Dioxo-2,5-dihydro-1H-pyrrol-1-yl)acetyl]-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid

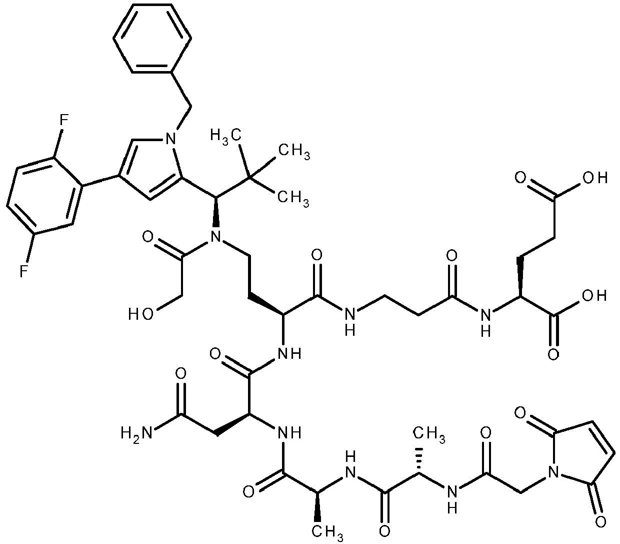

Die Titelverbindung wurde ausgehend von Verbindung C109 zunächst durch Kupplung mit Intermediat L107 in Gegenwart von HATU und N,N-Diisopropylethylamin und anschließende Entschützung mittels Zinkchlorid hergestellt.

LC-MS (Methode 1): Rₜ = 0.95 min; MS (ESIpos): m/z = 1107 [M+H]⁺.

### Intermediat Q28

### N-[6-(2,5-Dioxo-2,5-dihydro-1H-pyrrol-1-yl)hexanoyl]-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid

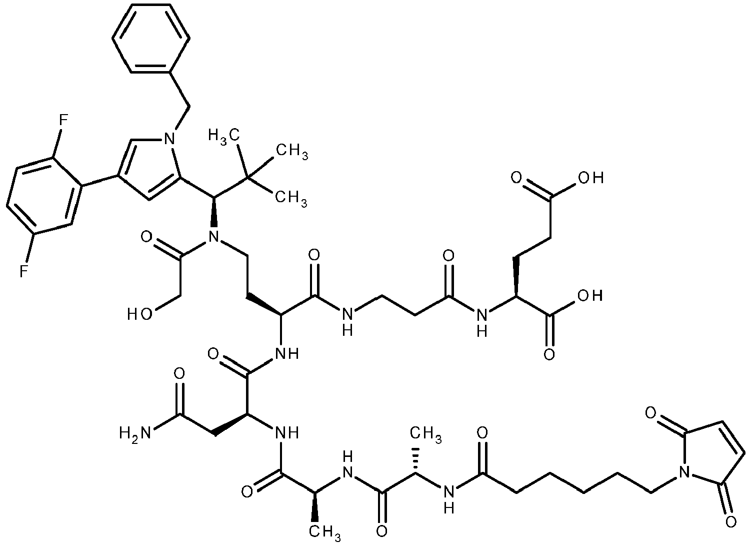

Die Titelverbindung wurde ausgehend von Verbindung C110 zunächst durch Kupplung mit Intermediat L92 in Gegenwart von HATU und N,N-Diisopropylethylamin hergestellt. Im nächsten Schritt wurden alle Schutzgruppen durch 1-stündige Hydrierung über 10%-igem Palladium auf Aktivkohle in DCM-Methanol 1:1 unter Wasserstoff-Normaldruck bei RT entfernt und das entschützte Intermediat anschließend durch Umsetzung mit 1-{6-[(2,5-Dioxopyrrolidin-1-yl)oxy]-6-oxohexyl}-1H-pyrrole-2,5-dion in Gegenwart von N,N-Diisopropylethylamin in die Titelverbindung überführt.

LC-MS (Methode 12): Rₜ = 1.74 min; MS (ESIneg): m/z = 1161 [M-H]⁻.

### Intermediat Q29

### N-{5-[(2,5-Dioxopyrrolidin-1-yl)oxy]-5-oxopentanoyl}L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid

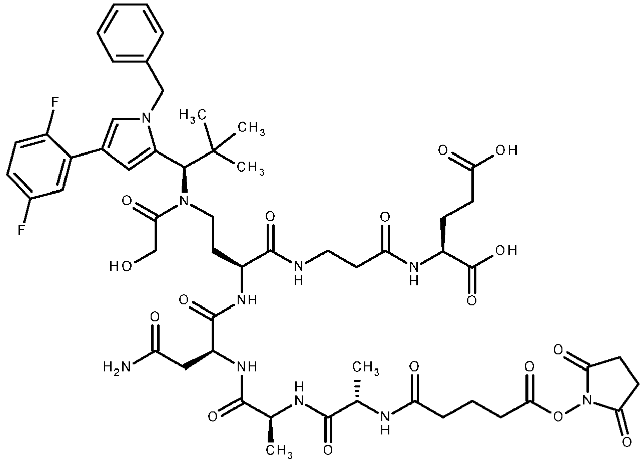

40 mg (39 µmol) von Intermediat C110 wurden in 9 mL DMF in Gegenwart von 30 mg HATU und 34 µL N,N-Diisopropylethylamin mit 19.6 mg (47 mmol) Intermediat L92 gekuppelt. Anschließend wurden alle Schutzgruppen durch 1-stündige Hydrierung über 10%-igem Palladium auf Aktivkohle in Methanol unter Wasserstoff-Normaldruck bei RT entfernt und das entschützte Intermediat anschließend durch Umsetzung mit 1-{6-[(2,5-Dioxopyrrolidin-1-yl)oxy]-6-oxohexyl}-1H-pyrrole-2,5-dion in Gegenwart von N,N-Diisopropylethylamin in die Titelverbindung überführt, die durch präparative HPLC gereinigt wurde.

LC-MS (Methode 1): Rₜ = 0.9 min; MS (ESIpos): m/z = 1181 (M+H)⁺.

### Intermediat Q30

### N-{6-[(2,5-Dioxopyrrolidin-1-yl)oxy]-6-oxohexyl}-N-methyl-L-alanyl-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl) amino]-1-[(3-{[(1S)-1,3-dicarboxypropyl]amino}-3-oxopropyl)amino]-1-oxobutan-2-yl}-L-aspartamid

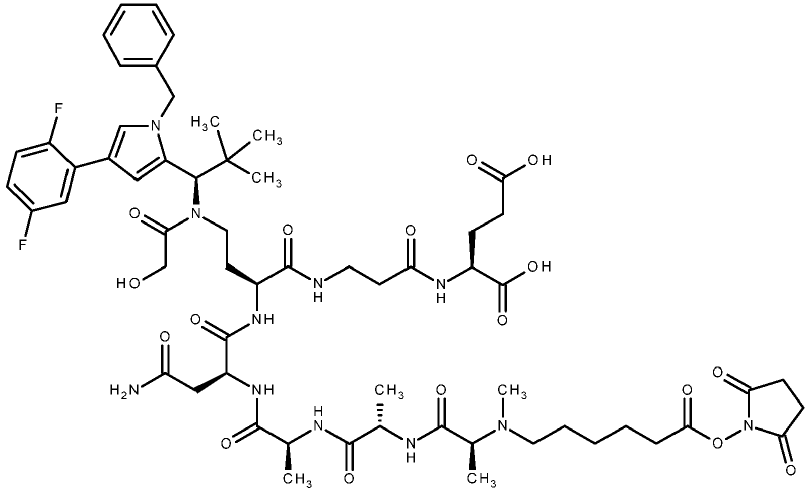

Zunächst wurde Trifluoressigsäure --4-nitrobenzyl-L-alanyl-L-alanyl-L-asparaginat (1:1) durch Kupplung von N-(tert-Butoxycarbonyl)-L-alanyl-L-alanin mit 4-Nitrobenzyl-L-asparaginat hydrobromid (1:1) in DMF in Gegenwart von HATU und N,N-Diisopropylethylamin und anschließender Entschützung der Aminogruppe mit Trifluoressigsäure in DCM hergestellt.

LC-MS (Methode 1): Rₜ = 0.43 min; MS (ESIpos): m/z = 410 (M+H)⁺.

Dieses Intermediat wurde in DMF in Gegenwart von HATU und N,N-Diisopropylethylamin mit N-(tert-butoxycarbonyl)-N-methyl-L-alanin gekuppelt. Anschließend wurde der p-Nitro-benzylester durch Hydrierung in DCM-Methanol 1:1 über 10% Palladium auf Aktivkohle abgespalten. LC-MS (Methode 1): Rₜ = 0.55 min; MS (ESIpos): m/z = 460 (M+H)⁺.

Das so erhaltene Intermediat wurde in DMF in Gegenwart von HATU und N,N-Diisopropylethylamin mit Intermediat C110 gekuppelt. Anschließend wurde die Boc-Schutzgruppe durch 1h Rühren bei 50°C mit 4 Equivalenten Zinkchlorid in Trifluorethanol abgespalten.

LC-MS (Methode 1): Rₜ = 1.07 min; MS (ESIpos): m/z = 1235 (M+H)⁺.

44 mg (32 µmol) von diesem Intermediat wurden mit 25.4 mg (195 µmol) 6-Oxohexansäure, die zuvor nach Litereaturvorschrift hergestellt wurde (J.Org.Chem. 1993, 58, 2196), in 20 mL Methanol zusammengegeben und mit 7.8 µL Essigsäure sowie 29 mg (313 µmol) Boran-PyridinKomplex versetzt. Der Ansatz wurde 1 h bei RT gerührt und anschließend im Vakuum eingeengt und durch präparative HPLC gereinigt. Es wurden 25 mg (56% d. Th.) von (6S,13S,16S,19S,22S,25S)-16-(2-Amino-2-oxoethyl)-13-{2-[{(1R)-1-[1-benzyl-4-(2,5-difluorphenyl)-1H-pyrrol-2-yl]-2,2-dimethylpropyl } (glycoloyl)amino]ethyl }-6-[(benzyloxy) carbonyl]-19,22,25,26-tetramethyl-3,8,12,15,18,21,24-heptaoxo-1-phenyl-2-oxa-7,11,14,17,20,23,26-heptaazadotriacontan-32-säure erhalten.

24.5 mg (0.018 mmol) von diesem Intermediat wurden in 6 ml DMF aufgenommen und mit 34 mg (0.27 mmol) 1-Hydroxypyrrolidin-2,5-dion und 16 µL *N,N*-Diisopropylethylamin sowie portionsweise mit insgesamt 50 mg (0.13 mmol) HATU versetzt. Nach 2 h Rühren bei RT wurde die Reaktionslösung mit TFA auf einen pH-Wert von 3-4 eingestellt und anschließend eingeengt und durch präparative HPLC gereinigt. Es wurden 23 mg (87%) des geschützten Intermediats erhalten, welches anschließend in 10 ml Ethanol aufgenommen wurde. Nach Zugabe von 10% Palladium auf Aktivkohle wurden die Benzylestergruppen hydrogenolytisch unter Wasserstoff-Normaldruck entfernt und nach Abfiltrieren des Katalysators, Einengen der verbliebenen Lösung, und anschließender Lyophilisation des Rückstandes aus Acetonitril/Wasser 9:1 wurden 20 mg (95% d. Th.) der Titelverboindung erhalten.

LC-MS (Methode 1): Rₜ = 0.82 min; MS (ESIpos): m/z = 1266 (M+H)⁺.

### Intermediat Q31

### Trifluoressigsäure --N-(pyridin-4-ylacetyl)-L-alanyl-L-alanyl-N¹-{(2S)-1-({2-[(N²-acetyl-L-lysyl) amino]ethyl}amino)-4-[{(1R)-1-[1-benzyl-4-(2,5-difluorphenyl)-1H-pyrrol-2-yl]-2,2-dimethyl propyl} (glycoloyl)amino] -1 -oxobutan-2-yl} -L-aspartamid (1:1)

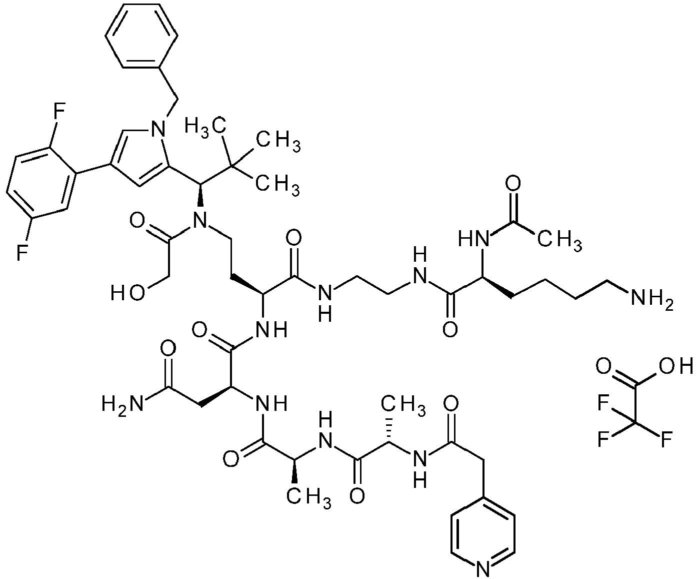

Die Titelverbindung wurde durch HATU-Kupplung von Intermediat C112 mit Intermediat L103 in DMF in Gegenwart von N,N-Diisopropylethylamin und anschließender Abspaltung der Boc-Schutzgruppe durch 30 min Rühren bei 50°C in Trifluorethanol mit 6 Equivalenten Zinkchlorid hergestellt.

LC-MS (Methode 1): Rₜ = 0.76 min; MS (ESIpos): m/z = 1101 (M+H)⁺.

### Intermediat Q32

### N²-Acetyl-L-lysyl-L-alanyl-L-alanyl-N¹-{(2S)-1-[(3-{[(5S)-5-amino-5-carboxypentyl]amino}-3-oxopropyl)amino]-4-[{(1R)-1-[1-benzyl-4-(2,5-difluorphenyl)-1H-pyrrol-2-yl]-2,2-dimethyl propyl}(glycoloyl)amino]-1-oxobutan-2-yl}-L-aspartamid -trifluoressigsäure (1:1)

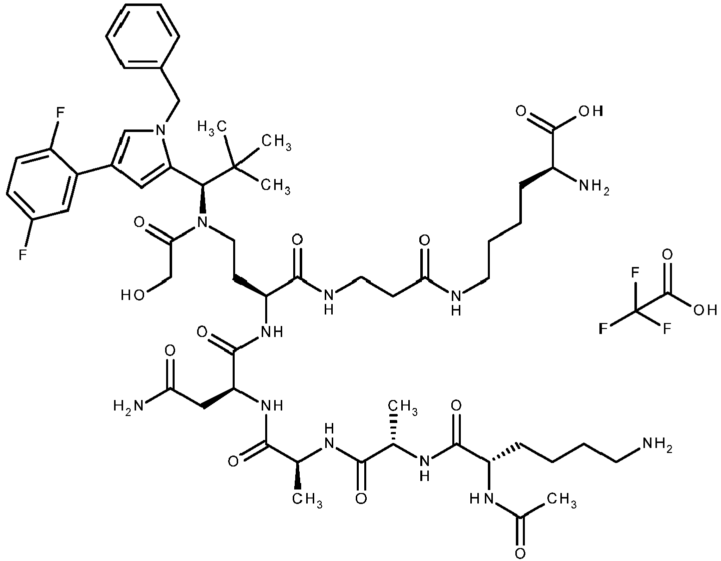

Zunächst wurde Intermediat C108 mit Intermediat L92 in DMF in Gegenwart von HATU und N,N-Diisopropylethylamin gekuppelt. Im nächsten Schritt wurde die Z-Schutzgruppe durch 1-stündige Hydrierung über 10%-igem Palladium auf Aktivkohle in DCM-Methanol 1:1 unter Wasserstoff-Normaldruck bei RT entfernt. Anschließend wurde das entschützte Intermediat durch Umsetzung mit Intermediat L109 in DMF in Gegenwart von HATU und N,N-Diisopropyl-ethylamin und anschließende Entschützung durch 90 min Rühren mit 12 Equivalenten Zinkchlorid in Trifluorethanol bei 50°C in die Titelverbindung überführt.

LC-MS (Methode 12): Rₜ = 1.17 min; MS (ESIneg): m/z = 1137 (M-H)⁻.

### Intermediat Q33

### N²-Acetyl-L-lysyl-L-alanyl-L-alanyl-N¹-[(2S)-4-[{(1R)-1-[1-benzyl-4-(2,5-difluorphenyl)-1H-pyrrol-2-yl]-2,2-dimethylpropyl}(glycoloyl)amino]-1-{[2-(L-gamma-glutamylamino)ethyl]amino} -1-oxobutan-2-yl]-L-aspartamid -trifluoressigsäure (1:1)

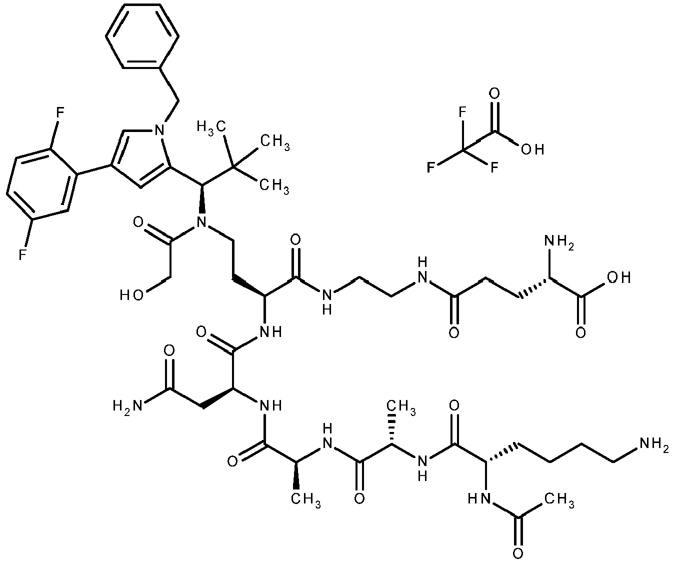

Die Titelverbindung wurde durch HATU-Kupplung von Intermediat C103 mit Intermediat L110 in DMF in Gegenwart von N,N-Diisopropylethylamin und anschließender vollständiger Entschützung durch 6 h Rühren in Trifluorethanol bei 50°C mit 10 Equivalenten Zinkchlorid hergestellt. LC-MS (Methode 1): Rₜ = 0.72 min; MS (ESIpos): m/z = 1111 (M+H)⁺.

### Intermediat Q34

### N²-Acetyl-L-lysyl-L-alanyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(3-{[(1S)-1,3-dicarboxypropyl]amino}-3-oxopropyl)amino]-1-oxobutan-2-yl}-L-aspartamid -trifluoressigsäure (1:1)

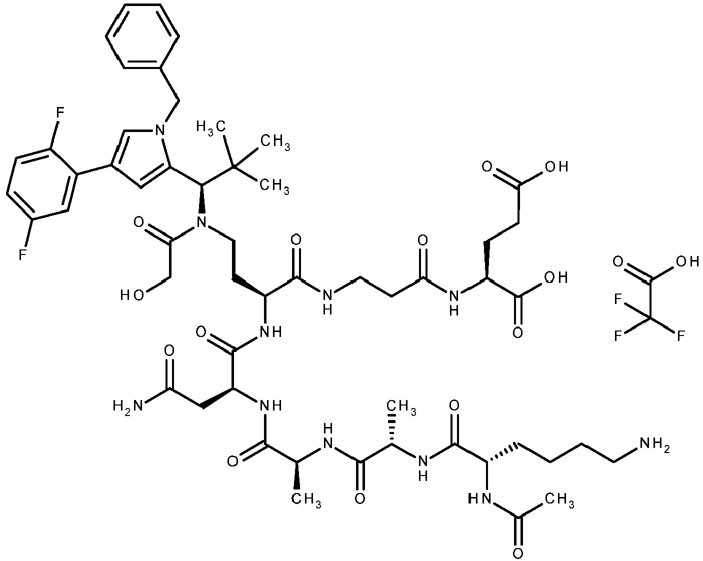

Die Synthese der Titelverbindung begann mit der HATU-Kupplung von Intermediat C110 mit Intermediat L110 in DMF in Gegenwart von N,N-Diisopropylethylamin. Im nächsten Schritt wurden die Benzylester-Schutzgruppe durch 1.5-stündige Hydrierung über 10%-igem Palladium auf Aktivkohle in DCM-Methanol 1: 1 unter Wasserstoff-Normaldruck bei RT entfernt. Im letzten Schritt erfolgte die Abspaltung der Teoc-Schutzgruppe durch 6 h Rühren in Trifluorethanol bei 50°C mit 8 Equivalenten Zinkchlorid.

LC-MS (Methode 12): Rₜ = 1.31 min; MS (ESIpos): m/z = 1140 (M+H)⁺.

### Intermediat Q35

### N-(Pyridin-4-ylacetyl)-L-alanyl-N-methyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro- IH-pyrrol-1 -yl)acetyl] amino } ethyl)amino] -1 -oxobutan-2-yl} -L-aspartamid

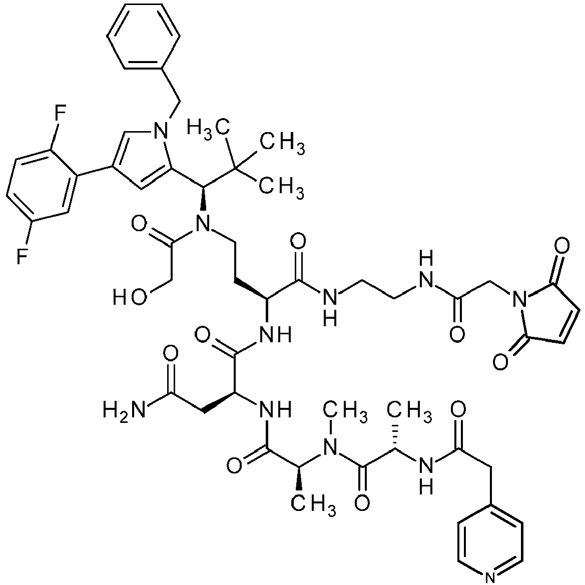

15 mg (0.019 mmol) von Intermediat F104 wurden in Analogie zu Intermediat Q1 mit 10 mg (0.024 mmol) Intermediat L111 gekuppelt. Nach Reinigung mittels präparativer HPLC wurden 4 mg (17% d.Th.) der Titelverbindung erhalten.

LC-MS (Methode 1): Rₜ = 0.82 min; MS (ESIpos): m/z = 1082 (M+H)⁺.

### Intermediat Q36

### N-Acetyl-L-alanyl-N-methyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid

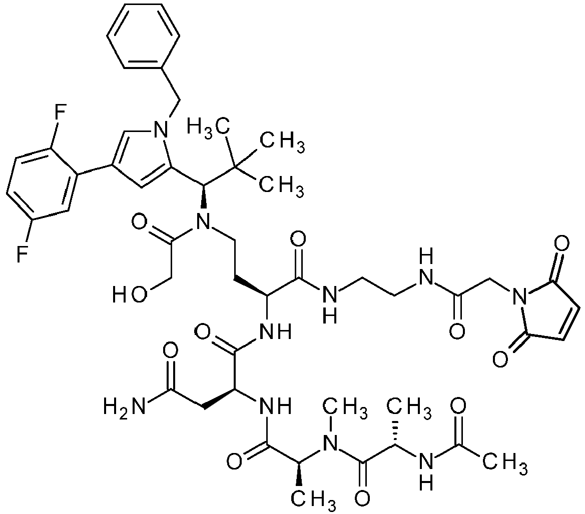

15 mg (0.019 mmol) von Intermediat F104 wurden in Analogie zu Intermediat Q1 mit 8 mg (0.024 mmol) Intermediat L112 gekuppelt. Nach Reinigung mittels präparativer HPLC wurden 5.8 mg (28% d.Th.) der Titelverbindung erhalten.

LC-MS (Methode 1): Rₜ = 0.95 min; MS (ESIpos): m/z = 1005 (M+H)⁺.

### Intermediat Q37

### N-(Pyridin-4-ylacetyl)-L-alanyl-L-alanyl-N¹-[(2S)-4-[{(1R)-1-[1-benzyl-4-(2,5-difluorphenyl)-1H-pyrrol-2-yl]-2,2-dimethylpropyl}(glycoloyl)amino]-1-({4-[(2,5-dioxopyrrolidin-1-yl)oxy]-4-oxo butyl} amino) -1 -oxobutan-2-yl] -L-aspartamid

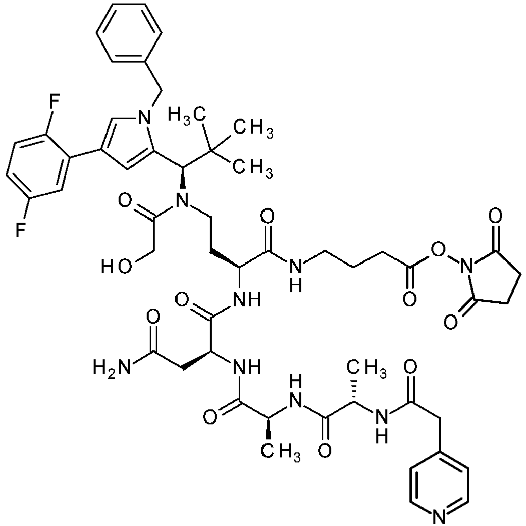

20 mg (31 µmol) von Intermediat C114 wurden zusammen mit 11.6 mg (22.9 µmol) N-(Pyridin-4-ylacetyl)-L-alanyl-L-alanyl-L-asparagin -trifluoressigsäure (1:1) (Intermediat L104) in 5.0 mL DMF vorgelegt. Dann wurden 11 µl N,N-Diisopropylethylamin und 21 mg (55 µmol) HATU zugegeben. Die Reaktionsmischung wurde 60 Minuten bei RT gerührt. Die Reaktionsmischung wurde direkt mittles präp. RP-HPLC (Säule: Reprosil 250x30; 10µ, Fluss: 50 mL/min, MeCN/Wasser, 0.1%TFA) gereinigt. Die Lösemittel wurden im Vakuum verdampft und der Rückstand im Hochvakuum getrocknet. Es folgte die Abspaltung des tert-Butylestergruppe durch 2-stündiges Rühren bei 50°C in Trifluorethanol mit 6 Equiv. Zinkchlorid. Nach Zugabe von 6 Equiv. EDTA erfolte die Reinigung durch präparative HPLC. Im letzten Schritt wurde das erhaltene Intermediat in DMF aufgenommen und mit 15 Equivalenten 1-Hydroxypyrrolidin-2,5-dion versetzt und durch 60 minütiges Rühren in Gegenwart von 5 Equiv. HATU und 5 Equiv. N,N-Diisopropyl ethylamin in die Titelverbindung überführt. Diese wurde durch präparative HPLC aufgereinigt.

LC-MS (Methode 1): Rₜ = 0.87 min; MS (ESIpos): m/z = 1071 (M+H)⁺.

### Intermediat Q38

### N-Methyl-N-(pyridin-4-ylacetyl)-L-alanyl-L-alanyl-N1-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro- IH-pyrrol-1 -yl)acetyl] amino } ethyl)amino] -1 -oxobutan-2-yl} -L-aspartamid

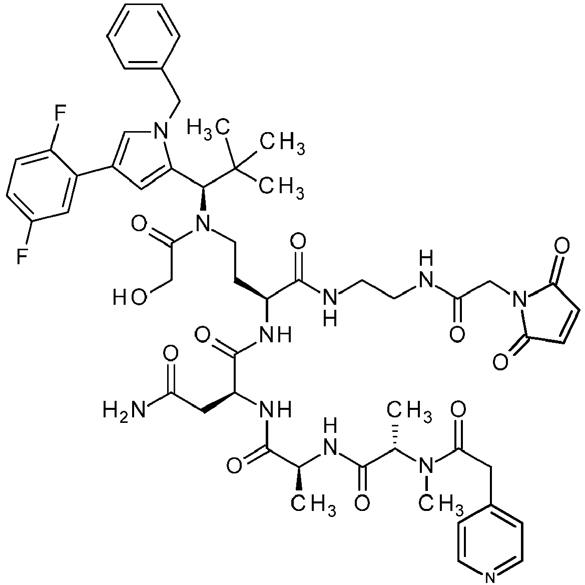

15 mg (0.019 mmol) von Intermediat F104 wurden in Analogie zu Intermediat Q1 mit 12.5 mg (0.024 mmol) Intermediat L113 gekuppelt. Nach Reinigung mittels präparativer HPLC wurden 4 mg (19% d.Th.) der Titelverbindung erhalten.

LC-MS (Methode 1): Rₜ = 0.83 min; MS (ESIpos): m/z = 1082 (M+H)⁺.

### Intermediat Q39

### N-[(2,5-Dioxo-2,5-dihydro-1H-pyrrol-1-yl)acetyl]-L-alanyl-L-alanyl-N¹-[(2S)-4-[{(1R)-1-[1-benzyl-4-(2,5-difluorphenyl)-1H-pyrrol-2-yl]-2,2-dimethylpropyl}(glycoloyl)amino]-1-(methylamino)-1-oxobutan-2-yl] -L-aspartamid

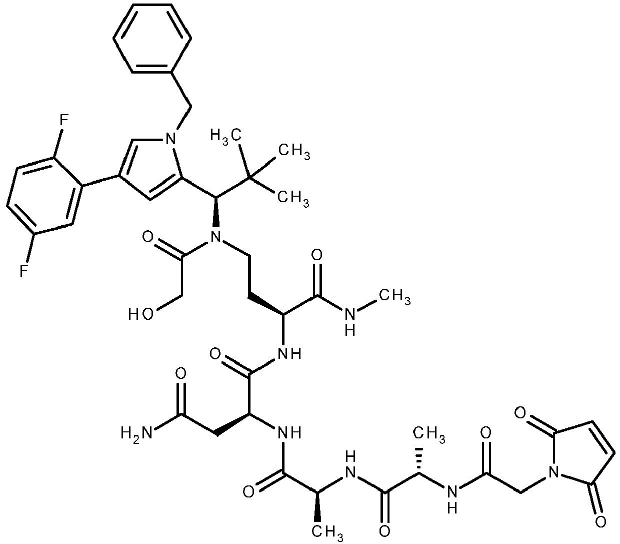

Die Titelverbindung wurde ausgehend von Verbindung C115 durch Kupplung mit Intermediat L107 in Gegenwart von HATU und N,N-Diisopropylethylamin hergestellt.

LC-MS (Methode 1): Rₜ = 0.97 min; MS (ESIpos): m/z = 920 [M+H]⁺.

### Intermediat Q40

### N-{[2-(2-Methoxyethoxy)ethoxy]acetyl}-L-alanyl-N-methyl-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5 -dihydro- IH-pyrrol-1 -yl)acetyl] amino }ethyl)amino] -1 -oxobutan-2-yl} -L-aspartamid

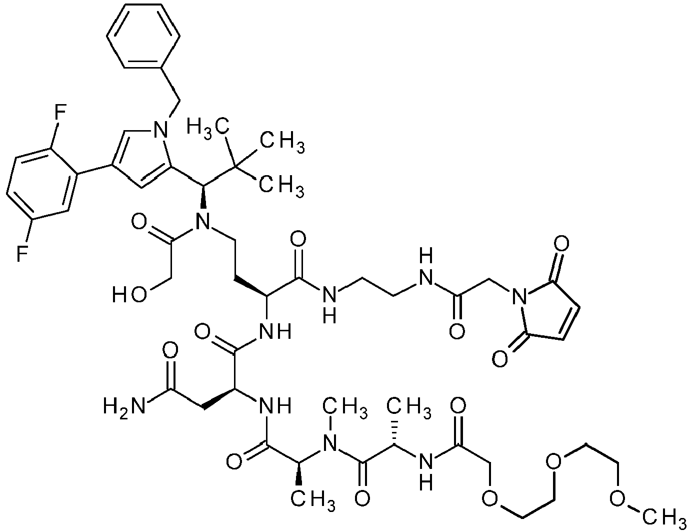

15 mg (0.019 mmol) von Intermediat F104 wurden in Analogie zu Intermediat Q1 mit 12.5 mg (0.024 mmol) Intermediat L114 gekuppelt. Nach Reinigung mittels präparativer HPLC wurden 10.8 mg (50% d.Th.) der Titelverbindung erhalten.

LC-MS (Methode 1): Rₜ = 0.99 min; MS (ESIpos): m/z = 1123 (M+H)⁺.

### Intermediat Q41

### N-[6-(2,5-Dioxo-2,5-dihydro-1H-pyrrol-1-yl)hexanoyl]-L-alanyl-L-alanyl-N¹-[(16S)-4-{(1R)-1-[1-benzyl-4-(2,5-difluorphenyl)-1H-pyrrol-2-yl]-2,2-dimethylpropyl}-16,18-dicarboxy-5,10,14-trioxo-7-thia-4,11,15-triazaoctadec-1-yl]-L-aspartamid-trifluoressigsäure (1:1)

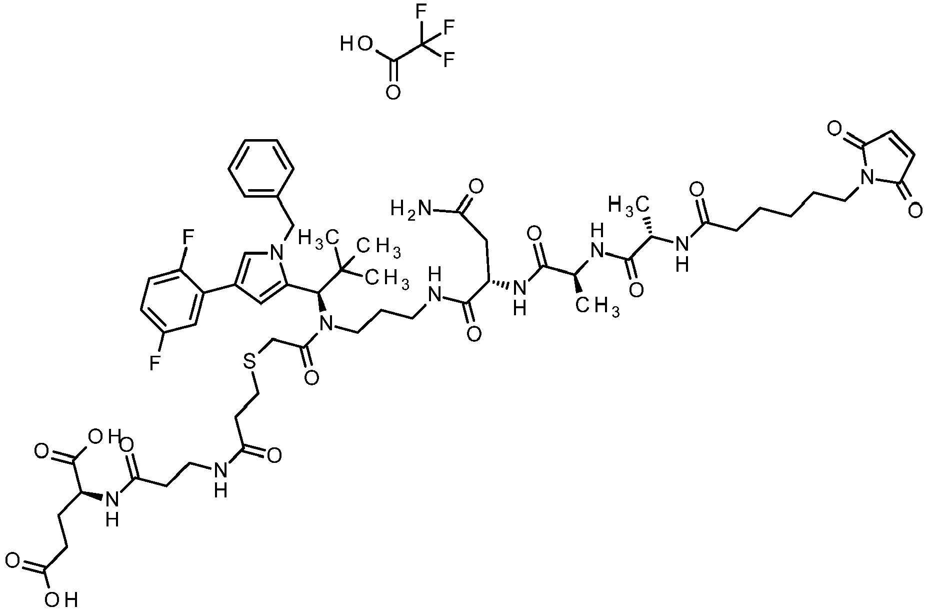

Unter Argon wurden 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,1 1-diaza-2-silaheptadecan-17-säure (174 mg, 94 % Reinheit, 234 µmol) (Intermediat C69) und Trifluoressigsäure dibenzyl-beta-alanyl-L-glutamat (1:1) (144 mg, 281 µmol) (Intermediat L115) in 4.0 ml DMF vorgelegt. Die Reaktionsmischung wurde mit HATU (107 mg, 281 µmol) und N,N-Diisopropylethylamin (120 µl, 700 µmol) versetzt und 10 min bei RT nachgerührt. Der Ansatz wurde mit Ethylacetat verdünnt und die organische Phase wurde mit Wasser und mit ges. NaCl-Lsg. gewaschen. Die organische Phase wurde über Magnesiumsulfat getrocknet und das Lösemittel im Vakuum verdampft. Der Rückstand wurde mittles präp. RP-HPLC (Säule: Reprosil 250x40; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 229 mg (85 % d. Th.) der Verbindung Dibenzyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,1 1-diaza-2-silaheptadecan-17-yl)-beta-alanyl-L-glutamat.

LC-MS (Methode 1): Rₜ = 1.55 min; MS (ESIpos): m/z = 1082 [M+H]⁺

Dibenzyl-N-(11-{(1R)-1-[1-benzyl-4-(2,5-difluorphenyl)-1H-pyrrol-2-yl]-2,2-dimethylpropyl}-2,2-dimethyl-6,12,17-trioxo-5-oxa-14-thia-7,11-diaza-2-silaheptadecan-17-yl)-beta-alanyl-L-glutamat (226 mg, 209 µmol) wurde in 10 ml Trifluorethanol gelöst. Die Reaktionsmischung wurde mit Zinkchlorid (171 mg, 1.25 mmol) versetzt und 1h bei 50°C nachgerührt. Es werden noch zweimal je 6eq. ZnCl2 nachgegeben und jeweils 1h bei 50°C nachgerührt. Der Ansatz wurde mit Ethylendiamin-N,N,N',N'-tetraessigsaeure (1.10 g, 3.75 mmol) versetzt und kurz nachgerührt. Der Reaktionsmischung wurde in Acetonitril aufgenommen, durch einen Spritzenfilter filtriert und mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 182 mg (83 % d. Th.) der Verbindung Trifluoressigsäure dibenzyl-N-[3-({2-[(3-aminopropyl){(1R)-1-[1-benzyl-4-(2,5-difluorphenyl)-1H-pyrrol-2-yl]-2,2-dimethylpropyl} amino] -2-oxoethyl} sulfanyl)propanoyl] -beta-alanyl-L-glutamat (1:1).

LC-MS (Methode 1): Rₜ = 1.07 min; MS (ESIpos): m/z = 938 [M+H]⁺

Unter Argon wurden Trifluoressigsäure dibenzyl-N-[3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)propanoyl]-beta-alanyl-L-glutamat (1:1) (55.0 mg, 52.3 µmol) und N-[(Benzyloxy)carbonyl]-L-alanyl-L-alanyl-L-asparagin (26.0 mg, 62.7 µmol) (Intermediat L92) in 2.5 ml DMF vorgelegt. Die Reaktionsmischung wurde mit HATU (23.9 mg, 62.7 µmol) und N,N-Diisopropylethylamin (27 µl, 160 µmol) versetzt und 10 min bei RT nachgerührt. Der Ansatz wurde mit 1 ml Wasser (0.1 % TFA) versetzt und direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 54.6 mg (79 % d. Th.) der Verbindung Dibenzyl-(2S)-2-{[(5S,8S,11S)-11-(2-amino-2-oxoethyl)-17-{(1R)-1-[1-benzyl-4-(2,5-difluorphenyl)-1H-pyrrol-2-yl]-2,2-dimethylpropyl}-5,8-dimethyl-3,6,9,12,18,23,27-heptaoxo-1-phenyl-2-oxa-20-thia-4,7,10,13,17,24-hexaazaheptacosan-27-yl]amino}pentandioat.

LC-MS (Methode 1): Rₜ = 1.32 min; MS (ESIpos): m/z = 1328 [M+H]⁺

Dibenzyl-(2S)-2-{[(5S,8S,11S)-11-(2-amino-2-oxoethyl)-17-{(1R)-1-[1-benzyl-4-(2,5-difluorphenyl)-1H-pyrrol-2-yl]-2,2-dimethylpropyl}-5,8-dimethyl-3,6,9,12,18,23,27-heptaoxo-1-phenyl-2-oxa-20-thia-4,7,10,13,17,24-hexaazaheptacosan-27-yl]amino}pentandioat (53.3 mg, 40.1 µmol) wurde in 3.0 ml Ethyl Acetat und 3.0 ml Ethanol gelöst und mit 10% Palladium auf Aktivkohle (5.37 mg) versetzt. Die Reaktionsmischung wurde bei RT und Normaldruck über Nacht hydriert und dann, über einen Pappfilter filtriert. Der Filterkuchen wurde mit Ethyl Actetat und Ethanol nachgewaschen. Das Lösemittel wurden im Vakuum verdampft. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand wurde lyophilisiert. Man erhielt 5.6 mg (11 % d. Th.) der Verbindung L-Alanyl-L-alanyl-N¹-[(16S)-4-{(1R)-1-[1-benzyl-4-(2,5-difluorphenyl)-1H-pyrrol-2-yl]-2,2-dimethylpropyl}-16,18-dicarboxy-5,10,14-trioxo-7-thia-4,11,15-triazaoctadec-1-yl]-L-aspartamid -trifluoressigsäure (1:1).

LC-MS (Methode 1): Rₜ = 0.81 min; MS (ESIpos): m/z = 1014 [M+H]⁺

L-Alanyl-L-alanyl-N¹-[(16S)-4-{(1R)-1-[1-benzyl-4-(2,5-difluorphenyl)-1H-pyrrol-2-yl]-2,2-dimethylpropyl}-16,18-dicarboxy-5,10,14-trioxo-7-thia-4,11,15-triazaoctadec-1-yl]-L-aspartamid - trifluoressigsäure (1:1) (5.40 mg, 4.30 µmol) und 1-{6-[(2,5-Dioxopyrrolidin-1-yl)oxy]-6-oxohexyl}-1H-pyrrol-2,5-dion (1.46 mg, 4.73 µmol) wurden in 0.49 ml DMF vorgelegt. Die Reaktionsmischung wurde mit N,N-Diisopropylethylamin (2.2 µl, 13 µmol) versetzt und 4h30 bei RT nachgerührt. Der Ansatz wurde mit 0.5 ml Wasser (0,1%TFA) versetzt und direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 43.1 mg 4.10 mg (72 % d. Th.) der titel Verbindung.

LC-MS (Methode 1): Rₜ = 0.97 min; MS (ESIpos): m/z = 1207 [M+H]⁺

### Intermediat Q42

### Trifluoressigsäure N-[(benzyloxy)carbonyl]-L-alanyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid (1:1)

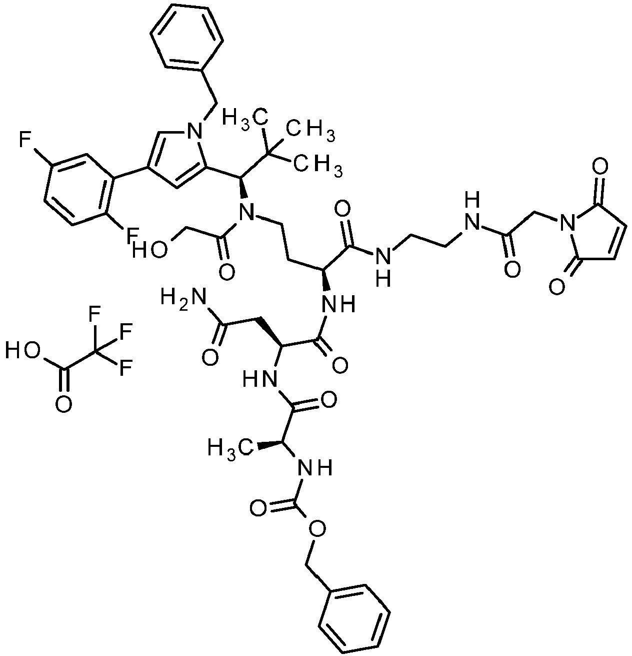

Trifluoressigsäure N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid (1:1) (10.0 mg, 10.9 µmol) (Intermediat C116) und N-[(Benzyloxy)carbonyl]-L-alanin (2.42 mg, 10.9 µmol) wurden in 1.0 ml DMF vorgelegt. Die Reaktionsmischung wurde mit HATU (4.95 mg, 13.0 µmol) und N,N-Diisopropylethylamin (9.5 µl, 54 µmol) versetzt und 10 min bei RT nachgerührt. Der Ansatz wurde mit 1.0 ml Wasser (0,1%TFA) versetzt und direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand wurde lyophilisiert. Man erhielt 5.6 mg (46 % d. Th.) der titel Verbindung.

LC-MS (Methode 1): Rₜ = 1.11 min; MS (ESIpos): m/z = 1012 [M+H]⁺

### Intermediat Q43

### N-[(Benzyloxy)carbonyl]-L-alpha-asparagyl-N¹-{(2S)-4-[{(1R)-1-[1-benzyl-4-(2,5-difluorphenyl)-1H-pyrrol-2-yl]-2,2-dimethylpropyl } (glycoloyl)amino]-1-[(2-{ [(2,5-dioxo-2,5-dihydro-1H-pyrrol-1-yl)acetyl]amino}ethyl)amino]-1-oxobutan-2-yl}-L-aspartamid -trifluoressigsäure (1:1)

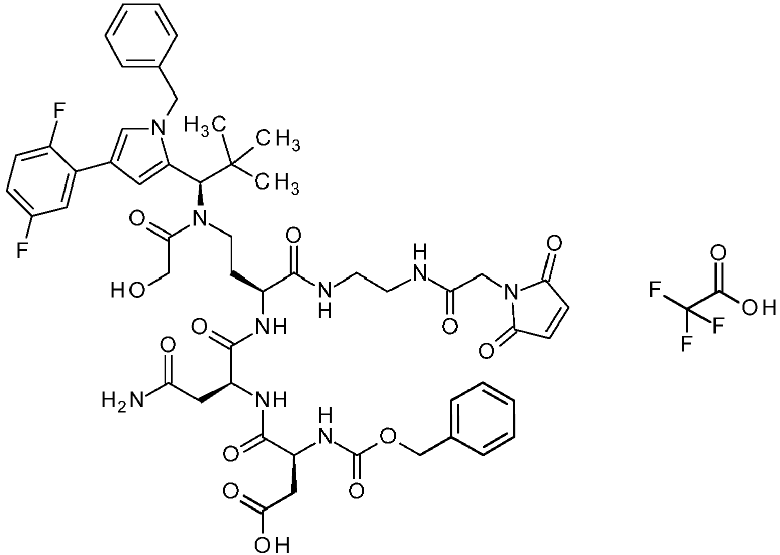

10 mg (0.011 mmol) von Intermediat C116 wurden in 4 mL DMF aufgenommen und mit 3.9 mg (0.012 mmol) (2S)-2-{[(benzyloxy)carbonyl]amino}-4-tert-butoxy-4-oxobutansäure sowie mit 6.2 mg (0.016 mmol) HATU und 6µL N,N-Diisopropylethylamin versetzt. Nach 15 min Rühren bei RT wurde der Ansatz eingeengt und der Rückstand durch präparative HPLC gereinigt. Die entsprechenden Fraktionen wurden eingeengt und der Rückstand aus Acetonitril/Wasser lyophilisiert. Es wurden 8.2 mg (68% d. Th.) des geschützten Intermediats erhalten, das anschließend mit 6 mg (0.044 mmol) Zinkchlorid in 4 ml Trifluorethanol bei 50°C entschützt wurde. Nach Zugabe von 13 mg (0.044 mmol) EDTA und anschließender HPLC-Reinigung wurden 3 mg (35% d. Th.) der Titelverbindung erhalten.

LC-MS (Methode 1): Rₜ = 1.05 min; MS (ESIpos): m/z = 1056 (M+H)⁺.

### Intermediat Q44

### N-[(Benzyloxy)carbonyl]-L-alanyl-L-alpha-asparagyl-N¹-{(2S)-4-[{(1R)-1-[1-benzyl-4-(2,5-difluorphenyl)-1H-pyrrol-2-yl]-2,2-dimethylpropyl}(glycoloyl)amino]-1-[(2-{[(2,5-dioxo-2,5-dihydro-1H-pyrrol-1-yl)acetyl]amino}ethyl)amino]-1-oxobutan-2-yl}-L-aspartamid-trifluoressigsäure (1:1)

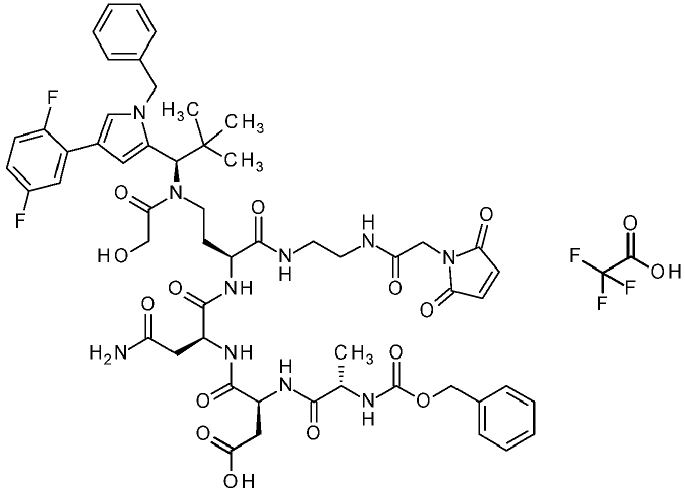

10 mg (0.011 mmol) von Intermediat C116 wurden in 4 mL DMF aufgenommen und mit 5.3 mg (0.013 mmol) (2S)-2-{[(2S)-2-{[(benzyloxy)carbonyl]amino}propanoyl]amino}-4-tert-butoxy-4-oxobutansäure sowie mit 6.2 mg (0.016 mmol) HATU und 6µL N,N-Diisopropylethylamin versetzt. Nach 15 min Rühren bei RT wurde der Ansatz eingeengt und der Rückstand durch präparative HPLC gereinigt. Die entsprechenden Fraktionen wurden eingeengt und der Rückstand aus Acetonitril/Wasser lyophilisiert. Es wurden 8 mg (57% d. Th.) des geschützten Intermediats erhalten, das anschließend mit 5 mg (0.037 mmol) Zinkchlorid in 2 ml Trifluorethanol bei 50°C entschützt wurde. Nach Zugabe von 11 mg (0.037 mmol) EDTA und anschließender HPLC-Reinigung wurden 3.5 mg (46% d. Th.) der Titelverbindung erhalten.

LC-MS (Methode 1): Rₜ = 1.04 min; MS (ESIpos): m/z = 1127 (M+H)⁺.

### B: Herstellung von Antikörper-Wirkstoff-Konjugaten (ADC)

### B-1. Allgemeines Verfahren zur Generierung von Antikörpern

Eine vollständig humane Antikörper-Phagen-Bibliothek (Hoet RM et al, Nat Biotechnol 2005;23(3):344-8) wurde verwendet, um TWEAKR-spezifische, humane, monoklonale Antikörper durch Protein-Panning (Hoogenboom H.R., Nat Biotechnol 2005;23(3):1105-16) mit dimeren, Fcfusionierten extrazellulären Domänen von humanem und Maus-TWEAKR als immobilisiertes Target zu isolieren. Fab-Phagen wurden identifiziert, und die entsprechenden Antikörper wurden ins humane IgG1 Format reformatiert. Der aglykosylierte Antiköper TPP-2658 wurde durch Einführung der Mutation N297A in der schweren Kette des TPP-2090 (Kabat numbering system of immunoglobulins) generiert. Der so gewonnene Antikörper wurden für die hier beschriebenen Ausführungsbeispiele verwendet. Darüber hinaus sind dem Fachmann Antikörper, die an TWEAKR binden bekannt, siehe z.B. WO2009/020933(A2) oder WO2009140177 (A2)

Die kommerziell erhältlichen Antiköper Cetuximab (Handelsname: Erbitux), Trastuzumab (Handelsame: Herceptin; INN 7637, CAS NR: RN: 180288-69-1) und Nimotuzumab (Handelsname: CIMAher) wurden für die hier beschriebenen Ausführungsbeispiele verwendet.

Für Trastuzumab-HC-N297A (entspricht TPP-7510) wurde eine schwere Kette mit der SEQ ID NO: 244 verwendet. Die leichte Kette ist identisch mit der leichten Kette aus Trastuzumab.

Für Trastuzumab-HC-N297Q (entspricht TPP-7511) wurde eine schwere Kette mit der SEQ ID NO: 245 verwendet. Die leichte Kette ist identisch mit der leichten Kette aus Trastuzumab.

### B-2. Allgemeines Verfahren zur Expression von Antikörpern in Säugerzellen

Die Antikörper, zum Beispiel TPP-2658, TPP-7510 oder TPP-7511, wurden in transienten Säugerzellkulturen produziert, wie von Tom et al., Kapitel 12 in Methods Express: Expression Systems herausgegeben von Micheal R. Dyson and Yves Durocher, Scion Publishing Ltd, 2007 beschrieben.

### B-3. Allgemeines Verfahren zur Aufreinigung von Antikörpern aus Zellüberständen.

Die Antikörper, zum Beispiel TPP-2658, TPP-7510 oder TPP-7511, wurden aus den Zellkulturüberständen gewonnen. Die Zellüberstände wurden durch Zentrifugation von Zellen geklärt. Anschließend wurde der Zellüberstand durch Affinitätschromatographie auf einer MabSelect Sure (GE Healthcare) Chromatographiesäule gereinigt. Dazu wurde die Säule in DPBS pH 7.4 (Sigma! Aldrich) equillibriert, der Zellüberstand aufgetragen und die Säule mit ca 10 Säulenvolumina DPBS pH 7.4 + 500 mM Natriumchlorid gewaschen. Die Antikörper wurden in 50 mM Natriumacetat pH 3.5 + 500 mM Natriumchlorid eluiert und anschliessend durch Gelfiltrationschromatographie auf einer Superdex 200 Säule (GE Healthcare) in DPBS pH 7.4 weiter gereinigt.

Die kommerziell erhältlichen Antikörper wurden mit Standard- Chromatographiemethoden aus den Handelsprodukten aufgereinigt (Protein A Chromatopgraphie, präparative Gelfiltrationschomatographie (SEC - size exclusion chromatographie)).

### B-4. Allgemeines Verfahren zur Kupplung an Cystein-Seitenketten

In die Kupplungsreaktionen wurde folgender Antikörper eingesetzt:

| | |
|---|---|
| Beispiele a: | Cetuximab (Anti EGFR AK) |
| Beispiele e: | Trastuzumab (Anti-Her2 AK) |
| Beispiele i: | Nimotuzumab (Anti-EGFR AK) |
| Beispiele k: | TPP-2658 (Anti-TWEAKR AK) |

Die Kupplungsreaktionen wurden üblicherweise unter Argon durchgeführt.

Zu einer Lösung des entsprechenden Antikörpers in PBS-Puffer im Konzentrationsbereich zwischen 1 mg/mL und 20 mg/mL, bevorzugt im Bereich von ungefähr 10mg/ml bis 15 mg/ml wurden zwischen 2 und 5 Äquivalenten Tris(2-carboxyethyl)phosphin-Hydrochlorid (TCEP), gelöst in PBS-Puffer, gegeben und 1h bei RT gerührt. Dazu kann die Lösung des jeweils eingesetzten Antikörpers in der in den Ausführungsbeispielen angegebenen Konzentration eingesetzt werden oder gegebenenfalls auch mit PBS Puffer bis etwa auf die Hälfte der angegebenen Startkonzentration verdünnt werden, um in den bevorzugten Konzentrationsbereich zu kommen. Anschließend wurden, je nach angestrebter Beladung, zwischen 2 und 12 Äquivalenten, bevorzugt etwa 5-10 Äquivalente der zu kuppelnden Maleinimid-Vorläufer-Verbindung oder Halogenid-Vorläufer-Verbindung als Lösung in DMSO hinzugefügt. Dabei sollte die Menge an DMSO 10% des Gesamtvolumens nicht überschreiten. Der Ansatz wurde bei Maleinimid-Precursern 60-240 min bei RT und bei Halogenid-Precursern zwischen 8 und 24 h bei RT gerührt und anschließend über in PBS equillibierte PD 10-Säulen (Sephadex^{®} G-25, GE Healthcare) gegeben und mit PBS-Puffer eluiert. Üblicherweise wurden, wenn nicht anders angegeben, 5 mg des entsprechenden Antikörpers in PBS-Puffer zur Reduktion und der nachfolgenden Kupplung eingesetzt. Nach Aufreinigung über die PD10 Säule wurden so jeweils Lösungen des entsprechenden ADCs in 3.5 mL PBS-Puffer erhalten. Anschließend wurde eine Aufkonzentration mittels Ultrazentrifugation durchgeführt und die Probe ggf. mit PBS-Puffer zurückverdünnt. Falls notwendig wurde zur besseren Abtrennung niedermolekularer Bestandteile die Aufkonzentration mittels Ultrafiltration nach Rückverdünnung mit PBS Puffer wiederholt. Für biologische Testungen wurden je nach Bedarf in den finalen ADC Proben ggf. durch Rückverdünnung Konzentrationen im Bereich von 0.5-15 mg/mL eingestellt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

Die in den Beispielen dargestellten ADCs können in Abhängigkeit vom Linker gegebenenfalls auch mehr oder weniger stark ausgeprägt in Form der hydrolysierten offenkettigen Bernsteinsäureamide mit den Antikörpern verknüpft vorliegen.

### Insbesondere die KSP-I-ADCs, die über die Linker-Substruktur

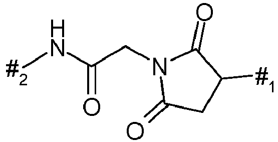

mit Thiolgruppen der Antikörper verknüpft sind, können auch ggf. durch Umpuffern im Anschluss an die Kupplung und ca. 20-24-stündiges Rühren bei pH 8 gemäß Schema 28 gezielt in die die über offenkettige Bersteinsäureamide verknüpften ADCs hergestellt werden.

#1 stellt die Schwefelbrücke zum Antikörper dar und #2 die Verknüpfungsstelle zum modifizierten KSP-Inhibitor

Solche ADCs, bei denen der Linker über hydrolysierte offenkettige Bernsteinsäureamide mit den Antikörpern verknüpft vorliegt, können gegebenenfalls auch gezielt nach einer exemplarischen Vorschrift wie folgt dargestellt werden:

### Small Scale Kupplung:

Zu einer Lösung von 2-5 mg des entsprechenden Antikörpers in PBS-Puffer im Konzentrationsbereich zwischen 1 mg/mL und 20 mg/mL, bevorzugt im Bereich von ungefähr 5 mg/ml bis 15 mg/ml wurden zwischen 2 und 5 Äquivalenten Tris(2-carboxyethyl)phosphin-Hydrochlorid (TCEP), gelöst in PBS-Puffer, gegeben und 30 min bis 1h bei RT gerührt. Dazu kann die Lösung des jeweils eingesetzten Antikörpers in der in den Ausführungsbeispielen angegebenen Konzentration eingesetzt werden oder gegebenenfalls auch mit PBS Puffer bis etwa auf die Hälfte der angegebenen Startkonzentration verdünnt werden, um in den bevorzugten Konzentrationsbereich zu kommen. Anschließend wurden, je nach angestrebter Beladung, zwischen 2 und 12 Äquivalenten, bevorzugt etwa 5-10 Äquivalente der zu kuppelnden Maleinimid-Vorläufer-Verbindung als Lösung in DMSO hinzugefügt. Dabei sollte die Menge an DMSO 10% des Gesamtvolumens nicht überschreiten. Der Ansatz wurde 60-240 min bei RT gerührt und anschließend mit PBS-Puffer, der zuvor auf pH 8 eingestellt wurde, auf ein Volumen von 3-7 ml verdünnt und über Nacht bei RT unter Argon gerührt. Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert. Anschließend wurde durch Ultrazentrifugation aufkonzentriert und rückverdünnt mit PBS-Puffer (pH 7.2).

### Medium scale Kupplung:

60 mg des betreffenden Antikörpers in 5 ml PBS-Puffer (c~12 mg/mL) wurden unter Argon mit einer Lösung aus 0,344mg TCEP in 100µl PBS-Puffer versetzt. Der Ansatz wurde 30min bei RT gerührt und anschließend wurden 0.003 mmol einer Maleinimid-Vorläuferverbindung gelöst in 600µl DMSO zugegeben. Nach weiteren 1.5h-2h Rühren bei RT wurde der Ansatz mit 1075µl PBS-Puffer, der zuvor auf pH 8 eingestellt wurde, verdünnt.

Diese Lösung wurde dann über mit PBS-Puffer pH8 equillibrierte PD 10-Säulen (Sephadex^{®} G-25, GE Healthcare) gegeben und mit PBS-Puffer pH8 eluiert. Das Eluat wurde mit PBS-Puffer pH8 auf ein Gesamtvolumen von 14 ml verdünnt. Diese Lösung wurde über Nacht bei RT unter Argon gerührt. Dann erfolgte ggf. wieder ein Umpuffern auf pH 7.2. Die ADC-Lösung wurde durch Ultrazentrifugation aufkonzentriert, rückverdünnt mit PBS-Puffer (pH7.2) und dann ggf. erneut bis auf eine Konzentration von etwa 10mg/mL aufkonzentriert.

Weitere potentiell hydrolyse-sensitive Thianylsuccinimid-Brücken zum Antikörper in den Ausführungsbeispielen enthalten folgende Linker-Substrukturen, wobei #1 die Thioetherverknüpfung zum Antikörper und #1 die Verknüpfungsstelle zum modifizierten KSP-Inhibitor darstellt:

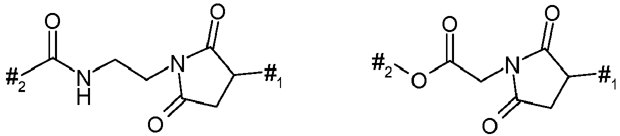

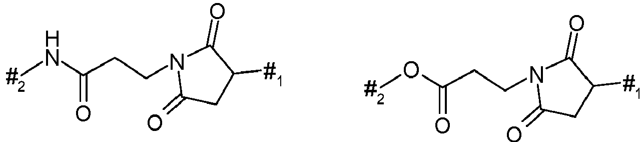

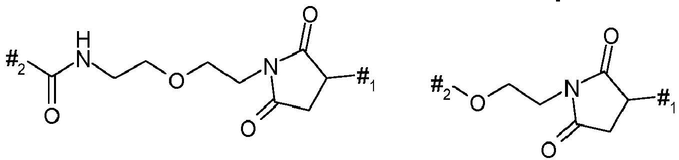

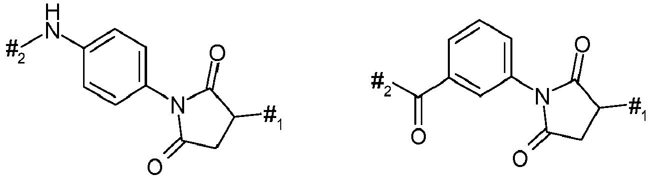

Diese Linker-Substrukturen stellen die Verknüpfungseinheit zum Antikörper dar und haben (neben der weiteren Linkerzusammensetzung) einen signifikanten Einfluß auf die Struktur und das Profil der in Tumorzellen gebildeten Metabolite.

In den dargestellten Strukturformeln kann dabei AK₁ die Bedeutung haben

| | |
|---|---|
| Beispiele a: | Cetuximab (partiell reduziert)- S§¹ |
| Beispiele e: | Trastuzumab (partiell reduziert)- S§¹ |
| Beispiele i: | Nimotuzumab (partiell reduziert)- S§¹ |
| Beispiele k: | Anti-TWEAKR Antikörper TPP-2658 (partiell reduziert)- S§¹ |

wobei
- §¹: die Verknüpfung mit der Succinimid-Gruppe oder mit ggf. daraus entstandenen isomeren hydrolysierten offenkettigen Bernsteinsäureamiden bzw. des Alkylenrestes bedeutet,
und
- S: für das Schwefelatom eines Cystein-Restes des partiell reduzierten Antikörpers steht.

### B-5. Allgemeines Verfahren zur Kupplung an Lysin-Seitenketten

In die Kupplungsreaktionen wurden folgende Antikörper eingesetzt:

| | |
|---|---|
| Beispiele a: | Cetuximab (Anti EGFR AK) |
| Beispiele e: | Trastuzumab (Anti-Her2 AK) |
| Beispiele i: | Nimotuzumab (Anti-EGFR AK) |
| Beispiele k: | TPP-2658 (Anti-TWEAKR Antikörper) |

Die Kupplungsreaktionen wurden üblicherweise unter Argon durchgeführt.

Zu einer Lösung des entsprechenden Antikörpers in PBS-Puffer im Konzentrationsbereich zwischen 1 mg/mL und 20 mg/mL, bevorzugt etwa 10 mg/mL, wurden, je nach angestrebter Beladung, zwischen 2 und 8 Äquivalente der zu kuppelnden Vorläufer-Verbindung als Lösung in DMSO gegeben. Nach 30 min bis 6 h Rühren bei RT wurde nochmals die gleiche Menge an Vorläufer-Verbindung in DMSO hinzugefügt. Dabei sollte die Menge an DMSO 10% des Gesamtvolumens nicht überschreiten. Nach weiteren 30 min bis 6 h Rühren bei RT wurde der Ansatz über in PBS equillibierte PD 10-Säulen (Sephadex^{®} G-25, GE Healthcare) gegeben und mit PBS-Puffer eluiert. Üblicherweise wurden, wenn nicht anders angegeben, 5 mg des entsprechenden Antikörpers in PBS-Puffer zur Kupplung eingesetzt. Nach Aufreinigung über die PD10 Säule wurden so jeweils Lösungen des entsprechenden ADCs in 3.5 mL PBS-Puffer erhalten. Anschließend wurde eine Aufkonzentration mittels Ultrazentrifugation durchgeführt und die Probe ggf. mit PBS-Puffer zurückverdünnt. Falls notwendig wurde zur besseren Abtrennung niedermolekularer Bestandteile die Aufkonzentration mittels Ultrafiltration nach Rückverdünnung mit PBS Puffer wiederholt. Für biologische Testungen wurden je nach Bedarf in den finalen ADC Proben ggf. durch Rückverdünnung Konzentrationen im Bereich von 0.5-15 mg/mL eingestellt.

Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

In den dargestellten Strukturformeln hat dabei AK₂ die Bedeutung

| | |
|---|---|
| Beispiele a: | Cetuximab - NH§² |
| Beispiele e: | Trastuzumab - NH§² |
| Beispiele i: | Nimotuzumab - NH§² |
| Beispiele k: | Anti-TWEAKR Antikörper TPP-2658 - NH-§² |

wobei
- §²: die Verknüpfung mit der Carbonylgruppe bedeutet,
und
- NH: für die Seitenketten-Aminogruppe eines Lysin-Restes des Antikörpers steht.

### B-5a. Allgemeines Verfahren zur ADC Synthese mittels bakterieller Transglutaminase

In die Kupplungsreaktionen in der Beispielserie t wurden folgende Antikörper eingesetzt (die nachfolgende Nomenklatur Antikörper-HC-N297Z meint den Antikörper, bei dem an beiden schweren Ketten die Aminosäure N297 (Kabat-Nummerierung) durch die Aminosäure Z ausgetauscht wurde, die Nomenklatur TPP-XXXX-HC-Q295N-HC-N297Q meint den Antikörper mit der TPP-XXXX, bei dem an beiden schweren Ketten die Aminosäure Q295 (Kabat-Nummerierung) durch die Aminosäure N und die Aminosäure N297 (Kabat-Nummerierung) durch die Aminosäure Q ausgetauscht wurde. Der Antikörpername des Ursprungsantikörpers kann dabei entweder als Name (Beispiel Trastuzumab) oder als TPP-XXXX angegeben sein (Antikörper mit der TPP-Nummer XXXX)):

| | |
|---|---|
| AK₃ₐ: | Anti-TWEAKR Antikörper TPP-2658 (entspricht TPP-2090-HC-N297A) |
| AK_{3b}: | Anti-TWEAKR Antikörper TPP-5442 (entspricht TPP-2090-HC-N297Q) |
| AK_{3c}: | Anti-TWEAKR Antikörper TPP-8825 (entspricht TPP-2090-HC-Q295N-HC-N297Q) |
| AK_{3d}: | TPP-7510 (entspricht Trastuzumab-HC-N297A) |
| AK₃ₑ: | TPP-7511 (entspricht Trastuzumab-HC-N297Q) |

### Allgemeine Vorschrift um eine maximale DAR von 2 zu erzielen:

Zu einer Lösung von 5 mg der entsprechenden aglyco Antikörper Variante (HC-N297A, oder HC-Q295N-HC-N297Q) in DPBS pH 7.4 (c~5-15 mg/mL) wurden 20 µL (6 Equivalente) einer Lösung eines geeigneten Toxophor-Linker-Precursors (e.g. Intermediate Q31-Q34; 10 mM Lösung in DMSO) hinzugegeben. Nach 5 min Inkubation bei 37°C wurden 50 µL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Gesamtvolumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.00625 µmol des b-Transglutaminase Blockers Zedira C100 in 12.5 µL DPBS zu der Lösung hinzugefügt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

### Allgemeine Vorschrift um eine maximale DAR von 4 zu erzielen:

Zu einer Lösung von 5 mg der entsprechenden aglyco Antikörper Variante (HC-N297Q) in DPBS pH 7.4 (c~5-15mg/mL) wurden 16-24 Euivalente einer Lösung eines geeigneten Toxophor-Linker-Precursors (e.g. Intermediat Q31-Q34; 10 mM Lösung in DMSO) hinzugegeben. Nach 5 min Inkubation bei 37°C wurden 400 µL (10 U) einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Gesamtvolumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.1 µmol des b-Transglutaminase Blockers Zedira C100 in 200 µL DPBS zu der Lösung hinzugefügt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

### Allgemeine Vorschrift für Transglutaminase vermittelte Kupplung im größeren Maßstab um eine maximale DAR von 2 zu erhalten:

Zu einer Lösung von 30 mg der aglycosylierten Variante (HC-N297A, HC-Q295N-HC-N297Q) des jeweiligen Antikörpers in DPBS pH 7.4 (c~5-15 mg/mL) wurden 6 Equivalente einer Lösung des entsprechenden Toxophor-Linker-Precursors (10 mM in DMSO) hinzugegeben. Nach 5 min Inkubation bei 37°C wurden 200 µL (7.5 U) einer Lösung von rekombinanter bakterieller Transglutaminase in Wasser (Produkt Nummer T001 von Zedira GmbH, Darmstadt, Germany) (25 U/mL) hinzugegeben und weitere 24h bei 37°C inkubiert. Die Reaktionsmischung wurde über Gelfiltrationschromatographie auf einer Superdex 200 Säule (GE Healthcare) in DPBS pH 7.4 gereinigt, um kleine Moleküle und die Transglutaminase vom ADC abzutrennen. Anschließend wurde die ADC-Lösung über Amicon Ultracel-30K Zentrifugationsröhrchen (Millipore) auf finale Konzentrationen von 5-25 mg/mL ankonzentriert. Die Lösung wurde anschließend sterilfiltriert.

Die jeweiligen in den Ausführungsbeispielen angegebenen Konzentrationen der ADC-Lösungen wurden bestimmt. Die Beladung wurde nach den in Kapitel B7 beschriebenen Methoden bestimmt. Die ADC batches wurden wie in den Ausführungsbeispielen angezeigt charakterisiert.

### Allgemeine Vorschrift für Transglutyminase vermittelte Kupplung im größeren Maßstab um eine maximale DAR von 4 zu erhalten:

Zu einer Lösung von 30 mg der aglycosylierten Variante (HC-N297Q) des jeweiligen Antikörpers in DPBS pH 7.4 (c~5-15 mg/mL) wurden 16-24 Equivalente einer Lösung des entsprechenden Toxophor-Linker-Precursors (10 mM in DMSO) hinzugegeben. Nach 5 min Inkubation bei 37°C wurden 2400 µL (60 U) einer Lösung von rekombinanter bakterieller Transglutaminase in Wasser (Produkt Nummer T001 von Zedira GmbH, Darmstadt, Germany) (25 U/mL) hinzugegeben und weitere 24h bei 37°C inkubiert. Die Reaktionsmischung wurde über Gelfiltrationschromatographie auf einer Superdex 200 Säule (GE Healthcare) in DPBS pH 7.4 gereinigt, um kleine Moleküle und die Transglutaminase vom ADC abzutrennen. Anschließend wurde die ADC-Lösung über Amicon Ultracel-30K Zentrifugationsröhrchen (Millipore) auf finale Konzentrationen von 5-25 mg/mL ankonzentriert. Die Lösung wurde anschleießend sterilfiltriert.

Die jeweiligen in den Ausführungsbeispielen angegebenen Konzentrationen der ADC-Lösungen wurden bestimmt. Die Beladung wurde nach den in Kapitel B7 beschriebenen Methoden bestimmt. Die ADC batches wurden wie in den Ausführungsbeispielen angezeigt charakterisiert.

In den dargestellten Strukturformeln der Beispielserie t hat AK₃ jeweils die Bedeutung

| | |
|---|---|
| AK₃ₐ: | Anti-TWEAKR Antikörper (TPP-2658) (entspricht TPP-2090-HC-N297A) - CO-§₂ |
| AK_{3b}: | Anti-TWEAKR Antikörper (TPP-5442) (entspricht TPP-2090-HC-N297Q) - CO-§₂ |
| AK_{3c}: | Anti-TWEAKR Antikörper (TPP-8825) (entspricht TPP-2090-HC-Q295N-HC-N297Q) -CO-§₂ |
| AK_{3d}: | TPP-7510 (entspricht Trastuzumab-HC-N297A) - CO-§₂ |
| AK₃ₑ: | TPP-7511 (entspricht Trastuzumab-HC-N297Q) - CO-§₂ |

wobei
- §²: die Verknüpfung mit der Aminogruppe eines Toxophor-Linker-Precursors bedeutet,
und
- CO: für die Seitenketten-Carbonylgruppe eines Glutamin-Restes des Antikörpers steht.

### B-6a. Allgemeines Verfahren zur Herstellung von geschlossenen Succinimid-Cystein-Addukten:

In einer beispielhaften Ausführung wurden 10 µmol der oben beschriebenen Maleinimid-Vorläuferverbindungen wurden in 3-5 mL DMF aufgenommen und mit 2.1 mg (20 µmol) L-Cystein versetzt. Das Reaktionsgemisch wurde zwischen 2h und 24 h bei RT gerührt, anschließend im Vakuum eingeengt und dann mittels präparativer HPLC gereinigt.

### B-6aa. Allgemeines Verfahren zur Herstellung von isomeren geöffneten Bernsteinsäureamid-Cystein-Addukten:

In einer beispielhaften Ausführung wurden 68 µmol der oben beschriebenen Maleinimid-Vorläuferverbindungen in 15 mL DMF aufgenommen und mit 36 mg (136 µmol) N-{[2-(Trimethylsilyl)ethoxy]carbonyl}-L-cystein versetzt. Das Reaktionsgemisch wurde ~20h bei RT gerührt, anschließend im Vakuum eingeengt und dann mittels präparativer HPLC gereinigt. Nach Vereinigung der entsprechenden Fraktionen und Abdampfen der Lösemittel im Vakuum wurde der Rückstand in 15 mL THF/Wasser 1:1 gelöst. Es wurden 131 µL einer 2M wässrigen Lithiumhydroxidlösung zugegeben und der Ansatz 1 h bei RT gerührt. Anschließend wurde der Ansatz mit einer 1M Salzsäure neutral gestellt, das Lösemittel im Vakuum abgedampft und der Rückstand durch präparative HPLC gereinigt. Es wurden -50% d. Th. der regioisomeren geschützten Intermediate als farbloser Schaum erhalten.

Im letzten Schritt wurden 0.023 mmol von diesen regiosisomeren Hydrolyseprodukten in 3 mL 2,2,2-Trifluorethanol gelöst. Es wurden 12.5 mg (0.092 mmol) Zinkchlorid zugegeben und der Ansatz 4 h bei 50°C gerührt. Anschließend wurden 27 mg (0.092 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden die hydrolysierten offenen Sulfanylbernsteinsäureamide als Regioisomerengemisch erhalten.

### Weitere Aufreinigung und Charakterisierung der erfindungsgemäßen Konjugate

Nach erfolgter Umsetzung wurde in einigen Fällen das Reaktionsgemisch beispielsweise durch Ultrafiltration aufkonzentriert und anschließend mittels Chromatographie, beispielsweise mittels einer Sephadex^{®} G-25, entsalzt und gereinigt. Die Elution erfolgte beispielsweise mit Phosphatgepufferter Salzlösung (PBS). Anschließend wurde die Lösung sterilfiltriert und eingefroren. Alternativ kann das Konjugat lyophylisiert werden.

### B-7. Bestimmung des Antikörpers, der Toxophorbeladung und des Anteils geöffneter Cystein-Addukte

Zur Protein-Identifizierung wurde neben der Molekulargewichtsbestimmung nach Deglykosilierung und/oder Denaturierung ein tryptischer Verdau durchgeführt, der nach Denaturierung, Reduktion und Derivatisierung die Identität des Proteins anhand der nachgewiesenen tryptischen Peptide bestätigt.

Von den erhaltenen Lösungen der in den Ausführungsbeispielen beschriebenen Konjugate in PBS Puffer wurde die Toxophorbeladung wie folgt bestimmt:
Die Bestimmung der Toxophor Beladung von Lysin-verknüpften ADCs erfolgte nach massenspektrometrischer Bestimmung der Molekulargewichte der einzelnen Konjugatspezies. Hierbei wurden vorab die Antikörperkonjugate mittels PNGaseF deglycosyliert, die Probe angesäuert und nach HPLC-Trennung/Entsalzung massenspektrometrisch mittels ESI-MicroTof_{Q} (Bruker Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution kalkuliert. Nach Signal Integration der verschiedenen Spezies wurde dann die DAR (= Drug/Antibody Ratio) berechnet.

Die Bestimmung der Toxophorbeladung von Cystein-verknüpften Konjugaten wurde über Reversed-Phase-Chromatographie des reduzierten und denaturierten ADCs bestimmt. Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymeric Reversed Phase Säule (Katalognummer PL1912-3802) (2.1 x150 mm, 8 µm particle size, 1000 Ä) bei einer Flussrate von 1 mL/min mit folgendem Gradienten verwendet: 0 min, 25 %B; 3 min, 25 %B; 28 min, 50 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden der leichten Kette mit einem Toxophor (L1) und den schweren Ketten mit einem, zwei und drei Toxophoren (H1, H2, H3) zugeordnet.

Die durchschnittliche Beladung des Antikörpers mit Toxophoren wurde aus den durch Integration bestimmten Peakflächen als die zweifache Summe der HC-Beladung und der LC-Beladung bestimmt, wobei sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks berechnet, und wobei sich die HC-load aus der Summe der Toxophor-Anzahl gewichteten Integrationsergebnisse aller HC-Peaks geteilt durch die Summe der einfach gewichteten Integrationsergebnisse aller HC- Peaks berechnet In vereinzelten Fällen kann es vorkommen, dass die Toxophorbeladung aufgrund von Ko-Elutionen einiger Peaks nicht exakt möglich ist.

In den Fällen, in denen keine ausreichende HPLC-Trennung von leichter und schwerer Kette möglich war, erfolgte die Bestimmung der Toxophorbeladung von Cystein verknüpften Konjugaten mittels massenspektrometrischer Bestimmung der Molekulargewichte der einzelnen Konjugatspezies an leichter und schwerer Kette.

Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und massenspektrometrisch nach online Entsaltzung mittels ESI-MicroTof_{Q} (Bruker Daltonik) analysiert.

Zur DAR-Bestimmung wurden alle Spektren über das Signal im TIC (Total Ion Chromatogramm) addiert und das Molekulargewicht der verschiedenen Konjugatspezies an leichter und schwerer Kette auf Basis von MaxEnt Deconvolution berechnet. Die durchschnittliche Beladung des Antikörpers mit Toxophoren wurde aus den durch Integration bestimmten Peakflächen als die zweifache Summe der HC-Beladung und der LC-Beladung bestimmt, wobei sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks berechnet, und wobei sich die HC-Beladung aus der Summe der Toxophor-Anzahl gewichteten Integrationsergebnisse aller HC-Peaks geteilt durch die Summe der einfach gewichteten Integrationsergebnisse aller HC- Peaks berechnet.

Zur Bestimmung des Anteils des geöffneten Cystein-Addukts wurde das Molekulargewichtsflächenverhältnis vom geschlossenen und offenen Cystein-Addukt (Molekulargewichtsdelta 18Dalton) aller einfach konjugierten leichten und schweren Kettenvarianten bestimmt. Der Mittelwert über alle Varianten ergab den Anteil des geöffneten Cystein-Addukts.

Die Bestimmung der Toxophorbeladung von Glutamin-verknüpften Konjugaten wurde über Reversed-Phase-Chromatographie des reduzierten und denaturierten ADCs bestimmt. Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymeric Reversed Phase Säule (Katalognummer PL1912-3802) (2.1 x150 mm, 8 µm particle size, 1000 Ä) bei einer Flussrate von 1 mL/min mit folgendem Gradienten verwendet: 0 min, 31 %B; 1 min, 31 %B; 14 min, 38 %B, 16 min, 95 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden der leichten Kette mit einem Toxophor (L1) und den schweren Ketten mit einem und zwei Toxophoren (H1, H2) zugeordnet.

Die durchschnittliche Beladung des Antikörpers mit Toxophoren wurde aus den durch Integration bestimmten Peakflächen als die zweifache Summe der HC-Beladung und der LC-Beladung bestimmt, wobei sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks berechnet, und wobei sich die HC-load aus der Summe der Toxophor-Anzahl gewichteten Integrationsergebnisse aller HC-Peaks geteilt durch die Summe der einfach gewichteten Integrationsergebnisse aller HC- Peaks berechnet.

Alternativ erfolgte die Bestimmung der Toxophor Beladung von Glutamin-verknüpften ADCs nach massenspektrometrischer Bestimmung der Molekulargewichte der einzelnen Konjugatspezies. Hierbei wurden vorab die Antikörperkonjugate mittels PNGaseF deglycosyliert, die Probe angesäuert und nach HPLC-Trennung/Entsalzung massenspektrometrisch mittels ESI-MicroTof_{Q} (Bruker Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution kalkuliert. Nach Signal Integration der verschiedenen Spezies wurde dann die DAR (= Drug/Antibody Ratio) berechnet.

### B-8. Überprüfung der Antigen-Bindung des ADCs

Die Bindefähigkeit des Binders an das Zielmolekül wurde nach erfolgter Kopplung überprüft. Dazu sind dem Fachmann vielfältige Methoden bekannt, beispielsweise kann die Affinität des Konjugats mittels ELISA-Technologie oder Oberflächenplasmonresonanzanalyse (BIAcore^{™} Messungen) überprüft werden. Die Konjugatkonzentration kann der Fachmann mit gängigen Methoden messen, beispielsweise für Antikörper-Konjugate mittels Proteinbestimmung. (siehe auch Doronina et al.; Nature Biotechnol. 2003; 21:778-784 und Polson et al., Blood 2007; 1102:616-623).

### Ausführungsbeispiele Metabolite

### Beispiel M1

### S-[1-(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl]amino } -2-oxoethyl)-2,5-dioxopyrrolidin-3-yl]-L-cystein -trifluoressigsäure (1:1)

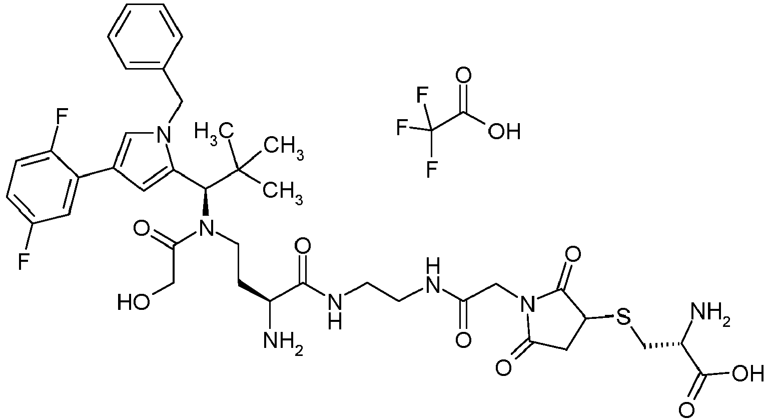

1.8 mg (2 µmol) von Intermediat F104 wurden in 1 ml DMF aufgenommen und mit 2.7 mg (22 µmol) L-Cystein versetzt. Das Reaktionsgemisch wurde 20 h bei RT gerührt, anschließend im Vakuum eingeengt und dann mittels präparativer HPLC gereinigt. Es verblieben 0.6 mg (26% d. Th.) der Titelverbindung als farbloser Schaum.

LC-MS (Methode 1): Rₜ = 0.80 min; MS (EIpos): m/z = 814 [M+H]⁺.

### Beispiel M2

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl] amino } -2-oxoethyl)amino] -3 - { [(2R)-2-amino-2-carboxyethyl]sulfanyl}-4-oxobutansäure -trifluoressigsäure (1:1) und 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl] amino } -2-oxoethyl)amino] -2- { [(2R)-2-amino-2-carboxyethyl]sulfanyl}-4-oxobutansäure -trifluoressigsäure (1:1)

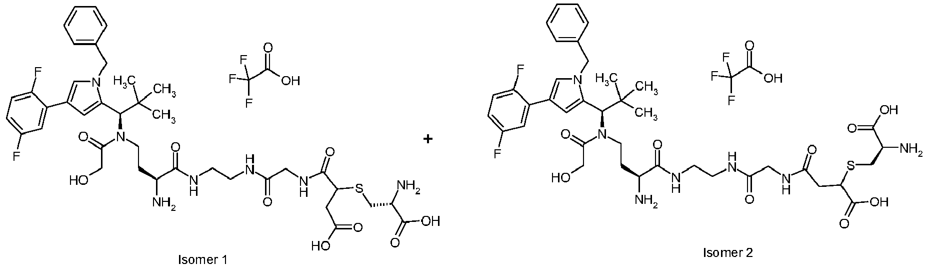

LC-MS (Methode 1): Rₜ = 0.80 min; MS (EIpos): m/z = 814 [M+H]⁺.

Zunächst wurde L-Cystein mit 1-({[2-(Trimethylsilyl)ethoxy]carbonyl}oxy)pyrrolidine-2,5-dion in DMF in Gegenwart von *N,N*-Diisopropylethylamin in N-{[2-(Trimethylsilyl)ethoxy]carbonyl}-L-cystein überführt.

406 mg (1.53 mmol) N-{[2-(Trimethylsilyl)ethoxy]carbonyl}-L-cystein wurden in 10 ml DMF gelöst und mit 157.5 mg (1.606 mmol) Maleinsäureanhydrid versetzt und der Ansatz 1 Stunde bei RT gerührt. 130 µL dieser Lösung wurden mit 7.5 mg (0.01 mmol) von Intermediat C66 versetzt und der Ansatz 5 min bei RT gerührt. Anschließend wurde im Vakuum eingeengt und der Rückstand durch präparative HPLC gereinigt. Das Lösemeittel wurde im Vakuum abgedampft und der Rückstand im Hochvakuum getrocknet. Es wurden 10 mg (89%) des geschützten Intermediats erhalten, wobei weder im HPLC noch im LC-MS die Regioisomere aufgetrennt werden konnten.

LC-MS (Methode 1): Rₜ = 1.38 min; MS (EIpos): m/z = 1120 [M+H]⁺.

Im letzten Schritt wurden die 10 mg von diesem Intermediat in 2 mL 2,2,2-Trifluorethanol gelöst. Es wurden 12 mg (0.088 mmol) Zinkchlorid zugegeben und der Ansatz 30 min bei 50°C gerührt. Anschließend wurden 26 mg (0.088 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 8.3 mg (99% d. Th.) der Titelverbindung als Regioisomerengemisch im Verhältnis 87:13 erhalten.

LC-MS (Methode 5): Rₜ = 2.3 min und 2.43 min; MS (ESIpos): m/z = 832 (M+H)⁺.

¹H-NMR Hauptregioisomer: (500 MHz, DMSO-d₆): δ = 8.7 (m, 1H), 8.5 (m, 2H), 8.1 (m, 1H), 7.6 (m, 1H), 7.5 (s, 1H) 7.4-7.15 (m, 6H), 6.9-7.0 (m, 1H), 6.85 (s, 1H), 5.61 (s, 1H), 4.9 und 5.2 (2d, 2H), 4.26 und 4.06 (2d, 2H), 3.5-3.8 (m, 5H), 3.0-3.4 (m, 5H), 2.75-3.0 (m, 3H), 2.58 und 2.57 (dd, 1H), 0.77 und 1,5 (2m, 2H), 0.81 (s, 9H).

Alternativ wurden die regioisomeren Titelverbindungen wie folgt hergestellt:
Zunächst wurde dazu L-Cystein mit 1-({[2-(Trimethylsilyl)ethoxy]carbonyl}oxy)pyrrolidine-2,5-dion in DMF in Gegenwart von *N*,*N*-Diisopropylethylamin in N-{ [2-(Trimethylsilyl)ethoxy]-carbonyl}-L-cystein überführt.

55 mg (0.068 mmol) von Intermediat F104 und 36 mg (0.136 mmol) N-{[2-(Trimethylsilyl) ethoxy]carbonyl}-L-cystein 15 ml DMF gelöst und der Ansatz 20 h bei RT gerührt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 15 mL THF/Wasser 1:1 gelöst. Es wurden 131 µL einer 2M wässrigen Lithiumhydroxidlösung zugegeben und der Ansatz 1 h bei RT gerührt. Anschließend wurde der Ansatz mit einer 1M Salzsäure neutral gestellt, das Lösemittel im Vakuum abgedampft und der Rückstand durch präparative HPLC gereinigt. Es wurden 37 mg (50% d. Th.) der regioisomeren geschützten Intermediate als farbloser Schaum erhalten.

LC-MS (Methode 5): Rₜ = 3.33 min und 3.36 min; MS (ESIpos): m/z = 976 (M+H)⁺.

Im letzten Schritt wurden 25 mg (0.023 mmol) von diesem Intermediat in 3 mL 2,2,2-Trifluorethanol gelöst. Es wurden 12.5 mg (0.092 mmol) Zinkchlorid zugegeben und der Ansatz 4 h bei 50°C gerührt. Anschließend wurden 27 mg (0.092 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 18.5 mg (85% d. Th.) der Titelverbindung als Regioisomerengemisch im Verhältnis 21:79 erhalten.

LC-MS (Methode 5): Rₜ = 2.37 min und 3.44 min; MS (ESIpos): m/z = 832 (M+H)⁺.

### Beispiel M3

### 4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)amino]-3-{[(2R)-2-amino-2-carboxyethyl]sulfanyl}-4-oxobutansäure -trifluoressigsäure (1:1) und 4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)amino]-2-{[(2R)-2-amino-2-carboxyethyl]sulfanyl}-4-oxobutansäure -trifluoressigsäure (1:1)

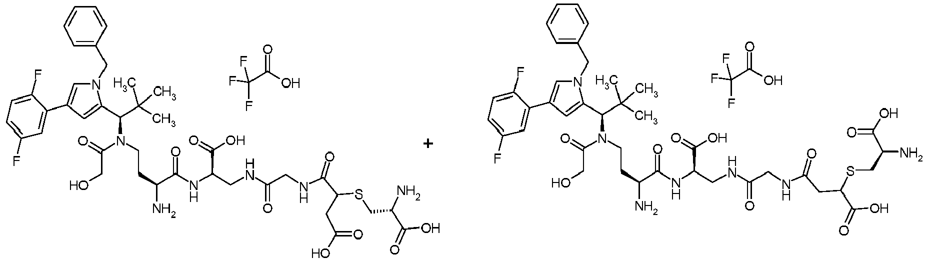

Zunächst wurde L-Cystein mit 1-({[2-(Trimethylsilyl)ethoxy]carbonyl}oxy)pyrrolidine-2,5-dion in DMF in Gegenwart von *N,N*-Diisopropylethylamin in N-{[2-(Trimethylsilyl)ethoxy]carbonyl}-L-cystein überführt.

11 mg (0.013 mmol) von Intermediat F193 und 8 mg (0.016 mmol) N-{[2-(Trimethylsilyl) ethoxy]carbonyl}-L-cystein wurden 3 ml DMF gelöst und der Ansatz 20 h bei RT gerührt. Anschließend wurde eingeengt und der Rückstand durch präparative HPLC gereinigt.

Nach Vereinigung der entsprechenden Fraktionen und Abdampfen der Lösemittel im Vakuum wurde der Rückstand in 2 mL THF/Wasser 1:1 gelöst. Es wurden 19 µL einer 2M wässrigen Lithiumhydroxidlösung zugegeben und der Ansatz 1 h bei RT gerührt. Dann wurden nochmals 19 µL der 2M wässrigen Lithiumhydroxidlösung zugegeben und der Ansatz über Nacht bei RT gerührt. Anschließend wurde mit einer 1M Salzsäure neutral gestellt, das Lösemittel im Vakuum abgedampft und der Rückstand durch präparative HPLC gereinigt. Es wurden 4.1 mg (38% d. Th.) der regioisomeren geschützten Intermediate als farbloser Schaum erhalten.

LC-MS (Methode 1): Rₜ = 1.03 min (breit); MS (ESIpos): m/z = 1020 (M+H)⁺.

Im letzten Schritt wurden 4.1 mg (0.004 mmol) von diesem Intermediat in 3 mL 2,2,2-Trifluorethanol gelöst. Es wurden 3 mg (0.022 mmol) Zinkchlorid zugegeben und der Ansatz 1 h bei 50°C gerührt. Anschließend wurden 6 mg (0.022 mmol) Ethylendiamin-N,N,N',N'-tetraessigsäure und 2 ml einer 0.1%-igen wässrigen Trifluoressigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 5 mg (quant.) der Titelverbindung als Regioisomerengemisch im Verhältnis 20:80 erhalten.

LC-MS (Methode 1): Rₜ = 0.78 min (breit); MS (ESIpos): m/z = 876 (M+H)⁺.

LC-MS (Methode 5): Rₜ = 2.36 min und 2.39 min; MS (ESIpos): m/z = 876 (M+H)⁺.

### Beispiel M4

### S-(1-{2-[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethoxy]ethyl} -2,5-dioxopyrrolidin-3-yl)-L-cystein -trifluoressigsäure (1:1)

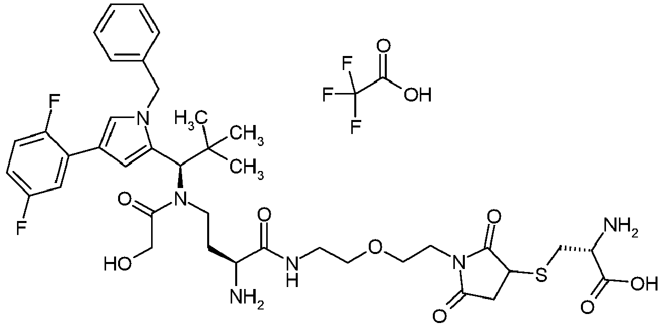

3 mg (4 µmol) von Intermediat F248 wurden in 2 ml DMF aufgenommen und mit 0.9 mg (8 µmol) L-Cystein versetzt. Das Reaktionsgemisch wurde 18 h bei RT gerührt und dann im Vakuum eingeengt. Der Rückstand wurde mittels präparativer HPLC gereinigt. Die entsprechenden Fraktionen wurden eingeengt und nach Lyophilisation des Rückstands aus Acetonitril/Wasser verblieben 1.1 mg (32% d. Th.) der Titelverbindung als weißer Feststoff.

LC-MS (Methode 1): Rₜ = 0.78 min; MS (EIpos): m/z = 801 [M+H]⁺.

### Beispiel M5

### (3R,7S)-7-Amino-17-{[(2R)-2-amino-2-carboxyethyl]sulfanyl}-3-[1-benzyl-4-(2,5-difluorphenyl)-1H-pyrrol-2-yl]-4-glycoloyl-2,2-dimethyl-8,16-dioxo-12-oxa-4,9,15-triazanonadecan-19-säure - trifluoressigsäure (1:1) und (3R,7S)-7-Amino-18-{[(2R)-2-amino-2-carboxyethyl]sulfanyl}-3-[1-benzyl-4-(2,5-difluorphenyl)-1H-pyrrol-2-yl]-4-glycoloyl-2,2-dimethyl-8,16-dioxo-12-oxa-4,9,15-triazanonadecan-19-säure - trifluoressigsäure (1:1)

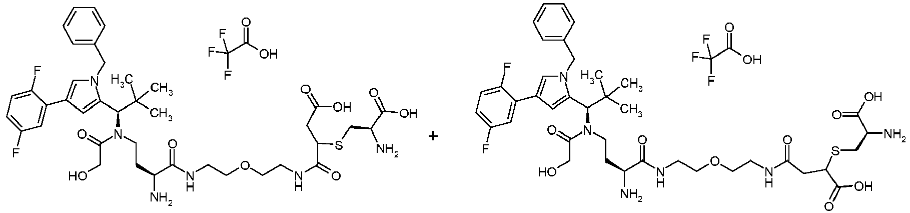

8 mg (0.010 mmol) von der geschützten Zwischenstufe von Intermediat F248 und 5.1 mg (0.02 mmol) N-{[2-(Trimethylsilyl) ethoxy]carbonyl}-L-cystein 3 ml DMF gelöst und der Ansatz 18 h bei RT gerührt und anschließend 2 h im Ultraschallbad behandelt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 2 mL THF/Wasser 1:1 gelöst. Es wurden 15 µL einer 2M wässrigen Lithiumhydroxidlösung zugegeben und der Ansatz 15 min bei RT gerührt. Anschließend wurde der Ansatz mit einer 1M Salzsäure auf einen pH-Wert von ~3 eingestellt, mit 20 ml Kochsalzlösung verdünnt und zweimal mit 20 ml Ethylacetat extrahiert. Die organische Phase wurde über Magnesiumsulfat getrocknet, eingeengt und der Rückstand aus Acetonitril/Wasser lyophilisiert. Es wurden 8.4 mg (78% d. Th. über 2 Stufen) der regioisomeren geschützten Intermediate als farbloser Schaum erhalten.

LC-MS (Methode 1): Rₜ = 1.44 min und 3.43 min; MS (ESIpos): m/z = 1107 (M+H)⁺.

Im letzten Schritt wurden 8 mg (0.007 mmol) von diesem Intermediat in 5 mL 2,2,2-Trifluorethanol gelöst. Es wurden 9.8 mg (0.072 mmol) Zinkchlorid zugegeben und der Ansatz 1.5 h bei 50°C gerührt. Anschließend wurde Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 4 mg (59% d. Th.) der Titelverbindung als Regioisomerengemisch im Verhältnis 31:67 erhalten.

LC-MS (Methode 1): Rₜ = 0.79 min und 0.81 min; MS (ESIpos): m/z = 819 (M+H)⁺.

### Beispiel M6

### 2-{[(2R)-2-Amino-2-carboxyethyl]sulfanyl}-4-({(14R)-13-(3-aminopropyl)-14-[1-benzyl-4-(2,5-difluorphenyl)-1H-pyrrol-2-yl]-15,15-dimethyl-2,7,12-trioxo-10-thia-3,6,13-triazahexadec-1-yl}amino)-4-oxobutansäure -trifluoressigsäure (1:2) und 3-{[(2R)-2-Amino-2-carboxyethyl]sulfanyl}-4-({(14R)-13-(3-aminopropyl)-14-[1-benzyl-4-(2,5-difluorphenyl)-1H-pyrrol-2-yl]-15,15-dimethyl-2,7,12-trioxo-10-thia-3,6,13-triazahexadec-1-yl}amino)-4-oxobutansäure -trifluoressigsäure (1:2)

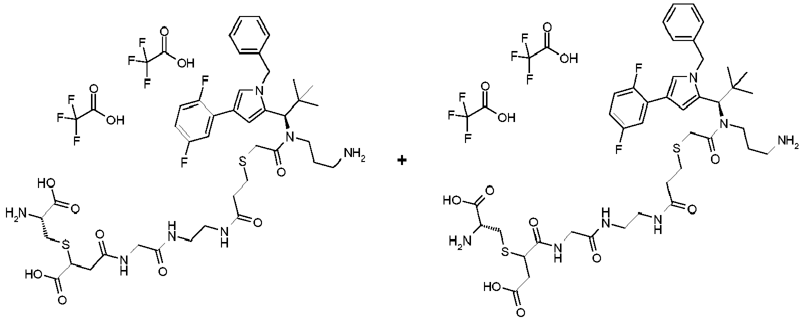

18 mg (0.021 mmol) von Intermediat F213 und 11.2 mg (0.04 mmol) N-{[2-(Trimethylsilyl) ethoxy]carbonyl}-L-cystein 2 ml DMF gelöst und der Ansatz 18 h bei RT gerührt. Die Reaktionsmischung wurde im Vakuum verdampft. Der Rückstand (21.2 mg) wurde in 3 mL THF/Wasser 1:1 gelöst. Es wurden 0.04 mL einer 2M wässrigen Lithiumhydroxidlösung zugegeben und der Ansatz 3 Stunden bei RT gerührt. Es wurden 0.02 mL einer 2M wässrigen Lithiumhydroxidlösung zugegeben und der Ansatz 1 Stunde bei RT gerührt. Anschließend wurde der Ansatz mit 7.2 mg (0.12 mmol) Essigsäure auf einen pH-Wert von ~7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 13 mg (57% über 2 Stufen) der regioisomeren geschützten Intermediate erhalten.

LC-MS (Methode 1): Rₜ = 1.03 min; MS (ESIpos): m/z = 1020 (M+H)⁺.

Im letzten Schritt wurden 13 mg (0.01 mmol) von diesem Intermediat in 2 mL 2,2,2-Trifluorethanol gelöst. Es wurden 6.2 mg (0.05 mmol) Zinkchlorid zugegeben und der Ansatz 7 h bei 50°C gerührt. Anschließend wurde 13.3 mg (0.05 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 10.3 mg (81.4%) der Titelverbindung als Regioisomerengemisch erhalten.

LC-MS (Methode 1): Rₜ = 1.03 min; MS (ESIpos): m/z = 875 (M+H)⁺.

### Beispiel M7

### S-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-L-cystein-trifluoressigsäure (1:1)

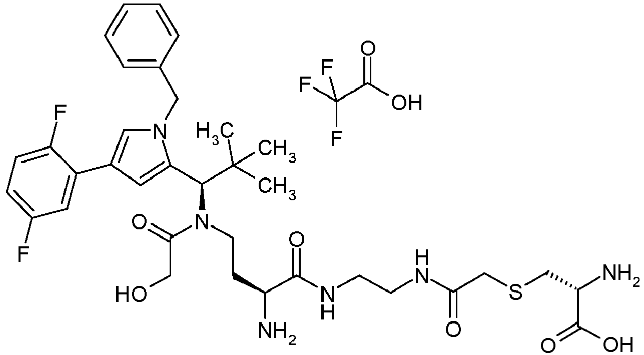

6 mg (8 µmol) von Intermediat F119 wurden in 3 mL DMF aufgenommen und mit 1.8 mg (15 µmol) L-Cystein versetzt. Das Reaktionsgemisch wurde 6 h bei RT gerührt und dann 3 Tage bei RT stehen gelassen. Anschließend wurde der Ansatz im Vakuum eingeengt und das Produkt mittels präparativer HPLC gereinigt.

LC-MS (Methode 1): Rₜ = 0.81 min; MS (ESIpos): m/z = 717 (M+H)⁺.

### Beispiel M8

### (3R)-6-{(11S,15R)-11-Amino-15-[1-benzyl-4-(2,5-difluorphenyl)-1H-pyrrol-2-yl]-14-glycoloyl-16,16-dimethyl-2,5,10-trioxo-3,6,9,14-tetraazaheptadec-1-yl}-5-oxothiomorpholin-3-carbonsäure - trifluoressigsäure (1:1)

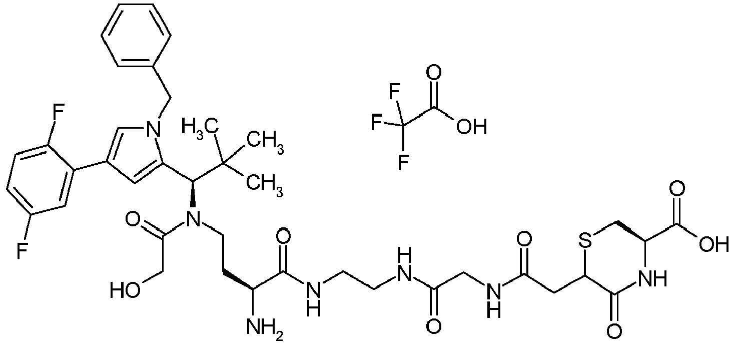

4 mg (0.004 mmol) der Verbindung aus Beispiel 135 wurden in 4 mL THF/Wasser gelöst und mit 48 µL einer 2molaren wässrigen Lithiumhydroxidlösung versetzt. Der Ansatz wurde 1 h bei RT gerührt und anschließend eingeengt und mittels präparativer HPLC gereinigt. Nach Vereinigung, Einengen und Lyophilisation aus Acetonitril/Wasser der entsprechenden Fraktionen wurden 2.4 mg (60% d. Th.) der Titelverbindung erhalten.

LC-MS (Methode 1): Rₜ = 0.86 min; MS (EIpos): m/z = 814 [M+H]⁺.

### Beispiel M9

### N-(3-Aminopropyl)-N-{(1R)-1-[1-benzyl-4-(2,5-difluorphenyl)-1H-pyrrol-2-yl]-2,2-dimethylpropyl} -2-hydroxyacetamid

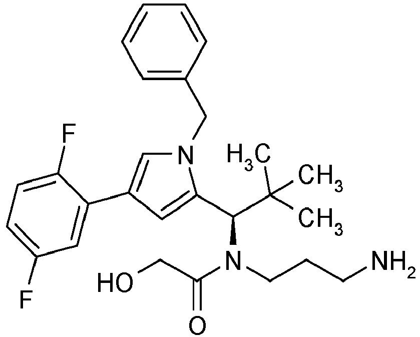

150.0 mg (0.42 mmol) (1R)-1-[1-Benzyl-4-(2,5-difluorphenyl)-1H-pyrrol-2-yl]-2,2-dimethylpropan-1-amin (Intermediat C52) wurden in 2.0 mL Dichlormethan vorgelegt und mit 29.2 mg (0.49 mmol) HOAc und 125.6 mg (0.59 mmol) Natriumtriacetoxyborhydrid versetzt und 5 min bei RT gerührt. Es wurden 98.9 mg (0.49 mmol) 3-(1,3-Dioxo-1,3-dihydro-2H-isoindol-2-yl)propanal zugegeben. Die Reaktionsmischung wurde über Nacht bei RT gerührt. Die Reaktionsmischung wurde mit Ethylacetat verdünnt und die organische Phase zweimal mit ges. Natriumcarbonat-Lösung und einmal mit ges. NaCl-Lösung gewaschen. Nach der Trocknung über Magnesiumsulfat wurde das Lösemittel im Vakuum verdampft und der Rückstand mittels Kieselgel (Laufinittel: Dichlormethan/Methanol 100:1) gereinigt. Die Lösemittel wurden im Vakuum verdampft und der Rückstand im Hochvakuum getrocknet. Man erhielt 188.6 mg (74 %) der Verbindung 2-[3-({(1R)-1-[1-Benzyl-4-(2,5-difluorphenyl)-1H-pyrrol-2-yl]-2,2-dimethylpropyl} amino)propyl]-1H-isoindol-1,3(2H)-dion.

LC-MS (Methode 1): Rₜ = 1.00 min; MS (ESIpos): m/z = 541 [M+H]⁺.

171.2 mg (0.32 mmol) 2-[3-({(1R)-1-[1-Benzyl-4-(2,5-difluorphenyl)-1H-pyrrol-2-yl]-2,2-dimethylpropyl}amino)propyl]-1H-isoindol-1,3(2H)-dion wurden in 5.0 mL Dichlormethan vorgelegt und mit 73.6 mg (0.73 mmol) Triethylamin versetzt. Bei 0 °C wurden 94.9 mg (0.70 mmol) Acetoxyessigsäurechlorid zugegeben und die Reaktionsmischung wurde über Nacht bei RT gerührt. Die Reaktionsmischung wurde mit Ethylacetat verdünnt und die organische Phase zweimal mit ges. Natriumhydrogencarbonat-Lösung und einmal mit ges. NaCl-Lösung gewaschen. Nach der Trocknung über Magnesiumsulfat wurde das Lösemittel im Vakuum verdampft und der Rückstand wurde mittels Biotage Isolera gereinigt (Kieselgel, Säule 10 g SNAP, Fluss 12 mL/min, Ethylacetat/Cyclohexan 1:3) gereinigt. Die Lösemittel wurden im Vakuum verdampft und der Rückstand im Hochvakuum getrocknet. Man erhielt 159.0 mg (77 %) der Verbindung 2-({(1R)-1-[1-Benzyl-4-(2,5-difluorphenyl)-1H-pyrrol-2-yl]-2,2-dimethylpropyl} [3-(1,3-dioxo-1,3-dihydro-2H-isoindol-2-yl)propyl]amino)-2-oxoethylacetat.

LC-MS (Methode 1): Rₜ = 1.35 min; MS (ESIpos): m/z = 642 [M+H]⁺.

147.2 mg (0.23 mmol) 2-({(1R)-1-[1-Benzyl-4-(2,5-difluorphenyl)-1H-pyrrol-2-yl]-2,2-dimethylpropyl}[3-(1,3-dioxo-1,3-dihydro-2H-isoindol-2-yl)propyl]amino)-2-oxoethylacetat wurden in 4.0 mL Ethanol vorgelegt und mit 356.2 mg (4.59 mmol) Methanamin (40 % in Wasser) versetzt. Die Reaktionsmischung wurde über Nacht bei 50 °C gerührt. Das Lösemittel wurde im Vakuum verdampft und der Rückstand dreimal mit Toluol kodestilliert. Der Rückstand wurde mittels Kieselgel (Laufinittel: Dichlormethan/Methanol 10:1) gereinigt. Die Lösemittel wurden im Vakuum verdampft und der Rückstand im Hochvakuum getrocknet. Man erhielt 67.4 mg (63 %) der Titelverbindung.

LC-MS (Methode 1): Rₜ = 0.91 min; MS (ESIpos): m/z = 470 [M+H]⁺.

### Beispiel M10

### (2R,28R)-28-Amino-2-[({2-[(3-aminopropyl){(1R)-1-[1-benzyl-4-(2,5-difluorphenyl)-1H-pyrrol-2-yl]-2,2-dimethylpropyl } amino]-2-oxoethyl } sulfanyl)methyl]-25-(carboxymethyl)-4,20,24-trioxo-7,10,13,16-tetraoxa-26-thia-3,19,23-triazanonacosan-1,29-disäure -trifluoressigsäure (1:2) und (1R,28R,34R)-1-Amino-33-(3-aminopropyl)-34-[1-benzyl-4-(2,5-difluorphenyl)-1H-pyrrol-2-yl]-35,35-dimethyl-6,10,26,32-tetraoxo-14,17,20,23-tetraoxa-3,30-dithia-7,1 1,27,33-tetraazahexatriacontan-1,4,28-tricarbonsäure -trifluoressigsäure (1:2)

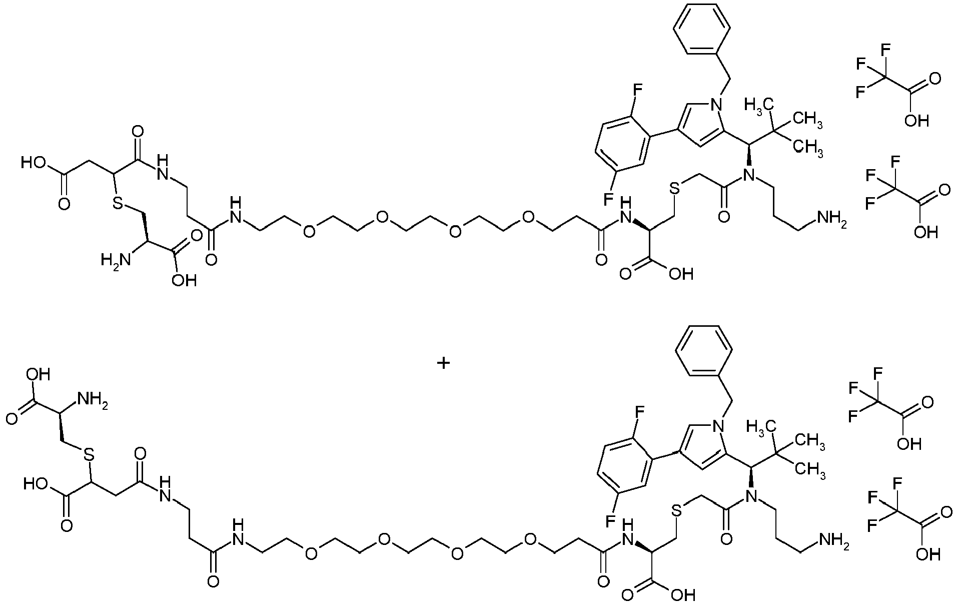

20 mg (0.018 mmol) R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cystein-trifluoressigsäure (1:1) (Intermediat F209) und 9.78 mg (0.036 mmol) N-{[2-(Trimethylsilyl) ethoxy]carbonyl}-L-cystein wurden in 2 ml DMF gelöst und der Ansatz 18 h bei RT gerührt. Die Reaktionsmischung wurde im Vakuum verdampft. Der Rückstand (47.7 mg) wurde in 3 mL THF/Wasser 1:1 gelöst. Es wurden 0.08 mL einer 2M wässrigen Lithiumhydroxidlösung zugegeben und der Ansatz 1 Stunde bei RT gerührt. Anschließend wurde der Ansatz mit 9.26 mg (0.15 mmol) Essigsäure auf einen pH-Wert von ~7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 15.3 mg (29% über 2 Stufen) der regioisomeren geschützten Intermediate erhalten.

LC-MS (Methode 6): Rₜ = 12.26 min und 12.30min; MS (ESIpos): m/z = 1254 (M+H)⁺.

Im letzten Schritt wurden 15.3 mg (0.01 mmol) von diesem Intermediat in 2 mL 2,2,2-Trifluorethanol gelöst. Es wurden 6.1 mg (0.05 mmol) Zinkchlorid zugegeben und der Ansatz 2 h bei 50°C gerührt. Anschließend wurde 13.1 mg (0.05 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 11.9 mg (79.5%) der Titelverbindung als Regioisomerengemisch erhalten.

LC-MS (Methode 1): Rₜ = 0.85 min; MS (ESIpos): m/z = 1110 (M+H)⁺.

### Beispiel M11

### S-{2-[(3-Aminopropyl) {(1R)-1-[1-benzyl-4-(2,5-difluorphenyl)-1H-pyrrol-2-yl]-2,2-dimethylpropyl}amino]-2-oxoethyl}-L-cystein -trifluoressigsäure (1:2)

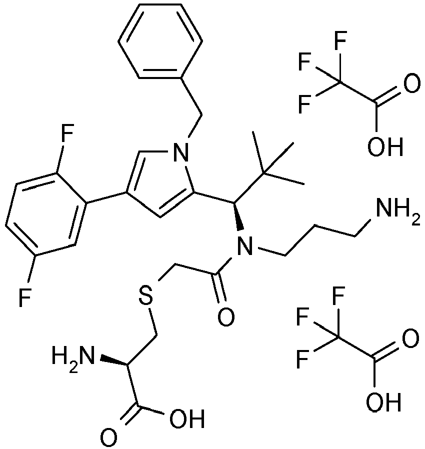

15.0 mg (0.018 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein - trifluoressigsäure (1:1) (Intermediat C71) wurden in 1.0 mL Trifluorethanol gelöst und mit 7.4 mg (0.054 mmol) Zinkdichlorid versetzt. Die Reaktionsmischung rührte über Nacht bei 50 °C. Die Reaktionsmischung wurde mit 15.8 mg (0.054 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 11.1 mg (77 %) der Titelverbindung.

LC-MS (Methode 1): Rₜ = 0.83 min; MS (ESIpos): m/z = 573 (M+H)⁺.

### Beispiel M12

### 4-{[(1R)-2-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)-1-carboxyethyl]amino}-4-oxobutansäure - trifluoressigsäure (1:1)

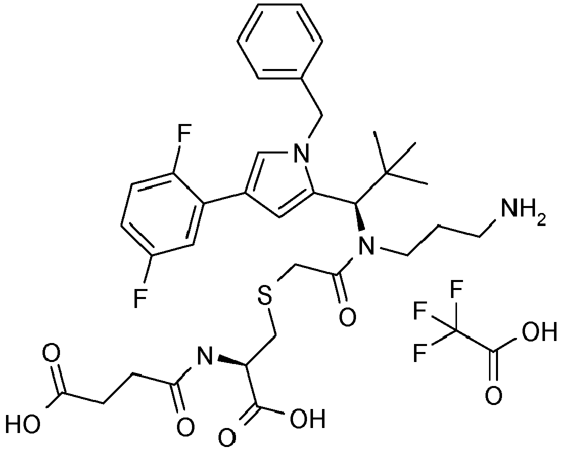

12.2 mg (0.014 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-(4-tert-butoxy-4-oxobutanoyl)-L-cystein (Intermediat C77) wurden in 2.0 mL Trifluorethanol gelöst und mit 11.4 mg (0.084 mmol) Zinkdichlorid versetzt. Die Reaktionsmischung rührte 3 h bei 50 °C. Die Reaktionsmischung wurde mit 24.5 mg (0.084 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 4.6 mg (42 %) der Titelverbindung.

LC-MS (Methode 1): Rₜ = 0.88 min; MS (ESIpos): m/z = 673 (M+H)⁺.

### Beispiel M13

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl] amino } -2-oxoethyl)amino] -2- { [(2R)-2-amino-2-carboxyethyl]sulfanyl}-4-oxobutansäure -trifluoressigsäure (1:1) Regioisomer 1, Epimer 1 (2R) oder (2S)

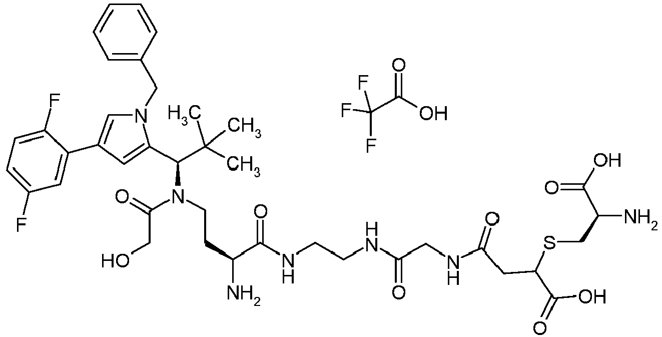

LC-MS (Methode 5): Rₜ = 2.44 min; MS (ESIpos): m/z = 832 [M+H]⁺.

Zunächst wurde Methyl-L-cysteinathydrochlorid (1:1) mit 1-({[2-(Trimethylsilyl)ethoxy] carbonyl}oxy)pyrrolidine-2,5-dion in DMF in Gegenwart von *N,N*-Diisopropylethylamin in Methyl-N-{[2-(trimethylsilyl)ethoxy]carbonyl}-L-cysteinat überführt.

408 mg (1.93 mmol) von kommerziell erhältlicher 3-Brom-4-methoxy-4-oxobutansäure und 180 mg (0.644 mmol) Methyl-N-{[2-(trimethylsilyl)ethoxy]carbonyl}-L-cysteinat wurden in 8 ml DMF gelöst und mit 147 mg (0.97 mmol) 1,8-Diazabicyclo(5.4.0)undec-7-en versetzt. Nach 18 h Rühren bei RT wurden nochmals 136 mg (0.64 mmol) 3-Brom-4-methoxy-4-oxobutansäure sowie 147 mg (0.97 mmol) 1,8-Diazabicyclo(5.4.0)undec-7-en hinzugefügt und der Ansatz weitere 12 h bei RT gerührt und anschließend im Vakuum eingeengt. Der Rückstand wurde durch präparative HPLC gereinigt. Nach Vereinigung der entsprechenden Fraktionen und Abdampfen der Lösemittel im Vakuum wurden 151 mg (57% d. Th.) 4-Methoxy-3-{[(2R)-3-methoxy-3-oxo-2-({[2-(trimethylsilyl)ethoxy]carbonyl}amino)propyl] sulfanyl}-4-oxobutansäure erhalten.

LC-MS (Methode 12): Rₜ = 1.74 min; MS (ESIneg): m/z = 408 (M-H)⁻.

Von diesem Intermediat wurden 145 mg mittels überkritischer Fluidchromatographie über chirale Säulen in die Einzeldiastereomere aufgetrennt (SFC; Säule: DAICEL, AD-H 5u 250x20 mm; Fluss: 80 mL/min; Methode: AD-25%ETOH-80ml; Druck: 100 bar; Wellenlänge: 210 nM) und dabei wurden von Epimer 1 63 mg (43%) und von Epimer 2 58 mg (40%) erhalten.

Epimer 1 wurde wie folgt charakterisiert:
LC-MS (Methode 5): Rₜ = 2.94 min; MS (ESIneg): m/z = 408 (M-H)⁻.

¹H-NMR: (400 MHz, DMSO-d₆): δ = 7.57 (d, 1H), 4.24 (m, 1H), 4.05 (t, 2H), 3.67 (t, 1H), 3.65 (s, 3H), 3.62 (s, 3H), 3.05 (dd, 1H), 2.70-2.88 (m, 2H), 2.59 (dd, 1H), 0.93 (t, 2H), 0.02 (s, 9H).

Epimer 2 wurde wie folgt charakterisiert:
LC-MS (Methode 5): Rₜ = 2.95 min; MS (ESIneg): m/z = 408 (M-H)⁻.

¹H-NMR: (400 MHz, DMSO-d₆): δ = 7.58 (d, 1H), 4.16-4.23 (m, 1H), 4.05 (t, 2H), 3.67 (dd, 1H), 3.65 (s, 3H), 3.64 (s, 3H), 3.04 (dd, 1H), 2.88 (dd, 1H), 2.77 (dd, 1H), 2.61 (dd, 1H), 0.92 (t, 2H), 0.02 (s, 9H).

32.5 mg (0.079 mmol) von Epimer 1 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 43 mg (57% d. Th.) vom voll geschützten Intermediat Methyl-4-{[(8S)-8-{2-[{(1R)-1-[1-benzyl-4-(2,5-difluorphenyl)-1H-pyrrol-2-yl]-2,2-dimethyl propyl } (glycoloyl)amino] ethyl }-2,2-dimethyl-6,9,14-trioxo-5-oxa-7,10,13-triaza-2-silapentadecan-15-yl]amino}-2-{[(2R)-3-methoxy-3-oxo-2-({[2-(trimethylsilyl)ethoxy]carbonyl}amino)propyl] sulfanyl}-4-oxobutanoat erhalten wurden.

Anschließend wurden 40 mg (0.035 mmol) dieses Intermediats mit 0.9 mL einer 2 molaren Lithiumhydroxidlösung in 11 ml Methanol 20 min lang bei RT gerührt, wobei beide Methylestergruppen abgespalten wurden. Nach HPLC-Reinigung wurden 12 mg (31% d. Th.) des Dicarbonsäurederivats erhalten.

LC-MS (Methode 5): Rₜ = 4.74 min; MS (ESIpos): m/z = 1120 [M+H]⁺.

10 mg (0.009 mmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 2.6 mg (30% d. Th.) der Titelverbindung erhalten.

LC-MS (Methode 5): Rₜ = 2.44 min; MS (ESIpos): m/z = 832 [M+H]⁺.

### Beispiel M14

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl] amino } -2-oxoethyl)amino] -2- { [(2R)-2-amino-2-carboxyethyl]sulfanyl}-4-oxobutansäure -trifluoressigsäure (1:1) Regioisomer 1, Epimer 2 (2R oder 2S)

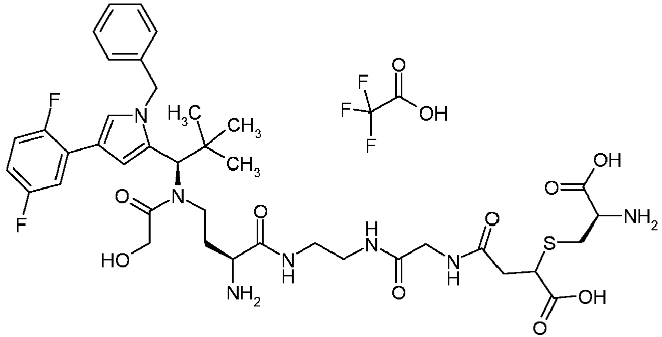

LC-MS (Methode 5): Rₜ = 2.44 min; MS (EIpos): m/z = 832 [M+H]⁺.

Das in Beispiel M13 beschriebene Intermediat Epimer 2 wurde in Analogie zu der Beschreibung in Beispiel M13 umgesetzt:
32.5 mg (0.079 mmol) von Epimer 2 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 43 mg (57% d. Th.) vom voll geschützten Intermediat Methyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-2-{[(2R)-3-methoxy-3-oxo-2-({[2-(trimethylsilyl)ethoxy]carbonyl}amino)propyl] sulfanyl}-4-oxobutanoat erhalten wurden.

Anschließend wurden 40 mg (0.035 mmol) dieses Intermediats mit 0.9 mL einer 2 molaren Lithiumhydroxidlösung in 11 ml Methanol 20 min lang bei RT gerührt, wobei beide Methylestergruppen abgespalten wurden. Nach HPLC-Reinigung wurden 11 mg (28% d. Th.) des Dicarbonsäurederivats erhalten.

LC-MS (Methode 5): Rₜ = 4.74 min; MS (ESIpos): m/z = 1120 [M+H]⁺.

10 mg (0.009 mmol) von diesem Intermediat wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 4.4 mg (52% d. Th.) der Titelverbindung erhalten.

LC-MS (Methode 5): Rₜ = 2.44 min; MS (ESIpos): m/z = 832 [M+H]⁺.

### Beispiel M15

### 4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure -trifluoressigsäure (1:1) Regioisomer 2, Epimer 1 (3R oder 3S)

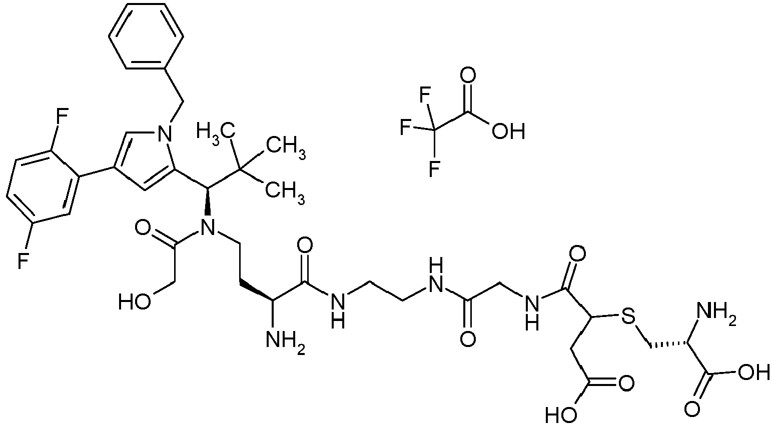

LC-MS (Methode 5): Rₜ = 2.45 min; MS (EIpos): m/z = 832 [M+H]⁺.

742.8 mg (3.3 mmol) von kommerziell erhältlicher 2-Brom-4-ethoxy-4-oxobutansäure und 802 mg (2.87 mmol) Methyl-N-{[2-(trimethylsilyl)ethoxy]carbonyl}-L-cysteinat wurden in 32 ml DMF gelöst und mit 655.4 mg (4.31 mmol) 1,8-Diazabicyclo(5.4.0)undec-7-en versetzt. Nach 20 h Rühren bei RT wurde der Ansatz im Vakuum eingeengt und der Rückstand durch präparative HPLC gereinigt. Nach Vereinigung der entsprechenden Fraktionen und Abdampfen der Lösemittel im Vakuum wurden 521 mg (43% d. Th.) 4-Ethoxy-2-{[(2R)-3-methoxy-3-oxo-2-({[2-(trimethylsilyl)ethoxy]carbonyl}amino)propyl]sulfanyl}-4-oxobutansäure erhalten.

LC-MS (Methode 5): Rₜ = 3.13 min; MS (ESIpos): m/z = 424 (M+H)⁺.

Von diesem Intermediat wurden 510 mg mittels überkritischer Fluidchromatographie über chirale Säulen in die Einzeldiastereomere aufgetrennt (SFC; Säule: DAICEL, AD-H 5u 250x20 mm; Fluss: 80 mL/min; Methode: AD-10%ETOH-80ml; Druck: 100 bar; Wellenlänge: 210 nM) und dabei wurden von Epimer 1 100 mg (20%) und von Epimer 2 141 mg (28%) erhalten.

Epimer 1 wurde wie folgt charakterisiert:
LC-MS (Methode 1): Rₜ = 0.99 min; MS (ESIneg): m/z = 422 (M-H)⁻.

¹H-NMR: (400 MHz, DMSO-d₆): δ = 7.60 (d, 1H), 4.18-4.26 (m, 1H), 4.01-4.08 (m, 4H), 3.63 (s, 3H), 3.59 (dd, 1H), 3.04 (dd, 1H), 2.92 (dd, 1H), 2.80 (dd, 1H), 2.63 (dd, 1H), 1.17 (t, 3H), 0.92 (t, 2H), 0.02 (s, 9H).

Epimer 2 wurde wie folgt charakterisiert:
LC-MS (Methode 5): Rₜ = 2.95 min; MS (ESIneg): m/z = 408 (M-H)⁻.

¹H-NMR: (400 MHz, DMSO-d₆): δ = 7.56 (d, 1H), 4.21-4.29 (m, 1H), 4.01-4.1 (m, 4H), 3.64 (s, 3H), 3.58 (dd, 1H), 3.08 (dd, 1H), 2.85 (dd, 1H), 2.78 (dd, 1H), 2.60 (dd, 1H), 1.17 (t, 3H), 0.93 (t, 2H), 0.02 (s, 9H).

33.6 mg (0.079 mmol) von Epimer 1 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 51 mg (63% d. Th.) vom voll geschützten Intermediat Ethyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-3-{[(2R)-3-methoxy-3-oxo-2-({[2-(trimethylsilyl)ethoxy] carbonyl}amino)propyl] sulfanyl}-4-oxobutanoat erhalten wurden.

Anschließend wurden 49 mg (0.042 mmol) dieses Intermediats mit 0.5 mL einer 2 molaren Lithiumhydroxidlösung in 12 ml THF/Wasser 1:1 30 min lang bei RT gerührt, wobei beide Methylestergruppen abgespalten wurden. Nach Ansäuern und HPLC-Reinigung wurden 11 mg (24% d. Th.) des Dicarbonsäurederivats erhalten.

LC-MS (Methode 5): Rₜ = 4.68 min; MS (ESIpos): m/z = 1120 [M+H]⁺.

11 mg (0.01 mmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 3.7 mg (39% d. Th.) der Titelverbindung erhalten.

LC-MS (Methode 5): Rₜ = 2.45 min; MS (ESIpos): m/z = 832 [M+H]⁺.

### Beispiel M16

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl] amino } -2-oxoethyl)amino] -3 - { [(2R)-2-amino-2-carboxyethyl]sulfanyl}-4-oxobutansäure -trifluoressigsäure (1:1) Regioisomer 2, Epimer 2 (3R oder 3S)

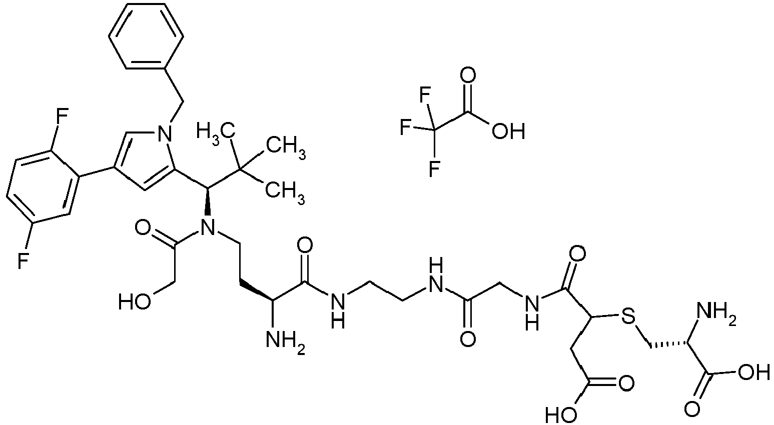

LC-MS (Methode 5): Rₜ = 2.44 min; MS (EIpos): m/z = 832 [M+H]⁺.

Das in Beispiel M15 beschriebene Intermediat Epimer 2 wurde in Analogie zu der Beschreibung in Beispiel M15 umgesetzt:
33.6 mg (0.079 mmol) von Epimer 2 wurden in Gegenwart von 30 mg (0.079 mmol) HATU und 13.4 mg (0.132 mmol) 4-Methylmorpholin mit 50 mg (0.066 mmol) von Intermediat C66 gekuppelt, wobei nach HPLC Reinigung 51 mg (63% d. Th.) vom voll geschützten Intermediat Ethyl-4-{[(8S)-8-{2-[{(1R)-1-[1-benzyl-4-(2,5-difluorphenyl)-1H-pyrrol-2-yl]-2,2-dimethyl propyl}(glycoloyl)amino]ethyl}-2,2-dimethyl-6,9,14-trioxo-5-oxa-7,10,13-triaza-2-silapentadecan-15-yl]amino}-3-{[(2R)-3-methoxy-3-oxo-2-({[2-(trimethylsilyl)ethoxy] carbonyl}amino)propyl] sulfanyl}-4-oxobutanoat erhalten wurden.

Anschließend wurden 49 mg (0.042 mmol) dieses Intermediats mit 0.5 mL einer 2 molaren Lithiumhydroxidlösung in 12 ml THF/Wasser 1:1 30 min lang bei RT gerührt, wobei beide Methylestergruppen abgespalten wurden. Nach Ansäuern und HPLC-Reinigung wurden 13.4 mg (28% d. Th.) des Dicarbonsäurederivats erhalten.

LC-MS (Methode 5): Rₜ = 4.66 min; MS (ESIpos): m/z = 1120 [M+H]⁺.

13.4 mg (0.012 mmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 7.5 mg (66% d. Th.) der Titelverbindung erhalten.

LC-MS (Methode 5): Rₜ = 2.44 min; MS (ESIpos): m/z = 832 [M+H]⁺.

### Beispiel M17

### (2S)-2-Amino-4-[{(1R)-1-[1-benzyl-4-(2,5-difluorphenyl)-1H-pyrrol-2-yl]-2,2-dimethylpropylt (glycoloyl)amino]butansäurehydrochlorid (1:1)

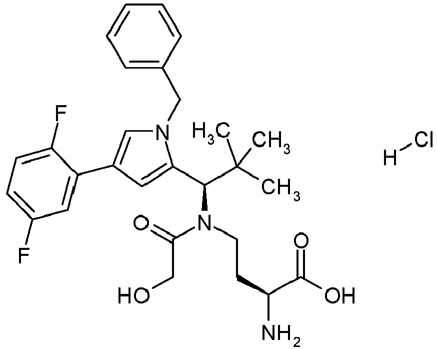

150 mg (0.2 mmol) von Intermediat C53 wurden in 15 mL DMF gelöst und mit 2.29 g (20.39 mmol) DABCO versetzt. Der Ansatz wurde 30 min im Ultraschallbad behandelt. Durch Zugabe von 1.17 ml Essigsäure wurde der Ansatz dann auf pH 3-4 gebracht und im Vakuum eingeengt. Der Rückstand wurde durch präparative HPLC gereinigt und die entsprechenden Fraktionen wurden bei RT im Vakuum eingeengt. Der Rückstand wurde in Acetonitril/Wasser 1:1 aufgenommen, mit 5 mL einer 4N Salzsäure versetzt und anschließend lyophilisiert. So wurden 81 mg (68% d. Th.) der Titelverbindung erhalten.

LC-MS (Methode 5): Rₜ = 2.69 min; MS (EIpos): m/z = 514 [M+H]⁺.

### Beispiel M18

### N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-L-glutamin -trifluoressigsäure (1:1)

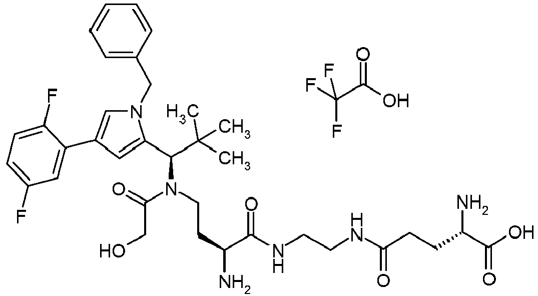

Zunächst wurde nach klassischen Methoden der Peptidchemie Trifluoressigsäure -benzyl-N-(2-aminoethyl)-N²-[(benzyloxy)carbonyl]-L-glutaminat (1:1) hergestellt. Diese Intermediat wurde dann in Gegenwart von HATU mit Intermediat C58 gekuppelt. Anschließend wurde zunächst durch hydrogenolytische Spaltung die Benzyloxycarbonyl-Schutzgruppe sowie der Benzylester entfernt und anschließend mit Zinkchlorid die 2-(Trimethylsilyl)ethoxycarbonyl-Schutzgrupe.

LC-MS (Methode 6): Rₜ = 1.91 min; MS (EIpos): m/z = 685 [M+H]⁺.

### Beispiel M19

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-L-lysin -trifluoressigsäure (1:1)

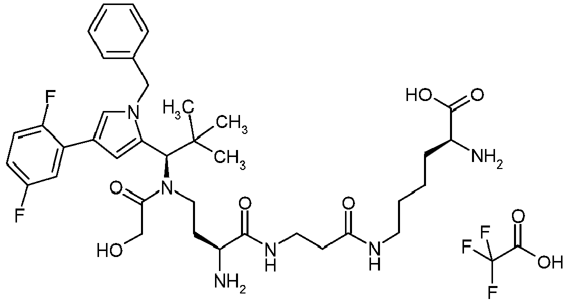

Zunächst wurde nach in der Peptidchemie bekannten klassischen Schutzgruppenoperationen Trifluoressigsäure --2-(trimethylsilyl)ethyl-N2-[(benzyloxy)carbonyl]-L-lysinat (1:1) hergestellt. Dieses Intermediat wurde dann in Gegenwart von HATU mit Intermediat C61 gekuppelt. Anschließend wurde zunächst mit Zinkchlorid die 2-(Trimethylsilyl)ethoxycarbonyl-Schutzgrupe sowie der 2-(Trimethylsilyl)ethylester gespalten. Schließlich wurde die Titelverbindung durch hydrogenolytische Spaltung der Benzyloxycarbonyl-Schutzgruppe und Reinigung durch präparative HPLC erhalten.
HPLC (Methode 11): Rₜ = 1.65 min;

### Beispiel M20

### (1R,4R,27R,33R)-1-Amino-32-(3-aminopropyl)-33-[1-benzyl-4-(2,5-difluorphenyl)-1H-pyrrol-2-yl]-34,34-dimethyl-6,9,25,31-tetraoxo-13,16,19,22-tetraoxa-3,29-dithia-7,10,26,32-tetraazapentatriacontan-1,4,27-tricarbonsäure -trifluoressigsäure (1:2)

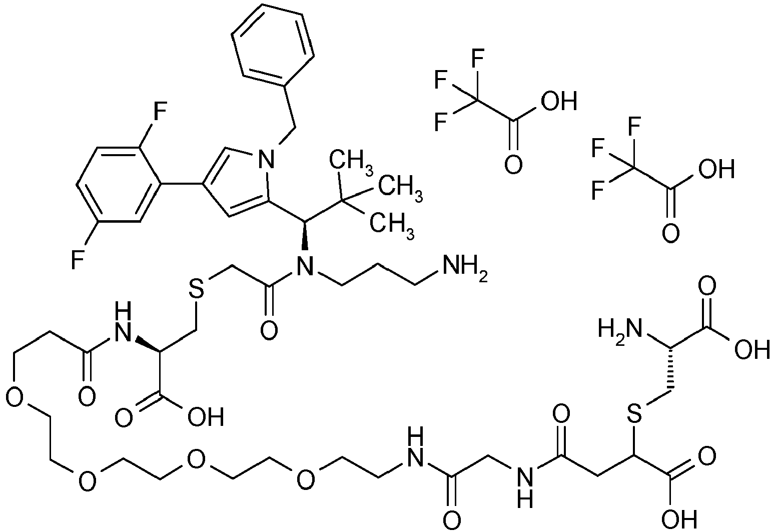

Zunächst wurde Methyl-L-cysteinathydrochlorid (1:1) mit 1-({[2-(Trimethylsilyl)ethoxy] carbonyl}oxy)pyrrolidine-2,5-dion in DMF in Gegenwart von *N*,*N*-Diisopropylethylamin in Methyl-N-{[2-(trimethylsilyl)ethoxy]carbonyl} -L-cysteinat überführt.

408 mg (1.93 mmol) von kommerziell erhältlicher 3-Brom-4-methoxy-4-oxobutansäure und 180 mg (0.644 mmol) Methyl-N-{[2-(trimethylsilyl)ethoxy]carbonyl}-L-cysteinat wurden in 8 ml DMF gelöst und mit 147 mg (0.97 mmol) 1,8-Diazabicyclo(5.4.0)undec-7-en versetzt. Nach 18 h Rühren bei RT wurden nochmals 136 mg (0.64 mmol) 3-Brom-4-methoxy-4-oxobutansäure sowie 147 mg (0.97 mmol) 1,8-Diazabicyclo(5.4.0)undec-7-en hinzugefügt und der Ansatz weitere 12 h bei RT gerührt und anschließend im Vakuum eingeengt. Der Rückstand wurde durch präparative HPLC gereinigt. Nach Vereinigung der entsprechenden Fraktionen und Abdampfen der Lösemittel im Vakuum wurden 151 mg (57% d. Th.) 4-Methoxy-3-{[(2R)-3-methoxy-3-oxo-2-({[2-(trimethylsilyl)ethoxy]carbonyl}amino)propyl] sulfanyl}-4-oxobutansäure erhalten.

LC-MS (Methode 12): Rₜ = 1.74 min; MS (ESIneg): m/z = 408 (M-H)⁻.

3.66 mg (8.93 µmol) von 4-Methoxy-3-{[(2R)-3-methoxy-3-oxo-2-({[2-(trimethylsilyl)ethoxy]-carbonyl}amino)propyl] sulfanyl}-4-oxobutansäure wurden in Gegenwart von 3.66 mg (8.93 µmol) HATU und 1.6 µl (15 µmol) 4-Methylmorpholin mit 13.0 mg (7.44 µmol) von S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-(glycylamino)-4,7,10,13-tetraoxapentadecan-1-oyl]-L-cystein -trifluoressigsäure (1:1) (Intermediat C80) gekuppelt, wobei nach HPLC Reinigung 3.9 mg (37% d. Th.) vom voll geschützten Intermediat S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(8R,11R)-8,11-bis(methoxycarbonyl)-2,2-dimethyl-6,13-dioxo-5-oxa-10-thia-7-aza-2-silatridecan-13-yl]glycyl}amino)-4,7,10,13-tetraoxapentadecan-1-oyl]-L-cystein erhalten wurden.

Anschließend wurden 3.90 mg (2.76 µmol) dieses Intermediats mit 35 µl einer 2 molaren Lithiumhydroxidlösung in 1.0 ml THF/Wasser 3:1 15 min lang bei RT gerührt, wobei beide Methylestergruppen abgespalten wurden. Nach HPLC-Reinigung wurden 3.60 mg (94% d. Th.) des Dicarbonsäurederivats erhalten.

LC-MS (Methode 5): Rₜ = 4.83 min; MS (ESIpos): m/z = 1385 [M+H]⁺.

3.6 mg (2.6 µmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 1.92 mg (55% d. Th.) der Titelverbindung erhalten.

LC-MS (Methode 5): Rₜ = 2.72 min; MS (ESIneg): m/z = 1094 [M-H]⁻.

### Beispiel M21

### (2R,24S,27R)-27-Amino-2-[({2-[(3-aminopropyl){(1R)-1-[1-benzyl-4-(2,5-difluorphenyl)-1H-pyrrol-2-yl] -2,2-dimethylpropyl} amino] -2-oxoethyl} sulfanyl)methyl] -24-(carboxymethyl)-4,20,23 - trioxo-7,10,13,16-tetraoxa-25-thia-3,19,22-triazaoctacosan-1,28-disäure -trifluoressigsäure (1:2)

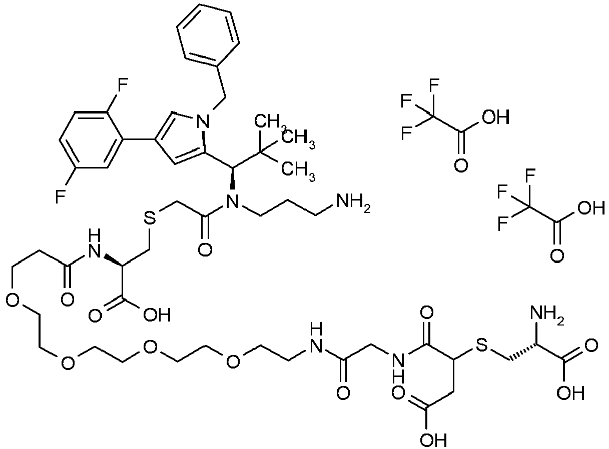

742.8 mg (3.3 mmol) von kommerziell erhältlicher 2-Brom-4-ethoxy-4-oxobutansäure und 802 mg (2.87 mmol) Methyl-N-{[2-(trimethylsilyl)ethoxy]carbonyl}-L-cysteinat wurden in 32 ml DMF gelöst und mit 655.4 mg (4.31 mmol) 1,8-Diazabicyclo(5.4.0)undec-7-en versetzt. Nach 20 h Rühren bei RT wurde der Ansatz im Vakuum eingeengt und der Rückstand durch präparative HPLC gereinigt. Nach Vereinigung der entsprechenden Fraktionen und Abdampfen der Lösemittel im Vakuum wurden 521 mg (43% d. Th.) 4-Ethoxy-2-{[(2R)-3-methoxy-3-oxo-2-({[2-(trimethylsilyl)ethoxy]carbonyl}amino)propyl]sulfanyl}-4-oxobutansäure erhalten.

LC-MS (Methode 5): Rₜ = 3.13 min; MS (ESIpos): m/z = 424 (M+H)⁺.

4.36 mg (10.3 µmol) von 4-Ethoxy-2-{[(2R)-3-methoxy-3-oxo-2-({[2-(trimethylsilyl)ethoxy]-carbonyl}amino)propyl]sulfanyl}-4-oxobutansäure wurden in Gegenwart von 3.92 mg (10.3 µmol) HATU und 1.9 µl (17 µmol) 4-Methylmorpholin mit 15.0 mg (8.59 µmol) von S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-(glycylamino)-4,7,10,13-tetraoxapentadecan-1-oyl]-L-cystein -trifluoressigsäure (1:1) (Intermediat C80) gekuppelt, wobei nach HPLC Reinigung 3.6 mg (26% d. Th.) vom voll geschützten Intermediat S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[15-({N-[(8R,11S)-11-(2-ethoxy-2-oxoethyl)-8-(methoxycarbonyl)-2,2-dimethyl-6,12-dioxo-5-oxa-10-thia-7-aza-2-siladodecan-12-yl]glycyl}amino)-4,7,10,13-tetraoxapentadecan-1-oyl]-L-cystein erhalten wurden.

Anschließend wurden 6.20 mg (2.82 µmol) dieses Intermediats mit 35 µl einer 2 molaren Lithiumhydroxidlösung in 1.0 ml THF/Wasser 1:1 15 min lang bei RT gerührt, wobei beide Estergruppen abgespalten wurden. Nach Ansäuern und HPLC-Reinigung wurden 3.60 mg (92% d. Th.) des Dicarbonsäurederivats erhalten.

LC-MS (Methode 5): Rₜ = 4.71 min; MS (ESIpos): m/z = 1385 [M+H]⁺.

3.60 mg (1.69 µmol) dieses Intermediats wurden schließlich mit Zinkchlorid in Trifluorethanol wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 0.88 mg (39% d. Th.) der Titelverbindung erhalten.

LC-MS (Methode 5): Rₜ = 2.72 min; MS (ESIneg): m/z = 1094 [M-H]⁻.

### Beispiel M22

### (2R,27R)-27-Amino-2-[({2-[(3-aminopropyl){(1R)-1-[1-benzyl-4-(2,5-difluorphenyl)-1H-pyrrol-2-yl]-2,2-dimethylpropyl} amino]-2-oxoethyl} sulfanyl)methyl]-24-(carboxymethyl)-4,20,23-trioxo-7,10,13,16-tetraoxa-25-thia-3,19,22-triazaoctacosan-1,28-disäure-trifluoressigsäure (1:2) und (1R,27R,33R)-1-Amino-32-(3-aminopropyl)-33-[1-benzyl-4-(2,5-difluorphenyl)-1H-pyrrol-2-yl]-34,34-dimethyl-6,9,25,31-tetraoxo-13,16,19,22-tetraoxa-3,29-dithia-7,10,26,32-tetraazapentatriacontan-1,4,27-tricarbonsäure-trifluoressigsäure (1:2)

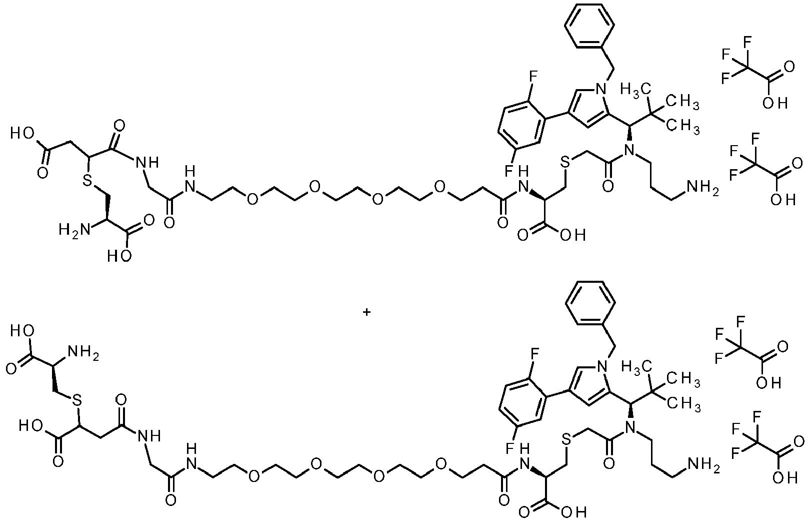

16.5 mg (0.015 mmol) S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[1-(2,5-dioxo-2,5-dihydro-1H-pyrrol-1-yl)-2,18-dioxo-6,9,12,15-tetraoxa-3-azaoctadecan-18-yl]-L-cystein -trifluoressigsäure (1:1) (Intermediat F257) und 8.18 mg (0.031 mmol) N-{[2-(Trimethylsilyl) ethoxy]carbonyl}-L-cystein wurden in 2 ml DMF gelöst und der Ansatz 18 h bei RT gerührt. Die Reaktionsmischung wurde im Vakuum verdampft. Der Rückstand (28.9 mg) wurde in 3 mL THF/Wasser 1:1 gelöst. Es wurden 0.046 mL einer 2M wässrigen Lithiumhydroxidlösung zugegeben und der Ansatz 3 Stunde bei RT gerührt. Anschließend wurde der Ansatz mit 5.2 µl (0.092 mmol) Essigsäure auf einen pH-Wert von ~7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 12.1 mg (58% über 2 Stufen) der regioisomeren geschützten Intermediate erhalten.

LC-MS (Methode 12): Rₜ = 1.82 min; MS (ESIpos): m/z = 1240 (M+H)⁺.

Im letzten Schritt wurden 12,1 mg (0.009 mmol) von diesem Intermediat in 2 mL 2,2,2-Trifluorethanol gelöst. Es wurden 7.3 mg (0.054 mmol) Zinkchlorid zugegeben und der Ansatz 2 h bei 50°C gerührt. Anschließend wurde 15.7 mg (0.054 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 6.4 mg (59%) der Titelverbindung als Regioisomerengemisch erhalten.

LC-MS (Methode 1): Rₜ = 0.86 min; MS (ESIpos): m/z = 1096 (M+H)⁺.

### Beispiel M23

### N-{(2S)-2-Amino-4-[{(1R)-1-[1-benzyl-4-(2,5-difluorphenyl)-1H-pyrrol-2-yl]-2,2-dimethyl propyl}(glycoloyl)amino]butanoyl}-beta-alanyl-L-glutaminsäure -trifluoressigsäure (1:1)

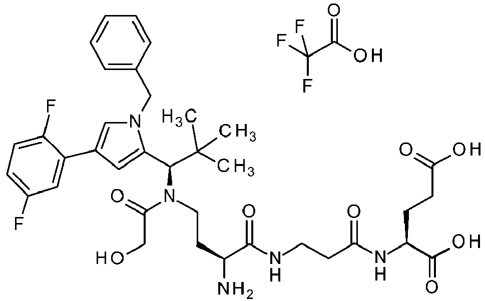

Zunächst wurde Di-tert-butyl L-glutamate hydrochloride (1:1) in Gegenwart von HATU und *N,N-*Diisopropylethylamin mit Intermediat C61 gekuppelt. Anschließend wurde das geschützte Intermediat in Trifluorethanol aufgenommen und durch Rühren über Nacht bei 50°C in Gegenwart von Zinkchlorid vollständig entschützt. Die Aufarbeitung erfolgte nach Zugabe von EDTA durch Reinigung mittels präparativer HPLC.

LC-MS (Methode 12): Rₜ = 1.45 min; MS (ESIpos): m/z = 714 [M+H]⁺.

### Beispiel M24

### N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-D-glutaminsäure -trifluoressigsäure (1:1)

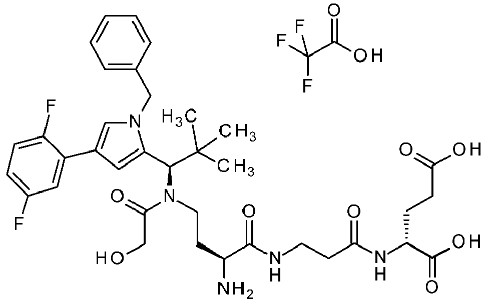

Zunächst wurde Di-tert-butyl D-glutamat Hydrochlorid (1:1) in Gegenwart von HATU und *N,N-*Diisopropylethylamin mit Intermediat C61 gekuppelt. Anschließend wurde das geschützte Intermediat in Trifluorethanol aufgenommen und durch Rühren bei 50°C in Gegenwart von Zinkchlorid vollständig entschützt. Die Aufarbeitung erfolgte nach Zugabe von EDTA durch Reinigung mittels präparativer HPLC.

LC-MS (Methode 12): Rₜ = 1.41 min; MS (ESIpos): m/z = 714 [M+H]⁺.

### Beispiel M25

### N-{(2S)-2-Amino-4-[{(1R)-1-[1-benzyl-4-(2,5-difluorphenyl)-1H-pyrrol-2-yl]-2,2-dimethyl propyl}(glycoloyl)amino]butanoyl}-L-glutaminsäure -trifluoressigsäure (1:1)

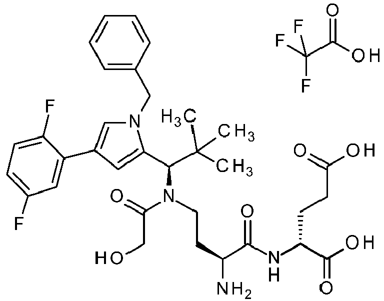

Zunächst wurde Di-tert-butyl L-glutamat Hydrochloride (1:1) in Gegenwart von HATU und *N,N-*Diisopropylethylamin mit Intermediat C61 gekuppelt. Im nächsten Schritt wurde die Z-Schutzgruppe durch 45-minütige Hydrierung über 10%-igem Palladium auf Aktivkohle in Methanol bei RT unter Wasserstoff-Normaldruck entfernt. Anschließend wurde das partiell geschützte Intermediat in Trifluorethanol aufgenommen und durch 7 stündiges Rühren bei 50°C in Gegenwart von Zinkchlorid vollständig entschützt. Die Aufarbeitung erfolgte nach Zugabe von EDTA durch Reinigung mittels präparativer HPLC.

LC-MS (Methode 12): Rₜ = 1.44 min; MS (ESIpos): m/z = 643 [M+H]⁺.

### Beispiel M26

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)ethyl] amino } -2-oxoethyl)amino] -2- { [(2R)-2-amino-2-carboxyethyl]sulfanyl}-4-oxobutansäure -trifluoressigsäure (1:1) Regioisomer 1, Epimerengemisch

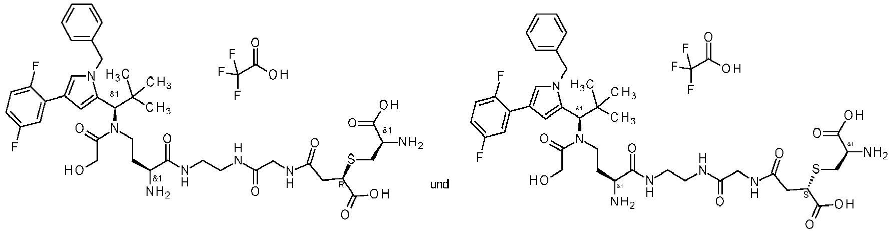

Dieses Beipiel beschreibt das Epimerengemisch der Verbindungen aus Beispiel 13 und Beispiel 14. Die Synthese erfolgte in Analogie zu Beispiel 13, wobei auf die Auftrennung der beiden Epimere durch überkritische Fluid-Chromatograpie verzichtet wurde und die Titelverbindung als Epimerengemisch hergestellt wurde.

LC-MS (Methode 5): Rₜ = 2.43 min; MS (ESIpos): m/z = 832 [M+H]⁺.

### Beispiel M27

### 4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure -trifluoressigsäure (1:1) Regioisomer 2, Epimerengemisch

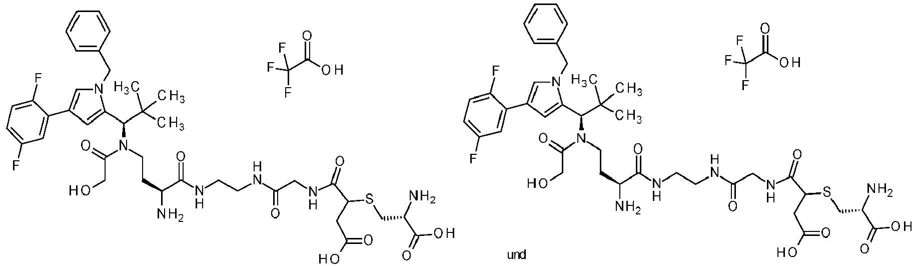

Dieses Beipiel beschreibt das Epimerengemisch der Verbindungen aus Beispiel 15 und Beispiel 16. Die Synthese erfolgte in Analogie zu Beispiel 15, wobei auf die Auftrennung der beiden Epimere durch überkritische Fluid-Chromatograpie verzichtet wurde und die Titelverbindung als Epimerengemisch hergestellt wurde.

LC-MS (Methode 5): Rₜ = 2.45 min; MS (EIpos): m/z = 832 [M+H]⁺.

### Ausführungsbeispiele APDCs und ADCs

Die in den Strukturformen der Ausführungsbeispiele dargestellten APDCs und ADCs, die über Maleinimid-Reste an Cysteinseitenketten der Antikörper gekuppelt wurden, liegen in Abhängigkeit vom Linker und von dem Kupplungsprotokoll zum überwiegenden Teil in den jeweils dargestellten ring-geöffneten bzw. ring-geschlossenen Formen vor. Zu einem kleinen Anteil kann jedoch die jeweils andere Form in der Präparation enthalten sein.

Die Kupplungsreaktionen wurden unter Argon durchgeführt.

### Beispiel 1a

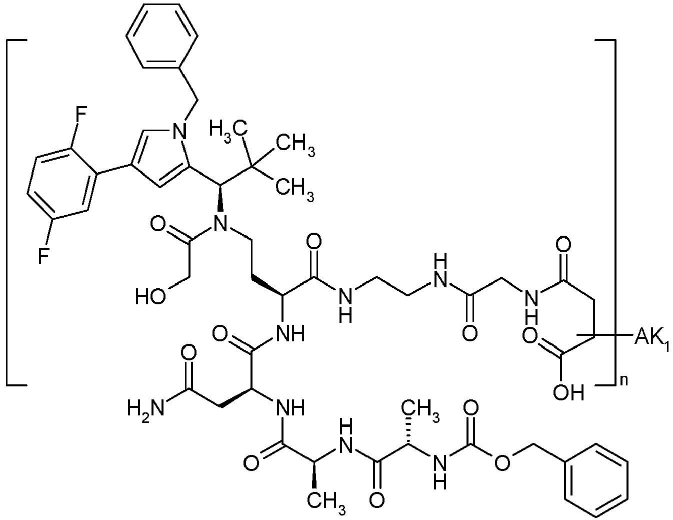

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat Q1 gelöst in 50 µl DMSO zugegeben. Nach weiteren 90 min Rühren bei RT wurde der Ansatz mit 1.95 ml PBS-Puffer, der zuvor auf pH 8 eingestellt wurde, verdünnt und über Nacht bei RT unter Argon gerührt.

Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert.

Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.46 mg/mL
Drug/mAb Ratio: 2.7

### Beispiel 1e

In analoger Weise wurde Intermediat Q1 mit 5 mg Trastuzumab gekuppelt. Der erhaltene ADC Batch wurde folgendermaßen charakterisiert:
Protein Konzentration: 1.61 mg/mL
Drug/mAb Ratio: 3.3

### Beispiel 1i

In analoger Weise wurde Intermediat Q1 mit 5 mg Nimotuzumab gekuppelt. Der erhaltene ADC Batch wurde folgendermaßen charakterisiert:
Protein Konzentration: 1.75 mg/mL
Drug/mAb Ratio: 3.1

### Beispiel 1k

In analoger Weise wurde Intermediat Q1 mit 5 mg anti-TWEAKR Antikörper TPP-2658 gekuppelt. Der erhaltene ADC Batch wurde folgendermaßen charakterisiert:
Protein Konzentration: 1.26 mg/mL
Drug/mAb Ratio: 3.0

### Beispiel 1k*

In analoger Weise wurde Intermediat Q1 mit 50 mg anti-TWEAKR Antikörper TPP-2658 gekuppelt.

50 mg anti-TWEAKR Antikörper TPP-2658 in 3.07 ml PBS (c=16.3 mg/mL) wurden unter Argon mit einer Lösung aus 0.29 mg TCEP in 0.682 ml PBS-Puffer versetzt. Der Ansatz wurde 30min bei RT gerührt und anschließend wurden 2.53 mg (0.0023 mmol) von Intermediat Q1 gelöst in 357 µl DMSO zugegeben. Nach weiteren 90 min Rühren bei RT wurde der Ansatz mit 3.391 ml PBS-Puffer, der zuvor auf pH 8 eingestellt wurde, auf 7.5 ml verdünnt und dann über eine mit PBS-Puffer pH 8 equillibrierte PD 10-Säule (Sephadex^{®} G-25, GE Healthcare) gegeben und mit PBS-Puffer pH 8 eluiert. Das Eluat wurde über Nacht bei RT unter Argon gerührt. Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert. Anschließend wurde durch Ultrazentrifugation aufkonzentriert und rückverdünnt mit PBS-Puffer (pH 7.2) und erneut aufkonzentriert. Der erhaltene ADC Batch wurde folgendermaßen charakterisiert:
Protein Konzentration: 9.56 mg/mL
Drug/mAb Ratio: 3.3

### Beispiel 2a

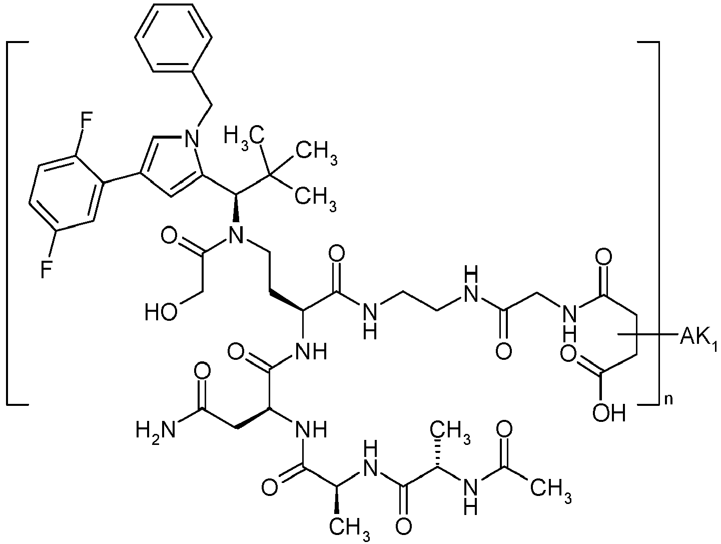

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat Q2 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt und über Nacht bei RT unter Argon gerührt.

Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert.

Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.87 mg/mL
Drug/mAb Ratio: 3.2

### Beispiel 2e

In analoger Weise wurde Intermediat Q2 mit 5 mg Trastuzumab gekuppelt. Der erhaltene ADC Batch wurde folgendermaßen charakterisiert:
Protein Konzentration: 1.41 mg/mL
Drug/mAb Ratio: 3.3

### Beispiel 2k

In analoger Weise wurde Intermediat Q2 mit 5 mg anti-TWEAKR Antikörper TPP-2658 gekuppelt. Der erhaltene ADC Batch wurde folgendermaßen charakterisiert:
Protein Konzentration: 1.79 mg/mL
Drug/mAb Ratio: 3.3

### Beispiel 2k*

In analoger Weise zu Beispiel 1k* wurden 2.775 mg (0.0028 mmol) von Intermediat Q2 mit 60 mg anti-TWEAKR Antikörper TPP-2658 gekuppelt. Der erhaltene ADC Batch wurde folgendermaßen charakterisiert:
Protein Konzentration:9.97 mg/mL
Drug/mAb Ratio: 3.7

### Beispiel 3k

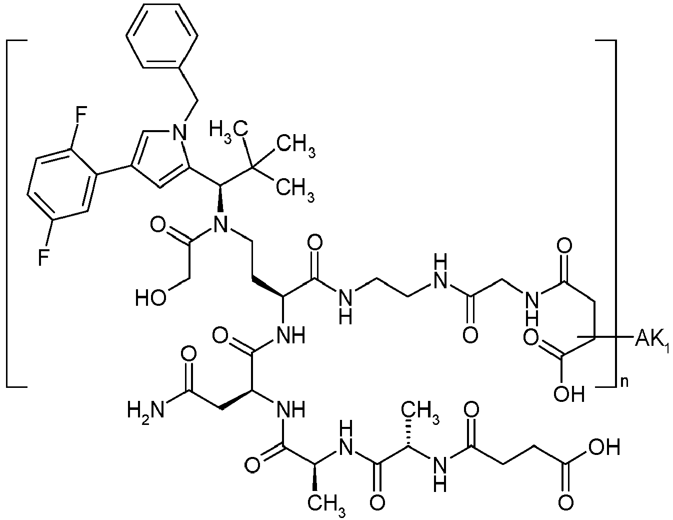

5 mg anti-TWEAKR Antikörper TPP-2658 in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat Q3 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt und über Nacht bei RT unter Argon gerührt. Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert.

Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.15 mg/mL
Drug/mAb Ratio: 4.3

### Beispiel 4a

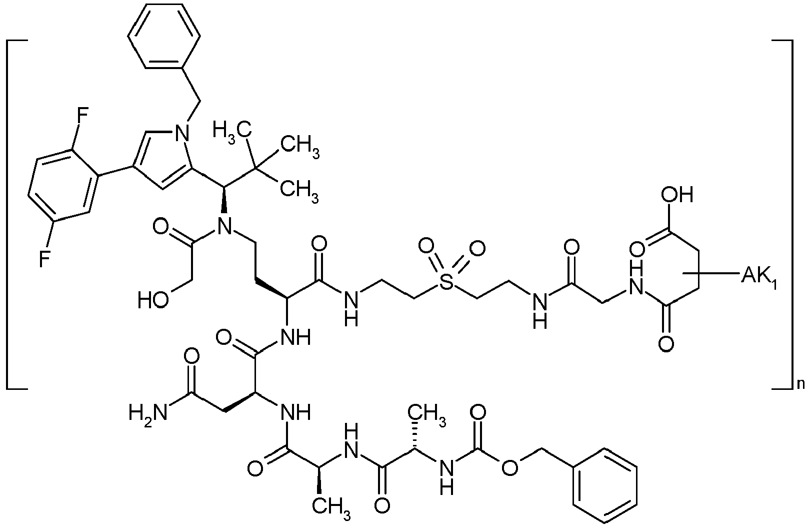

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat Q4 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.98 mg/mL
Drug/mAb Ratio: 2.0

### Beispiel 4k

In analoger Weise wurde Intermediat Q4 mit 5 mg anti-TWEAKR Antikörper TPP-2658 gekuppelt. Der erhaltene ADC Batch wurde folgendermaßen charakterisiert:
Protein Konzentration: 1.65 mg/mL
Drug/mAb Ratio: 3.3

### Beispiel 5a

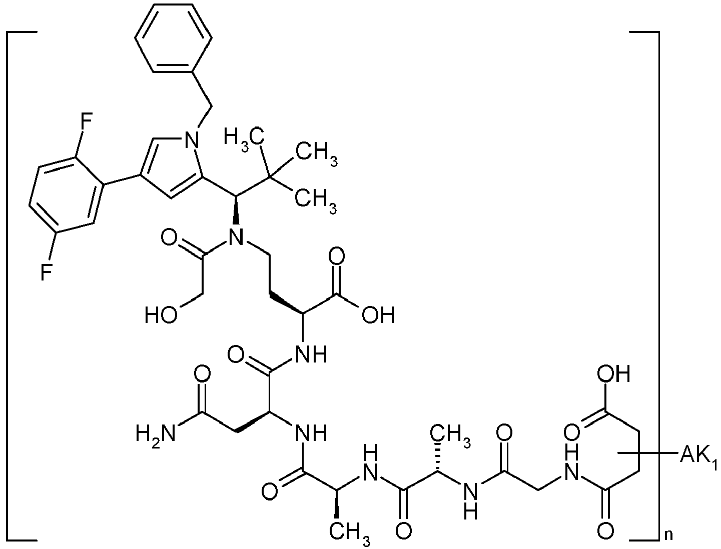

5 mg Cetuximab in 0.458 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.21 mg (0.00023 mmol) von Intermediat Q5 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.91 mg/mL
Drug/mAb Ratio: 2.4

### Beispiel 5e

In analoger Weise wurde Intermediat Q5 mit 5 mg Trastuzumab gekuppelt. Der erhaltene ADC Batch wurde folgendermaßen charakterisiert:
Protein Konzentration: 1.55 mg/mL
Drug/mAb Ratio: 3.3

### Beispiel 5k

In analoger Weise wurde Intermediat Q5 mit 5 mg anti-TWEAKR Antikörper TPP-2658 gekuppelt. Der erhaltene ADC Batch wurde folgendermaßen charakterisiert:
Protein Konzentration: 1.54 mg/mL
Drug/mAb Ratio: 3.5

### Beispiel 6a

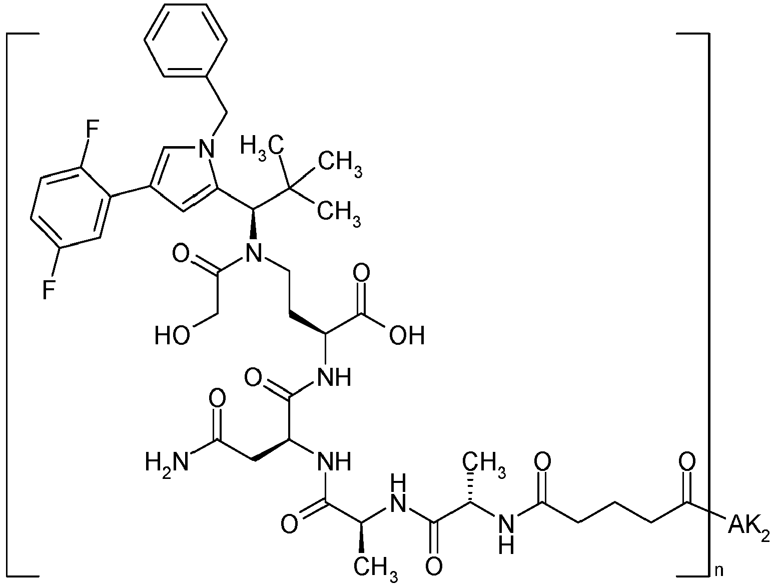

Eingesetzt wurden hier zur Kupplung mit Intermediat Q6 5 mg Cetuximab in PBS (c = 10 mg/mL). Zunächst wurden 5 Eq (0.24mg) von Intermediat Q6 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).
Protein Konzentration: 2.26 mg/mL
Drug/mAb Ratio: 3.7

### Beispiel 6e

In analoger Weise wurde Intermediat Q6 mit 5 mg Trastuzumab gekuppelt. Der erhaltene ADC Batch wurde folgendermaßen charakterisiert:
Protein Konzentration: 1.97 mg/mL
Drug/mAb Ratio: 5.4

### Beispiel 6k

In analoger Weise wurde Intermediat Q6 mit 5 mg anti-TWEAKR Antikörper TPP-2658 gekuppelt. Der erhaltene ADC Batch wurde folgendermaßen charakterisiert:
Protein Konzentration: 2.07 mg/mL
Drug/mAb Ratio: 6.7

### Beispiel 7a

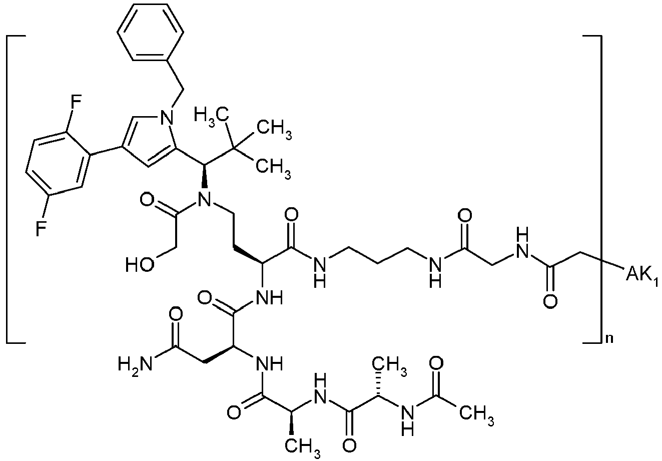

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt. Anschließend wurden 0.24 mg (0.00023 mmol) von Intermediat Q7 gelöst in 50 µl DMSO zugegeben und der Ansatz über Nacht bei RT gerührt. Dann wurde der Ansatz mit PBS Puffer (pH7.2) auf 2.5 mL verdünnt, über eine Sephadex-Säule gereinigt und anschließend durch Ultrazentrifugation aufkonzentriert und rückverdünnt mit PBS (pH7.2).
Protein Konzentration: 1.91 mg/mL
Drug/mAb Ratio: 1.6

### Beispiel 7k

In analoger Weise wurde Intermediat Q7 mit 5 mg anti-TWEAKR Antikörper TPP-2658 gekuppelt. Der erhaltene ADC Batch wurde folgendermaßen charakterisiert:
Protein Konzentration: 1.66 mg/mL
Drug/mAb Ratio: 2.4

### Beispiel 8a

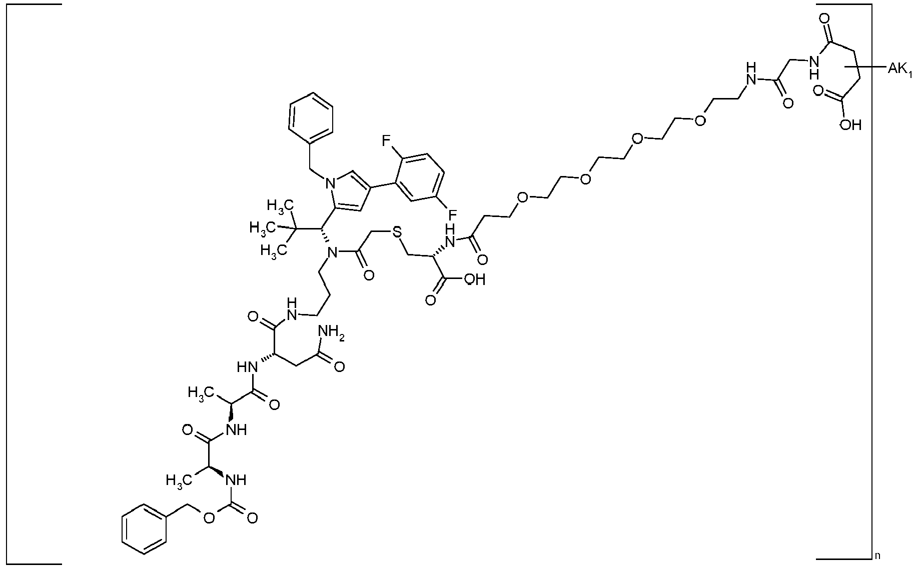

5 mg Cetuximab in 458 µL ml PBS (c= 10,9 mg/mL) wurden unter Argon mit einer Lösung aus 0.029 mg TCEP in 50µl PBS-Puffer versetzt. Der Ansatz wurde mit 1892 µl PBS-Puffer, der zuvor auf pH 8 eingestellt wurde, verdünnt und 1 h bei RT gerührt. Anschließend wurden 0.314 mg (0.00023 mmol) von Intermediat Q8 gelöst in 100µl DMSO zugegeben. Nach weiteren 90 min Rühren bei RT wurde der Ansatz über mit PBS-Puffer pH8 equillibrierte PD 10-Säulen (Sephadex^{®} G-25, GE Healthcare) gegeben und mit PBS-Puffer pH8 eluiert. Das Eluat über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der Ring-geschlossenen Form vorliegen. Der erhaltene ADC Batch wurde folgendermaßen charakterisiert:
Protein Konzentration: 1.15 mg/mL
Drug/mAb Ratio: 2.1

### Beispiel 8e

In analoger Weise wurde Intermediat Q8 mit 5 mg Trastuzumab gekuppelt. Der erhaltene ADC Batch wurde folgendermaßen charakterisiert:
Protein Konzentration: 1.21 mg/mL
Drug/mAb Ratio: 1.9

### Beispiel 8k

In analoger Weise wurde Intermediat Q8 mit 5 mg anti-TWEAKR Antikörper TPP-2658 gekuppelt. Der erhaltene ADC Batch wurde folgendermaßen charakterisiert:
Protein Konzentration: 0.65 mg/mL
Drug/mAb Ratio: 2.5

### Beispiel 9k

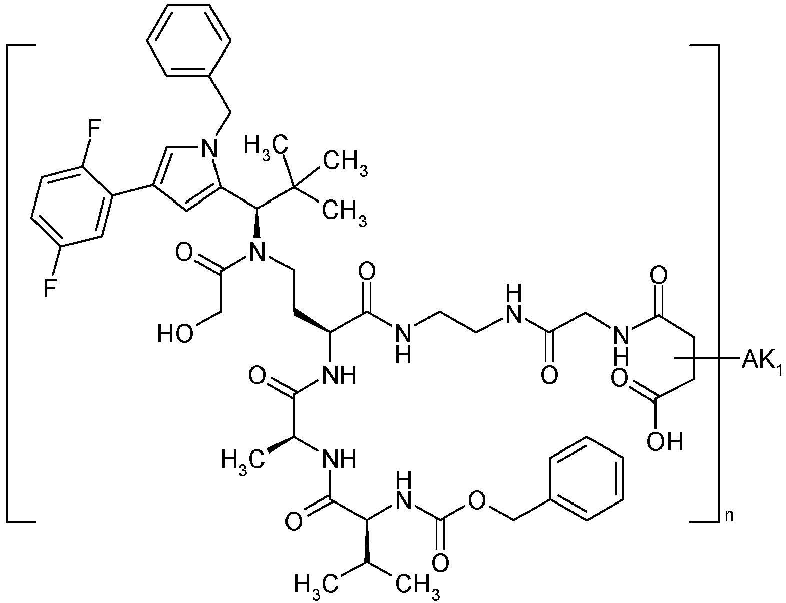

5 mg anti-TWEAKR Antikörper TPP-2658 in 0.450 ml PBS (c=11.1 mg/mL) wurden unter Argon mit einer Lösung aus 0.029 mg TCEP in 0.05 ml PBS-Puffer versetzt. Der Ansatz wurde 30min bei RT gerührt und anschließend wurden 0.24 mg (0.00023 mmol) von Intermediat Q9 gelöst in 50 µl DMSO zugegeben. Nach weiteren 90 min Rühren bei RT wurde der Ansatz mit 1.95 ml PBS-Puffer, der zuvor auf pH 8 eingestellt wurde, verdünnt und dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT gerührt und anschließend durch Ultrazentrifugation aufkonzentriert und rückverdünnt mit PBS-Puffer (pH 7.2). Der erhaltene ADC Batch wurde folgendermaßen charakterisiert:
Protein Konzentration: 0.86 mg/mL
Drug/mAb Ratio: 3.1

### Beispiel 10a

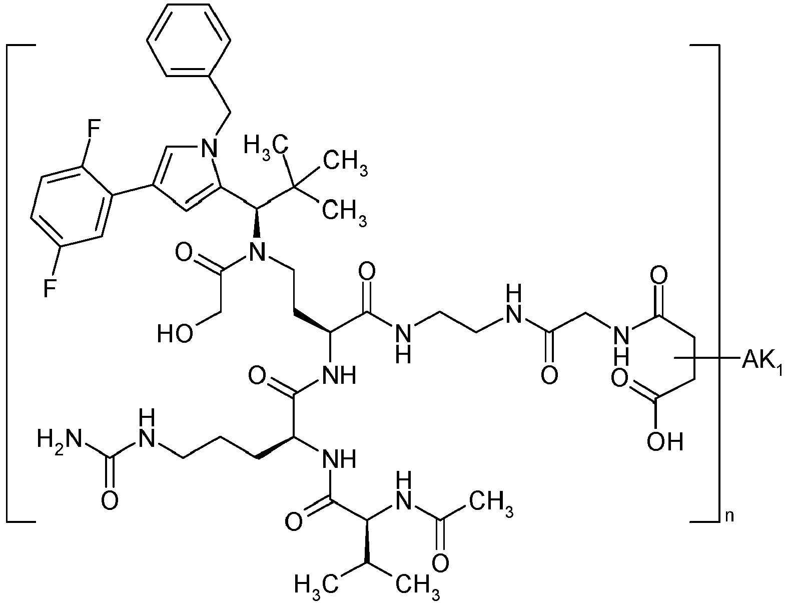

5 mg anti-TWEAKR Antikörper in 0.458 ml PBS (c=10.9 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat Q10 gelöst in 50 µl DMSO zugegeben. Nach weiteren 90 min Rühren bei RT wurde der Ansatz mit 1.95 ml PBS-Puffer, der zuvor auf pH 8 eingestellt wurde, verdünnt und dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT gerührt und anschließend durch Ultrazentrifugation aufkonzentriert und rückverdünnt mit PBS-Puffer (pH 7.2). Der erhaltene ADC Batch wurde folgendermaßen charakterisiert:
Protein Konzentration: 1.92 mg/mL
Drug/mAb Ratio: 2.9

### Beispiel 10k

In analoger Weise wurde Intermediat Q10 mit 5 mg anti-TWEAKR Antikörper TPP-2658 gekuppelt. Der erhaltene ADC Batch wurde folgendermaßen charakterisiert:
Protein Konzentration: 1.65 mg/mL
Drug/mAb Ratio: 3.3

### Beispiel 11a

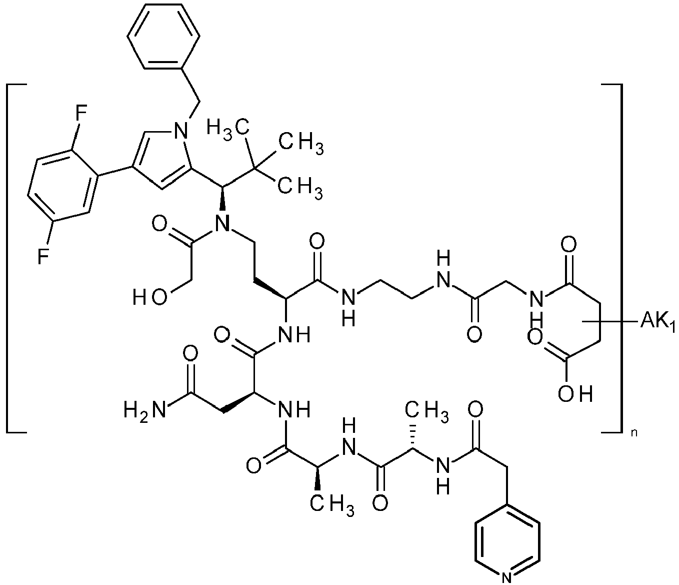

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.28 mg (0.00023 mmol) von Intermediat Q11 gelöst in 50 µl DMSO zugegeben. Nach weiteren 90 min Rühren bei RT wurde der Ansatz mit 1.942 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 1.99 mg/mL
Drug/mAb Ratio: 2.6

### Beispiel 11e

In analoger Weise wurde Intermediat Q11 mit 5 mg Trastuzumab gekuppelt. Der erhaltene ADC Batch wurde folgendermaßen charakterisiert:
Protein Konzentration: 1.70 mg/mL
Drug/mAb Ratio: 3.3

### Beispiel 11k

In analoger Weise wurde Intermediat Q11 mit 5 mg anti-TWEAKR Antikörper TPP-2658 gekuppelt. Der erhaltene ADC Batch wurde folgendermaßen charakterisiert:
Protein Konzentration: 1.71 mg/mL
Drug/mAb Ratio: 3.1

### Beispiel 12a

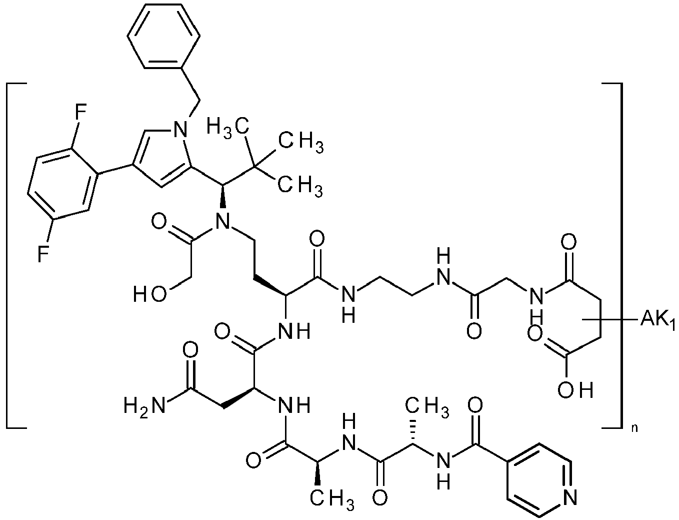

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.305 mg (0.00023 mmol) von Intermediat Q12 gelöst in 50 µl DMSO zugegeben. Nach weiteren 90 min Rühren bei RT wurde der Ansatz mit 1.942 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 2.23 mg/mL
Drug/mAb Ratio: 2.5

### Beispiel 12e

In analoger Weise wurde Intermediat Q12 mit 5 mg Trastuzumab gekuppelt. Der erhaltene ADC Batch wurde folgendermaßen charakterisiert:
Protein Konzentration: 1.73 mg/mL
Drug/mAb Ratio: 3.1

### Beispiel 12k

In analoger Weise wurde Intermediat Q12 mit 5 mg anti-TWEAKR Antikörper TPP-2658 gekuppelt. Der erhaltene ADC Batch wurde folgendermaßen charakterisiert:
Protein Konzentration: 1.91 mg/mL
Drug/mAb Ratio: 2.8

### Beispiel 13a

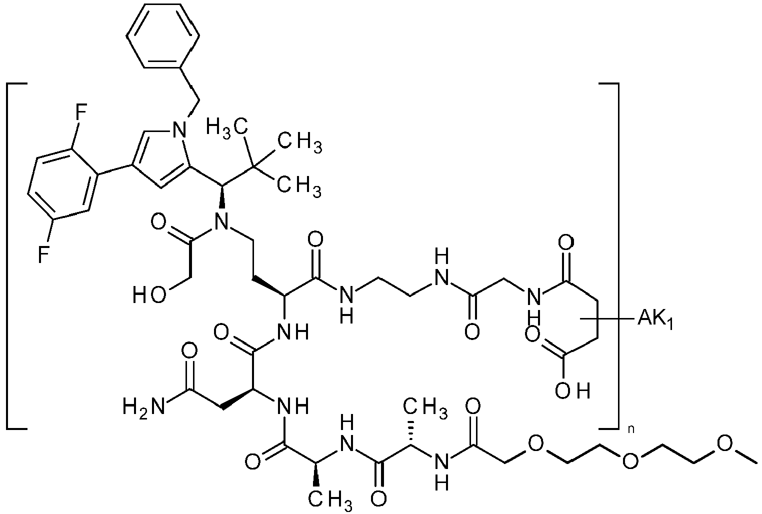

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q13 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.98 mg/mL
Drug/mAb Ratio: 2.1

### Beispiel 13e

In analoger Weise wurde Intermediat Q13 mit 5 mg Trastuzumab gekuppelt. Der erhaltene ADC Batch wurde folgendermaßen charakterisiert:
Protein Konzentration: 1.83 mg/mL
Drug/mAb Ratio: 3.1

### Beispiel 13k

In analoger Weise wurde Intermediat Q13 mit 5 mg anti-TWEAKR Antikörper TPP-2658 gekuppelt. Der erhaltene ADC Batch wurde folgendermaßen charakterisiert:
Protein Konzentration: 1.54 mg/mL
Drug/mAb Ratio: 3.2

### Beispiel 14a

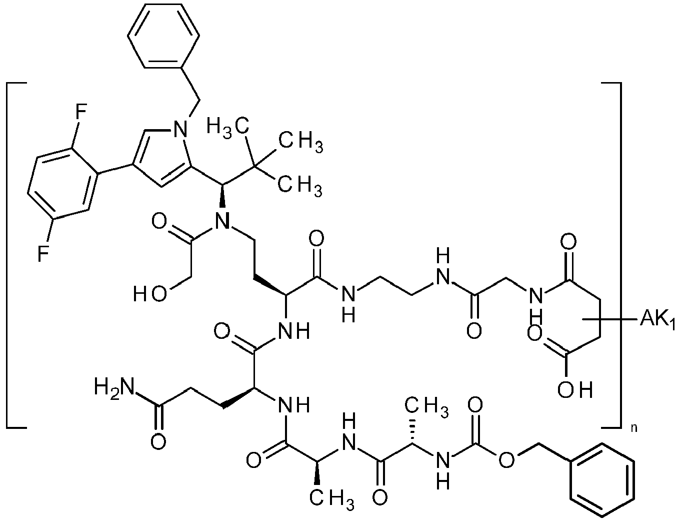

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.29 mg (0.00023 mmol) von Intermediat Q14 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.92 mg/mL
Drug/mAb Ratio: 2.9

### Beispiel 14e

In analoger Weise wurde Intermediat Q14 mit 5 mg Trastuzumab gekuppelt. Der erhaltene ADC Batch wurde folgendermaßen charakterisiert:
Protein Konzentration: 1.72 mg/mL
Drug/mAb Ratio: 3.4

### Beispiel 14k

In analoger Weise wurde Intermediat Q14 mit 5 mg anti-TWEAKR Antikörper TPP-2658 gekuppelt. Der erhaltene ADC Batch wurde folgendermaßen charakterisiert:
Protein Konzentration: 1.80 mg/mL
Drug/mAb Ratio: 2.9

### Beispiel 15a

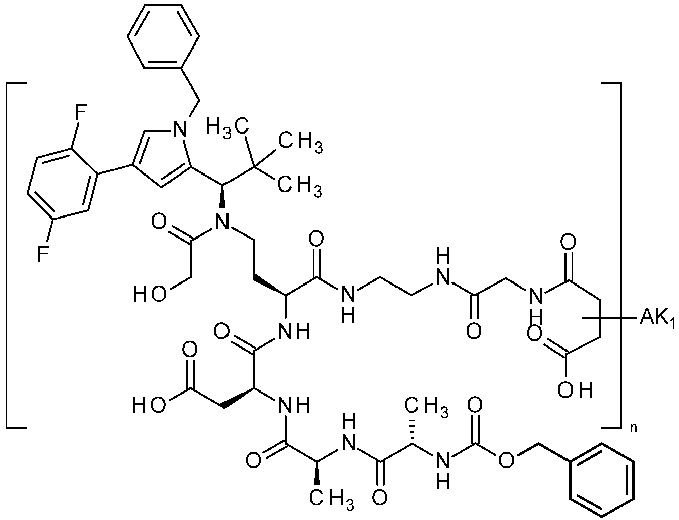

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat Q15 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.90 mg/mL
Drug/mAb Ratio: 2.8

### Beispiel 15k

In analoger Weise wurde Intermediat Q15 mit 5 mg anti-TWEAKR Antikörper TPP-2658 gekuppelt. Der erhaltene ADC Batch wurde folgendermaßen charakterisiert:
Protein Konzentration: 1.74 mg/mL
Drug/mAb Ratio: 2.9

### Beispiel 16a

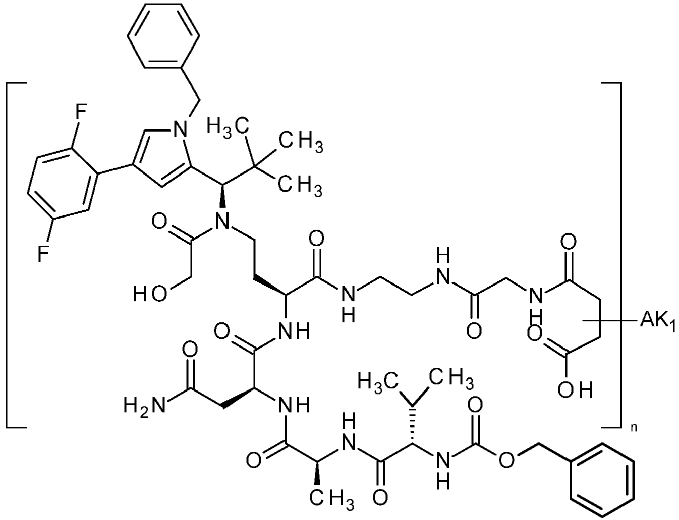

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q16 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.74 mg/mL
Drug/mAb Ratio: 2.7

### Beispiel 16e

In analoger Weise wurde Intermediat Q16 mit 5 mg Trastuzumab gekuppelt. Der erhaltene ADC Batch wurde folgendermaßen charakterisiert:
Protein Konzentration: 1.61 mg/mL
Drug/mAb Ratio: 3.3

### Beispiel 16k

In analoger Weise wurde Intermediat Q16 mit 5 mg anti-TWEAKR Antikörper TPP-2658 gekuppelt. Der erhaltene ADC Batch wurde folgendermaßen charakterisiert:
Protein Konzentration: 1.75 mg/mL
Drug/mAb Ratio: 3.2

### Beispiel 17a

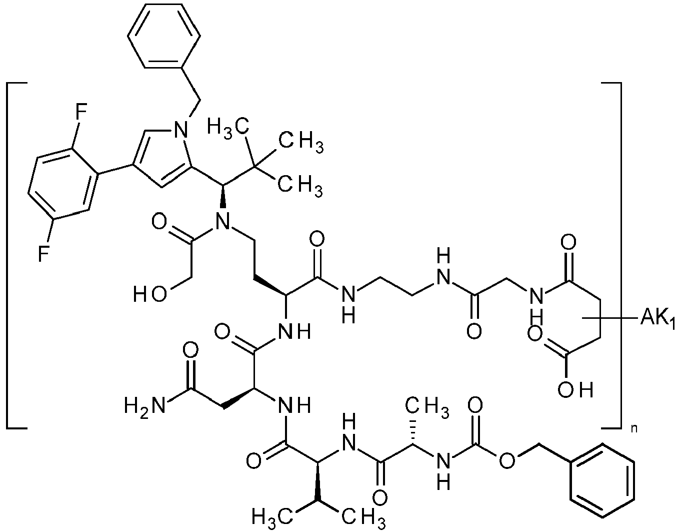

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q17 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.93 mg/mL
Drug/mAb Ratio: 2.8

### Beispiel 17e

In analoger Weise wurde Intermediat Q17 mit 5 mg Trastuzumab gekuppelt. Der erhaltene ADC Batch wurde folgendermaßen charakterisiert:
Protein Konzentration: 1.83 mg/mL
Drug/mAb Ratio: 2.9

### Beispiel 17k

In analoger Weise wurde Intermediat Q17 mit 5 mg anti-TWEAKR Antikörper TPP-2658 gekuppelt. Der erhaltene ADC Batch wurde folgendermaßen charakterisiert:
Protein Konzentration: 1.34 mg/mL
Drug/mAb Ratio: 2.8

### Beispiel 18a

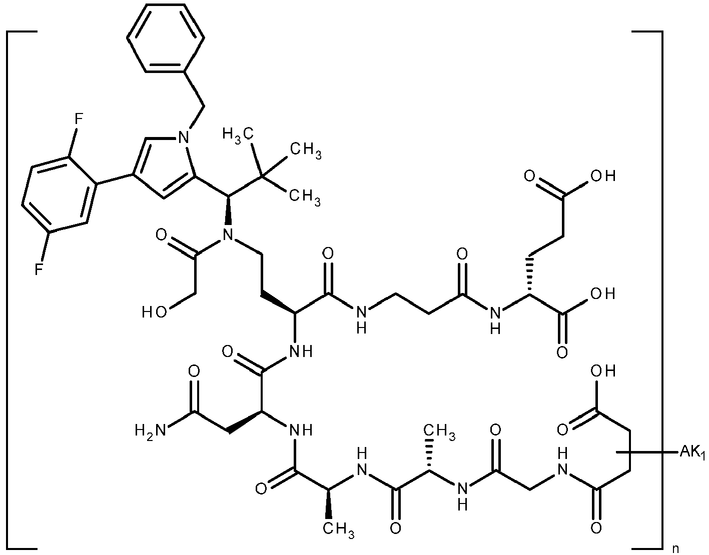

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q18 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.57 mg/mL
Drug/mAb Ratio: 2.4

### Beispiel 18e

In analoger Weise wurde Intermediat Q18 mit 5 mg Trastuzumab gekuppelt. Der erhaltene ADC Batch wurde folgendermaßen charakterisiert:
Protein Konzentration: 1.79 mg/mL
Drug/mAb Ratio: 3.1

### Beispiel 18k

In analoger Weise wurde Intermediat Q18 mit 5 mg anti-TWEAKR Antikörper TPP-2658 gekuppelt. Der erhaltene ADC Batch wurde folgendermaßen charakterisiert:
Protein Konzentration: 0.68 mg/mL
Drug/mAb Ratio: 2.8

### Beispiel 19a

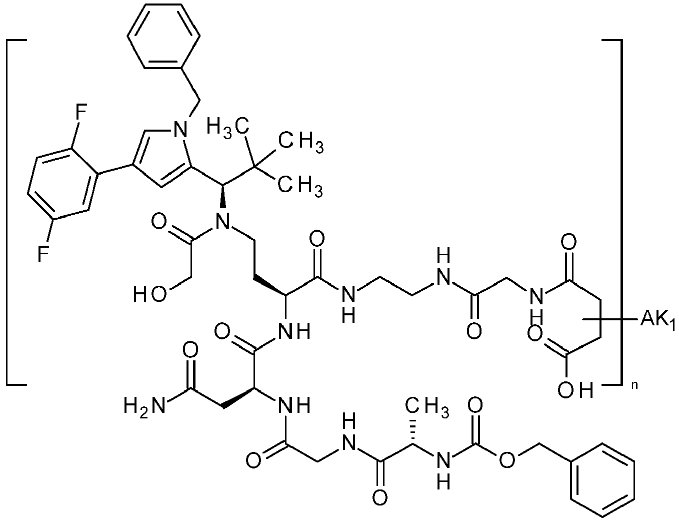

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.25 mg (0.00023 mmol) von Intermediat Q19 gelöst in 50 µl DMSO zugegeben. Nach weiteren 90 min Rühren bei RT wurde der Ansatz mit 1.95 ml PBS-Puffer, der zuvor auf pH 8 eingestellt wurde, verdünnt und über Nacht bei RT unter Argon gerührt.

Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert.

Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.85 mg/mL
Drug/mAb Ratio: 2.3

### Beispiel 19e

In analoger Weise wurde Intermediat Q19 mit 5 mg Trastuzumab gekuppelt. Der erhaltene ADC Batch wurde folgendermaßen charakterisiert:
Protein Konzentration: 1.88 mg/mL
Drug/mAb Ratio: 2.5

### Beispiel 19k

In analoger Weise wurde Intermediat Q19 mit 5 mg anti-TWEAKR Antikörper TPP-2658 gekuppelt. Der erhaltene ADC Batch wurde folgendermaßen charakterisiert:
Protein Konzentration: 1.80 mg/mL
Drug/mAb Ratio: 1.8

### Beispiel 20k

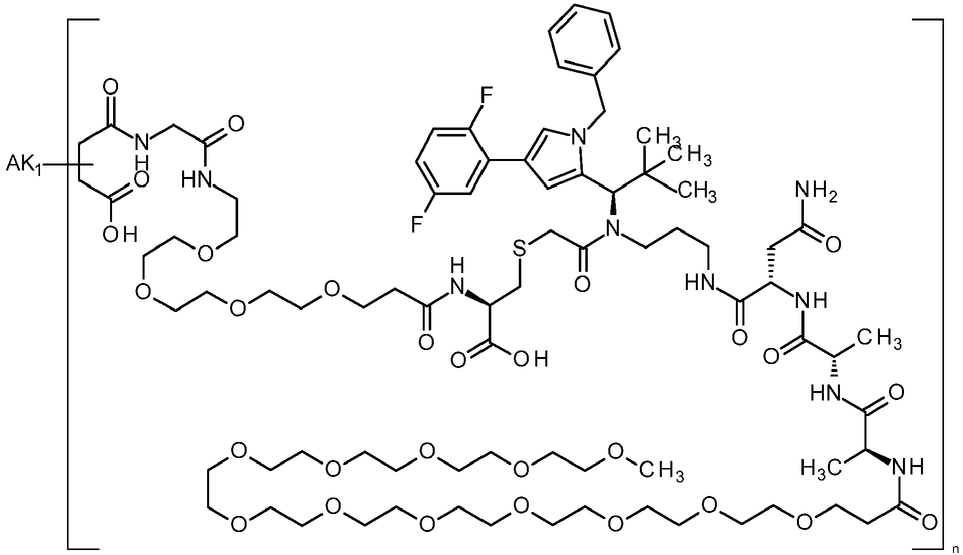

5 mg anti-TWEAKR Antikörper TPP-2658 in 0.239 ml PBS (c=20.90 mg/mL) wurden unter Argon mit einer Lösung aus 0.131 mg TCEP in 0.20 ml PBS-Puffer versetzt. Der Ansatz wurde mit 0,461 mL PBS gelöst, 30min bei RT gerührt und anschließend wurden 0.833 mg (0.00040 mmol) von Intermediat Q20 gelöst in 100 µl DMSO zugegeben. Nach weiteren 120 min Rühren bei RT wurde der Ansatz mit 1.50 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.96 mg/mL
Drug/mAb Ratio: 4.6

### Beispiel 20k*

5 mg anti-TWEAKR Antikörper TPP-2658 in 0.239 ml PBS (c=20.90 mg/mL) wurden unter Argon mit einer Lösung aus 0.076 mg TCEP in 0.05 ml PBS-Puffer versetzt. Der Ansatz wurde mit 0,20 mL PBS gelöst, 150min bei RT gerührt und anschließend wurden 0.833 mg (0.00040 mmol) von Intermediat Q20 gelöst in 50 µl DMSO zugegeben. Nach weiteren 120 min Rühren bei RT wurde der Ansatz mit 1.96 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 2.17 mg/mL
Drug/mAb Ratio: 7.3

### Beispiel 21a

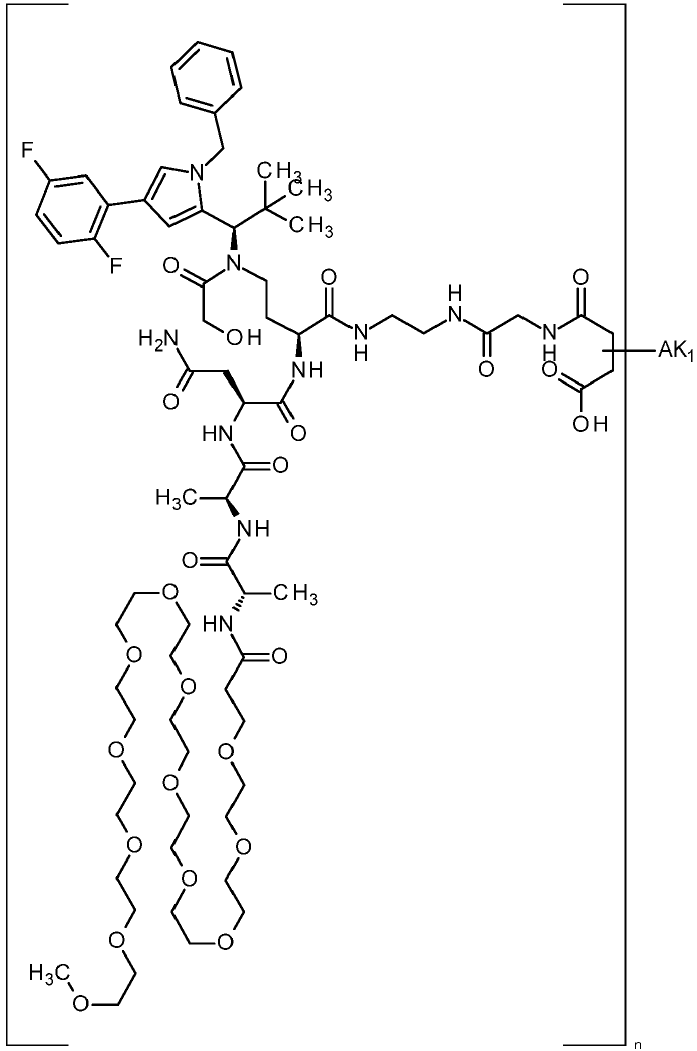

5 mg Cetuximab Antikörper in 0.427 ml PBS (c=11,7 mg/mL) wurden unter Argon mit einer Lösung aus 0.076 mg TCEP in 0.20 ml PBS-Puffer versetzt. Der Ansatz wurde 150 min bei RT gerührt und anschließend wurden 0.811 mg (0.000533 mmol) von Intermediat Q21 gelöst in 40 µl DMSO zugegeben. Nach weiteren 120 min Rühren bei RT wurde der Ansatz mit 1.98 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 2,37 mg/mL
Drug/mAb Ratio: 8,1

### Beispiel 21e

In analoger Weise wurde Intermediat Q21 mit 5 mg Trastuzumab gekuppelt. Der erhaltene ADC Batch wurde folgendermaßen charakterisiert:
Protein Konzentration: 2,27 mg/mL
Drug/mAb Ratio: 8,0

### Beispiel 21k

In analoger Weise wurde Intermediat Q21 mit 5 mg anti-TWEAKR Antikörper TPP-2658 gekuppelt. Der erhaltene ADC Batch wurde folgendermaßen charakterisiert:
Protein Konzentration: 2,73 mg/mL
Drug/mAb Ratio: 7,9

### Beispiel 22a

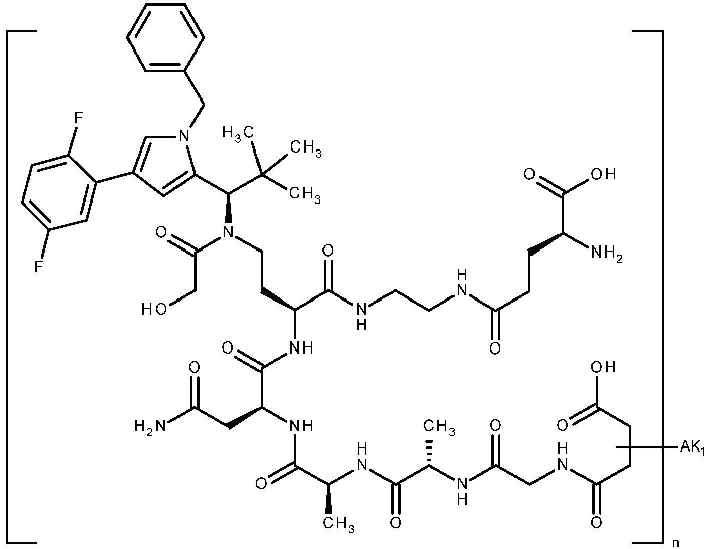

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.28 mg (0.00023 mmol) von Intermediat Q22 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.91 mg/mL
Drug/mAb Ratio: 2.2

### Beispiel 22e

In analoger Weise wurde Intermediat Q22 mit 5 mg Trastuzumab gekuppelt. Der erhaltene ADC Batch wurde folgendermaßen charakterisiert:
Protein Konzentration: 1.59 mg/mL
Drug/mAb Ratio: 1.7

### Beispiel 22k

In analoger Weise wurde Intermediat Q22 mit 5 mg anti-TWEAKR Antikörper TPP-2658 gekuppelt. Der erhaltene ADC Batch wurde folgendermaßen charakterisiert:
Protein Konzentration: 1.89 mg/mL
Drug/mAb Ratio: 2.8

### Beispiel 23a

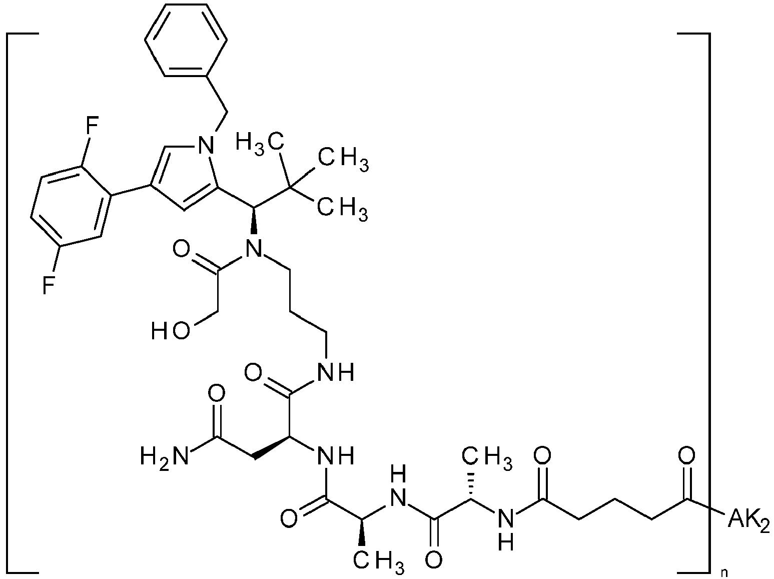

Eingesetzt wurden hier zur Kupplung mit Intermediat Q23 5 mg Cetuximab in PBS (c = 10 mg/mL). Zunächst wurden 5 Eq (0.2mg) von Intermediat Q23 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).
Protein Konzentration: 1.88 mg/mL
Drug/mAb Ratio: 2.6

### Beispiel 23e

In analoger Weise wurde Intermediat Q23 mit 5 mg Trastuzumab gekuppelt. Der erhaltene ADC Batch wurde folgendermaßen charakterisiert:
Protein Konzentration: 1.68 mg/mL
Drug/mAb Ratio: 3.7

### Beispiel 23k

In analoger Weise wurde Intermediat Q23 mit 5 mg anti-TWEAKR Antikörper TPP-2658 gekuppelt. Der erhaltene ADC Batch wurde folgendermaßen charakterisiert:
Protein Konzentration: 1.23 mg/mL
Drug/mAb Ratio: 3.9

### Beispiel 24a

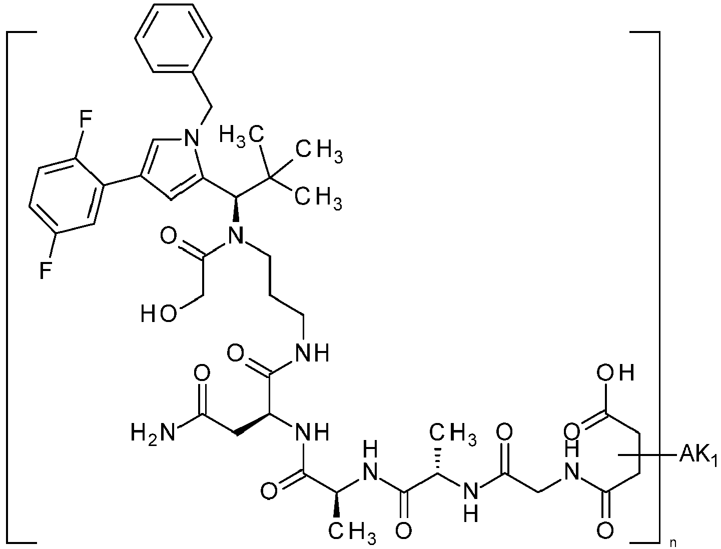

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat Q24 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.80 mg/mL
Drug/mAb Ratio: 2.2

### Beispiel 24e

In analoger Weise wurde Intermediat Q24 mit 5 mg Trastuzumab gekuppelt. Der erhaltene ADC Batch wurde folgendermaßen charakterisiert:
Protein Konzentration: 1.49 mg/mL
Drug/mAb Ratio: 2.3

### Beispiel 24k

In analoger Weise wurde Intermediat Q24 mit 5 mg anti-TWEAKR Antikörper TPP-2658 gekuppelt. Der erhaltene ADC Batch wurde folgendermaßen charakterisiert:
Protein Konzentration: 1.73 mg/mL
Drug/mAb Ratio: 2.2

### Beispiel 25a

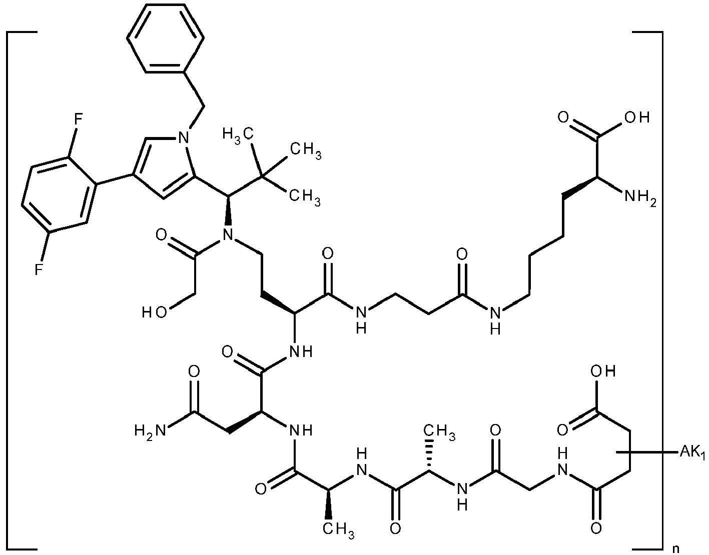

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.21 mg (0.00023 mmol) von Intermediat Q25 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.59 mg/mL
Drug/mAb Ratio: 2.9

### Beispiel 25e

In analoger Weise wurde Intermediat Q25 mit 5 mg Trastuzumab gekuppelt. Der erhaltene ADC Batch wurde folgendermaßen charakterisiert:
Protein Konzentration: 1.42 mg/mL
Drug/mAb Ratio: 3.5

### Beispiel 25k

In analoger Weise wurde Intermediat Q25 mit 5 mg anti-TWEAKR Antikörper TPP-2658 gekuppelt. Der erhaltene ADC Batch wurde folgendermaßen charakterisiert:
Protein Konzentration: 1.44 mg/mL
Drug/mAb Ratio: 3.2

### Beispiel 26a

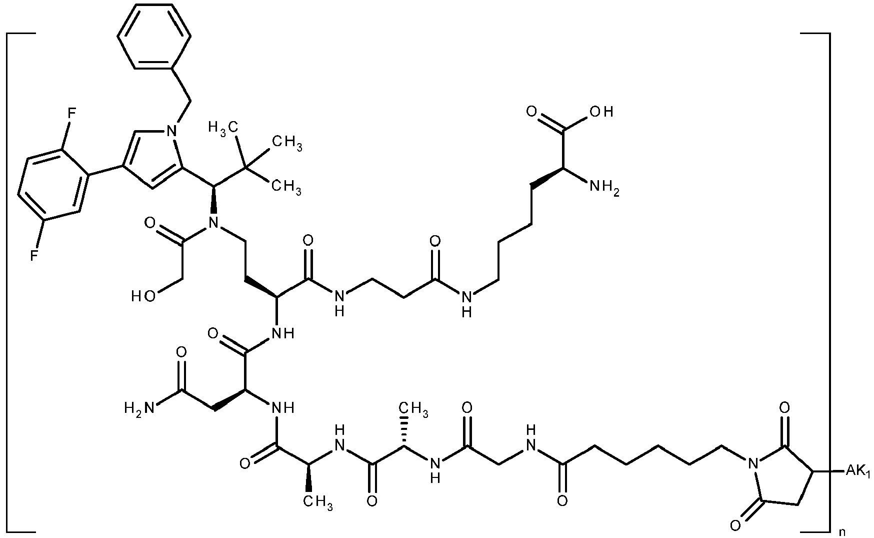

5 mg Cetuximab in 0.498 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.3 mg (0.00023 mmol) von Intermediat Q26 gelöst in 50 µl DMSO zugegeben. Nach weiteren 90 min Rühren bei RT wurde der Ansatz mit 1.9 ml PBS-Puffer verdünnt. Diese Lösung wurde dann über eine mit PBS-Puffer (pH 7.2) equillibrierte PD 10-Säule (Sephadex^{®} G-25, GE Healthcare) gegeben und mit PBS-Puffer (pH 7.2) eluiert. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 2.0 mg/mL
Drug/mAb Ratio: 2.9

### Beispiel 26e

In analoger Weise wurde Intermediat Q26 mit 5 mg Trastuzumab gekuppelt. Der erhaltene ADC Batch wurde folgendermaßen charakterisiert:
Protein Konzentration: 1.66 mg/mL
Drug/mAb Ratio: 2.9

### Beispiel 26k

In analoger Weise wurde Intermediat Q26 mit 5 mg anti-TWEAKR Antikörper TPP-2658 gekuppelt. Der erhaltene ADC Batch wurde folgendermaßen charakterisiert:
Protein Konzentration: 1.83 mg/mL
Drug/mAb Ratio: 3.4

### Beispiel 27a

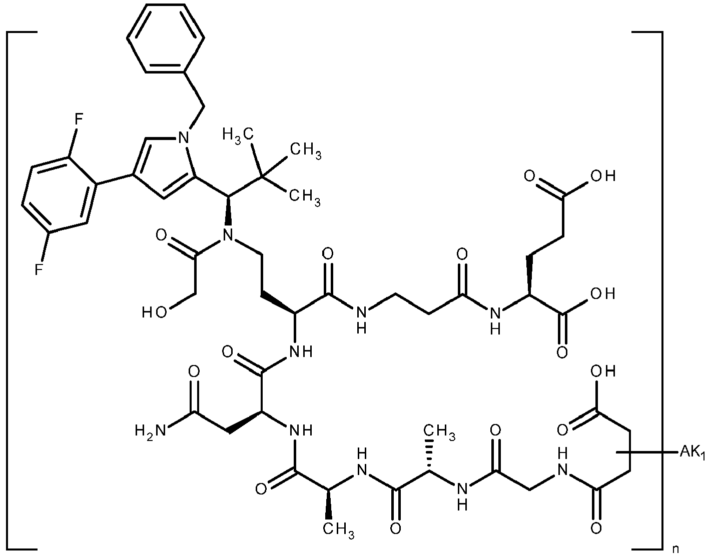

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q27 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 2.02 mg/mL
Drug/mAb Ratio: 3.5

### Beispiel 27e

In analoger Weise wurde Intermediat Q27 mit 5 mg Trastuzumab gekuppelt. Der erhaltene ADC Batch wurde folgendermaßen charakterisiert:
Protein Konzentration: 1.72 mg/mL
Drug/mAb Ratio: 4.2

### Beispiel 27k

In analoger Weise wurde Intermediat Q27 mit 5 mg anti-TWEAKR Antikörper TPP-2658 gekuppelt. Der erhaltene ADC Batch wurde folgendermaßen charakterisiert:
Protein Konzentration: 1.79 mg/mL
Drug/mAb Ratio: 3.1

### Beispiel 28a

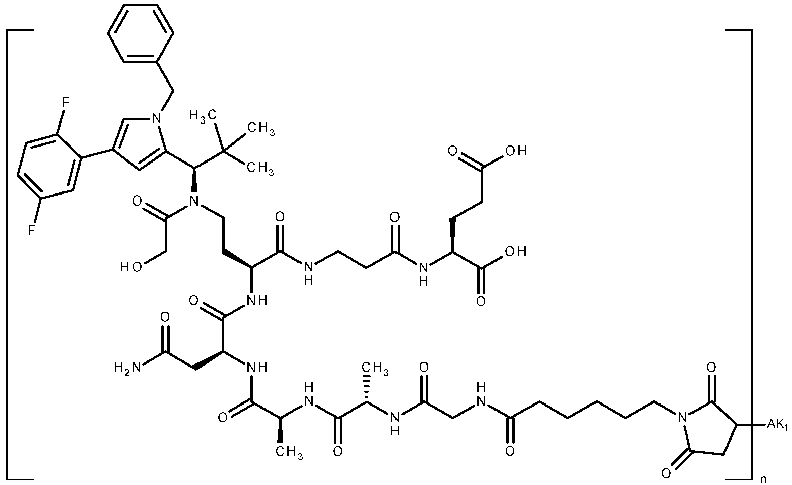

5 mg Cetuximab in 0.458 ml PBS (c=10.92 mg/mL) wurden unter Argon mit einer Lösung aus 0.029 mg TCEP in 0.05 ml PBS-Puffer versetzt. Der Ansatz wurde 30min bei RT gerührt und anschließend wurden 0.32 mg (0.00023 mmol) von Intermediat Q28 gelöst in 50 µl DMSO zugegeben. Nach weiteren 90 min Rühren bei RT wurde der Ansatz mit 1.9 ml PBS-Puffer verdünnt. Diese Lösung wurde dann über eine mit PBS-Puffer (pH 7.2) equillibrierte PD 10-Säule (Sephadex^{®} G-25, GE Healthcare) gegeben und mit PBS-Puffer (pH 7.2) eluiert. Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 2.01 mg/mL
Drug/mAb Ratio: 2.0

### Beispiel 28e

In analoger Weise wurde Intermediat Q28 mit 5 mg Trastuzumab gekuppelt. Der erhaltene ADC Batch wurde folgendermaßen charakterisiert:
Protein Konzentration: 1.86 mg/mL
Drug/mAb Ratio: 3.1

### Beispiel 28k

In analoger Weise wurde Intermediat Q28 mit 5 mg anti-TWEAKR Antikörper TPP-2658 gekuppelt. Der erhaltene ADC Batch wurde folgendermaßen charakterisiert:
Protein Konzentration: 2.03 mg/mL
Drug/mAb Ratio: 2.6

### Beispiel 29a

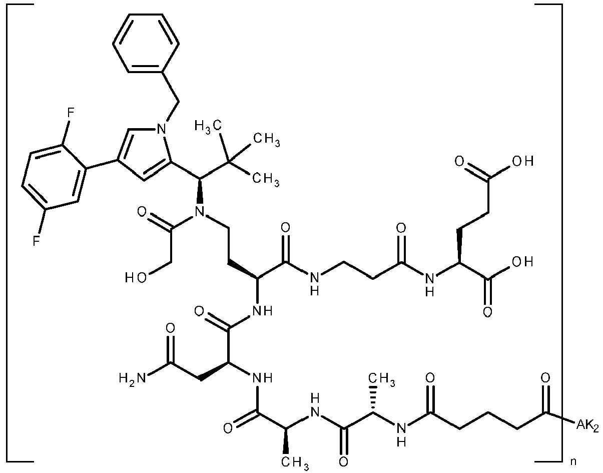

Eingesetzt wurden hier zur Kupplung mit Intermediat Q29 5 mg Cetuximab in PBS (c = 9.1 mg/mL). Zunächst wurden unter Argon 5 Eq (0.21 mg) von Intermediat Q29 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).
Protein Konzentration: 2.14 mg/mL
Drug/mAb Ratio: 2.6

### Beispiel 29e

In analoger Weise wurde Intermediat Q29 mit 5 mg Trastuzumab gekuppelt. Der erhaltene ADC Batch wurde folgendermaßen charakterisiert:
Protein Konzentration: 2.05 mg/mL
Drug/mAb Ratio: 3.8

### Beispiel 29k

In analoger Weise wurde Intermediat Q29 mit 5 mg anti-TWEAKR Antikörper TPP-2658 gekuppelt. Der erhaltene ADC Batch wurde folgendermaßen charakterisiert:
Protein Konzentration: 2.09 mg/mL
Drug/mAb Ratio: 3.9

### Beispiel 30a

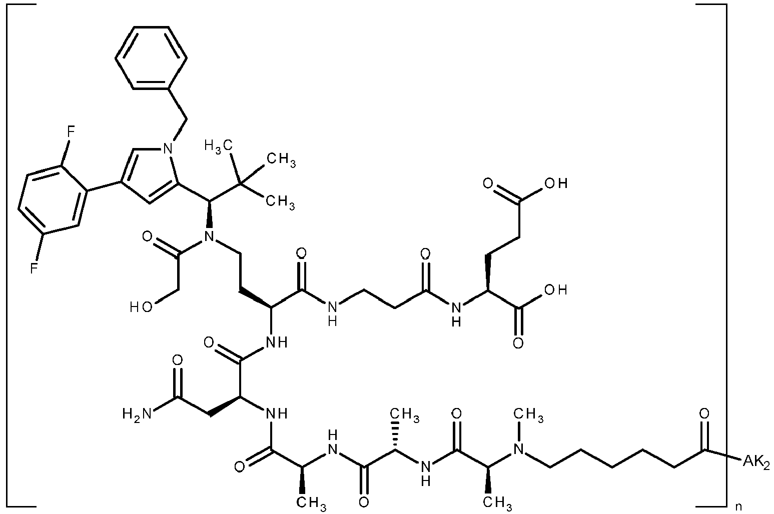

Eingesetzt wurden hier zur Kupplung mit Intermediat Q30 5 mg Cetuximab in PBS (c = 9.1 mg/mL). Zunächst wurden unter Argon 5 Eq (0.22 mg) von Intermediat Q29 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).
Protein Konzentration: 2.1 mg/mL
Drug/mAb Ratio: 4.5

### Beispiel 30e

In analoger Weise wurde Intermediat Q30 mit 5 mg Trastuzumab gekuppelt. Der erhaltene ADC Batch wurde folgendermaßen charakterisiert:
Protein Konzentration: 1.96 mg/mL
Drug/mAb Ratio: 5.6

### Beispiel 30k

In analoger Weise wurde Intermediat Q30 mit 5 mg anti-TWEAKR Antikörper TPP-2658 gekuppelt. Der erhaltene ADC Batch wurde folgendermaßen charakterisiert:
Protein Konzentration: 2.07 mg/mL
Drug/mAb Ratio: 5.8

### Beispiel 31t

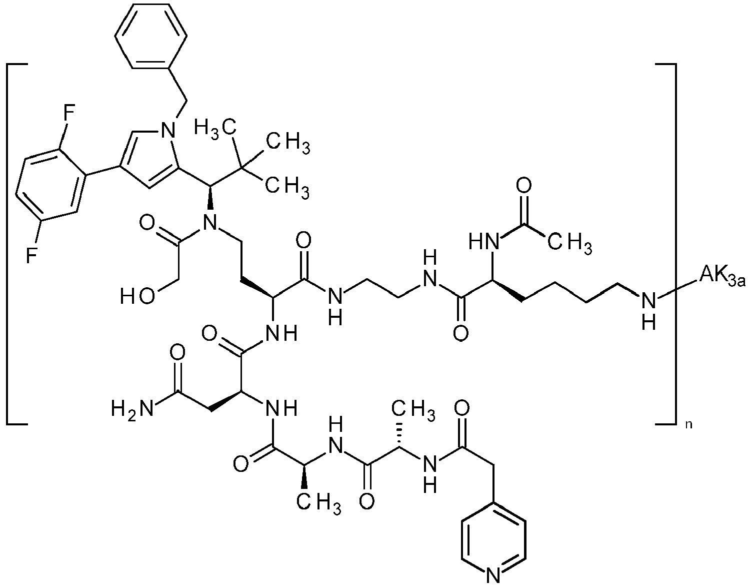

Zu einer Lösung von 5 mg des anti-TWEAKR Antikörpers TPP-2658 (entspricht TPP-2090-HC-N297A) in 530 µl DPBS pH 7.4 (c~10mg/mL) wurden 20 µL einer 10 mMol Lösung von Intermediat Q31 in DMSO hinzugegeben. Nach 5 min Inkubation bei 37°C wurden 50 µL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Volumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS. Schließlich wurden noch 0.00625 µmol des b-Transglutaminase Blockers Zedira C100 in 12.5 µL DPBS zu der Lösung hinzugefügt. Die erhaltene ADC Lösung wurde folgendermaßen charakterisiert:
Protein Konzentration: 2.11 mg/mL
Drug/mAb Ratio: 1.8

### Beispiel 31t-4

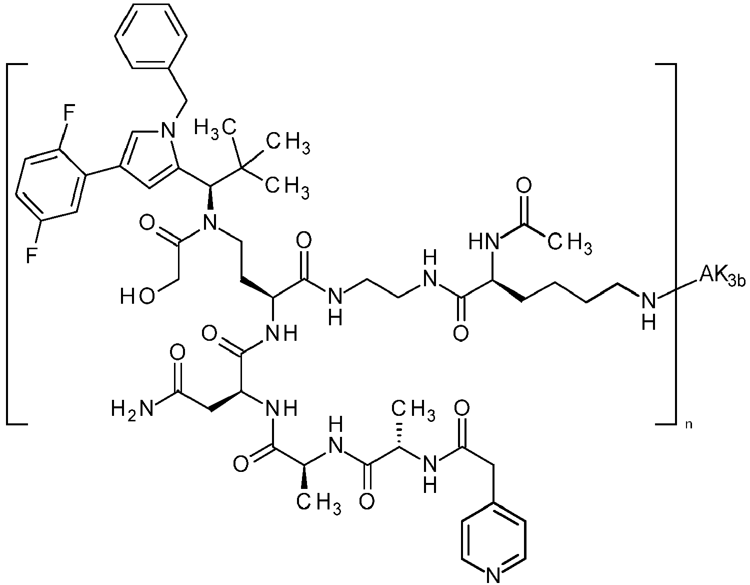

Zu einer Lösung von 30 mg des anti-TWEAKR Antikörpers TPP-5442 (entspricht TPP-2090-HC-N297Q) in DPBS pH 7.4 (c= 10 mg/mL) wurden 480 µL einer 10 mMol Lösung von Intermediat Q31 in DMSO hinzugegeben. Nach 5 min Inkubation bei 37°C wurden 2400 µL einer Lösung von rekombinanter bakterieller Transglutaminase Lösung in Wasser (Produkt Nummer T001 von Zedira GmbH, Darmstadt, Germany) (25 U/mL) hinzugegeben und 24 h bei 37°C inkubiert. Die Reaktionsmischung wurde über Gelfiltrationschromatographie auf einer Superdex 200 Säule (GE Healthcare) in DPBS pH 7.4 gereinigt, um kleine Moleküle und die Transglutaminase vom ADC abzutrennen. Anschließend wurde die ADC-Lösung über Amicon Ultracel-30K Zentrifugationsröhrchen (Millipore) auf die finale Konzentrationen von ca. 12 mg/mL ankonzentriert. Die Lösung wurde anschließend sterilfiltriert. Die erhaltene ADC Lösung wurde folgendermaßen charakterisiert:
Protein Konzentration: 12.0 mg/mL
Drug/mAb Ratio: 3.8

### Beispiel 32t

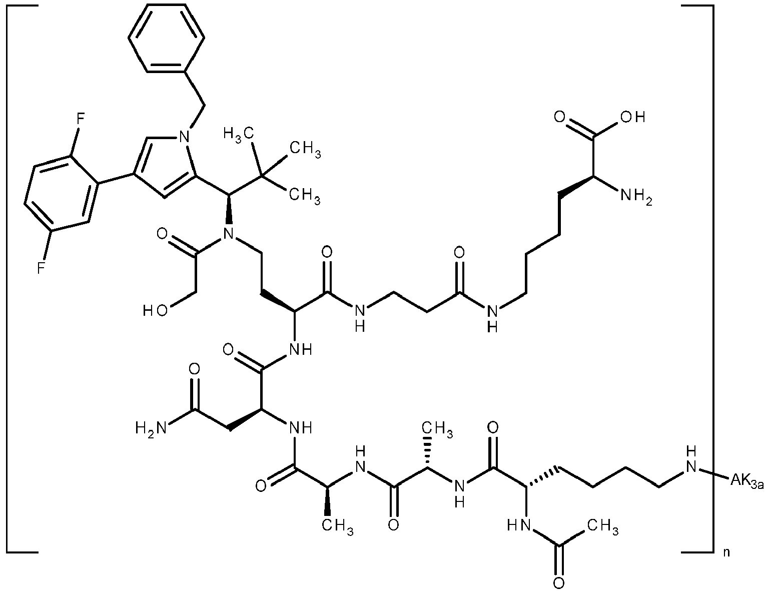

Zu einer Lösung von 5 mg des anti-TWEAKR Antikörpers TPP-2658 (entspricht TPP-2090-HC-N297A) in 530 µl DPBS pH 7.4 (c~10mg/mL) wurden 20 µL einer 10 mMol Lösung von Intermediat Q32 in DMSO hinzugegeben. Nach 5 min Inkubation bei 37°C wurden 50 µL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Volumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS. Schließlich wurden noch 0.00625 µmol des b-Transglutaminase Blockers Zedira C100 in 12.5 µL DPBS zu der Lösung hinzugefügt. Die erhaltene ADC Lösung wurde folgendermaßen charakterisiert:
Protein Konzentration: 1.8 mg/mL
Drug/mAb Ratio: 2.0

### Beispiel 33t

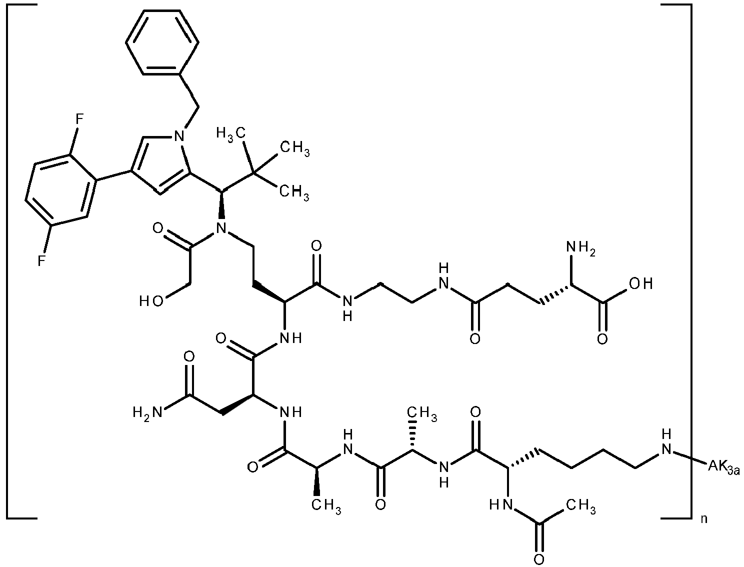

Zu einer Lösung von 5 mg des anti-TWEAKR Antikörpers TPP-2658 (entspricht TPP-2090-HC-N297A) in 530 µl DPBS pH 7.4 (c~10mg/mL) wurden 20 µL einer 10 mMol Lösung von Intermediat Q33 in DMSO hinzugegeben. Nach 5 min Inkubation bei 37°C wurden 50 µL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Volumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS. Schließlich wurden noch 0.00625 µmol des b-Transglutaminase Blockers Zedira C100 in 12.5 µL DPBS zu der Lösung hinzugefügt. Die erhaltene ADC Lösung wurde folgendermaßen charakterisiert:
Protein Konzentration: 1.68 mg/mL
Drug/mAb Ratio: 1.9

### Beispiel 34t

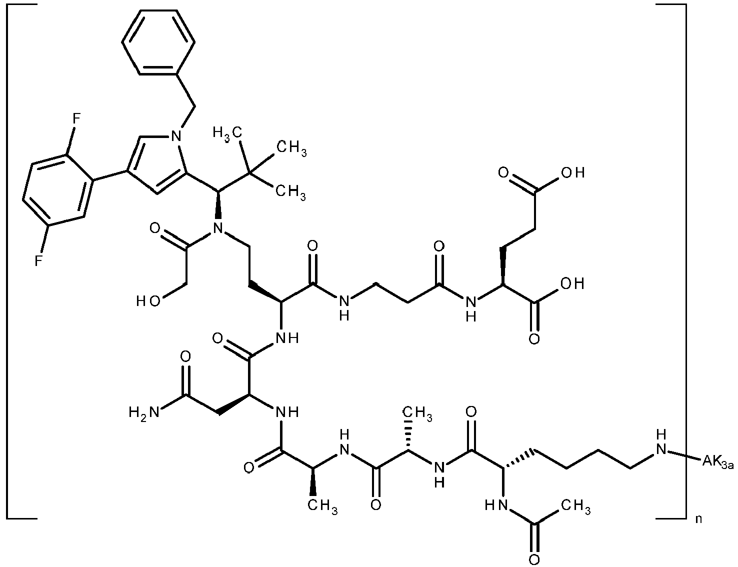

Zu einer Lösung von 5 mg des anti-TWEAKR Antikörpers TPP-2658 (entspricht TPP-2090-HC-N297A) in 530 µl DPBS pH 7.4 (c~10mg/mL) wurden 20 µL einer 10 mMol Lösung von Intermediat Q34 in DMSO hinzugegeben. Nach 5 min Inkubation bei 37°C wurden 50 µL einer Lösung von rekombinanter bakterieller Transglutaminase Lösung in Wasser (Produkt Nummer T001 von Zedira GmbH, Darmstadt, Germany) (25 U/mL) hinzugegeben und weitere 24h bei 37°C inkubiert. Dann wurde die Reaktionsmischung mit DPBS pH 7.4 auf ein Volumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS. Schließlich wurden noch 0.00625 µmol des b-Transglutaminase Blockers Zedira C100 in 12.5 µL DPBS zu der Lösung hinzugefügt. Die erhaltene ADC Lösung wurde folgendermaßen charakterisiert:
Protein Konzentration: 1.73 mg/mL
Drug/mAb Ratio: 1.8

### Beispiel 34t-4

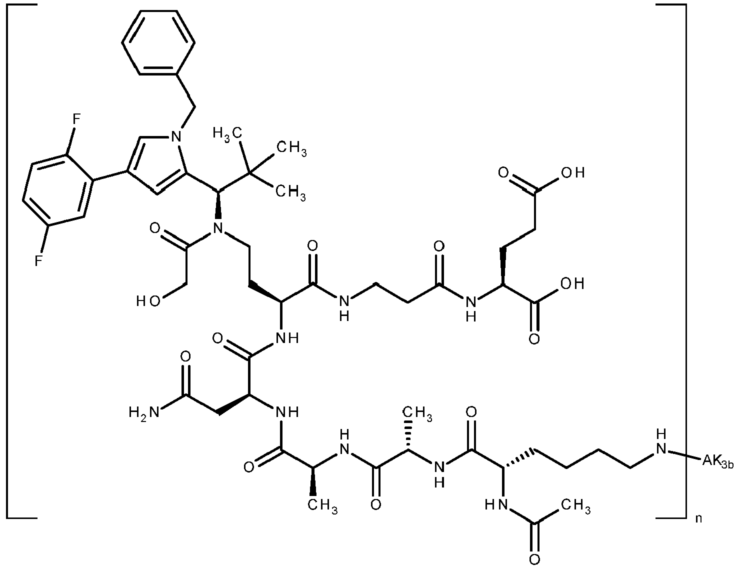

Zu einer Lösung von 5 mg des anti-TWEAKR Antikörpers TPP-5442 (entspricht TPP-2090-HC-N297Q) in DPBS pH 7.4 (c=7.4 mg/mL) wurden 83 µL einer 10 mMol Lösung von Intermediat Q34 in DMSO hinzugegeben. Nach 5 min Inkubation bei 37°C wurden 400 µL einer Lösung von rekombinanter bakterieller Transglutaminase Lösung in Wasser (Produkt Nummer T001 von Zedira GmbH, Darmstadt, Germany) (25 U/mL) hinzugegeben und 24 h bei 37°C inkubiert. Dann wurde die Reaktionsmischung mit DPBS pH 7.4 auf ein Gesamtvolumen von 2.5 mL verdünnt und durch Gelfiltration über mit DPBS equillibierte PD 10-Säulen (Sephadex^{®} G-25, GE Healthcare) gegeben und mit DPBS-Puffer bei pH 7.4 eluiert. Anschließend wurde die ADC Lösung mittels Amicon Ultracel-30K Zentrifugation (Millipore) aufkonzentriert und wieder rückverdünnt mit DPBS auf ein Volumen von etwa 2.5 mL. Schließlich wurden noch 0.1 µmol des b-Transglutaminase Blockers Zedira C100 in 200 µL DPBS zu der Lösung hinzugefügt. Die erhaltene ADC Lösung wurde folgendermaßen charakterisiert:
Protein Konzentration: 1.76 mg/mL
Drug/mAb Ratio: 3.9

### Beispiel 34te-4

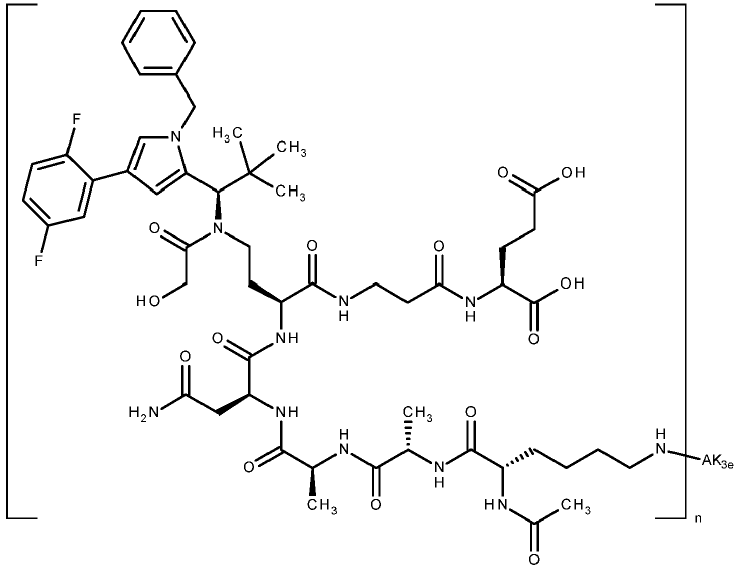

In analoger Weise zu Beispiel 34t-4 wurde auch TPP-7511 (entspricht Trastuzumab-HC-N297Q) eingesetzt und gekuppelt. Die erhaltene ADC Lösung wurde folgendermaßen charakterisiert:
Protein Konzentration: 1.56 mg/mL
Drug/mAb Ratio: 3.9

### Beispiel 35a

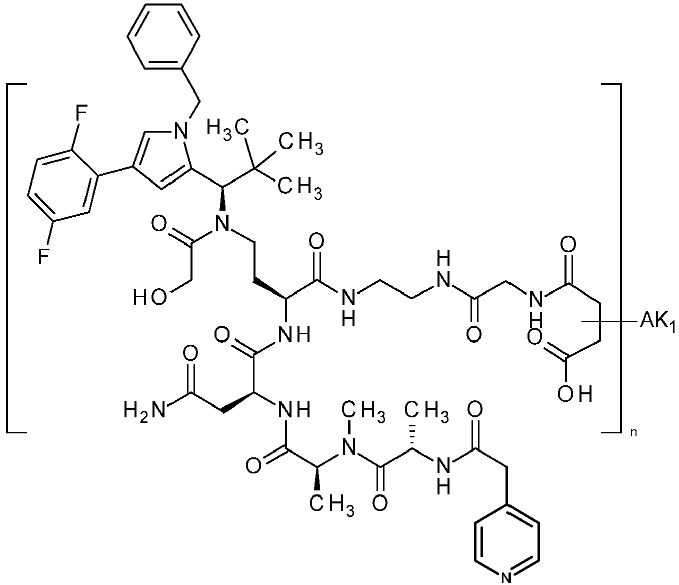

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.28 mg (0.00023 mmol) von Intermediat Q35 gelöst in 50 µl DMSO zugegeben. Nach weiteren 90 min Rühren bei RT wurde der Ansatz mit PBS-Puffer, der zuvor auf pH 8 eingestellt wurde, auf einVolumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 1.94 mg/mL
Drug/mAb Ratio: 3.3

### Beispiel 35e

In analoger Weise wurde Intermediat Q35 mit 5 mg Trastuzumab gekuppelt. Der erhaltene ADC Batch wurde folgendermaßen charakterisiert:
Protein Konzentration: 1.89 mg/mL
Drug/mAb Ratio: 3.5

### Beispiel 35k

In analoger Weise wurde Intermediat Q35 mit 5 mg anti-TWEAKR Antikörper TPP-2658 gekuppelt. Der erhaltene ADC Batch wurde folgendermaßen charakterisiert:
Protein Konzentration: 1.78 mg/mL
Drug/mAb Ratio: 3.5

### Beispiel 36a

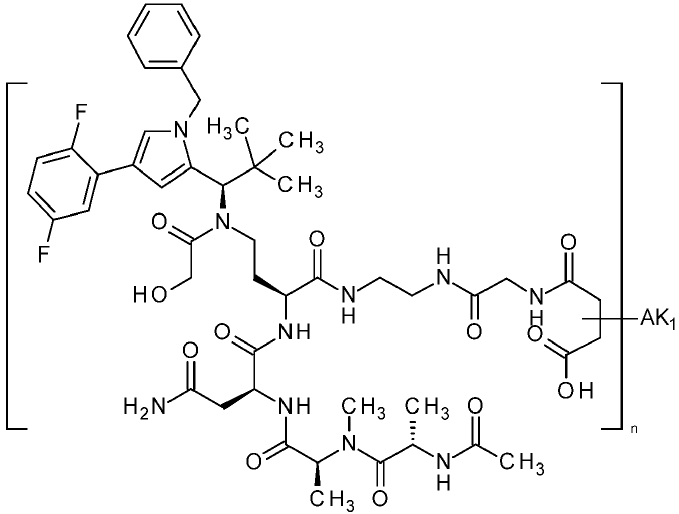

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q36 gelöst in 50 µl DMSO zugegeben. Nach weiteren 90 min Rühren bei RT wurde der Ansatz PBS-Puffer, der zuvor auf pH 8 eingestellt wurde, auf ein Volumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 2.0 mg/mL
Drug/mAb Ratio: 3.2

### Beispiel 36e

In analoger Weise wurde Intermediat Q36 mit 5 mg Trastuzumab gekuppelt. Der erhaltene ADC Batch wurde folgendermaßen charakterisiert:
Protein Konzentration: 1.84 mg/mL
Drug/mAb Ratio: 3.6

### Beispiel 36k

In analoger Weise wurde Intermediat Q36 mit 5 mg anti-TWEAKR Antikörper TPP-2658 gekuppelt. Der erhaltene ADC Batch wurde folgendermaßen charakterisiert:
Protein Konzentration: 1.75 mg/mL
Drug/mAb Ratio: 3.4

### Beispiel 37a

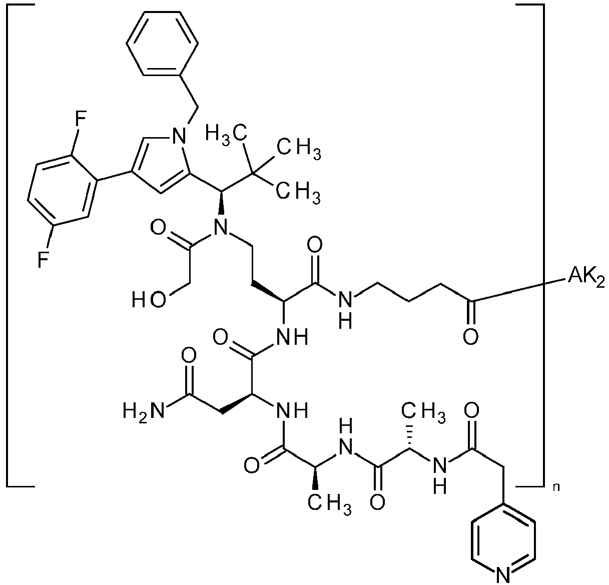

5 mg Cetuximab in 0.5 ml PBS (c=10 mg/mL) wurden unter Argon mit einer Lösung aus 5 Eq (0.2 mg) von Intermediat Q37 gelöst in 50µL DMSO zugesetzt und nach 1h Rühren bei RT wurde nochmals die gleiche Menge hinzugefügt und der Ansatz eine weitere Stunde bei RT gerührt. Anschließend wurde der Ansatz mit PBS Puffer (pH7.2) auf 2.5 mL verdünnt, über eine Sephadex-Säule gereinigt und dann durch Ultrazentrifugation aufkonzentriert und rückverdünnt mit PBS (pH7.2).
Protein Konzentration: 2.2 mg/mL
Drug/mAb Ratio: 4.1

### Beispiel 37e

In analoger Weise wurde Intermediat Q37 mit 5 mg Trastuzumab gekuppelt. Der erhaltene ADC Batch wurde folgendermaßen charakterisiert:
Protein Konzentration: 1.64 mg/mL
Drug/mAb Ratio: 4.5

### Beispiel 37k

In analoger Weise wurde Intermediat Q37 mit 5 mg anti-TWEAKR Antikörper TPP-2658 gekuppelt. Der erhaltene ADC Batch wurde folgendermaßen charakterisiert:
Protein Konzentration: 1.6 mg/mL
Drug/mAb Ratio: 6.2

### Beispiel 38a

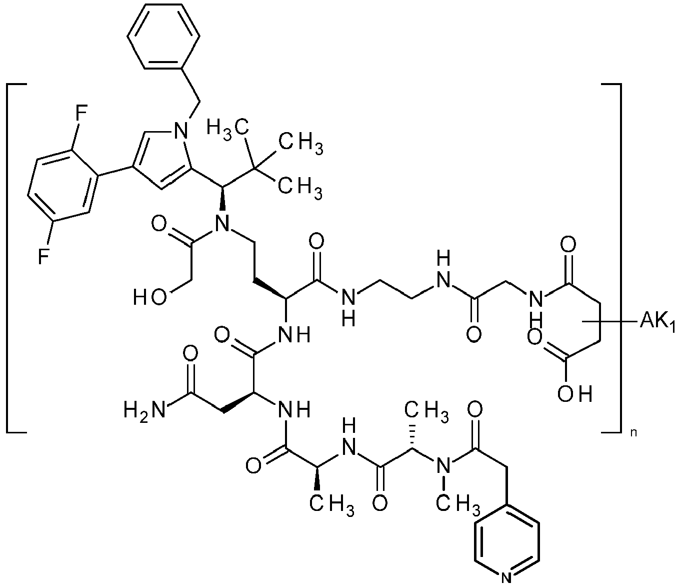

5 mg Cetuximab in 0.458 ml PBS (c=11 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q38 gelöst in 50 µl DMSO zugegeben. Nach weiteren 90 min Rühren bei RT wurde der Ansatz mit PBS-Puffer, der zuvor auf pH 8 eingestellt wurde, auf einVolumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 1.94 mg/mL
Drug/mAb Ratio: 2.9

### Beispiel 38e

In analoger Weise wurde Intermediat Q38 mit 5 mg Trastuzumab gekuppelt. Der erhaltene ADC Batch wurde folgendermaßen charakterisiert:
Protein Konzentration: 1.82 mg/mL
Drug/mAb Ratio: 3.5

### Beispiel 38k

In analoger Weise wurde Intermediat Q38 mit 5 mg anti-TWEAKR Antikörper TPP-2658 gekuppelt. Der erhaltene ADC Batch wurde folgendermaßen charakterisiert:
Protein Konzentration: 2.01 mg/mL
Drug/mAb Ratio: 3.3

### Beispiel 39a

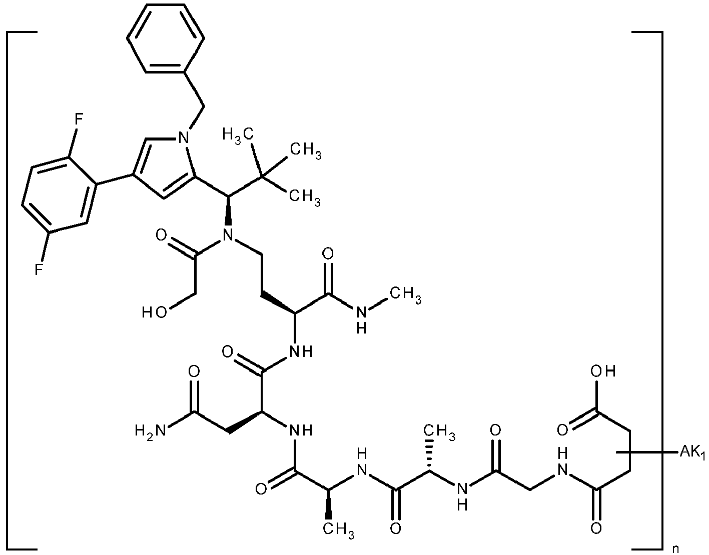

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.23 mg (0.00023 mmol) von Intermediat Q39 gelöst in 50 µl DMSO zugegeben. Nach weiteren 90 min Rühren bei RT wurde der Ansatz mit 1.9 ml PBS-Puffer, der zuvor auf pH 8 eingestellt wurde, verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.Anschließend wurde durch Ultrazentrifugation aufkonzentriert und rückverdünnt mit PBS-Puffer (pH 7.2). Der erhaltene ADC Batch wurde folgendermaßen charakterisiert:
Protein Konzentration: 1.97 mg/mL
Drug/mAb Ratio: 2.8

### Beispiel 39e

In analoger Weise wurde Intermediat Q39 mit 5 mg Trastuzumab gekuppelt. Der erhaltene ADC Batch wurde folgendermaßen charakterisiert:
Protein Konzentration: 1.77 mg/mL
Drug/mAb Ratio: 3.3

### Beispiel 39k

In analoger Weise wurde Intermediat Q39 mit 5 mg anti-TWEAKR Antikörper TPP-2658 gekuppelt. Der erhaltene ADC Batch wurde folgendermaßen charakterisiert:
Protein Konzentration: 1.74 mg/mL
Drug/mAb Ratio: 3.4

### Beispiel 40a

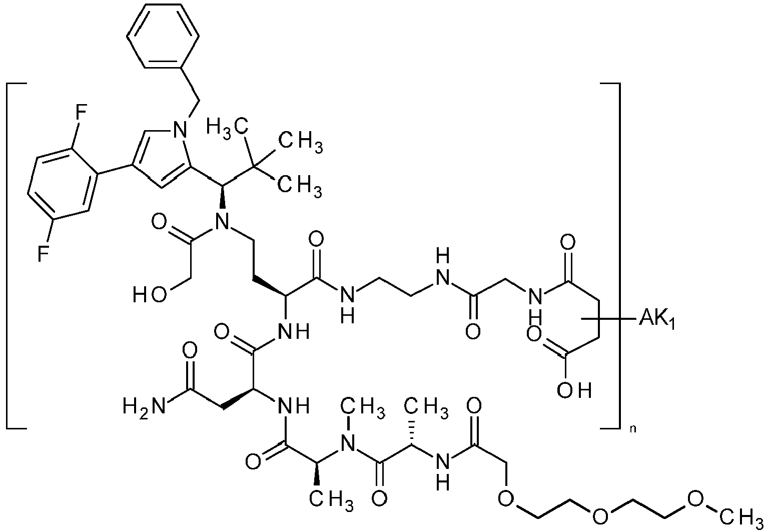

5 mg Cetuximab in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat Q40 gelöst in 50 µl DMSO zugegeben. Nach weiteren 90 min Rühren bei RT wurde der Ansatz mit PBS-Puffer, der zuvor auf pH 8 eingestellt wurde, auf einVolumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 1.95 mg/mL
Drug/mAb Ratio: 2.4

### Beispiel 40e

In analoger Weise wurde Intermediat Q40 mit 5 mg Trastuzumab gekuppelt. Der erhaltene ADC Batch wurde folgendermaßen charakterisiert:
Protein Konzentration: 1.84 mg/mL
Drug/mAb Ratio: 2.9

### Beispiel 40k

In analoger Weise wurde Intermediat Q40 mit 5 mg anti-TWEAKR Antikörper TPP-2658 gekuppelt. Der erhaltene ADC Batch wurde folgendermaßen charakterisiert:
Protein Konzentration: 1.93 mg/mL
Drug/mAb Ratio: 3.0

### Beispiel 41a

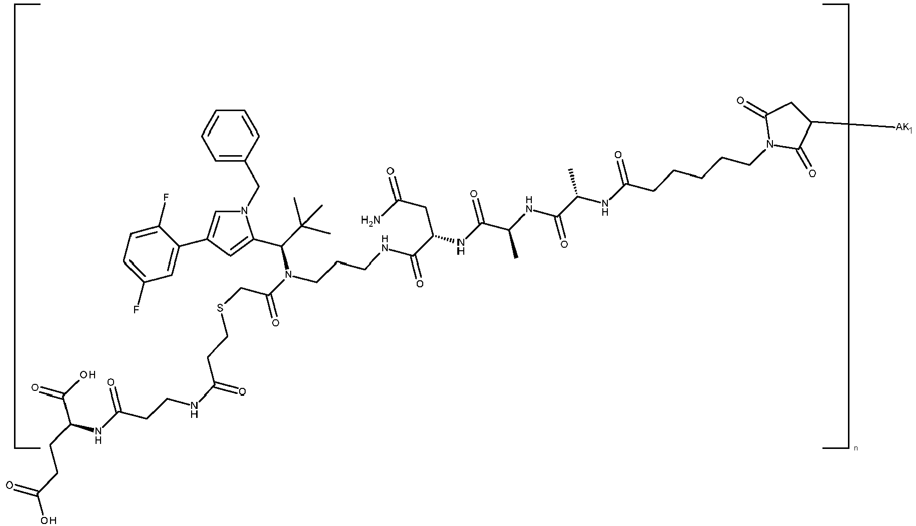

5 mg Cetuximab in 0.458 ml PBS (c=10.9 mg/mL) wurden unter Argon mit einer Lösung aus 0.029 mg TCEP in 0.05 ml PBS-Puffer versetzt. Der Ansatz wurde 30min bei RT gerührt und anschließend wurden 0.308 mg (0.00023 mmol) von Intermediat Q41 gelöst in 50 µl DMSO zugegeben. Nach weiteren 90 min Rühren bei RT wurde der Ansatz mit PBS-Pufferauf einVolumen von 2.5 mL verdünnt. Diese Lösung wurde dann über PD 10-Säule (Sephadex^{®} G-25, GE Healthcare) gereinigt. Diese Lösung wurde dann durch Ultrazentrifugation aufkonzentriert und rückverdünnt mit PBS-Puffer. Der erhaltene ADC Batch wurde folgendermaßen charakterisiert:
Protein Konzentration: 2.26 mg/mL
Drug/mAb Ratio: 3.9

### Beispiel 41e

In analoger Weise wurde Intermediat Q41 mit 5 mg Trastuzumab gekuppelt. Der erhaltene ADC Batch wurde folgendermaßen charakterisiert:
Protein Konzentration: 2.0 mg/mL
Drug/mAb Ratio: 4.3

### Beispiel 41k

In analoger Weise wurde Intermediat Q41 mit 5 mg anti-TWEAKR Antikörper TPP-2658 gekuppelt. Der erhaltene ADC Batch wurde folgendermaßen charakterisiert:
Protein Konzentration: 2.15 mg/mL
Drug/mAb Ratio: 4.0

### Beispiel 42a

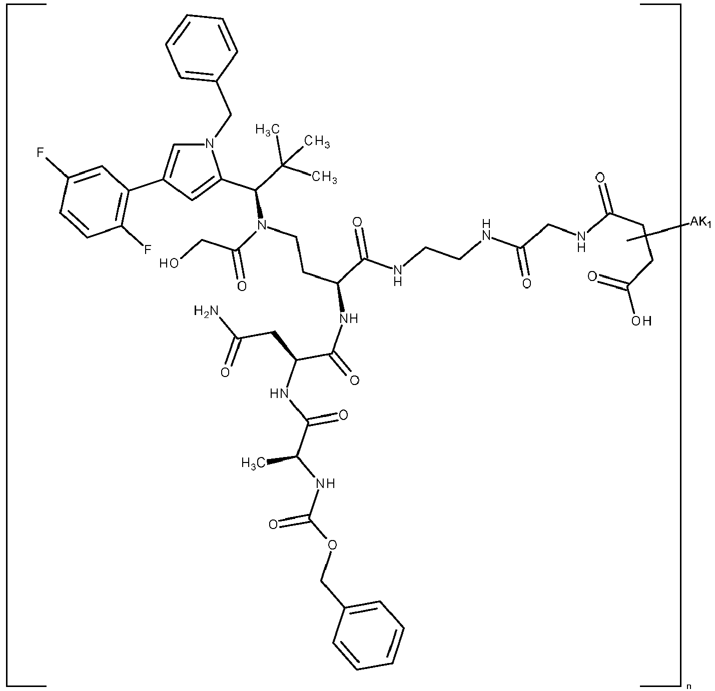

5 mg Cetuximab in 0.458 ml PBS (c=10.9 mg/mL) wurden unter Argon mit einer Lösung aus 0.029 mg TCEP in 0.05 ml PBS-Puffer versetzt. Der Ansatz wurde 30min bei RT gerührt und anschließend wurden 0.263 mg (0.00023 mmol) von Intermediat Q42 gelöst in 50 µl DMSO zugegeben. Nach weiteren 90 min Rühren bei RT wurde der Ansatz mit PBS-Puffer, der zuvor auf pH 8 eingestellt wurde, auf einVolumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 0.93 mg/mL
Drug/mAb Ratio: 3.5

### Beispiel 42e

In analoger Weise wurde Intermediat Q42 mit 5 mg Trastuzumab gekuppelt. Der erhaltene ADC Batch wurde folgendermaßen charakterisiert:
Protein Konzentration: 0.53 mg/mL
Drug/mAb Ratio: 4.2

### Beispiel 42k

In analoger Weise wurde Intermediat Q42 mit 5 mg anti-TWEAKR Antikörper TPP-2658 gekuppelt. Der erhaltene ADC Batch wurde folgendermaßen charakterisiert:
Protein Konzentration: 0.88 mg/mL
Drug/mAb Ratio: 3.7

### Beispiel 43a

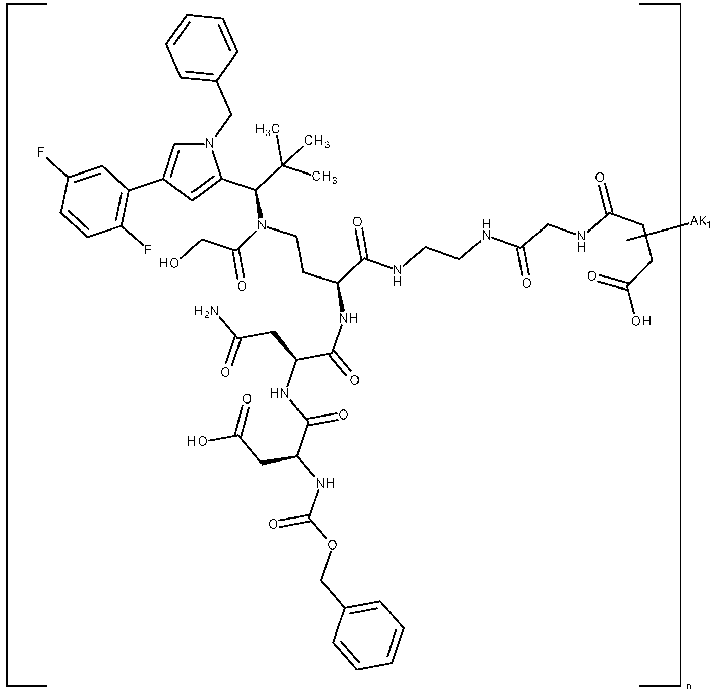

5 mg Cetuximab in 0.458 ml PBS (c=10.9 mg/mL) wurden unter Argon mit einer Lösung aus 0.029 mg TCEP in 0.05 ml PBS-Puffer versetzt. Der Ansatz wurde 30min bei RT gerührt und anschließend wurden 0.27 mg (0.00023 mmol) von Intermediat Q43 gelöst in 50 µl DMSO zugegeben. Nach weiteren 90 min Rühren bei RT wurde der Ansatz mit PBS-Puffer, der zuvor auf pH 8 eingestellt wurde, auf einVolumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 1.91 mg/mL
Drug/mAb Ratio: 2.6

### Beispiel 43e

In analoger Weise wurde Intermediat Q43 mit 5 mg Trastuzumab gekuppelt. Der erhaltene ADC Batch wurde folgendermaßen charakterisiert:
Protein Konzentration: 1.56 mg/mL
Drug/mAb Ratio: 3.6

### Beispiel 43k

In analoger Weise wurde Intermediat Q43 mit 5 mg anti-TWEAKR Antikörper TPP-2658 gekuppelt. Der erhaltene ADC Batch wurde folgendermaßen charakterisiert:
Protein Konzentration: 1.89 mg/mL
Drug/mAb Ratio: 3.6

### Beispiel 44a

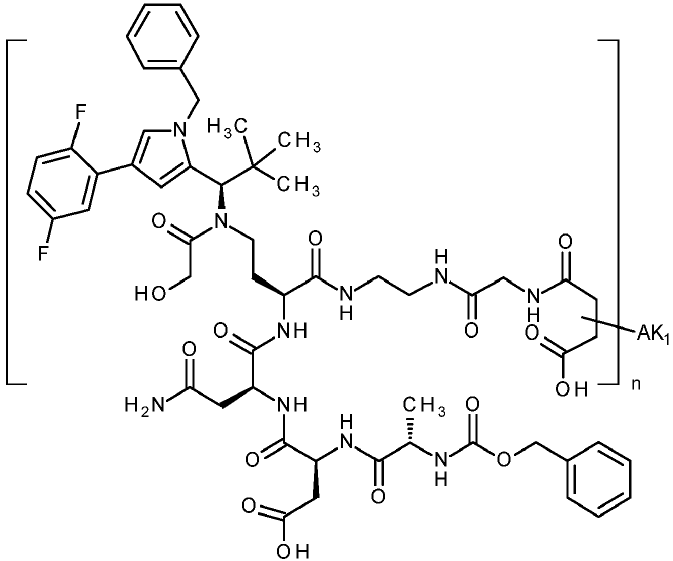

5 mg Cetuximab in 0.458 ml PBS (c=10.9 mg/mL) wurden unter Argon mit einer Lösung aus 0.029 mg TCEP in 0.05 ml PBS-Puffer versetzt. Der Ansatz wurde 30min bei RT gerührt und anschließend wurden 0.29 mg (0.00023 mmol) von Intermediat Q44 gelöst in 50 µl DMSO zugegeben. Nach weiteren 90 min Rühren bei RT wurde der Ansatz mit PBS-Puffer, der zuvor auf pH 8 eingestellt wurde, auf einVolumen von 2.5 mL verdünnt. Diese Lösung wurde dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt.

Diese Lösung wurde dann durch Ultrazentrifugation aufkonzentriert und rückverdünnt mit PBS-Puffer (pH 7.2). Der erhaltene ADC Batch wurde folgendermaßen charakterisiert:
Protein Konzentration: 1.82 mg/mL
Drug/mAb Ratio: 2.6

### Beispiel 44e

In analoger Weise wurde Intermediat Q44 mit 5 mg Trastuzumab gekuppelt. Der erhaltene ADC Batch wurde folgendermaßen charakterisiert:
Protein Konzentration: 1.79 mg/mL
Drug/mAb Ratio: 3.4

### Beispiel 44k

In analoger Weise wurde Intermediat Q44 mit 5 mg anti-TWEAKR Antikörper TPP-2658 gekuppelt. Der erhaltene ADC Batch wurde folgendermaßen charakterisiert:
Protein Konzentration: 1.72 mg/mL
Drug/mAb Ratio: 3.7

### C: Bewertung der biologischen Wirksamkeit

Die biologische Wirkung der erfindungsgemäßen Verbindungen kann durch die nachstehend beschriebenen Assays gezeigt werden:

### a. C-1a Bestimmung der cvtotoxischen Wirkung der ADCs

Die Analyse der cytototoxischen Wirkung der ADCs erfolgt auf verschiedenen Zelllinien:
NCI-H292: humane mukoepidermoide Lungenkarzinomzellen, ATCC-CRL-1848, Standardmedium: RPMI 1640 (Biochrom; #FG1215, stab. Glutamin) + 10% FCS (Sigma #F2442), TWEAKR-positiv; EGFR-positiv.
BxPC3: humane Bauchspeicheldrüsenkrebszellen, ATCC-CRL-1687, Standardmedium: RPMI 1640 (Biochrom; #FG1215, stab. Glutamin) + 10% FCS (Sigma #F2442), TWEAKR-positiv
LoVo humane kolorektale Krebszellen, ATCC No. CCL-229, Kultivierung für MTT-Assay: Standardmedium: Kaighn's + L-Glutamin (Invitrogen 21127) + 10% heat inactivated FCS (Fa. Gibco, No. 10500-064). Kultivierung für CTG-Assay: RPMI 1640 (Biochrom; #FG1215, stab. Glutamin) + 10% FCS (Sigma #F2442). TWEAKR-positiv.
KPL4: humane Brustkrebszelllinie, Bayer Pharma AG (identity checked and confirmed on 19.7.2012 at DSMZ), Standardmedium: RPMI 1640 (Fa. Gibco; #21875- 059, stab. L-Glutamin) + 10% heat inactivated FCS (Fa. Gibco, No. 10500-064); HER2-positiv.
SK-HEP-1: humane Leberkrebszelllinie, ATCC No. HTB-52, Standardmedium: MEM mit Earle's Salzen + Glutamax I (Invitrogen 41090) + 10% heat inactivated FCS (Fa. Gibco, No. 10500-064); EGFR-positiv, TWEAKR-positiv

Die Kultivierung der Zellen erfolgt nach Standard-Methode, wie bei der American Tissue Culture Collection (ATCC) für die jeweiligen Zelllinien angegeben.

### CTG-Assay

Die Kultivierung der Zellen erfolgte nach StandardMethode, mit den unter C-1 angegebenen Wachstumsmedien. Zur Durchführung wurden die Zellen mit einer Lösung von Trypsin (0.05%) und EDTA (0.02%) in PBS (Biochrom AG #L2143) abgelöst, pelletiert, in Kulturmedium resuspendiert, gezählt und in eine 96-Loch Kulturplatte mit weissem Boden (Costar #3610) ausgesät (in 75µl/Loch, folgende Zellzahlen je Loch: NCI-H292: 2500 Zellen/ Loch, BxPC3 2500 Zellen / Loch, LoVo 3000 Zellen / Loch) und im Brutschrank bei 37°C und 5% Kohlendioxid inkubiert. Nach 24h wurden die Antikörper-Wirkstoffkonjugate in 25µl Kulturmedium (vierfach konzentriert) auf die Zellen gegeben, so dass finale Konzentrationen der Antikörper-Wirkstoffkonjugate von 3 × 10⁻⁷ M bis 3 × 10⁻¹¹ M auf den Zellen erreicht wurden (Triplikate). Anschließend wurden die Zellen im Brutschrank bei 37°C und 5% Kohlendioxid inkubiert. In einer parallelen Platte wurde die Zellvitalität zu Beginn der Wirkstoffbehandlung (Tag 0) mit dem Cell Titer Glow (CTG) Luminescent Cell Viability Assay (Promega #G7573 und #G7571) bestimmt. Dazu wurden pro Zellansatz 100µl des Substrats hinzugefügt, die Platten anschließend mit Alufolie abgedeckt, für 2 Minuten mit dem Plattenschüttler bei 180 rpm geschüttelt, für 8 Minuten auf der Laborbank stehen gelassen und dann mit einem Luminometer (Victor X2, Perkin Elmer) gemessen. Das Substrat detektiert den ATP-Gehalt in den lebenden Zellen, wobei ein Lumineszenz-Signal erzeugt wird, dessen Höhe direkt proportional zur Vitalität der Zellen ist. Nach 72h Inkubation mit den Antikörper-Wirkstoffkonjugaten wurde nun auch in diesen Zellen die Vitalität mit dem Cell Titer Glow Luminescent Cell Viability Assay wie oben beschrieben bestimmt. Aus den gemessenen Daten wurde die IC₅₀ der Wachstumshemmung im Vergleich zum Tag 0 unter Verwendung des DRC (Dose Response Curve) Analysis Spreadsheets anhand einer 4-Parameter Anpassung berechnet. Das DRC Analysis Spreadsheet ist ein von Bayer Pharma AG und Bayer Business Services auf der Plattform IDBS E-WorkBook Suite entwickeltes Biobook Spreadsheet (IDBS: ID Business Solutions Ltd., Guildford, UK).

In der folgenden Tabelle 1a sind die ICso-Werte repräsentativer Ausführungsbeispiele aus diesem Assay aufgeführt:

**Tabelle 1a**

| **Beispiel** | **BxPC3 IC₅₀ [M] CTG** | **NCI-H292 IC₅₀ [M] CTG** | **LoVo IC₅₀ [M] CTG** |
|---|---|---|---|
| 1k | 1.70E-09 | 2.99E-10 | 1.24E-10 |
| 1k* | 1.27E-09 | 4.66E-10 | 1.25E-10 |
| 2k | 2.94E-09 | 5.84E-10 | 2.04E-10 |
| 2k* | 2.16E-09 | 8.45E-10 | 2.85E-10 |
| 3k | 1.30E-08 | 1.04E-09 | 4.67E-10 |
| 4k | 3.29E-09 | 1.04E-09 | 2.45E-10 |
| 5k | 6.52E-09 | >6.00E-07 | >6.00E-07 |
| 6k | 4.71E-09 | >6.00E-07 | >6.00E-07 |
| 7k | 4.77E-09 | 1.09E-08 | >6.00E-07 |
| 8k | 5.77E-10 | 2.90E-10 | 1.82E-10 |
| 9k | 6.70E-09 | 6.82E-10 | 6.94E-10 |
| 10k | 1.11E-09 | 6.10E-10 | 5.63E-10 |
| D-Asn Epimer von Beispiel 1k | >6.0E-07 | >6.0E-07 | >6.0E-07 |
| 11k | 1.43E-09 | 8.16E-10 | 1.66E-10 |
| 12k | 2.75E-09 | 1.16E-09 | 2.12E-10 |
| 13k | 9.04E-09 | 1.37E-09 | 4.45E-10 |
| 14k | 2.59E-09 | 3.32E-10 | 1.23E-10 |
| 15k | 2.09E-09 | 7.19E-10 | 1.52E-10 |
| 16k | 2.04E-09 | 1.23E-09 | 2.06E-10 |
| 17k | 5.08E-09 | 6.84E-10 | 2.28E-10 |
| 18k | 2.56E-10 | 3.91E-10 | 1.09E-10 |
| 19k | 5.94E-09 | 7.96E-10 | 1.70E-10 |
| 20k | 9.86E-10 | 3.48E-10 | 8.30E-11 |
| 20k* | 4.58E-10 | 6.03E-10 | 1.65E-11 |
| 21k | 2.09E-09 | 1.42E-09 | 2.36E-10 |
| 22k | 4.20E-09 | 4.31E-09 | >6.0E-07 |
| 23k | 1.34E.09 | 1.27E-09 | 1.56E-08 |
| 24k | 1.72E-09 | 1.99E-09 | 2.41E-07 |
| 25k | 1.37E-09 | 1.96E-09 | >6.0E-07 |
| 26k | 1.99E-09 | 2.54E-09 | >6.0E-07 |
| 27k | 5.34E-10 | 8.61E-10 | 3.46E-10 |
| 28k | 6.18E-10 | 1.17E-09 | 4.33E-10 |
| 29k | 5.66E-10 | 8.06E-10 | 3.13E-10 |
| 30k | 2.56E-10 | 6.83E-10 | 2.13E-10 |
| 35k | 1.65E-08 | 2.29E-08 | 2.86E-10 |
| 36k | 2.33E-08 | 2.26E-08 | 5.24E-10 |
| 37k | 1.69E-09 | 6.00E-09 | 6.00E-07 |
| 38k | 1.32E-08 | 1.69E-08 | 3.06E-10 |
| 39k | 1.46E-09 | 8.07E-08 | >6.00E-07 |
| 40k | 9.95E-09 | 4.65E-09 | 8.78E-10 |
| 41k | 7.36E-10 | 1. 15E-09 | 1.06E-09 |
| 42k | 9.80E-10 | 8.36E-10 | 1.22E-10 |
| 43k | 1.08E-09 | 1.11E-09 | 1.43E-10 |
| 44k | 1.40E-09 | 9.09E-10 | 2.31E-10 |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Antikörper-Wirkstoffkonjugate war selektiv versus der jeweiligen Isotypkontrolle, die den jeweils entsprechenden Linker und Toxophor enthielt.

Als weitere Referenzverbindung wurde in Analogie zu Beispiel 1k das D-Asparagin-Epimer von Intermediat Q1 mit dem anti-TWEAKR Antikörper TPP-2658 gekuppelt.

### Kontrollbeispiel zu Beispiel 1k

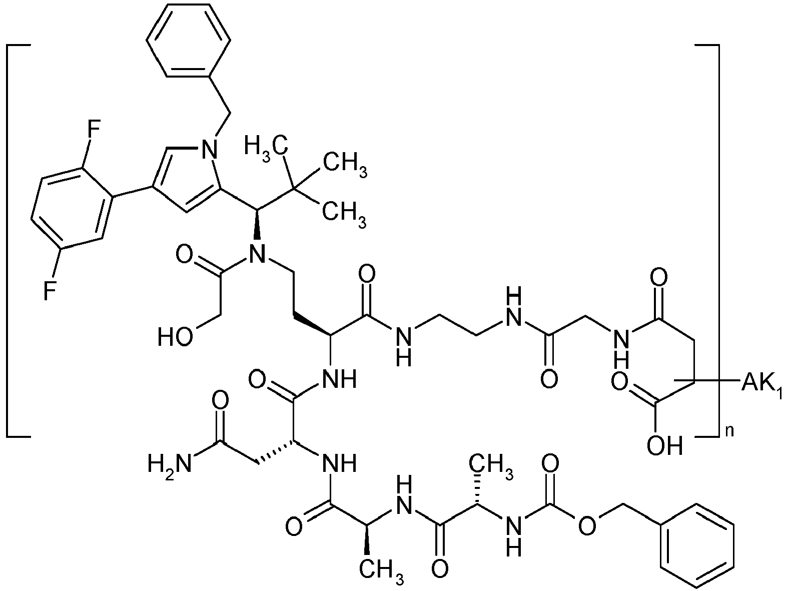

Hier wurde in Analogie zu Beispiel 1k das D-Asparagin-Epimer von Q1 mit 60 mg anti-TWEAKR Antikörper TPP-2658 gekuppelt. Der erhaltene ADC Batch wurde folgendermaßen charakterisiert: Protein Konzentration: 11.56 mg/mL

### Drug/mAb Ratio: 3.6

Wie in Tabelle 1a dargestellt zeigte dieses Kontroll-ADC keine signifikante zytotoxische Wirkung an den 3 Zelllinien.

### MTT-Assay

Die Kultivierung der Zellen erfolgte nach Standardmethode, mit den unter C-1 angegebenen Wachstumsmedien. Zur Durchführung wurden die Zellen mit einer Lösung von Accutase in PBS (Fa. Biochrom AG #L2143) abgelöst, pelletiert, in Kulturmedium resuspendiert, gezählt und in eine 96-Loch Kulturplatte mit weißem Boden (Fa. Costar #3610) ausgesät (NCI H292: 2500 Zellen/well; SK-HEP-1: 1000 Zellen/well; KPL-4: 1200 Zellen/well; in 100µL Gesamtvolumen). Anschließend wurden die Zellen im Brutschrank bei 37°C und 5% Kohlendioxid inkubiert. Nach 48h wurde ein Mediumwechsel durchgeführt. Dann wurden die Antikörper-Wirkstoff-Konjugate in 10µl Kulturmedium in Konzentrationen von 10⁻⁵M bis 10⁻¹³M zu den Zellen (Triplikate) pipettiert, bevor der Ansatz im Brutschrank bei 37°C und 5% Kohlendioxid inkubierte. Nach 96h erfolgte die Detektion der Zellproliferation mit Hilfe des MTT-Assays (ATCC, Manassas, Virginia, USA, Katalog-Nr. 30-1010K). Hierzu wurde das MTT Reagens für 4h mit den Zellen inkubiert, bevor durch Zugabe des Detergenzes die Lyse der Zellen über Nacht erfolgte. Die Detektion des gebildeten Farbstoffs erfolgte bei 570nm (Infinite M1000 pro, Fa. Tecan). Aus den gemessenen Daten wurde die IC₅₀ der Wachstumshemmung unter Verwendung der DRC (Dose Response Curve) berechnet. Die Proliferation ohne Testsubstanz, aber ansonsten identisch behandelten Zellen, wird als 100% Wert definiert.

In den folgenden Tabellen 1b und 1c sind die ICso-Werte repräsentativer Ausführungsbeispiele aus diesem Assay aufgeführt:

**Tabelle 1b**

| **Beispiel** | **NCI-H292 IC₅₀ [M] MTT Assay** | **SK-HEP-1 IC₅₀ [M] MTT Assay** |
|---|---|---|
| 1a | 1.51E-11 | 122E-10 |
| 1i | 4.91E-10 | 3.84E-09 |
| 2a | 6.69E-11 | 1.75E-10 |
| 4a | 1.42E-10 | 9.57E-10 |
| 5a | 6.03E-10 | 5.00E-07 |
| 6a | 2.95E-10 | 5.00E-07 |
| 7a | 2.18E-10 | 5.00E-07 |
| 8a | 9.13E-11 | 1.63E-10 |
| 10a | 4,79E-11 | 9.75E-08 |
| 11a | 2.80E-12 | 3,86E-10 |
| 12a | 7.76E-12 | 6.71E-11 |
| 13a | 7,69E-11 | 1,16E-10 |
| 14a | 9.04E-12 | 1,04E-10 |
| 15a | 2.36E-11 | 9.08E-11 |
| 16a | 4,21E-11 | 2,51E-10 |
| 17a | 6,69E-12 | 4,66E-12 |
| 18a | 1.69E-11 | 2.56E-09 |
| 19a | 2.39E-11 | 1.45E-09 |
| 21a | 7.24E-12 | 2.95E-11 |
| 22a | 2.41E-10 | 9.19E-09 |
| 23a | 1.09E-10 | 7.16E-10 |
| 24a | 2.47E-10 | 5.69E-08 |
| 25a | 4.99E-11 | 6.84E-08 |
| 26a | 7.91E-11 | 5.00E-07 |
| 27a | 3.17E-12 | 4.83E-09 |
| 28a | 6.21E-11 | 4.49E-08 |
| 29a | 1.00E-10 | 1.54E-08 |
| 30a | 1.13E-11 | 3.72E-10 |
| 31t | 1.50E-09 | 1.92E-09 |
| 32t | 2.09E-09 | 2.12E-09 |
| 33t | 3.07E-09 | 1.34E-09 |
| 34t | 8.16E-10 | 8.87E-10 |
| 34t-4 | 1.84E-10 | 1.71E-10 |
| 35a | 2.04E-12 | 3.06E-09 |
| 36a | 1.83E-12 | 1.35E-09 |
| 37a | 9.22E-11 | 1.09E-08 |
| 38a | 1.32E-10 | 1.27E-10 |
| 39a | 1.15E-09 | 2.66E-08 |
| 40a | 2.05E-11 | 3.10E-10 |
| 41a | 1.58E-10 | 4.88E-12 |
| 42a | 1.49E-12 | 1.04E-11 |
| 43a | 5.42E-12 | 1.50E-09 |
| 44a | 1.52E-11 | 3.58E-10 |

**Tabelle 1c**

| **Beispiel** | **KPL4 IC₅₀ [M] MTT Assay** |
|---|---|
| 1e | 3.75E-10 |
| D-Asn Epimer von Beispiel 1e | > 1.00E-7 |
| 2e | 3.89E-10 |
| 5e | 2.91E-09 |
| 6e | 3.11E-09 |
| 8e | 6.41E-11 |
| 11e | 9.37E-11 |
| 12e | 1.13E-10 |
| 13e | 5.71E-11 |
| 14e | 2.34E-10 |
| 16e | 1.44E-10 |
| 17e | 9.15E-11 |
| 18e | 8.25E-11 |
| 19e | 4.03E-10 |
| 21e | 1.22E-12 |
| 22e | 5.17E-10 |
| 23e | 5.13E-10 |
| 24e | 7.05E-09 |
| 25e | 7.51E-11 |
| 26e | 5.10E-11 |
| 27e | 4.81E-10 |
| 28e | 4.41E-10 |
| 29e | 9.54E-11 |
| 30e | 2.12E-10 |
| 34te-4 | 3.29E-10 |
| 35e | 7.71E-11 |
| 36e | 4.84E-11 |
| 37e | 9.53E-11 |
| 38e | 1.87E-10 |
| 39e | 4.42E-09 |
| 40e | 1.07E-10 |
| 41e | 7.84E-12 |
| 42e | 1.93E-10 |
| 43e | 1.04E-10 |
| 44e | 2.34E-10 |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Antikörper-Wirkstoffkonjugate war selektiv versus der jeweiligen Isotypkontrolle, die den jeweils entsprechenden Linker und Toxophor enthielt.

Auch hier wurde in Analogie zu Beispiel 1e das D-Asparagin-Epimer von Q1 mit 5 mg Trastuzumab gekuppelt. Der erhaltene ADC Batch wurde folgendermaßen charakterisiert:
Protein Konzentration: 1.68 mg/mL
Drug/mAb Ratio: 3.6

Wie in Tabelle 1c dargestellt zeigte auch dieses Kontroll-ADC keine signifikante zytotoxische Wirkung an der KPL4 Zelllinie.

### C-1b Bestimmung der Inhibition des Kinesinspindelproteins KSP/ Eg5 durch ausgewählte Beispiele

Die Motordomäne des humanen Kinesinspindelproteins KSP /Eg5 (Fa. tebu-bio/ Cytoskeleton Inc, No. 027EG01-XL) wurde in einer Konzentration von 10nM mit 50µg/ml Taxol (Fa. Sigma No. T7191-5MG) stabilisierten Microtubuli (bovine oder porcine, Fa. tebu-bio/ Cytoskeleton Inc) für 5 min bei RT in 15mM PIPES, pH 6,8 (5mM MgClz und 10mM DTT, Fa. Sigma) inkubiert. Die frisch hergestellte Mischung wurde in eine 384 MTP (Fa. Corning) aliquotiert. Es folgte die Zugabe der zu untersuchenden Inhibitoren in Konzentration von 1.0 x10-6M bis 1.0x 10-13M und ATP (finale Konzentration 500µM; Fa. Sigma). Die Inkubation erfolgte über 2h bei RT. Die ATPase-Aktivität wurde durch den Nachweis des entstehenden anorganischen Phosphats mit Malachit-Grün detektiert (Fa. Biomol). Nach der Zugabe des Reagenz erfolgte eine 50min Inkubation bei RT, bevor die Detektion der Absorption bei einer Wellenlänge von 620nm erfolgt. Als Positivkontrolle wurden Monastrol (Fa. Sigma, M8515-1mg) und Ispinesib (Fa. AdooQ Bioscience A10486) verwendet. Die Einzeldaten der Dosis-Wirkungskurve stellen achtfach Bestimmungen dar. Bei den ICso-Werten handelt es sich um Mittelwerte aus zwei unabhängigen Experimenten. Als 100% Kontrolle diente die nicht mit Inhibitoren behandelte Probe.

In der folgenden Tabelle 2 sind die ICso-Werte repräsentativer Ausführungsbeispiele aus dem beschriebenen Assay und den korrespondierenden zytotoxischen Daten (MTT-Assay) zusammengefasst:

**Tabelle 2**

| **Beispiele** | **KSP-Assay IC₅₀ [M]** | **NCI-H292 IC₅₀ [M] MTT Assay** | **KPL4 IC₅₀ [M] MTT Assay** |
|---|---|---|---|
| M1 | 2.01E-09 | 5.00E-07 | 5.00E-07 |
| M2 | 2.45E-09 | 2.04E-07 | 1.63E-07 |
| M3 | 1.52E-09 | 3.21E-08 | 9.00E-08 |
| M4 | 2.71E-10 | 4.43E-08 | 1.76E-07 |
| M5 | 4.57E-10 | 7.94E-08 | 2.22E-07 |
| M6 | 1.78E-09 | 4.63E-08 | 1.93E-07 |
| M7 | 6.21E-10 | 2.22E-08 | 9.25E-08 |
| M9 | 1.07E-09 | 7,74E-10 | 2.57E-10 |
| M10 | 4.70E-10 | 3.03E-07 | 2.26E-07 |
| M11 | 1.11E-09 | 4.32E-11 | |
| M12 | 4.46E-10 | 3.3E-08 | |
| M13 | 1.50E-09 | 1.52E-07 | 1.69E-07 |
| M14 | 2.16E-09 | 1.74E-07 | 1.82E-07 |
| M15 | 9.64E-10 | 1.33E-07 | 1.69E-07 |
| M16 | 1.48E-09 | 1.43E-07 | 1.95E-07 |
| M17 | 4.17E-09 | 7.35E-09 | |
| M18 | 5.17E-09 | 3.55E-08 | |
| M19 | 2.58E-09 | 1.21E-07 | |
| M20 | 1.50E-09 | 1,49E-07 | 2,13E-07 |
| M21 | 2.31E-09 | | |
| M22 | 8.27E-10 | 2.89E-08 | 1.82E-07 |
| M23 | 1.26E-09 | 5.00E-07 | 5.00E-07 |
| M24 | 2.90E-09 | 1.67E-07 | 5.00E-07 |
| M25 | 2.91E-09 | 5.00E-07 | 5.00E-07 |
| M26 | 9.44E-10 | 6.38E-08 | |
| M27 | 2.03E-09 | 2.76E-07 | |

Die angegebenen Wirkdaten beziehen sich auf die im vorliegenden experimentellen Teil beschriebenen Ausführungsbeispiele.

### C-1c Enzymatische Assays

### a: Cathepsin B-Assay

Für jede zu untersuchende Cathepsin B-spaltbare Prodrug wurde ein Ansatz in einem Mikroreaktionsgefäße (0.5ml, Fa. Eppendorf) angesetzt. Das hier verwendete Enzym wurde aus humanem Lebergewebe gewonnen. Es wurden 2µg Cathepsin B (Sigma C8571 25 µg) vorgelegt und mit 50mM Na-Phosphat Puffer, pH6.0, 2mM DTT auf ein Gesamtvolumen von 200µLaufgefüllt. Dann wurden 50 µL der zu untersuchenden Substratlösung zupipettiert. Die Inkubation des Ansatzes erfolgte im Thermoblock (Fa. Thermo Fisher Scientific) bei 40°C unter ständigem Schütteln bei 300rpm. Die enzymatische Reaktion wurde kinetisch kontrolliert. Hierzu wurde zu unterschiedlichen Zeitpunkten eine 10µL Probe entnommen. Die entnommene Probe wurde sofort mit 20µL eiskaltem Methanol versetzt, um die enzymatische Reaktion zu stoppen und dann bei -20°C eingefroren. Die gewählten Zeitpunkte zur Probenentnahme waren nach 10min, 2h, 4h und 24h. RP-HPLC-Analyse untersucht (reverse phase HPLC, Fa.Agilent Technologies 1200er Series). Die Bestimmung des freigesetzten Toxophors ermöglichte die Bestimmung der Halbwertszeit t_{1/2} der enzymatische Reaktion.

### b: Legumain Assay

Der Legumain Assay wurde mit rekombinatem humanem Enzym durchgeführt. Die rhLegumain Enzymlösung (Catalog # 2199-CY, R&D Systems) wurde in 50mM Na-Acetat Puffer/ 100mM NaCl, pH4.0 auf die gewünschte Konzentration verdünnt und 2h bei 37°C vorinkubiert. rhLegumain wurde dann in 50mM MES Puffer, 250mM NaCl, pH 5.0 auf eine finale Konzentration von 1ng/µL eingestellt. Für jede zu untersuchende Legumain-spaltbare Prodrug wurde ein Ansatz in einem Mikroreaktionsgefäße (0.5ml, Fa. Eppendorf) angesetzt. Hierzu wurde die Substratlösung mit 50mM MES Puffer, 250mM NaCl, pH 5.0 auf die gewünschte Konzentration (2-fach konzentriert) eingestellt. Für die kinetische Messung der enzymatischen Reaktion wurden zunächst 250µL der Legumainlösung vorgelegt und durch Zugabe von 250µL der Substratlösung (finale Konzentration einfach konzentriert) wurde die Enzymreaktion gestartet. Zu verschiedenen Zeitpunkten wurden je 50µL Proben entnommen Diese Probe wurde sofort mit 100µL eiskaltem Methanol versetzt, um die enzymatische Reaktion zu stoppen und dann bei -20°C eingefroren. Die gewählten Zeitpunkte zur Probenentnahme waren nach 0,5h, 1h, 3h und 24h. Die Proben wurden dann anschließend mittels RP-HPLC-Analyse und durch LC-MS Analytik untersucht. Die Bestimmung des freigesetzten Toxophors ermöglichte die Bestimmung der Halbwertszeit t_{1/2} der enzymatischen Reaktion .

Als repräsentative Beispiele um die Legumain-vermittelte Spaltung zu zeigen, wurden als Substrate im Legumain-Assay die Modellverbindungen A und B hergestellt.

### Referenzbeispiel Modellverbindung A

### N-(Pyridin-4-ylacetyl)-L-alanyl-L-alanyl-N¹-[(2S)-4-[{(1R)-1-[1-benzyl-4-(2,5-difluorphenyl)-1H-pyrrol-2-yl]-2,2-dimethylpropyl}(glycoloyl)amino]-1-(methylamino)-1-oxobutan-2-yl]-L-aspartamid

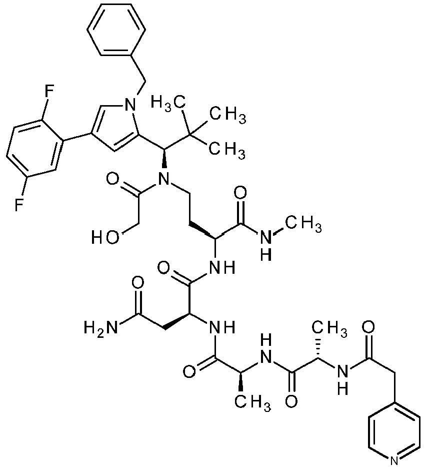

Zunächst wurde Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1) wie in WO 2015096982 A1 beschrieben, hergestellt. Anschließend wurde aus diesem Intermediat durch Kupplung mit Intermediat L103 in DMF in Gegenwart von HATU und von N,N-Diisopropylethylamin die Titelverbindung hergestellt.

LC-MS (Methode 1): Rₜ = 0.86 min; MS (ESIpos): m/z = 902 [M+H]⁺.

### Referenzbeispiel Modellverbindung B

### N-(Pyridin-4-ylacetyl)-L-alanyl-N-methyl-L-alanyl-N¹-[(2S)-4-[{(1R)-1-[1-benzyl-4-(2,5-difluorphenyl)-1H-pyrrol-2-yl]-2,2-dimethylpropyl}(glycoloyl)amino]-1-(methylamino)-1-oxobutan-2-yl] -L-aspartamid

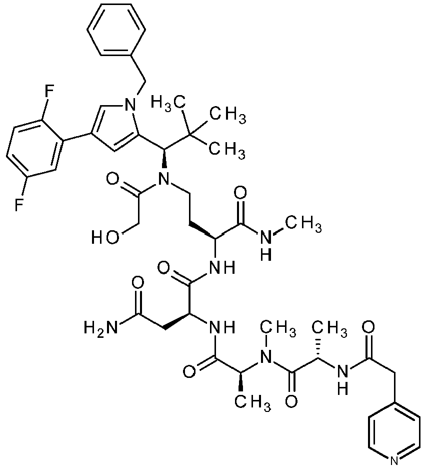

Zunächst wurde Trifluoressigsäure (2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1) wie in WO 2015096982 A1 beschrieben, hergestellt. Anschließend wurde aus diesem Intermediat durch Kupplung mit Intermediat L119 in DMF in Gegenwart von HATU und von N,N-Diisopropylethylamin die Titelverbindung hergestellt.

LC-MS (Methode 1): Rₜ = 0.83 min; MS (ESIpos): m/z = 916 [M+H]⁺.

Verbindung A wurde unter den oben beschriebenen Bedingungen von Legumain mit einer Halbwertszeit von 1.1 h zur Zielverbindung gespalten.

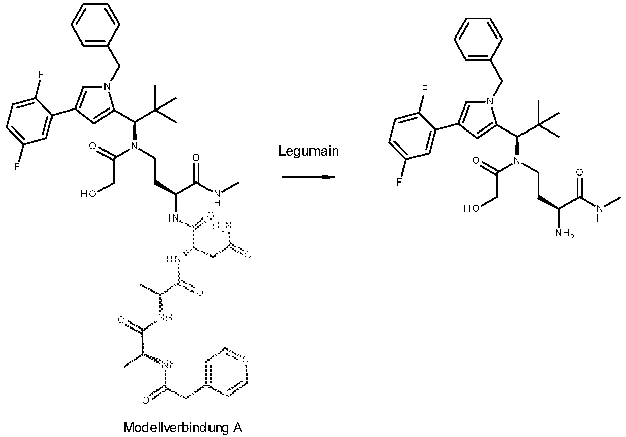

### C-2 Internalisierungsassay

Internalisierung ist der Schüsselprozess, um eine spezifische und effiziente Bereitstellung der zytotoxischen Payload in Antigen-exprimierenden Krebszellen durch Antikörper-Drug-Konjugate (ADC) zu ermöglichen. Dieser Prozess wird über Fluoreszenzmarkierung von spezifischen Antikörpern und einem Isotyp-Kontrollantikörper verfolgt. Hierzu wurde zunächst die Konjugation des Fluoreszenzfarbstoffes an Lysine des Antikörpers durchgeführt. Die Konjugation erfolgte mit zweifach molarem Überschuss von CypHer 5E mono NHS ester (Batch 357392, GE Healthcare) bei pH 8, 3. Nach erfolgter Kupplung wurde die Reaktionsmischung gelchromatographisch aufgereinigt (Zeba Spin Desalting Columns, 40K, Fa. Thermo Scientific, No. 87768; Elutionspuffer: DULBECCO'S PBS, Fa. Sima-Aldrich, No. D8537), um überschüssigen Farbstoff zu eliminieren und den pH-Wert zu adjustieren. Die Ankonzentrierung der Proteinlösung erfolgte mittels VIVASPIN 500 Säulen (Fa Sartorius stedim biotec). Die Bestimmung der dye load des Antikörpers erfolgte mittels spektrophotometrischer Analyse (Fa. NanoDrop) und anschließender Berechnung (D/P = A_{dye} εₚᵣₒₜₑᵢₙ:(A₂₈₀-0,16A_{dye})ε_{dye}).

Die dye load der hier untersuchten Antikörpern sowie der Isotyp-Kontrolle lagen in vergleichbarer Größenordnung. In Zellbindungs-Assays wurde getestet, dass die Kupplung zu keiner Affinitätsänderung der Antikörper führte.

Die markierten Antikörper wurden im Internalisierungs-Assays eingesetzt.

Vor dem Behandlungsstart wurden Zellen (2×10⁴/well) in 100µL Medium in einer 96-MTP ausgesät (fat, black, clear bottom No 4308776, Fa. Applied Biosystems). Nach 18h Inkubation bei 37°C/5%COz wurde das Medium gewechselt und markierte Antikörper in variierender Konzentration hinzugefügt (10, 5, 2.5, 1, 0.1µg/mL). Das gleiche Behandlungsschema erfolgte mit der markierten Isotyp-Kontrolle (negative control). Die gewählten Inkubationszeiten waren 0h, 0,25h, 0,5h, 1h, 1,5h 2h, 3h, 6h and 24h. Die Fluoreszenz- Messung wurde mit Hilfe des InCellAnalyzer 1000 (Fa. GE Healthcare) durchgeführt. Es erfolgte eine kinetische Evaluierung über die Messung der Parameter granule counts/cell und totale granule intensity/cell.

Antikörper wurden nach Bindung an den Rezeptor auf ihre Internalisierungsfähigkeit hin untersucht. Hierzu wurden Zellen mit verschiedenen Expressionsleveln des Rezeptors gewählt. Es konnte Target-vermittelte spezifische Internalisierung mit den Antikörpern beobachtet werden, wohingegen die Isotyp-Kontrolle keine Internalisierung zeigte.

### C-3 In vitro Tests zur Bestimmung der Zell-Permeabilität

Die Zell-Permeabilität einer Substanz kann mittels *in vitro*-Testung in einem Flux-Assay unter Verwendung von Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (AB SCIEX Deutschland GmbH, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von Pₐₚₚ (B-A) zu Pₐₚₚ (A-B) (efflux ratio) >2 oder <0.5 war.

Von entscheidender Bedeutung für Toxophore, die intrazellulär freigesetzt werden, sind die Permeabilität von B nach A [Pₐₚₚ (B-A)] und das Verhältnis von Pₐₚₚ (B-A) zu Pₐₚₚ (A-B) (efflux ratio): Je niedriger diese Permeabilität ist, desto langsamer sind die aktiven und passiven Transportvorgänge der Substanz durch die Monoschicht von Caco-2-Zellen. Gibt das efflux ratio zudem keine Hinweise auf aktiven Transport, kann die Substanz nach intrazellulärer Freisetzung länger in der Zelle verweilen. Damit steigt auch die Zeit, die für eine Interaktion mit dem biochemischen Target (hier: Kinesin-Spindelprotein, KSP / Eg5) zur Verfügung steht.

In der folgenden Tabelle 3 sind Permeabilitätsdaten repräsentativer Ausführungsbeispiele aus diesem Assay aufgeführt:

**Tabelle 3**

| **Ausführungsbeispiel** | **Pₐₚₚ (B-A) [nm/s]** | **Efflux ratio** |
|---|---|---|
| M1 | 7.8 | 4 |
| M2 | 4.8 | 6.4 |
| M3 | 1.4 | 1.3 |
| M4 | 21.3 | 18.7 |
| M5 | 20.3 | 26.5 |
| M6 | 1.7 | 0.7 |
| M7 | 5.6 | 2.2 |
| M9 | 213 | 16 |
| M11 | 24.3 | 27.7 |
| M12 | 3.3 | 1.8 |
| M13 | 7.1 | 3.6 |
| M14 | 12.7 | 6.6 |
| M15 | 6.4 | 4.4 |
| M16 | 9.0 | 7.0 |
| M17 | 93.6 | 81.5 |
| M18 | 1.6 | 2.9 |
| M19 | 1.9 | 2.9 |
| M21 | 0.5 | 1.5 |
| M22 | 0.9 | 0.9 |
| M23 | 2.8 | 2.0 |
| M24 | 3.9 | 1.0 |
| M25 | 8.1 | 3.6 |
| M26 | 13.0 | 9.6 |
| M27 | 13.2 | 11.9 |

### C-4 In vitro Tests zur Bestimmung der Substrateigenschaften für P-Glycoprotein (P-gp)

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis Pₐₚₚ (B-A) zu Pₐₚₚ (A-B) >2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

### C-5 Pharmakokinetik

### C5a: Identifizierung der ADC-Metabolite nach Internalisierung in vitro

### Methodenbeschreibung:

Internalisierungsuntersuchungen mit Immunkonjugaten werden durchgeführt, um intrazellulär entstandene Metaboliten zu analysieren. Hierzu werden humane Lungentumorzellen NCI H292 (3×10⁵/well) in 6-well Platten ausgesät und über Nacht inkubiert (37 °C, 5% COz). Es erfolgt eine Behandlung mit 10 µg/mL (66 nM) des zu untersuchenden ADCs. Die Internalisierung wurde bei 37 °C und 5% COz durchgeführt. Zu verschiedenen Zeitpunkten (0, 4, 24, 48, 72h) werden Zellproben zur weiteren Analyse genommen. Zunächst werden die Überstände (ca. 5 mL) geerntet und nach erfolgter Zentrifugation (2 min, RT, 1000 rpm Heraeus Variofuge 3.0R) bei -80 °C gelagert. Die Zellen werden mit PBS gewaschen, mit Accutase abgelöst und die Zellzahl bestimmt. Nach erneutem Waschen wird eine definierte Zellzahl (2×10⁵) mit 100 mL Lysis Puffer (Mammalian Cell Lysis Kit (Sigma MCL1) versetzt und unter ständigem Schütteln (Thermomixer, 15min, 4°C, 650 rpm) in Protein LoBind tubes (eppendorf Cat.No. 0030 108.116) inkubiert. Nach der Inkubation wird das Lysat zentrifugiert (10min, 4°C, 12000g, eppendorf 5415R) und der Überstand geerntet. Der gewonnene Überstand wird bei -80 °C gelagert. Alle Proben werden anschließend wie folgt analysiert.

Die Messung der Verbindungen im Kulturüberstand bzw. Zellysat erfolgt nach Fällung der Proteine mit Methanol oder Acetonitril durch eine Hochdruck-Flüssigkeits-Chromatographie (HPLC) gekoppelt an ein Triple-Quadrupol-Massenspektrometer (MS).

Zur Aufarbeitung von 50 µL Kulturüberstand/Zelllysat werden diese mit 150 µL Fällungsreagenz (in der Regel Acetonitril) versetzt und für 10 Sekunden geschüttelt. Das Fällungsreagenz enthält einen internen Standard (ISTD) in geeigneter Konzentration (in der Regel im Bereich 20-100 ng/mL). Nach dem 3minütigen Zentrifugieren bei 16000 g wird der Überstand in ein Autosampler-Vial überführt, mit 500 µL eines auf das Laufmittel abgestimmten Puffers aufgefüllt und erneut geschüttelt.

Die Messung beider Matrixproben erfolgt schließlich an dem mit einer HPLC gekoppelten Triple-Quadrupol-Massenspektrometer API6500 der Firma AB SCIEX Deutschland GmbH.

Zur Kalibrierung werden Plasmaproben mit Konzentrationen von 0.5 - 2000 µg/L versetzt. Die Nachweisgrenze (LOQ) liegt bei ca. 2 µg/L. Der lineare Bereich erstreckt sich von 2 bis 1000 µg/L.

Zur Kalibrierung der Tumorproben wird der Überstand unbehandelter Tumore mit Konzentrationen von 0.5 - 200 µg/L versetzt. Die Nachweisgrenze liegt bei 4 µg/L. Der lineare Bereich erstreckt sich von 4 bis 200 µg/L.

Qualitätskontrollen zur Gültigkeitsprüfung enthalten 5 und 50 µg/L.

### C5b: Identifizierung der ADC-Metabolite in vivo

Nach i.v. Applikation von 3-30 mg/kg verschiedener ADCs können die Plasma- und Tumorkonzentrationen des ADCs sowie potentiell auftretender Metaboliten gemessen und die pharmakokinetischen Parameter wie Clearance (CL), Fläche unter der Kurve (AUC) und Halbwertszeit (t_{1/2}) berechnet werden.

Als Referenzbeispiel (R10k) wurde ein ADC mit dem agonistischen Antikörper TPP-2658 mit einem nicht spaltbaren Linker hergestellt:

### Referenzbeispiel R10k

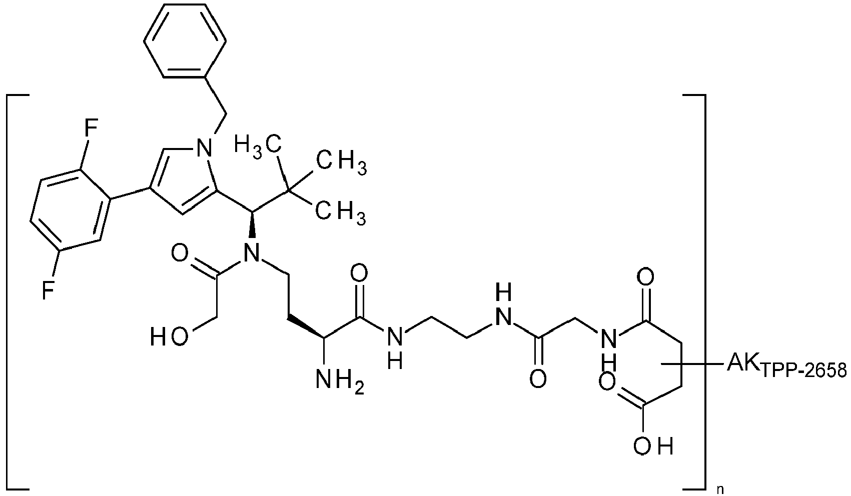

150 mg anti-TWEAKR Antikörper TPP-2658 in 10.5 mL ml PBS (c=14.28 mg/mL) wurden unter Argon mit einer Lösung aus 0.86 mg TCEP in 2 ml PBS-Puffer versetzt. Der Antikörper TPP-2658 und dessen Herstellung sind in WO 2015/189143 A1 im Detail beschrieben.

Der Ansatz wurde 30min bei RT gerührt und anschließend wurden 6.63 mg (0.008 mmol) von Intermediat F104 gelöst in 1250 µl DMSO zugegeben. Nach weiteren 90 min Rühren bei RT wurde der Ansatz mit 1250 µl PBS-Puffer, der zuvor auf pH 8 eingestellt wurde, verdünnt.

Diese Lösung wurde dann über mit PBS-Puffer pH8 equillibrierte PD 10-Säulen (Sephadex^{®} G-25, GE Healthcare) gegeben und mit PBS-Puffer pH8 eluiert. Das Eluat wurde mit PBS-Puffer pH8 auf ein Gesamtvolumen von 22.5 ml verdünnt. Diese Lösung wurde über Nacht bei RT unter Argon gerührt und anschließend wieder mittel PD-10 Säulen auf pH 7.2 umgepuffert. Anschließend wurde durch Ultrazentrifugation aufkonzentriert, rückverdünnt mit PBS-Puffer (pH 7.2) und erneut aufkonzentriert. Der erhaltene ADC Batch wurde folgendermaßen charakterisiert:
Protein Konzentration: 14.06 mg/mL
Drug/mAb Ratio: 3.4

### Analytik zur Quantifizierung der potentiell auftretenden Metabolite

Die Messung der Verbindungen in Tumor und Gewebe erfolgte nach Fällung der Proteine mit Methanol oder Acetonitril durch eine Hochdruck-Flüssigkeits-Chromatographie (HPLC) gekoppelt an ein Triple-Quadrupol-Massenspektrometer (MS).

Bei der Aufarbeitung eines Tumors oder Gewebes wurde dieser mit der 3fachen Menge an Extraktionspuffer versetzt. Der Extraktionspuffer enthielt 50 mL Tissue Protein Extraction Reagent (Pierce, Rockford, IL), zwei Pellets Complete-Protease-Inhibitor-Cocktail (Roche Diagnostics GmbH, Mannheim, Deutschland) und Phenylmethylsulfonylfluorid (Sigma, St. Louis, MO) in einer finaler Konzentration von 1 mM. Im Tissuelyser II (Qiagen) wird die Probe zweimal 20 Minuten bei maximaler Schlagzahl homogenisiert. 50 µL des Homogenats wurde in ein Autosampler-Vial überführt und mit 150 µL Methanol inklusive ISTD aufgefüllt. Nach 3minütigem Zentrifugieren bei 16000 g wurden 10 µL des Überstands mit 180 µl eines auf das Laufmittel abgestimmten Puffers aufgefüllt und erneut geschüttelt. Danach war die Tumorprobe messfertig.

Die Messung beider Matrixproben erfolgte schließlich an dem mit einer HPLC gekoppelten Triple-Quadrupol-Massenspektrometer API6500 der Firma AB SCIEX Deutschland GmbH.

Zur Kalibrierung der Tumor und Gewebeproben wurde der Überstand unbehandelter Tumore oder Gewebe mit Konzentrationen von 0.5 - 200 µg/L versetzt. Die Nachweisgrenze lag bei 3 µg/L. Der lineare Bereich erstreckt sich von 3 bis 200 µg/L.

Qualitätskontrollen zur Gültigkeitsprüfung enthalten 5 und 50 µg/L, in Plasma zusätzlich 500 µg/L. Tabelle: Katabolitkonzentrationen in NCI H292 Xenograft Maus -Tumor, -Leber und -Niere 24 h nach Applikation von 10 mg/kg des ADCs aus Beispiel 1k* (n=3) bzw. von 10 mg/kg des ADCs aus Referenzbeispiel R10k (n=2). Gemessener Katabolit war in beiden Fällen: M26.

| | | M26 | M26 |
|---|---|---|---|
| | | Mean [µg/L] | SD [µg/L] |
| Tumor | R10k | 98 | 10 |
| | Beispiel 1k* | 104 | 12 |
| Leber | R10k | 107 | 19 |
| | Beispiel 1k* | 63 | 6.9 |
| Niere | R10k | 96 | 19 |
| | Beispiel 1k* | 72 | 18 |

Nach Applikation des erfindungsgemäßen ADC Beispiels 1k* mit einem Legumain-spaltbaren Prodrug-Rest wurden im Tumor vergleichbare Konzentrationen vom aktiven Katabolit M26 gemessen als nach Applikation des ADCs mit dem gleichem Antikörper, jedoch ohne Legumainspaltbarem Prodrug-Rest. Im Gegensatz dazu lagen die gemessenen Konzentrationen des aktiven Metaboliten insbesondere in der Leber nach Gabe des ADCs aus Beispiel 1k* deutlich niedriger als nach Gabe des Referenz-ADCs R10k. Es kommt zu einer deutlich selektiveren Freisetzung des aktiven Wirkstoffes am Zielgewebe (Tumor) im Vergleich zu anderen Organen bei Verwendung eines Legumain-spaltbaren Prodrug-Rests auf dem Wirkstoff.

### Analytik zur Quantifizierung der verwendeten Antikörper

Der Antikörperteil der ADCs wurde mittels Liganden-Bindungs-Assay (ELISA) als Gesamt-IgG-Konzentration in Plasmaproben und Tumorlysaten bestimmt. Dabei wurde das Sandwich-ELISA-Format verwendet. Dieser ELISA war qualifiziert und validiert für die Bestimmung in Plasma- und Tumorproben. Die ELISA-Platten wurden mit anti-human-IgG-Fc-Antikörpern der Ziege beschichtet. Nach Inkubation mit der Probe wurden die Platten gewaschen und mit einem Detektor-Konjugat aus anti-human-IgG(H+L)-Antikörper des Affen und Meerrettichperoxidase (HRP) inkubiert. Nach einem weiteren Waschschritt wurde das HRP-Substrat OPD hinzugegeben und die Farbentwicklung über die Absorption bei 490 nm verfolgt. Standardproben mit bekannter IgG-Konzentration wurden mittels 4-Parameter-Gleichung gefittet. Innerhalb der unteren (LLOQ) und oberen (ULOQ) Quantifizierungsgrenzen wurden die unbekannten Konzentrationen über Interpolation ermittelt.

### C-6 Wirksamkeitstest in vivo

Die Wirksamkeit der erfindungsgemäßen Konjugate wurde in vivo beispielsweise mittels Xenograft-Modellen getestet. Der Fachmann kennt Methoden im Stand der Technik, anhand derer die Wirksamkeit der erfindungsgemäßen Verbindungen getestet werden kann (siehe z.B. WO 2005/081711; Polson et al., Cancer Res. 2009 Mar 15;69(6):2358-64). Beispielsweise wurde hierzu Nagern (z.B. Mäusen) eine Tumorzelllinie, welche das Zielmolekül des Binders exprimiert, inokuliert . Anschließend wurde den inokulierten Tieren entweder ein erfindungsgemäßes Konjugat, ein Isotyp-Antikörper-Kontrollkonjugate oder ein Kontrollantikörper oder isotonische Salzlösung appliziert. Die Applikation erfolgte einmalig oder öfter. Nach einer Inkubationszeit von mehreren Tagen wurde die Tumorgröße im Vergleich von Konjugat-behandelten Tieren und der Kontrollgruppe bestimmt. Die Konjugat-behandelten Tiere zeigten eine geringere Tumorgröße.

### C-6a. Wachstumshemmung / Regression von experimentellen Tumoren in der Maus

Humane Tumorzellen, die das Antigen für das Antikörper-Wirkstoffkonjugat exprimieren, werden subkutan in die Flanke von immunsupprimierten Mäusen inokuliert, beispielsweise NMRi Nude- oder SCID-Mäuse. 1-10 Millionen Zellen werden aus der Zellkultur abgelöst, zentrifugiert und mit Medium oder Medium / Matrigel resuspendiert. Die Zellsuspension wird unter die Haut der Maus gespritzt.

Innerhalb von einigen Tagen wächst ein Tumor heran. Die Behandlung beginnt nach Etablierung des Tumors, ungefähr bei einer Tumorgröße von 40 mm². Um die Wirkung auf größere Tumoren zu untersuchen, kann die Behandlung auch erst bei einer Tumorgröße von 50-100 mm² begonnen werden.

Die Behandlung mit APDCs und ADCs erfolgt über die intravenöse (i.v.) Route in die Schwanzvene der Maus. Das ADC wird mit einem Volumen von 5 mL / kg appliziert.

Das Behandlungsschema richtet sich nach der Pharmakokinetik des Antikörpers. Mit den erfindungsgemäßen Konjugaten wird als Standard einmal pro Woche für 2 oder 3 Wochen behandelt. Für eine zeitnahe Beurteilung kann sich auch ein Schema mit einer Einmalbehandlung eignen. Die Behandlung kann aber auch weiter fortgesetzt werden oder es kann sich zu einem späteren Zeitpunkt ein zweiter Zyklus mit drei Behandlungstagen anschließen.

Standardmäßig werden 8 Tiere pro Behandlungsgruppe eingesetzt. Neben den Gruppen, die die Wirksubstanzen bekommen, wird eine Gruppe als Kontrollgruppe nur mit dem Puffer nach dem gleichen Schema behandelt.

Im Verlauf des Experiments wird die Tumorfläche regelmäßig mit einer Schieblehre in zwei Dimensionen (Länge / Breite) gemessen. Die Tumorfläche wird mittels Länge x Breite bestimmt. Der Vergleich der mittleren Tumorfläche der Behandlungsgruppe mit der Kontrollgruppe wird als T/C area angegeben.

Werden alle Gruppen des Experimentes nach Behandlungsende gleichzeitig beendet, können die Tumore entnommen und gewogen werden. Der Vergleich der mittleren Tumorgewichte der Behandlungsgruppe mit der Kontrollgruppe wird als T/C weight angegeben.

### C-6b. Wirksamkeit der erfindungsgemäßen APDCs und ADCs in verschiedenen Tumormodellen

Die Tumorzellen werden subkutan in die Flanke von weiblichen NMRI-nude Mäusen (Janvier) inokuliert. Bei einer Tumorgröße von ~40 mm² wird intravenös mit dem Antikörper-Wirkstoffkonjugat behandelt. Im Anschluss an die Behandlung wird das Tumorwachstum ggf. weiterverfolgt.

Die Behandlung mit den erfindungsgemäßen APDCs führt zu einer deutlichen und zum Teil lang anhaltenden Wachstumshemmung der Tumore im Vergleich zur Kontrollgruppe und dem konjugierten Isotyp-Kontroll-Antikörper. Die Tabelle 8 gibt die T/C Werte an, ermittelt über die Tumorgewichte und Tumorfläche am jeweiligen Tag des Versuchsendes gerechnet nach Behandlungsstart.

**Tabelle 8:**

| **Beispiel** | **Tumor Modell** | **Dosis** | **Dosisschema** | **T/C area** |
|---|---|---|---|---|
| 1k* | LoVo (humanes kolorektales Karzinom) | 10 mg/kg | Q7dx3 | 0.53 (Tag 38, final) |
| 2k* | | 10 mg/kg | Q7dx3 | 0.43 (Tag 38, final) |
| 1k* | NCI-H292 (humanes nichtkleinzelliges Lungenkarzinom) | 10 mg/kg | Q7dx2 | 0.38 (Tag 11, final) |
| 2k* | | 10 mg/kg | Q7dx2 | 0.35 (Tag 11, final) |
| 1k* | KU-19-19 (humanes Blasenkarzinom) | 10 mg/kg | Q7dx2 | 0.19 (Tag 9, final) |

### Weitere Ausführungsformen:

1. Konjugat eines Binders oder Derivats hiervon mit einem oder mehreren Wirkstoffmolekülen bzw. einem oder mehreren Prodrugs hiervon der folgenden Formel: 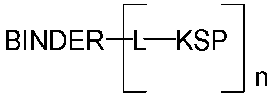 wobei
   - BINDER: für Binder bzw. ein Derivat hiervon , vorzugsweise einen Antikörper steht,
   - L: für einen Linker,
   - n: für eine Zahl von 1 bis 50, vorzugsweise 1 bis 20 und besonders bevorzugt 2 bis 8 steht, und
   - KSP: für einen Kinesin Spindel Protein-Inhibitor bzw. Prodrug hiervon steht, wobei -L-KSP die folgende Formel (IIa) aufweist: 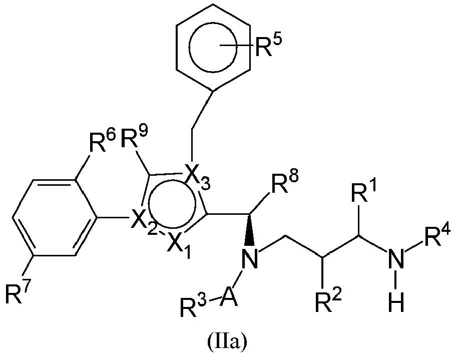 wobei
   X₁ N, X₂ N und X₃ C darstellt; oder
   X₁ N, X₂ C und X₃ N darstellt; oder
   X₁ CH oder CF, X₂ C und X₃ N darstellt; oder
   X₁ NH, X₂ C und X₃ C darstellt; oder
   X₁ CH, X₂ N und X₃ C darstellt;
   (wobei X₁ CH, X₂ C und X₃ N bevorzugt ist);
   - R¹: -H, -L-#1, -MOD oder -(CH₂)₀₋₃Z darstellt,
   wobei Z -H, -NHY³, -OY³, -SY³, Halogen, -C(=O)-NY¹Y², oder -CO-OY³ darstellt,
   wobei Y¹ und Y² unabhängig voneinander -H, -NH₂, -(CH₂CH₂O)₀₋₃-(CH₂)₀₋₃Z' (z.B. -(CH₂)₀₋₃Z'), oder -CH(CH₂W)Z' darstellen,
   Y³ -H oder -(CH₂)₀₋₃Z' darstellt, wobei Z` -H, -NH₂, -SO₃H, -COOH, -NH-C(=O)-CH₂-CH₂-CH(NH₂)COOH oder -(C(=O)-NH-CHY⁴)₁₋₃COOH darstellt;
   wobei W -H oder -OH darstellt,
   wobei Y⁴ lineares oder erzweigtes, gegebenenfalls mit -NH-C(=O)-NHz substituiertes, C₁₋₆ Alkyl oder gegebenenfalls mit -NH₂ substituiertes Aryl oder Benzyl darstellt;
   - R²: -L-#1, -H, -MOD, -C(=O)-CHY⁴-NHY⁵ oder -(CH₂)₀₋₃Z darstellt,
   wobei Z -H, Halogen, -OY³, -SY³, -NHY³, -C(=O)-NY¹Y², oder -C(=O)-OY³ darstellt,
   wobei Y¹ und Y² unabhängig voneinander -H, -NH₂, oder -(CH₂)₀₋₃Z' darstellen,
   wobei Y³ -H oder -(CH₂)₀₋₃Z' darstellt,
   wobei Z` -H, -SO₃H, -NH₂ oder -COOH darstellt;
   wobei Y⁴ lineares oder verzweigtes, gegebenenfalls mit wobei Y⁵ -NH-C(=O)-NHz substituiertes, C₁₋₆ Alkyl oder gegebenenfalls mit -NH₂ substituiertes Aryl oder Benzyl darstellt, -H oder -C(=O)-CHY⁶-NH₂ darstellt, wobei Y⁶
   lineares oder verzweigtes C₁₋₆ Alkyl darstellt;
   - R⁴: -L-#1, -H, -C(=O)-CHY⁴-NHY⁵ oder -(CH₂)₀₋₃Z darstellt,
   wobei Z -H, Halogen, -OY³, -SY³, -NHY³, -C(=O)-NY¹Y², oder -C(=O)-OY³ darstellt,
   wobei Y¹ und Y² unabhängig voneinander -H, -NH₂, oder -(CH₂)₀₋₃Z' darstellen,
   wobei Y³ -H oder -(CH₂)₀₋₃Z' darstellt,
   wobei Z' -H, -SO₃H, -NH₂ oder -COOH darstellt;
   wobei Y⁴ lineares oder verzweigtes, gegebenenfalls mit -NH-C(=O)-NH₂ substituiertes, C₁₋₆ Alkyl oder gegebenenfalls mit -NH₂ substituiertes Aryl oder Benzyl darstellt,
   wobei Y⁵ -H oder -C(=O)-CHY⁶-NH₂ darstellt,
   wobei Y⁶ lineares oder verzweigtes C₁₋₆ Alkyl darstellt; oder
   - R⁴: eine Gruppe der Formel R²¹-(C(=O))₍₀₋₁₎-(P3)₍₀₋₂₎-P2-NH-CH(CH₂C(=O)-NH₂)-C(=O)-oder R²¹-(C(=O))₍₀₋₁₎-(P3)₍₀₋₂₎-P2-NH-CH(CH₂COOH)-C(=O)- oder die durch Cathepsin spaltbare Gruppe der Formel R²¹-(C=O)₍₀₋₁₎-(P3)₍₁₋₂₎-P2-darstellt,
   wobei R²¹ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe
   wobei x 0 oder 1 ist darstellt, die ein oder mehrfach mit - NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)₂, oder -OH substituiert sein kann, -H oder eine Gruppe -Oₓ-(CH₂CH₂O)_{y}-R²² darstellt,
   wobei v eine Zahl von 1 bis 20 darstellt, und
   wobei R²² -H, -Alkyl (vorzugsweise C₁₋₁₂-Alkyl), -CH₂-COOH, -CH₂-CH₂-COOH, oder-CH₂-CH₂-NH₂) darstellt,
   wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   wobei P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist oder den jeweiligen N-Alkyl-Aminsoäuren, bevorzugt N-Methyl-Aminsoäuren; oder
   - R² und R⁴: gemeinsam (unter Bildung eines Pyrrolidinringes) -CH₂-CHR¹⁰- oder # -CHR¹⁰-CH₂- darstellen,
   wobei R¹⁰ -H, -NH₂, -SO₃H, -COOH, -SH, Halogen (insbesondere F oder Cl), C₁₋₄ Alkyl, C₁₋₄ Haloalkyl, C₁₋₄ Alkoxy, Hydroxyl-substituiertes C₁₋₄ Alkyl, COO(C₁₋₄ Alkyl), -OH oder R²¹-C(=O)-P3-P2-NH-CH(CH₂C(=O)-NH₂)-C(=O)-SIG-darstellt,
   wobei SIG eine self-immolative Gruppe darstellt, die nach Spaltung der C(=O)-SIG-Bindung das sekundäre Amin freisetzt;
   A -(C=O)-, -S(=O)-, -S(=O)z-, -S(=O)₂NH- oder -C(=N-NH₂)- darstellt;
   R³ -L-#1, -MOD, oder eine gegebenenfalls substituierte Alkyl-, Cycloalkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die jeweils substituiert sein können mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3-S-Alkyl Gruppen, 1-3 -O-C(=O)-Alkyl Gruppen, 1-3 -O-C(=O)-NH-Alkyl Gruppen, 1-3 -NH-C(=O)-Alkyl Gruppen, 1-3 -NH-C(=O)-NH-Alkyl Gruppen, 1-3 -S(O)ₙ-Alkyl Gruppen, 1-3 -S(=O)z-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen,
   wobei Z -H, Halogen, -OY³, -SY³, -NHY³, -C(=O)-NY¹Y², oder -C(=O)-OY³ darstellt,
   wobei n für 0, 1 oder 2 steht,
   wobeiY¹ und Y² unabhängig voneinander -H, -NH₂, oder -(CH₂)₀₋₃Z' darstellen,
   wobei Y³ -H, -(CH₂)₀₋₃-CH(NHOCH₃)Z', -(CH₂)₀₋₃-CH(NH₂)Z', oder -(CH₂)₀₋₃Z' darstellt,
   wobei Z' -H, -SO₃H, -NH₂ oder -COOH darstellt, substituiert sein kann (wobei "Alkyl" bevorzugt C₁₋₁₀-Alkyl bedeutet);
   R⁵ -H, -NH₂, -NOz, Halogen (insbesondere -F, -Cl, -Br), -CN, -CF₃, -OCF₃, -CH₂F, -CH₂F, -SH oder -(CH₂)₀₋₃Z darstellt,
   wobei Z -H, -OY³, -SY³, Halogen, -NHY³, -C(=O)-NY¹Y², oder -C(=O)-OY³ darstellt,
   wobei Y¹ und Y² unabhängig voneinander -H, -NH₂, oder -(CH₂)₀₋₃Z' darstellen,
   wobei Y³ -H oder -(CH₂)₀₋₃Z' darstellt,
   wobei Z' -H, -SO₃H, -NH₂ oder -COOH darstellt;
   R⁶ und R⁷ unabhängig voneinander -H, Cyano, C₁₋₁₀-Alkyl, Fluor-C₁₋₁₀-Alkyl, C₂₋₁₀-Alkenyl, Fluor-C₂₋₁₀-Alkenyl, C₂₋₁₀-Alkinyl, Fluor-C₂₋₁₀-Alkinyl, Hydroxy, -NOz, -NH₂, -COOH oder Halogen (insbesondere -F, -Cl, -Br) darstellen,
   R⁸ C₁₋₁₀-Alkyl, Fluor-C₁₋₁₀-Alkyl, C₂₋₁₀-Alkenyl, Fluor-C₂₋₁₀-Alkenyl, C₂₋₁₀-Alkinyl, Fluor-C₂₋₁₀-Alkinyl, C₄₋₁₀-Cycloalkyl, Fluor-C₄₋₁₀-Cycloalkyl, oder-(CH₂)₀₋₂-(HZ²) darstellt,
   wobei HZ² ein 4 bis 7-gliedriger Heterocyclus mit bis zu zwei Heteroatomen ausgewählt aus N, O und S darstellt, wobei jede dieser Gruppen mit -OH, -COOH, -NH₂ oder -L-#1 substituiert sein kann;
   R⁹ -H, -F, -CH₃, -CF₃, -CH₂F oder -CHF₂ darstellt;
   wobei einer der Substituenten R¹, R², R³, R⁴ und R⁸ -L-#1 darstellt bzw. (im Falle von R⁸) aufweist,
   --L- für den Linker und -# 1 für die Bindung zum Binder bzw. Derivat hiervon steht,
   wobei -MOD -(NR¹⁰)ₙ-(G1)ₒ-G2-G3 darstellt, wobei
   wobei R¹⁰ -H oder C₁-C₃-Alkyl darstellt;
   wobei G1 -NH-C(=O)-, -C(=O)-NH- oder 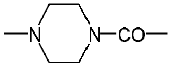 darstellt (wobei wenn G1 -NH-C(=O)- oder 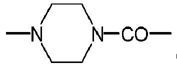 darstellt, R¹⁰ nicht -NH₂ ist);
   n 0 oder 1 ist;
   o 0 oder 1 ist;
   und
   wobei G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -S(=O)-, -S(=O)z-, -NRY-, -NRYC(=O)-, -C(=O)-NRY-, -NRYNRY-, -S(=O)₂-NR^{y}NR^{y}-, -C(=O)-NRYNRY-, -C(=O)-, -CR^{x}=N-O-unterbrochen sein kann,
   wobei R^{y} -H, Phenyl, C₁-C₁₀-Alkyl, C₂-C₁₀-Alkenyl, oder C₂-C₁₀-Alkinyl darstellt, die jeweils mit -NH-C(=O)-NH₂, -COOH, -OH, -NH₂, -NH-CN-NH₂, Sulfonamid, Sulfon, Sulfoxid, oder Sulfonsäure substituiert sein können
   wobei Rx -H, C₁-C₃-Alkyl oder Phenyl darstellt,

wobei die Kohlenwasserstoffkette einschließlich einer gegebenenfalls an der Kohlenwasserstoffgruppe substituierten C₁-C₁₀-Alkylgruppe als Seitenkette, sofern vorhanden, mit -NH-C(=O)-NH₂, -COOH, -OH, -NH₂, -NH-CN-NH₂, Sulfonamid, Sulfon, Sulfoxid, oder Sulfonsäure substituiert sein kann,
   - wobei: G3 -H oder -COOH darstellt;
   - wobei: -MOD vorzugsweise zumindest eine Gruppe -COOH aufweist;
wobei eine oder mehrere der folgenden Bedingungen (i) bis (iii) erfüllt ist:
   (i) -L-#1 - eine Gruppe der Formel -(C=O)₍₀₋₁₎-(P3)₍₀₋₂₎-P2-NH-CH(CH₂C(=O)-X)-C(=O)- umfasst,
      - wobei X: -NH₂ oder -COOH, vorzugsweise -NH₂ darstellt;
      - wobei: P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin, und His ist,
      - wobei: P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   (ii) R⁴ die Gruppe der Formel R²¹-(C=O)₍₀₋₁₎-(P3)₍₀₋₂₎-P2-NH-CH(CH₂C(=O)-NH₂)-C(=O)- oder R²¹-(C=O)₍₀₋₁₎-(P3)₍₀₋₂₎-P2-NH-CH(CH₂COOH)-C(=O)- oder die durch Cathepsin spaltbare Gruppe der Formel R²¹-(C=O)₍₀₋₁₎-(P3)₍₁₋₂₎-P2- darstellt;
      - wobei R²¹: eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)₂, oder -OH substituiert sein kann, -H oder eine Gruppe -Oₓ-(CH₂CH₂O)_{y}-R²² darstellt,
      - wobei: x 0 oder 1 ist
      - wobei: v eine Zahl von 1 bis 20 darstellt, und
      - wobei R²²: -H, -Alkyl (vorzugsweise C₁₋₁₂-Alkyl), -CH₂-COOH, -CH₂-CH₂-COOH, oder -CH₂-CH₂-NH₂) darstellt,
      - wobei: P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
      - wobei: P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist oder den jeweiligen N-Alkyl-Aminsoäuren, bevorzugt N-Methyl-Aminsoäuren;
   (iii) R² und R⁴ gemeinsam (unter Bildung eines Pyrrolidinringes) -CH₂-CHR¹⁰- oder -CHR¹⁰-CH₂- darstellen, wobei das sekundäre Wasserstoffatom der sekundären Amingruppe des Pyrrolidinrings durch R²¹-C(=O)-P3-P2-NH-CH(CH₂C(=O)-NH₂)-C(=O)-SIG-ersetzt ist,
      - wobei: SIG eine self-immolative Gruppe darstellt, die nach Spaltung der C(=O)-SIG-Bindung das sekundäre Amin freisetzt und
      - wobei R²¹: eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)₂, oder -OH substituiert sein kann, -H oder eine Gruppe -Oₓ-(CH₂CH₂O)_{y}-R²² darstellt,
      - wobei x 0 oder 1 ist wobei: v eine Zahl von 1 bis 20 darstellt, und
      - wobei R²²: -H, -Alkyl (vorzugsweise C₁₋₁₂-Alkyl), -CH₂-COOH, -CH₂-CH₂-COOH, oder -CH₂-CH₂-NH₂) darstellt,
      - wobei: P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
      - wobei: P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist oder den jeweiligen N-Alkyl-Aminsoäuren , bevorzugt N-Methyl-Aminsoäuren;
   sowie ihre Salze, Solvate, Salze der Solvate und Epimere.
2. Konjugat nach Ausführungsform 1, wobei R⁴ die Gruppe der Formel R²¹-(C=O)₍₀₋₁₎-(P3)₍₀₋₂₎-P2-NH-CH(CH₂C(=O)NH₂)-C(=O)- darstellt.
3. Konjugat nach Ausführungsform 1 oder 2, wobei P2 ausgewählt wird aus Ala, Gly, Val, Leu, Ile, Pro, Ser, Thr und Asn.
4. Konjugat nach einer oder mehrerer der vorhergehenden Ausführungsformen, wobei P3 ausgewählt wird aus Pro, Ala, Val, Leu, Ile, Gly, Ser und Gln.
5. Konjugat nach einer oder mehrerer der vorhergehenden Ausführungsformen, wobei P2 Ala ist.
6. Konjugat nach einer oder mehrerer der vorhergehenden Ausführungsformen, wobei P3 Ala ist.
7. Konjugat nach einer oder mehrerer der vorhergehenden Ausführungsformen,
   - wobei R²¹: -H, eine C₁₋₅-Alkyl-, C₅₋₁₀-Aralkyl-, C₁₋₅-Alkoxy-, C₆₋₁₀-Aryloxy-Gruppe, C₅₋₁₀-Heteroalkyl-, C₅₋₁₀-Heterocycloalkyl-, Heteroaryl-, Heteroaryl-alkyl-, C₅₋₁₀-Heteroalkoxy- oder eine C₅₋₁₀-Heterocycloalkoxy-Gruppe darstellt, die jeweils mit -COOH, -C(=O)-OAlkyl, -C(=O)O-NH₂, -NH₂ oder -N(Alkyl)₂, substituiert sein kann, oder eine Gruppe -Oₓ-(CH₂CH₂O)_{y}-R²² darstellt,
   wobei x 0 oder 1 ist,
   wobei v eine Zahl von 1 bis 20 darstellt, und
   R²² -H, -Alkyl (vorzugsweise C₁₋₁₂-Alkyl), -CH2-COOH, -CH₂-CH₂-COOH, oder -CH₂-CH₂-NH₂) bedeutet.
8. Konjugat nach einer oder mehrerer der vorhergehenden Ausführungsformen,
   - wobei: R⁴ -L-#1 darstellt und die Gruppe der Formel -(C=O)₍₀₋₁₎-(P3)₍₀₋₂₎-P2-NH-CH(CH₂C(=O)-NH₂)-C(=O)- umfasst,
   wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist und
   wobei P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist oder den jeweiligen N-Alkyl-Aminsoäuren , bevorzugt N-Methyl-Aminsoäuren.
9. Konjugat nach einer oder mehrerer der vorhergehenden Ausführungsformen 1 bis 7, wobei das Konjugat ein Binder-Prodrug-Konjugat ist,
   - wobei: R⁴ die Gruppe der Formel R²¹-(C=O)₍₀₋₁₎-(P3)₍₀₋₂₎-P2-NH-CH(CH₂C(=O)-NH₂)-C(=O)-darstellt,
   wobei R²¹ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NHz, -C(=O)-N(Alkyl)₂, oder -OH substituiert sein kann, oder -H oder eine Gruppe -Ox-(CH₂CH₂O)v-R²² darstellt,
   wobei x 0 oder 1 ist
   wobein v eine Zahl von 1 bis 20 darstellt, und
   wobei R²² -H, -Alkyl (vorzugsweise C₁₋₁₂-Alkyl), -CH₂-COOH, -CH₂-CH₂-COOH, oder -CH₂-CH₂-NH₂) darstellt,
   wobei P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   wobei P3 eine Aminosäure ist die jeweils unabhängig ausgewählt wird aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist oder den jeweiligen N-Alkyl-Aminsoäuren, bevorzugt N-Methyl-Aminsoäuren.
10. Konjugat nach nach einer oder mehrerer der vorhergehenden Ausführungsformen, wobei R¹ oder R³ -L-#1 darstellen.
11. Konjugat nach einer oder mehrerer der vorhergehenden Ausführungsformen, wobei X₁ CH, X₂ C und X₃ N ist.
12. Konjugat nach einer oder mehrerer der vorhergehenden Ausführungsformen, wobei R⁶ und R⁷ unabhängig voneinander -H, C₁₋₃-Alkyl oder Halogen darstellen.
13. Konjugat nach Ausführungsform 12, wobei R⁶ und R⁷ -F darstellen.
14. Konjugat nach einer oder mehrerer der vorhergehenden Ausführungsformen, wobei R⁸ C₁₋₄-Alkyl, vorzugsweise tert-Butyl) oder Cylcohexyl darstellt.
15. Konjugat nach einer oder mehrerer der vorhergehenden Ausführungsformen, wobei R⁹ H darstellt.
16. Konjugat nach einer oder mehrerer der vorhergehenden Ausführungsformen, wobei der Binder oder Derivat hiervon ein Binderpeptid oder -protein oder ein Derivat eines Binderpeptids oder -proteins ist.
17. Konjugat nach Ausführungsform 16, wobei jedes Wirkstoffmolekül an verschiedene Aminosäuren des Binderpeptids oder -proteins bzw. Derivats hiervon über den Linker gebunden ist.
18. Konjugat nach einer oder mehrerer der vorhergehenden Ausführungsformen, wobei das Konjugat im Durchschnitt 1,2 bis 20 Wirkstoffmoleküle bzw. Prodrugs pro Binder aufweist.
19. Konjugat nach einer oder mehrerer der vorhergehenden Ausführungsformen 16 bis 18, wobei das Binderpeptid oder -protein einen Antikörper bzw. das Derivat des Binderpeptids 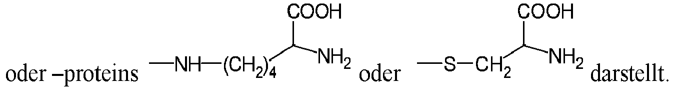
20. Konjugat nach einer oder mehrerer der vorhergehenden Ausführungsformen, wobei der Binder an ein Krebs-Zielmolekül bindet.
21. Konjugat nach Ausführungsform 20, wobei der Binder an ein extrazelluläres Zielmolekül bindet.
22. Konjugat nach Ausführungsform 21, wobei der Binder nach Bindung an das extrazelluläre Zielmolekül von der das Zielmolekül exprimierenden Zelle internalisiert und intrazellulär (vorzugsweise lysosomal) prozessiert wird.
23. Konjugat nach einer oder mehrerer der vorhergehenden Ausführungsformen 16 bis 22, wobei das Binderpeptid oder -protein ein humaner, humanisierter oder chimärer monoklonaler Antikörper oder ein Antigen-bindendes Fragment hiervon ist.
24. Konjugat nach Ausführungsform 23, wobei das Binderpeptid oder -protein ein anti-HER2-Antikörper, ein anti-EGFR-Antikörper, ein anti-TWEAKR-Antikörper, oder ein Antigen-bindendes Fragment hiervon ist.
25. Konjugat nach Ausführungsform 24, wobei der anti-TWEAKR-Antikörper spezifisch an die Aminosäure D in Position 47 (D47) von TWEAKR (SEQ ID NO:169) bindet, bevorzugt der anti-TWEAKR-Antikörper TPP-2090.
26. Konjugat nach einer oder mehrerer der vorhergehenden Ausführungsformen 24 und 25, wobei der anti-TWEAKR-Antikörper spezifisch an die Aminosäure D in Position 47 (D47) von TWEAKR (SEQ ID NO:169) bindet, bevorzugt der anti-TWEAKR-Antikörper TPP-2658.
27. Konjugat nach Ausführungsform 24, wobei das Binderpeptid oder -protein ein anti-EGFR-Antikörper ist und R³ -L-#1 darstellt.
28. Konjugat nach einer oder mehrerer der vorhergehenden Ausführungsformen, wobei der Linker -L- eine der folgenden Grundstrukturen (i) bis (iv) aufweist:
   (i) -(C=O)ₘ-SG1-L1-L2-
   (ii) -(C=O)ₘ -L1-SG-L1-L2-
   (iii) -(C=O)ₘ -L1-L2-
   (iv) -(C=O)ₘ -L1-SG-L2-
   wobei m 0 oder 1 ist, SG und SG1 eine *in vivo* spaltbare Gruppe ist, L1 unabhängig voneinander für *in vivo* nicht spaltbare organische Gruppen stehen, und L2 für eine Kopplungsgruppe an den Binder steht.
29. Konjugat nach Ausführungsform 28, wobei die *in vivo* spaltbare Gruppe SG eine 2-8 Oligopeptidgruppe, bevorzugt eine Dipeptidgruppe oder ein Disulfid, ein Hydrazon, ein Acetal oder ein Aminal ist und SG1 eine 2-8 Oligopeptidgruppe, bevorzugt eine Dipeptidgruppe ist.
30. Konjugat nach einer oder mehrerer der vorhergehenden Ausführungsformen, wobei der Linker an eine Cystein-Seitenkette bzw. Cysteinrest gebunden ist und die folgende Formel aufweist:

   §-(C=O)m-L1-L2-§ §

   wobei
   m 0 oder 1 ist;
   § die Bindung an das Wirkstoffmolekül darstellt und
   §§ die Bindung an das Binderpeptid oder -protein darstellt, und
   -L2- für 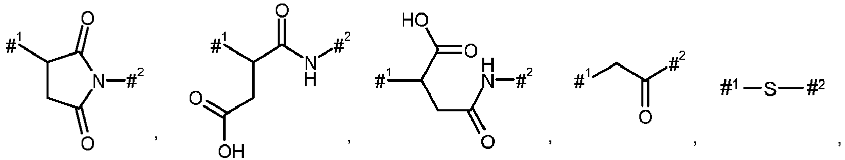 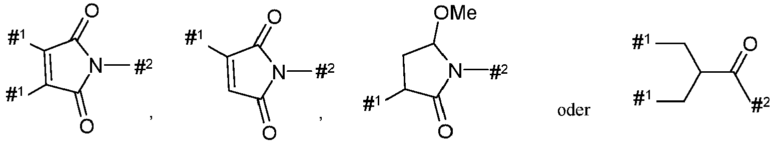 steht, wobei
      - #¹: die Verknüpfungsstelle mit dem Schwefelatom des Binders kennzeichnet,
      - #²: die Verknüpfungsstelle mit der Gruppe L1 kennzeichnet,
      - L1: -(NR¹⁰)ₙ-(G1)o-G2- darstellt, wobei
      - R¹⁰: -H, -NH₂ oder C₁-C₃-Alkyl darstellt;
      - G1: -NH-C(=O)- oder 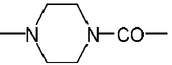 darstellt;
      - n: 0 oder 1 ist;
      - o: 0 oder 1 ist; und
      - G2: eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, -S(=O)z-, -NH-, -C(=O)-, -NMe-, -NHNH-, -S(=O)z-NHNH-,-NH-C(=O)-, -C(=O)-NH-, -C(=O)-NHNH-und einen 5 bis 10gliedriger, aromatischen oder nicht aromatischen Heterozyklus mit bis zu 4 Heteroatomen ausgewählt aus N, O und S, -S(=O)- oder -S(=O)₂-unterbrochen sein kann (vorzugsweise 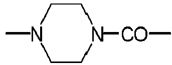 ), wobei die Seitenketten, sofern vorhanden, mit -NH-C(=O)-NHz, -COOH, -OH, -NH₂, -NH-CN-NH₂, Sulfonamid, Sulfon, Sulfoxid oder Sulfonsäure substituiert sein können, oder eine der folgenden Gruppen darstellt: 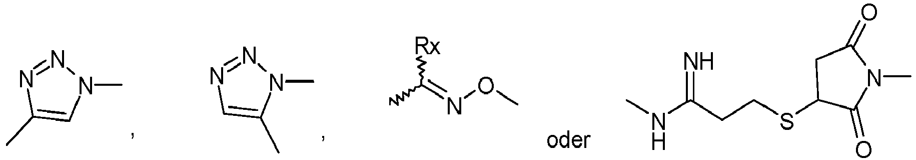
   wobei Rx -H, -C₁-C₃-Alkyl oder Phenyl darstellt.
31. Konjugat nach Ausführungsform 30, wobei L2 dargestellt wird durch eine oder beide der folgenden Formeln: 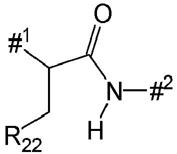 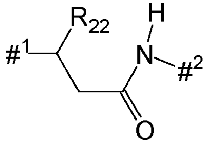 wobei
   - #¹: die Verknüpfungsstelle mit dem Schwefelatom des Binders kennzeichnet,
   - #²: die Verknüpfungsstelle mit der Gruppe L¹ kennzeichnet,
   - R²²: -COOH darstellt, und die Bindungen an das Schwefelatom des Binders zu über 80 %, (bezogen auf die Gesamtanzahl von Bindungen des Linkers an den Binder) in einer dieser beiden Strukturen vorliegen.
32. Konjugat nach einer oder mehrerer der vorhergehenden Ausführungsformen 30 oder 31, wobei L¹ die folgenden Formeln aufweist: 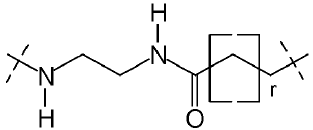 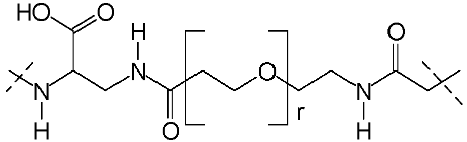 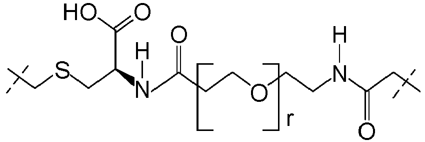 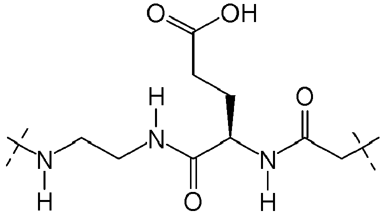 in denen
   r eine Zahl von 0 bis 8 ist.
33. Konjugat nach einer oder mehrerer der vorhergehenden Ausführungsformen 30 bis 32, wobei die Kohlenwasserstoffkette durch eine die folgenden Gruppen unterbrochen ist: 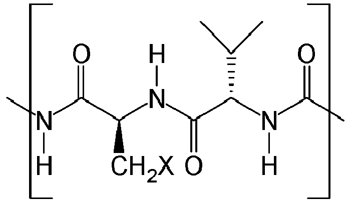 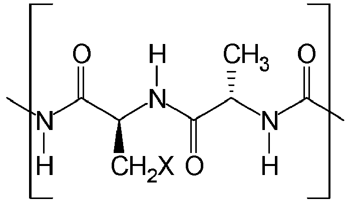 wobei X -H oder eine C₁₋₁₀-Alkylgruppe ist, die gegebenenfalls mit -NH-C(=O)-NH₂, -COOH, -OH, -NH₂, -NH-CN-NH₂, Sulfon, Sulfoxid oder Sulfonsäure substituiert sein kann.
34. Konjugat nach Ausführungsform 30, wobei der Linker die folgende Formel aufweist: 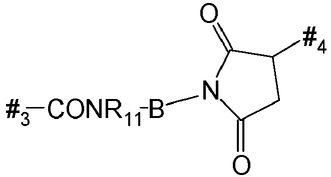 wobei
   - #3: die Bindung an das Wirkstoffmolekül darstellt,
   - #4: die Bindung an das Binderpeptid oder -protein darstellt,
   - R¹¹: -NH₂ darstellt;
   - B: -[(CH₂)ₓ-(X⁴)_{y}]_{w}-(CH₂)_{z}- darstellt,
   - w: 0 bis 20 ist;
   - x: 0 bis 5 ist;
   - y: 0 oder 1 ist;
   - z: 0 bis 5 ist; und

   - X⁴: -O-, -C(=O)-NH- oder -NH-C(=O)- 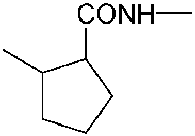 darstellt.
35. Konjugat nach einer oder mehrerer der vorhergehenden Ausführungsformen 26 bis 30, wobei das Konjugat eine der folgenden Formeln genügt, 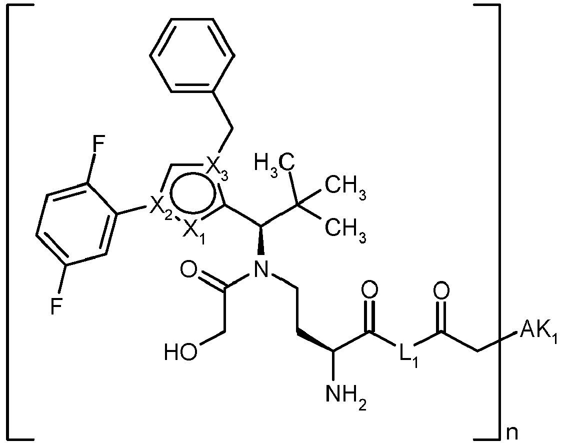 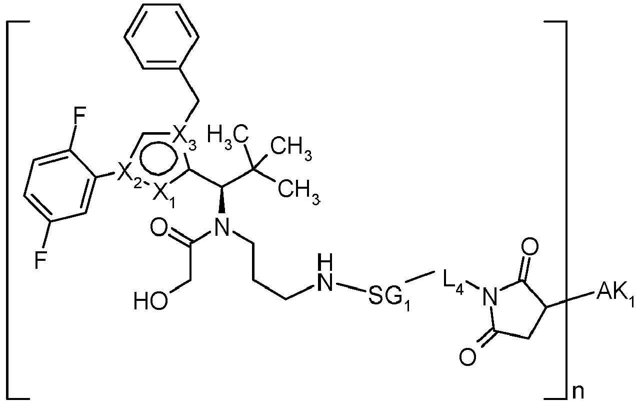 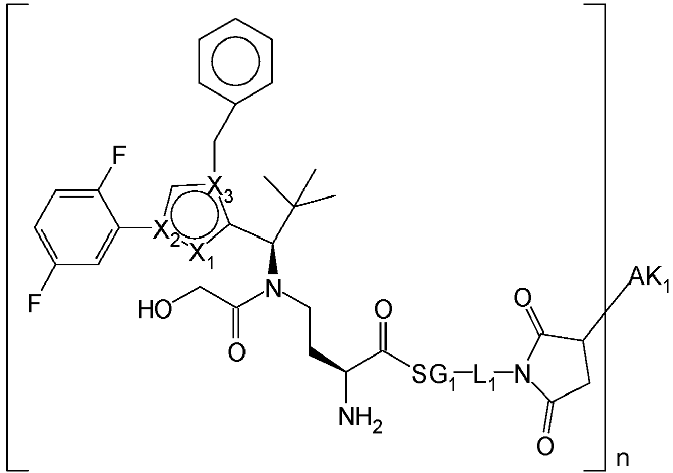 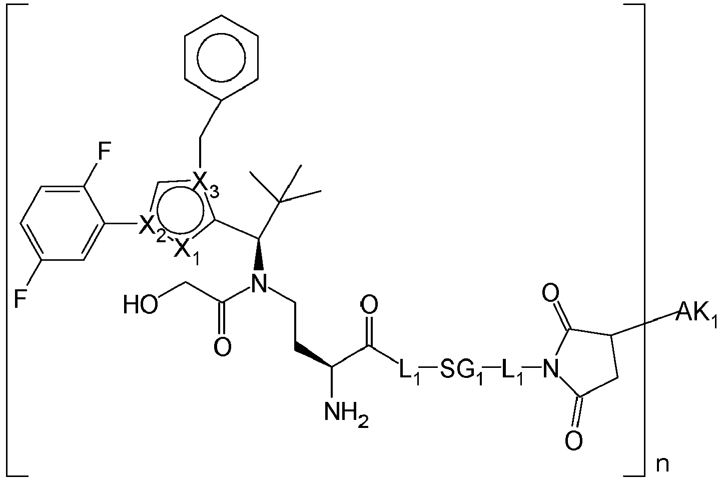 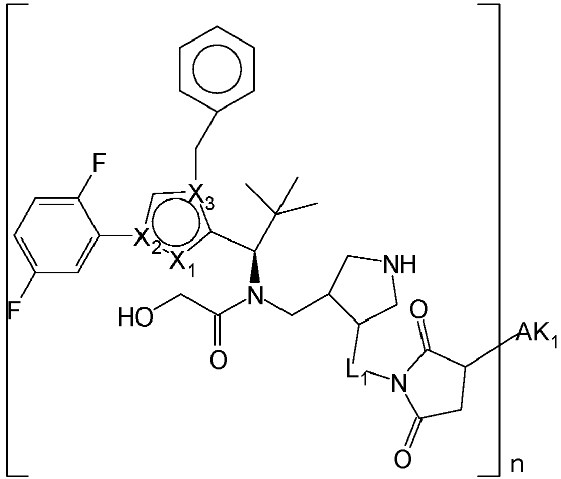 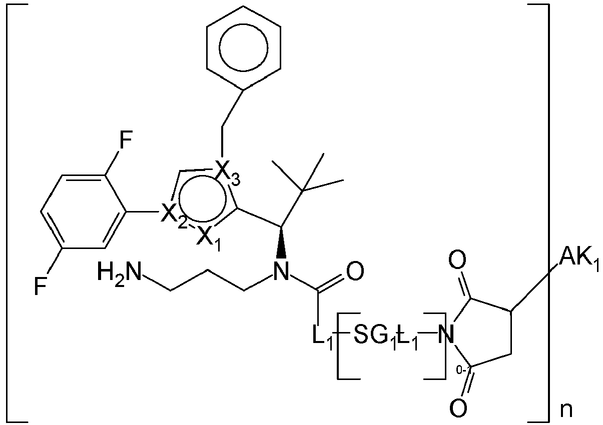 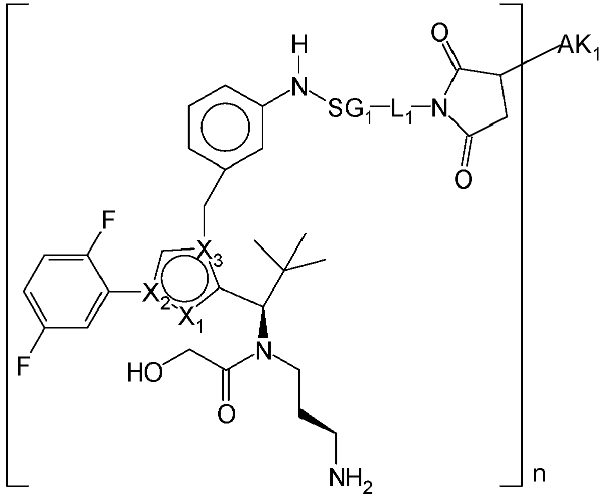 wobei
   - X₁, X₂ und: X₃ die gleiche Bedeutung aufweisen wie in Anspruch 1,
   - AK₁: ein Binderpeptid oder-protein darstellt, das über ein Schwefelatom des Binders gebunden ist;
   - n: eine Zahl von 1 bis 20 darstellt; und
   - L₁: eine gegebenenfalls verzweigte Kohlenwasserstoffgruppe mit 1 bis 70 Kohlenstoffatomen ist, oder eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, -SO₂-, -NH-, -C(=O)-, -C(=O)-NH-, -NH-C(=O)-, -NMe-, -NHNH-, -S(=O)z-NHNH-, -C(=O)-NHNH- und einen 5 bis 10gliedriger, aromatischen oder nicht aromatischen Heterozyklus mit bis zu 4 Heteroatomen ausgewählt aus N, O und S, -S(=O)- oder -S(=O)₂-unterbrochen sein kann, wobei die Seitenketten, sofern vorhanden, mit -NH-C(=O)-NHz, -COOH, -OH, -NH₂, -NH-CN-NH₂, Sulfonamid, Sulfon, Sulfoxid oder Sulfonsäure substituiert sein können, und
   - SG1: ein 2-8 Oligopeptid, bevorzugt ein Dipeptid ist;
   - L4: eine Einfachbindung oder eine Gruppe -(C=O)_{y}-G4- darstellt,
   wobei y 0 oder 1 darstellt, und
   wobei G4 eine geradkettige oder verzweigte Kohlenwasserstoffkette mit 1 bis 100
   Kohlenstoffatomen und/oder einfach -O-, aus Arylengruppen und/oder geradkettigen verzweigten und/oder cyclischen Alkylengruppen ist, die oder mehrfach durch eine oder mehrere der Gruppen -S-, -S(=O)-, -S(=O)z-, -NH-, -C(=O)-, -NH-C(=O)-, -C(=O)-NH-, -Nme-, -NHNH-, -S(=O)z-NHNH-, -C(=O)-NHNH-und einen 5 bis 10gliedriger, aromatischen oder nicht aromatischen Heterozyklus mit bis zu 4 Heteroatomen ausgewählt aus N, O und S, -S(=O)-oder -S(=O)z- unterbrochen sein kann, wobei die Seitenketten, sofern vorhanden, mit -NH-C(=O)-NHz, -COOH, -OH, -NH₂, -NH-CN-NH₂, Sulfonamid, Sulfon, Sulfoxid oder Sulfonsäure substituiert sein können; wobei das Wasserstoffatom in Position R⁴ gemäß Formel IIa (d.h. in der Gruppe -NH₂) durch Legumain spaltbare Gruppe der Formel R²¹-(C=O)₍₀₋₁₎-(P3)₍₀₋₂₎-P2-NH-CH(CH₂C(=O)-NH₂)-C(=O)-ersetzt ist, wobei
   R²¹ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)₂, oder -OH substituiert sein kann, -H oder eine Gruppe -Ox-(CH₂CH₂O)v-R²² darstellt,
   wobei x 0 oder 1 ist,
   wobei v eine Zahl von 1 bis 20 ist und
   R²² -H, -Alkyl (vorzugsweise C₁₋₁₂-Alkyl), -CH₂-COOH, -CH₂-CH₂-COOH, oder -CH₂-CH₂-NH₂ darstellt;
   P2 eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist;
   P3 eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His oder eine der jeweiligen N-Alkyl-Aminsoäuren, bevorzugt N-Methyl-Aminsoäuren ist.
36. Konjugat eines Binders oder Derivats hiervon mit einem oder mehreren Wirkstoffmolekülen, das Konjugat eine oder beide der folgenden Formeln aufweist: 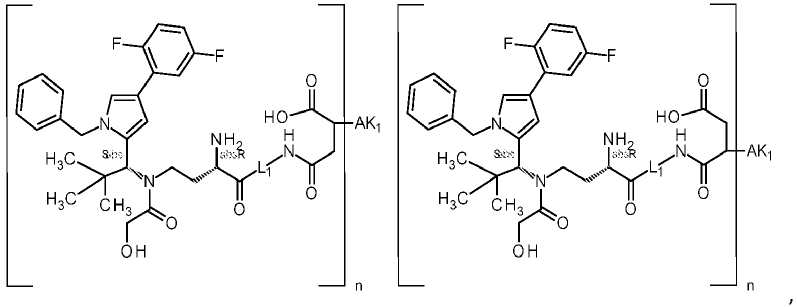 wobei
   - AK₁ bzw.: AK_{1A} ein Binderpeptid oder-protein darstellt, das über ein Schwefelatom des Binders gebunden ist;
   - n: eine Zahl von 1 bis 20 darstellt; und
   - L₁: eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -S(=O)-, -S(=O)z-, -NH-, -C(=O)-, -C(=O)-NH-, -NH-C(=O)-, -NMe-, -NHNH-, -S(=O)z-NHNH-, -C(=O)-NHNH- und einen 5 bis 10gliedriger, aromatischen oder nicht aromatischen Heterozyklus mit bis zu 4 Heteroatomen ausgewählt aus N, O und S, -S(=O)- oder -S(=O)₂-unterbrochen sein kann, wobei die Seitenketten, sofern vorhanden, mit -NH-C(=O)-NHz, -COOH, -OH, -NH₂, -NH-CN-NH₂, Sulfonamid, Sulfon, Sulfoxid oder Sulfonsäure substituiert sein können. wobei das Wasserstoffatom in Position R⁴ gemäß Formel IIa (d.h. in der Gruppe -NH₂) durch eine Legumain spaltbare Gruppe der Formel R²¹-(C=O)₍₀₋₁₎-(P3)₍₀₋₂₎-P2-NH-CH(CH₂-C(=O)-NH₂)-C(=O)- ersetzt ist, wobei
   - R²¹: eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit -NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)₂, oder -OH substituiert sein kann, -H oder eine Gruppe -Ox-(CH₂CH₂O)v-R²² darstellt,
   wobei x 0 oder 1 ist,
   wobei v eine Zahl von 1 bis 20 darstellt, und
   R²² -H, -Alkyl (vorzugsweise C₁₋₁₂-Alkyl), -CH₂-COOH, -CH₂-CH₂-COOH, oder -CH₂-CH₂-NH₂) ist;
   - P2: eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist;
   - P3: eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His oder eine der jeweiligen N-Alkyl-Aminsoäuren, bevorzugt N-Methyl-Aminsoäuren ist.
37. Konjugat nach einer oder mehrerer der vorhergehenden Ausführungsformen, wobei der Linker -L- an eine Cystein-Seitenkette bzw. einen Cysteinrest gebunden ist und die folgende Formel aufweist: 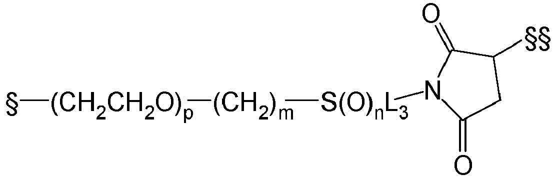 wobei
   - §: die Bindung an das Wirkstoffmolekül darstellt und
   - §§: die Bindung an das Binderpeptid oder -protein darstellt,
   - m: 0, 1, 2, oder 3 ist;
   - n: 0, 1 oder 2 ist;
   - p: 0 bis 20 beträgt; und
   - L3: die Gruppe 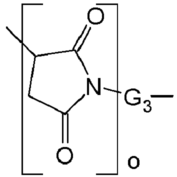 darstellt; wobei
   - o: 0 oder 1 ist; und
   - G3: eine geradkettige oder verzweigte Kohlenwasserstoffkette mit 1 bis 100 Kohlenstoffatomen aus Arylgruppen und/oder geradkettigen und/oder verzweigten und/oder cyclischen Alkylgruppen ist, die einfach oder mehrfach durch eine oder mehrere der Gruppen -O-, -S-, -S(=O)-, -S(=O)z-, -NH-, -C(=O)-, -NH-C(=O)-, -C(=O)-NH- und einen 5 bis 10gliedriger, aromatischen oder nicht aromatischen Heterozyklus mit bis zu 4 Heteroatomen ausgewählt aus N, O und S, -NMe-, -NHNH-, -S(=O)z-NHNH-, -C(=O)-NHNH-, -S(=O)- oder -S(=O)z- unterbrochen sein kann, wobei die Seitenketten, sofern vorhanden, mit -NH-C(=O)-NHz, -COOH, -OH, Sulfon, Sulfoxid oder Sulfonsäure substituiert sein können.
38. Konjugat nach Anspruch 37, wobei der Linker -L- an eine Cystein-Seitenkette bzw. Cysteinrest gebunden ist und die folgende Formel aufweist: 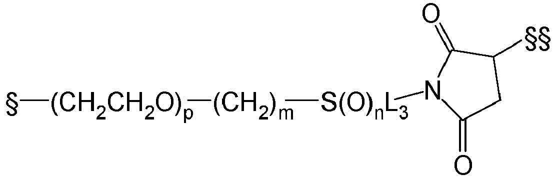 wobei
   - §: die Bindung an das Wirkstoffmolekül darstellt und
   - §§: die Bindung an das Binderpeptid oder -protein darstellt,
   - m: 1 ist;
   - p: 0 ist;
   - n: 0 ist; und
   - L3: die Gruppe 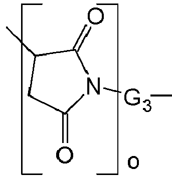 darstellt; wobei
   - o: 0 oder 1 ist; und
   - G3: -(CH₂CH₂O)ₛ(CH₂)ₜ(CONH)ᵤ (CH₂CH₂O)ᵥ(CH₂)_{w}- darstellt, wobei
   - s,: t, v und w jeweils unabhängig voneinander 0 bis 20 ist, und
   - u: 0 oder 1 ist.
39. Konjugat nach einer oder mehrerer der vorhergehenden Ausführungsformen, wobei R² oder R³ -L-# 1 darstellt.
40. Konjugat nach Ausführungsform 39, wobei das Konjugat eine der folgenden Formeln aufweist: 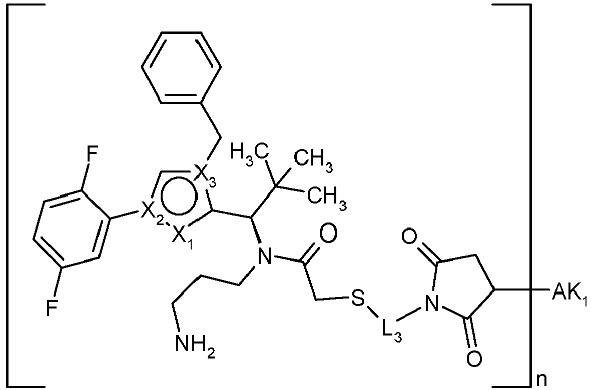 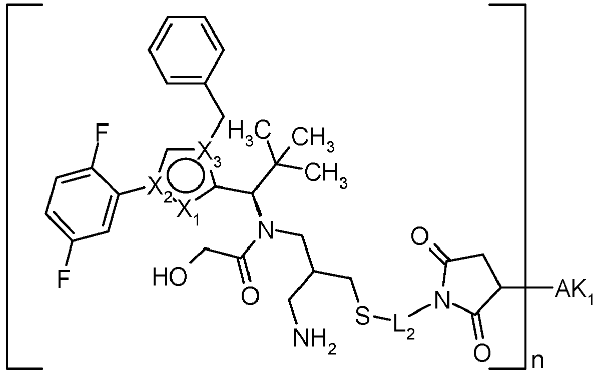 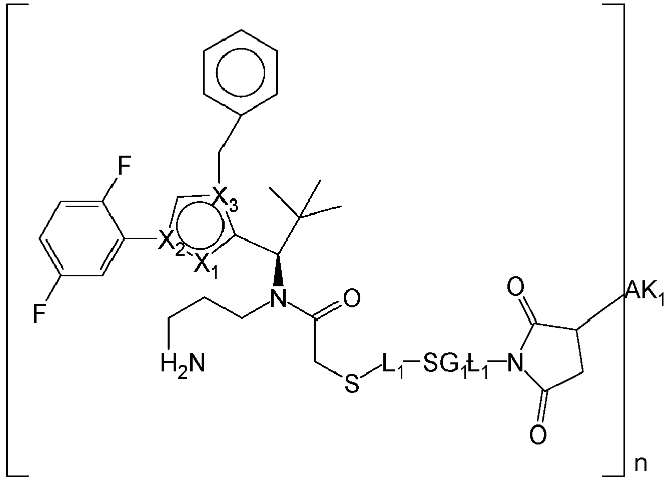 wobei
   - X₁, X₂ und: X₃ die gleiche Bedeutung aufweisen wie in Anspruch 4, AK₁ ein Binderpeptid oder-protein darstellt, das über ein Schwefelatom des Binders angebunden ist;
   - n: eine Zahl von 1 bis 20 darstellt; und
   - L₁: eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -S(=O)-, -S(=O)z-, -NH-, -C(=O)-, -C(=O)-NH-, -NH-C(=O)-, -NMe-, -NHNH-, -S(=O)z-NHNH-, -C(=O)-NHNH- und einen 5 bis 10gliedriger, aromatischen oder nicht aromatischen Heterozyklus mit bis zu 4 Heteroatomen ausgewählt aus N, O und S, -S(=O)- oder -S(=O)₂-unterbrochen sein kann, wobei die Seitenketten, sofern vorhanden, mit -NH-C(=O)-NHz, -COOH, -OH, -NH₂, -NH-CN-NH₂, Sulfonamid, Sulfon, Sulfoxid oder Sulfonsäure substituiert sein könnenL2 und L3 eine gegebenenfalls verzweigte geradkettige oder verzweigte Kohlenwasserstoffkette mit 1 bis 100 Kohlenstoffatomen aus Arylgruppen und/oder geradkettigen und/oder verzweigten und/oder cyclischen Alkylgruppen ist, die einfach oder mehrfach durch eine oder mehrere der Gruppen -O-, -S-, -S(=O)-, -S(=O)z-, -NH-, -C(=O)-, -NMe-, -NHNH-, -S(=O)z-NHNH-, -NH-C(=O)-, -C(=O)-NH-, -C(=O)-NHNH- und einen 5 bis 10gliedriger, aromatischen oder nicht aromatischen Heterozyklus mit bis zu 4 Heteroatomen ausgewählt aus N, O und S, -S(=O)- oder -S(=O)z- unterbrochen sein kann wobei die Seitenketten, sofern vorhanden, mit -NH-C(=O)-NH₂, -COOH, -OH, -NH₂, -NH-CN-NH₂, Sulfonamid, Sulfon, Sulfoxid oder Sulfonsäure substituiert sein können, und wobei das Konjugat eine der folgenden Formeln genügt, wobei
   - SG₁: eine spaltbare Gruppe, vorzugsweise ein 2-8 Oligopeptid, besonders bevorzugt ein Dipeptid darstellt,
   - AK₁: einen über Cystein verknüpften Antikörper darstellt,
41. Konjugat nach einer oder mehrerer der vorhergehenden Ausführungsformen 1 bis 32, wobei der Linker -L- an eine Lysin-Seitenkette gebunden ist und die folgende Formel aufweist:

   -§-(SG)ₓ-L4-CO-§§,

   wobei
   - §: die Bindung an das Wirkstoffmolekül darstellt und
   - §§: die Bindung an das Binderpeptid oder -protein darstellt,
   - x: 0 oder 1 darstellt,
   - SG: eine spaltbare Gruppe, vorzugsweise ein 2-8 Oligopeptid, besonders bevorzugt ein Dipeptid darstellt, und
   - L4: eine Einfachbindung oder eine Gruppe -(C=O)_{y}-G4- darstellt, wobei
   - y: 0 oder 1 darstellt, und
   - G4: eine geradkettige oder verzweigte Kohlenwasserstoffkette mit 1 bis 100 Kohlenstoffatomen aus Arylgruppen und/oder geradkettigen und/oder verzweigten und/oder cyclischen Alkylgruppen ist, die einfach oder mehrfach durch eine oder mehrere der Gruppen -O-, -S-, -S(=O)-, -S(=O)z-, -NH-, -C(=O)-, -NH-C(=O)-, -C(=O)-NH-, -Nme-, -NHNH-, -S(=O)z-NHNH-, -C(=O)-NHNH- und einen 5 bis 10gliedriger, aromatischen oder nicht aromatischen Heterozyklus mit bis zu 4 Heteroatomen ausgewählt aus N, O und S, oder -S(=O)- unterbrochen sein kann wobei die Seitenketten, sofern vorhanden, mit -NH-C(=O)-NH₂,-COOH, -OH, -NH₂, -NH-CN-NH₂, Sulfonamid, Sulfon, Sulfoxid oder Sulfonsäure substituiert sein können.
42. Konjugat eines Binderpeptids oder -proteins nach Ausführungsform 41, wobei das Konjugat eine der folgenden Formeln aufweist: 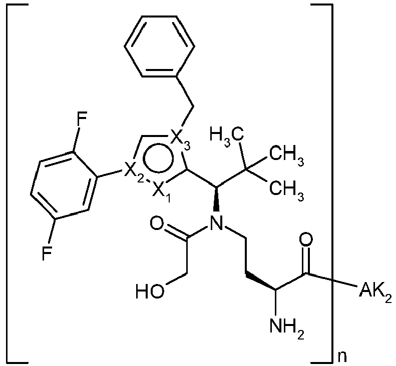 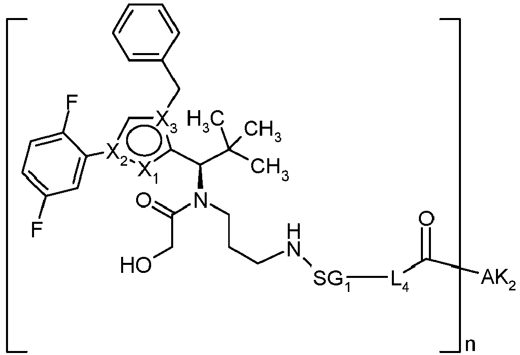 wobei
   - X₁, X₂ und: X₃ die gleiche Bedeutung aufweisen wie in Anspruch 1,
   - AK₂: einen über Lysin verknüpften Antikörper darstellt;;
   - n: eine Zahl von 1 bis 20 darstellt;
   - L4: eine gegebenenfalls geradkettige oder verzweigte Kohlenwasserstoffkette mit 1 bis 100 Kohlenstoffatomen aus Arylgruppen und/oder geradkettigen und/oder verzweigten und/oder cyclischen Alkylgruppen ist, die einfach oder mehrfach durch eine oder mehrere der Gruppen -O-, -S-, -S(=O)-, -S(=O)z-, -NH-, -C(=O)-, -Nme-, -NHNH-, -S(=O)z-NHNH-, -NH-C(=O)-, -C(=O)-NH-, -C(=O)-NHNH- und einen 5 bis 10gliedriger, aromatischen oder nicht aromatischen Heterozyklus mit bis zu 4 Heteroatomen ausgewählt aus N, O und S, -S(=O)- oder -S(=O)z- unterbrochen sein kann, wobei die Seitenketten, sofern vorhanden, mit -NH-C(=O)-NHz, -COOH, -OH, -NH₂, -NH-CN-NH₂, Sulfonamid, Sulfon, Sulfoxid oder Sulfonsäure substituiert sein können, und wobei das Konjugat eine der folgenden Formeln genügt, wobei n eine Zahl von 1 bis 20 ist, und das Wasserstoffatom in Position R⁴ gemäß Formel IIa (d.h. in der Gruppe -NH₂) durch Legumain spaltbare Gruppe der Formel R²¹-(C=O)₍₀₋₁₎-(P3)₍₀₋₂₎-P2-NH-CH(CH₂C(=O)-NH₂)-C(=O)- ersetzt ist,
   wobei R²¹ eine C₁₋₁₀-Alkyl-, C₅₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit -NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)₂, oder -OH substituiert sein kann, oder -H oder eine Gruppe -Oₓ-(CH₂CH₂O)ᵥ-R²² darstellt,
   wobei x 0 oder 1 ist,
   wobei v eine Zahl von 1 bis 20 darstellt, und
   R²² -H, -Alkyl (vorzugsweise C₁₋₁₂-Alkyl), -CH₂-COOH, -CH₂-CH₂-COOH, oder -CH₂-CH₂-NH₂);
   wobei P2 eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist;
   Wobei P3 eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His oder eine der jeweiligen N-Alkyl-Aminsoäuren, bevorzugt N-Methyl-Aminsoäuren ist; und
   - SG1: eine spaltbare Gruppe, vorzugsweise ein 2-8 Oligopeptid, besonders bevorzugt ein Dipeptid darstellt.
43. Konjugat nach einer oder mehrerer der vorhergehenden Ausführungsformen 23 bis 42, wobei der anti-TWEAKR-Antikörper ein agonistischer Antikörper ist.
44. Konjugat nach einer oder mehrerer der vorhergehenden Ausführungsformen 23 bis 43, wobei der anti-TWEAKR-Antikörper oder das Antigen-bindende Fragment hiervon umfasst:
   eine variable schwere Kette umfassend:
   a. eine CDR1 der schweren Kette, die von einer Aminosäuresequenz umfassend die Formel PYPMX (SEQ ID NO: 171) kodiert wird, wobei X I oder Mist;
   b. eine CDR2 der schweren Kette, die von einer Aminosäuresequenz umfassend die Formel YISPSGGXTHYADSVKG (SEQ ID NO: 172) kodiert wird, wobei X S oder K ist; und
   c. eine CDR3 der schweren Kette, die von einer Aminosäuresequenz umfassend die Formel GGDTYFDYFDY (SEQ ID NO: 173) kodiert wird; und eine variable leichte Kette umfassend:
   d. eine CDR1 der leichten Kette, die von einer Aminosäuresequenz umfassend die Formel RASQSISXYLN (SEQ ID NO: 174) kodiert wird, wobei X G oder S ist;
   e. eine CDR2 der leichten Kette, die von einer Aminosäuresequenz umfassend die Formel XASSLQS (SEQ ID NO: 175) kodiert wird, wobei X Q , A oder N ist; und
   f. eine CDR3 der leichten Kette, die von einer Aminosäuresequenz umfassend die Formel QQSYXXPXIT (SEQ ID NO: 176) kodiert wird, wobei X an der Position 5 T oder S ist, X an der Position 6 T oder S ist und X an der Position 8 G oder F ist.
45. Konjugat nach einer oder mehrerer der vorhergehenden Ausführungsformen 23 bis 44, wobei der anti-TWEAKR-Antikörper oder das Antigen-bindende Fragment hiervon umfasst:
   a. eine variable Sequenz der schweren Kette, wie dargestellt durch SEQ ID NO: 10 , sowie eine variable Sequenz der leichten Kette, wie dargestellt durch SEQ ID NO:9 oder
   b. eine variable Sequenz der schweren Kette, wie dargestellt durch SEQ ID NO:20 , sowie eine variable Sequenz der leichten Kette, wie dargestellt durch SEQ ID NO: 19 oder
   c. eine variable Sequenz der schweren Kette, wie dargestellt durch SEQ ID NO:30 , sowie eine variable Sequenz der leichten Kette, wie dargestellt durch SEQ ID NO:29 oder
   d. eine variable Sequenz der schweren Kette, wie dargestellt durch SEQ ID NO:40 , sowie eine variable Sequenz der leichten Kette, wie dargestellt durch SEQ ID NO:39 oder
   e. eine variable Sequenz der schweren Kette, wie dargestellt durch SEQ ID NO:50 , sowie eine variable Sequenz der leichten Kette, wie dargestellt durch SEQ ID NO:49 oder
   f. eine variable Sequenz der schweren Kette, wie dargestellt durch SEQ ID NO:60 , sowie eine variable Sequenz der leichten Kette, wie dargestellt durch SEQ ID NO:59 oder
   g. eine variable Sequenz der schweren Kette, wie dargestellt durch SEQ ID NO:70 , sowie eine variable Sequenz der leichten Kette, wie dargestellt durch SEQ ID NO:69 oder
   h. eine variable Sequenz der schweren Kette, wie dargestellt durch SEQ ID NO:80 , sowie eine variable Sequenz der leichten Kette, wie dargestellt durch SEQ ID NO:79 oder
   i. eine variable Sequenz der schweren Kette, wie dargestellt durch SEQ ID NO:90 , sowie eine variable Sequenz der leichten Kette, wie dargestellt durch SEQ ID NO:89 oder
   j. eine variable Sequenz der schweren Kette, wie dargestellt durch SEQ ID NO:100 , sowie eine variable Sequenz der leichten Kette, wie dargestellt durch SEQ ID NO:99 oder
   k. eine variable Sequenz der schweren Kette, wie dargestellt durch SEQ ID NO:110 , sowie eine variable Sequenz der leichten Kette, wie dargestellt durch SEQ ID NO:109 oder
   l. eine variable Sequenz der schweren Kette, wie dargestellt durch SEQ ID NO:120 , sowie eine variable Sequenz der leichten Kette, wie dargestellt durch SEQ ID NO:119.
46. Konjugat nach einer oder mehrerer der vorhergehenden Ausführungsformen 18 bis 45, wobei der Antikörper ein IgG Antikörper ist.
47. Konjugat nach einer oder mehrerer der vorhergehenden Ausführungsformen 20 bis 46, wobei der anti-TWEAKR-Antikörper umfasst:
   a. eine Sequenz der schweren Kette, wie dargestellt durch SEQ ID NO:2, sowie eine Sequenz der leichten Kette, wie dargestellt durch SEQ ID NO:1 oder
   b. eine Sequenz der schweren Kette, wie dargestellt durch SEQ ID NO:12, sowie eine Sequenz der leichten Kette, wie dargestellt durch SEQ ID NO:11 oder
   c. eine Sequenz der schweren Kette, wie dargestellt durch SEQ ID NO:22, sowie eine Sequenz der leichten Kette, wie dargestellt durch SEQ ID NO:21 oder
   d. eine Sequenz der schweren Kette, wie dargestellt durch SEQ ID NO:32, sowie eine Sequenz der leichten Kette, wie dargestellt durch SEQ ID NO:31 oder
   e. eine Sequenz der schweren Kette, wie dargestellt durch SEQ ID NO:42, sowie eine Sequenz der leichten Kette, wie dargestellt durch SEQ ID NO:41 oder
   f. eine Sequenz der schweren Kette, wie dargestellt durch SEQ ID NO:52, sowie eine Sequenz der leichten Kette, wie dargestellt durch SEQ ID NO:51 oder
   g. eine Sequenz der schweren Kette, wie dargestellt durch SEQ ID NO:62, sowie eine Sequenz der leichten Kette, wie dargestellt durch SEQ ID NO:61 oder
   h. eine Sequenz der schweren Kette, wie dargestellt durch SEQ ID NO:72, sowie eine Sequenz der leichten Kette, wie dargestellt durch SEQ ID NO:71 oder
   i. eine Sequenz der schweren Kette, wie dargestellt durch SEQ ID NO:82, sowie eine Sequenz der leichten Kette, wie dargestellt durch SEQ ID NO:81 oder
   j. eine Sequenz der schweren Kette, wie dargestellt durch SEQ ID NO:92, sowie eine Sequenz der leichten Kette, wie dargestellt durch SEQ ID NO:91 oder
   k. eine Sequenz der schweren Kette, wie dargestellt durch SEQ ID NO:102, sowie eine Sequenz der leichten Kette, wie dargestellt durch SEQ ID NO:101 oder
   l. eine Sequenz der schweren Kette, wie dargestellt durch SEQ ID NO:112, sowie eine Sequenz der leichten Kette, wie dargestellt durch SEQ ID NO:111.
48. Konjugat nach einer oder mehrerer der vorhergehenden Ausführungsformen, wobei das Konjugat 1 bis 10, vorzugsweise 2 bis 8, Wirkstoffmoleküle bzw. Prodrugmoleküle pro Binderpeptid oder -protein aufweist.
49. Antikörper-Prodrug-Konjugat nach einer oder mehrerer der vorhergehenden Ausführungsformen, wobei das Antikörper-Prodrug-Konjugat eine der folgenden Formeln genügt: 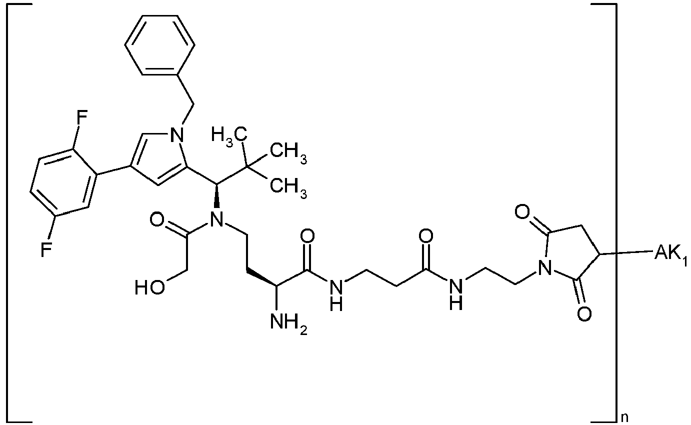 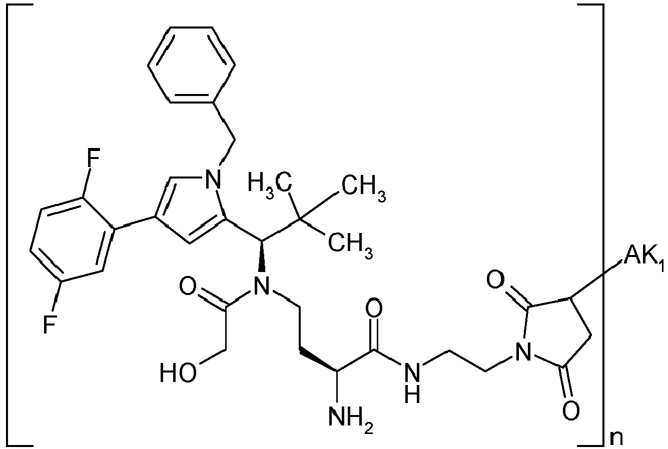 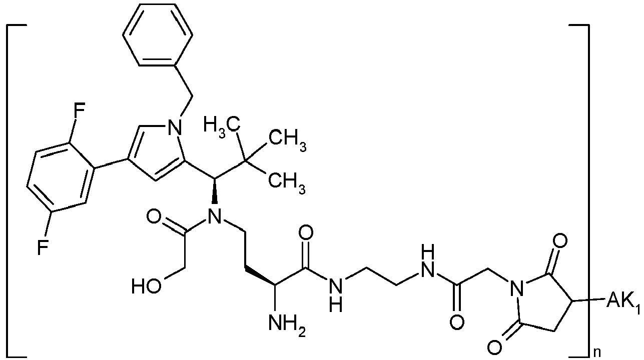 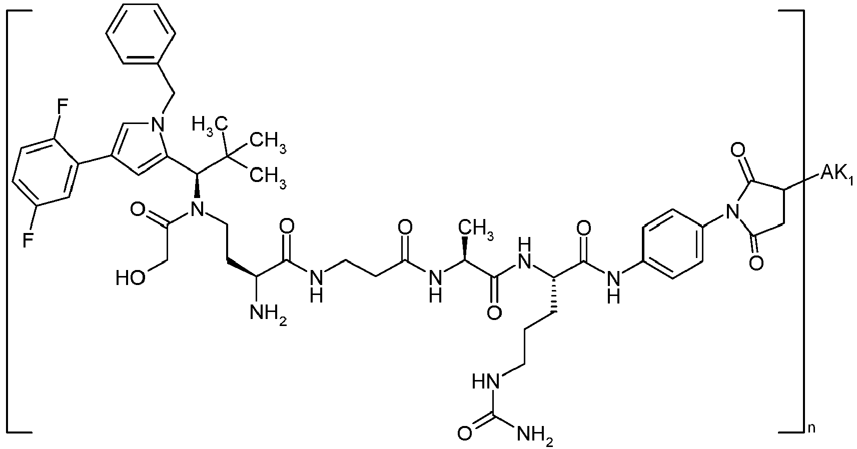 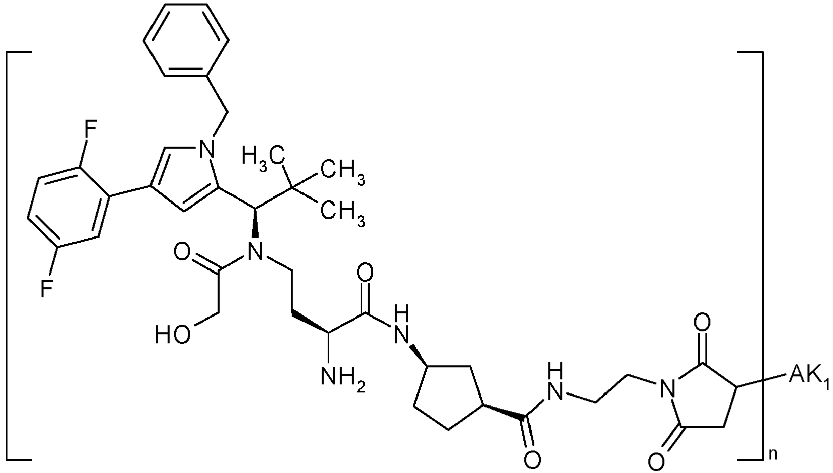 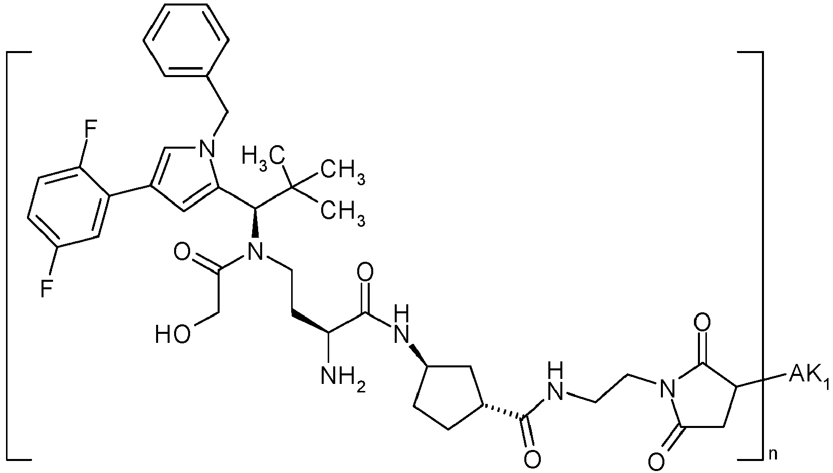 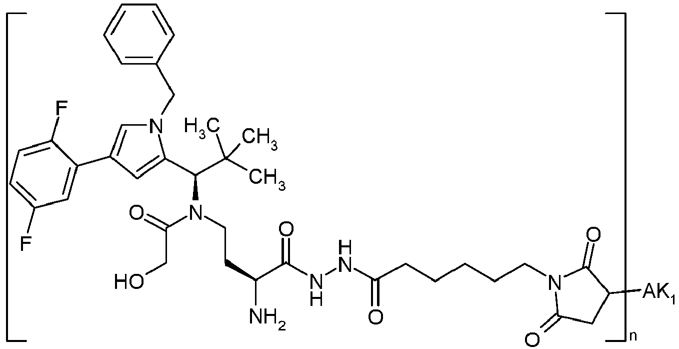 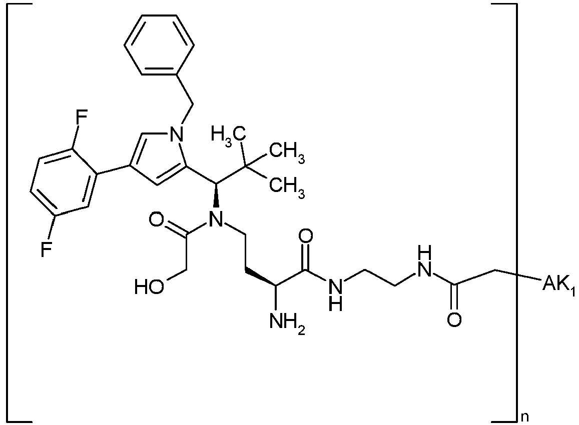 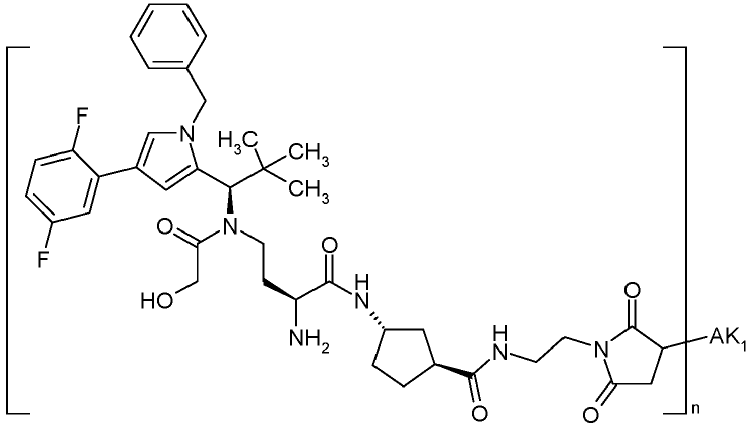 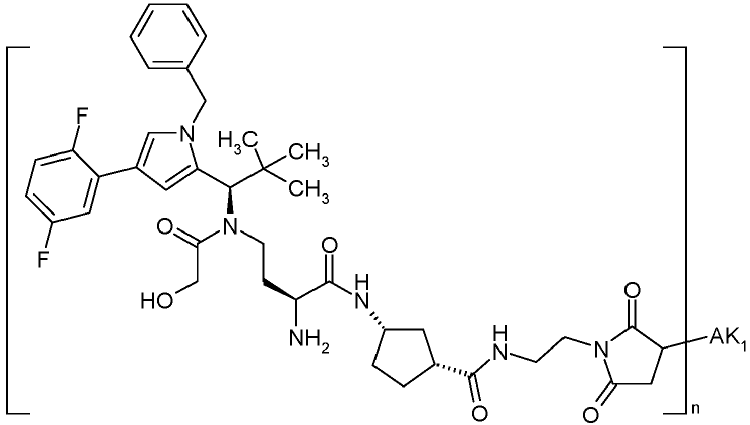 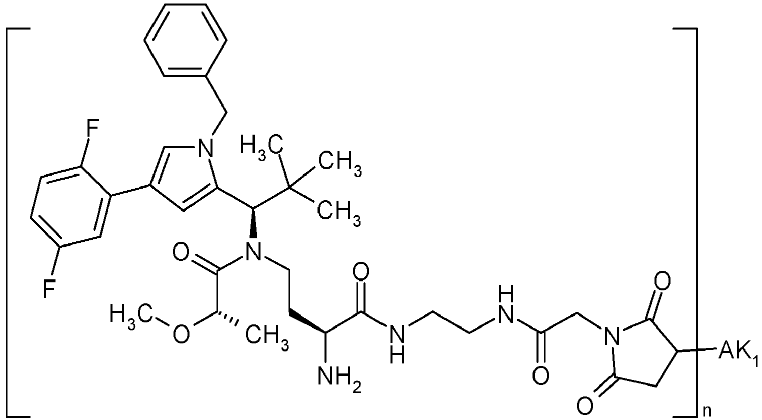 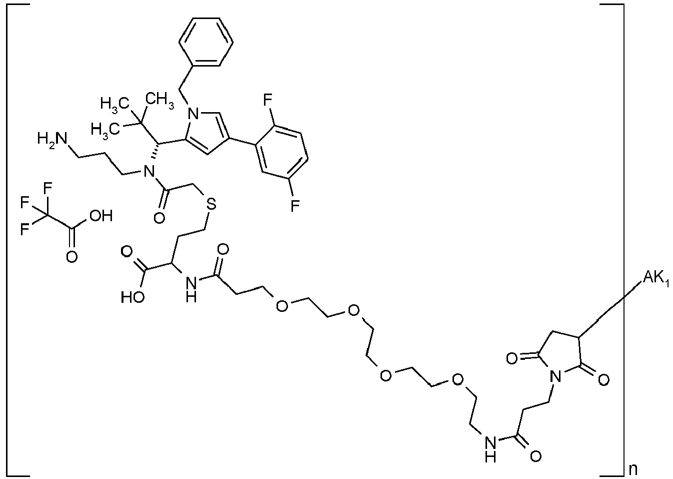 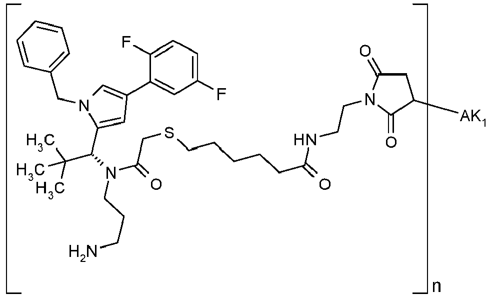 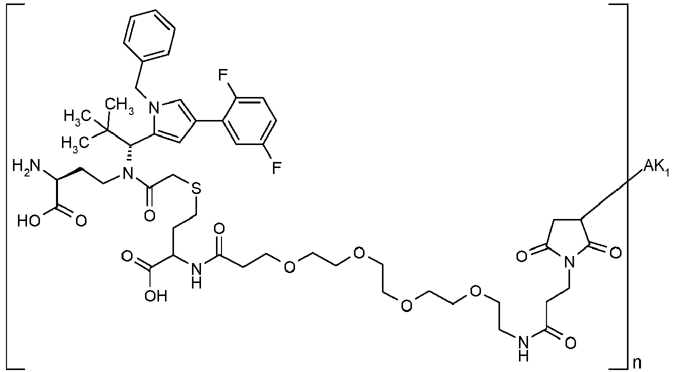 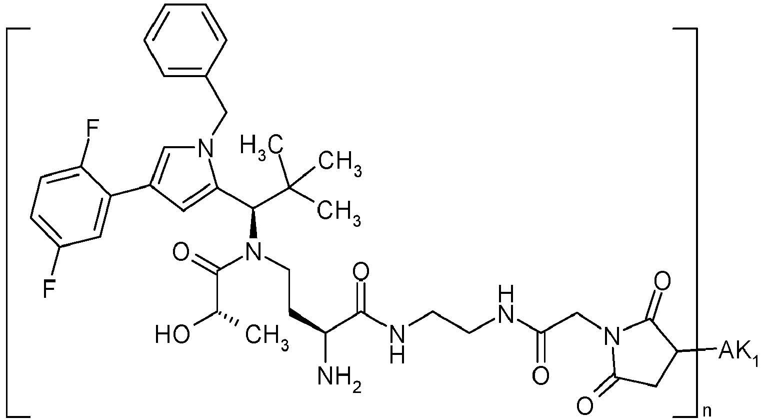 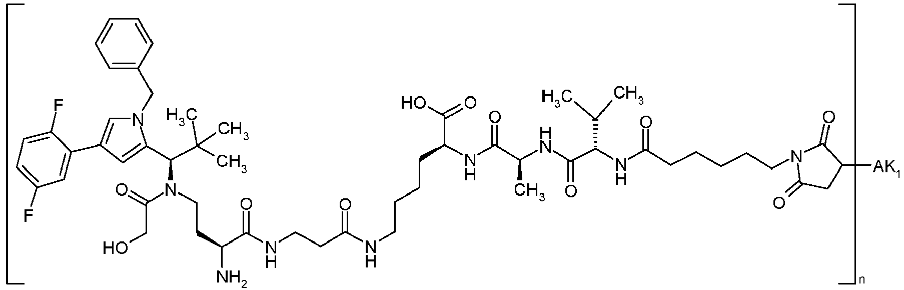 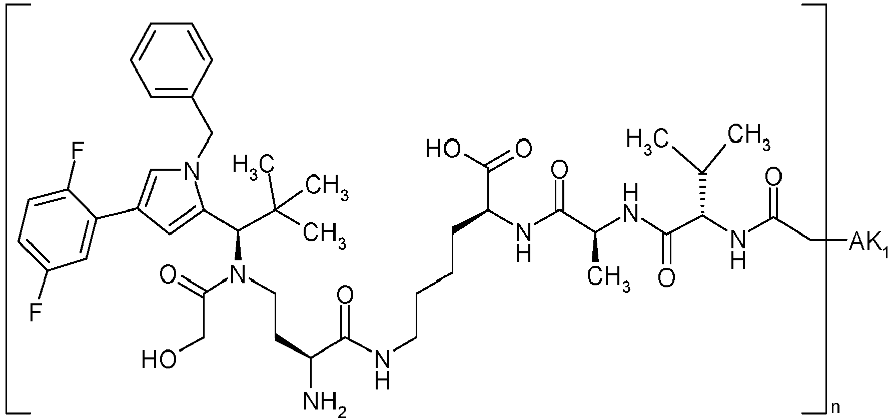 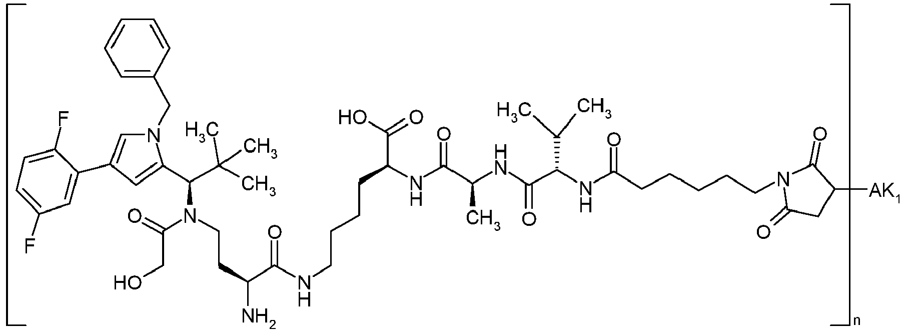 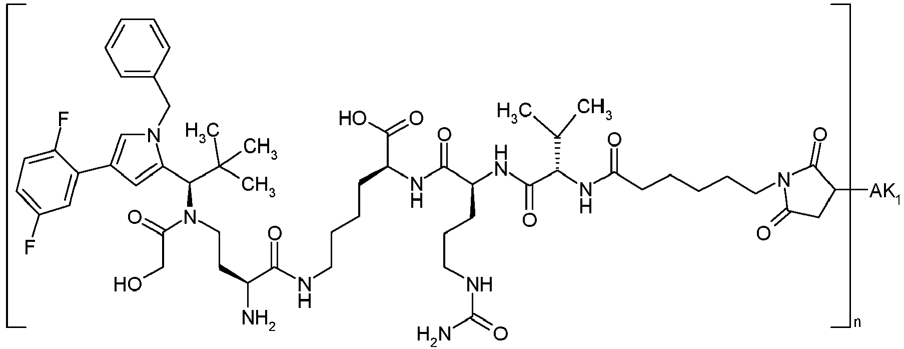 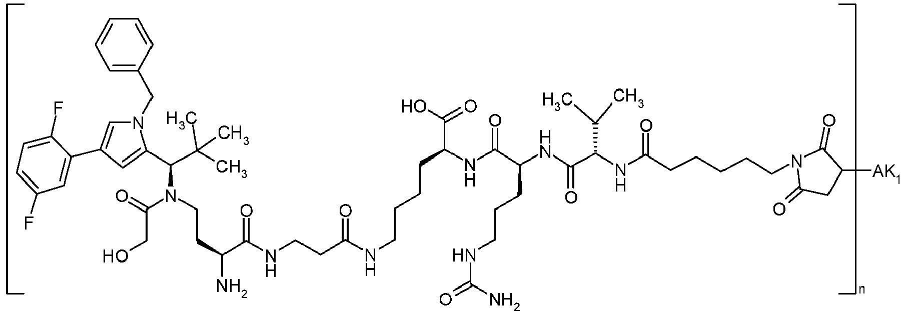 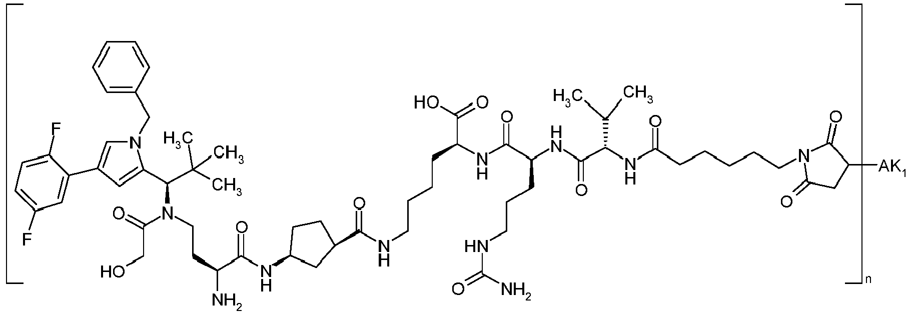 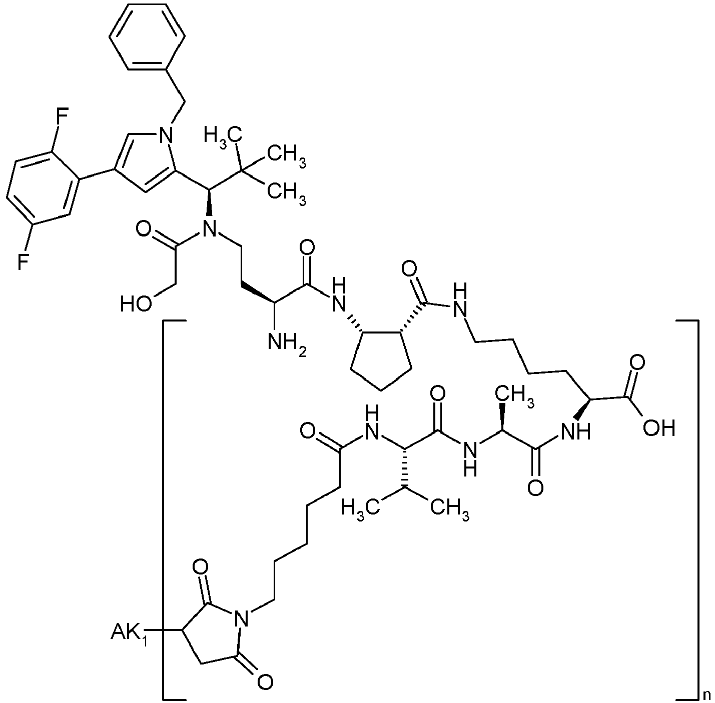 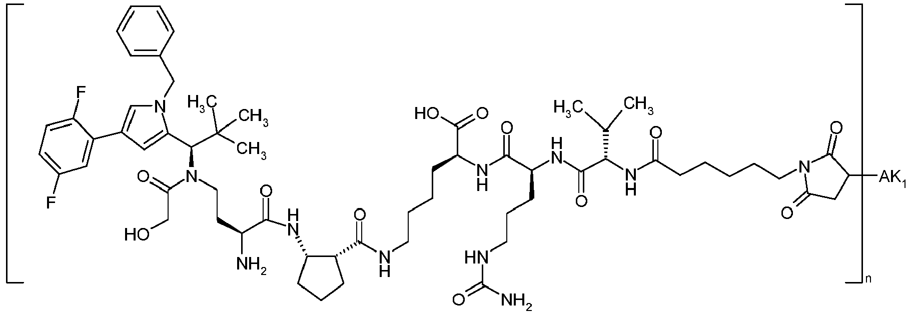 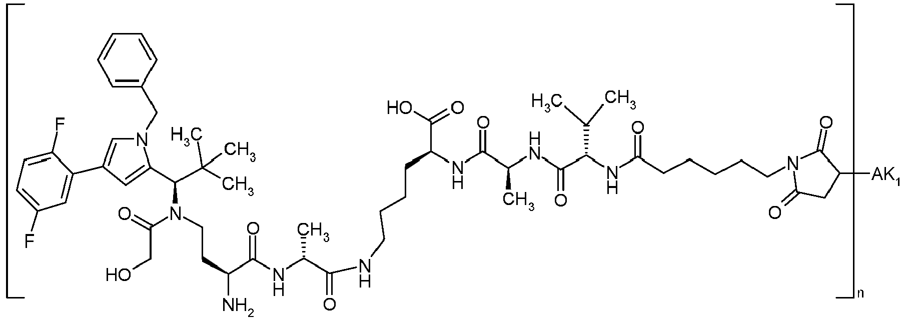 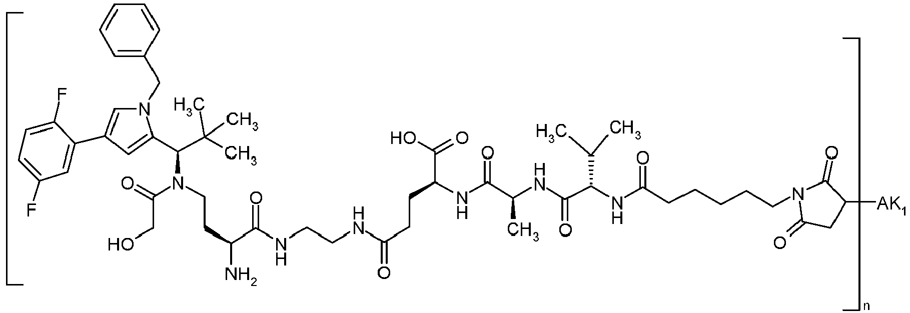 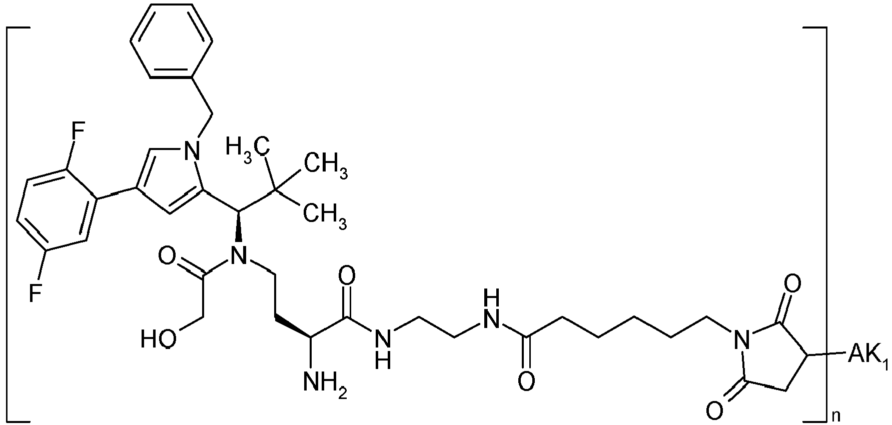 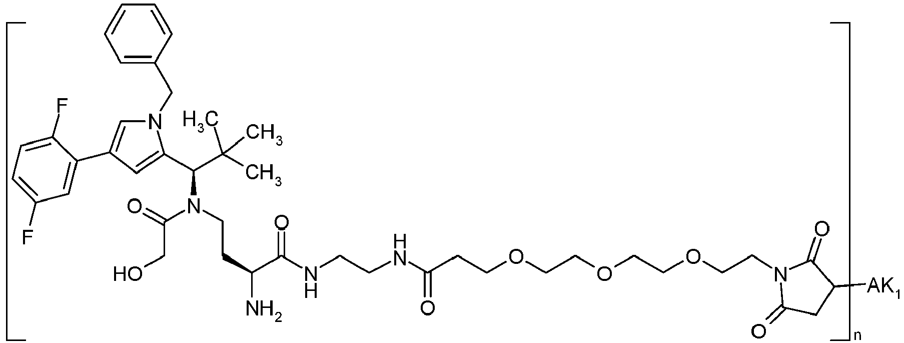 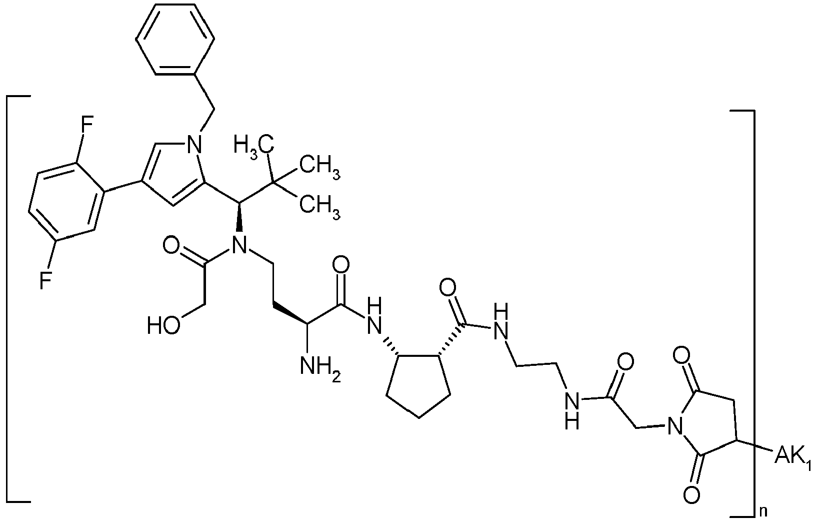 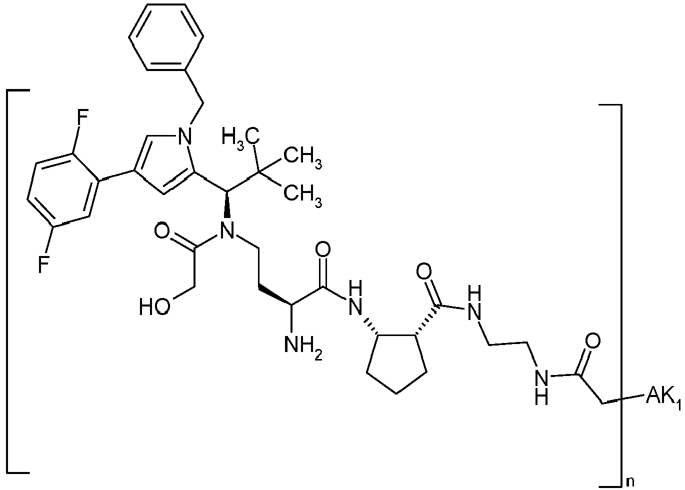 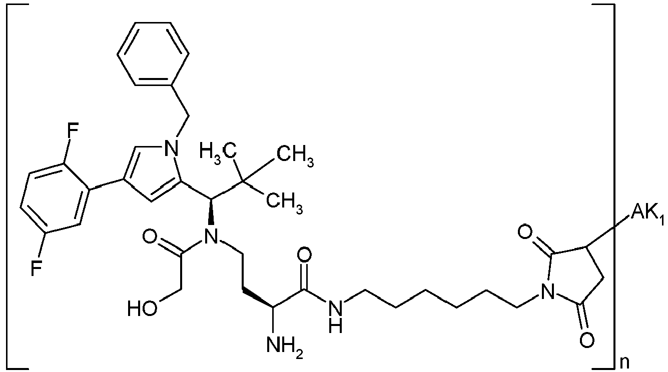 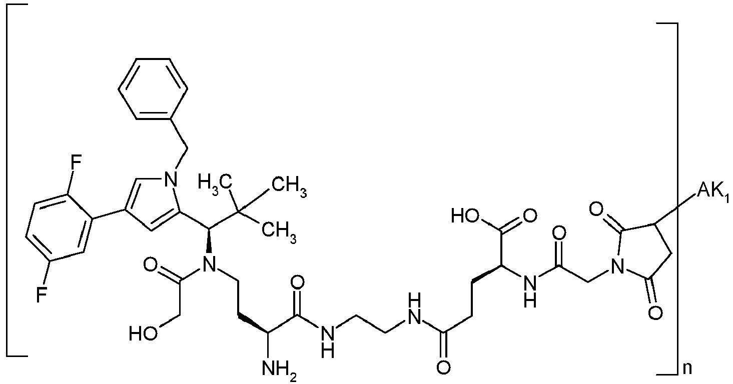 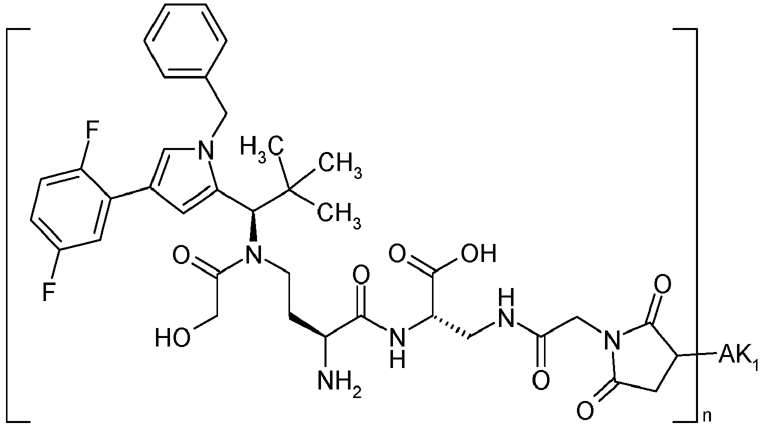 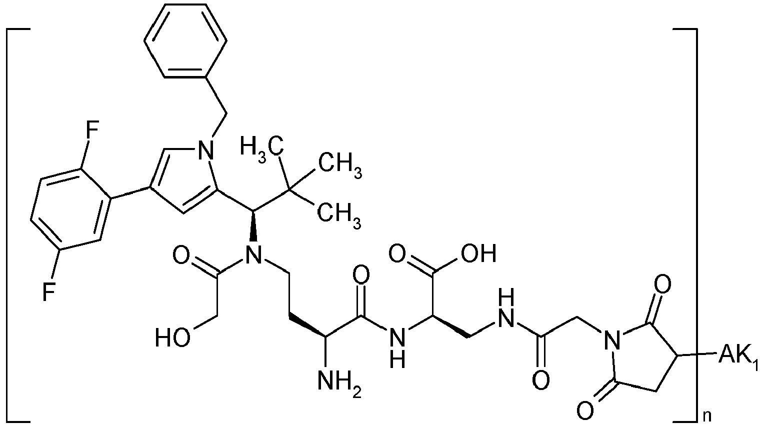 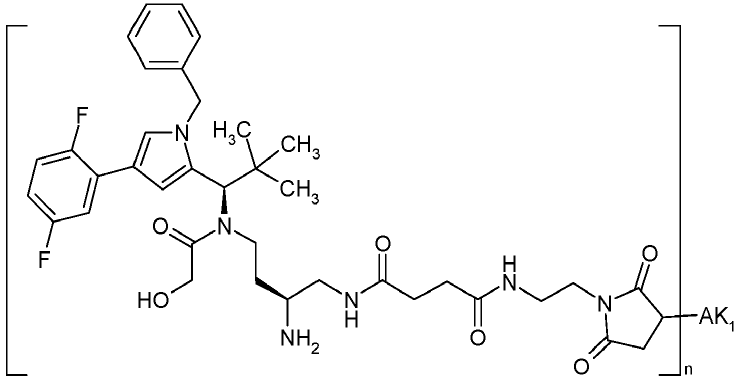 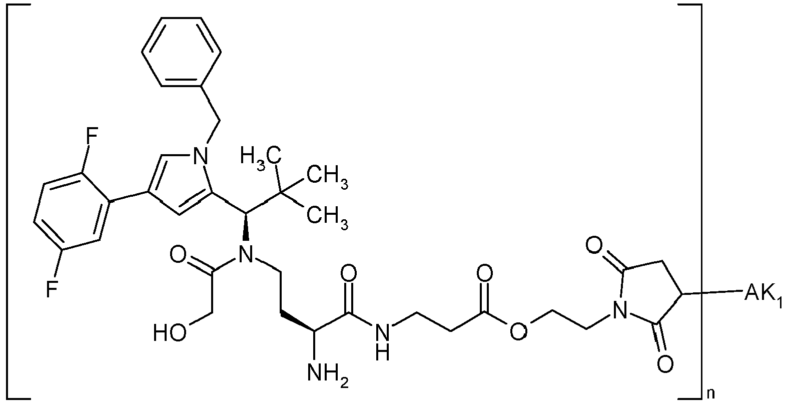 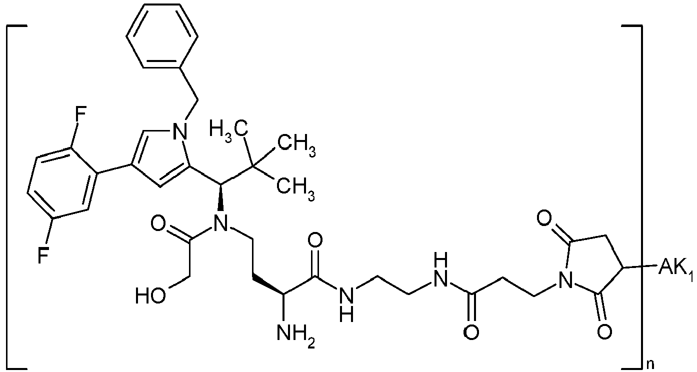 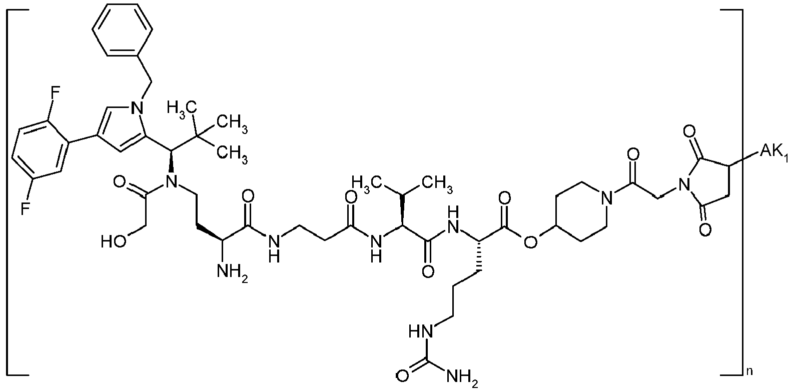 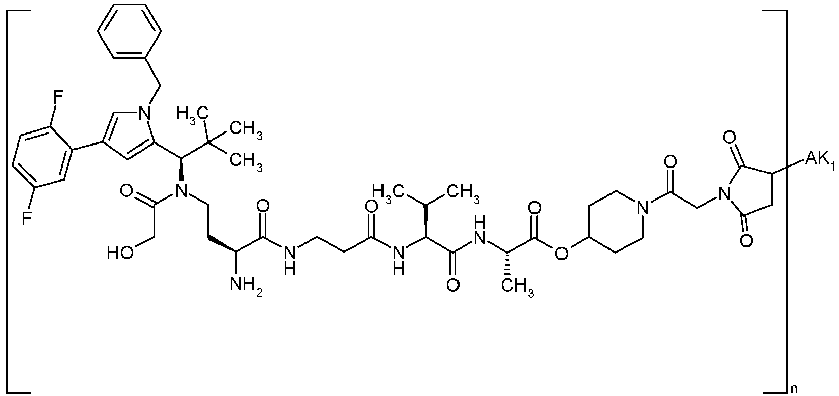 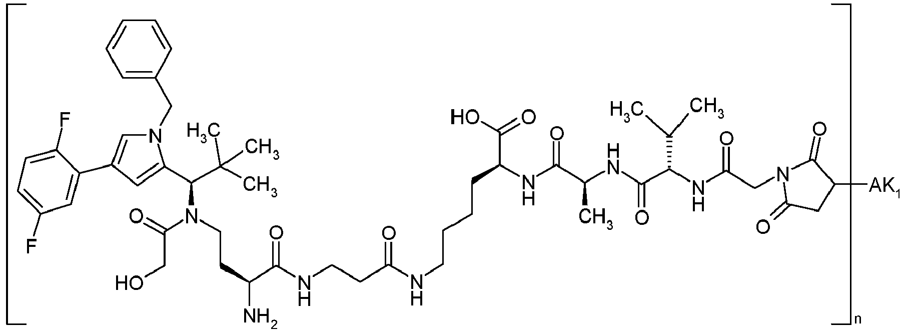 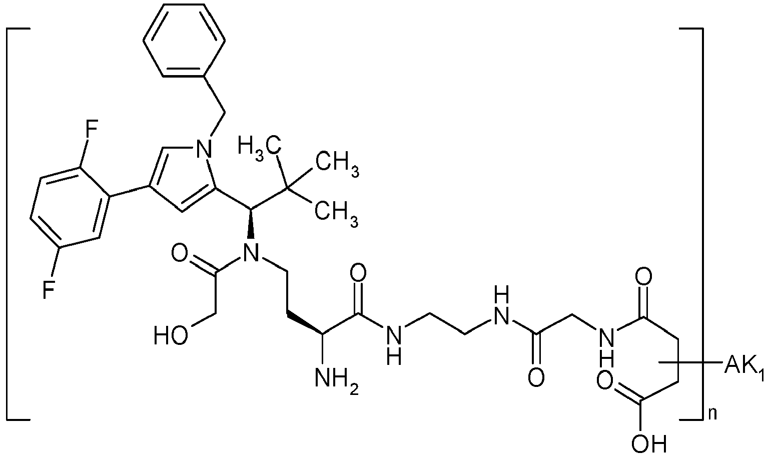 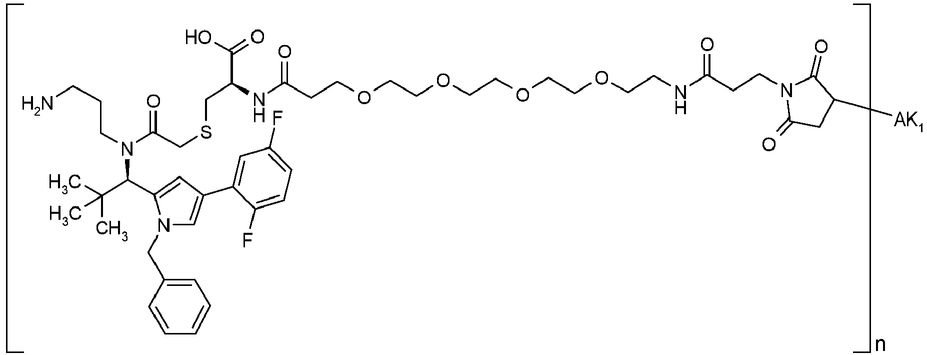 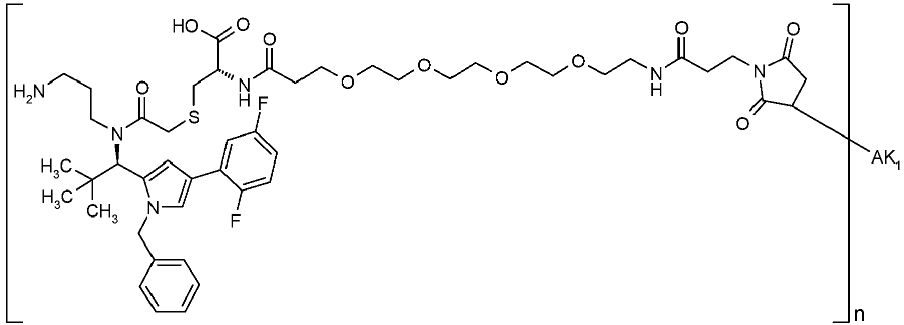 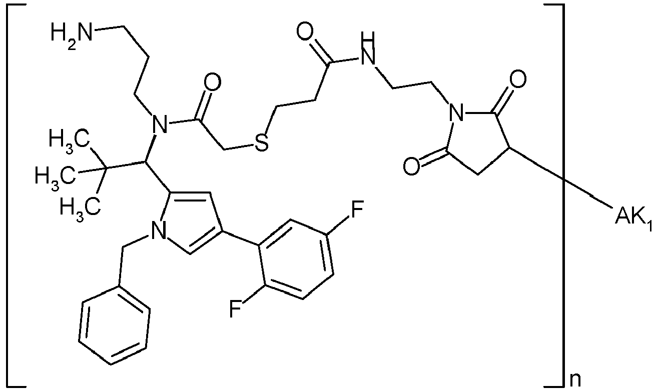 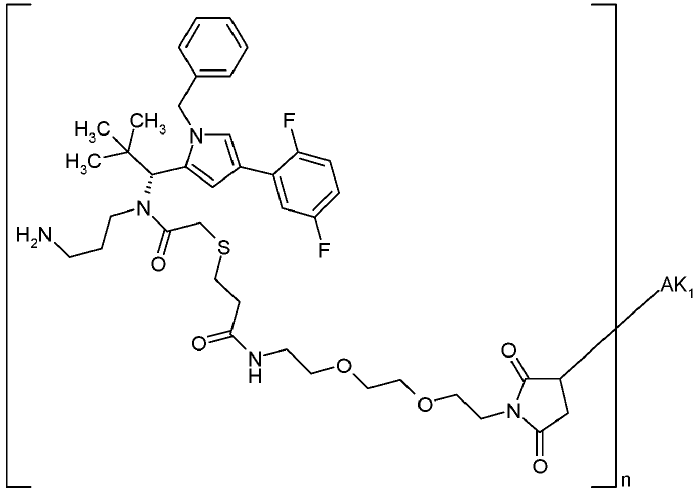 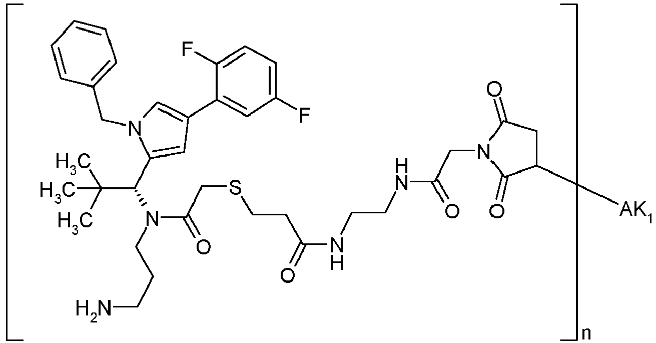 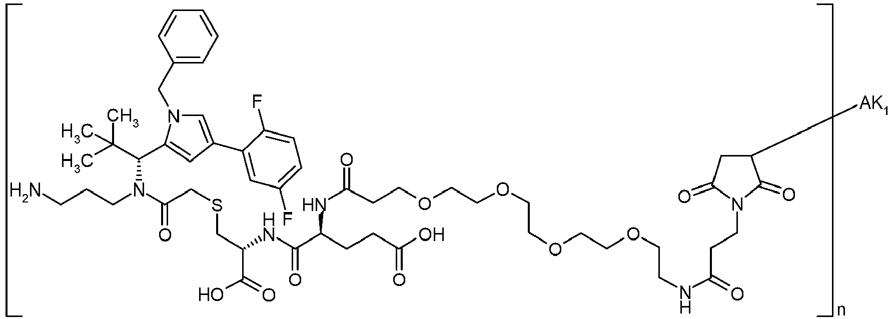 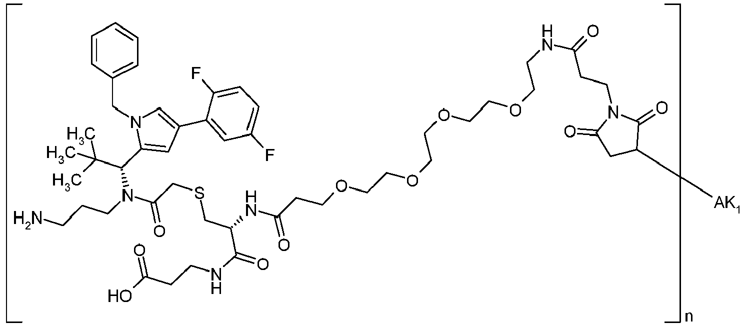 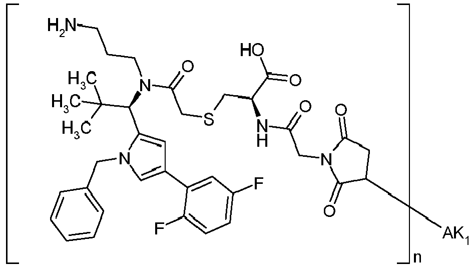 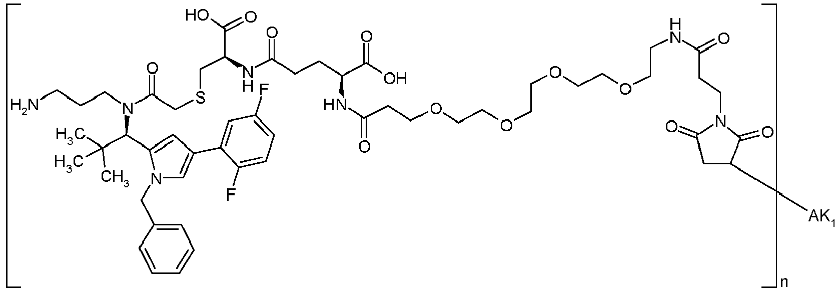 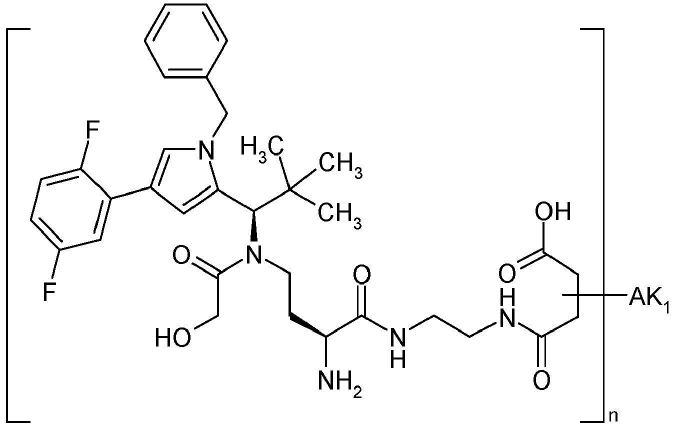 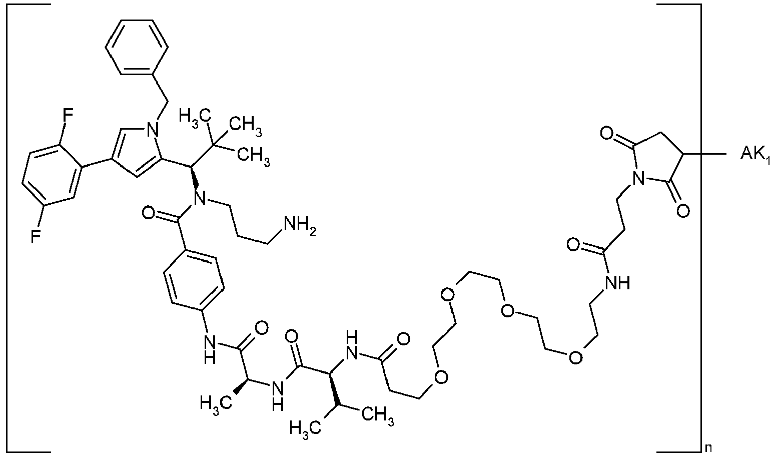 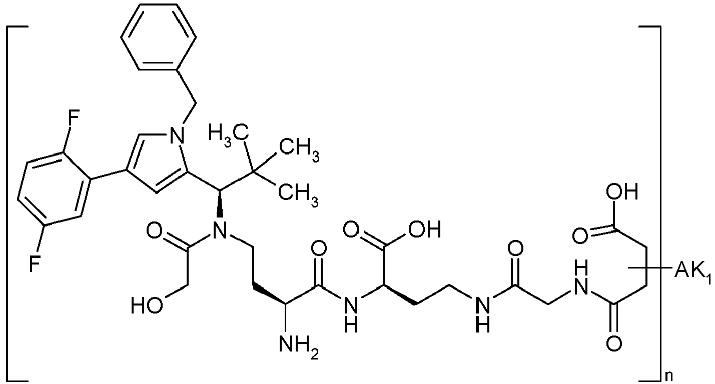 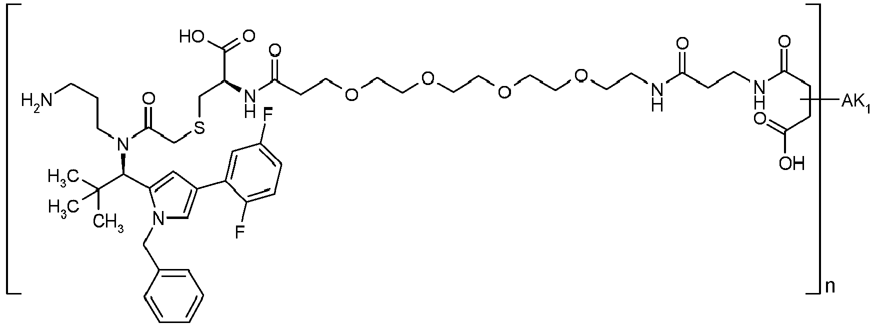 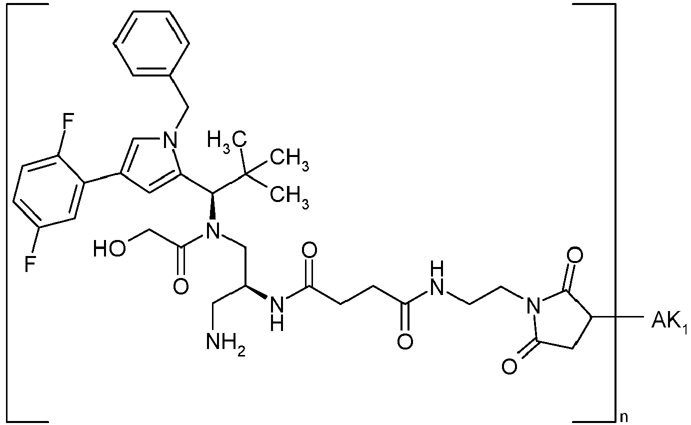 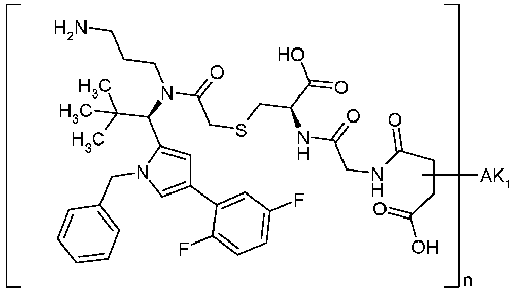 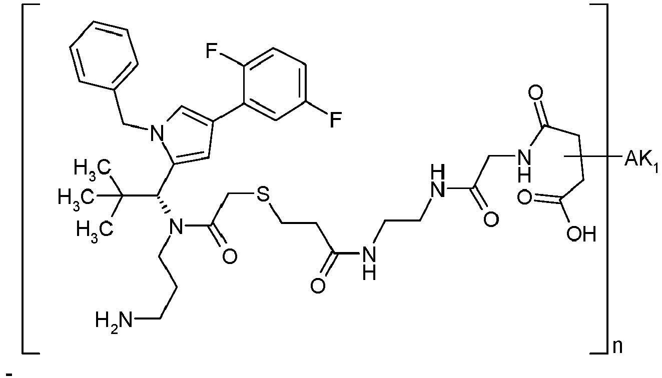 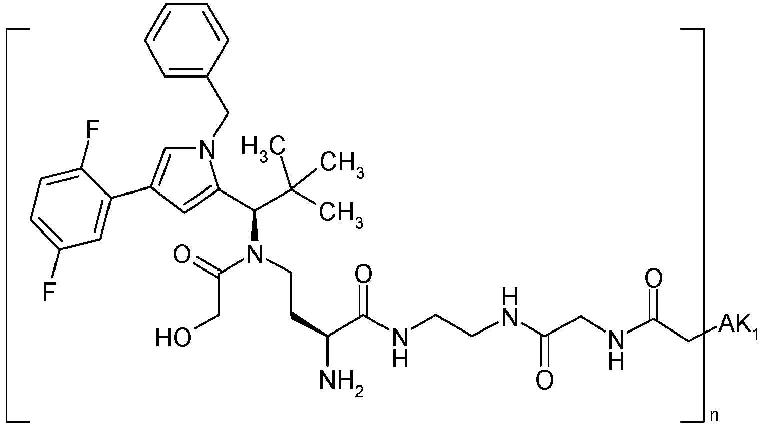 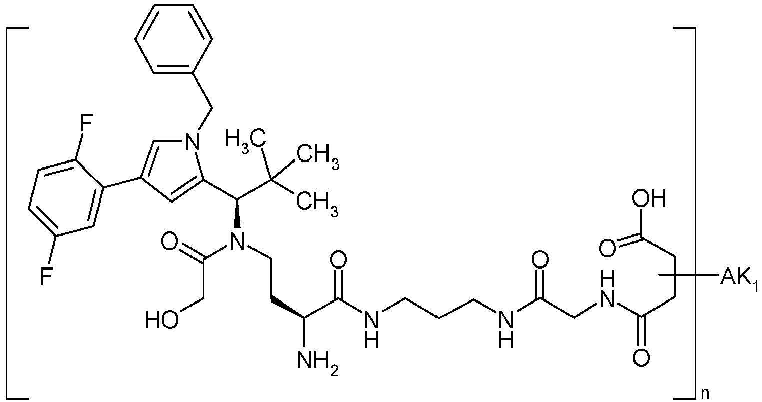 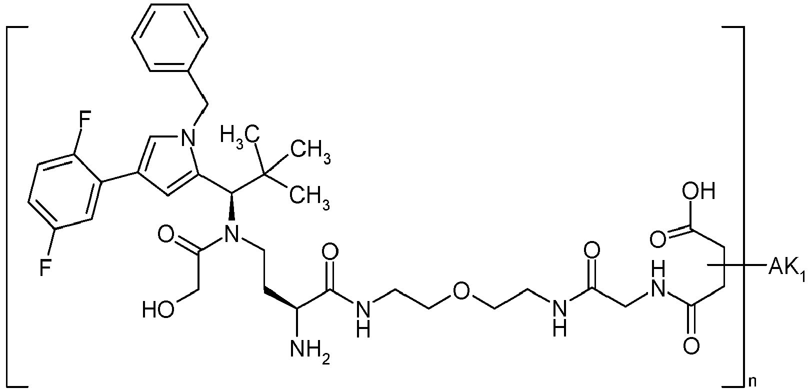 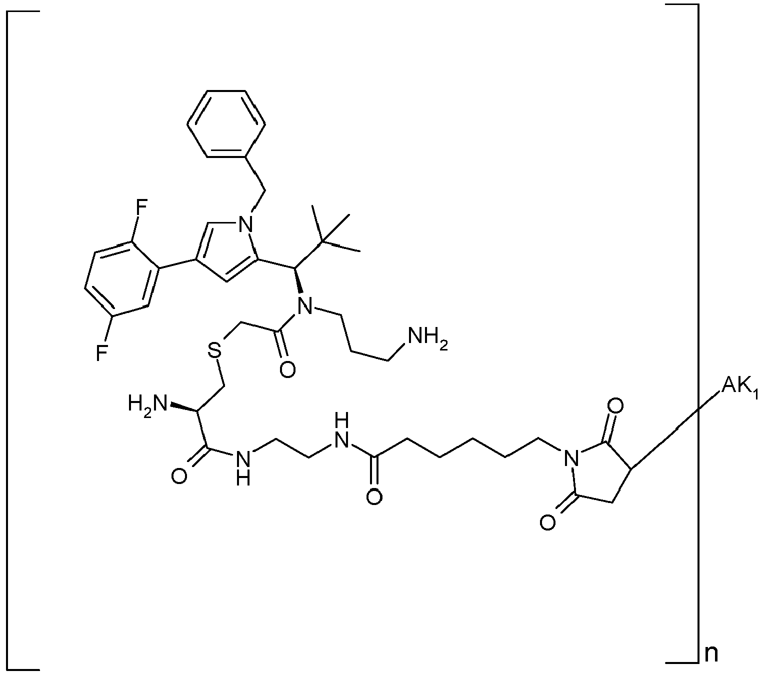 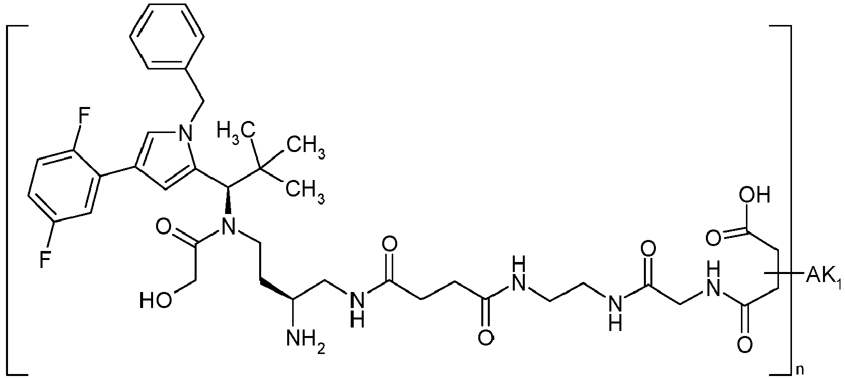 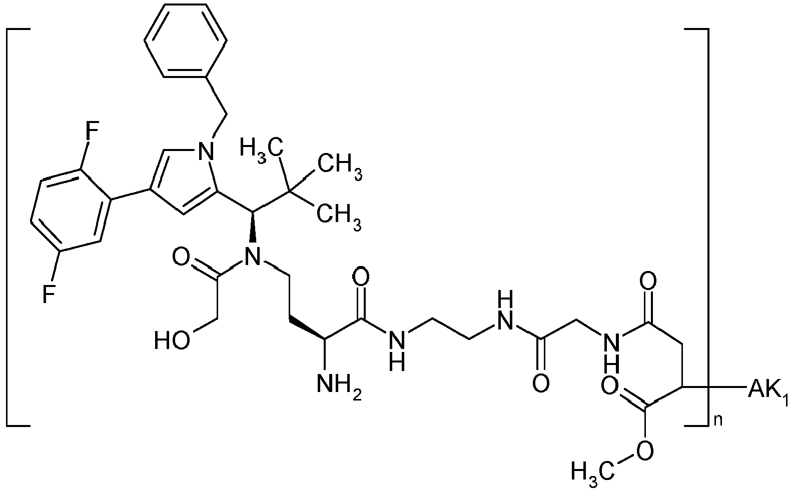 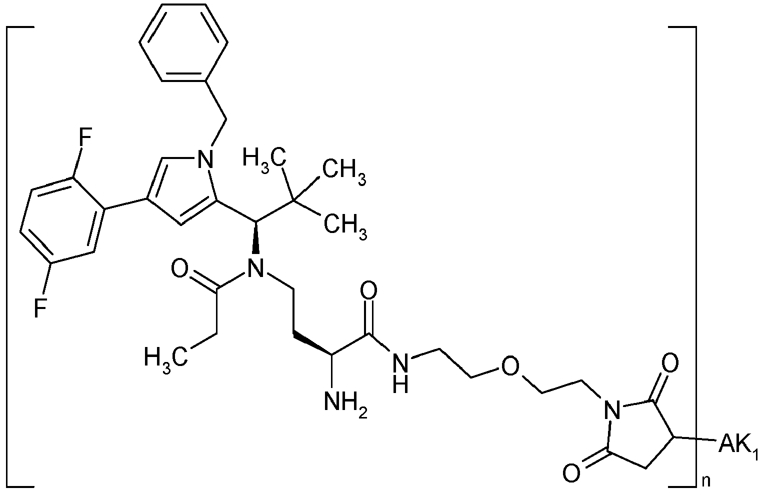 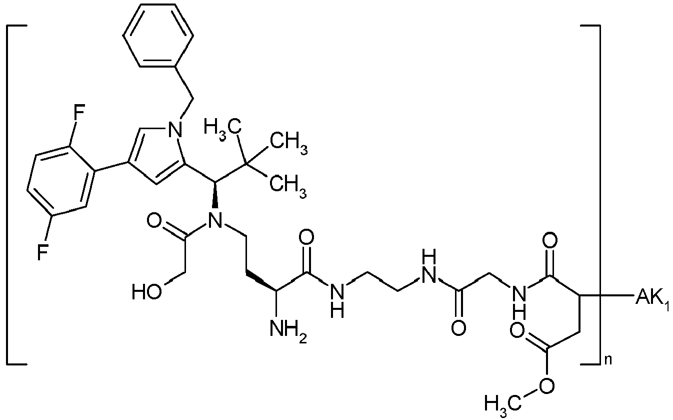 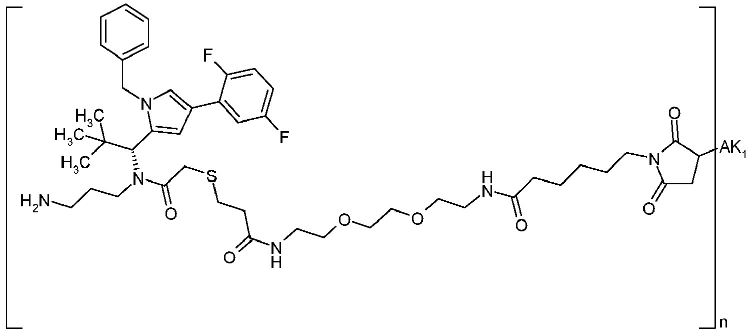 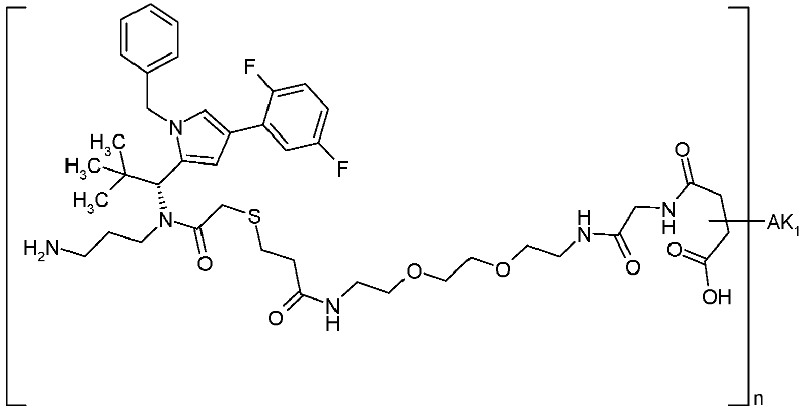 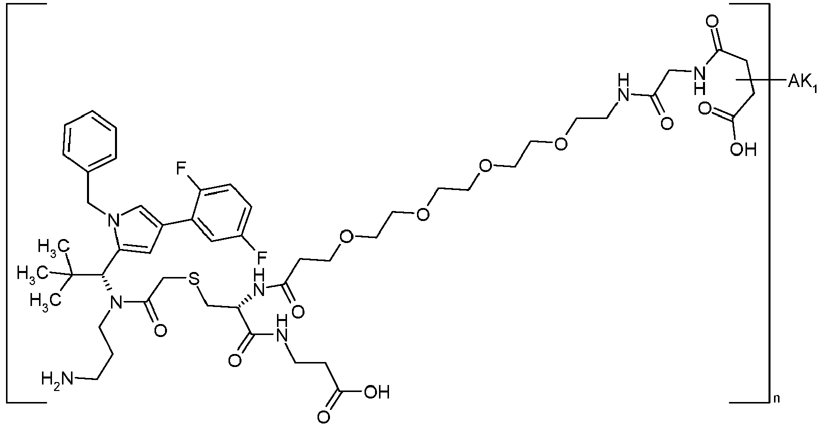 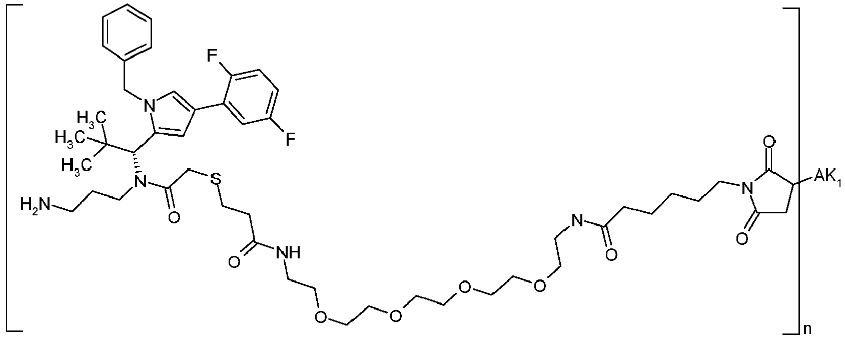 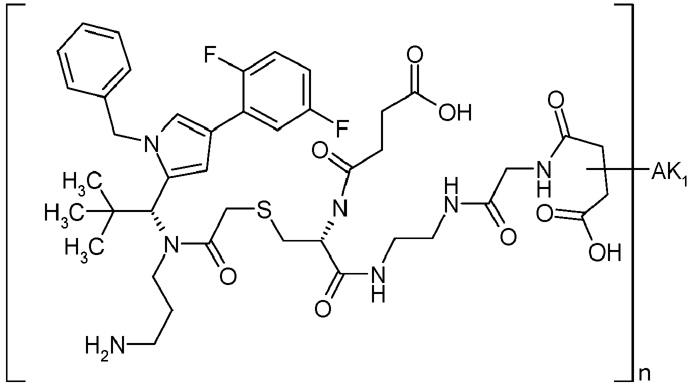 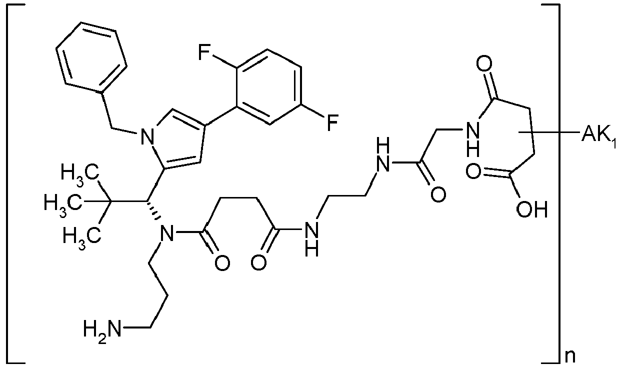 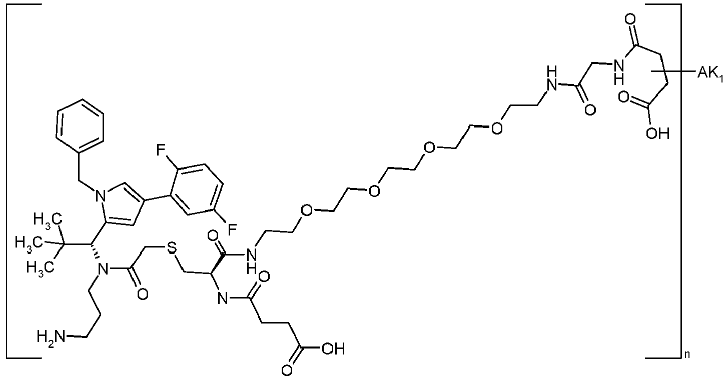 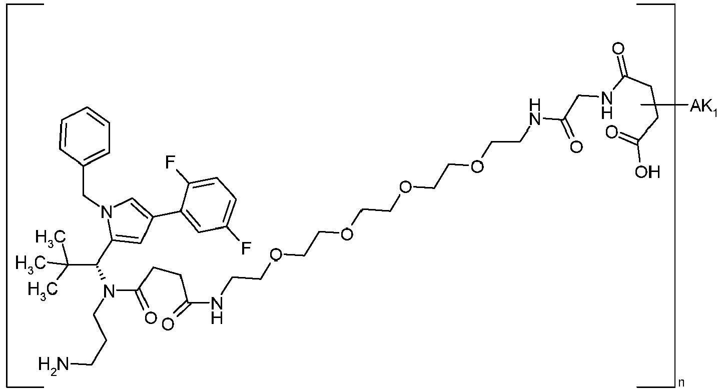 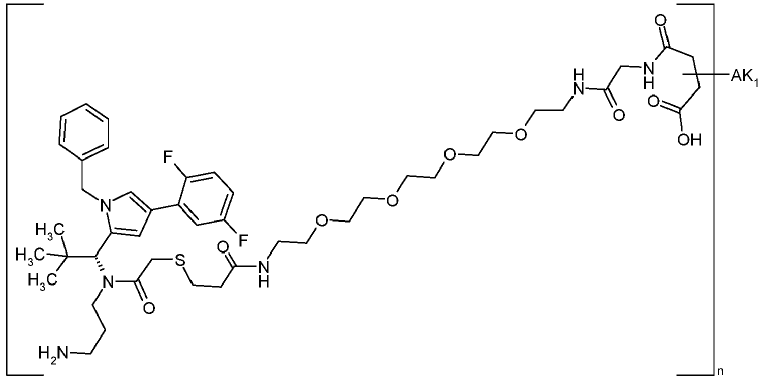 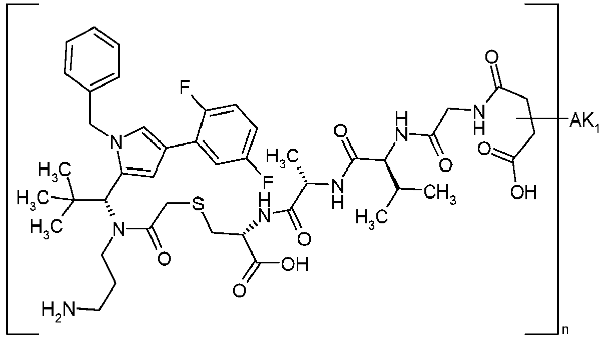 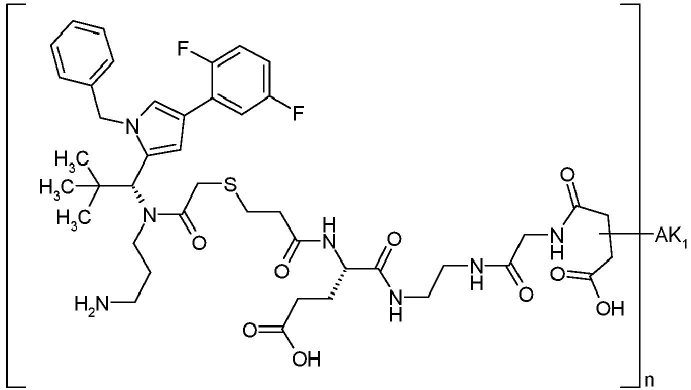 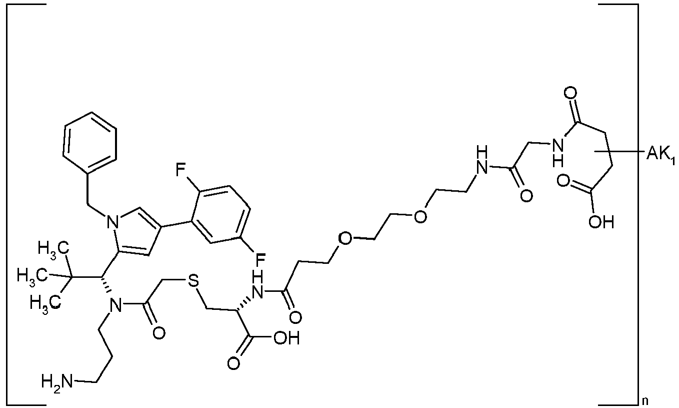 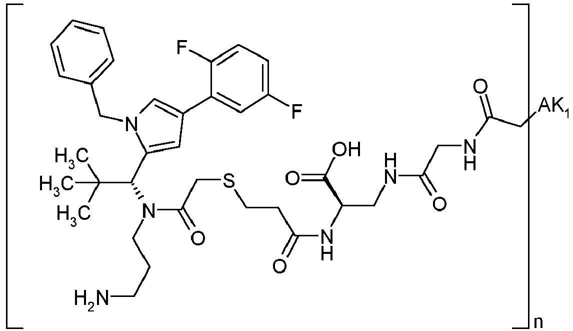 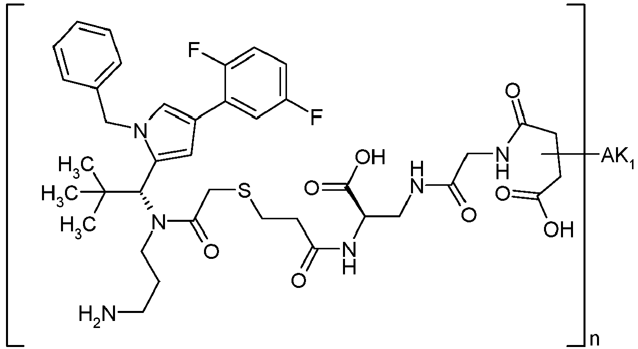 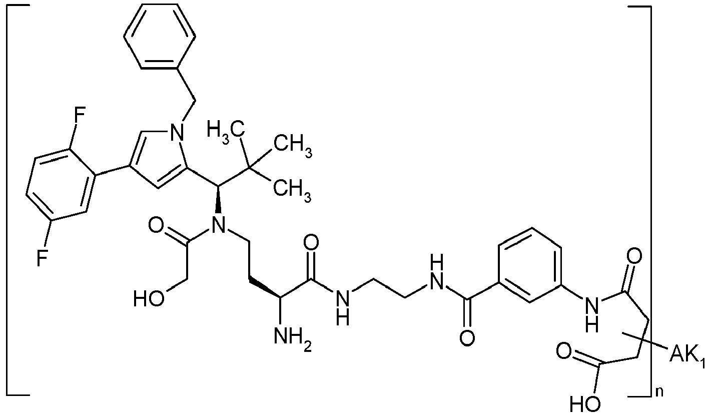 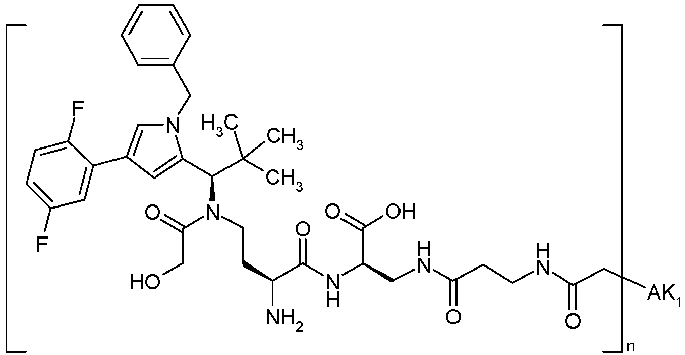 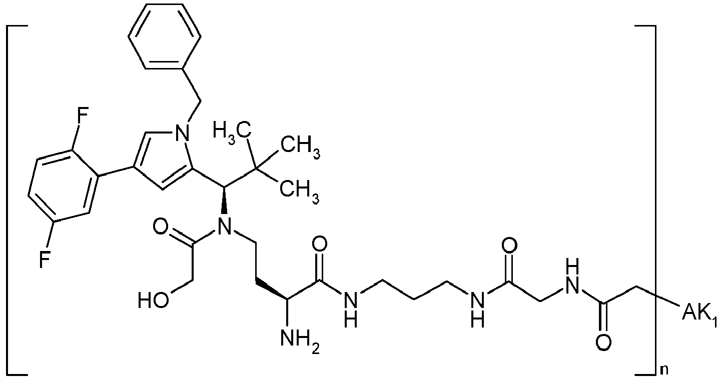 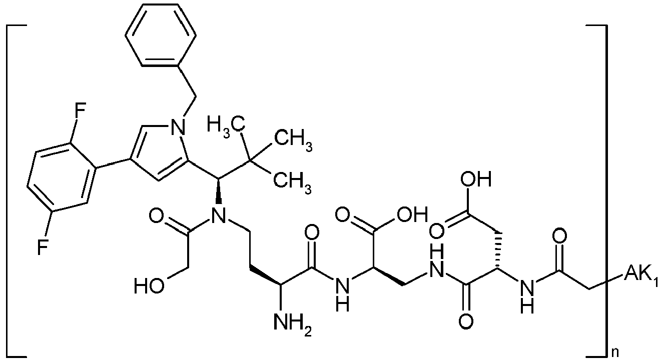 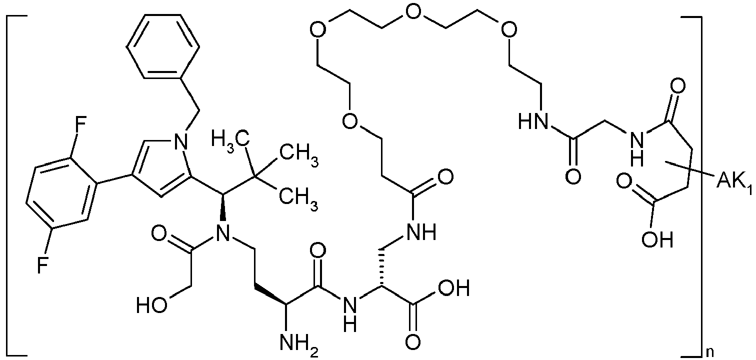 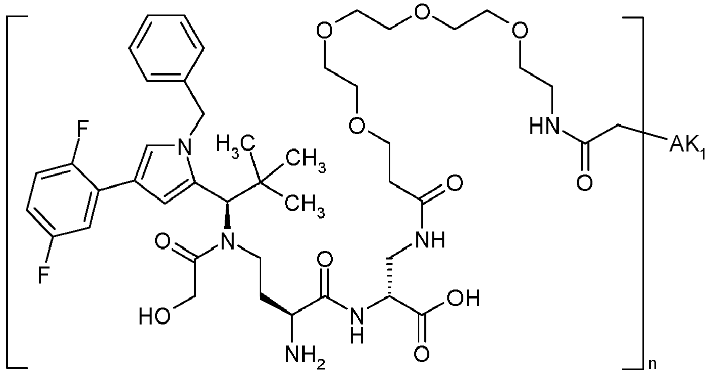 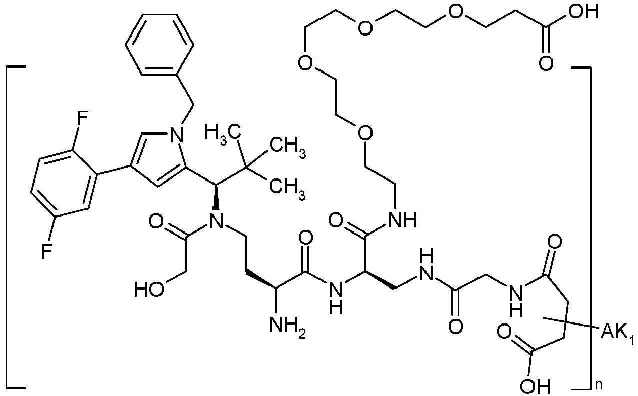 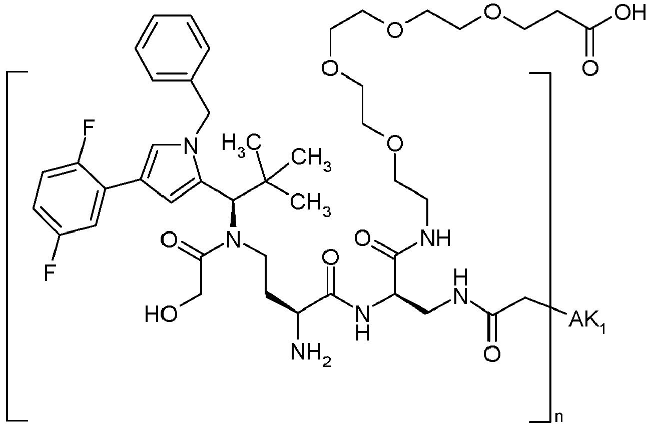 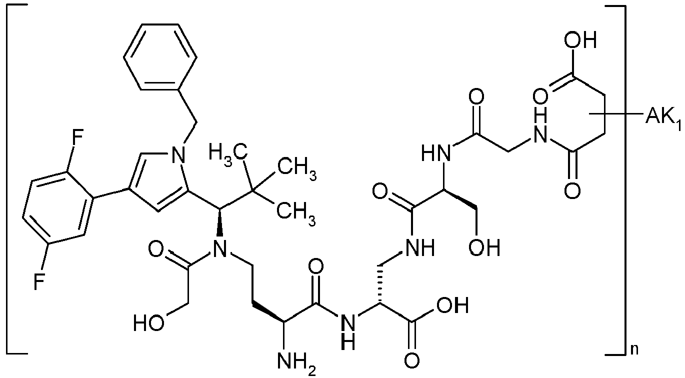 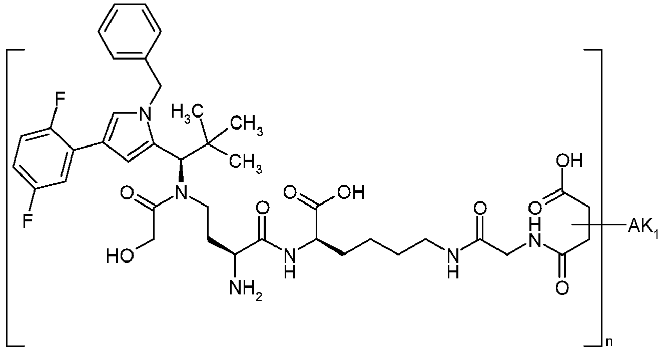 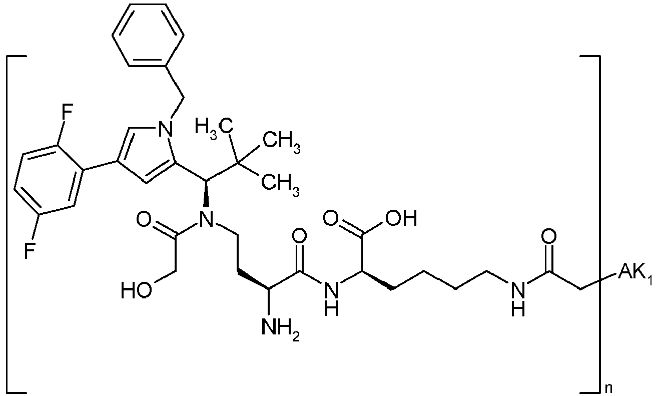 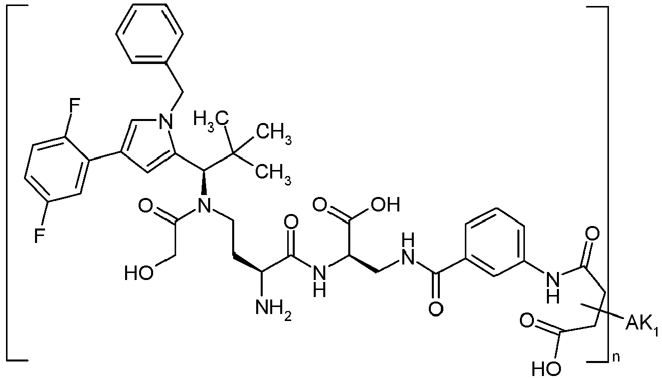 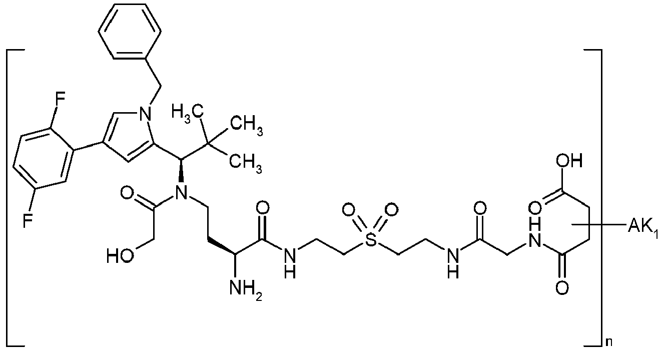 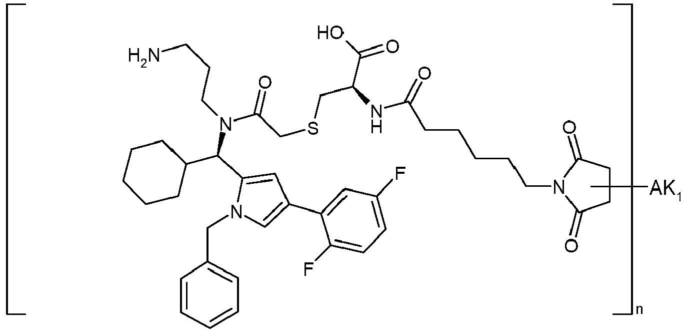 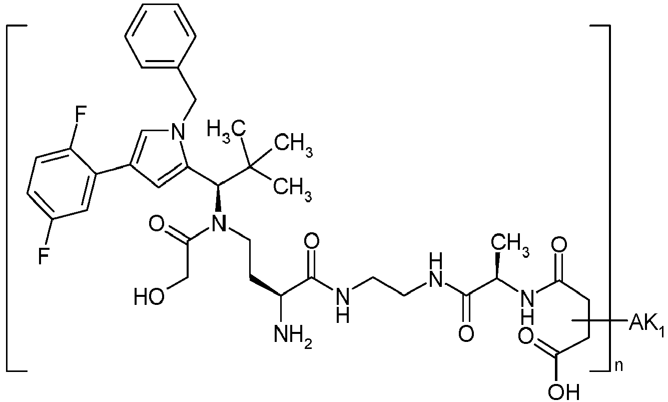 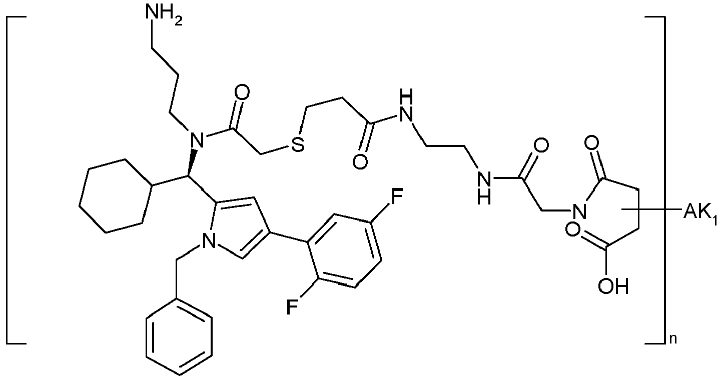
   - wobei: n eine Zahl von 1 bis 20 ist,
   - AK1: einen über Cystein verknüpften Antikörper darstellt,
   - AK2: einen über Lysin verknüpften Antikörper dar, und das Wasserstoffatom in Position R⁴ gemäß Formel IIa (d.h. in der Gruppe -NH₂) durch eine Gruppe der Formel R²¹-C(=O)-P3-P2-NH-CH(CH₂C(=O)-NH₂)-C(=O)- oder eine durch Cathepsin spaltbare Gruppe ersetzt ist, wobei
   R²¹ eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -SO₃H, -COOH, -SH, oder -OH substituiert sein kann,
   P2 eine Einfachbindung oder eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist;
   P3 eine Einfachbindung oder eine Aminosäure ausgewählt aus Gly, Pro, Ala, Val, Leu, Ile, Met, Phe, Tyr, Trp, Ser, Thr, Cys, Asn, Gln, Asp, Glu, Lys, Arg, und His ist.
50. Antikörper-Prodrug-Konjugat nach einem oder mehreren der Ansprüche 1 bis 48, wobei das Antikörper-Prodrug-Konjugat eine der folgenden Formeln genügt: 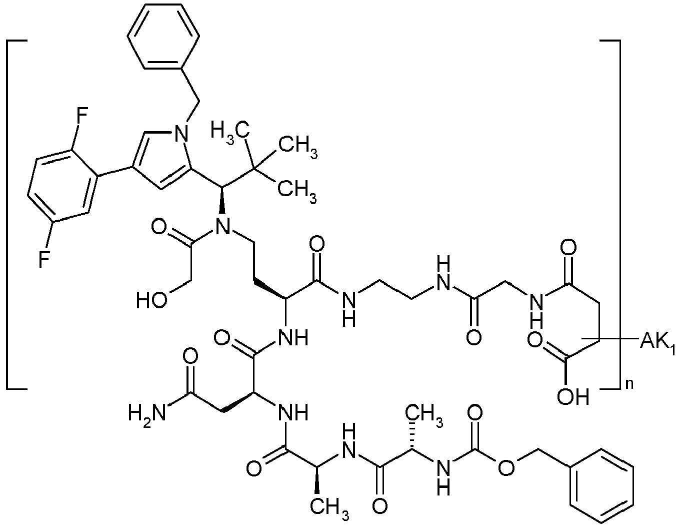 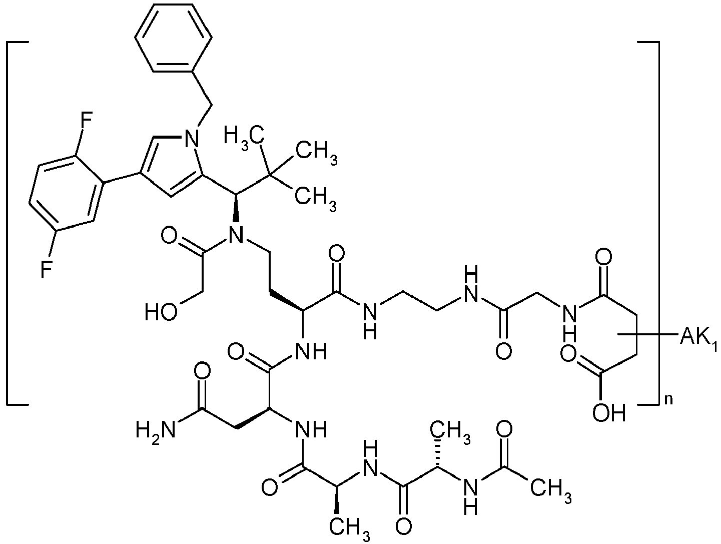 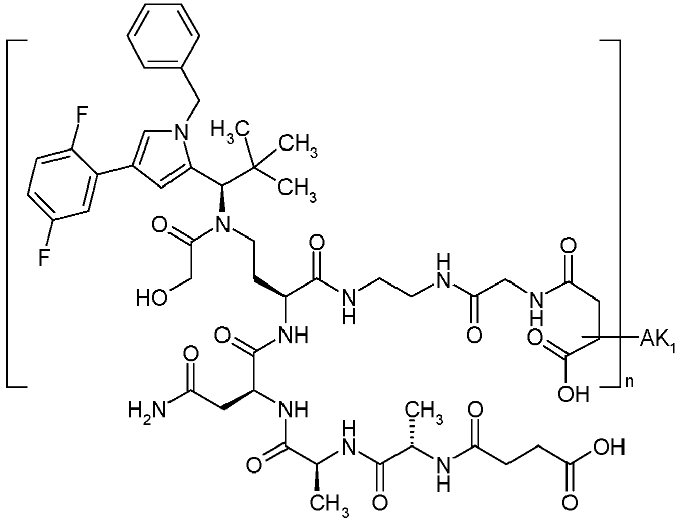 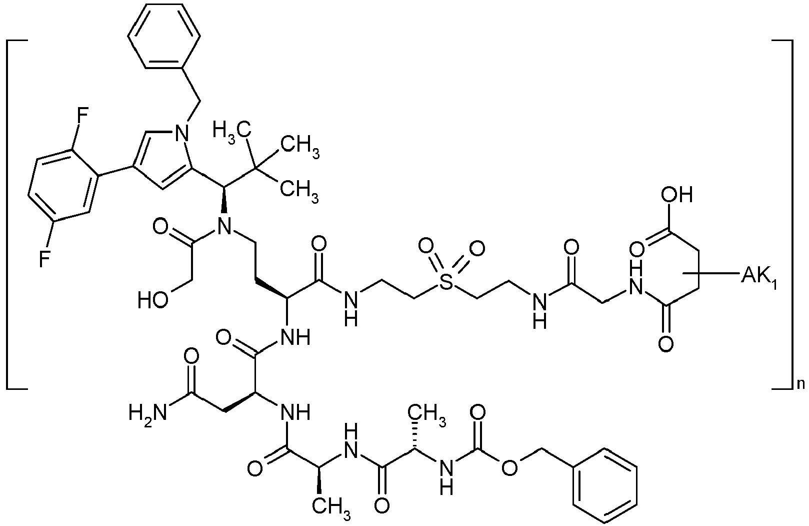 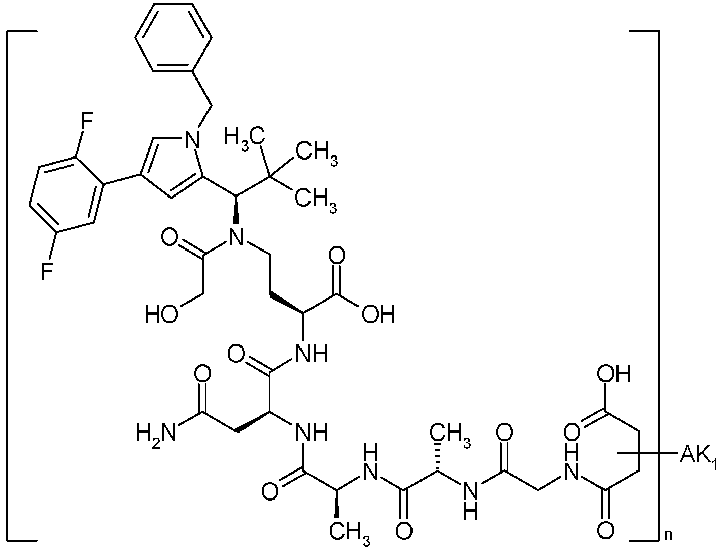 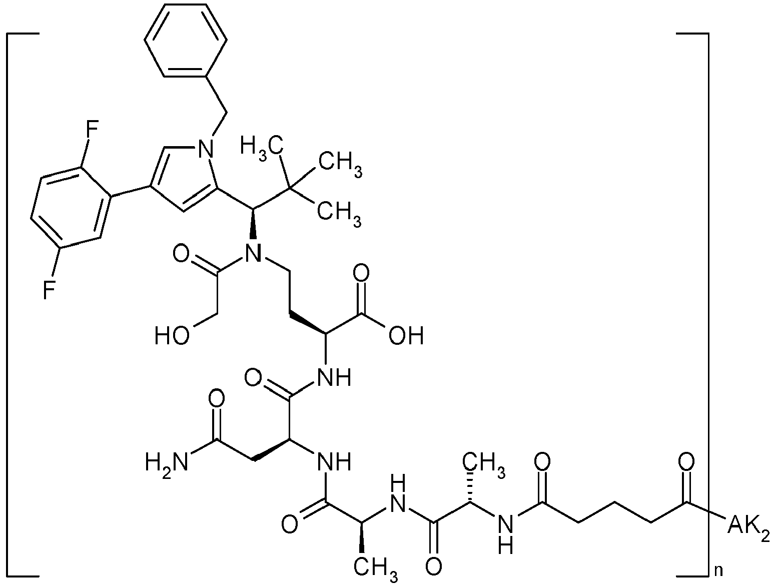 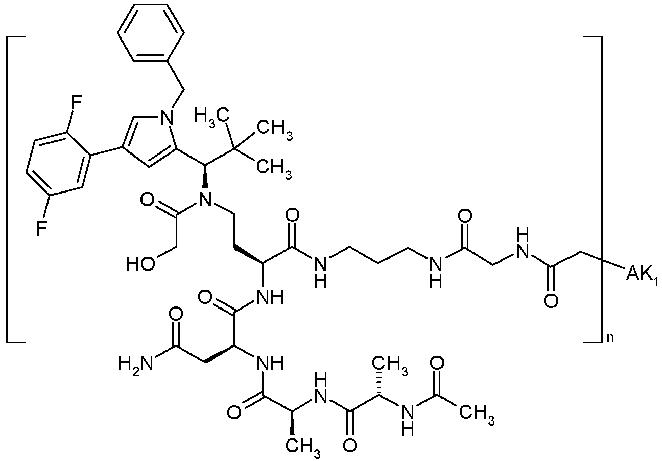 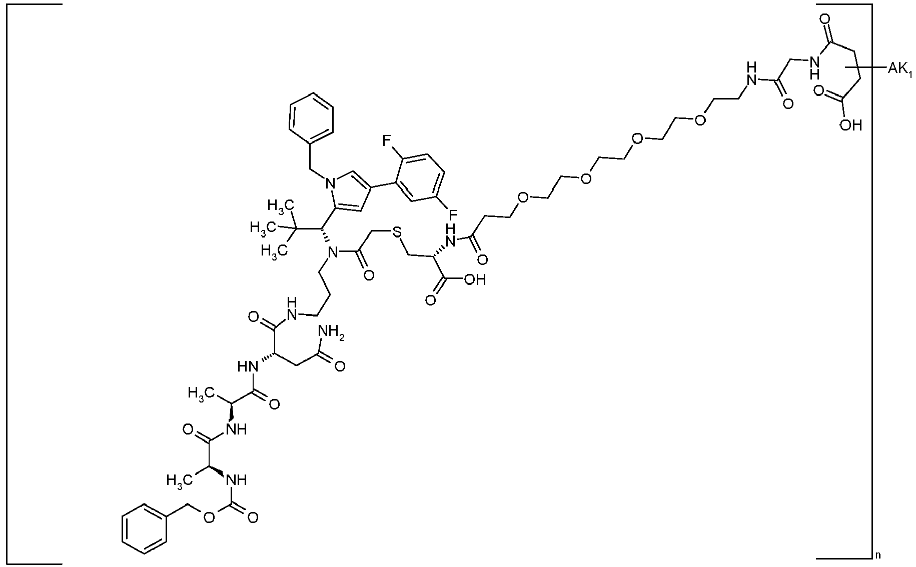 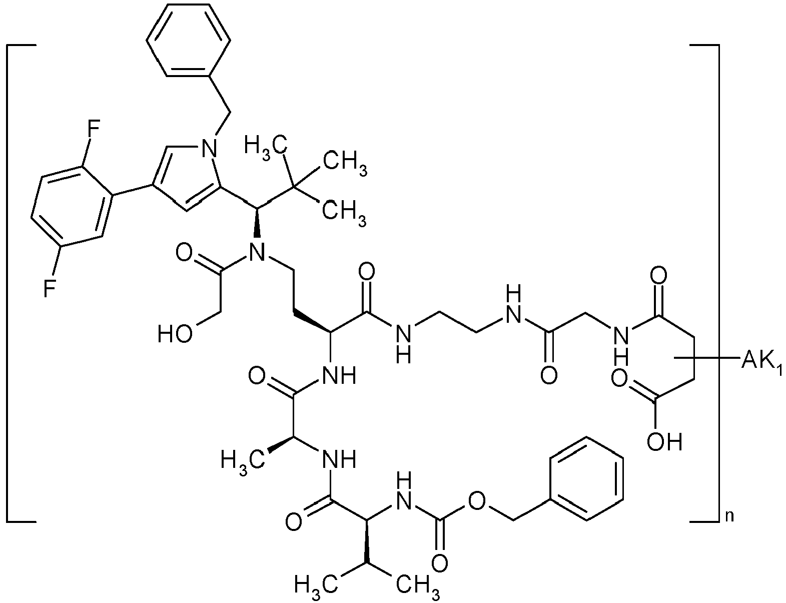 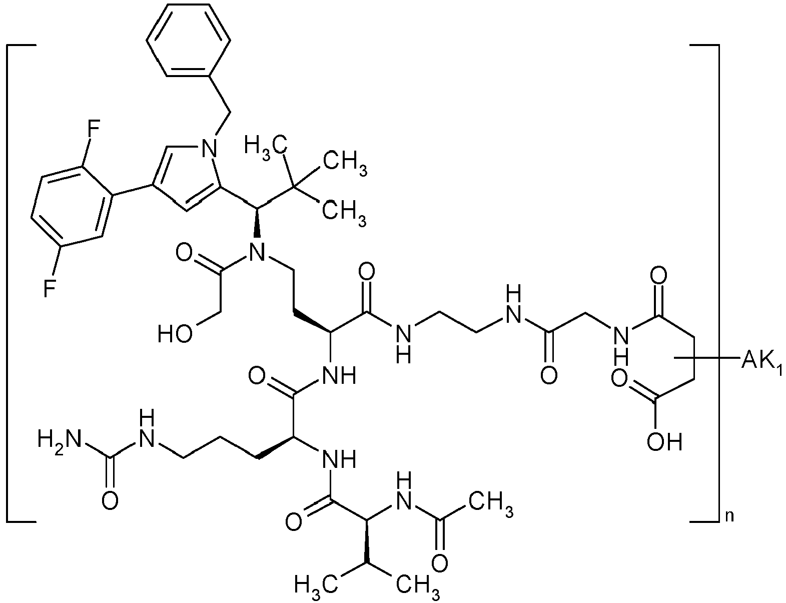 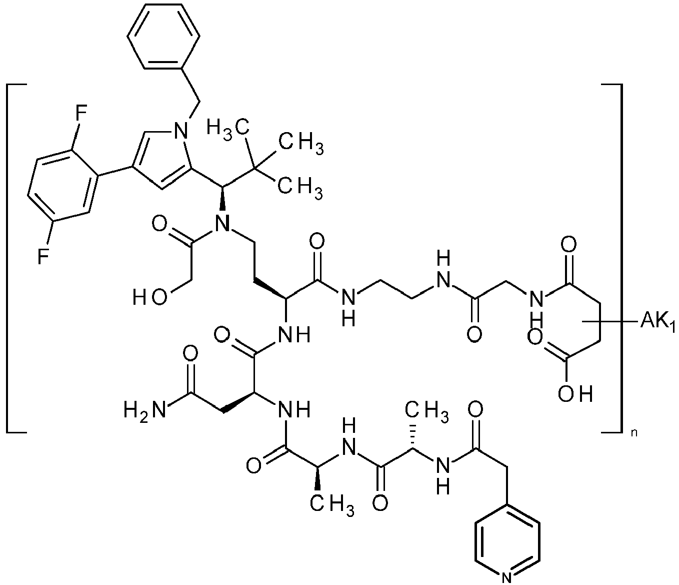 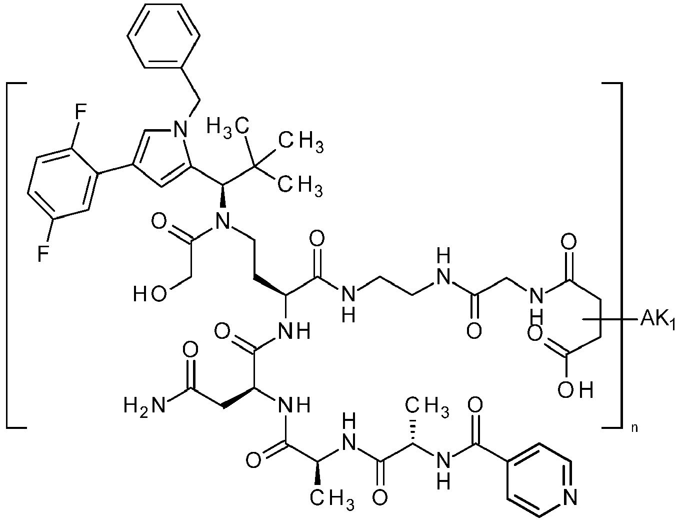 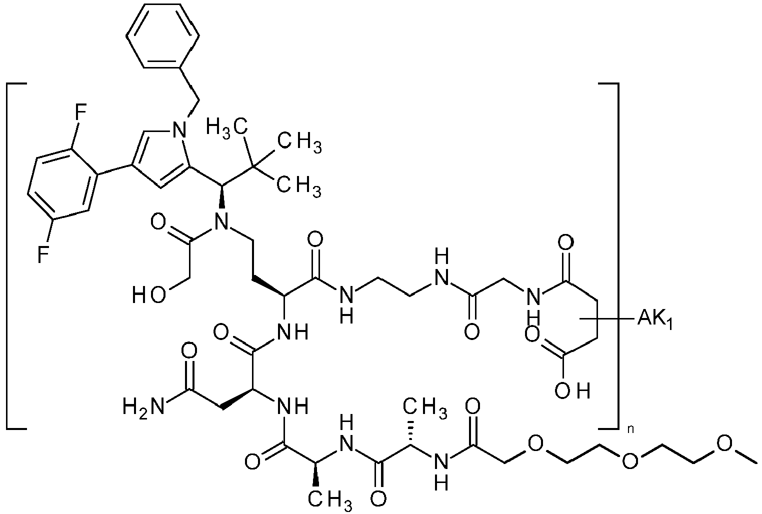 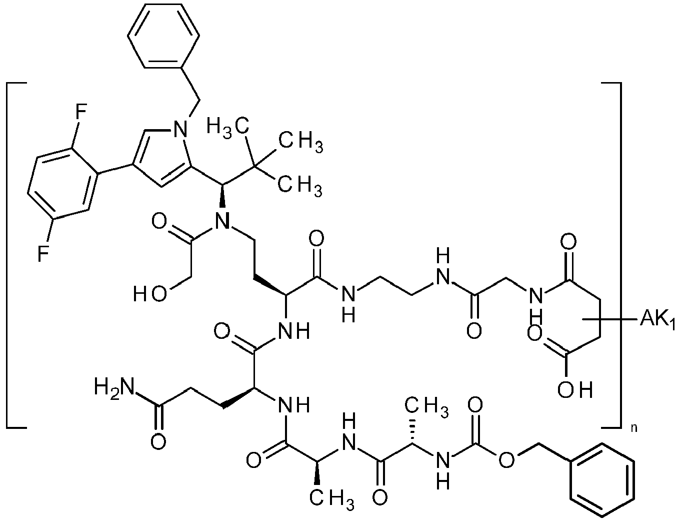 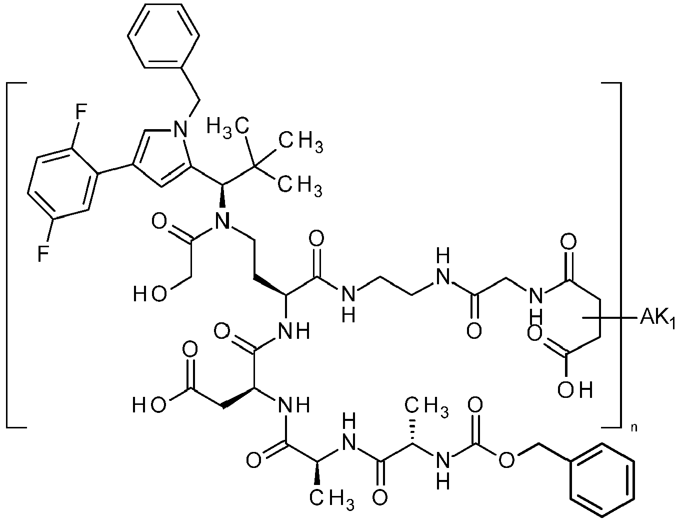 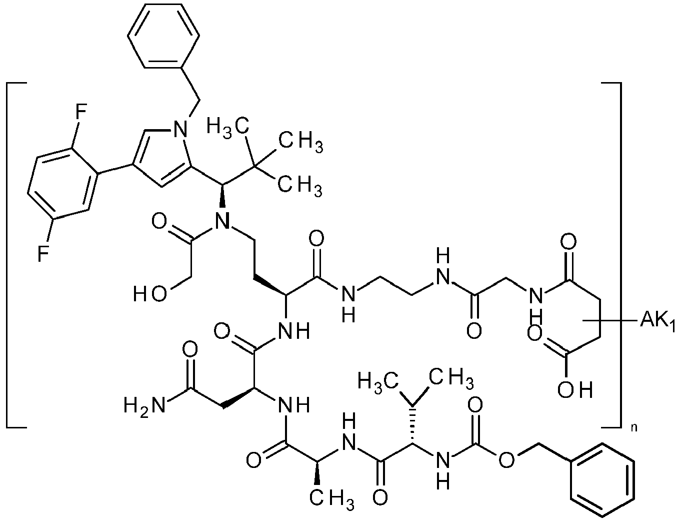 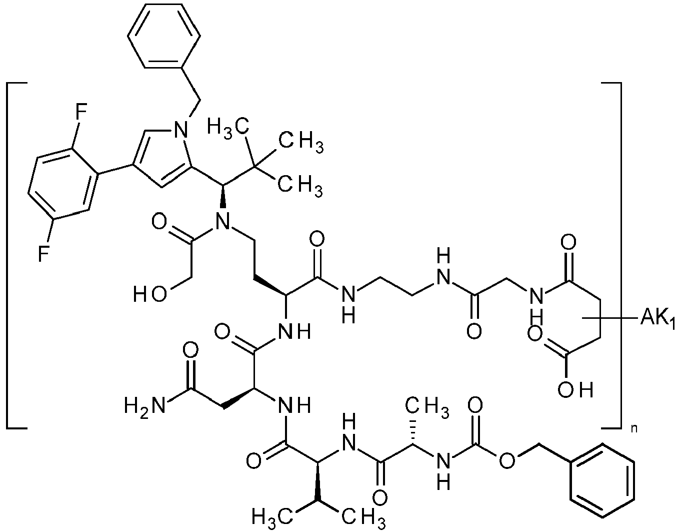 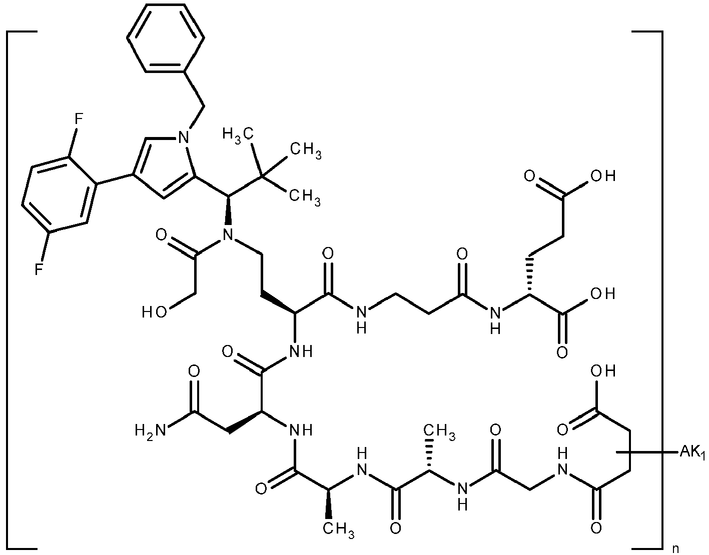 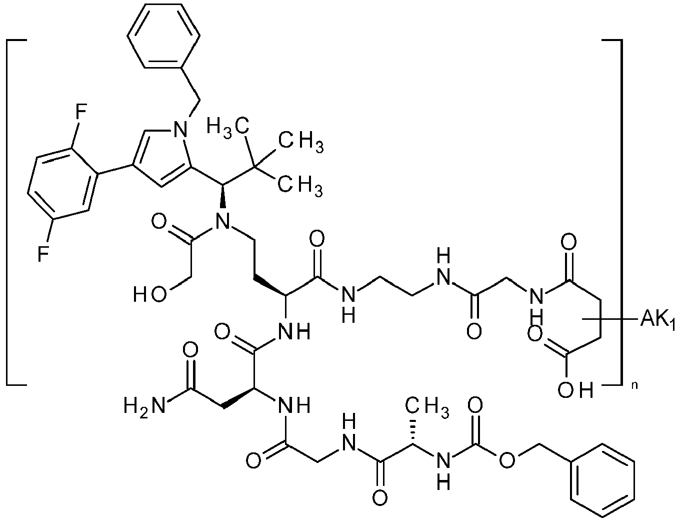 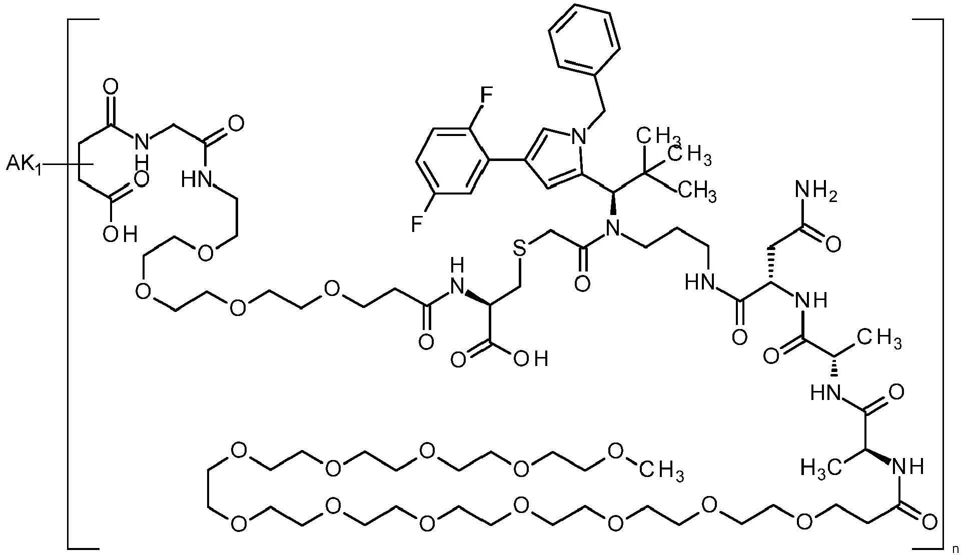 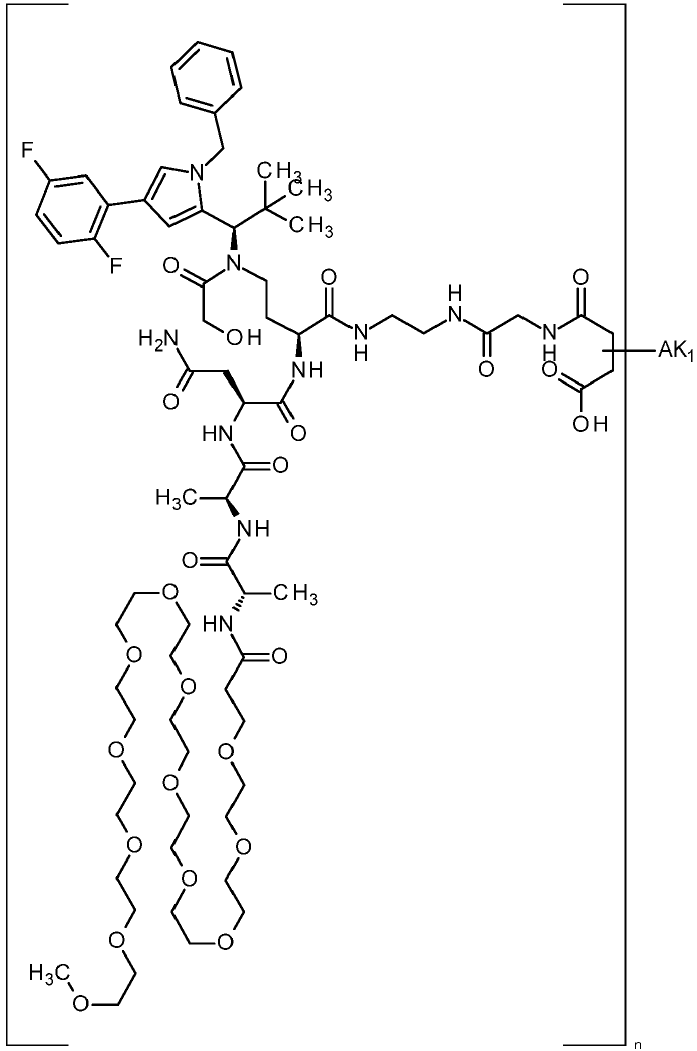 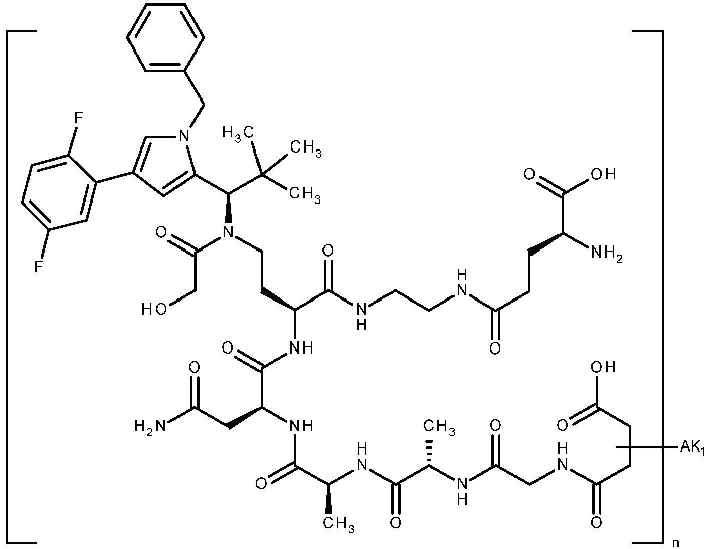 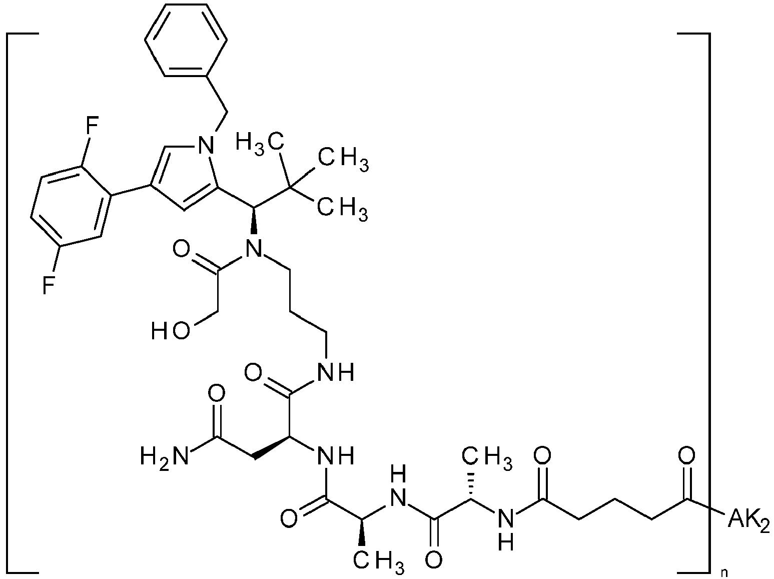 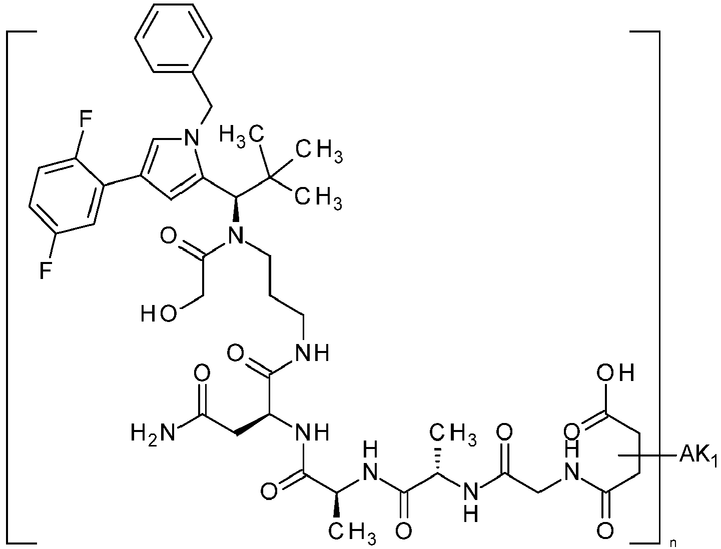 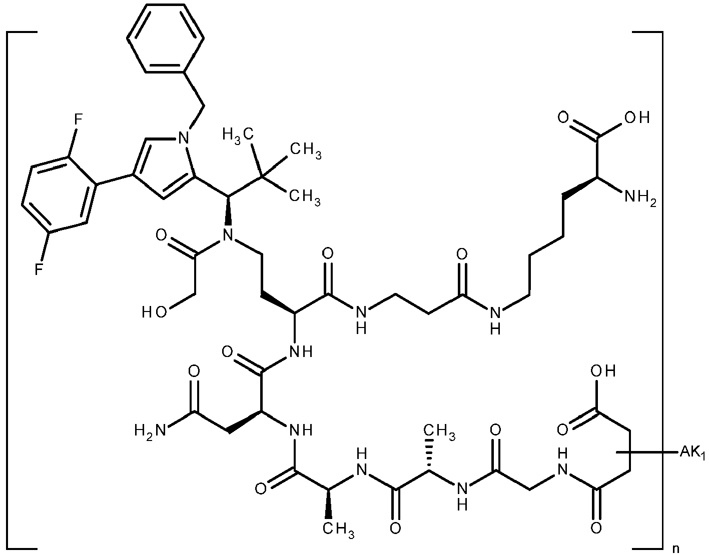 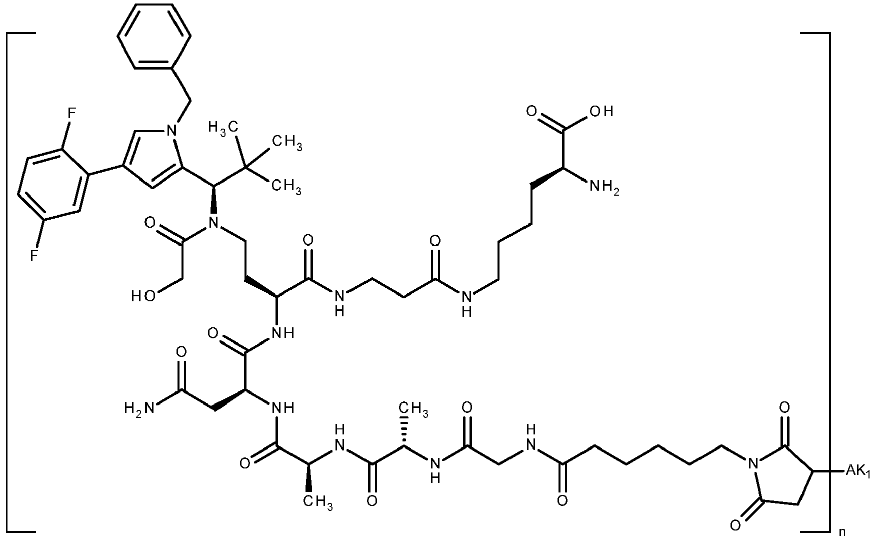 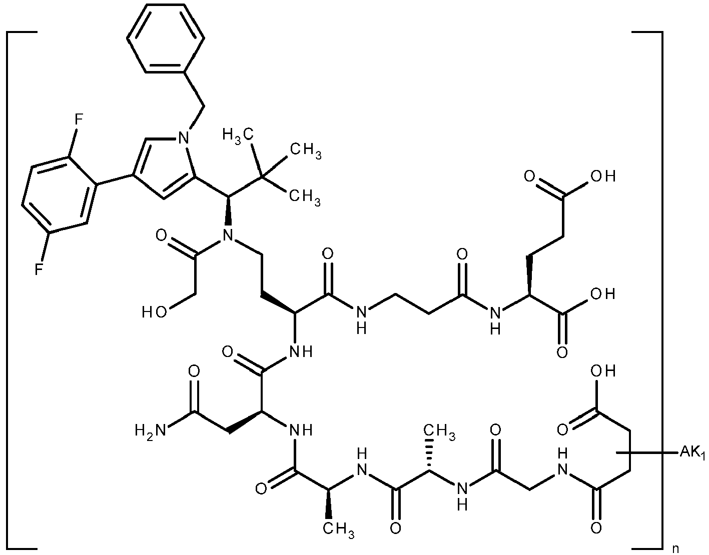 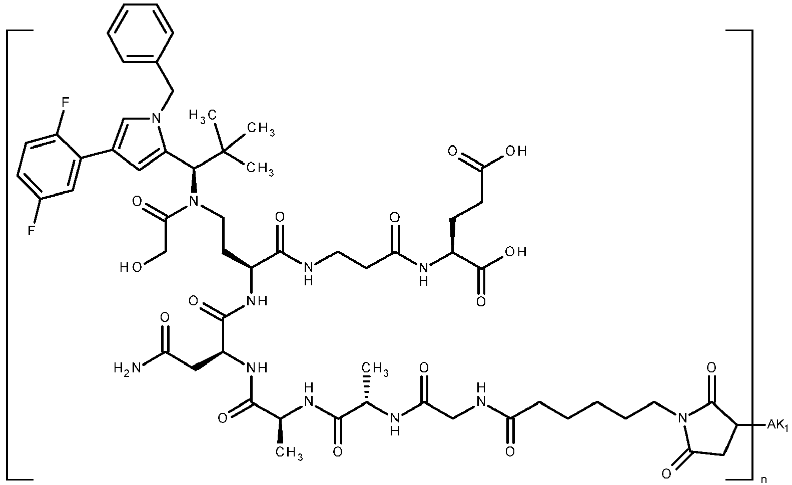 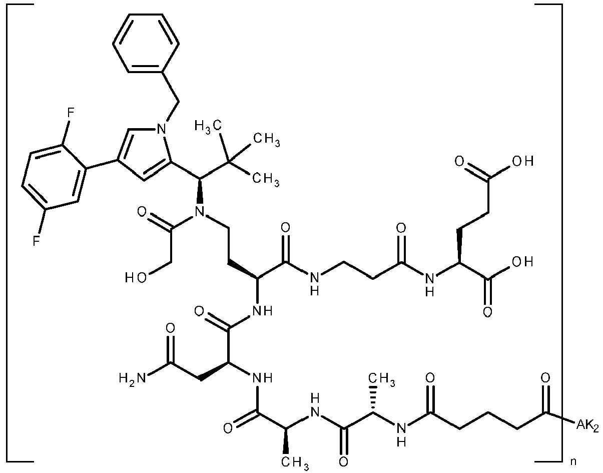 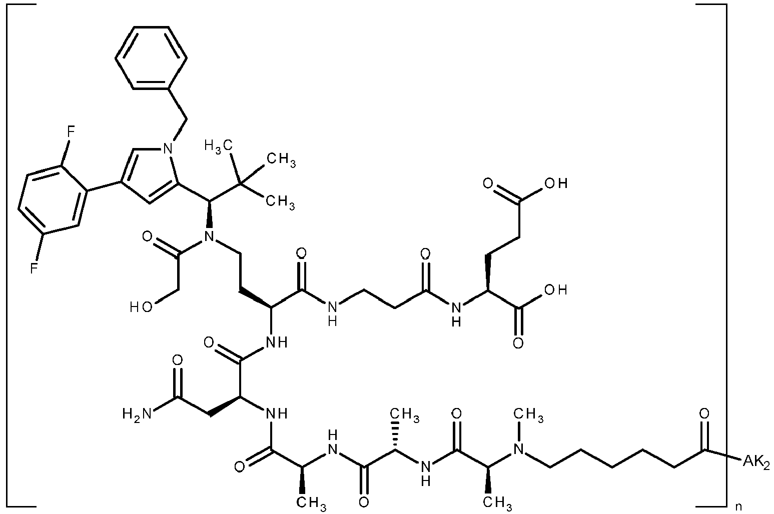 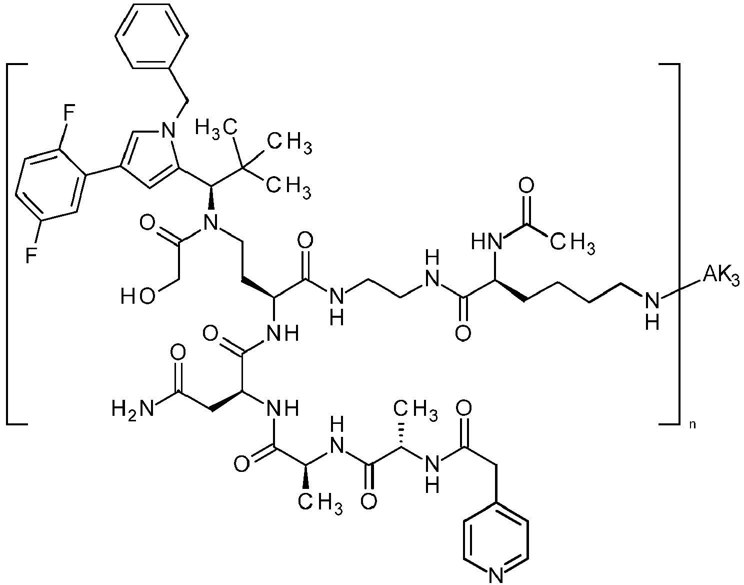 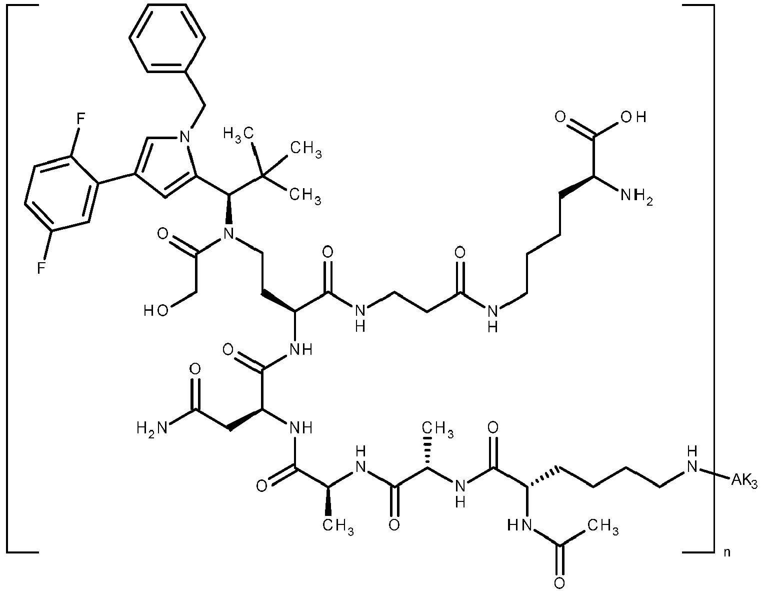 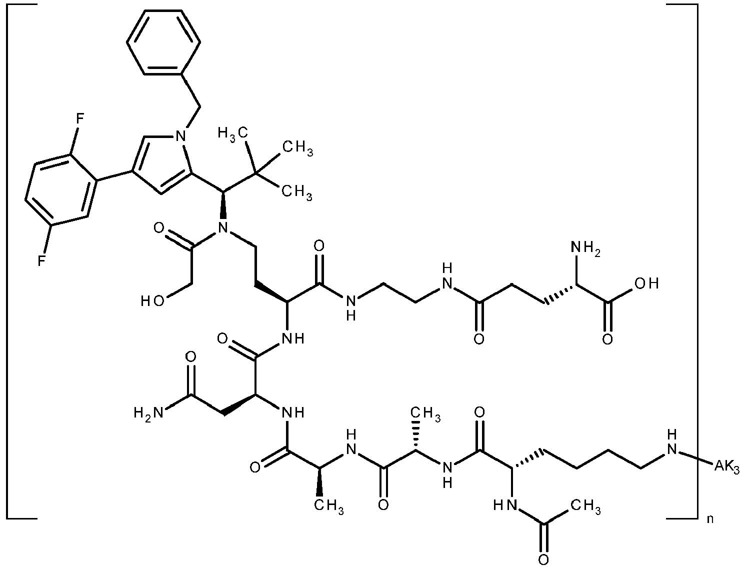 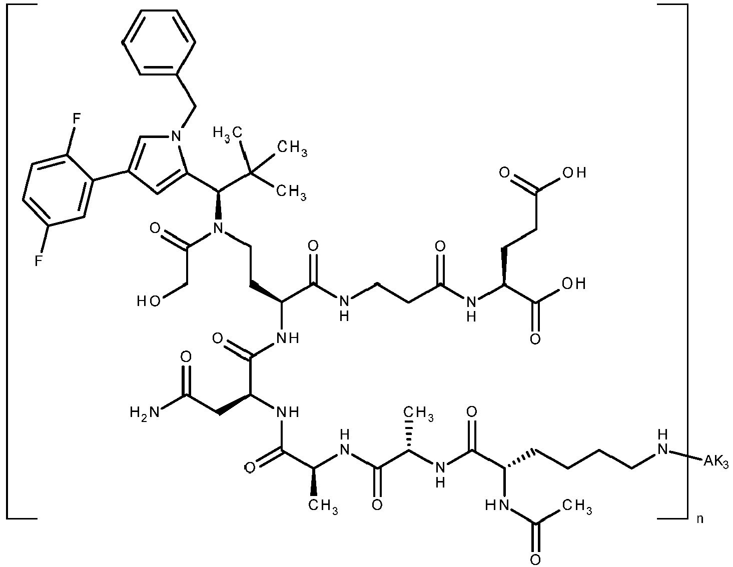 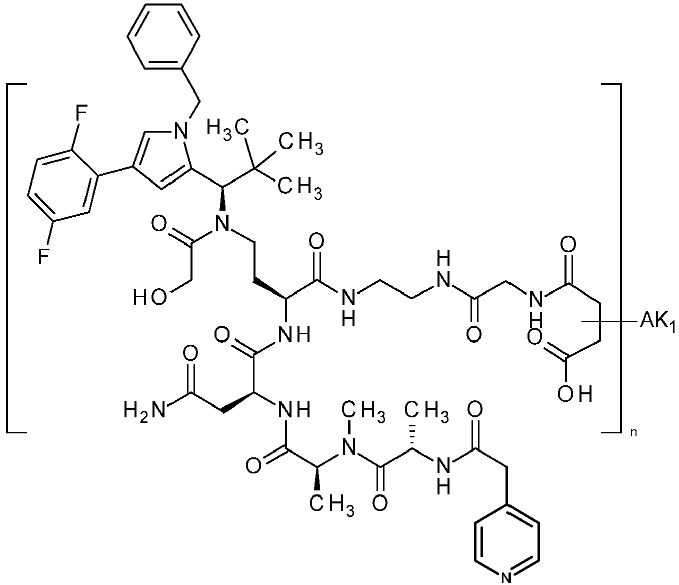 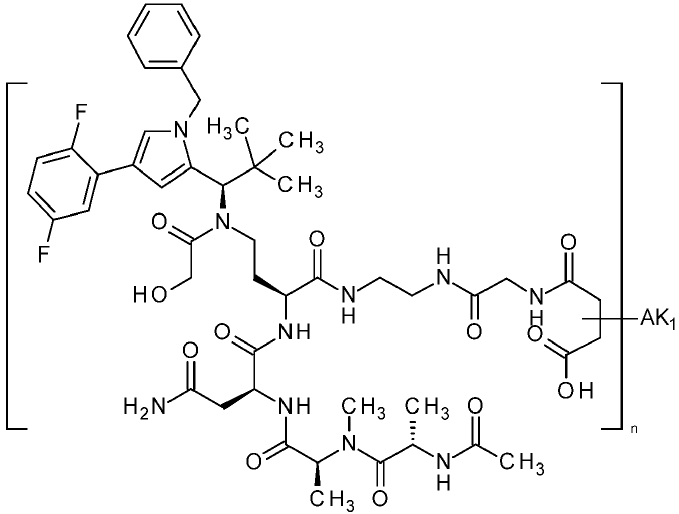 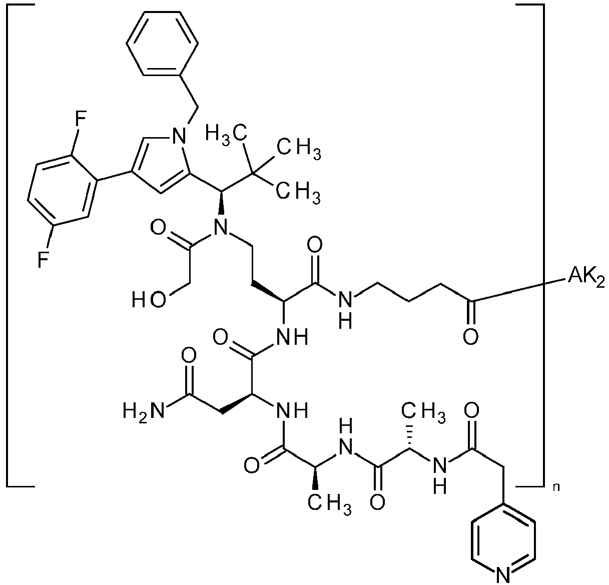 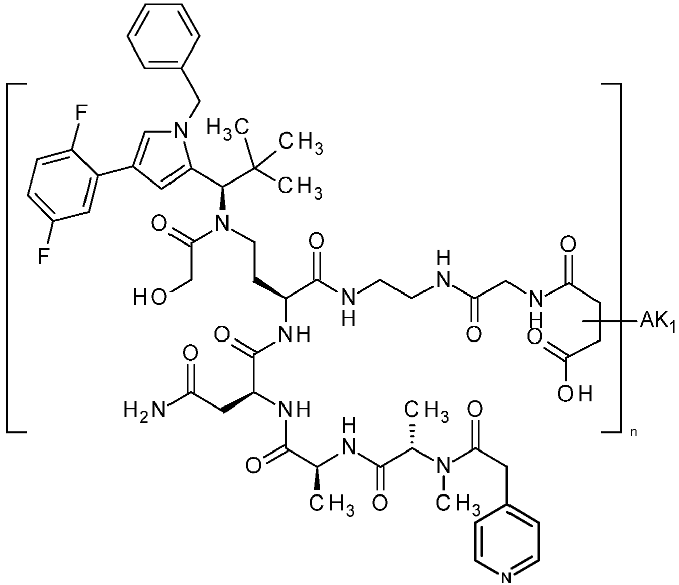 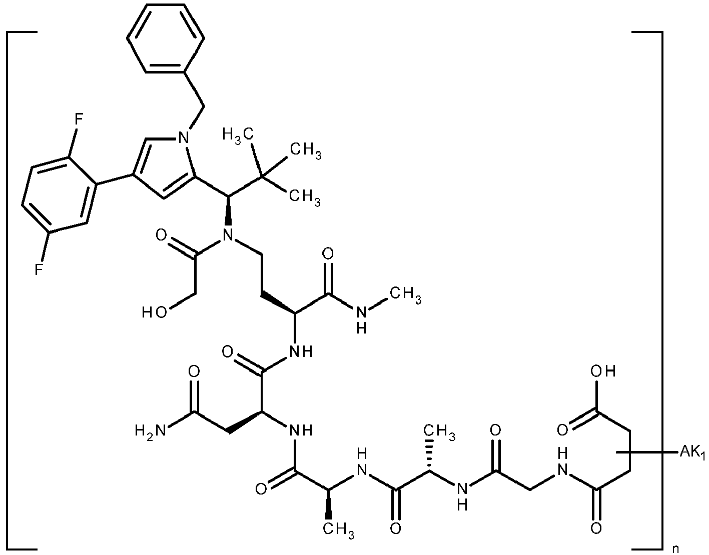 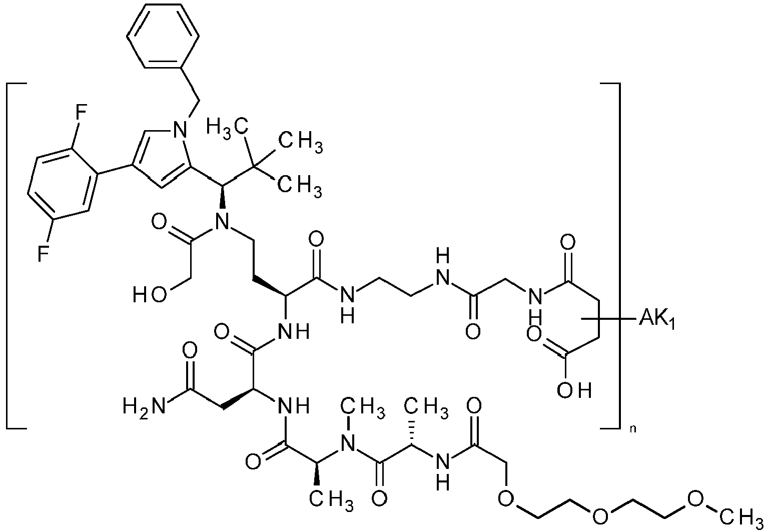 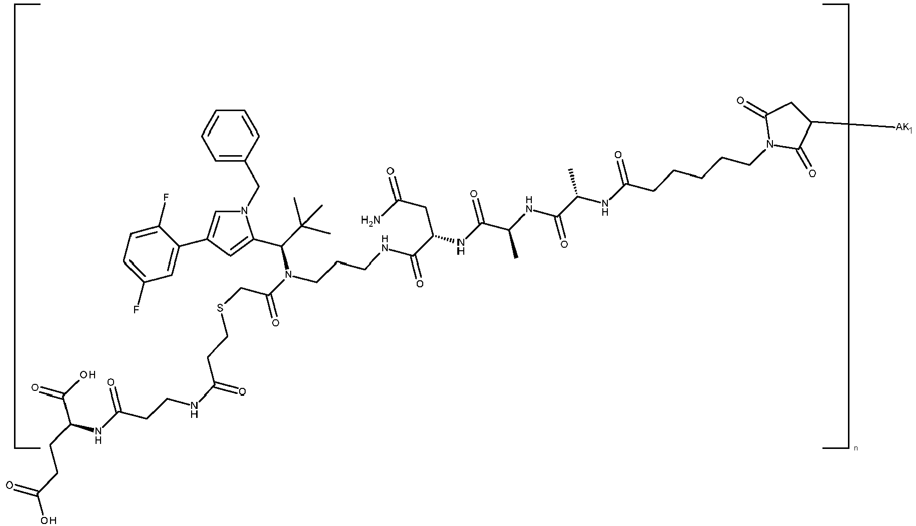 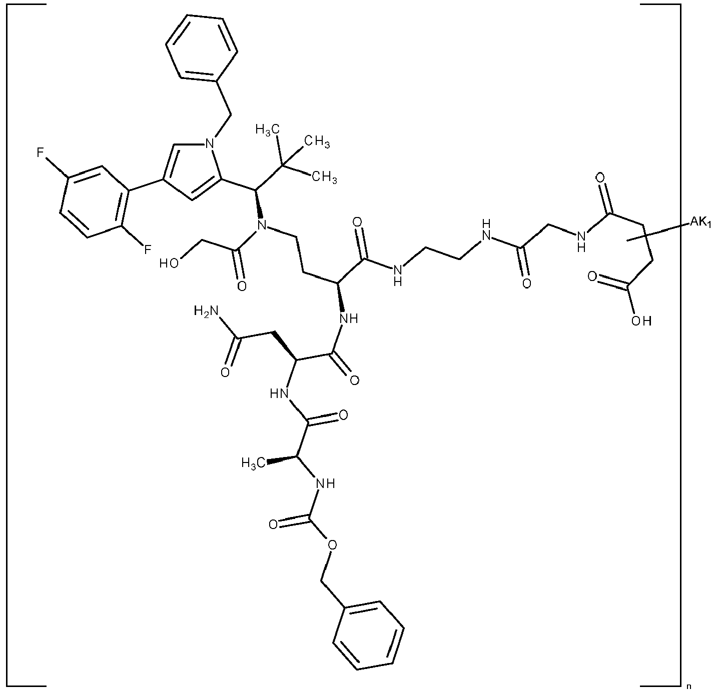 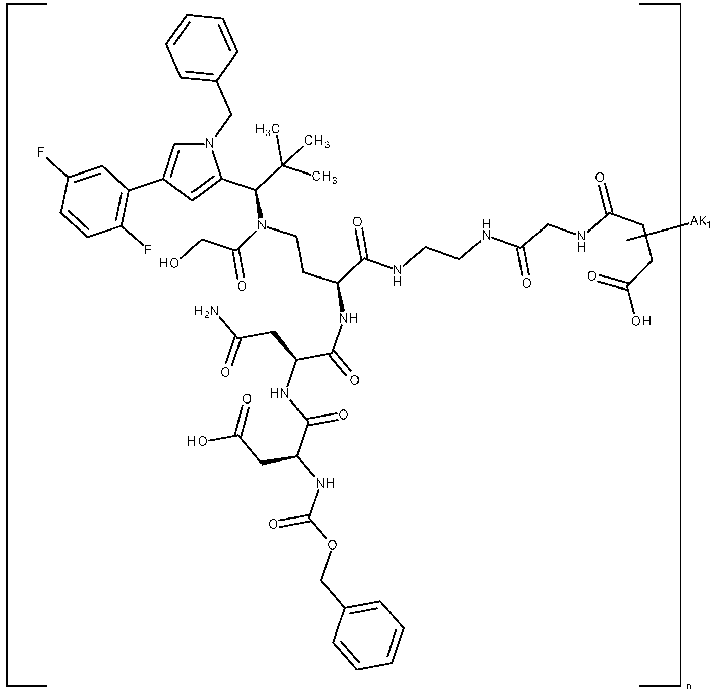 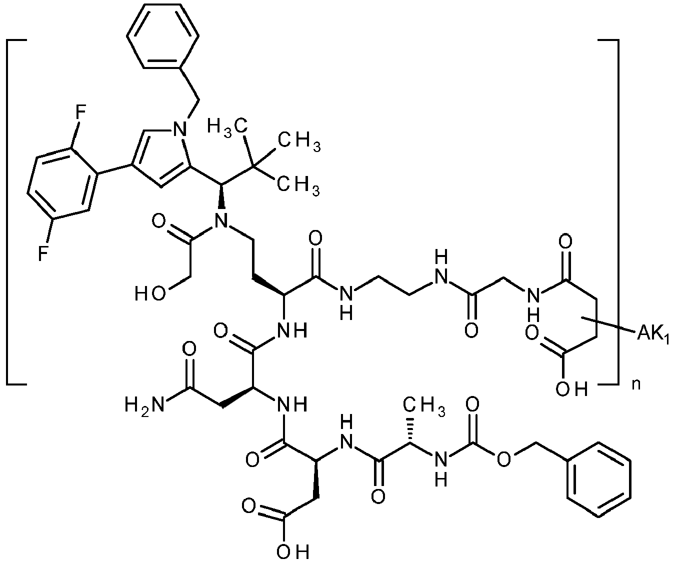 wobei
   - n: eine Zahl von 1 bis 20 ist,
   - AK₁: einen über Cystein verknüpften Antikörper darstellt,und
   - AK₂: einen über Lysin verknüpften Antikörper darstellt:
   - AK₃: einen über einen Glutamin verknüpften Antikörper darstellt;
51. Antikörper-Prodrug-Konjugat nach einer oder mehrerer der vorhergehenden Ausführungsformen 1 bis 50, wobei
   - n: eine Zahl von 1 bis 20 ist,
   - AK1: einen über Cystein verknüpften Antikörper darstellt,und
   - AK2: einen über Lysin verknüpften Antikörper darstellt:

   wobei das Antikörper-Prodrug-Konjugat, sofern es über eine Gruppe 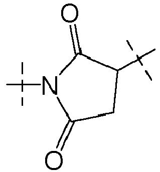 verfügt,
   mit dem Antikörper verbunden ist, statt dieser Gruppe auch zumindest teilweise in Form der hydrolysierten offenkettigen Bernsteinsäureamide mit dem Antikörper verknüpft vorliegen kann.
52. Pharmazeutische Zusammensetzung umfassend ein Konjugat nach einer oder mehrerer der vorhergehenden Ausführungsformen in Kombination mit einem inerten, nicht-toxischen, pharmazeutisch geeigneten Hilfsstoff.
53. Konjugat nach einer oder mehrerer der vorhergehenden Ausführungsformen zur Verwendung in einem Verfahren zur Behandlung und/oder Prophylaxe von Krankheiten.
54. Konjugat nach einer oder mehrerer der vorhergehenden Ausführungsformen zur Verwendung in einem Verfahren zur Behandlung von hyperproliferativen und/oder angiogenen Erkrankungen.
55. Kinesin Spindel Protein-Inhibitor- Prodrug der folgenden Formel (III): 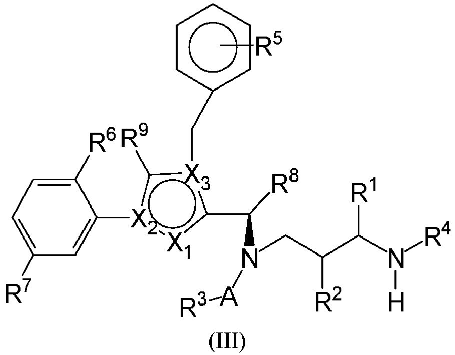 wobei
   X₁ N, X₂ N und X₃ C darstellt; oder
   X₁ N, X₂ C und X₃ N darstellt; oder
   X₁ CH oder CF, X₂ C und X₃ N darstellt; oder
   X₁ NH, X₂ C und X₃ C darstellt; oder
   X₁ CH, X₂ N und X₃ C darstellt;
   (wobei X₁ CH, X₂ C und X₃ N bevorzugt ist);

   - R¹: -H,-MOD oder -(CH₂)₀₋₃Z darstellt,
   wobei Z -H, -NHY³, -OY³, -SY³, Halogen, -C(=O)-NY¹Y², oder -C(=O)-OY³ darstellt,
   wobei Y¹ und Y² unabhängig voneinander -H, -NH₂, -(CH₂CH₂O)₀₋₃-(CH₂)₀₋₃Z' (z.B. -(CH₂)₀₋₃Z'), oder -CH(CH₂W)Z' darstellen,
   wobei Y³ -H oder -(CH₂)₀₋₃Z' darstellt,
   wobei Z' -H, -NH₂, -SO₃H, -COOH, -NH-C(=O)-CH₂-CH₂-CH(NH₂)COOH oder -(C(=O)-NH-CHY⁴)₁₋₃COOH darstellt;
   - wobei: W -H oder -OH darstellt,
   - wobei: Y⁴ lineares oder verzweigtes, gegebenenfalls mit -NH-C(=O)-NHz substituiertes, C₁₋₆ Alkyl oder gegebenenfalls mit -NH₂ substituiertes Aryl oder Benzyl darstellt;
   - R²: -H, -MOD, -C(=O)-CHY⁴-NHY⁵ oder -(CH₂)₀₋₃Z darstellt,
   wobei Z -H, Halogen, -OY³, -SY³, -NHY³, -C(=O)-NY¹Y², oder -C(=O)-OY³ darstellt,
   wobei Y¹ und Y² unabhängig voneinander -H, -NH₂, oder -(CH₂)₀₋₃Z' darstellen,
   wobei Y³ -H oder -(CH₂)₀₋₃Z' darstellt,
   wobei Z' -H, -SO₃H, -NH₂ oder -COOH darstellt;
   wobei Y⁴ lineares oder verzweigtes, gegebenenfalls mit -NH-C(=O)-NHz substituiertes, C₁₋₆ Alkyl oder gegebenenfalls mit -NH₂ substituiertes Aryl oder Benzyl darstellt
   wobei Y⁵ -H oder -C(=O)-CHY⁶-NH₂ darstellt,
   wobei Y⁶ lineares oder verzweigtes C₁₋₆ Alkyl darstellt;
   - R⁴: eine Gruppe der Formel R²¹-(C=O)₍₀₋₁₎-(P3)₍₀₋₂₎-P2-NH-CH(CH₂C(=O)-NH₂)-C(=O)- oder die durch Cathepsin spaltbare Gruppe der Formel R²¹-(C=O)₍₀₋₁₎-(P3)₍₀₋₂₎-P2- darstellt,
   wobei R²¹ eine C₁₋₁₀-Alkyl-, C₅₋₁₀-Aryl- oder C₆₋₁₀-Aralkyl-, C₅₋₁₀-Heteroalkyl-, C₁₋₁₀-Alkyl-O-C₆₋₁₀-Aryl-, C₅₋₁₀-Heterocycloalkyl-, Heteroaryl-, Heteroaryl-alkyl-, C₁₋₁₀-Alkoxy-, C₆₋₁₀-Aryloxy- oder C₆₋₁₀-Aralkoxy-, C₅₋₁₀-Heteroalkoxy-, C₁₋₁₀-Alkyl-O-C₆₋₁₀-Aryloxy-, C₅₋₁₀-Heterocycloalkoxy-Gruppe darstellt, die ein oder mehrfach mit - NH₂, -NH-Alkyl, -N(Alkyl)₂, -NH-C(=O)-Alkyl, -N(Alkyl)-C(=O)-Alkyl, -SO₃H, -S(=O)₂-NH₂, -S(=O)₂-N(Alkyl)₂, -COOH, -C(=O)-NH₂, -C(=O)-N(Alkyl)z, oder -OH substituiert sein kann, -H oder eine Gruppe -Oₓ-(CH₂CH₂O)ᵥ-R²² darstellt,
   wobei x 0 oder 1 ist,
   wobei v eine Zahl von 1 bis 20 darstellt,
   wobei R²² -H, -Alkyl (vorzugsweise C₁₋₁₂-Alkyl), -CH₂-COOH, -CH₂-CH₂-COOH, oder -CH₂-CH₂-NH₂ darstellt;
   P2 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His ist;
   P3 eine Aminosäure ausgewählt aus Gly, Pro, Ala, Val, Nva, Leu, Ile, Met, Phe, Tyr, Trp, Ser, Thr, Cys, Asn, Gln, Asp, Glu, Lys, Arg, Citrullin und His oder eine der jeweiligen N-Alkyl-Aminsoäuren, bevorzugt N-Methyl-Aminsoäuren ist;
   - A: -C(=O)-, -S(=O)-, -S(=O)z-, -S(=O)z-NH- oder -C(=N-NH₂)-darstellt;
   - R³: -MOD, oder eine gegebenenfalls substituierte Alkyl-, Cycloalkyl-, Aryl-, Heteroaryl- Heteroalkyl-, Heterocycloalkyl- Gruppe darstellt, eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 -O-Alkyl Gruppen, 1-3 -SH Gruppen, 1-3 -S-Alkyl Gruppen, 1-3 -O-CO-Alkyl Gruppen, 1-3 -O-C(=O)-NH-Alkyl Gruppen, 1-3-NH-C(=O)-Alkyl Gruppen, 1-3 -NH-C(=O)-NH-Alkyl Gruppen, 1-3-S(=O)ₙ-Alkyl Gruppen, 1-3 -S(=O)z-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen substituiert sein kann (wobei "Alkyl" bevorzugt C₁₋₁₀-Alkyl bedeutet)
   wobei Z -H, Halogen, -OY³, -SY³, -NHY³, -C(=O)-NY¹Y², oder -C(=O)-OY³ darstellt,
   wobei n für 0, 1 oder 2 steht,
   wobei Y¹ und Y² unabhängig voneinander -H, -NH₂, oder -(CH₂)₀₋₃Z' darstellen,
   wobei Y³ -H, -(CH₂)₀₋₃-CH(NH-C(=O)-CH₃)Z', -(CH₂)₀₋₃-CH(NH₂)Z', oder -(CH₂)₀₋₃Z' darstellt,
   wobei Z' -H, -SO₃H, -NH₂ oder -COOH darstellt,
   - R⁵: -H, -NH₂, -NOz, Halogen (insbesondere- F, -Cl, -Br), -CN, -CF₃, -OCF₃, -CH₂F, -CH₂F, -SH oder -(CH₂)₀₋₃Z darstellt,
   wobei Z -H, -OY³, -SY³, Halogen, -NHY³, -C(=O)-NY¹Y², oder -C(=O)-OY³ darstellt,
   wobei Y¹ und Y² unabhängig voneinander -H, -NH₂, oder -(CH₂)₀₋₃Z' darstellen,
   wobei Y³ -H oder -(CH₂)₀₋₃Z' darstellt,
   wobei Z' -H, -SO₃H, -NH₂ oder -COOH darstellt;
   - R⁶ und R⁷: unabhängig voneinander -H, Cyano, C₁₋₁₀-Alkyl, Fluor-C₁₋₁₀-Alkyl, C₂₋₁₀-Alkenyl, Fluor-C₂₋₁₀-Alkenyl, C₂₋₁₀-Alkinyl, Fluor-C₂₋₁₀-Alkinyl, Hydroxy, -NOz, -NH₂, -COOH oder Halogen (insbesondere -F, -Cl,-Br) darstellen,
   - R⁸: C₁₋₁₀-Alkyl, Fluor-C₁₋₁₀-Alkyl, C₂₋₁₀-Alkenyl, Fluor-C₂₋₁₀-Alkenyl, C₂₋₁₀-Alkinyl, Fluor-C₂₋₁₀-Alkinyl, C₄₋₁₀-Cycloalkyl, Fluor- C₄₋₁₀-Cycloalkyl oder-(CH₂)₀₋₂-(HZ²) darstellt,
   wobei HZ² ein 4 bis 7-gliedriger Heterocyclus mit bis zu zwei Heteroatomen ausgewählt aus N, O und S darstellt, wobei jede dieser Gruppen mit -OH, -COOH oder -NH₂ substituiert sein kann;
   - R⁹: -H, -F, -CH₃, -CF₃, -CH₂F oder -CHF₂ darstellt;
   - -MOD: -(NR¹⁰)ₙ-(G1)ₒ-G2-H darstellt,
   wobei
   R¹⁰ -H oder C₁-C₃-Alkyl darstellt;
   G1 -NH-C(=O)-, -C(=O)NH- oder 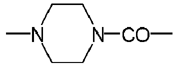 darstellt (wobei wenn G1 -NH-C(=O)- oder 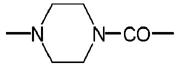 darstellt, R¹⁰ nicht -NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach, gleich oder verschieden durch die Gruppen -O-, -S-, -S(=O)-, S(=O)₂, -NR^{y}-, -NRYC(=O)-, -C(=O)NRY-, -NR^{y}NR^{y}-, -S(=O)z-NRYNRY-, -C(=O)-NR^{y}NR^{y}-C(=O)-, -CR^{x}=N-O-unterbrochen sein kann,
   wobei Rx -H, C₁-C₃-Alkyl oder Phenyl darstellt,
   wobei R^{y} -H, Phenyl, C₁-C₁₀-Alkyl, C₂-C₁₀-Alkenyl, oder C₂-C₁₀-Alkinyl darstellt, die jeweils mit -NH-C(=O)-NH₂, -COOH, -OH, -NH₂, -NH-CN-NH₂, Sulfonamid, Sulfon, Sulfoxid, oder Sulfonsäure substituiert sein können, wobei die Kohlenwasserstoffkette einschließlich der Seitenketten, sofern vorhanden, mit -NH-C(=O)-NH₂, -COOH, -OH, -NH₂, -NH-CN-NH₂, Sulfonamid, Sulfon, Sulfoxid, oder Sulfonsäure substituiert sein kann,
   wobei die Gruppe -MOD vorzugsweise zumindest eine Gruppe -COOH aufweist; sowie ihre Salze, Solvate, Salze der Solvate und Epimere.

## Patentansprüche

1. Verbindung der folgenden Formel (III): 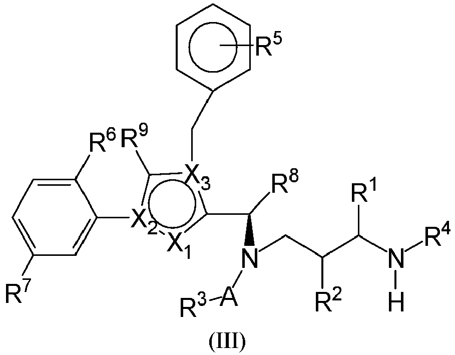 sowie ihre (pharmazeutisch akzeptablen) Salze, Solvate, Salze der Solvate und Epimere, wobei
X₁ N, X₂ N und X₃ C darstellt; oder
X₁ N, X₂ C und X₃ N darstellt; oder
X₁ CH oder CF, X₂ C und X₃ N darstellt; oder
X₁ NH, X₂ C und X₃ C darstellt; oder
X₁ CH or CF, X₂ N und X₃ C darstellt;
R¹ -H,-MOD oder -(CH₂)₀₋₃Z darstellt,
wobei Z -H, -NHY³, -OY³, -SY³, Halogen, -C(=O)-NY¹Y², oder -C(=O)-OY³ darstellt,
wobei Y¹ und Y² unabhängig voneinander -H, -NH₂, -(CH₂CH₂O)₀₋₃-(CH₂)₀₋₃Z', oder -CH(CH₂W)Z' darstellen,
wobei Y³ -H oder -(CH₂)₀₋₃Z' darstellt,
wobei W -H oder -OH darstellt,
wobei Z' -H, -NH₂, -SO₃H, -COOH, -NH-C(=O)-CH₂-CH₂-CH(NH₂)COOH oder -(C(=O)-NH-CHY⁴)₁₋₃COOH darstellt;
wobei Y⁴ gegebenenfalls mit -NH-C(=O)-NHz substituiertes C₁₋₆ Alkyl; oder
gegebenenfalls mit -NH₂ substituiertes Aryl oder Benzyl darstellt;
R² -H, -MOD, -C(=O)-CHY⁴-NHY⁵ oder -(CH₂)₀₋₃Z darstellt,
wobei Z -H, Halogen, -OY³, -SY³, -NHY³, -C(=O)-NY¹Y², oder -C(=O)-OY³ darstellt,
wobei Y¹ und Y² unabhängig voneinander -H, -NH₂, oder -(CH₂)₀₋₃Z' darstellen,
wobei Y³ -H oder -(CH₂)₀₋₃Z' darstellt,
wobei Z' -H, -SO₃H, -NH₂ oder -COOH darstellt;
wobei Y⁴ gegebenenfalls mit -NH-C(=O)-NHz substituiertes, C₁₋₆ Alkyl; oder gegebenenfalls mit -NH₂ substituiertes Aryl oder Benzyl darstellt
wobei Y⁵ -H oder -C(=O)-CHY⁶-NH₂ darstellt,
wobei Y⁶ C₁₋₆ Alkyl darstellt;
R⁴ -H darstellt;
A -C(=O)-, -S(=O)-, -S(=O)z-, -S(=O)z-NH- oder -C(=N-NH₂)-darstellt;
R³ -MOD, oder eine gegebenenfalls substituierte Alkyl-, Cycloalkyl-, Aryl-, Heteroaryl-, Heteroalkyl-, Heterocycloalkyl- Gruppe darstellt, C₆₋₁₀-Aryl, C₁₋₁₀-Alkyl-O-C₆₋₁₀-Aryl darstellt, die mit 1-3 -OH Gruppen, 1-3 Halogenatomen, 1-3 halogenierten Alkylgruppen (die jeweils 1-3 Halogenatome aufweisen), 1-3 -O-Alkyl Gruppen, 1-3 -SH Gruppen, 1-3 -S-Alkyl Gruppen, 1-3 -O-CO-Alkyl Gruppen, 1-3 -O-C(=O)-NH-Alkyl Gruppen, 1-3-NH-C(=O)-Alkyl Gruppen, 1-3 -NH-C(=O)-NH-Alkyl Gruppen, 1-3-S(=O)ₙ-Alkyl Gruppen, 1-3 -S(=O)z-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen substituiert sein kann
wobei Z -H, Halogen, -OY³, -SY³, -NHY³, -C(=O)-NY¹Y², oder -C(=O)-OY³ darstellt,
wobei n für 0, 1 oder 2 steht,
wobei Y¹ und Y² unabhängig voneinander -H, -NH₂, oder -(CH₂)₀₋₃Z' darstellen,
wobei Y³ -H, -(CH₂)₀₋₃-CH(NH-C(=O)-CH₃)Z', -(CH₂)₀₋₃-CH(NH₂)Z', oder -(CH₂)₀₋₃Z' darstellt,
wobei Z' -H, -SO₃H, -NH₂ oder -COOH darstellt,
R⁵ -H, -NH₂, -NOz, Halogen, -CN, -CF₃, -OCF₃, -CH₂F,-SH oder -(CH₂)₀₋₃Z darstellt,
wobei Z -H, -OY³, -SY³, Halogen, -NHY³, -C(=O)-NY¹Y², oder -C(=O)-OY³ darstellt,
wobei Y¹ und Y² unabhängig voneinander -H, -NH₂, oder -(CH₂)₀₋₃Z' darstellen,
wobei Y³ -H oder -(CH₂)₀₋₃Z' darstellt,
wobei Z' -H, -SO₃H, -NH₂ oder -COOH darstellt;
R⁶ und R⁷ unabhängig voneinander -H, -CN, C₁₋₁₀-Alkyl, Fluor-C₁₋₁₀-Alkyl, C₂₋₁₀-Alkenyl, Fluor-C₂₋₁₀-Alkenyl, C₂₋₁₀-Alkinyl, Fluor-C₂₋₁₀-Alkinyl,-OH, -NOz, -NH₂, -COOH oder Halogen darstellen,
R⁸ C₁₋₁₀-Alkyl, Fluor-C₁₋₁₀-Alkyl, C₂₋₁₀-Alkenyl, Fluor-C₂₋₁₀-Alkenyl, C₂₋₁₀-Alkinyl, Fluor-C₂₋₁₀-Alkinyl, C₄₋₁₀-Cycloalkyl, Fluor- C₄₋₁₀-Cycloalkyl oder -(CH₂)₀₋₂-(HZ²) darstellt,
wobei HZ² ein 4 bis 7-gliedriger Heterocyclus mit bis zu zwei Heteroatomen ausgewählt aus N, O und S darstellt, wobei jede dieser Gruppen mit -OH, -COOH oder -NH₂ substituiert sein kann;
R⁹ -H, -F, -CH₃, -CF₃, -CH₂F oder -CHF₂ darstellt;
-MOD -(NR¹⁰)ₙ-(G1)ₒ-G2-G3 darstellt,
wobei
R¹⁰ -H oder C₁-C₃-Alkyl darstellt;
G1 -NH-C(=O)-, -C(=O)NH- oder 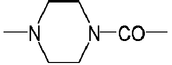 darstellt
n ; 0 oder 1 ist;
o 0 oder 1 ist; und
G2 eine geradkettige und/oder verzweigte Kohlenwasserstoffgruppe mit 1 bis 10 Kohlenstoffatomen ist, die einfach oder mehrfach, gleich oder verschieden durch die Gruppen -O-, -S-, -S(=O)-, S(=O)₂, -NR^{y}-, -NRYC(=O)-, -C(=O)NRY-, -NRYNRY-, -S(=O)₂-NR^{y}NR^{y}-, -C(=O)-NR^{y}NR^{y}-C(=O)-,-CR^{X}=N-O- unterbrochen sein kann, wobei die Kohlenwasserstoffkette of G2 einschließlich der Seitenketten, sofern vorhanden, mit -NH-C(=O)-NH₂, -COOH, -OH, -NH₂, -NH-CN-NH₂, Sulfonamid, Sulfon, Sulfoxid, oder Sulfonsäure substituiert sein kann,
wobei R^{X} -H, C₁-C₃-Alkyl oder Phenyl darstellt,
wobei R^{y} -H, Phenyl, C₁-C₁₀-Alkyl, C₂-C₁₀-Alkenyl, oder C₂-C₁₀-Alkinyl darstellt, die jeweils mit -NH-C(=O)-NH₂, -COOH, -OH, -NH₂, -NH-CN-NH₂, Sulfonamid, Sulfon, Sulfoxid, oder Sulfonsäure substituiert sein können,
G3 -H oder -COOH darstellt; und
wobei R¹, R² oder R³ -MOD darstellen; und
wobei die Gruppe -MOD zumindest eine Gruppe -COOH aufweist;

2. Verbindung nach Anspruch 1, wobei:
R⁶ und R⁷ jeweils -F darstellen;
R⁸ eine C₁₋₄-Alkyl-Gruppe darstellt; und
R⁹ -H darstellt.

3. Verbindung nach Anspruch 1 oder 2, wobei X₁ CH oder CF, X₂ C und X₃ N ist.

4. Verbidnung nach einem der Ansprüche 1 bis 3, wobei wobei A -C(=O)- darstellt; und R³ CH₂-OH, -CH₂OCH₃, -CH(CH₃)OH oder CH(CH₃)OCH₃ darstellt.

5. Verbindung nach einem der Ansprüche 1 bis 4, wobei R¹ -MOD darstellt.

6. Verbindung nach einem der Ansprüche 1 bis 5, wobei die Verbindung die folgende Strukturformel (VI) aufweist: 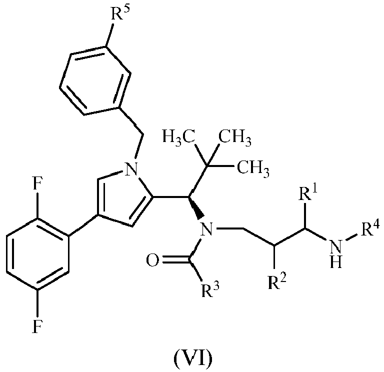 wobei:
R¹-MOD darstellt;
R² -H darstellt;
R³ -CH₂OH darstellt;
R⁴ -H darstellt; und
R⁵ -H darstellt.

7. Verbindung nach einem der Ansprüche 1 bis 6, wobei
-MOD -(NR¹⁰)ₙ-(G1)ₒ-G2-G3 darstellt, und
R¹⁰ -H oder C₁-C₃-Alkyl darstellt;
G1 -NH-C(=O)- , -C(=O)NH-darstellt ;
n 0 oder 1 ist;
o 0 oder 1 ist; und
G2 eine geradkettige und/oder verzweigte Kohlenwasserstoffgruppe mit 1 bis 10 Kohlenstoffatomen ist, die einfach oder mehrfach, gleich oder verschieden durch die Gruppen -O-, -S-, -S(=O)-, S(=O)₂, -NR^{y}-, -NRYC(=O)-, -C(=O)NRY-, -NRYNRY-, -S(=O)₂-NR^{y}NR^{y}-, -C(=O)-NR^{y}NR^{y}-C(=O)-,-CR^{x}=N-O- unterbrochen sein kann, wobei die Kohlenwasserstoffkette einschließlich der Seitenketten, sofern vorhanden, mit -NH-C(=O)-NH₂, -COOH, -OH, -NH₂, -NH-CN-NH₂, Sulfonamid, Sulfon, Sulfoxid, oder Sulfonsäure substituiert sein kann,
wobei R^{x} -H, C₁-C₃-Alkyl oder Phenyl darstellt,
wobei R^{y} -H, Phenyl, C₁-C₁₀-Alkyl, C₂-C₁₀-Alkenyl, oder C₂-C₁₀-Alkinyl darstellt, die jeweils mit -NH-C(=O)-NH₂, -COOH, -OH, -NH₂, -NH-CN-NH₂, Sulfonamid, Sulfon, Sulfoxid, oder Sulfonsäure substituiert sein können,
G3 -H oder -COOH darstellt; und
wobei die Gruppe -MOD zumindest eine Gruppe -COOH aufweist.

8. Verbindung nach einem der Ansprüche 1 bis 7, wobei
-MOD -(NR¹⁰)ₙ-(G1)ₒ-G2-G3 darstellt, n 0 ist; und o 1 ist.

9. Verbindung nach einem der Ansprüche 1 bis 8, wobei
-MOD -G1-G2-G3 darstellt;
G1 -CONH- darstellt;
G2 eine geradkettige und/oder verzweigte Kohlenwasserstoffgruppe mit 1 bis 10 Kohlenstoffatomen ist, die einfach oder mehrfach, gleich oder verschieden durch die Gruppen -O-, -S-, -NRY-,-NRYC(=O)-, -C(=O)NR^{Y}- unterbrochen sein kann; wobei die Kohlenwasserstofkette einschließlich Seitenketten durch -NH₂ or-COOH substituiert sein kann; und R^{y} -H oder C₁-C₁₀-Alkyl darstellt;
G3 -COOH darstellt; und
wobei die Gruppe -MOD zumindest eine Gruppe -COOH aufweist.

10. Verbindung nach einem der Ansprüche 1 bis 9, wobei R¹ ausgewählt is aus der Gruppe bestehend aus:
- CONH-CH₂CH₂-NHCO-CH₂-NHCO-CH(CH₂COOH)-S-CH₂CH(NH₂)-COOH;
- CONH-CH₂CH₂-NHCO-CH₂-NHCO-CH₂CH(COOH)-S-CH₂CH(NH₂)-COOH;
- CONH-CH(COOH)CH₂-NHCO-CH₂-NHCO-CH(CH₂COOH)-S-CH₂CH(NH₂)-COOH;
- CONH-CH(COOH)-CH₂-NHCO-CH₂-NHCO-CH₂CH(COOH)-S-CH₂CH(NH₂)-COOH;
- CONH-CH₂CH₂-O-(CH₂)₂-NHCO-CH(CH₂COOH)-S-CH₂CH(NH₂)-COOH;
- CONH-CH₂CH₂-O-CH₂CH₂-NHCO-CH₂CH(COOH)-S-CH₂CH(NH₂)-COOH;
- CONH-CH₂CH₂-NHCO-CH₂-S-CH₂CH(NH₂)-COOH;
- CONH-CH₂CH₂-NHCO-CH₂CH₂-CH(NH₂)-COOH;
- CONH-CH₂CH₂-CONH-(CH₂)₄-CH(NH₂)-COOH;
- CONH-CH₂CH₂-CONH-CH(COOH)-CH₂CH₂-COOH; und
- CONH-CH(COOH)-CH₂CH₂-COOH.

11. Verbindung nach einem der Ansprüche 1 bis 10, wobei R¹ -CONH-CH₂CH₂-CONH-CH(COOH)-CH₂CH₂-COOH darstellt.

12. Verbindung nach einem der Ansprüche 1 bis 11, wobei R¹ 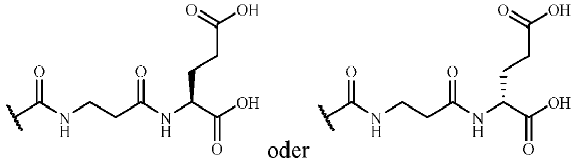 darstellt.

13. Verbindung nach einem der Ansprüche 1 bis 12, wobei die Verbindung folgende Struktur aufweist:

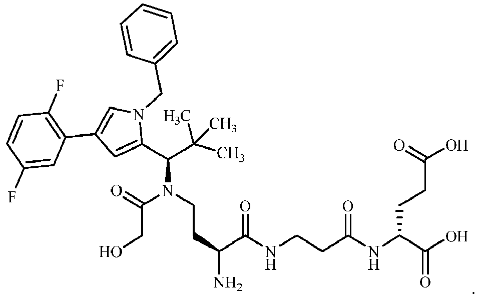

14. Verbindung nach einem der Ansprüche 1 bis 13, wobei die Verbindung folgende Strukturformel (VI) aufweist: 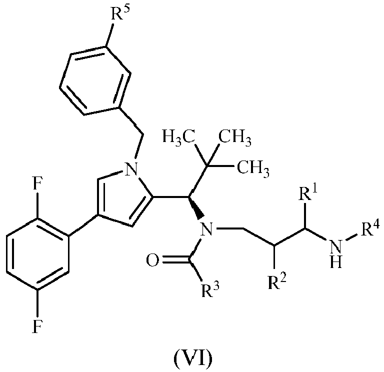 wobei:
R¹ -H darstellt;
R² -H darstellt;
R³ -MOD darstellt;
R⁴ -H darstellt; und
R⁵ -H darstellt.

15. Verbindung nach einem der Ansprüche 1 bis 14, wobei die Verbindung eine Verbindung aus Tabelle 3 umfasst.

16. Verbindung nach einem der Ansprüche 1 bis 15, wobei die Verbindung ein Efflux ratio zwischen 0,5 und 2 aufweist.

17. Verbindung nach einem der Ansprüche 1 bis 16, wobei die Verbindung eine Permeabilität (Pₐₚₚ) von einer basalen (B) Zellmembranoberfläche zu einer apikalen (A) Zellmembranoberfläche, Pₐₚₚ (B-A) aufweist, die weniger als 200 nm/s ist.

18. Verbindung nach einem der Ansprüche 1 bis 17, wobei die Verbindung eine Pₐₚₚ (B-A) aufweist, die weniger als 20 nm/s ist.

19. Verbindung nach einem der Ansprüche 1 bis 18, wobei
-MOD -G1-G2-G3 darstellt;
G1 -CONH- darstellt;
G2 eine Kohlenwasserstoffgruppe mit 1 bis 10 Kohlenstoffatomen ist, die durch die Gruppen -O-, -S-, -NHCO-, und/oder -CONH-unterbrochen sein kann; und durch -COOH substituiert ist; und
G3 -COOH darstellt.

20. Verbindung nach einem der Ansprüche 1 bis 19, welche eine Verbindung mit folgender Formel (VI) ist: 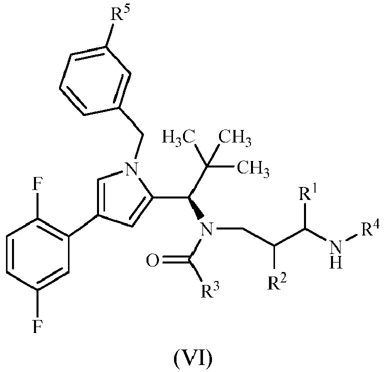 wobei:
R¹ -MOD darstellt;
R² -H darstellt;
R³ -CH₂OH darstellt;
R⁴ -H darstellt;
R⁵ -H darstellt; und
-MOD -CONH-CH₂CH₂-AM-(CHX)_{X}-COOH darstellt; wobei
X eine Zahl von 2 bis 6 darstellt;
jedes X unabhängig -H, NH₂ or -COOH darstellt; und
AM -CONH- oder -NHCO- darstellt.

21. Verbindung nach einem der Ansprüche 1 bis 20, wobei
-MOD -CONH-CH₂CH₂-CONH-CH(COOH)-CH₂CH₂-COOH; -CONH-CH₂CH₂-CONH-CH₂CH₂-CH(NH₂)-COOH; or -CONH-CH₂CH₂-CONH-(CH₂)₄-COOH darstellt.

22. Eine Verbindung die 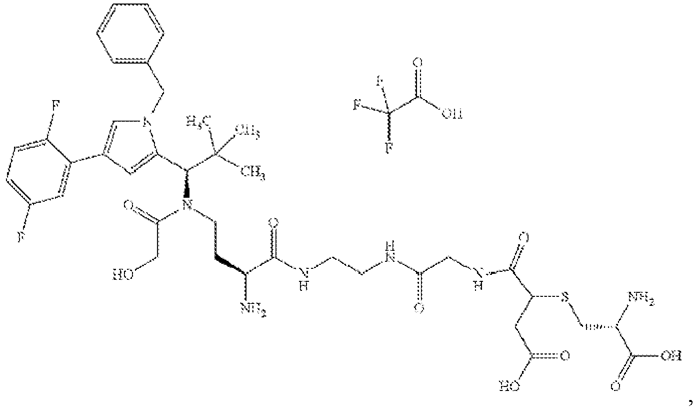 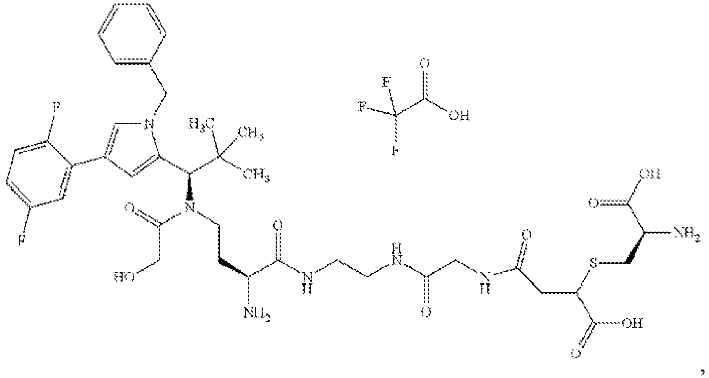 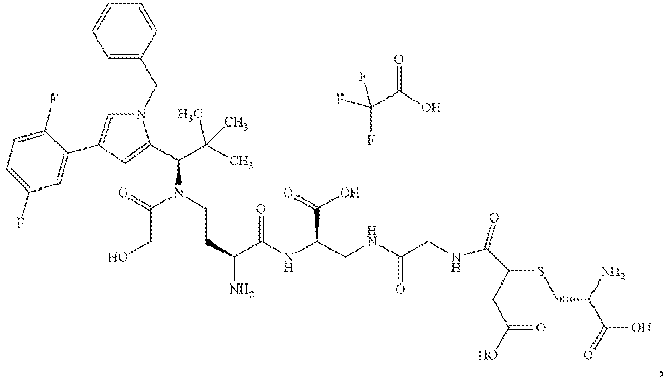 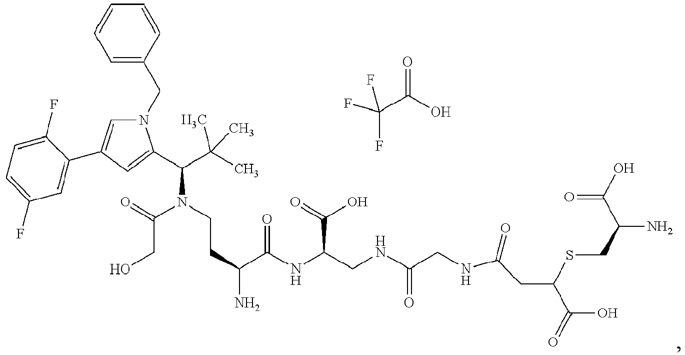 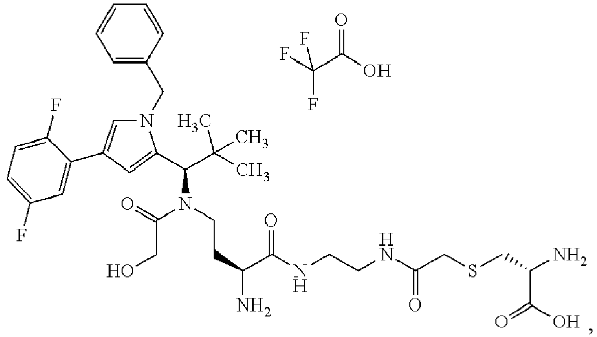 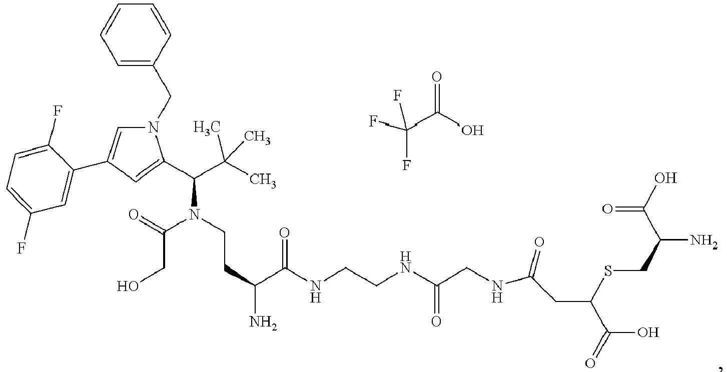 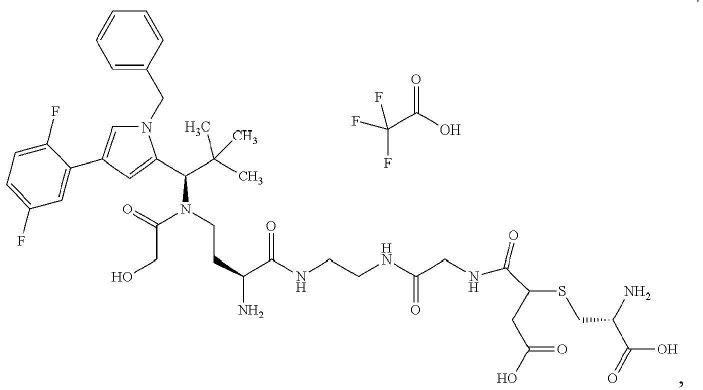 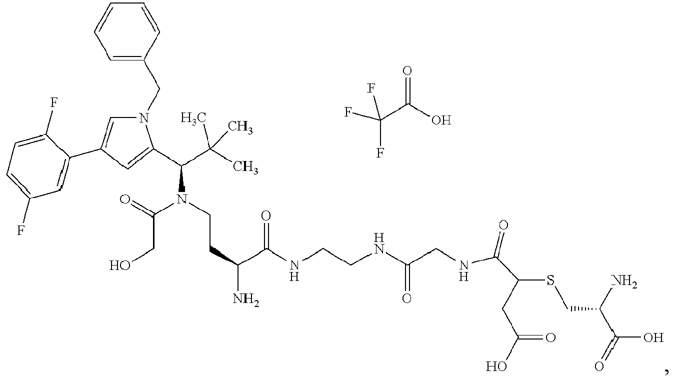 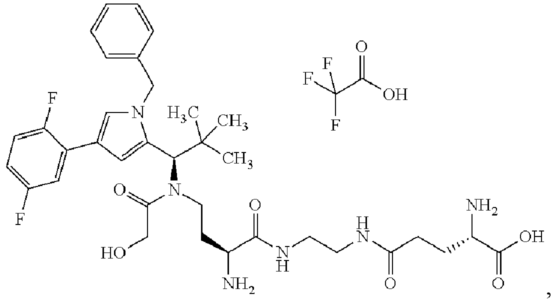 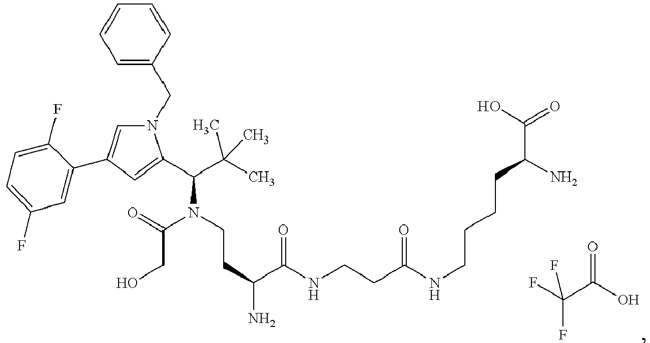 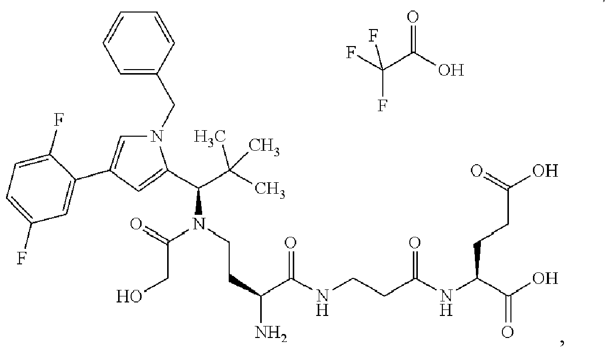 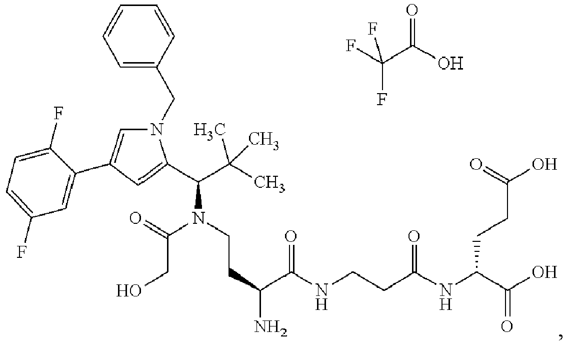 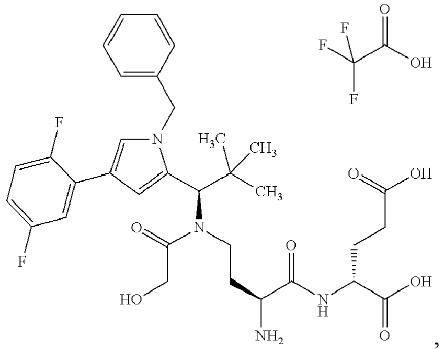 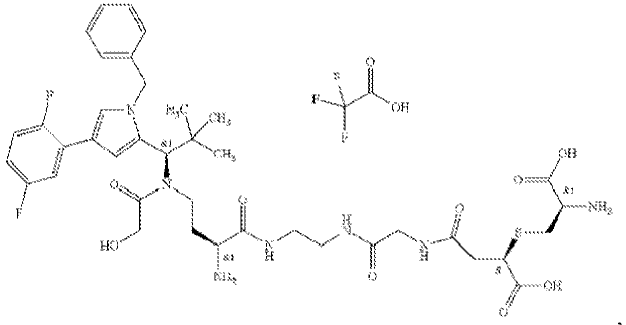 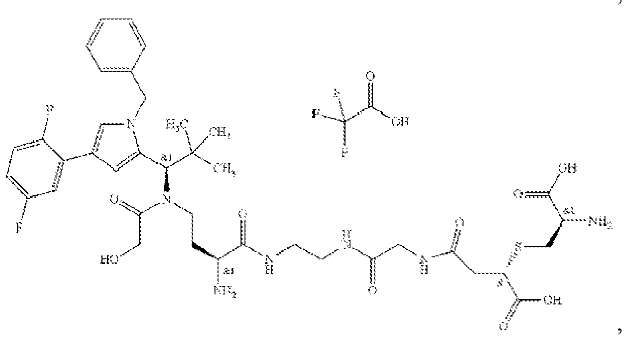 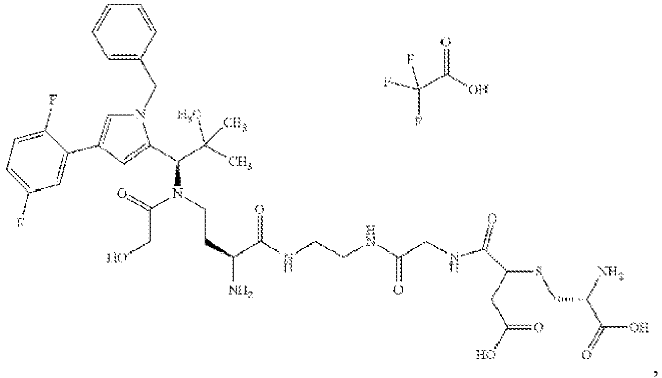 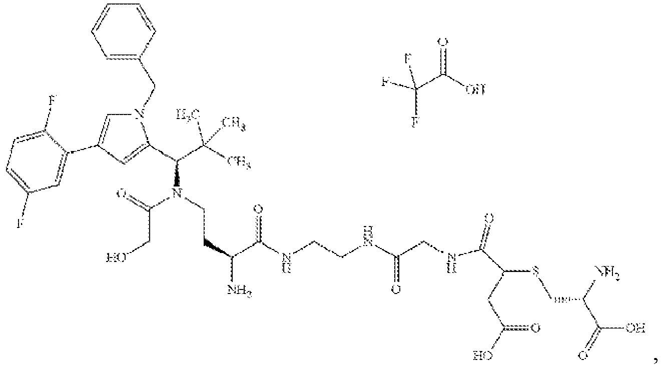 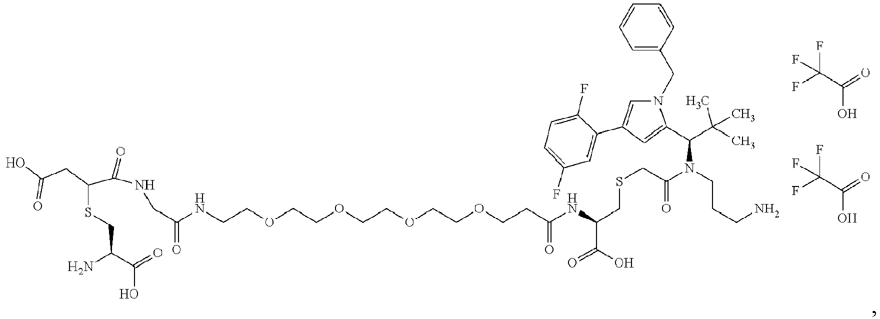 oder 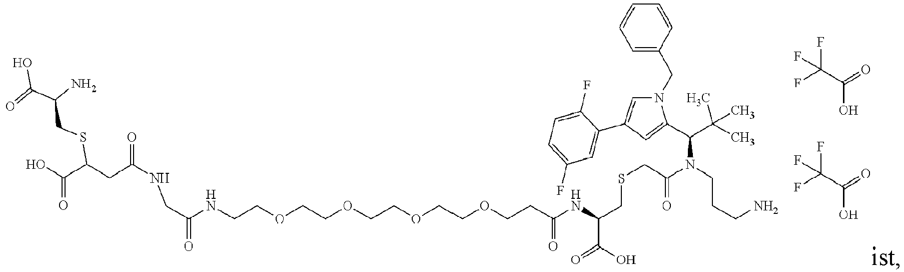 sowie ihre pharmzeutisch akzeptablen Salze, Solvate, Salze der Solvate und Epimere.

23. Zusammensetzung umfassend die Verbindung nach einem der Ansprüche 1 bis 22 oder ein pharmzeutisch akzeptables Salz, Solvat, Salz des Solvates und Epimere davon zur Verwendung in einem Verfahren zur Behandlung einer Krankheit oder gesundheitlichen Abweichung in einem Patienten mit Bedarf dafür.

24. Die Zusammensetzung zur Verwendung gemäß Anspruch 23, wobei die Krankheit oder gesundheitlich Abweichung eine hyperproliferative und/oder angiogene Krankheit oder gesundheitliche Abweichung ist.
